(19) Europäisches Patentamt
European Patent Office
Office européen des brevets

(11) EP 4 421 071 A1

(12) **EUROPEAN PATENT APPLICATION**
published in accordance with Art. 153(4) EPC

(43) Date of publication:
**28.08.2024 Bulletin 2024/35**

(21) Application number: **22882955.2**

(22) Date of filing: **20.10.2022**

(51) International Patent Classification (IPC):
***C07D 403/14*** *(2006.01)* ***A61K 31/4439*** *(2006.01)*
***A61K 31/4035*** *(2006.01)* ***A61P 31/00*** *(2006.01)*
***A61P 5/00*** *(2006.01)* ***A61P 9/00*** *(2006.01)*
***A61P 11/00*** *(2006.01)* ***A61P 35/00*** *(2006.01)*
***A61P 37/00*** *(2006.01)*

(52) Cooperative Patent Classification (CPC):
**A61K 31/4035; A61K 31/4439; A61K 31/4545; A61K 31/4709; A61K 31/4995; A61K 31/501; A61K 31/506; A61K 31/517; A61K 31/519; A61K 31/551; A61P 1/00; A61P 1/02; A61P 1/04; A61P 1/16; A61P 3/10;** (Cont.)

(86) International application number:
**PCT/CN2022/126516**

(87) International publication number:
**WO 2023/066350 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.10.2021 CN 202111231963**

(71) Applicant: **Gluetacs Therapeutics (Shanghai) Co., Ltd.**
**Shanghai 201306 (CN)**

(72) Inventors:
- **YANG, Xiaobao**
  **Shanghai 201306 (CN)**
- **SUN, Renhong**
  **Shanghai 201306 (CN)**
- **ZHAO, Baoyin**
  **Shanghai 201306 (CN)**

(74) Representative: **J A Kemp LLP**
**80 Turnmill Street**
**London EC1M 5QU (GB)**

(54) **CRBN E3 LIGASE LIGAND COMPOUND, PROTEIN DEGRADING AGENT DEVELOPED ON THE BASIS OF LIGAND COMPOUND, AND THEIR APPLICATIONS**

(57) The present disclosure provides compounds of Formula (I), (II) and (III) or salts, enantiomers, stereoisomers, solvates, isotopically enriched analogs, prodrugs, or polymorphs thereof, and uses thereof. The present disclosure also provides pharmaceutical compositions comprising, as an active ingredient, the compounds of Formula (I), (II) and (III) or salts, enantiomers, stereoisomers, solvates, isotopically enriched analogs, prodrugs, or polymorphs thereof, and uses thereof.

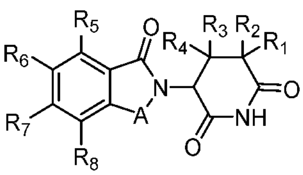

Formula (I)

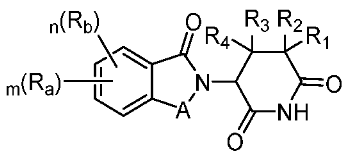

Formula (II)

PBM—$R^1$—$W^1$—LIN—ULM

Formula (III)

EP 4 421 071 A1

(52) Cooperative Patent Classification (CPC): (Cont.)
**A61P 5/00; A61P 5/14; A61P 7/06; A61P 9/00; A61P 9/04; A61P 9/10; A61P 11/00; A61P 15/00; A61P 17/00; A61P 17/08; A61P 17/14; A61P 19/02; A61P 19/06; A61P 25/00; A61P 27/04; A61P 29/00; A61P 31/00; A61P 31/04; A61P 31/06; A61P 31/14; A61P 31/18; A61P 33/06; A61P 35/00; A61P 35/02; A61P 37/00; A61P 37/06; C07D 401/04; C07D 401/14; C07D 403/14; C07D 405/14; C07D 417/14; C07D 471/04; C07D 471/08; C07D 487/04; C07D 487/08; C07D 495/14; C07D 519/00**

## Description

### Technical Field

The present disclosure relates to cereblon (CRBN) E3 ubiquitin ligase ligand compounds of Formula (I) and Formula (II), protein degrader compounds of Formula (III) comprising the same, and their applications. The CRBN E3 ubiquitin ligase ligand compounds can effectively treat or prevent cereblon protein mediated diseases or disorders. The protein degrader compounds of Formula (III) can effectively treat or prevent diseases or disorders associated with the proteins degraded.

### Background

Bifunctional proteolysis-targeting drugs consist of three moieties, one end of which is a ligand warhead that can bind to a specific target protein, and the other end is a small molecule ligand of E3 ubiquitin ligase, both of which are connected together through linkers of different lengths and categories located in the middle. The bifunctional proteolysis-targeting small molecules can utilize the binding effects of the ligands at both ends to simultaneously bind to a specific target protein and E3 ubiquitin ligase, thereby recruiting E3 ubiquitin ligase to the vicinity of the specific target protein and enabling E3 ubiquitin ligase to ubiquitinate the target protein. The polyubiquitinated target protein will be recognized and degraded by proteasomes in the body. The biggest difference between the bifunctional proteolysis-targeting drugs and traditional small molecule-based inhibitor drugs is that the former mobilize the entire cell as a drug effector unit. This drug action mode only requires small molecule drugs to briefly bind to the target protein and tag it for degradation. Therefore, a low drug dose can meet the requirements, greatly reducing the risk of off-target effects, and potentially eliminating tumor progression caused by abnormal expression of driver genes and drug resistance caused by acquired mutations in driver genes.

The E3 ubiquitin ligases involved in the design of proteolysis-targeting drugs include over 500 different proteins, each with a distinct cellular expression profile, and can be classified into multiple categories based on the structural elements related to their E3 functional activity. Currently, only a few ligases have been successfully applied in preclinical degraders research. Among them, Cereblon (CRBN) E3 ubiquitin ligase is one of the most widely used E3 ubiquitin ligases. The known ligands for CRBN-type E3 ubiquitin ligase (CRBN ligands) include thalidomide and its analogs pomalidomide and lenalidomide, all of which possess a phthalimide backbone. CRBN-type E3 ubiquitin ligase ligands can also act as molecular glues to induce the degradation of specific proteins. Cereblon (CRBN) forms a functional E3 ubiquitin ligase complex with damaged DNA binding protein 1 (DDB1) and Cullin 4A, where CRBN serves as the substrate receptor. Molecular glue degraders like thalidomide bind to CRBN, inducing it to recognize new substrate proteins. The binding leads to the ubiquitination of these proteins, followed by their recognition and degradation by proteasomes.

CRBN ligands have been widely used in protein degradation, and a series of small molecules protein degraders based on CRBN ubiquitin ligases, which utilize the function of the CRBN E3 ubiquitin ligase complex, have gradually been developed. Since CRBN ligands can serve not only as molecular glue degraders to induce the degradation of different substrate proteins but also as a component of bifunctional proteolysis-targeting drugs to achieve effective degradation of specific pathogenic proteins, and given the unavoidable side effects and drug resistance issues associated with various existing domides compounds, such as thalidomide, it is of great significance to design novel and optimized E3 ubiquitin ligase ligands and study their binding ability to CRBN and evaluate their biological activity. Furthermore, these E3 ubiquitin ligase ligands can potentially be applied in bifunctional proteolysis-targeting small molecules.

Therefore, there is an urgent need for a series of novel CRBN ligands that can serve as effective molecular glue degraders and can also be further used to synthesize corresponding bifunctional proteolysis-targeting degraders for the treatment and/or prevention of diseases or conditions mediated or associated with the proteins degraded.

### Summary of Invention

In view of the above, the objectives of the present disclosure are to provide novel CRBN ligands, protein degrader molecules designed based on the CRBN ligands and various target protein ligands, as well as their applications and usage methods. The advantages of the compounds of the present disclosure lie in their ability to exhibit a wide range of pharmacological activities through the degradation/inhibition of diverse types or families of pathogenic proteins.

To achieve the above objectives and other related goals, in one aspect, the present disclosure provides novel CRBN ligands that can themselves act as molecular glues binding to CRBN E3 ligase, and subsequently induce the degradation of CRBN substrate proteins, including but not limited to GSPT1, IKZF1, IKZF2, IKZF3 (Aiolos), and IKZF4 proteins.

In some embodiments, the novel CRBN ligands of the present disclosure can be compounds of Formula (I):

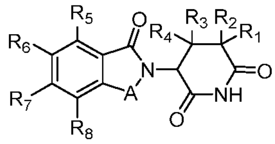

Formula (I)

or salts, stereoisomers (including enantiomers), solvates, isotopically enriched analogs, prodrugs, or polymorphs thereof

wherein A represents CO, $CH_2$ or $CD_2$;
$R_1$, $R_2$, $R_3$ and $R_4$ are the same or different and each independently represent e.g., hydrogen, deuterium, halogen, optionally deuterated $C_{1-6}$ alkyl, optionally deuterated $C_{1-6}$ alkoxy, or halogenated $C_{1-6}$ alkyl;
$R_5$, $R_6$, $R_7$ and $R_8$ are the same or different and each independently represent e.g., $R_9$, hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl, or $C_{2-6}$ alkynyl, wherein one to three of $R_5$, $R_6$, $R_7$ and $R_8$ represent $R_9$;
$R_9$ represents R-U- group, wherein

U represents a bond, or U represents $N(R_{10})$ or O, wherein $R_{10}$ represents e.g., H or optionally deuterated $C_{1-6}$ alkyl;
R represents optionally substituted nitrogen-containing heterocyclyl, said optionally substituted nitrogen-containing heterocyclyl is optionally substituted nitrogen-containing

monocyclic heterocyclyl or optionally substituted nitrogen-containing bridged heterocyclyl; wherein
when two to four of $R_5$, $R_6$, $R_7$, and $R_8$ independently represent $R_9$, the compound of Formula (I) is optionally partially or fully deuterated; or
when only one of $R_5$, $R_6$, $R_7$, and $R_8$ represents $R_9$, the compound of Formula (I) is optionally partially or fully deuterated;
wherein in case that only one of $R_5$, $R_6$, $R_7$, and $R_8$ represents $R_9$ and the compound of Formula (I) is not deuterated:

when R represents optionally substituted nitrogen-containing bridged heterocyclyl, the following compounds are excluded:

5-(2,5-diazabicyclo[2.2.1]heptan-2-yl)-2-(2,6-dioxopiperidin-3-yl)isoindoline-1,3-dione;
5-((1R,4R)2,5-diazabicyclo[2.2.1]heptan-2-yl)-2-(2,6-dioxopiperidin-3-yl)-6-fluoroisoindoline-1,3 -dione;
5-(2,5-diazabicyclo[2.2.1]heptan-2-yl)-2-(2,6-dioxopiperidin-3-yl)-6-fluoroisoindoline-1,3-dione;
3-(5-(2,5-diazabicyclo[2.2.1]heptan-2-yl)-1-oxoisoindolin-2-yl)piperidine-2,6-dione;
3-(5-(8-azabicyclo[3.2.1]octan-3-yl)-1-oxoisoindolin-2-yl)piperidine-2,6-dione; and
3-(5-(9-ethyl-3-methyl-3,9-diazabicyclo[3.3.1]nonan-7-yl)-1-oxoisoindolin-2-yl)piperidine-2,6-dione; or

when A represents CO and one of $R_6$ and $R_7$ represents $R_9$, where R represents substituted nitrogen-containing monocyclic heterocyclyl, and the other one of $R_6$ and $R_7$, as well as $R_5$ and

$R_8$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, one to three of the other one of $R_6$ and $R_7$ as well as $R_5$ and $R_8$ represents halogen, and the nitrogen-containing monocyclic heterocyclyl is substituted with one or more substituents selected from the group consisting of hydroxy, amino, mercapto, nitro, halogen, cyano, $C_{1-6}$ alkyl, $C_{1-6}$ alkoxy, halogenated $C_{1-6}$ alkyl, $NH_2$-$C_{1-6}$ alkylene, $C_{1-6}$ alkyl-NH-, $C_{1-6}$ alkyl-NHC(O)-, $C_{1-6}$ alkyl-C(O)NH- or any combination thereof;

when A represents CO and one of $R_6$ and $R_7$ represents $R_9$, where R represents unsubstituted nitrogen-containing monocyclic heterocyclyl, the other one of $R_6$ and $R_7$ represents hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, and $R_5$ and $R_8$ each independently represent halogen;
when A represents CO and one of $R_5$ and $R_8$ represents $R_9$, where R represents substituted nitrogen-containing monocyclic heterocyclyl, and the other one of $R_5$ and $R_8$, as well as $R_6$ and $R_7$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, one to three of the other one of $R_5$ and $R_8$ as well as $R_6$ and $R_7$ represent halogen, and

the nitrogen-containing monocyclic heterocyclyl is substituted with one or more substituents selected from the group consisting of hydroxy, amino, mercapto, nitro, halogen, cyano, CHO, $C_{1-6}$ alkyl, halogenated $C_{1-6}$ alkyl, $C_{3-6}$ cycloalkyl, $C_{1-6}$ alkoxy, $C_{1-6}$ alkyl-NH-, $NH_2$-$C_{1-6}$ alkylene, $C_{1-6}$ alkyl-NHC(O)-, $C_{1-6}$ alkyl-C(O)NH- or any combination thereof;

when A represents CO and one of $R_5$ and $R_8$ represents $R_9$, where R represents unsubstituted nitrogen-containing monocyclic heterocyclyl, the other one of $R_5$ and $R_8$ and/or $R_7$ each independently represent halogen, and $R_6$ represents hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl;

when A represents $CH_2$ and one of $R_6$ and $R_7$ represents $R_9$, where R represents substituted nitrogen-containing monocyclic heterocyclyl, and the other one of $R_6$ and $R_7$, as well as $R_5$ and $R_8$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, one to three of the other one of $R_6$ and $R_7$ as well as $R_5$ and $R_8$ represents halogen, and the nitrogen-containing monocyclic heterocyclyl is substituted with one or more substituents selected from the group consisting of hydroxy, amino, mercapto, nitro, halogen, CHO, cyano, $C_{1-6}$ alkyl, halogenated $C_{1-6}$ alkyl, $C_{3-6}$ cycloalkyl, $C_{1-6}$ alkoxy, $C_{1-6}$ alkyl-NH-, $NH_2$-$C_{1-6}$ alkylene, $C_{1-6}$ alkyl-NHC(O)-, $C_{1-6}$ alkyl-C(O)NH- or any combination thereof;

when A represents $CH_2$ and one of $R_6$ and $R_7$ represents $R_9$, where R represents unsubstituted nitrogen-containing monocyclic heterocyclyl, $R_5$ represents halogen, and the other one of $R_6$ and $R_7$ and/or $R_8$ each independently represent halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl;

when A represents $CH_2$ and one of $R_5$ and $R_8$ represents $R_9$, where R represents substituted nitrogen-containing monocyclic heterocyclyl, and the other one of $R_5$ and $R_8$, as well as $R_6$ and $R_7$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, one to three of the other one of $R_5$ and $R_8$ as well as $R_6$ and $R_7$ represent halogen, and the nitrogen-containing monocyclic heterocyclyl is substituted with one or more substituents selected from the group consisting of hydroxy, amino, mercapto, nitro, halogen, CHO, cyano, $C_{1-6}$ alkyl, halogenated $C_{1-6}$ alkyl, $C_{3-6}$ cycloalkyl, $C_{1-6}$ alkoxy, $C_{1-6}$ alkyl-NH-, $NH_2$-$C_{1-6}$ alkylene, $C_{1-6}$ alkyl-NHC(O)-, $C_{1-6}$ alkyl-C(O)NH- or any combination thereof; or

when A represents $CH_2$ and one of $R_5$ and $R_8$ represents $R_9$, where R represents unsubstituted nitrogen-containing monocyclic heterocyclyl, and the other one of $R_5$ and $R_8$, as well as $R_6$ and $R_7$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, the other one of $R_5$ and $R_8$ as well as $R_6$ and $R_7$ are not simultaneously hydrogen, and when $R_6$ is halogen, the other one of $R_5$ and $R_8$, as well as $R_7$ are not simultaneously hydrogen.

In another aspect, the CRBN ligands of the present disclosure can be compounds of Formula (II):

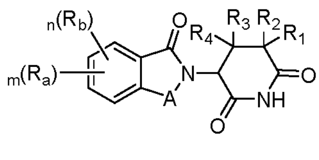

Formula (II)

or salts, stereoisomers (including enantiomers), solvates, isotopically enriched analogs, prodrugs, or polymorphs thereof

wherein A represents CO, $CH_2$ or $CD_2$;

$R_1$, $R_2$, $R_3$ and $R_4$ are the same or different and each independently represent e.g., hydrogen, deuterium, halogen, optionally deuterated $C_{1-6}$ alkyl, optionally deuterated $C_{1-6}$ alkoxy, or halogenated $C_{1-6}$ alkyl;

$(R_b)_n$ indicates that benzene ring is substituted with n $R_b$ substituents, and each $R_b$ are the same or different and each independently represent e.g., hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl or $C_{2-6}$ alkynyl;

$(R_a)_m$ indicates that the benzene ring is substituted with m $R_a$ substituents, and each $R_a$ are the same or different and each independently represents the structure of the following formula:

$$W\text{-}LIN\text{-}U_2\text{-}R_c\text{-}U_1\text{-} ,$$

wherein

$U_1$ and $U_2$ are the same or different and each independently represent e.g., a bond, $N(R_d)$ or O, wherein $R_d$ represents e.g., H or optionally deuterated $C_{1-6}$ alkyl;

$R_c$ represents optionally substituted nitrogen-containing monocyclic heterocyclylene or optionally substituted nitrogen-containing bridged heterocyclylene;

W represents hydrogen, optionally substituted nitrogen-containing monocyclic heterocyclyl, optionally substituted nitrogen-containing bridged heterocyclyl, optionally substituted cycloalkyl, optionally substituted heteroaryl, optionally substituted aryl, $C_{2-6}$ alkenyl, $C_{2-6}$ alkynyl, a leaving group, CHO, COOH, $NH_2$, hydroxy or mercapto; and wherein in case that the compound of Formula (II) is partially or fully deuterated:

when W represents optionally substituted nitrogen-containing monocyclic heterocyclyl, optionally substituted nitrogen-containing bridged heterocyclyl, optionally substituted cycloalkyl, optionally substituted heteroaryl or optionally substituted aryl, LIN represents a bond, optionally substituted methylene, or optionally substituted linear or branched $C_{2-30}$ alkylene, wherein one or more groups $R_e$ and/or one or more groups $R_f$ and/or any combination of one or more groups $R_e$ and $R_f$ are optionally inserted into the backbone carbon chain of the linear or branched $C_{2-30}$ alkylene group, wherein carbon-carbon bond between one or more pairs of adjacent carbon atoms in the backbone carbon chain is interrupted by the group $R_e$, $R_f$, or a combination of $R_e$ and $R_f$; wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl, and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of e.g., deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof; or

when W represents hydrogen, $C_{2-6}$ alkenyl, $C_{2-6}$ alkynyl, a leaving group, CHO, COOH, $NH_2$, hydroxy or mercapto, LIN represents optionally substituted methylene, or optionally substituted linear or branched $C_{2-30}$ alkylene, wherein one or more groups $R_e$ and/or one or more groups $R_f$ and/or any combination of one or more groups $R_e$ and $R_f$ are optionally inserted into the backbone carbon chain of the linear or branched $C_{2-30}$ alkylene group, wherein carbon-carbon bond between one or more pairs of adjacent carbon atoms in the backbone carbon chain is interrupted by the group $R_e$, $R_f$, or a combination of $R_e$ and $R_f$; wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl, and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of e.g., deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof; or

in case that the compound of Formula (II) is not deuterated, LIN represents optionally substituted methylene, and W represents optionally substituted nitrogen-containing monocyclic heterocyclyl or optionally substituted nitrogen-containing bridged heterocyclyl; and

m represents an integer of 1, 2, 3 or 4, n represents an integer of 0, 1, 2 or 3, and the sum of m and n is an integer of 1, 2, 3 or 4;

with the proviso that when the compound of Formula (II) is not deuterated, the following compounds are excluded:

3-(4,6-difluoro-1-oxo-5-(4-(piperidin-4-ylmethyl)piperazin-1-yl)isoindolin-2-yl)piperidine-2,6-dione;
2-(2,6-dioxopiperidin-3-yl)-5-(4-(piperidin-4-ylmethyl)piperazin-1-yl)isoindoline-1,3-dione;
3-(4-chloro-1-oxo-5-(4-(piperidin-4-ylmethyl)piperidin-1-yl)isoindolin-2-yl)piperidine-2,6-dione;
5-(4-(azetidin-3-ylmethyl)piperazin-1-yl)-2-(2,6-dioxopiperidin-3-yl)isoindoline-1,3-dione; and
2-(2,6-dioxopiperidin-3-yl)-5-(3-(piperazin-1-ylmethyl)azetidin-1-yl)isoindoline-1,3-dione.

In another aspect, the protein degrader molecules of the present disclosure are compounds of Formula (III):

PBM-$R^1$-$W^1$-LIN-ULM Formula (III)

or salts, stereoisomers (including enantiomers), solvates, isotopically enriched analogs, prodrugs, or polymorphs thereof,

wherein PBM-$R^1$-$W^1$- represents a targeting moiety capable of binding protein, LTLM represents a E3 ubiquitin ligase ligand moiety, LIN is a linker moiety, and PBM-$R^1$-$W^1$- is covalently bonded to LTLM through LIN;
LTLM represents the structure of Formula (LTLM):

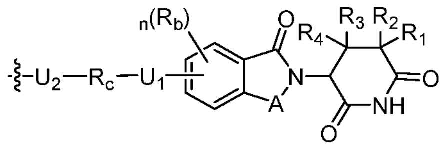

Formula (ULM)

wherein A represents CO, $CH_2$ or $CD_2$;
$R_1$, $R_2$, $R_3$ and $R_4$ are the same or different and each independently represent e.g., hydrogen, deuterium, halogen, optionally deuterated $C_{1-6}$ alkyl, optionally deuterated $C_{1-6}$ alkoxy, or halogenated $C_{1-6}$ alkyl;
$(R_b)_n$ indicates that benzene ring is optionally substituted with n $R_b$ groups, and each $R_b$ are the same or different and each independently represent e.g., hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl or $C_{2-6}$ alkynyl;
n represents an integer of 0, 1, 2 or 3;
$U_1$ and $U_2$ are the same or different and each independently represent e.g., a bond, $N(R_d)$ or O, wherein $R_d$ represents e.g., H or optionally deuterated $C_{1-6}$ alkyl;
$R_c$ represents optionally substituted nitrogen-containing monocyclic heterocyclylene or optionally substituted nitrogen-containing bridged heterocyclylene;

LIN represents a bond, optionally substituted methylene, or optionally substituted linear or branched $C_{2-30}$ alkylene, wherein one or more groups $R_e$ and/or one or more groups $R_f$ and/or any combination of one or more groups $R_e$ and $R_f$ are optionally inserted into the backbone carbon chain of the linear or branched $C_{2-30}$ alkylene group, wherein carbon-carbon bond between one or more pairs of adjacent carbon atoms in the backbone carbon chain is interrupted by the group $R_e$, $R_f$, or a combination of $R_e$ and $R_f$; wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl, and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of e.g., deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof;
$W^1$ is t interconnected rings $W^2$ and represented by the following formula:

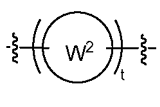

each ring $W^2$ are the same or different and each independently represent optionally substituted nitrogen-containing monocyclic heterocyclylene, optionally substituted nitrogen-containing bridged heterocyclylene, or optionally substituted cycloalkylene, and
t represents an integer of 1 or 2; and

$R^1$ represents:

a bond, -O-, optionally substituted $C_{1-6}$ alkylene (e.g., methylene), -N($R^{1a}$)-$R^{2a}$-*** (e.g.,

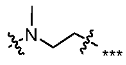, 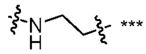),

-O-$R^{2a}$-***

(e.g., 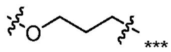, 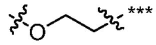),

-C(O)NH-$R^{2a}$-***, or-NHC(O)-$R^{2a}$ -*** (e.g.,

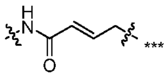

), wherein symbol *** indicates the point of attachment to $W^1$, $R^{1a}$ is H or $C_{1-6}$ alkyl, and $R^{2a}$ is optionally substituted $C_{1-6}$ alkylene or optionally substituted $C_{1-6}$ alkenylene; or

$C_{6-10}$ aryl or heteroaryl containing one or two 5- to 7-membered rings and 1 to 4 heteroatoms selected from nitrogen, oxygen, and sulfur, wherein the $C_{6-10}$ aryl and heteroaryl are each independently optionally substituted with one or more $R^{3a}$, and each $R^{3a}$ are the same or different and are each independently e.g., halogen, hydroxy, cyano, amino, nitro, $C_{1-6}$ alkyl, halogenated $C_{1-6}$ alkyl, $C_{1-6}$ alkoxy, or halogenated $C_{1-6}$ alkoxy;

wherein one or more hydrogens of the compound of Formula (III) are optionally replaced with deuterium;
wherein when the compound of Formula (III) is not deuterated, the following compounds are excluded:

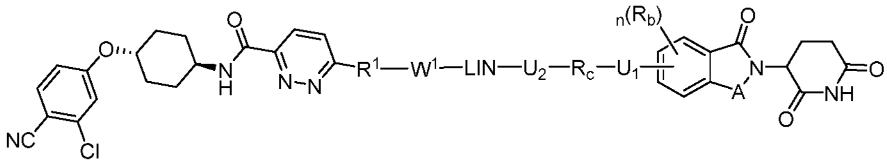

Formula (IV-1)

in Formula (IV-1), $R^1$ represents a bond, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, n represents an integer of 1, 2 or 3, and $U_1$ and $U_2$ are the same or different and each independently represent a bond, NH or O; or
in Formula (IV-1), $R^1$ represents a bond, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, n represents an integer of 1, 2 or 3, and $U_1$ and $U_2$ are the same or different and each independently represent a bond, NH or O;

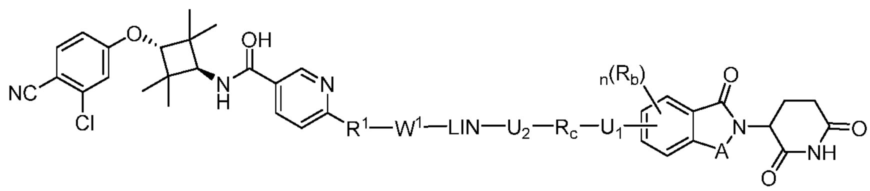

Formula (IV-2)

in Formula (IV-2), $R^1$ represents a bond, $W^1$ represents piperidinylene, LIN represents methylene, $R_c$ represents

piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, n represents an integer of 1, 2 or 3, and $U_1$ and $U_2$ are the same or different and each independently represent a bond, NH or O; or

in Formula (IV-2), $R^1$ represents a bond, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, and n represents an integer of 1, 2 or 3, 以及 $U_1$ and $U_2$ are the same or different and each independently represent a bond, NH or O;

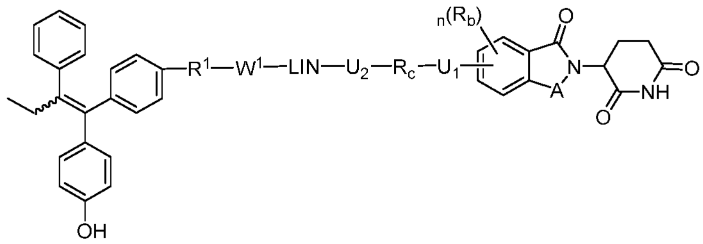

Formula (IV-3)

in Formula (IV-3), $R^1$ represents a bond or $-O-CH_2-CH_2-$, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O; or

in Formula (IV-3), $R^1$ represents a bond or $-O-CH_2-CH_2-$, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O; and

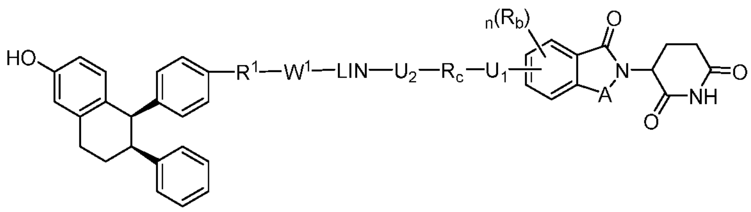

Formula (IV-4)

in Formula (IV-4), $R^1$ represents a bond or represents $-O-CH_2-CH_2-$, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O; or

in Formula (IV-4), $R^1$ represents a bond or represents $-O-CH_2-CH_2-$, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O.

In a further aspect, the present disclosure provides a pharmaceutical composition comprising the compound of Formula (I) or salts, stereoisomers (including enantiomers), solvates, isotopically enriched analogs, prodrugs, or polymorphs thereof, and at least one pharmaceutically acceptable carrier.

In a further aspect, the present disclosure provides a pharmaceutical composition comprising the compound of Formula (II) or salts, stereoisomers (including enantiomers), solvates, isotopically enriched analogs, prodrugs, or polymorphs thereof, and at least one pharmaceutically acceptable carrier.

In a further aspect, the present disclosure provides a pharmaceutical composition comprising the compound of Formula (III) or salts, stereoisomers (including enantiomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt, or the pharmaceutical composition comprising the same; or the compound of Formula (II) or a pharmaceutically acceptable salt, or the pharmaceutical composition comprising the same; or the compound of Formula (III) or a pharmaceutically acceptable salt, or the pharmaceutical composition comprising the same.

In a further aspect, the present disclosure provides the compound of Formula (I) or salts, stereoisomers (including enantiomers), solvates, isotopically enriched analogs, prodrugs, or polymorphs thereof, or the pharmaceutical composition comprising the same as active ingredient for use as a medicament.

In a further aspect, the present disclosure provides the compound of Formula (I) or salts, stereoisomers (including enantiomers), solvates, isotopically enriched analogs, prodrugs, or polymorphs thereof, or the pharmaceutical composition comprising the same as active ingredient for use in the prevention or treatment of cereblon protein-mediated disease or disorder.

In some embodiments, the cereblon protein-mediated disease or disorder includes, but not limited to, tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, or diabetes.

In a further aspect, the present disclosure provides the compound of Formula (II) or salts, stereoisomers (including enantiomers), solvates, isotopically enriched analogs, prodrugs, or polymorphs thereof, or the pharmaceutical composition comprising the same as active ingredient for use as a medicament.

In a further aspect, the present disclosure provides the compound of Formula (II) or salts, stereoisomers (including enantiomers), solvates, isotopically enriched analogs, prodrugs, or polymorphs thereof, or the pharmaceutical composition comprising the same as active ingredient for use in the prevention or treatment of cereblon protein-mediated disease or disorder.

In a further aspect, the present disclosure provides the compound of Formula (III) or salts, stereoisomers (including enantiomers), solvates, isotopically enriched analogs, prodrugs, or polymorphs thereof, or the pharmaceutical composition comprising the same as active ingredient for use as a medicament.

In a further aspect, the present disclosure provides the compound of Formula (III) or salts, stereoisomers (including enantiomers), solvates, isotopically enriched analogs, prodrugs, or polymorphs thereof, or the pharmaceutical composition comprising the same as active ingredient for use in the treatment and/or prevention of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, or thyroid disease.

In a further aspect, the present disclosure provides use of the compound of Formula (I) or salts, stereoisomers (including enantiomers), solvates, isotopically enriched analogs, prodrugs, or polymorphs thereof for the preparation of the compound of Formula (III) or salts, enantiomers, stereoisomers, solvates, isotopically enriched analogs, prodrugs, or polymorphs thereof of the present disclosure.

In a further aspect, the present disclosure also provides a method for preparing the compound of Formula (III) or salts, enantiomers, stereoisomers, solvates, isotopically enriched analogs, prodrugs, or polymorphs thereof of the present disclosure by using the compound of Formula (I) or salts, stereoisomers (including enantiomers), solvates, isotopically enriched analogs, prodrugs, or polymorphs thereof.

In a further aspect, the present disclosure provides use of the compound of Formula (II) or salts, stereoisomers (including enantiomers), solvates, isotopically enriched analogs, prodrugs, or polymorphs thereof for the preparation of the compound of Formula (III) or salts, enantiomers, stereoisomers, solvates, isotopically enriched analogs, prodrugs, or polymorphs thereof.

In a further aspect, the present disclosure also provides a method for preparing the compound of Formula (III) or salts, enantiomers, stereoisomers, solvates, isotopically enriched analogs, prodrugs, or polymorphs thereof of the present disclosure by using the compound of Formula (II) or salts, stereoisomers (including enantiomers), solvates, isotopically enriched analogs, prodrugs, or polymorphs thereof.

In a further aspect, the present disclosure provides use of the compound of Formula (I) or salts, stereoisomers (including enantiomers), solvates, isotopically enriched analogs, prodrugs, or polymorphs thereof for the manufacture of a medicament for the prevention or treatment of cereblon protein-mediated disease or disorder.

In a further aspect, the present disclosure provides use of the compound of Formula (II) or salts, stereoisomers (including enantiomers), solvates, isotopically enriched analogs, prodrugs, or polymorphs thereof for the manufacture of a medicament for the prevention or treatment of cereblon protein-mediated disease or disorder.

In a further aspect, the present disclosure provides use of the compound of Formula (III) or salts, stereoisomers (including enantiomers), solvates, isotopically enriched analogs, prodrugs, or polymorphs thereof for the manufacture of a medicament for the prevention and/or treatment of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, or thyroid disease.

In a further aspect, the present disclosure provides a method for treating or preventing a cereblon protein-mediated disease or disorder in a subject, comprising administering to the subject a therapeutically effective amount of the compound of Formula (I) or salts, enantiomers, stereoisomers, solvates, isotopically enriched analogs, prodrugs, or polymorphs thereof, or the pharmaceutical composition comprising the same.

In a further aspect, the present disclosure provides a method for treating or preventing a cereblon protein-mediated disease or disorder in a subject, comprising administering to the subject a therapeutically effective amount of the compound of Formula (II) or salts, enantiomers, stereoisomers, solvates, isotopically enriched analogs, prodrugs, or polymorphs thereof, or the pharmaceutical composition comprising the same.

In a further aspect, the present disclosure provides a method for treating or preventing a disease or disorder in a subject, comprising administering to the subject a therapeutically effective amount of the compound of Formula (III) or salts, enantiomers, stereoisomers, solvates, isotopically enriched analogs, prodrugs, or polymorphs thereof, or the pharmaceutical composition comprising the same, wherein the disease or disorder comprises tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

## Brief Description of Drawings

Figure 1 shows that the compounds of the present invention can effectively inhibit the proliferation of the tumor cell line DU-145, with a better inhibitory effect than the positive control enzalutamide.

Figure 2 illustrates the degradation ability of the compounds of the present invention on full-length androgen receptor (AR-FL) and androgen receptor splice variant 7 (AR-V7) proteins in human prostate cancer 22RV1 cells, as determined by Westem-blot.

Figure 3 illustrates the degradation ability of the compounds of the present invention on full-length androgen receptor (AR-FL) protein in human prostate cancer C4-2 cells, as determined by Westem-blot.

## Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

## I. Compounds

### Compounds of Formula (I)

The present disclosure provides a compound of Formula (I) or salts (including pharmaceutically acceptable salts), stereoisomers (including enantiomers), solvates, isotopically enriched analogs, prodrugs, or polymorphs thereof:

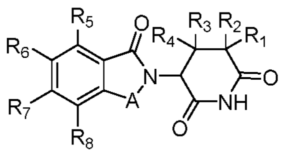

Formula (I)

wherein A, $R_1$, $R_2$, $R_3$, $R_4$, $R_5$, $R_6$, $R_7$ and $R_8$ are as defined in the compounds of Formula (I) above.

In some embodiments, $R_1$, $R_2$, $R_3$ and $R_4$ of the compound of Formula (I) are the same or different and each independently represent hydrogen, deuterium, halogen (e.g., fluorine, chlorine, bromine, or iodine), optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-).

In some embodiments, $R_1$, $R_2$, $R_3$ and $R_4$ of the compound of Formula (I) are the same or different and each independently represent hydrogen or deuterium.

In some embodiments, $R_1$, $R_2$, $R_3$ and $R_4$ of the compound of Formula (I) represents hydrogen.

In some embodiments, A of the compound of Formula (I) represents CO.

In some embodiments, A of the compound of Formula (I) represents $CH_2$.

In some embodiments, A of the compound of Formula (I) represents $CD_2$.

In some embodiments, when two to four (e.g., 2, 3, or 4) of $R_5$, $R_6$, $R_7$ and $R_8$ of the compound of Formula (I) independently represent $R_9$, the compound of Formula (I) is optionally partially deuterated, for example, carrying 1 to 20 deuterium atoms (e.g., 1-15, 1-10, 1-6, 1-5, 1-4, 1-3, 1-2, or 1 deuterium atom), or the compound of Formula (I) is optionally fully deuterated. The number of deuterium atoms is not theoretically limited in any way or automatically limited by the size of the building units.

In some embodiments, when only one of $R_5$, $R_6$, $R_7$ and $R_8$ of the compound of Formula (I) represents $R_9$, the compound of Formula (I) is partially deuterated, for example, carrying 1 to 15 deuterium atoms (e.g., 1-10, 1-6, 1-5, 1-4, 1-3, 1-2, or 1 deuterium atom), or the compound of Formula (I) is fully deuterated. The number of deuterium atoms is not theoretically limited in any way or automatically limited by the size of the building units. In a sub-embodiment, when $R_5$ of the compound of Formula (I) represents $R_9$, $R_6$ and $R_7$ as well as $R_8$ are the same or different and each independently represent hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl, or $C_{2-6}$ alkynyl. In a sub-embodiment, when $R_6$ of the compound of Formula (I) represents $R_9$, $R_5$ and $R_7$ as well as $R_8$ are the same or different and each independently represent hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl, or $C_{2-6}$ alkynyl. In a sub-embodiment, when $R_7$ of the compound of Formula (I) represents $R_9$, $R_5$ and $R_6$ as well as $R_8$ are the same or different and each independently represent hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl, or $C_{2-6}$ alkynyl. In a sub-embodiment, when $R_8$ of the compound of Formula (I) represents $R_9$, $R_5$ and $R_6$ as well as $R_7$ are the same or different and each independently represent hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl, or $C_{2-6}$ alkynyl.

In some embodiments, in case that only one of $R_5$, $R_6$, $R_7$ and $R_8$ of the compound of Formula (I) represents $R_9$ and the compound of Formula (I) is not deuterated, when R represents optionally substituted nitrogen-containing bridged heterocyclyl, the compound of Formula (I) does not comprise the following compounds:

5-(2,5-diazabicyclo[2.2.1]heptan-2-yl)-2-(2,6-dioxopiperidin-3-yl)isoindoline-1,3-dione;
5-((1R,4R)-2,5-diazabicyclo[2.2.1]heptan-2-yl)-2-(2,6-dioxopiperidin-3-yl)-6-fluoroisoindoline-1,3-dione;
5-(2,5-diazabicyclo[2.2.1]heptan-2-yl)-2-(2,6-dioxopiperidin-3-yl)-6-fluoroisoindoline-1,3-dione;
3-(5-(2,5-diazabicyclo[2.2.1]heptan-2-yl)-1-oxoisoindolin-2-yl)piperidine-2,6-dione;
3-(5-(8-azabicyclo[3.2.1]octan-3-yl)-1-oxoisoindolin-2-yl)piperidine-2,6-dione; and
3-(5-(9-ethyl-3-methyl-3,9-diazabicyclo[3.3.1]nonan-7-yl)-1-oxoisoindolin-2-yl)piperidine-2,6-dione.

In some embodiments, in case that only one of $R_5$, $R_6$, $R_7$ and $R_8$ of the compound of Formula (I) represents $R_9$ and the compound of Formula (I) is not deuterated, when A represents CO and one of $R_6$ and $R_7$ represents $R_9$, where R represents substituted nitrogen-containing monocyclic heterocyclyl, and the other one of $R_6$ and $R_7$, as well as $R_5$ and $R_8$ each independently represent hydrogen, halogen (e.g., fluorine, chlorine, bromine, or iodine), hydroxy, mer-

capto, nitro, amino, cyano, $C_{1-6}$ alkyl (e.g., $C_{1-4}$ alkyl, such as methyl, ethyl, propyl, isopropyl, butyl, sec-butyl or tert-butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl (e.g., vinyl, propenyl, or butenyl), or $C_{2-6}$ alkynyl (e.g., ethynyl, propynyl, or butynyl), one to three (e.g., 1, 2 or 3) of the other one of $R_6$ and $R_7$ as well as $R_5$ and $R_8$ represents halogen, and the nitrogen-containing monocyclic heterocyclyl is substituted with one or more (e.g., 1-6, such as 1, 2, 3, 4, 5 or 6) substituents selected from the group consisting of hydroxy, amino, mercapto, nitro, halogen (e.g., fluorine, chlorine, bromine, or iodine), cyano, $C_{1-6}$ alkyl (e.g., $C_{1-4}$ alkyl, such as methyl, ethyl, propyl, isopropyl, butyl, sec-butyl or tert-butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-6}$ alkyl-NH- (e.g., $C_{1-3}$ alkyl-NH-, such as $CH_3NH$-, $CH_3CH_2NH$- or $CH_3CH_2CH_2NH$-), $C_{1-6}$ alkyl-NHC(O)- (e.g., $CH_3$-NHC(O)-, $CH_3CH_2$-NHC(O)-, and $CH_3CH_2CH_2$-NHC(O)-), $C_{1-6}$ alkyl-C(O)NH- (e.g., $CH_3$-C(O)NH-, $CH_3CH_2$-C(O)NH-, and $CH_3CH_2CH_2$-C(O)NH-) or any combination thereof. The number of substituents is not theoretically limited in any way or automatically limited by the size of the building units.

In some embodiments, in case that only one of $R_5$, $R_6$, $R_7$ and $R_8$ of the compound of Formula (I) represents $R_9$ and the compound of Formula (I) is not deuterated, when A represents CO and one of $R_6$ and $R_7$ represents $R_9$, where R represents unsubstituted nitrogen-containing monocyclic heterocyclyl, the other one of $R_6$ and $R_7$ represents hydrogen, halogen (e.g., fluorine, chlorine, bromine, or iodine), hydroxy, mercapto, nitro, amino, cyano, $C_{1-6}$ alkyl (e.g., $C_{1-4}$ alkyl, such as methyl, ethyl, propyl, isopropyl, butyl, sec-butyl or tert-butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl (e.g., vinyl, propenyl, or butenyl), or $C_{2-6}$ alkynyl (e.g., ethynyl, propynyl, or butynyl), and $R_5$ and $R_8$ each independently represent halogen (e.g., fluorine, chlorine, bromine, or iodine).

In some embodiments, in case that only one of $R_5$, $R_6$, $R_7$ and $R_8$ of the compound of Formula (I) represents $R_9$ and the compound of Formula (I) is not deuterated, when A represents CO and one of $R_5$ and $R_8$ represents $R_9$, where R represents substituted nitrogen-containing monocyclic heterocyclyl, and the other one of $R_5$ and $R_8$, as well as $R_6$ and $R_7$ each independently represent hydrogen, halogen (e.g., fluorine, chlorine, bromine, or iodine), hydroxy, mercapto, nitro, amino, cyano, $C_{1-6}$ alkyl (e.g., $C_{1-4}$ alkyl, such as methyl, ethyl, propyl, isopropyl, butyl, sec-butyl or tert-butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl (e.g., vinyl, propenyl, or butenyl), or $C_{2-6}$ alkynyl (e.g., ethynyl, propynyl, or butynyl), one to three (e.g., 1, 2 or 3) of the other one of $R_5$ and $R_8$ as well as $R_6$ and $R_7$ represents halogen (e.g., fluorine, chlorine, bromine, or iodine), and the nitrogen-containing monocyclic heterocyclyl is substituted with one or more (e.g., 1-6, such as 1, 2, 3, 4, 5 or 6) substituents selected from the group consisting of hydroxy, amino, mercapto, nitro, halogen (e.g., fluorine, chlorine, bromine, or iodine), cyano, CHO, $C_{1-6}$ alkyl (e.g., $C_{1-4}$ alkyl, such as methyl, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{3-6}$ cycloalkyl (e.g., cyclopropyl, cyclobutyl, cyclopentyl, or cyclohexyl), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl-NH- (e.g., $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-6}$ alkyl-NHC(O)- (e.g., $CH_3$-NHC(O)-, $CH_3CH_2$-NHC(O)-, and $CH_3CH_2CH_2$-NHC(O)-), $C_{1-6}$ alkyl-C(O)NH- (e.g., $CH_3$-C(O)NH-, $CH_3CH_2$-C(O)NH-, and $CH_3CH_2CH_2$-C(O)NH-) or any combination thereof. The number of substituents is not theoretically limited in any way or automatically limited by the size of the building units.

In some embodiments, in case that only one of $R_5$, $R_6$, $R_7$ and $R_8$ of the compound of Formula (I) represents $R_9$ and the compound of Formula (I) is not deuterated, when A represents CO and one of $R_5$ and $R_8$ represents $R_9$, where R represents unsubstituted nitrogen-containing monocyclic heterocyclyl, the other one of $R_5$ and $R_8$ and/or $R_7$ each independently represent halogen, and $R_6$ represents hydrogen, halogen (e.g., fluorine, chlorine, bromine, or iodine), hydroxy, mercapto, nitro, amino, cyano, $C_{1-6}$ alkyl (e.g., $C_{1-4}$ alkyl, such as methyl, ethyl, propyl, isopropyl, butyl, sec-butyl or tert-butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl (e.g., vinyl, propenyl, or butenyl), or $C_{2-6}$ alkynyl (e.g., ethynyl, propynyl, or butynyl).

In some embodiments, in case that only one of $R_5$, $R_6$, $R_7$ and $R_8$ of the compound of Formula (I) represents $R_9$ and the compound of Formula (I) is not deuterated, when A represents $CH_2$ and one of $R_6$ and $R_7$ represents $R_9$, where R represents substituted nitrogen-containing monocyclic heterocyclyl, and the other one of $R_6$ and $R_7$, as well as $R_5$ and $R_8$ each independently represent hydrogen, halogen (e.g., fluorine, chlorine, bromine, or iodine), hydroxy, mercapto, nitro, amino, cyano, $C_{1-6}$ alkyl (e.g., $C_{1-4}$ alkyl, such as methyl, ethyl, propyl, isopropyl, butyl, sec-butyl or tert-

butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl (e.g., vinyl, propenyl, or butenyl), or $C_{2-6}$ alkynyl (e.g., ethynyl, propynyl, or butynyl), one to three (e.g., 1, 2 or 3) of the other one of $R_6$ and $R_7$ as well as $R_5$ and $R_8$ represents halogen (e.g., fluorine, chlorine, bromine, or iodine), and the nitrogen-containing monocyclic heterocyclyl is substituted with one or more (e.g., 1-6, such as 1, 2, 3, 4, 5 or 6) substituents selected from the group consisting of hydroxy, amino, mercapto, nitro, halogen (e.g., fluorine, chlorine, bromine, or iodine), CHO, cyano, $C_{1-6}$ alkyl (e.g., $C_{1-4}$ alkyl, such as methyl, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{3-6}$ cycloalkyl (e.g., cyclopropyl, cyclobutyl, cyclopentyl, or cyclohexyl), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl-NH- (e.g., $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-6}$ alkyl-NHC(O)- (e.g., $CH_3$-NHC(O)-, $CH_3CH_2$-NHC(O)-, and $CH_3CH_2CH_2$-NHC(O)-), $C_{1-6}$ alkyl-C(O)NH- (e.g., $CH_3$-C(O)NH-, $CH_3CH_2$-C(O)NH-, and $CH_3CH_2CH_2$-C(O)NH-) or any combination thereof. The number of substituents is not theoretically limited in any way or automatically limited by the size of the building units.

In some embodiments, in case that only one of $R_5$, $R_6$, $R_7$ and $R_8$ of the compound of Formula (I) represents $R_9$ and the compound of Formula (I) is not deuterated, when A represents $CH_2$ and one of $R_6$ and $R_7$ represents $R_9$, where R represents unsubstituted nitrogen-containing monocyclic heterocyclyl, $R_5$ represents halogen (e.g., fluorine, chlorine, bromine, or iodine), and the other one of $R_6$ and $R_7$ and/or $R_8$ each independently represent halogen (e.g., fluorine, chlorine, bromine, or iodine), hydroxy, mercapto, nitro, amino, cyano, $C_{1-6}$ alkyl (e.g., $C_{1-4}$ alkyl, such as methyl, ethyl, propyl, isopropyl, butyl, sec-butyl or tert-butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl (e.g., vinyl, propenyl, or butenyl), or $C_{2-6}$ alkynyl (e.g., ethynyl, propynyl, or butynyl).

In some embodiments, in case that only one of $R_5$, $R_6$, $R_7$ and $R_8$ of the compound of Formula (I) represents $R_9$ and the compound of Formula (I) is not deuterated, when A represents $CH_2$ and one of $R_5$ and $R_8$ represents $R_9$, where R represents substituted nitrogen-containing monocyclic heterocyclyl, and the other one of $R_5$ and $R_8$, as well as $R_6$ and $R_7$ each independently represent hydrogen, halogen (e.g., fluorine, chlorine, bromine, or iodine), hydroxy, mercapto, nitro, amino, cyano, $C_{1-6}$ alkyl (e.g., $C_{1-4}$ alkyl, such as methyl, ethyl, propyl, isopropyl, butyl, sec-butyl or tert-butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl (e.g., vinyl, propenyl, or butenyl), or $C_{2-6}$ alkynyl (e.g., ethynyl, propynyl, or butynyl), one to three (e.g., 1, 2 or 3) of the other one of $R_5$ and $R_8$ as well as $R_6$ and $R_7$ represents halogen (e.g., fluorine, chlorine, bromine, or iodine), and the nitrogen-containing monocyclic heterocyclyl is substituted with one or more (e.g., 1-6, such as 1, 2, 3, 4, 5 or 6) substituents selected from the group consisting of hydroxy, amino, mercapto, nitro, halogen (e.g., fluorine, chlorine, bromine, or iodine), CHO, cyano, $C_{1-6}$ alkyl (e.g., $C_{1-4}$ alkyl, such as methyl, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{3-6}$ cycloalkyl (e.g., cyclopropyl, cyclobutyl, cyclopentyl, or cyclohexyl), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl-NH- (e.g., $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-6}$ alkyl-NHC(O)- (e.g., $CH_3$-NHC(O)-, $CH_3CH_2$-NHC(O)-, and $CH_3CH_2CH_2$-NHC(O)-), $C_{1-6}$ alkyl-C(O)NH- (e.g., $CH_3$-C(O)NH-, $CH_3CH_2$-C(O)NH-, and $CH_3CH_2CH_2$-C(O)NH-) or any combination thereof. The number of substituents is not theoretically limited in any way or automatically limited by the size of the building units.

In some embodiments, in case that only one of $R_5$, $R_6$, $R_7$ and $R_8$ of the compound of Formula (I) represents $R_9$ and the compound of Formula (I) is not deuterated, when A represents $CH_2$ and one of $R_5$ and $R_8$ represents $R_9$, where R represents unsubstituted nitrogen-containing monocyclic heterocyclyl, and the other one of $R_5$ and $R_8$, as well as $R_6$ and $R_7$ each independently represent hydrogen, halogen (e.g., fluorine, chlorine, bromine, or iodine), hydroxy, mercapto, nitro, amino, cyano, $C_{1-6}$ alkyl (e.g., $C_{1-4}$ alkyl, such as methyl, ethyl, propyl, isopropyl, butyl, sec-butyl or tert-butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl (e.g., vinyl, propenyl, or butenyl), or $C_{2-6}$ alkynyl (e.g., ethynyl, propynyl, or butynyl), the other one of $R_5$ and $R_8$, as well as $R_6$ and $R_7$ are not simultaneously hydrogen, and when $R_6$ is halogen (e.g., fluorine, chlorine, bromine, or iodine), the other one of $R_5$ and $R_8$ as well as $R_7$ are not simultaneously hydrogen.

In some embodiments, the group R of $R_9$ of the compound of Formula (I) represents optionally substituted nitrogen-containing heterocyclyl, which is optionally substituted nitrogen-containing monocyclic heterocyclyl or optionally substituted nitrogen-containing bridged heterocyclyl. In a sub-embodiment, the nitrogen-containing monocyclic heterocyclyl is a 4- to 10-membered monocyclic heterocyclyl group containing one nitrogen atom and optionally a heteroatom selected from nitrogen, oxygen, and sulfur, including but not limited to the following groups: piperidinyl, piperazinyl, morpholinyl, azetidinyl, pyrrolidinyl, imidazolidinyl, pyrazolidyl, oxazolidinyl, thiazolidinyl, thiomorpholinyl, azepanyl, diazacycloheptanyl, azacyclooctyl or diazacyclooctyl. In a sub-embodiment, the nitrogen-containing bridged heterocyclyl is a $C_{5-20}$ bridged heterocyclyl group containing one nitrogen atom and optionally a heteroatom selected from nitrogen, oxygen, and sulfur, including but not limited to the following groups: azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl or diazabicyclo[2.2.2]octanyl, such as the following groups:

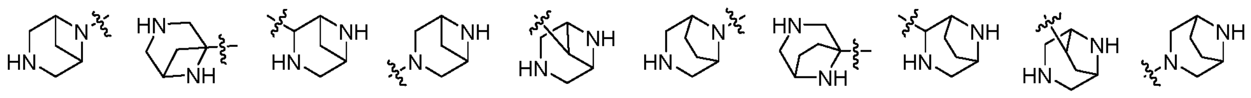

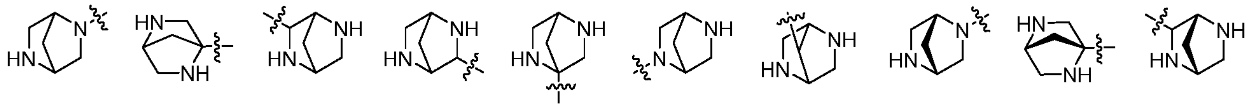

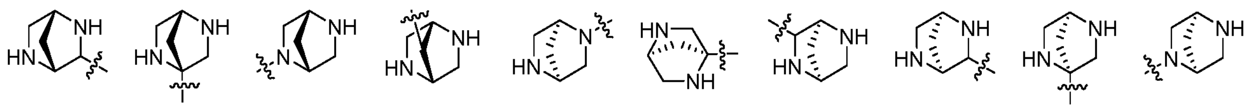

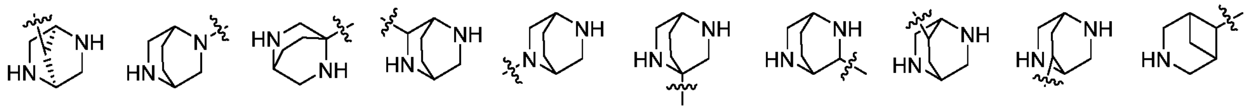

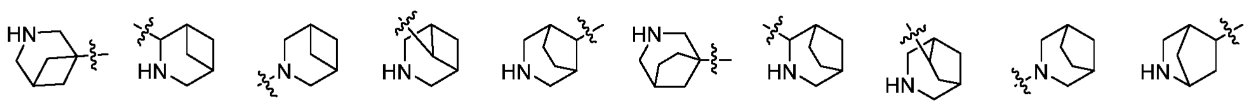

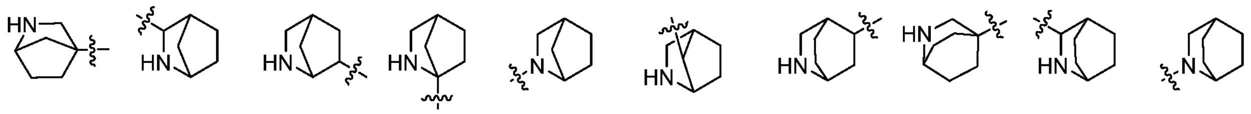

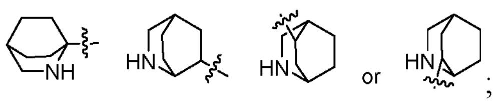
or ;

wherein the nitrogen-containing monocyclic heterocyclyl and nitrogen-containing bridged heterocyclyl are optionally substituted. In a sub-embodiment, the nitrogen-containing monocyclic heterocyclyl and nitrogen-containing bridged heterocyclyl are optionally substituted with one or more (e.g., 1-10, 1-8 or 1-6, such as 1, 2, 3, 4, 5 or 6) substituents selected from the group consisting of deuterium, hydroxy, amino, mercapto, nitro, halogen (e.g., fluorine, chlorine, bromine, or iodine), cyano, CHO, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally deuterated cyclopropyl, cyclobutyl, cyclopentyl, or cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-6}$ alkyl-NHC(O)- (e.g., $CH_3$-NHC(O)-, $CH_3CH_2$-NHC(O)-, and $CH_3CH_2CH_2$-NHC(O)-), $C_{1-6}$ alkyl-C(O)NH- (e.g., $CH_3$-C(O)NH-, $CH_3CH_2$-C(O)NH-, and $CH_3CH_2CH_2$-C(O)NH-) or any combination thereof.

In some embodiments, $R_9$ of the compound of Formula (I) represents the following groups:

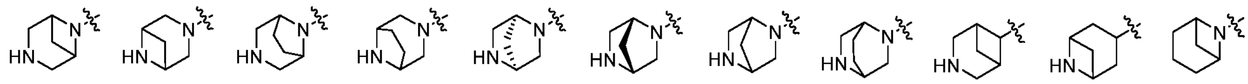

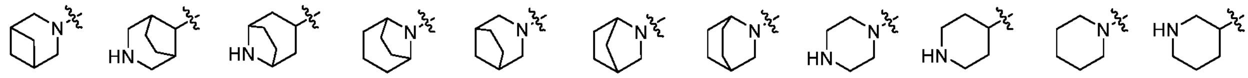

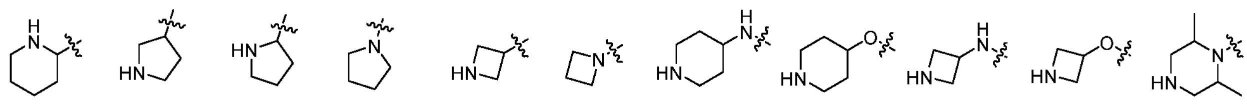

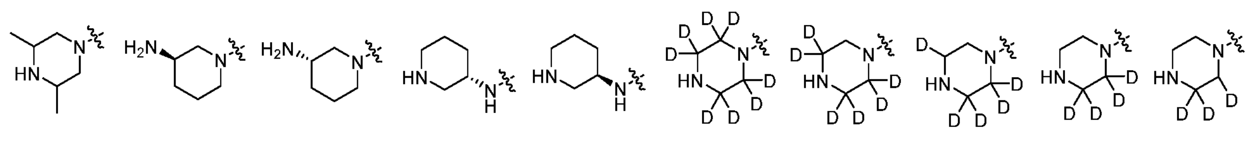

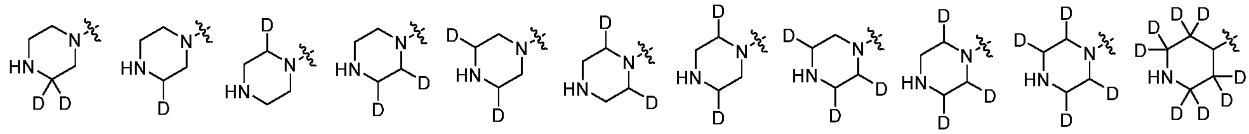

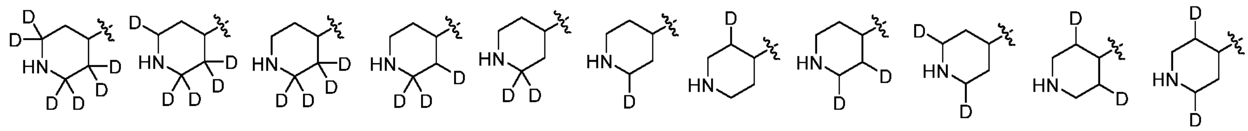

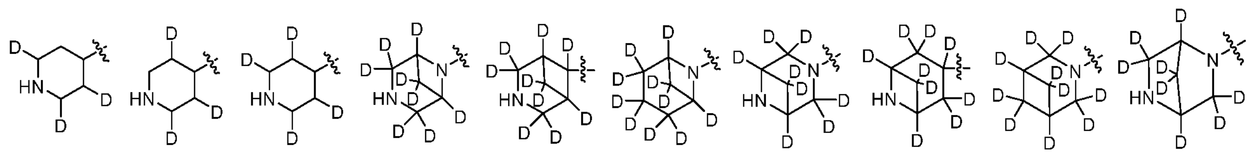

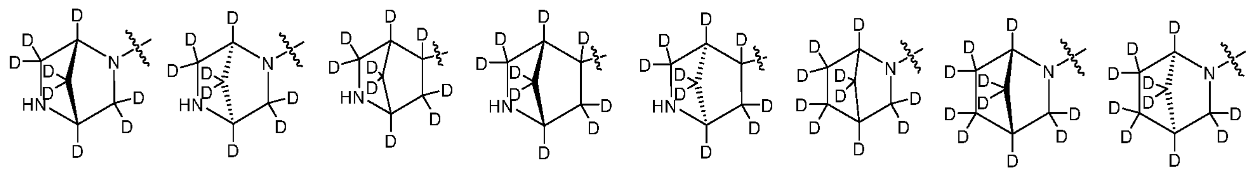

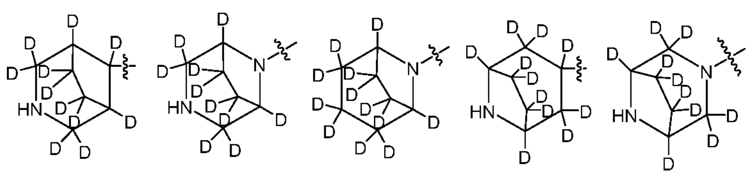 or 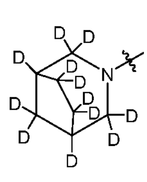.

In some embodiments, the compound of Formula (I) is also of Formula (I-1) or Formula (I-2):

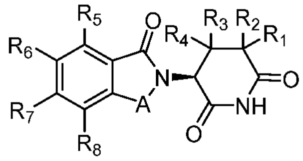

Formula (I-1)

or

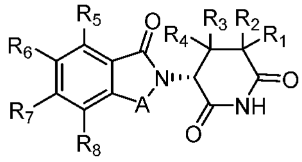

Formula (I-2)

wherein A, $R_1$, $R_2$, $R_3$, $R_4$, $R_5$, $R_6$, $R_7$ and $R_8$ are as defined in the compound of Formula (I) above.

In some embodiments, the compound of Formula (I) is also of Formula (I-3), Formula (I-4) or Formula (I-5):

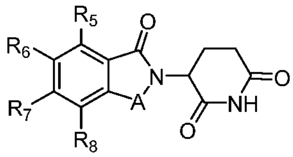

Formula (I-3)

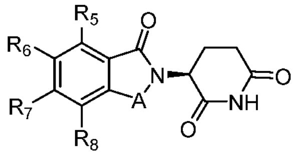

Formula (I-4)

or

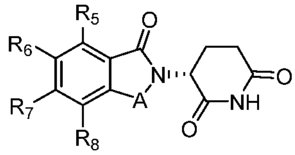

Formula (I-5)

wherein A, $R_5$, $R_6$, $R_7$ and $R_8$ are as defined in the compound of Formula (I) above.

In some embodiments, the compounds of Formula (II) of the present invention and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 1 below are provided.

Table 1. The compounds of the present disclosure

| Compound No. | Structure of compound | Compound's name | Chinese translation of Compound's name |
|---|---|---|---|
| GT-03100 | 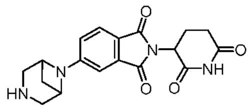 | 5-(3,6-diazabicyclo[3.1.1]heptan-6-yl)-2-(2,6-dioxopiperidin-3-yl)isoindoline-1,3-dione | 5-(3,6-diazabicyclo[3.1.1]heptan-6-yl)-2-(2,6-dioxopiperidin-3-yl)isoindoline-1,3-dione |
| GT-03075 | 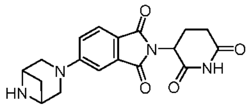 | 5-(3,6-diazabicyclo[3.1.1]heptan-3-yl)-2-(2,6-dioxopiperidin-3-yl)isoindoline-1,3-dione | 5-(3,6-diazabicyclo[3.1.1]heptan-3-yl)-2-(2,6-dioxopiperidin-3-yl)isoindoline-1,3-dione |
| GT-03210 | 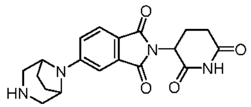 | 5-(3,8-diazabicyclo[3.2.1]octan-8-yl)-2-(2,6-dioxopiperidin-3-yl)isoindoline-1,3-dione | 5-(3,8-diazabicyclo[3.2.1]octan-8-yl)-2-(2,6-dioxopiperidin-3-yl)isoindoline-1,3-dione |
| GT-03077 | 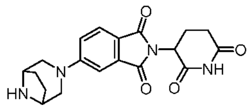 | 5-(3,8-diazabicyclo[3.2.1]octan-3-yl)-2-(2,6-dioxopiperidin-3-yl)isoindoline-1,3-dione | 5-(3,8-diazabicyclo[3.2.1] octan-3-yl)-2-(2,6-dioxopiperidin-3-yl)isoindoline-1,3-dione |
| GT-03072 | 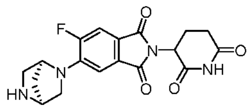 | 5-((1S,4S)-2,5-diazabicyclo[2.2. 1]heptan-2-yl)-2-(2,6-dioxopiperidin-3-yl)-6-fluoroisoindoline-1,3-dione | 5-((1S,4S)-2,5-diazabicyclo[2.2.1]heptan-2-yl)-2-(2,6-dioxopiperidin-3-yl)-6-fluoroisoindoline-1,3-dione |
| GT-03076 | 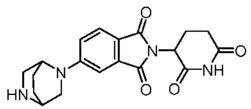 | 5-(2,5-diazabicyclo[2.2.2]octan-2-yl)-2-(2,6-dioxopiperidin-3-yl)isoindoline-1,3-dione | 5-(2,5-diazabicyclo[2.2.2]octan-2-yl)-2-(2,6-dioxopiperidin-3-yl)isoindoline-1,3-dione |
| | 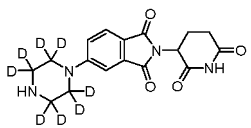 | 2-(2,6-dioxopiperidin-3 -yl)-5-(piperazin-1-yl-2,2,3,3,5,5,6,6-*d*$_8$)isoindoline-1,3-dione | 2-(2,6-dioxopiperidin-3-yl)-5- (piperazin-1-yl-2,2,3,3,5,5,6,6-*d*$_8$)isoindoline-1,3-dione |
| | 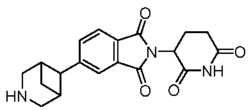 | 5-(3-azabicyclo[3.1.1]heptan-6-yl)-2-(2,6-dioxopiperidin-3-yl)isoindoline-1,3-dione | 5-(3-azabicyclo[3.1.1]heptan-6-yl)-2-(2,6-dioxopiperidin-3-yl)isoindoline-1,3-dione |

(continued)

| Compound No. | Structure of compound | Compound's name | Chinese translation of Compound's name |
|---|---|---|---|
| | 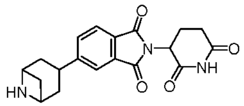 | 5-(6-azabicyclo[3.1.1]heptan-3- yl)-2-(2,6-dioxopiperidin-3-yl)isoindoline-1,3-dione | 5-(6-azabicyclo[3.1.1]heptan-3-yl)-2-(2,6-dioxopiperidin-3-yl)isoindoline-1,3-dione |
| | 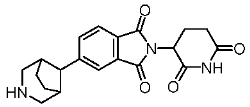 | 5-(3-azabicyclo[3.2.1]octan-8-yl)-2-(2,6-dioxopiperidin-3-yl)isoindoline-1,3-dione | 5-(3-azabicyclo[3.2.1]octan-8-yl)-2-(2,6-dioxopiperidin-3-yl)isoindoline-1,3-dione |
| | 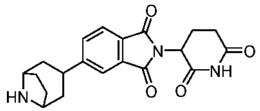 | 5-(8-azabicyclo[3.2.1]octan-3-yl)-2-(2,6-dioxopiperidin-3-yl)isoindoline-1,3-dione | 5-(8-azabicyclo[3.2.1]octan-3-yl)-2-(2,6-dioxopiperidin-3-yl)isoindoline-1,3-dione |
| | 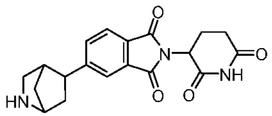 | 5-(2-azabicyclo[2.2.1]heptan-5-yl)-2-(2,6-dioxopiperidin-3-yl)isoindoline-1,3-dione | 5-(2-azabicyclo[2.2.1]heptan-5-yl)-2-(2,6-dioxopiperidin-3-yl)isoindoline-1,3-dione |
| | 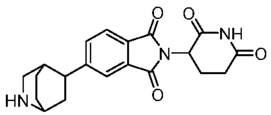 | 5-(2-azabicyclo[2.2.2]octan-5-yl)-2-(2,6-dioxopiperidin-3-yl)isoindoline-1,3-dione | 5-(2-azabicyclo[2.2.2]octan-5- yl)-2-(2,6-dioxopiperidin-3-yl)isoindoline-1,3-dione |
| GT-03106 | 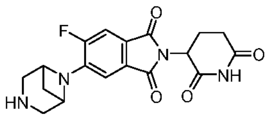 | 5-(3,6-diazabicyclo[3.1.1]heptan-6-yl)-2-(2,6-dioxopiperidin-3-yl)-6-fluoroisoindoline-1,3-dione | 5-(3,6-diazabicyclo[3.1.1]heptan-6-yl)-2-(2,6-dioxopiperidin-3-yl)-6-fluoroisoindoline-1,3-dione |
| GT-03109 | 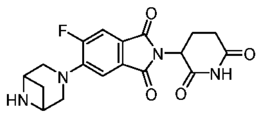 | 5-(3,6-diazabicyclo[3.1.1]heptan-3-yl)-2-(2,6-dioxopiperidin-3-yl)-6-fluoroisoindoline-1,3-dione | 5-(3,6-diazabicyclo[3.1.1]heptan-3-yl)-2-(2,6-dioxopiperidin-3-yl)-6-fluoroisoindoline-1,3-dione |
| GT-03211 | 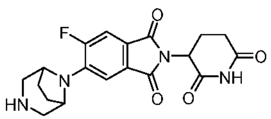 | 5-(3,8-diazabicyclo[3.2.1]octan-8-yl)-2-(2,6-dioxopiperidin-3-yl)-6-fluoroisoindoline-1,3-dione | 5-(3,8-diazabicyclo[3.2.1]octan-8-yl)-2-(2,6-dioxopiperidin-3-yl)-6-fluoroisoindoline-1,3-dione |

(continued)

| Compound No. | Structure of compound | Compound's name | Chinese translation of Compound's name |
|---|---|---|---|
| GT-03107 | 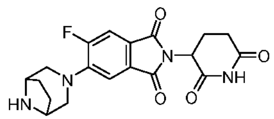 | 5-(3,8-diazabicyclo[3.2.1]octan-3-yl)-2-(2,6-dioxopiperidin-3-yl)-6-fluoroisoindoline-1,3-dione | 5-(3,8-diazabicyclo[3.2.1]octan-3-yl)-2-(2,6-dioxopiperidin-3-yl)-6-fluoroisoindoline-1,3-dione |
| GT-03103 | 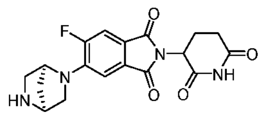 | 5-((1S,4S)-2,5-diazabicyclo[2.2.1]heptan-2-yl)-2-(2,6-dioxopiperidin-3-yl)-6-fluoroisoindoline-1,3-dione | 5-((1S,4S)-2,5-diazabicyclo[2.2.1]heptan-2-yl)-2-(2,6-dioxopiperidin-3-yl)-6-fluoroisoindoline-1,3-dione |
| GT-03105 | 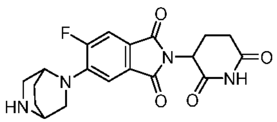 | 5-(2,5-diazabicyclo[2.2.2]octan-2-yl)-2-(2,6-dioxopiperidin-3-yl)-6-fluoroisoindoline-1,3-dione | 5-(2,5-diazabicyclo[2.2.2]octan-2-yl)-2-(2,6-dioxopiperidin-3-yl)-6-fluoroisoindoline-1,3-dione |
| | 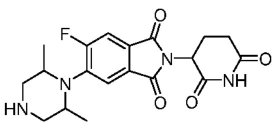 | 5-(2,6-dimethylpiperazin-1-yl)-2-(2,6-dioxopiperidin-3-yl)-6-fluoroisoindoline-1,3-dione | 5-(2,6-dimethylpiperazin-1-yl)-2-(2,6-dioxopiperidin-3-yl)-6-fluoroisoindoline-1,3- dione |
| GT-03104 | 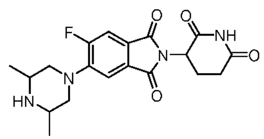 | 5-(3,5-dimethylpiperazin-1-yl)-2-(2,6-dioxopiperidin-3-yl)-6-fluoroisoindoline-1,3-dione | 5-(3,5-dimethylpiperazin-1-yl)-2-(2,6-dioxopiperidin-3-yl)-6-fluoroisoindoline-1,3- dione |
| GT-03108 | 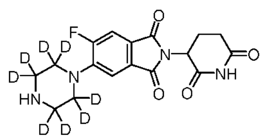 | 2-(2,6-dioxopiperidin-3-yl)-5-fluoro-6-(piperazin-1-yl-2,2,3,3,5,5,6,6-$d_8$)isoindoline-1,3-dione | 2-(2,6-dioxopiperidin-3-yl)-5-fluoro-6-(piperazin-1-yl-2,2,3,3,5,5,6,6-$d_8$)isoindoline-1,3-dione |
| | 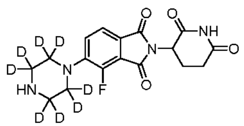 | 2-(2,6-dioxopiperidin-3-yl)-4-fluoro-5-(piperazin-1-yl-2,2,3,3,5,5,6,6-$d_8$)isoindoline-1,3-dione | 2-(2,6-dioxopiperidin-3-yl)-4-fluoro-5-(piperazin-1-yl-2,2,3,3,5,5,6,6-$d_8$)isoindoline-1,3-dione |

(continued)

| Compound No. | Structure of compound | Compound's name | Chinese translation of Compound's name |
|---|---|---|---|
| | 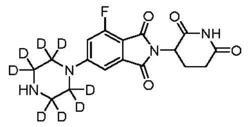 | 2-(2,6-dioxopiperidin-3-yl)-4-fluoro-6-(piperazin-1-yl-2,2,3,3,5,5,6,6-$d_8$)isoindoline-1,3-dione | 2-(2,6-dioxopiperidin-3-yl)-4-fluoro-6-(piperazin-1-yl-2,2,3,3,5,5,6,6-$d_8$)isoindoline-1,3-dione |
| | 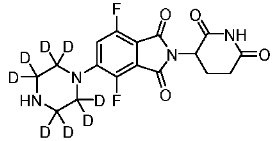 | 2-(2,6-dioxopiperidin-3-yl)-4,7-difluoro-5-(piperazin-1-yl-2,2,3,3,5,5,6,6-$d_8$)isoindoline-1,3-dione | 2-(2,6-dioxopiperidin-3-yl)-4,7-difluoro-5-(piperazin-1-yl-2,2,3,3,5,5,6,6-$d_8$)isoindoline-1,3-dione |
| | 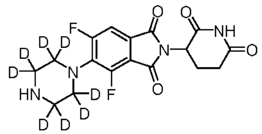 | 2-(2,6-dioxopiperidin-3-yl)-4,6-difluoro-5-(piperazin-1-yl-2,2,3,3,5,5,6,6-$d_8$)isoindoline-1,3-dione | 2-(2,6-dioxopiperidin-3-yl)-4,6-difluoro-5-(piperazin-1-yl-2,2,3,3,5,5,6,6-$d_8$)isoindoline-1,3-dione |
| | 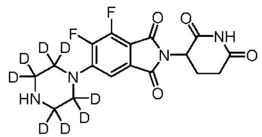 | 2-(2,6-dioxopiperidin-3-yl)-4,5-difluoro-6-(piperazin-1-yl-2,2,3,3,5,5,6,6-$d_8$)isoindoline-1,3-dione | 2-(2,6-dioxopiperidin-3-yl)-4,5-difluoro-6-(piperazin-1-yl-2,2,3,3,5,5,6,6-$d_8$)isoindoline-1,3-dione |
| | 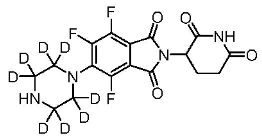 | 2-(2,6-dioxopiperidin-3-yl)-4,5,7-trifluoro-6-(piperazin-1-yl-2,2,3,3,5,5,6,6-$d_8$)isoindoline-1,3-dione | 2-(2,6-dioxopiperidin-3-yl)-4,5,7-trifluoro-6-(piperazin-1- yl-2,2,3,3,5,5,6,6-$d_8$)isoindoline-1,3-dione |
| | 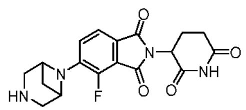 | 5-(3,6-diazabicyclo[3.1.1]heptan-6-yl)-2-(2,6-dioxopiperidin-3-yl)-4-fluoroisoindoline-1,3-dione | 5-(3,6-diazabicyclo[3.1.1]heptan-6-yl)-2-(2,6-dioxopiperidin-3-yl)-4-fluoroisoindoline-1,3-dione |
| | 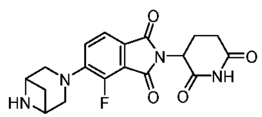 | 5-(3,6-diazabicyclo[3.1.1]heptan-3-yl)-2-(2,6-dioxopiperidin-3-yl)-4-fluoroisoindoline-1,3-dione | 5-(3,6-diazabicyclo[3.1.1]heptan-3-yl)-2-(2,6-dioxopiperidin-3-yl)-4-fluoroisoindoline-1,3-dione |

(continued)

| Compoun d No. | Structure of compound | Compound's name | Chinese translation of Compound's name |
|---|---|---|---|
| | 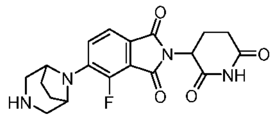 | 5-(3,8-diazabicyclo[3.2.1]octan-8-yl)-2-(2,6-dioxopiperidin-3-yl)-4-fluoroisoindoline-1,3-dione | 5-(3,8-diazabicyclo[3.2.1]octan-8-yl)-2-(2,6-dioxopiperidin-3-yl)-4-fluoroisoindoline-1,3-dione |
| | 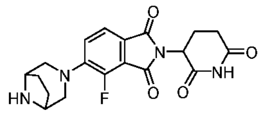 | 5-(3,8-diazabicyclo[3.2.1]octan-3-yl)-2-(2,6-dioxopiperidin-3-yl)-4-fluoroisoindoline-1,3-dione | 5-(3,8-diazabicyclo[3.2.1]octan-3-yl)-2-(2,6-dioxopiperidin-3-yl)-4-fluoroisoindoline-1,3- dione |
| | 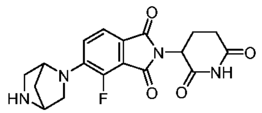 | 5-(2,5-diazabicyclo[2.2.1]heptan-2-yl)-2-(2,6-dioxopiperidin-3-yl)-4-fluoroisoindoline-1,3-dione | 5-(2,5-diazabicyclo[2.2.1]heptan-2-yl)-2-(2,6-dioxopiperidin-3-yl)-4-fluoroisoindoline-1,3- dione |
| | 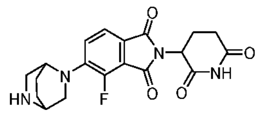 | 5-(2,5-diazabicyclo[2.2.2]octan-2-yl)-2-(2,6-dioxopiperidin-3-yl)-4-fluoroisoindoline-1,3-dione | 5-(2,5-diazabicyclo[2.2.2]octan-2-yl)-2-(2,6-dioxopiperidin-3-yl)-4-fluoroisoindoline-1,3-dione |
| | 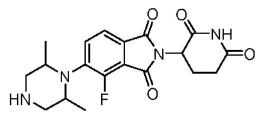 | 5-(2,6-dimethylpiperazin-1-yl)-2-(2,6-dioxopiperidin-3-yl)-4-fluoroisoindoline-1,3-dione | 5-(2,6-dimethylpiperazin-1-yl)-2-(2,6-dioxopiperidin-3-yl)-4-fluoroisoindoline-1,3- dione |
| | 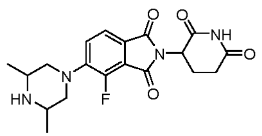 | 5-(3,5-dimethylpiperazin-1-yl)-2-(2,6-dioxopiperidin-3-yl)-4-fluoroisoindoline-1,3-dione | 5-(3,5-dimethylpiperazin-1-yl)-2-(2,6-dioxopiperidin-3-yl)-4-fluoroisoindoline-1,3- dione |
| | 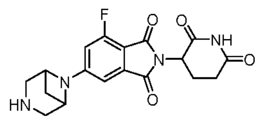 | 6-(3,6-diazabicyclo[3.1.1]heptan-6-yl)-2-(2,6-dioxopiperidin-3-yl)-4-fluoroisoindoline-1,3-dione | 6-(3,6-diazabicyclo[3.1.1]heptan-6-yl)-2-(2,6-dioxopiperidin-3-yl)-4-fluoroisoindoline-1,3-dione |

(continued)

| Compound No. | Structure of compound | Compound's name | Chinese translation of Compound's name |
|---|---|---|---|
| | 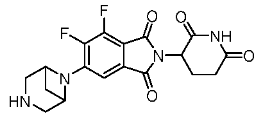 | 6-(3,6-diazabicyclo[3.1.1]heptan-6-yl)-2-(2,6-dioxopiperidin-3-yl)-4,5-difluoroisoindoline-1,3-dione | 6-(3,6-diazabicyclo[3.1.1]heptan-6-yl)-2-(2,6-dioxopiperidin-3-yl)-4,5-difluoroisoindoline-1,3-dione |
| | 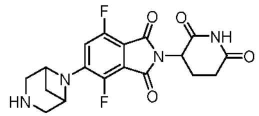 | 5-(3,6-diazabicyclo[3.1.1]heptan-6-yl)-2-(2,6-dioxopiperidin-3-yl)-4,7-difluoroisoindoline-1,3-dione | 5-(3,6-diazabicyclo[3.1.1]heptan-6-yl)-2-(2,6-dioxopiperidin-3-yl)-4,7-difluoroisoindoline-1,3-dione |
| | 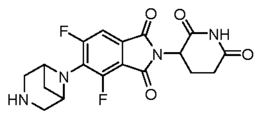 | 5-(3,6-diazabicyclo[3.1.1]heptan-6-yl)-2-(2,6-dioxopiperidin-3-yl)-4,6-difluoroisoindoline-1,3-dione | 5-(3,6-diazabicyclo[3.1.1]heptan-6-yl)-2-(2,6-dioxopiperidin-3-yl)-4,6-difluoroisoindoline-1,3-dione |
| | 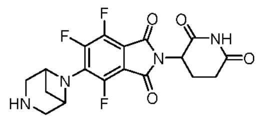 | 5-(3,6-diazabicyclo[3.1.1]heptan-6-yl)-2-(2,6-dioxopiperidin-3-yl)-4,6,7-trifluoroisoindoline-1,3- dione | 5-(3,6-diazabicyclo[3.1.1]heptan-6-yl)-2-(2,6-dioxopiperidin-3-yl)-4,6,7-trifluoroisoindoline-1,3-dione |
| | 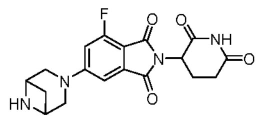 | 6-(3,6-diazabicyclo[3.1.1]heptan-3-yl)-2-(2,6-dioxopiperidin-3-yl)-4-fluoroisoindoline-1,3-dione | 6-(3,6-diazabicyclo[3.1.1]heptan-3-yl)-2-(2,6-dioxopiperidin-3-yl)-4-fluoroisoindoline-1,3-dione |
| | 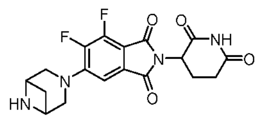 | 6-(3,6-diazabicyclo[3.1.1]heptan-3-yl)-2-(2,6-dioxopiperidin-3-yl)-4,5-difluoroisoindoline-1,3-dione | 6-(3,6-diazabicyclo[3.1.1]heptan-3-yl)-2-(2,6-dioxopiperidin-3-yl)-4,5-difluoroisoindoline-1,3-dione |
| | 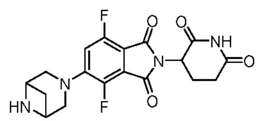 | 5-(3,6-diazabicyclo[3.1.1]heptan-3-yl)-2-(2,6-dioxopiperidin-3-yl)-4,7-difluoroisoindoline-1,3-dione | 5-(3,6-diazabicyclo[3.1.1]heptan-3-yl)-2-(2,6-dioxopiperidin-3-yl)-4,7-difluoroisoindoline-1,3-dione |

(continued)

| Compound No. | Structure of compound | Compound's name | Chinese translation of Compound's name |
|---|---|---|---|
| | 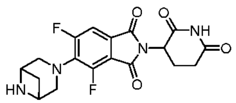 | 5-(3,6-diazabicyclo[3.1.1]heptan-3-yl)-2-(2,6-dioxopiperidin-3-yl)-4,6-difluoroisoindoline-1,3-dione | 5-(3,6-diazabicyclo[3.1.1]heptan-3-yl)-2-(2,6-dioxopiperidin-3-yl)-4,6-difluoroisoindoline-1,3-dione |
| | 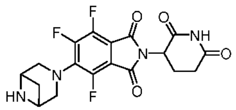 | 5-(3,6-diazabicyclo[3.1.1]heptan-3-yl)-2-(2,6-dioxopiperidin-3-yl)-4,6,7-trifluoroisoindoline-1,3-dione | 5-(3,6-diazabicyclo[3.1.1]heptan-3-yl)-2-(2,6-dioxopiperidin-3-yl)-4,6,7-trifluoroisoindoline-1,3-dione |
| | 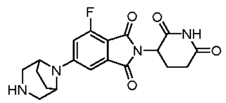 | 6-(3,8-diazabicyclo[3.2.1]octan-8-yl)-2-(2,6-dioxopiperidin-3-yl)-4-fluoroisoindoline-1,3-dione | 6-(3,8-diazabicyclo[3.2.1]octan-8-yl)-2-(2,6-dioxopiperidin-3-yl)-4-fluoroisoindoline-1,3-dione |
| | 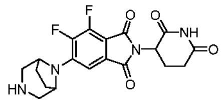 | 6-(3,8-diazabicyclo[3.2.1]octan-8-yl)-2-(2,6-dioxopiperidin-3-yl)-4,5-difluoroisoindoline-1,3-dione | 6-(3,8-diazabicyclo[3.2.1]octan-8-yl)-2-(2,6-dioxopiperidin-3-yl)-4,5-difluoroisoindoline-1,3-dione |
| | 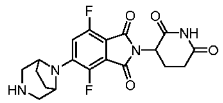 | 5-(3,8-diazabicyclo[3.2.1]octan-8-yl)-2-(2,6-dioxopiperidin-3-yl)-4,7-difluoroisoindoline-1,3-dione | 5-(3,8-diazabicyclo[3.2.1]octan-8-yl)-2-(2,6-dioxopiperidin-3-yl)-4,7-difluoroisoindoline-1,3-dione |
| | 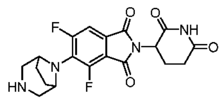 | 5-(3,8-diazabicyclo[3.2.1]octan-8-yl)-2-(2,6-dioxopiperidin-3-yl)-4,6-difluoroisoindoline-1,3-dione | 5-(3,8-diazabicyclo[3.2.1]octan-8-yl)-2-(2,6-dioxopiperidin-3-yl)-4,6-difluoroisoindoline-1,3-dione |
| | 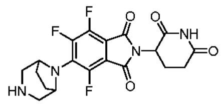 | 5-(3,8-diazabicyclo[3.2.1]octan-8-yl)-2-(2,6-dioxopiperidin-3-yl)-4,6,7-trifluoroisoindoline-1,3-dione | 5-(3,8-diazabicyclo[3.2.1]octan-8-yl)-2-(2,6-dioxopiperidin-3-yl)-4,6,7-trifluoroisoindoline-1,3-dione |
| | 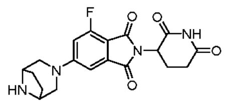 | 6-(3,8-diazabicyclo[3.2.1]octan-3-yl)-2-(2,6-dioxopiperidin-3-yl)-4-fluoroisoindoline-1,3-dione | 6-(3,8-diazabicyclo[3.2.1]octan-3-yl)-2-(2,6-dioxopiperidin-3-yl)-4-fluoroisoindoline-1,3-dione |

(continued)

| Compound No. | Structure of compound | Compound's name | Chinese translation of Compound's name |
|---|---|---|---|
| | 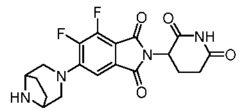 | 6-(3,8-diazabicyclo[3.2.1]octan-3-yl)-2-(2,6-dioxopiperidin-3-yl)-4,5-difluoroisoindoline-1,3-dione | 6-(3,8-diazabicyclo[3.2.1]octan-3-yl)-2-(2,6-dioxopiperidin-3-yl)-4,5-difluoroisoindoline-1,3-dione |
| | 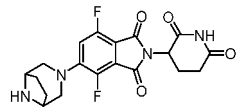 | 5-(3,8-diazabicyclo[3.2.1]octan-3-yl)-2-(2,6-dioxopiperidin-3-yl)-4,7-difluoroisoindoline-1,3-dione | 5-(3,8-diazabicyclo[3.2.1]octan-3-yl)-2-(2,6-dioxopiperidin-3-yl)-4,7-difluoroisoindoline-1,3-dione |
| | 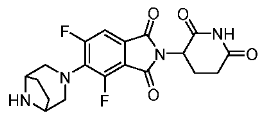 | 5-(3,8-diazabicyclo[3.2.1]octan-3-yl)-2-(2,6-dioxopiperidin-3-yl)-4,6-difluoroisoindoline-1,3-dione | 5-(3,8-diazabicyclo[3.2.1]octan-3-yl)-2-(2,6-dioxopiperidin-3-yl)-4,6-difluoroisoindoline-1,3-dione |
| | 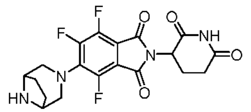 | 5-(3,8-diazabicyclo[3.2.1]octan-3-yl)-2-(2,6-dioxopiperidin-3-yl)-4,6,7-trifluoroisoindoline-1,3- dione | 5-(3,8-diazabicyclo[3.2.1]octan-3-yl)-2-(2,6-dioxopiperidin-3-yl)-4,6,7-trifluoroisoindoline-1,3-dione |
| | 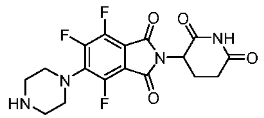 | 2-(2,6-dioxopiperidin-3-yl)-4,5,7-trifluoro-6-(piperazin-1-yl)isoindoline-1,3-dione | 2-(2,6-dioxopiperidin-3-yl)-4,5,7-trifluoro-6-(piperazin-1-yl)isoindoline-1,3-dione |
| | 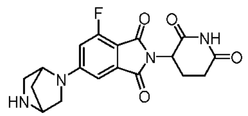 | 6-(2,5-diazabicyclo[2.2.1]heptan-2-yl)-2-(2,6-dioxopiperidin-3-yl)-4-fluoroisoindoline-1,3-dione | 6-(2,5-diazabicyclo[2.2.1]heptan-2-yl)-2-(2,6-dioxopiperidin-3-yl)-4-fluoroisoindoline-1,3-dione |
| | 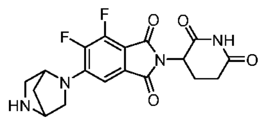 | 6-(2,5-diazabicyclo[2.2.1]heptan-2-yl)-2-(2,6-dioxopiperidin-3-yl)-4,5-difluoroisoindoline-1,3-dione | 6-(2,5-diazabicyclo[2.2.1]heptan-2-yl)-2-(2,6-dioxopiperidin-3-yl)-4,5-difluoroisoindoline-1,3-dione |
| | 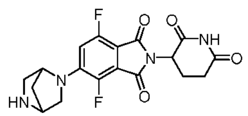 | 5-(2,5-diazabicyclo[2.2.1]heptan-2-yl)-2-(2,6-dioxopiperidin-3-yl)-4,7-difluoroisoindoline-1,3-dione | 5-(2,5-diazabicyclo[2.2.1]heptan-2-yl)-2-(2,6-dioxopiperidin-3-yl)-4,7-difluoroisoindoline-1,3-dione |

(continued)

| Compound No. | Structure of compound | Compound's name | Chinese translation of Compound's name |
|---|---|---|---|
| | 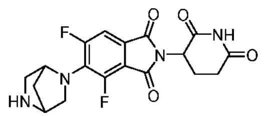 | 5-(2,5-diazabicyclo[2.2.1]heptan-2-yl)-2-(2,6-dioxopiperidin-3-yl)-4,6-difluoroisoindoline-1,3-dione | 5-(2,5-diazabicyclo[2.2.1]heptan-2-yl)-2-(2,6-dioxopiperidin-3-yl)-4,6-difluoroisoindoline-1,3-dione |
| | 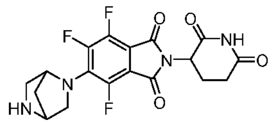 | 5-(2,5-diazabicyclo[2.2.1]heptan-2-yl)-2-(2,6-dioxopiperidin-3-yl)-4,6,7-trifluoroisoindoline-1,3- dione | 5-(2,5-diazabicyclo[2.2.1]heptan-2-yl)-2-(2,6-dioxopiperidin-3-yl)-4,6,7-trifluoroisoindoline-1,3-dione |
| | 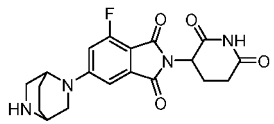 | 6-(2,5-diazabicyclo[2.2.2]octan-2-yl)-2-(2,6-dioxopiperidin-3-yl)-4-fluoroisoindoline-1,3-dione | 6-(2,5-diazabicyclo[2.2.2]octan-2-yl)-2-(2,6-dioxopiperidin-3-yl)-4-fluoroisoindoline-1,3-dione |
| | 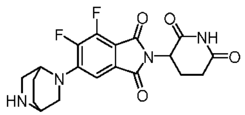 | 5-(2,5-diazabicyclo[2.2.1]heptan-2-yl)-2-(2,6-dioxopiperidin-3-yl)-4,6-difluoroisoindoline-1,3-dione | 5-(2,5-diazabicyclo[2.2.1]heptan-2-yl)-2-(2,6-dioxopiperidin-3-yl)-4,6-difluoroisoindoline-1,3-dione |
| | 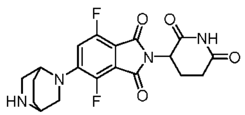 | 5-(2,5-diazabicyclo[2.2.2]octan-2-yl)-2-(2,6-dioxopiperidin-3-yl)-4,7-difluoroisoindoline-1,3-dione | 5-(2,5-diazabicyclo[2.2.2]octan-2-yl)-2-(2,6-dioxopiperidin-3-yl)-4,7-difluoroisoindoline-1,3-dione |
| | 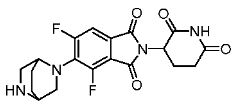 | 5-(2,5-diazabicyclo[2.2.2]octan-2-yl)-2-(2,6-dioxopiperidin-3-yl)-4,6-difluoroisoindoline-1,3-dione | 5-(2,5-diazabicyclo[2.2.2]octan-2-yl)-2-(2,6-dioxopiperidin-3-yl)-4,6-difluoroisoindoline-1,3-dione |
| | 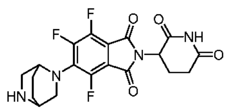 | 5-(2,5-diazabicyclo[2.2.2]octan-2-yl)-2-(2,6-dioxopiperidin-3-yl)-4,6,7-trifluoroisoindoline-1,3- dione | 5-(2,5-diazabicyclo[2.2.2]octan-2-yl)-2-(2,6-dioxopiperidin-3-yl)-4,6,7-trifluoroisoindoline-1,3-dione |
| | 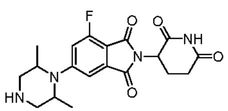 | 6-(2,6-dimethylpiperazin-1-yl)-2-(2,6-dioxopiperidin-3-yl)-4-fluoroisoindoline-1,3-dione | 6-(2,6-dimethylpiperazin-1-yl)-2-(2,6-dioxopiperidin-3-yl)-4-fluoroisoindoline-1,3- dione |

(continued)

| Compound No. | Structure of compound | Compound's name | Chinese translation of Compound's name |
|---|---|---|---|
| | 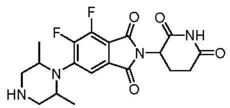 | 6-(2,6-dimethylpiperazin-1-yl)-2-(2,6-dioxopiperidin-3-yl)-4,5-difluoroisoindoline-1,3-dione | 6-(2,6-dimethylpiperazin-1-yl)-2-(2,6-dioxopiperidin-3-yl)-4,5-difluoroisoindoline-1,3-dione |
| | 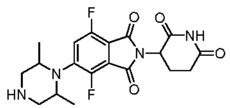 | 5-(2,6-dimethylpiperazin-1-yl)-2-(2,6-dioxopiperidin-3-yl)-4,7-difluoroisoindoline-1,3-dione | 5-(2,6-dimethylpiperazin-1-yl)-2-(2,6-dioxopiperidin-3-yl)-4,7-difluoroisoindoline-1,3-dione |
| | 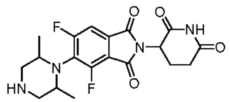 | 5-(2,6-dimethylpiperazin-1-yl)-2-(2,6-dioxopiperidin-3-yl)-4,6-difluoroisoindoline-1,3-dione | 5-(2,6-dimethylpiperazin-1-yl)-2-(2,6-dioxopiperidin-3-yl)-4,6-difluoroisoindoline-1,3-dione |
| | 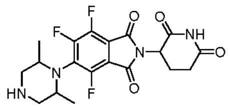 | 5-(2,6-dimethylpiperazin-1-yl)-2-(2,6-dioxopiperidin-3-yl)-4,6,7-trifluoroisoindoline-1,3-dione | 5-(2,6-dimethylpiperazin-1-yl)-2-(2,6-dioxopiperidin-3-yl)-4,6,7-trifluoroisoindoline-1,3-dione |
| | 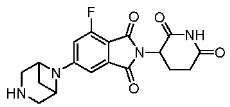 | 6-(3,6-diazabicyclo[3.1.1]heptan-6-yl)-2-(2,6-dioxopiperidin-3-yl)-4-fluoroisoindoline-1,3-dione | 6-(3,6-diazabicyclo[3.1.1]heptan-6-yl)-2-(2,6-dioxopiperidin-3-yl)-4-fluoroisoindoline-1,3-dione |
| | 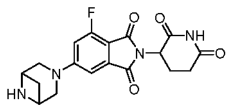 | 6-(3,6-diazabicyclo[3.1.1]heptan-3-yl)-2-(2,6-dioxopiperidin-3-yl)-4-fluoroisoindoline-1,3-dione | 6-(3,6-diazabicyclo[3.1.1]heptan-3-yl)-2-(2,6-dioxopiperidin-3-yl)-4-fluoroisoindoline-1,3-dione |
| | 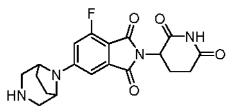 | 6-(3,8-diazabicyclo[3.2.1]octan-8-yl)-2-(2,6-dioxopiperidin-3-yl)-4-fluoroisoindoline-1,3-dione | 6-(3,8-diazabicyclo[3.2.1]octan-8-yl)-2-(2,6-dioxopiperidin-3-yl)-4-fluoroisoindoline-1,3-dione |
| | 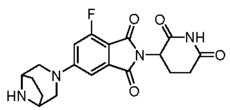 | 6-(3,8-diazabicyclo[3.2.1]octan-3-yl)-2-(2,6-dioxopiperidin-3-yl)-4-fluoroisoindoline-1,3-dione | 6-(3,8-diazabicyclo[3.2.1] octan-3-yl)-2-(2,6-dioxopiperidin-3-yl)-4-fluoroisoindoline-1,3- dione |

(continued)

| Compound No. | Structure of compound | Compound's name | Chinese translation of Compound's name |
|---|---|---|---|
| | 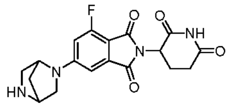 | 6-(2,5-diazabicyclo[2.2.1]heptan-2-yl)-2-(2,6-dioxopiperidin-3-yl)-4-fluoroisoindoline-1,3-dione | 6-(2,5-diazabicyclo[2.2.1]heptan-2-yl)-2-(2,6-dioxopiperidin-3-yl)-4-fluoroisoindoline-1,3- dione |
| | 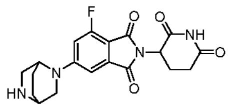 | 6-(2,5-diazabicyclo[2.2.2]octan-2-yl)-2-(2,6-dioxopiperidin-3-yl)-4-fluoroisoindoline-1,3-dione | 6-(2,5-diazabicyclo[2.2.2]octan-2-yl)-2-(2,6-dioxopiperidin-3-yl)-4-fluoroisoindoline-1,3-dione |
| | 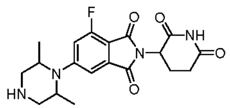 | 6-(2,6-dimethylpiperazin-1-yl)-2-(2,6-dioxopiperidin-3-yl)-4-fluoroisoindoline-1,3-dione | 6-(2,6-dimethylpiperazin-1-yl)-2-(2,6-dioxopiperidin-3-yl)-4-fluoroisoindoline-1,3- dione |
| | 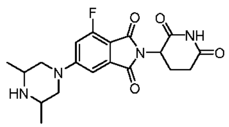 | 6-(3,5-dimethylpiperazin-1-yl)-2-(2,6-dioxopiperidin-3-yl)-4-fluoroisoindoline-1,3-dione | 6-(3,5-dimethylpiperazin-1-yl)-2-(2,6-dioxopiperidin-3-yl)-4-fluoroisoindoline-1,3- dione |
| | 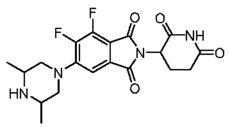 | 6-(3,5-dimethylpiperazin-1-yl)-2-(2,6-dioxopiperidin-3-yl)-4,5-difluoroisoindoline-1,3-dione | 6-(3,5-dimethylpiperazin-1-yl)-2-(2,6-dioxopiperidin-3-yl)-4,5-difluoroisoindoline-1,3-dione |
| | 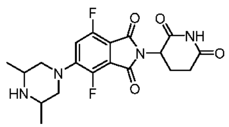 | 5-(3,5-dimethylpiperazin-1-yl)-2-(2,6-dioxopiperidin-3-yl)-4,7-difluoroisoindoline-1,3-dione | 5-(3,5-dimethylpiperazin-1-yl)-2-(2,6-dioxopiperidin-3-yl)-4,7-difluoroisoindoline-1,3-dione |
| | 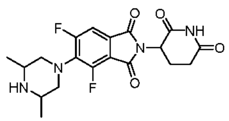 | 5-(3,5-dimethylpiperazin-1-yl)-2-(2,6-dioxopiperidin-3-yl)-4,6-difluoroisoindoline-1,3-dione | 5-(3,5-dimethylpiperazin-1-yl)-2-(2,6-dioxopiperidin-3-yl)-4,6-difluoroisoindoline-1,3-dione |

(continued)

| Compound No. | Structure of compound | Compound's name | Chinese translation of Compound's name |
|---|---|---|---|
| | 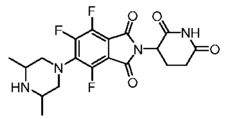 | 5-(3,5-dimethylpiperazin-1-yl)-2-(2,6-dioxopiperidin-3-yl)-4,6,7-trifluoroisoindoline-1,3-dione | 5-(3,5-dimethylpiperazin-1-yl)-2-(2,6-dioxopiperidin-3-yl)-4,6,7-trifluoroisoindoline-1,3-dione |
| | 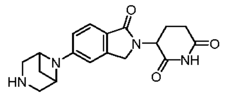 | 3-(5-(3,6-diazabicyclo[3.1.1]heptan-6-yl)-1-oxoisoindolin-2-yl)piperidine-2,6-dione | 3-(5-(3,6-diazabicyclo[3.1.1]heptan-6-yl)-1-oxoisoindolin-2-yl)piperidine-2,6-dione |
| | 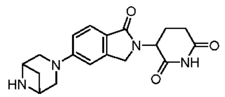 | 3-(5-(3,6-diazabicyclo[3.1.1]heptan-3-yl)-1-oxoisoindolin-2-yl)piperidine-2,6-dione | 3-(5-(3,6-diazabicyclo[3.1.1]heptan-3-yl)-1-oxoisoindolin-2-yl)piperidine-2,6-dione |
| | 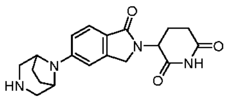 | 3-(5-(3,8-diazabicyclo[3.2.1]octan-8-yl)-1-oxoisoindolin-2-yl)piperidine-2,6-dione | 3-(5-(3,8-diazabicyclo[3.2.1]octan-8-yl)-1-oxoisoindolin-2-yl)piperidine-2,6-dione |
| | 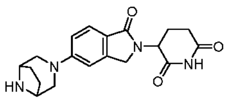 | 3-(5-(3,8-diazabicyclo[3.2.1]octan-3-yl)-1-oxoisoindolin-2-yl)piperidine-2,6-dione | 3-(5-(3,8-diazabicyclo[3.2.1]octan-3-yl)-1-oxoisoindolin-2-yl)piperidine-2,6-dione |
| | 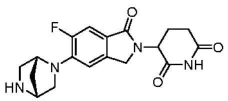 | 3-(5-((1R,4R)-2,5-diazabicyclo[2.2.1]heptan-2-yl)-6-fluoro-1-oxoisoindolin-2- yl)piperidine-2,6-dione | 3-(5-((1R,4R)-2,5-diazabicyclo[2.2.1]heptan-2-yl)-6-fluoro-1-oxoisoindolin-2-yl)piperidine-2,6-dione |
| | 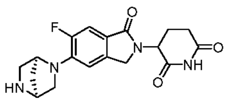 | 3-(5-((1S,4S)-2,5-diazabicyclo[2.2.1]heptan-2-yl)-6-fluoro-1-oxoisoindolin-2- yl)piperidine-2,6-dione | 3-(5-((1S,4S)-2,5-diazabicyclo[2.2.1]heptan-2-yl)-6-fluoro-1-oxoisoindolin-2-yl)piperidine-2,6-dione |
| | 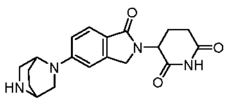 | 3-(5-(2,5-diazabicyclo[2.2.2]octan-2-yl)-1-oxoisoindolin-2-yl)piperidine-2,6-dione | 3-(5-(2,5-diazabicyclo[2.2.2]octan-2-yl)-1-oxoisoindolin-2-yl)piperidine-2,6-dione |

(continued)

| Compound No. | Structure of compound | Compound's name | Chinese translation of Compound's name |
|---|---|---|---|
| | 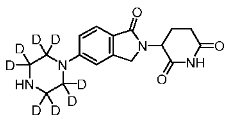 | 3-(1-oxo-5-(piperazin-1-yl-2,2,3,3,5,5,6,6-$d_8$)isoindolin-2-yl)piperidine-2,6-dione | 3-(1-oxo-5-(piperazin-1-yl-2,2,3,3,5,5,6,6-$d_8$)isoindolin-2-yl)piperidine-2,6-dione |
| | 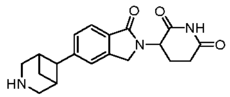 | 3-(5-(3-azabicyclo[3.1.1]heptan-6-yl)-1-oxoisoindolin-2-yl)piperidine-2,6-dione | 3-(5-(3-azabicyclo[3.1.1]heptan-6-yl)-1-oxoisoindolin-2-yl)piperidine-2,6-dione |
| | 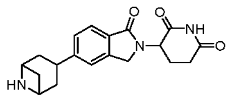 | 3-(5-(6-azabicyclo[3.1.1]heptan-3-yl)-1-oxoisoindolin-2-yl)piperidine-2,6-dione | 3-(5-(6-azabicyclo[3.1.1]heptan-3-yl)-1-oxoisoindolin-2-yl)piperidine-2,6-dione |
| | 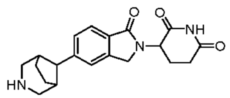 | 3-(5-(3-azabicyclo[3.2.1]octan-8-yl)-1-oxoisoindolin-2-yl)piperidine-2,6-dione | 3-(5-(3-azabicyclo[3.2.1]octan-8-yl)-1-oxoisoindolin-2-yl)piperidine-2,6-dione |
| | 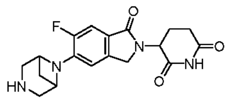 | 3-(5-(3,6-diazabicyclo[3.1.1]heptan-6-yl)-6-fluoro-1-oxoisoindolin-2- yl)piperidine-2,6-dione | 3-(5-(3,6-diazabicyclo[3.1.1]heptan-6-yl)-6-fluoro-1-oxoisoindolin-2-yl)piperidine-2,6-dione |
| | 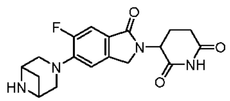 | 3-(5-(3,6-diazabicyclo[3.1.1]heptan-3 -yl)-6-fluoro-1-oxoisoindolin-2-yl)piperidine-2,6-dione | 3-(5-(3,6-diazabicyclo[3.1.1]heptan-3-yl)-6-fluoro-1-oxoisoindolin-2-yl)piperidine-2,6-dione |
| | 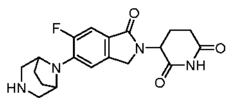 | 3-(5-(3,8-diazabicyclo[3.2.1]octan-8-yl)-6-fluoro-1-oxoisoindolin-2-yl)piperidine-2,6-dione | 3-(5-(3,8-diazabicyclo[3.2.1]octan-8-yl)-6-fluoro-1-oxoisoindolin-2-yl)piperidine-2,6-dione |
| | 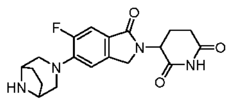 | 3-(5-(3,8-diazabicyclo[3.2.1]octan-3-yl)-6-fluoro-1-oxoisoindolin-2-yl)piperidine-2,6-dione | 3-(5-(3,8-diazabicyclo[3.2.1]octan-3-yl)-6-fluoro-1-oxoisoindolin-2-yl)piperidine-2,6-dione |

(continued)

| Compound No. | Structure of compound | Compound's name | Chinese translation of Compound's name |
|---|---|---|---|
| | 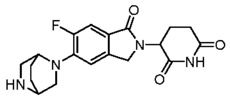 | 3-(5-(2,5-diazabicyclo[2.2.2]octan-2-yl)-6-fluoro-1-oxoisoindolin-2-yl)piperidine-2,6-dione | 3-(5-(2,5-diazabicyclo[2.2.2]octan-2-yl)-6-fluoro-1-oxoisoindolin-2-yl)piperidine-2,6-dione |
| | 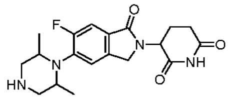 | 3-(5-(2,6-dimethylpiperazin-1-yl)-6-fluoro-1-oxoisoindolin-2-yl)piperidine-2,6-dione | 3 -(5 -(2,6-dimethylpiperazin-1-yl)-6-fluoro-1-oxoisoindolin-2-yl)piperidine-2,6-dione |
| | 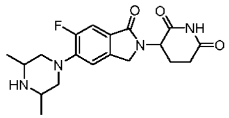 | 3-(5-(3,5-dimethylpiperazin-1-yl)-6-fluoro-1-oxoisoindolin-2-yl)piperidine-2,6-dione | 3-(5-(3,5-dimethylpiperazin-1-yl)-6-fluoro-1-oxoisoindolin-2-yl)piperidine-2,6-dione |
| | 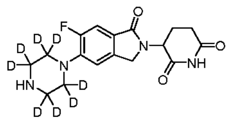 | 3-(6-fluoro-1-oxo-5-(piperazin-1-yl-2,2,3,3,5,5,6,6-ds)isoindolin-2-yl)piperidine-2,6-dione | 3-(6-fluoro-1-oxo-5-(piperazin-1-yl-2,2,3,3,5,5,6,6-$d_8$)isoindolin-2-yl)piperidine-2,6-dione |
| | 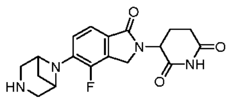 | 3-(5-(3,6-diazabicyclo[3.1.1]heptan-6-yl)-4-fluoro-1-oxoisoindolin-2- yl)piperidine-2,6-dione | 3-(5-(3,6-diazabicyclo[3.1.1]heptan-6-yl)-4-fluoro-1-oxoisoindolin-2-yl)piperidine-2,6-dione |
| | 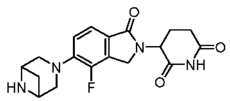 | 3-(5-(3,6-diazabicyclo[3.1.1]heptan-3 -yl)-4-fluoro-1-oxoisoindolin-2-yl)piperidine-2,6-dione | 3-(5-(3,6-diazabicyclo[3.1.1]heptan-3-yl)-4-fluoro-1-oxoisoindolin-2-yl)piperidine-2,6-dione |
| | 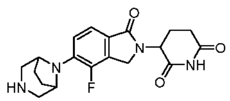 | 3-(5-(3,8-diazabicyclo[3.2.1]octan-8-yl)-4-fluoro-1-oxoisoindolin-2-yl)piperidine-2,6-dione | 3-(5-(3,8-diazabicyclo[3.2.1]octan-8-yl)-4-fluoro-1-oxoisoindolin-2-yl)piperidine-2,6-dione |
| | 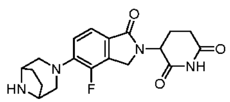 | 3-(5-(3,8-diazabicyclo[3.2.1]octan-3-yl)-4-fluoro-1-oxoisoindolin-2-yl)piperidine-2,6-dione | 3-(5-(3,8-diazabicyclo[3.2.1]octan-3-yl)-4-fluoro-1-oxoisoindolin-2-yl)piperidine-2,6-dione |

(continued)

| Compound No. | Structure of compound | Compound's name | Chinese translation of Compound's name |
|---|---|---|---|
| | 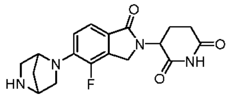 | 3-(5-(2,5-diazabicyclo[2.2.1]heptan-2-yl)-4-fluoro-1-oxoisoindolin-2-yl)piperidine-2,6-dione | 3-(5-(2,5-diazabicyclo[2.2.1]heptan-2-yl)-4-fluoro-1-oxoisoindolin-2-yl)piperidine-2,6-dione |
| | 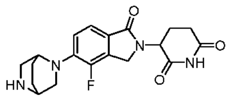 | 3-(5-(2,5-diazabicyclo[2.2.2]octan-2-yl)-4-fluoro-1-oxoisoindolin-2-yl)piperidine-2,6-dione | 3-(5-(2,5-diazabicyclo[2.2.2]octan-2-yl)-4-fluoro-1-oxoisoindolin-2-yl)piperidine-2,6-dione |
| | 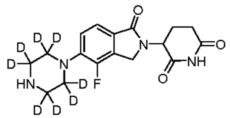 | 3-(4-fluoro-1-oxo-5-(piperazin-1-yl-2,2,3,3,5,5,6,6-$d_8$)isoindolin-2-yl)piperidine-2,6-dione | 3-(4-fluoro-1-oxo-5-(piperazin-1-yl-2,2,3,3,5,5,6,6-$d_8$)isoindolin-2-yl)piperidine-2,6-dione |
| | 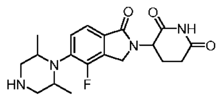 | 3-(5-(2,6-dimethylpiperazin-1-yl)-4-fluoro-1-oxoisoindolin-2-yl)piperidine-2,6-dione | 3 -(5-(2,6-dimethylpiperazin-1-yl)-4-fluoro-1-oxoisoindolin-2-yl)piperidine-2,6-dione |
| | 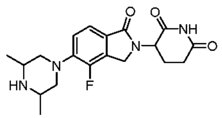 | 3-(5-(3,5-dimethylpiperazin-1-yl)-4-fluoro-1-oxoisoindolin-2-yl)piperidine-2,6-dione | 3-(5-(3,5-dimethylpiperazin-1-yl)-4-fluoro-1-oxoisoindolin-2-yl)piperidine-2,6-dione |
| | 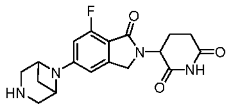 | 3-(5-(3,6-diazabicyclo[3.1.1]heptan-6-yl)-7-fluoro-1-oxoisoindolin-2-yl)piperidine-2,6-dione | 3-(5-(3,6-diazabicyclo[3.1.1]heptan-6-yl)-7-fluoro-1-oxoisoindolin-2-yl)piperidine-2,6-dione |
| | 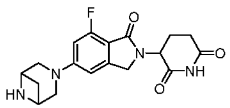 | 3-(5-(3,6-diazabicyclo[3.1.1]heptan-3-yl)-7-fluoro-1-oxoisoindolin-2-yl)piperidine-2,6-dione | 3-(5-(3,6-diazabicyclo[3.1.1]heptan-3-yl)-7-fluoro-1-oxoisoindolin-2-yl)piperidine-2,6-dione |
| | 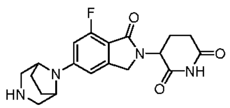 | 3-(5-(3,8-diazabicyclo[3.2.1]octan-8-yl)-7-fluoro-1-oxoisoindolin-2-yl)piperidine-2,6-dione | 3-(5-(3,8-diazabicyclo[3.2.1]octan-8-yl)-7-fluoro-1-oxoisoindolin-2-yl)piperidine-2,6-dione |

(continued)

| Compound No. | Structure of compound | Compound's name | Chinese translation of Compound's name |
|---|---|---|---|
| | 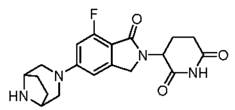 | 3-(5-(3,8-diazabicyclo[3.2.1]octan-3-yl)-7-fluoro-1-oxoisoindolin-2-yl)piperidine-2,6-dione | 3-(5-(3,8-diazabicyclo[3.2.1]octan-3-yl)-7-fluoro-1-oxoisoindolin-2-yl)piperidine-2,6-dione |
| | 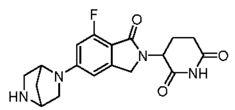 | 3-(5-(2,5-diazabicyclo[2.2. 1]heptan-2-yl)-7-fluoro-1-oxoisoindolin-2-yl)piperidine-2,6-dione | 3-(5-(2,5-diazabicyclo[2.2.1]heptan-2-yl)-7-fluoro-1-oxoisoindolin-2-yl)piperidine-2,6-dione |
| | 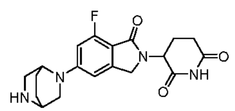 | 3-(5-(2,5-diazabicyclo[2.2.2]octan-2-yl)-7 - fluoro-1-oxoisoindolin-2-yl)piperidine-2,6-dione | 3-(5-(2,5-diazabicyclo[2.2.2]octan-2-yl)-7-fluoro-1-oxoisoindolin-2-yl)piperidine-2,6-dione |
| | 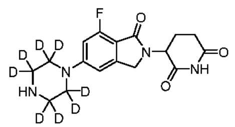 | 3-(7-fluoro-1-oxo-5-(piperazin-1-yl-2,2,3,3,5,5,6,6-*d*$_8$)isoindolin-2-yl)piperidine-2,6-dione | 3-(7-fluoro-1-oxo-5-(piperazin-1-yl-2,2,3,3,5,5,6,6-*d*$_8$)isoindolin-2-yl)piperidine-2,6-dione |
| | 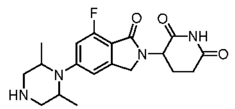 | 3-(5-(2,6-dimethylpiperazin-1-yl)-7-fluoro-1-oxoisoindolin-2-yl)piperidine-2,6-dione | 3-(5-(2,6-dimethylpiperazin-1-yl)-7-fluoro-1-oxoisoindolin-2-yl)piperidine-2,6-dione |
| | 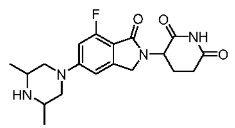 | 3-(5-(3,5-dimethylpiperazin-1-yl)-7-fluoro -1 -oxoisoindolin-2 - yl)piperidine-2,6-dione | 3-(5-(3,5-dimethylpiperazin-1-yl)-7-fluoro-1-oxoisoindolin-2-yl)piperidine-2,6-dione |
| | 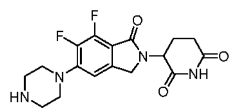 | 3-(6,7-difluoro-1-oxo-5-(piperazin-1-yl)isoindolin-2-yl)piperidine-2,6-dione | 3-(6,7-difluoro-1-oxo-5-(piperazin-1-yl)isoindolin-2-yl)piperidine-2,6-dione |
| | 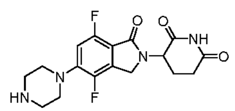 | 3-(4,7-difluoro-1-oxo-5-(piperazin-1-yl)isoindolin-2-yl)piperidine-2,6-dione | 3-(4,7-difluoro-1-oxo-5-(piperazin-1-yl)isoindolin-2-yl)piperidine-2,6-dione |

(continued)

| Compound No. | Structure of compound | Compound's name | Chinese translation of Compound's name |
|---|---|---|---|
| | 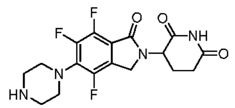 | 3-(4,6,7-trifluoro-1-oxo-5-(piperazin-1-yl)isoindolin-2-yl)piperidine-2,6-dione | 3-(4,6,7-trifluoro-1-oxo-5-(piperazin-1-yl)isoindolin-2-yl)piperidine-2,6-dione |
| | 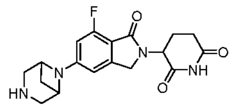 | 3-(5-(3,6-diazabicyclo[3.1.1]heptan-6-yl)-7-fluoro-1-oxoisoindolin-2- yl)piperidine-2,6-dione | 3-(5-(3,6-diazabicyclo[3.1.1]heptan-6-yl)-7-fluoro-1-oxoisoindolin-2-yl)piperidine-2,6-dione |
| | 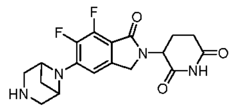 | 3-(5-(3,6-diazabicyclo[3.1.1]heptan-6-yl)-6,7-difluoro-1-oxoisoindolin-2-yl)piperidine-2,6-dione | 3-(5-(3,6-diazabicyclo[3.1.1]heptan-6-yl)-6,7-difluoro-1-oxoisoindolin-2-yl)piperidine-2,6-dione |
| | 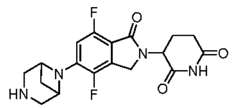 | 3-(5-(3,6-diazabicyclo[3.1.1]heptan-6-yl)-4,7-difluoro-1-oxoisoindolin-2-yl)piperidine-2,6-dione | 3-(5-(3,6-diazabicyclo[3.1.1]heptan-6-yl)-4,7-difluoro-1-oxoisoindolin-2-yl)piperidine-2,6-dione |
| | 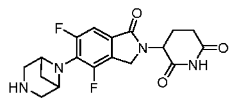 | 3-(5-(3,6-diazabicyclo[3.1.1]heptan-6-yl)-4,6-difluoro-1-oxoisoindolin-2-yl)piperidine-2,6-dione | 3-(5-(3,6-diazabicyclo[3.1.1]heptan-6-yl)-4,6-difluoro-1-oxoisoindolin-2-yl)piperidine-2,6-dione |
| | 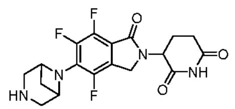 | 3-(5-(3,6-diazabicyclo[3.1.1]heptan-6-yl)-4,6,7-trifluoro-1-oxoisoindolin-2-yl)piperidine-2,6-dione | 3-(5-(3,6-diazabicyclo[3.1.1]heptan-6-yl)-4,6,7-trifluoro-1-oxoisoindolin-2-yl)piperidine-2,6-dione |
| | 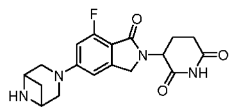 | 3-(5-(3,6-diazabicyclo[3.1.1]heptan-3 -yl)-7-fluoro-1-oxoisoindolin-2-yl)piperidine-2,6-dione | 3-(5-(3,6-diazabicyclo[3.1.1]heptan-3-yl)-7-fluoro-1-oxoisoindolin-2-yl)piperidine-2,6-dione |
| | 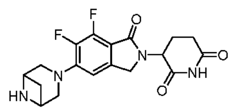 | 3-(5-(3,6-diazabicyclo[3.1.1]heptan-3 -yl)-6,7-difluoro-1-oxoisoindolin-2-yl)piperidine-2,6-dione | 3-(5-(3,6-diazabicyclo[3.1.1]heptan-3-yl)-6,7-difluoro-1-oxoisoindolin-2-yl)piperidine-2,6-dione |

(continued)

| Compound No. | Structure of compound | Compound's name | Chinese translation of Compound's name |
|---|---|---|---|
| | 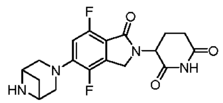 | 3-(5-(3,6-diazabicyclo[3.1.1]heptan-3 -yl)-4,7-difluoro-1-oxoisoindolin-2-yl)piperidine-2,6-dione | 3-(5-(3,6-diazabicyclo[3.1.1]heptan-3-yl)-4,7-difluoro-1-oxoisoindolin-2-yl)piperidine-2,6-dione |
| | 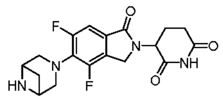 | 3-(5-(3,6-diazabicyclo[3.1.1]heptan-3 -yl)-4,6-difluoro-1-oxoisoindolin-2-yl)piperidine-2,6-dione | 3-(5-(3,6-diazabicyclo[3.1.1]heptan-3-yl)-4,6-difluoro-1-oxoisoindolin-2-yl)piperidine-2,6-dione |
| | 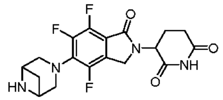 | 3-(5-(3,6-diazabicyclo[3.1.1]heptan-3-yl)-4,6,7-trifluoro-1-oxoisoindolin-2-yl)piperidine-2,6-dione | 3-(5-(3,6-diazabicyclo[3.1.1]heptan-3-yl)-4,6,7-trifluoro-1-oxoisoindolin-2-yl)piperidine-2,6-dione |
| | 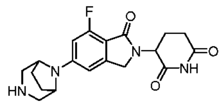 | 3-(5-(3,8-diazabicyclo[3.2.1]octan-8-yl)-7-fluoro-1-oxoisoindolin-2-yl)piperidine-2,6-dione | 3-(5-(3,8-diazabicyclo[3.2.1]octan-8-yl)-7-fluoro-1-oxoisoindolin-2-yl)piperidine-2,6-dione |
| | 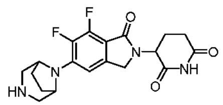 | 3-(5-(3,8-diazabicyclo[3.2.1]octan-8-yl)-6,7-difluoro-1-oxoisoindolin-2-yl)piperidine-2,6-dione | 3-(5-(3,8-diazabicyclo[3.2.1]octan-8-yl)-6,7-difluoro-1-oxoisoindolin-2-yl)piperidine-2,6-dione |
| | 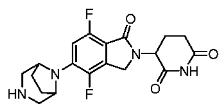 | 3-(5-(3,8-diazabicyclo[3.2.1]octan-8-yl)-4,7-difluoro-1-oxoisoindolin-2-yl)piperidine-2,6-dione | 3-(5-(3,8-diazabicyclo[3.2.1]octan-8-yl)-4,7-difluoro-1-oxoisoindolin-2-yl)piperidine-2,6-dione |
| | 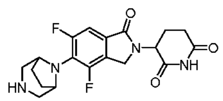 | 3-(5-(3,8-diazabicyclo[3.2.1]octan-8-yl)-4,6-difluoro-1-oxoisoindolin-2- yl)piperidine-2,6-dione | 3-(5-(3,8-diazabicyclo[3.2.1]octan-8-yl)-4,6-difluoro-1-oxoisoindolin-2-yl)piperidine-2,6-dione |
| | 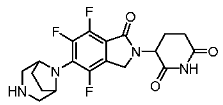 | 3-(5-(3,8-diazabicyclo[3.2.1]octan-8-yl)-4,6,7-trifluoro-1-oxoisoindolin-2-yl)piperidine-2,6-dione | 3-(5-(3,8-diazabicyclo[3.2.1]octan-8-yl)-4,6,7-trifluoro-1-oxoisoindolin-2-yl)piperidine-2,6-dione |

(continued)

| Compound No. | Structure of compound | Compound's name | Chinese translation of Compound's name |
|---|---|---|---|
| | 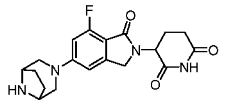 | 3-(5-(3,8-diazabicyclo[3.2.1]octan-3-yl)-7-fluoro-1-oxoisoindolin-2-yl)piperidine-2,6-dione | 3-(5-(3,8-diazabicyclo[3.2.1]octan-3-yl)-7-fluoro-1-oxoisoindolin-2-yl)piperidine-2,6-dione |
| | 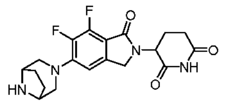 | 3-(5-(3,8-diazabicyclo[3.2.1]octan-3-yl)-6,7-difluoro-1-oxoisoindolin-2- yl)piperidine-2,6-dione | 3-(5-(3,8-diazabicyclo[3.2.1]octan-3-yl)-6,7-difluoro-1-oxoisoindolin-2-yl)piperidine-2,6-dione |
| | 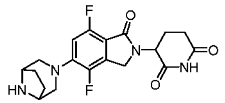 | 3-(5-(3,8-diazabicyclo[3.2.1]octan-3-yl)-4,7-difluoro-1-oxoisoindolin-2-yl)piperidine-2,6-dione | 3-(5-(3,8-diazabicyclo[3.2.1]octan-3-yl)-4,7-difluoro-1-oxoisoindolin-2-yl)piperidine-2,6-dione |
| | 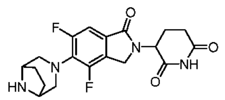 | 3-(5-(3,8-diazabicyclo[3.2.1]octan-3-yl)-4,6-difluoro-1-oxoisoindolin-2-yl)piperidine-2,6-dione | 3-(5-(3,8-diazabicyclo[3.2.1]octan-3-yl)-4,6-difluoro-1-oxoisoindolin-2-yl)piperidine-2,6-dione |
| | 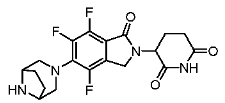 | 3-(5-(3,8-diazabicyclo[3.2.1]octan-3-yl)-4,6,7-trifluoro-1-oxoisoindolin-2-yl)piperidine-2,6-dione | 3-(5-(3,8-diazabicyclo[3.2.1]octan-3-yl)-4,6,7-trifluoro-1-oxoisoindolin-2-yl)piperidine-2,6-dione |
| | 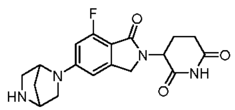 | 3-(5-(2,5-diazabicyclo[2.2.1]heptan-2-yl)-7-fluoro-1-oxoisoindolin-2-yl)piperidine-2,6-dione | 3-(5-(2,5-diazabicyclo[2.2.1]heptan-2-yl)-7-fluoro-1-oxoisoindolin-2-yl)piperidine-2,6-dione |
| | 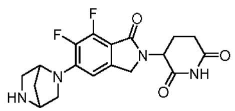 | 3-(5-(2,5-diazabicyclo[2.2. 1]heptan-2-yl)-6,7-difluoro-1-oxoisoindolin-2-yl)piperidine-2,6-dione | 3-(5-(2,5-diazabicyclo[2.2.1]heptan-2-yl)-6,7-difluoro-1-oxoisoindolin-2-yl)piperidine-2,6-dione |
| | 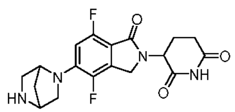 | 3-(5-(2,5-diazabicyclo[2.2.1]heptan-2-yl)-4,7-difluoro-1-oxoisoindolin-2-yl)piperidine-2,6-dione | 3-(5-(2,5-diazabicyclo[2.2.1]heptan-2-yl)-4,7-difluoro-1-oxoisoindolin-2-yl)piperidine-2,6-dione |

(continued)

| Compound No. | Structure of compound | Compound's name | Chinese translation of Compound's name |
|---|---|---|---|
| | 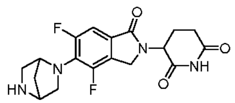 | 3-(5-(2,5-diazabicyclo[2.2.1]heptan-2-yl)-4,6-difluoro-1-oxoisoindolin-2-yl)piperidine-2,6-dione | 3-(5-(2,5-diazabicyclo[2.2.1]heptan-2-yl)-4,6-difluoro-1-oxoisoindolin-2-yl)piperidine-2,6-dione |
| | 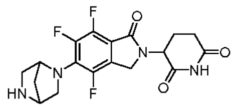 | 3-(5-(2,5-diazabicyclo[2.2.1]heptan-2-yl)-4,6,7-trifluoro-1-oxoisoindolin-2-yl)piperidine-2,6-dione | 3-(5-(2,5-diazabicyclo[2.2.1]heptan-2-yl)-4,6,7-trifluoro-1-oxoisoindolin-2-yl)piperidine-2,6-dione |
| | 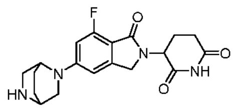 | 3-(5-(2,5-diazabicyclo[2.2.2]octan-2-yl)-7-fluoro-1-oxoisoindolin-2-yl)piperidine-2,6-dione | 3-(5-(2,5-diazabicyclo[2.2.2]octan-2-yl)-7-fluoro-1-oxoisoindolin-2-yl)piperidine-2,6-dione |
| | 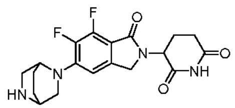 | 3-(5-(2,5-diazabicyclo[2.2.2]octan-2-yl)-6,7-difluoro-1-oxoisoindolin-2-yl)piperidine-2,6-dione | 3-(5-(2,5-diazabicyclo[2.2.2]octan-2-yl)-6,7-difluoro-1-oxoisoindolin-2-yl)piperidine-2,6-dione |
| | 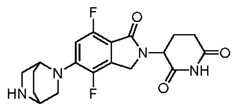 | 3-(5-(2,5-diazabicyclo[2.2.2]octan-2-yl)-4,7-difluoro-1-oxoisoindolin-2-yl)piperidine-2,6-dione | 3-(5-(2,5-diazabicyclo[2.2.2]octan-2-yl)-4,7-difluoro-1-oxoisoindolin-2-yl)piperidine-2,6-dione |
| | 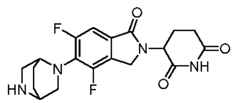 | 3-(5-(2,5-diazabicyclo[2.2.2]octan-2-yl)-4,6-difluoro-1-oxoisoindolin-2- yl)piperidine-2,6-dione | 3-(5-(2,5-diazabicyclo[2.2.2]octan-2-yl)-4,6-difluoro-1-oxoisoindolin-2-yl)piperidine-2,6-dione |
| | 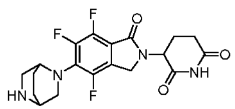 | 3-(5-(2,5-diazabicyclo[2.2.2]octan-2-yl)-4,6,7-trifluoro-1-oxoisoindolin-2-yl)piperidine-2,6-dione | 3-(5-(2,5-diazabicyclo[2.2.2]octan-2-yl)-4,6,7-trifluoro-1-oxoisoindolin-2-yl)piperidine-2,6-dione |
| | 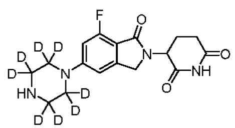 | 3-(7-fluoro-1-oxo-5-(piperazin-1-yl-2,2,3,3,5,5,6,6-$d_8$)isoindolin-2-yl)piperidine-2,6-dione | 3-(7-fluoro-1-oxo-5-(piperazin-1-yl-2,2,3,3,5,5,6,6-$d_8$)isoindolin-2-yl)piperidine-2,6-dione |

(continued)

| Compound No. | Structure of compound | Compound's name | Chinese translation of Compound's name |
|---|---|---|---|
| | 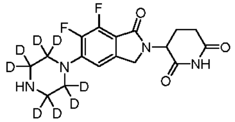 | 3-(6,7-difluoro-1-oxo-5-(piperazin-1-yl-2,2,3,3,5,5,6,6-$d_8$)isoindolin-2-yl)piperidine-2,6-dione | 3-(6,7-difluoro-1-oxo-5-(piperazin-1-yl-2,2,3,3,5,5,6,6-$d_8$)isoindolin-2-yl)piperidine-2,6-dione |
| | 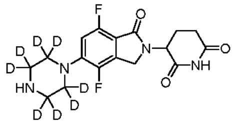 | 3-(4,7-difluoro-1-oxo-5-(piperazin-1-yl-2,2,3,3,5,5,6,6-$d_8$)isoindolin-2-yl)piperidine-2,6-dione | 3-(4,7-difluoro-1-oxo-5-(piperazin-1-yl-2,2,3,3,5,5,6,6-$d_8$)isoindolin-2-yl)piperidine-2,6-dione |
| | 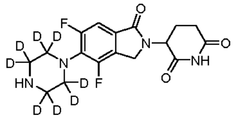 | 3-(4,6-difluoro-1-oxo-5-(piperazin-1-yl-2,2,3,3,5,5,6,6-$d_8$)isoindolin-2-yl)piperidine-2,6-dione | 3-(4,6-difluoro-1-oxo-5-(piperazin-1-yl-2,2,3,3,5,5,6,6-$d_8$)isoindolin-2-yl)piperidine-2,6-dione |
| | 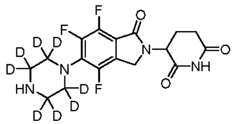 | 3-(4,6,7-trifluoro-1-oxo-5-(piperazin-1-yl-2,2,3,3,5,5,6,6-$d_8$)isoindolin-2-yl)piperidine-2,6-dione | 3-(4,6,7-trifluoro-1-oxo-5-(piperazin-1-yl-2,2,3,3,5,5,6,6-$d_8$)isoindolin-2-yl)piperidine-2,6-dione |
| | 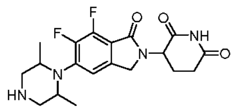 | 3-(5-(2,6-dimethylpiperazin-1-yl)-6,7-difluoro-1-oxoisoindolin-2-yl)piperidine-2,6-dione | 3-(5-(2,6-dimethylpiperazin-1-yl)-6,7-difluoro-1-oxoisoindolin-2-yl)piperidine-2,6-dione |
| | 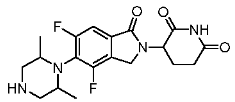 | 3-(5-(2,6-dimethylpiperazin-1-yl)-4,6-difluoro-1-oxoisoindolin-2- yl)piperidine-2,6-dione | 3-(5-(2,6-dimethylpiperazin-1-yl)-4,6-difluoro-1-oxoisoindolin-2-yl)piperidine-2,6-dione |
| | 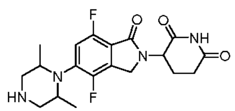 | 3-(5-(2,6-dimethylpiperazin-1-yl)-4,7-difluoro-1-oxoisoindolin-2- yl)piperidine-2,6-dione | 3-(5-(2,6-dimethylpiperazin-1-yl)-4,7-difluoro-1-oxoisoindolin-2-yl)piperidine-2,6-dione |

(continued)

| Compound No. | Structure of compound | Compound's name | Chinese translation of Compound's name |
|---|---|---|---|
| | 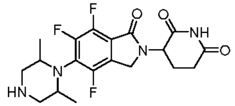 | 3-(5-(2,6-dimethylpiperazin-1-yl)-4,6,7-trifluoro-1-oxoisoindolin-2-yl)piperidine-2,6-dione | 3-(5-(2,6-dimethylpiperazin-1-yl)-4,6,7-trifluoro-1-oxoisoindolin-2-yl)piperidine-2,6-dione |
| | 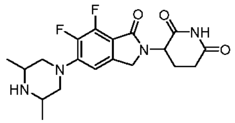 | 3-(5-(3,5-dimethylpiperazin-1-yl)-6,7-difluoro-1-oxoisoindolin-2-yl)piperidine-2,6-dione | 3-(5-(3,5-dimethylpiperazin-1-yl)-6,7-difluoro-1-oxoisoindolin-2-yl)piperidine-2,6-dione |
| | 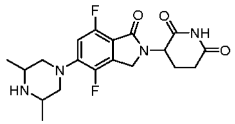 | 3-(5-(3,5-dimethylpiperazin-1-yl)-4,7-difluoro-1-oxoisoindolin-2-yl)piperidine-2,6-dione | 3-(5-(3,5-dimethylpiperazin-1-yl)-4,7-difluoro-1-oxoisoindolin-2-yl)piperidine-2,6-dione |
| | 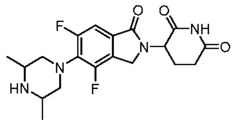 | 3-(5-(3,5-dimethylpiperazin-1-yl)-4,6-difluoro-1-oxoisoindolin-2-yl)piperidine-2,6-dione | 3-(5-(3,5-dimethylpiperazin-1-yl)-4,6-difluoro-1-oxoisoindolin-2-yl)piperidine-2,6-dione |
| | 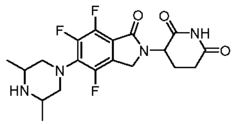 | 3-(5-(3,5-dimethylpiperazin-1-yl)-4,6,7-trifluoro-1-oxoisoindolin-2-yl)piperidine-2,6-dione | 3-(5-(3,5-dimethylpiperazin-1-yl)-4,6,7-trifluoro-1-oxoisoindolin-2-yl)piperidine-2,6-dione |
| | 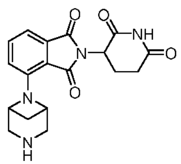 | 4-(3,6-diazabicyclo[3.1.1]heptan-6-yl)-2-(2,6-dioxopiperidin-3-yl)isoindoline-1,3-dione | 4-(3,6-diazabicyclo[3.1.1]heptan-6-yl)-2-(2,6-dioxopiperidin-3-yl)isoindoline-1,3-dione |
| | 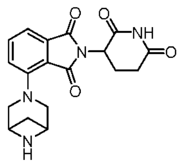 | 4-(3,6-diazabicyclo[3.1.1]heptan-3-yl)-2-(2,6-dioxopiperidin-3-yl)isoindoline-1,3-dione | 4-(3,6-diazabicyclo[3.1.1]heptan-3-yl)-2-(2,6-dioxopiperidin-3-yl)isoindoline-1,3-dione |

(continued)

| Compound No. | Structure of compound | Compound's name | Chinese translation of Compound's name |
|---|---|---|---|
| | 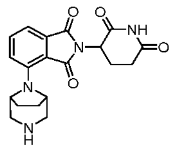 | 4-((1R,5S)-3,8-diazabicyclo[3.2.1]octan-8-yl)-2-(2,6-dioxopiperidin-3-yl)isoindoline-1,3-dione | 4-((1R,5S)-3,8-diazabicyclo[3.2.1]octan-8-yl)-2-(2,6-dioxopiperidin-3-yl)isoindoline-1,3-dione |
| | 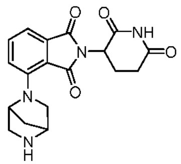 | 4-(2,5-diazabicyclo[2.2.1]heptan-2-yl)-2-(2,6-dioxopiperidin-3-yl)isoindoline-1,3-dione | 4-(2,5-diazabicyclo[2.2.1]heptan-2-yl)-2-(2,6-dioxopiperidin-3-yl)isoindoline-1,3-dione |
| | 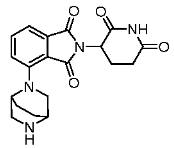 | 4-(2,5-diazabicyclo[2.2.2]octan-2-yl)-2-(2,6-dioxopiperidin-3- yl)isoindoline-1,3-dione | 4-(2,5-diazabicyclo[2.2.2]octan-2-yl)-2-(2,6-dioxopiperidin-3-yl)isoindoline-1,3-dione |
| | 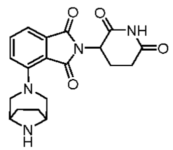 | 4-((1R,5S)-3,8-diazabicyclo[3.2.1]octan-3-yl)-2-(2,6-dioxopiperidin-3-yl)isoindoline-1,3-dione | 4-((1R,5S)-3,8-diazabicyclo[3.2.1]octan-3-yl)-2-(2,6-dioxopiperidin-3-yl)isoindoline-1,3-dione |
| | 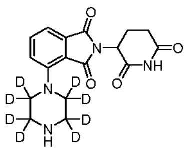 | 2-(2,6-dioxopiperidin-3-yl)-4-(piperazin-1-yl-2,2,3,3,5,5,6,6-$d_8$)isoindoline-1,3-dione | 2-(2,6-dioxopiperidin-3-yl)-4-(piperazin-1-yl-2,2,3,3,5,5,6,6-$d_8$)isoindoline-1,3-dione |
| | 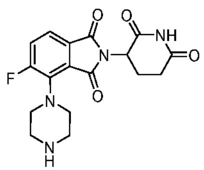 | 2-(2,6-dioxopiperidin-3-yl)-5-fluoro-4-(piperazin-1-yl)isoindoline-1,3-dione | 2-(2,6-dioxopiperidin-3-yl)-5-fluoro-4-(piperazin-1-yl)isoindoline-1,3-dione |

(continued)

| Compound No. | Structure of compound | Compound's name | Chinese translation of Compound's name |
|---|---|---|---|
| | 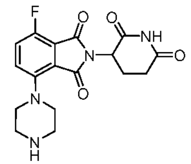 | 2-(2,6-dioxopiperidin-3-yl)-4-fluoro-7-(piperazin-1-yl)isoindoline-1,3-dione | 2-(2,6-dioxopiperidin-3-yl)-4-fluoro-7-(piperazin-1-yl)isoindoline-1,3-dione |
| | 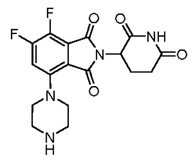 | 2-(2,6-dioxopiperidin-3-yl)-4,5-difluoro-7-(piperazin-1-yl)isoindoline-1,3-dione | 2-(2,6-dioxopiperidin-3-yl)-4,5-difluoro-7-(piperazin-1-yl)isoindoline-1,3-dione |
| | 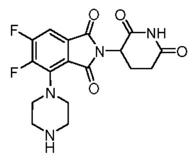 | 2-(2,6-dioxopiperidin-3-yl)-5,6-difluoro-4-(piperazin-1-yl)isoindoline-1,3-dione | 2-(2,6-dioxopiperidin-3-yl)-5,6-difluoro-4-(piperazin-1-yl)isoindoline-1,3-dione |
| | 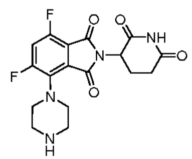 | 2-(2,6-dioxopiperidin-3-yl)-5,7-difluoro-4-(piperazin-1-yl)isoindoline-1,3-dione | 2-(2,6-dioxopiperidin-3-yl)-5,7-difluoro-4-(piperazin-1-yl)isoindoline-1,3-dione |
| | 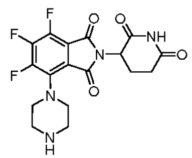 | 2-(2,6-dioxopiperidin-3-yl)-4,5,6-trifluoro-7-(piperazin-1-yl)isoindoline-1,3-dione | 2-(2,6-dioxopiperidin-3-yl)-4,5,6-trifluoro-7-(piperazin-1-yl)isoindoline-1,3-dione |
| | 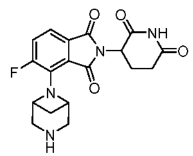 | 4-(3,6-diazabicyclo[3.1.1]heptan-6-yl)-2-(2,6-dioxopiperidin-3-yl)-5-fluoroisoindoline-1,3-dione | 4-(3,6-diazabicyclo[3.1.1]heptan-6-yl)-2-(2,6-dioxopiperidin-3-yl)-5-fluoroisoindoline-1,3- dione |

(continued)

| Compound No. | Structure of compound | Compound's name | Chinese translation of Compound's name |
|---|---|---|---|
| | 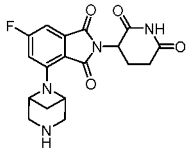 | 4-(3,6-diazabicyclo[3.1.1]heptan-6-yl)-2-(2,6-dioxopiperidin-3-yl)-6-fluoroisoindoline-1,3-dione | 4-(3,6-diazabicyclo[3.1.1]heptan-6-yl)-2-(2,6-dioxopiperidin-3-yl)-6-fluoroisoindoline-1,3-dione |
| | 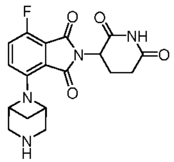 | 4-(3,6-diazabicyclo[3.1.1]heptan-6-yl)-2-(2,6-dioxopiperidin-3-yl)-7-fluoroisoindoline-1,3-dione | 4-(3,6-diazabicyclo[3.1.1]heptan-6-yl)-2-(2,6-dioxopiperidin-3-yl)-7-fluoroisoindoline-1,3-dione |
| | 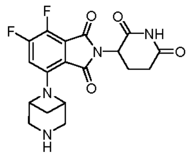 | 7-(3,6-diazabicyclo[3.1.1]heptan-6-yl)-2-(2,6-dioxopiperidin-3-yl)-4,5-difluoroisoindoline-1,3-dione | 7-(3,6-diazabicyclo[3.1.1]heptan-6-yl)-2-(2,6-dioxopiperidin-3-yl)-4,5-difluoroisoindoline-1,3-dione |
| | 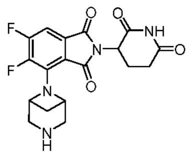 | 4-(3,6-diazabicyclo[3.1.1]heptan-6-yl)-2-(2,6-dioxopiperidin-3-yl)-5,6-difluoroisoindoline-1,3-dione | 4-(3,6-diazabicyclo[3.1.1]heptan-6-yl)-2-(2,6-dioxopiperidin-3-yl)-5,6-difluoroisoindoline-1,3-dione |
| | 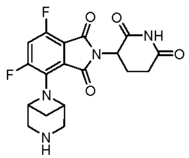 | 4-(3,6-diazabicyclo[3.1.1]heptan-6-yl)-2-(2,6-dioxopiperidin-3-yl)-5,7-difluoroisoindoline-1,3-dione | 4-(3,6-diazabicyclo[3.1.1]heptan-6-yl)-2-(2,6-dioxopiperidin-3-yl)-5,7-difluoroisoindoline-1,3-dione |
| | 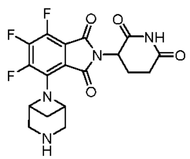 | 4-(3,6-diazabicyclo[3.1.1]heptan-6-yl)-2-(2,6-dioxopiperidin-3-yl)-5,6,7-trifluoroisoindoline-1,3-dione | 4-(3,6-diazabicyclo[3.1.1]heptan-6-yl)-2-(2,6-dioxopiperidin-3-yl)-5,6,7-trifluoroisoindoline-1,3-dione |

(continued)

| Compound No. | Structure of compound | Compound's name | Chinese translation of Compound's name |
|---|---|---|---|
| | 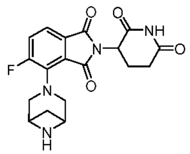 | 4-(3,6-diazabicyclo[3.1.1]heptan-3-yl)-2-(2,6-dioxopiperidin-3-yl)-5-fluoroisoindoline-1,3-dione | 4-(3,6-diazabicyclo[3.1.1]heptan-3-yl)-2-(2,6-dioxopiperidin-3-yl)-5-fluoroisoindoline-1,3- dione |
| | 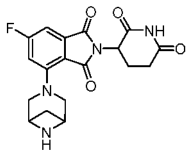 | 4-(3,6-diazabicyclo[3.1.1]heptan-3-yl)-2-(2,6-dioxopiperidin-3-yl)-6-fluoroisoindoline-1,3-dione | 4-(3,6-diazabicyclo[3.1.1]heptan-3-yl)-2-(2,6-dioxopiperidin-3-yl)-6-fluoroisoindoline-1,3-dione |
| | 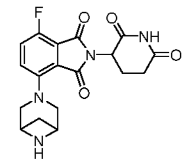 | 4-(3,6-diazabicyclo[3.1.1]heptan-3-yl)-2-(2,6-dioxopiperidin-3-yl)-7-fluoroisoindoline-1,3-dione | 4-(3,6-diazabicyclo[3.1.1]heptan-3-yl)-2-(2,6-dioxopiperidin-3-yl)-7-fluoroisoindoline-1,3- dione |
| | 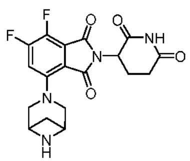 | 7-(3,6-diazabicyclo[3.1.1]heptan-3-yl)-2-(2,6-dioxopiperidin-3-yl)-4,5-difluoroisoindoline-1,3-dione | 7-(3,6-diazabicyclo[3.1.1]heptan-3-yl)-2-(2,6-dioxopiperidin-3-yl)-4,5-difluoroisoindoline-1,3-dione |
| | 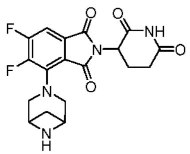 | 4-(3,6-diazabicyclo[3.1.1]heptan-3-yl)-2-(2,6-dioxopiperidin-3-yl)-5,6-difluoroisoindoline-1,3-dione | 4-(3,6-diazabicyclo[3.1.1]heptan-3-yl)-2-(2,6-dioxopiperidin-3-yl)-5,6-difluoroisoindoline-1,3-dione |
| | 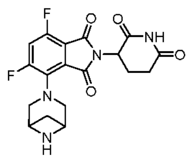 | 4-(3,6-diazabicyclo[3.1.1]heptan-3-yl)-2-(2,6-dioxopiperidin-3-yl)-5,7-difluoroisoindoline-1,3-dione | 4-(3,6-diazabicyclo[3.1.1]heptan-3-yl)-2-(2,6-dioxopiperidin-3-yl)-5,7-difluoroisoindoline-1,3-dione |

(continued)

| Compound No. | Structure of compound | Compound's name | Chinese translation of Compound's name |
|---|---|---|---|
| | 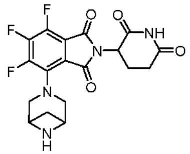 | 4-(3,6-diazabicyclo[3.1.1]heptan-3-yl)-2-(2,6-dioxopiperidin-3-yl)-5,6,7-trifluoroisoindoline-1,3-dione | 4-(3,6-diazabicyclo[3.1.1]heptan-3-yl)-2-(2,6-dioxopiperidin-3-yl)-5,6,7-trifluoroisoindoline-1,3-dione |
| | 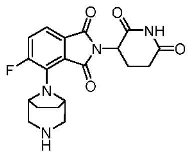 | 4-((1R,5S)-3,8-diazabicyclo[3.2.1]octan-8-yl)-2-(2,6-dioxopiperidin-3-yl)-5-fluoroisoindoline-1,3-dione | 4-((1R,5S)-3,8-diazabicyclo[3.2.1]octan-8-yl)-2-(2,6-dioxopiperidin-3-yl)-5-fluoroisoindoline-1,3- dione |
| | 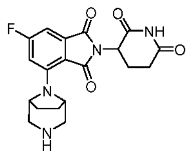 | 4-((1R,5S)-3,8-diazabicyclo[3.2.1]octan-8-yl)-2-(2,6-dioxopiperidin-3-yl)-6-fluoroisoindoline-1,3-dione | 4-((1R,5S)-3,8-diazabicyclo[3.2.1]octan-8-yl)-2-(2,6-dioxopiperidin-3-yl)-6-fluoroisoindoline-1,3-dione |
| | 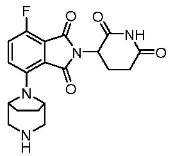 | 4-((1R,5S)-3,8-diazabicyclo[3.2.1]octan-8-yl)-2-(2,6-dioxopiperidin-3-yl)-7-fluoroisoindoline-1,3-dione | 4-((1R,5S)-3,8-diazabicyclo[3.2.1]octan-8-yl)-2-(2,6-dioxopiperidin-3-yl)-7-fluoroisoindoline-1,3-dione |
| | 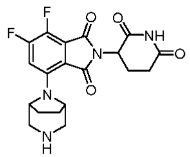 | 7-((1R,5S)-3,8-diazabicyclo[3.2.1]octan-8-yl)-2-(2,6-dioxopiperidin-3-yl)-4,5-difluoroisoindoline-1,3-dione | 7-((1R,5S)-3,8-diazabicyclo[3.2.1]octan-8-yl)-2-(2,6-dioxopiperidin-3-yl)-4,5-difluoroisoindoline-1,3-dione |
| | 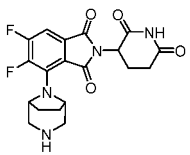 | 4-((1R,5S)-3,8-diazabicyclo[3.2.1]octan-8-yl)-2-(2,6-dioxopiperidin-3-yl)-5,6-difluoroisoindoline-1,3-dione | 4-((1R,5S)-3,8-diazabicyclo[3.2.1]octan-8-yl)-2-(2,6-dioxopiperidin-3-yl)-5,6-difluoroisoindoline-1,3-dione |

(continued)

| Compound No. | Structure of compound | Compound's name | Chinese translation of Compound's name |
|---|---|---|---|
| | 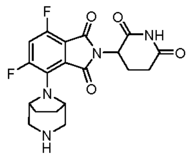 | 4-((1R,5S)-3,8-diazabicyclo[3.2.1]octan-8-yl)-2-(2,6-dioxopiperidin-3-yl)-5,7-difluoroisoindoline-1,3-dione | 4-((1R,5S)-3,8-diazabicyclo[3.2.1]octan-8-yl)-2-(2,6-dioxopiperidin-3-yl)-5,7-difluoroisoindoline-1,3-dione |
| | 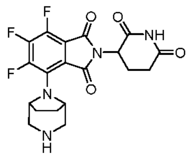 | 4-((1R,5S)-3,8-diazabicyclo[3.2.1]octan-8-yl)-2-(2,6-dioxopiperidin-3-yl)-5,6,7-trifluoroisoindoline-1,3-dione | 4-((1R,5S)-3,8-diazabicyclo[3.2.1]octan-8-yl)-2-(2,6-dioxopiperidin-3-yl)-5,6,7-trifluoroisoindoline-1,3-dione |
| | 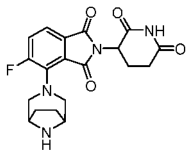 | 4-((1R,5S)-3,8-diazabicyclo[3.2.1]octan-3-yl)-2-(2,6-dioxopiperidin-3-yl)-5-fluoroisoindoline-1,3-dione | 4-((1R,5S)-3,8-diazabicyclo[3.2.1]octan-3-yl)-2-(2,6-dioxopiperidin-3-yl)-5-fluoroisoindoline-1,3-dione |
| | 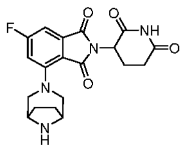 | 4-((1R,5S)-3,8-diazabicyclo[3.2.1]octan-3-yl)-2-(2,6-dioxopiperidin-3-yl)-6-fluoroisoindoline-1,3-dione | 4-((1R,5S)-3,8-diazabicyclo[3.2.1]octan-3-yl)-2-(2,6-dioxopiperidin-3-yl)-6-fluoroisoindoline-1,3- dione |
| | 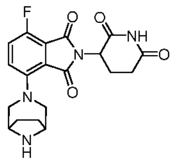 | 4-((1R,5S)-3,8-diazabicyclo[3.2.1]octan-3-yl)-2-(2,6-dioxopiperidin-3-yl)-7-fluoroisoindoline-1,3-dione | 4-((1R,5S)-3,8-diazabicyclo[3.2.1]octan-3-yl)-2-(2,6-dioxopiperidin-3-yl)-7-fluoroisoindoline-1,3- dione |
| | 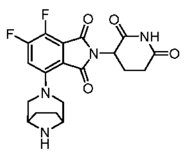 | 7-((1R,5S)-3,8-diazabicyclo[3.2.1]octan-3-yl)-2-(2,6-dioxopiperidin-3-yl)-4,5-difluoroisoindoline-1,3-dione | 7-((1R,5S)-3,8-diazabicyclo[3.2.1]octan-3-yl)-2-(2,6-dioxopiperidin-3-yl)-4,5-difluoroisoindoline-1,3-dione |

(continued)

| Compound No. | Structure of compound | Compound's name | Chinese translation of Compound's name |
|---|---|---|---|
| | 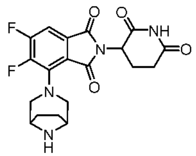 | 4-((1R,5S)-3,8-diazabicyclo[3.2.1]octan-3-yl)-2-(2,6-dioxopiperidin-3-yl)-5,6-difluoroisoindoline-1,3-dione | 4-((1R,5S)-3,8-diazabicyclo[3.2.1]octan-3-yl)-2-(2,6-dioxopiperidin-3-yl)-5,6-difluoroisoindoline-1,3-dione |
| | 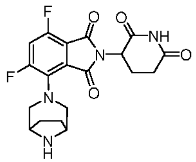 | 4-((1R,5S)-3,8-diazabicyclo[3.2.1]octan-3-yl)-2-(2,6-dioxopiperidin-3-yl)-5,7-difluoroisoindoline-1,3-dione | 4-((1R,5S)-3,8-diazabicyclo[3.2.1]octan-3-yl)-2-(2,6-dioxopiperidin-3-yl)-5,7-difluoroisoindoline-1,3-dione |
| | 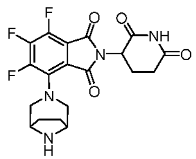 | 4-((1R,5S)-3,8-diazabicyclo[3.2.1]octan-3-yl)-2-(2,6-dioxopiperidin-3-yl)-5,6,7-trifluoroisoindoline-1,3-dione | 4-((1R,5S)-3,8-diazabicyclo[3.2.1]octan-3-yl)-2-(2,6-dioxopiperidin-3-yl)-5,6,7-trifluoroisoindoline-1,3-dione |
| | 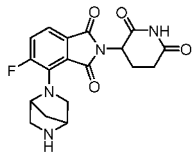 | 4-(2,5-diazabicyclo[2.2.1]heptan-2-yl)-2-(2,6-dioxopiperidin-3-yl)-5-fluoroisoindoline-1,3-dione | 4-(2,5-diazabicyclo[2.2.1]heptan-2-yl)-2-(2,6-dioxopiperidin-3-yl)-5-fluoroisoindoline-1,3-dione |
| | 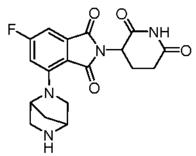 | 4-(2,5-diazabicyclo[2.2.1]heptan-2-yl)-2-(2,6-dioxopiperidin-3-yl)-6-fluoroisoindoline-1,3-dione | 4-(2,5-diazabicyclo[2.2.1]heptan-2-yl)-2-(2,6-dioxopiperidin-3-yl)-6-fluoroisoindoline-1,3- dione |
| | 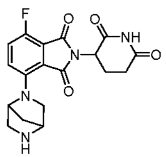 | 4-(2,5-diazabicyclo[2.2.1]heptan-2-yl)-2-(2,6-dioxopiperidin-3-yl)-7-fluoroisoindoline-1,3-dione | 4-(2,5-diazabicyclo[2.2.1]heptan-2-yl)-2-(2,6-dioxopiperidin-3-yl)-7-fluoroisoindoline-1,3- dione |

(continued)

| Compound No. | Structure of compound | Compound's name | Chinese translation of Compound's name |
|---|---|---|---|
| | 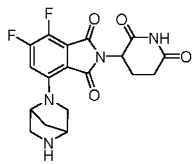 | 7-(2,5-diazabicyclo[2.2.1]heptan-2-yl)-2-(2,6-dioxopiperidin-3-yl)-4,5-difluoroisoindoline-1,3-dione | 7-(2,5-diazabicyclo[2.2.1]heptan-2-yl)-2-(2,6-dioxopiperidin-3-yl)-4,5-difluoroisoindoline-1,3-dione |
| | 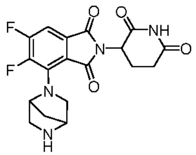 | 4-(2,5-diazabicyclo[2.2.1]heptan-2-yl)-2-(2,6-dioxopiperidin-3-yl)-5,6-difluoroisoindoline-1,3-dione | 4-(2,5-diazabicyclo[2.2.1]heptan-2-yl)-2-(2,6-dioxopiperidin-3-yl)-5,6-difluoroisoindoline-1,3-dione |
| | 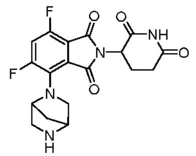 | 4-(2,5-diazabicyclo[2.2.1]heptan-2-yl)-2-(2,6-dioxopiperidin-3-yl)-5,7-difluoroisoindoline-1,3-dione | 4-(2,5-diazabicyclo[2.2.1]heptan-2-yl)-2-(2,6-dioxopiperidin-3-yl)-5,7-difluoroisoindoline-1,3-dione |
| | 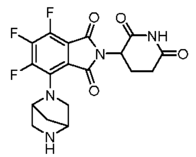 | 4-(2,5-diazabicyclo[2.2.1]heptan-2-yl)-2-(2,6-dioxopiperidin-3-yl)-5,6,7-trifluoroisoindoline-1,3- dione | 4-(2,5-diazabicyclo[2.2.1]heptan-2-yl)-2-(2,6-dioxopiperidin-3-yl)-5,6,7-trifluoroisoindoline-1,3-dione |
| | 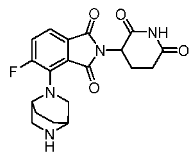 | 4-(2,5-diazabicyclo[2.2.2]octan-2-yl)-2-(2,6-dioxopiperidin-3-yl)-5-fluoroisoindoline-1,3-dione | 4-(2,5-diazabicyclo[2.2.2]octan-2-yl)-2-(2,6-dioxopiperidin-3-yl)-5-fluoroisoindoline-1,3- dione |
| | 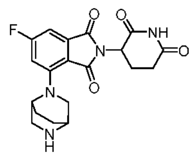 | 4-(2,5-diazabicyclo[2.2.2]octan-2-yl)-2-(2,6-dioxopiperidin-3-yl)-6-fluoroisoindoline-1,3-dione | 4-(2,5-diazabicyclo[2.2.2]octan-2-yl)-2-(2,6-dioxopiperidin-3-yl)-6-fluoroisoindoline-1,3-dione |

(continued)

| Compound No. | Structure of compound | Compound's name | Chinese translation of Compound's name |
|---|---|---|---|
| | 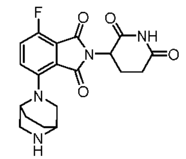 | 4-(2,5-diazabicyclo[2.2.2]octan-2-yl)-2-(2,6-dioxopiperidin-3-yl)-7-fluoroisoindoline-1,3-dione | 4-(2,5-diazabicyclo[2.2.2]octan-2-yl)-2-(2,6-dioxopiperidin-3-yl)-7-fluoroisoindoline-1,3- dione |
| | 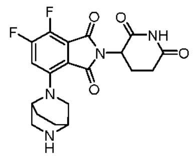 | 7-(2,5-diazabicyclo[2.2.2]octan-2-yl)-2-(2,6-dioxopiperidin-3-yl)-4,5-difluoroisoindoline-1,3-dione | 7-(2,5-diazabicyclo[2.2.2]octan-2-yl)-2-(2,6-dioxopiperidin-3-yl)-4,5-difluoroisoindoline-1,3-dione |
| | 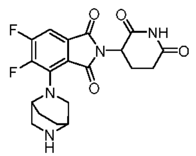 | 4-(2,5-diazabicyclo[2.2.2]octan-2-yl)-2-(2,6-dioxopiperidin-3-yl)-5,6-difluoroisoindoline-1,3-dione | 4-(2,5-diazabicyclo[2.2.2]octan-2-yl)-2-(2,6-dioxopiperidin-3-yl)-5,6-difluoroisoindoline-1,3-dione |
| | 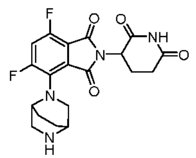 | 4-(2,5-diazabicyclo[2.2.2]octan-2-yl)-2-(2,6-dioxopiperidin-3-yl)-5,7-difluoroisoindoline-1,3-dione | 4-(2,5-diazabicyclo[2.2.2]octan-2-yl)-2-(2,6-dioxopiperidin-3-yl)-5,7-difluoroisoindoline-1,3-dione |
| | 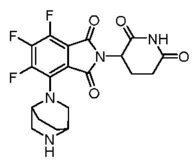 | 4-(2,5-diazabicyclo[2.2.2]octan-2-yl)-2-(2,6-dioxopiperidin-3-yl)-5,6,7-trifluoroisoindoline-1,3- dione | 4-(2,5-diazabicyclo[2.2.2]octan-2-yl)-2-(2,6-dioxopiperidin-3-yl)-5,6,7-trifluoroisoindoline-1,3-dione |
| | 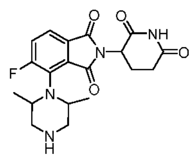 | 4-(2,6-dimethylpiperazin-1-yl)-2-(2,6-dioxopiperidin-3-yl)-5-fluoroisoindoline-1,3-dione | 4-(2,6-dimethylpiperazin-1-yl)-2-(2,6-dioxopiperidin-3-yl)-5-fluoroisoindoline-1,3-dione |

(continued)

| Compound No. | Structure of compound | Compound's name | Chinese translation of Compound's name |
|---|---|---|---|
| | 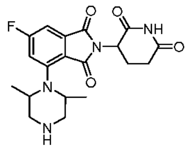 | 4-(2,6-dimethylpiperazin-1-yl)-2-(2,6-dioxopiperidin-3-yl)-6-fluoroisoindoline-1,3-dione | 4-(2,6-dimethylpiperazin-1-yl)-2-(2,6-dioxopiperidin-3-yl)-6-fluoroisoindoline-1,3- dione |
| | 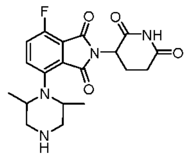 | 4-(2,6-dimethylpiperazin-1-yl)-2-(2,6-dioxopiperidin-3-yl)-7-fluoroisoindoline-1,3-dione | 4-(2,6-dimethylpiperazin-1-yl)-2-(2,6-dioxopiperidin-3-yl)-7-fluoroisoindoline-1,3- dione |
| | 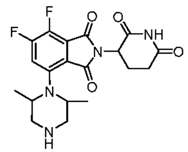 | 7-(2,6-dimethylpiperazin-1-yl)-2-(2,6-dioxopiperidin-3-yl)-4,5-difluoroisoindoline-1,3-dione | 7-(2,6-dimethylpiperazin-1-yl)-2-(2,6-dioxopiperidin-3-yl)-4,5-difluoroisoindoline-1,3-dione |
| | 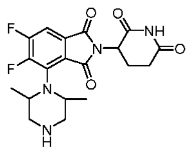 | 4-(2,6-dimethylpiperazin-1-yl)-2-(2,6-dioxopiperidin-3-yl)-5,6-difluoroisoindoline-1,3-dione | 4-(2,6-dimethylpiperazin-1-yl)-2-(2,6-dioxopiperidin-3-yl)-5,6-difluoroisoindoline-1,3-dione |
| | 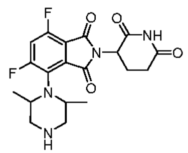 | 4-(2,6-dimethylpiperazin-1-yl)-2-(2,6-dioxopiperidin-3-yl)-5,7-difluoroisoindoline-1,3-dione | 4-(2,6-dimethylpiperazin-1-yl)-2-(2,6-dioxopiperidin-3-yl)-5,7-difluoroisoindoline-1,3-dione |
| | 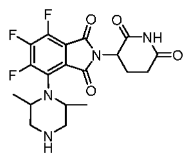 | 4-(2,6-dimethylpiperazin-1-yl)-2-(2,6-dioxopiperidin-3-yl)-5,6,7-trifluoroisoindoline-1,3-dione | 4-(2,6-dimethylpiperazin-1-yl)-2-(2,6-dioxopiperidin-3-yl)-5,6,7-trifluoroisoindoline-1,3-dione |

(continued)

| Compound No. | Structure of compound | Compound's name | Chinese translation of Compound's name |
|---|---|---|---|
| | 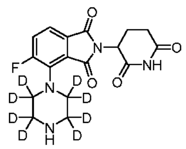 | 2-(2,6-dioxopiperidin-3-yl)-5-fluoro-4-(piperazin-1-yl-2,2,3,3,5,5,6,6-$d_8$)isoindoline-1,3-dione | 2-(2,6-dioxopiperidin-3-yl)-5- fluoro-4-(piperazin-1-yl-2,2,3,3,5,5,6,6-$d_8$)isoindoline-1,3-dione |
| | 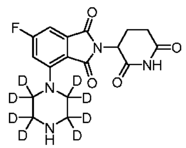 | 2-(2,6-dioxopiperidin-3-yl)-6-fluoro-4-(piperazin-1-yl-2,2,3,3,5,5,6,6-$d_8$)isoindoline-1,3-dione | 2-(2,6-dioxopiperidin-3-yl)-6-fluoro-4-(piperazin-1-yl-2,2,3,3,5,5,6,6-$d_8$)isoindoline-1,3-dione |
| | 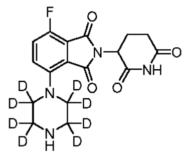 | 2-(2,6-dioxopiperidin-3-yl)-4-fluoro-7-(piperazin-1-yl-2,2,3,3,5,5,6,6-$d_8$)isoindoline-1,3-dione | 2-(2,6-dioxopiperidin-3-yl)-4-fluoro-7-(piperazin-1-yl-2,2,3,3,5,5,6,6-$d_8$)isoindoline-1,3-dione |
| | 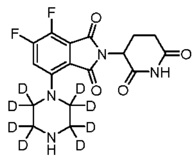 | 2-(2,6-dioxopiperidin-3-yl)-4,5-difluoro-7-(piperazin-1-yl-2,2,3,3,5,5,6,6-$d_8$)isoindoline-1,3-dione | 2-(2,6-dioxopiperidin-3-yl)-4,5-difluoro-7-(piperazin-1-yl-2,2,3,3,5,5,6,6-$d_8$)isoindoline-1,3-dione |
| | 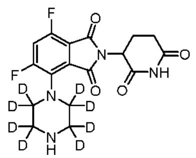 | 2-(2,6-dioxopiperidin-3-yl)-5,7-difluoro-4-(piperazin-1-yl-2,2,3,3,5,5,6,6-$d_8$)isoindoline-1,3-dione | 2-(2,6-dioxopiperidin-3-yl)-5,7-difluoro-4-(piperazin-1-yl-2,2,3,3,5,5,6,6-$d_8$)isoindoline-1,3-dione |
| | 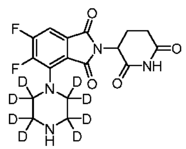 | 2-(2,6-dioxopiperidin-3-yl)-5,6-difluoro-4-(piperazin-1-yl-2,2,3,3,5,5,6,6-$d_8$)isoindoline-1,3-dione | 2-(2,6-dioxopiperidin-3-yl)-5,6-difluoro-4-(piperazin-1-yl-2,2,3,3,5,5,6,6-$d_8$)isoindoline-1,3-dione |

(continued)

| Compound No. | Structure of compound | Compound's name | Chinese translation of Compound's name |
|---|---|---|---|
| | 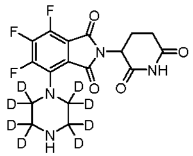 | 2-(2,6-dioxopiperidin-3-yl)-4,5,6-trifluoro-7-(piperazin-1-yl-2,2,3,3,5,5,6,6-$d_8$)isoindoline-1,3-dione | 2-(2,6-dioxopiperidin-3-yl)-4,5,6-trifluoro-7-(piperazin-1-yl-2,2,3,3,5,5,6,6-$d_8$)isoindoline-1,3-dione |
| | 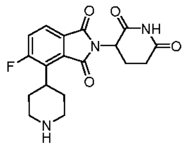 | 2-(2,6-dioxopiperidin-3-yl)-5- fluoro-4-(piperidin-4-yl)isoindoline-1,3-dione | 2-(2,6-dioxopiperidin-3-yl)-5-fluoro-4-(piperidin-4-yl)isoindoline-1,3-dione |
| | 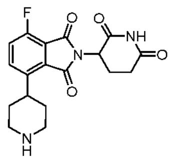 | 2-(2,6-dioxopiperidin-3-yl)-4-fluoro-7-(piperidin-4-yl)isoindoline-1,3-dione | 2-(2,6-dioxopiperidin-3-yl)-4-fluoro-7-(piperidin-4-yl)isoindoline-1,3-dione |
| | 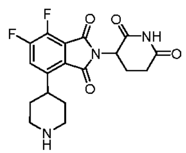 | 2-(2,6-dioxopiperidin-3-yl)-4,5-difluoro-7-(piperidin-4-yl)isoindoline-1,3-dione | 2-(2,6-dioxopiperidin-3-yl)-4,5-difluoro-7-(piperidin-4-yl)isoindoline-1,3-dione |
| | 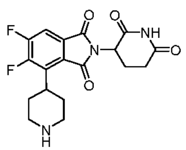 | 2-(2,6-dioxopiperidin-3-yl)-5,6-difluoro-4-(piperidin-4-yl)isoindoline-1,3-dione | 2-(2,6-dioxopiperidin-3-yl)-5,6-difluoro-4-(piperidin-4-yl)isoindoline-1,3-dione |
| | 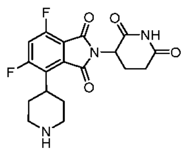 | 2-(2,6-dioxopiperidin-3-yl)-5,7-difluoro-4-(piperidin-4-yl)isoindoline-1,3-dione | 2-(2,6-dioxopiperidin-3-yl)-5,7-difluoro-4-(piperidin-4-yl)isoindoline-1,3-dione |

(continued)

| Compound No. | Structure of compound | Compound's name | Chinese translation of Compound's name |
|---|---|---|---|
| | 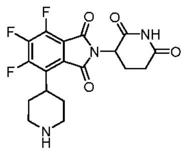 | 2-(2,6-dioxopiperidin-3-yl)-4,5,6-trifluoro-7-(piperidin-4-yl)isoindoline-1,3-dione | 2-(2,6-dioxopiperidin-3-yl)-4,5,6-trifluoro-7-(piperidin-4-yl)isoindoline-1,3-dione |
| | 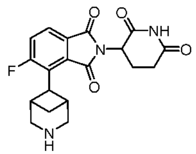 | 4-(3-azabicyclo[3.1.1]heptan-6-yl)-2-(2,6-dioxopiperidin-3-yl)-5-fluoroisoindoline-1,3-dione | 4-(3-azabicyclo[3.1.1]heptan-6-yl)-2-(2,6-dioxopiperidin-3-yl)-5-fluoroisoindoline-1,3-dione |
| | 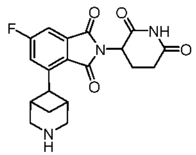 | 4-(3-azabicyclo[3.1.1]heptan-6-yl)-2-(2,6-dioxopiperidin-3-yl)-6-fluoroisoindoline-1,3-dione | 4-(3-azabicyclo[3.1.1]heptan-6-yl)-2-(2,6-dioxopiperidin-3-yl)-6-fluoroisoindoline-1,3-dione |
| | 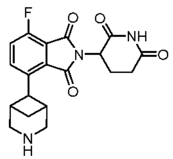 | 4-(3-azabicyclo[3.1.1]heptan-6-yl)-2-(2,6-dioxopiperidin-3-yl)-7-fluoroisoindoline-1,3-dione | 4-(3-azabicyclo[3.1.1]heptan-6-yl)-2-(2,6-dioxopiperidin-3-yl)-7-fluoroisoindoline-1,3- dione |
| | 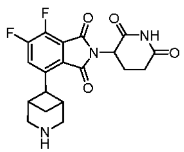 | 7-(3-azabicyclo[3.1.1]heptan-6-yl)-2-(2,6-dioxopiperidin-3-yl)-4,5-difluoroisoindoline-1,3-dione | 7-(3-azabicyclo[3.1.1]heptan-6-yl)-2-(2,6-dioxopiperidin-3-yl)-4,5-difluoroisoindoline-1,3-dione |
| | 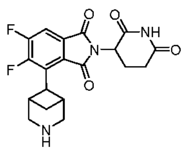 | 4-(3-azabicyclo[3.1.1]heptan-6-yl)-2-(2,6-dioxopiperidin-3-yl)-5,6-difluoroisoindoline-1,3-dione | 4-(3-azabicyclo[3.1.1]heptan-6-yl)-2-(2,6-dioxopiperidin-3-yl)-5,6-difluoroisoindoline-1,3-dione |

(continued)

| Compound No. | Structure of compound | Compound's name | Chinese translation of Compound's name |
|---|---|---|---|
| | 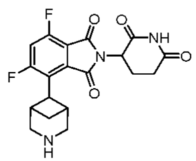 | 4-(3-azabicyclo[3.1.1]heptan-6-yl)-2-(2,6-dioxopiperidin-3-yl)-5,7-difluoroisoindoline-1,3-dione | 4-(3-azabicyclo[3.1.1]heptan-6-yl)-2-(2,6-dioxopiperidin-3-yl)-5,7-difluoroisoindoline-1,3-dione |
| | 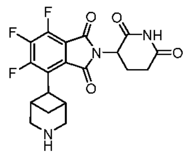 | 4-(3-azabicyclo[3.1.1]heptan-6-yl)-2-(2,6-dioxopiperidin-3-yl)-5,6,7-trifluoroisoindoline-1,3- dione | 4-(3-azabicyclo[3.1.1]heptan-6-yl)-2-(2,6-dioxopiperidin-3-yl)-5,6,7-trifluoroisoindoline-1,3-dione |
| | 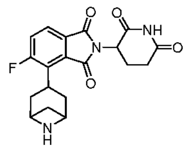 | 4-(6-azabicyclo[3.1.1]heptan-3-yl)-2-(2,6-dioxopiperidin-3-yl)-5-fluoroisoindoline-1,3-dione | 4-(6-azabicyclo[3.1.1]heptan-3-yl)-2-(2,6-dioxopiperidin-3-yl)-5-fluoroisoindoline-1,3- dione |
| | 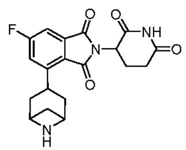 | 4-(6-azabicyclo[3.1.1]heptan-3-yl)-2-(2,6-dioxopiperidin-3-yl)-6-fluoroisoindoline-1,3-dione | 4-(6-azabicyclo[3.1.1]heptan-3-yl)-2-(2,6-dioxopiperidin-3-yl)-6-fluoroisoindoline-1,3-dione |
| | 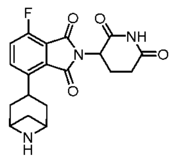 | 4-(6-azabicyclo[3.1.1]heptan-3-yl)-2-(2,6-dioxopiperidin-3-yl)-7-fluoroisoindoline-1,3-dione | 4-(6-azabicyclo[3.1.1]heptan-3-yl)-2-(2,6-dioxopiperidin-3-yl)-7-fluoroisoindoline-1,3- dione |
| | 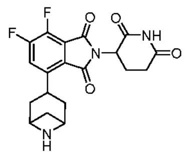 | 7-(6-azabicyclo[3.1.1]heptan-3-yl)-2-(2,6-dioxopiperidin-3-yl)-4,5-difluoroisoindoline-1,3-dione | 7-(6-azabicyclo[3.1.1]heptan-3-yl)-2-(2,6-dioxopiperidin-3-yl)-4,5-difluoroisoindoline-1,3-dione |

(continued)

| Compound No. | Structure of compound | Compound's name | Chinese translation of Compound's name |
|---|---|---|---|
| | 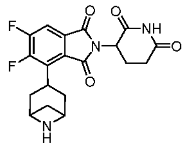 | 4-(6-azabicyclo[3.1.1]heptan-3-yl)-2-(2,6-dioxopiperidin-3-yl)-5,6-difluoroisoindoline-1,3-dione | 4-(6-azabicyclo[3.1.1]heptan-3-yl)-2-(2,6-dioxopiperidin-3-yl)-5,6-difluoroisoindoline-1,3-dione |
| | 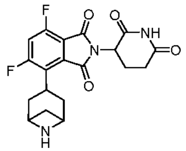 | 4-(6-azabicyclo[3.1.1]heptan-3-yl)-2-(2,6-dioxopiperidin-3-yl)-5,7-difluoroisoindoline-1,3-dione | 4-(6-azabicyclo[3.1.1]heptan-3-yl)-2-(2,6-dioxopiperidin-3-yl)-5,7-difluoroisoindoline-1,3-dione |
| | 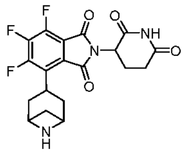 | 4-(6-azabicyclo[3.1.1]heptan-3-yl)-2-(2,6-dioxopiperidin-3-yl)-5,6,7-trifluoroisoindoline-1,3- dione | 4-(6-azabicyclo[3.1.1]heptan-3-yl)-2-(2,6-dioxopiperidin-3-yl)-5,6,7-trifluoroisoindoline-1,3-dione |
| | 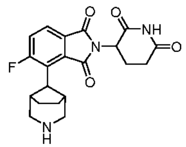 | 4-((1R,5S,8r)-3-azabicyclo[3.2.1]octan-8-yl)-2-(2,6-dioxopiperidin-3-yl)-5-fluoroisoindoline-1,3-dione | 4-((1R,5S,8r)-3-azabicyclo[3.2.1]octan-8-yl)-2-(2,6-dioxopiperidin-3-yl)-5-fluoroisoindoline-1,3-dione |
| | 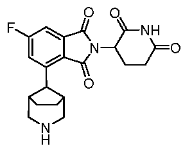 | 4-((1R,5S,8r)-3-azabicyclo[3.2.1]octan-8-yl)-2-(2,6-dioxopiperidin-3-yl)-6-fluoroisoindoline-1,3-dione | 4-((1R,5S,8r)-3-azabicyclo[3.2.1]octan-8-yl)-2-(2,6-dioxopiperidin-3-yl)-6-fluoroisoindoline-1,3-dione |
| | 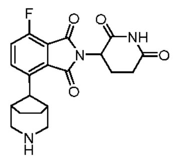 | 4-((1R,5S,8r)-3-azabicyclo[3.2.1]octan-8-yl)-2-(2,6-dioxopiperidin-3-yl)-7-fluoroisoindoline-1,3-dione | 4-((1R,5S,8r)-3-azabicyclo[3.2.1]octan-8-yl)-2-(2,6-dioxopiperidin-3-yl)-7-fluoroisoindoline-1,3-dione |

(continued)

| Compound No. | Structure of compound | Compound's name | Chinese translation of Compound's name |
|---|---|---|---|
| | 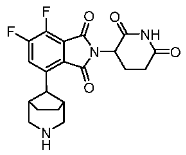 | 7-((1R,5S,8r)-3-azabicyclo[3.2.1]octan-8-yl)-2-(2,6-dioxopiperidin-3-yl)-4,5-difluoroisoindoline-1,3-dione | 7-((1R,5S,8r)-3-azabicyclo[3.2.1]octan-8-yl)-2-(2,6-dioxopiperidin-3-yl)-4,5-difluoroisoindoline-1,3-dione |
| | 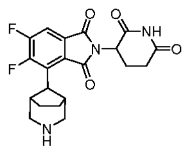 | 4-((1R,5S,8r)-3-azabicyclo[3.2.1]octan-8-yl)-2-(2,6-dioxopiperidin-3-yl)-5,6-difluoroisoindoline-1,3-dione | 4-((1R,5S,8r)-3-azabicyclo[3.2.1]octan-8-yl)-2-(2,6-dioxopiperidin-3-yl)-5,6-difluoroisoindoline-1,3-dione |
| | 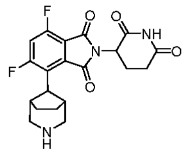 | 4-((1R,5S,8r)-3-azabicyclo[3.2.1]octan-8-yl)-2-(2,6-dioxopiperidin-3-yl)-5,7-difluoroisoindoline-1,3-dione | 4-((1R,5S,8r)-3-azabicyclo[3.2.1]octan-8-yl)-2-(2,6-dioxopiperidin-3-yl)-5,7-difluoroisoindoline-1,3-dione |
| | 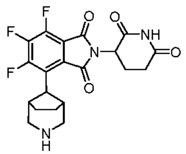 | 4-((1R,5S,8r)-3-azabicyclo[3.2.1]octan-8-yl)-2-(2,6-dioxopiperidin-3-yl)-5,6,7-trifluoroisoindoline-1,3-dione | 4-((1R,5S,8r)-3-azabicyclo[3.2.1]octan-8-yl)-2-(2,6-dioxopiperidin-3-yl)-5,6,7-trifluoroisoindoline-1,3-dione |
| | 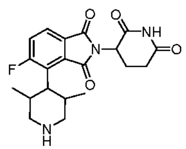 | 4-(3,5-dimethylpiperidin-4-yl)-2-(2,6-dioxopiperidin-3-yl)-5-fluoroisoindoline-1,3-dione | 4-(3,5-dimethylpiperidin-4-yl)-2-(2,6-dioxopiperidin-3-yl)-5-fluoroisoindoline-1,3- dione |
| | 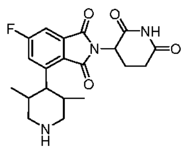 | 4-(3,5-dimethylpiperidin-4-yl)-2-(2,6-dioxopiperidin-3-yl)-6-fluoroisoindoline-1,3-dione | 4-(3,5-dimethylpiperidin-4-yl)-2-(2,6-dioxopiperidin-3-yl)-6-fluoroisoindoline-1,3-dione |

(continued)

| Compound No. | Structure of compound | Compound's name | Chinese translation of Compound's name |
|---|---|---|---|
| | 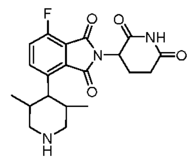 | 4-(3,5-dimethylpiperidin-4-yl)-2-(2,6-dioxopiperidin-3-yl)-7-fluoroisoindoline-1,3-dione | 4-(3,5-dimethylpiperidin-4-yl)-2-(2,6-dioxopiperidin-3-yl)-7-fluoroisoindoline-1,3- dione |
| | 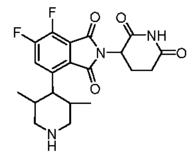 | 7-(3,5-dimethylpiperidin-4-yl)-2-(2,6-dioxopiperidin-3-yl)-4,5-difluoroisoindoline-1,3-dione | 7-(3,5-dimethylpiperidin-4-yl)-2-(2,6-dioxopiperidin-3-yl)-4,5-difluoroisoindoline-1,3-dione |
| | 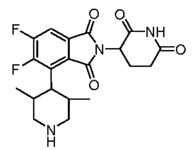 | 4-(3,5-dimethylpiperidin-4-yl)-2-(2,6-dioxopiperidin-3-yl)-5,6-difluoroisoindoline-1,3-dione | 4-(3,5-dimethylpiperidin-4-yl)-2-(2,6-dioxopiperidin-3-yl)-5,6-difluoroisoindoline-1,3-dione |
| | 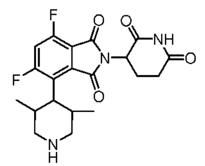 | 4-(3,5-dimethylpiperidin-4-yl)-2-(2,6-dioxopiperidin-3-yl)-5,7-difluoroisoindoline-1,3-dione | 4-(3,5-dimethylpiperidin-4-yl)-2-(2,6-dioxopiperidin-3-yl)-5,7-difluoroisoindoline-1,3-dione |
| | 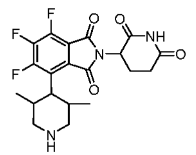 | 4-(3,5-dimethylpiperidin-4-yl)-2-(2,6-dioxopiperidin-3-yl)-5,6,7-trifluoroisoindoline-1,3-dione | 4-(3,5-dimethylpiperidin-4-yl)-2-(2,6-dioxopiperidin-3-yl)-5,6,7-trifluoroisoindoline-1,3-dione |
| | 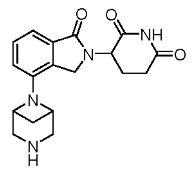 | 3-(4-(3,6-diazabicyclo[3.1.1]heptan-6-yl)-1-oxoisoindolin-2-yl)piperidine-2,6-dione | 3-(4-(3,6-diazabicyclo[3.1.1]heptan-6-yl)-1-oxoisoindolin-2-yl)piperidine-2,6-dione |

(continued)

| Compound No. | Structure of compound | Compound's name | Chinese translation of Compound's name |
|---|---|---|---|
| | 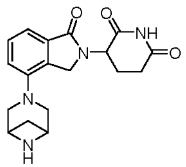 | 3-(4-(3,6-diazabicyclo[3.1.1]heptan-3-yl)-1-oxoisoindolin-2-yl)piperidine-2,6-dione | 3-(4-(3,6-diazabicyclo[3.1.1]heptan-3-yl)-1-oxoisoindolin-2-yl)piperidine-2,6-dione |
| | 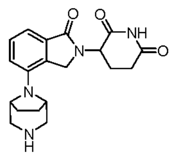 | 3-(4-((1R,5S)-3,8-diazabicyclo[3.2.1]octan-8-yl)-1-oxoisoindolin-2-yl)piperidine-2,6-dione | 3-(4-((1R,5S)-3,8-diazabicyclo[3.2.1]octan-8-yl)-1-oxoisoindolin-2-yl)piperidine-2,6-dione |
| | 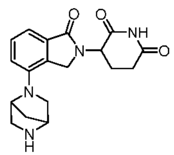 | 3-(4-(2,5-diazabicyclo[2.2.1]heptan-2-yl)-1-oxoisoindolin-2-yl)piperidine-2,6-dione | 3-(4-(2,5-diazabicyclo[2.2.1]heptan-2-yl)-1-oxoisoindolin-2-yl)piperidine-2,6-dione |
| | 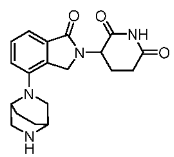 | 3-(4-(2,5-diazabicyclo[2.2.2]octan-2-yl)-1-oxoisoindolin-2-yl)piperidine-2,6-dione | 3-(4-(2,5-diazabicyclo[2.2.2]octan-2-yl)-1-oxoisoindolin-2-yl)piperidine-2,6-dione |
| | 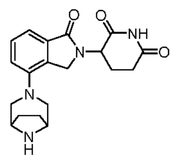 | 3-(4-((1R,5S)-3,8-diazabicyclo[3.2.1]octan-3-yl)-1-oxoisoindolin-2-yl)piperidine-2,6-dione | 3-(4-((1R,5S)-3,8-diazabicyclo[3.2.1]octan-3-yl)-1-oxoisoindolin-2-yl)piperidine-2,6-dione |
| | 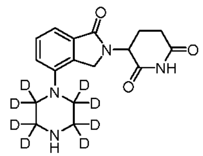 | 3-(1-oxo-4-(piperazin-1-yl-2,2,3,3,5,5,6,6-$d_8$)isoindolin-2-yl)piperidine-2,6-dione | 3-(1-oxo-4-(piperazin-1-yl-2,2,3,3,5,5,6,6-$d_8$)isoindolin-2-yl)piperidine-2,6-dione |

(continued)

| Compound No. | Structure of compound | Compound's name | Chinese translation of Compound's name |
|---|---|---|---|
| | 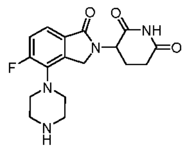 | 3-(5-fluoro-1-oxo-4-(piperazin-1-yl)isoindolin-2-yl)piperidine-2,6-dione | 3-(5-fluoro-1-oxo-4-(piperazin-1-yl)isoindolin-2-yl)piperidine-2,6-dione |
| | 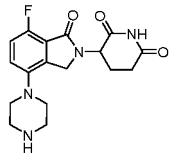 | 3-(7-fluoro-1-oxo-4-(piperazin-1-yl)isoindolin-2-yl)piperidine-2,6-dione | 3-(7-fluoro-1-oxo-4-(piperazin-1-yl)isoindolin-2-yl)piperidine-2,6-dione |
| | 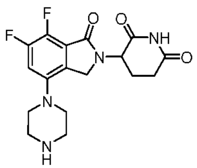 | 3-(6,7-difluoro-1-oxo-4-(piperazin-1-yl)isoindolin-2-yl)piperidine-2,6-dione | 3-(6,7-difluoro-1-oxo-4-(piperazin-1-yl)isoindolin-2-yl)piperidine-2,6-dione |
| | 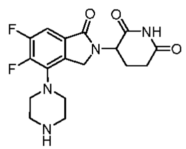 | 3-(5,6-difluoro-1-oxo-4-(piperazin-1-yl)isoindolin-2-yl)piperidine-2,6-dione | 3-(5,6-difluoro-1-oxo-4-(piperazin-1-yl)isoindolin-2-yl)piperidine-2,6-dione |
| | 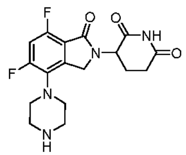 | 3-(5,7-difluoro-1-oxo-4-(piperazin-1-yl)isoindolin-2-yl)piperidine-2,6-dione | 3-(5,7-difluoro-1-oxo-4-(piperazin-1-yl)isoindolin-2-yl)piperidine-2,6-dione |
| | 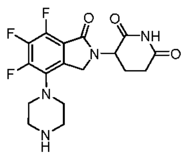 | 3-(5,6,7-trifluoro-1-oxo-4-(piperazin-1-yl)isoindolin-2-yl)piperidine-2,6-dione | 3-(5,6,7-trifluoro-1-oxo-4-(piperazin-1-yl)isoindolin-2-yl)piperidine-2,6-dione |

(continued)

| Compound No. | Structure of compound | Compound's name | Chinese translation of Compound's name |
|---|---|---|---|
| | 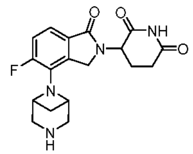 | 3-(4-(3,6-diazabicyclo[3.1.1]heptan-6-yl)-5-fluoro-1-oxoisoindolin-2-yl)piperidine-2,6-dione | 3-(4-(3,6-diazabicyclo[3.1.1]heptan-6-yl)-5-fluoro-1-oxoisoindolin-2-yl)piperidine-2,6-dione |
| | 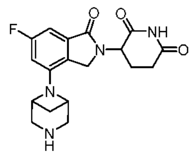 | 3-(4-(3,6-diazabicyclo[3.1.1]heptan-6-yl)-6-fluoro-1-oxoisoindolin-2- yl)piperidine-2,6-dione | 3-(4-(3,6-diazabicyclo[3.1.1]heptan-6-yl)-6-fluoro-1-oxoisoindolin-2-yl)piperidine-2,6-dione |
| | 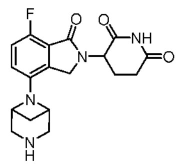 | 3-(4-(3,6-diazabicyclo[3.1.1]heptan-6-yl)-7-fluoro-1-oxoisoindolin-2-yl)piperidine-2,6-dione | 3-(4-(3,6-diazabicyclo[3.1.1]heptan-6-yl)-7-fluoro-1-oxoisoindolin-2-yl)piperidine-2,6-dione |
| | 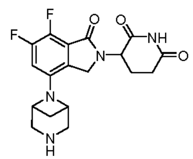 | 3-(4-(3,6-diazabicyclo[3.1.1]heptan-6-yl)-6,7-difluoro-1-oxoisoindolin-2-yl)piperidine-2,6-dione | 3-(4-(3,6-diazabicyclo[3.1.1]heptan-6-yl)-6,7-difluoro-1-oxoisoindolin-2-yl)piperidine-2,6-dione |
| | 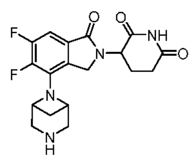 | 3-(4-(3,6-diazabicyclo[3.1.1]heptan-6-yl)-5,6-difluoro-1-oxoisoindolin-2-yl)piperidine-2,6-dione | 3-(4-(3,6-diazabicyclo[3.1.1]heptan-6-yl)-5,6-difluoro-1-oxoisoindolin-2-yl)piperidine-2,6-dione |
| | 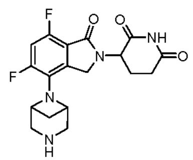 | 3-(4-(3,6-diazabicyclo[3.1.1]heptan-6-yl)-5,7-difluoro-1-oxoisoindolin-2-yl)piperidine-2,6-dione | 3-(4-(3,6-diazabicyclo[3.1.1]heptan-6-yl)-5,7-difluoro-1-oxoisoindolin-2-yl)piperidine-2,6-dione |

(continued)

| Compound No. | Structure of compound | Compound's name | Chinese translation of Compound's name |
|---|---|---|---|
| | 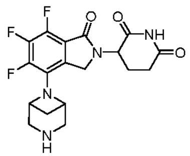 | 3-(4-(3,6-diazabicyclo[3.1.1]heptan-6-yl)-5,6,7-trifluoro-1-oxoisoindolin-2-yl)piperidine-2,6-dione | 3-(4-(3,6-diazabicyclo[3.1.1]heptan-6-yl)-5,6,7-trifluoro-1-oxoisoindolin-2-yl)piperidine-2,6-dione |
| | 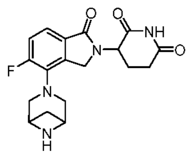 | 3-(4-(3,6-diazabicyclo[3.1.1]heptan-3-yl)-5-fluoro-1-oxoisoindolin-2-yl)piperidine-2,6-dione | 3-(4-(3,6-diazabicyclo[3.1.1]heptan-3-yl)-5-fluoro-1-oxoisoindolin-2-yl)piperidine-2,6-dione |
| | 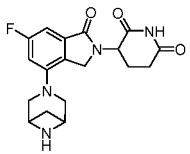 | 3-(4-(3,6-diazabicyclo[3.1.1]heptan-3-yl)-6-fluoro-1-oxoisoindolin-2-yl)piperidine-2,6-dione | 3-(4-(3,6-diazabicyclo[3.1.1]heptan-3-yl)-6-fluoro-1-oxoisoindolin-2-yl)piperidine-2,6-dione |
| | 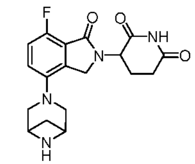 | 3-(4-(3,6-diazabicyclo[3.1.1]heptan-3-yl)-7-fluoro-1-oxoisoindolin-2-yl)piperidine-2,6-dione | 3-(4-(3,6-diazabicyclo[3.1.1]heptan-3-yl)-7-fluoro-1-oxoisoindolin-2-yl)piperidine-2,6-dione |
| | 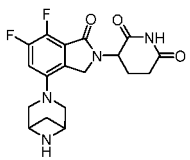 | 3-(4-(3,6-diazabicyclo[3.1.1]heptan-3-yl)-6,7-difluoro-1-oxoisoindolin-2-yl)piperidine-2,6-dione | 3-(4-(3,6-diazabicyclo[3.1.1]heptan-3-yl)-6,7-difluoro-1-oxoisoindolin-2-yl)piperidine-2,6-dione |
| | 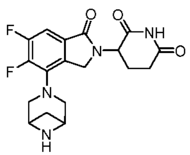 | 3-(4-(3,6-diazabicyclo[3.1.1]heptan-3-yl)-5,6-difluoro-1-oxoisoindolin-2-yl)piperidine-2,6-dione | 3-(4-(3,6-diazabicyclo[3.1.1]heptan-3-yl)-5,6-difluoro-1-oxoisoindolin-2-yl)piperidine-2,6-dione |

(continued)

| Compound No. | Structure of compound | Compound's name | Chinese translation of Compound's name |
|---|---|---|---|
| | 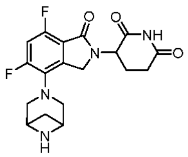 | 3-(4-(3,6-diazabicyclo[3.1.1]heptan-3-yl)-5,7-difluoro-1-oxoisoindolin-2-yl)piperidine-2,6-dione | 3-(4-(3,6-diazabicyclo[3.1.1]heptan-3-yl)-5,7-difluoro-1-oxoisoindolin-2-yl)piperidine-2,6-dione |
| | 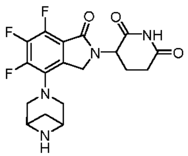 | 3-(4-(3,6-diazabicyclo[3.1.1]heptan-3-yl)-5,6,7-trifluoro-1-oxoisoindolin-2-yl)piperidine-2,6-dione | 3-(4-(3,6-diazabicyclo[3.1.1]heptan-3-yl)-5,6,7-trifluoro-1-oxoisoindolin-2-yl)piperidine-2,6-dione |
| | 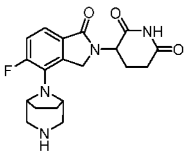 | 3-(4-((1R,5S)-3,8-diazabicyclo[3.2.1]octan-8-yl)-5-fluoro-1-oxoisoindolin-2-yl)piperidine-2,6-dione | 3-(4-((1R,5S)-3,8-diazabicyclo[3.2.1]octan-8-yl)-5-fluoro-1-oxoisoindolin-2-yl)piperidine-2,6-dione |
| | 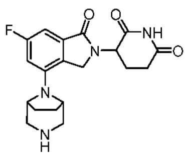 | 3-(4-((1R,5S)-3,8-diazabicyclo[3.2.1]octan-8-yl)-6-fluoro-1-oxoisoindolin-2-yl)piperidine-2,6-dione | 3-(4-((1R,5S)-3,8-diazabicyclo[3.2.1]octan-8-yl)-6-fluoro-1-oxoisoindolin-2-yl)piperidine-2,6-dione |
| | 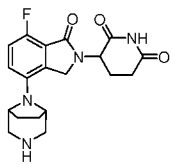 | 3-(4-((1R,5S)-3,8-diazabicyclo[3.2.1]octan-8-yl)-7-fluoro-1-oxoisoindolin-2-yl)piperidine-2,6-dione | 3-(4-((1R,5S)-3,8-diazabicyclo[3.2.1]octan-8-yl)-7-fluoro-1-oxoisoindolin-2-yl)piperidine-2,6-dione |
| | 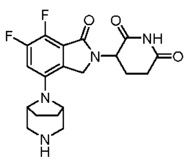 | 3-(4-((1R,5S)-3,8-diazabicyclo[3.2.1]octan-8-yl)-6,7-difluoro-1-oxoisoindolin-2-yl)piperidine-2,6-dione | 3-(4-((1R,5S)-3,8-diazabicyclo[3.2.1]octan-8-yl)-6,7-difluoro-1-oxoisoindolin-2-yl)piperidine-2,6-dione |

(continued)

| Compound No. | Structure of compound | Compound's name | Chinese translation of Compound's name |
|---|---|---|---|
| | 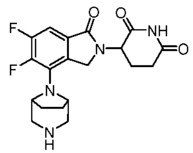 | 3-(4-((1R,5S)-3,8-diazabicyclo[3.2.1]octan-8-yl)-5,6-difluoro-1-oxoisoindolin-2-yl)piperidine-2,6-dione | 3-(4-((1R,5S)-3,8-diazabicyclo[3.2.1]octan-8-yl)-5,6-difluoro-1-oxoisoindolin-2-yl)piperidine-2,6-dione |
| | 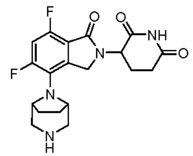 | 3-(4-((1R,5S)-3,8-diazabicyclo[3.2.1]octan-8-yl)-5,7-difluoro-1-oxoisoindolin-2-yl)piperidine-2,6-dione | 3-(4-((1R,5S)-3,8-diazabicyclo[3.2.1]octan-8-yl)-5,7-difluoro-1-oxoisoindolin-2-yl)piperidine-2,6-dione |
| | 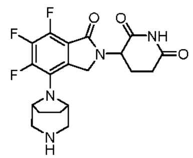 | 3-(4-((1R,5S)-3,8-diazabicyclo[3.2.1]octan-8-yl)-5, 6, 7-trifluoro-1-oxoisoindolin-2-yl)piperidine-2,6-dione | 3-(4-((1R,5S)-3,8-diazabicyclo[3.2.1]octan-8-yl)-5,6,7-trifluoro-1-oxoisoindolin-2-yl)piperidine-2,6-dione |
| | 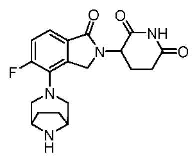 | 3-(4-((1R,5S)-3,8-diazabicyclo[3.2.1]octan-3-yl)-5-fluoro-1-oxoisoindolin-2-yl)piperidine-2,6-dione | 3-(4-((1R,5S)-3,8-diazabicyclo[3.2.1]octan-3-yl)-5-fluoro-1-oxoisoindolin-2-yl)piperidine-2,6-dione |
| | 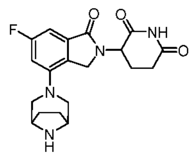 | 3-(4-((1R,5S)-3,8-diazabicyclo[3.2.1]octan-3-yl)-6-fluoro-1-oxoisoindolin-2-yl)piperidine-2,6-dione | 3-(4-((1R,5S)-3,8-diazabicyclo[3.2.1]octan-3-yl)-6-fluoro-1-oxoisoindolin-2-yl)piperidine-2,6-dione |
| | 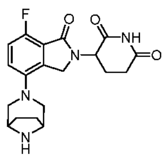 | 3-(4-((1R,5S)-3,8-diazabicyclo[3.2.1]octan-3-yl)-7-fluoro-1-oxoisoindolin-2-yl)piperidine-2,6-dione | 3-(4-((1R,5S)-3,8-diazabicyclo[3.2.1]octan-3-yl)-7-fluoro-1-oxoisoindolin-2-yl)piperidine-2,6-dione |

(continued)

| Compound No. | Structure of compound | Compound's name | Chinese translation of Compound's name |
|---|---|---|---|
| | 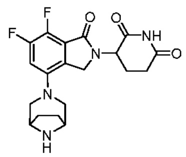 | 3-(4-((1R,5S)-3,8-diazabicyclo[3.2.1]octan-3-yl)-6,7-difluoro-1-oxoisoindolin-2-yl)piperidine-2,6-dione | 3-(4-((1R,5S)-3,8-diazabicyclo[3.2.1]octan-3-yl)-6,7-difluoro-1-oxoisoindolin-2-yl)piperidine-2,6-dione |
| | 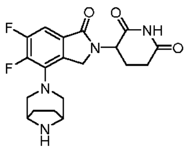 | 3-(4-((1R,5S)-3,8-diazabicyclo[3.2.1]octan-3-yl)-5,6-difluoro-1-oxoisoindolin-2-yl)piperidine-2,6-dione | 3-(4-((1R,5S)-3,8-diazabicyclo[3.2.1]octan-3-yl)-5,6-difluoro-1-oxoisoindolin-2-yl)piperidine-2,6-dione |
| | 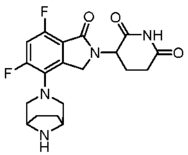 | 3-(4-((1R,5S)-3,8-diazabicyclo[3.2.1]octan-3-yl)-5,7-difluoro-1-oxoisoindolin-2- yl)piperidine-2,6-dione | 3-(4-((1R,5S)-3,8-diazabicyclo[3.2.1]octan-3-yl)-5,7-difluoro-1-oxoisoindolin-2-yl)piperidine-2,6-dione |
| | 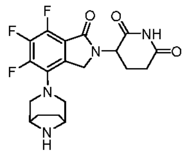 | 3-(4-((1R,5S)-3,8-diazabicyclo[3.2.1]octan-3-yl)-5,6,7-trifluoro-1-oxoisoindolin-2-yl)piperidine-2,6-dione | 3-(4-((1R,5S)-3,8-diazabicyclo[3.2.1]octan-3-yl)-5,6,7-trifluoro-1-oxoisoindolin-2-yl)piperidine-2,6-dione |
| | 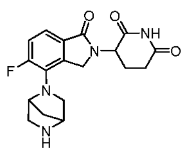 | 3-(4-(2,5-diazabicyclo[2.2.1]heptan-2-yl)-5-fluoro-1-oxoisoindolin-2- yl)piperidine-2,6-dione | 3-(4-(2,5-diazabicyclo[2.2.1]heptan-2-yl)-5-fluoro-1-oxoisoindolin-2-yl)piperidine-2,6-dione |
| | 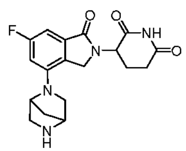 | 3-(4-(2,5-diazabicyclo[2.2.1]heptan-2-yl)-6-fluoro-1-oxoisoindolin-2- yl)piperidine-2,6-dione | 3-(4-(2,5-diazabicyclo[2.2.1]heptan-2-yl)-6-fluoro-1-oxoisoindolin-2-yl)piperidine-2,6-dione |

(continued)

| Compound No. | Structure of compound | Compound's name | Chinese translation of Compound's name |
|---|---|---|---|
| | 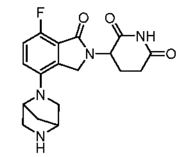 | 3-(4-(2,5-diazabicyclo[2.2.1]heptan-2-yl)-7-fluoro-1-oxoisoindolin-2-yl)piperidine-2,6-dione | 3-(4-(2,5-diazabicyclo[2.2.1]heptan-2-yl)-7-fluoro-1-oxoisoindolin-2-yl)piperidine-2,6-dione |
| | 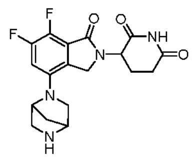 | 3-(4-(2,5-diazabicyclo[2.2.1]heptan-2-yl)-6,7-difluoro-1-oxoisoindolin-2- yl)piperidine-2,6-dione | 3-(4-(2,5-diazabicyclo[2.2.1]heptan-2-yl)-6,7-difluoro-1-oxoisoindolin-2-yl)piperidine-2,6-dione |
| | 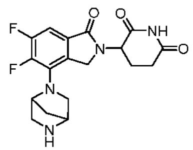 | 3-(4-(2,5-diazabicyclo[2.2.1]heptan-2-yl)-5,6-difluoro-1-oxoisoindolin-2- yl)piperidine-2,6-dione | 3-(4-(2,5-diazabicyclo[2.2.1]heptan-2-yl)-5,6-difluoro-1-oxoisoindolin-2-yl)piperidine-2,6-dione |
| | 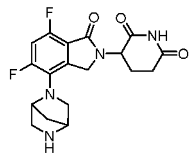 | 3-(4-(2,5-diazabicyclo[2.2.1]heptan-2-yl)-5,7-difluoro-1-oxoisoindolin-2- yl)piperidine-2,6-dione | 3-(4-(2,5-diazabicyclo[2.2.1]heptan-2-yl)-5,7-difluoro-1-oxoisoindolin-2-yl)piperidine-2,6-dione |
| | 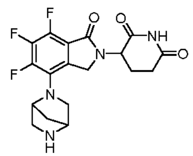 | 3-(4-(2,5-diazabicyclo[2.2.1]heptan-2-yl)-5,6,7-trifluoro-1-oxoisoindolin-2-yl)piperidine-2,6-dione | 3-(4-(2,5-diazabicyclo[2.2.1]heptan-2-yl)-5,6,7-trifluoro-1-oxoisoindolin-2-yl)piperidine-2,6-dione |
| | 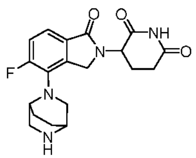 | 3-(4-(2,5-diazabicyclo[2.2.2]octan-2-yl)-5- fluoro-1-oxoisoindolin-2-yl)piperidine-2,6-dione | 3-(4-(2,5-diazabicyclo[2.2.2]octan-2-yl)-5-fluoro-1-oxoisoindolin-2-yl)piperidine-2,6-dione |

(continued)

| Compound No. | Structure of compound | Compound's name | Chinese translation of Compound's name |
|---|---|---|---|
| | 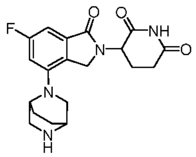 | 3-(4-(2,5-diazabicyclo[2.2.2]octan-2-yl)-6-fluoro-1-oxoisoindolin-2-yl)piperidine-2,6-dione | 3-(4-(2,5-diazabicyclo[2.2.2]octan-2-yl)-6-fluoro-1-oxoisoindolin-2-yl)piperidine-2,6-dione |
| | 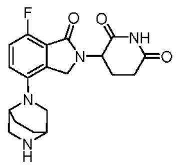 | 3-(4-(2,5-diazabicyclo[2.2.2]octan-2-yl)-7- fluoro-1-oxoisoindolin-2-yl)piperidine-2,6-dione | 3-(4-(2,5-diazabicyclo[2.2.2]octan-2-yl)-7-fluoro-1-oxoisoindolin-2-yl)piperidine-2,6-dione |
| | 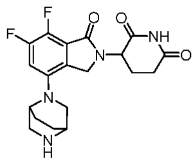 | 3-(4-(2,5-diazabicyclo[2.2.2]octan-2-yl)-6,7-difluoro-1-oxoisoindolin-2-yl)piperidine-2,6-dione | 3-(4-(2,5-diazabicyclo[2.2.2]octan-2-yl)-6,7-difluoro-1-oxoisoindolin-2-yl)piperidine-2,6-dione |
| | 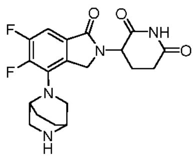 | 3-(4-(2,5-diazabicyclo[2.2.2]octan-2-yl)-5,6-difluoro-1-oxoisoindolin-2-yl)piperidine-2,6-dione | 3-(4-(2,5-diazabicyclo[2.2.2]octan-2-yl)-5,6-difluoro-1-oxoisoindolin-2-yl)piperidine-2,6-dione |
| | 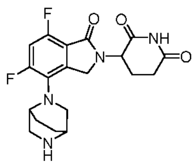 | 3-(4-(2,5-diazabicyclo[2.2.2]octan-2-yl)-5,7-difluoro-1-oxoisoindolin-2- yl)piperidine-2,6-dione | 3-(4-(2,5-diazabicyclo[2.2.2]octan-2-yl)-5,7-difluoro-1-oxoisoindolin-2-yl)piperidine-2,6-dione |
| | 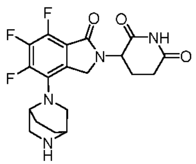 | 3-(4-(2,5-diazabicyclo[2.2.2]octan-2-yl)-5,6,7-trifluoro-1-oxoisoindolin-2-yl)piperidine-2,6-dione | 3-(4-(2,5-diazabicyclo[2.2.2]octan-2-yl)-5,6,7-trifluoro-1-oxoisoindolin-2-yl)piperidine-2,6-dione |

(continued)

| Compound No. | Structure of compound | Compound's name | Chinese translation of Compound's name |
|---|---|---|---|
| | 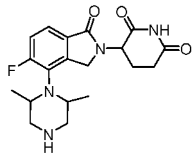 | 3-(4-(2,6-dimethylpiperazin-1-yl)-5-fluoro-1-oxoisoindolin-2-yl)piperidine-2,6-dione | 3-(4-(2,6-dimethylpiperazin-1-yl)-5-fluoro-1-oxoisoindolin-2-yl)piperidine-2,6-dione |
| | 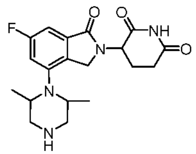 | 3-(4-(2,6-dimethylpiperazin-1-yl)-6-fluoro-1-oxoisoindolin-2-yl)piperidine-2,6-dione | 3-(4-(2,6-dimethylpiperazin-1-yl)-6-fluoro-1-oxoisoindolin-2-yl)piperidine-2,6-dione |
| | 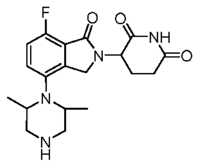 | 3-(4-(2,6-dimethylpiperazin-1-yl)-7-fluoro-1-oxoisoindolin-2-yl)piperidine-2,6-dione | 3-(4-(2,6-dimethylpiperazin-1-yl)-7-fluoro-1-oxoisoindolin-2-yl)piperidine-2,6-dione |
| | 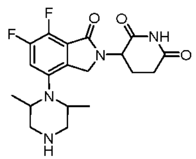 | 3-(4-(2,6-dimethylpiperazin-1-yl)-6,7-difluoro-1-oxoisoindolin-2- yl)piperidine-2,6-dione | 3-(4-(2,6-dimethylpiperazin-1-yl)-6,7-difluoro-1-oxoisoindolin-2-yl)piperidine-2,6-dione |
| | 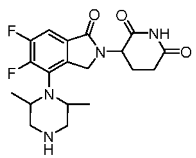 | 3-(4-(2,6-dimethylpiperazin-1-yl)-5,6-difluoro-1-oxoisoindolin-2- yl)piperidine-2,6-dione | 3-(4-(2,6-dimethylpiperazin-1-yl)-5,6-difluoro-1-oxoisoindolin-2-yl)piperidine-2,6-dione |
| | 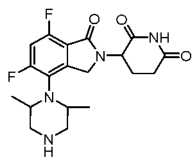 | 3-(4-(2,6-dimethylpiperazin-1-yl)-5,7-difluoro-1-oxoisoindolin-2- yl)piperidine-2,6-dione | 3-(4-(2,6-dimethylpiperazin-1-yl)-5,7-difluoro-1-oxoisoindolin-2-yl)piperidine-2,6-dione |

(continued)

| Compound No. | Structure of compound | Compound's name | Chinese translation of Compound's name |
|---|---|---|---|
| | 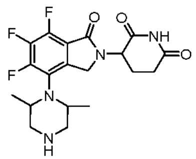 | 3-(4-(2,6-dimethylpiperazin-1-yl)-5,6,7-trifluoro-1-oxoisoindolin-2-yl)piperidine-2,6-dione | 3-(4-(2,6-dimethylpiperazin-1-yl)-5,6,7-trifluoro-1-oxoisoindolin-2-yl)piperidine-2,6-dione |
| | 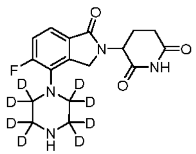 | 3-(5-fluoro-1-oxo-4-(piperazin-1-yl-2,2,3,3,5,5,6,6-*d*$_8$)isoindolin-2-yl)piperidine-2,6-dione | 3-(5-fluoro-1-oxo-4-(piperazin-1-yl-2,2,3,3,5,5,6,6-*d*$_8$)isoindolin-2-yl)piperidine-2,6-dione |
| | 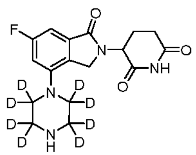 | 3-(6-fluoro-1-oxo-4-(piperazin-1-yl-2,2,3,3,5,5,6,6-*d*$_8$)isoindolin-2-yl)piperidine-2,6-dione | 3-(6-fluoro-1-oxo-4-(piperazin-1-yl-2,2,3,3,5,5,6,6-*d*$_8$)isoindolin-2-yl)piperidine-2,6-dione |
| | 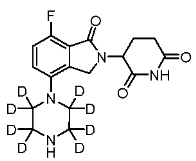 | 3-(7-fluoro-1-oxo-4-(piperazin-1-yl-2,2,3,3,5,5,6,6-*d*$_8$)isoindolin-2-yl)piperidine-2,6-dione | 3-(7-fluoro-1-oxo-4-(piperazin-1-yl-2,2,3,3,5,5,6,6-*d*$_8$)isoindolin-2-yl)piperidine-2,6-dione |
| | 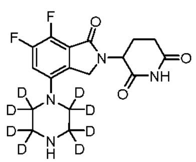 | 3-(6,7-difluoro-1-oxo-4-(piperazin-1-yl-2,2,3,3,5,5,6,6-*d*$_8$)isoindolin-2-yl)piperidine-2,6-dione | 3-(6,7-difluoro-1-oxo-4-(piperazin-1-yl-2,2,3,3,5,5,6,6-*d*$_8$)isoindolin-2-yl)piperidine-2,6-dione |
| | 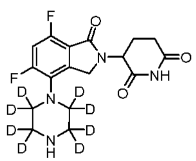 | 3-(5,7-difluoro-1-oxo-4-(piperazin-1-yl-2,2,3,3,5,5,6,6-*d*$_8$)isoindolin-2-yl)piperidine-2,6-dione | 3-(5,7-difluoro-1-oxo-4-(piperazin-1-yl-2,2,3,3,5,5,6,6-*d*$_8$)isoindolin-2-yl)piperidine-2,6-dione |

(continued)

| Compound No. | Structure of compound | Compound's name | Chinese translation of Compound's name |
|---|---|---|---|
| | 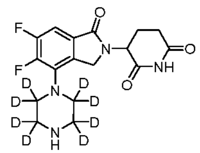 | 3-(5,6-difluoro-1-oxo-4-(piperazin-1-yl-2,2,3,3,5,5,6,6-*$d_8$*)isoindolin-2-yl)piperidine-2,6-dione | 3-(5,6-difluoro-1-oxo-4-(piperazin-1-yl-2,2,3,3,5,5,6,6-*$d_8$*)isoindolin-2-yl)piperidine-2,6-dione |
| | 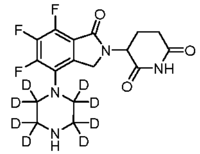 | 3-(5,6,7-trifluoro-1-oxo-4-(piperazin-1-yl-2,2,3,3,5,5,6,6-*$d_8$*)isoindolin-2-yl)piperidine-2,6-dione | 3-(5,6,7-trifluoro-1-oxo-4-(piperazin-1-yl-2,2,3,3,5,5,6,6-*$d_8$*)isoindolin-2-yl)piperidine-2,6-dione |
| | 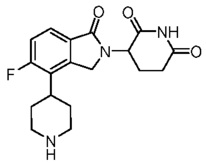 | 3-(5-fluoro-1-oxo-4-(piperidin-4-yl)isoindolin-2-yl)piperidine-2,6-dione | 3-(5-fluoro-1-oxo-4-(piperidin-4-yl)isoindolin-2-yl)piperidine-2,6-dione |
| | 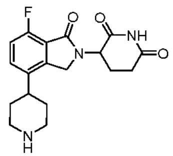 | 3-(7-fluoro-1-oxo-4-(piperidin-4-yl)isoindolin-2-yl)piperidine-2,6-dione | 3-(7-fluoro-1-oxo-4-(piperidin-4-yl)isoindolin-2-yl)piperidine-2,6-dione |
| | 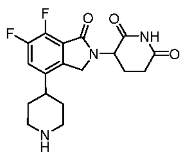 | 3-(6,7-difluoro-1-oxo-4-(piperidin-4-yl)isoindolin-2-yl)piperidine-2,6-dione | 3-(6,7-difluoro-1-oxo-4-(piperidin-4-yl)isoindolin-2-yl)piperidine-2,6-dione |
| | 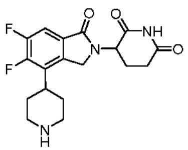 | 3-(5,6-difluoro-1-oxo-4-(piperidin-4-yl)isoindolin-2-yl)piperidine-2,6-dione | 3-(5,6-difluoro-1-oxo-4-(piperidin-4-yl)isoindolin-2-yl)piperidine-2,6-dione |

(continued)

| Compound No. | Structure of compound | Compound's name | Chinese translation of Compound's name |
|---|---|---|---|
| | 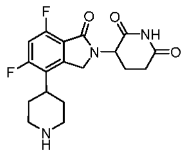 | 3-(5,7-difluoro-1-oxo-4-(piperidin-4-yl)isoindolin-2-yl)piperidine-2,6-dione | 3-(5,7-difluoro-1-oxo-4-(piperidin-4-yl)isoindolin-2-yl)piperidine-2,6-dione |
| | 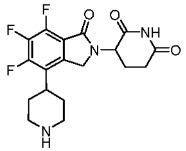 | 3-(5,6,7-trifluoro-1-oxo-4-(piperidin-4-yl)isoindolin-2-yl)piperidine-2,6-dione | 3-(5,6,7-trifluoro-1-oxo-4-(piperidin-4-yl)isoindolin-2-yl)piperidine-2,6-dione |
| | 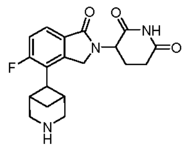 | 3-(4-(3-azabicyclo[3.1.1]heptan-6-yl)-5-fluoro-1-oxoisoindolin-2-yl)piperidine-2,6-dione | 3-(4-(3-azabicyclo[3.1.1]heptan-6-yl)-5-fluoro-1-oxoisoindolin-2-yl)piperidine-2,6-dione |
| | 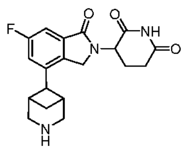 | 3-(4-(3-azabicyclo[3.1.1]heptan-6-yl)-6-fluoro-1-oxoisoindolin-2-yl)piperidine-2,6-dione | 3-(4-(3-azabicyclo[3.1.1]heptan-6-yl)-6-fluoro-1-oxoisoindolin-2-yl)piperidine-2,6-dione |
| | 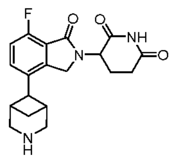 | 3-(4-(3-azabicyclo[3.1.1]heptan-6-yl)-7-fluoro-1-oxoisoindolin-2-yl)piperidine-2,6-dione | 3-(4-(3-azabicyclo[3.1.1]heptan-6-yl)-7-fluoro-1-oxoisoindolin-2-yl)piperidine-2,6-dione |
| | 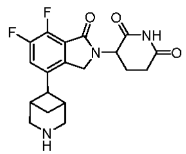 | 3-(4-(3-azabicyclo[3.1.1]heptan-6-yl)-6,7-difluoro-1-oxoisoindolin-2-yl)piperidine-2,6-dione | 3-(4-(3-azabicyclo[3.1.1]heptan-6-yl)-6,7-difluoro-1-oxoisoindolin-2-yl)piperidine-2,6-dione |

(continued)

| Compound No. | Structure of compound | Compound's name | Chinese translation of Compound's name |
|---|---|---|---|
| | 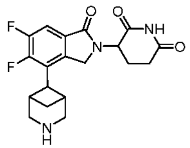 | 3-(4-(3-azabicyclo[3.1.1]heptan-6-yl)-5,6-difluoro-1-oxoisoindolin-2-yl)piperidine-2,6-dione | 3-(4-(3-azabicyclo[3.1.1]heptan-6-yl)-5,6-difluoro-1-oxoisoindolin-2-yl)piperidine-2,6-dione |
| | 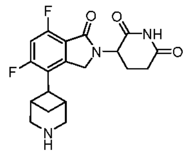 | 3-(4-(3-azabicyclo[3.1.1]heptan-6-yl)-5,7-difluoro-1-oxoisoindolin-2-yl)piperidine-2,6-dione | 3-(4-(3-azabicyclo[3.1.1]heptan-6-yl)-5,7-difluoro-1-oxoisoindolin-2-yl)piperidine-2,6-dione |
| | 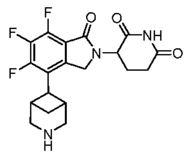 | 3-(4-(3-azabicyclo[3.1.1]heptan-6-yl)-5,6,7-trifluoro-1-oxoisoindolin-2-yl)piperidine-2,6-dione | 3-(4-(3-azabicyclo[3.1.1]heptan-6-yl)-5,6,7-trifluoro-1-oxoisoindolin-2-yl)piperidine-2,6-dione |
| | 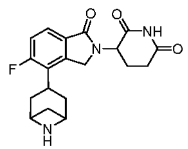 | 3-(4-(6-azabicyclo[3.1.1]heptan-3-yl)-5-fluoro-1-oxoisoindolin-2-yl)piperidine-2,6-dione | 3-(4-(6-azabicyclo[3.1.1]heptan-3-yl)-5-fluoro-1-oxoisoindolin-2-yl)piperidine-2,6-dione |
| | 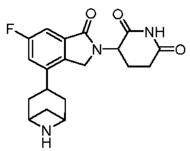 | 3-(4-(6-azabicyclo[3.1.1]heptan-3-yl)-6-fluoro-1-oxoisoindolin-2-yl)piperidine-2,6-dione | 3-(4-(6-azabicyclo[3.1.1]heptan-3-yl)-6-fluoro-1-oxoisoindolin-2-yl)piperidine-2,6-dione |
| | 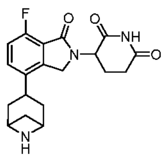 | 3-(4-(6-azabicyclo[3.1.1]heptan-3-yl)-7-fluoro-1-oxoisoindolin-2-yl)piperidine-2,6-dione | 3-(4-(6-azabicyclo[3.1.1]heptan-3 -yl)-7-fluoro-1-oxoisoindolin-2-yl)piperidine-2,6-dione |

(continued)

| Compound No. | Structure of compound | Compound's name | Chinese translation of Compound's name |
|---|---|---|---|
| | 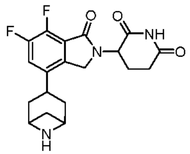 | 3-(4-(6-azabicyclo[3.1.1]heptan-3-yl)-6,7-difluoro-1-oxoisoindolin-2-yl)piperidine-2,6-dione | 3-(4-(6-azabicyclo[3.1.1]heptan-3-yl)-6,7-difluoro-1-oxoisoindolin-2-yl)piperidine-2,6-dione |
| | 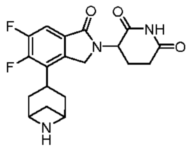 | 3-(4-(6-azabicyclo[3.1.1]heptan-3-yl)-5,6-difluoro-1-oxoisoindolin-2-yl)piperidine-2,6-dione | 3-(4-(6-azabicyclo[3.1.1]heptan-3-yl)-5,6-difluoro-1-oxoisoindolin-2-yl)piperidine-2,6-dione |
| | 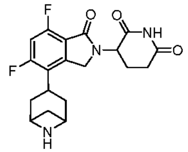 | 3-(4-(6-azabicyclo[3.1.1]heptan-3-yl)-5,7-difluoro-1-oxoisoindolin-2-yl)piperidine-2,6-dione | 3-(4-(6-azabicyclo[3.1.1]heptan-3-yl)-5,7-difluoro-1-oxoisoindolin-2-yl)piperidine-2,6-dione |
| | 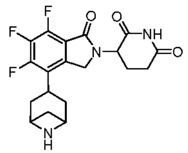 | 3-(4-(6-azabicyclo[3.1.1]heptan-3-yl)-5,6,7-trifluoro-1-oxoisoindolin-2-yl)piperidine-2,6-dione | 3-(4-(6-azabicyclo[3.1.1]heptan-3-yl)-5,6,7-trifluoro-1-oxoisoindolin-2-yl)piperidine-2,6-dione |
| | 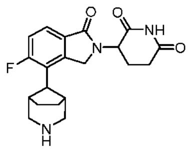 | 3-(4-((1R,5S,8r)-3-azabicyclo[3.2.1]octan-8-yl)-5-fluoro-1-oxoisoindolin-2-yl)piperidine-2,6-dione | 3-(4-((1R,5S,8r)-3-azabicyclo[3.2.1]octan-8-yl)-5-fluoro-1-oxoisoindolin-2- yl)piperidine-2,6-dione |
| | 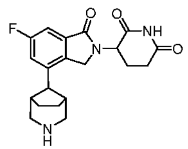 | 3-(4-((1R,5S,8r)-3-azabicyclo[3.2.1]octan-8-yl)-6-fluoro-1-oxoisoindolin-2-yl)piperidine-2,6-dione | 3-(4-((1R,5S,8r)-3-azabicyclo[3.2.1]octan-8-yl)-6-fluoro-1-oxoisoindolin-2-yl)piperidine-2,6-dione |

(continued)

| Compound No. | Structure of compound | Compound's name | Chinese translation of Compound's name |
|---|---|---|---|
| | 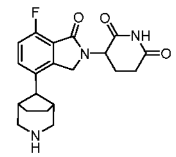 | 3-(4-((1R,5S,8r)-3-azabicyclo[3.2.1]octan-8-yl)-7-fluoro-1-oxoisoindolin-2-yl)piperidine-2,6-dione | 3-(4-((1R,5S,8r)-3-azabicyclo[3.2.1]octan-8-yl)-7-fluoro-1-oxoisoindolin-2- yl)piperidine-2,6-dione |
| | 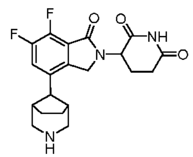 | 3-(4-((1R,5S,8r)-3-azabicyclo[3.2.1]octan-8-yl)-6,7-difluoro-1-oxoisoindolin-2-yl)piperidine-2,6-dione | 3-(4-((1R,5S,8r)-3-azabicyclo[3.2.1]octan-8-yl)-6,7-difluoro-1-oxoisoindolin-2-yl)piperidine-2,6-dione |
| | 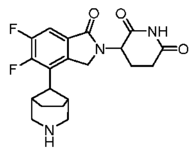 | 3-(4-((1R,5S,8r)-3-azabicyclo[3.2.1]octan-8-yl)-5,6-difluoro-1-oxoisoindolin-2-yl)piperidine-2,6-dione | 3-(4-((1R,5S,8r)-3-azabicyclo[3.2.1]octan-8-yl)-5,6-difluoro-1-oxoisoindolin-2-yl)piperidine-2,6-dione |
| | 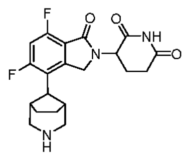 | 3-(4-((1R,5S,8r)-3-azabicyclo[3.2.1]octan-8-yl)-5,7-difluoro-1-oxoisoindolin-2-yl)piperidine-2,6-dione | 3-(4-((1R,5S,8r)-3-azabicyclo[3.2.1]octan-8-yl)-5,7-difluoro-1-oxoisoindolin-2-yl)piperidine-2,6-dione |
| | 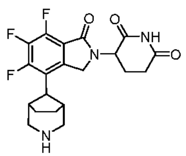 | 3-(4-((1R,5S,8r)-3-azabicyclo[3.2.1]octan-8-yl)-5,6,7-trifluoro-1-oxoisoindolin-2-yl)piperidine-2,6-dione | 3-(4-((1R,5S,8r)-3-azabicyclo[3.2.1]octan-8-yl)-5,6,7-trifluoro-1-oxoisoindolin-2-yl)piperidine-2,6-dione |
| | 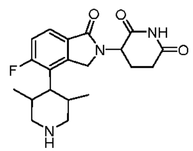 | 3-(4-(3,5-dimethylpiperidin-4-yl)-5-fluoro-1-oxoisoindolin-2-yl)piperidine-2,6-dione | 3-(4-(3,5-dimethylpiperidin-4-yl)-5-fluoro-1-oxoisoindolin-2-yl)piperidine-2,6-dione |

(continued)

| Compound No. | Structure of compound | Compound's name | Chinese translation of Compound's name |
|---|---|---|---|
| | 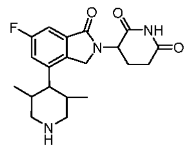 | 3-(4-(3,5-dimethylpiperidin-4-yl)-6-fluoro-1-oxoisoindolin-2-yl)piperidine-2,6-dione | 3-(4-(3,5-dimethylpiperidin-4-yl)-6-fluoro-1-oxoisoindolin-2-yl)piperidine-2,6-dione |
| | 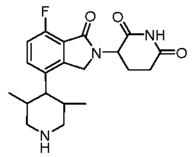 | 3-(4-(3,5-dimethylpiperidin-4-yl)-7-fluoro-1-oxoisoindolin-2-yl)piperidine-2,6-dione | 3-(4-(3,5-dimethylpiperidin-4-yl)-7-fluoro-1-oxoisoindolin-2-yl)piperidine-2,6-dione |
| | 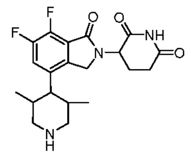 | 3-(4-(3,5-dimethylpiperidin-4-yl)-6,7-difluoro-1-oxoisoindolin-2- yl)piperidine-2,6-dione | 3-(4-(3,5-dimethylpiperidin-4-yl)-6,7-difluoro-1-oxoisoindolin-2-yl)piperidine-2,6-dione |
| | 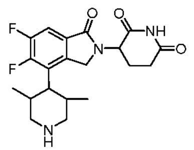 | 3-(4-(3,5-dimethylpiperidin-4-yl)-5,6-difluoro-1-oxoisoindolin-2-yl)piperidine-2,6-dione | 3-(4-(3,5-dimethylpiperidin-4-yl)-5,6-difluoro-1-oxoisoindolin-2-yl)piperidine-2,6-dione |
| | 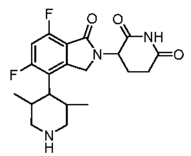 | 3-(4-(3,5-dimethylpiperidin-4-yl)-5,7-difluoro-1-oxoisoindolin-2-yl)piperidine-2,6-dione | 3-(4-(3,5-dimethylpiperidin-4-yl)-5,7-difluoro-1-oxoisoindolin-2-yl)piperidine-2,6-dione |
| | 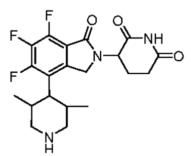 | 3-(4-(3,5-dimethylpiperidin-4-yl)-5,6,7-trifluoro-1-oxoisoindolin-2-yl)piperidine-2,6-dione | 3-(4-(3,5-dimethylpiperidin-4-yl)-5,6,7-trifluoro-1-oxoisoindolin-2-yl)piperidine-2,6-dione |

**Compounds of Formula (II)**

The present disclosure provides a compound of Formula (II) or salts (including pharmaceutically acceptable salts), stereoisomers (including enantiomers), solvates, isotopically enriched analogs, prodrugs, or polymorphs thereof:

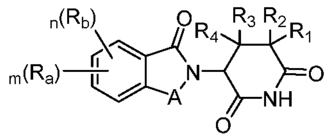

Formula (II)

wherein A, $R_1$, $R_2$, $R_3$, $R_4$, $(R_b)_n$ and $(R_a)_m$ are as defined in the compound of Formula (II) above.

In some embodiments, $R_1$, $R_2$, $R_3$ and $R_4$ of the compound of Formula (II) are the same or different and each independently represent hydrogen, deuterium, halogen (e.g., fluorine, chlorine, bromine, or iodine), optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-).

In some embodiments, $R_1$, $R_2$, $R_3$ and $R_4$ of the compound of Formula (II) are the same or different and each independently represent hydrogen or deuterium.

In some embodiments, $R_1$, $R_2$, $R_3$ and $R_4$ of the compound of Formula (II) represents hydrogen.

In some embodiments, A of the compound of Formula (II) represents CO.

In some embodiments, A of the compound of Formula (II) represents $CH_2$.

In some embodiments, A of the compound of Formula (II) represents $CD_2$.

In some embodiments, m of the compound of Formula (II) represents an integer of 1, n represents an integer of 0, 1, 2, or 3.

In some embodiments, m of the compound of Formula (II) represents an integer of 2, n represents an integer of 0, 1 or 2.

In some embodiments, in case that the compound of Formula (II) is partially or fully deuterated, when W represents optionally substituted nitrogen-containing monocyclic heterocyclyl, optionally substituted nitrogen-containing bridged heterocyclyl, optionally substituted cycloalkyl, optionally substituted heteroaryl or optionally substituted aryl, LIN represents a bond, optionally substituted methylene, or optionally substituted linear or branched $C_{2-30}$ alkylene, wherein one or more (e.g., 1-10, 1-8, or 1-6, such as 1, 2, 3, 4, 5 or 6) groups $R_e$ and/or one or more (e.g., 1-10, 1-8, or 1-6, such as 1, 2, 3, 4, 5 or 6) groups $R_f$ and/or any combination of one or more (e.g., 1-10, 1-8, or 1-6, such as 1, 2, 3, 4, 5 or 6) groups $R_e$ and $R_f$ are optionally inserted into the backbone carbon chain of the linear or branched $C_{2-30}$ alkylene group, wherein carbon-carbon bond between one or more (e.g., 1-10, 1-8, or 1-6, such as 1, 2, 3, 4, 5 or 6) pairs of adjacent carbon atoms in the backbone carbon chain is interrupted by the group $R_e$, $R_f$, or a combination of $R_e$ and $R_f$; wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl (e.g., $C_{1-4}$ alkyl, such as methyl, ethyl, propyl, isopropyl, butyl, sec-butyl or tert-butyl), and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), cyano or any combination thereof.

In some embodiments, in case that the compound of Formula (II) is partially deuterated (for example, carrying 1 to 20 deuterium atoms) or fully deuterated, when W represents optionally substituted nitrogen-containing monocyclic heterocyclyl, optionally substituted nitrogen-containing bridged heterocyclyl, optionally substituted cycloalkyl, optionally substituted heteroaryl or optionally substituted aryl, LIN represents a bond, optionally substituted methylene, or optionally substituted linear or branched $C_{2-30}$ alkylene, wherein one or more (e.g., 1-10, 1-8, or 1-6, such as 1, 2, 3, 4, 5 or 6) groups $R_e$ and/or one or more (e.g., 1-10, 1-8, or 1-6, such as 1, 2, 3, 4, 5 or 6) groups $R_f$ and/or any combination of one or more (e.g., 1-10, 1-8, or 1-6, such as 1, 2, 3, 4, 5 or 6) groups $R_e$ and $R_f$ are optionally inserted into the backbone carbon chain of the linear or branched $C_{2-30}$ alkylene group, wherein carbon-carbon bond between one or more (e.g., 1-10, 1-8, or 1-6, such as 1, 2, 3, 4, 5 or 6) pairs of adjacent carbon atoms in the backbone carbon chain is interrupted by the group $R_e$, $R_f$, or a combination of $R_e$ and $R_f$; wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl (e.g., $C_{1-4}$ alkyl, such as methyl, ethyl, propyl, isopropyl, butyl, sec-butyl or tert-butyl), and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), hydroxy, amino, mercapto, halogen, optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), cyano or any combination thereof;

wherein the methylene and the linear or branched $C_{2-30}$ alkylene are optionally substituted with one or more (e.g., 1-10, 1-8, or 1-6, such as 1, 2, 3, 4, 5 or 6) substituents selected from the group consisting of deuterium, hydroxy, amino, mercapto, nitro, halogen (e.g., fluorine, chlorine, bromine, or iodine), cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-) or any combination thereof.

In some embodiments, in case that the compound of Formula (II) is partially or fully deuterated, when W represents hydrogen, $C_{2-6}$ alkenyl, $C_{2-6}$ alkynyl, a leaving group, CHO, COOH, $NH_2$, hydroxy or mercapto, LIN represents optionally substituted methylene or optionally substituted linear or branched $C_{2-30}$ alkylene, wherein one or more (e.g., 1-10, 1-8, or 1-6, such as 1, 2, 3, 4, 5 or 6) groups $R_e$ and/or one or more (e.g., 1-10, 1-8, or 1-6, such as 1, 2, 3, 4, 5 or 6) groups $R_f$ and/or any combination of one or more (e.g., 1-10, 1-8, or 1-6, such as 1, 2, 3, 4, 5 or 6) groups $R_e$ and $R_f$ are optionally inserted into the backbone carbon chain of the linear or branched $C_{2-30}$ alkylene group, wherein carbon-carbon bond between one or more (e.g., 1-10, 1-8, or 1-6, such as 1, 2, 3, 4, 5 or 6) pairs of adjacent carbon atoms in the backbone carbon chain is interrupted by the group $R_e$, $R_f$, or a combination of $R_e$ and $R_f$; each $R_e$ are selected from the group consisting of O, $N(R_g)$, C(O), C(O)O, S(O), $S(O)_2$, $S(O)_2NH$, $NHS(O)_2$, OC(O), $C(O)N(R_g)$, $N(R_g)C(O)$, or $N(R_g)C(O)N(R_g)$, where each $R_g$ independently represent H or $C_{1-6}$ alkyl (e.g., $C_{1-4}$ alkyl, such as methyl, ethyl, propyl, isopropyl, butyl, sec-butyl or tert-butyl), and in case that two or more groups $R_e$ are inserted into the backbone carbon chain of the linear or branched $C_{2-30}$ alkylene group, the two or more groups $R_e$ are not directly connected to each other; and each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deu-

terated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), hydroxyl, amino, mercapto, halogen (e.g., fluorine, chlorine, bromine, or iodine), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), cyano or any combination thereof.

In some embodiments, in case that the compound of Formula (II) is partially deuterated (for example, carrying 1 to 20 deuterium atoms) or fully deuterated, when W represents hydrogen, $C_{2-6}$ alkenyl, $C_{2-6}$ alkynyl, a leaving group, CHO, COOH, $NH_2$, hydroxy or mercapto, LIN represents optionally substituted methylene or optionally substituted linear or branched $C_{2-30}$ alkylene, wherein one or more (e.g., 1-10, 1-8, or 1-6, such as 1, 2, 3, 4, 5 or 6) groups $R_e$ and/or one or more (e.g., 1-10, 1-8, or 1-6, such as 1, 2, 3, 4, 5 or 6) groups $R_f$ and/or any combination of one or more (e.g., 1-10, 1-8, or 1-6, such as 1, 2, 3, 4, 5 or 6) groups $R_e$ and $R_f$ are optionally inserted into the backbone carbon chain of the linear or branched $C_{2-30}$ alkylene group, wherein carbon-carbon bond between one or more (e.g., 1-10, 1-8, or 1-6, such as 1, 2, 3, 4, 5 or 6) pairs of adjacent carbon atoms in the backbone carbon chain is interrupted by the group $R_e$, $R_f$, or a combination of $R_e$ and $R_f$; each $R_e$ are selected from the group consisting of O, N($R_g$), C(O), C(O)O, S(O), $S(O)_2$, $S(O)_2NH$, $NHS(O)_2$, OC(O), C(O)N($R_g$), N($R_g$)C(O), or N($R_g$)C(O)N($R_g$), where each $R_g$ independently represent H or $C_{1-6}$ alkyl (e.g., $C_{1-4}$ alkyl, such as methyl, ethyl, propyl, isopropyl, butyl, sec-butyl or tert-butyl), and in case that two or more groups $R_e$ are inserted into the backbone carbon chain of the linear or branched $C_{2-30}$ alkylene group, the two or more groups $R_e$ are not directly connected to each other; and each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), hydroxyl, amino, mercapto, halogen (e.g., fluorine, chlorine, bromine, or iodine), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), cyano or any combination thereof;

wherein the methylene and the linear or branched $C_{2-30}$ alkylene are optionally substituted with one or more (e.g., 1-10, 1-8, or 1-6, such as 1, 2, 3, 4, 5 or 6) substituents selected from the group consisting of deuterium, hydroxy, amino, mercapto, nitro, halogen (e.g., fluorine, chlorine, bromine, or iodine), cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-) or any combination thereof.

In some embodiments, in case that the compound of Formula (II) is not deuterated, LIN represents optionally substituted methylene, and W represents optionally substituted nitrogen-containing monocyclic heterocyclyl or optionally substituted nitrogen-containing bridged heterocyclyl;

wherein the methylene is optionally substituted with one or more (e.g., 1 or 2) substituents selected from the group consisting of deuterium, hydroxy, amino, mercapto, nitro, halogen (e.g., fluorine, chlorine, bromine, or iodine), cyano,

optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-) or any combination thereof.

In some embodiments, in case that the compound of Formula (II) is not deuterated, when LIN represents optionally substituted methylene, $R_c$ represents piperazinylene, and W represents optionally substituted nitrogen-containing monocyclic heterocyclyl, the piperazinylene is substituted. In some embodiments, the piperazinylene of $R_c$ is substituted with 1-4 (e.g., 1-3 or 1-2, such as 1, 2, 3 or 4) substituents selected from the group consisting of deuterium, hydroxy, amino, mercapto, nitro, halogen (e.g., fluorine, chlorine, bromine, or iodine), cyano, CHO, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-) or any combination thereof. In some embodiments, the methylene of LIN is optionally substituted with one or more (e.g., 1 or 2) substituents selected from the group consisting of deuterium, hydroxy, amino, mercapto, nitro, halogen (e.g., fluorine, chlorine, bromine, or iodine), cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-) or any combination thereof. In some embodiments, the nitrogen-containing monocyclic heterocyclyl of W is optionally substituted with one or more (e.g., 1-10, 1-8, or 1-6, such as 1, 2, 3, 4, 5 or 6) substituents selected from the group consisting of deuterium, hydroxy, amino, mercapto, nitro, halogen (e.g., fluorine, chlorine, bromine, or iodine), cyano, CHO, COOH, $N_3$, methanesulfonyloxy, trifluoromethanesulfonyloxy, p-toluenesulfonyloxy, $C_{1-6}$ alkyl, deuterated $C_{1-6}$ alkyl, halogenated $C_{1-6}$ alkyl, optionally substituted $C_{3-8}$ cycloalkyl (e.g., substituted with one or more, e.g., 1-6, such as 1, 2, 3, 4, 5 or 6 substituents selected from the group consisting of deuterium, halogen, amino, hydroxy, mercapto, cyano, CHO, COOH, $N_3$, methanesulfonyloxy, trifluoromethanesulfonyloxy and p-toluenesulfonyloxy), optionally substituted 4- to 9-membered heterocyclyl (e.g., substituted with one or more, e.g., 1-6, such as 1, 2, 3, 4, 5 or 6 substituents selected from the group consisting of deuterium, halogen, amino, hydroxy, mercapto, cyano, CHO, COOH, $N_3$, methanesulfonyloxy, trifluoromethanesulfonyloxy and p-toluenesulfonyloxy), $C_{1-6}$ alkoxy (e.g., $C_{1-4}$ alkoxy, such as methoxy, $CD_3$-O-, ethoxy, propoxy, isopropoxy, butoxy, sec-butoxy or tert-butoxy), deuterated $C_{1-6}$ alkyl-O- (e.g., perdeuterated $C_{1-4}$ alkoxy, such as $CD_3$-O-, perdeuterated ethoxy, perdeuterated propoxy, perdeuterated isopropoxy, perdeuterated butoxy, perdeuterated sec-butoxy or perdeuterated tert-butoxy), $C_{1-6}$ alkyl-NH- (e.g., $C_{1-3}$ alkyl-NH-, such as $CH_3NH$-, $CH_3CH_2NH$- or $CH_3CH_2CH_2NH$-), deuterated $C_{1-6}$ alkyl-NH- (e.g., perdeuterated $C_{1-3}$ alkyl-NH-, such as $CD_3NH$-, $CD_3CD_2NH$- or $CD_3CD_2CD_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-6}$ alkyl-NHC(O)- (e.g., $C_{1-4}$ alkyl-NHC(O)-, such as $CH_3$-NHC(O)-, $CH_3CH_2$-NHC(O)-, and $CH_3CH_2CH_2$-NHC(O)-), deuterated $C_{1-6}$ alkyl-NHC(O)- (e.g., perdeuterated $C_{1-4}$ alkyl-NHC(O)-, such as $CD_3$-NHC(O)-, $CD_3CD_2$-NHC(O)-, and $CD_3CD_2CD_2$-NHC(O)-), $C_{1-6}$ alkyl-C(O)NH- (e.g., $C_{1-4}$ alkyl-C(O)NH-, such as $CH_3$-C(O)NH-, $CH_3CH_2$-C(O)NH-, and $CH_3CH_2CH_2$-C(O)NH-), deuterated $C_{1-6}$ alkyl-C(O)NH- (e.g., perdeu-

terated $C_{1-4}$ alkyl-C(O)NH-, such as $CD_3$-C(O)NH-, $CD_3CD_2$-C(O)NH-, and $CD_3CD_2CD_2$-C(O)NH-) or any combination thereof.

In some embodiments, $R_c$ of the compound of Formula (II) represents the following groups:

monocyclic $C_{3-10}$ heterocyclylene group containing one nitrogen atom and optionally a heteroatom selected from nitrogen, oxygen, and sulfur, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene or diazacyclooctylene; or

$C_{5-20}$ bridged heterocyclylene group containing one nitrogen atom and optionally a heteroatom selected from nitrogen, oxygen, and sulfur, e.g., azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene or diazabicyclo[2.2.2]octanylene, such as the following groups:

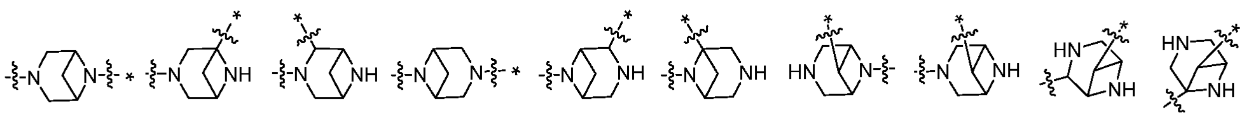

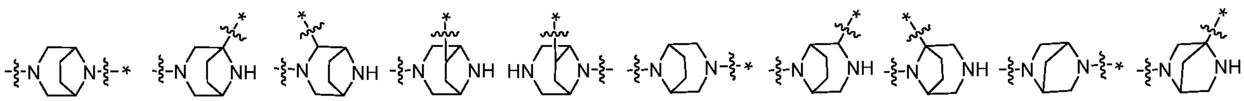

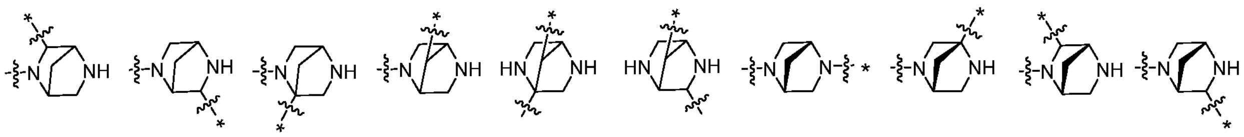

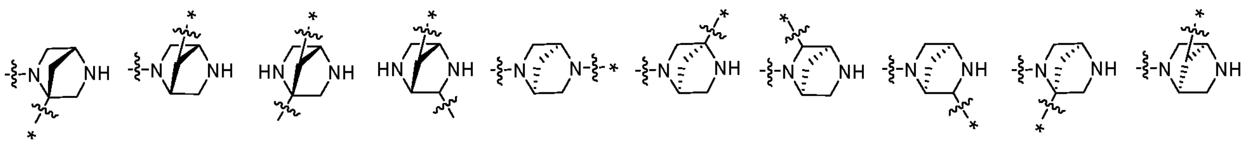

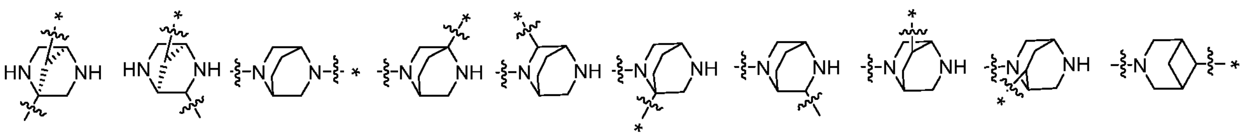

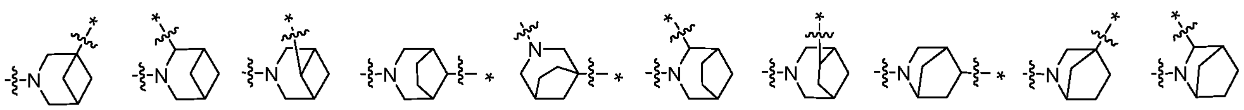

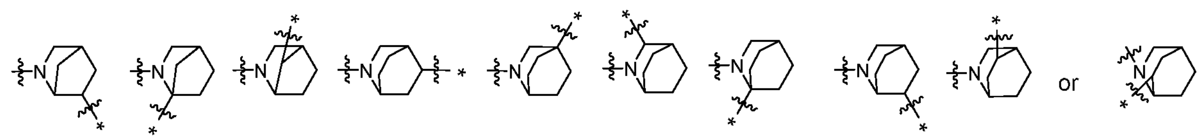
or

wherein symbol * indicates the point of attachment to $U_1$,

wherein the monocyclic $C_{3-10}$ heterocyclylene and the $C_{5-20}$ bridged heterocyclylene group are each independently optionally substituted with one or more (e.g., 1-10, 1-8 or 1-6, such as 1, 2, 3, 4, 5 or 6) substituents selected from the group consisting of deuterium, hydroxy, amino, mercapto, nitro, halogen (e.g., fluorine, chlorine, bromine, or iodine), cyano, CHO, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as

$F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-) or any combination thereof.

In some embodiments, $R_c$ of the compound of Formula (II) represents the following groups:

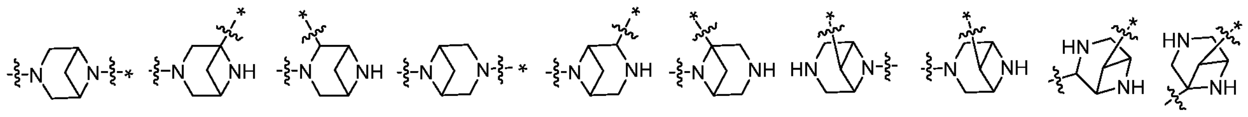

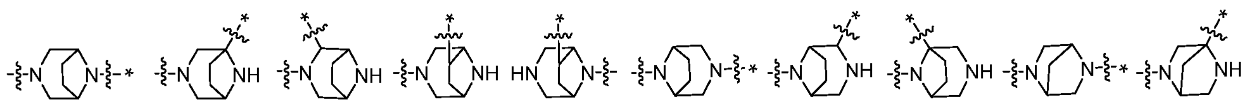

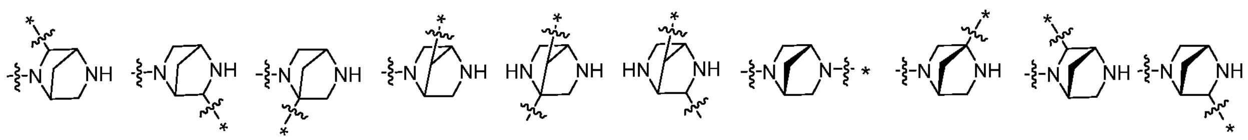

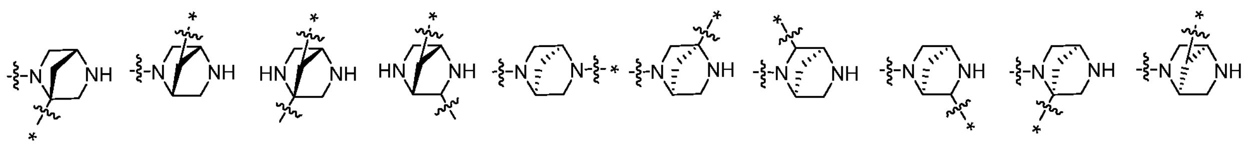

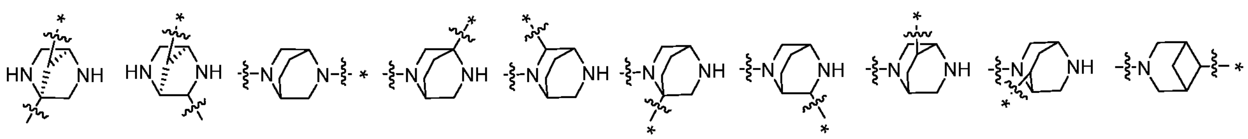

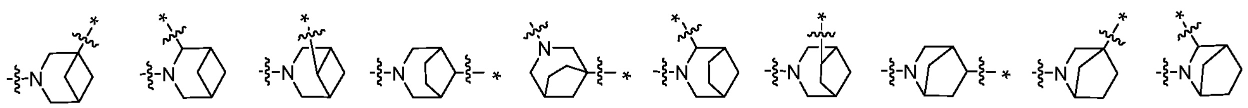

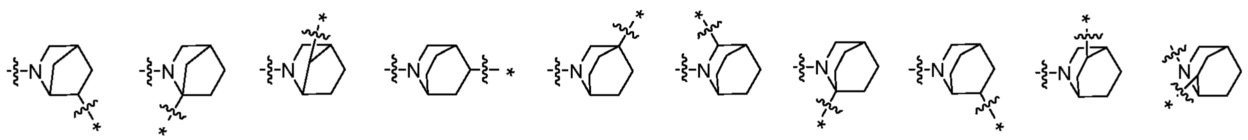

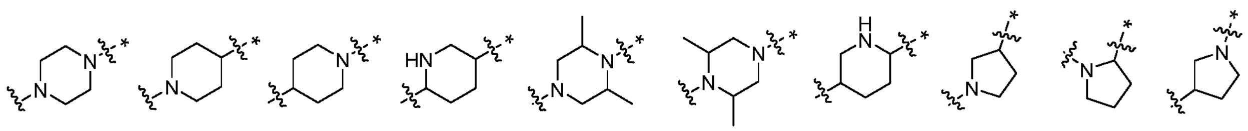

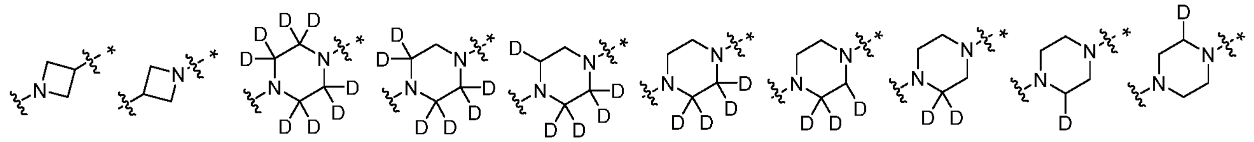

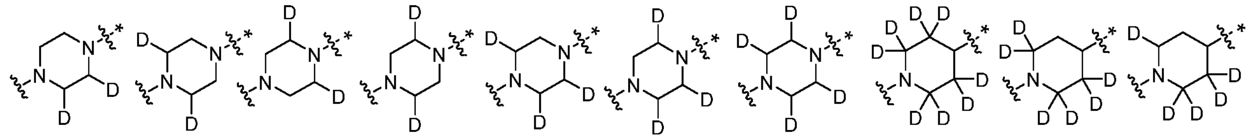

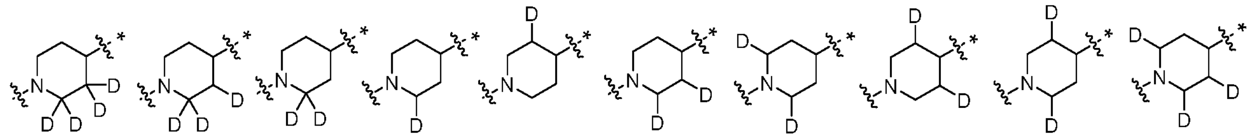

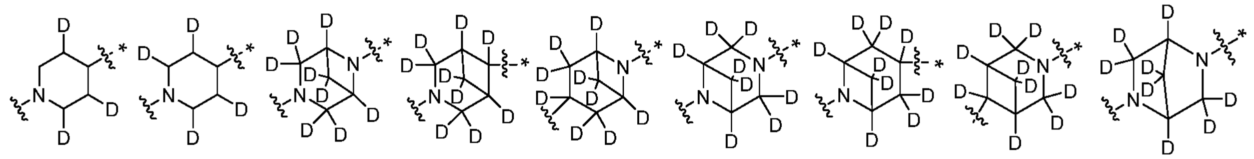

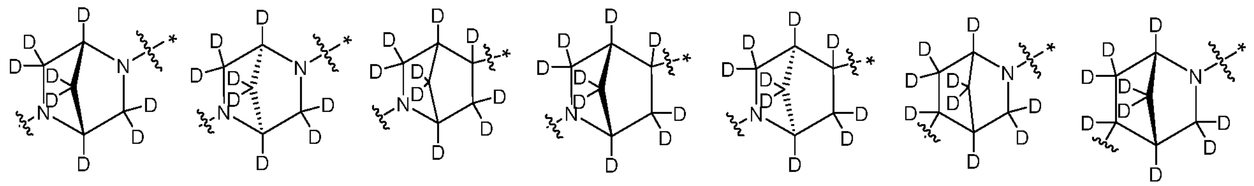

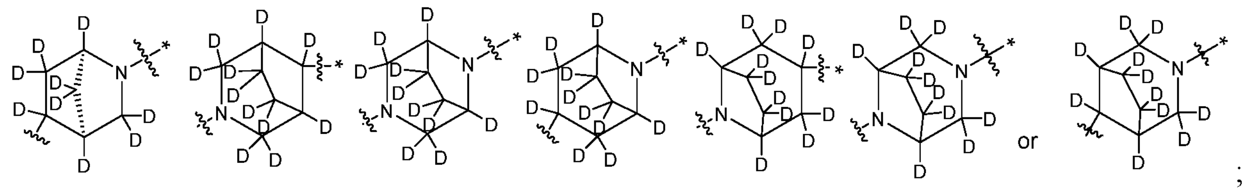

;

wherein symbol * indicates the point of attachment to $U_1$.

In some embodiments, W of the compound of Formula (II) represents the following groups:

monocyclic $C_{3-10}$ heterocyclyl group containing one nitrogen atom and optionally a heteroatom selected from nitrogen, oxygen, and sulfur, e.g., piperidinyl, piperazinyl, morpholinyl, azetidinyl, pyrrolidinyl, imidazolidyl, pyrazolidyl, oxazolidinyl, thiazolidinyl, thiomorpholinyl, azepanyl, diazacycloheptanyl, azacyclooctyl or diazacyclooctyl; or $C_{5-20}$ bridged heterocyclyl group containing one nitrogen atom and optionally a heteroatom selected from nitrogen, oxygen, and sulfur, e.g., azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl or diazabicyclo[2.2.2]octanyl, such as the following groups:

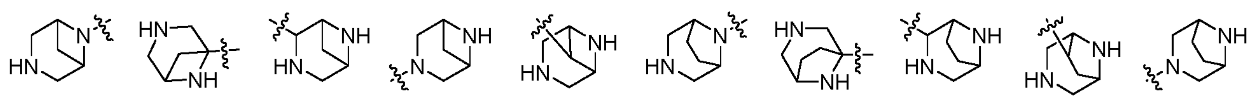

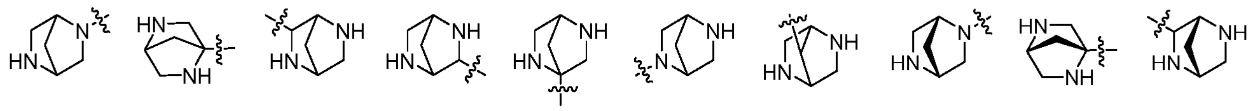

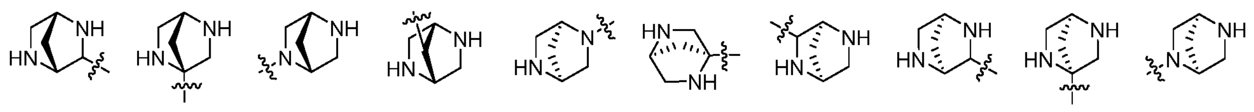

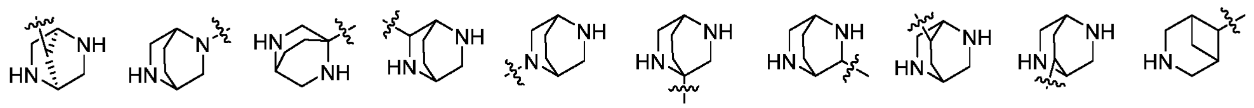

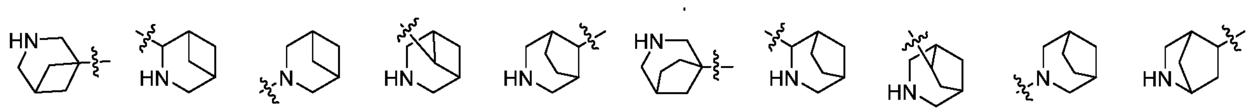

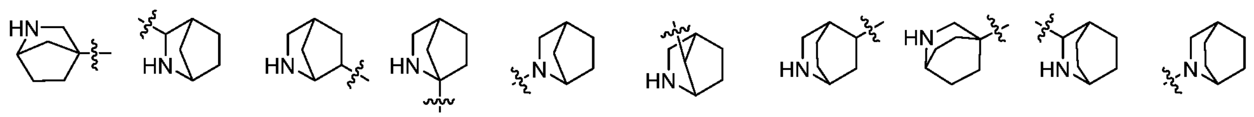

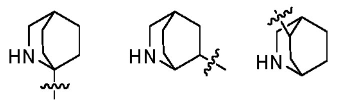 or 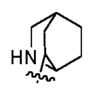 ;

or saturated or partially unsaturated monocyclic or bicyclic or polycyclic $C_{3-20}$ cycloalkyl, e.g., cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, $C_{7-11}$ spiro-cycloalkyl, adamantanyl, noradamantanyl, bornyl, bicyclo[2.2.1]heptyl or bicyclo[2.2.1]heptenyl; or

heteroaryl, e.g., 5- to 20-membered monocyclic or bicyclic $C_{5-20}$ aromatic ring group containing one or more (e.g., 1-4, such as 1, 2, 3 or 4) heteroatoms independently selected from the group consisting of oxygen, nitrogen, and sulfur, e.g., furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl; or

$C_{5-14}$ aryl optionally containing one or more (e.g., 1-3, such as 1, 2 or 3) fused rings, e.g., phenyl, naphthyl or fluorenyl; and

wherein the monocyclic $C_{3-10}$ heterocyclyl, $C_{5-20}$ bridged heterocyclyl, $C_{3-20}$ cycloalkyl, heteroaryl and $C_{5-14}$ aryl are each independently optionally substituted with one or more (e.g., 1-10, 1-8, or 1-6, such as 1, 2, 3, 4, 5 or 6) substituents selected from the group consisting of deuterium, hydroxy, amino, mercapto, nitro, halogen (e.g., fluorine, chlorine, bromine, or iodine), cyano, CHO, COOH, $N_3$, methanesulfonyloxy, trifluoromethanesulfonyloxy, p-toluenesulfonyloxy, $C_{1-6}$ alkyl, deuterated $C_{1-6}$ alkyl, halogenated $C_{1-6}$ alkyl, optionally substituted $C_{3-8}$ cycloalkyl (e.g., substituted with one or more, e.g., 1-6, such as 1, 2, 3, 4, 5 or 6 substituents selected from the group consisting of deuterium, halogen, amino, hydroxy, mercapto, cyano, CHO, COOH, $N_3$, methanesulfonyloxy, trifluoromethanesulfonyloxy and p-toluenesulfonyloxy), optionally substituted 4- to 9-membered heterocyclyl (e.g., substituted with one or more, e.g., 1-6, such as 1, 2, 3, 4, 5 or 6 substituents selected from the group consisting of deuterium, halogen, amino, hydroxy, mercapto, cyano, CHO, COOH, $N_3$, methanesulfonyloxy, trifluoromethanesulfonyloxy and p-toluenesulfonyloxy), $C_{1-6}$ alkoxy (e.g., $C_{1-4}$ alkoxy, such as methoxy, $CD_3$-O-, ethoxy, propoxy, isopropoxy, butoxy, sec-butoxy or tert-butoxy), deuterated $C_{1-6}$ alkyl-O- (e.g., perdeuterated $C_{1-4}$ alkoxy, such as $CD_3$-O-, perdeuterated ethoxy, perdeuterated propoxy, perdeuterated isopropoxy, perdeuterated butoxy, perdeuterated sec-butoxy or per-

deuterated tert-butoxy), $C_{1-6}$ alkyl-NH- (e.g., $C_{1-3}$ alkyl-NH-, such as $CH_3NH$-, $CH_3CH_2NH$- or $CH_3CH_2CH_2NH$-), deuterated $C_{1-6}$ alkyl-NH- (e.g., perdeuterated $C_{1-3}$ alkyl-NH-, such as $CD_3NH$-, $CD_3CD_2NH$- or $CD_3CD_2CD_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-6}$ alkyl-NHC(O)- (e.g., $C_{1-4}$ alkyl-NHC(O)-, such as $CH_3$-NHC(O)-, $CH_3CH_2$-NHC(O)-, and $CH_3CH_2CH_2$-NHC(O)-), deuterated $C_{1-6}$ alkyl-NHC(O)- (e.g., perdeuterated $C_{1-4}$ alkyl-NHC(O)-, such as $CD_3$-NHC(O)-, $CD_3CD_2$-NHC(O)-, and $CD_3CD_2CD_2$-NHC(O)-), $C_{1-6}$ alkyl-C(O)NH- (e.g., $C_{1-4}$ alkyl-C(O)NH-, such as $CH_3$-C(O)NH-, $CH_3CH_2$-C(O)NH-, and $CH_3CH_2CH_2$-C(O)NH-), deuterated $C_{1-6}$ alkyl-C(O)NH- (e.g., perdeuterated $C_{1-4}$ alkyl-C(O)NH-, such as $CD_3$-C(O)NH-, $CD_3CD_2$-C(O)NH-, and $CD_3CD_2CD_2$-C(O)NH-) or any combination thereof.

In some embodiments, LIN of the compound of Formula (II) represents the structure of the following formula:

#-$C_{1-30}$ alkylene-;
#-$(C(R^{a1})(R^{a2}))_{n1}$-$(R_e(C(R^{a3})(R^{a4}))_{n2})_{m1}$-;
#-$(C(R^{a1})(R^{a2}))_{n1}$-$(R_e(C(R^{a3})(R^{a4}))_{n2})_{m1}$-$(R_e(C(R^{a5})(R^{a6}))_{n3})_{m2}$-;
#-$(C(R^{a1})(R^{a2}))_{n1}$-$(R_e(C(R^{a3})(R^{a4}))_{n2})_{m1}$-$(R_e(C(R^{a5})(R^{a6}))_{n3})_{m2}$-$(R_e(C(R^{a7})(R^{a8}))_{n4})_{m3}$-;
#-$(C(R^{a1})(R^{a2}))_{n1}$-$(R_f(C(R^{a3})(R^{a4}))_{n2})_{m1}$-;
#-$(C(R^{a1})(R^{a2}))_{n1}$-$(R_f(C(R^{a3})(R^{a4}))_{n2})_{m1}$-$(R_f(C(R^{a5})(R^{a6}))_{n3})_{m2}$-;
#-$(C(R^{a1})(R^{a2}))_{n1}$-$(R_f(C(R^{a3})(R^{a4}))_{n2})_{m1}$-$(R_f(C(R^{a7})(R^{a8}))_{n4})_{m3}$-;
#-$(C(R^{a1})(R^{a2}))_{n1}$-$(R_e$-$R_f$-$(C(R^{a3})(R^{a4}))_{n2})_{m1}$-;
#-$(C(R^{a1})(R^{a2}))_{n1}$-$(R_e(C(R^{a3})(R^{a4}))_{n2})_{m1}$-$(R_f(C(R^{a5})(R^{a6}))_{n3})_{m2}$-;
#-$(C(R^{a1})(R^{a2}))_{n1}$-$(R_f$-$R_e$-$(C(R^{a3})(R^{a4}))_{n2})_{m1}$-; or
#-$(C(R^{a1})(R^{a2}))_{n1}$-$(R_f(C(R^{a3})(R^{a4}))_{n2})_{m1}$-$(R_e(C(R^{a5})(R^{a6}))_{n3})_{m2}$-;

wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl;

wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), cyano, or any combination thereof;

a hydrogen atom of one or more (e.g., 1-30) $CH_2$ groups of the $C_{1-30}$ alkylene group is optionally replaced with a substituent selected from the group consisting of deuterium, hydroxy, amino, mercapto, nitro, halogen (e.g., fluorine, chlorine, bromine, or iodine), cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), or any combination thereof;

$R^{a1}$, $R^{a2}$, $R^{a3}$, $R^{a4}$, $R^{a5}$, $R^{a6}$, $R^{a7}$, and $R^{a8}$ each independently represent H, deuterium, hydroxy, amino, mercapto, nitro, halogen (e.g., fluorine, chlorine, bromine, or iodine), cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), or any combination thereof;

symbol # indicates the point of attachment to $U_2$; and

n1, n2, n3, n4, m1, m2, and m3 each independently represent an integer of 1, 2, 3, 4, 5, 6, 7, 8, 9, 10, 11, 12, 13, 14, or 15.

In some embodiments, LIN of the compound of Formula (II) represents the structure of the following formula:

#-$(C(R^{a1})(R^{a2}))_{n1}$-$(O(C(R^{a3})(R^{a4}))_{n2})_{m1}$-;
#-$(C(R^{a1})(Ra^{2}))_{n1}$-$(O(C(R^{a5})(R^{a4}))_{n2})_{m1}$-$(O(C(R^{a5})(R^{a6}))_{n3})_{m2}$-;
#-$(C(R^{a1})(R^{a2}))_{n1}$-$(O(C(R^{a3})(R^{a4}))_{n2})_{m1}$-$(O(C(R^{a5})(R^{a6}))_{n3})_{m2}$-$(O(C(R^{a7})(R^{a8}))_{n4})_{m3}$-;
#-$(C(R^{a1})(R^{a2}))_{n1}$-$(N(R_g)(C(R^{a3})(R^{a4}))_{n2})_{m1}$-;
#-$(C(R^{a1})(R^{a2}))_{n1}$-$(N(R_g)(C(R^{a3})(R^{a4}))_{n2})_{m1}$-$(N(R_g)(C(R^{a5})(R^{a6}))_{n3})_{m2}$-;
#-$(C(R^{a1})(R^{a2}))_{n1}$-$(N(R_g)(C(R^{a3})(R^{a4}))_{n2})_{m1}$-$(N(R_g)(C(R^{a5})(R^{a6}))_{n3})_{m2}$-$(N(R_g)(C(R^{a7})(R^{a8}))_{n4})_{m3}$-;
#-$(C(R^{al})(R^{a2}))_{ni}$-$(C(O)N(R_g)$-$(C(R^{a3})(R^{a4}))_{n2})_{mi}$-;
#-$(C(R^{a1})(R^{a2}))_{n1}$-$(C(O)N(R_g)$-$(C(R^{a3})(R^{a4}))_{n2})_{m1}$-$(C(O)N(R_g)$-$(C(R^{a5})(R^{a6}))_{n3})_{m2}$-;
#-$(C(R^{a1})(R^{a2}))_{n1}$-$(C(O)N(R_g)$-$(C(R^{a3})(R^{a4}))_{n2})_{m1}$-$(C(O)N(R_g)$-$(C(R^{a5})(R^{a6}))_{n3})_{m2}$-$(C(O)N(R_g)$-$(C(R^{a7})(R^{a8}))_{n4})_{m3}$-;
#-$(C(R^{a1})(Ra^{2}))_{n1}$-$(C(O)N(R_g)$-$(C(R^{a3})(R^{a4}))_{n2})_{m1}$-$(O$-$(C(R^{a5})(R^{a6}))_{n3})_{m2}$-;
#-$(C(R^{a1})(R^{a2}))_{n1}$-$C(O)N(R_g)$-$(C(R^{a3})(R^{a4}))_{n2}$-$(O(C(R^{a5})(R^{a6}))_{n3})_{m1}$-;
#-$(C(R^{a1})(R^{a2}))_{n1}$-$(N(R_g)C(O)$-$(C(R^{a3})(R^{a4}))_{n2})_{m1}$-;
#-$(C(R^{a1})(R^{a2}))_{n1}$-$(N(R_g)C(O)$-$(C(R^{a3})(R^{a4}))_{n2})_{m1}$-$(O$-$(C(R^{a5})(R^{a6}))_{n3})_{m2}$-;
#-$(C(R^{a1})(R^{a2}))_{n1}$-$(N(R_g)C(O)$-$(C(R^{a3})(R^{a4}))_{n2})_{m1}$-$(O$-$(C(R^{a5})(R^{a6}))_{n3})_{m2}$-$(O$-$(C(R_g)(R^{a8}))_{n4})_{m3}$-;
#-$(C(R^{a1})(R^{a2}))_{n1}$-$N(R_g)C(O)$-$(C(R^{a3})(R^{a4}))_{n2}$-$(O(C(R^{a5})(R^{a6}))_{n3})_{m1}$-;
#-$(C(R^{a1})(R^{a2}))_{n1}$-$N(R_g)C(O)N(R_g)$-$(C(R^{a3})(R^{a4}))_{n2}$-;
#-$(C(R^{a1})(R^{a2}))_{n1}$-$C(O)$-$(C(R^{a3})(R^{a4}))_{n2}$-;
#-$(C(R^{a1})(R^{a2}))_{n1}$-(arylene-$(C(R^{a3})(R^{a4}))_{n2})_{m1}$-;
#-$(C(R^{a1})(R^{a2}))_{n1}$-(arylene-$(C(R^{a3})(R^{a4}))_{n2})_{m1}$-arylene-$(C(R^{a5})(R^{a6}))_{n3}$-;
#-$(C(R^{a1})(R^{a2}))_{n1}$-(heterocyclylene-$(C(R^{a3})(R^{a4}))_{n2})_{m1}$-;
#-$(C(R^{a1})(R^{a2}))_{n1}$-(heterocyclylene-$(C(R^{a3})(R^{a4}))_{n2})_{m1}$-(heterocyclylene-$(C(R^{a5})(R^{a6}))_{n3})_{m2}$-;
#-$(C(R^{a1})(R^{a2}))_{n1}$-(heteroarylene-$(C(R^{a3})(R^{a4}))_{n2})_{m1}$-;
#-$(C(R^{a1})(R^{a2}))_{n1}$-(heteroarylene-$(C(R^{a3})(R^{a4}))_{n2})_{m1}$-(heteroarylene-$(C(R^{a5})(R^{a6}))_{n3})_{m2}$-;
#-$(C(R^{a1})(R^{a2}))_{n1}$-(cycloalkylene-$(C(R^{a3})(R^{a4}))_{n2})_{m1}$-; or
#-$(C(R^{a1})(R^{a2}))_{n1}$-(cycloalkylene-$(C(R^{a3})(R^{a4}))_{n2})_{m1}$-(cycloalkylene-$(C(R^{a5})(R^{a6}))_{n3})_{m2}$-;

wherein each $R_g$ independently represent H or $C_{1-6}$ alkyl (e.g., $C_{1-4}$ alkyl, such as methyl, ethyl, propyl, isopropyl, butyl, sec-butyl or tert-butyl);

the cycloalkylene, arylene, heterocyclylene, and heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), cyano, or any combination thereof;

$R^{a1}$, $R^{a2}$, $R^{a3}$, $R^{a4}$, $R^{a5}$, $R^{a6}$, $R^{a7}$ and $R^{a8}$ each independently represent H, deuterium, hydroxy, amino, mercapto,

nitro, halogen (e.g., fluorine, chlorine, bromine, or iodine), cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), or any combination thereof; symbol # indicates the point of attachment to $U_2$; and

n1, n2, n3, n4, m1, m2, and m3 each independently represent an integer of 1, 2, 3, 4, 5, 6, 7, 8, 9, 10, 11, 12, 13, 14, or 15.

In some embodiments, LIN of the compound of Formula (II) represents the following groups:

#-$CH_2$-, #-$(CH_2)_2$-, #-$(CH_2)_3$-, #-$(CH_2)_4$-, #-$(CH_2)_5$-, #-$(CH_2)_6$-, #-$(CH_2)_7$-, #-$(CH_2)_8$-, #-$(CH_2)_9$-, #-$(CH_2)_{10}$-, #-$(CH_2)_{11}$-, #-$(CH_2)_{12}$-, #-$(CH_2)_{13}$-, #-$(CH_2)_{14}$-, #-$(CH_2)_{15}$-, #-$(CH_2)_{16}$-, #-$(CH_2)_{17}$-, #-$(CH_2)_{18}$-, #-$(CH_2)_{19}$-, #-$(CH_2)_{20}$-, #-$(CH_2)_{21}$-, #-$(CH_2)_{22}$-, #-$(CH_2)_{25}$-, or #-$(CH_2)_{30}$-;#-$CH_2$-O-$CH_2$-, #-$CH_2$-O-$(CH_2)_2$-, #-$(CH_2)_1$-O-$(CH_2)_3$-, #-$(CH_2)_1$-O-$(CH_2)_4$-, #-$(CH_2)_1$-O-$(CH_2)_5$-, #-$(CH_2)_1$-O-$(CH_2)_6$-, #-$(CH_2)_1$-O-$(CH_2)_7$-, #-$(CH_2)_1$-O-$(CH_2)_8$-, #-$(CH_2)_1$-O-$(CH_2)_9$-, #-$(CH_2)_1$-O-$(CH_2)_{10}$-, #-$(CH_2)_2$-O-$(CH_2)_1$-, #-$(CH_2)_2$-O-$(CH_2)_2$-, #-$(CH_2)_2$-O-$(CH_2)_3$-, #-$(CH_2)_2$-O-$(CH_2)_4$-, #-$(CH_2)_2$-O-$(CH_2)_5$-, #-$(CH_2)_2$-O-$(CH_2)_6$-, #-$(CH_2)_2$-O-$(CH_2)_7$-, #-$(CH_2)_2$-O-$(CH_2)_8$-, #-$(CH_2)_2$-O-$(CH_2)_9$-, #-$(CH_2)_2$-O-$(CH_2)_{10}$-, #-$(CH_2)_2$-O-$(CH_2)_{11}$-, #-$(CH_2)_2$-O-$(CH_2)_{12}$-, #-$(CH_2)_3$-O-$(CH_2)_1$-, #-$(CH_2)_3$-O-$(CH_2)_2$-, #-$(CH_2)_3$-O-$(CH_2)_3$-, #-$(CH_2)_3$-O-$(CH_2)_4$-, #-$(CH_2)_3$-O-$(CH_2)_5$-, #-$(CH_2)_3$-O-$(CH_2)_6$-, #-$(CH_2)_3$-O-$(CH_2)_7$-, #-$(CH_2)_4$-O-$(CH_2)_1$-, #-$(CH_2)_4$-O-$(CH_2)_2$-, #-$(CH_2)_4$-O-$(CH_2)_3$-, #-$(CH_2)_4$-O-$(CH_2)_4$-, #-$(CH_2)_4$-O-$(CH_2)_5$-, #-$(CH_2)_4$-O-$(CH_2)_6$-, #-$(CH_2)_5$-O-$(CH_2)_1$-, #-$(CH_2)_5$-O-$(CH_2)_2$-, #-$(CH_2)_5$-O-$(CH_2)_3$-, #-$(CH_2)_5$-O-$(CH_2)_4$-, #-$(CH_2)_5$-O-$(CH_2)_5$-, #-$(CH_2)_6$-O-$(CH_2)_1$-, #-$(CH_2)_6$-O-$(CH_2)_2$-, #-$(CH_2)_6$-O-$(CH_2)_3$-, #-$(CH_2)_6$-O-$(CH_2)_4$-, #-$(CH_2)_7$-O-$(CH_2)_1$-, #-$(CH_2)_7$-O-$(CH_2)_2$-, #-$(CH_2)_7$-O-$(CH_2)_3$-, #-$(CH_2)_8$-O-$(CH_2)_1$-, #-$(CH_2)_8$-O-$(CH_2)_2$-, #-$CH(CH_3)$-O-$(CH_2)_1$-, #-$CH(CH_3)$-O-$(CH_2)_2$-, #-$CH(CH_3)$-O-$(CH_2)_3$-, #-$CH(CH_3)$-O-$(CH_2)_4$-, #-$CH(CH_3)$-O-$(CH_2)_5$-, #-$CH(CH_3)$-O-$(CH_2)_6$-, #-$CH(CH_3)$-O-$(CH_2)_7$-, #-$CH(CH_3)$-O-$(CH_2)_8$-, #-$CH(CH_3)$-O-$(CH_2)_9$-, #-$CH(CH_3)$-O-$(CH_2)_{10}$-, #-$CH_2$-$(O(CH_2)_2)_2$-, #-$CH_2$-$(O(CH_2)_2)_3$-, #-$CH_2$-$(O(CH_2)_2)_4$-, #-$CH_2$-$(O(CH_2)_2)_5$-, #-$CH_2$-$(O(CH_2)_2)_6$-, #-$CH_2$-$(O(CH_2)_2)_7$-, #-$CH_2$-$(O(CH_2)_2)_8$-, #-$CH_2$-$(O(CH_2)_2)_9$-, #-$CH_2$-$(O(CH_2)_2)_{10}$-, #-$CH_2$-$(O(CH_2)_2)_1$-$OCH_2$-, #-$CH_2$-$(O(CH_2)_2)_2$-$OCH_2$-, #-$CH_2$-$(O(CH_2)_2)_3$-$OCH_2$-, #-$CH_2$-$(O(CH_2)_2)_4$-$OCH_2$-, #-$CH_2$-$(O(CH_2)_2)_5$-$OCH_2$-, #-$CH_2$-$(O(CH_2)_2)_6$-$OCH_2$-, #-$CH_2$-$(O(CH_2)_2)_7$-$OCH_2$-, #-$CH_2$-$(O(CH_2)_2)_8$-$OCH_2$-, #-$CH_2$-$(O(CH_2)_2)_9$-$OCH_2$-, #-$CH_2$-$(O(CH_2)_2)_{10}$-$OCH_2$-, #-$(CH_2)_2$-$(O(CH_2)_2)_2$-, #-$(CH_2)_2$-$(O(CH_2)_2)_3$-, #-$(CH_2)_2$-$(O(CH_2)_2)_4$-, #-$(CH_2)_2$-$(O(CH_2)_2)_5$-, #-$(CH_2)_2$-$(O(CH_2)_2)_6$-, #-$(CH_2)_2$-$(O(CH_2)_2)_7$-, #-$(CH_2)_2$-$(O(CH_2)_2)_8$-, #-$(CH_2)_2$-$(O(CH_2)_2)_9$-, #-$(CH_2)_2$-$(O(CH_2)_2)_{10}$-, #-$(CH_2)_3$-$(O(CH_2)_2)_2$-, #-$(CH_2)_3$-$(O(CH_2)_2)_3$-, #-$(CH_2)_3$-$(O(CH_2)_2)_4$-, #-$(CH_2)_3$-$(O(CH_2)_2)_5$-, #-$(CH_2)_3$-$(O(CH_2)_2)_6$-, #-$(CH_2)_3$-$(O(CH_2)_2)_7$-, #-$(CH_2)_3$-$(O(CH_2)_2)_8$-, #-$(CH_2)_3$-$(O(CH_2)_2)_9$-, #-$(CH_2)_3$-$(O(CH_2)_2)_{10}$-, #-$(CH_2)_4$-$(O(CH_2)_2)_2$-, #-$(CH_2)_4$-$(O(CH_2)_2)_3$-, #-$(CH_2)_4$-$(O(CH_2)_2)_4$-, #-$(CH_2)_4$-$(O(CH_2)_2)_5$-, #-$(CH_2)_4$-$(O(CH_2)_2)_6$-, #-$(CH_2)_4$-$(O(CH_2)_2)_7$-, #-$(CH_2)_4$-$(O(CH_2)_2)_8$-, #-$(CH_2)_4$-$(O(CH_2)_2)_9$-, #-$(CH_2)_4$-$(O(CH_2)_2)_{10}$-, #-$CH_2$-$(O(CH_2)_3)_2$-, 9-$CH_2$-$(O(CH_2)_3)_3$-, #-$CH_2$-$(O(CH_2)_3)_4$-, #-$CH_2$-$(O(CH_2)_3)_5$-, #-$CH_2$-$(O(CH_2)_3)_6$-, #-$CH_2$-$(O(CH_2)_3)_7$-, #-$CH_2$-$(O(CH_2)_3)_8$-, #-$CH_2$-$(O(CH_2)_3)_9$-, #-$CH_2$-$(O(CH_2)_3)_{10}$-, #-$(CH_2)_2$-$(O(CH_2)_3)_2$-, #-$(CH_2)_2$-$(O(CH_2)_3)_3$-, #-$(CH_2)_2$-$(O(CH_2)_3)_4$-, #-$(CH_2)_2$-$(O(CH_2)_3)_5$-, #-$(CH_2)_2$-$(O(CH_2)_3)_6$-, #-$(CH_2)_2$-$(O(CH_2)_3)_7$-, #-$(CH_2)_2$-$(O(CH_2)_3)_8$-, #-$(CH_2)_2$-$(O(CH_2)_3)_9$-, #-$(CH_2)_2$-$(O(CH_2)_3)_{10}$-, #-$(CH_2)_3$-$(O(CH_2)_3)_2$-, #-$(CH_2)_3$-$(O(CH_2)_3)_3$-, #-$(CH_2)_3$-$(O(CH_2)_3)_4$-, #-$(CH_2)_3$-$(O(CH_2)_3)_5$-, #-$(CH_2)_3$-$(O(CH_2)_3)_6$-, #-$(CH_2)_3$-$(O(CH_2)_3)_7$-, #-$(CH_2)_3$-$(O(CH_2)_3)_8$-, #-$(CH_2)_3$-$(O(CH_2)_3)_9$-, #-$(CH_2)_3$-$(O(CH_2)_3)_{10}$-, #-$CH_2$-O-$(CH_2)_2$-O-$(CH_2)_3$-, #-$CH_2$-$(O(CH_2)_2)_2$-$(O(CH_2)_3)_2$-, #-$CH_2$-$(O(CH_2)_2)_3$-$(O(CH_2)_3)_3$-, #-$CH_2$-$(O(CH_2)_2)_4$-$(O(CH_2)_3)_4$-, #-$CH_2$-$(O(CH_2)_2)_5$-$(O(CH_2)_3)_5$-, #-$CH_2$-$(O(CH_2)_2)_6$-$(O(CH_2)_3)_6$-, #-$(CH_2)_2$-O-$(CH_2)_2$-O-$(CH_2)_3$-, #-$(CH_2)_2$-$(O(CH_2)_2)_2$-$(O(CH_2)_3)_2$-, #-$(CH_2)_2$-$(O(CH_2)_2)_3$-$(O(CH_2)_3)_3$-, #-$(CH_2)_2$-$(O(CH_2)_2)_4$-$(O(CH_2)_3)_4$-, #-$(CH_2)_2$-$(O(CH_2)_2)_5$-$(O(CH_2)_3)_5$-, #-$(CH_2)_2$-$(O(CH_2)_2)_6$-$(O(CH_2)_3)_6$-, #-$(CH_2)_3$-O-$(CH_2)_2$-O-$(CH_2)_3$-, #-$(CH_2)_3$-$(O(CH_2)_2)_2$-$(O(CH_2)_3)_2$-, #-$(CH_2)_3$-$(O(CH_2)_2)_3$-$(O(CH_2)_3)_3$-, #-$(CH_2)_3$-$(O(CH_2)_2)_4$-$(O(CH_2)_3)_4$-, #-$(CH_2)_3$-$(O(CH_2)_2)_5$-$(O(CH_2)_3)_5$-, #-$(CH_2)_3$-$(O(CH_2)_2)_6$-$(O(CH_2)_3)_6$-, #-$CH_2$-O-$(CH_2)_3$-O-$(CH_2)_2$-, #-$CH_2$-$(O(CH_2)_3)_2$-$(O(CH_2)_2)_2$-, #-$CH_2$-$(O(CH_2)_3)_3$-$(O(CH_2)_2)_3$-, #-$CH_2$-$(O(CH_2)_3)_4$-$(O(CH_2)_2)_4$-, #-$CH_2$-$(O(CH_2)_3)_5$-$(O(CH_2)_2)_5$-, #-$CH_2$-$(O(CH_2)_3)_6$-$(O(CH_2)_2)_6$-, #-$(CH_2)_2$-O-$(CH_2)_3$-O-$(CH_2)_2$-, #-$(CH_2)_2$-$(O(CH_2)_3)_2$-$(O(CH_2)_2)_2$-, #-$(CH_2)_2$-$(O(CH_2)_3)_3$-$(O(CH_2)_2)_3$-, #-$(CH_2)_2$-$(O(CH_2)_3)_4$-$(O(CH_2)_2)_4$-, #-$(CH_2)_2$-$(O(CH_2)_3)_5$-$(O(CH_2)_2)_5$-, #-$(CH_2)_2$-$(O(CH_2)_3)_6$-$(O(CH_2)_2)_6$-, #-$(CH_2)_3$-O-$(CH_2)_3$-O-$(CH_2)_2$-, #-$(CH_2)_3$-$(O(CH_2)_3)_2$-$(O(CH_2)_2)_2$-,

#-(CH$_2$)$_3$-(O(CH$_2$)$_3$)$_3$-(O(CH$_2$)$_2$)$_3$-, #-(CH$_2$)$_3$-(O(CH$_2$)$_3$)$_4$-(O(CH$_2$)$_2$)$_4$-, #-(CH$_2$)$_3$-(O(CH$_2$)$_3$)$_5$-(O(CH$_2$)$_2$)$_5$-, #-(CH$_2$)$_3$-(O(CH$_2$)$_3$)$_6$-(O(CH$_2$)$_2$)$_6$-, #-CH$_2$-O-(CH$_2$)$_2$-O-CH$_2$-, #-(CH$_2$)$_2$-O-(CH$_2$)$_2$-O-CH$_2$-, #-(CH$_2$)$_2$-(O(CH$_2$)$_2$)$_2$-O-(CH$_2$)$_3$-, #-(CH$_2$)$_2$-(O(CH$_2$)$_2$)$_3$-O-(CH$_2$)$_3$-, #-(CH$_2$)$_2$-(O(CH$_2$)$_2$)$_4$-O-(CH$_2$)$_3$-, #-(CH$_2$)$_5$-(O(CH$_2$)$_2$)$_2$-O-(CH$_2$)$_5$-, #-(CH$_2$)$_5$-(O(CH$_2$)$_2$)$_2$-O-(CH$_2$)$_6$-, #-(CH$_2$)$_1$-N(R$_g$)-(CH$_2$)$_1$-, #-(CH$_2$)$_1$-N(R$_g$)-(CH$_2$)$_2$-, #-(CH$_2$)$_1$-N(R$_g$)-(CH$_2$)$_3$-, #-(CH$_2$)$_1$-N(R$_g$)-(CH$_2$)$_4$-, #-(CH$_2$)$_1$-N(R$_g$)-(CH$_2$)$_5$-, #-(CH$_2$)$_1$-N(R$_g$)-(CH$_2$)$_6$-, #-(CH$_2$)$_1$-N(R$_g$)-(CH$_2$)$_7$-, #-(CH$_2$)$_1$-N(R$_g$)-(CH$_2$)$_8$-, #-(CH$_2$)$_1$-N(R$_g$)-(CH$_2$)$_9$-, #-(CH$_2$)$_1$-N(R$_g$)-(CH$_2$)$_{10}$-, #-(CH$_2$)$_2$-N(R$_g$)-(CH$_2$)$_1$-, #-(CH$_2$)$_2$-N(R$_g$)-(CH$_2$)$_2$-, #-(CH$_2$)$_2$-N(R$_g$)-(CH$_2$)$_3$-, #-(CH$_2$)$_2$-N(R$_g$)-(CH$_2$)$_4$-, #-(CH$_2$)$_2$-N(R$_g$)-(CH$_2$)$_5$-, #-(CH$_2$)$_2$-N(R$_g$)-(CH$_2$)$_6$-, #-(CH$_2$)$_2$-N(R$_g$)-(CH$_2$)$_7$-, #-(CH$_2$)$_2$-N(R$_g$)-(CH$_2$)$_8$-, #-(CH$_2$)$_2$-N(R$_g$)-(CH$_2$)$_9$-, #-(CH$_2$)$_2$-N(R$_g$)-(CH$_2$)$_{1O}$-, #-(CH$_2$)$_2$-N(R$_g$)-(CH$_2$) $_{11}$-, #-(CH$_2$)$_2$-N(R$_g$)-(CH$_2$)$_{12}$-, #-(CH$_2$)$_3$-N(R$_g$)-(CH$_2$)$_1$-, #-(CH$_2$)$_3$-N(R$_g$)-(CH$_2$)$_2$-, #-(CH$_2$)$_3$-N(R$_g$)-(CH$_2$)$_3$-, #-(CH$_2$)$_4$-N(R$_g$)-(CH$_2$)$_1$-, #-(CH$_2$)$_4$-N(R$_g$)-(CH$_2$)$_2$-, #-(CH$_2$)$_4$-N(R$_g$)-(CH$_2$)$_3$-, #-(CH$_2$)$_4$-N(R$_g$)-(CH$_2$)$_4$-, #-(CH$_2$)$_5$-N(R$_g$)-(CH$_2$)i-, #-(CH$_2$)$_5$-N(R$_g$)-(CH$_2$)$_2$-, #-(CH$_2$)$_5$-N(R$_g$)-(CH$_2$)$_3$-, #-(CH$_2$)$_5$-N(R$_g$)-(CH$_2$)$_4$-, #-(CH$_2$)$_5$-N(R$_g$)-(CH$_2$)$_5$-, #-(CH$_2$)$_6$-N(R$_g$)-(CH$_2$)$_i$-, #-(CH$_2$)$_6$-N(R$_g$)-(CH$_2$)$_2$-, #-(CH$_2$)$_6$-N(R$_g$)-(CH$_2$)$_3$-, #-(CH$_2$)$_7$-N(R$_g$)-(CH$_2$)i-, #-(CH$_2$)$_7$-N(R$_g$)-(CH$_2$)$_2$-, #-(CH$_2$)$_7$-N(R$_g$)-(CH$_2$)$_3$-, #-(CH$_2$)$_8$-N(R$_g$)-(CH$_2$)$_1$-, #-(CH$_2$)$_8$-N(R$_g$)-(CH$_2$)$_2$-, #-(CH$_2$)$_s$-N(R$_g$)-(CH$_2$)$_3$-, #-CH(CH$_3$)-N(R$_g$)-(CH$_2$)$_1$-, #-CH(CH$_3$)-N(R$_g$)-(CH$_2$)$_2$-, #-CH(CH$_3$)-N(R$_g$)-(CH$_2$)$_3$-, #-CH(CH$_3$)-N(R$_g$)-(CH$_2$)$_4$-, #-CH(CH$_3$)-N(R$_g$)-(CH$_2$)$_5$-, #-CH(CH$_3$)-N(R$_g$)-(CH$_2$)$_6$-, #-CH(CH$_3$)-N(R$_g$)-(CH$_2$)$_7$-, #-CH(CH$_3$)-N(R$_g$)-(CH$_2$)$_8$-, #-CH(CH$_3$)-N(R$_g$)-(CH$_2$)$_9$-, #-CH(CH$_3$)-N(R$_g$)-(CH$_2$)$_{10}$-, #-CH$_2$C(O)NHCH$_2$-, #-(CH$_2$)$_2$C(O)NH(CH$_2$)$_2$-, #-(CH$_2$)$_2$C(O)NH(CH$_2$)$_3$-, #-(CH$_2$)$_2$C(O)NH(CH$_2$)$_4$-, #-(CH$_2$)$_2$C(O)NH(CH$_2$)$_5$-, #-(CH$_2$)$_3$C(O)NH(CH$_2$)$_3$-, #-(CH$_2$)$_3$C(O)NH(CH$_2$)$_4$-, #-(CH$_2$)$_4$C(O)NH(CH$_2$)$_4$-, #-(CH$_2$)$_5$C(O)NH(CH$_2$)$_5$-, #-(CH$_2$)$_6$C(O)NH(CH$_2$)$_7$-, #-(CH$_2$)$_6$C(O)NH(CH$_2$)$_6$-, #-(CH$_2$)$_7$C(O)NH(CH$_2$)$_7$-, #-(CH$_2$)$_8$C(O)NH(CH$_2$)$_8$, U-(CH$_2$)$_9$C(O)NH(CH$_2$)$_9$-, #-(CH$_2$)$_{10}$C(O)NH(CH$_2$)$_{10}$-, #-(CH$_2$)$_2$C(O)NH(CH$_2$)$_2$-O-(CH$_2$)$_2$-, #-CH$_2$NHC(O)CH$_2$-, #-(CH$_2$)$_2$NHC(O)(CH$_2$)$_2$-, #-(CH$_2$)$_2$NHC(O)(CH$_2$)$_3$-, #-(CH$_2$)$_2$NHC(O)(CH$_2$)$_4$-, #-(CH$_2$)$_2$NHC(O)(CH$_2$)$_5$-, #-(CH$_2$)$_3$NHC(O)(CH$_2$)$_3$-, #-(CH$_2$)$_3$NHC(O)(CH$_2$)$_4$-, #-(CH$_2$)$_4$NHC(O)(CH$_2$)$_4$-, #-(CH$_2$)$_5$NHC(O)(CH$_2$)$_5$-, #-(CH$_2$)$_6$NHC(O)(CH$_2$)$_7$-, #-(CH$_2$)$_6$NHC(O)(CH$_2$)$_6$-, #-(CH$_2$)$_7$NHC(O)(CH$_2$)$_7$-, #-(CH$_2$)$_8$NHC(O)(CH$_2$)$_8$, #-(CH$_2$)$_9$NHC(O)(CH$_2$)$_9$-, #-(CH$_2$)$_{10}$NHC(O)(CH$_2$)$_{10}$-, #-(CH$_2$)$_4$NHC(O)(CH$_2$)$_8$-, #-(CH$_2$)$_2$NHC(O)(CH$_2$)$_2$-O-(CH$_2$)$_2$-, #-(CH$_2$)$_4$NHC(O)CH$_2$-, #-CH$_2$-piperidinylene-CH$_2$-, #-CH$_2$-piperidinylene-(CH$_2$)$_2$-, #-CH$_2$-piperidinylene-(CH$_2$)$_3$-, #-CH$_2$-piperidinylene-(CH$_2$)$_4$-, #-CH$_2$-piperidinylene-(CH$_2$)$_5$-, #-CH$_2$-piperidinylene-(CH$_2$)$_6$-, #-CH$_2$-piperidinylene-(CH$_2$)$_7$-, #-CH$_2$-piperidinylene-(CH$_2$)$_8$-, #-(CH$_2$)$_2$-piperidinylene-(CH$_2$)$_1$-, #-(CH$_2$)$_2$-piperidinylene-(CH$_2$)$_2$-, #-(CH$_2$)$_2$-piperidinylene-(CH$_2$)$_3$-, #-(CH$_2$)$_2$-piperidinylene-(CH$_2$)$_4$-, #-(CH$_2$)$_2$-piperidinylene-(CH$_2$)$_5$-, #-(CH$_2$)$_2$-piperidinylene-(CH$_2$)$_6$-, #-(CH$_2$)$_2$-piperidinylene-(CH$_2$)$_7$-, #-(CH$_2$)$_2$-piperidinylene-(CH$_2$)$_8$-, #-(CH$_2$)$_3$-piperidinylene-CH$_2$-, #-(CH$_2$)$_3$-piperidinylene-(CH$_2$)$_2$-, #-(CH$_2$)$_3$-piperidinylene-(CH$_2$)$_3$-, #-(CH$_2$)$_3$-piperidinylene-(CH$_2$)$_4$-, #-(CH$_2$)$_3$-piperidinylene-(CH$_2$)$_5$-, #-(CH$_2$)$_3$-piperidinylene-(CH$_2$)$_6$-, #-(CH$_2$)$_3$-piperidinylene-(CH$_2$)$_7$-, #-(CH$_2$)$_3$-piperidinylene-(CH$_2$)$_8$-, #-(CH$_2$)$_4$-piperidinylene-CH$_2$-, #-(CH$_2$)$_4$-piperidinylene-(CH$_2$)$_2$-, #-(CH$_2$)$_4$-piperidinylene-(CH$_2$)$_3$-, #-(CH$_2$)$_4$-piperidinylene-(CH$_2$)$_4$-, #-(CH$_2$)$_4$-piperidinylene-(CH$_2$)$_5$-, #-(CH$_2$)$_4$-piperidinylene-(CH$_2$)$_6$-, #-(CH$_2$)$_4$-piperidinylene-(CH$_2$)$_7$-, #-(CH$_2$)$_4$-piperidinylene-(CH$_2$)$_8$-, #-(CH$_2$)$_5$-piperidinylene-(CH$_2$)$_1$-, #-(CH$_2$)$_5$-piperidinylene-(CH$_2$)$_2$-, #-(CH$_2$)$_5$-piperidinylene-(CH$_2$)$_3$-, #-(CH$_2$)$_5$-piperidinylene-(CH$_2$)$_4$-, #-(CH$_2$)$_5$-piperidinylene-(CH$_2$)$_5$-, #-(CH$_2$)$_5$-piperidinylene-(CH$_2$)$_6$-, #-(CH$_2$)$_5$-piperidinylene-(CH$_2$)$_7$-, #-(CH$_2$)$_5$-piperidinylene-(CH$_2$)$_5$-, #-(CH$_2$)$_6$-piperidinylene-(CH$_2$)$_1$-, #-(CH$_2$)$_6$-piperidinylene-(CH$_2$)$_2$-, #-(CH$_2$)$_6$-piperidinylene-(CH$_2$)$_3$-, #-(CH$_2$)$_6$-piperidinylene-(CH$_2$)$_4$-, #-(CH$_2$)$_6$-piperidinylene-(CH$_2$)$_5$-, #-(CH$_2$)$_6$-piperidinylene-(CH$_2$)$_6$-, #-(CH$_2$)$_6$-piperidinylene-(CH$_2$)$_7$-, #-(CH$_2$)$_6$-piperidinylene-(CH$_2$)$_5$-, #-(CH$_2$)$_7$-piperidinylene-(CH$_2$)$_1$-, #-(CH$_2$)$_7$-piperidinylene-(CH$_2$)$_2$-, #-(CH$_2$)$_7$-piperidinylene-(CH$_2$)$_3$-, #-(CH$_2$)$_7$-piperidinylene-(CH$_2$)$_4$-, #-(CH$_2$)$_7$-piperidinylene-(CH$_2$)$_8$-, #-(CH$_2$)$_8$-piperidinylene-CH$_2$-, #-(CH$_2$)$_8$-piperidinylene-(CH$_2$)$_2$-, #-(CH$_2$)$_8$-piperidinylene-(CH$_2$)$_3$-, #-(CH$_2$)$_8$-piperidinylene-(CH$_2$)$_4$-, #-(CH$_2$)$_5$-piperidinylene-(CH$_2$)$_5$-, #-(CH$_2$)$_8$-piperidinylene-(CH$_2$)$_6$-, #-(CH$_2$)$_8$-piperidinylene-(CH$_2$)$_7$-, #-(CH$_2$)$_5$-piperidinylene-(CH$_2$)$_5$-, #-CH$_2$-N(R$_g$)-CH$_2$-piperidinylene-CH$_2$-, #-CH$_2$-N(R$_g$)-CH$_2$-piperidinylene-(CH$_2$)$_2$-, #-CH$_2$-N(R$_g$)-CH$_2$-piperidinylene-(CH$_2$)$_3$-, #-CH$_2$-N(R$_g$)-CH$_2$-piperidinylene-(CH$_2$)$_4$-, #-CH$_2$-N(R$_g$)-CH$_2$-piperidinylene-(CH$_2$)$_5$-, #-CH$_2$-N(R$_g$)-CH$_2$-piperidinylene-(CH$_2$)$_6$-, #-CH$_2$-N(R$_g$)-CH$_2$-piperidinylene-(CH$_2$)$_7$-, #-CH$_2$-N(R$_g$)-CH$_2$-piperidinylene-(CH$_2$)$_8$-, #-CH$_2$-N(R$_g$)-(CH$_2$)$_2$-piperidinylene-CH$_2$-, #-CH$_2$-N(R$_g$)-(CH$_2$)$_2$-piperidinylene-(CH$_2$)$_2$-, #-CH$_2$-N(R$_g$)-(CH$_2$)$_2$-piperidinylene-(CH$_2$)$_3$-, #-CH$_2$-N(R$_g$)-(CH$_2$)$_2$-piperidinylene-(CH$_2$)$_4$-, #-CH$_2$-N(R$_g$)-(CH$_2$)$_2$-piperidinylene-(CH$_2$)$_5$-, #-CH$_2$-N(R$_g$)-(CH$_2$)$_2$-piperidinylene-(CH$_2$)$_6$-, #-CH$_2$-N(R$_g$)-(CH$_2$)$_2$-piperidinylene-(CH$_2$)$_7$-, #-CH$_2$-N(R$_g$)-(CH$_2$)$_2$-piperidinylene-(CH$_2$)$_8$-, #-(CH$_2$)$_2$-N(R$_g$)-CH$_2$-piperidinylene-CH$_2$-, #-(CH$_2$)$_2$-N(R$_g$)-CH$_2$-piperidinylene-(CH$_2$)$_2$-, #-(CH$_2$)$_2$-N(R$_g$)-CH$_2$-piperidinylene-(CH$_2$)$_3$-, #-(CH$_2$)$_2$-N(R$_g$)-CH$_2$-piperidinylene-(CH$_2$)$_4$-, #-(CH$_2$)$_2$-N(R$_g$)-CH$_2$-piperidinylene-(CH$_2$)$_5$-, #-(CH$_2$)$_2$-N(R$_g$)-CH$_2$-piperidinylene-(CH$_2$)$_6$-, #-(CH$_2$)$_2$-N(R$_g$)-CH$_2$-piperidinylene-(CH$_2$)$_7$-, #-(CH$_2$)$_2$-N(R$_g$)-CH$_2$-piperidinylene-(CH$_2$)$_8$-, #-(CH$_2$)$_3$-N(R$_g$)-CH$_2$-piperidinylene-CH$_2$-, #-(CH$_2$)$_3$-N(R$_g$)-CH$_2$-piperidinylene-(CH$_2$)$_2$-, #-(CH$_2$)$_3$-N(R$_g$)-CH$_2$-piperidinylene-(CH$_2$)$_3$-, #-(CH$_2$)$_3$-N(R$_g$)-CH$_2$-piperidinylene-(CH$_2$)$_8$-, #-(CH$_2$)$_4$-N(R$_g$)-CH$_2$-piperidinylene-CH$_2$-, #-(CH$_2$)$_4$-N(R$_g$)-CH$_2$-piperidinylene-(CH$_2$)$_2$-, #-(CH$_2$)$_4$-N(R$_g$)-CH$_2$-piperidinylene-(CH$_2$)$_3$-, #-(CH$_2$)$_4$-N(R$_g$)-CH$_2$-piperidinylene-(CH$_2$)$_s$-, #-(CH$_2$)$_5$-N(R$_g$)-CH$_2$-piperidinylene-(CH$_2$)$_3$-, #-(CH$_2$)$_5$-N(R$_g$)-CH$_2$-piperidinylene-(CH$_2$)$_8$-, #-(CH$_2$)$_6$-N(R$_g$)-CH$_2$-piperidinylene-(CH$_2$)$_3$-, #-(CH$_2$)$_6$-N(R$_g$)-CH$_2$-piperidinylene-(CH$_2$)$_8$-, #-(CH$_2$)$_7$-N(R$_g$)-CH$_2$-piperidinylene-(CH$_2$)$_3$-,

#-$(CH_2)_7$-N($R_g$)-$CH_2$-piperidinylene-$(CH_2)_8$-, #-$(CH_2)_8$-N($R_g$)-$CH_2$-piperidinylene-$CH_2$-, #-$(CH_2)_8$-N($R_g$)-$CH_2$-pipendinylene-$(CH_2)_2$-, #-$(CH_2)_8$-N($R_g$)-$CH_2$-piperidinylene-$(CH_2)_3$-, #-$(CH_2)_8$-N($R_g$)-$CH_2$-piperidmylene-$(CH_2)_4$-, #-$(CH_2)_8$-N($R_g$)-$CH_2$-piperidinylene-$(CH_2)_5$-, #-$(CH_2)_8$-N($R_g$)-$CH_2$-piperidmylene-$(CH_2)_6$-, #-$(CH_2)_8$-N($R_g$)-$CH_2$-piperidinylene-$(CH_2)_7$-, #-$(CH_2)_8$-N($R_g$)-$CH_2$-piperidinylene-$(CH_2)_8$-, #-$CH_2$-piperazinylene-$CH_2$-, #-$CH_2$-piperazinylene-$(CH_2)_2$-, #-$CH_2$-piperazinylene-$(CH_2)_3$-, #-$CH_2$-piperazinylene-$(CH_2)_4$-, #-$CH_2$-piperazinylene-$(CH_2)_5$-, #-$CH_2$-piperazinylene-$(CH_2)_6$-, #-$CH_2$-piperazinylene-$(CH_2)_7$-, #-$CH_2$-piperazinylene-$(CH_2)_8$-, #-$(CH_2)_2$-piperazinylene-$(CH_2)_1$-, #-$(CH_2)_2$-piperazinylene-$(CH_2)_2$-, #-$(CH_2)_2$-piperazinylene-$(CH_2)_3$-, #-$(CH_2)_2$-piperazinylene-$(CH_2)_4$-, #-$(CH_2)_2$-piperazinylene-$(CH_2)_5$-, #-$(CH_2)_2$-piperazinylene-$(CH_2)_6$-, #-$(CH_2)_2$-piperazinylene-$(CH_2)_7$-, #-$(CH_2)_2$-piperazinylene-$(CH_2)_8$-, #-$(CH_2)_3$-piperazinylene-$CH_2$-, #-$(CH_2)_3$-piperazinylene-$(CH_2)_2$-, #-$(CH_2)_3$-piperazinylene-$(CH_2)_3$-, #-$(CH_2)_3$-piperazinylene-$(CH_2)_4$-, #-$(CH_2)_3$-piperazinylene-$(CH_2)_5$-, #-$(CH_2)_3$-piperazinylene-$(CH_2)_6$-, #-$(CH_2)_3$-piperazinylene-$(CH_2)_7$-, #-$(CH_2)_3$-piperazinylene-$(CH_2)_8$-, #-$(CH_2)_4$-piperazinylene-$CH_2$-, #-$(CH_2)_4$-piperazinylene-$(CH_2)_2$-, #-$(CH_2)_4$-piperazinylene-$(CH_2)_3$-, #-$(CH_2)_4$-piperazinylene-$(CH_2)_4$-, #-$(CH_2)_4$-piperazinylene-$(CH_2)_5$-, #-$(CH_2)_4$-piperazinylene-$(CH_2)_6$-, #-$(CH_2)_4$-piperazinylene-$(CH_2)_7$-, #-$(CH_2)_4$-piperazinylene-$(CH_2)_8$-, #-$(CH_2)_5$-piperazinylene-$(CH_2)_1$-, #-$(CH_2)_5$-piperazinylene-$(CH_2)_2$-, #-$(CH_2)_5$-piperazinylene-$(CH_2)_3$-, #-$(CH_2)_5$-piperazinylene-$(CH_2)_4$-, #-$(CH_2)_5$-piperazinylene-$(CH_2)_5$-, #-$(CH_2)_5$-piperazinylene-$(CH_2)_6$-, #-$(CH_2)_5$-piperazinylene-$(CH_2)_7$-, #-$(CH_2)_5$-piperazinylene-$(CH_2)_8$-, #-$(CH_2)_6$-piperazinylene-$(CH_2)_1$-, #-$(CH_2)_6$-piperazinylene-$(CH_2)_2$-, #-$(CH_2)_6$-piperazinylene-$(CH_2)_3$-, #-$(CH_2)_6$-piperazinylene-$(CH_2)_4$-, #-$(CH_2)_6$-piperazinylene-$(CH_2)_5$-, #-$(CH_2)_6$-piperazinylene-$(CH_2)_6$-, #-$(CH_2)_6$-piperazinylene-$(CH_2)_7$-, #-$(CH_2)_6$-piperazinylene-$(CH_2)_8$-, #-$(CH_2)_7$-piperazinylene-$(CH_2)_1$-, #-$(CH_2)_7$-piperazinylene-$(CH_2)_2$-, #-$(CH_2)_7$-piperazinylene-$(CH_2)_3$-, #-$(CH_2)_7$-piperazinylene-$(CH_2)_4$-, #-$(CH_2)_7$-piperazinylene-$(CH_2)_8$-, #-$(CH_2)_8$-piperazinylene-$CH_2$-, #-$(CH_2)_8$-piperazinylene-$(CH_2)_2$-, #-$(CH_2)_8$-piperazinylene-$(CH_2)_3$-, #-$(CH_2)_8$-piperazinylene-$(CH_2)_4$-, #-$(CH_2)_8$-piperazinylene-$(CH_2)_5$-, #-$(CH_2)_8$-piperazinylene-$(CH_2)_6$-, #-$(CH_2)_8$-piperazinylene-$(CH_2)_7$-, or #-$(CH_2)_8$-piperazinylene-$(CH_2)_8$-;

wherein the piperidinylene and the piperazinylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), cyano, or any combination thereof;

each $R_g$ independently represent H or $C_{1-6}$ alkyl (e.g., $C_{1-4}$ alkyl, such as methyl, ethyl, propyl, isopropyl, butyl, sec-butyl or tert-butyl);

symbol # indicates the point of attachment to $U_2$; and

n1, n2, n3, n4, m1, m2, and m3 each independently represent an integer of 1, 2, 3, 4, 5, 6, 7, 8, 9, 10, 11, 12, 13, 14, or 15.

In some embodiments, $R_a$ of the compound of Formula (II) represents the following groups:

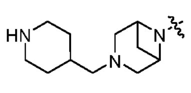 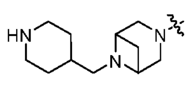 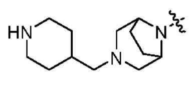 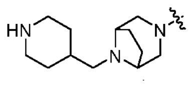 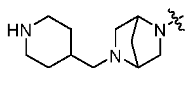 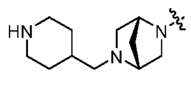

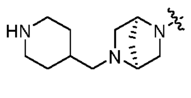 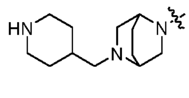 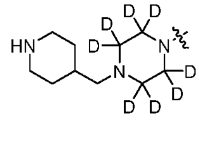 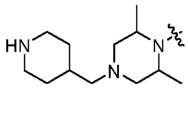 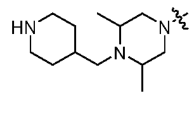 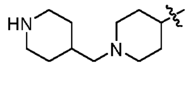

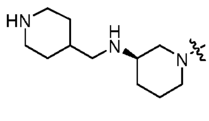 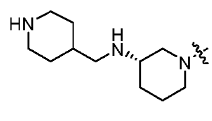 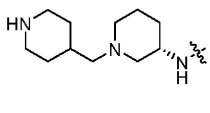 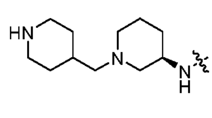 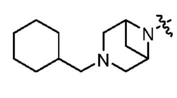 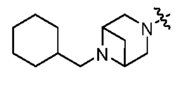

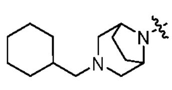 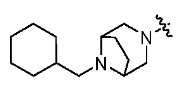 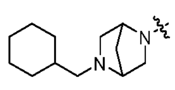 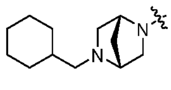 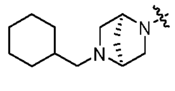 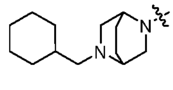

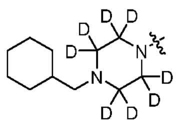 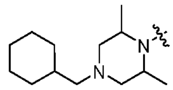 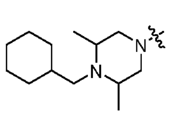 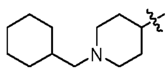 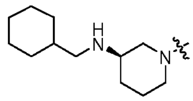 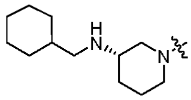

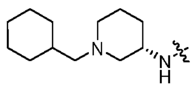 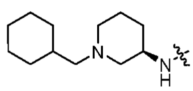 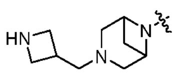 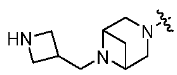 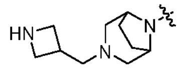 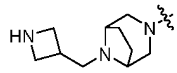

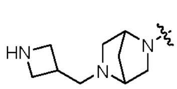 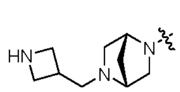 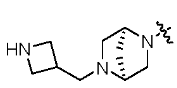 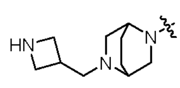 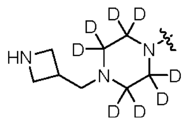 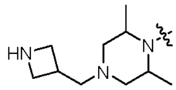

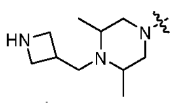 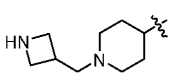 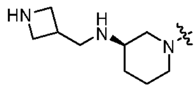 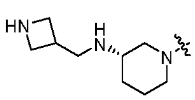 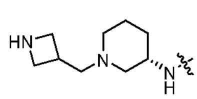 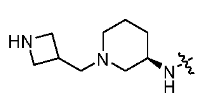

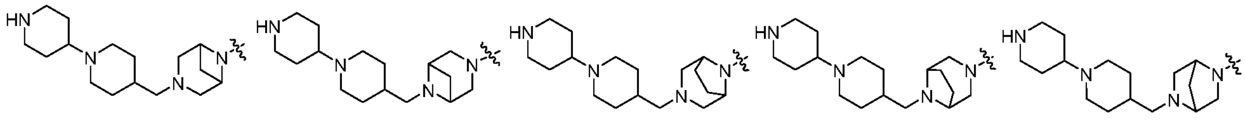

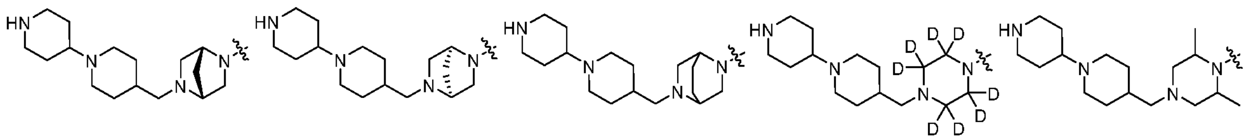

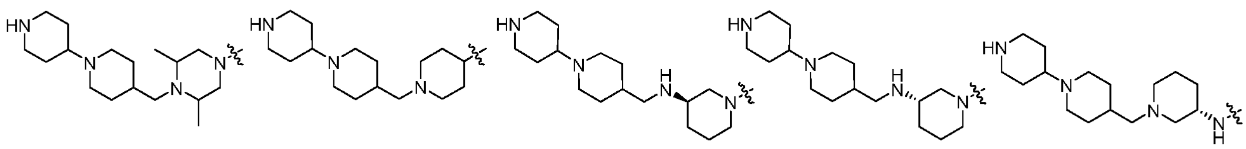

or

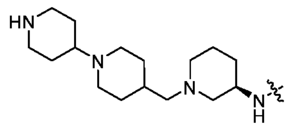 ;

wherein the groups are optionally partially or fully deuterated.

In some embodiments, the compound of Formula (II) is also of Formula (II-1) or Formula (II-2):

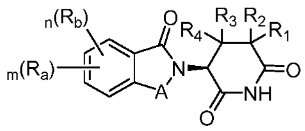

Formula (II)-1 or

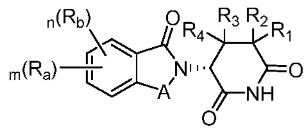

Formula (II)-2

wherein A, $R_1$, $R_2$, $R_3$, $R_4$, $(R_b)_n$ and $(R_a)_m$ are as defined in the compound of Formula (II) above and embodiments thereof.

In some embodiments, the compound of Formula (II) is also of Formula (II-3) or Formula (II-4):

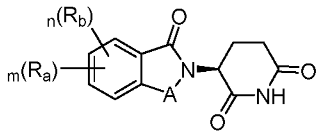

Formula (II-3) or

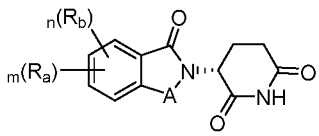

Formula (II-4)

wherein A, $(R_b)_n$ and $(R_a)_m$ are as defined in the compound of Formula (II) above and embodiments thereof.

In some embodiments, the compound of Formula (II) is also of Formula (II-5), Formula (II-6), Formula (II-7) or Formula (II-8):

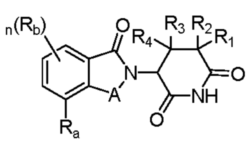

Formula (II-5)

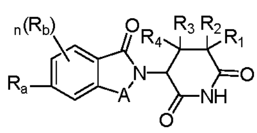

Formula (II-6)

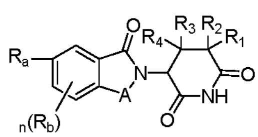

Formula (II-7) or

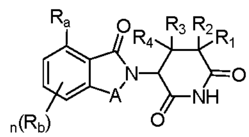

Formula (II-8)

wherein A, $R_1$, $R_2$, $R_3$, $R_4$, $(R_b)_n$ and $R_a$ are as defined in the compound of Formula (II) above and embodiments thereof.

In some embodiments, in the compounds of Formula (II-5), Formula (II-6), Formula (II-7) or Formula (II-8),

A represents CO, $CH_2$ or $CD_2$;

$R_1$, $R_2$, $R_3$ and $R_4$ are the same or different and each independently represent hydrogen, deuterium, halogen (e.g., fluorine, chlorine, bromine, or iodine), optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

$(R_b)_n$ indicates that benzene ring is substituted with n $R_b$ groups, and each $R_b$ are the same or different and each independently represent hydrogen, deuterium, halogen (e.g., fluorine, chlorine, bromine, or iodine), hydroxy, mercapto, nitro, amino, cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl (e.g., vinyl, propenyl, or butenyl), or $C_{2-6}$ alkynyl (e.g., ethynyl, propynyl, or butynyl);

wherein n represents an integer of 0, 1, 2, or 3;

$R_a$ represents the structure of the following formula:

$$W\text{-}LIN\text{-}U_2\text{-}R_c\text{-}U_1\text{-} ,$$

wherein $U_1$ and $U_2$ are the same or different and each independently represent a bond, $N(R_d)$ or O, wherein $R_d$ represents H or optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl);

$R_c$ represents optionally substituted nitrogen-containing monocyclic heterocyclylene or optionally substituted nitrogen-containing bridged heterocyclylene;

W represents hydrogen, optionally substituted nitrogen-containing monocyclic heterocyclyl, optionally substituted nitrogen-containing bridged heterocyclyl, optionally substituted cycloalkyl, optionally substituted heteroaryl, option-

ally substituted aryl, $C_{2-6}$ alkenyl (e.g., vinyl, propenyl, or butenyl), $C_{2-6}$ alkynyl (e.g., ethynyl, propynyl, or butynyl), a leaving group, CHO, COOH, $NH_2$, hydroxy or mercapto; and
wherein in case that the compound is partially or fully deuterated:

when W represents optionally substituted nitrogen-containing monocyclic heterocyclyl, optionally substituted nitrogen-containing bridged heterocyclyl, optionally substituted cycloalkyl, optionally substituted heteroaryl or optionally substituted aryl, LIN represents a bond, optionally substituted methylene, or optionally substituted linear or branched $C_{2-30}$ alkylene, wherein one or more (e.g., 1-10, 1-8 or 1-6, such as 1, 2, 3, 4, 5 or 6) groups $R_e$ and/or one or more (e.g., 1-10, 1-8 or 1-6, such as 1, 2, 3, 4, 5 or 6) groups $R_f$ and/or any combination of one or more (e.g., 1-10, 1-8 or 1-6, such as 1, 2, 3, 4, 5 or 6) groups $R_e$ and $R_f$ are optionally inserted into the backbone carbon chain of the linear or branched $C_{2-30}$ alkylene group, wherein carbon-carbon bond between one or more (e.g., 1-10, 1-8 or 1-6, such as 1, 2, 3, 4, 5 or 6) pairs of adjacent carbon atoms in the backbone carbon chain is interrupted by the group $R_e$, $R_f$, or a combination of $R_e$ and $R_f$; wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl (e.g., $C_{1-4}$ alkyl, such as methyl, ethyl, propyl, isopropyl, butyl, sec-butyl or tert-butyl), and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)-(e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), cyano or any combination thereof; or
when W represents hydrogen, $C_{2-6}$ alkenyl, $C_{2-6}$ alkynyl, a leaving group, CHO, COOH, $NH_2$, hydroxy or mercapto, LIN represents optionally substituted methylene, or optionally substituted linear or branched $C_{2-30}$ alkylene, wherein one or more (e.g., 1-10, 1-8 or 1-6, such as 1, 2, 3, 4, 5 or 6) groups $R_e$ and/or one or more (e.g., 1-10, 1-8 or 1-6, such as 1, 2, 3, 4, 5 or 6) groups $R_f$ and/or any combination of one or more (e.g., 1-10, 1-8 or 1-6, such as 1, 2, 3, 4, 5 or 6) groups $R_e$ and $R_f$ are optionally inserted into the backbone carbon chain of the linear or branched $C_{2-30}$ alkylene group, wherein carbon-carbon bond between one or more (e.g., 1-10, 1-8 or 1-6, such as 1, 2, 3, 4, 5 or 6) pairs of adjacent carbon atoms in the backbone carbon chain is interrupted by the group $R_e$, $R_f$, or a combination of $R_e$ and $R_f$; wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl (e.g., $C_{1-4}$ alkyl, such as methyl, $CD_3$, ethyl, propyl, isopropyl, butyl, sec-butyl or tert-butyl), and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally perdeuterated cyclopropyl, optionally perdeuterated cyclobutyl, optionally perdeuterated cyclopentyl, or optionally perdeuterated cyclohexyl), hydroxy, amino, mercapto, halogen (e.g., fluorine, chlorine, bromine, or iodine), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH- (e.g., optionally deuterated $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such

as $NH_2CH_2$-, $NH_2CH_2CH_2$- and $NH_2CH_2CH_2CH_2$-), optionally deuterated $C_{1-6}$ alkyl-NHC(O)- (e.g., optionally deuterated $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH-(e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-), cyano or any combination thereof; or

in case that the compound of Formula (II) is not deuterated, LIN represents optionally substituted methylene, and W represents optionally substituted nitrogen-containing monocyclic heterocyclyl or optionally substituted nitrogen-containing bridged heterocyclyl; and

wherein the nitrogen-containing monocyclic heterocyclylene, the nitrogen-containing bridged heterocyclylene, the nitrogen-containing monocyclic heterocyclyl, the nitrogen-containing bridged heterocyclyl, the cycloalkyl, the heteroaryl and the aryl are each independently optionally substituted with one or more (e.g., 1-10, 1-8, or 1-6, such as 1, 2, 3, 4, 5 or 6) substituents selected from the group consisting of deuterium, hydroxy, amino, mercapto, nitro, halogen (e.g., fluorine, chlorine, bromine, or iodine), cyano, CHO, COOH, $N_3$, methanesulfonyloxy, trifluoromethanesulfonyloxy, p-toluenesulfonyloxy, $C_{1-6}$ alkyl (e.g., $C_{1-4}$ alkyl, such as methyl, ethyl, propyl, isopropyl, butyl, sec-butyl or tert-butyl), deuterated $C_{1-6}$ alkyl (e.g., perdeuterated $C_{1-4}$ alkyl, such as $CD_3$, perdeuterated ethyl, perdeuterated propyl, perdeuterated isopropyl, perdeuterated butyl, perdeuterated sec-butyl or perdeuterated tert-butyl), halogenated $C_{1-6}$ alkyl, optionally substituted $C_{3-8}$ cycloalkyl (e.g., substituted with one or more, e.g., 1-10, 1-8 or 1-6, such as 1, 2, 3, 4, 5 or 6 substituents selected from the group consisting of deuterium, halogen, amino, hydroxy, mercapto, cyano, CHO, COOH, $N_3$, methanesulfonyloxy, trifluoromethanesulfonyloxy and p-toluenesulfonyloxy), optionally substituted 4- to 9-membered heterocyclyl (e.g., substituted with one or more substituents selected from the group consisting of deuterium, halogen, amino, hydroxy, mercapto, cyano, CHO, COOH, $N_3$, methanesulfonyloxy, trifluoromethanesulfonyloxy and p-toluenesulfonyloxy), $C_{1-6}$ alkoxy (e.g., $C_{1-4}$ alkoxy, such as methoxy, $CD_3$-O-, ethoxy, propoxy, isopropoxy, butoxy, sec-butoxy or tert-butoxy), deuterated $C_{1-6}$ alkyl-O-, $C_{1-6}$ alkyl-NH- (e.g., $C_{1-3}$ alkyl-NH-, such as $CH_3NH$-, $CH_3CH_2NH$- or $CH_3CH_2CH_2NH$-), deuterated $C_{1-6}$ alkyl-NH- (e.g., perdeuterated $C_{1-3}$ alkyl-NH-, such as $CD_3NH$-, $CD_3CD_2NH$- or $CD_3CD_2CD_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-6}$ alkyl-NHC(O)- (e.g., $C_{1-4}$ alkyl-NHC(O)-, such as $CH_3$-NHC(O)-, $CH_3CH_2$-NHC(O)-, and $CH_3CH_2CH_2$-NHC(O)-), deuterated $C_{1-6}$ alkyl-NHC(O)- (e.g., perdeuterated $C_{1-4}$ alkyl-NHC(O)-, such as $CD_3$-NHC(O)-, $CD_3CD_2$-NHC(O)-, and $CD_3CD_2CD_2$-NHC(O)-), $C_{1-6}$ alkyl-C(O)NH-, deuterated $C_{1-6}$ alkyl-C(O)NH- (e.g., optionally deuterated $C_{1-4}$ alkyl-C(O)NH-, such as $CH_3$-C(O)NH-, $CH_3CH_2$-C(O)NH-, and $CH_3CH_2CH_2$-C(O)NH-) or any combination thereof.

In some embodiments, the compounds of Formula (II) of the present invention and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 2 below are provided.

Table 2. The compounds of the present disclosure

| Compound No. | Structure of compound | Compound's name | Chinese translation of Compound's name |
|---|---|---|---|
| | 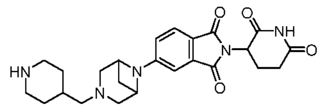 | 2-(2,6-dioxopiperidin-3-yl)-5-(3-(piperidin-4-ylmethyl)-3,6-diazabicyclo[3.1.1]heptan-6-yl)isoindoline-1,3-dione | 2-(2,6-dioxopiperidin-3-yl)-5-(3-(piperidin-4-ylmethyl)-3,6-diazabicyclo[3.1.1]heptan-6-yl)isoindoline-1,3-dione |
| | 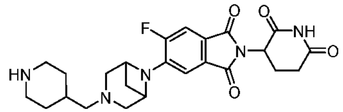 | 2-(2,6-dioxopiperidin-3-yl)-5-fluoro-6-(3-(piperidin-4-ylmethyl)-3,6-diazabicyclo[3.1.1]heptan-6-yl)isoindoline-1,3-dione | 2-(2,6-dioxopiperidin-3-yl)-5-fluoro-6-(3-(piperidin-4-ylmethyl)-3,6-diazabicyclo[3.1.1]heptan-6-yl)isoindoline-1,3-dione |
| | 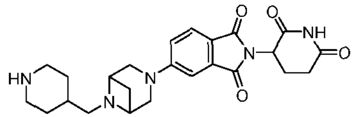 | 2-(2,6-dioxopiperidin-3-yl)-5-(6-(piperidin-4-ylmethyl)-3,6-diazabicyclo[3.1.1]heptan-3-yl)isoindoline-1,3-dione | 2-(2,6-dioxopiperidin-3-yl)-5-(6-(piperidin-4-ylmethyl)-3,6-diazabicyclo[3.1.1]heptan-3- yl)isoindoline-1,3-dione |
| | 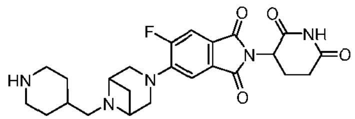 | 2-(2,6-dioxopiperidin-3-yl)-5-fluoro-6-(6-(piperidin-4-ylmethyl)-3,6-diazabicyclo[3.1.1]heptan-3-yl)isoindoline-1,3-dione | 2-(2,6-dioxopiperidin-3-yl)-5-fluoro-6-(6-(piperidin-4-ylmethyl)-3,6-diazabicyclo[3.1.1]heptan-3-vl)isoindoline-1,3-dione |
| | 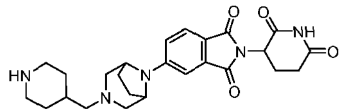 | 2-(2,6-dioxopiperidin-3-yl)-5-(3-(piperidin-4-ylmethyl)-3,8-diazabicyclo[3.2.1]octan-8-yl)isoindoline-1,3-dione | 2-(2,6-dioxopiperidin-3-yl)-5-(3-(piperidin-4-ylmethyl)-3,8-diazabicyclo[3.2.1]octan-8-yl)isoindoline-1,3-dione |
| | 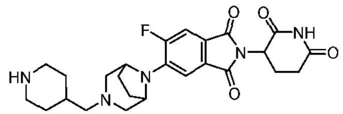 | 2-(2,6-dioxopiperidin-3-yl)-5 -fluoro-6-(3 -(piperidin-4-ylmethyl)-3,8-diazabicyclo[3.2.1]octan-8-yl)isoindoline-1,3-dione | 2-(2,6-dioxopiperidin-3-yl)-5-fluoro-6-(3-(piperidin-4-ylmethyl)-3,8-diazabicyclo[3.2.1]octan-8-yl)isoindoline-1,3-dione |
| | 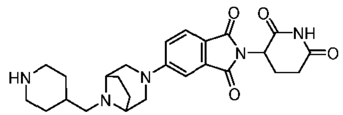 | 2-(2,6-dioxopiperidin-3-yl)-5-(8-(piperidin-4-ylmethyl)-3,8-diazabicyclo[3.2.1]octan-3-yl)isoindoline-1,3-dione | 2-(2,6-dioxopiperidin-3-yl)-5-(8-(piperidin-4-ylmethyl)-3,8-diazabicyclo[3.2.1]octan-3-vl)isoindoline-1,3-dione |

(continued)

| Compound No. | Structure of compound | Compound's name | Chinese translation of Compound's name |
|---|---|---|---|
| | 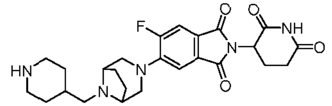 | 2-(2,6-dioxopiperidin-3-yl)-5 -fluoro-6-(8 -(piperidin-4-ylmethyl)-3,8-diazabicyclo[3.2.1]octan-3-yl)isoindoline-1,3-dione | 2-(2,6-dioxopiperidin-3-yl)-5-fluoro-6-(8-(piperidin-4-ylmethyl)-3,8-diazabicyclo[3.2.1]octan-3- vl)isoindoline-1,3-dione |
| | 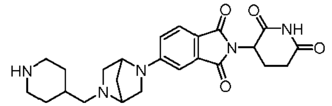 | 2-(2,6-dioxopiperidin-3-yl)-5-(5-(piperidin-4-ylmethyl)-2,5-diazabicyclo[2.2.1]heptan-2-yl)isoindoline-1,3-dione | 2-(2,6-dioxopiperidin-3-yl)-5-(5-(piperidin-4-ylmethyl)-2,5-diazabicyclo[2.2.1]heptan-2-yllisoindoline-1,3-dione |
| | 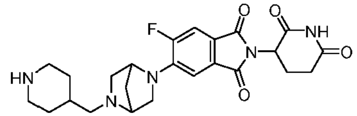 | 2-(2,6-dioxopiperidin-3-yl)-5 -fluoro-6-(5 -(piperidin-4-ylmethyl)-2,5-diazabicyclo[2.2.1]heptan-2-yl)isoindoline-1,3-dione | 2-(2,6-dioxopiperidin-3-yl)-5-fluoro-6-(5-(piperidin-4-ylmethyl)-2,5-diazabicyclo[2.2.1]heptan-2-yl)isoindoline-1,3-dione |
| | 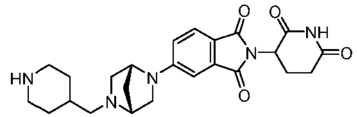 | 2-(2,6-dioxopiperidin-3-yl)-5-((1R,4R)-5-(piperidin-4-ylmethyl)-2,5-diazabicyclo[2.2.1]heptan-2-yl)isoindoline-1,3-dione | 2-(2,6-dioxopiperidin-3-yl)-5-((1R,4R)-5-(piperidin-4-ylmethyl)-2,5-diazabicyclo[2.2.1]heptan-2-yl)isoindoline-1,3-dione |
| | 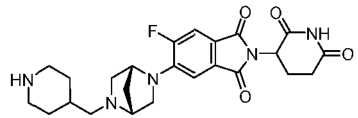 | 2-(2,6-dioxopiperidin-3-yl)-5-fluoro-6-((1R,4R)-5-(piperidin-4-ylmethyl)-2,5- diazabicyclo[2.2.1]heptan-2-yl)isoindoline-1,3-dione | 2-(2,6-dioxopiperidin-3-yl)-5-fluoro-6-((1R,4R)-5-(piperidin-4-ylmethyl)-2,5-diazabicyclo[2.2.1]heptan-2-vl)isoindoline-1,3-dione |
| | 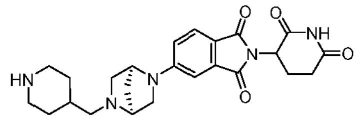 | 2-(2,6-dioxopiperidin-3-yl)-5-((1S,4S)-5-(piperidin-4-ylmethyl)-2,5-diazabicyclo[2.2.1]heptan-2-yl)isoindoline-1,3-dione | 2-(2,6-dioxopiperidin-3-yl)-5-((1S,4S)-5-(piperidin-4-ylmethyl)-2,5-diazabicyclo[2.2.1]heptan-2-yl)isoindoline-1,3-dione |
| | 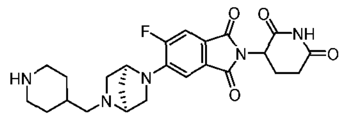 | 2-(2,6-dioxopiperidin-3-yl)-5-fluoro-6-((1S,4S)-5-(piperidin-4-ylmethyl)-2,5- diazabicyclo[2.2.1]heptan-2-yl)isoindoline-1,3-dione | 2-(2,6-dioxopiperidin-3-yl)-5-fluoro-6-((1S,4S)-5-(piperidin-4-ylmethyl)-2,5- diazabicyclo[2.2.1]heptan-2-yl)isoindoline-1,3-dione |

(continued)

| Compound No. | Structure of compound | Compound's name | Chinese translation of Compound's name |
|---|---|---|---|
| | 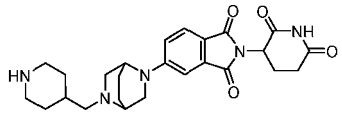 | 2-(2,6-dioxopiperidin-3-yl)-5-(5-(piperidin-4-ylmethyl)-2,5-diazabicyclo[2.2.2]octan-2-yl)isoindoline-1,3-dione | 2-(2,6-dioxopiperidin-3-yl)-5-(5-(piperidin-4-ylmethyl)-2,5-diazabicyclo[2.2.2]octan-2-yl)isoindoline-1,3-dione |
| | 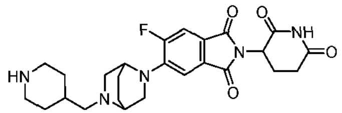 | 2-(2,6-dioxopiperidin-3-yl)-5-fluoro-6-(5-(piperidin-4-ylmethyl)-2,5-diazabicyclo[2.2.2]octan-2-yl)isoindoline-1,3-dione | 2-(2,6-dioxopiperidin-3-yl)-5-fluoro-6-(5-(piperidin-4-ylmethyl)-2,5-diazabicyclo[2.2.2]octan-2-yl)isoindoline-1,3-dione |
| | 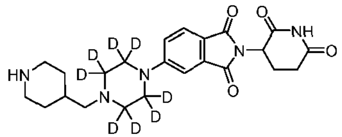 | 2-(2,6-dioxopiperidin-3-yl)-5-(4-(piperidin-4-ylmethyl)piperazin-1-yl-2,2,3,3,5,5,6,6-d8)isoindoline-1,3-dione | 2-(2,6-dioxopiperidin-3-yl)-5-(4-(piperidin-4-ylmethyl)piperazin-1-yl-2,2,3,3,5,5,6,6-d8)isoindoline-1,3-dione |
| | 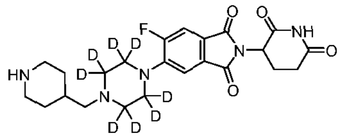 | 2-(2,6-dioxopiperidin-3-yl)-5-fluoro-6-(4-(piperidin-4-ylmethyl)piperazin-1-yl-2,2,3,3,5,5,6,6-d8)isoindoline-1,3-dione | 2-(2,6-dioxopiperidin-3-yl)-5-fluoro-6-(4-(piperidin-4-ylmethyl)piperazin-1-yl-2,2,3,3,5,5,6,6-d8)isoindoline-1,3-dione |
| | 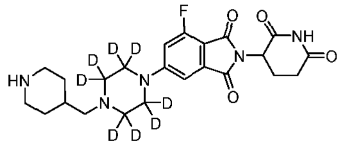 | 2-(2,6-dioxopiperidin-3-yl)-4-fluoro-6-(4-(piperidin-4-ylmethyl)piperazin-1-yl-2,2,3,3,5,5,6,6-d8)isoindoline-1,3-dione | 2-(2,6-dioxopiperidin-3-yl)-4-fluoro-6-(4-(piperidin-4-ylmethyl)piperazin-1-yl-2,2,3,3,5,5,6,6-d8)isoindoline-1,3-dione |
| | 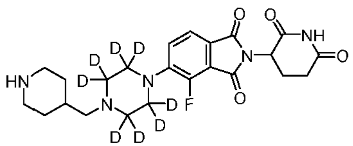 | 2-(2,6-dioxopiperidin-3-yl)-4-fluoro-5-(4-(piperidin-4-ylmethyl)piperazin-1-yl-2,2,3,3,5,5,6,6-d8)isoindoline-1,3-dione | 2-(2,6-dioxopiperidin-3-yl)-4-fluoro-5-(4-(piperidin-4-ylmethyl)piperazin-1-yl-2,2,3,3,5,5,6,6-d8)isoindoline-1,3-dione |

(continued)

| Compound No. | Structure of compound | Compound's name | Chinese translation of Compound's name |
|---|---|---|---|
| | 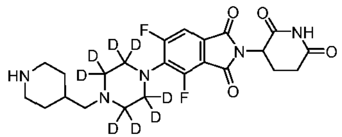 | 2-(2,6-dioxopiperidin-3-yl)-4,6-difluoro-5-(4-(piperidin-4-ylmethyl)piperazin-1-yl-2,2,3,3,5,5,6,6-d8)isoindoline-1,3-dione | 2-(2,6-dioxopiperidin-3-yl)-4,6-difluoro-5-(4-(piperidin-4-ylmethyl)piperazin-1-yl-2,2,3,3,5,5,6,6-d8)isoindoline-1,3-dione |
| | 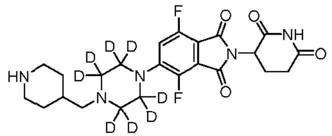 | 2-(2,6-dioxopiperidin-3-yl)-4,7-difluoro-5-(4-(piperidin-4-ylmethyl)piperazin-1-yl-2,2,3,3,5,5,6,6-d8)isoindoline-1,3-dione | 2-(2,6-dioxopiperidin-3-yl)-4,7-difluoro-5-(4-(piperidin-4-ylmethyl)piperazin-1-yl-2,2,3,3,5,5,6,6-d8)isoindoline-1,3-dione |
| | 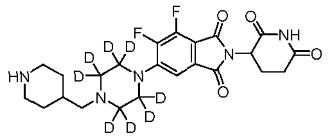 | 2-(2,6-dioxopiperidin-3-yl)-4,5-difluoro-6-(4-(piperidin-4-ylmethyl)piperazin-1-yl-2,2,3,3,5,5,6,6-d8)isoindoline-1,3-dione | 2-(2,6-dioxopiperidin-3-yl)-4,5-difluoro-6-(4-(piperidin-4-ylmethyl)piperazin-1-yl-2,2,3,3,5,5,6,6-d8)isoindoline-1,3-dione |
| | 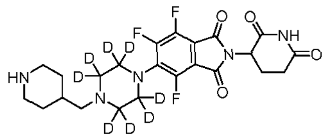 | 2-(2,6-dioxopiperidin-3-yl)-4,5,7-trifluoro-6-(4-(piperidin-4-ylmethyl)piperazin-1-yl-2,2,3,3,5,5,6,6-d8)isoindoline-1,3-dione | 2-(2,6-dioxopiperidin-3-yl)-4,5,7-trifluoro-6-(4-(piperidin-4-ylmethyl)piperazin-1-yl-2,2,3,3,5,5,6,6-d8)isoindoline-1,3-dione |
| | 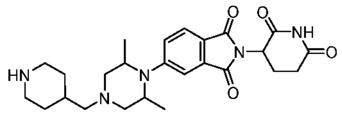 | 5-(2,6-dimethyl-4-(piperidin-4-ylmethyl)piperazin-1-yl)-2-(2,6-dioxopiperidin-3- yl)isoindoline-1,3-dione | 5-(2,6-dimethyl-4-(piperidin-4-ylmethyl)piperazin-1-yl)-2-(2,6-dioxopiperidin-3-yl)isoindoline-1,3-dione |
| | 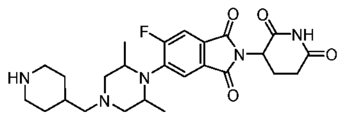 | 5-(2,6-dimethyl-4-(piperidin-4-ylmethyl)piperazin-1-yl)-2-(2,6-dioxopiperidin-3-yl)-6-fluoroisoindoline-1,3-dione | 5-(2,6-dimethyl-4-(piperidin-4-ylmethyl)piperazin-1-yl)-2-(2,6-dioxopiperidin-3-yl)-6-fluoroisoindoline-1,3-dione |
| | 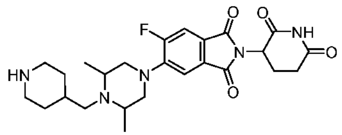 | 5-(3,5-dimethyl-4-(piperidin-4-ylmethyl)piperazin-1-yl)-2-(2,6-dioxopiperidin-3-yl)-6-fluoroisoindoline-1,3-dione | 5-(3,5-dimethyl-4-(piperidin-4-ylmethyl)piperazin-1-yl)-2-(2,6-dioxopiperidin-3-yl)-6-fluoroisoindoline-1,3-dione |

(continued)

| Compound No. | Structure of compound | Compound's name | Chinese translation of Compound's name |
|---|---|---|---|
| | 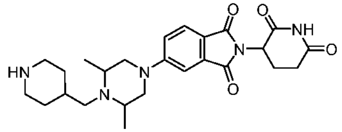 | 5-(3,5-dimethyl-4-(piperidin-4-ylmethyl)piperazin-1-yl)-2-(2,6-dioxopiperidin-3- yl)isoindoline-1,3-dione | 5-(3,5-dimethyl-4-(piperidin-4-ylmethyl)piperazin-1-yl)-2-(2,6-dioxopiperidin-3-yl)isoindoline-1,3-dione |
| | 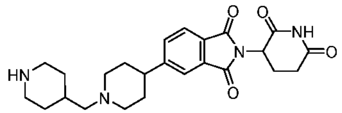 | 2-(2,6-dioxopiperidin-3-yl)-5-(1-(piperidin-4-ylmethyl)piperidin-4-yl)isoindoline-1,3-dione | 2-(2,6-dioxopiperidin-3-yl)-5-(1-(piperidin-4-ylmethyl)piperidin-4-yl)isoindoline-1,3-dione |
| | 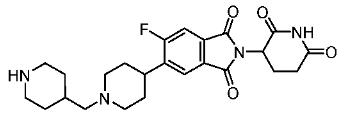 | 2-(2,6-dioxopiperidin-3-yl)-5-fluoro-6-(1-(piperidin-4-ylmethyl)piperidin-4-yl)isoindoline-1,3-dione | 2-(2,6-dioxopiperidin-3-yl)-5-fluoro-6-(1-(piperidin-4-ylmethyl)piperidin-4-yl)isoindoline-1,3-dione |
| | 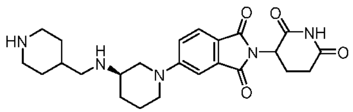 | 2-(2,6-dioxopiperidin-3-yl)-5-((R)-3-((piperidin-4-ylmethyl)amino)piperidin-1-vl)isoindoline-1,3-dione | 2-(2,6-dioxopiperidin-3-yl)-5-((R)-3-((piperidin-4-ylmethyl)amino)piperidin-1- yl)isoindoline-1,3-dione |
| | 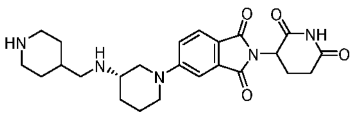 | 2-(2,6-dioxopiperidin-3-yl)-5-((S)-3-((piperidin-4-ylmethyl)amino)piperidin-1-yl)isoindoline-1,3-dione | 2-(2,6-dioxopiperidin-3-yl)-5-((S)-3-((piperidin-4-ylmethyl)amino)piperidin-1- yl)isoindoline-1,3-dione |
| | 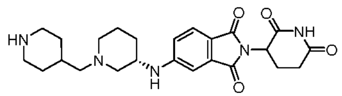 | 2-(2,6-dioxopiperidin-3-yl)-5-(((S)-1-(piperidin-4-ylmethyl)piperidin-3-yl)amino)isoindoline-1,3-dione | 2-(2,6-dioxopiperidin-3-yl)-5-(((S)-1-(piperidin-4-ylmethyl)piperidin-3-yl)amino)isoindoline-1,3-dione |
| | 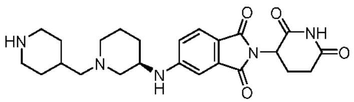 | 2-(2,6-dioxopiperidin-3-yl)-5-(((R)-1-(piperidin-4-ylmethyl)piperidin-3-yl)amino)isoindoline-1,3-dione | 2-(2,6-dioxopiperidin-3-yl)-5-(((R)-1-(piperidin-4-ylmethyl)piperidin-3-yl)amino)isoindoline-1,3-dione |
| | 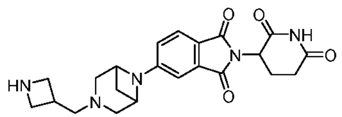 | 5-(3-(azetidin-3-ylmethyl)-3,6-diazabicyclo[3.1.1]heptan-6-yl)-2-(2,6-dioxopiperidin-3-yl)isoindoline-1,3-dione | 5-(3-(azetidin-3-ylmethyl)-3,6-diazabicyclo[3.1.1]heptan-6-yl)-2-(2,6-dioxopiperidin-3-yl)isoindoline-1,3-dione |

(continued)

| Compound No. | Structure of compound | Compound's name | Chinese translation of Compound's name |
|---|---|---|---|
| | 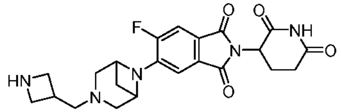 | 5-(3-(azetidin-3-ylmethyl)-3,6-diazabicyclo[3.1.1]heptan-6-yl)-2-(2,6-dioxopiperidin-3-yl)-6-fluoroisoindoline-1,3-dione | 5-(3-(azetidin-3-ylmethyl)-3,6-diazabicyclo[3.1.1]heptan-6-yl)-2-(2,6-dioxopiperidin-3-yl)-6-fluoroisoindoline-1,3- dione |
| | 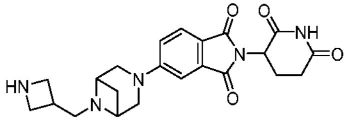 | 5-(6-(azetidin-3-ylmethyl)-3,6-diazabicyclo[3.1.1]heptan-3-yl)-2-(2,6-dioxopiperidin-3-yl)isoindoline-1,3-dione | 5-(6-(azetidin-3-ylmethyl)-3,6-diazabicyclo[3.1.1]heptan-3- yl)-2-(2,6-dioxopiperidin-3- yl)isoindoline-1,3-dione |
| | 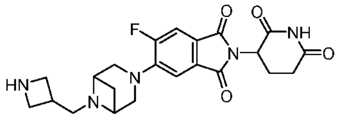 | 5-(6-(azetidin-3-ylmethyl)-3,6-diazabicyclo[3.1.1]heptan-3-yl)-2-(2,6-dioxopiperidin-3-yl)-6-fluoroisoindoline-1,3-dione | 5-(6-(azetidin-3-ylmethyl)-3,6-diazabicyclo[3.1.1]heptan-3- yl)-2-(2,6-dioxopiperidin-3- yl)-6-fluoroisoindoline-1,3- dione |
| | 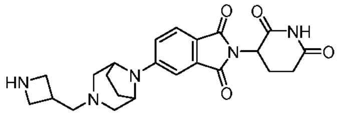 | 5-(3-(azetidin-3-ylmethyl)-3,8-diazabicyclo[3.2.1]octan-8-yl)-2-(2,6-dioxopiperidin-3-yl)isoindoline-1,3-dione | 5-(3-(azetidin-3-ylmethyl)-3,8-diazabicyclo[3.2.1]octan-8-yl)-2-(2,6-dioxopiperidin-3- yl)isoindoline-1,3-dione |
| | 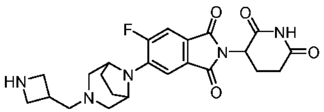 | 5-(3-(azetidin-3-ylmethyl)-3,8-diazabicyclo[3.2.1]octan-8-yl)-2-(2,6-dioxopiperidin-3-yl)-6-fluoroisoindoline-1,3-dione | 5-(3-(azetidin-3-ylmethyl)-3,8-diazabicyclo[3.2.1]octan-8-yl)-2-(2,6-dioxopiperidin-3- yl)-6-fluoroisoindoline-1,3- dione |
| | 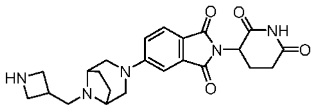 | 5-(8-(azetidin-3-ylmethyl)-3,8-diazabicyclo[3.2.1]octan-3-yl)-2-(2,6-dioxopiperidin-3-yl)isoindoline-1,3-dione | 5-(8-(azetidin-3-ylmethyl)-3,8-diazabicyclo[3.2.1]octan-3- yl)-2-(2,6-dioxopiperidin-3- yl)isoindoline-1,3-dione |
| | 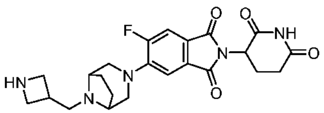 | 2-(2,6-dioxopiperidin-3-yl)-5-fluoro-6-(8-(piperidin-4-ylmethyl)-3,8-diazabicyclo[3.2.1]octan-3-yl)isoindoline-1,3-dione | 2-(2,6-dioxopiperidin-3-yl)-5-fluoro-6-(8-(piperidin-4-ylmethyl)-3,8-diazabicyclo[3.2.1]octan-3- yl)isoindoline-1,3-dione |

(continued)

| Compound No. | Structure of compound | Compound's name | Chinese translation of Compound's name |
|---|---|---|---|
| | 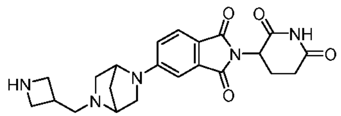 | 5-(5-(azetidin-3-ylmethyl)-2,5-diazabicyclo[2.2.1]heptan-2-yl)-2-(2,6-dioxopiperidin-3-yl)isoindoline-1,3-dione | 5-(5-(azetidin-3-ylmethyl)-2,5-diazabicyclo[2.2.1]heptan-2-yl)-2-(2,6-dioxopiperidin-3-yl)isoindoline-1,3-dione |
| | 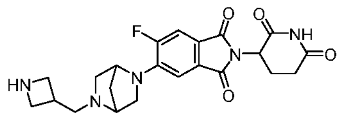 | 5-(5-(azetidin-3-ylmethyl)-2,5-diazabicyclo[2.2.1]heptan-2-yl)-2-(2,6-dioxopiperidin-3-yl)-6-fluoroisoindoline-1,3-dione | 5-(5-(azetidin-3-ylmethyl)-2,5-diazabicyclo[2.2.1]heptan-2-yl)-2-(2,6-dioxopiperidin-3-yl)-6-fluoroisoindoline-1,3- dione |
| | 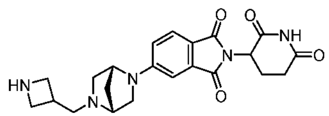 | 5-((1R,4R)-5-(azetidin-3- ylmethyl)-2,5-diazabicyclo[2.2.1]heptan-2-yl)-2-(2,6-dioxopiperidin-3-yl)isoindoline-1,3-dione | 5-((1R,4R)-5-(azetidin-3- ylmethyl)-2,5-diazabicyclo[2.2.1]heptan-2-yl)-2-(2,6-dioxopiperidin-3-yl)isoindoline-1,3-dione |
| | 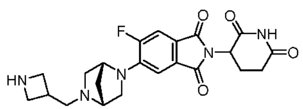 | 5-((1R,4R)-5-(azetidin-3- ylmethyl)-2,5-diazabicyclo[2.2.1]heptan-2-yl)-2-(2,6-dioxopiperidin-3-yl)-6-fluoroisoindoline-1,3-dione | 5-((1R,4R)-5-(azetidin-3- ylmethyl)-2,5-diazabicyclo[2.2.1]heptan-2-yl)-2-(2,6-dioxopiperidin-3-yl)-6-fluoroisoindoline-1,3-dione |
| | 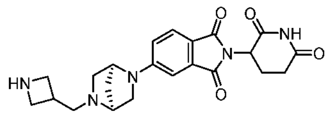 | 5-((1S,4S)-5-(azetidin-3-ylmethyl)-2,5-diazabicyclo[2.2.1]heptan-2-yl)-2-(2,6-dioxopiperidin-3-yl)isoindoline-1,3-dione | 5-((1S,4S)-5-(azetidin-3-ylmethyl)-2,5-diazabicyclo[2.2.1]heptan-2-yl)-2-(2,6-dioxopiperidin-3-yl)isoindoline-1,3-dione |
| | 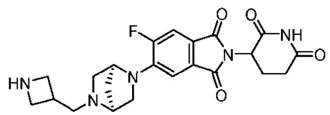 | 5-((1S,4S)-5-(azetidin-3-ylmethyl)-2,5-diazabicyclo[2.2.1]heptan-2-yl)-2-(2,6-dioxopiperidin-3-yl)-6-fluoroisoindoline-1,3-dione | 5-((1S,4S)-5-(azetidin-3-ylmethyl)-2,5-diazabicyclo[2.2.1]heptan-2-yl)-2-(2,6-dioxopiperidin-3-yl)-6-fluoroisoindoline-1,3- dione |
| | 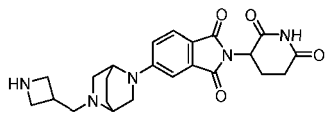 | 5-(5-(azetidin-3-ylmethyl)-2,5-diazabicyclo[2.2.2]octan-2-yl)-2-(2,6-dioxopiperidin-3-yl)isoindoline-1,3-dione | 5-(5-(azetidin-3-ylmethyl)-2,5-diazabicyclo[2.2.2]octan-2-yl)-2-(2,6-dioxopiperidin-3- yl)isoindoline-1,3-dione |
| | 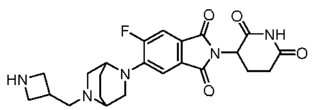 | 5-(5-(azetidin-3-ylmethyl)-2,5-diazabicyclo[2.2.2]octan-2-yl)-2-(2,6-dioxopiperidin-3-yl)-6-fluoroisoindoline-1,3-dione | 5-(5-(azetidin-3-ylmethyl)-2,5-diazabicyclo[2.2.2]octan-2-yl)-2-(2,6-dioxopiperidin-3- yl)-6-fluoroisoindoline-1,3- dione |

(continued)

| Compound No. | Structure of compound | Compound's name | Chinese translation of Compound's name |
|---|---|---|---|
| | 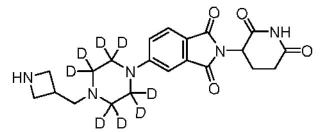 | 5-(4-(azetidin-3-ylmethyl)piperazin-1-yl-2,2,3,3,5,5,6,6-d8)-2-(2,6-dioxopiperidin-3- yl)isoindoline-1,3-dione | 5-(4-(azetidin-3- ylmethyl)piperazin-1-yl-2,2,3,3,5,5,6,6-d8)-2-(2,6-dioxopiperidin-3-yl)isoindoline-1,3-dione |
| | 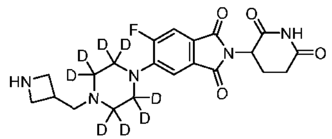 | 5-(4-(azetidin-3-ylmethyl)piperazin-1-yl-2,2,3,3,5,5,6,6-d8)-2-(2,6-dioxopiperidin-3-yl)-6-fluoroisoindoline-1,3-dione | 5-(4-(azetidin-3-ylmethyl)piperazin-1-yl-2,2,3,3,5,5,6,6-d8)-2-(2,6-dioxopiperidin-3-yl)-6-fluoroisoindoline-1,3-dione |
| | 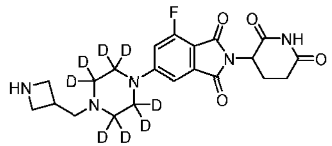 | 6-(4-(azetidin-3-ylmethyl)piperazin-1-yl-2,2,3,3,5,5,6,6-d8)-2-(2,6-dioxopiperidin-3-yl)-4-fluoroisoindoline-1,3-dione | 6-(4-(azetidin-3-ylmethyl)piperazin-1-yl-2,2,3,3,5,5,6,6-d8)-2-(2,6-dioxopiperidin-3-yl)-4-fluoroisoindoline-1,3-dione |
| | 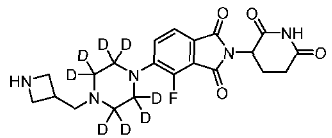 | 5-(4-(azetidin-3-ylmethyl)piperazin-1-yl-2,2,3,3,5,5,6,6-d8)-2-(2,6-dioxopiperidin-3-yl)-4-fluoroisoindoline-1,3-dione | 5-(4-(azetidin-3-ylmethyl)piperazin-1-yl-2,2,3,3,5,5,6,6-d8)-2-(2,6-dioxopiperidin-3-yl)-4-fluoroisoindoline-1,3-dione |
| | 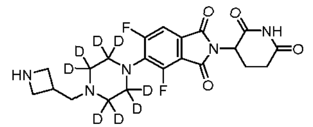 | 5-(4-(azetidin-3- ylmethyl)piperazin-1-yl-2,2,3,3,5,5,6,6-d8)-2-(2,6-dioxopiperidin-3-yl)-4,6-difluoroisoindoline-1,3- dione | 5-(4-(azetidin-3-ylmethyl)piperazin-1-yl-2,2,3,3,5,5,6,6-d8)-2-(2,6-dioxopiperidin-3-yl)-4,6-difluoroisoindoline-1,3- dione |
| | 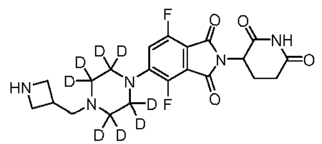 | 5-(4-(azetidin-3- ylmethyl)piperazin-1-yl-2,2,3,3,5,5,6,6-d8)-2-(2,6-dioxopiperidin-3-yl)-4,7-difluoroisoindoline-1,3- dione | 5-(4-(azetidin-3-ylmethyl)piperazin-1-yl-2,2,3,3,5,5,6,6-d8)-2-(2,6-dioxopiperidin-3-yl)-4,7-difluoroisoindoline-1,3- dione |

(continued)

| Compound No. | Structure of compound | Compound's name | Chinese translation of Compound's name |
|---|---|---|---|
| | 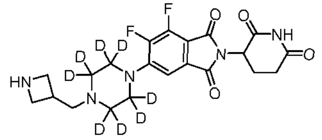 | 6-(4-(azetidin-3-ylmethyl)piperazin-1-yl-2,2,3,3,5,5,6,6-d8)-2-(2,6-dioxopiperidin-3-yl)-4,5- difluoroisoindoline-1,3- dione | 6-(4-(azetidin-3-ylmethyl)piperazin-1-yl-2,2,3,3,5,5,6,6-d8)-2-(2,6-dioxopiperidin-3-yl)-4,5- difluoroisoindoline-1,3- dione |
| | 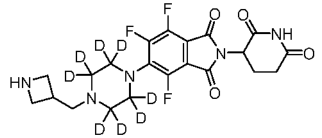 | 5-(4-(azetidin-3-ylmethyl)piperazin-1-yl-2,2,3,3,5,5,6,6-d8)-2-(2,6-dioxopiperidin-3-yl)-4,6,7-trifluoroisoindoline -1,3-dione | 5-(4-(azetidin-3-ylmethyl)piperazin-1-yl-2,2,3,3,5,5,6,6-d8)-2-(2,6-dioxopiperidin-3-yl)-4,6,7-trifluoroisoindoline-1,3- dione |
| | 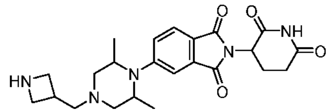 | 5-(4-(azetidin-3-ylmethyl)-2,6-dimethylpiperazin-1- yl)-2-(2,6-dioxopiperidin-3-yl)isoindoline-1,3-dione | 5-(4-(azetidin-3-ylmethyl)-2,6-dimethylpiperazin-1-yl)-2-(2,6-dioxopiperidin-3-yl)isoindoline-1,3-dione |
| | 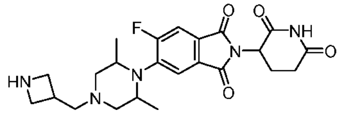 | 5-(4-(azetidin-3-ylmethyl)-2,6-dimethylpiperazin-1- yl)-2-(2,6-dioxopiperidin-3-yl)-6-fluoroisoindoline-1,3-dione | 5-(4-(azetidin-3-ylmethyl)-2,6-dimethylpiperazin-1-yl)-2-(2,6-dioxopiperidin-3-yl)-6-fluoroisoindoline-1,3-dione |
| | 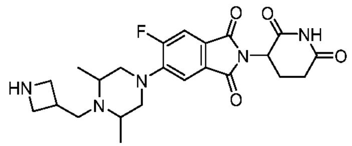 | 5-(4-(azetidin-3-ylmethyl)-3,5-dimethylpiperazin-1-yl)-2-(2,6-dioxopiperidin-3-yl)-6-fluoroisoindoline-1,3-dione | 5-(4-(azetidin-3-ylmethyl)-3,5-dimethylpiperazin-1-yl)-2-(2,6-dioxopiperidin-3-yl)-6-fluoroisoindoline-1,3-dione |
| | 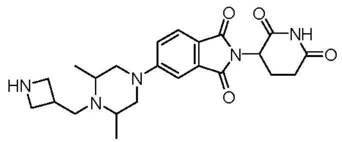 | 5-(4-(azetidin-3-ylmethyl)-3,5-dimethylpiperazin-1-yl)-2-(2,6-dioxopiperidin-3-yl)isoindoline-1,3-dione | 5-(4-(azetidin-3-ylmethyl)-3,5-dimethylpiperazin-1-yl)-2-(2,6-dioxopiperidin-3- yl)isoindoline-1,3-dione |
| | 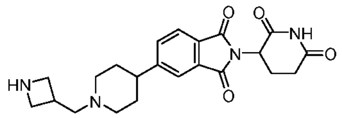 | 5-(1-(azetidin-3-ylmethyl)piperidin-4-yl)-2-(2,6-dioxopiperidin-3- yl)isoindoline-1,3-dione | 5-(1-(azetidin-3- ylmethyl)piperidin-4-yl)-2-(2,6-dioxopiperidin-3-yl)isoindoline-1,3-dione |

(continued)

| Compound No. | Structure of compound | Compound's name | Chinese translation of Compound's name |
|---|---|---|---|
| | 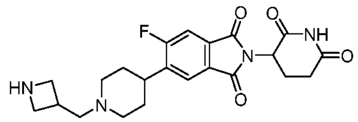 | 5-(1-(azetidin-3-ylmethyl)piperidin-4-yl)-2-(2,6-dioxopiperidin-3-yl)-6-fluoroisoindoline-1,3-dione | 5-(1-(azetidin-3-ylmethyl)piperidin-4-yl)-2-(2,6-dioxopiperidin-3-yl)-6-fluoroisoindoline-1,3-dione |
| | 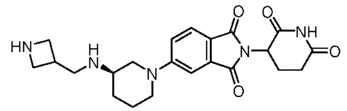 | 5-((R)-3-((azetidin-3- ylmethyl)amino)piperidin-1-yl)-2-(2,6-dioxopiperidin-3-yl)isoindoline-1,3-dione | 5-((R)-3-((azetidin-3- ylmethyl)amino)piperidin-1- yl)-2-(2,6-dioxopiperidin-3- yl)isoindoline-1,3-dione |
| | 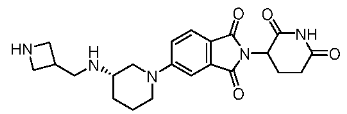 | 5-((S)-3-((azetidin-3- ylmethyl)amino)piperidin-1-yl)-2-(2,6-dioxopiperidin-3-yl)isoindoline-1,3-dione | 5-((S)-3-((azetidin-3-ylmethyl)amino)piperidin-1- yl)-2-(2,6-dioxopiperidin-3- yl)isoindoline-1,3-dione |
| | 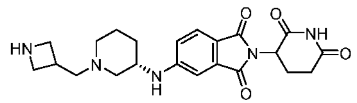 | 5-(((S)-1-(azetidin-3-ylmethyl)piperidin-3-yl)amino)-2-(2,6-dioxopiperidin-3- yl)isoindoline-1,3-dione | 5-(((S)-1-(azetidin-3-ylmethyl)piperidin-3-yl)amino)-2-(2,6-dioxopiperidin-3- yl)isoindoline-1,3-dione |
| | 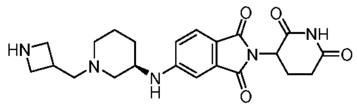 | 5-(((R)-1-(azetidin-3-ylmethyl)piperidin-3-yl)amino)-2-(2,6-dioxopiperidin-3-yl)isoindoline-1,3-dione | 5-(((R)-1-(azetidin-3-ylmethyl)piperidin-3-yl)amino)-2-(2,6-dioxopiperidin-3- yl)isoindoline-1,3-dione |

**Compounds of Formula (III)**

The present disclosure provides a compound of Formula (III) or salts (including pharmaceutically acceptable salts), stereoisomers (including enantiomers), solvates, isotopically enriched analogs, prodrugs, or polymorphs thereof:

PBM-$R^1$-$W^1$-LIN-ULM Formula (III)

wherein PBM, $R^1$, $W^1$, LIN and ULM are as defined in the compounds of Formula (III) above.

When the compound of Formula (III) of the present disclosure is not deuterated, the compound of Formula (III) does not comprise the following compounds of Formual (IV-1):

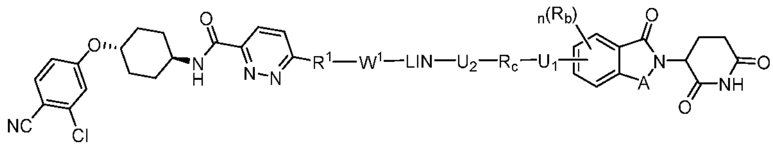

Formula (IV-1)

in Formula (IV-1), $R^1$ represents a bond, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, n represents an integer of 1, 2 or 3, and $U_1$ and $U_2$ are the same or different and each independently represent a bond, NH or O; or

in Formula (IV-1), $R^1$ represents a bond, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, n represents an integer of 1, 2 or 3, and $U_1$ and $U_2$ are the same or different and each independently represent a bond, NH or O.

When the compound of Formula (III) of the present disclosure is not deuterated, the compound of Formula (III) does not comprise the following compound of Formual (IV-2):

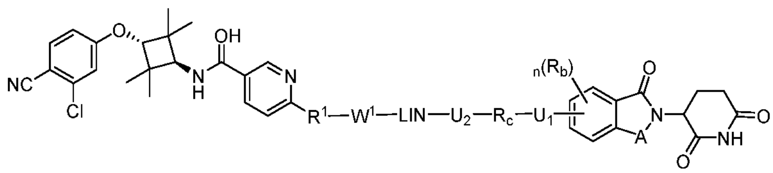

Formula (IV-2)

in Formula (IV-2), $R^1$ represents a bond, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, n represents an integer of 1, 2 or 3, and $U_1$ and $U_2$ are the same or different and each independently represent a bond, NH or O; or

in Formula (IV-2), $R^1$ represents a bond, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, and n represents an integer of 1, 2 or 3, and $U_1$ and $U_2$ are the same or different and each independently represent a bond, NH or O.

When the compound of Formula (III) of the present disclosure is not deuterated, the compound of Formula (III) does not comprise the following compounds of Formual (IV-3):

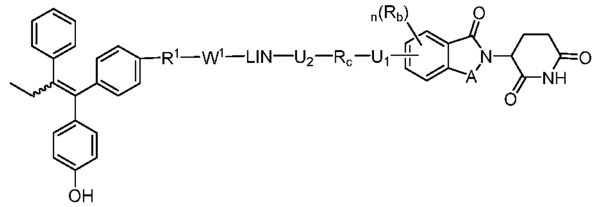

Formula (IV-3)

in Formula (IV-3), $R^1$ represents a bond or $-O-CH_2-CH_2-$, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O; or

in Formula (IV-3), $R^1$ represents a bond or $-O-CH_2-CH_2-$, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O.

When the compound of Formula (III) of the present disclosure is not deuterated, the compound of Formula (III) does not comprise the following compounds of Formual (IV-4):

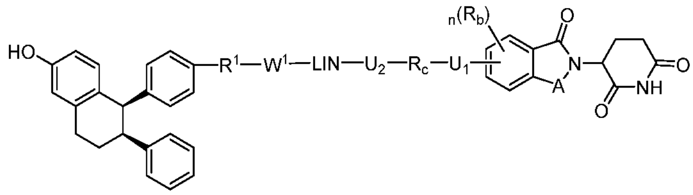

Formula (IV-4)

in Formula (IV-4), $R^1$ represents a bond or represents $-O-CH_2-CH_2-$, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O; or

in Formula (IV-4), $R^1$ represents a bond or represents $-O-CH_2-CH_2-$, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O.

The compound of Formula (III) or salts (including pharmaceutically acceptable salts), stereoisomers (including enantiomers), solvates, isotope enriched analogues, prodrugs, or polymorphs thereof of the present disclosure can bind to target proteins, recruit target proteins to E3 ubiquitin ligases for ubiquitination labeling and degradation. The compound of Formula (III) of the present disclosure can specifically target specific type or family of target protein, and exhibit a wide range of pharmacological activities by degrading/inhibiting diverse types or families of target proteins.

In some embodiments, specific target protein includes, but is not limited to, the target proteins in Table 3:

Table 3. Applicable specific target proteins

| abbreviation of target protein | target protein's name | Chinese translation of target protein's name |
|---|---|---|
| ESR1 | Estrogen receptor | Estrogen receptor |
| AR | Androgen receptor | Androgen receptor |

(continued)

| abbreviation of target protein | target protein's name | Chinese translation of target protein's name |
|---|---|---|
| BTK | Tyrosine-protein kinase BTK | Tyrosine-protein kinase BTK |
| IRAK4 | Interleukin-1 receptor-associated kinase 4 | Interleukin-1 receptor-associated kinase 4 |
| EGFR | Epidermal growth factor receptor | Epidermal growth factor receptor |
| MET | Hepatocyte growth factor receptor | Hepatocyte growth factor receptor |
| KIT | Mast/stem cell growth factor receptor Kit | Mast/stem cell growth factor receptor Kit |
| EPHA2 | Ephrin type-A receptor 2 | Ephrin type-A receptor 2 |
| PDE4D | cAMP-specific 3',5'-cyclic phosphodiesterase 4D | cAMP-specific 3',5'-cyclic phosphodiesterase 4D |
| SRC | Proto-oncogene tyrosine-protein kinase Src | Proto-oncogene tyrosine-protein kinase Src |
| BRAF | Serine/threonine-protein kinase B-raf | Serine/threonine-protein kinase B-raf |
| FGFR2 | Fibroblast growth factor receptor 2 | Fibroblast growth factor receptor 2 |
| FGFR1 | Fibroblast growth factor receptor 1 | Fibroblast growth factor receptor 1 |
| LYN | Tyrosine-protein kinase Lyn | Tyrosine-protein kinase Lyn |
| ITK | Tyrosine-protein kinase ITK/TSK | Tyrosine-protein kinase ITK/TSK |
| PARP1 | Poly [ADP-ribose] polymerase 1 | Poly [ADP-ribose] polymerase 1 |
| HDAC2 | Histone deacetylase 2 | Histone deacetylase 2 |
| HDAC3 | Histone deacetylase 3 | Histone deacetylase 3 |
| JAK1 | Tyrosine-protein kinase JAK1 | Tyrosine-protein kinase JAK1 |
| BCL2 | Apoptosis regulator Bcl-2 | Apoptosis regulator Bcl-2 |
| HDAC6 | Histone deacetylase 6 | Histone deacetylase 6 |
| CRBN | Protein cereblon | Protein cereblon |
| EPHB2 | Ephrin type-B receptor 2 | Ephrin type-B receptor 2 |
| BLK | Tyrosine-protein kinase Blk | Tyrosine-protein kinase Blk |
| HDAC1 | Histone deacetylase 1 | Histone deacetylase 1 |
| IGF1R | Insulin-like growth factor 1 receptor | Insulin-like growth factor 1 receptor |
| TGFBR1 | TGF-beta receptor type-1 | TGF-beta receptor type-1 |
| AKT2 | RAC-beta serine/threonine-protein kinase | RAC-beta serine/threonine-protein kinase |
| AKT1 | RAC-alpha serine/threonine-protein kinase | RAC-alpha serine/threonine-protein kinase |
| FAK | Focal adhesion kinase | Focal adhesion kinase |
| MAPK1 | Mitogen-activated protein kinase 1 | Mitogen-activated protein kinase 1 |
| MAPK14 | Mitogen-activated protein kinase 14 | Mitogen-activated protein kinase 14 |
| CDK9 | Cyclin-dependent kinase 9 | Cyclin-dependent kinase 9 |
| MCL1 | Induced myeloid leukemia cell | Induced myeloid leukemia cell |
| | differentiation protein Mcl-1 | differentiation protein Mcl-1 |
| BRD4 | Bromodomain-containing protein 4 | Bromodomain-containing protein 4 |
| BRD3 | Bromodomain-containing protein 3 | Bromodomain-containing protein 3 |
| CDK13 | Cyclin-dependent kinase 13 | Cyclin-dependent kinase 13 |

(continued)

| abbreviation of target protein | target protein's name | Chinese translation of target protein's name |
|---|---|---|
| BCL2L1 | Bcl-2-like protein 1 | Bcl-2-like protein 1 |
| CDK12 | Cyclin-dependent kinase 12 | Cyclin-dependent kinase 12 |
| CDK1 | Cyclin-dependent kinase 1 | Cyclin-dependent kinase 1 |
| AKT3 | RAC-gamma serine/threonine-protein kinase | RAC-gamma serine/threonine-protein kinase |
| CDK11B | Cyclin-dependent kinase 11B | Cyclin-dependent kinase 11B |
| PAK4 | Serine/threonine-protein kinase PAK 4 | Serine/threonine-protein kinase PAK 4 |
| MAPKAPK2 | MAP kinase-activated protein kinase 2 | MAP kinase-activated protein kinase 2 |
| TNK2 | Activated CDC42 kinase 1 | Activated CDC42 kinase 1 |
| SIRT2 | NAD-dependent protein deacetylase sirtuin-2 | NAD-dependent protein deacetylase sirtuin-2 |
| DAPK1 | Death-associated protein kinase 1 | Death-associated protein kinase 1 |
| ABL2 | Tyrosine-protein kinase ABL2 | Tyrosine-protein kinase ABL2 |
| PRKAA2 | 5'-AMP-activated protein kinase catalytic subunit alpha-2 | 5'-AMP-activated protein kinase catalytic subunit alpha-2 |
| KAT2A | Histone acetyltransferase KAT2A | Histone acetyltransferase KAT2A |
| PBRM1 | Protein polybromo-1 | Protein polybromo-1 |
| EIF2AK2 | Interferon-induced, double-stranded RNA-activated protein kinase | Interferon-induced, double-stranded RNA-activated protein kinase |
| MAP3K7 | Mitogen-activated protein kinase kinase kinase 7 | Mitogen-activated protein kinase kinase kinase 7 |
| MAPT | Microtubule-associated protein tau | Microtubule-associated protein tau |
| RIPK1 | Receptor-interacting serine/threonine-protein kinase 1 | Receptor-interacting serine/threonine-protein kinase 1 |
| IRAK1 | Interleukin-1 receptor-associated kinase 1 | Interleukin-1 receptor-associated kinase 1 |
| MAP4K1 | Mitogen-activated protein kinase kinase kinase kinase 1 | Mitogen-activated protein kinase kinase kinase kinase 1 |
| MARK4 | MAP/microtubule affinity-regulating kinase 4 | MAP/microtubule affinity-regulating kinase 4 |
| BRD9 | Bromodomain-containing protein 9 | Bromodomain-containing protein 9 |
| RIPK2 | Receptor-interacting serine/threonine-protein kinase 2 | Receptor-interacting serine/threonine-protein kinase 2 |
| LIMK1 | LIM domain kinase 1 | LIM domain kinase 1 |
| STIG 8 | Serine/threonine-protein kinase 38 | Serine/threonine-protein kinase 38 |
| TRIM24 | Transcription intermediary factor 1-alpha | Transcription intermediary factor 1-alpha |
| SMARCA4 | Transcription activator BRG1 | Transcription activator BRG1 |
| PRKAA1 | 5'-AMP-activated protein kinase catalytic subunit alpha-1 | 5'-AMP-activated protein kinase catalytic subunit alpha-1 |
| TBK1 | Serine/threonine-protein kinase TBK1 | Serine/threonine-protein kinase TBK1 |
| KRAS | GTPase KRas | GTPase KRas |
| SMARCA2 | Probable global transcription activator SNF2L2 | Probable global transcription activator SNF2L2 |

(continued)

| abbreviation of target protein | target protein's name | Chinese translation of target protein's name |
|---|---|---|
| PCNA | Proliferating cell nuclear antigen | Proliferating cell nuclear antigen |
| BRD7 | Bromodomain-containing protein 7 | Bromodomain-containing protein 7 |
| SUZ12 | Polycomb protein SUZ12 | Polycomb protein SUZ12 |
| IKZF1 | DNA-binding protein Ikaros | DNA-binding protein Ikaros |
| HTT | Huntingtin | Huntingtin |
| SNCA | Alpha-synuclein | Alpha-synuclein |
| SLC9A1 | Sodium/hydrogen exchanger 1 | Sodium/hydrogen exchanger 1 |
| FER | Tyrosine-protein kinase Fer | Tyrosine-protein kinase Fer |
| MAP4K2 | Mitogen-activated protein kinase kinase kinase kinase 2 | Mitogen-activated protein kinase kinase kinase kinase 2 |
| DLG4 | Disks large homolog 4 | Disks large homolog 4 |
| IKZF3 | Zinc finger protein Aiolos | Zinc finger protein Aiolos |
| PDE4A | cAMP-specific 3',5'-cyclic phosphodiesterase 4A | cAMP-specific 3',5'-cyclic phosphodiesterase 4A |
| HMGCR | 3-hydroxy-3-methylglutaryl-coenzyme A reductase | 3-hydroxy-3-methylglutaryl-coenzyme A reductase |
| ABL1 | Tyrosine-protein kinase ABL1 | Tyrosine-protein kinase ABL1 |
| RARA | Retinoic acid receptor alpha | Retinoic acid receptor alpha |
| FLT3 | Receptor-type tyrosine-protein kinase FLT3 | Receptor-type tyrosine-protein kinase FLT3 |
| RARG | Retinoic acid receptor gamma | Retinoic acid receptor gamma |
| VEGFR-1 | Vascular endothelial growth factor receptor 1 | Vascular endothelial growth factor receptor 1 |
| EZH2 | Histone-lysine N-methyltransferase EZH2 | Histone-lysine N-methyltransferase EZH2 |
| EPHA1 | Ephrin type-A receptor 1 | Ephrin type-A receptor 1 |
| AURKA | Aurora kinase A | Aurora kinase A |
| CHEK1 | Serine/threonine-protein kinase Chk1 | Serine/threonine-protein kinase Chk1 |
| WEE1 | Wee1-like protein kinase | Wee1-like protein kinase |
| PLK1 | Serine/threonine-protein kinase PLK1 | Serine/threonine-protein kinase PLK1 |
| CDC7 | Cell division cycle 7-related protein kinase | Cell division cycle 7-related protein kinase |
| AURKB | Aurora kinase B | Aurora kinase B |
| PLK4 | Serine/threonine-protein kinase PLK4 | Serine/threonine-protein kinase PLK4 |
| MDM2 | E3 ubiquitin-protein ligase Mdm2 | E3 ubiquitin-protein ligase Mdm2 |
| MAPK6 | Mitogen-activated protein kinase 6 | Mitogen-activated protein kinase 6 |
| BUB1 | Mitotic checkpoint serine/threonine-protein kinase BUB1 | Mitotic checkpoint serine/threonine-protein kinase BUB 1 |
| ESRRA | Steroid hormone receptor ERR1 | Steroid hormone receptor ERR1 |
| BCL6 | B-cell lymphoma 6 protein | B-cell lymphoma 6 protein |
| MAPK12 | Mitogen-activated protein kinase 12 | Mitogen-activated protein kinase 12 |

(continued)

| abbreviation of target protein | target protein's name | Chinese translation of target protein's name |
|---|---|---|
| CRABP2 | Cellular retinoic acid-binding protein 2 | Cellular retinoic acid-binding protein 2 |
| HIPK1 | Homeodomain-interacting protein kinase 1 | Homeodomain-interacting protein kinase 1 |
| ADRM1 | Proteasomal ubiquitin receptor ADRM1 | Proteasomal ubiquitin receptor ADRM1 |
| CDC20 | Cell division cycle protein 20 homolog | Cell division cycle protein 20 homolog |
| BUB1B | Mitotic checkpoint serine/threonine-protein kinase BUB1 beta | Mitotic checkpoint serine/threonine-protein kinase BUB 1 beta |
| NTRK2 | BDNF/NT-3 growth factors receptor | BDNF/NT-3 growth factors receptor |
| NTRK1 | High affinity nerve growth factor receptor | High affinity nerve growth factor receptor |
| CD274 | Programmed cell death 1 ligand 1 | Programmed cell death 1 ligand 1 |
| NUAK1 | NUAK family SNF1-like kinase 1 | NUAK family SNF1-like kinase 1 |
| PDCD1 | Programmed cell death protein 1 | Programmed cell death protein 1 |
| ERBB2 | Receptor tyrosine-protein kinase erbB-2 | Receptor tyrosine-protein kinase erbB-2 |
| EPHA3 | Ephrin type-A receptor 3 | Ephrin type-A receptor 3 |
| PIK3CG | Phosphatidylinositol 4,5-bisphosphate 3-kinase catalytic subunit gamma isoform | Phosphatidylinositol 4,5-bisphosphate 3-kinase catalytic subunit gamma isoform |
| MAP2K1 | Dual specificity mitogen-activated protein kinase kinase 1 | Dual specificity mitogen-activated protein kinase kinase 1 |
| LCK | Tyrosine-protein kinase Lck | Tyrosine-protein kinase Lck |
| MAP2K2 | Dual specificity mitogen-activated protein kinase kinase 2 | Dual specificity mitogen-activated protein kinase kinase 2 |
| JAK3 | Tyrosine-protein kinase JAK3 | Tyrosine-protein kinase JAK3 |
| GSK3B | Glycogen synthase kinase-3 beta | Glycogen synthase kinase-3 beta |
| JAK2 | Tyrosine-protein kinase JAK2 | Tyrosine-protein kinase JAK2 |
| PRKCI | Protein kinase C iota type | Protein kinase C iota type |
| FKBP1A | Peptidyl-prolyl cis-trans isomerase FKBP1A | Peptidyl-prolyl cis-trans isomerase FKBP1A |
| DHODH | Dihydroorotate dehydrogenase | Dihydroorotate dehydrogenase |
| PIK3CA | Phosphatidylinositol 4,5-bisphosphate 3-kinase catalytic subunit alpha isoform | Phosphatidylinositol 4,5-bisphosphate 3-kinase catalytic subunit alpha isoform |
| CDK6 | Cyclin-dependent kinase 6 | Cyclin-dependent kinase 6 |
| CDK4 | Cyclin-dependent kinase 4 | Cyclin-dependent kinase 4 |
| PDE4B | cAMP-specific 3',5'-cyclic phosphodiesterase 4B | cAMP-specific 3',5'-cyclic phosphodiesterase 4B |
| FYN | Tyrosine-protein kinase Fyn | Tyrosine-protein kinase Fyn |
| TYK2 | Non-receptor tyrosine-protein kinase TYK2 | Non-receptor tyrosine-protein kinase TYK2 |
| PDK1 | Pyruvate dehydrogenase 1 | Pyruvate dehydrogenase 1 |
| PDK2 | Pyruvate dehydrogenase 2 | Pyruvate dehydrogenase 2 |
| PDK3 | Pyruvate dehydrogenase 3 | Pyruvate dehydrogenase 3 |
| YES1 | Tyrosine-protein kinase Yes | Tyrosine-protein kinase Yes |

(continued)

| abbreviation of target protein | target protein's name | Chinese translation of target protein's name |
|---|---|---|
| GSK3A | Glycogen synthase kinase-3 alpha | Glycogen synthase kinase-3 alpha |
| CDK16 | Cyclin-dependent kinase 16 | Cyclin-dependent kinase 16 |
| CSNK2A1 | Casein kinase II subunit alpha | Casein kinase II subunit alpha |
| CDK7 | Cyclin-dependent kinase 7 | Cyclin-dependent kinase 7 |
| PTK2B | Protein-tyrosine kinase 2-beta | Protein-tyrosine kinase 2-beta |
| MAPK10 | Mitogen-activated protein kinase 10 | Mitogen-activated protein kinase 10 |
| MAPK9 | Mitogen-activated protein kinase 9 | Mitogen-activated protein kinase 9 |
| MAPK8 | Mitogen-activated protein kinase 8 | Mitogen-activated protein kinase 8 |
| CDK5 | Cyclin-dependent-like kinase 5 | Cyclin-dependent-like kinase 5 |
| METAP2 | Methionine aminopeptidase 2 | Methionine aminopeptidase 2 |
| BRD2 | Bromodomain-containing protein 2 | Bromodomain-containing protein 2 |
| MAPK3 | Mitogen-activated protein kinase 3 | Mitogen-activated protein kinase 3 |
| TEC | Tyrosine-protein kinase Tec | Tyrosine-protein kinase Tec |
| CDK14 | Cyclin-dependent kinase 14 | Cyclin-dependent kinase 14 |
| CDK17 | Cyclin-dependent kinase 17 | Cyclin-dependent kinase 17 |
| MAP3K11 | Mitogen-activated protein kinase kinase kinase 11 | Mitogen-activated protein kinase kinase kinase 11 |
| RPS6KB1 | Ribosomal protein S6 kinase beta-1 | Ribosomal protein S6 kinase beta-1 |
| CSK | Tyrosine-protein kinase CSK | Tyrosine-protein kinase CSK |
| MERTK | Tyrosine-protein kinase Mer | Tyrosine-protein kinase Mer |
| STK17B | Serine/threonine-protein kinase 17B | Serine/threonine-protein kinase 17B |
| CSNK2A2 | Casein kinase II subunit alpha' | Casein kinase II subunit alpha' |
| RPS6KA1 | Ribosomal protein S6 kinase alpha-1 | Ribosomal protein S6 kinase alpha-1 |
| MAPK13 | Mitogen-activated protein kinase 13 | Mitogen-activated protein kinase 13 |
| GAK | Cyclin-G-associated kinase | Cyclin-G-associated kinase |
| CLK1 | Dual specificity protein kinase CLK1 | Dual specificity protein kinase CLK1 |
| STK4 | Serine/threonine-protein kinase 4 | Serine/threonine-protein kinase 4 |
| EIF4E | Eukaryotic translation initiation factor 4E | Eukaryotic translation initiation factor 4E |
| STK10 | Serine/threonine-protein kinase 10 | Serine/threonine-protein kinase 10 |
| LRRK2 | Leucine-rich repeat serine/threonine-protein kinase 2 | Leucine-rich repeat serine/threonine-protein kinase 2 |
| TAOK3 | Serine/threonine-protein kinase TAO3 | Serine/threonine-protein kinase TAO3 |
| MARK2 | Serine/threonine-protein kinase MARK2 | Serine/threonine-protein kinase MARK2 |
| CSNK1D | Casein kinase I isoform delta | Casein kinase I isoform delta |
| AAK1 | AP2-associated protein kinase 1 | AP2-associated protein kinase 1 |
| IRAK3 | Interleukin-1 receptor-associated kinase 3 | Interleukin-1 receptor-associated kinase 3 |

(continued)

| abbreviation of target protein | target protein's name | Chinese translation of target protein's name |
|---|---|---|
| STAT3 | Signal transducer and activator of transcription 3 | Signal transducer and activator of transcription 3 |
| CAMKK1 | Calcium/calmodulin-dependent protein kinase kinase 1 | Calcium/calmodulin-dependent protein kinase kinase 1 |
| EED | Polycomb protein EED | Polycomb protein EED |
| CSNK1A1 | Casein kinase I isoform alpha | Casein kinase I isoform alpha |
| NEK1 | Serine/threonine-protein kinase Nek1 | Serine/threonine-protein kinase Nek1 |
| BMP2K | BMP-2-inducible protein kinase | BMP-2-inducible protein kinase |
| MAPK7 | Mitogen-activated protein kinase 7 | Mitogen-activated protein kinase 7 |
| ULK1 | Serine/threonine-protein kinase ULK1 | Serine/threonine-protein kinase ULK1 |
| RPS6KA3 | Ribosomal protein S6 kinase alpha-3 | Ribosomal protein S6 kinase alpha-3 |
| PTPN11 | Tyrosine-protein phosphatase non-receptor type 11 | Tyrosine-protein phosphatase non-receptor type 11 |
| LIMK2 | LIM domain kinase 2 | LIM domain kinase 2 |
| CSNK1E | Casein kinase I isoform epsilon | Casein kinase I isoform epsilon |
| EIF2AK4 | eIF-2-alpha kinase GCN2 | eIF-2-alpha kinase GCN2 |
| MAP2K5 | Dual specificity mitogen-activated protein kinase kinase 5 | Dual specificity mitogen-activated protein kinase kinase 5 |
| MAP4K3 | Mitogen-activated protein kinase kinase kinase kinase 3 | Mitogen-activated protein kinase kinase kinase kinase 3 |
| VHL | von Hippel-Lindau disease tumor suppressor | von Hippel-Lindau disease tumor suppressor |
| MARK3 | MAP/microtubule affinity-regulating kinase 3 | MAP/microtubule affinity-regulating kinase 3 |
| TAOK2 | Serine/threonine-protein kinase TAO2 | Serine/threonine-protein kinase TAO2 |
| MAP4K5 | Mitogen-activated protein kinase kinase kinase kinase 5 | Mitogen-activated protein kinase kinase kinase kinase 5 |
| SNRK | SNF-related serine/threonine-protein kinase | SNF-related serine/threonine-protein kinase |
| EEF2K | Eukaryotic elongation factor 2 kinase | Eukaryotic elongation factor 2 kinase |
| SGK3 | Serine/threonine-protein kinase Sgk3 | Serine/threonine-protein kinase Sgk3 |
| AHR | Aryl hydrocarbon receptor | Aryl hydrocarbon receptor |
| NEK4 | Serine/threonine-protein kinase Nek4 | Serine/threonine-protein kinase Nek4 |
| NEK9 | Serine/threonine-protein kinase Nek9 | Serine/threonine-protein kinase Nek9 |
| CIT | Citron Rho-interacting kinase | Citron Rho-interacting kinase |
| LATS1 | Serine/threonine-protein kinase LATS1 | Serine/threonine-protein kinase LATS1 |
| MINK1 | Misshapen-like kinase 1 | Misshapen-like kinase 1 |
| SIK3 | Serine/threonine-protein kinase SIK3 | Serine/threonine-protein kinase SIK3 |
| RPS6KA4 | Ribosomal protein S6 kinase alpha-4 | Ribosomal protein S6 kinase alpha-4 |
| NEK3 | Serine/threonine-protein kinase Nek3 | Serine/threonine-protein kinase Nek3 |
| SIK2 | Serine/threonine-protein kinase SIK2 | Serine/threonine-protein kinase SIK2 |

(continued)

| abbreviation of target protein | target protein's name | Chinese translation of target protein's name |
|---|---|---|
| MAST3 | Microtubule-associated serine/threonine-protein kinase 3 | Microtubule-associated serine/threonine-protein kinase 3 |
| STK32C | Serine/threonine-protein kinase 32C | Serine/threonine-protein kinase 32C |
| ALK | ALK tyrosine kinase receptor | ALK tyrosine kinase receptor |
| EPHB4 | Ephrin type-B receptor 4 | Ephrin type-B receptor 4 |
| PARP2 | Poly [ADP-ribose] polymerase 2 | Poly [ADP-ribose] polymerase 2 |
| PTK6 | Protein-tyrosine kinase 6 | Protein-tyrosine kinase 6 |
| PARP3 | Protein mono-ADP-ribosyltransferase PARP3 | Protein mono-ADP-ribosyltransferase PARP3 |
| CDK2 | Cyclin-dependent kinase 2 | Cyclin-dependent kinase 2 |
| CDK8 | Cyclin-dependent kinase 8 | Cyclin-dependent kinase 8 |
| BRDT | Bromodomain testis-specific protein | Bromodomain testis-specific protein |
| MAPKAPK5 | MAP kinase-activated protein kinase 5 | MAP kinase-activated protein kinase 5 |
| MAP3K1 | Mitogen-activated protein kinase kinase kinase 1 | Mitogen-activated protein kinase kinase kinase 1 |
| CDK18 | Cyclin-dependent kinase 18 | Cyclin-dependent kinase 18 |
| CDK10 | Cyclin-dependent kinase 10 | Cyclin-dependent kinase 10 |
| TTK | Dual specificity protein kinase TTK | Dual specificity protein kinase TTK |
| PIM2 | Serine/threonine-protein kinase pim-2 | Serine/threonine-protein kinase pim-2 |
| PKMYT1 | Membrane-associated tyrosine- and threonine-specific cdc2-inhibitory kinase | Membrane-associated tyrosine- and threonine-specific cdc2-inhibitory kinase |
| MKNK2 | MAP kinase-interacting serine/threonine-protein kinase 2 | MAP kinase-interacting serine/threonine-protein kinase 2 |
| KAT2B | Histone acetyltransferase KAT2B | Histone acetyltransferase KAT2B |
| NEK2 | Serine/threonine-protein kinase Nek2 | Serine/threonine-protein kinase Nek2 |
| HASPIN | Serine/threonine-protein kinase haspin | Serine/threonine-protein kinase haspin |
| PIR | Pirin | Pirin |
| CYP1B1 | Cytochrome P450 1B1 | Cytochrome P450 1B1 |
| ERN1 | Serine/threonine-protein kinase/endoribonuclease IRE1 | Serine/threonine-protein kinase/endoribonuclease IRE1 |
| MELK | Maternal embryonic leucine zipper kinase | Maternal embryonic leucine zipper kinase |
| COQ8A | Atypical kinase COQ8A, mitochondrial | Atypical kinase COQ8A, mitochondrial |
| RIOK2 | Serine/threonine-protein kinase RIO2 | Serine/threonine-protein kinase RIO2 |
| RPS6KA6 | Ribosomal protein S6 kinase alpha-6 | Ribosomal protein S6 kinase alpha-6 |
| MAP3K20 | Mitogen-activated protein kinase kinase kinase 20 | Mitogen-activated protein kinase kinase kinase 20 |
| MAPKAPK3 | MAP kinase-activated protein kinase 3 | MAP kinase-activated protein kinase 3 |
| ULK3 | Serine/threonine-protein kinase ULK3 | Serine/threonine-protein kinase ULK3 |

(continued)

| abbreviation of target protein | target protein's name | Chinese translation of target protein's name |
|---|---|---|
| MAP3K21 | Mitogen-activated protein kinase kinase kinase 21 | Mitogen-activated protein kinase kinase kinase 21 |
| COQ8B | Atypical kinase COQ8B, mitochondrial | Atypical kinase COQ8B, mitochondrial |
| TNK1 | Non-receptor tyrosine-protein kinase TNK1 | Non-receptor tyrosine-protein kinase TNK1 |
| BMPR1A | Bone morphogenetic protein receptor type-1A | Bone morphogenetic protein receptor type-1A |
| STK17A | Serine/threonine-protein kinase 17A | Serine/threonine-protein kinase 17A |
| CDK11A | Cyclin-dependent kinase 11A | Cyclin-dependent kinase 11A |
| TESK2 | Dual specificity testis-specific protein kinase 2 | Dual specificity testis-specific protein kinase 2 |
| NLK | Serine/threonine-protein kinase NLK | Serine/threonine-protein kinase NLK |
| STK35 | Serine/threonine-protein kinase 35 | Serine/threonine-protein kinase 35 |
| PKN3 | Serine/threonine-protein kinase N3 | Serine/threonine-protein kinase N3 |
| STK33 | Serine/threonine-protein kinase 33 | Serine/threonine-protein kinase 33 |
| SBK1 | Serine/threonine-protein kinase SBK1 | Serine/threonine-protein kinase SBK1 |
| SLC9A2 | Sodium/hydrogen exchanger 2 | Sodium/hydrogen exchanger 2 |
| CSNK2A3 | Casein kinase II subunit alpha 3 | Casein kinase II subunit alpha 3 |
| TACC3 | Transforming acidic coiled-coil-containing protein 3 | Transforming acidic coiled-coil-containing protein 3 |
| GSPT1 | Eukaryotic peptide chain release factor GTP-binding subunit ERF3A | Eukaryotic peptide chain release factor GTP-binding subunit ERF3A |
| SLC9A7 | Sodium/hydrogen exchanger 7 | Sodium/hydrogen exchanger 7 |
| RPS6KC1 | Ribosomal protein S6 kinase delta-1 | Ribosomal protein S6 kinase delta-1 |
| TBCK | TBC domain-containing protein kinase-like protein | TBC domain-containing protein kinase-like protein |
| CRABP1 | Cellular retinoic acid-binding protein 1 | Cellular retinoic acid-binding protein 1 |
| BCKDK | [3-methyl-2-oxobutanoate dehydrogenase [lipoamide]] kinase, mitochondrial | [3-methyl-2-oxobutanoate dehydrogenase [lipoamide]] kinase, mitochondrial |
| TRPM7 | Transient receptor potential cation channel subfamily M member 7 | Transient receptor potential cation channel subfamily M member 7 |
| TRIB3 | Tribbles homolog 3 | Tribbles homolog 3 |
| UHMK1 | Serine/threonine-protein kinase Kist | Serine/threonine-protein kinase Kist |
| PDIK1L | Serine/threonine-protein kinase PDIK1L | Serine/threonine-protein kinase PDIK1L |
| STK40 | Serine/threonine-protein kinase 40 | Serine/threonine-protein kinase 40 |
| SLC9A4 | Sodium/hydrogen exchanger 4 | Sodium/hydrogen exchanger 4 |
| EPHB3 | Ephrin type-B receptor 3 | Ephrin type-B receptor 3 |
| NTRK3 | NT-3 growth factor receptor | NT-3 growth factor receptor |
| MAP3K12 | Mitogen-activated protein kinase kinase kinase 12 | Mitogen-activated protein kinase kinase kinase 12 |
| MAPK11 | Mitogen-activated protein kinase 11 | Mitogen-activated protein kinase 11 |

(continued)

| abbreviation of target protein | target protein's name | Chinese translation of target protein's name |
|---|---|---|
| DDR2 | Discoidin domain-containing receptor 2 | Discoidin domain-containing receptor 2 |
| RARB | Retinoic acid receptor beta | Retinoic acid receptor beta |
| PDE4C | cAMP-specific 3',5'-cyclic phosphodiesterase 4C | cAMP-specific 3',5'-cyclic phosphodiesterase 4C |
| IDOI | Indoleamine 2,3-dioxygenase 1 | Indoleamine 2,3-dioxygenase 1 |
| SLC9B1 | Sodium/hydrogen exchanger 9B1 | Sodium/hydrogen exchanger 9B1 |
| PRAG1 | Inactive tyrosine-protein kinase PRAG1 | Inactive tyrosine-protein kinase PRAG1 |
| TOP1 | DNA topoisomerase 1 | DNA topoisomerase 1 |
| TOP2A | DNA topoisomerase 2-alpha | DNA topoisomerase 2-alpha |
| CXCR4 | C-X-C chemokine receptor type 4 | C-X-C chemokine receptor type 4 |
| ERBB4 | Receptor tyrosine-protein kinase erbB-4 | Receptor tyrosine-protein kinase erbB-4 |
| HCK | Tyrosine-protein kinase HCK | Tyrosine-protein kinase HCK |
| SYK | Tyrosine-protein kinase SYK | Tyrosine-protein kinase SYK |
| ACLY | ATP-citrate synthase | ATP-citrate synthase |
| IMPDH2 | Inosine-5'-monophosphate dehydrogenase 2 | Inosine-5'-monophosphate dehydrogenase 2 |
| CYP3A4 | Cytochrome P450 3A4 | Cytochrome P450 3A4 |
| TOP2B | DNA topoisomerase 2-beta | DNA topoisomerase 2-beta |
| KEAP1 | Kelch-like ECH-associated protein 1 | Kelch-like ECH-associated protein 1 |
| NR3C1 | Glucocorticoid receptor | Glucocorticoid receptor |
| DNMT1 | DNA (cytosine-5)-methyltransferase 1 | DNA (cytosine-5)-methyltransferase 1 |
| TXNRD1 | Thioredoxin reductase 1, cytoplasmic | Thioredoxin reductase 1, cytoplasmic |
| HDAC4 | Histone deacetylase 4 | Histone deacetylase 4 |
| PDGFRA | Platelet-derived growth factor receptor alpha | Platelet-derived growth factor receptor alpha |
| TUBB | Tubulin beta chain | Tubulin beta chain |
| TUBB2B | Tubulin beta-2B chain | Tubulin beta-2B chain |
| GABRA5 | Gamma-aminobutyric acid receptor subunit alpha-5 | Gamma-aminobutyric acid receptor subunit alpha-5 |
| EPHB1 | Ephrin type-B receptor 1 | Ephrin type-B receptor 1 |
| KCNQ2 | Potassium voltage-gated channel subfamily KQT member 2 | Potassium voltage-gated channel subfamily KQT member 2 |
| GRIK2 | Glutamate receptor ionotropic, kainate 2 | Glutamate receptor ionotropic, kainate 2 |
| CALM1 | Calmodulin-1 | Calmodulin-1 |
| HDAC5 | Histone deacetylase 5 | Histone deacetylase 5 |
| DNMT3A | DNA (cytosine-5)-methyltransferase 3A | DNA (cytosine-5)-methyltransferase 3A |
| KIF5B | Kinesin-1 heavy chain | Kinesin-1 heavy chain |
| POLD1 | DNA polymerase delta catalytic subunit | DNA polymerase delta catalytic subunit |
| CUL4A | Cullin-4A | Cullin-4A |

(continued)

| abbreviation of target protein | target protein's name | Chinese translation of target protein's name |
|---|---|---|
| TUBB2A | Tubulin beta-2A chain | Tubulin beta-2A chain |
| PDGFRB | Platelet-derived growth factor receptor beta | Platelet-derived growth factor receptor beta |
| FGR | Tyrosine-protein kinase Fgr | Tyrosine-protein kinase Fgr |
| PRKCG | Protein kinase C gamma type | Protein kinase C gamma type |
| SCN3A | Sodium channel protein type 3 subunit alpha | Sodium channel protein type 3 subunit alpha |
| HDAC9 | Histone deacetylase 9 | Histone deacetylase 9 |
| BACE1 | Beta-secretase 1 | Beta-secretase 1 |
| HSP90AA1 | Heat shock protein HSP 90-alpha | Heat shock protein HSP 90-alpha |
| HSP90AB1 | Heat shock protein HSP 90-beta | Heat shock protein HSP 90-beta |
| PDPK1 | 3-phosphoinositide-dependent protein kinase 1 | 3-phosphoinositide-dependent protein kinase 1 |
| BIRC2 | Baculoviral IAP repeat-containing protein 2 | Baculoviral IAP repeat-containing protein 2 |
| CHEK2 | Serine/threonine-protein kinase Chk2 | Serine/threonine-protein kinase Chk2 |
| MAP3K5 | Mitogen-activated protein kinase kinase kinase 5 | Mitogen-activated protein kinase kinase kinase 5 |
| CASP1 | Caspase-1 | Caspase-1 |
| NAE1 | NEDD8-activating enzyme E1 regulatory subunit | NEDD8-activating enzyme E1 regulatory subunit |
| PIK3R1 | Phosphatidylinositol 3-kinase regulatory subunit alpha | Phosphatidylinositol 3-kinase regulatory subunit alpha |
| IKBKB | Inhibitor of nuclear factor kappa-B kinase subunit beta | Inhibitor of nuclear factor kappa-B kinase subunit beta |
| PSMA1 | Proteasome subunit alpha type-1 | Proteasome subunit alpha type-1 |
| ITGB1 | Integrin beta-1 | Integrin beta-1 |
| PRKDC | DNA-dependent protein kinase catalytic subunit | DNA-dependent protein kinase catalytic subunit |
| PIK3R2 | Phosphatidylinositol 3-kinase regulatory subunit beta | Phosphatidylinositol 3-kinase regulatory subunit beta |
| FGF2 | Fibroblast growth factor 2 | Fibroblast growth factor 2 |
| ZAP70 | Tyrosine-protein kinase ZAP-70 | Tyrosine-protein kinase ZAP-70 |
| PIP4K2B | Phosphatidylinositol 5-phosphate 4-kinase type-2 beta | Phosphatidylinositol 5-phosphate 4-kinase type-2 beta |
| GAPDH | Glyceraldehyde-3-phosphate dehydrogenase | Glyceraldehyde-3 -phosphate dehydrogenase |
| FASN | Fatty acid synthase | Fatty acid synthase |
| PKM | Pyruvate kinase PKM | Pyruvate kinase PKM |
| VCP | Transitional endoplasmic reticulum ATPase | Transitional endoplasmic reticulum ATPase |
| KDM1A | Lysine-specific histone demethylase 1A | Lysine-specific histone demethylase 1A |
| TAB1 | TGF-beta-activated kinase 1 and MAP3K7-binding protein 1 | TGF-beta-activated kinase 1 and MAP3K7-binding protein 1 |

(continued)

| abbreviation of target protein | target protein's name | Chinese translation of target protein's name |
|---|---|---|
| EP300 | Histone acetyltransferase p300 | Histone acetyltransferase p300 |
| VRK1 | Serine/threonine-protein kinase VRK1 | Serine/threonine-protein kinase VRK1 |
| RPS6KA5 | Ribosomal protein S6 kinase alpha-5 | Ribosomal protein S6 kinase alpha-5 |
| UBA2 | SUMO-activating enzyme subunit 2 | SUMO-activating enzyme subunit 2 |
| LDHA | L-lactate dehydrogenase A chain | L-lactate dehydrogenase A chain |
| TKT | Transketolase | Transketolase |
| HPRT1 | Hypoxanthine-guanine phosphoribosyltransferase | Hypoxanthine-guanine phosphoribosyltransferase |
| KDM2A | Lysine-specific demethylase 2A | Lysine-specific demethylase 2A |
| EHMT1 | Histone-lysine N-methyltransferase EHMT1 | Histone-lysine N-methyltransferase EHMT1 |
| CAMK1D | Calcium/calmodulin-dependent protein kinase type 1D | Calcium/calmodulin-dependent protein kinase type 1D |
| WDR5 | WD repeat-containing protein 5 | WD repeat-containing protein 5 |
| EHMT2 | Histone-lysine N-methyltransferase EHMT2 | Histone-lysine N-methyltransferase EHMT2 |
| RAC1 | Ras-related C3 botulinum toxin substrate 1 | Ras-related C3 botulinum toxin substrate 1 |
| OGT | UDP-N-acetylglucosamine--peptide N-acetylglucosaminyltransferase 110 kDa subunit | UDP-N-acetylglucosamine--peptide N-acetylglucosaminyltransferase 110 kDa subunit |
| NCOA1 | Nuclear receptor coactivator 1 | Nuclear receptor coactivator 1 |
| PHGDH | D-3-phosphoglycerate dehydrogenase | D-3-phosphoglycerate dehydrogenase |
| ARHGDIA | Rho GDP-dissociation inhibitor 1 | Rho GDP-dissociation inhibitor 1 |
| RBBP4 | Histone-binding protein RBBP4 | Histone-binding protein RBBP4 |
| BPTF | Nucleosome-remodeling factor subunit BPTF | Nucleosome-remodeling factor subunit BPTF |
| KAT5 | Histone acetyltransferase KAT5 | Histone acetyltransferase KAT5 |
| NOTCH1 | Neurogenic locus notch homolog protein 1 | Neurogenic locus notch homolog protein 1 |
| PPIA | Peptidyl-prolyl cis-trans isomerase A | Peptidyl-prolyl cis-trans isomerase A |
| FKBP4 | Peptidyl-prolyl cis-trans isomerase FKBP4 | Peptidyl-prolyl cis-trans isomerase FKBP4 |
| CREBBP | CREB-binding protein | CREB-binding protein |
| POLB | DNA polymerase beta | DNA polymerase beta |
| APEX1 | DNA-(apurinic or apyrimidinic site) endonuclease | DNA-(apurinic or apyrimidinic site) endonuclease |
| CCNT1 | Cyclin-T1 | Cyclin-T 1 |
| EIF4A1 | Eukaryotic initiation factor 4A-I | Eukaryotic initiation factor 4A-I |
| USP7 | Ubiquitin carboxyl-terminal hydrolase 7 | Ubiquitin carboxyl-terminal hydrolase 7 |
| CTNNB1 | Catenin beta-1 | Catenin beta-1 |
| TXN | Thioredoxin | Thioredoxin |
| DDX3X | ATP-dependent RNA helicase DDX3X | ATP-dependent RNA helicase DDX3X |

(continued)

| abbreviation of target protein | target protein's name | Chinese translation of target protein's name |
|---|---|---|
| NLRP3 | NACHT, LRR and PYD domains-containing protein 3 | NACHT, LRR and PYD domains-containing protein 3 |
| TNKS | Poly [ADP-ribose] polymerase tankyrase-1 | Poly [ADP-ribose] polymerase tankyrase-1 |
| PAK3 | Serine/threonine-protein kinase PAK 3 | Serine/threonine-protein kinase PAK 3 |
| PKN1 | Serine/threonine-protein kinase N1 | Serine/threonine-protein kinase N1 |
| CISD1 | CDGSH iron-sulfur domain-containing protein 1 | CDGSH iron-sulfur domain-containing protein 1 |
| WNK1 | Serine/threonine-protein kinase WNK1 | Serine/threonine-protein kinase WNK1 |
| BRD1 | Bromodomain-containing protein 1 | Bromodomain-containing protein 1 |
| XIAP | E3 ubiquitin-protein ligase XIAP | E3 ubiquitin-protein ligase XIAP |
| PRDX1 | Peroxiredoxin-1 | Peroxiredoxin-1 |
| KMT2A | Histone-lysine N-methyltransferase 2A | Histone-lysine N-methyltransferase 2A |
| KDM5C | Lysine-specific demethylase 5C | Lysine-specific demethylase 5C |
| RPA1 | Replication protein A 70 kDa DNA-binding subunit | Replication protein A 70 kDa DNA-binding subunit |
| CAMK1 | Calcium/calmodulin-dependent protein kinase type 1 | Calcium/calmodulin-dependent protein kinase type 1 |
| EIF4A3 | Eukaryotic initiation factor 4A-III | Eukaryotic initiation factor 4A-III |
| TAF1 | Transcription initiation factor TFIID subunit 1 | Transcription initiation factor TFIID subunit 1 |
| ALKBH3 | Alpha-ketoglutarate-dependent dioxygenase alkB homolog 3 | Alpha-ketoglutarate-dependent dioxygenase alkB homolog 3 |
| MAP3K2 | Mitogen-activated protein kinase kinase kinase 2 | Mitogen-activated protein kinase kinase kinase 2 |
| RELA | Transcription factor p65 | Transcription factor p65 |
| NCOR2 | Nuclear receptor corepressor 2 | Nuclear receptor corepressor 2 |
| PRPF4B | Serine/threonine-protein kinase PRP4 homolog | Serine/threonine-protein kinase PRP4 homolog |
| RPS3 | 40S ribosomal protein S3 | 40S ribosomal protein S3 |
| RPSA | 40S ribosomal protein SA | 40S ribosomal protein SA |
| RPS27A | Ubiquitin-40S ribosomal protein S27a | Ubiquitin-40S ribosomal protein S27a |
| RPS20 | 40S ribosomal protein S20 | 40S ribosomal protein S20 |
| RPL18 | 60S ribosomal protein L18 | 60S ribosomal protein L18 |
| RPS5 | 40S ribosomal protein S5 | 40S ribosomal protein S5 |
| RPL6 | 60S ribosomal protein L6 | 60S ribosomal protein L6 |
| RPLP0 | 60S acidic ribosomal protein P0 | 60S acidic ribosomal protein P0 |
| RPL3 | 60S ribosomal protein L3 | 60S ribosomal protein L3 |
| RPL18A | 60S ribosomal protein L18a | 60S ribosomal protein L18a |
| RPL28 | 60S ribosomal protein L28 | 60S ribosomal protein L28 |

(continued)

| abbreviation of target protein | target protein's name | Chinese translation of target protein's name |
|---|---|---|
| RPL19 | 60S ribosomal protein L19 | 60S ribosomal protein L19 |
| RPL34 | 60S ribosomal protein L34 | 60S ribosomal protein L34 |
| RPS13 | 40S ribosomal protein S13 | 40S ribosomal protein S13 |
| RPL24 | 60S ribosomal protein L24 | 60S ribosomal protein L24 |
| RPS15 | 40S ribosomal protein S15 | 40S ribosomal protein S15 |
| RPL5 | 60S ribosomal protein L5 | 60S ribosomal protein L5 |
| RPS10 | 40S ribosomal protein S10 | 40S ribosomal protein S10 |
| RPL35 | 60S ribosomal protein L35 | 60S ribosomal protein L35 |
| RPS6 | 40S ribosomal protein S6 | 40S ribosomal protein S6 |
| RPS24 | 40S ribosomal protein S24 | 40S ribosomal protein S24 |
| RPS2 | 40S ribosomal protein S2 | 40S ribosomal protein S2 |
| RPL11 | 60S ribosomal protein L11 | 60S ribosomal protein L11 |
| RPS8 | 40S ribosomal protein S8 | 40S ribosomal protein S8 |
| RPL32 | 60S ribosomal protein L32 | 60S ribosomal protein L32 |
| RPS3A | 40S ribosomal protein S3a | 40S ribosomal protein S3a |
| RPL10 | 60S ribosomal protein L10 | 60S ribosomal protein L10 |
| RPL7A | 60S ribosomal protein L7a | 60S ribosomal protein L7a |
| RPL30 | 60S ribosomal protein L30 | 60S ribosomal protein L30 |
| RPL8 | 60S ribosomal protein L8 | 60S ribosomal protein L8 |
| RPL26 | 60S ribosomal protein L26 | 60S ribosomal protein L26 |
| RPS14 | 40S ribosomal protein S14 | 40S ribosomal protein S14 |
| RPL13 | 60S ribosomal protein L13 | 60S ribosomal protein L13 |
| RPS9 | 40S ribosomal protein S9 | 40S ribosomal protein S9 |
| RPS21 | 40S ribosomal protein S21 | 40S ribosomal protein S21 |
| RPS7 | 40S ribosomal protein S7 | 40S ribosomal protein S7 |
| RPL38 | 60S ribosomal protein L38 | 60S ribosomal protein L38 |
| RPL4 | 60S ribosomal protein L4 | 60S ribosomal protein L4 |
| RPL15 | 60S ribosomal protein L15 | 60S ribosomal protein L15 |
| RPS17 | 40S ribosomal protein S17 | 40S ribosomal protein S17 |
| RPS23 | 40S ribosomal protein S23 | 40S ribosomal protein S23 |
| RPL14 | 60S ribosomal protein L14 | 60S ribosomal protein L14 |
| RPL12 | 60S ribosomal protein L12 | 60S ribosomal protein L12 |
| RPS4X | 40S ribosomal protein S4, X isoform | 40S ribosomal protein S4, X isoform |
| RPL23A | 60S ribosomal protein L23a | 60S ribosomal protein L23a |
| RPL10A | 60S ribosomal protein L23a | 60S ribosomal protein L23a |
| RPS18 | 40S ribosomal protein S18 | 40S ribosomal protein S18 |

(continued)

| abbreviation of target protein | target protein's name | Chinese translation of target protein's name |
|---|---|---|
| SLC7A5 | Large neutral amino acids transporter small subunit 1 | Large neutral amino acids transporter small subunit 1 |
| ORAI1 | Calcium release-activated calcium channel protein 1 | Calcium release-activated calcium channel protein 1 |
| PI4K2A | Phosphatidylinositol 4-kinase type 2-alpha | Phosphatidylinositol 4-kinase type 2-alpha |
| SUMO1 | Small ubiquitin-related modifier 1 | Small ubiquitin-related modifier 1 |
| HSPA8 | Heat shock cognate 71 kDa protein | Heat shock cognate 71 kDa protein |
| HSPD1 | 60 kDa heat shock protein, mitochondrial | 60 kDa heat shock protein, mitochondrial |
| CSNK2B | Casein kinase II subunit beta | Casein kinase II subunit beta |
| PPP2CA | Serine/threonine-protein phosphatase 2A catalytic subunit alpha isoform | Serine/threonine-protein phosphatase 2A catalytic subunit alpha isoform |
| FSCN1 | Fascin | Fascin |
| BID | BH3-interacting domain death agonist | BH3-interacting domain death agonist |
| DCUN1D1 | DCN1-like protein 1 | DCN1-like protein 1 |
| USP28 | Ubiquitin carboxyl-terminal hydrolase 28 | Ubiquitin carboxyl-terminal hydrolase 28 |
| TP53BP1 | TP53-binding protein 1 | TP53-binding protein 1 |
| KDM5A | Lysine-specific demethylase 5A | Lysine-specific demethylase 5A |
| BAZ2A | Bromodomain adjacent to zinc finger domain protein 2A | Bromodomain adjacent to zinc finger domain protein 2A |
| UBE2I | SUMO-conjugating enzyme UBC9 | SUMO-conjugating enzyme UBC9 |
| SPIN1 | Spindlin-1 | Spindlin-1 |
| TDP2 | Tyrosyl-DNA phosphodiesterase 2 | Tyrosyl-DNA phosphodiesterase 2 |
| KDM5B | Lysine-specific demethylase 5B | Lysine-specific demethylase 5B |
| RBBP5 | Retinoblastoma-binding protein 5 | Retinoblastoma-binding protein 5 |
| PRMT1 | Protein arginine N-methyltransferase 1 | Protein arginine N-methyltransferase 1 |
| MLLT1 | Protein ENL | Protein ENL |
| SETDB1 | Histone-lysine N-methyltransferase SETDB1 | Histone-lysine N-methyltransferase SETDB1 |
| ARNT | Aryl hydrocarbon receptor nuclear translocator | Aryl hydrocarbon receptor nuclear translocator |
| SNRNP200 | U5 small nuclear ribonucleoprotein 200 kDa helicase | U5 small nuclear ribonucleoprotein 200 kDa helicase |
| ATAD2 | ATPase family AAA domain-containing protein 2 | ATPase family AAA domain-containing protein 2 |
| SQSTM1 | Sequestosome-1 | Sequestosome-1 |
| DPY30 | Protein dpy-30 homolog | Protein dpy-30 homolog |
| WRN | Werner syndrome ATP-dependent helicase | Werner syndrome ATP-dependent helicase |
| JMJD1C | Probable JmjC domain-containing histone demethylation protein 2C | Probable JmjC domain-containing histone demethylation protein 2C |
| MDM4 | Protein Mdm4 | Protein Mdm4 |

(continued)

| abbreviation of target protein | target protein's name | Chinese translation of target protein's name |
|---|---|---|
| L3MBTL3 | Lethal(3)malignant brain tumor-like protein 3 | Lethal(3)malignant brain tumor-like protein 3 |
| LDLR | Low-density lipoprotein receptor | Low-density lipoprotein receptor |
| GLUL | Glutamine synthetase | Glutamine synthetase |
| HRAS | GTPase HRas | GTPase HRas |
| APOBEC3G | DNA dC->dU-editing enzyme APOBEC-3G | DNA dC->dU-editing enzyme APOBEC-3G |
| TSG101 | Tumor susceptibility gene 101 protein | Tumor susceptibility gene 101 protein |
| LRP6 | Low-density lipoprotein receptor-related protein 6 | Low-density lipoprotein receptor-related protein 6 |
| ENO1 | Alpha-enolase | Alpha-enolase |
| ANXA2 | Annexin A2 | Annexin A2 |
| SOD1 | Superoxide dismutase [Cu-Zn] | Superoxide dismutase [Cu-Zn] |
| KAT6A | Histone acetyltransferase KAT6A | Histone acetyltransferase KAT6A |
| CBS | Cystathionine beta-synthase | Cystathionine beta-synthase |
| RUVBL1 | RuvB-like 1 | RuvB-like 1 |
| UHRF1 | E3 ubiquitin-protein ligase UHRF1 | E3 ubiquitin-protein ligase UHRF1 |
| HSPA5 | Endoplasmic reticulum chaperone BiP | Endoplasmic reticulum chaperone BiP |
| SQLE | Squalene monooxygenase | Squalene monooxygenase |
| NEDD4 | E3 ubiquitin-protein ligase NEDD4 | E3 ubiquitin-protein ligase NEDD4 |
| RBBP7 | Histone-binding protein RBBP7 | Histone-binding protein RBBP7 |
| UBE2N | Ubiquitin-conjugating enzyme E2 N | Ubiquitin-conjugating enzyme E2 N |
| NRAS | GTPase NRas | GTPase NRas |
| PTEN | Phosphatidylinositol 3,4,5-trisphosphate 3-phosphatase and dual-specificity protein phosphatase PTEN | Phosphatidylinositol 3,4,5-trisphosphate 3-phosphatase and dual-specificity protein phosphatase PTEN |
| RHOA | Transforming protein RhoA | Transforming protein RhoA |
| RNF31 | E3 ubiquitin-protein ligase RNF31 | E3 ubiquitin-protein ligase RNF31 |
| PIN4 | Peptidyl-prolyl cis-trans isomerase NIMA-interacting 4 | Peptidyl-prolyl cis-trans isomerase NIMA-interacting 4 |
| TPI1 | Triosephosphate isomerase | Triosephosphate isomerase |
| PPIG | Peptidyl-prolyl cis-trans isomerase G | Peptidyl-prolyl cis-trans isomerase G |
| NCOA2 | Nuclear receptor coactivator 2 | Nuclear receptor coactivator 2 |
| FOXO1 | Forkhead box protein O1 | Forkhead box protein O1 |
| PSIP1 | PC4 and SFRS1-interacting protein | PC4 and SFRS1-interacting protein |
| MACROD2 | ADP-ribose glycohydrolase MACROD2 | ADP-ribose glycohydrolase MACROD2 |
| SETD2 | Histone-lysine N-methyltransferase SETD2 | Histone-lysine N-methyltransferase SETD2 |
| NFKBIA | NF-kappa-B inhibitor alpha | NF-kappa-B inhibitor alpha |
| PLA2G4A | Cytosolic phospholipase A2 | Cytosolic phospholipase A2 |

(continued)

| abbreviation of target protein | target protein's name | Chinese translation of target protein's name |
|---|---|---|
| ICAM1 | Intercellular adhesion molecule 1 | Intercellular adhesion molecule 1 |
| ACVR1B | Activin receptor type-1B | Activin receptor type-1B |
| CYBB | Cytochrome b-245 heavy chain | Cytochrome b-245 heavy chain |
| STAMBP | STAM-binding protein | STAM-binding protein |
| NFKB1 | Nuclear factor NF-kappa-B p105 subunit | Nuclear factor NF-kappa-B p105 subunit |
| PAK2 | Serine/threonine-protein kinase PAK 2 | Serine/threonine-protein kinase PAK 2 |
| ITPR1 | Inositol 1,4,5-trisphosphate receptor type 1 | Inositol 1,4,5-trisphosphate receptor type 1 |
| ELAVL1 | ELAV-like protein 1 | ELAV-like protein 1 |
| NFKB2 | Nuclear factor NF-kappa-B p100 subunit | Nuclear factor NF-kappa-B p100 subunit |
| STAT1 | Signal transducer and activator of transcription 1-alpha/beta | Signal transducer and activator of transcription 1-alpha/beta |
| STAT5A | Signal transducer and activator of transcription 5A | Signal transducer and activator of transcription 5A |
| RIPK3 | Receptor-interacting serine/threonine-protein kinase 3 | Receptor-interacting serine/threonine-protein kinase 3 |
| BRSK2 | Serine/threonine-protein kinase BRSK2 | Serine/threonine-protein kinase BRSK2 |
| ERCC4 | DNA repair endonuclease XPF | DNA repair endonuclease XPF |
| NPM1 | Nucleophosmin | Nucleophosmin |
| IKBKG | NF-kappa-B essential modulator | NF-kappa-B essential modulator |
| PBK | Lymphokine-activated killer T-cell-originated protein kinase | Lymphokine-activated killer T-cell-originated protein kinase |
| CD22 | B-cell receptor CD22 | B-cell receptor CD22 |
| APC | Adenomatous polyposis coli protein | Adenomatous polyposis coli protein |
| PLCG1 | 1-phosphatidylinositol 4,5-bisphosphate phosphodiesterase gamma-1 | 1-phosphatidylinositol 4,5-bisphosphate phosphodiesterase gamma-1 |
| ING2 | Inhibitor of growth protein 2 | Inhibitor of growth protein 2 |
| ERCC1 | DNA excision repair protein ERCC-1 | DNA excision repair protein ERCC-1 |
| KDM2B | Lysine-specific demethylase 2B | Lysine-specific demethylase 2B |
| ETV6 | Transcription factor ETV6 | Transcription factor ETV6 |
| CBX4 | E3 SUMO-protein ligase CBX4 | E3 SUMO-protein ligase CBX4 |
| USP25 | Ubiquitin carboxyl-terminal hydrolase 25 | Ubiquitin carboxyl-terminal hydrolase 25 |
| HSF1 | Heat shock factor protein 1 | Heat shock factor protein 1 |
| TRIM33 | E3 ubiquitin-protein ligase TRIM33 | E3 ubiquitin-protein ligase TRIM33 |
| HNRNPA1 | Heterogeneous nuclear ribonucleoprotein A1 | Heterogeneous nuclear ribonucleoprotein A1 |
| TERF2 | Telomeric repeat-binding factor 2 | Telomeric repeat-binding factor 2 |
| PTBP1 | Polypyrimidine tract-binding protein 1 | Polypyrimidine tract-binding protein 1 |
| NR2C2 | Nuclear receptor subfamily 2 group C member 2 | Nuclear receptor subfamily 2 group C member 2 |

(continued)

| abbreviation of target protein | target protein's name | Chinese translation of target protein's name |
|---|---|---|
| NOTCH2 | Neurogenic locus notch homolog protein 2 | Neurogenic locus notch homolog protein 2 |
| USP8 | Ubiquitin carboxyl-terminal hydrolase 8 | Ubiquitin carboxyl-terminal hydrolase 8 |
| RGS12 | Regulator of G-protein signaling 12 | Regulator of G-protein signaling 12 |
| ATF1 | Cyclic AMP-dependent transcription factor ATF-1 | Cyclic AMP-dependent transcription factor ATF-1 |
| PPM1B | Protein phosphatase 1B | Protein phosphatase 1B |
| PDCD4 | Programmed cell death protein 4 | Programmed cell death protein 4 |
| LMNA | Prelamin-A/C | Prelamin-A/C |
| MITF | Microphthalmia-associated transcription factor | Microphthalmia-associated transcription factor |
| ADCY3 | Adenylate cyclase type 3 | Adenylate cyclase type 3 |
| EEF1A1 | Elongation factor 1-alpha 1 | Elongation factor 1-alpha 1 |
| MYH9 | Myosin-9 | Myosin-9 |
| BCL3 | B-cell lymphoma 3 protein | B-cell lymphoma 3 protein |
| XRCC5 | X-ray repair cross-complementing protein 5 | X-ray repair cross-complementing protein 5 |
| USP4 | Ubiquitin carboxyl-terminal hydrolase 4 | Ubiquitin carboxyl-terminal hydrolase 4 |
| AGL | Glycogen debranching enzyme | Glycogen debranching enzyme |
| XRCC6 | X-ray repair cross-complementing protein 6 | X-ray repair cross-complementing protein 6 |
| MAGI3 | Membrane-associated guanylate kinase, WW and PDZ domain-containing protein 3 | Membrane-associated guanylate kinase, WW and PDZ domain-containing protein 3 |
| DVL1 | Segment polarity protein dishevelled homolog DVL-1 | Segment polarity protein dishevelled homolog DVL-1 |
| VDAC2 | Voltage-dependent anion-selective channel protein 2 | Voltage-dependent anion-selective channel protein 2 |
| MDH1 | Malate dehydrogenase, cytoplasmic | Malate dehydrogenase, cytoplasmic |
| CBX5 | Chromobox protein homolog 5 | Chromobox protein homolog 5 |
| USP10 | Ubiquitin carboxyl-terminal hydrolase 10 | Ubiquitin carboxyl-terminal hydrolase 10 |
| CBX1 | Chromobox protein homolog 1 | Chromobox protein homolog 1 |
| BRD8 | Bromodomain-containing protein 8 | Bromodomain-containing protein 8 |
| TSNAX | Translin-associated protein X | Translin-associated protein X |
| CREB1 | Cyclic AMP-responsive element-binding protein 1 | Cyclic AMP-responsive element-binding protein 1 |
| RBCK1 | RanBP-type and C3HC4-type zinc finger-containing protein 1 | RanBP-type and C3HC4-type zinc finger-containing protein 1 |
| UBA1 | Ubiquitin-like modifier-activating enzyme 1 | Ubiquitin-like modifier-activating enzyme 1 |
| MACROD1 | ADP-ribose glycohydrolase MACROD1 | ADP-ribose glycohydrolase MACROD1 |
| PELP1 | Proline-, glutamic acid- and leucine-rich protein 1 | Proline-, glutamic acid- and leucine-rich protein 1 |
| BAP1 | Ubiquitin carboxyl-terminal hydrolase BAP 1 | Ubiquitin carboxyl-terminal hydrolase BAP 1 |

(continued)

| abbreviation of target protein | target protein's name | Chinese translation of target protein's name |
|---|---|---|
| TERF2IP | Telomeric repeat-binding factor 2-interacting protein 1 | Telomeric repeat-binding factor 2-interacting protein 1 |
| LARP7 | La-related protein 7 | La-related protein 7 |
| UBA7 | Ubiquitin-like modifier-activating enzyme 7 | Ubiquitin-like modifier-activating enzyme 7 |
| SUMO3 | Small ubiquitin-related modifier 3 | Small ubiquitin-related modifier 3 |
| SUMO2 | Small ubiquitin-related modifier 2 | Small ubiquitin-related modifier 2 |
| GIGYF2 | GRB10-interacting GYF protein 2 | GRB10-interacting GYF protein 2 |
| S100A10 | Protein S100-A10 | Protein S100-A10 |
| GRIK3 | Glutamate receptor ionotropic, kainate 3 | Glutamate receptor ionotropic, kainate 3 |
| TLR8 | Toll-like receptor 8 | Toll-like receptor 8 |
| DYRK2 | Dual specificity tyrosine-phosphorylation-regulated kinase 2 | Dual specificity tyrosine-phosphorylation-regulated kinase 2 |
| NOS1 | Nitric oxide synthase, brain | Nitric oxide synthase, brain |
| WNK3 | Serine/threonine-protein kinase WNK3 | Serine/threonine-protein kinase WNK3 |
| TLR2 | Toll-like receptor 2 | Toll-like receptor 2 |
| BCL2L11 | Bcl-2-like protein 11 | Bcl-2-like protein 11 |
| SLC11A2 | Natural resistance-associated macrophage protein 2 | Natural resistance-associated macrophage protein 2 |
| ABCB11 | Bile salt export pump | Bile salt export pump |
| ITGAL | Integrin alpha-L | Integrin alpha-L |
| ROCK1 | Rho-associated protein kinase 1 | Rho-associated protein kinase 1 |
| CA12 | Carbonic anhydrase 12 | Carbonic anhydrase 12 |
| EPHA7 | Ephrin type-A receptor 7 | Ephrin type-A receptor 7 |
| PRKCA | Protein kinase C alpha type | Protein kinase C alpha type |
| CA2 | Carbonic anhydrase 2 | Carbonic anhydrase 2 |
| PDE2A | cGMP-dependent 3',5'-cyclic phosphodiesterase | cGMP-dependent 3',5'-cyclic phosphodiesterase |
| EPHA4 | Ephrin type-A receptor 4 | Ephrin type-A receptor 4 |
| ACE | Angiotensin-converting enzyme | Angiotensin-converting enzyme |
| RAF1 | RAF proto-oncogene serine/threonine-protein kinase | RAF proto-oncogene serine/threonine-protein kinase |
| GLA | Alpha-galactosidase A | Alpha-galactosidase A |
| MAOB | Amine oxidase [flavin-containing] B | Amine oxidase [flavin-containing] B |
| AKR1B1 | Aldo-keto reductase family 1 member B1 | Aldo-keto reductase family 1 member B1 |
| GSR | Glutathione reductase, mitochondrial | Glutathione reductase, mitochondrial |
| UMPS | Uridine 5'-monophosphate synthase | Uridine 5'-monophosphate synthase |
| THRA | Thyroid hormone receptor alpha | Thyroid hormone receptor alpha |

(continued)

| abbreviation of target protein | target protein's name | Chinese translation of target protein's name |
|---|---|---|
| GART | Trifunctional purine biosynthetic protein adenosine-3 [Includes: Phosphoribosylamine--glycine ligase | Trifunctional purine biosynthetic protein adenosine-3 [Includes: Phosphoribosylamine--glycine ligase |
| FDPS | Farnesyl pyrophosphate synthase | Farnesyl pyrophosphate synthase |
| ADH5 | Alcohol dehydrogenase class-3 | Alcohol dehydrogenase class-3 |
| RXRB | Retinoic acid receptor RXR-beta | Retinoic acid receptor RXR-beta |
| DHFR | Dihydrofolate reductase | Dihydrofolate reductase |
| PDE10A | cAMP and cAMP-inhibited cGMP 3',5'-cyclic phosphodiesterase 10A | cAMP and cAMP-inhibited cGMP 3',5'-cyclic phosphodiesterase 10A |
| IDH1 | Isocitrate dehydrogenase [NADP] cytoplasmic | Isocitrate dehydrogenase [NADP] cytoplasmic |
| ITGB2 | Integrin beta-2 | Integrin beta-2 |
| COMT | Catechol O-methyltransferase | Catechol O-methyltransferase |
| PRKCQ | Protein kinase C theta type | Protein kinase C theta type |
| IMPDH1 | Inosine-5'-monophosphate dehydrogenase 1 | Inosine-5'-monophosphate dehydrogenase 1 |
| HDAC7 | Histone deacetylase 7 | Histone deacetylase 7 |
| TUBB3 | Tubulin beta-3 chain | Tubulin beta-3 chain |
| TUBA1A | Tubulin alpha-1A chain | Tubulin alpha-1A chain |
| ADA | Adenosine deaminase | Adenosine deaminase |
| GABRG2 | Gamma-aminobutyric acid receptor subunit gamma-2 | Gamma-aminobutyric acid receptor subunit gamma-2 |
| GABBR2 | Gamma-aminobutyric acid type B receptor subunit 2 | Gamma-aminobutyric acid type B receptor subunit 2 |
| GABRB2 | Gamma-aminobutyric acid receptor subunit beta-2 | Gamma-aminobutyric acid receptor subunit beta-2 |
| GABRB3 | Gamma-aminobutyric acid receptor subunit beta-3 | Gamma-aminobutyric acid receptor subunit beta-3 |
| GABBR1 | Gamma-aminobutyric acid type B receptor subunit 1 | Gamma-aminobutyric acid type B receptor subunit 1 |
| PRKCE | Protein kinase C epsilon type | Protein kinase C epsilon type |
| SIGMAR1 | Sigma non-opioid intracellular receptor 1 | Sigma non-opioid intracellular receptor 1 |
| MTOR | Serine/threonine-protein kinase mTOR | Serine/threonine-protein kinase mTOR |
| SLC29A1 | Equilibrative nucleoside transporter 1 | Equilibrative nucleoside transporter 1 |
| KCNJ11 | ATP-sensitive inward rectifier potassium channel 11 | ATP-sensitive inward rectifier potassium channel 11 |
| GAA | Lysosomal alpha-glucosidase | Lysosomal alpha-glucosidase |
| PRKCH | Protein kinase C eta type | Protein kinase C eta type |
| PIK3CD | Phosphatidylinositol 4,5-bisphosphate 3-kinase catalytic subunit delta isoform | Phosphatidylinositol 4,5-bisphosphate 3-kinase catalytic subunit delta isoform |
| ROCK2 | Rho-associated protein kinase 2 | Rho-associated protein kinase 2 |

(continued)

| abbreviation of target protein | target protein's name | Chinese translation of target protein's name |
|---|---|---|
| MAOA | Amine oxidase [flavin-containing] A | Amine oxidase [flavin-containing] A |
| SOAT1 | Sterol O-acyltransferase 1 | Sterol O-acyltransferase 1 |
| BCR | Breakpoint cluster region protein | Breakpoint cluster region protein |
| PSMBS | Proteasome subunit beta type-5 | Proteasome subunit beta type-5 |
| XPO1 | Exportin-1 | Exportin-1 |
| MALT1 | Mucosa-associated lymphoid tissue lymphoma translocation protein 1 | Mucosa-associated lymphoid tissue lymphoma translocation protein 1 |
| IDH2 | Isocitrate dehydrogenase [NADP], mitochondrial | Isocitrate dehydrogenase [NADP], mitochondrial |
| RRM1 | Ribonucleoside-diphosphate reductase large subunit | Ribonucleoside-diphosphate reductase large subunit |
| NDUFA10 | NADH dehydrogenase [ubiquinone] 1 alpha subcomplex subunit 10, mitochondrial | NADH dehydrogenase [ubiquinone] 1 alpha subcomplex subunit 10, mitochondrial |
| EPHA5 | Ephrin type-A receptor 5 | Ephrin type-A receptor 5 |
| TUBB4B | Tubulin beta-4B chain | Tubulin beta-4B chain |
| TUBB1 | Tubulin beta-1 chain | Tubulin beta-1 chain |
| TUBB4A | Tubulin beta-4A chain | Tubulin beta-4A chain |
| TUBB6 | Tubulin beta-6 chain | Tubulin beta-6 chain |
| TUBA4A | Tubulin alpha-4A chain | Tubulin alpha-4A chain |
| TUBA1C | Tubulin alpha-1C chain | Tubulin alpha-1C chain |
| CACNB1 | Voltage-dependent L-type calcium channel subunit beta-1 | Voltage-dependent L-type calcium channel subunit beta-1 |
| ATP1A2 | Sodium/potassium-transporting ATPase subunit alpha-2 | Sodium/potassium-transporting ATPase subunit alpha-2 |
| ATP1A3 | Sodium/potassium-transporting ATPase subunit alpha-3 | Sodium/potassium-transporting ATPase subunit alpha-3 |
| GRIA1 | Glutamate receptor 1 | Glutamate receptor 1 |
| GABRB1 | Gamma-aminobutyric acid receptor subunit beta-1 | Gamma-aminobutyric acid receptor subunit beta-1 |
| ATP1A1 | Sodium/potassium-transporting ATPase subunit alpha-1 | Sodium/potassium-transporting ATPase subunit alpha-1 |
| KCNQ5 | Potassium voltage-gated channel subfamily KQT member 5 | Potassium voltage-gated channel subfamily KQT member 5 |
| PRKCD | Protein kinase C delta type | Protein kinase C delta type |
| CACNA1H | Voltage-dependent T-type calcium channel subunit alpha-1H | Voltage-dependent T-type calcium channel subunit alpha-1H |
| GRIA4 | Glutamate receptor 4 | Glutamate receptor 4 |
| KCNA3 | Potassium voltage-gated channel subfamily A member 3 | Potassium voltage-gated channel subfamily A member 3 |
| S1PR3 | Sphingosine 1-phosphate receptor 3 | Sphingosine 1-phosphate receptor 3 |

(continued)

| abbreviation of target protein | target protein's name | Chinese translation of target protein's name |
|---|---|---|
| NISCH | Nischarin | Nischarin |
| PRKCB | Protein kinase C beta type | Protein kinase C beta type |
| TLR7 | Toll-like receptor 7 | Toll-like receptor 7 |
| CACNA1B | Voltage-dependent N-type calcium channel | Voltage-dependent N-type calcium |
| | subunit alpha-1B | channel subunit alpha-1B |
| CACNA2D2 | Voltage-dependent calcium channel subunit alpha-2/delta-2 | Voltage-dependent calcium channel subunit alpha-2/delta-2 |
| CPT2 | Carnitine O-palmitoyltransferase 2, mitochondrial | Carnitine O-palmitoyltransferase 2, mitochondrial |
| FAAH | Fatty-acid amide hydrolase 1 | Fatty-acid amide hydrolase 1 |
| UGCG | Ceramide glucosyltransferase | Ceramide glucosyltransferase |
| PDE1A | Calcium/calmodulin-dependent 3',5 '-cyclic nucleotide phosphodiesterase 1A | Calcium/calmodulin-dependent 3 ',5'-cyclic nucleotide phosphodiesterase 1A |
| HPD | 4-hydroxyphenylpyruvate dioxygenase | 4-hydroxyphenylpyruvate dioxygenase |
| CA5B | Carbonic anhydrase 5B, mitochondrial | Carbonic anhydrase 5B, mitochondrial |
| CASA | Carbonic anhydrase 5A, mitochondrial | Carbonic anhydrase 5A, mitochondrial |
| ANO1 | Anoctamin-1 | Anoctamin-1 |
| DHCR24 | Delta(24)-sterol reductase | Delta(24)-sterol reductase |
| CCDC6 | Coiled-coil domain-containing protein 6 | Coiled-coil domain-containing protein 6 |
| EML4 | Echinoderm microtubule-associated protein-like 4 | Echinoderm microtubule-associated protein-like 4 |
| KCNA2 | Potassium voltage-gated channel subfamily A member 2 | Potassium voltage-gated channel subfamily A member 2 |
| KCNB2 | Potassium voltage-gated channel subfamily B member 2 | Potassium voltage-gated channel subfamily B member 2 |
| PLA2G7 | Platelet-activating factor acetylhydrolase | Platelet-activating factor acetylhydrolase |
| GRMS | Metabotropic glutamate receptor 5 | Metabotropic glutamate receptor 5 |
| PLA2G2A | Phospholipase A2, membrane associated | Phospholipase A2, membrane associated |
| MIF | Macrophage migration inhibitory factor | Macrophage migration inhibitory factor |
| GBA | Lysosomal acid glucosylceramidase | Lysosomal acid glucosylceramidase |
| INSR | Insulin receptor | Insulin receptor |
| GSTP1 | Glutathione S-transferase P | Glutathione S-transferase P |
| PNP | Purine nucleoside phosphorylase | Purine nucleoside phosphorylase |
| FDFT1 | Squalene synthase | Squalene synthase |
| CASP8 | Caspase-8 | Caspase-8 |
| CASP9 | Caspase-9 | Caspase-9 |
| CASP6 | Caspase-6 | Caspase-6 |
| CASP3 | Caspase-3 | Caspase-3 |

(continued)

| abbreviation of target protein | target protein’s name | Chinese translation of target protein’s name |
|---|---|---|
| CASP7 | Caspase-7 | Caspase-7 |
| ERAP1 | Endoplasmic reticulum aminopeptidase 1 | Endoplasmic reticulum aminopeptidase 1 |
| UBA3 | NEDD8-activating enzyme E1 catalytic subunit | NEDD8-activating enzyme E1 catalytic subunit |
| PSEN1 | Presenilin-1 | Presenilin-1 |
| FNTA | Protein farnesyltransferase/geranylgeranyltransferase type-1 subunit alpha | Protein farnesyltransferase/geranylgeranyltransferase type-1 subunit alpha |
| METAP1 | Methionine aminopeptidase 1 | Methionine aminopeptidase 1 |
| CASP2 | Caspase-2 | Caspase-2 |
| ITGA4 | Integrin alpha-4 | Integrin alpha-4 |
| ABCB1 | ATP-dependent translocase ABCB1 | ATP-dependent translocase ABCB1 |
| NTSR1 | Neurotensin receptor type 1 | Neurotensin receptor type 1 |
| GRM3 | Metabotropic glutamate receptor 3 | Metabotropic glutamate receptor 3 |
| LNPEP | Leucyl-cystinyl aminopeptidase | Leucyl-cystinyl aminopeptidase |
| APH1A | Gamma-secretase subunit APH-1A | Gamma-secretase subunit APH-1A |
| NCSTN | Nicastrin | Nicastrin |
| PSMB1 | Proteasome subunit beta type-1 | Proteasome subunit beta type-1 |
| PSMA2 | Proteasome subunit alpha type-2 | Proteasome subunit alpha type-2 |
| PSMB7 | Proteasome subunit beta type-7 | Proteasome subunit beta type-7 |
| PSMA5 | Proteasome subunit alpha type-5 | Proteasome subunit alpha type-5 |
| PSMA4 | Proteasome subunit alpha type-4 | Proteasome subunit alpha type-4 |
| PSMA3 | Proteasome subunit alpha type-3 | Proteasome subunit alpha type-3 |
| PSMA6 | Proteasome subunit alpha type-6 | Proteasome subunit alpha type-6 |
| PSMA7 | Proteasome subunit alpha type-7 | Proteasome subunit alpha type-7 |
| PSMB2 | Proteasome subunit beta type-2 | Proteasome subunit beta type-2 |
| EGLN1 | Egl nine homolog 1 | Egl nine homolog 1 |
| PSMB6 | Proteasome subunit beta type-6 | Proteasome subunit beta type-6 |
| PSMB4 | Proteasome subunit beta type-4 | Proteasome subunit beta type-4 |
| PSMB8 | Proteasome subunit beta type-8 | Proteasome subunit beta type-8 |
| PSMB10 | Proteasome subunit beta type-10 | Proteasome subunit beta type-10 |
| PSMB9 | Proteasome subunit beta type-9 | Proteasome subunit beta type-9 |
| FNTB | Protein farnesyltransferase subunit beta | Protein farnesyltransferase subunit beta |
| PSMB3 | Proteasome subunit beta type-3 | Proteasome subunit beta type-3 |
| ERAP2 | Endoplasmic reticulum aminopeptidase 2 | Endoplasmic reticulum aminopeptidase 2 |
| DNPEP | Aspartyl aminopeptidase | Aspartyl aminopeptidase |
| STS | Steryl-sulfatase | Steryl-sulfatase |
| SELL | L-selectin | L-selectin |

(continued)

| abbreviation of target protein | target protein's name | Chinese translation of target protein's name |
|---|---|---|
| CCR1 | C-C chemokine receptor type 1 | C-C chemokine receptor type 1 |
| PIK3CB | Phosphatidylinositol 4,5-bisphosphate 3-kinase catalytic subunit beta isoform | Phosphatidylinositol 4,5-bisphosphate 3-kinase catalytic subunit beta isoform |
| PORCN | Protein-serine O-palmitoleoyltransferase porcupine | Protein-serine O-palmitoleoyltransferase porcupine |
| P4HTM | Transmembrane prolyl 4-hydroxylase | Transmembrane prolyl 4-hydroxylase |
| ATR | Serine/threonine-protein kinase ATR | Serine/threonine-protein kinase ATR |
| CDK19 | Cyclin-dependent kinase 19 | Cyclin-dependent kinase 19 |
| CDK3 | Cyclin-dependent kinase 3 | Cyclin-dependent kinase 3 |
| CASP4 | Caspase-4 | Caspase-4 |
| PIK3R5 | Phosphoinositide 3-kinase regulatory subunit 5 | Phosphoinositide 3-kinase regulatory subunit 5 |
| CASP10 | Caspase-10 | Caspase-10 |
| NPEPPS | Puromycin-sensitive aminopeptidase | Puromycin-sensitive aminopeptidase |
| RNPEP | Aminopeptidase B | Aminopeptidase B |
| LAP3 | Cytosol aminopeptidase | Cytosol aminopeptidase |
| CDK20 | Cyclin-dependent kinase 20 | Cyclin-dependent kinase 20 |
| DPYD | Dihydropyrimidine dehydrogenase [NADP(+)] | Dihydropyrimidine dehydrogenase [NADP(+)] |
| XPNPEP1 | Xaa-Pro aminopeptidase 1 | Xaa-Pro aminopeptidase 1 |
| NAMPT | Nicotinamide phosphoribosyltransferase | Nicotinamide phosphoribosyltransferase |
| PRKACA | cAMP-dependent protein kinase catalytic subunit alpha | cAMP-dependent protein kinase catalytic subunit alpha |
| PSMD14 | 26S proteasome non-ATPase regulatory subunit 14 | 26S proteasome non-ATPase regulatory subunit 14 |
| ARAF | Serine/threonine-protein kinase A-Raf | Serine/threonine-protein kinase A-Raf |
| PRKACB | cAMP-dependent protein kinase catalytic subunit beta | cAMP-dependent protein kinase catalytic subunit beta |
| ITGAV | Integrin alpha-V | Integrin alpha-V |
| TGM2 | Protein-glutamine gamma-glutamyltransferase 2 | Protein-glutamine gamma-glutamyltransferase 2 |
| ACE2 | Angiotensin-converting enzyme 2 | Angiotensin-converting enzyme 2 |
| PAK1 | Serine/threonine-protein kinase PAK 1 | Serine/threonine-protein kinase PAK 1 |
| CAPN1 | Calpain-1 catalytic subunit | Calpain-1 catalytic subunit |
| CDC42 | Cell division control protein 42 homolog | Cell division control protein 42 homolog |
| NMT1 | Glycylpeptide N-tetradecanoyltransferase 1 | Glycylpeptide N-tetradecanoyltransferase 1 |
| CAMK2D | Calcium/calmodulin-dependent protein kinase type II subunit delta | Calcium/calmodulin-dependent protein kinase type II subunit delta |
| AKR1B10 | Aldo-keto reductase family 1 member B10 | Aldo-keto reductase family 1 member B10 |
| P4HB | Protein disulfide-isomerase | Protein disulfide-isomerase |

(continued)

| abbreviation of target protein | target protein's name | Chinese translation of target protein's name |
|---|---|---|
| CDC42BPB | Serine/threonine-protein kinase MRCK beta | Serine/threonine-protein kinase MRCK beta |
| CAMK2G | Calcium/calmodulin-dependent protein kinase type II subunit gamma | Calcium/calmodulin-dependent protein kinase type II subunit gamma |
| PTP4A1 | Protein tyrosine phosphatase type IVA 1 | Protein tyrosine phosphatase type IVA 1 |
| G6PD | Glucose-6-phosphate 1-dehydrogenase | Glucose-6-phosphate 1-dehydrogenase |
| LSS | Lanosterol synthase | Lanosterol synthase |
| ALB | Albumin | Albumin |
| ECE1 | Endothelin-converting enzyme 1 | Endothelin-converting enzyme 1 |
| CTSD | Cathepsin D | Cathepsin D |
| CTSB | Cathepsin B | Cathepsin B |
| ILK | Integrin-linked protein kinase | Integrin-linked protein kinase |
| MGLL | Monoglyceride lipase | Monoglyceride lipase |
| CASK | Peripheral plasma membrane protein CASK | Peripheral plasma membrane protein CASK |
| ADAM17 | Disintegrin and metalloproteinase domain-containing protein 17 | Disintegrin and metalloproteinase domain-containing protein 17 |
| PYGL | Glycogen phosphorylase, liver form | Glycogen phosphorylase, liver form |
| ATIC | Bifunctional purine biosynthesis protein ATIC | Bifunctional purine biosynthesis protein ATIC |
| HSD17B10 | 3-hydroxyacyl-CoA dehydrogenase type-2 | 3-hydroxyacyl-CoA dehydrogenase type-2 |
| SRPK1 | SRSF protein kinase 1 | SRSF protein kinase 1 |
| LTA4H | Leukotriene A-4 hydrolase | Leukotriene A-4 hydrolase |
| STK16 | Serine/threonine-protein kinase 16 | Serine/threonine-protein kinase 16 |
| ADK | Adenosine kinase | Adenosine kinase |
| GRK6 | G protein-coupled receptor kinase 6 | G protein-coupled receptor kinase 6 |
| ACVR1 | Activin receptor type-1 | Activin receptor type-1 |
| CES1 | Liver carboxylesterase 1 | Liver carboxylesterase 1 |
| CSNK1G3 | Casein kinase I isoform gamma-3 | Casein kinase I isoform gamma-3 |
| CYP51A1 | Lanosterol 14-alpha demethylase | Lanosterol 14-alpha demethylase |
| CAMK2A | Calcium/calmodulin-dependent protein kinase type II subunit alpha | Calcium/calmodulin-dependent protein kinase type II subunit alpha |
| BCAT1 | Branched-chain-amino -acid aminotransferase, cytosolic | Branched-chain-amino -acid aminotransferase, cytosolic |
| MAP2K4 | Dual specificity mitogen-activated protein kinase kinase 4 | Dual specificity mitogen-activated protein kinase kinase 4 |
| ACACB | Acetyl-CoA carboxylase 2 | Acetyl-CoA carboxylase 2 |
| MTAP | S-methyl-5'-thioadenosine phosphorylase | S-methyl-5'-thioadenosine phosphorylase |
| AHCY | Adenosylhomocysteinase | Adenosylhomocysteinase |
| STK24 | Serine/threonine-protein kinase 24 | Serine/threonine-protein kinase 24 |
| GALK1 | Galactokinase | Galactokinase |

(continued)

| abbreviation of target protein | target protein's name | Chinese translation of target protein's name |
|---|---|---|
| MAP2K6 | Dual specificity mitogen-activated protein kinase kinase 6 | Dual specificity mitogen-activated protein kinase kinase 6 |
| DCPS | m7GpppX diphosphatase | m7GpppX diphosphatase |
| LDHB | L-lactate dehydrogenase B chain | L-lactate dehydrogenase B chain |
| EPHX2 | Bifunctional epoxide hydrolase 2 [Includes: Cytosolic epoxide hydrolase 2 | Bifunctional epoxide hydrolase 2 [Includes: Cytosolic epoxide hydrolase 2 |
| NQO2 | Ribosyldihydronicotinamide dehydrogenase [quinone] | Ribosyldihydronicotinamide dehydrogenase [quinone] |
| SRPK2 | SRSF protein kinase 2 | SRSF protein kinase 2 |
| SAE1 | SUMO-activating enzyme subunit 1 | SUMO-activating enzyme subunit 1 |
| CARM1 | Histone-arginine methyltransferase CARM1 | Histone-arginine methyltransferase CARM1 |
| CAMK4 | Calcium/calmodulin-dependent protein kinase type IV | Calcium/calmodulin-dependent protein kinase type IV |
| TNIK | TRAF2 and NCK-interacting protein kinase | TRAF2 and NCK-interacting protein kinase |
| DCK | Deoxycytidine kinase | Deoxycytidine kinase |
| PHKG2 | Phosphorylase b kinase gamma catalytic chain, liver/testis isoform | Phosphorylase b kinase gamma catalytic chain, liver/testis isoform |
| DYRK1A | Dual specificity tyrosine-phosphorylation-regulated kinase 1A | Dual specificity tyrosine-phosphorylation-regulated kinase 1A |
| CBR1 | Carbonyl reductase [NADPH] 1 | Carbonyl reductase [NADPH] 1 |
| DAPK3 | Death-associated protein kinase 3 | Death-associated protein kinase 3 |
| FEN1 | Flap endonuclease 1 | Flap endonuclease 1 |
| OXSR1 | Serine/threonine-protein kinase OSR1 | Serine/threonine-protein kinase OSR1 |
| CLK3 | Dual specificity protein kinase CLK3 | Dual specificity protein kinase CLK3 |
| UPP1 | Uridine phosphorylase 1 | Uridine phosphorylase 1 |
| AKR1C1 | Aldo-keto reductase family 1 member C1 | Aldo-keto reductase family 1 member C1 |
| AKR1C3 | Aldo-keto reductase family 1 member C3 | Aldo-keto reductase family 1 member C3 |
| CD38 | ADP-ribosyl cyclase/cyclic ADP-ribose hydrolase 1 | ADP-ribosyl cyclase/cyclic ADP-ribose hydrolase 1 |
| SORT1 | Sortilin | Sortilin |
| GALNT2 | Polypeptide N-acetylgalactosaminyltransferase 2 | Polypeptide N-acetylgalactosaminyltransferase 2 |
| LGALS1 | Galectin-1 | Galectin-1 |
| HK2 | Hexokinase-2 | Hexokinase-2 |
| HK1 | Hexokinase-1 | Hexokinase-1 |
| HMOX1 | Heme oxygenase 1 | Heme oxygenase 1 |
| PARP14 | Protein mono-ADP-ribosyltransferase PARP14 | Protein mono-ADP-ribosyltransferase PARP14 |
| GRB2 | Growth factor receptor-bound protein 2 | Growth factor receptor-bound protein 2 |

(continued)

| abbreviation of target protein | target protein's name | Chinese translation of target protein's name |
|---|---|---|
| HMOX2 | Heme oxygenase 2 | Heme oxygenase 2 |
| DDAH1 | N(G),N(G)-dimethylarginine dimethylaminohydrolase 1 | N(G),N(G)-dimethylarginine dimethylaminohydrolase 1 |
| PARP12 | Protein mono-ADP-ribosyltransferase PARP12 | Protein mono-ADP-ribosyltransferase PARP12 |
| OAT | Ornithine aminotransferase, mitochondrial | Ornithine aminotransferase, mitochondrial |
| SIRT6 | NAD-dependent protein deacetylase sirtuin-6 | NAD-dependent protein deacetylase sirtuin-6 |
| PGK1 | Phosphoglycerate kinase 1 | Phosphoglycerate kinase 1 |
| KAT8 | Histone acetyltransferase KAT8 | Histone acetyltransferase KAT8 |
| SPR | Sepiapterin reductase | Sepiapterin reductase |
| CTH | Cystathionine gamma-lyase | Cystathionine gamma-lyase |
| SIRT5 | NAD-dependent protein deacylase sirtuin-5, mitochondrial | NAD-dependent protein deacylase sirtuin-5, mitochondrial |
| GSTO1 | Glutathione S-transferase omega-1 | Glutathione S-transferase omega-1 |
| PFKFB3 | 6-phosphofructo-2-kinase/fructose-2,6-bisphosphatase 3 | 6-phosphofructo-2-kinase/fructose-2,6-bisphosphatase 3 |
| CTBP2 | C-terminal-binding protein 2 | C-terminal-binding protein 2 |
| PARP10 | Protein mono-ADP-ribosyltransferase PARP10 | Protein mono-ADP-ribosyltransferase PARP10 |
| SMYD3 | Histone-lysine N-methyltransferase SMYD3 | Histone-lysine N-methyltransferase SMYD3 |
| SULT1A1 | Sulfotransferase 1A1 | Sulfotransferase 1A1 |
| AKR1C4 | Aldo-keto reductase family 1 member C4 | Aldo-keto reductase family 1 member C4 |
| GSTM2 | Glutathione S-transferase Mu 2 | Glutathione S-transferase Mu 2 |
| STRADA | STE20-related kinase adapter protein alpha | STE20-related kinase adapter protein alpha |
| BMPR2 | Bone morphogenetic protein receptor type-2 | Bone morphogenetic protein receptor type-2 |
| PIK3C2A | Phosphatidylinositol 4-phosphate 3-kinase C2 domain-containing subunit alpha | Phosphatidylinositol 4-phosphate 3-kinase C2 domain-containing subunit alpha |
| PTPN1 | Tyrosine-protein phosphatase non-receptor type 1 | Tyrosine-protein phosphatase non-receptor type 1 |
| IDE | Insulin-degrading enzyme | Insulin-degrading enzyme |
| CAPNS1 | Calpain small subunit 1 | Calpain small subunit 1 |
| GLO1 | Lactoylglutathione lyase | Lactoylglutathione lyase |
| UCHL1 | Ubiquitin carboxyl-terminal hydrolase isozyme L1 | Ubiquitin carboxyl-terminal hydrolase isozyme L1 |
| NPC1 | NPC intracellular cholesterol transporter 1 | NPC intracellular cholesterol transporter 1 |
| FES | Tyrosine-protein kinase Fes/Fps | Tyrosine-protein kinase Fes/Fps |
| CSNK1G1 | Casein kinase I isoform gamma-1 | Casein kinase I isoform gamma-1 |
| SLK | STE20-like serine/threonine-protein kinase | STE20-like serine/threonine-protein kinase |

(continued)

| abbreviation of target protein | target protein's name | Chinese translation of target protein's name |
|---|---|---|
| MAP2K7 | Dual specificity mitogen-activated protein kinase kinase 7 | Dual specificity mitogen-activated protein kinase kinase 7 |
| PIK3C3 | Phosphatidylinositol 3-kinase catalytic subunit type 3 | Phosphatidylinositol 3-kinase catalytic subunit type 3 |
| HAO1 | Hydroxyacid oxidase 1 | Hydroxyacid oxidase 1 |
| PASK | PAS domain-containing serine/threonine-protein kinase | PAS domain-containing serine/threonine-protein kinase |
| STK25 | Serine/threonine-protein kinase 25 | Serine/threonine-protein kinase 25 |
| KYNU | Kynureninase | Kynureninase |
| DUT | Deoxyuridine 5'-triphosphate nucleotidohydrolase, mitochondrial | Deoxyuridine 5'-triphosphate nucleotidohydrolase, mitochondrial |
| PTPN22 | Tyrosine-protein phosphatase non-receptor type 22 | Tyrosine-protein phosphatase non-receptor type 22 |
| NEK7 | Serine/threonine-protein kinase Nek7 | Serine/threonine-protein kinase Nek7 |
| NNMT | Nicotinamide N-methyltransferase | Nicotinamide N-methyltransferase |
| NRP1 | Neuropilin-1 | Neuropilin-1 |
| RPS27 | 40S ribosomal protein S27 | 40S ribosomal protein S27 |
| DDR1 | Epithelial discoidin domain-containing receptor 1 | Epithelial discoidin domain-containing receptor 1 |
| LPL | Lipoprotein lipase | Lipoprotein lipase |
| ABCC1 | Multidrug resistance-associated protein 1 | Multidrug resistance-associated protein 1 |
| ASIC1 | Acid-sensing ion channel 1 | Acid-sensing ion channel 1 |
| SLC2A1 | Solute carrier family 2, facilitated glucose transporter member 1 | Solute carrier family 2, facilitated glucose transporter member 1 |
| PTPRS | Receptor-type tyrosine-protein phosphatase S | Receptor-type tyrosine-protein phosphatase S |
| ABCG2 | Broad substrate specificity ATP-binding cassette transporter ABCG2 | Broad substrate specificity ATP-binding cassette transporter ABCG2 |
| SLC1A3 | Excitatory amino acid transporter 1 | Excitatory amino acid transporter 1 |
| SLC1A1 | Excitatory amino acid transporter 3 | Excitatory amino acid transporter 3 |
| TRPM2 | Transient receptor potential cation channel subfamily M member 2 | Transient receptor potential cation channel subfamily M member 2 |
| GRM1 | Metabotropic glutamate receptor 1 | Metabotropic glutamate receptor 1 |
| MLKL | Mixed lineage kinase domain-like protein | Mixed lineage kinase domain-like protein |
| RAB7A | Ras-related protein Rab-7a | Ras-related protein Rab-7a |
| AHCYL1 | S-adenosylhomocysteine hydrolase-like protein 1 | S-adenosylhomocysteine hydrolase-like protein 1 |
| NAAA | N-acylethanolamine-hydrolyzing acid amidase | N-acylethanolamine-hydrolyzing acid amidase |
| CAMK2B | Calcium/calmodulin-dependent protein kinase type II subunit beta | Calcium/calmodulin-dependent protein kinase type II subunit beta |
| NT5E | 5'-nucleotidase | 5'-nucleotidase |

(continued)

| abbreviation of target protein | target protein’s name | Chinese translation of target protein’s name |
|---|---|---|
| CTSL | Procathepsin L | Procathepsin L |
| HSPA1A | Heat shock 70 kDa protein 1A | Heat shock 70 kDa protein 1A |
| FABP5 | Fatty acid-binding protein 5 | Fatty acid-binding protein 5 |
| CTSC | Dipeptidyl peptidase 1 | Dipeptidyl peptidase 1 |
| ACVR2A | Activin receptor type-2A | Activin receptor type-2A |
| MMP 14 | Matrix metalloproteinase-14 | Matrix metalloproteinase-14 |
| HSP90B1 | Endoplasmin | Endoplasmin |
| GLB1 | Beta-galactosidase | Beta-galactosidase |
| PTPRF | Receptor-type tyrosine-protein phosphatase | Receptor-type tyrosine-protein |
| | F | phosphatase F |
| ST14 | Suppressor of tumorigenicity 14 protein | Suppressor of tumorigenicity 14 protein |
| PKN2 | Serine/threonine-protein kinase N2 | Serine/threonine-protein kinase N2 |
| ARG1 | Arginase-1 | Arginase-1 |
| PGAM1 | Phosphoglycerate mutase 1 | Phosphoglycerate mutase 1 |
| PPPICA | Serine/threonine-protein phosphatase PP1-alpha catalytic subunit | Serine/threonine-protein phosphatase PP1-alpha catalytic subunit |
| PTP4A2 | Protein tyrosine phosphatase type IVA 2 | Protein tyrosine phosphatase type IVA 2 |
| MAST1 | Microtubule-associated serine/threonine-protein kinase 1 | Microtubule-associated serine/threonine-protein kinase 1 |
| PI4KB | Phosphatidylinositol 4-kinase beta | Phosphatidylinositol 4-kinase beta |
| VRK2 | Serine/threonine-protein kinase VRK2 | Serine/threonine-protein kinase VRK2 |
| SCD | Stearoyl-CoA desaturase | Stearoyl-CoA desaturase |
| NUDT1 | 7,8-dihydro-8-oxoguanine triphosphatase | 7,8-dihydro-8-oxoguanine triphosphatase |
| CSNK1G2 | Casein kinase I isoform gamma-2 | Casein kinase I isoform gamma-2 |
| EBP | 3-beta-hydroxysteroid-Delta(8),Delta(7)-isomerase | 3-beta-hydroxysteroid-Delta(8),Delta(7)-isomerase |
| EIF2AK3 | Eukaryotic translation initiation factor 2-alpha kinase 3 | Eukaryotic translation initiation factor 2-alpha kinase 3 |
| ATP2A2 | Sarcoplasmic/endoplasmic reticulum calcium ATPase 2 | Sarcoplasmic/endoplasmic reticulum calcium ATPase 2 |
| MAN1B1 | Endoplasmic reticulum mannosyl-oligosaccharide 1,2-alpha-mannosidase | Endoplasmic reticulum mannosyl-oligosaccharide 1,2-alpha-mannosidase |
| CDKLS | Cyclin-dependent kinase-like 5 | Cyclin-dependent kinase-like 5 |
| MAP4K4 | Mitogen-activated protein kinase kinase kinase kinase 4 | Mitogen-activated protein kinase kinase kinase kinase 4 |
| DPP8 | Dipeptidyl peptidase 8 | Dipeptidyl peptidase 8 |
| MKNK1 | MAP kinase-interacting serine/threonine-protein kinase 1 | MAP kinase-interacting serine/threonine-protein kinase 1 |
| PREP | Prolyl endopeptidase | Prolyl endopeptidase |

(continued)

| abbreviation of target protein | target protein's name | Chinese translation of target protein's name |
|---|---|---|
| FKBPS | Peptidyl-prolyl cis-trans isomerase FKBPS | Peptidyl-prolyl cis-trans isomerase FKBPS |
| SIRT1 | NAD-dependent protein deacetylase sirtuin-1 | NAD-dependent protein deacetylase sirtuin-1 |
| STK3 | Serine/threonine-protein kinase 3 | Serine/threonine-protein kinase 3 |
| LIG1 | DNA ligase 1 | DNA ligase 1 |
| DUSP3 | Dual specificity protein phosphatase 3 | Dual specificity protein phosphatase 3 |
| CAMKK2 | Calcium/calmodulin-dependent protein kinase kinase 2 | Calcium/calmodulin-dependent protein kinase kinase 2 |
| PRKAB1 | 5'-AMP-activated protein kinase subunit beta-1 | 5'-AMP-activated protein kinase subunit beta-1 |
| MTR | Methionine synthase | Methionine synthase |
| UCHL3 | Ubiquitin carboxyl-terminal hydrolase isozyme L3 | Ubiquitin carboxyl-terminal hydrolase isozyme L3 |
| ESRRB | Steroid hormone receptor ERR2 | Steroid hormone receptor ERR2 |
| RIOK1 | Serine/threonine-protein kinase RIO1 | Serine/threonine-protein kinase RIO1 |
| TRAP1 | Heat shock protein 75 kDa, mitochondrial | Heat shock protein 75 kDa, mitochondrial |
| PIN1 | Peptidyl-prolyl cis-trans isomerase NIMA-interacting 1 | Peptidyl-prolyl cis-trans isomerase NIMA-interacting 1 |
| ALDH1A2 | Retinal dehydrogenase 2 | Retinal dehydrogenase 2 |
| PAK6 | Serine/threonine-protein kinase PAK 6 | Serine/threonine-protein kinase PAK 6 |
| AOX1 | Aldehyde oxidase | Aldehyde oxidase |
| DPP9 | Dipeptidyl peptidase 9 | Dipeptidyl peptidase 9 |
| ACP1 | Low molecular weight phosphotyrosine protein phosphatase | Low molecular weight phosphotyrosine protein phosphatase |
| PTPN7 | Tyrosine-protein phosphatase non-receptor type 7 | Tyrosine-protein phosphatase non-receptor type 7 |
| TLK2 | Serine/threonine-protein kinase tousled-like 2 | Serine/threonine-protein kinase tousled-like 2 |
| SMG1 | Serine/threonine-protein kinase SMG1 | Serine/threonine-protein kinase SMG1 |
| YWHAZ | 14-3-3 protein zeta/delta | 14-3-3 protein zeta/delta |
| ALDH1A1 | Retinal dehydrogenase 1 | Retinal dehydrogenase 1 |
| PHF8 | Histone lysine demethylase PHF8 | Histone lysine demethylase PHF8 |
| PRKAG1 | 5'-AMP-activated protein kinase subunit gamma-1 | 5'-AMP-activated protein kinase subunit gamma-1 |
| PRMT3 | Protein arginine N-methyltransferase 3 | Protein arginine N-methyltransferase 3 |
| SF3B3 | Splicing factor 3B subunit 3 | Splicing factor 3B subunit 3 |
| AKT1S1 | Proline-rich AKT1 substrate 1 | Proline-rich AKT1 substrate 1 |
| CHUK | Inhibitor of nuclear factor kappa-B kinase subunit alpha | Inhibitor of nuclear factor kappa-B kinase subunit alpha |
| RPL36A | 60S ribosomal protein L36a | 60S ribosomal protein L36a |
| RPL31 | 60S ribosomal protein L31 | 60S ribosomal protein L31 |

(continued)

| abbreviation of target protein | target protein's name | Chinese translation of target protein's name |
|---|---|---|
| RPS16 | 40S ribosomal protein S16 | 40S ribosomal protein S16 |
| RPS19 | 40S ribosomal protein S19 | 40S ribosomal protein S19 |
| RPS12 | 40S ribosomal protein S12 | 40S ribosomal protein S12 |
| RPL22 | 60S ribosomal protein L22 | 60S ribosomal protein L22 |
| RPS25 | 40S ribosomal protein S25 | 40S ribosomal protein S25 |
| RPL21 | 60S ribosomal protein L21 | 60S ribosomal protein L21 |
| RPL23 | 60S ribosomal protein L23 | 60S ribosomal protein L23 |
| RPS4Y1 | 40S ribosomal protein S4, Y isoform 1 | 40S ribosomal protein S4, Y isoform 1 |
| RPL36 | 60S ribosomal protein L36 | 60S ribosomal protein L36 |
| RPL27 | 60S ribosomal protein L27 | 60S ribosomal protein L27 |
| RPS15A | 40S ribosomal protein S15a | 40S ribosomal protein S15a |
| RPS11 | 40S ribosomal protein S11 | 40S ribosomal protein S11 |
| RPL13A | 60S ribosomal protein L13a | 60S ribosomal protein L13a |
| RPL37 | 60S ribosomal protein L37 | 60S ribosomal protein L37 |
| RPL7 | 60S ribosomal protein L7 | 60S ribosomal protein L7 |
| RPL29 | 60S ribosomal protein L29 | 60S ribosomal protein L29 |
| RPL27A | 60S ribosomal protein L27a | 60S ribosomal protein L27a |
| RPL35A | 60S ribosomal protein L35a | 60S ribosomal protein L35a |
| RPS26 | 40S ribosomal protein S26 | 40S ribosomal protein S26 |
| RPL37A | 60S ribosomal protein L37a | 60S ribosomal protein L37a |
| RPS29 | 40S ribosomal protein S29 | 40S ribosomal protein S29 |
| RPS28 | 40S ribosomal protein S28 | 40S ribosomal protein S28 |
| RPL17 | 60S ribosomal protein L17 | 60S ribosomal protein L17 |
| FAU | 40S ribosomal protein S30 | 40S ribosomal protein S30 |
| SLC2A3 | Solute carrier family 2, facilitated glucose transporter member 3 | Solute carrier family 2, facilitated glucose transporter member 3 |
| ASAH1 | Acid ceramidase | Acid ceramidase |
| BTN3A1 | Butyrophilin subfamily 3 member A1 | Butyrophilin subfamily 3 member A1 |
| NMT2 | Glycylpeptide N-tetradecanoyltransferase 2 | Glycylpeptide N-tetradecanoyltransferase 2 |
| PI4KA | Phosphatidylinositol 4-kinase alpha | Phosphatidylinositol 4-kinase alpha |
| GNG2 | Guanine nucleotide-binding protein G(I)/G(S)/G(O) subunit gamma-2 | Guanine nucleotide-binding protein G(I)/G(S)/G(O) subunit gamma-2 |
| GNA11 | Guanine nucleotide-binding protein subunit alpha-11 | Guanine nucleotide-binding protein subunit alpha-11 |
| PRMT8 | Protein arginine N-methyltransferase 8 | Protein arginine N-methyltransferase 8 |
| LYPLA1 | Acyl-protein thioesterase 1 | Acyl-protein thioesterase 1 |
| LGALS3 | Galectin-3 | Galectin-3 |

(continued)

| abbreviation of target protein | target protein's name | Chinese translation of target protein's name |
|---|---|---|
| UBE2D3 | Ubiquitin-conjugating enzyme E2 D3 | Ubiquitin-conjugating enzyme E2 D3 |
| S100A4 | Protein S100-A4 | Protein S100-A4 |
| LGALS9 | Galectin-9 | Galectin-9 |
| PIP4K2A | Phosphatidylinositol 5-phosphate 4-kinase type-2 alpha | Phosphatidylinositol 5-phosphate 4-kinase type-2 alpha |
| LGMN | Legumain | Legumain |
| GNB1 | Guanine nucleotide-binding protein G(I)/G(S)/G(T) subunit beta-1 | Guanine nucleotide-binding protein G(I)/G(S)/G(T) subunit beta-1 |
| HINT1 | Histidine triad nucleotide-binding protein 1 | Histidine triad nucleotide-binding protein 1 |
| FTO | Alpha-ketoglutarate-dependent dioxygenase FTO | Alpha-ketoglutarate-dependent dioxygenase FTO |
| BRPF1 | Peregrin | Peregrin |
| ST6GAL1 | Beta-galactoside alpha-2,6-sialyltransferase 1 | Beta-galactoside alpha-2,6-sialyltransferase 1 |
| INPPL1 | Phosphatidylinositol 3,4,5-trisphosphate 5-phosphatase 2 | Phosphatidylinositol 3,4,5-trisphosphate 5-phosphatase 2 |
| PI4K2B | Phosphatidylinositol 4-kinase type 2-beta | Phosphatidylinositol 4-kinase type 2-beta |
| PARP16 | Protein mono-ADP-ribosyltransferase PARP16 | Protein mono-ADP-ribosyltransferase PARP16 |
| HEXA | Beta-hexosaminidase subunit alpha | Beta-hexosaminidase subunit alpha |
| SGPL1 | Sphingosine-1-phosphate lyase 1 | Sphingosine-1-phosphate lyase 1 |
| MGAT2 | Alpha-1,6-mannosyl-glycoprotein 2-beta-N-acetylglucosaminyltransferase | Alpha-1,6-mannosyl-glycoprotein 2-beta-N-acetylglucosaminyltransferase |
| LGALS8 | Galectin-8 | Galectin-8 |
| ASH2L | Set1/Ash2 histone methyltransferase complex subunit ASH2 | Set1/Ash2 histone methyltransferase complex subunit ASH2 |
| SARM1 | NAD(+) hydrolase SARM1 | NAD(+) hydrolase SARM1 |
| LYPLA2 | Acyl-protein thioesterase 2 | Acyl-protein thioesterase 2 |
| ASNS | Asparagine synthetase [glutamine-hydrolyzing] | Asparagine synthetase [glutamine-hydrolyzing] |
| PABPC1 | Polyadenylate-binding protein 1 | Polyadenylate-binding protein 1 |
| PRMT5 | Protein arginine N-methyltransferase 5 | Protein arginine N-methyltransferase 5 |
| DNPH1 | 2'-deoxynucleoside 5'-phosphate N-hydrolase 1 | 2'-deoxynucleoside 5'-phosphate N-hydrolase 1 |
| WDR48 | WD repeat-containing protein 48 | WD repeat-containing protein 48 |
| WDR77 | Methylosome protein 50 | Methylosome protein 50 |
| ATAD2B | ATPase family AAA domain-containing protein 2B | ATPase family AAA domain-containing protein 2B |
| COPS5 | COP9 signalosome complex subunit 5 | COP9 signalosome complex subunit 5 |
| PFKFB2 | 6-phosphofructo-2-kinase/fructose-2,6-bisphosphatase 2 | 6-phosphofructo-2-kinase/fructose-2,6-bisphosphatase 2 |

(continued)

| abbreviation of target protein | target protein's name | Chinese translation of target protein's name |
|---|---|---|
| KDM4B | Lysine-specific demethylase 4B | Lysine-specific demethylase 4B |
| PRKAB2 | 5'-AMP-activated protein kinase subunit beta-2 | 5'-AMP-activated protein kinase subunit beta-2 |
| MEN1 | Menin | Menin |
| PMM2 | Phosphomannomutase 2 | Phosphomannomutase 2 |
| RPTOR | Regulatory-associated protein of mTOR | Regulatory-associated protein of mTOR |
| MLST8 | Target of rapamycin complex subunit LST8 | Target of rapamycin complex subunit LST8 |
| DTYMK | Thymidylate kinase | Thymidylate kinase |
| PTPN9 | Tyrosine-protein phosphatase non-receptor type 9 | Tyrosine-protein phosphatase non-receptor type 9 |
| SHMT2 | Serine hydroxymethyltransferase, mitochondrial | Serine hydroxymethyltransferase, mitochondrial |
| PARG | Poly(ADP-ribose) glycohydrolase | Poly(ADP-ribose) glycohydrolase |
| GLS | Glutaminase kidney isoform, mitochondrial | Glutaminase kidney isoform, mitochondrial |
| APAF1 | Apoptotic protease-activating factor 1 | Apoptotic protease-activating factor 1 |
| KSR2 | Kinase suppressor of Ras 2 | Kinase suppressor of Ras 2 |
| SORD | Sorbitol dehydrogenase | Sorbitol dehydrogenase |
| SMS | Spermine synthase | Spermine synthase |
| GALE | UDP-glucose 4-epimerase | UDP-glucose 4-epimerase |
| PGD | 6-phosphogluconate dehydrogenase, decarboxylating | 6-phosphogluconate dehydrogenase, decarboxylating |
| HNMT | Histamine N-methyltransferase | Histamine N-methyltransferase |
| QDPR | Dihydropteridine reductase | Dihydropteridine reductase |
| GSTK1 | Glutathione S-transferase kappa 1 | Glutathione S-transferase kappa 1 |
| SRM | Spermidine synthase | Spermidine synthase |
| FARS2 | Phenylalanine--tRNA ligase, mitochondrial | Phenylalanine--tRNA ligase, mitochondrial |
| GNAI3 | Guanine nucleotide-binding protein G(i) subunit alpha-3 | Guanine nucleotide-binding protein G(i) subunit alpha-3 |
| CLTC | Clathrin heavy chain 1 | Clathrin heavy chain 1 |
| RHOC | Rho-related GTP-binding protein RhoC | Rho-related GTP-binding protein RhoC |
| DNM1 | Dynamin-1 | Dynamin-1 |
| GALNT10 | Polypeptide N-acetylgalactosaminyltransferase 10 | Polypeptide N-acetylgalactosaminyltransferase 10 |
| MTHFD1 | C-1-tetrahydrofolate synthase, cytoplasmic | C-1-tetrahydrofolate synthase, cytoplasmic |
| RAB2A | Ras-related protein Rab-2A | Ras-related protein Rab-2A |
| USP5 | Ubiquitin carboxyl-terminal hydrolase 5 | Ubiquitin carboxyl-terminal hydrolase 5 |
| GDA | Guanine deaminase | Guanine deaminase |
| UCK2 | Uridine-cytidine kinase 2 | Uridine-cytidine kinase 2 |

(continued)

| abbreviation of target protein | target protein's name | Chinese translation of target protein's name |
|---|---|---|
| HIF1AN | Hypoxia-inducible factor 1-alpha inhibitor | Hypoxia-inducible factor 1-alpha inhibitor |
| MDH2 | Malate dehydrogenase, mitochondrial | Malate dehydrogenase, mitochondrial |
| CYP2E1 | Cytochrome P450 2E1 | Cytochrome P450 2E1 |
| YWHAG | 14-3-3 protein gamma | 14-3-3 protein gamma |
| GFPT1 | Glutamine--fructose-6-phosphate aminotransferase [isomerizing] 1 | Glutamine-fructose-6-phosphate aminotransferase [isomerizing] 1 |
| FN1 | Fibronectin | Fibronectin |
| PLAUR | Urokinase plasminogen activator surface receptor | Urokinase plasminogen activator surface receptor |
| NCS1 | Neuronal calcium sensor 1 | Neuronal calcium sensor 1 |
| GNAI1 | Guanine nucleotide-binding protein G(i) subunit alpha-1 | Guanine nucleotide-binding protein G(i) subunit alpha-1 |
| HCN2 | Potassium/sodium hyperpolarization-activated cyclic nucleotide-gated channel 2 | Potassium/sodium hyperpolarization-activated cyclic nucleotide-gated channel 2 |
| QSOX1 | Sulfhydryl oxidase 1 | Sulfhydryl oxidase 1 |
| TLR1 | Toll-like receptor 1 | Toll-like receptor 1 |
| USP14 | Ubiquitin carboxyl-terminal hydrolase 14 | Ubiquitin carboxyl-terminal hydrolase 14 |
| KSR1 | Kinase suppressor of Ras 1 | Kinase suppressor of Ras 1 |
| RAB9A | Ras-related protein Rab-9A | Ras-related protein Rab-9A |
| CCR7 | C-C chemokine receptor type 7 | C-C chemokine receptor type 7 |
| PLCG2 | 1-phosphatidylinositol 4,5-bisphosphate phosphodiesterase gamma-2 | 1-phosphatidylinositol 4,5-bisphosphate phosphodiesterase gamma-2 |
| GFER | FAD-linked sulfhydryl oxidase ALR | FAD-linked sulfhydryl oxidase ALR |
| PTPRG | Receptor-type tyrosine-protein phosphatase gamma | Receptor-type tyrosine-protein phosphatase gamma |
| FUT8 | Alpha-(1,6)-fucosyltransferase | Alpha-(1,6)-fucosyltransferase |
| POR | NADPH-cytochrome P450 reductase | NADPH-cytochrome P450 reductase |
| RECQL | ATP-dependent DNA helicase Q1 | ATP-dependent DNA helicase Q1 |
| UNG | Uracil-DNA glycosylase | Uracil-DNA glycosylase |
| CAD | CAD protein [Includes: Glutamine-dependent carbamoyl-phosphate synthase | CAD protein [Includes: Glutamine-dependent carbamoyl-phosphate synthase |
| NCOA3 | Nuclear receptor coactivator 3 | Nuclear receptor coactivator 3 |
| CTDSP1 | Carboxy-terminal domain RNA polymerase II polypeptide A small phosphatase 1 | Carboxy-terminal domain RNA polymerase II polypeptide A small phosphatase 1 |
| KDM1B | Lysine-specific histone demethylase 1B | Lysine-specific histone demethylase 1B |
| TEAD1 | Transcriptional enhancer factor TEF-1 | Transcriptional enhancer factor TEF-1 |
| DDO | D-aspartate oxidase | D-aspartate oxidase |
| CD4 | T-cell surface glycoprotein CD4 | T-cell surface glycoprotein CD4 |
| SLC1A5 | Neutral amino acid transporter B(0) | Neutral amino acid transporter B(0) |

(continued)

| abbreviation of target protein | target protein's name | Chinese translation of target protein's name |
|---|---|---|
| CD81 | CD81 antigen | CD81 antigen |
| GGT1 | Glutathione hydrolase 1 proenzyme | Glutathione hydrolase 1 proenzyme |
| ADIPOR1 | Adiponectin receptor protein 1 | Adiponectin receptor protein 1 |
| RAP1A | Ras-related protein Rap-1A | Ras-related protein Rap-1A |
| RAB29 | Ras-related protein Rab-7L1 | Ras-related protein Rab-7L1 |
| GOPC | Golgi-associated PDZ and coiled-coil motif-containing protein | Golgi-associated PDZ and coiled-coil motif-containing protein |
| UTRN | Utrophin | Utrophin |
| GNAO1 | Guanine nucleotide-binding protein G(o) subunit alpha | Guanine nucleotide-binding protein G(o) subunit alpha |
| DDOST | Dolichyl-diphosphooligosaccharide--protein glycosyltransferase 48 kDa subunit | Dolichyl-diphosphooligosaccharide-protein glycosyltransferase 48 kDa subunit |
| PYGB | Glycogen phosphorylase, brain form | Glycogen phosphorylase, brain form |
| RABGGTB | Geranylgeranyl transferase type-2 subunit beta | Geranylgeranyl transferase type-2 subunit beta |
| VAV1 | Proto-oncogene vav | Proto-oncogene vav |
| SDHA | Succinate dehydrogenase [ubiquinone] flavoprotein subunit, mitochondrial | Succinate dehydrogenase [ubiquinone] flavoprotein subunit, mitochondrial |
| QTRT1 | Queuine tRNA-ribosyltransferase catalytic subunit 1 | Queuine tRNA-ribosyltransferase catalytic subunit 1 |
| CISD2 | CDGSH iron-sulfur domain-containing protein 2 | CDGSH iron-sulfur domain-containing protein 2 |
| DHPS | Deoxyhypusine synthase | Deoxyhypusine synthase |
| PPP2RSA | Serine/threonine-protein phosphatase 2A 56 kDa regulatory subunit alpha isoform | Serine/threonine-protein phosphatase 2A 56 kDa regulatory subunit alpha isoform |
| PANK2 | Pantothenate kinase 2, mitochondrial | Pantothenate kinase 2, mitochondrial |
| NEDD8 | NEDD8 | NEDD8 |
| DOHH | Deoxyhypusine hydroxylase | Deoxyhypusine hydroxylase |
| UBE2M | NEDD8-conjugating enzyme Ubc12 | NEDD8-conjugating enzyme Ubc12 |
| RNASEL | 2-SA-dependent ribonuclease | 2-SA-dependent ribonuclease |
| SYNJ1 | Synaptojanin-1 | Synaptojanin-1 |
| DUS2 | tRNA-dihydrouridine(20) synthase [NAD(P)+]-like | tRNA-dihydrouridine(20) synthase [NAD(P)+]-like |
| CYCS | Cytochrome c | Cytochrome c |
| PPP1CC | Serine/threonine-protein phosphatase PP1-gamma catalytic subunit | Serine/threonine-protein phosphatase PP1-gamma catalytic subunit |
| HMBS | Porphobilinogen deaminase | Porphobilinogen deaminase |
| PNPO | Pyridoxine-5'-phosphate oxidase | Pyridoxine-5'-phosphate oxidase |
| PPIF | Peptidyl-prolyl cis-trans isomerase F, mitochondrial | Peptidyl-prolyl cis-trans isomerase F, mitochondrial |

(continued)

| abbreviation of target protein | target protein's name | Chinese translation of target protein's name |
|---|---|---|
| DPP7 | Dipeptidyl peptidase 2 | Dipeptidyl peptidase 2 |
| MARK1 | Serine/threonine-protein kinase MARK1 | Serine/threonine-protein kinase MARK1 |
| PRCP | Lysosomal Pro-X carboxypeptidase | Lysosomal Pro-X carboxypeptidase |
| PIP4K2C | Phosphatidylinositol 5-phosphate 4-kinase type-2 gamma | Phosphatidylinositol 5-phosphate 4-kinase type-2 gamma |
| AKR1A1 | Aldo-keto reductase family 1 member A1 | Aldo-keto reductase family 1 member A1 |
| ME1 | NADP-dependent malic enzyme | NADP-dependent malic enzyme |
| ACACA | Acetyl-CoA carboxylase 1 | Acetyl-CoA carboxylase 1 |
| CD74 | HLA class II histocompatibility antigen gamma chain | HLA class II histocompatibility antigen gamma chain |
| NFATC1 | Nuclear factor of activated T-cells, cytoplasmic 1 | Nuclear factor of activated T-cells, cytoplasmic 1 |
| RUNX1 | Runt-related transcription factor 1 | Runt-related transcription factor 1 |
| PTPRC | Receptor-type tyrosine-protein phosphatase C | Receptor-type tyrosine-protein phosphatase C |
| CAPN2 | Calpain-2 catalytic subunit | Calpain-2 catalytic subunit |
| RB1 | Retinoblastoma-associated protein | Retinoblastoma-associated protein |
| ITGA5 | Integrin alpha-5 | Integrin alpha-5 |
| ITGA2 | Integrin alpha-2 | Integrin alpha-2 |
| ADAM10 | Disintegrin and metalloproteinase domain-containing protein 10 | Disintegrin and metalloproteinase domain-containing protein 10 |
| DAGLB | Diacylglycerol lipase-beta | Diacylglycerol lipase-beta |
| SLC16A1 | Monocarboxylate transporter 1 | Monocarboxylate transporter 1 |
| ABCC2 | ATP-binding cassette sub-family C member 2 | ATP-binding cassette sub-family C member 2 |
| SCARB1 | Scavenger receptor class B member 1 | Scavenger receptor class B member 1 |
| DAGLA | Diacylglycerol lipase-alpha | Diacylglycerol lipase-alpha |
| SLC47A1 | Multidrug and toxin extrusion protein 1 | Multidrug and toxin extrusion protein 1 |
| PIK3C2B | Phosphatidylinositol 4-phosphate 3-kinase C2 domain-containing subunit beta | Phosphatidylinositol 4-phosphate 3-kinase C2 domain-containing subunit beta |
| GBA2 | Non-lysosomal glucosylceramidase | Non-lysosomal glucosylceramidase |
| NCEH1 | Neutral cholesterol ester hydrolase 1 | Neutral cholesterol ester hydrolase 1 |
| WNK2 | Serine/threonine-protein kinase WNK2 | Serine/threonine-protein kinase WNK2 |
| LIPE | Hormone-sensitive lipase | Hormone-sensitive lipase |
| SLC27A1 | Long-chain fatty acid transport protein 1 | Long-chain fatty acid transport protein 1 |
| PTPRA | Receptor-type tyrosine-protein phosphatase alpha | Receptor-type tyrosine-protein phosphatase alpha |
| PTPN2 | Tyrosine-protein phosphatase non-receptor type 2 | Tyrosine-protein phosphatase non-receptor type 2 |
| ITGB5 | Integrin beta-5 | Integrin beta-5 |

(continued)

| abbreviation of target protein | target protein's name | Chinese translation of target protein's name |
|---|---|---|
| CTSH | Pro-cathepsin H [Cleaved into: Cathepsin H mini chain; Cathepsin H | Pro-cathepsin H [Cleaved into: Cathepsin H mini chain; Cathepsin H |
| MAN2B1 | Lysosomal alpha-mannosidase | Lysosomal alpha-mannosidase |
| ITPR2 | Inositol 1,4,5-trisphosphate receptor type 2 | Inositol 1,4,5-trisphosphate receptor type 2 |
| ITPR3 | Inositol 1,4,5-trisphosphate receptor type 3 | Inositol 1,4,5-trisphosphate receptor type 3 |
| ABHD12 | Lysophosphatidylserine lipase ABHD12 | Lysophosphatidylserine lipase ABHD12 |
| CTSZ | Cathepsin Z | Cathepsin Z |
| SLC6A12 | Sodium- and chloride-dependent betaine transporter | Sodium- and chloride-dependent betaine transporter |
| SLC6A11 | Sodium- and chloride-dependent GABA transporter 3 | Sodium- and chloride-dependent GABA transporter 3 |
| KCNN3 | Small conductance calcium-activated potassium channel protein 3 | Small conductance calcium-activated potassium channel protein 3 |
| SLC27A4 | Long-chain fatty acid transport protein 4 | Long-chain fatty acid transport protein 4 |
| PTPN6 | Tyrosine-protein phosphatase non-receptor type 6 | Tyrosine-protein phosphatase non-receptor type 6 |
| AMPD3 | AMP deaminase 3 | AMP deaminase 3 |
| PLEC | Plectin | Plectin |
| PLD1 | Phospholipase D1 | Phospholipase D1 |
| PAM | Peptidyl-glycine alpha-amidating monooxygenase | Peptidyl-glycine alpha-amidating monooxygenase |
| PRKD2 | Serine/threonine-protein kinase D2 | Serine/threonine-protein kinase D2 |
| TYRO3 | Tyrosine-protein kinase receptor TYRO3 | Tyrosine-protein kinase receptor TYRO3 |
| RYR2 | Ryanodine receptor 2 | Ryanodine receptor 2 |
| PIP5K1C | Phosphatidylinositol 4-phosphate 5-kinase type-1 gamma | Phosphatidylinositol 4-phosphate 5-kinase type-1 gamma |
| LIPA | Lysosomal acid lipase/cholesteryl ester hydrolase | Lysosomal acid lipase/cholesteryl ester hydrolase |
| PPIB | Peptidyl-prolyl cis-trans isomerase B | Peptidyl-prolyl cis-trans isomerase B |
| ATP2A3 | Sarcoplasmic/endoplasmic reticulum calcium ATPase 3 | Sarcoplasmic/endoplasmic reticulum calcium ATPase 3 |
| CPT1A | Carnitine O-palmitoyltransferase 1, liver isoform | Carnitine O-palmitoyltransferase 1, liver isoform |
| ICMT | Protein-S-isoprenylcysteine O-methyltransferase | Protein-S-isoprenylcysteine O-methyltransferase |
| HSD17B7 | 3-keto-steroid reductase/17-beta-hydroxysteroid dehydrogenase 7 | 3-keto-steroid reductase/17-beta-hydroxysteroid dehydrogenase 7 |
| ELOVL6 | Elongation of very long chain fatty acids protein 6 | Elongation of very long chain fatty acids protein 6 |
| DHCR7 | 7-dehydrocholesterol reductase | 7-dehydrocholesterol reductase |

(continued)

| abbreviation of target protein | target protein's name | Chinese translation of target protein's name |
|---|---|---|
| ATP2A1 | Sarcoplasmic/endoplasmic reticulum calcium ATPase 1 | Sarcoplasmic/endoplasmic reticulum calcium ATPase 1 |
| RIOK3 | Serine/threonine-protein kinase RIO3 | Serine/threonine-protein kinase RIO3 |
| HSPB1 | Heat shock protein beta-1 | Heat shock protein beta-1 |
| PTPN12 | Tyrosine-protein phosphatase non-receptor type 12 | Tyrosine-protein phosphatase non-receptor type 12 |
| PLAA | Phospholipase A-2-activating protein | Phospholipase A-2-activating protein |
| MPI | Mannose-6-phosphate isomerase | Mannose-6-phosphate isomerase |
| MATK | Megakaryocyte-associated tyrosine-protein kinase | Megakaryocyte-associated tyrosine-protein kinase |
| UBA6 | Ubiquitin-like modifier-activating enzyme 6 | Ubiquitin-like modifier-activating enzyme 6 |
| MAP2K3 | Dual specificity mitogen-activated protein kinase kinase 3 | Dual specificity mitogen-activated protein kinase kinase 3 |
| RPS6KA2 | Ribosomal protein S6 kinase alpha-2 | Ribosomal protein S6 kinase alpha-2 |
| MAP3K4 | Mitogen-activated protein kinase kinase kinase 4 | Mitogen-activated protein kinase kinase kinase 4 |
| PARP4 | Protein mono-ADP-ribosyltransferase PARP4 | Protein mono-ADP-ribosyltransferase PARP4 |
| EIF4H | Eukaryotic translation initiation factor 4H | Eukaryotic translation initiation factor 4H |
| TESK1 | Dual specificity testis-specific protein kinase 1 | Dual specificity testis-specific protein kinase 1 |
| MLX | Max-like protein X | Max-like protein X |
| NEK6 | Serine/threonine-protein kinase Nek6 | Serine/threonine-protein kinase Nek6 |
| ALDH1B1 | Aldehyde dehydrogenase X, mitochondrial | Aldehyde dehydrogenase X, mitochondrial |
| ATM | Serine-protein kinase ATM | Serine-protein kinase ATM |
| CDYL | Chromodomain Y-like protein | Chromodomain Y-like protein |
| TAOK1 | Serine/threonine-protein kinase TAO1 | Serine/threonine-protein kinase TAO1 |
| STAT6 | Signal transducer and activator of transcription 6 | Signal transducer and activator of transcription 6 |
| MGMT | Methylated-DNA--protein-cysteine methyltransferase | Methylated-DNA--protein-cysteine methyltransferase |
| PPID | Peptidyl-prolyl cis-trans isomerase D | Peptidyl-prolyl cis-trans isomerase D |
| STAT5B | Signal transducer and activator of transcription 5B | Signal transducer and activator of transcription 5B |
| TXNRD2 | Thioredoxin reductase 2, mitochondrial | Thioredoxin reductase 2, mitochondrial |
| TXNRD3 | Thioredoxin reductase 3 | Thioredoxin reductase 3 |
| STK39 | STE20/SPS1-related proline-alanine-rich protein kinase | STE20/SPS1-related proline-alanine-rich protein kinase |
| STK38L | Serine/threonine-protein kinase 38-like | Serine/threonine-protein kinase 38-like |
| CDC42BPA | Serine/threonine-protein kinase MRCK alpha | Serine/threonine-protein kinase MRCK alpha |
| ATG4B | Cysteine protease ATG4B | Cysteine protease ATG4B |

(continued)

| abbreviation of target protein | target protein’s name | Chinese translation of target protein’s name |
|---|---|---|
| DCLK2 | Serine/threonine-protein kinase DCLK2 | Serine/threonine-protein kinase DCLK2 |
| TUBAL3 | Tubulin alpha chain-like 3 | Tubulin alpha chain-like 3 |
| MAP3K3 | Mitogen-activated protein kinase kinase kinase 3 | Mitogen-activated protein kinase kinase kinase 3 |
| PPME1 | Protein phosphatase methylesterase 1 | Protein phosphatase methylesterase 1 |
| RPLP1 | 60S acidic ribosomal protein P1 | 60S acidic ribosomal protein P1 |
| GSDMD | Gasdermin-D | Gasdermin-D |
| C9 | Complement component C9 [Cleaved into: Complement component C9a; Complement component C9b] | Complement component C9 [Cleaved into: Complement component C9a; Complement component C9b] |
| HMGB1 | High mobility group protein B1 | High mobility group protein B1 |
| DAB2IP | Disabled homolog 2-interacting protein | Disabled homolog 2-interacting protein |
| PHLPP1 | PH domain leucine-rich repeat-containing protein phosphatase 1 | PH domain leucine-rich repeat-containing protein phosphatase 1 |
| PHKA2 | Phosphorylase b kinase regulatory subunit alpha, liver isoform | Phosphorylase b kinase regulatory subunit alpha, liver isoform |
| PHKA1 | Phosphorylase b kinase regulatory subunit alpha, skeletal muscle isoform | Phosphorylase b kinase regulatory subunit alpha, skeletal muscle isoform |
| PHKB | Phosphorylase b kinase regulatory subunit beta | Phosphorylase b kinase regulatory subunit beta |
| EWSR1 | RNA-binding protein EWS | RNA-binding protein EWS |
| SLC16A3 | Monocarboxylate transporter 4 | Monocarboxylate transporter 4 |
| PTPRE | Receptor-type tyrosine-protein phosphatase epsilon | Receptor-type tyrosine-protein phosphatase epsilon |
| NEU1 | Sialidase-1 | Sialidase-1 |
| PCSK6 | Proprotein convertase subtilisin/kexin type 6 | Proprotein convertase subtilisin/kexin type 6 |
| ANTXR2 | Anthrax toxin receptor 2 | Anthrax toxin receptor 2 |
| STIM1 | Stromal interaction molecule 1 | Stromal interaction molecule 1 |
| DNM2 | Dynamin-2 | Dynamin-2 |
| SDHB | Succinate dehydrogenase [ubiquinone] iron-sulfur subunit, mitochondrial | Succinate dehydrogenase [ubiquinone] iron-sulfur subunit, mitochondrial |
| MAPKAP1 | Target of rapamycin complex 2 subunit MAPKAP1 | Target of rapamycin complex 2 subunit MAPKAP1 |
| PTGES2 | Prostaglandin E synthase 2 | Prostaglandin E synthase 2 |
| TMEM97 | Sigma intracellular receptor 2 | Sigma intracellular receptor 2 |
| SPPL2A | Signal peptide peptidase-like 2A | Signal peptide peptidase-like 2A |
| SLC7A11 | Cystine/glutamate transporter | Cystine/glutamate transporter |
| ABHD6 | Monoacylglycerol lipase ABHD6 | Monoacylglycerol lipase ABHD6 |
| DGKA | Diacylglycerol kinase alpha | Diacylglycerol kinase alpha |

(continued)

| abbreviation of target protein | target protein's name | Chinese translation of target protein's name |
|---|---|---|
| NAPRT | Nicotinate phosphoribosyltransferase | Nicotinate phosphoribosyltransferase |
| BAX | Apoptosis regulator BAX | Apoptosis regulator BAX |
| SPTLC1 | Serine palmitoyltransferase 1 | Serine palmitoyltransferase 1 |
| SPTLC2 | Serine palmitoyltransferase 2 | Serine palmitoyltransferase 2 |
| MGAT1 | Alpha-1,3-mannosyl-glycoprotein 2-beta-N-acetylglucosaminyltransferase | Alpha-1,3-mannosyl-glycoprotein 2-beta-N-acetylglucosaminyltransferase |
| EPHX1 | Epoxide hydrolase 1 | Epoxide hydrolase 1 |
| FADS1 | Acyl-CoA | Acyl-CoA |
| ABHD5 | 1-acylglycerol-3-phosphate O-acyltransferase ABHD5 | 1-acylglycerol-3-phosphate O-acyltransferase ABHD5 |
| CBFB | Core-binding factor subunit beta | Core-binding factor subunit beta |
| POLR1A | DNA-directed RNA polymerase I subunit RPA1 | DNA-directed RNA polymerase I subunit RPA1 |
| MPG | DNA-3-methyladenine glycosylase | DNA-3-methyladenine glycosylase |
| GYS1 | Glycogen [starch] synthase, muscle | Glycogen [starch] synthase, muscle |
| PRKAG2 | 5'-AMP-activated protein kinase subunit gamma-2 | 5'-AMP-activated protein kinase subunit gamma-2 |
| ASF1A | Histone chaperone ASF1A | Histone chaperone ASF1A |
| MED23 | Mediator of RNA polymerase II transcription subunit 23 | Mediator of RNA polymerase II transcription subunit 23 |
| PFKFB4 | 6-phosphofructo-2-kinase/fructose-2,6-bisphosphatase 4 | 6-phosphofructo-2-kinase/fructose-2,6-bisphosphatase 4 |
| KDM3A | Lysine-specific demethylase 3A | Lysine-specific demethylase 3A |
| USP9X | Probable ubiquitin carboxyl-terminal hydrolase FAF-X | Probable ubiquitin carboxyl-terminal hydrolase FAF-X |
| MAX | Protein max | Protein max |
| MBD2 | Methyl-CpG-binding domain protein 2 | Methyl-CpG-binding domain protein 2 |
| CBX8 | Chromobox protein homolog 8 | Chromobox protein homolog 8 |
| FLI1 | Friend leukemia integration 1 transcription factor | Friend leukemia integration 1 transcription factor |
| ATRIP | ATR-interacting protein | ATR-interacting protein |
| RICTOR | Rapamycin-insensitive companion of mTOR | Rapamycin-insensitive companion of mTOR |
| UBLCP1 | Ubiquitin-like domain-containing CTD phosphatase 1 | Ubiquitin-like domain-containing CTD phosphatase 1 |
| DCTPP1 | dCTP pyrophosphatase 1 | dCTP pyrophosphatase 1 |
| TCF4 | Transcription factor 4 | Transcription factor 4 |
| CNOT7 | CCR4-NOT transcription complex subunit 7 | CCR4-NOT transcription complex subunit 7 |
| OGFRL1 | Opioid growth factor receptor-like protein 1 | Opioid growth factor receptor-like protein 1 |
| MVD | Diphosphomevalonate decarboxylase | Diphosphomevalonate decarboxylase |
| IL6ST | Interleukin-6 receptor subunit beta | Interleukin-6 receptor subunit beta |

(continued)

| abbreviation of target protein | target protein's name | Chinese translation of target protein's name |
|---|---|---|
| DOCK2 | Dedicator of cytokinesis protein 2 | Dedicator of cytokinesis protein 2 |
| C3 | Complement C3 | Complement C3 |
| PPM1A | Protein phosphatase 1A | Protein phosphatase 1A |
| SHC1 | SHC-transforming protein 1 | SHC-transforming protein 1 |
| DVL2 | Segment polarity protein dishevelled homolog DVL-2 | Segment polarity protein dishevelled homolog DVL-2 |
| NPR3 | Atrial natriuretic peptide receptor 3 | Atrial natriuretic peptide receptor 3 |
| GSTM1 | Glutathione S-transferase Mu 1 | Glutathione S-transferase Mu 1 |
| PPP3CA | Serine/threonine-protein phosphatase 2B catalytic subunit alpha isoform | Serine/threonine-protein phosphatase 2B catalytic subunit alpha isoform |
| SLC12A2 | Solute carrier family 12 member 2 | Solute carrier family 12 member 2 |
| ABCC3 | ATP-binding cassette sub-family C member 3 | ATP-binding cassette sub-family C member 3 |
| ABCC4 | ATP-binding cassette sub-family C member 4 | ATP-binding cassette sub-family C member 4 |
| GGH | Gamma-glutamyl hydrolase | Gamma-glutamyl hydrolase |
| HCN3 | Potassium/sodium hyperpolarization-activated cyclic nucleotide-gated channel 3 | Potassium/sodium hyperpolarization-activated cyclic nucleotide-gated channel 3 |
| ADCY1 | Adenylate cyclase type 1 | Adenylate cyclase type 1 |
| ADCY5 | Adenylate cyclase type 5 | Adenylate cyclase type 5 |
| CYP4F2 | Cytochrome P450 4F2 | Cytochrome P450 4F2 |
| HKDC1 | Hexokinase HKDC1 | Hexokinase HKDC1 |
| PPT1 | Palmitoyl-protein thioesterase 1 | Palmitoyl-protein thioesterase 1 |
| PRNP | Major prion protein | Major prion protein |
| CXCR5 | C-X-C chemokine receptor type 5 | C-X-C chemokine receptor type 5 |
| SMPD2 | Sphingomyelin phosphodiesterase 2 | Sphingomyelin phosphodiesterase 2 |
| SLC6A15 | Sodium-dependent neutral amino acid transporter B(0)AT2 | Sodium-dependent neutral amino acid transporter B(0)AT2 |
| MANBA | Beta-mannosidase | Beta-mannosidase |
| AGPAT2 | 1-acyl-sn-glycerol-3-phosphate acyltransferase beta | 1-acyl-sn-glycerol-3-phosphate acyltransferase beta |
| ABHD16A | Phosphatidylserine lipase ABHD16A | Phosphatidylserine lipase ABHD16A |
| GCLC | Glutamate-cysteine ligase catalytic subunit | Glutamate-cysteine ligase catalytic subunit |
| HAGH | Hydroxyacylglutathione hydrolase, mitochondrial | Hydroxyacylglutathione hydrolase, mitochondrial |
| SENP1 | Sentrin-specific protease 1 | Sentrin-specific protease 1 |
| DNAJA1 | DnaJ homolog subfamily A member 1 | DnaJ homolog subfamily A member 1 |
| SCP2 | Sterol carrier protein 2 | Sterol carrier protein 2 |
| AMPD2 | AMP deaminase 2 | AMP deaminase 2 |
| ERCCS | DNA repair protein complementing XP-G cells | DNA repair protein complementing XP-G cells |

(continued)

| abbreviation of target protein | target protein's name | Chinese translation of target protein's name |
|---|---|---|
| SENP7 | Sentrin-specific protease 7 | Sentrin-specific protease 7 |
| RBPJ | Recombining binding protein suppressor of hairless | Recombining binding protein suppressor of hairless |
| USP47 | Ubiquitin carboxyl-terminal hydrolase 47 | Ubiquitin carboxyl-terminal hydrolase 47 |
| NADK | NAD kinase | NAD kinase |
| RBBP9 | Serine hydrolase RBBP9 | Serine hydrolase RBBP9 |
| CD2 | T-cell surface antigen CD2 | T-cell surface antigen CD2 |
| CD44 | CD44 antigen | CD44 antigen |
| SLC27A2 | Very long-chain acyl-CoA synthetase | Very long-chain acyl-CoA synthetase |
| PLEKHA1 | Pleckstrin homology domain-containing family A member 1 | Pleckstrin homology domain-containing family A member 1 |
| ITGA3 | Integrin alpha-3 | Integrin alpha-3 |
| CD47 | Leukocyte surface antigen CD47 | Leukocyte surface antigen CD47 |
| SPPL2B | Signal peptide peptidase-like 2B | Signal peptide peptidase-like 2B |
| PLTP | Phospholipid transfer protein | Phospholipid transfer protein |
| CD63 | CD63 antigen | CD63 antigen |
| SLC20A2 | Sodium-dependent phosphate transporter 2 | Sodium-dependent phosphate transporter 2 |
| ADCY6 | Adenylate cyclase type 6 | Adenylate cyclase type 6 |
| IGF2R | Cation-independent mannose-6-phosphate receptor | Cation-independent mannose-6-phosphate receptor |
| EZR | Ezrin | Ezrin |
| GNAQ | Guanine nucleotide-binding protein G(q) subunit alpha | Guanine nucleotide-binding protein G(q) subunit alpha |
| INPP5A | Inositol polyphosphate-5-phosphatase A | Inositol polyphosphate-5-phosphatase A |
| CRACR2A | EF-hand calcium-binding domain-containing protein 4B | EF-hand calcium-binding domain-containing protein 4B |
| LANCL2 | LanC-like protein 2 | LanC-like protein 2 |
| SMPD3 | Sphingomyelin phosphodiesterase 3 | Sphingomyelin phosphodiesterase 3 |
| EHD1 | EH domain-containing protein 1 | EH domain-containing protein 1 |
| PDIA6 | Protein disulfide-isomerase A6 | Protein disulfide-isomerase A6 |
| CHN2 | Beta-chimaerin | Beta-chimaerin |
| CDH2 | Cadherin-2 | Cadherin-2 |
| TFPI | Tissue factor pathway inhibitor | Tissue factor pathway inhibitor |
| CD58 | Lymphocyte function-associated antigen 3 | Lymphocyte function-associated antigen 3 |
| SLC2A8 | Solute carrier family 2, facilitated glucose transporter member 8 | Solute carrier family 2, facilitated glucose transporter member 8 |
| PTPRM | Receptor-type tyrosine-protein phosphatase mu | Receptor-type tyrosine-protein phosphatase mu |

(continued)

| abbreviation of target protein | target protein's name | Chinese translation of target protein's name |
|---|---|---|
| STIM2 | Stromal interaction molecule 2 | Stromal interaction molecule 2 |
| ADCY7 | Adenylate cyclase type 7 | Adenylate cyclase type 7 |
| SLC20A1 | Sodium-dependent phosphate transporter 1 | Sodium-dependent phosphate transporter 1 |
| ITGA1 | Integrin alpha-1 | Integrin alpha-1 |
| RABGGTA | Geranylgeranyl transferase type-2 subunit alpha | Geranylgeranyl transferase type-2 subunit alpha |
| MAP1B | Microtubule-associated protein 1B | Microtubule-associated protein 1B |
| PAFAH1B2 | Platelet-activating factor acetylhydrolase IB subunit alpha2 | Platelet-activating factor acetylhydrolase IB subunit alpha2 |
| RIN1 | Ras and Rab interactor 1 | Ras and Rab interactor 1 |
| PLCD1 | 1-phosphatidylinositol 4,5-bisphosphate phosphodiesterase delta-1 | 1-phosphatidylinositol 4,5-bisphosphate phosphodiesterase delta-1 |
| RGS19 | Regulator of G-protein signaling 19 | Regulator of G-protein signaling 19 |
| PNPLA2 | Patatin-like phospholipase domain-containing protein 2 | Patatin-like phospholipase domain-containing protein 2 |
| ST3GAL3 | CMP-N-acetylneuraminate-beta-1,4-galactoside alpha-2,3-sialyltransferase | CMP-N-acetylneuraminate-beta-1,4-galactoside alpha-2,3-sialyltransferase |
| GALNT1 | Polypeptide N-acetylgalactosaminyltransferase 1 | Polypeptide N-acetylgalactosaminyltransferase 1 |
| ATP6V1B2 | V-type proton ATPase subunit B, brain isoform | V-type proton ATPase subunit B, brain isoform |
| ATP6AP1 | V-type proton ATPase subunit S1 | V-type proton ATPase subunit S1 |
| PHLPP2 | PH domain leucine-rich repeat-containing protein phosphatase 2 | PH domain leucine-rich repeat-containing protein phosphatase 2 |
| GNPAT | Dihydroxyacetone phosphate acyltransferase | Dihydroxyacetone phosphate acyltransferase |
| PPP3CB | Serine/threonine-protein phosphatase 2B catalytic subunit beta isoform | Serine/threonine-protein phosphatase 2B catalytic subunit beta isoform |
| SLC25A20 | Mitochondrial carnitine/acylcarnitine carrier protein | Mitochondrial carnitine/acylcarnitine carrier protein |
| ELOVL1 | Elongation of very long chain fatty acids protein 1 | Elongation of very long chain fatty acids protein 1 |
| MAN2A1 | Alpha-mannosidase 2 | Alpha-mannosidase 2 |
| SLC33A1 | Acetyl-coenzyme A transporter 1 | Acetyl-coenzyme A transporter 1 |
| GAB1 | GRB2-associated-binding protein 1 | GRB2-associated-binding protein 1 |
| ARHGEF12 | Rho guanine nucleotide exchange factor 12 | Rho guanine nucleotide exchange factor 12 |
| MAP2 | Microtubule-associated protein 2 | Microtubule-associated protein 2 |
| MBTD1 | MBT domain-containing protein 1 | MBT domain-containing protein 1 |
| PNKP | Bifunctional polynucleotide phosphatase/kinase | Bifunctional polynucleotide phosphatase/kinase |
| NLRP1 | NACHT, LRR and PYD domains-containing protein 1 | NACHT, LRR and PYD domains-containing protein 1 |

(continued)

| abbreviation of target protein | target protein's name | Chinese translation of target protein's name |
|---|---|---|
| TXN2 | Thioredoxin, mitochondrial | Thioredoxin, mitochondrial |
| EEF1A2 | Elongation factor 1-alpha 2 | Elongation factor 1-alpha 2 |
| GOT1 | Aspartate aminotransferase, cytoplasmic | Aspartate aminotransferase, cytoplasmic |
| PRMT7 | Protein arginine N-methyltransferase 7 | Protein arginine N-methyltransferase 7 |
| MYH10 | Myosin-10 | Myosin-10 |
| TPP2 | Tripeptidyl-peptidase 2 | Tripeptidyl-peptidase 2 |
| EYA3 | Eyes absent homolog 3 | Eyes absent homolog 3 |
| SSU72 | RNA polymerase II subunit A C-terminal domain phosphatase SSU72 | RNA polymerase II subunit A C-terminal domain phosphatase SSU72 |
| AMDHD2 | N-acetylglucosamine-6-phosphate deacetylase | N-acetylglucosamine-6-phosphate deacetylase |
| CLPX | ATP-dependent Clp protease ATP-binding subunit clpX-like, mitochondrial | ATP-dependent Clp protease ATP-binding subunit clpX-like, mitochondrial |
| MECP2 | Methyl-CpG-binding protein 2 | Methyl-CpG-binding protein 2 |
| KPNA2 | Importin subunit alpha-1 | Importin subunit alpha-1 |
| PSMG1 | Proteasome assembly chaperone 1 | Proteasome assembly chaperone 1 |
| CBX6 | Chromobox protein homolog 6 | Chromobox protein homolog 6 |
| PPP2R2A | Serine/threonine-protein phosphatase 2A 55 kDa regulatory subunit B alpha isoform | Serine/threonine-protein phosphatase 2A 55 kDa regulatory subunit B alpha isoform |
| UBE2V1 | Ubiquitin-conjugating enzyme E2 variant 1 | Ubiquitin-conjugating enzyme E2 variant 1 |
| PSMG2 | Proteasome assembly chaperone 2 | Proteasome assembly chaperone 2 |
| PPIC | Peptidyl-prolyl cis-trans isomerase C | Peptidyl-prolyl cis-trans isomerase C |
| PGK2 | Phosphoglycerate kinase 2 | Phosphoglycerate kinase 2 |

In some embodiments, the PBM is a small molecule ligand that targets specific target proteins including but not limited to EGFR, CDK4/6, ALK, RET, FAK, BCR-ABL, BTK, AR, ER or BET.

In some embodiments, the PBM comprises the structures of the following formula:

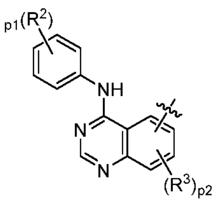

Formula (PBM-1)

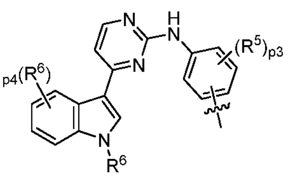

Formula (PBM-2)

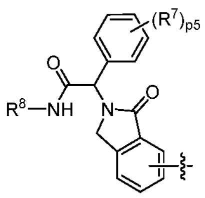

Formula (PBM-3)

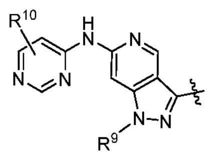

Formula (PBM-4)

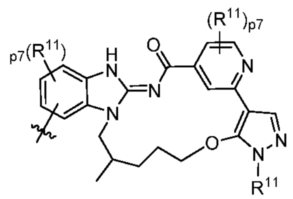

Formula (PBM-5)

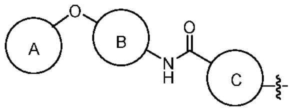

Formula (PBM-6)

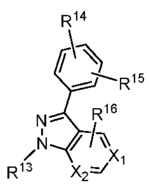

Formula (PBM-7)

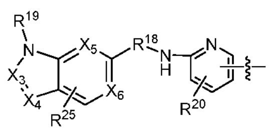

Formula (PBM-8)

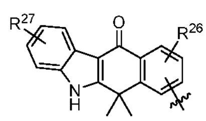

Formula (PBM-9)

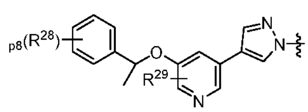

Formula (PBM-10)

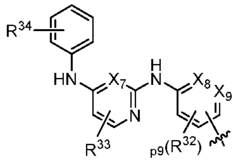
Formula (PBM-11)

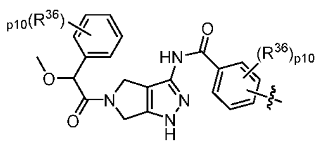
Formula (PBM-12)

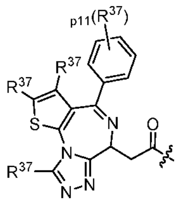
Formula (PBM-13)

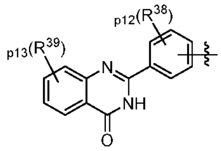
Formula (PBM-14)

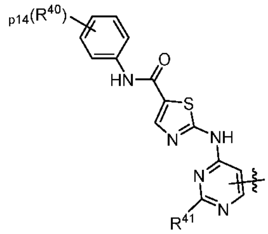
Formula (PBM-15)

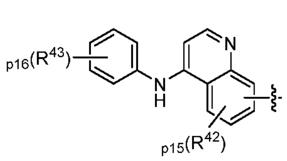
Formula (PBM-16)

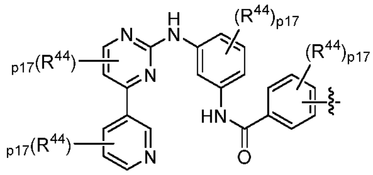
Formula (PBM-17)

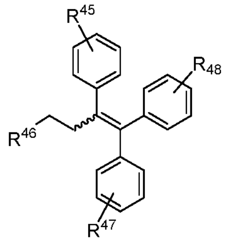
Formula (PBM-18)

or

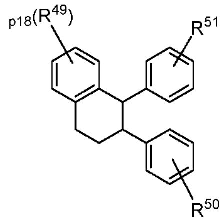
Formula (PBM-19) ;

wherein

$(R^2)_{p1}$ indicates that the benzene ring in Formula (PBM-1) is substituted with p1 $R^2$, and each $R^2$ are the same or different and each independently represent halogen or halogenated $C_{1-6}$ alkyl;
$(R^3)_{p2}$ indicates that the quinazoline ring in Formula (PBM-1) is substituted with p2 $R^3$, and each $R^3$ are the same or different and each independently represent $C_{1-10}$ alkyl, deuterated $C_{1-10}$ alkyl, $C_{1-10}$ alkoxy, heterocyclyloxy, or -NHC(O)$R^4$, wherein $R^4$ is $C_{1-10}$ alkyl or $C_{2-10}$ alkenyl, e.g., $R^3$ represents methyl, methoxy,

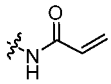 or 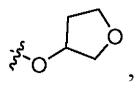 ,

such as

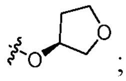 ;

p1 represents an integer of 1, 2, 3, 4 or 5;
p2 represents an integer of 1, 2, or 3;
$(R^5)_{p3}$ indicates that the benzene ring in Formula (PBM-2) is substituted with p3 $R^5$, and each $R^5$ are the same or different and each independently represent $C_{1-10}$ alkyl, deuterated $C_{1-10}$ alkyl, $C_{1-10}$ alkoxy, or -NHC(O)$R^4$, wherein $R^4$ is $C_{1-10}$ alkyl, deuterated $C_{1-10}$ alkyl or $C_{2-10}$ alkenyl, e.g., $R^5$ represents methyl, methoxy or

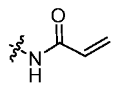 ;

p3 represents an integer of 1, 2, 3 or 4;
$(R^6)_{p4}$ indicates that the 1H-indole ring in Formula (PBM-2) is substituted with p4 $R^6$, and each $R^6$ are the same or different and each independently represent $C_{1-10}$ alkyl or deuterated $C_{1-10}$ alkyl;
p4 represents an integer of 0, 1, 2 or 3;
$(R^7)_{p5}$ indicates that the benzene ring in Formula (PBM-3) is substituted with p5 $R^7$, and each $R^7$ are the same or different and each independently represent halogen, hydroxy, $C_{1-6}$ alkyl, halogenated $C_{1-6}$ alkyl, deuterated $C_{1-6}$ alkyl, $NO_2$, $NH_2$, or cyano;

p5 represents an integer of 2, 3, 4 or 5;
$R^8$ represents $C_{6-10}$ aryl or heteroaryl containing one or two 5- to 7-membered rings and 1 to 4 heteroatoms selected from nitrogen, oxygen, and sulfur, wherein the $C_{6-10}$ aryl and heteroaryl are each optionally substituted with one or more $R^{4a}$, and each $R^{4a}$ are the same or different and are each independently halogen, hydroxy, cyano, amino, nitro, $C_{1-6}$ alkyl, halogenated $C_{1-6}$ alkyl, deuterated $C_{1-6}$ alkyl, $C_{1-6}$ alkoxy, or halogenated $C_{1-6}$ alkoxy;
$R^9$ represents $C_{1-10}$ alkyl (e.g.,

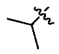

) or deuterated $C_{1-10}$ alkyl;
$R^{10}$ represents

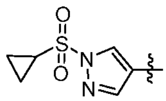 or 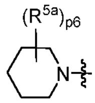,

wherein $(R^{5a})_{p6}$ indicates that the piperidine ring is substituted with p6 $R^{5a}$, and each $R^{5a}$ are the same or different and each independently represent deuterium, halogen, hydroxy, $C_{1-6}$ alkyl, halogenated $C_{1-6}$ alkyl, deuterated $C_{1-6}$ alkyl, $C_{1-6}$ alkoxy, deuterated $C_{1-6}$ alkoxy, $NO_2$, $NH_2$, or cyano, p6 represents an integer of 1, 2, 3, 4 or 5;
$(R^{11})_{p7}$ indicates that the benzene ring and the pyridine ring in Formula (PBM-5) are each optionally substituted with p7 $R^{11}$, and each $R^{11}$ are the same or different and each independently represent halogen, $C_{1-6}$ alkyl, halogenated $C_{1-6}$ alkyl, or deuterated $C_{1-6}$ alkyl, p7 represents an integer of 0, 1, 2 or 3;
ring A in Formula (PBM-6) represents $C_{6-10}$ aryl or heteroaryl containing one or two 5- to 7-membered rings and from 1 to 4 heteroatoms selected from the group consisting of oxygen, nitrogen, and sulfur, and the ring A is optionally substituted with one or more $R^{6a}$, and each $R^{6a}$ are the same or different and each independently represent deuterium, halogen, hydroxy, cyano, amino, nitro, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{2-6}$ alkenyl, or $C_{2-6}$ alkynyl;
ring B in Formula (PBM-6) represents $C_{3-15}$ cycloalkyl or heterocyclyl containing one or two 3-to 7-membered rings and from 1 to 4 heteroatoms selected from the group consisting of oxygen, nitrogen, and sulfur, and the ring B is optionally substituted with one or more $R^{7a}$, and each $R^{7a}$ are the same or different and each independently represent deuterium, halogen, $C_{1-6}$ alkyl, halogenated $C_{1-6}$ alkyl, deuterated $C_{1-6}$ alkyl, $C_{1-6}$ alkoxy, or halogenated $C_{1-6}$ alkoxy;
ring C in Formula (PBM-6) represents heteroaryl containing one or two 5- to 7-membered rings and from 1 to 4 heteroatoms selected from the group consisting of oxygen, nitrogen, and sulfur, and the ring C is optionally substituted with one or more $R^{8a}$, and each $R^{8a}$ are the same or different and each independently represent deuterium, halogen, hydroxy, cyano, amino, $C_{1-6}$ alkyl, halogenated $C_{1-6}$ alkyl, deuterated $C_{1-6}$ alkyl, $C_{1-6}$ alkoxy, or halogenated $C_{1-6}$ alkoxy;
$X_1$ in Formula (PBM-7) represents N or $CR^{12}$, wherein $R^{12}$ is hydrogen or represents a bond, and $X_2$ represents CH or N;
$R^{13}$ is hydrogen or represents a bond, wherein when $R^{13}$ represents a bond, $X_1$ represents N or CH, and the nitrogen atom in the ring connected to $R^{13}$ is directly connected to $R^1$; or when $R^{13}$ is hydrogen, $X_1$ represents $CR^{12}$, wherein $R^{12}$ represents a bond, and the carbon atom in the ring connected to $R^{12}$ is directly connected to $R^1$;
$R^{14}$ represents -O-aryl, -O-heteroaryl, -$C_{1-3}$-alkylene-NHC(O)-heteroaryl, or -$C_{1-3}$-alkylene-C(O)NH-heteroaryl, wherein the aryl and heteroaryl are each independently optionally substituted with one or more $R^{9a}$, and each $R^{9a}$ are the same or different and each independently represent deuterium, halogen, hydroxy, cyano, amino, $C_{1-6}$ alkyl, halogenated $C_{1-6}$ alkyl, deuterated $C_{1-6}$ alkyl, $C_{1-6}$ alkoxy, or halogenated $C_{1-6}$ alkoxy;
$R^{15}$ represents deuterium, hydrogen, halogen, $C_{1-6}$ alkyl, halogenated $C_{1-6}$ alkyl, deuterated $C_{1-6}$ alkyl or -NHC(O)$R^{17}$, wherein $R^{17}$ is $C_{1-10}$ alkyl or $C_{2-10}$ alkenyl, e.g., $R^{15}$ represents hydrogen, methyl,

or 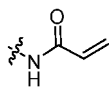;

$R^{16}$ represents hydrogen, cyano, or amino;
$X_3$ in Formula (PBM-8) represents $CR^{21}$ or a fragment

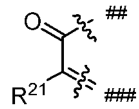
,

wherein symbol ## indicates the point of attachment to N atom adjacent to $X_3$, symbol ### indicates the point of attachment to $X_4$, and $R^{21}$ represents deuterium, hydrogen, halogen, $C_{1-6}$ alkyl, halogenated $C_{1-6}$ alkyl, deuterated $C_{1-6}$ alkyl, -C(O)-$C_{1-6}$ alkyl, -C(O)-deuterated $C_{1-6}$ alkyl, or -C(O)$NR^{22}R^{23}$, wherein $R^{22}$ and $R^{23}$ are the same or different and each independently represent hydrogen, $C_{1-6}$ alkyl or deuterated $C_{1-6}$ alkyl, and $R^{22}$ and $R^{23}$ are not simultaneously hydrogen;

$X_4$ in Formula (PBM-8) represents N or $CR^{24}$, wherein $R^{24}$ represents hydrogen, deuterium, halogen, $C_{1-6}$ alkyl, deuterated $C_{1-6}$ alkyl or halogenated $C_{1-6}$ alkyl;

$X_5$ and $X_6$ each independently represents CH or N;

$R^{18}$ represents a bond or optionally substituted heteroarylene (e.g., halogenated heteroarylene);

$R^{19}$ represents hydrogen, $C_{1-6}$ alkyl, deuterated $C_{1-6}$ alkyl, halogenated $C_{1-6}$ alkyl, or optionally substituted $C_{3-7}$ cycloalkyl;

$R^{20}$ and $R^{25}$ are the same or different and each independently represent hydrogen, halogen, $C_{1-6}$ alkyl, deuterated $C_{1-6}$ alkyl or halogenated $C_{1-6}$ alkyl;

$R^{26}$ represents hydrogen, halogen, $C_{1-6}$ alkyl, deuterated $C_{1-6}$ alkyl or halogenated $C_{1-6}$ alkyl;

$R^{27}$ represents hydrogen, cyano, halogen, $C_{1-6}$ alkyl, deuterated $C_{1-6}$ alkyl or halogenated $C_{1-6}$ alkyl;

$(R^{28})_{p8}$ indicates that the benzene ring in Formula (PBM-10) is substituted with p8 $R^{28}$ substituents, and each $R^{28}$ are the same or different and each independently represent halogen, $C_{1-6}$ alkyl, deuterated $C_{1-6}$ alkyl or halogenated $C_{1-6}$ alkyl;

p8 represents an integer of 1, 2, 3 or 4;

$R^{29}$ represents $NR^{30}R^{31}$, wherein $R^{30}$ and $R^{31}$ are the same or different and each independently represent hydrogen, $C_{1-6}$ alkyl, or deuterated $C_{1-6}$ alkyl;

$X_7$, $X_8$ and $X_9$ in Formula (PBM-11) are the same or different and each independently represent N or CH;

$(R^{32})_{p9}$ indicates that the 6-membered ring containing $X_8$ and $X_9$ in Formula (PBM-11) is optionally substituted with p9 $R^{32}$ groups, and each $R^{32}$ are the same or different and each independently represent halogen, $C_{1-6}$ alkoxy, $C_{1-6}$ alkyl, deuterated $C_{1-6}$ alkyl or halogenated $C_{1-6}$ alkyl;

p9 represents an integer of 0, 1, 2, 3 or 4;

$R^{33}$ represents halogen, $C_{1-6}$ alkoxy, $C_{1-6}$ alkyl, deuterated $C_{1-6}$ alkyl or halogenated $C_{1-6}$ alkyl;

$R^{34}$ represents -C(O)$NHR^{35}$, -NHC(O)-$R^{35}$, -$S(O)_2NHR^{35}$, -$S(O)_2R^{35}$ or -$P(O)(R^{35})_2$, wherein each $R^{35}$ are the same or different and each independently represent $C_{1-6}$ alkyl or deuterated $C_{1-6}$ alkyl;

$(R^{36})_{p10}$ indicates that the benzene ring in Formula (PBM-12) is optionally substituted with p10 $R^{36}$ groups, and each $R^{36}$ are the same or different and each independently represent halogen, $C_{1-6}$ alkoxy, $C_{1-6}$ alkyl, deuterated $C_{1-6}$ alkyl or halogenated $C_{1-6}$ alkyl;

p10 represents an integer of 0, 1, 2, 3 or 4;

$(R^{37})_{p11}$ indicates that the benzene ring in Formula (PBM-13) is substituted with p11 $R^{37}$ groups, and each $R^{37}$ are the same or different and each independently represent halogen, $C_{1-6}$ alkoxy, $C_{1-6}$ alkyl, deuterated $C_{1-6}$ alkyl or halogenated $C_{1-6}$ alkyl;

p11 represents an integer of 0, 1, 2, 3 or 4;

$(R^{38})_{p12}$ indicates that the benzene ring in Formula (PBM-14) is substituted with p12 $R^{38}$ groups, and each $R^{38}$ are the same or different and each independently represent halogen, $C_{1-6}$ alkoxy, $C_{1-6}$ alkyl, deuterated $C_{1-6}$ alkyl or halogenated $C_{1-6}$ alkyl;

p12 represents an integer of 0, 1, 2, 3 or 4;

$(R^{39})_{p13}$ indicates that the 4-oxo-3,4-dihydroquinazoline ring in Formula (PBM-14) is substituted with p13 $R^{39}$ groups, and each $R^{39}$ are the same or different and each independently represent halogen, $C_{1-6}$ alkoxy, $C_{1-6}$ alkyl, deuterated $C_{1-6}$ alkyl or halogenated $C_{1-6}$ alkyl;

p13 represents an integer of 1, 2, 3 or 4;

$(R^{40})_{p14}$ indicates that the benzene ring in Formula (PBM-15) is substituted with p14 $R^{40}$ groups, and each $R^{40}$ are the same or different and each independently represent halogen, $C_{1-6}$ alkoxy, $C_{1-6}$ alkyl, deuterated $C_{1-6}$ alkyl or halogenated $C_{1-6}$ alkyl;

p14 represents an integer of 1, 2, 3 or 4;

$R^{41}$ represents halogen, $C_{1-6}$ alkyl, deuterated $C_{1-6}$ alkyl or halogenated $C_{1-6}$ alkyl;

$(R^{42})_{p15}$ indicates that the quinoline ring in Formula (PBM-16) is substituted with p15 $R^{42}$ groups, and each $R^{42}$ are the same or different and each independently represent halogen, cyano, $C_{1-6}$ alkoxy, $C_{1-6}$ alkyl, deuterated $C_{1-6}$ alkyl or halogenated $C_{1-6}$ alkyl;

p15 represents an integer of 2, 3 or 4;
$(R^{43})_{p16}$ indicates that the benzene ring in Formula (PBM-16) is substituted with p16 $R^{43}$ groups, and each $R^{43}$ are the same or different and each independently represent halogen, $C_{1-6}$ alkoxy, $C_{1-6}$ alkyl, deuterated $C_{1-6}$ alkyl or halogenated $C_{1-6}$ alkyl;
p16 represents an integer of 3 or 4;
$(R^{44})_{p17}$ indicates that the benzene, pyrimidine and pyridine rings in Formula (PBM-17) are each optionally substituted with p17 $R^{44}$ groups, and each $R^{44}$ are the same or different and each independently represent halogen, $C_{1-6}$ alkoxy, $C_{1-6}$ alkyl, deuterated $C_{1-6}$ alkyl or halogenated $C_{1-6}$ alkyl;
p17 represents an integer of 0, 1, 2, 3 or 4;
one of $R^{45}$, $R^{46}$, $R^{47}$ and $R^{48}$ represents a bond, and the remaining are the same or different and each independently represent hydrogen, halogen, or hydroxy, wherein when one of $R^{45}$, $R^{46}$, $R^{47}$ and $R^{48}$ represents a bond, the benzene ring or methylene in Formula (PBM-18) connected to the bond is directly connected to $R^1$;
symbol "" connected to double bond in Formula (PBM-18) represents a bond in stereo-configuration (cis or trans configuration);
$(R^{49})_{p18}$ indicates that 1,2,3,4-tetrahydronaphthalene ring in Formula (PBM-19) is substituted with p18 $R^{49}$ groups, and each $R^{49}$ are the same or different and each independently represent hydrogen, hydroxy, halogen, $C_{1-6}$ alkyl, deuterated $C_{1-6}$ alkyl or halogenated $C_{1-6}$ alkyl;
p18 represents an integer of 0, 1, 2, 3 or 4; and
one of $R^{50}$ and $R^{51}$ represents a bond, and another represents hydrogen, halogen, $C_{1-6}$ alkyl, deuterated $C_{1-6}$ alkyl or halogenated $C_{1-6}$ alkyl, wherein when one of $R^{50}$ and $R^{51}$ represents a bond, the benzene ring in Formula (PBM-19) connected to the bond is directly connected to $R^1$.

In some embodiments, PBM represents a small molecule ligand targeting EGFR, including but not limited to represent the structures of Formula (PBM-1-1A), Formula (PBM-1-1B), Formula (PBM-1-1C) or Formula (PBM-1-1D):

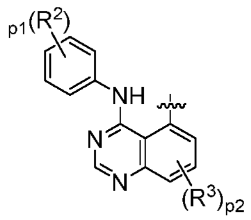
Formula (PBM-1-1A)

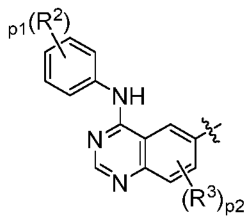
Formula (PBM-1-1B)

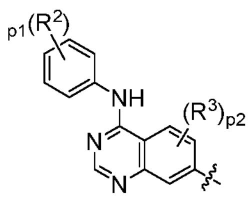
Formula (PBM-1-1C)

or

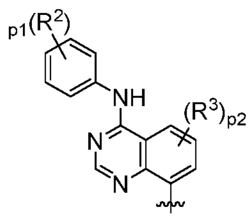
Formula (PBM-1-1D)

wherein $(R^2)_{p1}$ indicates that the benzene ring in Formula (PBM-1-1A), Formula (PBM-1-1B), Formula (PBM-1-1C) or Formula (PBM-1-1D) is substituted with p1 $R^2$, and each $R^2$ are the same or different and each independently represent halogen (e.g., fluorine, chlorine, bromine, or iodine) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);
$(R^3)_{p2}$ indicates that the quinazoline ring in Formula Formula (PBM-1-1A), Formula (PBM-1-1B), Formula (PBM-1-1C) or Formula (PBM-1-1D) is substituted with p2 $R^3$, and each $R^3$ are the same or different and each independently represent $C_{1-10}$ alkyl (e.g., $C_{1-8}$ alkyl, $C_{1-6}$ alkyl or $C_{1-4}$ alkyl, such as methyl, ethyl, propyl, isopropyl, butyl, sec-butyl, tert-butyl, pentyl, hexyl, heptyl or octyl), deuterated $C_{1-10}$ alkyl (e.g., perdeuterated $C_{1-8}$ alkyl, perdeuterated $C_{1-6}$ alkyl or perdeuterated $C_{1-4}$ alkyl, such as $CD_3$, $CD_3CD_2$-, $CD_3CD_2CD_2$- etc.), $C_{1-10}$ alkoxy (e.g., Ci-s alkoxy, $C_{1-6}$ alkoxy or $C_{1-4}$ alkoxy, such as methoxy, ethoxy, propoxy, isopropoxy, butoxy, sec-butoxy or tert-butoxy), heterocyclyloxy (in which the heterocyclyl is, for example, a 4- to 20-membered monocyclic or bicyclic heterocyclyl group containing one or more, such as 1-4 or 1, 2, 3, or 4 heteroatoms selected from nitrogen, oxygen, and sulfur) or -NHC(O)$R^4$, where $R^4$ is $C_{1-10}$ alkyl (e.g., $C_{1-8}$ alkyl, $C_{1-6}$ alkyl or $C_{1-4}$ alkyl, such as methyl, ethyl, propyl, isopropyl, butyl, sec-butyl, tert-butyl, pentyl, hexyl, heptyl or octyl) or $C_{2-10}$ alkenyl (e.g., $C_{2-6}$ alkenyl or $C_{2-4}$ alkenyl, such as vinyl, propenyl, butenyl or pentenyl), e.g., $R^3$ represents methyl, methoxy,

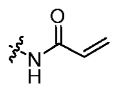 or 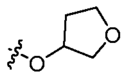,

such as

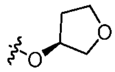
;

p1 represents an integer of 1, 2, 3, 4 or 5; and
p2 represents an integer of 1, 2 or 3;

In some embodiments, $R^3$ represents heterocyclyl-O-, and the heterocyclyl is 4- to 20-membered (e.g., 4- to 15-membered, 4- to 12-membered, 4- to 10-membered, 4- to 7-membered, or 4-to 6-membered) monocyclic or bicyclic heterocyclyl group containing one or more (e.g., 1-4 or 1, 2, 3, or 4) heteroatoms selected from nitrogen, oxygen, and sulfur, and includes but is not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo [2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), diazabicyclo[2.2.2]octanyl (e.g., 2,5-diazabicyclo[2.2.2]octan-2-yl).
In some embodiments, $R^3$ represents:

methyl, ethyl, propyl, isopropyl, butyl, sec-butyl, tert-butyl, pentyl, hexyl, heptyl or octyl;
methoxy, ethoxy, propoxy, isopropoxy, butoxy, sec-butoxy or tert-butoxy;
-NHC(O)-$C_{2-10}$ alkenyl, e.g., -NHC(O)-vinyl, -NHC(O)-propenyl or -NHC(O)-butenyl; or

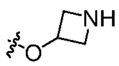 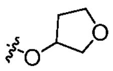 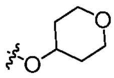 or 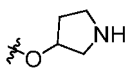 .

In some embodiments, PBM represents the structures of Formula (PBM-1-1), Formula (PBM-1-2), Formula (PBM-1-3) or Formula (PBM-1-4):

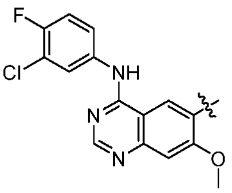
Formula (PBM-1-1)

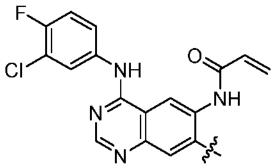
Formula (PBM-1-2)

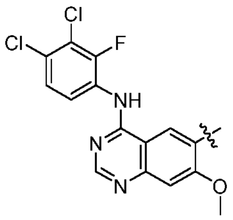
Formula (PBM-1-3)

or

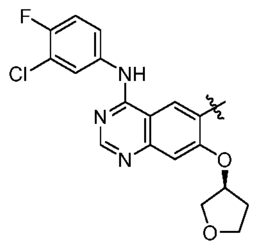
Formula (PBM-1-4)

.

In some embodiments, PBM represents a small molecule ligand targeting EGFR, including but not limited to represent the structures of Formula (PBM-2-1A), Formula (PBM-2-1B), Formula (PBM-2-1C) or Formula (PBM-2-1D):

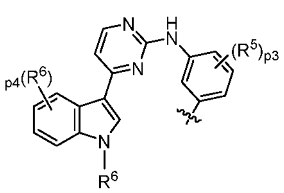
Formula (PBM-2-1A)

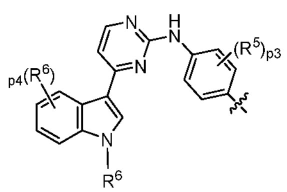
Formula (PBM-2-1B)

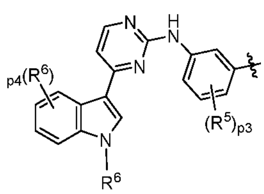
Formula (PBM-2-1C)

or

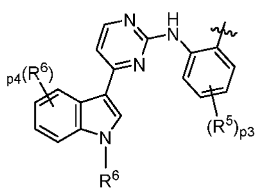
Formula (PBM-2-1D)

wherein $(R^5)_{p3}$ indicates that the benzene ring in Formula (PBM-2-1A), Formula (PBM-2-1B), Formula (PBM-2-1C) or Formula (PBM-2-1D) is substituted with p3 $R^5$, and each $R^5$ are the same or different and each independently represent $C_{1-10}$ alkyl (e.g., $C_{1-8}$ alkyl, $C_{1-6}$ alkyl or $C_{1-4}$ alkyl, such as methyl, ethyl, propyl, isopropyl, butyl, sec-butyl, tert-butyl, pentyl, hexyl, heptyl or octyl), deuterated $C_{1-10}$ alkyl (e.g., perdeuterated $C_{1-8}$ alkyl, perdeuterated $C_{1-6}$ alkyl or perdeuterated $C_{1-4}$ alkyl, such as $CD_3$, $CD_3CD_2$-, $CD_3CD_2CD_2$- etc.), $C_{1-10}$ alkoxy (e.g., Ci-s alkoxy, $C_{1-6}$ alkoxy or $C_{1-4}$ alkoxy, such as methoxy, ethoxy, propoxy, isopropoxy, butoxy, sec-butoxy or tert-butoxy), or -NHC(O)$R^4$, where $R^4$ is $C_{1-10}$ alkyl (e.g., $C_{1-8}$ alkyl, $C_{1-6}$ alkyl or $C_{1-4}$ alkyl, such as methyl, ethyl, propyl, isopropyl,

butyl, sec-butyl, tert-butyl, pentyl, hexyl, heptyl or octyl), deuterated $C_{1-10}$ alkyl (e.g., perdeuterated $C_{1-8}$ alkyl, perdeuterated $C_{1-6}$ alkyl or perdeuterated $C_{1-4}$ alkyl, such as $CD_3$, $CD_3CD_2$-, $CD_3CD_2CD_2$- etc.) or $C_{2-10}$ alkenyl (e.g., $C_{2-6}$ alkenyl or $C_{2-4}$ alkenyl, such as vinyl, propenyl, butenyl or pentenyl), e.g., $R^5$ represents methyl, methoxy or

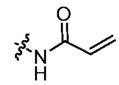
;

p3 represents an integer of 1, 2, 3 or 4;

$(R^6)_{p4}$ indicates that the 1H-indole ring in Formula (PBM-2-1A), Formula (PBM-2-1B), Formula (PBM-2-1C) or Formula (PBM-2-1D) is substituted with p4 $R^6$, and each $R^6$ are the same or different and each independently represent $C_{1-10}$ alkyl (e.g., $C_{1-8}$ alkyl, $C_{1-6}$ alkyl or $C_{1-4}$ alkyl, such as methyl, ethyl, propyl, isopropyl, butyl, sec-butyl, tert-butyl, pentyl, hexyl, heptyl or octyl) or deuterated $C_{1-10}$ alkyl (e.g., perdeuterated $C_{1-8}$ alkyl, perdeuterated $C_{1-6}$ alkyl or perdeuterated $C_{1-4}$ alkyl, such as $CD_3$, $CD_3CD_2$-, $CD_3CD_2CD_2$- etc.); and

p4 represents an integer of 0, 1, 2 or 3.

In some embodiments, PBM represents the structure of Formula (PBM-2-1):

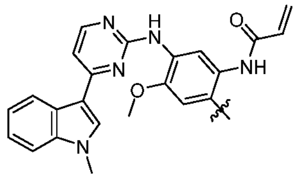
Formula (PBM-2-1)
.

In some embodiments, PBM represents a small molecule ligand targeting EGFR, including but not limited to represent the structures of Formula (PBM-3-1A), Formula (PBM-3-1B), Formula (PBM-3-1C) or Formula (PBM-3-1D):

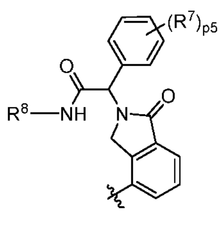
Formula (PBM-3-1A)

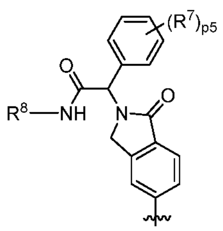
Formula (PBM-3-1B)

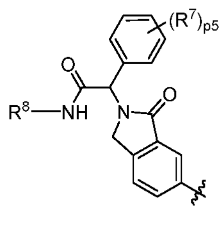
Formula (PBM-3-1C)

or

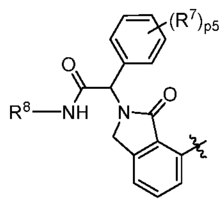
Formula (PBM-3-1D)

wherein $(R^7)_{p5}$ indicates that the benzene ring in Formula (PBM-3-1A), Formula (PBM-3-1B), Formula (PBM-3-1C) or Formula (PBM-3-1D) is substituted with p5 $R^7$, and each $R^7$ are the same or different and each independently represent halogen (e.g., fluorine, chlorine, bromine, or iodine), hydroxy, $C_{1-6}$ alkyl (e.g., $C_{1-8}$ alkyl, $C_{1-6}$ alkyl or $C_{1-4}$ alkyl, such as methyl, ethyl, propyl, isopropyl, butyl, sec-butyl, tert-butyl, pentyl, hexyl, heptyl or octyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $NO_2$, $NH_2$, or cyano;

p5 represents an integer of 2, 3, 4 or 5; and

$R^8$ represents $C_{6-10}$ aryl or heteroaryl containing one or two 5- to 7-membered rings and 1 to 4 heteroatoms selected from nitrogen, oxygen, and sulfur, wherein the $C_{6-10}$ aryl and heteroaryl are each independently optionally substituted with one or more (e.g., 1-6, such as 1, 2, 3, 4, 5 or 6) $R^{4a}$, and each $R^{4a}$ are the same or different and are each independently halogen (e.g., fluorine, chlorine, bromine, or iodine), hydroxy, cyano, amino, nitro, $C_{1-6}$ alkyl (e.g., $C_{1-8}$ alkyl, $C_{1-6}$ alkyl or $C_{1-4}$ alkyl, such as methyl, ethyl, propyl, isopropyl, butyl, sec-butyl, tert-butyl, pentyl, hexyl, heptyl or octyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-,

$CF_3CF_2$-O-, $CF_3CHF$-O-, $CHF_2CF_2$-O-, $CHF_2CHF$-O-, $CF_3CH_2$-O- or $CH_2ClCH_2$-O-).

In some embodiments, $R^8$ represents $C_{6-10}$ aryl, such as but not limited to phenyl or naphthyl, each of which is optionally substituted with one or more (e.g., 1-6, such as 1, 2, 3, 4, 5 or 6) $R^{4a}$ as defined above.

In some embodiments, $R^8$ represents heteroaryl containing one or two 5- to 7-membered rings and 1 to 4 heteroatoms selected from nitrogen, oxygen, and sulfur. In a sub-embodiment, $R^8$ represents 5- to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2a]pyridyl, 1H-pyrrolo[3,2-b]pyridyl, 1H-pyrrolo[2,3-b]pyridyl, pyrrolo[2,1-b]thiazolyl or imidazo[2,1-b]thiazolyl. The heteroaryl group is optionally substituted with one or more (e.g., 1-6, such as 1, 2, 3, 4, 5 or 6) $R^{4a}$ as defined above.

In some embodiments, PBM represents the structure of Formula (PBM-3-1):

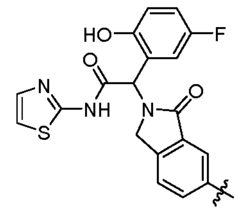

Formula (PBM-3-1) .

In some embodiments, PBM represents a small molecule ligand targeting EGFR, including but not limited to represent the structure of Formula (PBM-4):

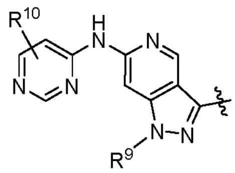

Formula (PBM-4)

wherein $R^9$ represents $C_{1-10}$ alkyl (e.g., $C_{1-8}$ alkyl, $C_{1-6}$ alkyl or $C_{1-4}$ alkyl, such as methyl, ethyl, propyl, isopropyl

(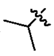),

butyl, sec-butyl, tert-butyl, pentyl, hexyl, heptyl or octyl) or deuterated $C_{1-10}$ alkyl (e.g., perdeuterated $C_{1-8}$ alkyl, perdeuterated $C_{1-6}$ alkyl or perdeuterated $C_{1-4}$ alkyl, such as $CD_3$, $CD_3CD_2$-, $CD_3CD_2CD_2$- etc.); and

$R^{10}$ represents

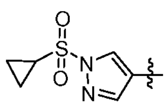 or 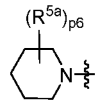

, wherein $(R^{5a})_{p6}$ indicates that the piperidine ring is substituted with p6 $R^{5a}$, and each $R^{5a}$ are the same or different and each independently represent deuterium, halogen (e.g., fluorine, chlorine, bromine, or iodine), hydroxy, $C_{1-6}$ alkyl (e.g., $C_{1-8}$ alkyl, $C_{1-6}$ alkyl or $C_{1-4}$ alkyl, such as methyl, ethyl, propyl, isopropyl, butyl, sec-butyl, tert-butyl, pentyl, hexyl, heptyl or octyl), halogenated $C_{1-6}$ alkyl, deuterated $C_{1-6}$ alkyl (e.g., perdeuterated $C_{1-6}$ alkyl, perdeu-

terated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), deuterated $C_{1-6}$ alkoxy (e.g., perdeuterated $C_{1-4}$ alkoxy, such as $CD_3$-O-, $CD_3CD_2$-O-, or $CD_3CD_2CD_2$-O-), $NO_2$, $NH_2$, or cyano, p6 represents an integer of 1, 2, 3, 4 or 5.

In some embodiments, PBM represents the structures of Formula (PBM-4-1) or Formula (PBM-4-2):

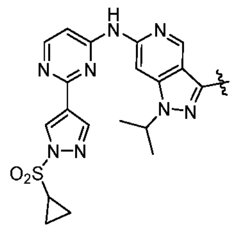
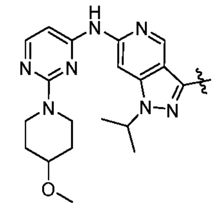

Formula (PBM-4-1) or Formula (PBM-4-2) .

In some embodiments, PBM represents a small molecule ligand targeting EGFR, including but not limited to represent the structure of Formula (PBM-5-1A), Formula (PBM-5-1B), Formula (PBM-5-1C) or Formula (PBM-5-1D):

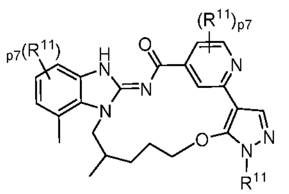

Formula (PBM-5-1A)

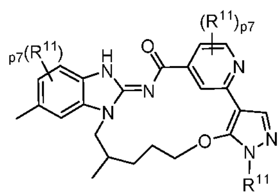

Formula (PBM-5-1B)

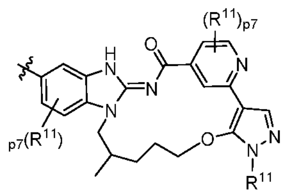

Formula (PBM-5-1C) or

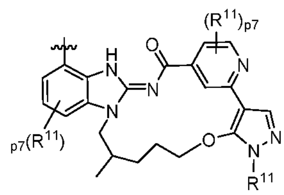

Formula (PBM-5-1D)

wherein $(R^{11})_{p7}$ indicates that the benzene ring and the pyridine ring in Formula (PBM-5-1A), Formula (PBM-5-1B), Formula (PBM-5-1C) or Formula (PBM-5-1D) are each optionally substituted with p7 $R^{11}$, and each $R^{11}$ are the same or different and each independently represent halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), or deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), p7 represents an integer of 0, 1, 2 or 3.

In some embodiments, PBM represents the structure of Formula (PBM-5-1):

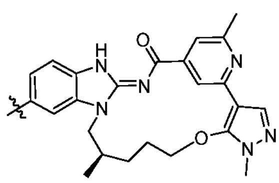

Formula (PBM-5-1) .

In some embodiments, PBM represents a small molecule ligand targeting AR, including but not limited to represent the structure of Formula (PBM-6-1A):

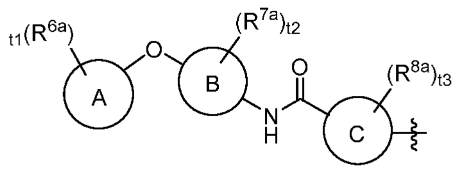

Formula (PBM-6-1A)

wherein ring A in Formula (PBM-6-1A) represents $C_{6-10}$ aryl or 5- to 20-membered monocyclic or bicyclic heteroaryl containing from 1 to 4 heteroatoms selected from the group consisting of oxygen, nitrogen, and sulfur, wherein the ring A is optionally substituted with t1 (e.g., 1-6, such as 1, 2, 3, 4, 5 or 6) $R^{6a}$, and each $R^{6a}$ are the same or different and each independently represent deuterium, halogen (e.g., fluorine, chlorine, bromine, or iodine), hydroxy, cyano, amino, nitro, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), $C_{2-6}$ alkenyl (e.g., vinyl, propenyl or butenyl), or $C_{2-6}$ alkynyl (e.g., ethynyl, propynyl, or butynyl);

ring B in Formula (PBM-6-1A) represents $C_{3-15}$ cycloalkyl or 3- to 20-membered heterocyclyl containing from 1 to 4 heteroatoms selected from the group consisting of oxygen, nitrogen, and sulfur, wherein the ring B is optionally substituted with t2 (e.g., 1-6, such as 1, 2, 3, 4, 5 or 6) $R^{7a}$, and each $R^{7a}$ are the same or different and each independently represent deuterium, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CHO$-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-);

ring C in Formula (PBM-6-1A) represents 5- to 20-membered monocyclic or bicyclic heteroaryl containing from 1 to 4 heteroatoms selected from the group consisting of oxygen, nitrogen, and sulfur, wherein the ring C is optionally substituted with t3 (e.g., 1-6, such as 1, 2, 3, 4, 5 or 6) $R^{8a}$, and each $R^{8a}$ are the same or different and each independently represent deuterium, halogen (e.g., fluorine, chlorine, bromine, or iodine), hydroxy, cyano, amino, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-);

t1 represents an integer of 1, 2, 3, 4, 5 or 6;

t2 represents an integer of 1, 2, 3, 4, 5 or 6; and

t3 represents an integer of 1, 2, 3, 4, 5 or 6.

In some embodiments, PBM represents the structure of Formula (PBM-6-1A-1):

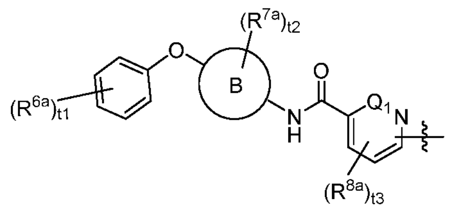

Formula (PBM-6-1A-1)

wherein $(R^{6a})_{t1}$ indicates that the benzene ring in Formula (PBM-6-1A-1) is optionally substituted with t1 $R^{6a}$, and each $R^{6a}$ are the same or different and each independently represent deuterium, halogen (e.g., fluorine, chlorine, bromine, or iodine), hydroxy, cyano, amino, nitro, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-,

$CF_3CH_2$-O- or $CH_2ClCH_2$-O-), $C_{2-6}$ alkenyl (e.g., vinyl, propenyl or butenyl), or $C_{2-6}$ alkynyl (e.g., ethynyl, propynyl, or butynyl);

ring B in Formula (PBM-6-1A-1) represents $C_{3-15}$ cycloalkyl or 3- to 20-membered heterocyclyl containing from 1 to 4 heteroatoms selected from the group consisting of oxygen, nitrogen, and sulfur, wherein the ring B is optionally substituted with t2 $R^{7a}$, and each $R^{7a}$ are the same or different and each independently represent deuterium, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-);

$Q_1$ represents N or CH; and

$(R^{8a})_{t3}$ indicates that the nitrogen-containing heteroaryl in Formula (PBM-6-1A-1) is optionally substituted with t3 $R^{8a}$, and each $R^{8a}$ are the same or different and each independently represent deuterium, halogen (e.g., fluorine, chlorine, bromine, or iodine), hydroxy, cyano, amino, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-);

t1 represents an integer of 1, 2, 3, 4, 5 or 6;

t2 represents an integer of 1, 2, 3, 4, 5 or 6; and

t3 represents an integer of 1 or 2.

In some embodiments, $Q_1$ represents N. In some embodiments, $Q_1$ represents CH.

In some embodiments, PBM represents the structures of Formula (PBM-6-1), Formula (PBM-6-2) or Formula (PBM-6-3):

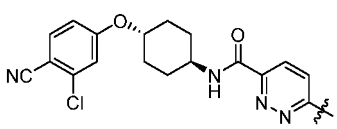
Formula (PBM-6-1)

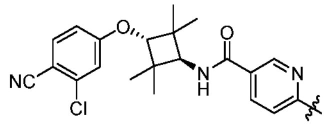
Formula (PBM-6-2)

or

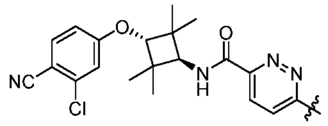
Formula (PBM-6-3)

.

In some embodiments, PBM represents a small molecule ligand targeting BTK, including but not limited to represent the structures of Formula (PBM-7-1A), Formula (PBM-7-1B) or Formula (PBM-7-1C):

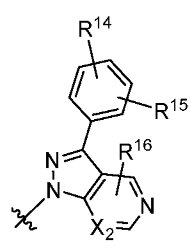
Formula (PBM-7-1A)

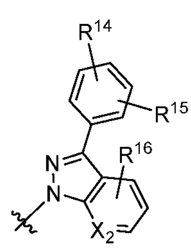
Formula (PBM-7-1B)

or

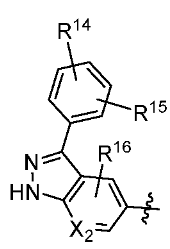
Formula (PBM-7-1C)

wherein $X_2$ represents CH or N;

$R^{14}$ represents -O-aryl, -O-heteroaryl, -$C_{1-3}$-alkylene-NHC(O)-heteroaryl, or -$C_{1-3}$-alkylene-C(O)NH-heteroaryl, wherein the aryl and heteroaryl are each independently optionally substituted with one or more (e.g., 1-5, such as 1, 2, 3, 4 or 5) $R^{9a}$, and each $R^{9a}$ are the same or different and each independently represent deuterium, halogen (e.g., fluorine, chlorine, bromine, or iodine), hydroxy, cyano, amino, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl

(e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CHO$-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-);

$R^{15}$ represents deuterium, hydrogen, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or -NHC(O)$R^{17}$, wherein $R^{17}$ is $C_{1-10}$ alkyl (e.g., $C_{1-8}$ alkyl, $C_{1-6}$ alkyl or $C_{1-4}$ alkyl, such as methyl, ethyl, propyl, isopropyl, butyl, sec-butyl, tert-butyl, pentyl, hexyl, heptyl or octyl) or $C_{2-10}$ alkenyl (e.g., $C_{2-6}$ alkenyl or $C_{2-4}$ alkenyl, such as vinyl, propenyl, butenyl or pentenyl), e.g., $R^{15}$ represents hydrogen, methyl, or

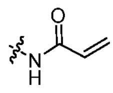 ;

and

$R^{16}$ represents hydrogen, cyano, or amino.

In some embodiments, $R^{14}$ represents -O-aryl, -O-heteroaryl, -$C_{1-3}$-alkylene-NHC(O)-heteroaryl, or -$C_{1-3}$-alkylene-C(O)NH-heteroaryl, wherein the aryl can be optionally substituted $C_{6-10}$ aryl, such as but not limited to phenyl or naphthyl, and the heteroaryl can be optionally substituted 5-to 14-membered monocyclic or bicyclic aromatic ring group containing one or more (e.g., from 1 to 6, or from 1 to 5, or from 1 to 4, or from 1 to 3) heteroatoms selected from the group consisting of oxygen, nitrogen, and sulfur. In a sub-embodiment, $R^{14}$ represents -O-phenyl or -O-naphthyl, where the phenyl or naphthyl are optionally substituted with one or more (e.g., 1-5, such as 1, 2, 3, 4 or 5) $R^{9a}$, and each $R^{9a}$ are the same or different and each independently represent deuterium, halogen (e.g., fluorine, chlorine, bromine, or iodine), hydroxy, cyano, amino, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-). In a sub-embodiment, $R^{14}$ represents -O-heteroaryl, - $C_{1-3}$-alkylene-NHC(O)-heteroaryl, or -$C_{1-3}$-alkylene-C(O)NH-heteroaryl, wherein the heteroaryl is optionally substituted with one or more (e.g., 1-5, such as 1, 2, 3, 4 or 5) $R^{9a}$, and each $R^{9a}$ are the same or different and each independently represent deuterium, halogen (e.g., fluorine, chlorine, bromine, or iodine), hydroxy, cyano, amino, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CHO$-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-).

In some embodiments, PBM represents the structures of Formula (PBM-7-1) or Formula (PBM-7-2):

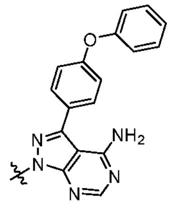

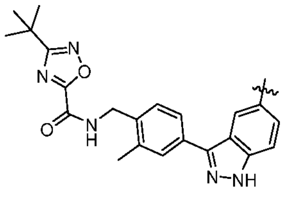

Formula (PBM-7-1) or Formula (PBM-7-2) .

In some embodiments, PBM represents a small molecule ligand targeting CDK4/6, including but not limited to represent the structures of Formula (PBM-8-1A), Formula (PBM-8-1B), Formula (PBM-8-1C) or Formula (PBM-8-1D):

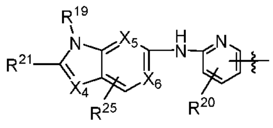
Formula (PBM-8-1A)

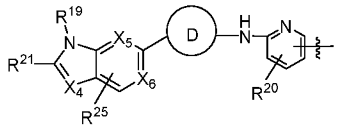
Formula (PBM-8-1B)

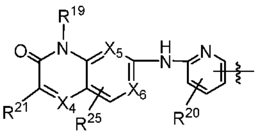
Formula (PBM-8-1C)

or

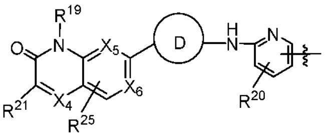
Formula (PBM-8-1D)

wherein $R^{21}$ represents deuterium, hydrogen, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), -C(O)-$C_{1-6}$ alkyl, -C(O)-deuterated $C_{1-6}$ alkyl, or - C(O)$NR^{22}R^{23}$, wherein $R^{22}$ and $R^{23}$ are the same or different and each independently represent hydrogen, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl) or deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), and $R^{22}$ and $R^{23}$ are not simultaneously hydrogen;

$X_4$ represents N or $CR^{24}$, wherein $R^{24}$ represents hydrogen, deuterium, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

$X_5$ and $X_6$ each independently represent CH or N;

ring D represents optionally substituted heteroarylene (e.g., halogenated heteroarylene);

$R^{19}$ represents hydrogen, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), or optionally substituted $C_{3-7}$ cycloalkyl (e.g., cyclopropyl, cyclobutyl, cyclopentyl, cyclopentenyl, cyclohexyl, cyclohexenyl, or cycloheptyl, which are optionally substituted with a substituent selected from the group consisting of halogen, $C_{1-6}$ alkyl, halogenated $C_{1-6}$ alkyl or deuterated $C_{1-6}$ alkyl); and

$R^{20}$ and $R^{25}$ are the same or different and each independently represent hydrogen, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-).

In some embodiments, ring D represents optionally substituted heteroarylene, e.g., optionally substituted 5- to 20-membered monocyclic or bicyclic heteroarylene containing one or more (e.g., from 1 to 6, or from 1 to 5, or from 1 to 4, or from 1 to 3) heteroatoms independently selected from the group consisting of oxygen, nitrogen, and sulfur. Examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene, and imidazo[2,1-b]thiazolylene. The heteroarylene is optionally substituted with one or more (e.g., 1-5, such as 1, 2, 3, 4 or 5) substituents selected from the group consisting of halogen (e.g., fluorine, chlorine, bromine, or iodine), hydroxy, cyano, amino, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such

as methoxy, ethoxy, propoxy, isopropoxy, butoxy, sec-butoxy or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-).

In some embodiments, PBM represents the structures of Formula (PBM-8-1), Formula (PBM-8-2) or Formula (PBM-8-3):

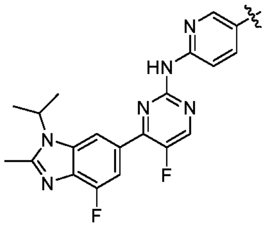

Formula (PBM-8-1)

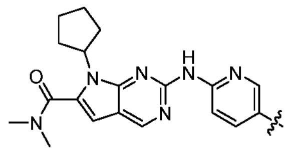

Formula (PBM-8-2)

or

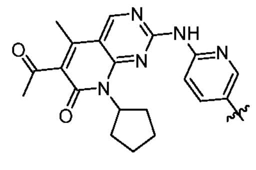

Formula (PBM-8-3)

.

In some embodiments, PBM represents a small molecule ligand targeting ALK, including but not limited to represent the structures of Formula (PBM-9-1A), Formula (PBM-9-1B), Formula (PBM-9-1C) or Formula (PBM-9-1D):

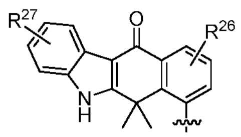

Formula (PBM-9-1A)

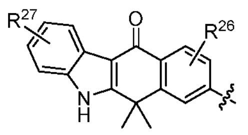

Formula (PBM-9-1B)

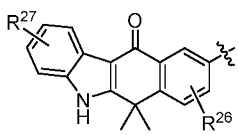

Formula (PBM-9-1C)

or

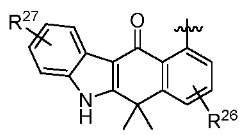

Formula (PBM-9-1D)

wherein $R^{26}$ represents hydrogen, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), or deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.); and

$R^{27}$ represents hydrogen, cyano, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), or deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.).

In some embodiments, PBM represents the structure of Formula (PBM-9-1):

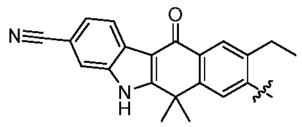

Formula (PBM-9-1)

.

In some embodiments, PBM represents a small molecule ligand targeting ALK, including but not limited to represent the structure of Formula (PBM-10):

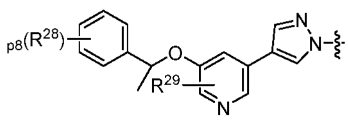

Formula (PBM-10)

wherein $(R^{28})_{p8}$ indicates that the benzene ring in Formula (PBM-10) is substituted with p8 $R^{28}$ substituents, and each $R^{28}$ are the same or different and each independently represent halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl,

tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), or deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$-etc.);

p8 represents an integer of 1, 2, 3 or 4; or

$R^{29}$ represents $NR^{30}R^{31}$, wherein $R^{30}$, $R^{31}$ are the same or different and each independently represent hydrogen, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), or deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$-etc.).

In some embodiments, PBM represents the structure of Formula (PBM-10-1):

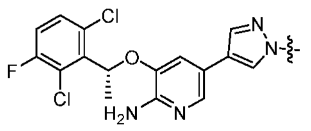

Formula (PBM-10-1) .

In some embodiments, PBM represents the structures of Formula (PBM-11-1A), Formula (PBM-11-1B) or Formula (PBM-11-1C):

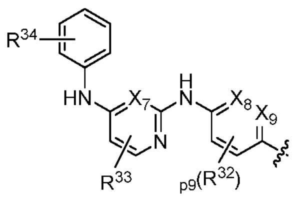

Formula (PBM-11-1A)

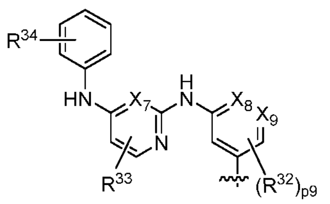

Formula (PBM-11-1B) or

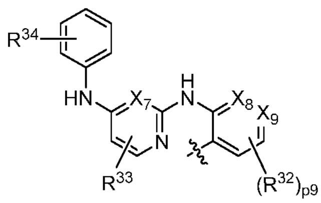

Formula (PBM-11-1C)

wherein $X_7$, Xs and $X_9$ are the same or different and each independently represent N or CH;

$(R^{32})_{p9}$ indicates that the 6-membered ring containing $X_8$ and $X_9$ is optionally substituted with p9 $R^{32}$ groups, and each $R^{32}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

p9 represents an integer of 0, 1, 2, 3 or 4;

$R^{33}$ represents halogen (e.g., fluorine, chlorine, bromine, or iodine), $C_{1-6}$ alkoxy, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-); and

$R^{34}$ represents $-C(O)NHR^{35}$, $-NHC(O)-R^{35}$, $-S(O)_2NHR^{35}$, $-S(O)_2R^{35}$ or $-P(O)(R^{35})_2$, wherein each $R^{35}$ are the same or different and each independently represent $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl) or deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.).

In some embodiments, PBM represents a small molecule ligand targeting ALK, including but not limited to represent the structures of Formula (PBM-11-1D), Formula (PBM-11-1E) or Formula (PBM-11-1F):

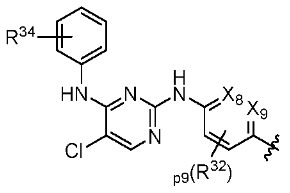
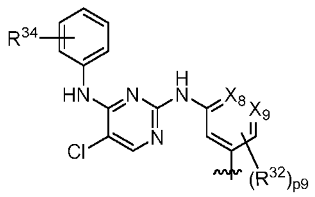
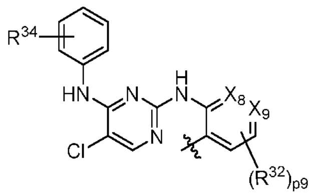

Formula (PBM-11-1D) Formula (PBM-11-1E) or Formula (PBM-11-1F)

wherein $X_8$ and $X_9$ are the same or different and each independently represent N or CH;

$(R^{32})_{p9}$ indicates that the 6-membered ring containing $X_8$ and $X_9$ is optionally substituted with p9 $R^{32}$ groups, and each $R^{32}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

p9 represents an integer of 0, 1, 2, 3 or 4; and

$R^{34}$ represents -C(O)NHR$^{35}$, -NHC(O)-R$^{35}$, -S(O)$_2$NHR$^{35}$, -S(O)$_2$R$^{35}$ or -P(O)(R$^{35}$)$_2$, wherein each $R^{35}$ are the same or different and each independently represent $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl) or deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.).

In some embodiments, PBM represents a small molecule ligand targeting ALK, including but not limited to represent the structures of Formula (PBM-11-1) or Formula (PBM-11-2):

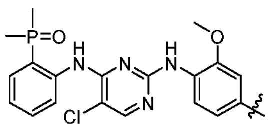
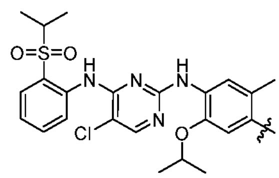

Formula (PBM-11-1) or Formula (PBM-11-2) .

In some embodiments, PBM represents a small molecule ligand targeting FAK, including but not limited to represent the structures of Formula (PBM-11-1G), Formula (PBM-11-1H) or Formula (PBM-11-11):

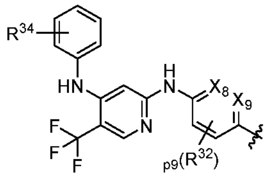
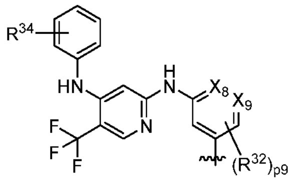
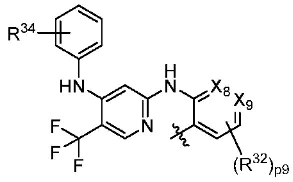

Formula (PBM-11-1G) Formula (PBM-11-1H) or Formula (PBM-11-1I)

wherein $X_8$ and $X_9$ are the same or different and each independently represent N or CH;

$(R^{32})_{p9}$ indicates that the 6-membered ring containing $X_8$ and $X_9$ is optionally substituted with p9 $R^{32}$ groups, and each $R^{32}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

p9 represents an integer of 0, 1, 2, 3 or 4; and

$R^{34}$ represents -C(O)NHR$^{35}$, -NHC(O)-R$^{35}$, -S(O)$_2$NHR$^{35}$, -S(O)$_2$R$^{35}$ or -P(O)(R$^{35}$)$_2$, wherein each $R^{35}$ are the same or different and each independently represent $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl) or deuterated $C_{1-6}$ alkyl (e.g., perdeuterated $C_{1-6}$

alkyl, perdeuterated $C_{1-5}$ alkyl or perdeuterated $C_{1-4}$ alkyl, such as $CD_3$, $CD_3CD_2$-, $CD_3CD_2CD_2$- etc.).

In some embodiments, PBM represents a small molecule ligand targeting FAK, including but not limited to represent the structures of Formula (PBM-11-1G), Formula (PBM-11-1H) or Formula (PBM-11-11):

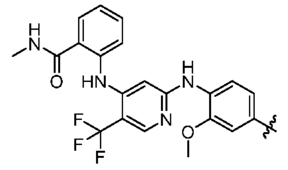

Formula (PBM-11-3)

.

In some embodiments, PBM represents a small molecule ligand targeting RET, including but not limited to represent the structures of Formula (PBM-11-1J), Formula (PBM-11-1K) or Formula (PBM-11-1L):

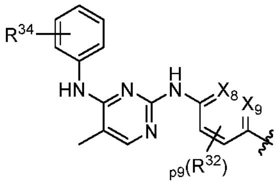

Formula (PBM-11-1J)

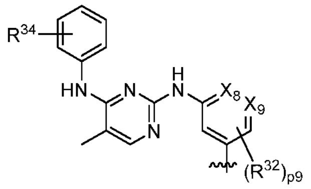

Formula (PBM-11-1K)

or

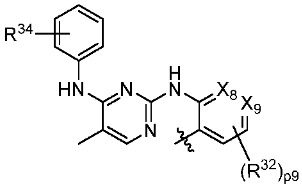

Formula (PBM-11-1L)

wherein $X_8$ and $X_9$ are the same or different and each independently represent N or CH;

$(R^{32})_{p9}$ indicates that the 6-membered ring containing $X_8$ and $X_9$ is optionally substituted with p9 $R^{32}$ groups, and each $R^{32}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

p9 represents an integer of 0, 1, 2, 3 or 4; and

$R^{34}$ represents -C(O)NHR$^{35}$, -NHC(O)-R$^{35}$, -S(O)$_2$NHR$^{35}$, -S(O)$_2$R$^{35}$ or -P(O)(R$^{35}$)$_2$, wherein each $R^{35}$ are the same or different and each independently represent $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl) or deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.).

In some embodiments, PBM represents a small molecule ligand targeting RET, including but not limited to represent the structures of Formula (PBM-11-4) or Formula (PBM-11-5):

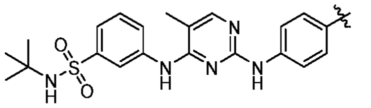

Formula (PBM-11-4)

or

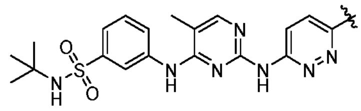

Formula (PBM-11-5)

.

In some embodiments, PBM represents a small molecule ligand targeting RET, including but not limited to represent the structures of Formula (PBM-12-1A), Formula (PBM-12-1B), Formula (PBM-12-1C), Formula (PBM-12-1D) or Formula (PBM-12-1E):

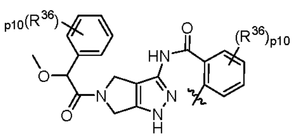

Formula (PBM-12-1A)

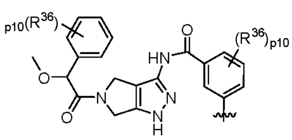

Formula (PBM-12-1B)

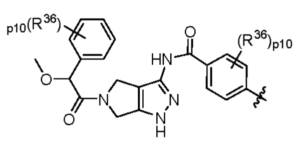

Formula (PBM-12-1C)

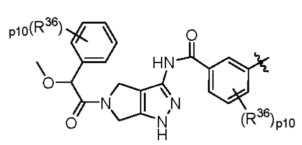

Formula (PBM-12-1D)

or

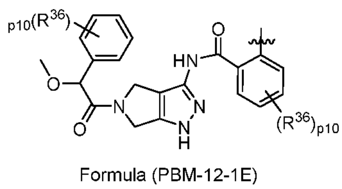

Formula (PBM-12-1E)

wherein $(R^{36})_{p10}$ indicates that the benzene ring in Formula (PBM-12-1A), Formula (PBM-12-1B), Formula (PBM-12-1C), Formula (PBM-12-1D) or Formula (PBM-12-1E) is optionally substituted with p10 $R^{36}$ groups, and each $R^{36}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-); and p10 represents an integer of 0, 1, 2, 3 or 4.

In some embodiments, PBM represents a small molecule ligand targeting RET, including but not limited to represent the structure of Formula (PBM-12-1):

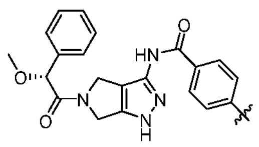

Formula (PBM-12-1) .

In some embodiments, PBM represents a small molecule ligand targeting BET, including but not limited to represent the structure of Formula (PBM-13):

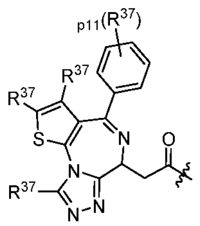

Formula (PBM-13)

wherein $(R^{37})_{p11}$ indicates that the benzene ring in Formula (PBM-13) is substituted with p11 $R^{37}$ groups, and each $R^{37}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-); and p II represents an integer of 0, 1, 2, 3 or 4.

In some embodiments, PBM represents a small molecule ligand targeting BET, including but not limited to represent the structure of Formula (PBM-13-1):

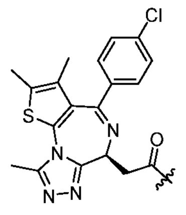

Formula (PBM-13-1) .

In some embodiments, PBM represents a small molecule ligand targeting BET, including but not limited to represent the structures of Formula (PBM-14-1A), Formula (PBM-14-1B), Formula (PBM-14-1C), Formula (PBM-14-1D) or Formula (PBM-14-1E):

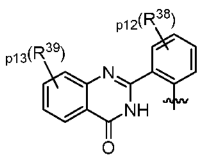

Formula (PBM-14-1A)

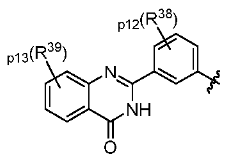

Formula (PBM-14-1B)

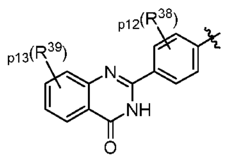

Formula (PBM-14-1C)

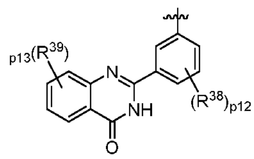

Formula (PBM-14-1D) or

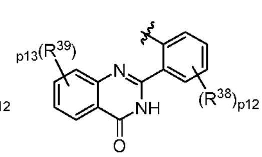

Formula (PBM-14-1E)

wherein $(R^{38})_{p12}$ indicates that the benzene ring in Formula (PBM-14-1A), Formula (PBM-14-1B), Formula (PBM-14-1C), Formula (PBM-14-1D) or Formula (PBM-14-1E) is substituted with p12 $R^{38}$ groups, and each $R^{38}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

p12 represents an integer of 0, 1, 2, 3 or 4 ;

$(R^{39})_{p13}$ indicates that the 4-oxo-3,4-dihydroquinazoline ring in Formula (PBM-14-1A), Formula (PBM-14-1B), Formula (PBM-14-1C), Formula (PBM-14-1D) or Formula (PBM-14-1E) is substituted with p13 $R^{39}$ groups, and each $R^{39}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-); and

p13 represents an integer of 1, 2, 3 or 4 .

In some embodiments, PBM represents a small molecule ligand targeting BET, including but not limited to represent the structure of Formula (PBM-14-1):

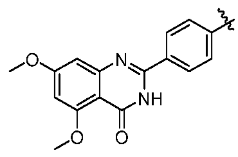

Formula (PBM-14-1) .

In some embodiments, PBM represents a small molecule ligand targeting BCR-ABL, including but not limited to represent the structures of Formula (PBM-15-1A) or Formula (PBM-15-1B):

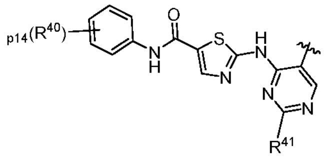
Formula (PBM-15-1A)

or

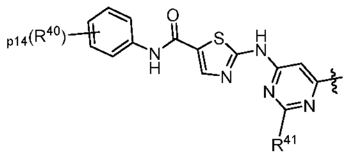
Formula (PBM-15-1B)

wherein $(R^{40})_{p14}$ indicates that the benzene ring in Formula (PBM-15-1A) or Formula (PBM-15-1B) is substituted with p14 $R^{40}$ groups, and each $R^{40}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

p14 represents an integer of 1, 2, 3 or 4 ; and

$R^{41}$ represents halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-).

In some embodiments, PBM represents a small molecule ligand targeting BCR-ABL, including but not limited to represent the structure of Formula (PBM-15-1):

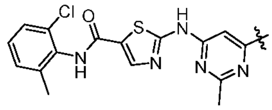
Formula (PBM-15-1) .

In some embodiments, PBM represents a small molecule ligand targeting BCR-ABL, including but not limited to represent the structures of Formula (PBM-16-1A), Formula (PBM-16-1B), Formula (PBM-16-1C) or Formula (PBM-16-1D):

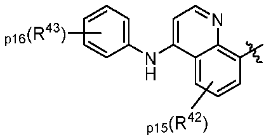
Formula (PBM-16-1A)

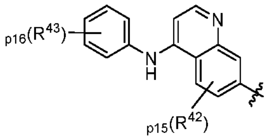
Formula (PBM-16-1B)

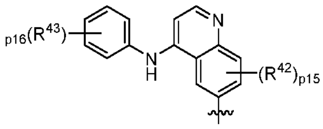
Formula (PBM-16-1C)

or

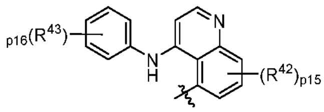
Formula (PBM-16-1D)

wherein $(R^{42})_{p15}$ indicates that the quinoline ring in Formula (PBM-16-1A), Formula (PBM-16-1B), Formula (PBM-16-1C) or Formula (PBM-16-1D) is substituted with p15 $R^{42}$ groups, and each $R^{42}$ are the same or different and each independently represent halogen (e.g., fluorine, chlorine, bromine, or iodine), cyano, $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

p15 represents an integer of 2, 3 or 4 ;

$(R^{43})_{p16}$ indicates that the benzene ring in Formula (PBM-16-1A), Formula (PBM-16-1B), Formula (PBM-16-1C) or Formula (PBM-16-1D) is substituted with p16 $R^{43}$ groups, and each $R^{43}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-,

$CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-); and
p16 represents an integer of 3 or 4 .

In some embodiments, PBM represents a small molecule ligand targeting BCR-ABL, including but not limited to represent the structure of Formula (PBM-16-1):

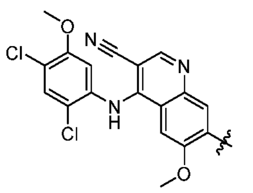

Formula (PBM-16-1) .

In some embodiments, PBM represents a small molecule ligand targeting BCR-ABL, including but not limited to represent the structures of Formula (PBM-17-1A), Formula (PBM-17-1B), Formula (PBM-17-1C), Formula (PBM-17-1D) or Formula (PBM-17-1E):

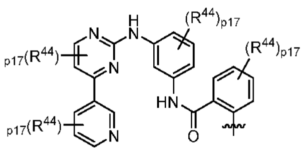

Formula (PBM-17-1A)

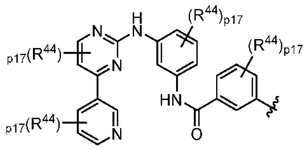

Formula (PBM-17-1B)

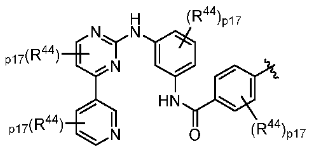

Formula (PBM-17-1C)

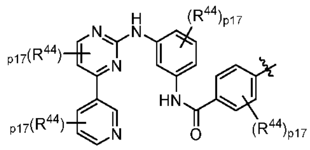

Formula (PBM-17-1D)

or

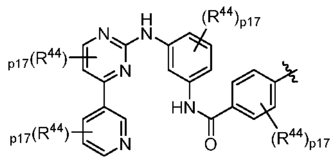

Formula (PBM-17-1E)

wherein $(R^{44})_{p17}$ indicates that the benzene, pyrimidine and pyridine rings in Formula (PBM-17-1A), Formula (PBM-17-1B), Formula (PBM-17-1C), Formula (PBM-17-1D) or Formula (PBM-17-1E) are each optionally substituted with p17 $R^{44}$ groups, and each $R^{44}$ are the same or different and each independently represent halogen (e.g., fluorine, chlorine, bromine, or iodine), $C_{1-6}$ alkoxy (e.g., $C_{1-4}$ alkoxy, such as methoxy, ethoxy, propoxy, isopropoxy, butoxy, sec-butoxy or tert-butoxy), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-); and

p17 represents an integer of 0, 1, 2, 3 or 4 .

In some embodiments, PBM represents a small molecule ligand targeting BCR-ABL, including but not limited to represent the structure of Formula (PBM-17-1):

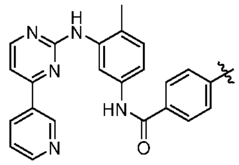

Formula (PBM-17-1) .

In some embodiments, PBM represents a small molecule ligand targeting ER, including but not limited to represent the structures of Formula (PBM-18-1A), Formula (PBM-18-1B), Formula (PBM-18-1C) or Formula (PBM-18-1D):

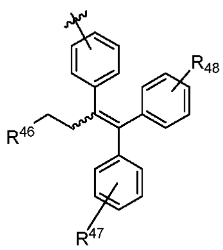

Formula (PBM-18-1A)

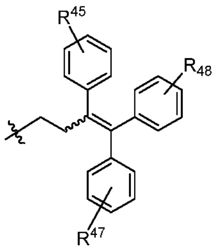

Formula (PBM-18-1B)

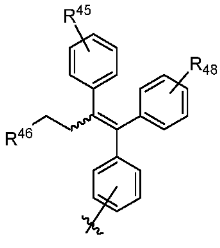

Formula (PBM-18-1C) or

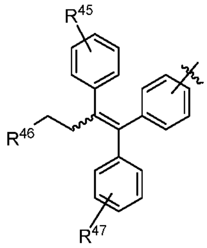

Formula (PBM-18-1D)

wherein $R^{45}$, $R^{46}$, $R^{47}$ and $R^{48}$ are the same or different and each independently represent hydrogen, halogen (e.g., fluorine, chlorine, bromine, or iodine), or hydroxy; and

symbol " " connected to the double bond in Formula (PBM-18-1A), Formula (PBM-18-1B), Formula (PBM-18-1C) or Formula (PBM-18-1D) represents a bond in stereo-configuration (cis or trans configuration).

In some embodiments, PBM represents a small molecule ligand targeting ER, including but not limited to represent the structures of Formula (PBM-18-1), Formula (PBM-18-2), Formula (PBM-18-3), Formula (PBM-18-4), Formula (PBM-18-5) or Formula (PBM-18-6):

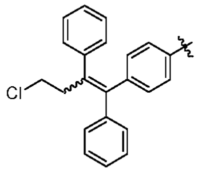

Formula (PBM-18-1)

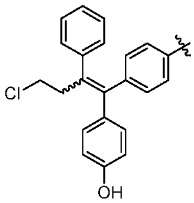

Formula (PBM-18-2)

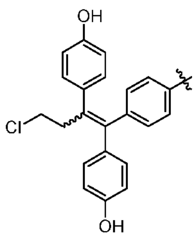

Formula (PBM-18-3)

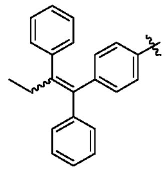

Formula (PBM-18-4)

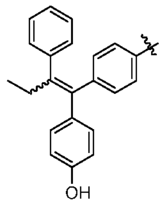

Formula (PBM-18-5) or

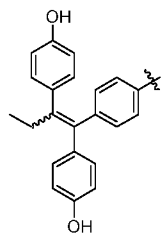

Formula (PBM-18-6) .

In some embodiments, PBM represents a small molecule ligand targeting ER, including but not limited to represent the structures of Formula (PBM-19-1A) or Formula (PBM-19-1B):

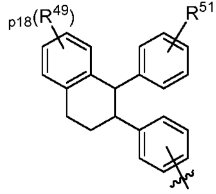

Formula (PBM-19-1A) or

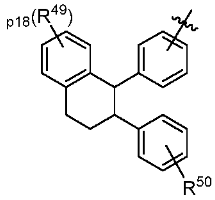

Formula (PBM-19-1B)

wherein $(R^{49})_{p18}$ indicates that 1,2,3,4-tetrahydronaphthalene ring in Formula (PBM-19-1A) or Formula (PBM-19-1B) is substituted with p18 $R^{49}$ groups, and each $R^{49}$ are the same or different and each independently represent hydrogen, hydroxy, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g.,

perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

p18 represents an integer of 0, 1, 2, 3 or 4 ; and

$R^{50}$ and $R^{51}$ each independently hydrogen, halogen (e.g., fluorine, chlorine, bromine, or iodine), $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.) or halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-).

In some embodiments, PBM represents a small molecule ligand targeting ER, including but not limited to represent the structure of Formula (PBM-19-1):

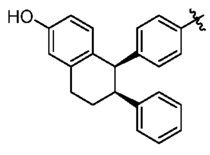

Formula (PBM-19-1) .

In some embodiments, $R^1$ represents the structures of the following formula:

a bond, -O-, $C_{1-6}$ alkylene (e.g., methylene, ethylene, propylene, isopropylene, butylene, tert-butylene, pentylene and hexylene) optionally substituted with a substituent selected from halogen and $C_{1-3}$ alkyl, -N($R^{1a}$)-$R^{2a}$-*** (e.g., -N($CH_3$)-$CH_2CH_2$-***, -NH-$CH_2CH_2$-***), -O-$R^{2a}$-*** (e.g., -O-$(CH_2)_3$-***, -O-$CH_2$-*** and -O-$(CH_2)_2$-***), -C(O)NH-$R^{2a}$-*** (e.g., -C(O)NH-$(CH_2)_2$-***, - C(O)NH-$(CH_2)_3$-***, -C(O)NH-CH=CH-$CH_2$-***, and -C(O)NH-$CH_2$-CH=CH-$CH_2$-***, ), or - NHC(O)-$R^{2a}$ -*** (e.g., -NHC(O)-CH=CH-$CH_2$-***, -NHC(O)-$CH_2$-CH=CH-$CH_2$-***, -NHC(O)-$(CH_2)_2$-***, and -NHC(O)-$(CH_2)_3$-***), wherein symbol *** indicates the point of attachment to $W^1$, $R^{1a}$ is hydrogen or $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), and $R^{2a}$ is $C_{1-6}$ alkylene (e.g., methylene, ethylene, propylene, isopropylene, butylene, tert-butylene, pentylene and hexylene) optionally substituted with a substituent selected from halogen and $C_{1-3}$ alkyl, or $C_{2-6}$ alkenylene (e.g., vinylene, propenylene, allylidene, butenylene, pentenylene, and hexenylene) optionally substituted with a substituent selected from halogen and $C_{1-3}$ alkyl; or

phenylene or naphthylene, wherein the phenylene or naphthylene is each independently optionally substituted with one or more $R^{3a}$, and each $R^{3a}$ are the same or different and each independently represent halogen (e.g., fluorine, chlorine, bromine, or iodine), hydroxy, cyano, amino, nitro, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-); or

furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene, and imidazo[2,1-b]thiazolylene, with each group being independently optionally substituted with one or more $R^{3a}$, and each $R^{3a}$ are the same or different and are each independently halogen (e.g., fluorine, chlorine, bromine, or iodine), hydroxy, cyano, amino, nitro, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-6}$ alkoxy (e.g., $C_{1-4}$ alkoxy, such as methoxy, ethoxy, propoxy, isopropoxy, butoxy, sec-butoxy or tert-butoxy), or halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-).

In some embodiments, $W^1$ is t interconnected rings $W^2$ and represented by the following formula:

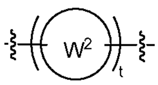

wherein each ring $W^2$ are the same or different and each independently represent the following groups:

monocyclic $C_{3-10}$ heterocyclylene group containing one nitrogen atom and optionally a heteroatom selected from nitrogen, oxygen, and sulfur, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene or diazacyclooctylene; or

$C_{5-20}$ bridged heterocyclylene group containing one nitrogen atom and optionally a heteroatom selected from nitrogen, oxygen, and sulfur, e.g., azabicyclo[3.1. 1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene or diazabicyclo[2.2.2]octanylene, such as the following groups:

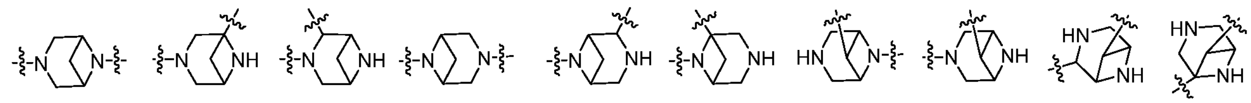

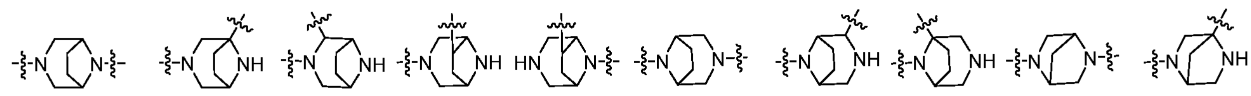

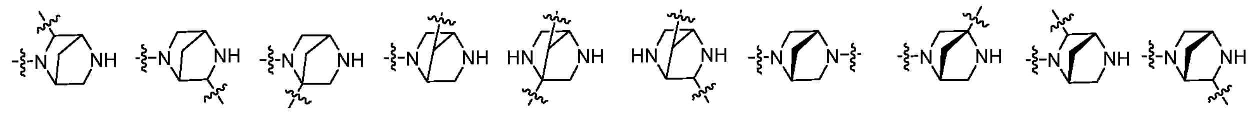

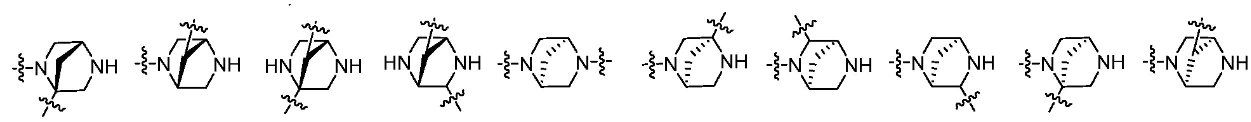

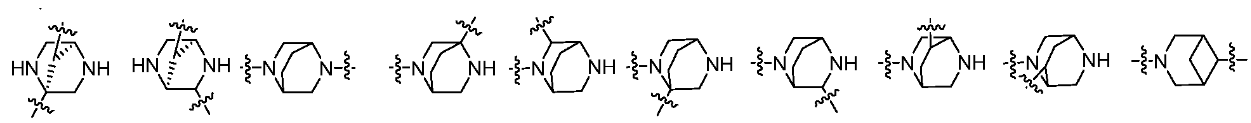

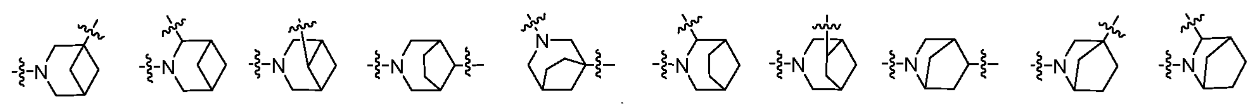

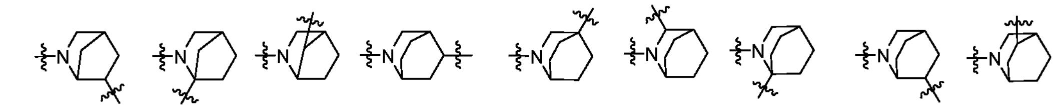 or 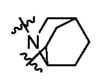 ;

or

$C_{3-15}$ cycloalkylene, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, $C_{5-15}$ spiro-cycloalkylene, adamantanylene, noradamantanylene, bornylene, bicyclo[2.2.1]heptylene or bicyclo[2.2.1]heptenylene;

wherein the monocyclic $C_{3-10}$ heterocyclylene, $C_{5-20}$ bridged heterocyclylene, and $C_{3-15}$ cycloalkylene are each independently optionally substituted with one or more substituents selected from the group consisting of deuterium,

hydroxy, amino, mercapto, nitro, halogen (e.g., fluorine, chlorine, bromine, or iodine), cyano, optionally deuterated $C_{1-6}$ alkyl, halogenated $C_{1-6}$ alkyl (e.g., halogenated $C_{1-4}$ alkyl, such as $F_3C$-, $FCH_2$-, $F_2CH$-, $ClCH_2$-, $C1_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally deuterated cyclopropyl, cyclobutyl, cyclopentyl, or cyclohexyl), optionally deuterated $C_{1-6}$ alkoxy (e.g., $C_{1-4}$ alkoxy, such as methoxy, ethoxy, propoxy, isopropoxy, butoxy, sec-butoxy or tert-butoxy), optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof; and

t represents an integer of 1 or 2.

In some embodiments, $W^1$ represents the following groups:

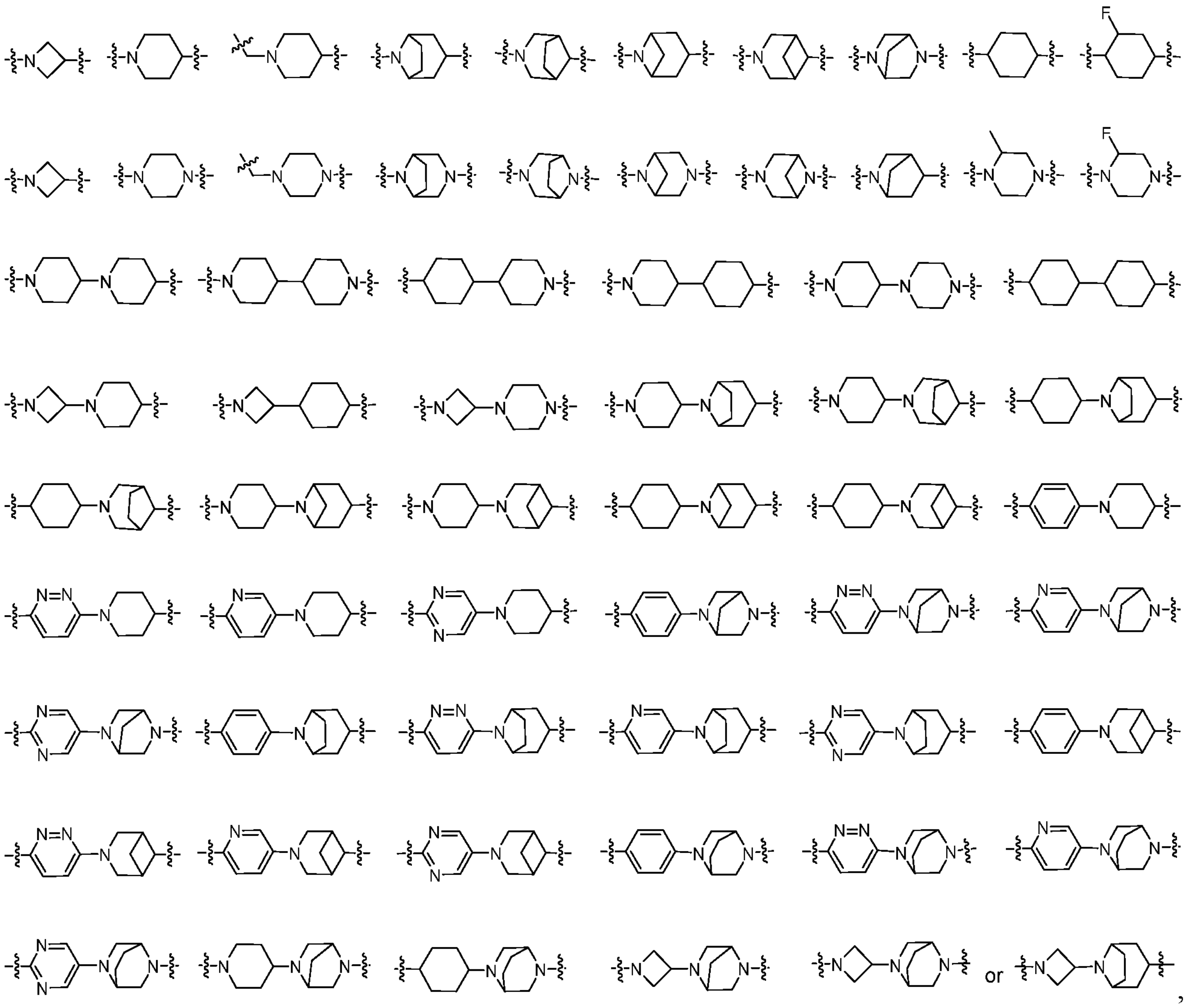

or ,

wherein the groups are optionally substituted with one or more substituents selected from the group consisting of deuterium, halogen (e.g., fluorine, chlorine, bromine, or iodine), cyano, amino, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $C1_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), deuterated $C_{1-6}$ alkoxy, or any combination thereof.

In some embodiments, $R^1$-$W^1$ represents the following groups:

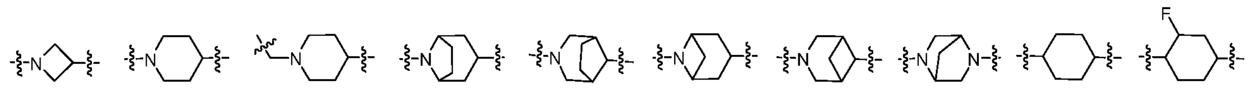

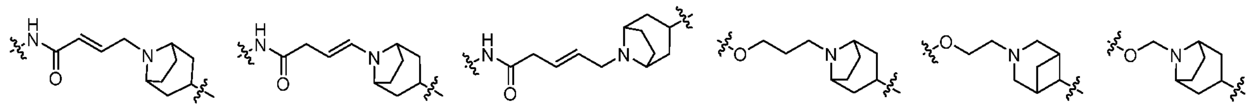

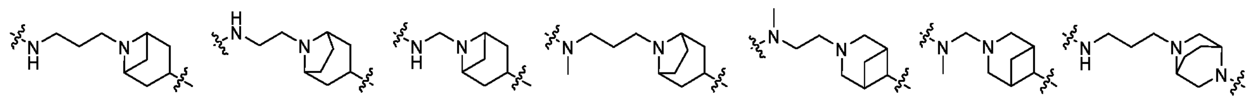

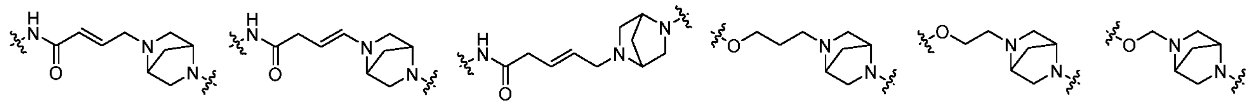

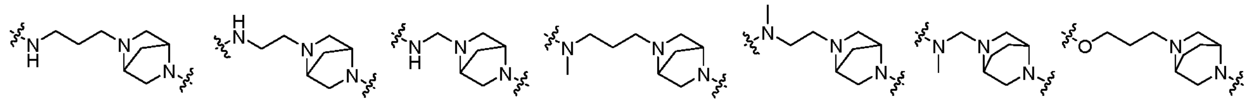

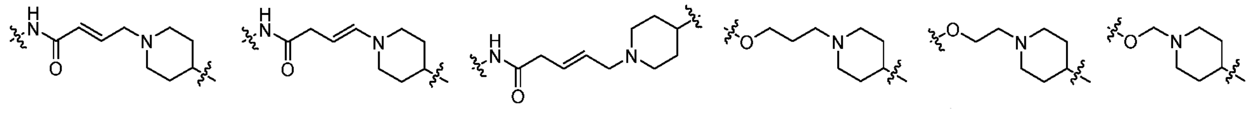

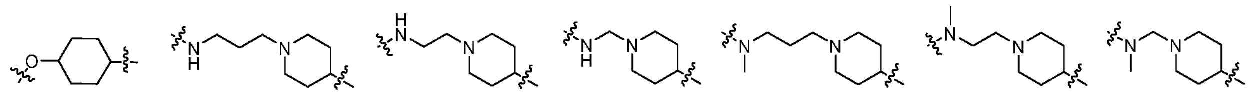

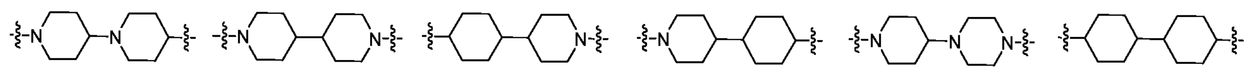

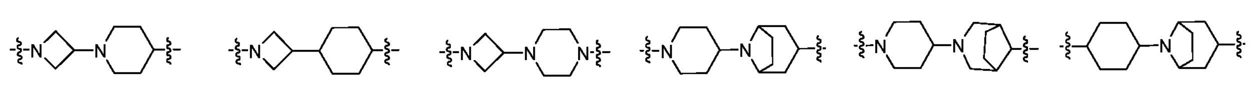

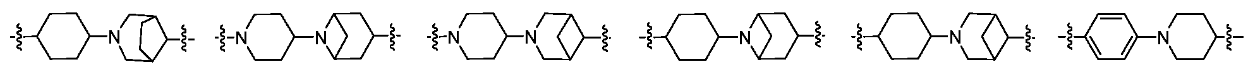

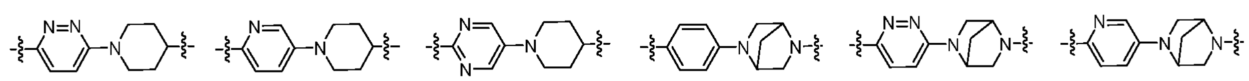

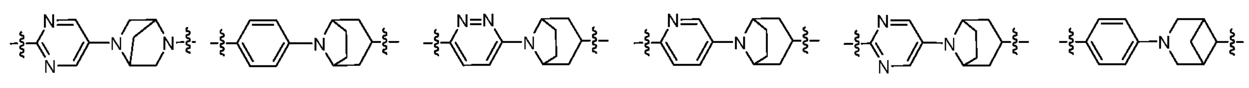

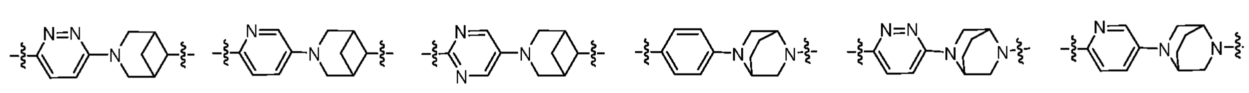

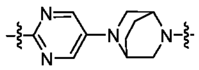 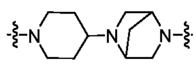 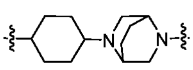 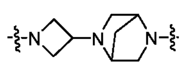 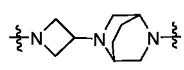 or 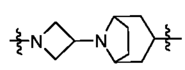 ,

wherein the groups are optionally substituted with one or more substituents selected from the group consisting of deuterium, halogen (e.g., fluorine, chlorine, bromine, or iodine), cyano, amino, $C_{1-6}$ alkyl (e.g., $C_{1-6}$ alkyl, $C_{1-5}$ alkyl or $C_{1-4}$ alkyl, such as methyl, ethyl, propyl, isopropyl, butyl, sec-butyl, tert-butyl, pentyl or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $C1_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), deuterated $C_{1-6}$ alkoxy, or any combination thereof.

In some embodiments, ULM represents the structures of Formula (ULM-1), Formula (ULM-2), Formula (ULM-3), Formula (ULM-4), Formula (ULM-5), Formula (ULM-6), Formula (ULM-7), Formula (ULM-8), Formula (ULM-9), or Formula (ULM-10):

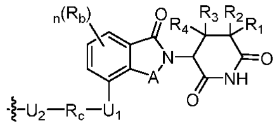
Formula (ULM-1)

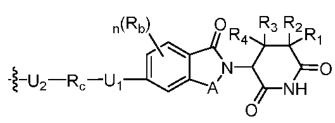
Formula (ULM-2)

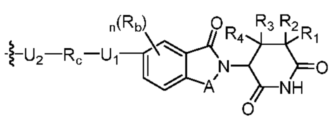
Formula (ULM-3)

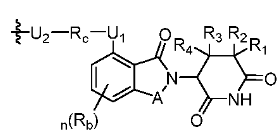
Formula (ULM-4)

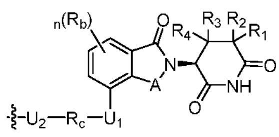
Formula (ULM-5)

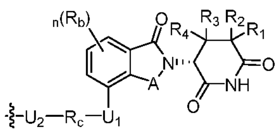
Formula (ULM-6)

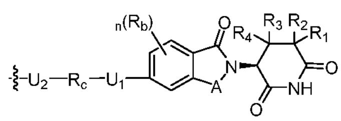
Formula (ULM-7)

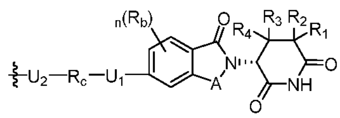
Formula (ULM-8)

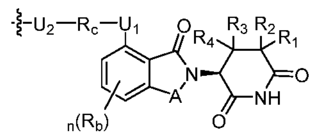
Formula (ULM-9)

or

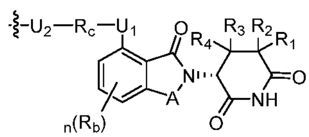
Formula (ULM-10) ;

wherein A represents CO, $CH_2$ or $CD_2$;

$R_1$, $R_2$, $R_3$ and $R_4$ are the same or different and each independently represent hydrogen, deuterium, halogen (e.g., fluorine, chlorine, bromine, or iodine), optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), or halogenated $C_{1-6}$ alkyl (e.g., halogenated $C_{1-4}$ alkyl, such as $F_3C$-, $FCH_2$-, $F_2CH$-, $ClCH_2$-, $C1_2CH$-, $CF_3CF_2$-, $CF_3CHF$-, $CHF_2CF_2$-, $CHF_2CHF$-, $CF_3CH_2$- or $CH_2ClCH_2$-);

$(R_b)_n$ indicates that benzene ring is optionally substituted with n $R_b$ groups, and each $R_b$ are the same or different and each independently represent hydrogen, deuterium, halogen (e.g., fluorine, chlorine, bromine, or iodine), hydroxy, mercapto, nitro, amino, cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), halogenated $C_{1-6}$ alkyl (e.g., halogenated $C_{1-4}$ alkyl, such as $F_3C$-, $FCH_2$-, $F_2CH$-, $ClCH_2$-, $C1_2CH$-, $CF_3CF_2$-, $CF_3CHF$-, $CHF_2CF_2$-, $CHF_2CHF$-, $CF_3CH_2$- or $CH_2ClCH_2$-), $C_{2-6}$ alkenyl or $C_{2-6}$ alkynyl;

n represents an integer of 0, 1, 2 or 3;

$U_1$ and $U_2$ are the same or different and each independently represent a bond, $N(R_d)$ or O, wherein $R_d$ represents H or optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl);

$R_c$ represents monocyclic $C_{3-10}$ heterocyclylene group containing one nitrogen atom and optionally a heteroatom selected from nitrogen, oxygen, and sulfur, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene or diazacyclooctylene; or

$R_c$ represents $C_{5-20}$ bridged heterocyclylene group containing one nitrogen atom and optionally a heteroatom selected from nitrogen, oxygen, and sulfur, e.g., azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene or diazabicyclo[2.2.2]octanylene, such as the following groups:

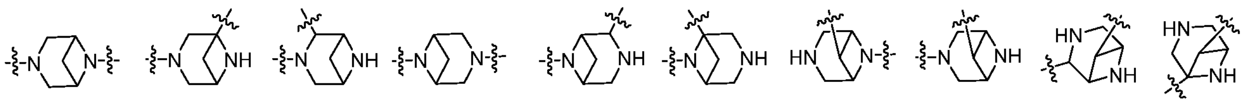

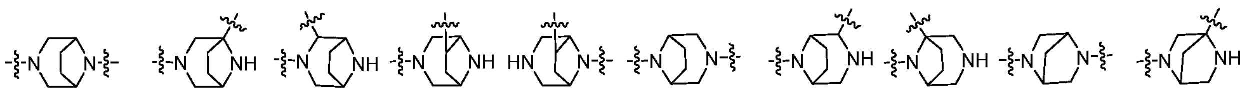

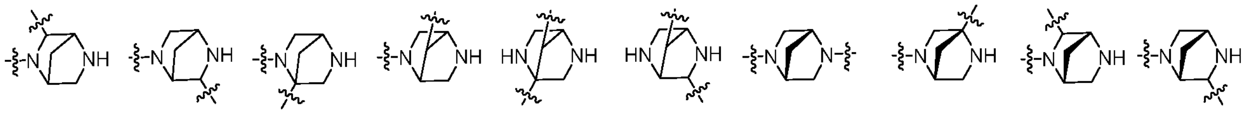

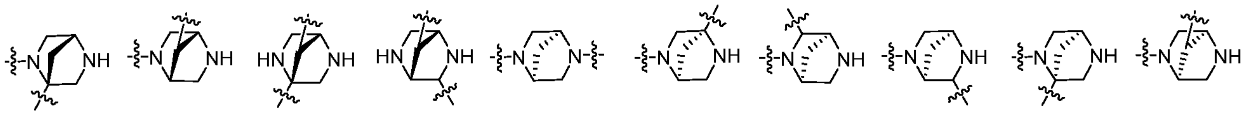

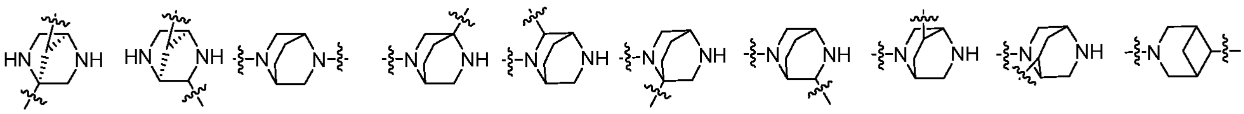

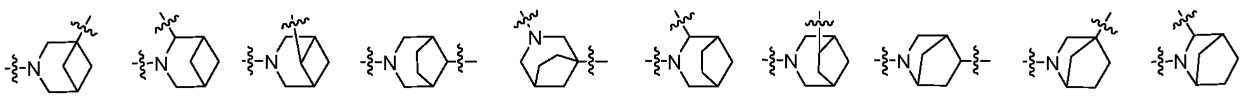

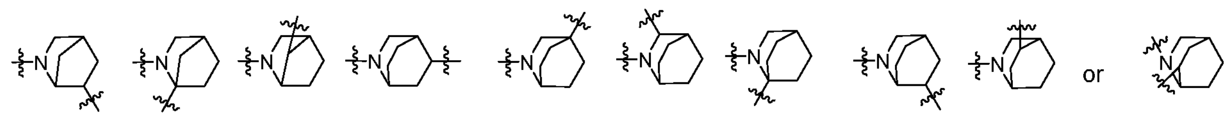

,

wherein the monocyclic $C_{3-10}$ heterocyclylene and $C_{5-20}$ bridged heterocyclylene are each independently optionally substituted with one or more substituents selected from the group consisting of deuterium, hydroxy, amino, mercapto, nitro, halogen (e.g., fluorine, chlorine, bromine, or iodine), cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally deuterated cyclopropyl, cyclobutyl, cyclopentyl, or cyclohexyl), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

In some embodiments, $R_c$ represents the following groups:

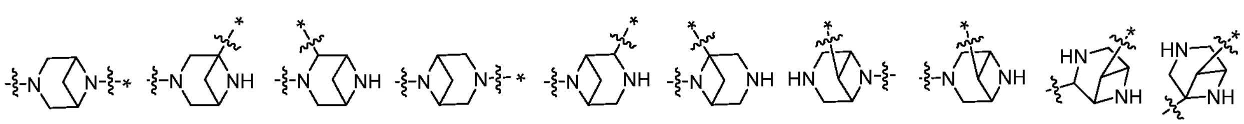

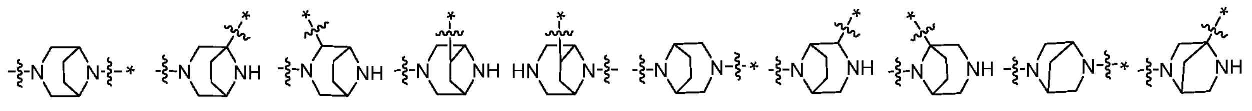
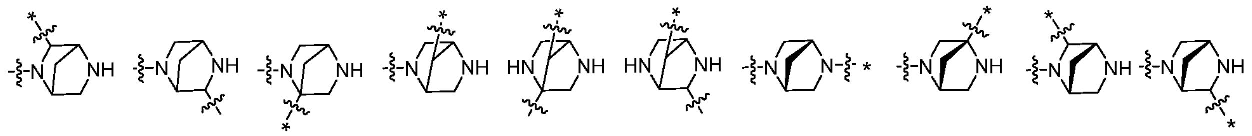
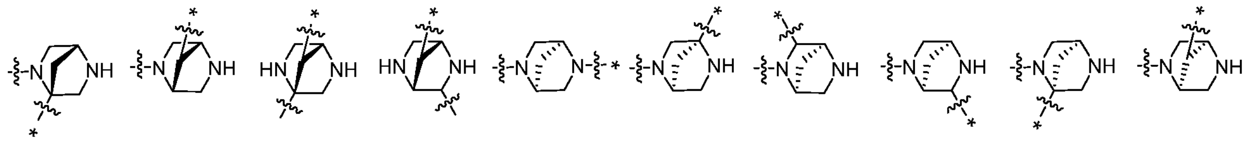
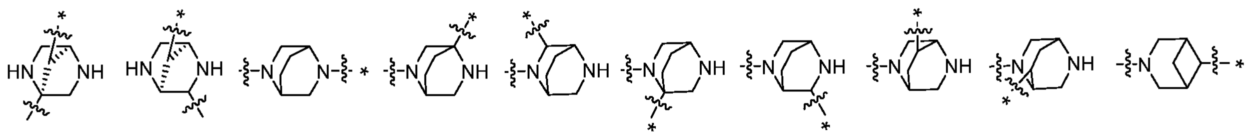
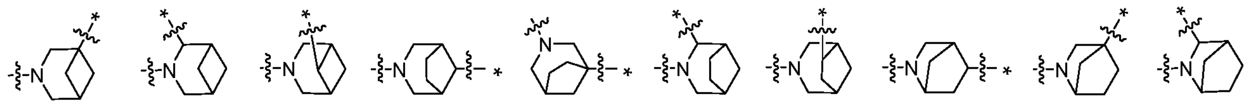
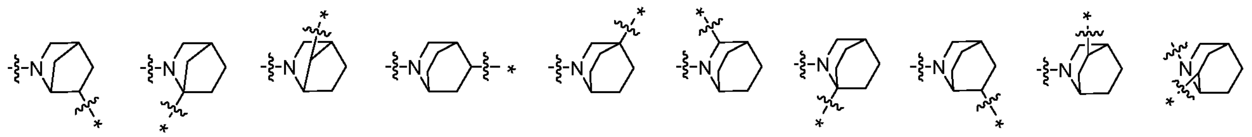
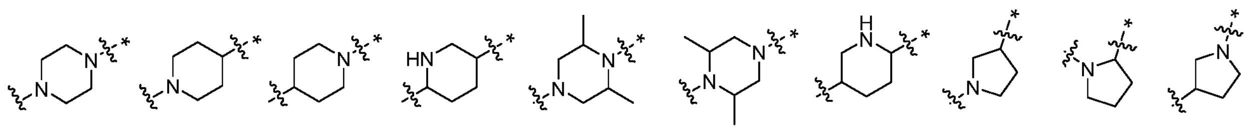
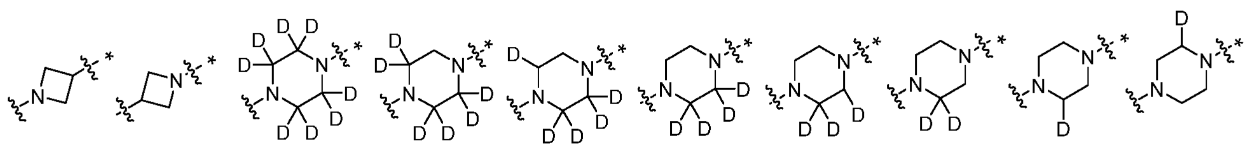
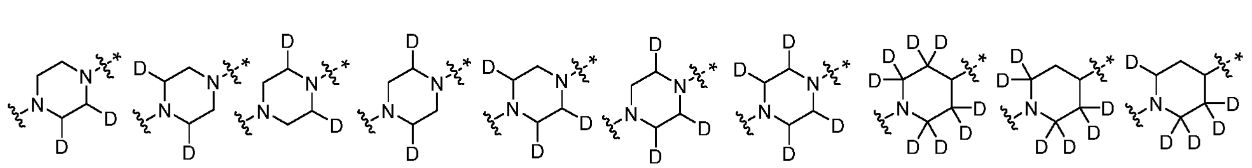
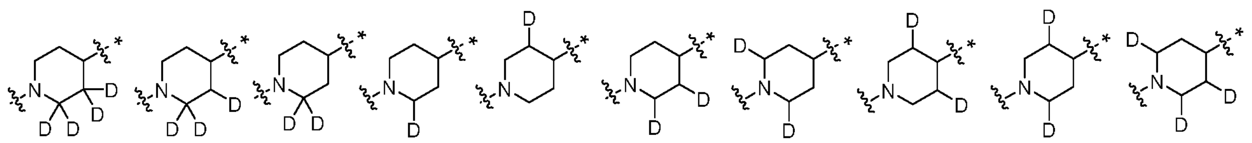

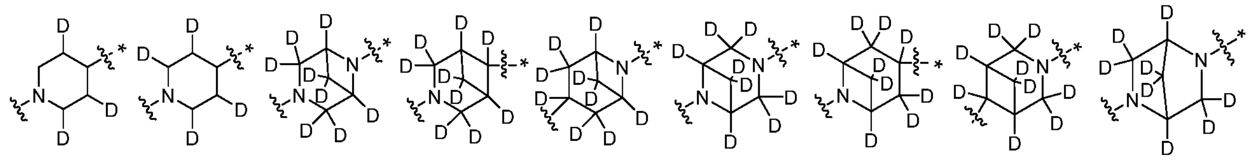

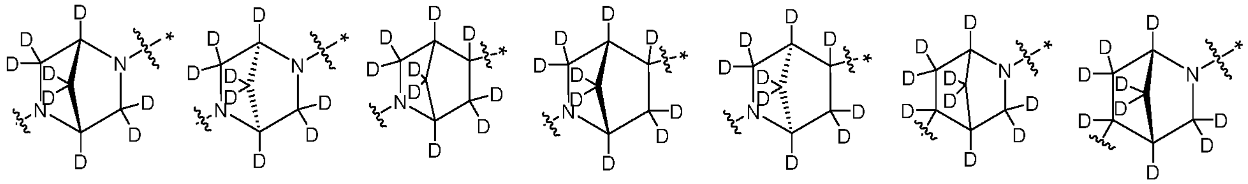

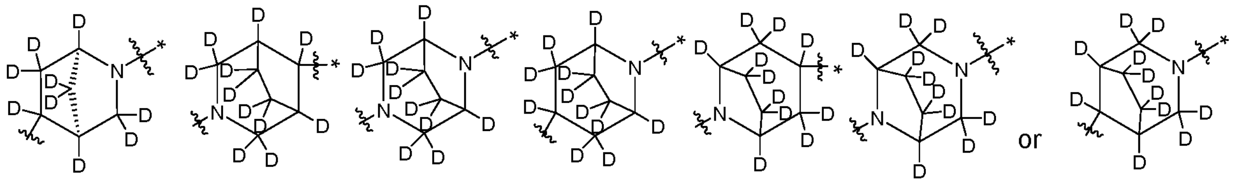

or

;

wherein symbol * indicates the point of attachment to $U_1$.

In some embodiments, ULM represents the structures of Formula (ULM-11), Formula (ULM-12), Formula (ULM-13), Formula (ULM-14), Formula (ULM-15), Formula (ULM-16), Formula (ULM-17), Formula (ULM-18), Formula (ULM-19), Formula (ULM-20), Formula (ULM-21) or Formula (ULM-22):

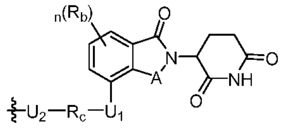

Formula (ULM-11)

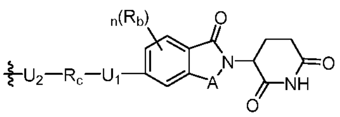

Formula (ULM-12)

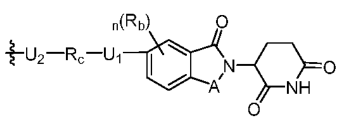

Formula (ULM-13)

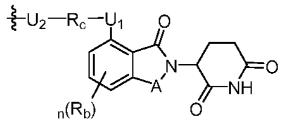

Formula (ULM-14)

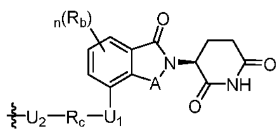

Formula (ULM-15)

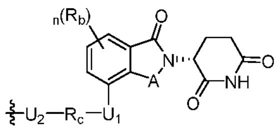

Formula (ULM-16)

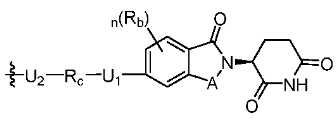

Formula (ULM-17)

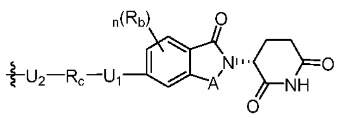

Formula (ULM-18)

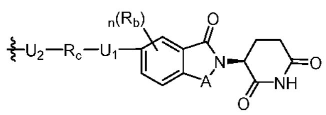

Formula (ULM-19)

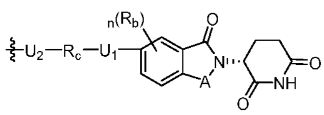

Formula (ULM-20)

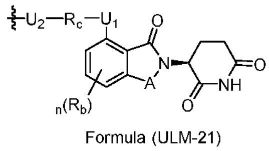

Formula (ULM-21) or

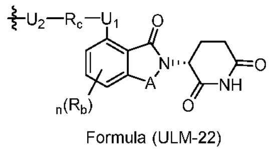

Formula (ULM-22) ;

wherein A, $(R_b)_n$, $R_b$, $R_c$, $U_1$, $U_2$ are as defined above.

In some embodiments, ULM represents the structure of the following formula:

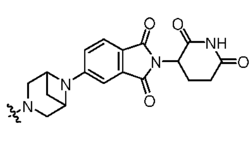

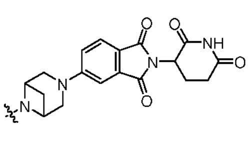

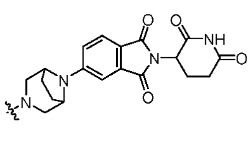

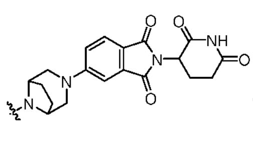

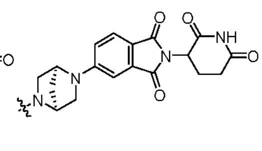

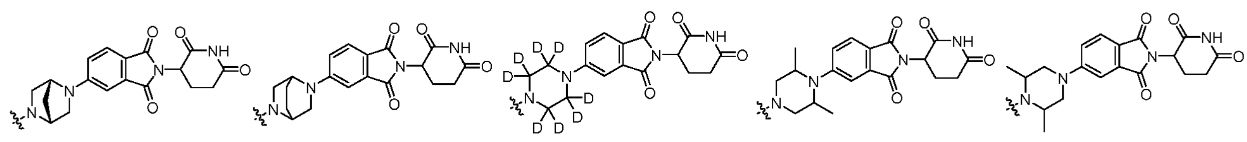

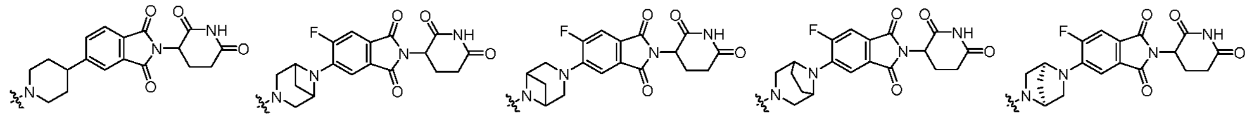

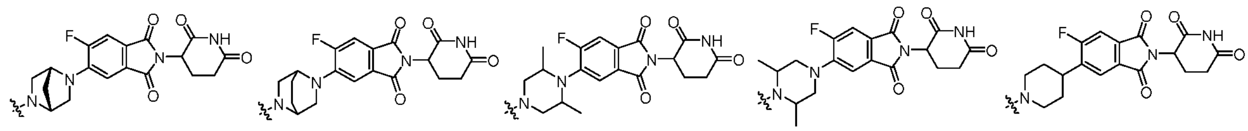

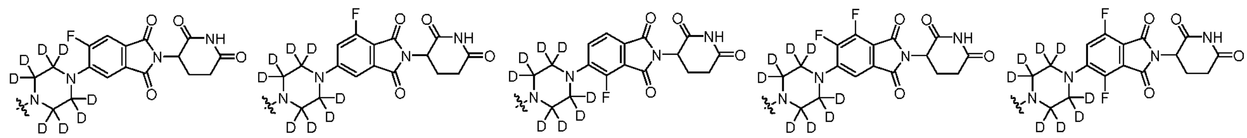

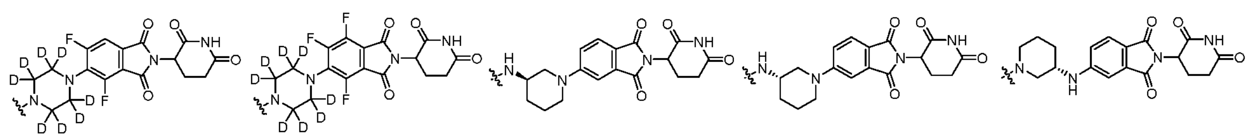

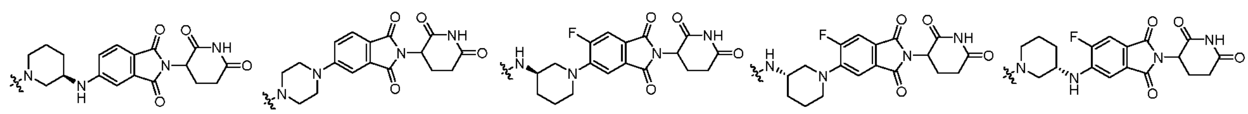

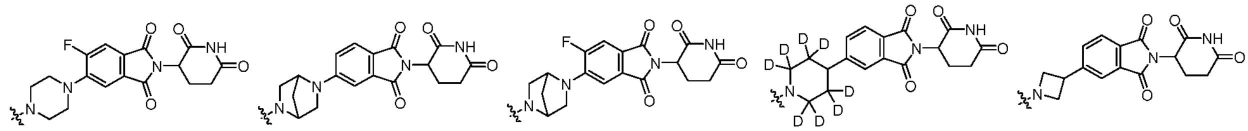

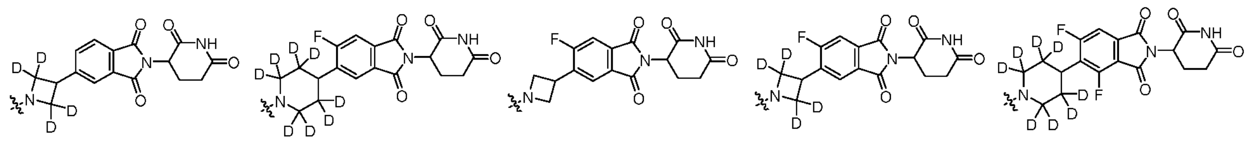

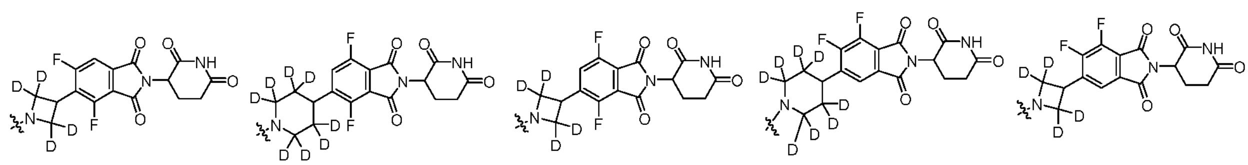

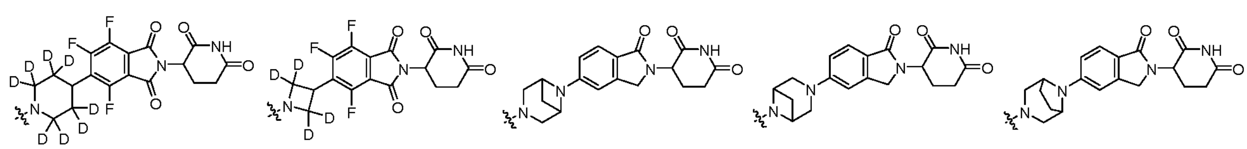

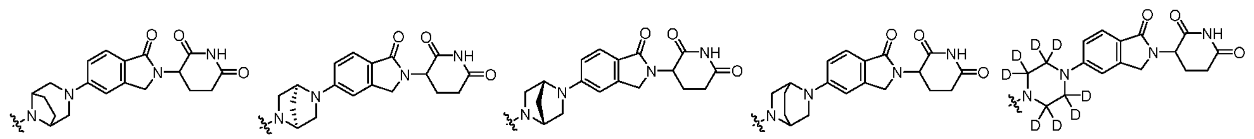

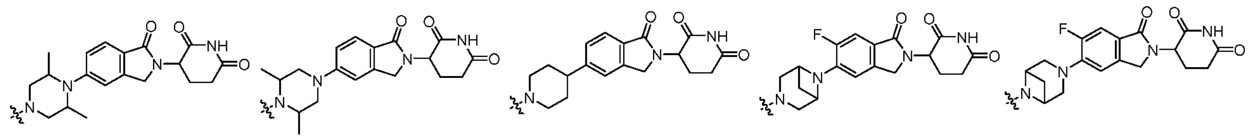

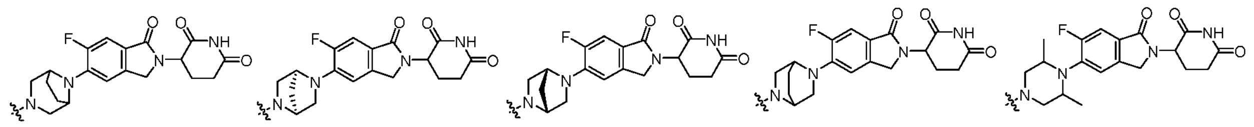

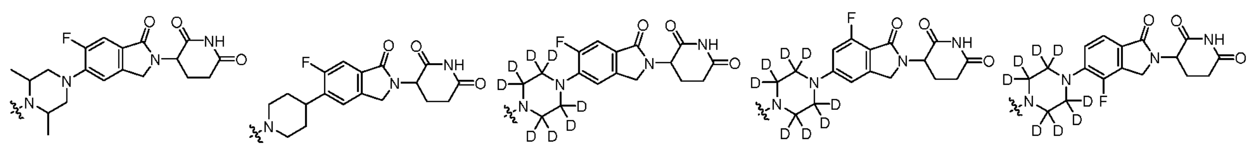

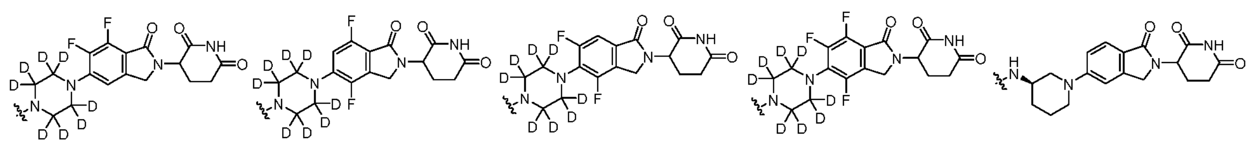

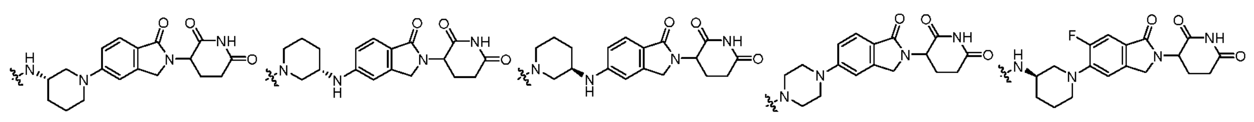

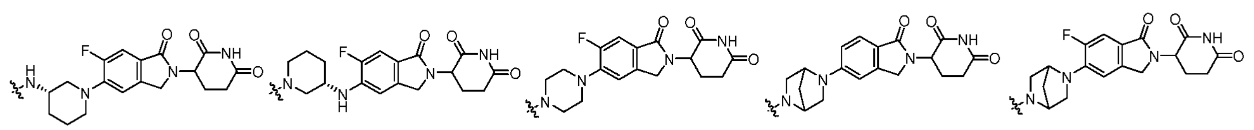

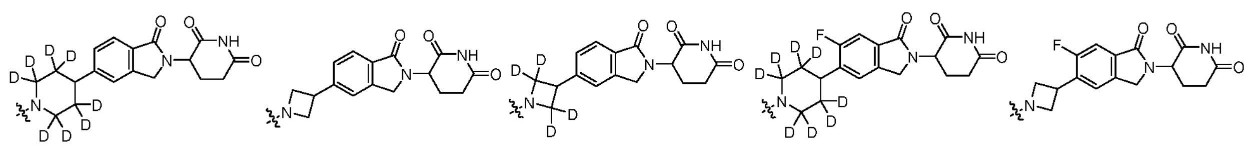

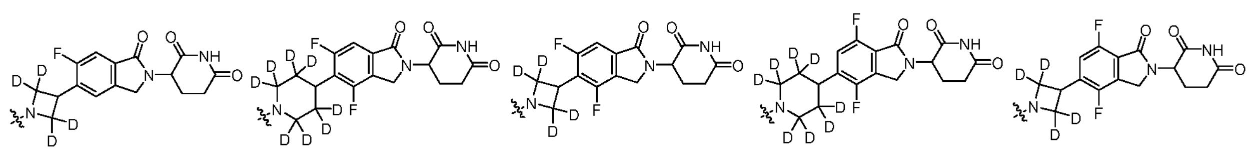

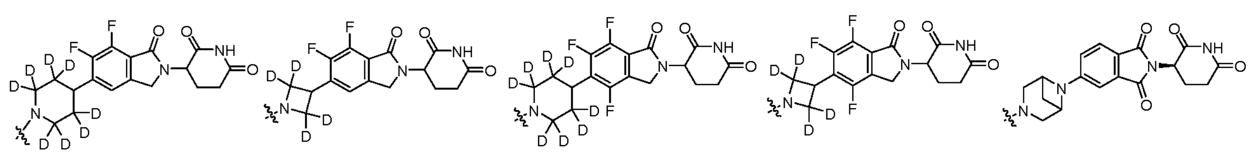

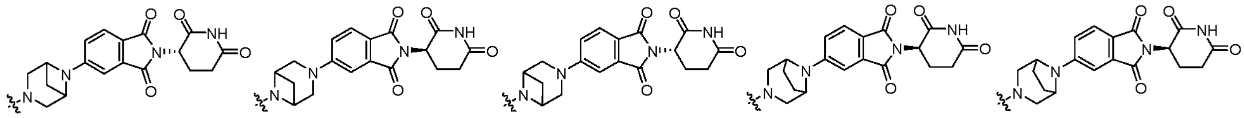

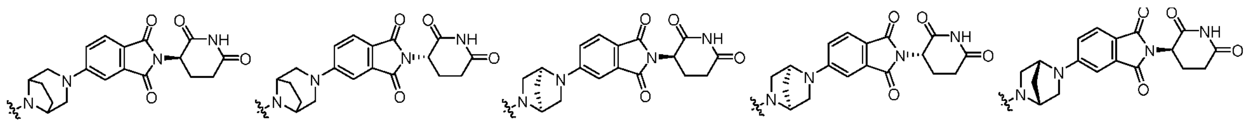

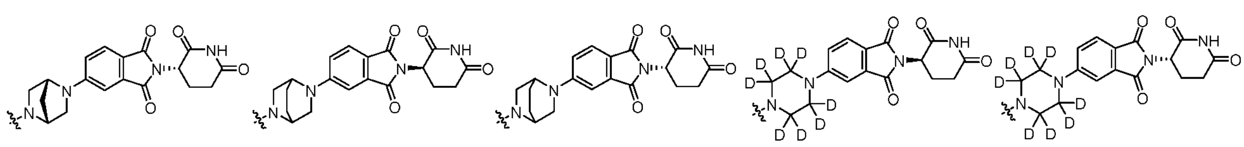

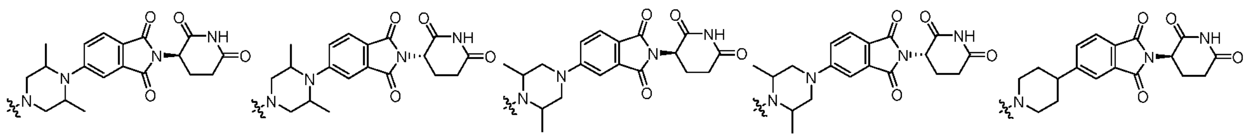

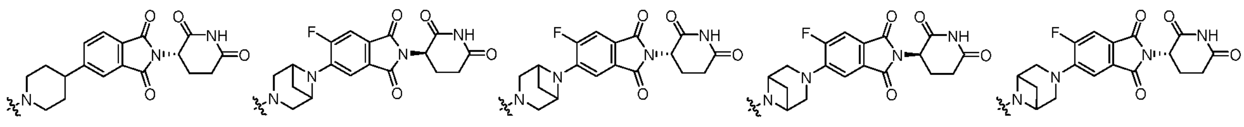

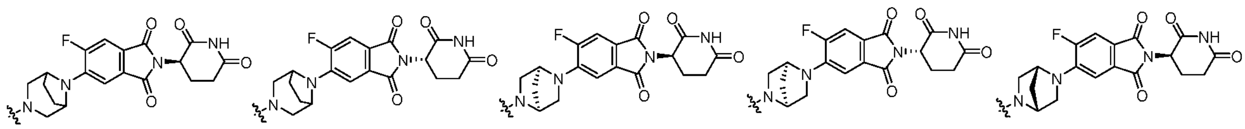

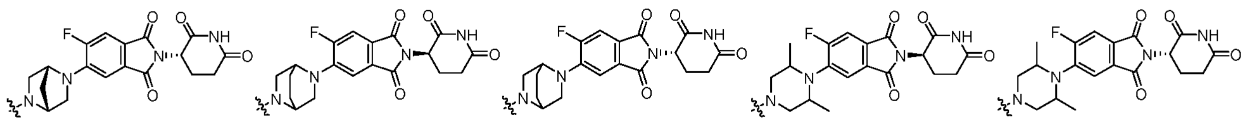

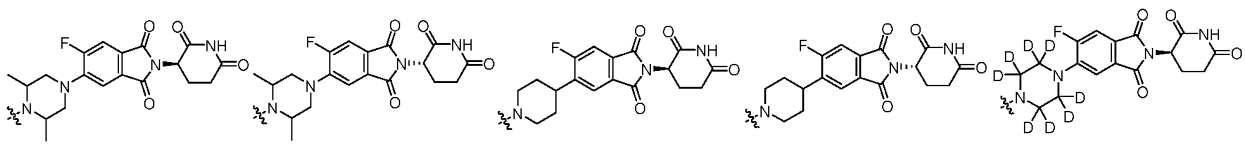

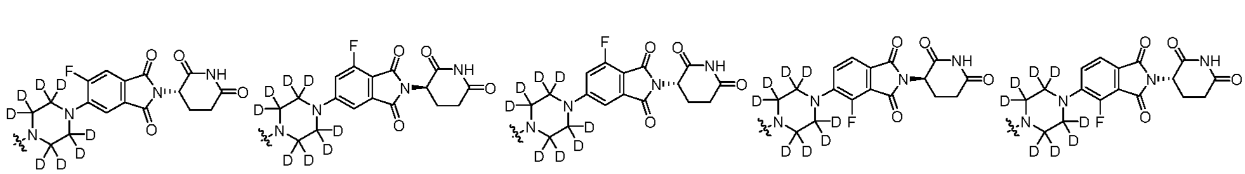

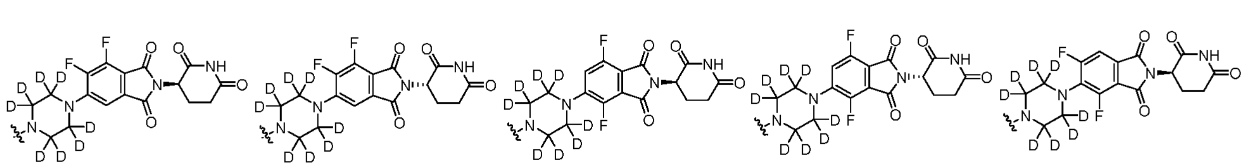

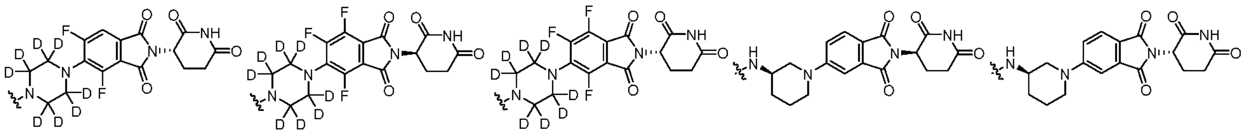

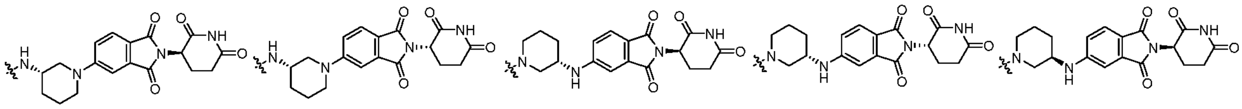

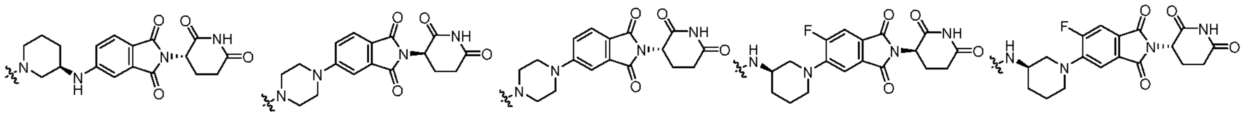

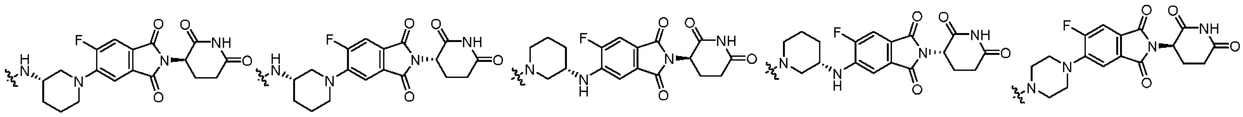

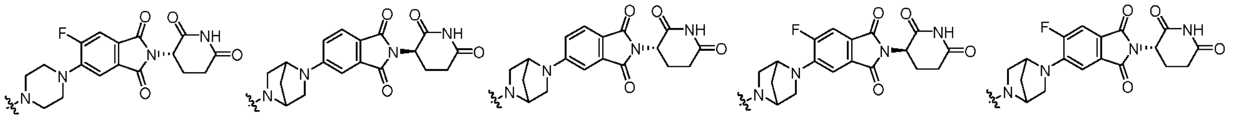

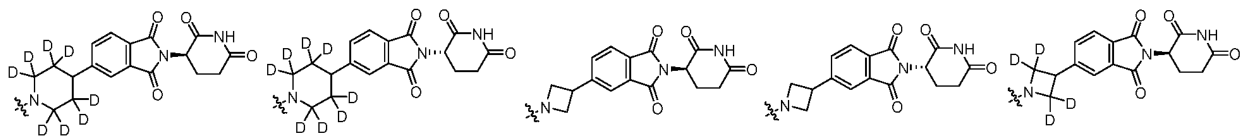

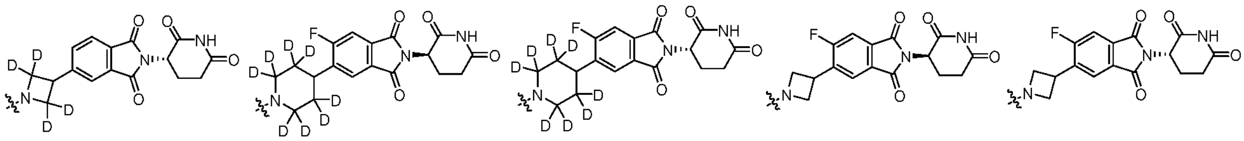

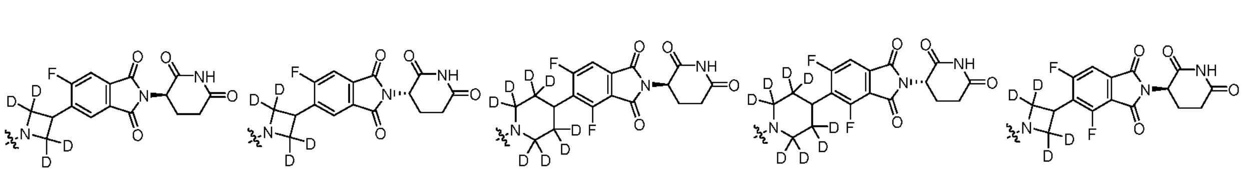

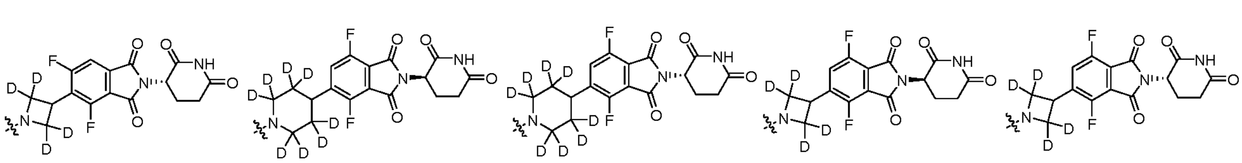

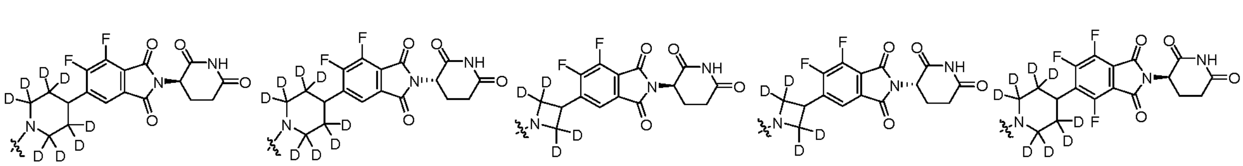

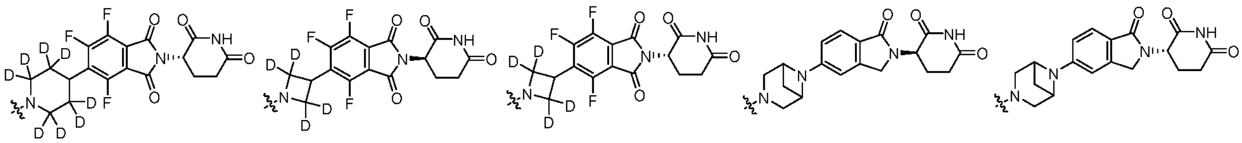

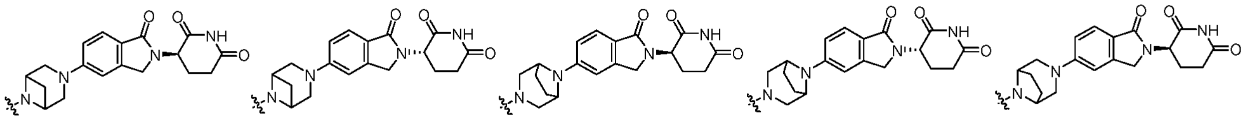

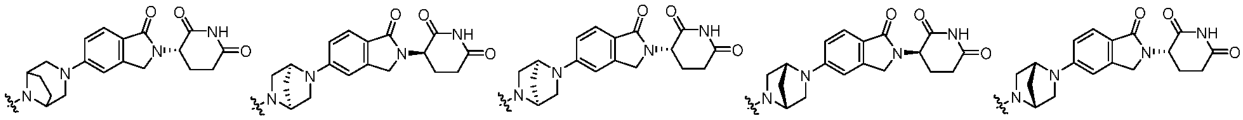

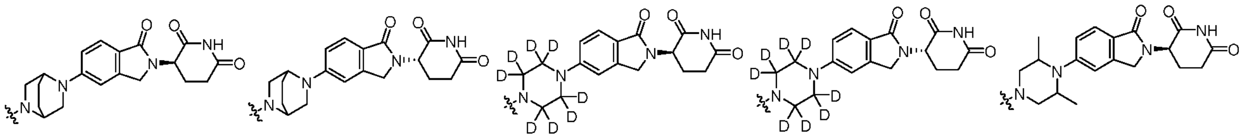

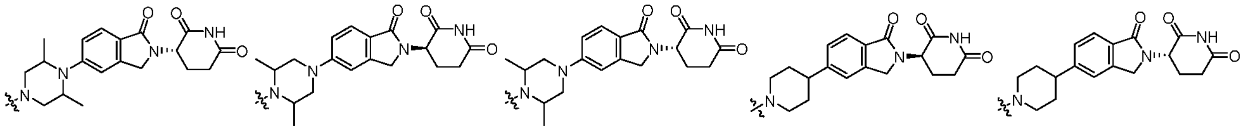

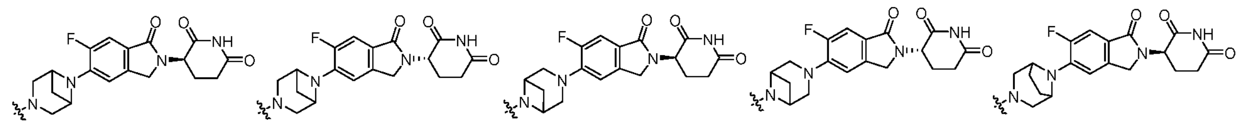

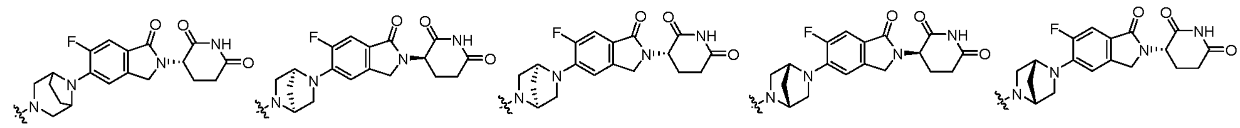

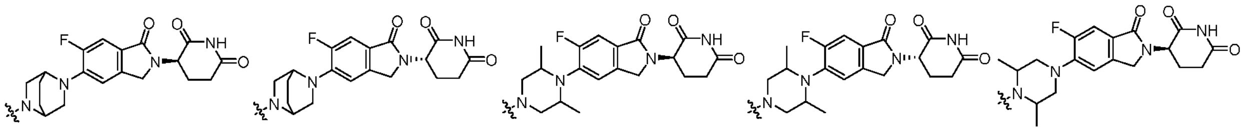

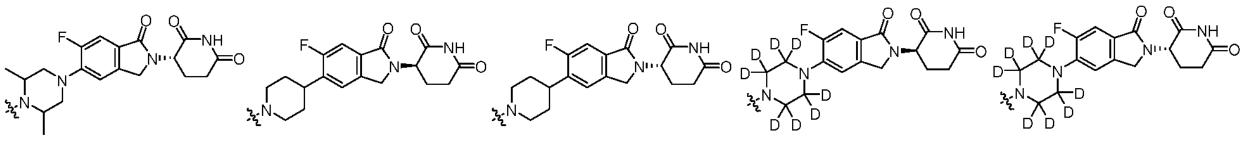

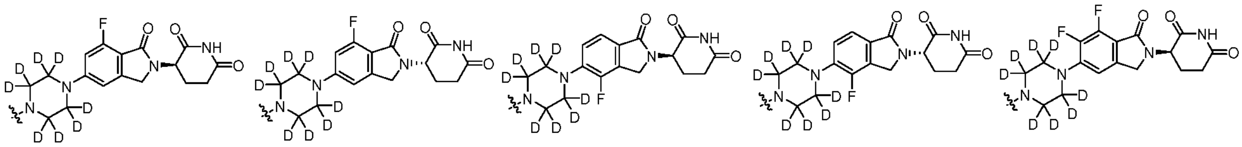

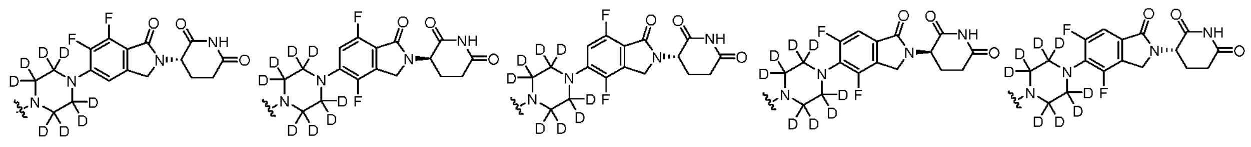

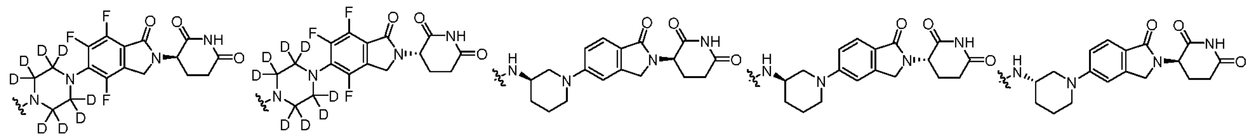

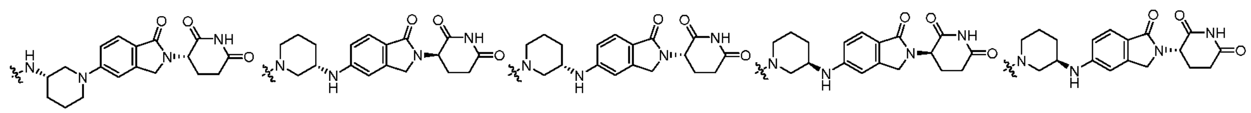

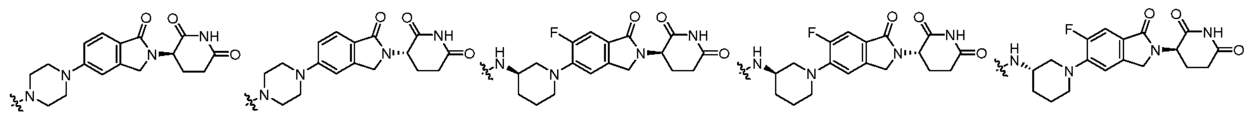

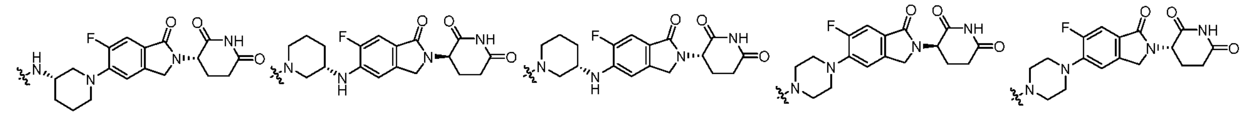

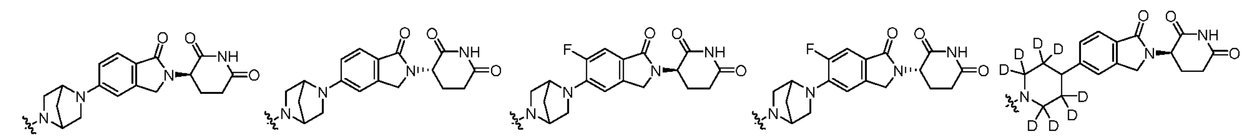

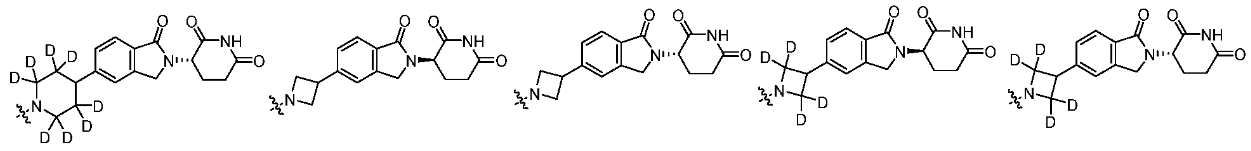

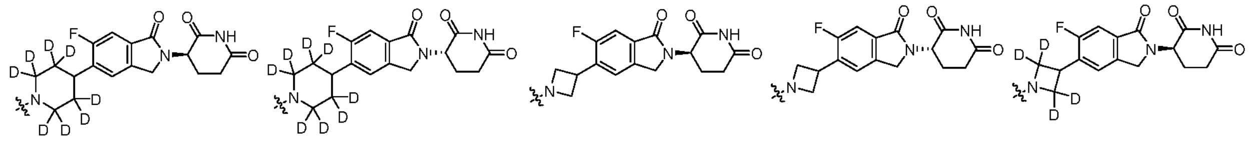

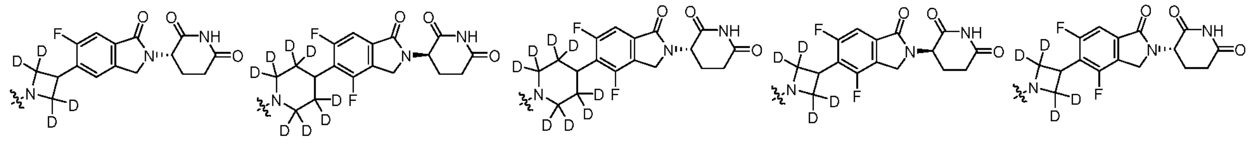

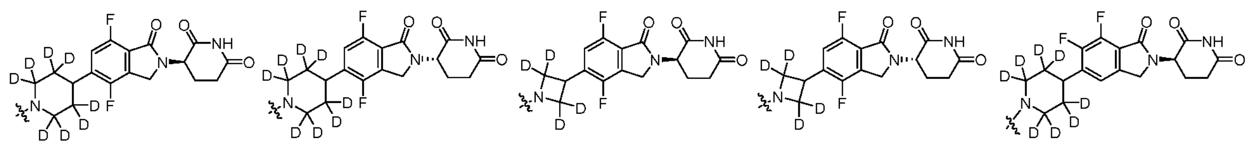

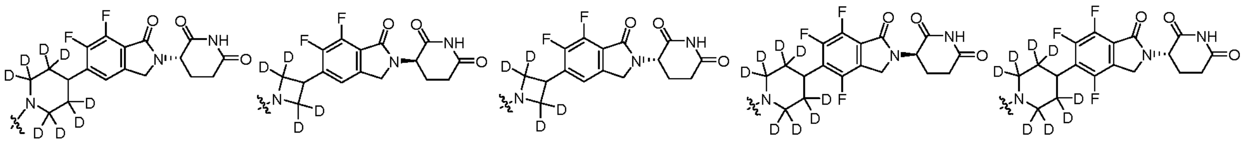

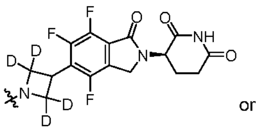 or 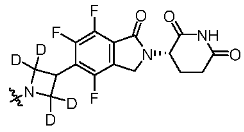 .

In some embodiments, LIN represents the structures of the following formula:

#-$C_{1-30}$ alkylene-;
#-$(C(R^{a1})(R^{a2}))_{n1}$-$(R_e(C(R^{a3})(R^{a4}))_{n2})_{m1}$-;
#-$(C(R^{a1})(R^{a2}))_{n1}$-$(R_e(C(R^{a3})(R^{a4}))_{n2})_{m1}$-$(R_e(C(R^{a5})(R^{a6}))_{n3})_{m2}$-;
#-$(C(R^{a1})(R^{a2}))_{n1}$-$(R_e(C(R^{a3})(R^{a4}))_{n2})_{m1}$-$(R_e(C(R^{a5})(R^{a6}))_{n3})_{m2}$-$(R_e(C(R^{a7})(R^{a8}))_{n4})_{m3}$-;
#-$(C(R^{a1})(R^{a2}))_{n1}$-$(R_f(C(R^{a3})(R^{a4}))_{n2})_{m1}$-;
#-$(C(R^{a1})(R^{a2}))$n1-$(R_f(C(R^{a3})(R^{a4}))_{n2})_{m1}$-$(R_f(C(R^{a5})(R^{a6}))_{n3})_{m2}$-;
#-$(C(R^{a1})(R^{a2}))_{n1}$-$(R_f(C(R^{a3})(R^{a4}))_{n2})_{m1}$-$(R_f(C(R^{a5})(R^{a6}))_{n3})_{m2}$-$(R_f(C(R^{a7})(R^{a8}))_{n4})_{m3}$-;
#-$(C(R^{a1})(R^{a2}))_{n1}$-$(R_e$-$R_f$-$(C(R^{a3})(R^{a4}))_{n2})$m1-;
#-$(C(R^{a1})(R^{a2}))_{n1}$-$(R_e(C(R^{a3})(R^{a4}))_{n2})_{m1}$-$(R_f(C(R^{a5})(R^{a6}))_{n3})_{m2}$-;
#-$(C(R^{a1})(R^{a2}))_{n1}$-$(R_f$-$R_e$-$(C(R^{a3})(R^{a4}))_{n2})_{m1}$-; or
#-$(C(R^{a1})(R^{a2}))$n1-$(R_f(C(R^{a3})(R^{a4}))_{n2})_{m1}$-$(R_e(C(R^{a5})(R^{a6}))_{n3})_{m2}$-;

wherein each $R_e$ are selected from the group consisting of O, N($R_g$), C(O), C(O)O, OC(O), S(O), $S(O)_2$, $S(O)_2NH$, $NHS(O)_2$, C(O)N($R_g$), N($R_g$)C(O), or N($R_g$)C(O)N($R_g$), where each $R_g$ independently represent H or $C_{1-6}$ alkyl (e.g., $C_{1-4}$ alkyl, such as methyl, ethyl, propyl, isopropyl, butyl, sec-butyl or tert-butyl); wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally deuterated cyclopropyl, cyclobutyl, cyclopentyl, or cyclohexyl), hydroxy, amino, mercapto, halogen (e.g., fluorine, chlorine, bromine, or iodine), optionally deuterated $C_{1-6}$ alkoxy, optionally deuterated $C_{1-6}$ alkyl-NH-, halogenated $C_{1-6}$ alkyl (e.g., halogenated $C_{1-4}$ alkyl, such as $F_3C$-, $FCH_2$-, $F_2CH$-, $ClCH_2$-, $C1_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene, optionally deuterated $C_{1-6}$ alkyl-NHC(O)-, optionally deuterated $C_{1-6}$ alkyl-C(O)NH-, cyano, or any combination thereof;

a hydrogen atom of one or more $CH_2$ groups of the $C_{1-30}$ alkylene group is optionally replaced with a substituent selected from the group consisting of deuterium, hydroxy, amino, mercapto, nitro, halogen (e.g., fluorine, chlorine, bromine, or iodine), cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $C1_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally deuterated cyclopropyl, cyclobutyl, cyclopentyl, or cyclohexyl), optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, or any combination thereof;

$R^{a1}$, $R^{a2}$, $R^{a3}$, $R^{a4}$, $R^{a5}$, $R^{a6}$, $R^{a7}$, and $R^{a8}$ each independently represent H, deuterium, hydroxy, amino, mercapto, nitro, halogen, cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally deuterated cyclopropyl, cyclobutyl, cyclopentyl, or cyclohexyl), optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, or any combination thereof;

symbol # indicates the point of attachment to $U_2$; and

n1, n2, n3, n4, m1, m2, m3 each independently represent an integer of 1, 2, 3, 4, 5, 6, 7, 8, 9, 10, 11, 12, 13,

14, or 15.

In some embodiments, LIN represents the structures of the following formula:

$\#\text{-}(C(R^{a1})(R^{a2}))_{n1}\text{-}(O(C(R^{a3})(R^{a4}))_{n2})_{m1}\text{-};$
$\#\text{-}(C(R^{a1})(R^{a2}))_{n1}\text{-}(O(C(R^{a3})(R^{a4}))_{n2})_{m1}\text{-}(O(C(R^{a5})(R^{a6}))_{n3})_{m2}\text{-};$
$\#\text{-}(C(R^{a1})(R^{a2}))_{n1}\text{-}(O(C(R^{a3})(R^{a4}))_{n2})_{m1}\text{-}(O(C(R^{a5})(R^{a6}))_{n3})m2\text{-}(O(C(R^{a7})(R^{a8}))_{n4})_{m3}\text{-};$
$\#\text{-}(C(R^{a1})(R^{a2}))_{n1}\text{-}(N(R_g)(C(R^{a3})(R^{a4}))_{n2})_{m1}\text{-};$
$\#\text{-}(C(R^{a1})(R^{a2}))_{n1}\text{-}(N(R_g)(C(R^{a3})(R^{a4}))_{n2})_{m1}\text{-}(N(R_g)(C(R^{a5})(R^{a6}))_{n3})_{m2}\text{-};$
$\#\text{-}(C(R^{a1})(R^{a2}))_{n1}\text{-}(N(R_g)(C(R^{a3})(R^{a4}))_{n2})_{m1}\text{-}(N(R_g)(C(R^{a5})(R^{a6}))_{n3})_{m2}\text{-}(N(R_g)(C(R^{a7})(R^{a8}))_{n4})_{m3}\text{-};$
$\#\text{-}(C(R^{a1})(R^{a2}))_{n1}\text{-}(C(O)N(R_g)\text{-}(C(R^{a3})(R^{a4}))_{n2})_{m1}\text{-};$
$\#\text{-}(C(R^{a1})(Ra^{2}))_{n1}\text{-}(C(O)N(R_g)\text{-}(C(R^{a3})(R^{a4}))_{n2})_{m1}\text{-}(C(O)N(R_g)\text{-}(C(R^{a5})(R^{a6}))_{n3})_{m2}\text{-};$
$\#\text{-}(C(R^{a1})(R^{a2}))_{n1}\text{-}(C(O)N(R_g)\text{-}(C(R^{a3})(R^{a4}))_{n2})_{m1}\text{-}(C(O)N(R_g)\text{-}(C(R^{a5})(R^{a6}))_{n3})_{m2}\text{-}(C(O)N(R_g)\text{-}(C(R^{a7})(R^{a8}))_{n4})_{m3}\text{-};$
$\#\text{-}(C(R^{a1})(R^{a2}))_{n1}\text{-}(C(O)N(R_g)\text{-}(C(R^{a3})(R^{a4}))_{n2})_{m1}\text{-}(O\text{-}(C(R^{a5})(R^{a6}))_{n3})_{m2}\text{-};$
$\#\text{-}(C(R^{a1})(R^{a2}))_{n1}\text{-}C(O)N(R_g)\text{-}(C(R^{a3})(R^{a4}))_{n2}\text{-}(O(C(R^{a5})(R^{a6}))_{n3})_{m1}\text{-};$
$\#\text{-}(C(R^{a1})(R^{a2}))_{n1}\text{-}(N(R_g)C(O)\text{-}(C(R^{a3})(R^{a4}))_{n2})_{m1}\text{-};$
$\#\text{-}(C(R^{a1})(R^{a2}))_{n1}\text{-}(N(R_g)C(O)\text{-}(C(R^{a3})(R^{a4}))_{n2})_{m1}\text{-}(O\text{-}(C(R^{a5})(R^{a6}))_{n3})_{m2}\text{-};$
$\#\text{-}(C(R^{a1})(R^{a2}))_{n1}\text{-}(N(R_g)C(O)\text{-}(C(R^{a3})(R^{a4}))_{n2})_{m1}\text{-}(O\text{-}(C(R^{a5})(R^{a6}))_{n3})_{m2}\text{-}(O\text{-}(C(R_g)(R^{a8}))_{n4})_{m3}\text{-};$
$\#\text{-}(C(R^{a1})(R^{a2}))_{n1}\text{-}N(R_g)C(O)\text{-}(C(R^{a3})(R^{a4}))_{n2}\text{-}(O(C(R^{a5})(R^{a6}))_{n3})_{m1}\text{-};$
$\#\text{-}(C(R^{a1})(R^{a2}))_{n1}\text{-}N(R_g)C(O)N(R_g)\text{-}(C(R^{a3})(R^{a4}))_{n2}\text{-};$
$\#\text{-}(C(R^{a1})(R^{a2}))_{n1}\text{-}C(O)\text{-}(C(R^{a3})(R^{a4}))_{n2}\text{-};$
$\#\text{-}(C(R^{a1})(R^{a2}))n1\text{-}(\text{arylene-}(C(R^{a3})(R^{a4}))_{n2})_{m1}\text{-};$
$\#\text{-}(C(R^{a1})(R^{a2}))_{n1}\text{-}(\text{arylene-}(C(R^{a3})(R^{a4}))_{n2})_{m1}\text{-arylene-}(C(R^{a5})(R^{a6}))_{n3\text{-};}$
$\#\text{-}(C(R^{a1})(R^{a2}))_{n1}\text{-}(\text{heterocyclylene-}(C(R^{a3})(R^{a4}))_{n2})_{m1}\text{-};$
$\#\text{-}(C(R^{a1})(R^{a2}))_{n1}\text{-}(\text{heterocyclylene-}(C(R^{a3})(R^{a4}))_{n2})_{m1}\text{-}(\text{heterocyclylene-}(C(R^{a5})(R^{a6}))_{n3})_{m2}\text{-};$
$\#\text{-}(C(R^{a1})(R^{a2}))_{n1}\text{-}(\text{heteroarylene-}(C(R^{a3})(R^{a4}))_{n2})m1\text{-};$
$\#\text{-}(C(R^{a1})(R^{a2}))_{n1}\text{-}(\text{heteroarylene-}(C(R^{a3})(R^{a4}))_{n2})_{m1}\text{-}(\text{heteroarylene-}(C(R^{a5})(R^{a6}))_{n3})_{m2}\text{-};$
$\#\text{-}(C(R^{a1})(R^{a2}))_{n1}\text{-}(\text{cycloalkylene-}(C(R^{a3})(R^{a4}))_{n2})_{m1}\text{-};$ or
$\#\text{-}(C(R^{a1})(R^{a2}))_{n1}\text{-}(\text{cycloalkylene-}(C(R^{a3})(R^{a4}))_{n2})_{m1}\text{-}(\text{cycloalkylene-}(C(R^{a5})(R^{a6}))_{n3})_{m2}\text{-};$

wherein each $R_g$ independently represent H or $C_{1-6}$ alkyl;
the cycloalkylene, arylene, heterocyclylene, and heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally deuterated cyclopropyl, cyclobutyl, cyclopentyl, or cyclohexyl), hydroxy, amino, mercapto, halogen (e.g., fluorine, chlorine, bromine, or iodine), optionally deuterated $C_{1-6}$ alkoxy, optionally deuterated $C_{1-6}$ alkyl-NH-, halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene, optionally deuterated $C_{1-6}$ alkyl-NHC(O)-, optionally deuterated $C_{1-6}$ alkyl-C(O)NH-, cyano, or any combination thereof;
$R^{a1}$, $R^{a2}$, $R^{a3}$, $R^{a4}$, $R^{a5}$, $R^{a6}$, $R^{a7}$, and $R^{a8}$ each independently represent H, deuterium, hydroxy, amino, mercapto, nitro, halogen (e.g., fluorine, chlorine, bromine, or iodine), cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $C1_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$ cycloalkyl (e.g., optionally deuterated cyclopropyl, cyclobutyl, cyclopentyl, or cyclohexyl), optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, or any combination thereof;
symbol # indicates the point of attachment to $U_2$; and
n1, n2, n3, n4, m1, m2, m3 each independently represent an integer of 1, 2, 3, 4, 5, 6, 7, 8, 9, 10, 11, 12, 13, 14, or 15.

In some embodiments, LIN represents the following groups:

$\#\text{-}CH_2\text{-}$, $\#\text{-}(CH_2)_2\text{-}$, $\#\text{-}(CH_2)_3\text{-}$, $\#\text{-}(CH_2)_4\text{-}$, $\#\text{-}(CH_2)_5\text{-}$, $\#\text{-}(CH_2)_6\text{-}$, $\#\text{-}(CH_2)_7\text{-}$, $\#\text{-}(CH_2)_8\text{-}$, $\#\text{-}(CH_2)_9\text{-}$, $\#\text{-}(CH_2)_{10}\text{-}$, $\#\text{-}(CH_2)_{11}\text{-}$, $\#\text{-}(CH_2)_{12}\text{-}$, $\#\text{-}(CH_2)_{13}\text{-}$, $\#\text{-}(CH_2)_{14}\text{-}$, $\#\text{-}(CH_2)_{15}\text{-}$, $\#\text{-}(CH_2)_{16}\text{-}$, $\#\text{-}(CH_2)_{17}\text{-}$, $\#\text{-}(CH_2)_{18}\text{-}$, $\#\text{-}(CH_2)_{19}\text{-}$,

#-$(CH_2)_{20}$-, #-$(CH_2)_{21}$-, #-$(CH_2)_{22}$-, #-$(CH_2)_{25}$-, or #-$(CH_2)_{30}$-;#-$CH_2$-O-$CH_2$-, #-$CH_2$-O-$(CH_2)_2$-, #-$(CH_2)_1$-O-$(CH_2)_3$-, #-$(CH_2)_1$-O-$(CH_2)_4$-, #-$(CH_2)_1$-O-$(CH_2)_5$-, #-$(CH_2)_1$-O-$(CH_2)_6$-, #-$(CH_2)_1$-O-$(CH_2)_7$-, #-$(CH_2)_1$-O-$(CH_2)_8$-, #-$(CH_2)_1$-O-$(CH_2)_9$-, #-$(CH_2)_1$-O-$(CH_2)_{10}$-, #-$(CH_2)_2$-O-$(CH_2)_1$-, #-$(CH_2)_2$-O-$(CH_2)_2$-, #-$(CH_2)_2$-O-$(CH_2)_3$-, #-$(CH_2)_2$-O-$(CH_2)_4$-, #-$(CH_2)_2$-O-$(CH_2)_5$-, #-$(CH_2)_2$-O-$(CH_2)_6$-, #-$(CH_2)_2$-O-$(CH_2)_7$-, #-$(CH_2)_2$-O-$(CH_2)_8$-, #-$(CH_2)_2$-O-$(CH_2)_9$-, #-$(CH_2)_2$-O-$(CH_2)_{10}$-, #-$(CH_2)_2$-O-$(CH_2)_{11}$-, #-$(CH_2)_2$-O-$(CH_2)_{12}$-, #-$(CH_2)_3$-O-$(CH_2)_1$-, #-$(CH_2)_3$-O-$(CH_2)_2$-, #-$(CH_2)_3$-O-$(CH_2)_3$-, #-$(CH_2)_3$-O-$(CH_2)_4$-, #-$(CH_2)_3$-O-$(CH_2)_5$-, #-$(CH_2)_3$-O-$(CH_2)_6$-, #-$(CH_2)_3$-O-$(CH_2)_7$-, #-$(CH_2)_4$-O-$(CH_2)_1$-, #-$(CH_2)_4$-O-$(CH_2)_2$-, #-$(CH_2)_4$-O-$(CH_2)_3$-, #-$(CH_2)_4$-O-$(CH_2)_4$-, #-$(CH_2)_4$-O-$(CH_2)_5$-, #-$(CH_2)_4$-O-$(CH_2)_6$-, #-$(CH_2)_5$-O-$(CH_2)_1$-, #-$(CH_2)_5$-O-$(CH_2)_2$-, #-$(CH_2)_5$-O-$(CH_2)_3$-, #-$(CH_2)_5$-O-$(CH_2)_4$-, #-$(CH_2)_5$-O-$(CH_2)_5$-, #-$(CH_2)_6$-O-$(CH_2)_1$-, #-$(CH_2)_6$-O-$(CH_2)_2$-, #-$(CH_2)_6$-O-$(CH_2)_3$-, #-$(CH_2)_6$-O-$(CH_2)_4$-, #-$(CH_2)_7$-O-$(CH_2)_1$-, #-$(CH_2)_7$-O-$(CH_2)_2$-, #-$(CH_2)_7$-O-$(CH_2)_3$-, #-$(CH_2)_8$-O-$(CH_2)_1$-, #-$(CH_2)_8$-O-$(CH_2)_2$-, #-$CH(CH_3)$-O-$(CH_2)_1$-, #-$CH(CH_3)$-O-$(CH_2)_2$-, #-$CH(CH_3)$-O-$(CH_2)_3$-, #-$CH(CH_3)$-O-$(CH_2)_4$-, #-$CH(CH_3)$-O-$(CH_2)_5$-, #-$CH(CH_3)$-O-$(CH_2)_6$-, #-$CH(CH_3)$-O-$(CH_2)_7$-, #-$CH(CH_3)$-O-$(CH_2)_8$-, #-$CH(CH_3)$-O-$(CH_2)_9$-, #-$CH(CH_3)$-O-$(CH_2)_{10}$-, #-$CH_2$-$(O(CH_2)_2)_2$-, #-$CH_2$-$(O(CH_2)_2)_3$-, #-$CH_2$-$(O(CH_2)_2)_4$-, #-$CH_2$-$(O(CH_2)_2)_5$-, #-$CH_2$-$(O(CH_2)_2)_6$-, #-$CH_2$-$(O(CH_2)_2)_7$-, #-$CH_2$-$(O(CH_2)_2)_8$-, #-$CH_2$-$(O(CH_2)_2)_9$-, #-$CH_2$-$(O(CH_2)_2)_{10}$-, #-$CH_2$-$(O(CH_2)_2)_1$-$OCH_2$-, #-$CH_2$-$(O(CH_2)_2)_2$-$OCH_2$-, #-$CH_2$-$(O(CH_2)_2)_3$-$OCH_2$-, #-$CH_2$-$(O(CH_2)_2)_4$-$OCH_2$-, #-$CH_2$-$(O(CH_2)_2)_5$-$OCH_2$-, #-$CH_2$-$(O(CH_2)_2)_6$-$OCH_2$-, #-$CH_2$-$(O(CH_2)_2)_7$-$OCH_2$-, #-$CH_2$-$(O(CH_2)_2)_8$-$OCH_2$-, #-$CH_2$-$(O(CH_2)_2)_9$-$OCH_2$-, #-$CH_2$-$(O(CH_2)_2)_{10}$-$OCH_2$-, #-$(CH_2)_2$-$(O(CH_2)_2)_2$-, #-$(CH_2)_2$-$(O(CH_2)_2)_3$-, #-$(CH_2)_2$-$(O(CH_2)_2)_4$-, #-$(CH_2)_2$-$(O(CH_2)_2)_5$-, #-$(CH_2)_2$-$(O(CH_2)_2)_6$-, #-$(CH_2)_2$-$(O(CH_2)_2)_7$-, #-$(CH_2)_2$-$(O(CH_2)_2)_8$-, #-$(CH_2)_2$-$(O(CH_2)_2)_9$-, #-$(CH_2)_2$-$(O(CH_2)_2)_{10}$-, #-$(CH_2)_3$-$(O(CH_2)_2)_2$-, #-$(CH_2)_3$-$(O(CH_2)_2)_3$-, #-$(CH_2)_3$-$(O(CH_2)_2)_4$-, #-$(CH_2)_3$-$(O(CH_2)_2)_5$-, #-$(CH_2)_3$-$(O(CH_2)_2)_6$-, #-$(CH_2)_3$-$(O(CH_2)_2)_7$-, #-$(CH_2)_3$-$(O(CH_2)_2)_8$-, #-$(CH_2)_3$-$(O(CH_2)_2)_9$-, #-$(CH_2)_3$-$(O(CH_2)_2)_{10}$-, #-$(CH_2)_4$-$(O(CH_2)_2)_2$-, #-$(CH_2)_4$-$(O(CH_2)_2)_3$-, #-$(CH_2)_4$-$(O(CH_2)_2)_4$-, #-$(CH_2)_4$-$(O(CH_2)_2)_5$-, #-$(CH_2)_4$-$(O(CH_2)_2)_6$-, #-$(CH_2)_4$-$(O(CH_2)_2)_7$-, #-$(CH_2)_4$-$(O(CH_2)_2)_8$-, #-$(CH_2)_4$-$(O(CH_2)_2)_9$-, #-$(CH_2)_4$-$(O(CH_2)_2)_{10}$-, #-$CH_2$-$(O(CH_2)_3)_2$-, 9-$CH_2$-$(O(CH_2)_3)_3$-, #-$CH_2$-$(O(CH_2)_3)_4$-, #-$CH_2$-$(O(CH_2)_3)_5$-, #-$CH_2$-$(O(CH_2)_3)_6$-, #-$CH_2$-$(O(CH_2)_3)_7$-, #-$CH_2$-$(O(CH_2)_3)_8$-, #-$CH_2$-$(O(CH_2)_3)_9$-, #-$CH_2$-$(O(CH_2)_3)_{10}$-, #-$(CH_2)_2$-$(O(CH_2)_3)_2$-, #-$(CH_2)_2$-$(O(CH_2)_3)_3$-, #-$(CH_2)_2$-$(O(CH_2)_3)_4$-, #-$(CH_2)_2$-$(O(CH_2)_3)_5$-, #-$(CH_2)_2$-$(O(CH_2)_3)_6$-, #-$(CH_2)_2$-$(O(CH_2)_3)_7$-, #-$(CH_2)_2$-$(O(CH_2)_3)_8$-, #-$(CH_2)_2$-$(O(CH_2)_3)_9$-, #-$(CH_2)_2$-$(O(CH_2)_3)_{10}$-, #-$(CH_2)_3$-$(O(CH_2)_3)_2$-, #-$(CH_2)_3$-$(O(CH_2)_3)_3$-, #-$(CH_2)_3$-$(O(CH_2)_3)_4$-, #-$(CH_2)_3$-$(O(CH_2)_3)_5$-, #-$(CH_2)_3$-$(O(CH_2)_3)_6$-, #-$(CH_2)_3$-$(O(CH_2)_3)_7$-, #-$(CH_2)_3$-$(O(CH_2)_3)_8$-, #-$(CH_2)_3$-$(O(CH_2)_3)_9$-, #-$(CH_2)_3$-$(O(CH_2)_3)_{10}$-, #-$CH_2$-O-$(CH_2)_2$-O-$(CH_2)_3$-, #-$CH_2$-$(O(CH_2)_2)_2$-$(O(CH_2)_3)_2$-, #-$CH_2$-$(O(CH_2)_2)_3$-$(O(CH_2)_3)_3$-, #-$CH_2$-$(O(CH_2)_2)_4$-$(O(CH_2)_3)_4$-, #-$CH_2$-$(O(CH_2)_2)_5$-$(O(CH_2)_3)_5$-, #-$CH_2$-$(O(CH_2)_2)_6$-$(O(CH_2)_3)_6$-, #-$(CH_2)_2$-O-$(CH_2)_2$-O-$(CH_2)_3$-, #-$(CH_2)_2$-$(O(CH_2)_2)_2$-$(O(CH_2)_3)_2$-, #-$(CH_2)_2$-$(O(CH_2)_2)_3$-$(O(CH_2)_3)_3$-, #-$(CH_2)_2$-$(O(CH_2)_2)_4$-$(O(CH_2)_3)_4$-, #-$(CH_2)_2$-$(O(CH_2)_2)_5$-$(O(CH_2)_3)_5$-, #-$(CH_2)_2$-$(O(CH_2)_2)_6$-$(O(CH_2)_3)_6$-, #-$(CH_2)_3$-O-$(CH_2)_2$-O-$(CH_2)_3$-, #-$(CH_2)_3$-$(O(CH_2)_2)_2$-$(O(CH_2)_3)_2$-, #-$(CH_2)_3$-$(O(CH_2)_2)_3$-$(O(CH_2)_3)_3$-, #-$(CH_2)_3$-$(O(CH_2)_2)_4$-$(O(CH_2)_3)_4$-, #-$(CH_2)_3$-$(O(CH_2)_2)_5$-$(O(CH_2)_3)_5$-, #-$(CH_2)_3$-$(O(CH_2)_2)_6$-$(O(CH_2)_3)_6$-, #-$CH_2$-O-$(CH_2)_3$-O-$(CH_2)_2$-, #-$CH_2$-$(O(CH_2)_3)_2$-$(O(CH_2)_2)_2$-, #-$CH_2$-$(O(CH_2)_3)_3$-$(O(CH_2)_2)_3$-, #-$CH_2$-$(O(CH_2)_3)_4$-$(O(CH_2)_2)_4$-, #-$CH_2$-$(O(CH_2)_3)_5$-$(O(CH_2)_2)_5$-, #-$CH_2$-$(O(CH_2)_3)_6$-$(O(CH_2)_2)_6$-, #-$(CH_2)_2$-O-$(CH_2)_3$-O-$(CH_2)_2$-, #-$(CH_2)_2$-$(O(CH_2)_3)_2$-$(O(CH_2)_2)_2$-, #-$(CH_2)_2$-$(O(CH_2)_3)_3$-$(O(CH_2)_2)_3$-, #-$(CH_2)_2$-$(O(CH_2)_3)_4$-$(O(CH_2)_2)_4$-, #-$(CH_2)_2$-$(O(CH_2)_3)_5$-$(O(CH_2)_2)_5$-, #-$(CH_2)_2$-$(O(CH_2)_3)_6$-$(O(CH_2)_2)_6$-, #-$(CH_2)_3$-O-$(CH_2)_3$-O-$(CH_2)_2$-, #-$(CH_2)_3$-$(O(CH_2)_3)_2$-$(O(CH_2)_2)_2$-, #-$(CH_2)_3$-$(O(CH_2)_3)_3$-$(O(CH_2)_2)_3$-, #-$(CH_2)_3$-$(O(CH_2)_3)_4$-$(O(CH_2)_2)_4$-, #-$(CH_2)_3$-$(O(CH_2)_3)_5$-$(O(CH_2)_2)_5$-, #-$(CH_2)_3$-$(O(CH_2)_3)_6$-$(O(CH_2)_2)_6$-, #-$CH_2$-O-$(CH_2)_2$-O-$CH_2$-, #-$(CH_2)_2$-O-$(CH_2)_2$-O-$CH_2$-, #-$(CH_2)_2$-$(O(CH_2)_2)_2$-O-$(CH_2)_3$-, #-$(CH_2)_2$-$(O(CH_2)_2)_3$-O-$(CH_2)_3$-, #-$(CH_2)_2$-$(O(CH_2)_2)_4$-O-$(CH_2)_3$-, #-$(CH_2)_5$-$(O(CH_2)_2)_2$-O-$(CH_2)_5$-, #-$(CH_2)_5$-$(O(CH_2)_2)_2$-O-$(CH_2)_6$-, #-$(CH_2)_1$-$N(R_g)$-$(CH_2)_1$-, #-$(CH_2)_1$-$N(R_g)$-$(CH_2)_2$-, #-$(CH_2)_1$-$N(R_g)$-$(CH_2)_3$-, #-$(CH_2)_1$-$N(R_g)$-$(CH_2)_4$-, #-$(CH_2)_1$-$N(R_g)$-$(CH_2)_5$-, #-$(CH_2)_1$-$N(R_g)$-$(CH_2)_6$-, #-$(CH_2)_1$-$N(R_g)$-$(CH_2)_7$-, #-$(CH_2)_1$-$N(R_g)$-$(CH_2)_8$-, #-$(CH_2)_1$-$N(R_g)$-$(CH_2)_9$-, #-$(CH_2)_1$-$N(R_g)$-$(CH_2)_{10}$-, #-$(CH_2)_2$-$N(R_g)$-$(CH_2)_1$-, #-$(CH_2)_2$-$N(R_g)$-$(CH_2)_2$-, #-$(CH_2)_2$-$N(R_g)$-$(CH_2)_3$-, #-$(CH_2)_2$-$N(R_g)$-$(CH_2)_4$-, #-$(CH_2)_2$-$N(R_g)$-$(CH_2)_5$-, #-$(CH_2)_2$-$N(R_g)$-$(CH_2)_6$-, #-$(CH_2)_2$-$N(R_g)$-$(CH_2)_7$-, #-$(CH_2)_2$-$N(R_g)$-$(CH_2)_8$-, #-$(CH_2)_2$-$N(R_g)$-$(CH_2)_9$-, #-$(CH_2)_2$-$N(R_g)$-$(CH_2)_{10}$-, #-$(CH_2)_2$-$N(R_g)$-$(CH_2)_{11}$-, #-$(CH_2)_2$-$N(R_g)$-$(CH_2)_{12}$-, #-$(CH_2)_3$-$N(R_g)$-$(CH_2)_1$-, #-$(CH_2)_3$-$N(R_g)$-$(CH_2)_2$-, #-$(CH_2)_3$-$N(R_g)$-$(CH_2)_3$-, #-$(CH_2)_4$-$N(R_g)$-$(CH_2)_1$-, #-$(CH_2)_4$-$N(R_g)$-$(CH_2)_2$-, #-$(CH_2)_4$-$N(R_g)$-$(CH_2)_3$-, #-$(CH_2)_4$-$N(R_g)$-$(CH_2)_4$-, #-$(CH_2)_5$-$N(R_g)$-$(CH_2)_1$-, #-$(CH_2)_5$-$N(R_g)$-$(CH_2)_2$-, #-$(CH_2)_5$-$N(R_g)$-$(CH_2)_3$-, #-$(CH_2)_5$-$N(R_g)$-$(CH_2)_4$-, #-$(CH_2)_5$-$N(R_g)$-$(CH_2)_5$-, #-$(CH_2)_6$-$N(R_g)$-$(CH_2)_1$-, #-$(CH_2)_6$-$N(R_g)$-$(CH_2)_2$-, #-$(CH_2)_6$-$N(R_g)$-$(CH_2)_3$-, #-$(CH_2)_7$-$N(R_g)$-$(CH_2)_1$-, #-$(CH_2)_7$-$N(R_g)$-$(CH_2)_2$-, #-$(CH_2)_7$-$N(R_g)$-$(CH_2)_3$-, #-$(CH_2)_8$-$N(R_g)$-$(CH_2)_1$-, #-$(CH_2)_8$-$N(R_g)$-$(CH_2)_2$-, #-$(CH_2)_8$-$N(R_g)$-$(CH_2)_3$-, #-$CH(CH_3)$-$N(R_g)$-$(CH_2)_1$-, #-$CH(CH_3)$-$N(R_g)$-$(CH_2)_2$-, #-$CH(CH_3)$-$N(R_g)$-$(CH_2)_3$-, #-$CH(CH_3)$-$N(R_g)$-$(CH_2)_4$-, 9-$CH(CH_3)$-$N(R_g)$-$(CH_2)_5$-, #-$CH(CH_3)$-$N(R_g)$-$(CH_2)_6$-, #-$CH(CH_3)$-$N(R_g)$-$(CH_2)_7$-, #-$CH(CH_3)$-$N(R_g)$-$(CH_2)_8$-, #-$CH(CH_3)$-$N(R_g)$-$(CH_2)_9$-, #-$CH(CH_3)$-$N(R_g)$-$(CH_2)_{10}$-, #-$CH_2C(O)NHCH_2$-, #-$(CH_2)_2C(O)NH(CH_2)_2$-, #-$(CH_2)_2C(O)NH(CH_2)_3$-, #-$(CH_2)_2C(O)NH(CH_2)_4$-, #-$(CH_2)_2C(O)NH(CH_2)_5$-, #-$(CH_2)_3C(O)NH(CH_2)_3$-, #-$(CH_2)_3C(O)NH(CH_2)_4$-, #-$(CH_2)_4C(O)NH(CH_2)_4$-, #-$(CH_2)_5C(O)NH(CH_2)_5$-, #-$(CH_2)_6C(O)NH(CH_2)_7$-, #-$(CH_2)_6C(O)NH(CH_2)_6$-, #-$(CH_2)_7C(O)NH(CH_2)_7$-, #-$(CH_2)_8C(O)NH(CH_2)_8$,

U-$(CH_2)_9$C(O)NH$(CH_2)_9$-, #-$(CH_2)_{10}$C(O)NH$(CH_2)_{10}$-, #-$(CH_2)_2$C(O)NH$(CH_2)_2$-O-$(CH_2)_2$-, #-$CH_2$NHC(O)$CH_2$-, #-$(CH_2)_2$NHC(O)$(CH_2)_2$-, #-$(CH_2)_2$NHC(O)$(CH_2)_3$-, #-$(CH_2)_2$NHC(O)$(CH_2)_4$-, #-$(CH_2)_2$NHC(O)$(CH_2)_5$-, #-$(CH_2)_3$NHC(O)$(CH_2)_3$-, #-$(CH_2)_3$NHC(O)$(CH_2)_4$-, #-$(CH_2)_4$NHC(O)$(CH_2)_4$-, #-$(CH_2)_5$NHC(O)$(CH_2)_5$-, #-$(CH_2)_6$NHC(O)$(CH_2)_7$-, #-$(CH_2)_6$NHC(O)$(CH_2)_6$-, #-$(CH_2)_7$NHC(O)$(CH_2)_7$-, #-$(CH_2)_8$NHC(O)$(CH_2)_8$, #-$(CH_2)_9$NHC(O)$(CH_2)_9$-, #-$(CH_2)_{10}$NHC(O)$(CH_2)_{10}$-, #-$(CH_2)_4$NHC(O)$(CH_2)_8$-, #-$(CH_2)_2$NHC(O)$(CH_2)_2$-O-$(CH_2)_2$-, #-$(CH_2)_4$NHC(O)$CH_2$-, #-$CH_2$-piperidinylene-$CH_2$-, #-$CH_2$-piperidinylene-$(CH_2)_2$-, #-$CH_2$-piperidinylene-$(CH_2)_3$-, #-$CH_2$-piperidinylene-$(CH_2)_4$-, #-$CH_2$-piperidinylene-$(CH_2)_5$-, #-$CH_2$-piperidinylene-$(CH_2)_6$-, #-$CH_2$-piperidinylene-$(CH_2)_7$-, #-$CH_2$-piperidinylene-$(CH_2)_8$-, #-$(CH_2)_2$-piperidinylene-$(CH_2)_1$-, #-$(CH_2)_2$-piperidinylene-$(CH_2)_2$-, #-$(CH_2)_2$-piperidinylene-$(CH_2)_3$-, #-$(CH_2)_2$-piperidinylene-$(CH_2)_4$-, #-$(CH_2)_2$-piperidinylene-$(CH_2)_5$-, #-$(CH_2)_2$-piperidinylene-$(CH_2)_6$-, #-$(CH_2)_2$-piperidinylene-$(CH_2)_7$-, #-$(CH_2)_2$-piperidinylene-$(CH_2)_8$-, #-$(CH_2)_3$-piperidinylene-$CH_2$-, #-$(CH_2)_3$-piperidinylene-$(CH_2)_2$-, #-$(CH_2)_3$-piperidinylene-$(CH_2)_3$-, #-$(CH_2)_3$-piperidinylene-$(CH_2)_4$-, #-$(CH_2)_3$-piperidinylene-$(CH_2)_5$-, #-$(CH_2)_3$-piperidinylene-$(CH_2)_6$-, #-$(CH_2)_3$-piperidinylene-$(CH_2)_7$-, #-$(CH_2)_3$-piperidinylene-$(CH_2)_8$-, #-$(CH_2)_4$-piperidinylene-$CH_2$-, #-$(CH_2)_4$-piperidinylene-$(CH_2)_2$-, #-$(CH_2)_4$-piperidinylene-$(CH_2)_3$-, #-$(CH_2)_4$-piperidinylene-$(CH_2)_4$-, #-$(CH_2)_4$-piperidinylene-$(CH_2)_5$-, #-$(CH_2)_4$-piperidinylene-$(CH_2)_6$-, #-$(CH_2)_4$-piperidinylene-$(CH_2)_7$-, #-$(CH_2)_4$-piperidinylene-$(CH_2)_8$-, #-$(CH_2)_5$-piperidinylene-$(CH_2)_1$-, #-$(CH_2)_5$-piperidinylene-$(CH_2)_2$-, #-$(CH_2)_5$-piperidinylene-$(CH_2)_3$-, #-$(CH_2)_5$-piperidinylene-$(CH_2)_4$-, #-$(CH_2)_5$-piperidinylene-$(CH_2)_5$-, #-$(CH_2)_5$-piperidinylene-$(CH_2)_6$-, #-$(CH_2)_5$-piperidinylene-$(CH_2)_7$-, #-$(CH_2)_5$-piperidinylene-$(CH_2)_8$-, #-$(CH_2)_6$-piperidinylene-$(CH_2)_1$-, #-$(CH_2)_6$-piperidinylene-$(CH_2)_2$-, #-$(CH_2)_6$-piperidinylene-$(CH_2)_3$-, #-$(CH_2)_6$-piperidinylene-$(CH_2)_4$-, #-$(CH_2)_6$-piperidinylene-$(CH_2)_5$-, #-$(CH_2)_6$-piperidinylene-$(CH_2)$e-, #-$(CH_2)_6$-piperidinylene-$(CH_2)_7$-, #-$(CH_2)_6$-piperidinylene-$(CH_2)_5$-, #-$(CH_2)_7$-piperidinylene-$(CH_2)_1$-, #-$(CH_2)_7$-piperidinylene-$(CH_2)_2$-, #-$(CH_2)_7$-piperidinylene-$(CH_2)_3$-, #-$(CH_2)_7$-piperidinylene-$(CH_2)_4$-, #-$(CH_2)_7$-piperidinylene-$(CH_2)_8$-, #-$(CH_2)_8$-piperidinylene-$CH_2$-, #-$(CH_2)_8$-piperidinylene-$(CH_2)_2$-, #-$(CH_2)_8$-piperidinylene-$(CH_2)_3$-, #-$(CH_2)_8$-piperidinylene-$(CH_2)_4$-, #-$(CH_2)_8$-pipendinylene-$(CH_2)_5$-, #-$(CH_2)_8$-piperidinylene-$(CH_2)_6$-, #-$(CH_2)_8$-piperidinylene-$(CH_2)_7$-, #-$(CH_2)_8$-pipendinylene-$(CH_2)_8$-, #-$CH_2$-N($R_g$)-$CH_2$-piperidinylene-$CH_2$-, #-$CH_2$-N($R_g$)-$CH_2$-piperidinylene-$(CH_2)_2$-, #-$CH_2$-N($R_g$)-$CH_2$-piperidinylene-$(CH_2)_3$-, #-$CH_2$-N($R_g$)-$CH_2$-piperidinylene-$(CH_2)_4$-, #-$CH_2$-N($R_g$)-$CH_2$-piperidinylene-$(CH_2)_5$-, #-$CH_2$-N($R_g$)-$CH_2$-piperidinylene-$(CH_2)_6$-, #-$CH_2$-N($R_g$)-$CH_2$-piperidinylene-$(CH_2)_7$-, #-$CH_2$-N($R_g$)-$CH_2$-piperidinylene-$(CH_2)_8$-, #-$CH_2$-N($R_g$)-$(CH_2)_2$-piperidinylene-$CH_2$-, #-$CH_2$-N($R_g$)-$(CH_2)_2$-piperidinylene-$(CH_2)_2$-, #-$CH_2$-N($R_g$)-$(CH_2)_2$-piperidinylene-$(CH_2)_3$-, #-$CH_2$-N($R_g$)-$(CH_2)_2$-piperidinylene-$(CH_2)_4$-, #-$CH_2$-N($R_g$)-$(CH_2)_2$-piperidinylene-$(CH_2)_5$-, #-$CH_2$-N($R_g$)-$(CH_2)_2$-piperidinylene-$(CH_2)_6$-, #-$CH_2$-N($R_g$)-$(CH_2)_2$-piperidinylene-$(CH_2)_7$-, #-$CH_2$-N($R_g$)-$(CH_2)_2$-piperidinylene-$(CH_2)_8$-, #-$(CH_2)_2$-N($R_g$)-$CH_2$-piperidinylene-$CH_2$-, #-$(CH_2)_2$-N($R_g$)-$CH_2$-piperidinylene-$(CH_2)_2$-, #-$(CH_2)_2$-N($R_g$)-$CH_2$-piperidinylene-$(CH_2)_3$-, #-$(CH_2)_2$-N($R_g$)-$CH_2$-piperidinylene-$(CH_2)_4$-, #-$(CH_2)_2$-N($R_g$)-$CH_2$-piperidinylene-$(CH_2)_5$-, #-$(CH_2)_2$-N($R_g$)-$CH_2$-piperidinylene-$(CH_2)_6$-, #-$(CH_2)_2$-N($R_g$)-$CH_2$-piperidinylene-$(CH_2)_7$-, #-$(CH_2)_2$-N($R_g$)-$CH_2$-piperidinylene-$(CH_2)_8$-, #-$(CH_2)_3$-N($R_g$)-$CH_2$-piperidinylene-$CH_2$-, #-$(CH_2)_3$-N($R_g$)-$CH_2$-piperidinylene-$(CH_2)_2$-, #-$(CH_2)_3$-N($R_g$)-$CH_2$-piperidinylene-$(CH_2)_3$-, #-$(CH_2)_3$-N($R_g$)-$CH_2$-piperidinylene-$(CH_2)_8$-, #-$(CH_2)_4$-N($R_g$)-$CH_2$-piperidinylene-$CH_2$-, #-$(CH_2)_4$-N($R_g$)-$CH_2$-piperidinylene-$(CH_2)_2$-, #-$(CH_2)_4$-N($R_g$)-$CH_2$-piperidinylene-$(CH_2)_3$-, #-$(CH_2)_4$-N($R_g$)-$CH_2$-piperidinylene-$(CH_2)_8$-, #-$(CH_2)_5$-N($R_g$)-$CH_2$-piperidinylene-$(CH_2)_3$-, #-$(CH_2)_5$-N($R_g$)-$CH_2$-piperidinylene-$(CH_2)_8$-, #-$(CH_2)_6$-N($R_g$)-$CH_2$-piperidinylene-$(CH_2)_3$-, #-$(CH_2)_6$-N($R_g$)-$CH_2$-piperidinylene-$(CH_2)_8$-, #-$(CH_2)_7$-N($R_g$)-$CH_2$-piperidinylene-$(CH_2)_3$-, #-$(CH_2)_7$-N($R_g$)-$CH_2$-piperidinylene-$(CH_2)_8$-, #-$(CH_2)_8$-N($R_g$)-$CH_2$-piperidinylene-$CH_2$-, #-$(CH_2)_8$-N($R_g$)-$CH_2$-piperidinylene-$(CH_2)_2$-, #-$(CH_2)_8$-N($R_g$)-$CH_2$-piperidinylene-$(CH_2)_3$-, #-$(CH_2)_8$-N($R_g$)-$CH_2$-piperidinylene-$(CH_2)_4$-, #-$(CH_2)_8$-N($R_g$)-$CH_2$-piperidinylene-$(CH_2)_5$-, #-$(CH_2)_8$-N($R_g$)-$CH_2$-piperidinylene-$(CH_2)_6$-, #-$(CH_2)_8$-N($R_g$)-$CH_2$-piperidinylene-$(CH_2)_7$-, #-$(CH_2)_8$-N($R_g$)-$CH_2$-piperidinylene-$(CH_2)_8$-, #-$CH_2$-piperazinylene-$CH_2$-, #-$CH_2$-piperazinylene-$(CH_2)_2$-, #-$CH_2$-piperazinylene-$(CH_2)_3$-, #-$CH_2$-piperazinylene-$(CH_2)_4$-, #-$CH_2$-piperazinylene-$(CH_2)_5$-, #-$CH_2$-piperazinylene-$(CH_2)_6$-, #-$CH_2$-piperazinylene-$(CH_2)_7$-, #-$CH_2$-piperazinylene-$(CH_2)_8$-, #-$(CH_2)_2$-piperazinylene-$(CH_2)_1$-, #-$(CH_2)_2$-piperazinylene-$(CH_2)_2$-, #-$(CH_2)_2$-piperazinylene-$(CH_2)_3$-, #-$(CH_2)_2$-piperazinylene-$(CH_2)_4$-, #-$(CH_2)_2$-piperazinylene-$(CH_2)_5$-, #-$(CH_2)_2$-piperazinylene-$(CH_2)_6$-, #-$(CH_2)_2$-piperazinylene-$(CH_2)_7$-, #-$(CH_2)_2$-piperazinylene-$(CH_2)_8$-, #-$(CH_2)_3$-piperazinylene-$CH_2$-, #-$(CH_2)_3$-piperazinylene-$(CH_2)_2$-, #-$(CH_2)_3$-piperazinylene-$(CH_2)_3$-, #-$(CH_2)_3$-piperazinylene-$(CH_2)_4$-, #-$(CH_2)_3$-piperazinylene-$(CH_2)_5$-, #-$(CH_2)_3$-piperazinylene-$(CH_2)_6$-, #-$(CH_2)_3$-piperazinylene-$(CH_2)_7$-, #-$(CH_2)_3$-piperazinylene-$(CH_2)_8$-, #-$(CH_2)_4$-piperazinylene-$CH_2$-, #-$(CH_2)_4$-piperazinylene-$(CH_2)_2$-, #-$(CH_2)_4$-piperazinylene-$(CH_2)_3$-, #-$(CH_2)_4$-piperazinylene-$(CH_2)_4$-, #-$(CH_2)_4$-piperazinylene-$(CH_2)_5$-, #-$(CH_2)_4$-piperazinylene-$(CH_2)_6$-, #-$(CH_2)_4$-piperazinylene-$(CH_2)_7$-, #-$(CH_2)_4$-piperazinylene-$(CH_2)_8$-, #-$(CH_2)_5$-piperazinylene-$(CH_2)_1$-, #-$(CH_2)_5$-piperazinylene-$(CH_2)_2$-, #-$(CH_2)_5$-piperazinylene-$(CH_2)_3$-, #-$(CH_2)_5$-piperazinylene-$(CH_2)_4$-, #-$(CH_2)_5$-piperazinylene-$(CH_2)_5$-, #-$(CH_2)_5$-piperazinylene-$(CH_2)_6$-, #-$(CH_2)_5$-piperazinylene-$(CH_2)_7$-, #-$(CH_2)_5$-piperazinylene-$(CH_2)_8$-, #-$(CH_2)_6$-piperazinylene-$(CH_2)_1$-, #-$(CH_2)_6$-piperazinylene-$(CH_2)_2$-, #-$(CH_2)_6$-piperazinylene-$(CH_2)_3$-, #-$(CH_2)_6$-piperazinylene-$(CH_2)_4$-, #-$(CH_2)_6$-piperazinylene-$(CH_2)_5$-, #-$(CH_2)_6$-piperazinylene-$(CH_2)_6$-, #-$(CH_2)_6$-piperazinylene-$(CH_2)_7$-, #-$(CH_2)_6$-piperazinylene-$(CH_2)_8$-, #-$(CH_2)_7$-piperazinylene-$(CH_2)_1$-, #-$(CH_2)_7$-piperazinylene-$(CH_2)_2$-, #-$(CH_2)_7$-piperazinylene-$(CH_2)_3$-, #-$(CH_2)_7$-piperazinylene-$(CH_2)_4$-, #-$(CH_2)_7$-piperazi-

nylene-$(CH_2)_8$-, #-$(CH_2)_8$-piperazinylene-$CH_2$-, #-$(CH_2)_8$-piperazinylene-$(CH_2)_2$-, #-$(CH_2)_8$-piperazinylene-$(CH_2)_3$-, #-$(CH_2)_8$-piperazinylene-$(CH_2)_4$-, #-$(CH_2)_8$-piperazinylene-$(CH_2)_5$-, #-$(CH_2)_8$-piperazinylene-$(CH_2)_6$-, #-$(CH_2)_8$-piperazinylene-$(CH_2)_7$-, or #-$(CH_2)_8$-piperazinylene-$(CH_2)_8$-;

wherein the piperidinylene and piperazinylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$ cycloalkyl (e.g., optionally deuterated cyclopropyl, cyclobutyl, cyclopentyl, or cyclohexyl), hydroxy, amino, mercapto, halogen (e.g., fluorine, chlorine, bromine, or iodine), optionally deuterated $C_{1-6}$ alkoxy (e.g., optionally deuterated $C_{1-4}$ alkoxy, such as methoxy, $CD_3$-O-, ethoxy, propoxy, isopropoxy, butoxy, sec-butoxy or tert-butoxy), optionally deuterated $C_{1-6}$ alkyl-NH-, halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene, optionally deuterated $C_{1-6}$ alkyl-NHC(O)-, optionally deuterated $C_{1-6}$ alkyl-C(O)NH-, cyano, or any combination thereof;

each $R_g$ independently represent H or $C_{1-6}$ alkyl;

symbol # indicates the point of attachment to $U_2$; and

n1, n2, n3, n4, m1, m2, m3 each independently represent an integer of 1, 2, 3, 4, 5, 6, 7, 8, 9, 10, 11, 12, 13, 14, or 15.

In some embodiments, -$W^1$-LIN-$U_2$-$R_c$-$U_1$- represents the following groups:

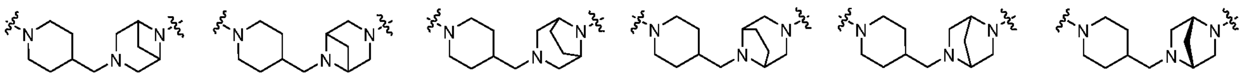

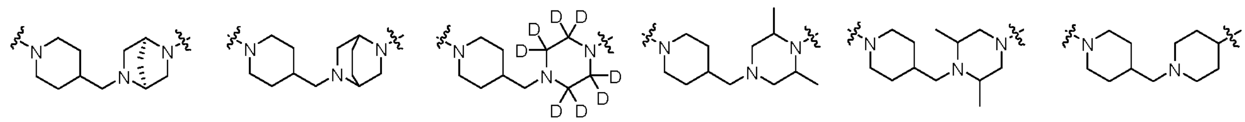

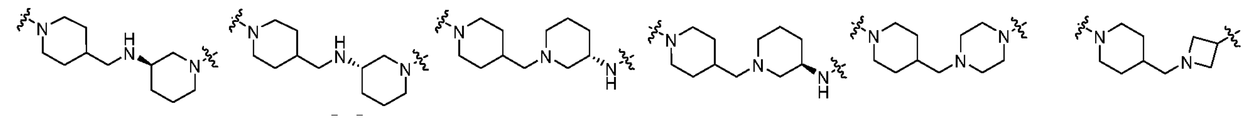

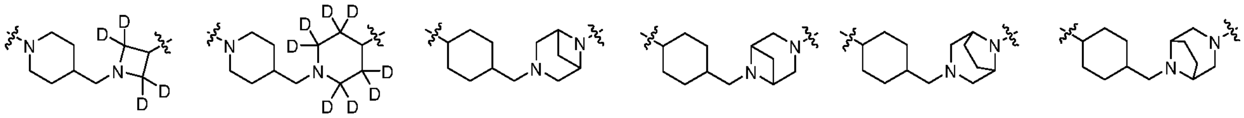

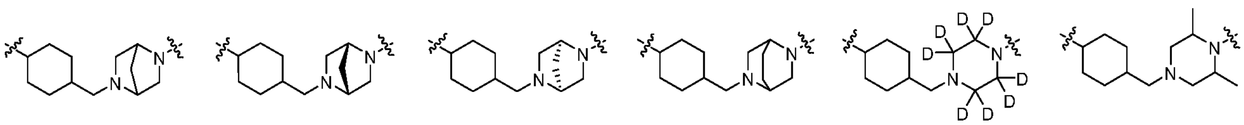

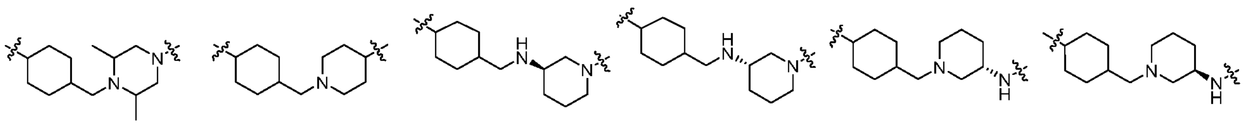

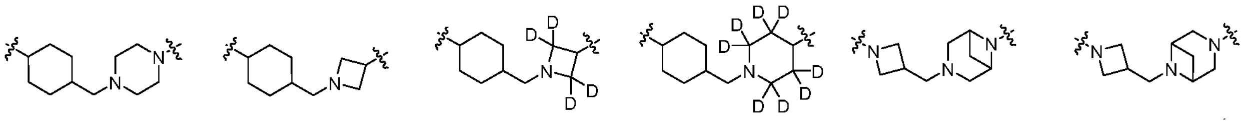

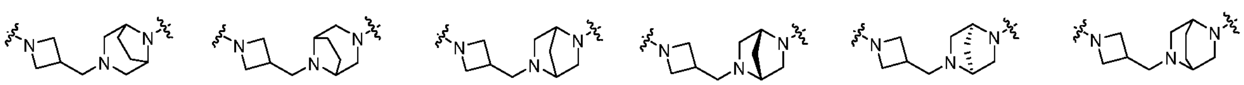

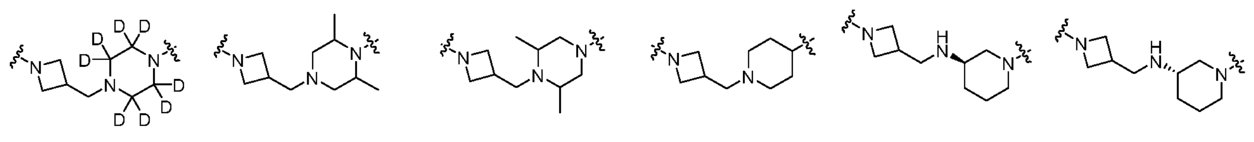

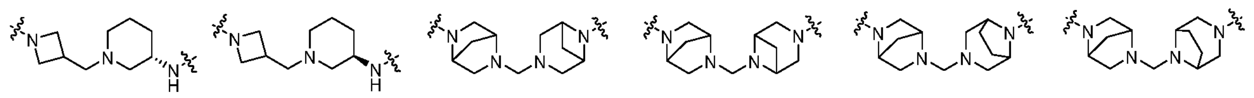

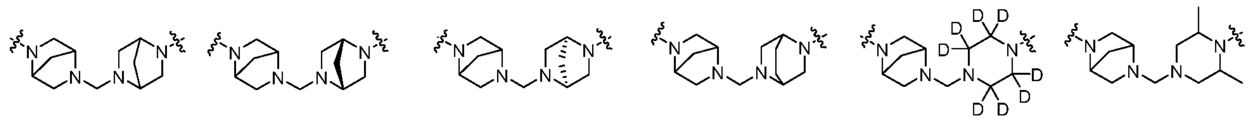

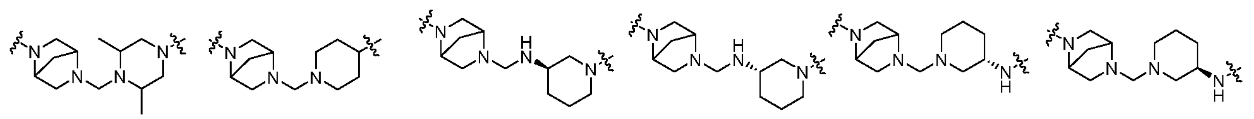

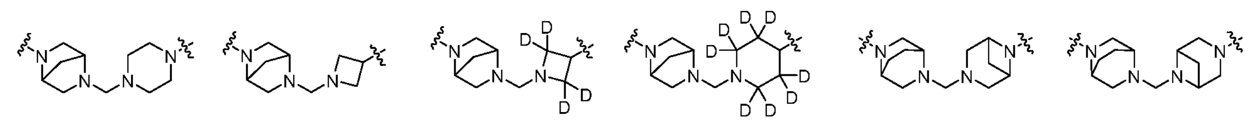

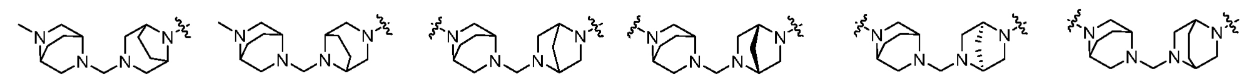

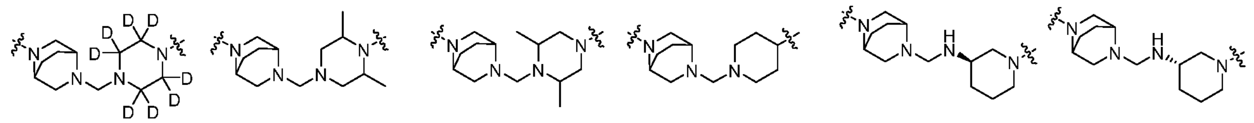

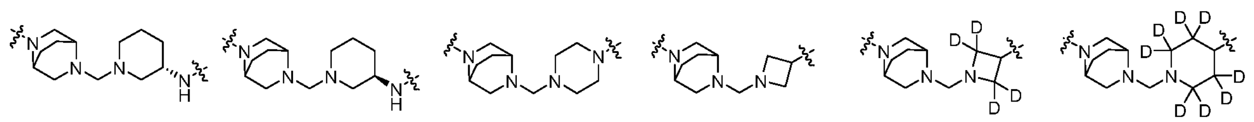

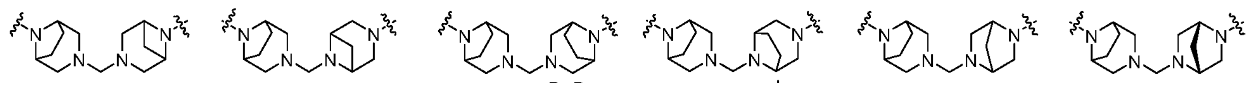

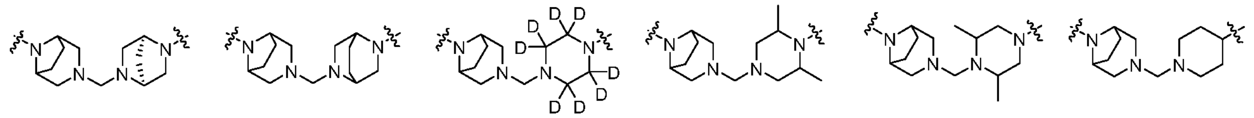

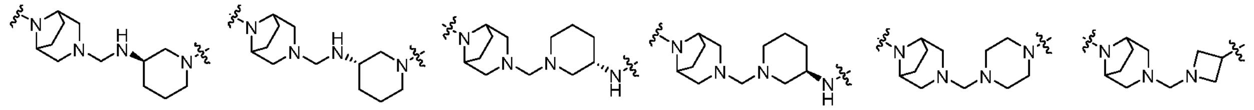

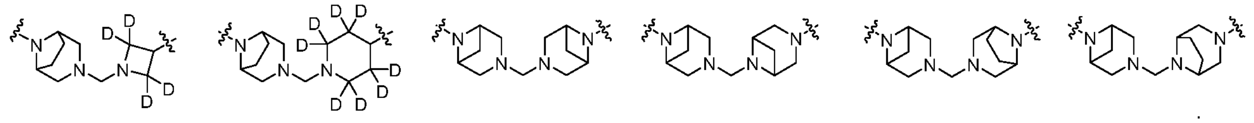

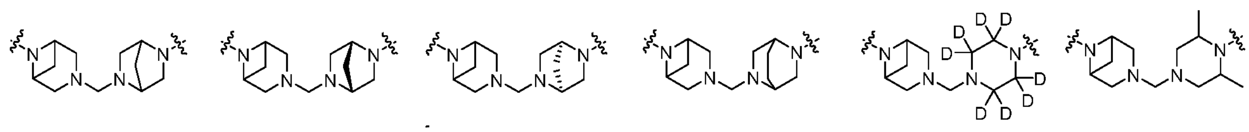

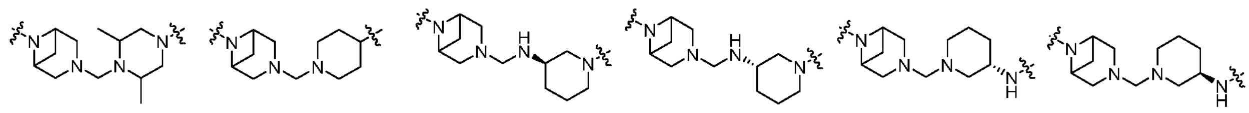

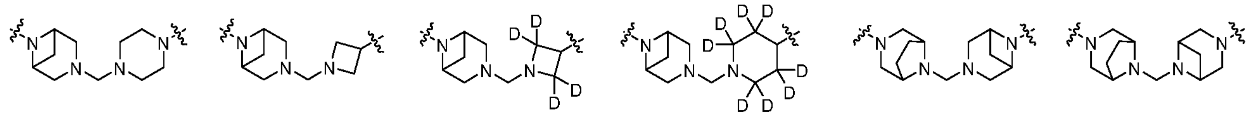

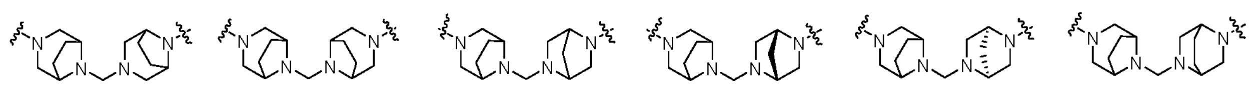

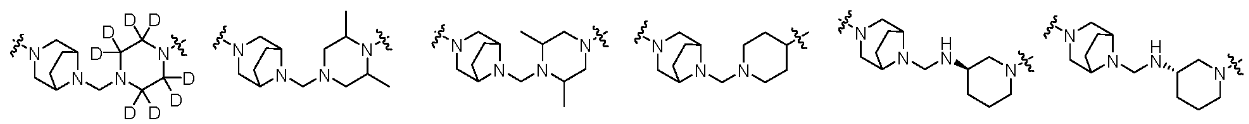

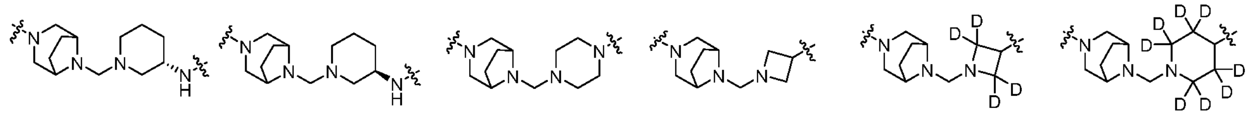

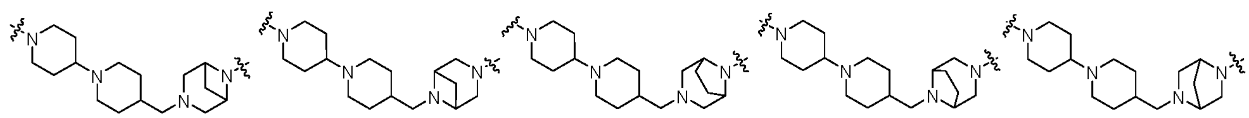

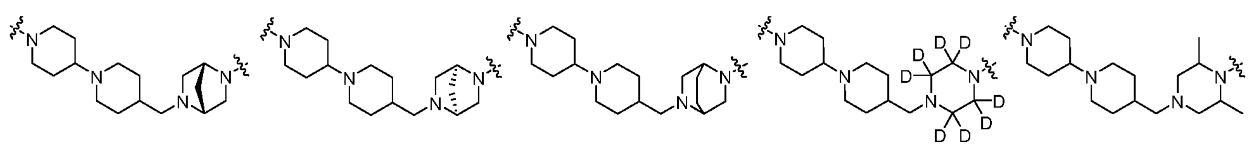

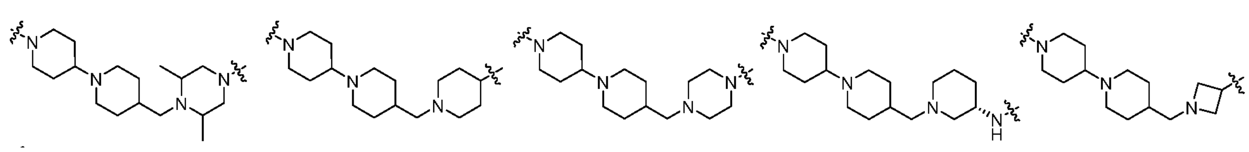

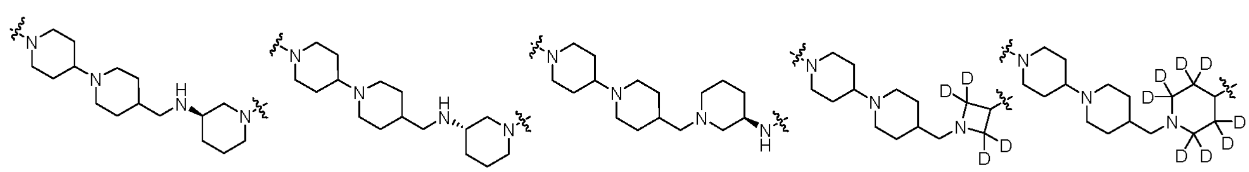

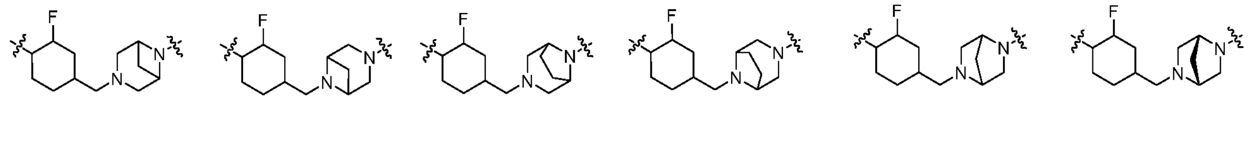

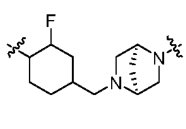 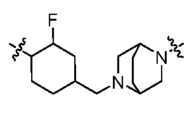 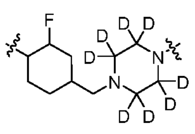 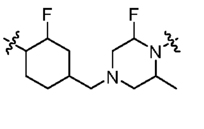 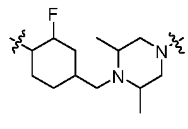 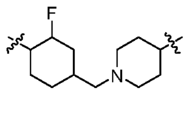

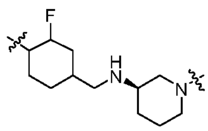 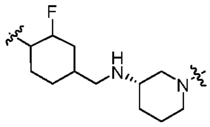 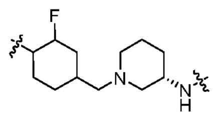 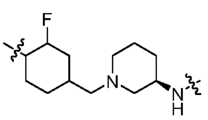 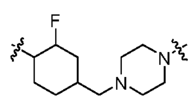 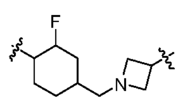

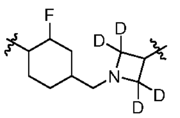 or 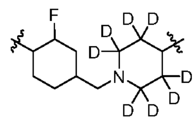 ;

wherein the groups are optionally substituted with one or more (e.g., 1-10, 1-8, 1-6, such as 1, 2, 3, 4, 5 or 6) substituents selected from the group consisting of deuterium, halogen (e.g., fluorine, chlorine, bromine, or iodine), cyano, amino, $C_{1-6}$ alkyl (e.g., $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-4}$ alkyl, such as perdeuterated methyl ($CD_3$), perdeuterated ethyl, perdeuterated propyl, perdeuterated isopropyl, perdeuterated butyl, perdeuterated sec-butyl or perdeuterated tert-butyl), $C_{1-6}$ alkoxy (e.g., $C_{1-4}$ alkoxy, such as methoxy, ethoxy, propoxy, isopropoxy, butoxy, sec-butoxy or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), deuterated $C_{1-6}$ alkoxy (e.g., perdeuterated $C_{1-4}$ alkoxy, such as $D_3C$-O-, $CD_3CD_2$-O-, or $CD_3CD_2CD_2$-O-), or any combination thereof.

In some embodiments, the compound of Formula (III) is also of Formula (III-1), Formula (III-2), Formula (III-3), Formula (III-4), Formula (III-5), Formula (III-6), Formula (III-7), Formula (III-8), Formula (III-9), Formula (III-10), Formula (III-11), Formula (III-12), Formula (111-13), Formula (III-14), Formula (III-15), Formula (III-16), Formula (III-17), Formula (III-18), Formula (III-19), Formula (III-20), Formula (III-21), Formula (III-22), Formula (III-23) or Formula (III-24):

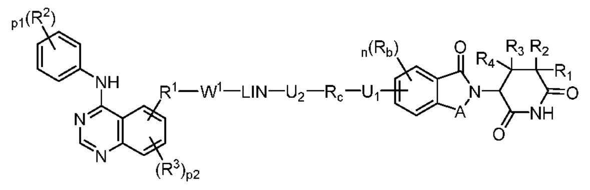

Formula (III-1)

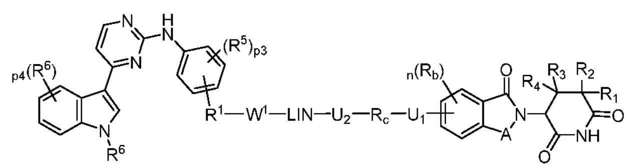

Formula (III-2)

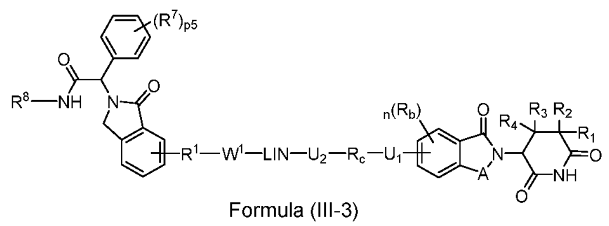

Formula (III-3)

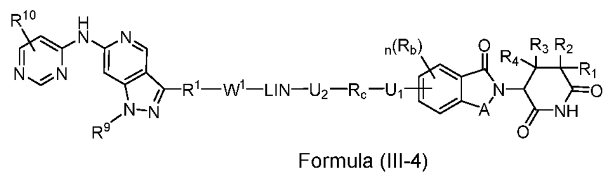

Formula (III-4)

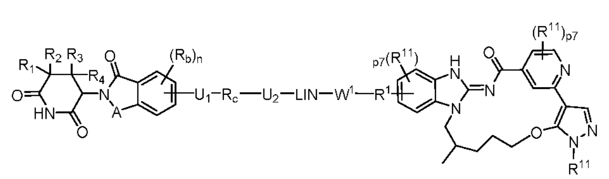

Formula (III-5)

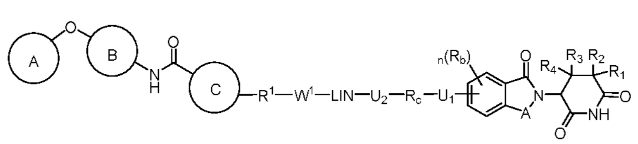

Formula (III-6)

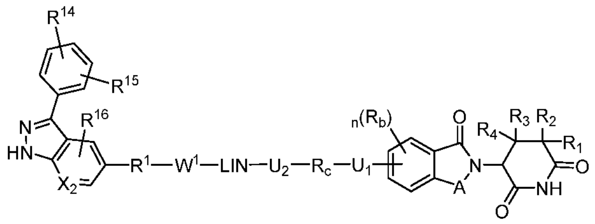

Formula (III-7)

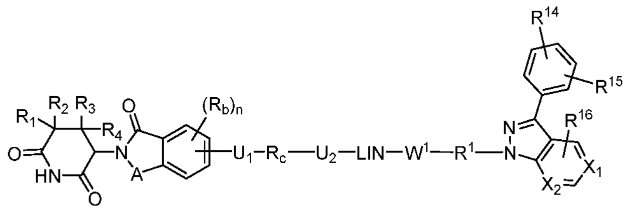

Formula (III-8)

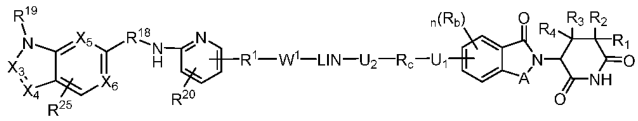

Formula (III-9)

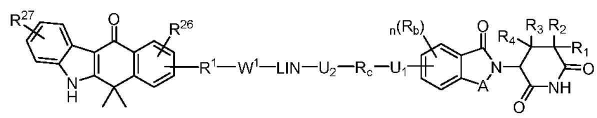

Formula (III-10)

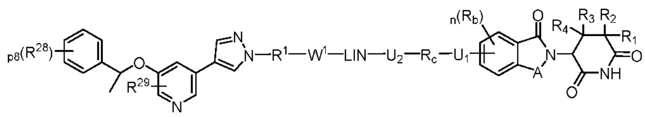

Formula (III-11)

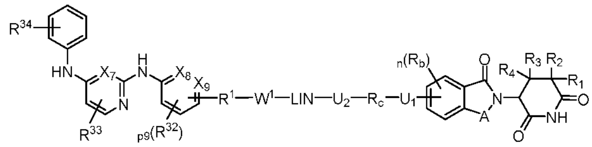

Formula (III-12)

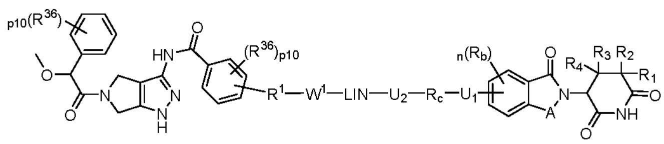

Formula (III-13)

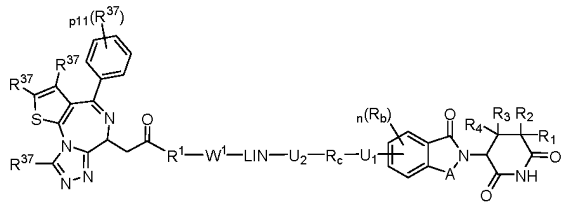

Formula (III-14)

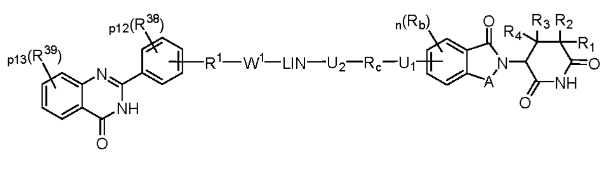

Formula (III-15)

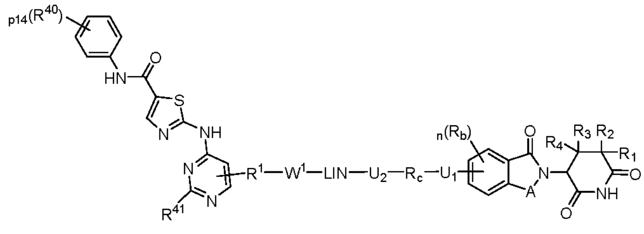

Formula (III-16)

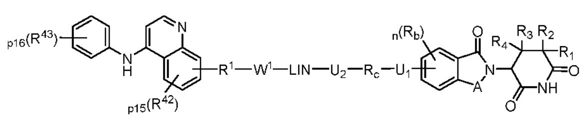

Formula (III-17)

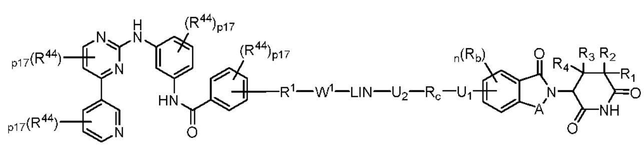

Formula (III-18)

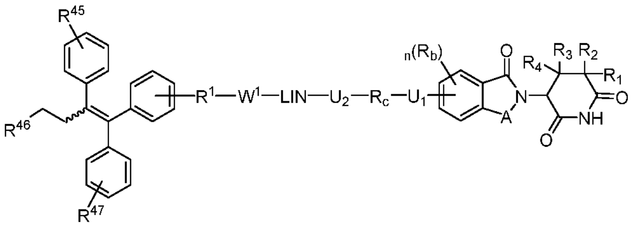

Formula (III-19)

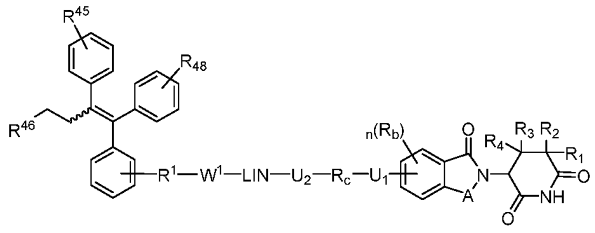

Formula (III-20)

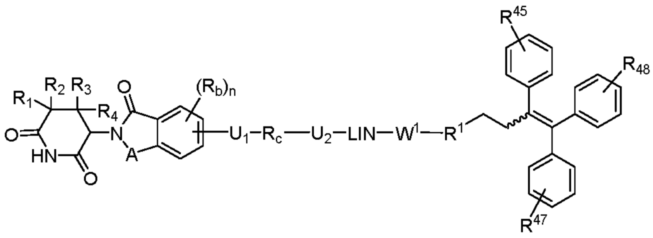

Formula (III-21)

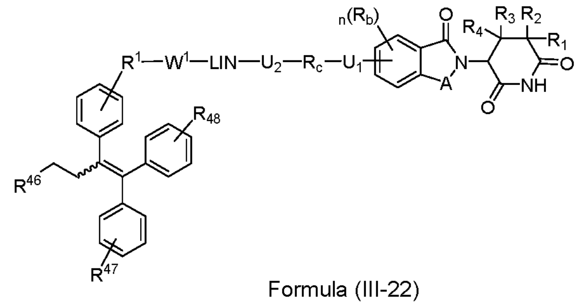

Formula (III-22)

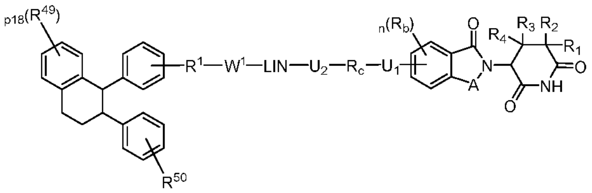

Formula (III-23)

and

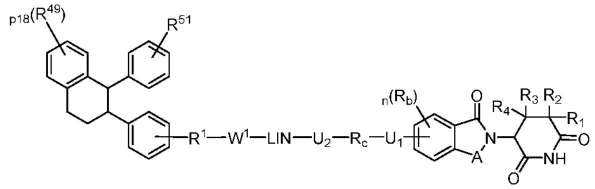

Formula (III-24)

wherein A, $R_1$, $R_2$, $R_3$, $R_4$, $(R_b)_n$, $U_1$, $U_2$, $R_c$, LIN, $W^1$, $R^1$, $(R^2)_{p1}$, $(R^3)_{p2}$, $(R^5)_{p3}$, $(R^6)_{p4}$, $(R^7)_{p5}$, $R^8$, $R^9$, $R^{10}$, $(R^{11})_{p7}$, ring A, ring B, ring C, $X_1$, $X_2$, $R^{14}$, $R^{15}$, $R^{16}$, $X_3$, $X_4$, $X_5$, $X_6$, $R^{18}$, $R^{19}$, $R^{20}$, $R^{25}$, $R^{26}$, $R^{27}$, $(R^{28})_{p8}$, $R^{29}$ $X_7$, $X_8$, $X_9$, $(R^{32})_{p9}$, $R^{33}$, $R^{34}$, $(R^{36})_{p10}$, $(R^{37})_{p11}$, $(R^{38})_{p12}$ $(R^{39})_{p13}$, $(R^{40})_{p14}$, $R^{41}$, $(R^{42})_{p15}$, $(R^{43})_{p16}$, $(R^{44})_{b17}$, $R^{45}$, $R^{46}$, $R^{47}$ $R^{48}$ $(R^{49})_{p18}$, $R^{50}$, and $R^{51}$ are as defined in the compound of Formula (III) above and each sub-embodiments thereof.

In some embodiments, when PBM of the compound of Formula (III) of the present disclosure represents the structrues of Formula (PBM-6-1A-1) or Formula (PBM-6-1), and ULM represents the structrue of Formula (ULM):

PBM-$R^1$-$W^1$-LIN-ULM

Formula (III)

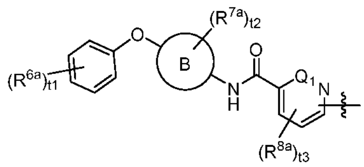

Formula (PBM-6-1A-1)

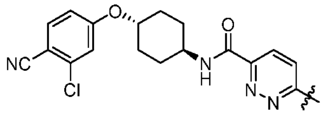

Formula (PBM-6-1)

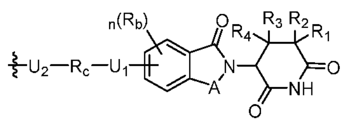

Formula (ULM)

$W^1$ represents optionally substituted piperidinylene, $R_c$ represents optionally substituted piperidinylene, $U_1$ represents a bond, A, $R_1$, $R_2$, $R_3$, $R_4$, $(R_b)_n$, $U_2$, LIN, $R^1$, $(R^{6a})_{t1}$, ring B, $(R^{7a})_{t2}$, $(R^{8a})_{t3}$, $Q_1$ are as defined in the compound of Formula (III) above and each sub-embodiments thereof, wherein substituents of the piperidinylene of $W^1$ and $R_c$ are each independently as defined herein.

In some embodiments, the compounds of Formula (III) of the present invention and their salts (including pharmaceutically acceptable salts, such as hydrochloride, etc.), prodrugs, solvates, isotopically enriched analogs, polymorphs, stereoisomers (including enantiomers and diastereomers), or mixture of stereoisomers thereof in Table 4 below are provided.

Table 4. The compounds of the present disclosure

| Compound No. | Structure of compound | Compound's name | Chinese translation of Compound's name |
|---|---|---|---|
| GT-03339 | 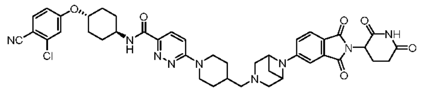 | N-((1r,4r)-4-(3-chloro-4-cyanophenoxy)cyclohexyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazine-3-carboxamide | N-((1r,4r)-4-(3-chloro-4-cyanophenoxy)cyclohexyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazine-3-carboxamide |
| GT-03490 | 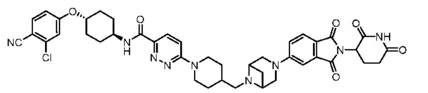 | N-((1r,4r)-4-(3-chloro-4-cyanophenoxy)cyclohexyl)-6-(4-((3-(2-(2,6-dioxopiperidin-3-yl)-1,3-dioxoisoindolin-5-yl)-3,6- | N-((1r,4r)-4-(3-chloro-4-cyanophenoxy)cyclohexyl)-6-(4-((3-(2-(2,6-dioxopiperidin-3-yl)-1,3-dioxoisoindolin-5-yl)-3,6- |

| | | | |
|---|---|---|---|
| | | diazabicyclo[3.1.1]heptan-6-yl)methyl)piperidin-1-yl)pyridazine-3-carboxamide | diazabicyclo[3.1.1]heptan-6-yl)methyl)piperidin-1-yl)pyridazine-3-carboxamide |
| GT-03488 | 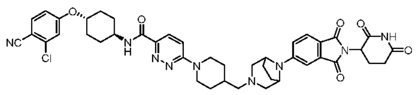 | N-((1r,4r)-4-(3-chloro-4-cyanophenoxy)cyclohexyl)-6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridazine-3-carboxamide | N-((1r,4r)-4-(3-chloro-4-cyanophenoxy)cyclohexyl)-6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridazine-3-carboxamide |
| GT-03489 | 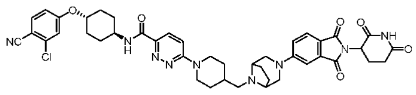 | N-((1r,4r)-4-(3-chloro-4-cyanophenoxy)cyclohexyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazine-3-carboxamide | N-((1r,4r)-4-(3-chloro-4-cyanophenoxy)cyclohexyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazine-3-carboxamide |
| | 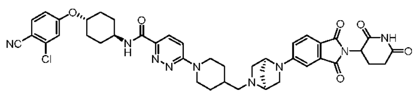 | N-((1r,4r)-4-(3-chloro-4-cyanophenoxy)cyclohexyl)-6-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridazine-3-carboxamide | N-((1r,4r)-4-(3-chloro-4-cyanophenoxy)cyclohexyl)-6-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridazine-3-carboxamide |
| | 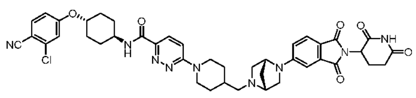 | N-((1r,4r)-4-(3-chloro-4-cyanophenoxy)cyclohexyl)-6-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridazine-3-carboxamide | N-((1r,4r)-4-(3-chloro-4-cyanophenoxy)cyclohexyl)-6-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridazine-3-carboxamide |
| GT-03885 | 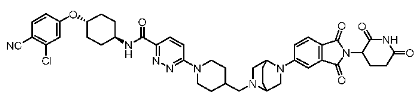 | N-((1r,4r)-4-(3-chloro-4-cyanophenoxy)cyclohexyl)-6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)pyridazine-3-carboxamide | N-((1r,4r)-4-(3-chloro-4-cyanophenoxy)cyclohexyl)-6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)pyridazine-3-carboxamide |
| | 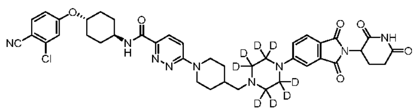 | N-((1r,4r)-4-(3-chloro-4-cyanophenoxy)cyclohexyl)-6-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)pyridazine-3- |

| | | | |
|---|---|---|---|
| | | | carboxamide |
| | 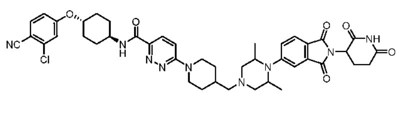 | N-((1r,4r)-4-(3-chloro-4-cyanophenoxy)cyclohexyl)-6-(4-((4-(2-(2,6-dioxopiperidin-3-yl)-1,3-dioxoisoindolin-5-yl)-3,5-dimethylpiperazin-1-yl)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-1,3-dioxoisoindolin-5-yl)-3,5-dimethylpiperazin-1-yl)methyl)piperidin-1-yl)pyridazine-3-carboxamide |
| | 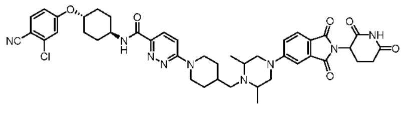 | N-((1r,4r)-4-(3-chloro-4-cyanophenoxy)cyclohexyl)-6-(4-((4-(2-(2,6-dioxopiperidin-3-yl)-1,3-dioxoisoindolin-5-yl)-2,6-dimethylpiperazin-1-yl)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-1,3-dioxoisoindolin-5-yl)-2,6-dimethylpiperazin-1-yl)methyl)piperidin-1-yl)pyridazine-3-carboxamide |
| | 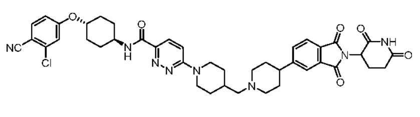 | N-((1r,4r)-4-(3-chloro-4-cyanophenoxy)cyclohexyl)-6-(4-((4-(2-(2,6-dioxopiperidin-3-yl)-1,3-dioxoisoindolin-5-yl)piperidin-1-yl)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-1,3-dioxoisoindolin-5-yl)piperidin-1-yl)methyl)piperidin-1-yl)pyridazine-3-carboxamide |
| GT-03486 | 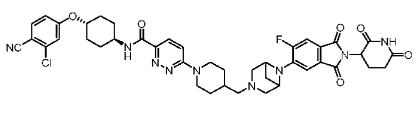 | N-((1r,4r)-4-(3-chloro-4-cyanophenoxy)cyclohexyl)-6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridazine-3-carboxamide | N-((1r,4r)-4-(3-chloro-4-cyanophenoxy)cyclohexyl)-6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridazine-3-carboxamide |
| GT-03487 | 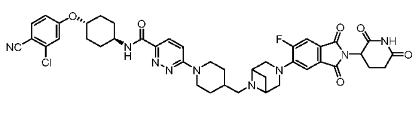 | N-((1r,4r)-4-(3-chloro-4-cyanophenoxy)cyclohexyl)-6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridazine-3-carboxamide | N-((1r,4r)-4-(3-chloro-4-cyanophenoxy)cyclohexyl)-6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridazine-3-carboxamide |
| GT-02765 | 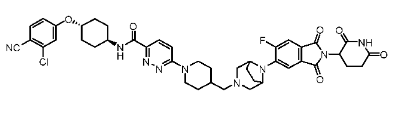 | N-((1r,4r)-4-(3-chloro-4-cyanophenoxy)cyclohexyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazine-3-carboxamide | N-((1r,4r)-4-(3-chloro-4-cyanophenoxy)cyclohexyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazine-3-carboxamide |

| | | | |
|---|---|---|---|
| GT-02752 | 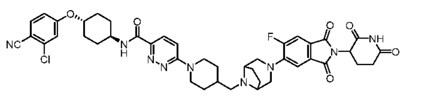 | N-((1r,4r)-4-(3-chloro-4-cyanophenoxy)cyclohexyl)-6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridazine-3-carboxamide | N-((1r,4r)-4-(3-chloro-4-cyanophenoxy)cyclohexyl)-6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridazine-3-carboxamide |
| GT-03773 | 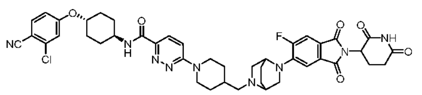 | N-((1r,4r)-4-(3-chloro-4-cyanophenoxy)cyclohexyl)-6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)pyridazine-3-carboxamide | N-((1r,4r)-4-(3-chloro-4-cyanophenoxy)cyclohexyl)-6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)pyridazine-3-carboxamide |
| | 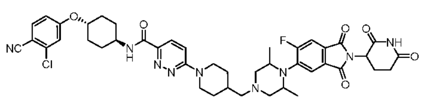 | N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)pyridazine-3-carboxamide |
| | 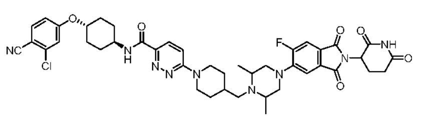 | N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)pyridazine-3-carboxamide |
| | 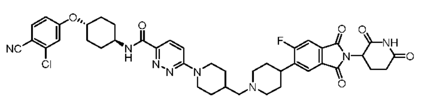 | N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)piperidin-1-yl)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)piperidin-1-yl)methyl)piperidin-1-yl)pyridazine-3-carboxamide |
| GT-03016 | 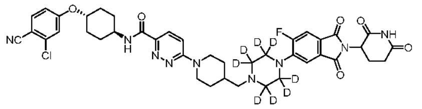 | N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)piperazin-1-yl-2,2,3,3,5,5,6,6-*d*$_8$)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)piperazin-1-yl-2,2,3,3,5,5,6,6-*d*$_8$)methyl)piperidin-1-yl)pyridazine-3- |

| | | | |
|---|---|---|---|
| | | | carboxamide |
| | 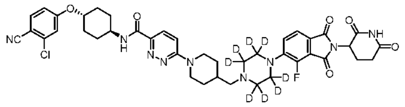 | N-((1r,4r)-4-(3-chloro-4-cyanophenoxy)cyclohexyl)-6-(4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)pyridazine-3-carboxamide |
| | 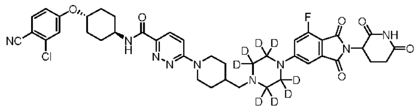 | N-((1r,4r)-4-(3-chloro-4-cyanophenoxy)cyclohexyl)-6-(4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1-yl)pyridazine-3-carboxamide |
| | 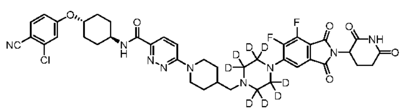 | N-((1r,4r)-4-(3-chloro-4-cyanophenoxy)cyclohexyl)-6-(4-((4-(2-(2,6-dioxopiperidin-3-yl)-6,7-difluoro-1,3-dioxoisoindolin-5-yl)piperazin-1-yl-2,2,3,3,5,5,6,6-*d*$_8$)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-6,7-difluoro-1,3-dioxoisoindolin-5-yl)piperazin-1-yl-2,2,3,3,5,5,6,6-*d*$_8$)methyl)piperidin-1-yl)pyridazine-3-carboxamide |
| | 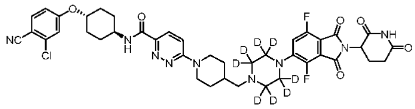 | N-((1r,4r)-4-(3-chloro-4-cyanophenoxy)cyclohexyl)-6-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)pyridazine-3-carboxamide |
| | 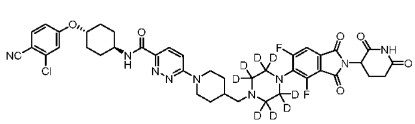 | N-((1r,4r)-4-(3-chloro-4-cyanophenoxy)cyclohexyl)-6-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)pyridazine-3-carboxamide | N-((1r,4r)-4-(3-chloro-4-cyanophenoxy)cyclohexyl)-6-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)pyridazine-3-carboxamide |
| | 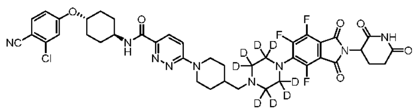 | N-((1r,4r)-4-(3-chloro-4-cyanophenoxy)cyclohexyl)-6-(4-((4-(2-(2,6-dioxopiperidin-3-yl)-4,6,7- | N-((1r,4r)-4-(3-chloro-4-cyanophenoxy)cyclohexyl)-6-(4-((4-(2-(2,6-dioxopiperidin-3-yl)-4,6,7- |

| | | | |
|---|---|---|---|
| | | trifluoro-1,3-dioxoisoindolin-5-yl)piperazin-1-yl-2,2,3,3,5,5,6,6-*d*8)methyl)piperidin-1-yl)pyridazine-3-carboxamide | trifluoro-1,3-dioxoisoindolin-5-yl)piperazin-1-yl-2,2,3,3,5,5,6,6-*d*8)methyl)piperidin-1-yl)pyridazine-3-carboxamide |
| | 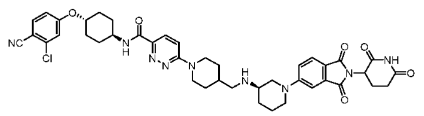 | N-((1r,4r)-4-(3-chloro-4-cyanophenoxy)cyclohexyl)-6-(4-((((3R)-1-(2-(2,6-dioxopiperidin-3-yl)-1,3-dioxoisoindolin-5-yl)piperidin-3-yl)amino)methyl)piperidin-1-yl)pyridazine-3-carboxamide | N-((1r,4r)-4-(3-chloro-4-cyanophenoxy)cyclohexyl)-6-(4-((((3R)-1-(2-(2,6-dioxopiperidin-3-yl)-1,3-dioxoisoindolin-5-yl)piperidin-3-yl)amino)methyl)piperidin-1-yl)pyridazine-3-carboxamide |
| | 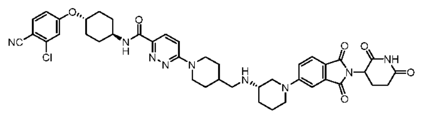 | N-((1r,4r)-4-(3-chloro-4-cyanophenoxy)cyclohexyl)-6-(4-((((3S)-1-(2-(2,6-dioxopiperidin-3-yl)-1,3-dioxoisoindolin-5-yl)piperidin-3-yl)amino)methyl)piperidin-1-yl)pyridazine-3-carboxamide | N-((1r,4r)-4-(3-chloro-4-cyanophenoxy)cyclohexyl)-6-(4-((((3S)-1-(2-(2,6-dioxopiperidin-3-yl)-1,3-dioxoisoindolin-5-yl)piperidin-3-yl)amino)methyl)piperidin-1-yl)pyridazine-3-carboxamide |
| | 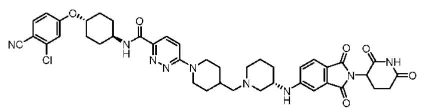 | N-((1r,4r)-4-(3-chloro-4-cyanophenoxy)cyclohexyl)-6-(4-(((3S)-3-((2-(2,6-dioxopiperidin-3-yl)-1,3-dioxoisoindolin-5-yl)amino)piperidin-1-yl)methyl)piperidin-1-yl)pyridazine-3-carboxamide | N-((1r,4r)-4-(3-chloro-4-cyanophenoxy)cyclohexyl)-6-(4-(((3S)-3-((2-(2,6-dioxopiperidin-3-yl)-1,3-dioxoisoindolin-5-yl)amino)piperidin-1-yl)methyl)piperidin-1-yl)pyridazine-3-carboxamide |
| | 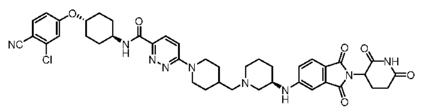 | N-((1r,4r)-4-(3-chloro-4-cyanophenoxy)cyclohexyl)-6-(4-(((3R)-3-((2-(2,6-dioxopiperidin-3-yl)-1,3-dioxoisoindolin-5-yl)amino)piperidin-1-yl)methyl)piperidin-1-yl)pyridazine-3-carboxamide | N-((1r,4r)-4-(3-chloro-4-cyanophenoxy)cyclohexyl)-6-(4-(((3R)-3-((2-(2,6-dioxopiperidin-3-yl)-1,3-dioxoisoindolin-5-yl)amino)piperidin-1-yl)methyl)piperidin-1-yl)pyridazine-3-carboxamide |
| GT-03449 | 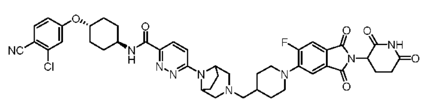 | *N*-((1r,4r)-4-(3-chloro-4-cyanophenoxy)cyclohexyl)-6-(3-((1-(2-(2,6-dioxopiperidin-3-yl)-6-fluoro-1,3-dioxoisoindolin-5-yl)piperidin-4-yl)methyl)-3,8-diazabicyclo[3.2.1]octan-8-yl)pyridazine-3-carboxamide | *N*-((1r,4r)-4-(3-chloro-4-cyanophenoxy)cyclohexyl)-6-(3-((1-(2-(2,6-dioxopiperidin-3-yl)-6-fluoro-1,3-dioxoisoindolin-5-yl)piperidin-4-yl)methyl)-3,8-diazabicyclo[3.2.1]octan-8-yl)pyridazine-3-carboxamide |
| GT-03450 | 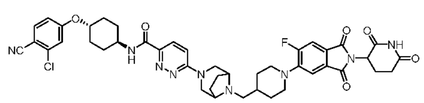 | *N*-((1r,4r)-4-(3-chloro-4-cyanophenoxy)cyclohexyl)-6-(8-((1-(2-(2,6-dioxopiperidin-3-yl)-6-fluoro-1,3-dioxoisoindolin- | *N*-((1r,4r)-4-(3-chloro-4-cyanophenoxy)cyclohexyl)-6-(8-((1-(2-(2,6-dioxopiperidin-3-yl)-6-fluoro-1,3-dioxoisoindolin- |

| | | | |
|---|---|---|---|
| | | 5-yl)piperidin-4-yl)methyl)-3,8-diazabicyclo[3.2.1]octan-3-yl)pyridazine-3-carboxamide | 5-yl)piperidin-4-yl)methyl)-3,8-diazabicyclo[3.2.1]octan-3-yl)pyridazine-3-carboxamide |
| GT-03451 | 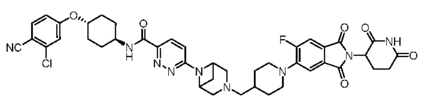 | *N*-((1r,4r)-4-(3-chloro-4-cyanophenoxy)cyclohexyl)-6-(3-((1-(2-(2,6-dioxopiperidin-3-yl)-6-fluoro-1,3-dioxoisoindolin-5-yl)piperidin-4-yl)methyl)-3,6-diazabicyclo[3.1.1]heptan-6-yl)pyridazine-3-carboxamide | *N*-((1r,4r)-4-(3-chloro-4-cyanophenoxy)cyclohexyl)-6-(3-((1-(2-(2,6-dioxopiperidin-3-yl)-6-fluoro-1,3-dioxoisoindolin-5-yl)piperidin-4-yl)methyl)-3,6-diazabicyclo[3.1.1]heptan-6-yl)pyridazine-3-carboxamide |
| GT-03470 | 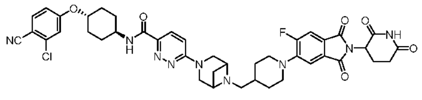 | *N*-((1r,4r)-4-(3-chloro-4-cyanophenoxy)cyclohexyl)-6-(6-((1-(2-(2,6-dioxopiperidin-3-yl)-6-fluoro-1,3-dioxoisoindolin-5-yl)piperidin-4-yl)methyl)-3,6-diazabicyclo[3.1.1]heptan-3-yl)pyridazine-3-carboxamide | *N*-((1r,4r)-4-(3-chloro-4-cyanophenoxy)cyclohexyl)-6-(6-((1-(2-(2,6-dioxopiperidin-3-yl)-6-fluoro-1,3-dioxoisoindolin-5-yl)piperidin-4-yl)methyl)-3,6-diazabicyclo[3.1.1]heptan-3-yl)pyridazine-3-carboxamide |
| GT-03427 | 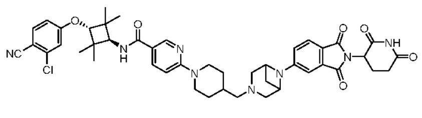 | N-((1r,3r)-3-(3-chloro-4-cyanophenoxy)-2,2,4,4-tetramethylcyclobutyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)nicotinamide | N-((1r,3r)-3-(3-chloro-4-cyanophenoxy)-2,2,4,4-tetramethylcyclobutyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)nicotinamide |
| GT-03471 | 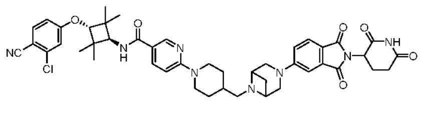 | N-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)nicotinamide | N-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)nicotinamide |
| GT-03425 | 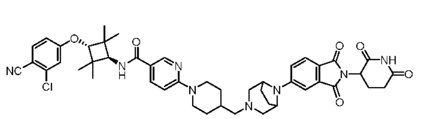 | N-((1r,3r)-3-(3-chloro-4-cyanophenoxy)-2,2,4,4-tetramethylcyclobutyl)-6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)nicotinamide | N-((1r,3r)-3-(3-chloro-4-cyanophenoxy)-2,2,4,4-tetramethylcyclobutyl)-6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)nicotinamide |
| GT-03426 | 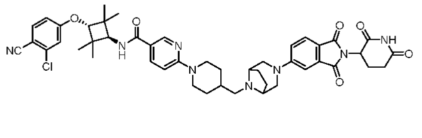 | N-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,8- | N-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,8- |

| | | | |
|---|---|---|---|
| | | diazabicyclo[3.2.1]octan-8-yl)methyl)piperidin-1-yl)nicotinamide | diazabicyclo[3.2.1]octan-8-yl)methyl)piperidin-1-yl)nicotinamide |
| | 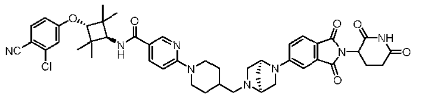 | N-((1r,3S)-3-(3-chloro-4-cyanophenoxy)-2,2,4,4-tetramethylcyclobutyl)-6-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)nicotinamide | N-((1r,3S)-3-(3-chloro-4-cyanophenoxy)-2,2,4,4-tetramethylcyclobutyl)-6-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)nicotinamide |
| | 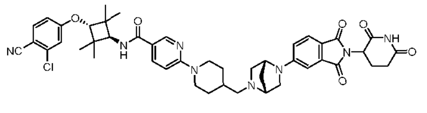 | N-((1r,3R)-3-(3-chloro-4-cyanophenoxy)-2,2,4,4-tetramethylcyclobutyl)-6-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)nicotinamide | N-((1r,3R)-3-(3-chloro-4-cyanophenoxy)-2,2,4,4-tetramethylcyclobutyl)-6-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)nicotinamide |
| GT-03481 | 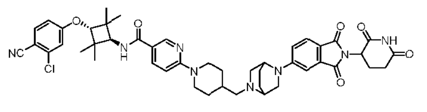 | N-((1r,3r)-3-(3-chloro-4-cyanophenoxy)-2,2,4,4-tetramethylcyclobutyl)-6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)nicotinamide | N-((1r,3r)-3-(3-chloro-4-cyanophenoxy)-2,2,4,4-tetramethylcyclobutyl)-6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)nicotinamide |
| | 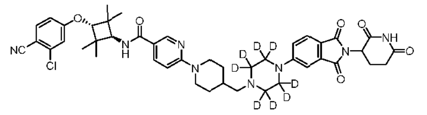 | N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)nicotinamide |
| | 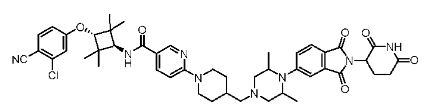 | N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)-3,5-dimethylpiperazin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)-3,5-dimethylpiperazin-1-yl)methyl)piperidin-1-yl)nicotinamide |
| | 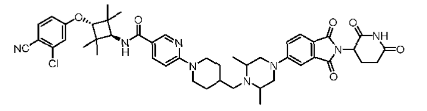 | N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)-2,6-dimethylpiperazin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)-2,6-dimethylpiperazin-1-yl)methyl)piperidin-1-yl)nicotinamide |

| | | | |
|---|---|---|---|
| | 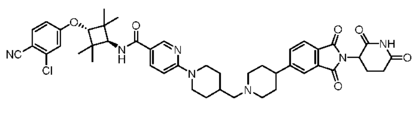 | N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)piperidin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)piperidin-1-yl)methyl)piperidin-1-yl)nicotinamide |
| GT-03403 | 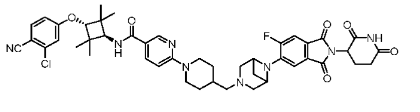 | N-((1r,3r)-3-(3-chloro-4-cyanophenoxy)-2,2,4,4-tetramethylcyclobutyl)-6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)nicotinamide | N-((1r,3r)-3-(3-chloro-4-cyanophenoxy)-2,2,4,4-tetramethylcyclobutyl)-6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)nicotinamide |
| GT-03404 | 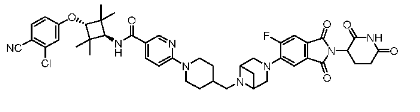 | N-((1r,3r)-3-(3-chloro-4-cyanophenoxy)-2,2,4,4-tetramethylcyclobutyl)-6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)nicotinamide | N-((1r,3r)-3-(3-chloro-4-cyanophenoxy)-2,2,4,4-tetramethylcyclobutyl)-6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)nicotinamide |
| GT-03402 | 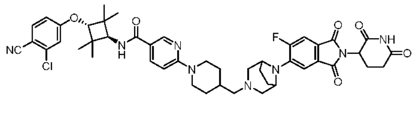 | N-((1r,3r)-3-(3-chloro-4-cyanophenoxy)-2,2,4,4-tetramethylcyclobutyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)nicotinamide | N-((1r,3r)-3-(3-chloro-4-cyanophenoxy)-2,2,4,4-tetramethylcyclobutyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)nicotinamide |
| GT-03482 | 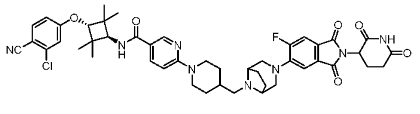 | N-((1r,3r)-3-(3-chloro-4-cyanophenoxy)-2,2,4,4-tetramethylcyclobutyl)-6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)nicotinamide | N-((1r,3r)-3-(3-chloro-4-cyanophenoxy)-2,2,4,4-tetramethylcyclobutyl)-6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)nicotinamide |
| GT-03494 | 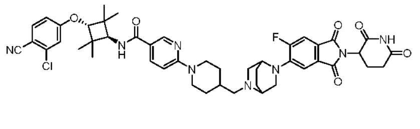 | N-((1r,3r)-3-(3-chloro-4-cyanophenoxy)-2,2,4,4-tetramethylcyclobutyl)-6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)nicotinamide | N-((1r,3r)-3-(3-chloro-4-cyanophenoxy)-2,2,4,4-tetramethylcyclobutyl)-6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1- |

| | | | |
|---|---|---|---|
| | | | yl)nicotinamide |
| | 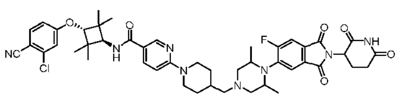 | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)nicotinamide |
| | 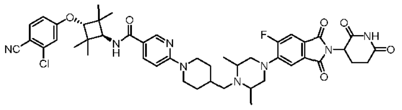 | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)nicotinamide |
| | 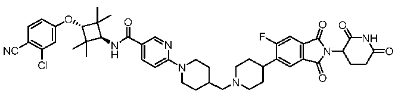 | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)piperidin-1-yl)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)piperidin-1-yl)methyl)piperidin-1-yl)nicotinamide |
| | 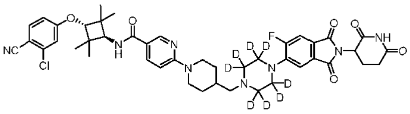 | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)nicotinamide |
| | 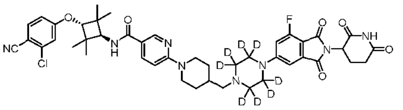 | N-((1r,3r)-3-(3-chloro-4-cyanophenoxy)-2,2,4,4-tetramethylcyclobutyl)-6-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)nicotinamide |
| | 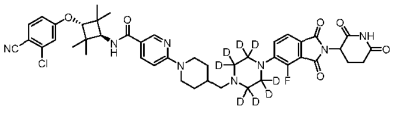 | N-((1r,3r)-3-(3-chloro-4-cyanophenoxy)-2,2,4,4-tetramethylcyclobutyl)-6-(4-((4-(2-(2,6-dioxopiperidin-3-yl)-4-fluoro-1,3-dioxoisoindolin-5-yl)piperazin-1-yl-2,2,3,3,5,5,6,6- | N-((1r,3r)-3-(3-chloro-4-cyanophenoxy)-2,2,4,4-tetramethylcyclobutyl)-6-(4-((4-(2-(2,6-dioxopiperidin-3-yl)-4-fluoro-1,3-dioxoisoindolin-5-yl)piperazin-1-yl-2,2,3,3,5,5,6,6- |

| | | | |
|---|---|---|---|
| | | $d_8$)methyl)piperidin-1-yl)nicotinamide | $d_8$)methyl)piperidin-1-yl)nicotinamide |
| | 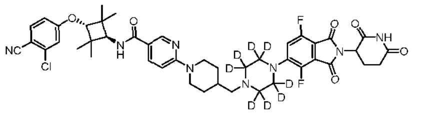 | N-((1r,3r)-3-(3-chloro-4-cyanophenoxy)-2,2,4,4-tetramethylcyclobutyl)-6-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)nicotinamide |
| | 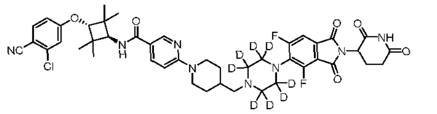 | N-((1r,3r)-3-(3-chloro-4-cyanophenoxy)-2,2,4,4-tetramethylcyclobutyl)-6-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)nicotinamide |
| | 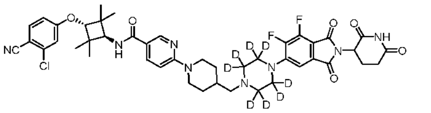 | N-((1r,3r)-3-(3-chloro-4-cyanophenoxy)-2,2,4,4-tetramethylcyclobutyl)-6-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)nicotinamide |
| | 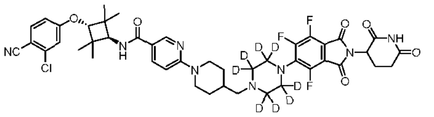 | N-((1r,3r)-3-(3-chloro-4-cyanophenoxy)-2,2,4,4-tetramethylcyclobutyl)-6-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)nicotinamide | N-((1r,3r)-3-(3-chloro-4-cyanophenoxy)-2,2,4,4-tetramethylcyclobutyl)-6-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)nicotinamide |
| | 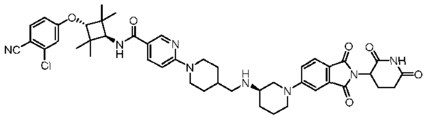 | N-((1r,3R)-3-(3-chloro-4-cyanophenoxy)-2,2,4,4-tetramethylcyclobutyl)-6-(4-((((3R)-1-(2-(2,6-dioxopiperidin-3-yl)-1,3-dioxoisoindolin-5-yl)piperidin-3-yl)amino)methyl)piperidin-1-yl)nicotinamide | N-((1r,3R)-3-(3-chloro-4-cyanophenoxy)-2,2,4,4-tetramethylcyclobutyl)-6-(4-((((3R)-1-(2-(2,6-dioxopiperidin-3-yl)-1,3-dioxoisoindolin-5-yl)piperidin-3-yl)amino)methyl)piperidin-1-yl)nicotinamide |

| | | | |
|---|---|---|---|
| | 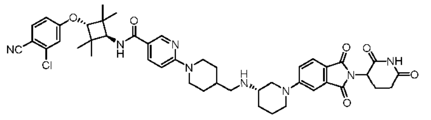 | N-((1r,3S)-3-(3-chloro-4-cyanophenoxy)-2,2,4,4-tetramethylcyclobutyl)-6-(4-((((3S)-1-(2-(2,6-dioxopiperidin-3-yl)-1,3-dioxoisoindolin-5-yl)piperidin-3-yl)amino)methyl)piperidin-1-yl)nicotinamide | N-((1r,3S)-3-(3-chloro-4-cyanophenoxy)-2,2,4,4-tetramethylcyclobutyl)-6-(4-((((3S)-1-(2-(2,6-dioxopiperidin-3-yl)-1,3-dioxoisoindolin-5-yl)piperidin-3-yl)amino)methyl)piperidin-1-yl)nicotinamide |
| | 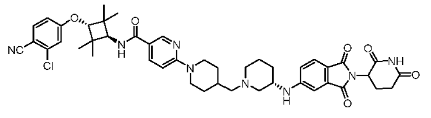 | N-((1r,3r)-3-(3-chloro-4-cyanophenoxy)-2,2,4,4-tetramethylcyclobutyl)-6-(4-(((3S)-3-((2-(2,6-dioxopiperidin-3-yl)-1,3-dioxoisoindolin-5-yl)amino)piperidin-1-yl)methyl)piperidin-1-yl)nicotinamide | N-((1r,3r)-3-(3-chloro-4-cyanophenoxy)-2,2,4,4-tetramethylcyclobutyl)-6-(4-(((3S)-3-((2-(2,6-dioxopiperidin-3-yl)-1,3-dioxoisoindolin-5-yl)amino)piperidin-1-yl)methyl)piperidin-1-yl)nicotinamide |
| | 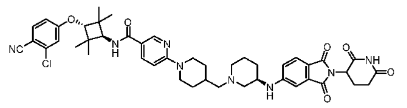 | N-((1r,3r)-3-(3-chloro-4-cyanophenoxy)-2,2,4,4-tetramethylcyclobutyl)-6-(4-(((3R)-3-((2-(2,6-dioxopiperidin-3-yl)-1,3-dioxoisoindolin-5-yl)amino)piperidin-1-yl)methyl)piperidin-1-yl)nicotinamide | N-((1r,3r)-3-(3-chloro-4-cyanophenoxy)-2,2,4,4-tetramethylcyclobutyl)-6-(4-(((3R)-3-((2-(2,6-dioxopiperidin-3-yl)-1,3-dioxoisoindolin-5-yl)amino)piperidin-1-yl)methyl)piperidin-1-yl)nicotinamide |
| GT-03400 | 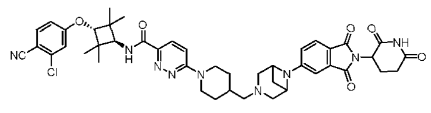 | *N*-((1r,3r)-3-(3-chloro-4-cyanophenoxy)-2,2,4,4-tetramethylcyclobutyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazine-3-carboxamide | *N*-((1r,3r)-3-(3-chloro-4-cyanophenoxy)-2,2,4,4-tetramethylcyclobutyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazine-3-carboxamide |
| GT-03401 | 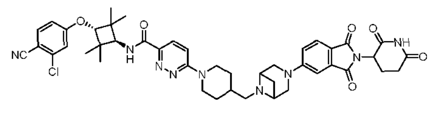 | *N*-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)pyridazine-3-carboxamide | *N*-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)pyridazine-3-carboxamide |
| GT-03399 | 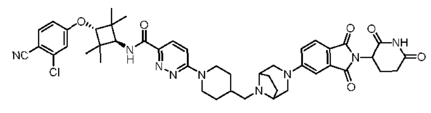 | *N*-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazine-3-carboxamide | *N*-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazine-3-carboxamide |

| | | | |
|---|---|---|---|
| GT-03398 | 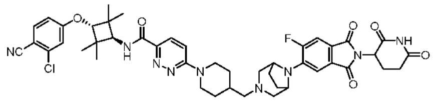 | *N*-((1r,3r)-3-(3-chloro-4-cyanophenoxy)-2,2,4,4-tetramethylcyclobutyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazine-3-carboxamide | *N*-((1r,3r)-3-(3-chloro-4-cyanophenoxy)-2,2,4,4-tetramethylcyclobutyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazine-3-carboxamide |
| | 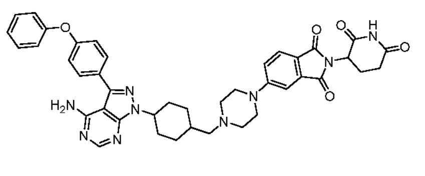 | 5-(4-((4-(4-amino-3-(4-phenoxyphenyl)-1H-pyrazolo[3,4-d]pyrimidin-1-yl)cyclohexyl)methyl)piperazin-1-yl)-2-(2,6-dioxopiperidin-3-yl)isoindoline-1,3-dione | 5-(4-((4-(4-amino-3-(4-phenoxyphenyl)-1H-pyrazolo[3,4-d]pyrimidin-1-yl)cyclohexyl)methyl)piperazin-1-yl)-2-(2,6-dioxopiperidin-3-yl)isoindoline-1,3-dione |
| | 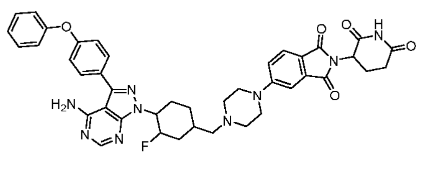 | 5-(4-((4-(4-amino-3-(4-phenoxyphenyl)-1H-pyrazolo[3,4-d]pyrimidin-1-yl)-3-fluorocyclohexyl)methyl)piperazin-1-yl)-2-(2,6-dioxopiperidin-3-yl)isoindoline-1,3-dione | 5-(4-((4-(4-amino-3-(4-phenoxyphenyl)-1H-pyrazolo[3,4-d]pyrimidin-1-yl)-3-fluorocyclohexyl)methyl)piperazin-1-yl)-2-(2,6-dioxopiperidin-3-yl)isoindoline-1,3-dione |
| | 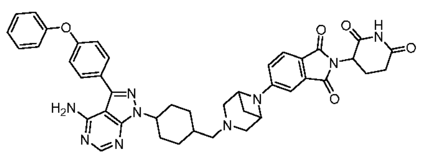 | 5-(3-((4-(4-amino-3-(4-phenoxyphenyl)-1H-pyrazolo[3,4-d]pyrimidin-1-yl)cyclohexyl)methyl)-3,6-diazabicyclo[3.1.1]heptan-6-yl)-2-(2,6-dioxopiperidin-3-yl)isoindoline-1,3-dione | 5-(3-((4-(4-amino-3-(4-phenoxyphenyl)-1H-pyrazolo[3,4-d]pyrimidin-1-yl)cyclohexyl)methyl)-3,6-diazabicyclo[3.1.1]heptan-6-yl)-2-(2,6-dioxopiperidin-3-yl)isoindoline-1,3-dione |
| | 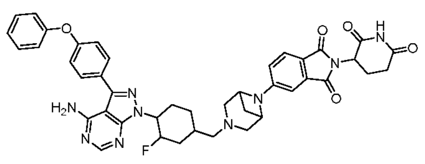 | 5-(3-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-6-yl)-2-(2,6-dioxopiperidin-3-yl)isoindoline-1,3-dione | 5-(3-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-6-yl)-2-(2,6-dioxopiperidin-3-yl)isoindoline-1,3-dione |
| | 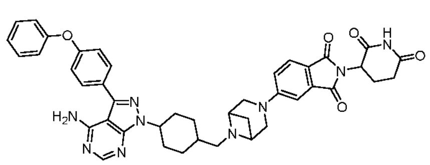 | 5-(6-((4-(4-amino-3-(4-phenoxyphenyl)-1H-pyrazolo[3,4-d]pyrimidin-1-yl)cyclohexyl)methyl)-3,6-diazabicyclo[3.1.1]heptan-3-yl)-2-(2,6-dioxopiperidin-3-yl)isoindoline-1,3-dione | 5-(6-((4-(4-amino-3-(4-phenoxyphenyl)-1H-pyrazolo[3,4-d]pyrimidin-1-yl)cyclohexyl)methyl)-3,6-diazabicyclo[3.1.1]heptan-3-yl)-2-(2,6-dioxopiperidin-3-yl)isoindoline-1,3-dione |

| | | | |
|---|---|---|---|
| | 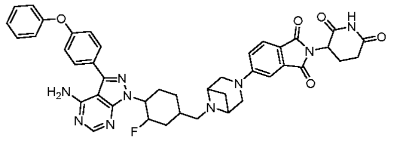 | 5-(6-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-3-yl)-2-(2,6-dioxopiperidin-3-yl)isoindoline-1,3-dione | 5-(6-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-3-yl)-2-(2,6-dioxopiperidin-3-yl)isoindoline-1,3-dione |
| | 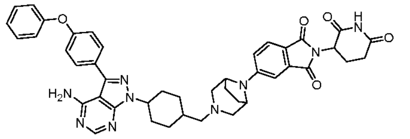 | 5-(3-((4-(4-amino-3-(4-phenoxyphenyl)-1H-pyrazolo[3,4-d]pyrimidin-1-yl)cyclohexyl)methyl)-3,8-diazabicyclo[3.2.1]octan-8-yl)-2-(2,6-dioxopiperidin-3-yl)isoindoline-1,3-dione | 5-(3-((4-(4-amino-3-(4-phenoxyphenyl)-1H-pyrazolo[3,4-d]pyrimidin-1-yl)cyclohexyl)methyl)-3,8-diazabicyclo[3.2.1]octan-8-yl)-2-(2,6-dioxopiperidin-3-yl)isoindoline-1,3-dione |
| | 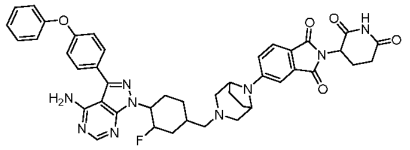 | 5-(3-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-8-yl)-2-(2,6-dioxopiperidin-3-yl)isoindoline-1,3-dione | 5-(3-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-8-yl)-2-(2,6-dioxopiperidin-3-yl)isoindoline-1,3-dione |
| | 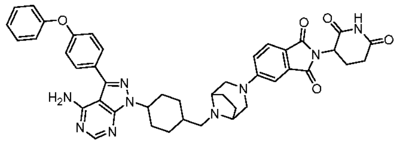 | 5-(8-((4-(4-amino-3-(4-phenoxyphenyl)-1H-pyrazolo[3,4-d]pyrimidin-1-yl)cyclohexyl)methyl)-3,8-diazabicyclo[3.2.1]octan-3-yl)-2-(2,6-dioxopiperidin-3-yl)isoindoline-1,3-dione | 5-(8-((4-(4-amino-3-(4-phenoxyphenyl)-1H-pyrazolo[3,4-d]pyrimidin-1-yl)cyclohexyl)methyl)-3,8-diazabicyclo[3.2.1]octan-3-yl)-2-(2,6-dioxopiperidin-3-yl)isoindoline-1,3-dione |
| | 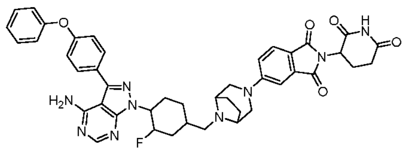 | 5-(8-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-3-yl)-2-(2,6-dioxopiperidin-3-yl)isoindoline-1,3-dione | 5-(8-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-3-yl)-2-(2,6-dioxopiperidin-3-yl)isoindoline-1,3-dione |
| | 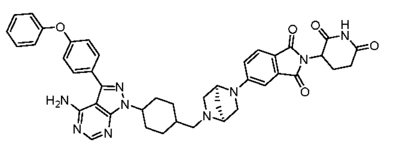 | 5-((1S,4S)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)isoindoline-1,3-dione | 5-((1S,4S)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)isoindoline-1,3-dione |
| | 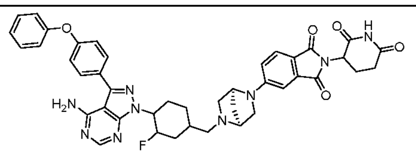 | 5-((1S,4S)-5-((4-(4-amino-3-(4-phenoxyphenyl)-1H-pyrazolo[3,4-d]pyrimidin-1-yl)-3-fluorocyclohexyl)methyl)- | 5-((1S,4S)-5-((4-(4-amino-3-(4-phenoxyphenyl)-1H-pyrazolo[3,4-d]pyrimidin-1-yl)-3-fluorocyclohexyl)methyl)- |

| | | | |
|---|---|---|---|
| | | 2,5-diazabicyclo[2.2.1]heptan-2-yl)-2-(2,6-dioxopiperidin-3-yl)isoindoline-1,3-dione | 2,5-diazabicyclo[2.2.1]heptan-2-yl)-2-(2,6-dioxopiperidin-3-yl)isoindoline-1,3-dione |
| | 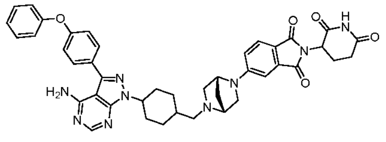 | 5-((1R,4R)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)isoindoline-1,3-dione | 5-((1R,4R)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)isoindoline-1,3-dione |
| | 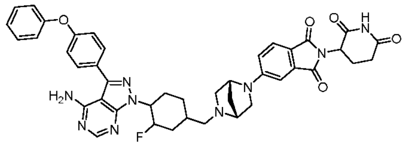 | 5-((1R,4R)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)isoindoline-1,3-dione | 5-((1R,4R)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)isoindoline-1,3-dione |
| | 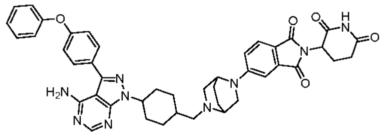 | 5-(5-((4-(4-amino-3-(4-phenoxyphenyl)-1H-pyrazolo[3,4-d]pyrimidin-1-yl)cyclohexyl)methyl)-2,5-diazabicyclo[2.2.2]octan-2-yl)-2-(2,6-dioxopiperidin-3-yl)isoindoline-1,3-dione | 5-(5-((4-(4-amino-3-(4-phenoxyphenyl)-1H-pyrazolo[3,4-d]pyrimidin-1-yl)cyclohexyl)methyl)-2,5-diazabicyclo[2.2.2]octan-2-yl)-2-(2,6-dioxopiperidin-3-yl)isoindoline-1,3-dione |
| | 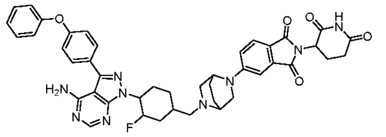 | 5-(5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.2]octan-2-yl)-2-(2,6-dioxopiperidin-3-yl)isoindoline-1,3-dione | 5-(5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.2]octan-2-yl)-2-(2,6-dioxopiperidin-3-yl)isoindoline-1,3-dione |
| | 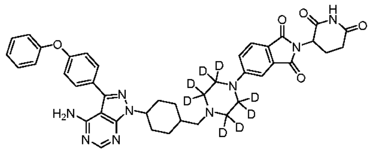 | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)isoindoline-1,3-dione | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)isoindoline-1,3-dione |
| | 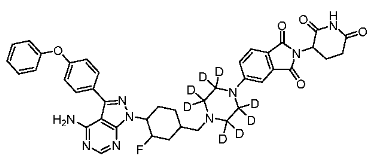 | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)isoindoline-1,3-dione | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)isoindoline-1,3-dione |

| | | | |
|---|---|---|---|
| | 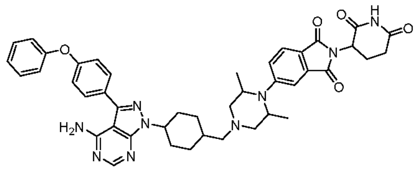 | 5-(4-((4-(4-amino-3-(4-phenoxyphenyl)-1H-pyrazolo[3,4-d]pyrimidin-1-yl)cyclohexyl)methyl)-2,6-dimethylpiperazin-1-yl)-2-(2,6-dioxopiperidin-3-yl)isoindoline-1,3-dione | 5-(4-((4-(4-amino-3-(4-phenoxyphenyl)-1H-pyrazolo[3,4-d]pyrimidin-1-yl)cyclohexyl)methyl)-2,6-dimethylpiperazin-1-yl)-2-(2,6-dioxopiperidin-3-yl)isoindoline-1,3-dione |
| | 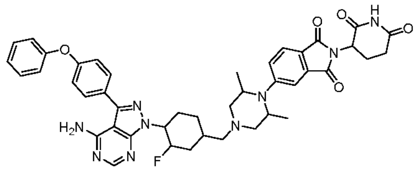 | 5-(4-((4-(4-amino-3-(4-phenoxyphenyl)-1H-pyrazolo[3,4-d]pyrimidin-1-yl)-3-fluorocyclohexyl)methyl)-2,6-dimethylpiperazin-1-yl)-2-(2,6-dioxopiperidin-3-yl)isoindoline-1,3-dione | 5-(4-((4-(4-amino-3-(4-phenoxyphenyl)-1H-pyrazolo[3,4-d]pyrimidin-1-yl)-3-fluorocyclohexyl)methyl)-2,6-dimethylpiperazin-1-yl)-2-(2,6-dioxopiperidin-3-yl)isoindoline-1,3-dione |
| | 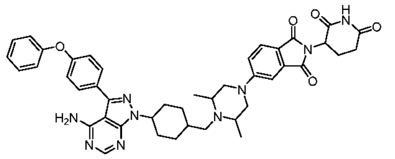 | 5-(4-((4-(4-amino-3-(4-phenoxyphenyl)-1H-pyrazolo[3,4-d]pyrimidin-1-yl)cyclohexyl)methyl)-3,5-dimethylpiperazin-1-yl)-2-(2,6-dioxopiperidin-3-yl)isoindoline-1,3-dione | 5-(4-((4-(4-amino-3-(4-phenoxyphenyl)-1H-pyrazolo[3,4-d]pyrimidin-1-yl)cyclohexyl)methyl)-3,5-dimethylpiperazin-1-yl)-2-(2,6-dioxopiperidin-3-yl)isoindoline-1,3-dione |
| | 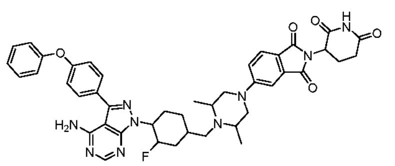 | 5-(4-((4-(4-amino-3-(4-phenoxyphenyl)-1H-pyrazolo[3,4-d]pyrimidin-1-yl)-3-fluorocyclohexyl)methyl)-3,5-dimethylpiperazin-1-yl)-2-(2,6-dioxopiperidin-3-yl)isoindoline-1,3-dione | 5-(4-((4-(4-amino-3-(4-phenoxyphenyl)-1H-pyrazolo[3,4-d]pyrimidin-1-yl)-3-fluorocyclohexyl)methyl)-3,5-dimethylpiperazin-1-yl)-2-(2,6-dioxopiperidin-3-yl)isoindoline-1,3-dione |
| | 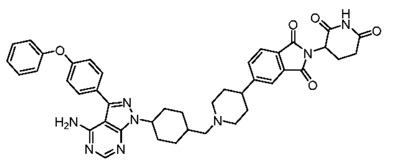 | 5-(1-((4-(4-amino-3-(4-phenoxyphenyl)-1H-pyrazolo[3,4-d]pyrimidin-1-yl)cyclohexyl)methyl)piperidin-4-yl)-2-(2,6-dioxopiperidin-3-yl)isoindoline-1,3-dione | 5-(1-((4-(4-amino-3-(4-phenoxyphenyl)-1H-pyrazolo[3,4-d]pyrimidin-1-yl)cyclohexyl)methyl)piperidin-4-yl)-2-(2,6-dioxopiperidin-3-yl)isoindoline-1,3-dione |
| | 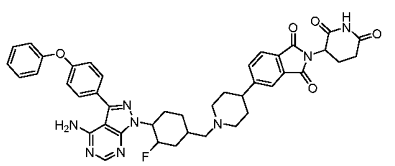 | 5-(1-((4-(4-amino-3-(4-phenoxyphenyl)-1H-pyrazolo[3,4-d]pyrimidin-1-yl)-3-fluorocyclohexyl)methyl)piperidin-4-yl)-2-(2,6-dioxopiperidin-3-yl)isoindoline-1,3-dione | 5-(1-((4-(4-amino-3-(4-phenoxyphenyl)-1H-pyrazolo[3,4-d]pyrimidin-1-yl)-3-fluorocyclohexyl)methyl)piperidin-4-yl)-2-(2,6-dioxopiperidin-3-yl)isoindoline-1,3-dione |
| | 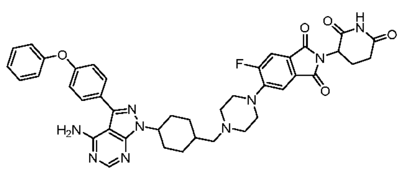 | 5-(4-((4-(4-amino-3-(4-phenoxyphenyl)-1H-pyrazolo[3,4-d]pyrimidin-1-yl)cyclohexyl)methyl)piperazin-1-yl)-2-(2,6-dioxopiperidin-3-yl)-6-fluoroisoindoline-1,3-dione | 5-(4-((4-(4-amino-3-(4-phenoxyphenyl)-1H-pyrazolo[3,4-d]pyrimidin-1-yl)cyclohexyl)methyl)piperazin-1-yl)-2-(2,6-dioxopiperidin-3-yl)-6-fluoroisoindoline-1,3-dione |

| | | | |
|---|---|---|---|
| | 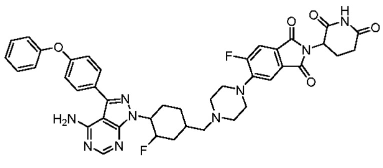 | 5-(4-((4-(4-amino-3-(4-phenoxyphenyl)-1H-pyrazolo[3,4-d]pyrimidin-1-yl)-3-fluorocyclohexyl)methyl)piperazin-1-yl)-2-(2,6-dioxopiperidin-3-yl)-6-fluoroisoindoline-1,3-dione | 5-(4-((4-(4-amino-3-(4-phenoxyphenyl)-1H-pyrazolo[3,4-d]pyrimidin-1-yl)-3-fluorocyclohexyl)methyl)piperazin-1-yl)-2-(2,6-dioxopiperidin-3-yl)-6-fluoroisoindoline-1,3-dione |
| | 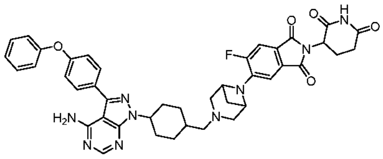 | 5-(3-((4-(4-amino-3-(4-phenoxyphenyl)-1H-pyrazolo[3,4-d]pyrimidin-1-yl)cyclohexyl)methyl)-3,6-diazabicyclo[3.1.1]heptan-6-yl)-2-(2,6-dioxopiperidin-3-yl)-6-fluoroisoindoline-1,3-dione | 5-(3-((4-(4-amino-3-(4-phenoxyphenyl)-1H-pyrazolo[3,4-d]pyrimidin-1-yl)cyclohexyl)methyl)-3,6-diazabicyclo[3.1.1]heptan-6-yl)-2-(2,6-dioxopiperidin-3-yl)-6-fluoroisoindoline-1,3-dione |
| | 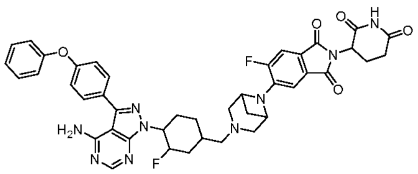 | 5-(3-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-6-yl)-2-(2,6-dioxopiperidin-3-yl)-6-fluoroisoindoline-1,3-dione | 5-(3-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-6-yl)-2-(2,6-dioxopiperidin-3-yl)-6-fluoroisoindoline-1,3-dione |
| | 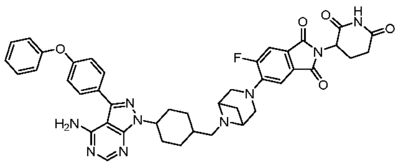 | 5-(6-((4-(4-amino-3-(4-phenoxyphenyl)-1H-pyrazolo[3,4-d]pyrimidin-1-yl)cyclohexyl)methyl)-3,6-diazabicyclo[3.1.1]heptan-3-yl)-2-(2,6-dioxopiperidin-3-yl)-6-fluoroisoindoline-1,3-dione | 5-(6-((4-(4-amino-3-(4-phenoxyphenyl)-1H-pyrazolo[3,4-d]pyrimidin-1-yl)cyclohexyl)methyl)-3,6-diazabicyclo[3.1.1]heptan-3-yl)-2-(2,6-dioxopiperidin-3-yl)-6-fluoroisoindoline-1,3-dione |
| | 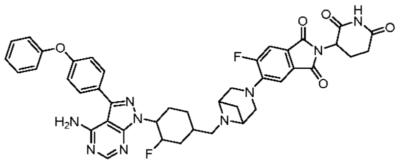 | 5-(6-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-3-yl)-2-(2,6-dioxopiperidin-3-yl)-6-fluoroisoindoline-1,3-dione | 5-(6-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-3-yl)-2-(2,6-dioxopiperidin-3-yl)-6-fluoroisoindoline-1,3-dione |
| | 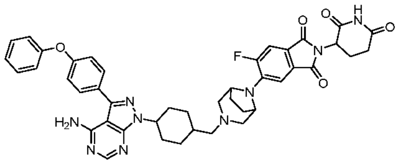 | 5-(3-((4-(4-amino-3-(4-phenoxyphenyl)-1H-pyrazolo[3,4-d]pyrimidin-1-yl)cyclohexyl)methyl)-3,8-diazabicyclo[3.2.1]octan-8-yl)-2-(2,6-dioxopiperidin-3-yl)-6-fluoroisoindoline-1,3-dione | 5-(3-((4-(4-amino-3-(4-phenoxyphenyl)-1H-pyrazolo[3,4-d]pyrimidin-1-yl)cyclohexyl)methyl)-3,8-diazabicyclo[3.2.1]octan-8-yl)-2-(2,6-dioxopiperidin-3-yl)-6-fluoroisoindoline-1,3-dione |

| | | | |
|---|---|---|---|
| | 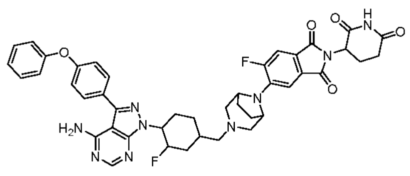 | 5-(3-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-8-yl)-2-(2,6-dioxopiperidin-3-yl)-6-fluoroisoindoline-1,3-dione | 5-(3-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-8-yl)-2-(2,6-dioxopiperidin-3-yl)-6-fluoroisoindoline-1,3-dione |
| | 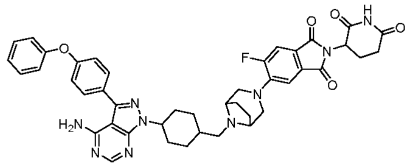 | 5-(8-((4-(4-amino-3-(4-phenoxyphenyl)-1H-pyrazolo[3,4-d]pyrimidin-1-yl)cyclohexyl)methyl)-3,8-diazabicyclo[3.2.1]octan-3-yl)-2-(2,6-dioxopiperidin-3-yl)-6-fluoroisoindoline-1,3-dione | 5-(8-((4-(4-amino-3-(4-phenoxyphenyl)-1H-pyrazolo[3,4-d]pyrimidin-1-yl)cyclohexyl)methyl)-3,8-diazabicyclo[3.2.1]octan-3-yl)-2-(2,6-dioxopiperidin-3-yl)-6-fluoroisoindoline-1,3-dione |
| | 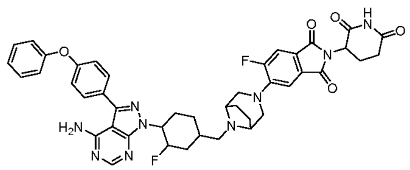 | 5-(8-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-3-yl)-2-(2,6-dioxopiperidin-3-yl)-6-fluoroisoindoline-1,3-dione | 5-(8-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-3-yl)-2-(2,6-dioxopiperidin-3-yl)-6-fluoroisoindoline-1,3-dione |
| | 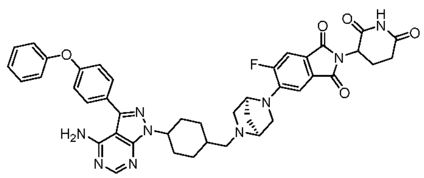 | 5-((1S,4S)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)-6-fluoroisoindoline-1,3-dione | 5-((1S,4S)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)-6-fluoroisoindoline-1,3-dione |
| | 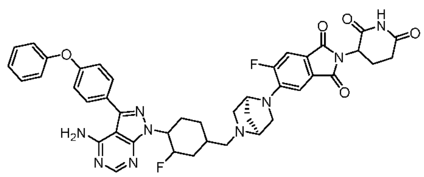 | 5-((1S,4S)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)-6-fluoroisoindoline-1,3-dione | 5-((1S,4S)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)-6-fluoroisoindoline-1,3-dione |
| | 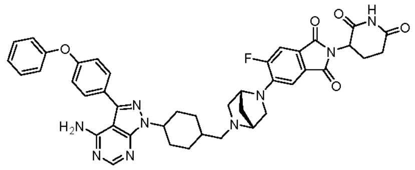 | 5-((1R,4R)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)-6-fluoroisoindoline-1,3-dione | 5-((1R,4R)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)-6-fluoroisoindoline-1,3-dione |

| | | | |
|---|---|---|---|
| | 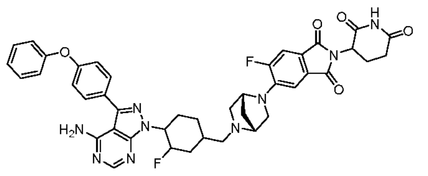 | 5-((1R,4R)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)-6-fluoroisoindoline-1,3-dione | 5-((1R,4R)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)-6-fluoroisoindoline-1,3-dione |
| | 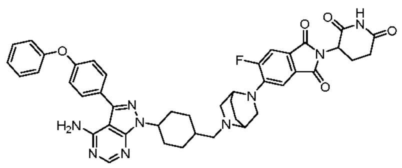 | 5-(5-((4-(4-amino-3-(4-phenoxyphenyl)-1H-pyrazolo[3,4-d]pyrimidin-1-yl)cyclohexyl)methyl)-2,5-diazabicyclo[2.2.2]octan-2-yl)-2-(2,6-dioxopiperidin-3-yl)-6-fluoroisoindoline-1,3-dione | 5-(5-((4-(4-amino-3-(4-phenoxyphenyl)-1H-pyrazolo[3,4-d]pyrimidin-1-yl)cyclohexyl)methyl)-2,5-diazabicyclo[2.2.2]octan-2-yl)-2-(2,6-dioxopiperidin-3-yl)-6-fluoroisoindoline-1,3-dione |
| | 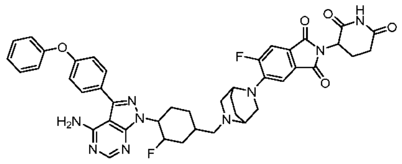 | 5-(5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.2]octan-2-yl)-2-(2,6-dioxopiperidin-3-yl)-6-fluoroisoindoline-1,3-dione | 5-(5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.2]octan-2-yl)-2-(2,6-dioxopiperidin-3-yl)-6-fluoroisoindoline-1,3-dione |
| | 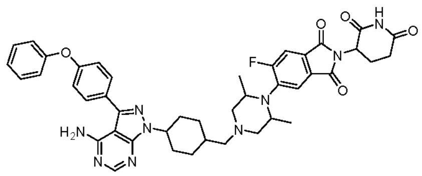 | 5-(4-((4-(4-amino-3-(4-phenoxyphenyl)-1H-pyrazolo[3,4-d]pyrimidin-1-yl)cyclohexyl)methyl)-2,6-dimethylpiperazin-1-yl)-2-(2,6-dioxopiperidin-3-yl)-6-fluoroisoindoline-1,3-dione | 5-(4-((4-(4-amino-3-(4-phenoxyphenyl)-1H-pyrazolo[3,4-d]pyrimidin-1-yl)cyclohexyl)methyl)-2,6-dimethylpiperazin-1-yl)-2-(2,6-dioxopiperidin-3-yl)-6-fluoroisoindoline-1,3-dione |
| | 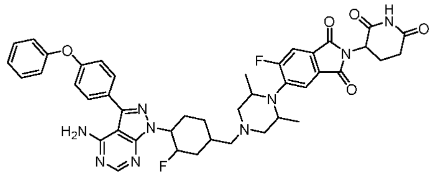 | 5-(4-((4-(4-amino-3-(4-phenoxyphenyl)-1H-pyrazolo[3,4-d]pyrimidin-1-yl)-3-fluorocyclohexyl)methyl)-2,6-dimethylpiperazin-1-yl)-2-(2,6-dioxopiperidin-3-yl)-6-fluoroisoindoline-1,3-dione | 5-(4-((4-(4-amino-3-(4-phenoxyphenyl)-1H-pyrazolo[3,4-d]pyrimidin-1-yl)-3-fluorocyclohexyl)methyl)-2,6-dimethylpiperazin-1-yl)-2-(2,6-dioxopiperidin-3-yl)-6-fluoroisoindoline-1,3-dione |
| | 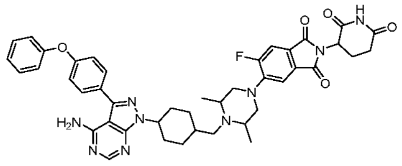 | 5-(4-((4-(4-amino-3-(4-phenoxyphenyl)-1H-pyrazolo[3,4-d]pyrimidin-1-yl)cyclohexyl)methyl)-3,5-dimethylpiperazin-1-yl)-2-(2,6-dioxopiperidin-3-yl)-6-fluoroisoindoline-1,3-dione | 5-(4-((4-(4-amino-3-(4-phenoxyphenyl)-1H-pyrazolo[3,4-d]pyrimidin-1-yl)cyclohexyl)methyl)-3,5-dimethylpiperazin-1-yl)-2-(2,6-dioxopiperidin-3-yl)-6-fluoroisoindoline-1,3-dione |

| | | | |
|---|---|---|---|
| | 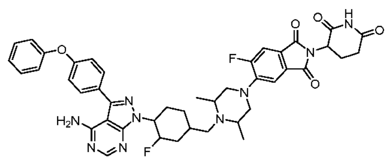 | 5-(4-((4-(4-amino-3-(4-phenoxyphenyl)-1H-pyrazolo[3,4-d]pyrimidin-1-yl)-3-fluorocyclohexyl)methyl)-3,5-dimethylpiperazin-1-yl)-2-(2,6-dioxopiperidin-3-yl)-6-fluoroisoindoline-1,3-dione | 5-(4-((4-(4-amino-3-(4-phenoxyphenyl)-1H-pyrazolo[3,4-d]pyrimidin-1-yl)-3-fluorocyclohexyl)methyl)-3,5-dimethylpiperazin-1-yl)-2-(2,6-dioxopiperidin-3-yl)-6-fluoroisoindoline-1,3-dione |
| | 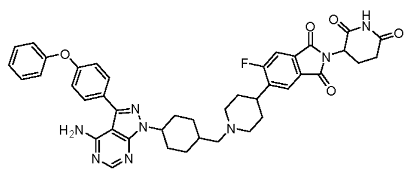 | 5-(1-((4-(4-amino-3-(4-phenoxyphenyl)-1H-pyrazolo[3,4-d]pyrimidin-1-yl)cyclohexyl)methyl)piperidin-4-yl)-2-(2,6-dioxopiperidin-3-yl)-6-fluoroisoindoline-1,3-dione | 5-(1-((4-(4-amino-3-(4-phenoxyphenyl)-1H-pyrazolo[3,4-d]pyrimidin-1-yl)cyclohexyl)methyl)piperidin-4-yl)-2-(2,6-dioxopiperidin-3-yl)-6-fluoroisoindoline-1,3-dione |
| | 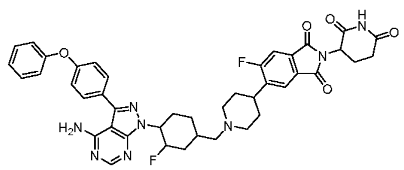 | 5-(1-((4-(4-amino-3-(4-phenoxyphenyl)-1H-pyrazolo[3,4-d]pyrimidin-1-yl)-3-fluorocyclohexyl)methyl)piperidin-4-yl)-2-(2,6-dioxopiperidin-3-yl)-6-fluoroisoindoline-1,3-dione | 5-(1-((4-(4-amino-3-(4-phenoxyphenyl)-1H-pyrazolo[3,4-d]pyrimidin-1-yl)-3-fluorocyclohexyl)methyl)piperidin-4-yl)-2-(2,6-dioxopiperidin-3-yl)-6-fluoroisoindoline-1,3-dione |
| | 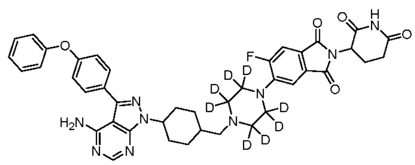 | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-6-fluoroisoindoline-1,3-dione | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-6-fluoroisoindoline-1,3-dione |
| | 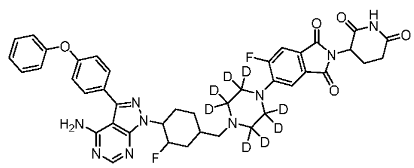 | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-6-fluoroisoindoline-1,3-dione | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-6-fluoroisoindoline-1,3-dione |
| | 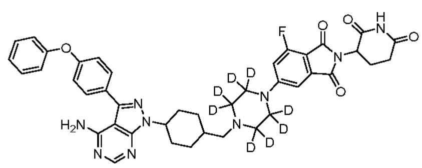 | 6-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4-fluoroisoindoline-1,3-dione | 6-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4-fluoroisoindoline-1,3-dione |
| | 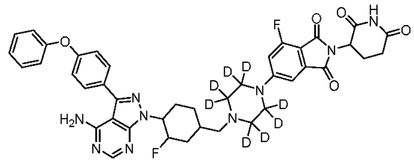 | 6-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6- | 6-(4-((4-(4-amino-3-(4-phenoxyphenyl)-1H-pyrazolo[3,4-d]pyrimidin-1-yl)-3-fluorocyclohexyl)methyl)piperazin-1-yl- |

| | | | |
|---|---|---|---|
| | | $d_8$)-2-(2,6-dioxopiperidin-3-yl)-4-fluoroisoindoline-1,3-dione | 2,2,3,3,5,5,6,6-$d_8$)-2-(2,6-dioxopiperidin-3-yl)-4-fluoroisoindoline-1,3-dione |
| | 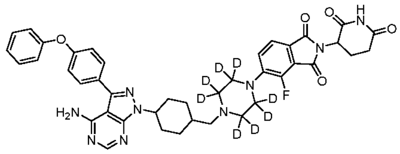 | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4-fluoroisoindoline-1,3-dione | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4-fluoroisoindoline-1,3-dione |
| | 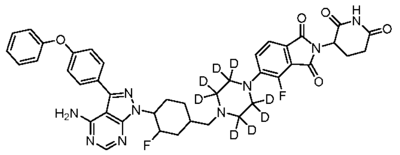 | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4-fluoroisoindoline-1,3-dione | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4-fluoroisoindoline-1,3-dione |
| | 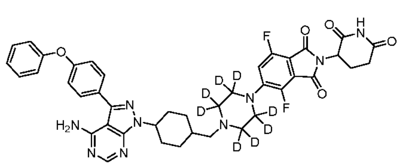 | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,7-difluoroisoindoline-1,3-dione | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,7-difluoroisoindoline-1,3-dione |
| | 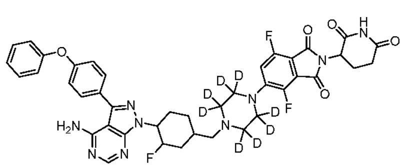 | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,7-difluoroisoindoline-1,3-dione | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,7-difluoroisoindoline-1,3-dione |
| | 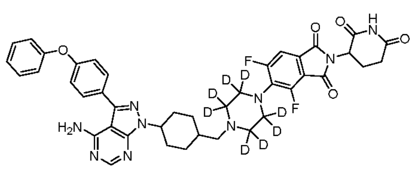 | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,6-difluoroisoindoline-1,3-dione | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,6-difluoroisoindoline-1,3-dione |
| | 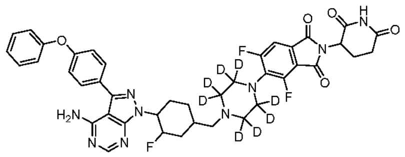 | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3- | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6- |

| | | | |
|---|---|---|---|
| | | yl)-4,6-difluoroisoindoline-1,3-dione | dioxopiperidin-3-yl)-4,6-difluoroisoindoline-1,3-dione |
| | 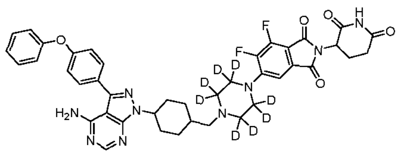 | 6-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,5-difluoroisoindoline-1,3-dione | 6-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,5-difluoroisoindoline-1,3-dione |
| | 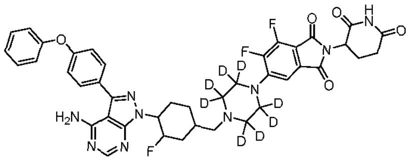 | 6-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,5-difluoroisoindoline-1,3-dione | 6-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,5-difluoroisoindoline-1,3-dione |
| | 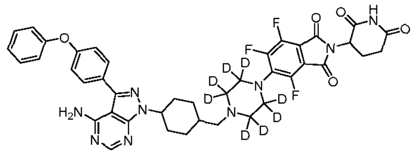 | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,6,7-trifluoroisoindoline-1,3-dione |
| | 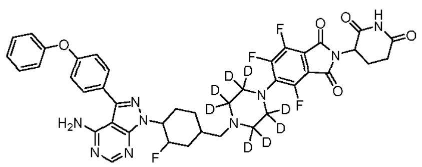 | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,6,7-trifluoroisoindoline-1,3-dione |
| | 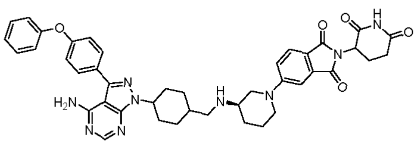 | 5-((R)-3-(((4-(4-amino-3-(4-phenoxyphenyl)-1H-pyrazolo[3,4-d]pyrimidin-1-yl)cyclohexyl)methyl)amino)piperidin-1-yl)-2-(2,6-dioxopiperidin-3-yl)isoindoline-1,3-dione | 5-((R)-3-(((4-(4-amino-3-(4-phenoxyphenyl)-1H-pyrazolo[3,4-d]pyrimidin-1-yl)cyclohexyl)methyl)amino)piperidin-1-yl)-2-(2,6-dioxopiperidin-3-yl)isoindoline-1,3-dione |
| | 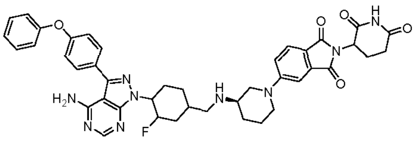 | 5-((3R)-3-(((4-(4-amino-3-(4-phenoxyphenyl)-1H-pyrazolo[3,4-d]pyrimidin-1-yl)-3-fluorocyclohexyl)methyl)amino)piperidin-1-yl)-2-(2,6-dioxopiperidin-3- | 5-((3R)-3-(((4-(4-amino-3-(4-phenoxyphenyl)-1H-pyrazolo[3,4-d]pyrimidin-1-yl)-3-fluorocyclohexyl)methyl)amino)piperidin-1-yl)-2-(2,6-dioxopiperidin-3- |

| | | | |
|---|---|---|---|
| | | yl)isoindoline-1,3-dione | yl)isoindoline-1,3-dione |
| | 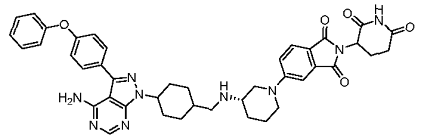 | 5-((S)-3-(((4-(4-amino-3-(4-phenoxyphenyl)-1H-pyrazolo[3,4-d]pyrimidin-1-yl)cyclohexyl)methyl)amino)piperidin-1-yl)-2-(2,6-dioxopiperidin-3-yl)isoindoline-1,3-dione | 5-((S)-3-(((4-(4-amino-3-(4-phenoxyphenyl)-1H-pyrazolo[3,4-d]pyrimidin-1-yl)cyclohexyl)methyl)amino)piperidin-1-yl)-2-(2,6-dioxopiperidin-3-yl)isoindoline-1,3-dione |
| | 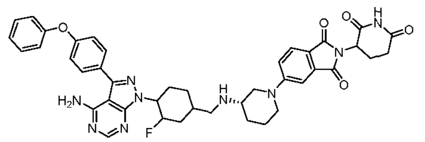 | 5-((3S)-3-(((4-(4-amino-3-(4-phenoxyphenyl)-1H-pyrazolo[3,4-d]pyrimidin-1-yl)-3-fluorocyclohexyl)methyl)amino)piperidin-1-yl)-2-(2,6-dioxopiperidin-3-yl)isoindoline-1,3-dione | 5-((3S)-3-(((4-(4-amino-3-(4-phenoxyphenyl)-1H-pyrazolo[3,4-d]pyrimidin-1-yl)-3-fluorocyclohexyl)methyl)amino)piperidin-1-yl)-2-(2,6-dioxopiperidin-3-yl)isoindoline-1,3-dione |
| | 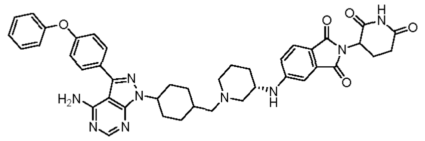 | 5-(((S)-1-((4-(4-amino-3-(4-phenoxyphenyl)-1H-pyrazolo[3,4-d]pyrimidin-1-yl)cyclohexyl)methyl)piperidin-3-yl)amino)-2-(2,6-dioxopiperidin-3-yl)isoindoline-1,3-dione | 5-(((S)-1-((4-(4-amino-3-(4-phenoxyphenyl)-1H-pyrazolo[3,4-d]pyrimidin-1-yl)cyclohexyl)methyl)piperidin-3-yl)amino)-2-(2,6-dioxopiperidin-3-yl)isoindoline-1,3-dione |
| | 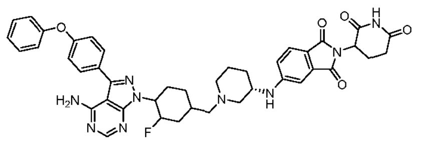 | 5-(((3S)-1-((4-(4-amino-3-(4-phenoxyphenyl)-1H-pyrazolo[3,4-d]pyrimidin-1-yl)-3-fluorocyclohexyl)methyl)piperidin-3-yl)amino)-2-(2,6-dioxopiperidin-3-yl)isoindoline-1,3-dione | 5-(((3S)-1-((4-(4-amino-3-(4-phenoxyphenyl)-1H-pyrazolo[3,4-d]pyrimidin-1-yl)-3-fluorocyclohexyl)methyl)piperidin-3-yl)amino)-2-(2,6-dioxopiperidin-3-yl)isoindoline-1,3-dione |
| | 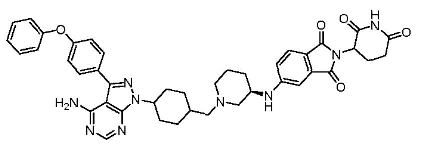 | 5-(((R)-1-((4-(4-amino-3-(4-phenoxyphenyl)-1H-pyrazolo[3,4-d]pyrimidin-1-yl)cyclohexyl)methyl)piperidin-3-yl)amino)-2-(2,6-dioxopiperidin-3-yl)isoindoline-1,3-dione | 5-(((R)-1-((4-(4-amino-3-(4-phenoxyphenyl)-1H-pyrazolo[3,4-d]pyrimidin-1-yl)cyclohexyl)methyl)piperidin-3-yl)amino)-2-(2,6-dioxopiperidin-3-yl)isoindoline-1,3-dione |
| | 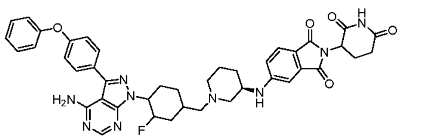 | 5-(((3R)-1-((4-(4-amino-3-(4-phenoxyphenyl)-1H-pyrazolo[3,4-d]pyrimidin-1-yl)-3-fluorocyclohexyl)methyl)piperidin-3-yl)amino)-2-(2,6-dioxopiperidin-3-yl)isoindoline-1,3-dione | 5-(((3R)-1-((4-(4-amino-3-(4-phenoxyphenyl)-1H-pyrazolo[3,4-d]pyrimidin-1-yl)-3-fluorocyclohexyl)methyl)piperidin-3-yl)amino)-2-(2,6-dioxopiperidin-3-yl)isoindoline-1,3-dione |
| | 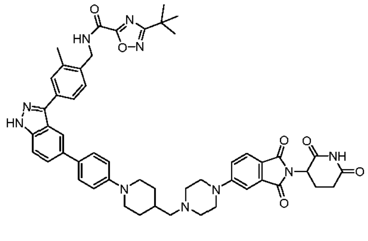 | 3-(tert-butyl)-N-(4-(5-(4-(4-((4-(2-(2,6-dioxopiperidin-3-yl)-1,3-dioxoisoindolin-5-yl)piperazin-1-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-1,3-dioxoisoindolin-5-yl)piperazin-1-yl)methyl)piperidin-1-yl)phenyl)-1H-indazol-3-yl)-2-methylbenzyl)-1,2,4-oxadiazole-5-carboxamide |

| | | | |
|---|---|---|---|
| | 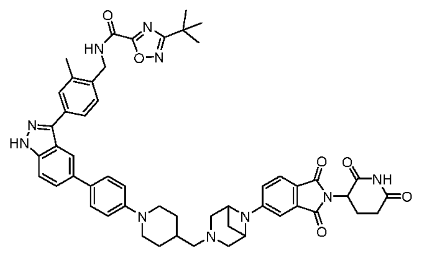 | 3-(tert-butyl)-N-(4-(5-(4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)phenyl)-1H-indazol-3-yl)-2-methylbenzyl)-1,2,4-oxadiazole-5-carboxamide |
| | 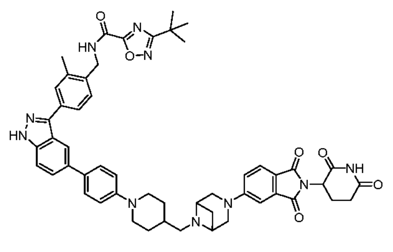 | 3-(tert-butyl)-N-(4-(5-(4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)phenyl)-1H-indazol-3-yl)-2-methylbenzyl)-1,2,4-oxadiazole-5-carboxamide |
| | 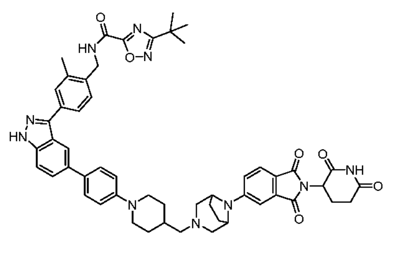 | 3-(tert-butyl)-N-(4-(5-(4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)phenyl)-1H-indazol-3-yl)-2-methylbenzyl)-1,2,4-oxadiazole-5-carboxamide |
| | 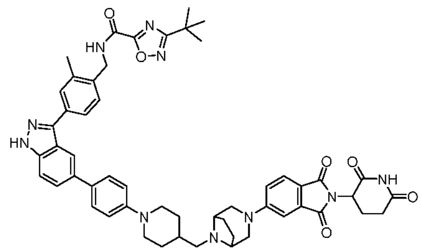 | 3-(tert-butyl)-N-(4-(5-(4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)phenyl)-1H-indazol-3-yl)-2-methylbenzyl)-1,2,4-oxadiazole-5-carboxamide |
| | 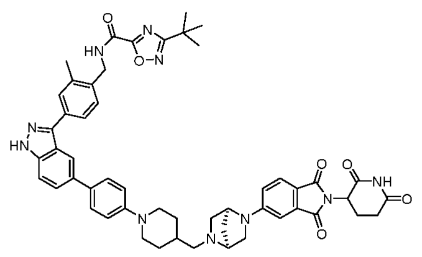 | 3-(tert-butyl)-N-(4-(5-(4-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide |
| | 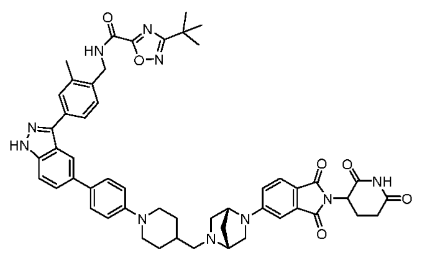 | 3-(tert-butyl)-N-(4-(5-(4-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide |

| | | | |
|---|---|---|---|
| | 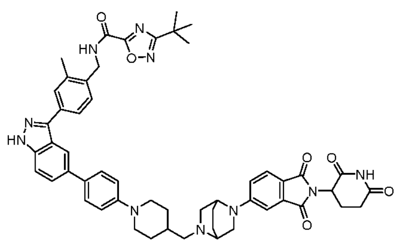 | 3-(tert-butyl)-N-(4-(5-(4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)phenyl)-1H-indazol-3-yl)-2-methylbenzyl)-1,2,4-oxadiazole-5-carboxamide |
| | 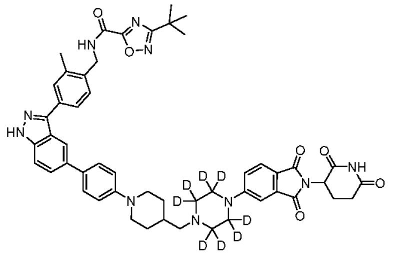 | 3-(tert-butyl)-N-(4-(5-(4-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide |
| | 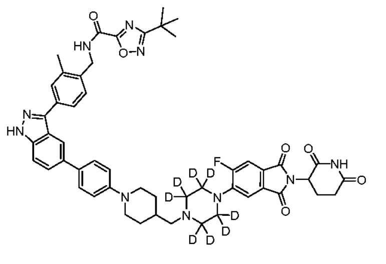 | 3-(tert-butyl)-N-(4-(5-(4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide |
| | 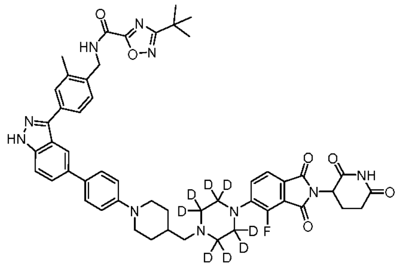 | 3-(tert-butyl)-N-(4-(5-(4-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide |
| | 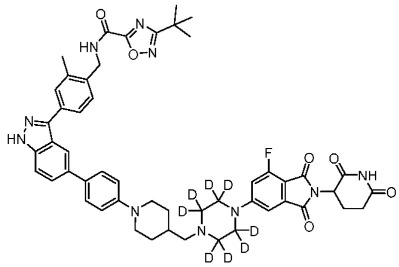 | 3-(tert-butyl)-N-(4-(5-(4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide |
| | 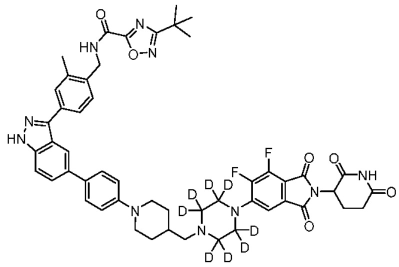 | 3-(tert-butyl)-N-(4-(5-(4-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4- | 3-(tert-butyl)-N-(4-(5-(4-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)phenyl)-1H-indazol-3- |

| | | | |
|---|---|---|---|
| | | oxadiazole-5-carboxamide | yl)-2-methylbenzyl)-1,2,4-oxadiazole-5-carboxamide |
| | 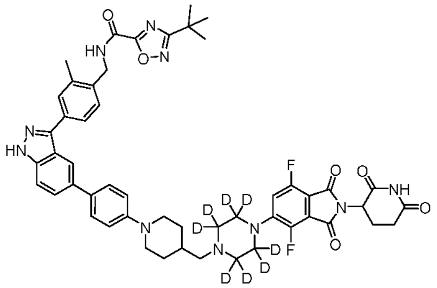 | 3-(tert-butyl)-N-(4-(5-(4-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)phenyl)-1H-indazol-3-yl)-2-methylbenzyl)-1,2,4-oxadiazole-5-carboxamide |
| | 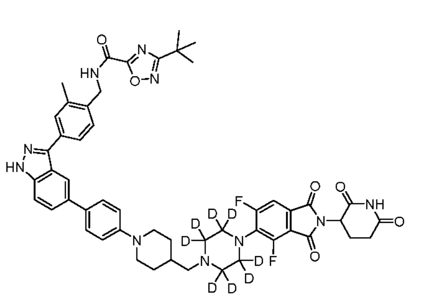 | 3-(tert-butyl)-N-(4-(5-(4-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)phenyl)-1H-indazol-3-yl)-2-methylbenzyl)-1,2,4-oxadiazole-5-carboxamide |
| | 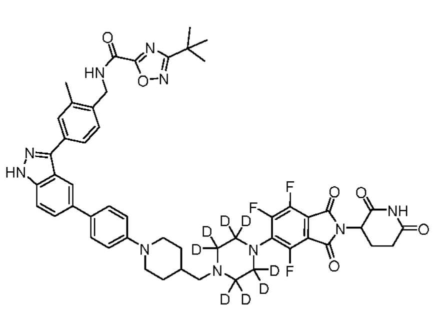 | 3-(tert-butyl)-N-(4-(5-(4-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*$_8$)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*$_8$)methyl)piperidin-1-yl)phenyl)-1H-indazol-3-yl)-2-methylbenzyl)-1,2,4-oxadiazole-5-carboxamide |
| | 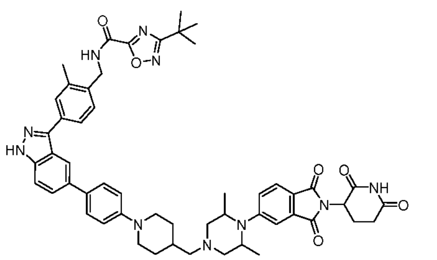 | 3-(tert-butyl)-N-(4-(5-(4-(4-((4-(2-(2,6-dioxopiperidin-3-yl)-1,3-dioxoisoindolin-5-yl)-3,5-dimethylpiperazin-1-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-1,3-dioxoisoindolin-5-yl)-3,5-dimethylpiperazin-1-yl)methyl)piperidin-1-yl)phenyl)-1H-indazol-3-yl)-2-methylbenzyl)-1,2,4-oxadiazole-5-carboxamide |
| | 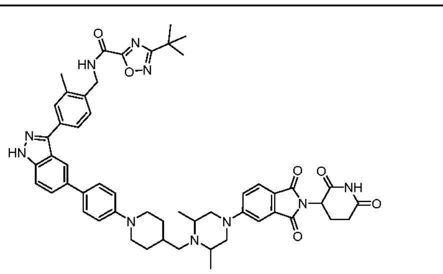 | 3-(tert-butyl)-N-(4-(5-(4-(4-((4-(2-(2,6-dioxopiperidin-3-yl)-1,3-dioxoisoindolin-5-yl)-2,6-dimethylpiperazin-1-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-1,3-dioxoisoindolin-5-yl)-2,6-dimethylpiperazin-1-yl)methyl)piperidin-1-yl)phenyl)-1H-indazol-3-yl)-2-methylbenzyl)-1,2,4-oxadiazole-5-carboxamide |

| | | | |
|---|---|---|---|
| | 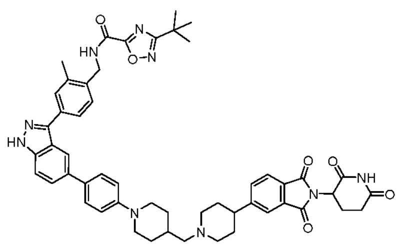 | 3-(tert-butyl)-N-(4-(5-(4-(4-((4-(2-(2,6-dioxopiperidin-3-yl)-1,3-dioxoisoindolin-5-yl)piperidin-1-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-1,3-dioxoisoindolin-5-yl)piperidin-1-yl)methyl)piperidin-1-yl)phenyl)-1H-indazol-3-yl)-2-methylbenzyl)-1,2,4-oxadiazole-5-carboxamide |
| | 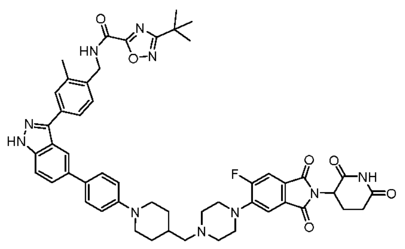 | 3-(tert-butyl)-N-(4-(5-(4-(4-((4-(2-(2,6-dioxopiperidin-3-yl)-6-fluoro-1,3-dioxoisoindolin-5-yl)piperazin-1-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-6-fluoro-1,3-dioxoisoindolin-5-yl)piperazin-1-yl)methyl)piperidin-1-yl)phenyl)-1H-indazol-3-yl)-2-methylbenzyl)-1,2,4-oxadiazole-5-carboxamide |
| | 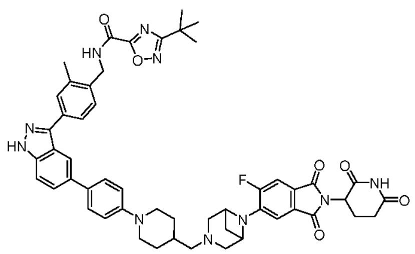 | 3-(tert-butyl)-N-(4-(5-(4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)phenyl)-1H-indazol-3-yl)-2-methylbenzyl)-1,2,4-oxadiazole-5-carboxamide |
| | 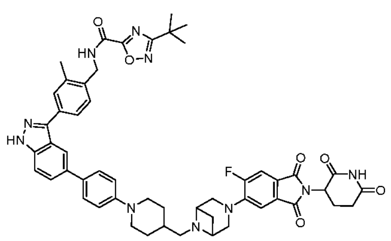 | 3-(tert-butyl)-N-(4-(5-(4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)phenyl)-1H-indazol-3-yl)-2-methylbenzyl)-1,2,4-oxadiazole-5-carboxamide |
| | 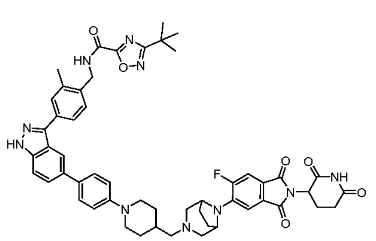 | 3-(tert-butyl)-N-(4-(5-(4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)phenyl)-1H-indazol-3-yl)-2-methylbenzyl)-1,2,4-oxadiazole-5-carboxamide |
| | 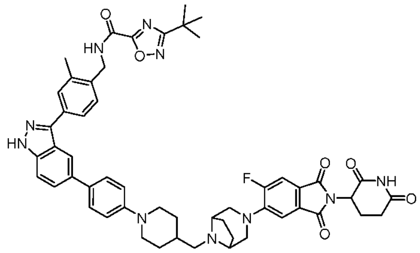 | 3-(tert-butyl)-N-(4-(5-(4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)phenyl)-1H-indazol-3-yl)-2-methylbenzyl)-1,2,4-oxadiazole-5-carboxamide |

| | | | |
|---|---|---|---|
| | 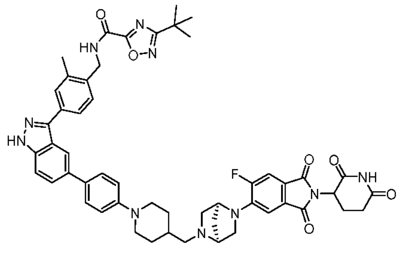 | 3-(tert-butyl)-N-(4-(5-(4-(4-(((1S,4S)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-(((1S,4S)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide |
| | 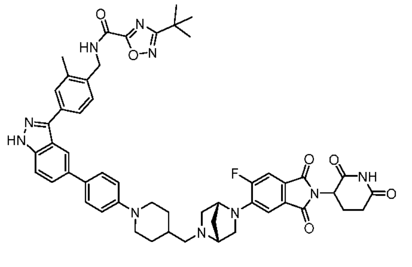 | 3-(tert-butyl)-N-(4-(5-(4-(4-(((1R,4R)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-(((1R,4R)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide |
| | 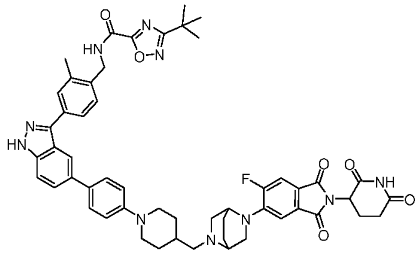 | 3-(tert-butyl)-N-(4-(5-(4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)phenyl)-1H-indazol-3-yl)-2-methylbenzyl)-1,2,4-oxadiazole-5-carboxamide |
| | 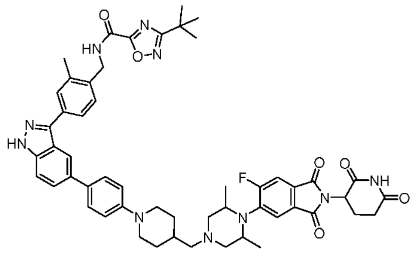 | 3-(tert-butyl)-N-(4-(5-(4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)phenyl)-1H-indazol-3-yl)-2-methylbenzyl)-1,2,4-oxadiazole-5-carboxamide |
| | 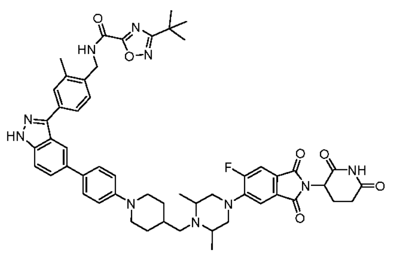 | 3-(tert-butyl)-N-(4-(5-(4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)phenyl)-1H-indazol-3-yl)-2-methylbenzyl)-1,2,4-oxadiazole-5-carboxamide |
| | 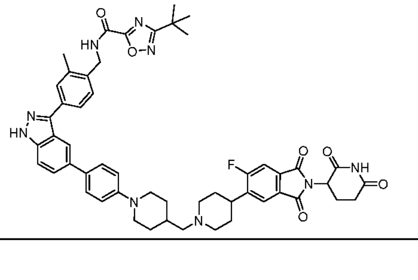 | 3-(tert-butyl)-N-(4-(5-(4-(4-((4-(2-(2,6-dioxopiperidin-3-yl)-6-fluoro-1,3-dioxoisoindolin-5-yl)piperidin-1-yl)methyl)piperidin-1-yl)phenyl)-1H-indazol-3-yl)-2-methylbenzyl)-1,2,4- | 3-(tert-butyl)-N-(4-(5-(4-(4-((4-(2-(2,6-dioxopiperidin-3-yl)-6-fluoro-1,3-dioxoisoindolin-5-yl)piperidin-1-yl)methyl)piperidin-1-yl)phenyl)-1H-indazol-3-yl)-2-methylbenzyl)-1,2,4- |

| | | | |
|---|---|---|---|
| | | oxadiazole-5-carboxamide | oxadiazole-5-carboxamide |
| | 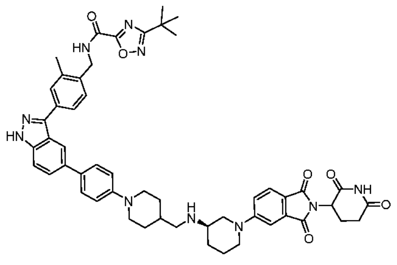 | 3-(tert-butyl)-N-(4-(5-(4-(4-((((3R)-1-(2-(2,6-dioxopiperidin-3-yl)-1,3-dioxoisoindolin-5-yl)piperidin-3-yl)amino)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((((3R)-1-(2-(2,6-dioxopiperidin-3-yl)-1,3-dioxoisoindolin-5-yl)piperidin-3-yl)amino)methyl)piperidin-1-yl)phenyl)-1H-indazol-3-yl)-2-methylbenzyl)-1,2,4-oxadiazole-5-carboxamide |
| | 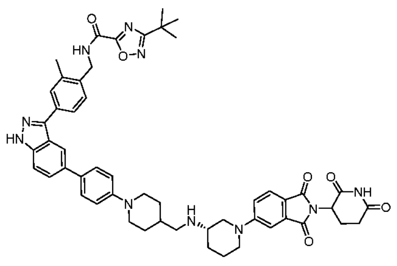 | 3-(tert-butyl)-N-(4-(5-(4-(4-((((3S)-1-(2-(2,6-dioxopiperidin-3-yl)-1,3-dioxoisoindolin-5-yl)piperidin-3-yl)amino)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-((((3S)-1-(2-(2,6-dioxopiperidin-3-yl)-1,3-dioxoisoindolin-5-yl)piperidin-3-yl)amino)methyl)piperidin-1-yl)phenyl)-1H-indazol-3-yl)-2-methylbenzyl)-1,2,4-oxadiazole-5-carboxamide |
| | 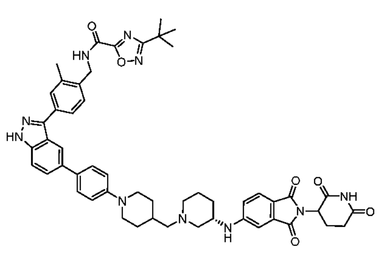 | 3-(tert-butyl)-N-(4-(5-(4-(4-(((3S)-3-((2-(2,6-dioxopiperidin-3-yl)-1,3-dioxoisoindolin-5-yl)amino)piperidin-1-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-(((3S)-3-((2-(2,6-dioxopiperidin-3-yl)-1,3-dioxoisoindolin-5-yl)amino)piperidin-1-yl)methyl)piperidin-1-yl)phenyl)-1H-indazol-3-yl)-2-methylbenzyl)-1,2,4-oxadiazole-5-carboxamide |
| | 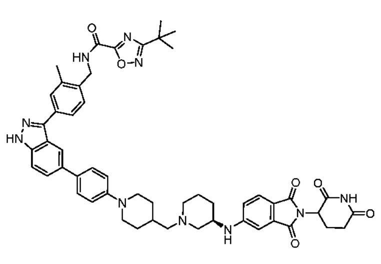 | 3-(tert-butyl)-N-(4-(5-(4-(4-(((3R)-3-((2-(2,6-dioxopiperidin-3-yl)-1,3-dioxoisoindolin-5-yl)amino)piperidin-1-yl)methyl)piperidin-1-yl)phenyl)-1H-indazol-3-yl)-2-methylbenzyl)-1,2,4-oxadiazole-5-carboxamide | 3-(tert-butyl)-N-(4-(5-(4-(4-(((3R)-3-((2-(2,6-dioxopiperidin-3-yl)-1,3-dioxoisoindolin-5-yl)amino)piperidin-1-yl)methyl)piperidin-1-yl)phenyl)-1H-indazol-3-yl)-2-methylbenzyl)-1,2,4-oxadiazole-5-carboxamide |
| | 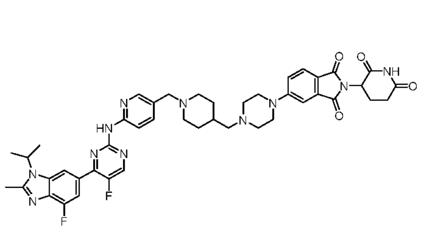 | 2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl)isoindoline-1,3-dione | 2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl)isoindoline-1,3-dione |
| | 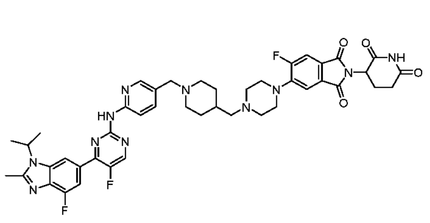 | 2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl)isoindoline-1,3-dione | 2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl)isoindoline-1,3-dione |

| | | | |
|---|---|---|---|
| | 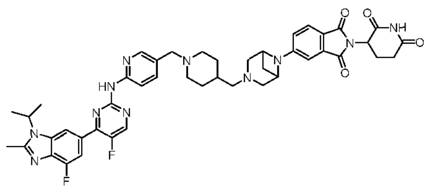 | 2-(2,6-dioxopiperidin-3-yl)-5-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-6-yl)isoindoline-1,3-dione | 2-(2,6-dioxopiperidin-3-yl)-5-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-6-yl)isoindoline-1,3-dione |
| | 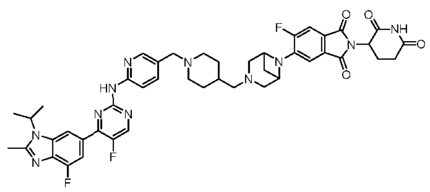 | 2-(2,6-dioxopiperidin-3-yl)-5-fluoro-6-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-6-yl)isoindoline-1,3-dione | 2-(2,6-dioxopiperidin-3-yl)-5-fluoro-6-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-6-yl)isoindoline-1,3-dione |
| | 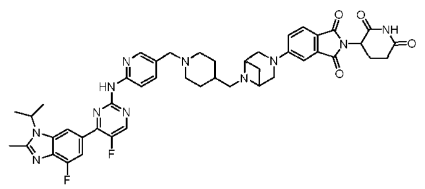 | 2-(2,6-dioxopiperidin-3-yl)-5-(6-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-3-yl)isoindoline-1,3-dione | 2-(2,6-dioxopiperidin-3-yl)-5-(6-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-3-yl)isoindoline-1,3-dione |
| | 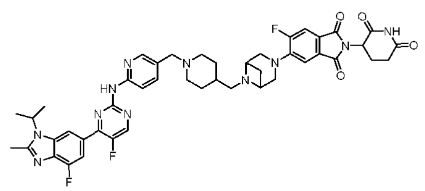 | 2-(2,6-dioxopiperidin-3-yl)-5-fluoro-6-(6-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-3-yl)isoindoline-1,3-dione | 2-(2,6-dioxopiperidin-3-yl)-5-fluoro-6-(6-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-3-yl)isoindoline-1,3-dione |
| | 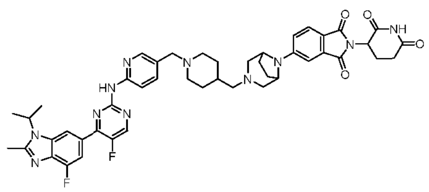 | 2-(2,6-dioxopiperidin-3-yl)-5-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-8-yl)isoindoline-1,3-dione | 2-(2,6-dioxopiperidin-3-yl)-5-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-8-yl)isoindoline-1,3-dione |

| | | | |
|---|---|---|---|
| | 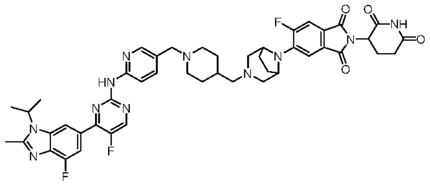 | 2-(2,6-dioxopiperidin-3-yl)-5-fluoro-6-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-8-yl)isoindoline-1,3-dione | 2-(2,6-dioxopiperidin-3-yl)-5-fluoro-6-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-8-yl)isoindoline-1,3-dione |
| | 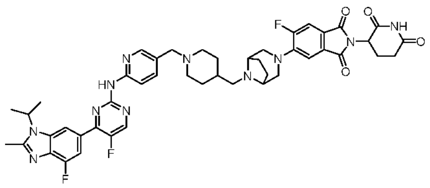 | 2-(2,6-dioxopiperidin-3-yl)-5-fluoro-6-(8-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-3-yl)isoindoline-1,3-dione | 2-(2,6-dioxopiperidin-3-yl)-5-fluoro-6-(8-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-3-yl)isoindoline-1,3-dione |
| | 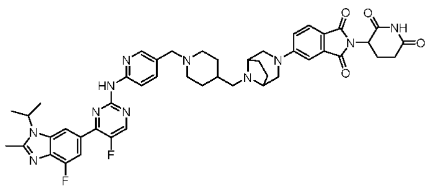 | 2-(2,6-dioxopiperidin-3-yl)-5-(8-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-3-yl)isoindoline-1,3-dione | 2-(2,6-dioxopiperidin-3-yl)-5-(8-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-3-yl)isoindoline-1,3-dione |
| | 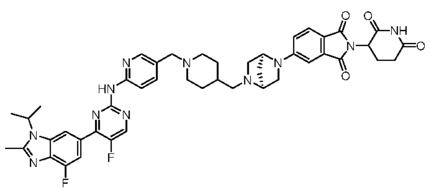 | 2-(2,6-dioxopiperidin-3-yl)-5-((1S,4S)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione | 2-(2,6-dioxopiperidin-3-yl)-5-((1S,4S)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione |
| | 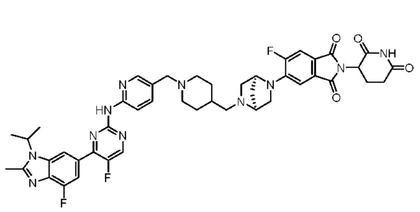 | 2-(2,6-dioxopiperidin-3-yl)-5-fluoro-6-((1S,4S)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione | 2-(2,6-dioxopiperidin-3-yl)-5-fluoro-6-((1S,4S)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione |

| | | | |
|---|---|---|---|
| | 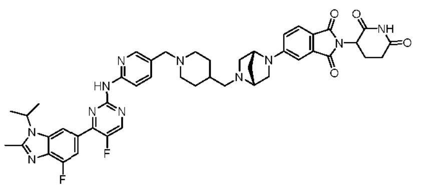 | 2-(2,6-dioxopiperidin-3-yl)-5-((1R,4R)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione | 2-(2,6-dioxopiperidin-3-yl)-5-((1R,4R)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione |
| | 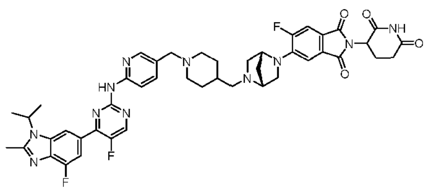 | 2-(2,6-dioxopiperidin-3-yl)-5-fluoro-6-((1R,4R)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione | 2-(2,6-dioxopiperidin-3-yl)-5-fluoro-6-((1R,4R)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione |
| | 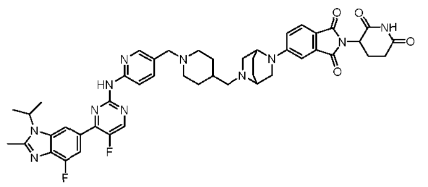 | 2-(2,6-dioxopiperidin-3-yl)-5-(5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.2]octan-2-yl)isoindoline-1,3-dione | 2-(2,6-dioxopiperidin-3-yl)-5-(5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.2]octan-2-yl)isoindoline-1,3-dione |
| | 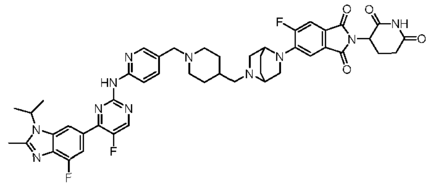 | 2-(2,6-dioxopiperidin-3-yl)-5-fluoro-6-(5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.2]octan-2-yl)isoindoline-1,3-dione | 2-(2,6-dioxopiperidin-3-yl)-5-fluoro-6-(5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.2]octan-2-yl)isoindoline-1,3-dione |
| | 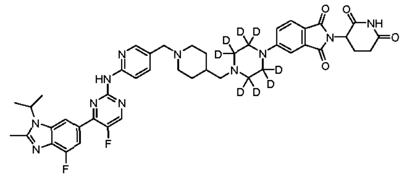 | 2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-*d*$_8$)isoindoline-1,3-dione | 2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-*d*$_8$)isoindoline-1,3-dione |

| | | | |
|---|---|---|---|
| | 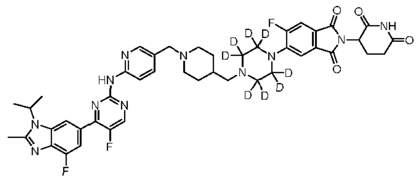 | 2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione |
| | 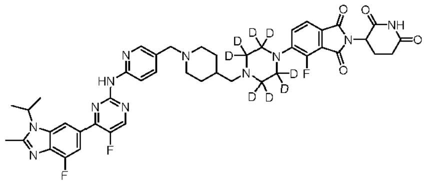 | 2-(2,6-dioxopiperidin-3-yl)-4-fluoro-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione |
| | 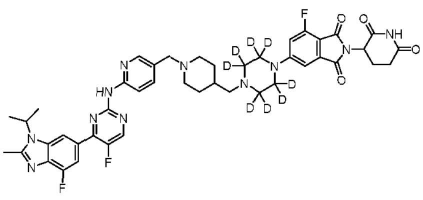 | 2-(2,6-dioxopiperidin-3-yl)-4-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione |
| | 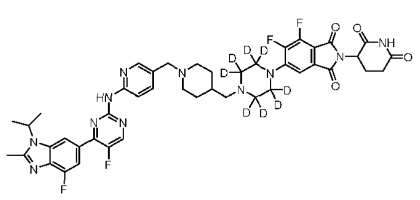 | 2-(2,6-dioxopiperidin-3-yl)-4,5-difluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-4,5-difluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione |
| | 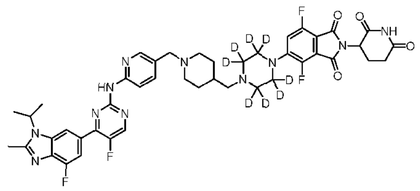 | 2-(2,6-dioxopiperidin-3-yl)-4,7-difluoro-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-4,7-difluoro-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione |

| | | | |
|---|---|---|---|
| | 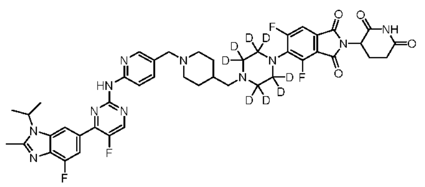 | 2-(2,6-dioxopiperidin-3-yl)-4,6-difluoro-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-*d*8)isoindoline-1,3-dione | 2-(2,6-dioxopiperidin-3-yl)-4,6-difluoro-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-*d*8)isoindoline-1,3-dione |
| | 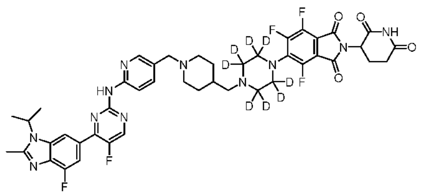 | 2-(2,6-dioxopiperidin-3-yl)-4,5,7-trifluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-*d*8)isoindoline-1,3-dione | 2-(2,6-dioxopiperidin-3-yl)-4,5,7-trifluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-*d*8)isoindoline-1,3-dione |
| | 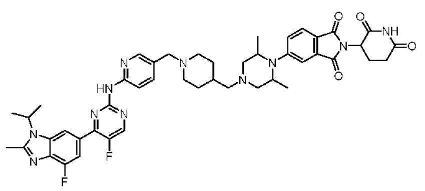 | 2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,6-dimethylpiperazin-1-yl)isoindoline-1,3-dione | 2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,6-dimethylpiperazin-1-yl)isoindoline-1,3-dione |
| | 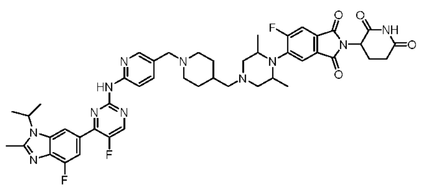 | 2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,6-dimethylpiperazin-1-yl)isoindoline-1,3-dione | 2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,6-dimethylpiperazin-1-yl)isoindoline-1,3-dione |
| | 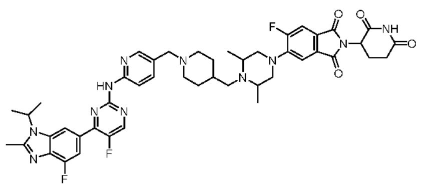 | 2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,5-dimethylpiperazin-1-yl)isoindoline-1,3-dione | 2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,5-dimethylpiperazin-1-yl)isoindoline-1,3-dione |

| | | | |
|---|---|---|---|
| | 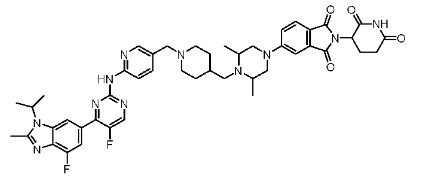 | 2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,5-dimethylpiperazin-1-yl)isoindoline-1,3-dione | 2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,5-dimethylpiperazin-1-yl)isoindoline-1,3-dione |
| | 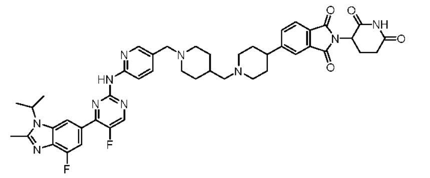 | 2-(2,6-dioxopiperidin-3-yl)-5-(1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperidin-4-yl)isoindoline-1,3-dione | 2-(2,6-dioxopiperidin-3-yl)-5-(1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperidin-4-yl)isoindoline-1,3-dione |
| | 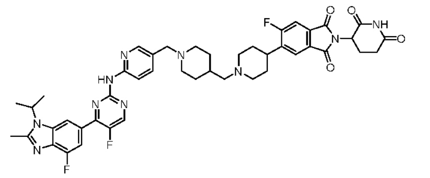 | 2-(2,6-dioxopiperidin-3-yl)-5-fluoro-6-(1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperidin-4-yl)isoindoline-1,3-dione | 2-(2,6-dioxopiperidin-3-yl)-5-fluoro-6-(1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperidin-4-yl)isoindoline-1,3-dione |
| | 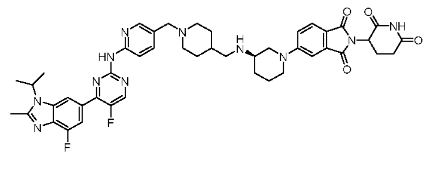 | 2-(2,6-dioxopiperidin-3-yl)-5-((R)-3-(((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)amino)piperidin-1-yl)isoindoline-1,3-dione | 2-(2,6-dioxopiperidin-3-yl)-5-((R)-3-(((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)amino)piperidin-1-yl)isoindoline-1,3-dione |
| | 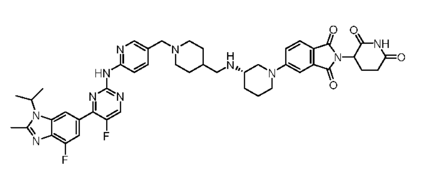 | 2-(2,6-dioxopiperidin-3-yl)-5-((S)-3-(((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)amino)piperidin-1-yl)isoindoline-1,3-dione | 2-(2,6-dioxopiperidin-3-yl)-5-((S)-3-(((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)amino)piperidin-1-yl)isoindoline-1,3-dione |
| | 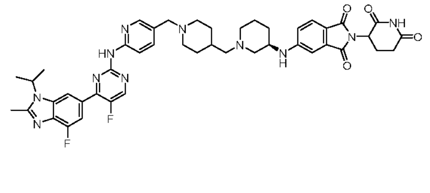 | 2-(2,6-dioxopiperidin-3-yl)-5-(((R)-1-((1-((6-((5-fluoro-4-(4-fluoro-1-isopropyl-2-methyl-1H-benzo[d]imidazol-6-yl)pyrimidin-2-yl)amino)pyridin-3- | 2-(2,6-dioxopiperidin-3-yl)-5-(((R)-1-((1-((6-((5-fluoro-4-(4-fluoro-1-isopropyl-2-methyl-1H-benzo[d]imidazol-6-yl)pyrimidin-2-yl)amino)pyridin-3- |

| | | | |
|---|---|---|---|
| | | yl)methyl)piperidin-4-yl)methyl)piperidin-3-yl)amino)isoindoline-1,3-dione | yl)methyl)piperidin-4-yl)methyl)piperidin-3-yl)amino)isoindoline-1,3-dione |
| | 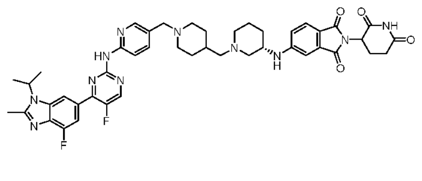 | 2-(2,6-dioxopiperidin-3-yl)-5-(((S)-1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperidin-3-yl)amino)isoindoline-1,3-dione | 2-(2,6-dioxopiperidin-3-yl)-5-(((S)-1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperidin-3-yl)amino)isoindoline-1,3-dione |
| GT-03660 | 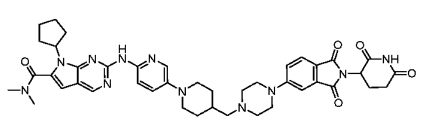 | 7-cyclopentyl-2-((5-(4-((4-(2-(2,6-dioxopiperidin-3-yl)-1,3-dioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-1,3-dioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide |
| GT-03656 | 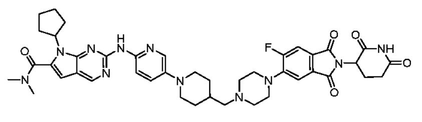 | 7-cyclopentyl-2-((5-(4-((4-(2-(2,6-dioxopiperidin-3-yl)-6-fluoro-1,3-dioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-6-fluoro-1,3-dioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide |
| GT-03654 | 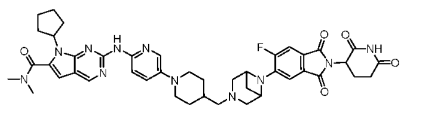 | 7-cyclopentyl-2-((5-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridin-2-yl)amino)-N,N-dimethyl-7H-pyrrolo[2,3-d]pyrimidine-6-carboxamide |
| GT-03659 | 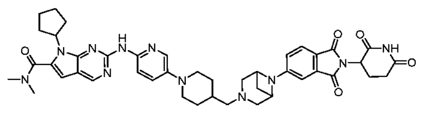 | 7-cyclopentyl-2-((5-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridin-2-yl)amino)-N,N-dimethyl-7H-pyrrolo[2,3-d]pyrimidine-6-carboxamide |
| GT-03661 | 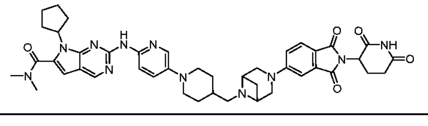 | 7-cyclopentyl-2-((5-(4-((3-(2-(2,6-dioxopiperidin-3-yl)-1,3-dioxoisoindolin-5-yl)-3,6- | 7-cyclopentyl-2-((5-(4-((3-(2-(2,6-dioxopiperidin-3-yl)-1,3-dioxoisoindolin-5-yl)-3,6- |

| | | | |
|---|---|---|---|
| | | diazabicyclo[3.1.1]heptan-6-yl)methyl)piperidin-1-yl)pyridin-2-yl)amino)-N,N-dimethyl-7H-pyrrolo[2,3-d]pyrimidine-6-carboxamide | diazabicyclo[3.1.1]heptan-6-yl)methyl)piperidin-1-yl)pyridin-2-yl)amino)-N,N-dimethyl-7H-pyrrolo[2,3-d]pyrimidine-6-carboxamide |
| GT-03657 | 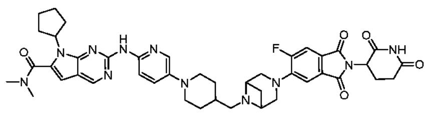 | 7-cyclopentyl-2-((5-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridin-2-yl)amino)-N,N-dimethyl-7H-pyrrolo[2,3-d]pyrimidine-6-carboxamide |
| GT-03655 | 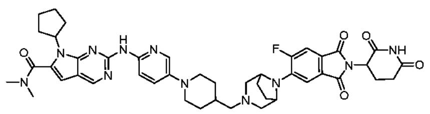 | 7-cyclopentyl-2-((5-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3-d]pyrimidine-6-carboxamide |
| GT-03651 | 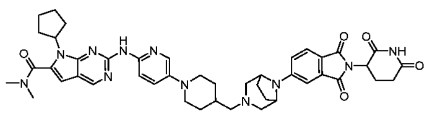 | 7-cyclopentyl-2-((5-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3-d]pyrimidine-6-carboxamide |
| GT-03652 | 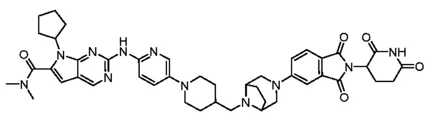 | 7-cyclopentyl-2-((5-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridin-2-yl)amino)-N,N-dimethyl-7H-pyrrolo[2,3-d]pyrimidine-6-carboxamide |
| GT-03658 | 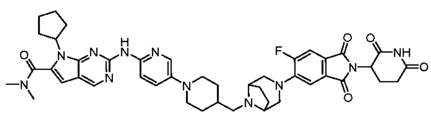 | 7-cyclopentyl-2-((5-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridin-2-yl)amino)-N,N-dimethyl-7H-pyrrolo[2,3-d]pyrimidine-6-carboxamide |

| | | | |
|---|---|---|---|
| | 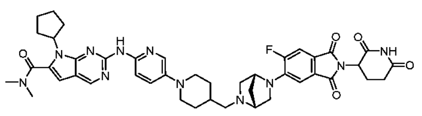 | 7-cyclopentyl-2-((5-(4-(((1R,4R)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-(((1R,4R)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide |
| | 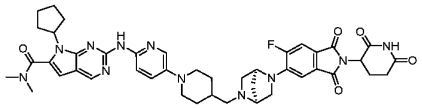 | 7-cyclopentyl-2-((5-(4-(((1S,4S)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-(((1S,4S)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide |
| | 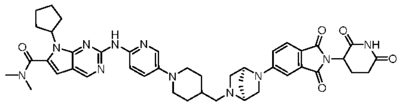 | 7-cyclopentyl-2-((5-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide |
| | 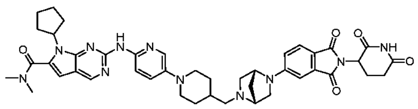 | 7-cyclopentyl-2-((5-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3-d]pyrimidine-6-carboxamide |
| | 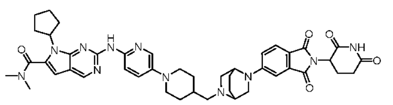 | 7-cyclopentyl-2-((5-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)pyridin-2-yl)amino)-N,N-dimethyl-7H-pyrrolo[2,3-d]pyrimidine-6-carboxamide |
| | 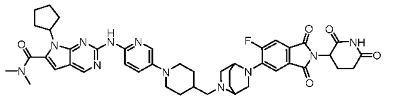 | 7-cyclopentyl-2-((5-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1- | 7-cyclopentyl-2-((5-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1- |

| | | | |
|---|---|---|---|
| | | yl)pyridin-2-yl)amino)-N,N-dimethyl-7H-pyrrolo[2,3-d]pyrimidine-6-carboxamide | yl)pyridin-2-yl)amino)-N,N-dimethyl-7H-pyrrolo[2,3-d]pyrimidine-6-carboxamide |
| | 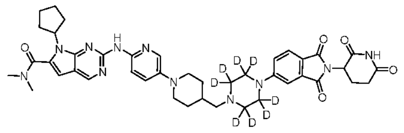 | 7-cyclopentyl-2-((5-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide |
| | 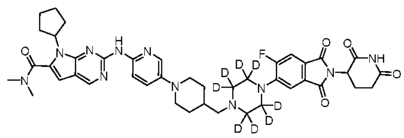 | 7-cyclopentyl-2-((5-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide |
| | 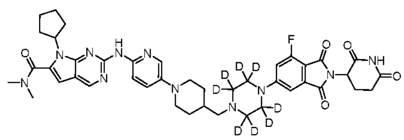 | 7-cyclopentyl-2-((5-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide |
| | 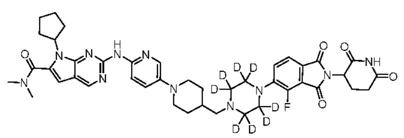 | 7-cyclopentyl-2-((5-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide |
| | 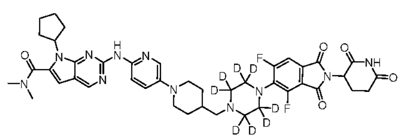 | 7-cyclopentyl-2-((5-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide |

| | | | |
|---|---|---|---|
| | 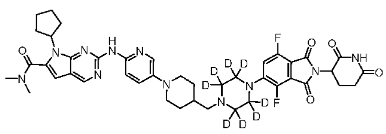 | 7-cyclopentyl-2-((5-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide |
| | 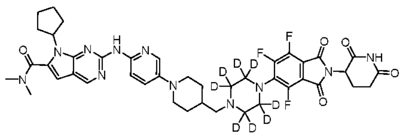 | 7-cyclopentyl-2-((5-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide |
| | 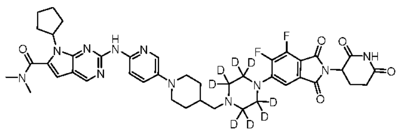 | 7-cyclopentyl-2-((5-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide |
| | 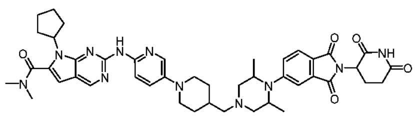 | 7-cyclopentyl-2-((5-(4-((4-(2-(2,6-dioxopiperidin-3-yl)-1,3-dioxoisoindolin-5-yl)-3,5-dimethylpiperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-1,3-dioxoisoindolin-5-yl)-3,5-dimethylpiperazin-1-yl)methyl)piperidin-1-yl)pyridin-2-yl)amino)-N,N-dimethyl-7H-pyrrolo[2,3-d]pyrimidine-6-carboxamide |
| | 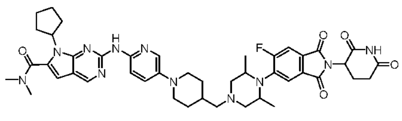 | 7-cyclopentyl-2-((5-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)pyridin-2-yl)amino)-N,N-dimethyl-7H-pyrrolo[2,3-d]pyrimidine-6-carboxamide |
| | 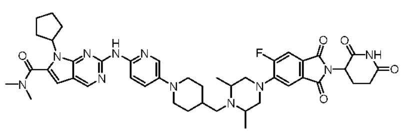 | 7-cyclopentyl-2-((5-(4-((4-(2-(2,6-dioxopiperidin-3-yl)-6-fluoro-1,3-dioxoisoindolin-5-yl)-2,6-dimethylpiperazin-1-yl)methyl)piperidin-1- | 7-cyclopentyl-2-((5-(4-((4-(2-(2,6-dioxopiperidin-3-yl)-6-fluoro-1,3-dioxoisoindolin-5-yl)-2,6-dimethylpiperazin-1-yl)methyl)piperidin-1- |

| | | | |
|---|---|---|---|
| | | yl)pyridin-2-yl)amino)-N,N-dimethyl-7H-pyrrolo[2,3-d]pyrimidine-6-carboxamide | yl)pyridin-2-yl)amino)-N,N-dimethyl-7H-pyrrolo[2,3-d]pyrimidine-6-carboxamide |
| | 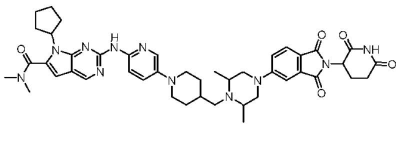 | 7-cyclopentyl-2-((5-(4-((4-(2-(2,6-dioxopiperidin-3-yl)-1,3-dioxoisoindolin-5-yl)-2,6-dimethylpiperazin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-1,3-dioxoisoindolin-5-yl)-2,6-dimethylpiperazin-1-yl)methyl)piperidin-1-yl)pyridin-2-yl)amino)-N,N-dimethyl-7H-pyrrolo[2,3-d]pyrimidine-6-carboxamide |
| | 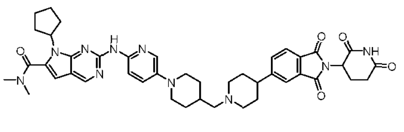 | 7-cyclopentyl-2-((5-(4-((4-(2-(2,6-dioxopiperidin-3-yl)-1,3-dioxoisoindolin-5-yl)piperidin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-1,3-dioxoisoindolin-5-yl)piperidin-1-yl)methyl)piperidin-1-yl)pyridin-2-yl)amino)-N,N-dimethyl-7H-pyrrolo[2,3-d]pyrimidine-6-carboxamide |
| | 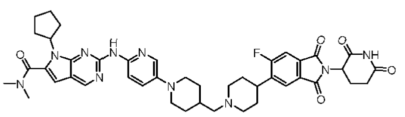 | 7-cyclopentyl-2-((5-(4-((4-(2-(2,6-dioxopiperidin-3-yl)-6-fluoro-1,3-dioxoisoindolin-5-yl)piperidin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-(2-(2,6-dioxopiperidin-3-yl)-6-fluoro-1,3-dioxoisoindolin-5-yl)piperidin-1-yl)methyl)piperidin-1-yl)pyridin-2-yl)amino)-N,N-dimethyl-7H-pyrrolo[2,3-d]pyrimidine-6-carboxamide |
| | 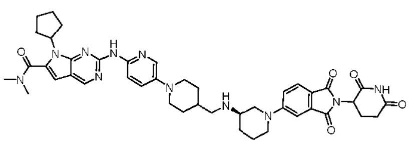 | 7-cyclopentyl-2-((5-(4-((((3R)-1-(2-(2,6-dioxopiperidin-3-yl)-1,3-dioxoisoindolin-5-yl)piperidin-3-yl)amino)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((((3R)-1-(2-(2,6-dioxopiperidin-3-yl)-1,3-dioxoisoindolin-5-yl)piperidin-3-yl)amino)methyl)piperidin-1-yl)pyridin-2-yl)amino)-N,N-dimethyl-7H-pyrrolo[2,3-d]pyrimidine-6-carboxamide |
| | 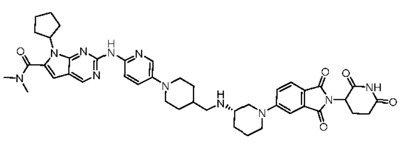 | 7-cyclopentyl-2-((5-(4-((((3S)-1-(2-(2,6-dioxopiperidin-3-yl)-1,3-dioxoisoindolin-5-yl)piperidin-3-yl)amino)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((((3S)-1-(2-(2,6-dioxopiperidin-3-yl)-1,3-dioxoisoindolin-5-yl)piperidin-3-yl)amino)methyl)piperidin-1-yl)pyridin-2-yl)amino)-N,N-dimethyl-7H-pyrrolo[2,3-d]pyrimidine-6-carboxamide |
| | 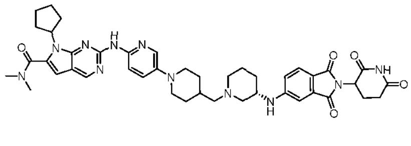 | 7-cyclopentyl-2-((5-(4-(((3S)-3-((2-(2,6-dioxopiperidin-3-yl)-1,3-dioxoisoindolin-5-yl)amino)piperidin-1-yl)methyl)piperidin-1- | 7-cyclopentyl-2-((5-(4-(((3S)-3-((2-(2,6-dioxopiperidin-3-yl)-1,3-dioxoisoindolin-5-yl)amino)piperidin-1-yl)methyl)piperidin-1- |

| | | | |
|---|---|---|---|
| | | yl)pyridin-2-yl)amino)-N,N-dimethyl-7H-pyrrolo[2,3-d]pyrimidine-6-carboxamide | yl)pyridin-2-yl)amino)-N,N-dimethyl-7H-pyrrolo[2,3-d]pyrimidine-6-carboxamide |
| | 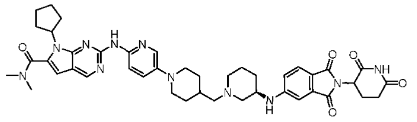 | 7-cyclopentyl-2-((5-(4-(((3R)-3-((2-(2,6-dioxopiperidin-3-yl)-1,3-dioxoisoindolin-5-yl)amino)piperidin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-(((3R)-3-((2-(2,6-dioxopiperidin-3-yl)-1,3-dioxoisoindolin-5-yl)amino)piperidin-1-yl)methyl)piperidin-1-yl)pyridin-2-yl)amino)-N,N-dimethyl-7H-pyrrolo[2,3-d]pyrimidine-6-carboxamide |
| | 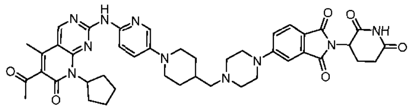 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl)-2-(2,6-dioxopiperidin-3-yl)isoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl)-2-(2,6-dioxopiperidin-3-yl)isoindoline-1,3-dione |
| | 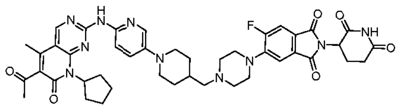 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl)-2-(2,6-dioxopiperidin-3-yl)-6-fluoroisoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl)-2-(2,6-dioxopiperidin-3-yl)-6-fluoroisoindoline-1,3-dione |
| | 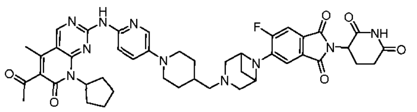 | 5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 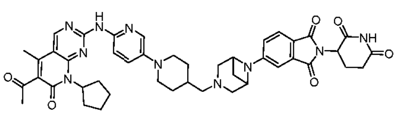 | 5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-6-yl)-2-(2,6-dioxopiperidin-3-yl)isoindoline-1,3-dione | 5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-6-yl)-2-(2,6-dioxopiperidin-3-yl)isoindoline-1,3-dione |
| | 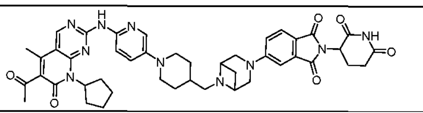 | 5-(6-((1-(6-((6-acetyl-8-cyclopentyl-5-methyl-7-oxo-7,8-dihydropyrido[2,3- | 5-(6-((1-(6-((6-acetyl-8-cyclopentyl-5-methyl-7-oxo-7,8-dihydropyrido[2,3- |

| | | | |
|---|---|---|---|
| | | d]pyrimidin-2-yl)amino)pyridin-3-yl)piperidin-4-yl)methyl)-3,6-diazabicyclo[3.1.1]heptan-3-yl)-2-(2,6-dioxopiperidin-3-yl)isoindoline-1,3-dione | d]pyrimidin-2-yl)amino)pyridin-3-yl)piperidin-4-yl)methyl)-3,6-diazabicyclo[3.1.1]heptan-3-yl)-2-(2,6-dioxopiperidin-3-yl)isoindoline-1,3-dione |
| | 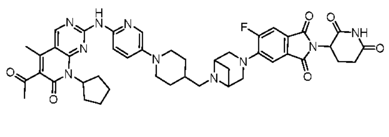 | 5-(6-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-3-yl)-2-(2,6-dioxopiperidin-3-yl)-6-fluoroisoindoline-1,3-dione | 5-(6-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-3-yl)-2-(2,6-dioxopiperidin-3-yl)-6-fluoroisoindoline-1,3-dione |
| | 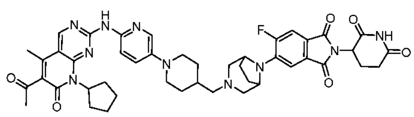 | 5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-8-yl)-2-(2,6-dioxopiperidin-3-yl)-6-fluoroisoindoline-1,3-dione |
| | 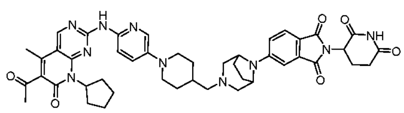 | 5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-8-yl)-2-(2,6-dioxopiperidin-3-yl)isoindoline-1,3-dione | 5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-8-yl)-2-(2,6-dioxopiperidin-3-yl)isoindoline-1,3-dione |
| | 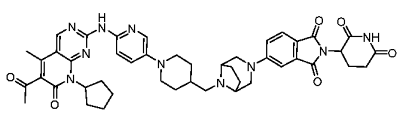 | 5-(8-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-3-yl)-2-(2,6-dioxopiperidin-3-yl)isoindoline-1,3-dione | 5-(8-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-3-yl)-2-(2,6-dioxopiperidin-3-yl)isoindoline-1,3-dione |
| | 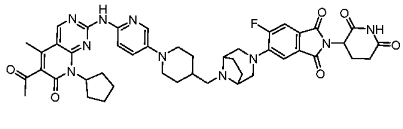 | 5-(8-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-3- | 5-(8-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-3- |

| | | | |
|---|---|---|---|
| | | yl)-2-(2,6-dioxopiperidin-3-yl)-6-fluoroisoindoline-1,3-dione | yl)-2-(2,6-dioxopiperidin-3-yl)-6-fluoroisoindoline-1,3-dione |
| | 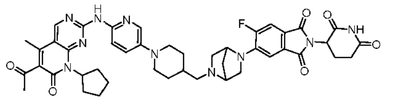 | 5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 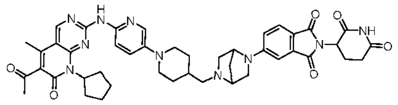 | 5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.1]heptan-2-yl)-2-(2,6-dioxopiperidin-3-yl)isoindoline-1,3-dione | 5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.1]heptan-2-yl)-2-(2,6-dioxopiperidin-3-yl)isoindoline-1,3-dione |
| | 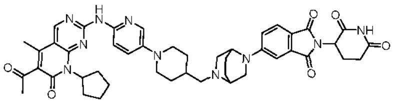 | 5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.2]octan-2-yl)-2-(2,6-dioxopiperidin-3-yl)isoindoline-1,3-dione | 5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.2]octan-2-yl)-2-(2,6-dioxopiperidin-3-yl)isoindoline-1,3-dione |
| | 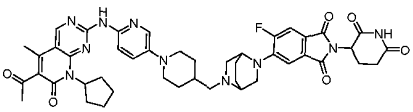 | 5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.2]octan-2-yl)-2-(2,6-dioxopiperidin-3-yl)-6-fluoroisoindoline-1,3-dione |
| | 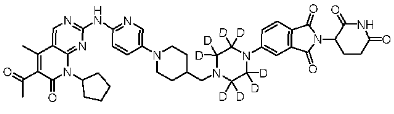 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)isoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)isoindoline-1,3-dione |

| | | | |
|---|---|---|---|
| | 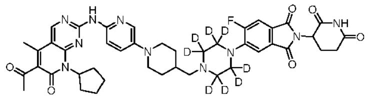 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-6-fluoroisoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-6-fluoroisoindoline-1,3-dione |
| | 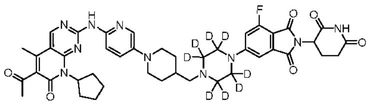 | 6-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 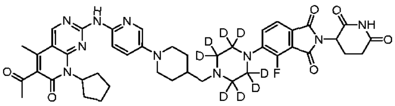 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 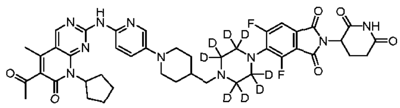 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |
| | 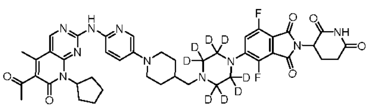 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione |
| | 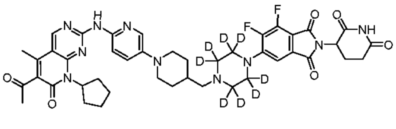 | 6-(4-((1-(6-((6-acetyl-8-cyclopentyl-5-methyl-7-oxo-7,8-dihydropyrido[2,3-d]pyrimidin-2-yl)amino)pyridin-3-yl)piperidin-4- | 6-(4-((1-(6-((6-acetyl-8-cyclopentyl-5-methyl-7-oxo-7,8-dihydropyrido[2,3-d]pyrimidin-2-yl)amino)pyridin-3-yl)piperidin-4- |

| | | | |
|---|---|---|---|
| | | yl)methyl)piperazin-1-yl-2,2,3,3,5,5,6,6-*d*8)-2-(2,6-dioxopiperidin-3-yl)-4,5-difluoroisoindoline-1,3-dione | yl)methyl)piperazin-1-yl-2,2,3,3,5,5,6,6-*d*8)-2-(2,6-dioxopiperidin-3-yl)-4,5-difluoroisoindoline-1,3-dione |
| | 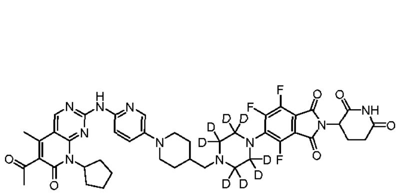 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-*d*8)-2-(2,6-dioxopiperidin-3-yl)-4,6,7-trifluoroisoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-*d*8)-2-(2,6-dioxopiperidin-3-yl)-4,6,7-trifluoroisoindoline-1,3-dione |
| | 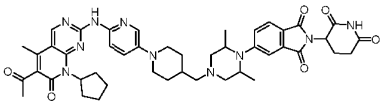 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,6-dimethylpiperazin-1-yl)-2-(2,6-dioxopiperidin-3-yl)isoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,6-dimethylpiperazin-1-yl)-2-(2,6-dioxopiperidin-3-yl)isoindoline-1,3-dione |
| | 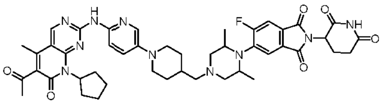 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,6-dimethylpiperazin-1-yl)-2-(2,6-dioxopiperidin-3-yl)-6-fluoroisoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,6-dimethylpiperazin-1-yl)-2-(2,6-dioxopiperidin-3-yl)-6-fluoroisoindoline-1,3-dione |
| | 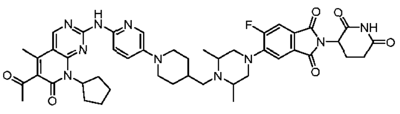 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,5-dimethylpiperazin-1-yl)-2-(2,6-dioxopiperidin-3-yl)-6-fluoroisoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,5-dimethylpiperazin-1-yl)-2-(2,6-dioxopiperidin-3-yl)-6-fluoroisoindoline-1,3-dione |
| | 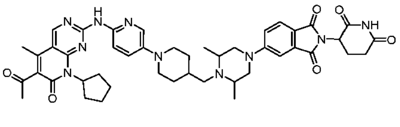 | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,5-dimethylpiperazin-1-yl)-2-(2,6-dioxopiperidin-3-yl)isoindoline-1,3-dione | 5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,5-dimethylpiperazin-1-yl)-2-(2,6-dioxopiperidin-3-yl)isoindoline-1,3-dione |
| | 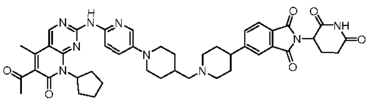 | 5-(1-((1-(6-((6-acetyl-8-cyclopentyl-5-methyl-7-oxo-7,8-dihydropyrido[2,3-d]pyrimidin-2- | 5-(1-((1-(6-((6-acetyl-8-cyclopentyl-5-methyl-7-oxo-7,8-dihydropyrido[2,3-d]pyrimidin-2- |

| | | | |
|---|---|---|---|
| | | yl)amino)pyridin-3-yl)piperidin-4-yl)methyl)piperidin-4-yl)-2-(2,6-dioxopiperidin-3-yl)isoindoline-1,3-dione | yl)amino)pyridin-3-yl)piperidin-4-yl)methyl)piperidin-4-yl)-2-(2,6-dioxopiperidin-3-yl)isoindoline-1,3-dione |
| | 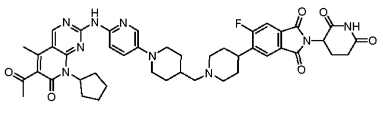 | 5-(1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-4-yl)-2-(2,6-dioxopiperidin-3-yl)-6-fluoroisoindoline-1,3-dione | 5-(1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-4-yl)-2-(2,6-dioxopiperidin-3-yl)-6-fluoroisoindoline-1,3-dione |
| | 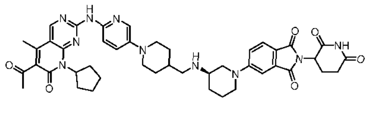 | 5-((R)-3-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)amino)piperidin-1-yl)-2-(2,6-dioxopiperidin-3-yl)isoindoline-1,3-dione | 5-((R)-3-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)amino)piperidin-1-yl)-2-(2,6-dioxopiperidin-3-yl)isoindoline-1,3-dione |
| | 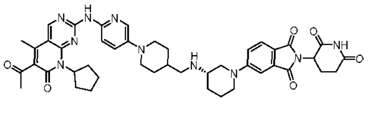 | 5-((S)-3-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)amino)piperidin-1-yl)-2-(2,6-dioxopiperidin-3-yl)isoindoline-1,3-dione | 5-((S)-3-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)amino)piperidin-1-yl)-2-(2,6-dioxopiperidin-3-yl)isoindoline-1,3-dione |
| | 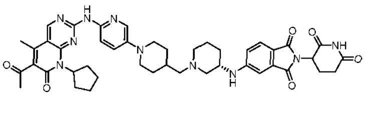 | 5-(((S)-1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-3-yl)amino)-2-(2,6-dioxopiperidin-3-yl)isoindoline-1,3-dione | 5-(((S)-1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-3-yl)amino)-2-(2,6-dioxopiperidin-3-yl)isoindoline-1,3-dione |
| | 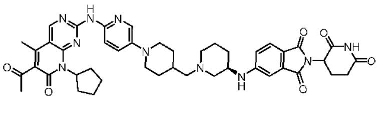 | 5-(((R)-1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-3-yl)amino)-2-(2,6-dioxopiperidin-3-yl)isoindoline-1,3-dione | 5-(((R)-1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-3-yl)amino)-2-(2,6-dioxopiperidin-3-yl)isoindoline-1,3-dione |
| | 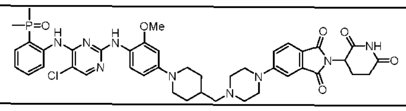 | 5-(4-((1-(4-((5-chloro-4-((2-(dimethylphosphoryl)phenyl)amino)pyrimidin-2- | 5-(4-((1-(4-((5-chloro-4-((2-(dimethylphosphoryl)phen |

| | | | |
|---|---|---|---|
| | | yl)amino)-3-methoxyphenyl)piperidin-4-yl)methyl)piperazin-1-yl)-2-(2,6-dioxopiperidin-3-yl)isoindoline-1,3-dione | yl)amino)pyrimidin-2-yl)amino)-3-methoxyphenyl)piperidin-4-yl)methyl)piperazin-1-yl)-2-(2,6-dioxopiperidin-3-yl)isoindoline-1,3-dione |
| | 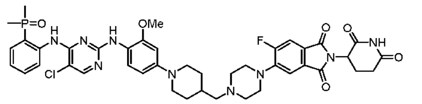 | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl)-2-(2,6-dioxopiperidin-3-yl)-6-fluoroisoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl)-2-(2,6-dioxopiperidin-3-yl)-6-fluoroisoindoline-1,3-dione |
| | 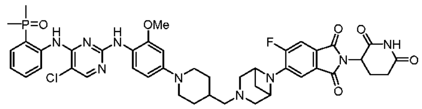 | 5-(3-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 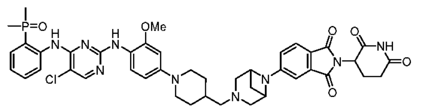 | 5-(3-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-6-yl)-2-(2,6-dioxopiperidin-3-yl)isoindoline-1,3-dione |
| | 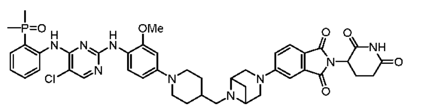 | 5-(6-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-3-yl)-2-(2,6-dioxopiperidin-3-yl)isoindoline-1,3-dione |
| | 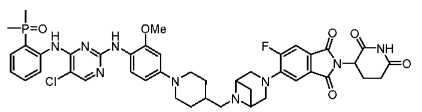 | 5-(6-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-3-yl)-2-(2,6-dioxopiperidin-3-yl)-6-fluoroisoindoline-1,3- | 5-(6-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-3-yl)-2-(2,6- |

| | | | |
|---|---|---|---|
| | | dione | dioxopiperidin-3-yl)-6-fluoroisoindoline-1,3-dione |
| | 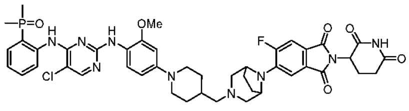 | 5-(3-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-8-yl)-2-(2,6-dioxopiperidin-3-yl)-6-fluoroisoindoline-1,3-dione |
| | 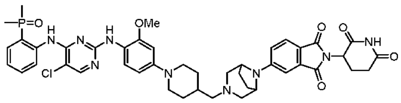 | 5-(3-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-8-yl)-2-(2,6-dioxopiperidin-3-yl)isoindoline-1,3-dione | 5-(3-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-8-yl)-2-(2,6-dioxopiperidin-3-yl)isoindoline-1,3-dione |
| | 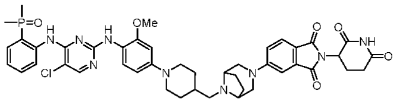 | 5-(8-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-3-yl)-2-(2,6-dioxopiperidin-3-yl)isoindoline-1,3-dione | 5-(8-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-3-yl)-2-(2,6-dioxopiperidin-3-yl)isoindoline-1,3-dione |
| | 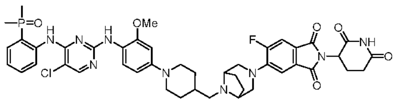 | 5-(8-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 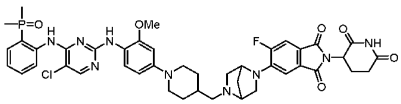 | 5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 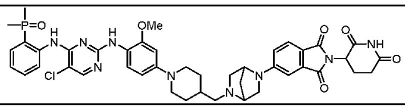 | 5-(5-((1-(4-((5-chloro-4-((2-(dimethylphosphoryl)phenyl)amino)pyrimidin-2- | 5-(5-((1-(4-((5-chloro-4-((2-(dimethylphosphoryl)phen |

| | | | |
|---|---|---|---|
| | | yl)amino)-3-methoxyphenyl)piperidin-4-yl)methyl)-2,5-diazabicyclo[2.2.1]heptan-2-yl)-2-(2,6-dioxopiperidin-3-yl)isoindoline-1,3-dione | yl)amino)pyrimidin-2-yl)amino)-3-methoxyphenyl)piperidin-4-yl)methyl)-2,5-diazabicyclo[2.2.1]heptan-2-yl)-2-(2,6-dioxopiperidin-3-yl)isoindoline-1,3-dione |
| | 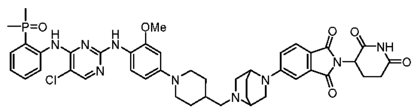 | 5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.2]octan-2-yl)-2-(2,6-dioxopiperidin-3-yl)isoindoline-1,3-dione | 5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.2]octan-2-yl)-2-(2,6-dioxopiperidin-3-yl)isoindoline-1,3-dione |
| | 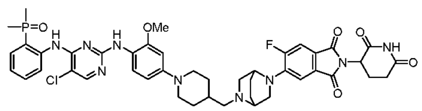 | 5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 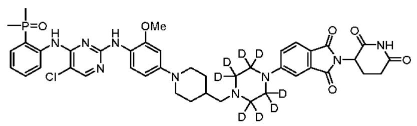 | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)isoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)isoindoline-1,3-dione |
| | 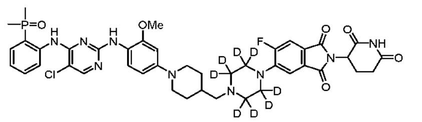 | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione |
| | 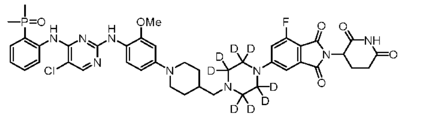 | 6-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4- |

| | | | |
|---|---|---|---|
| | | | fluoroisoindoline-1,3-dione |
| | 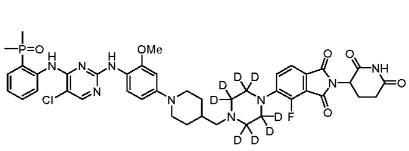 | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 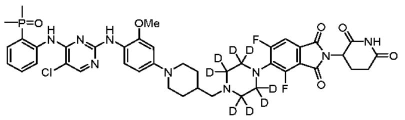 | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |
| | 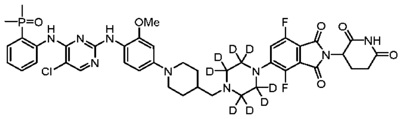 | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione |
| | 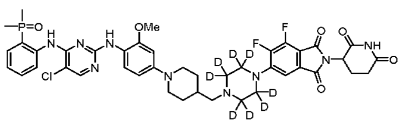 | 6-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione | 6-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione |
| | 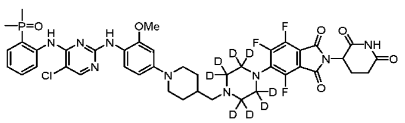 | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |
| | 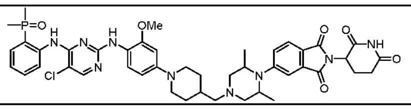 | 5-(4-((1-(4-((5-chloro-4-((2-(dimethylphosphoryl)phenyl)amino)pyrimidin-2- | 5-(4-((1-(4-((5-chloro-4-((2-(dimethylphosphoryl)phen |

| | | | |
|---|---|---|---|
| | | yl)amino)-3-methoxyphenyl)piperidin-4-yl)methyl)-2,6-dimethylpiperazin-1-yl)-2-(2,6-dioxopiperidin-3-yl)isoindoline-1,3-dione | yl)amino)pyrimidin-2-yl)amino)-3-methoxyphenyl)piperidin-4-yl)methyl)-2,6-dimethylpiperazin-1-yl)-2-(2,6-dioxopiperidin-3-yl)isoindoline-1,3-dione |
| | 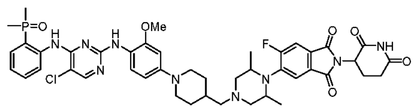 | 5-(4-((1-(4-((5-chloro-4-((2-(dimethylphosphoryl)phenyl)amino)pyrimidin-2-yl)amino)-3-methoxyphenyl)piperidin-4-yl)methyl)-2,6-dimethylpiperazin-1-yl)-2-(2,6-dioxopiperidin-3-yl)-6-fluoroisoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)-2,6-dimethylpiperazin-1-yl)-2-(2,6-dioxopiperidin-3-yl)-6-fluoroisoindoline-1,3-dione |
| | 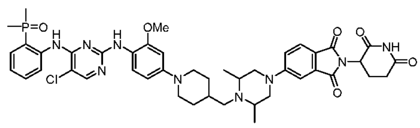 | 5-(4-((1-(4-((5-chloro-4-((2-(dimethylphosphoryl)phenyl)amino)pyrimidin-2-yl)amino)-3-methoxyphenyl)piperidin-4-yl)methyl)-3,5-dimethylpiperazin-1-yl)-2-(2,6-dioxopiperidin-3-yl)isoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)-3,5-dimethylpiperazin-1-yl)-2-(2,6-dioxopiperidin-3-yl)isoindoline-1,3-dione |
| | 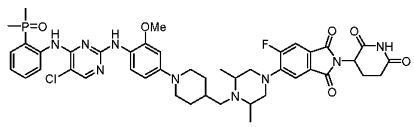 | 5-(4-((1-(4-((5-chloro-4-((2-(dimethylphosphoryl)phenyl)amino)pyrimidin-2-yl)amino)-3-methoxyphenyl)piperidin-4-yl)methyl)-3,5-dimethylpiperazin-1-yl)-2-(2,6-dioxopiperidin-3-yl)-6-fluoroisoindoline-1,3-dione | 5-(4-((1-(4-((5-chloro-4-((2-(dimethylphosphoryl)phenyl)amino)pyrimidin-2-yl)amino)-3-methoxyphenyl)piperidin-4-yl)methyl)-3,5-dimethylpiperazin-1-yl)-2-(2,6-dioxopiperidin-3-yl)-6-fluoroisoindoline-1,3-dione |
| | 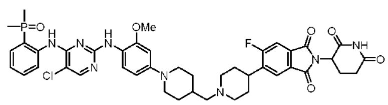 | 5-(1-((1-(4-((5-chloro-4-((2-(dimethylphosphoryl)phenyl)amino)pyrimidin-2-yl)amino)-3-methoxyphenyl)piperidin-4-yl)methyl)piperidin-4-yl)-2-(2,6-dioxopiperidin-3-yl)-6-fluoroisoindoline-1,3-dione | 5-(1-((1-(4-((5-chloro-4-((2-(dimethylphosphoryl)phenyl)amino)pyrimidin-2-yl)amino)-3-methoxyphenyl)piperidin-4-yl)methyl)piperidin-4-yl)-2-(2,6-dioxopiperidin-3-yl)-6-fluoroisoindoline-1,3-dione |
| | 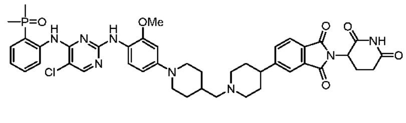 | 5-(1-((1-(4-((5-chloro-4-((2-(dimethylphosphoryl)phenyl)amino)pyrimidin-2-yl)amino)-3-methoxyphenyl)piperidin-4-yl)methyl)piperidin-4-yl)-2-(2,6-dioxopiperidin-3-yl)isoindoline-1,3-dione | 5-(1-((1-(4-((5-chloro-4-((2-(dimethylphosphoryl)phenyl)amino)pyrimidin-2-yl)amino)-3-methoxyphenyl)piperidin-4-yl)methyl)piperidin-4-yl)-2-(2,6-dioxopiperidin-3-yl)isoindoline-1,3-dione |

| | | | |
|---|---|---|---|
| | 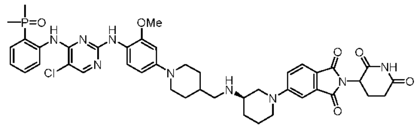 | 5-((R)-3-(((1-(4-((5-chloro-4-((2-(dimethylphosphoryl)phenyl)amino)pyrimidin-2-yl)amino)-3-methoxyphenyl)piperidin-4-yl)methyl)amino)piperidin-1-yl)-2-(2,6-dioxopiperidin-3-yl)isoindoline-1,3-dione | 5-((R)-3-(((1-(4-((5-chloro-4-((2-(dimethylphosphoryl)phenyl)amino)pyrimidin-2-yl)amino)-3-methoxyphenyl)piperidin-4-yl)methyl)amino)piperidin-1-yl)-2-(2,6-dioxopiperidin-3-yl)isoindoline-1,3-dione |
| | 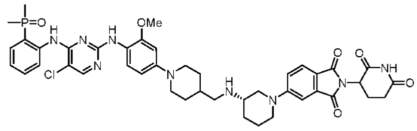 | 5-((S)-3-(((1-(4-((5-chloro-4-((2-(dimethylphosphoryl)phenyl)amino)pyrimidin-2-yl)amino)-3-methoxyphenyl)piperidin-4-yl)methyl)amino)piperidin-1-yl)-2-(2,6-dioxopiperidin-3-yl)isoindoline-1,3-dione | 5-((S)-3-(((1-(4-((5-chloro-4-((2-(dimethylphosphoryl)phenyl)amino)pyrimidin-2-yl)amino)-3-methoxyphenyl)piperidin-4-yl)methyl)amino)piperidin-1-yl)-2-(2,6-dioxopiperidin-3-yl)isoindoline-1,3-dione |
| | 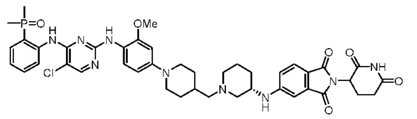 | 5-(((S)-1-((1-(4-((5-chloro-4-((2-(dimethylphosphoryl)phenyl)amino)pyrimidin-2-yl)amino)-3-methoxyphenyl)piperidin-4-yl)methyl)piperidin-3-yl)amino)-2-(2,6-dioxopiperidin-3-yl)isoindoline-1,3-dione | 5-(((S)-1-((1-(4-((5-chloro-4-((2-(dimethylphosphoryl)phenyl)amino)pyrimidin-2-yl)amino)-3-methoxyphenyl)piperidin-4-yl)methyl)piperidin-3-yl)amino)-2-(2,6-dioxopiperidin-3-yl)isoindoline-1,3-dione |
| | 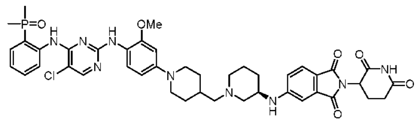 | 5-(((R)-1-((1-(4-((5-chloro-4-((2-(dimethylphosphoryl)phenyl)amino)pyrimidin-2-yl)amino)-3-methoxyphenyl)piperidin-4-yl)methyl)piperidin-3-yl)amino)-2-(2,6-dioxopiperidin-3-yl)isoindoline-1,3-dione | 5-(((R)-1-((1-(4-((5-chloro-4-((2-(dimethylphosphoryl)phenyl)amino)pyrimidin-2-yl)amino)-3-methoxyphenyl)piperidin-4-yl)methyl)piperidin-3-yl)amino)-2-(2,6-dioxopiperidin-3-yl)isoindoline-1,3-dione |
| | 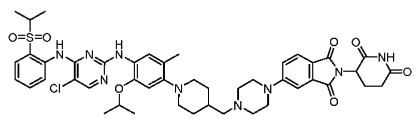 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl)-2-(2,6-dioxopiperidin-3-yl)isoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl)-2-(2,6-dioxopiperidin-3-yl)isoindoline-1,3-dione |
| | 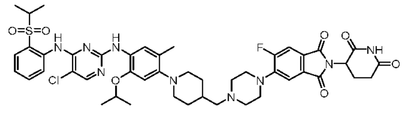 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl)-2-(2,6-dioxopiperidin-3-yl)-6- | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl)- |

| | | | |
|---|---|---|---|
| | | fluoroisoindoline-1,3-dione | 2-(2,6-dioxopiperidin-3-yl)-6-fluoroisoindoline-1,3-dione |
| | 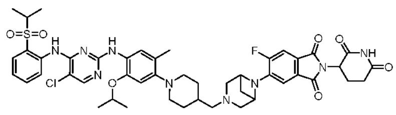 | 5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 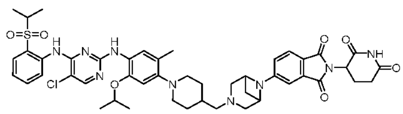 | 5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-6-yl)-2-(2,6-dioxopiperidin-3-yl)isoindoline-1,3-dione |
| | 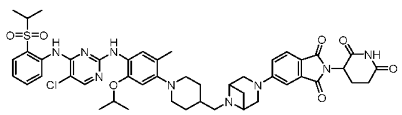 | 5-(6-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-3-yl)-2-(2,6-dioxopiperidin-3-yl)isoindoline-1,3-dione |
| | 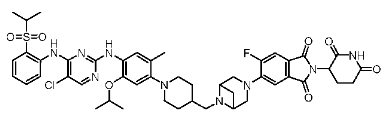 | 5-(6-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-3-yl)-2-(2,6-dioxopiperidin-3-yl)-6-fluoroisoindoline-1,3-dione | 5-(6-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-3-yl)-2-(2,6-dioxopiperidin-3-yl)-6-fluoroisoindoline-1,3-dione |
| | 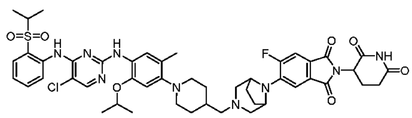 | 5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-8-yl)-2-(2,6-dioxopiperidin-3-yl)-6-fluoroisoindoline-1,3-dione |

| | | | |
|---|---|---|---|
| | 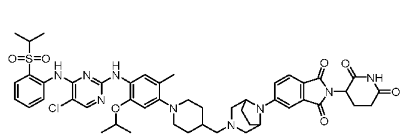 | 5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-8-yl)-2-(2,6-dioxopiperidin-3-yl)isoindoline-1,3-dione | 5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-8-yl)-2-(2,6-dioxopiperidin-3-yl)isoindoline-1,3-dione |
| | 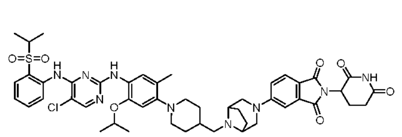 | 5-(8-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-3-yl)-2-(2,6-dioxopiperidin-3-yl)isoindoline-1,3-dione | 5-(8-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-3-yl)-2-(2,6-dioxopiperidin-3-yl)isoindoline-1,3-dione |
| | 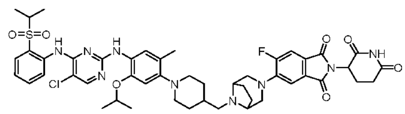 | 5-(8-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 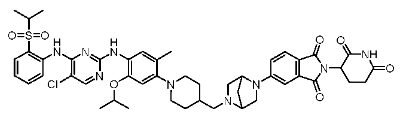 | 5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.1]heptan-2-yl)-2-(2,6-dioxopiperidin-3-yl)isoindoline-1,3-dione |
| | 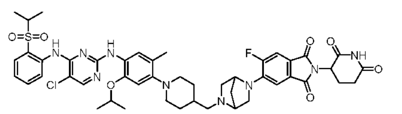 | 5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 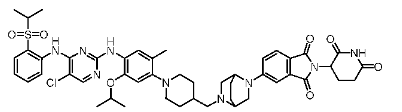 | 5-(5-((1-(4-((5-chloro-4-((2-(isopropylsulfonyl)phenyl)amino)pyrimidin-2-yl)amino)-5-isopropoxy-2-methylphenyl)piperidin-4- | 5-(5-((1-(4-((5-chloro-4-((2-(isopropylsulfonyl)phenyl)amino)pyrimidin-2-yl)amino)-5-isopropoxy-2- |

| | | | |
|---|---|---|---|
| | | yl)methyl)-2,5-diazabicyclo[2.2.2]octan-2-yl)-2-(2,6-dioxopiperidin-3-yl)isoindoline-1,3-dione | methylphenyl)piperidin-4-yl)methyl)-2,5-diazabicyclo[2.2.2]octan-2-yl)-2-(2,6-dioxopiperidin-3-yl)isoindoline-1,3-dione |
| | 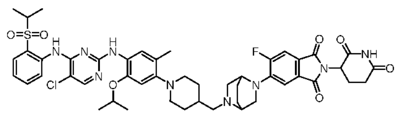 | 5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 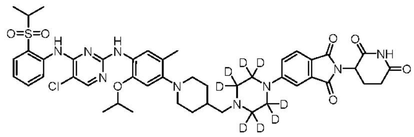 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)isoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)isoindoline-1,3-dione |
| | 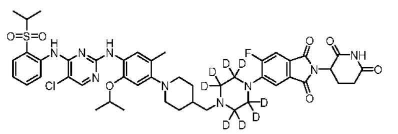 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-6-fluoroisoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-6-fluoroisoindoline-1,3-dione |
| | 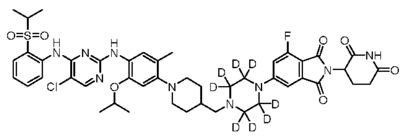 | 6-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4-fluoroisoindoline-1,3-dione | 6-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4-fluoroisoindoline-1,3-dione |
| | 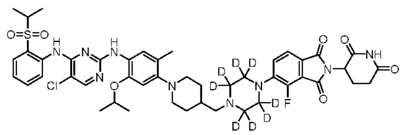 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4-fluoroisoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4-fluoroisoindoline-1,3-dione |

| | | | |
|---|---|---|---|
| | 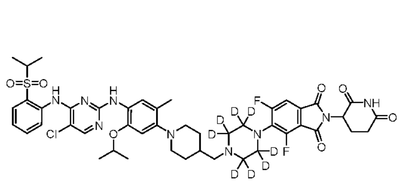 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4,6-difluoroisoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4,6-difluoroisoindoline-1,3-dione |
| | 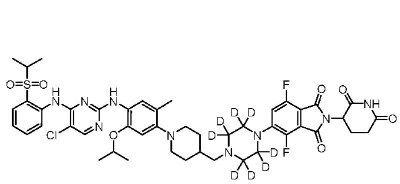 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4,7-difluoroisoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4,7-difluoroisoindoline-1,3-dione |
| | 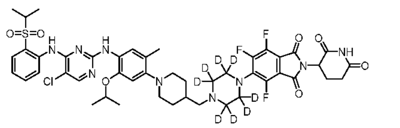 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4,6,7-trifluoroisoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4,6,7-trifluoroisoindoline-1,3-dione |
| | 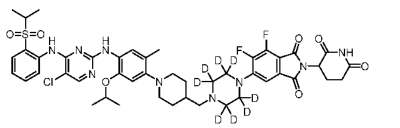 | 6-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4,5-difluoroisoindoline-1,3-dione | 6-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-*d*8)-2-(2,6-dioxopiperidin-3-yl)-4,5-difluoroisoindoline-1,3-dione |
| | 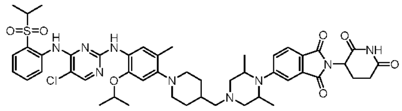 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,6-dimethylpiperazin-1-yl)-2-(2,6-dioxopiperidin-3-yl)isoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,6-dimethylpiperazin-1-yl)-2-(2,6-dioxopiperidin-3-yl)isoindoline-1,3-dione |
| | 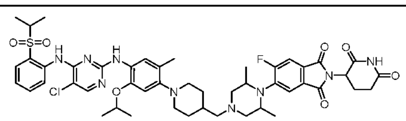 | 5-(4-((1-(4-((5-chloro-4-((2-(isopropylsulfonyl)phenyl)amino)pyrimidin-2-yl)amino)-5-isopropoxy-2- | 5-(4-((1-(4-((5-chloro-4-((2-(isopropylsulfonyl)phenyl)amino)pyrimidin-2- |

| | | | |
|---|---|---|---|
| | | methylphenyl)piperidin-4-yl)methyl)-2,6-dimethylpiperazin-1-yl)-2-(2,6-dioxopiperidin-3-yl)-6-fluoroisoindoline-1,3-dione | yl)amino)-5-isopropoxy-2-methylphenyl)piperidin-4-yl)methyl)-2,6-dimethylpiperazin-1-yl)-2-(2,6-dioxopiperidin-3-yl)-6-fluoroisoindoline-1,3-dione |
| | 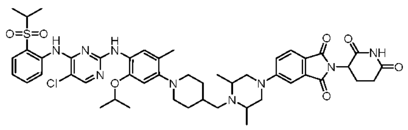 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,5-dimethylpiperazin-1-yl)-2-(2,6-dioxopiperidin-3-yl)isoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,5-dimethylpiperazin-1-yl)-2-(2,6-dioxopiperidin-3-yl)isoindoline-1,3-dione |
| | 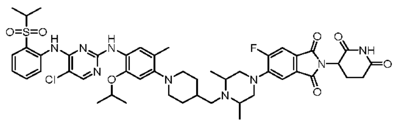 | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,5-dimethylpiperazin-1-yl)-2-(2,6-dioxopiperidin-3-yl)-6-fluoroisoindoline-1,3-dione | 5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,5-dimethylpiperazin-1-yl)-2-(2,6-dioxopiperidin-3-yl)-6-fluoroisoindoline-1,3-dione |
| | 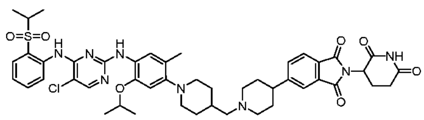 | 5-(1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-4-yl)-2-(2,6-dioxopiperidin-3-yl)isoindoline-1,3-dione | 5-(1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-4-yl)-2-(2,6-dioxopiperidin-3-yl)isoindoline-1,3-dione |
| | 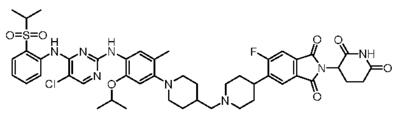 | 5-(1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-4-yl)-2-(2,6-dioxopiperidin-3-yl)-6-fluoroisoindoline-1,3-dione | 5-(1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-4-yl)-2-(2,6-dioxopiperidin-3-yl)-6-fluoroisoindoline-1,3-dione |
| | 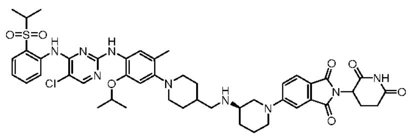 | 5-((R)-3-(((1-(4-((5-chloro-4-((2-(isopropylsulfonyl)phenyl)amino)pyrimidin-2-yl)amino)-5-isopropoxy-2-methylphenyl)piperidin-4-yl)methyl)amino)piperidin-1-yl)-2-(2,6-dioxopiperidin-3-yl)isoindoline-1,3-dione | 5-((R)-3-(((1-(4-((5-chloro-4-((2-(isopropylsulfonyl)phenyl)amino)pyrimidin-2-yl)amino)-5-isopropoxy-2-methylphenyl)piperidin-4-yl)methyl)amino)piperidin-1-yl)-2-(2,6-dioxopiperidin-3-yl)isoindoline-1,3-dione |

| | | | |
|---|---|---|---|
| | 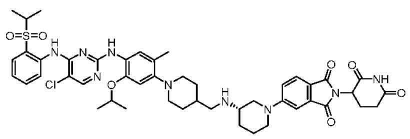 | 5-((S)-3-(((1-(4-((5-chloro-4-((2-(isopropylsulfonyl)phenyl)amino)pyrimidin-2-yl)amino)-5-isopropoxy-2-methylphenyl)piperidin-4-yl)methyl)amino)piperidin-1-yl)-2-(2,6-dioxopiperidin-3-yl)isoindoline-1,3-dione | 5-((S)-3-(((1-(4-((5-chloro-4-((2-(isopropylsulfonyl)phenyl)amino)pyrimidin-2-yl)amino)-5-isopropoxy-2-methylphenyl)piperidin-4-yl)methyl)amino)piperidin-1-yl)-2-(2,6-dioxopiperidin-3-yl)isoindoline-1,3-dione |
| | 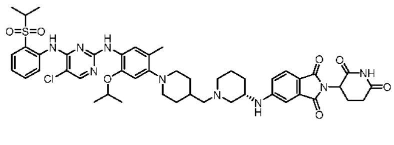 | 5-(((S)-1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-3-yl)amino)-2-(2,6-dioxopiperidin-3-yl)isoindoline-1,3-dione | 5-(((S)-1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-3-yl)amino)-2-(2,6-dioxopiperidin-3-yl)isoindoline-1,3-dione |
| | 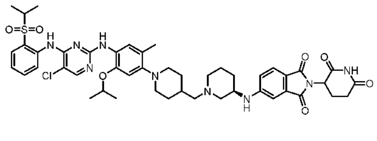 | 5-(((R)-1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-3-yl)amino)-2-(2,6-dioxopiperidin-3-yl)isoindoline-1,3-dione | 5-(((R)-1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-3-yl)amino)-2-(2,6-dioxopiperidin-3-yl)isoindoline-1,3-dione |
| | 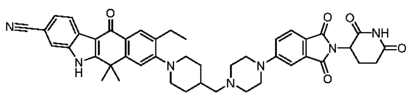 | 8-(4-((4-(2-(2,6-dioxopiperidin-3-yl)-1,3-dioxoisoindolin-5-yl)piperazin-1-yl)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-1,3-dioxoisoindolin-5-yl)piperazin-1-yl)methyl)piperidin-1-yl)-9-ethyl-6,6-dimethyl-11-oxo-6,11-dihydro-5H-benzo[b]carbazole-3-carbonitrile |
| | 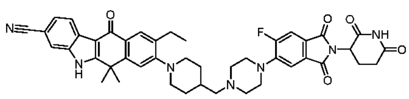 | 8-(4-((4-(2-(2,6-dioxopiperidin-3-yl)-6-fluoro-1,3-dioxoisoindolin-5-yl)piperazin-1-yl)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-6-fluoro-1,3-dioxoisoindolin-5-yl)piperazin-1-yl)methyl)piperidin-1-yl)-9-ethyl-6,6-dimethyl-11-oxo-6,11-dihydro-5H-benzo[b]carbazole-3-carbonitrile |
| | 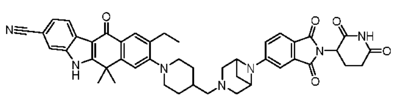 | 8-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-9-ethyl-6,6-dimethyl-11-oxo-6,11-dihydro-5H-benzo[b]carbazole-3-carbonitrile | 8-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-9-ethyl-6,6-dimethyl-11-oxo-6,11-dihydro-5H-benzo[b]carbazole-3-carbonitrile |

| | | | |
|---|---|---|---|
| | 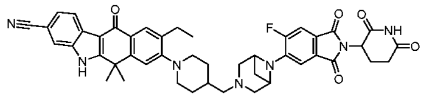 | 8-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-9-ethyl-6,6-dimethyl-11-oxo-6,11-dihydro-5H-benzo[b]carbazole-3-carbonitrile | 8-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-9-ethyl-6,6-dimethyl-11-oxo-6,11-dihydro-5H-benzo[b]carbazole-3-carbonitrile |
| | 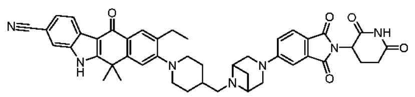 | 8-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-9-ethyl-6,6-dimethyl-11-oxo-6,11-dihydro-5H-benzo[b]carbazole-3-carbonitrile | 8-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-9-ethyl-6,6-dimethyl-11-oxo-6,11-dihydro-5H-benzo[b]carbazole-3-carbonitrile |
| | 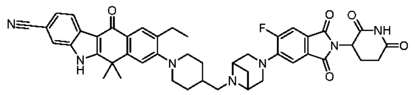 | 8-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-9-ethyl-6,6-dimethyl-11-oxo-6,11-dihydro-5H-benzo[b]carbazole-3-carbonitrile | 8-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-9-ethyl-6,6-dimethyl-11-oxo-6,11-dihydro-5H-benzo[b]carbazole-3-carbonitrile |
| | 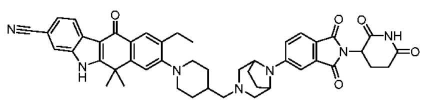 | 8-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-9-ethyl-6,6-dimethyl-11-oxo-6,11-dihydro-5H-benzo[b]carbazole-3-carbonitrile | 8-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-9-ethyl-6,6-dimethyl-11-oxo-6,11-dihydro-5H-benzo[b]carbazole-3-carbonitrile |
| | 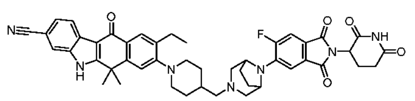 | 8-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-9-ethyl-6,6-dimethyl-11-oxo-6,11-dihydro-5H-benzo[b]carbazole-3-carbonitrile | 8-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-9-ethyl-6,6-dimethyl-11-oxo-6,11-dihydro-5H-benzo[b]carbazole-3-carbonitrile |
| | 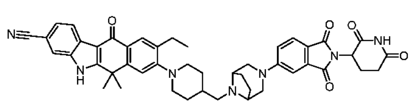 | 8-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-9-ethyl-6,6-dimethyl-11-oxo-6,11-dihydro-5H-benzo[b]carbazole-3-carbonitrile | 8-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-9-ethyl-6,6-dimethyl-11-oxo-6,11-dihydro-5H-benzo[b]carbazole-3-carbonitrile |

| | | | |
|---|---|---|---|
| | 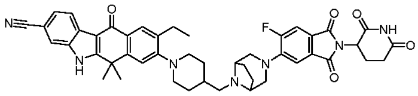 | 8-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-9-ethyl-6,6-dimethyl-11-oxo-6,11-dihydro-5H-benzo[b]carbazole-3-carbonitrile | 8-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-9-ethyl-6,6-dimethyl-11-oxo-6,11-dihydro-5H-benzo[b]carbazole-3-carbonitrile |
| | 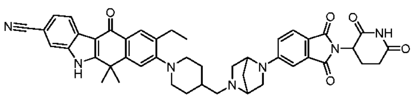 | 8-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-9-ethyl-6,6-dimethyl-11-oxo-6,11-dihydro-5H-benzo[b]carbazole-3-carbonitrile | 8-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-9-ethyl-6,6-dimethyl-11-oxo-6,11-dihydro-5H-benzo[b]carbazole-3-carbonitrile |
| | 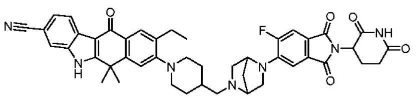 | 8-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-9-ethyl-6,6-dimethyl-11-oxo-6,11-dihydro-5H-benzo[b]carbazole-3-carbonitrile | 8-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-9-ethyl-6,6-dimethyl-11-oxo-6,11-dihydro-5H-benzo[b]carbazole-3-carbonitrile |
| | 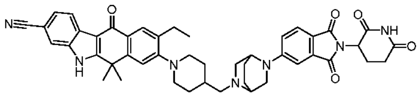 | 8-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-9-ethyl-6,6-dimethyl-11-oxo-6,11-dihydro-5H-benzo[b]carbazole-3-carbonitrile | 8-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-9-ethyl-6,6-dimethyl-11-oxo-6,11-dihydro-5H-benzo[b]carbazole-3-carbonitrile |
| | 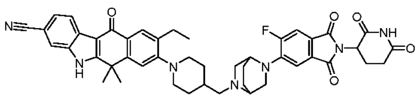 | 8-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-9-ethyl-6,6-dimethyl-11-oxo-6,11-dihydro-5H-benzo[b]carbazole-3-carbonitrile | 8-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-9-ethyl-6,6-dimethyl-11-oxo-6,11-dihydro-5H-benzo[b]carbazole-3-carbonitrile |
| | 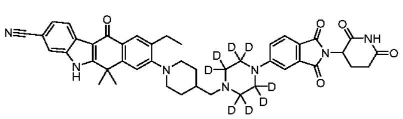 | 8-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile |

| | | | |
|---|---|---|---|
| | 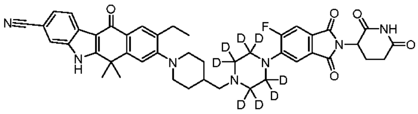 | 8-(4-((4-(2-(2,6-dioxopiperidin-3-yl)-6-fluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-6-fluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile |
| | 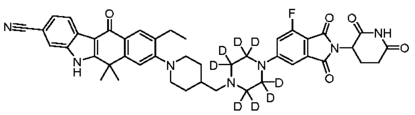 | 8-(4-((4-(2-(2,6-dioxopiperidin-3-yl)-7-fluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-7-fluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile |
| | 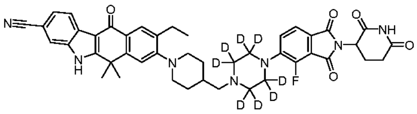 | 8-(4-((4-(2-(2,6-dioxopiperidin-3-yl)-4-fluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-4-fluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile |
| | 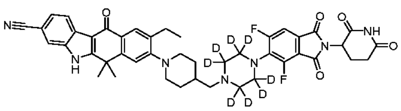 | 8-(4-((4-(2-(2,6-dioxopiperidin-3-yl)-4,6-difluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-4,6-difluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile |
| | 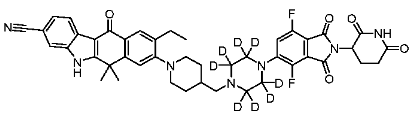 | 8-(4-((4-(2-(2,6-dioxopiperidin-3-yl)-4,7-difluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-4,7-difluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile |
| | 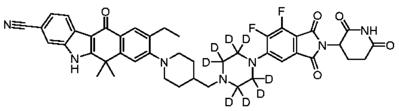 | 8-(4-((4-(2-(2,6-dioxopiperidin-3-yl)-6,7-difluoro-1,3-dioxoisoindolin-5-yl)piperazin-1-yl-2,2,3,3,5,5,6,6-*d*8)methyl)piperidin-1-yl)-9- | 8-(4-((4-(2-(2,6-dioxopiperidin-3-yl)-6,7-difluoro-1,3-dioxoisoindolin-5-yl)piperazin-1-yl-2,2,3,3,5,5,6,6- |

| | | | |
|---|---|---|---|
| | | ethyl-6,6-dimethyl-11-oxo-6,11-dihydro-5H-benzo[b]carbazole-3-carbonitrile | *d*8)methyl)piperidin-1-yl)-9-ethyl-6,6-dimethyl-11-oxo-6,11-dihydro-5H-benzo[b]carbazole-3-carbonitrile |
| | 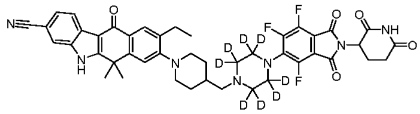 | 8-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*8)methyl)piperidin-1-yl)-9-ethyl-6,6-dimethyl-11-oxo-6,11-dihydro-5H-benzo[b]carbazole-3-carbonitrile |
| | 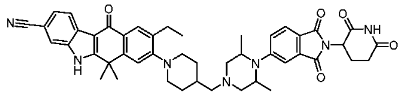 | 8-(4-((4-(2-(2,6-dioxopiperidin-3-yl)-1,3-dioxoisoindolin-5-yl)-3,5-dimethylpiperazin-1-yl)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-1,3-dioxoisoindolin-5-yl)-3,5-dimethylpiperazin-1-yl)methyl)piperidin-1-yl)-9-ethyl-6,6-dimethyl-11-oxo-6,11-dihydro-5H-benzo[b]carbazole-3-carbonitrile |
| | 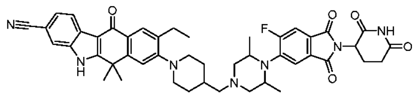 | 8-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-9-ethyl-6,6-dimethyl-11-oxo-6,11-dihydro-5H-benzo[b]carbazole-3-carbonitrile |
| | 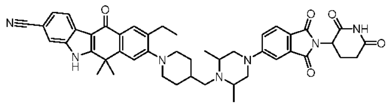 | 8-(4-((4-(2-(2,6-dioxopiperidin-3-yl)-1,3-dioxoisoindolin-5-yl)-2,6-dimethylpiperazin-1-yl)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-1,3-dioxoisoindolin-5-yl)-2,6-dimethylpiperazin-1-yl)methyl)piperidin-1-yl)-9-ethyl-6,6-dimethyl-11-oxo-6,11-dihydro-5H-benzo[b]carbazole-3-carbonitrile |
| | 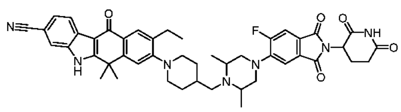 | 8-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-9-ethyl-6,6-dimethyl-11-oxo-6,11-dihydro-5H-benzo[b]carbazole-3-carbonitrile |
| | 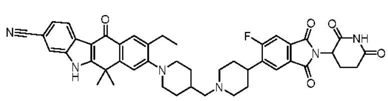 | 8-(4-((4-(2-(2,6-dioxopiperidin-3-yl)-6-fluoro-1,3-dioxoisoindolin-5-yl)piperidin-1- | 8-(4-((4-(2-(2,6-dioxopiperidin-3-yl)-6-fluoro-1,3-dioxoisoindolin-5-yl)piperidin-1- |

| | | | |
|---|---|---|---|
| | | yl)methyl)piperidin-1-yl)-9-ethyl-6,6-dimethyl-11-oxo-6,11-dihydro-5H-benzo[b]carbazole-3-carbonitrile | yl)methyl)piperidin-1-yl)-9-ethyl-6,6-dimethyl-11-oxo-6,11-dihydro-5H-benzo[b]carbazole-3-carbonitrile |
| | 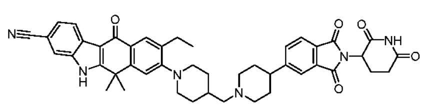 | 8-(4-((4-(2-(2,6-dioxopiperidin-3-yl)-1,3-dioxoisoindolin-5-yl)piperidin-1-yl)methyl)piperidin-1-yl)-9-ethyl-6,6-dimethyl-11-oxo-6,11-dihydro-5H-benzo[b]carbazole-3-carbonitrile | 8-(4-((4-(2-(2,6-dioxopiperidin-3-yl)-1,3-dioxoisoindolin-5-yl)piperidin-1-yl)methyl)piperidin-1-yl)-9-ethyl-6,6-dimethyl-11-oxo-6,11-dihydro-5H-benzo[b]carbazole-3-carbonitrile |
| | 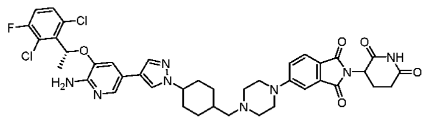 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl)-2-(2,6-dioxopiperidin-3-yl)isoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl)-2-(2,6-dioxopiperidin-3-yl)isoindoline-1,3-dione |
| | 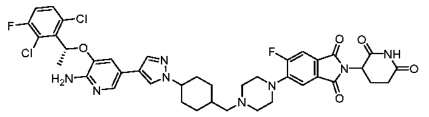 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl)-2-(2,6-dioxopiperidin-3-yl)-6-fluoroisoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl)-2-(2,6-dioxopiperidin-3-yl)-6-fluoroisoindoline-1,3-dione |
| | 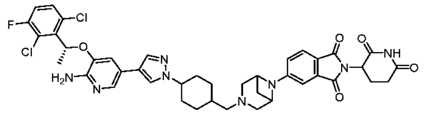 | 5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-6-yl)-2-(2,6-dioxopiperidin-3-yl)isoindoline-1,3-dione | 5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-6-yl)-2-(2,6-dioxopiperidin-3-yl)isoindoline-1,3-dione |
| | 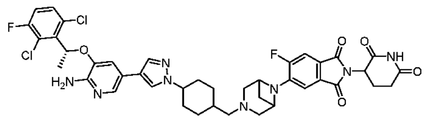 | 5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-6-yl)-2-(2,6-dioxopiperidin-3-yl)-6-fluoroisoindoline-1,3-dione | 5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-6-yl)-2-(2,6-dioxopiperidin-3-yl)-6-fluoroisoindoline-1,3-dione |
| | 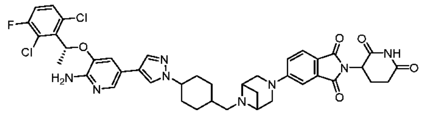 | 5-(6-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-3-yl)-2-(2,6-dioxopiperidin-3-yl)isoindoline-1,3-dione | 5-(6-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-3-yl)-2-(2,6-dioxopiperidin-3-yl)isoindoline-1,3-dione |

| | | | |
|---|---|---|---|
| | 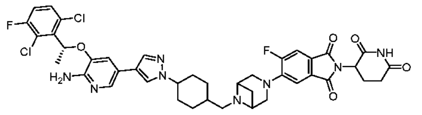 | 5-(6-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-3-yl)-2-(2,6-dioxopiperidin-3-yl)-6-fluoroisoindoline-1,3-dione | 5-(6-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-3-yl)-2-(2,6-dioxopiperidin-3-yl)-6-fluoroisoindoline-1,3-dione |
| | 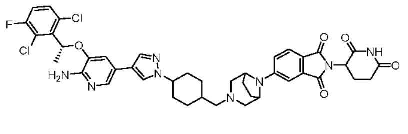 | 5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-8-yl)-2-(2,6-dioxopiperidin-3-yl)isoindoline-1,3-dione | 5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-8-yl)-2-(2,6-dioxopiperidin-3-yl)isoindoline-1,3-dione |
| | 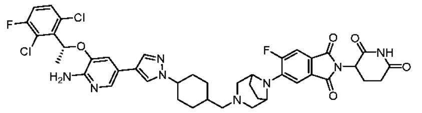 | 5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-8-yl)-2-(2,6-dioxopiperidin-3-yl)-6-fluoroisoindoline-1,3-dione | 5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-8-yl)-2-(2,6-dioxopiperidin-3-yl)-6-fluoroisoindoline-1,3-dione |
| | 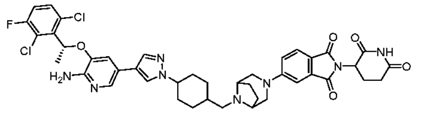 | 5-(8-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-3-yl)-2-(2,6-dioxopiperidin-3-yl)isoindoline-1,3-dione | 5-(8-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-3-yl)-2-(2,6-dioxopiperidin-3-yl)isoindoline-1,3-dione |
| | 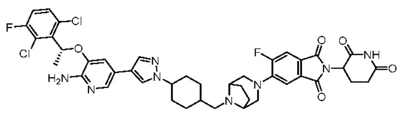 | 5-(8-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-3-yl)-2-(2,6-dioxopiperidin-3-yl)-6-fluoroisoindoline-1,3-dione | 5-(8-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-3-yl)-2-(2,6-dioxopiperidin-3-yl)-6-fluoroisoindoline-1,3-dione |
| | 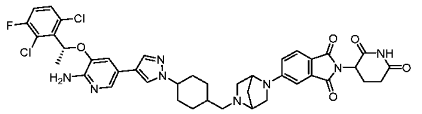 | 5-(5-((4-(4-(6-amino-5-((R)-1-(2,6-dichloro-3-fluorophenyl)ethoxy)pyridin-3-yl)-1H-pyrazol-1-yl)cyclohexyl)methyl)-2,5-diazabicyclo[2.2.1]heptan-2-yl)-2-(2,6-dioxopiperidin-3-yl)isoindoline-1,3-dione | 5-(5-((4-(4-(6-amino-5-((R)-1-(2,6-dichloro-3-fluorophenyl)ethoxy)pyridin-3-yl)-1H-pyrazol-1-yl)cyclohexyl)methyl)-2,5-diazabicyclo[2.2.1]heptan-2-yl)-2-(2,6-dioxopiperidin-3-yl)isoindoline-1,3-dione |
| | 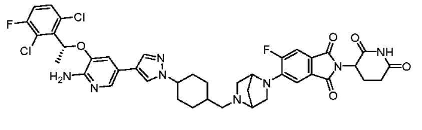 | 5-(5-((4-(4-(6-amino-5-((R)-1-(2,6-dichloro-3-fluorophenyl)ethoxy)pyridin-3-yl)-1H-pyrazol-1-yl)cyclohexyl)methyl)-2,5-diazabicyclo[2.2.1]heptan-2- | 5-(5-((4-(4-(6-amino-5-((R)-1-(2,6-dichloro-3-fluorophenyl)ethoxy)pyridin-3-yl)-1H-pyrazol-1-yl)cyclohexyl)methyl)-2,5-diazabicyclo[2.2.1]heptan- |

| | | | |
|---|---|---|---|
| | | yl)-2-(2,6-dioxopiperidin-3-yl)-6-fluoroisoindoline-1,3-dione | 2-yl)-2-(2,6-dioxopiperidin-3-yl)-6-fluoroisoindoline-1,3-dione |
| | 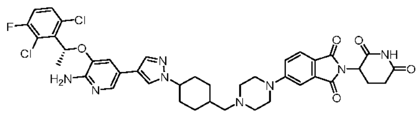 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl)-2-(2,6-dioxopiperidin-3-yl)isoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl)-2-(2,6-dioxopiperidin-3-yl)isoindoline-1,3-dione |
| | 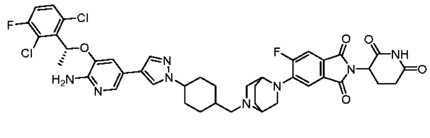 | 5-(5-((4-(4-(6-amino-5-((R)-1-(2,6-dichloro-3-fluorophenyl)ethoxy)pyridin-3-yl)-1H-pyrazol-1-yl)cyclohexyl)methyl)-2,5-diazabicyclo[2.2.2]octan-2-yl)-2-(2,6-dioxopiperidin-3-yl)-6-fluoroisoindoline-1,3-dione | 5-(5-((4-(4-(6-amino-5-((R)-1-(2,6-dichloro-3-fluorophenyl)ethoxy)pyridin-3-yl)-1H-pyrazol-1-yl)cyclohexyl)methyl)-2,5-diazabicyclo[2.2.2]octan-2-yl)-2-(2,6-dioxopiperidin-3-yl)-6-fluoroisoindoline-1,3-dione |
| | 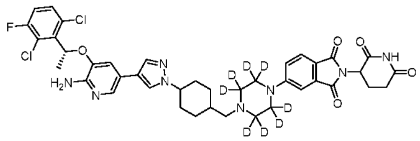 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-*d*$_8$)-2-(2,6-dioxopiperidin-3-yl)isoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-*d*$_8$)-2-(2,6-dioxopiperidin-3-yl)isoindoline-1,3-dione |
| | 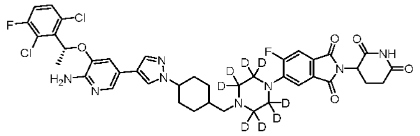 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-*d*$_8$)-2-(2,6-dioxopiperidin-3-yl)-6-fluoroisoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-*d*$_8$)-2-(2,6-dioxopiperidin-3-yl)-6-fluoroisoindoline-1,3-dione |
| | 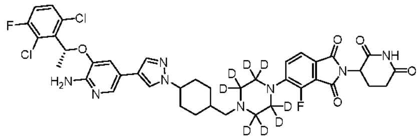 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-*d*$_8$)-2-(2,6-dioxopiperidin-3-yl)-4-fluoroisoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-*d*$_8$)-2-(2,6-dioxopiperidin-3-yl)-4-fluoroisoindoline-1,3-dione |
| | 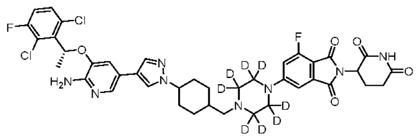 | 6-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-*d*$_8$)-2-(2,6-dioxopiperidin-3-yl)-4-fluoroisoindoline-1,3-dione | 6-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-*d*$_8$)-2-(2,6-dioxopiperidin-3-yl)-4-fluoroisoindoline-1,3-dione |

| | | | |
|---|---|---|---|
| | 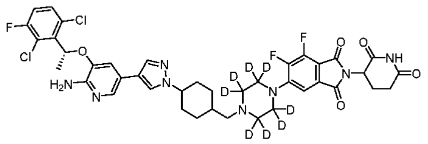 | 6-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,5-difluoroisoindoline-1,3-dione | 6-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,5-difluoroisoindoline-1,3-dione |
| | 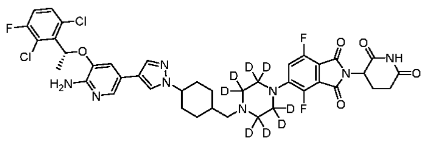 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,7-difluoroisoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,7-difluoroisoindoline-1,3-dione |
| | 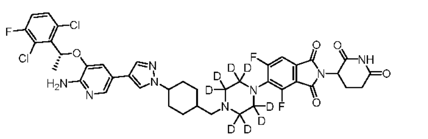 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,6-difluoroisoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,6-difluoroisoindoline-1,3-dione |
| | 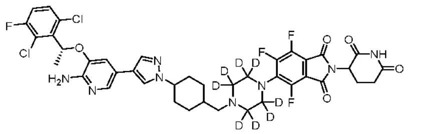 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,6,7-trifluoroisoindoline-1,3-dione |
| | 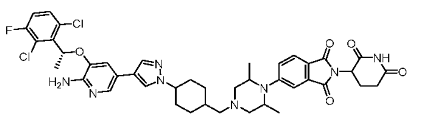 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)-2,6-dimethylpiperazin-1-yl)-2-(2,6-dioxopiperidin-3-yl)isoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)-2,6-dimethylpiperazin-1-yl)-2-(2,6-dioxopiperidin-3-yl)isoindoline-1,3-dione |
| | 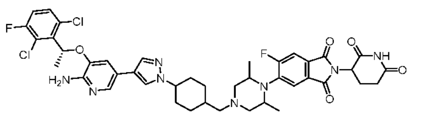 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)-2,6-dimethylpiperazin-1-yl)-2-(2,6-dioxopiperidin-3-yl)-6-fluoroisoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)-2,6-dimethylpiperazin-1-yl)-2-(2,6-dioxopiperidin-3-yl)-6-fluoroisoindoline-1,3-dione |

| | | | |
|---|---|---|---|
| | 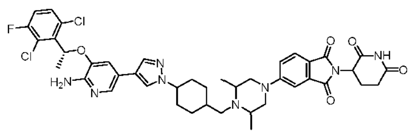 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)-3,5-dimethylpiperazin-1-yl)-2-(2,6-dioxopiperidin-3-yl)isoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)-3,5-dimethylpiperazin-1-yl)-2-(2,6-dioxopiperidin-3-yl)isoindoline-1,3-dione |
| | 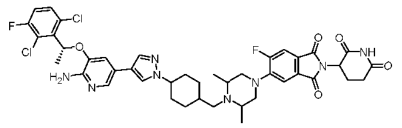 | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)-3,5-dimethylpiperazin-1-yl)-2-(2,6-dioxopiperidin-3-yl)-6-fluoroisoindoline-1,3-dione | 5-(4-((4-(4-(6-amino-5-((R)-1-(2,6-dichloro-3-fluorophenyl)ethoxy)pyridin-3-yl)-1H-pyrazol-1-yl)cyclohexyl)methyl)-3,5-dimethylpiperazin-1-yl)-2-(2,6-dioxopiperidin-3-yl)-6-fluoroisoindoline-1,3-dione |
| | 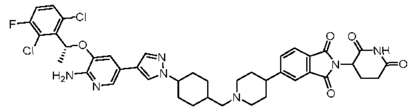 | 5-(1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-4-yl)-2-(2,6-dioxopiperidin-3-yl)isoindoline-1,3-dione | 5-(1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-4-yl)-2-(2,6-dioxopiperidin-3-yl)isoindoline-1,3-dione |
| | 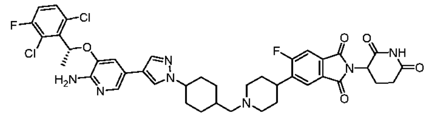 | 5-(1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-4-yl)-2-(2,6-dioxopiperidin-3-yl)-6-fluoroisoindoline-1,3-dione | 5-(1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-4-yl)-2-(2,6-dioxopiperidin-3-yl)-6-fluoroisoindoline-1,3-dione |
| | 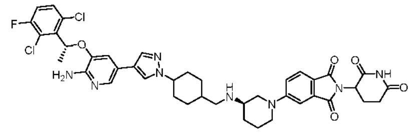 | 5-((R)-3-(((4-(4-(6-amino-5-((R)-1-(2,6-dichloro-3-fluorophenyl)ethoxy)pyridin-3-yl)-1H-pyrazol-1-yl)cyclohexyl)methyl)amino)piperidin-1-yl)-2-(2,6-dioxopiperidin-3-yl)isoindoline-1,3-dione | 5-((R)-3-(((4-(4-(6-amino-5-((R)-1-(2,6-dichloro-3-fluorophenyl)ethoxy)pyridin-3-yl)-1H-pyrazol-1-yl)cyclohexyl)methyl)amino)piperidin-1-yl)-2-(2,6-dioxopiperidin-3-yl)isoindoline-1,3-dione |
| | 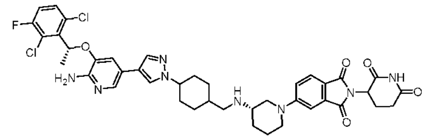 | 5-((S)-3-(((4-(4-(6-amino-5-((R)-1-(2,6-dichloro-3-fluorophenyl)ethoxy)pyridin-3-yl)-1H-pyrazol-1-yl)cyclohexyl)methyl)amino)piperidin-1-yl)-2-(2,6-dioxopiperidin-3-yl)isoindoline-1,3-dione | 5-((S)-3-(((4-(4-(6-amino-5-((R)-1-(2,6-dichloro-3-fluorophenyl)ethoxy)pyridin-3-yl)-1H-pyrazol-1-yl)cyclohexyl)methyl)amino)piperidin-1-yl)-2-(2,6-dioxopiperidin-3-yl)isoindoline-1,3-dione |
| | 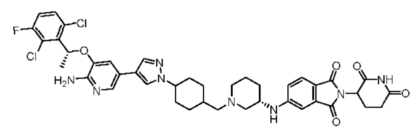 | 5-(((S)-1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-3-yl)amino)-2-(2,6-dioxopiperidin-3-yl)isoindoline-1,3-dione | 5-(((S)-1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-3-yl)amino)-2-(2,6-dioxopiperidin-3-yl)isoindoline-1,3-dione |

| | | | |
|---|---|---|---|
| | 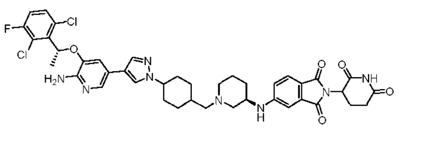 | 5-(((R)-1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-3-yl)amino)-2-(2,6-dioxopiperidin-3-yl)isoindoline-1,3-dione | 5-(((R)-1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-3-yl)amino)-2-(2,6-dioxopiperidin-3-yl)isoindoline-1,3-dione |
| | 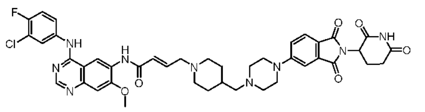 | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)piperazin-1-yl)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)piperazin-1-yl)methyl)piperidin-1-yl)but-2-enamide |
| | 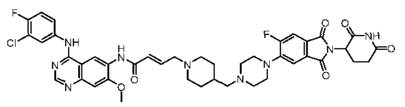 | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)piperazin-1-yl)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)piperazin-1-yl)methyl)piperidin-1-yl)but-2-enamide |
| | 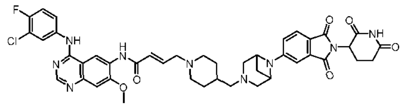 | (E)-N-(4-((3-chloro-4-fluorophenyl)amino)-7-methoxyquinazolin-6-yl)-4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)but-2-enamide | (E)-N-(4-((3-chloro-4-fluorophenyl)amino)-7-methoxyquinazolin-6-yl)-4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)but-2-enamide |
| | 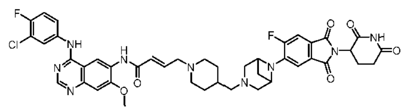 | (E)-N-(4-((3-chloro-4-fluorophenyl)amino)-7-methoxyquinazolin-6-yl)-4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)but-2-enamide | (E)-N-(4-((3-chloro-4-fluorophenyl)amino)-7-methoxyquinazolin-6-yl)-4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)but-2-enamide |
| | 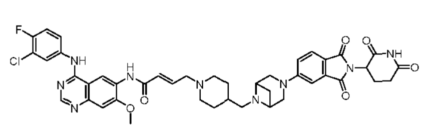 | (E)-N-(4-((3-chloro-4-fluorophenyl)amino)-7-methoxyquinazolin-6-yl)-4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)but-2-enamide | (E)-N-(4-((3-chloro-4-fluorophenyl)amino)-7-methoxyquinazolin-6-yl)-4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)but-2-enamide |
| | 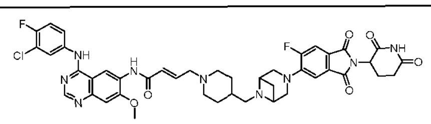 | (E)-N-(4-((3-chloro-4-fluorophenyl)amino)-7-methoxyquinazolin-6-yl)-4-(4-((3-(2-(2,6- | (E)-N-(4-((3-chloro-4-fluorophenyl)amino)-7-methoxyquinazolin-6-yl)-4-(4-((3-(2-(2,6- |

| | | | |
|---|---|---|---|
| | | dioxopiperidin-3-yl)-6-fluoro-1,3-dioxoisoindolin-5-yl)-3,6-diazabicyclo[3.1.1]heptan-6-yl)methyl)piperidin-1-yl)but-2-enamide | dioxopiperidin-3-yl)-6-fluoro-1,3-dioxoisoindolin-5-yl)-3,6-diazabicyclo[3.1.1]heptan-6-yl)methyl)piperidin-1-yl)but-2-enamide |
| | 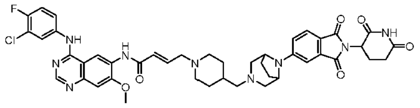 | (E)-N-(4-((3-chloro-4-fluorophenyl)amino)-7-methoxyquinazolin-6-yl)-4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)but-2-enamide | (E)-N-(4-((3-chloro-4-fluorophenyl)amino)-7-methoxyquinazolin-6-yl)-4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)but-2-enamide |
| | 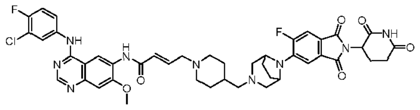 | (E)-N-(4-((3-chloro-4-fluorophenyl)amino)-7-methoxyquinazolin-6-yl)-4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)but-2-enamide | (E)-N-(4-((3-chloro-4-fluorophenyl)amino)-7-methoxyquinazolin-6-yl)-4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)but-2-enamide |
| | 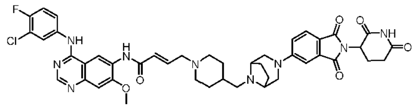 | (E)-N-(4-((3-chloro-4-fluorophenyl)amino)-7-methoxyquinazolin-6-yl)-4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)but-2-enamide | (E)-N-(4-((3-chloro-4-fluorophenyl)amino)-7-methoxyquinazolin-6-yl)-4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)but-2-enamide |
| | 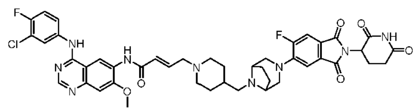 | (E)-N-(4-((3-chloro-4-fluorophenyl)amino)-7-methoxyquinazolin-6-yl)-4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)but-2-enamide | (E)-N-(4-((3-chloro-4-fluorophenyl)amino)-7-methoxyquinazolin-6-yl)-4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)but-2-enamide |
| | 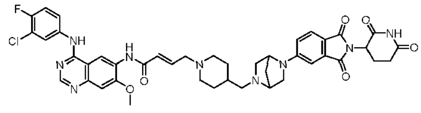 | (E)-N-(4-((3-chloro-4-fluorophenyl)amino)-7-methoxyquinazolin-6-yl)-4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)but-2-enamide | (E)-N-(4-((3-chloro-4-fluorophenyl)amino)-7-methoxyquinazolin-6-yl)-4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)but-2-enamide |
| | 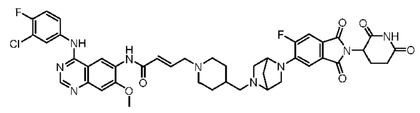 | (E)-N-(4-((3-chloro-4-fluorophenyl)amino)-7-methoxyquinazolin-6-yl)-4-(4-((5-(2-(2,6-dioxopiperidin-3-yl)-6- | (E)-N-(4-((3-chloro-4-fluorophenyl)amino)-7-methoxyquinazolin-6-yl)-4-(4-((5-(2-(2,6-dioxopiperidin-3-yl)-6- |

| | | | |
|---|---|---|---|
| | | fluoro-1,3-dioxoisoindolin-5-yl)-2,5-diazabicyclo[2.2.1]heptan-2-yl)methyl)piperidin-1-yl)but-2-enamide | fluoro-1,3-dioxoisoindolin-5-yl)-2,5-diazabicyclo[2.2.1]heptan-2-yl)methyl)piperidin-1-yl)but-2-enamide |
| | 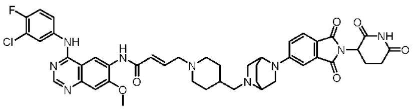 | (E)-N-(4-((3-chloro-4-fluorophenyl)amino)-7-methoxyquinazolin-6-yl)-4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)but-2-enamide | (E)-N-(4-((3-chloro-4-fluorophenyl)amino)-7-methoxyquinazolin-6-yl)-4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)but-2-enamide |
| | 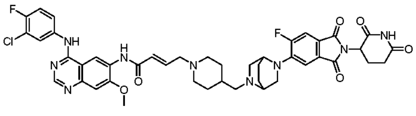 | (E)-N-(4-((3-chloro-4-fluorophenyl)amino)-7-methoxyquinazolin-6-yl)-4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)but-2-enamide | (E)-N-(4-((3-chloro-4-fluorophenyl)amino)-7-methoxyquinazolin-6-yl)-4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)but-2-enamide |
| | 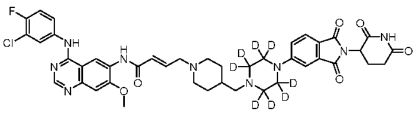 | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)but-2-enamide |
| | 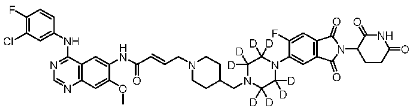 | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)but-2-enamide |
| | 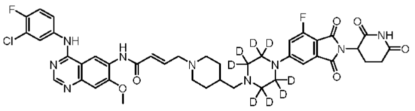 | (E)-N-(4-((3-chloro-4-fluorophenyl)amino)-7-methoxyquinazolin-6-yl)-4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)but-2-enamide |
| | 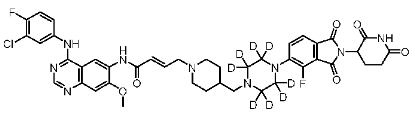 | (E)-N-(4-((3-chloro-4-fluorophenyl)amino)-7-methoxyquinazolin-6-yl)-4-(4-((4-(2-(2,6- | (E)-N-(4-((3-chloro-4-fluorophenyl)amino)-7-methoxyquinazolin-6-yl)-4-(4-((4-(2-(2,6- |

| | | | |
|---|---|---|---|
| | | dioxopiperidin-3-yl)-4-fluoro-1,3-dioxoisoindolin-5-yl)piperazin-1-yl-2,2,3,3,5,5,6,6-*d*8)methyl)piperidin-1-yl)but-2-enamide | dioxopiperidin-3-yl)-4-fluoro-1,3-dioxoisoindolin-5-yl)piperazin-1-yl-2,2,3,3,5,5,6,6-*d*8)methyl)piperidin-1-yl)but-2-enamide |
| | 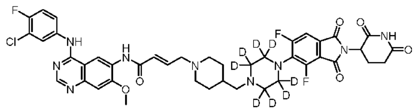 | (E)-N-(4-((3-chloro-4-fluorophenyl)amino)-7-methoxyquinazolin-6-yl)-4-(4-((4-(2-(2,6-dioxopiperidin-3-yl)-4,6-difluoro-1,3-dioxoisoindolin-5-yl)piperazin-1-yl-2,2,3,3,5,5,6,6-*d*8)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-4,6-difluoro-1,3-dioxoisoindolin-5-yl)piperazin-1-yl-2,2,3,3,5,5,6,6-*d*8)methyl)piperidin-1-yl)but-2-enamide |
| | 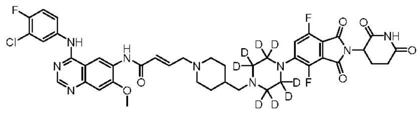 | (E)-N-(4-((3-chloro-4-fluorophenyl)amino)-7-methoxyquinazolin-6-yl)-4-(4-((4-(2-(2,6-dioxopiperidin-3-yl)-4,7-difluoro-1,3-dioxoisoindolin-5-yl)piperazin-1-yl-2,2,3,3,5,5,6,6-*d*8)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-4,7-difluoro-1,3-dioxoisoindolin-5-yl)piperazin-1-yl-2,2,3,3,5,5,6,6-*d*8)methyl)piperidin-1-yl)but-2-enamide |
| | 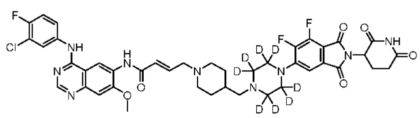 | (E)-N-(4-((3-chloro-4-fluorophenyl)amino)-7-methoxyquinazolin-6-yl)-4-(4-((4-(2-(2,6-dioxopiperidin-3-yl)-6,7-difluoro-1,3-dioxoisoindolin-5-yl)piperazin-1-yl-2,2,3,3,5,5,6,6-*d*8)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-6,7-difluoro-1,3-dioxoisoindolin-5-yl)piperazin-1-yl-2,2,3,3,5,5,6,6-*d*8)methyl)piperidin-1-yl)but-2-enamide |
| | 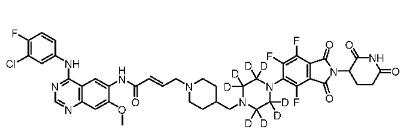 | (E)-N-(4-((3-chloro-4-fluorophenyl)amino)-7-methoxyquinazolin-6-yl)-4-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*8)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*8)methyl)piperidin-1-yl)but-2-enamide |
| | 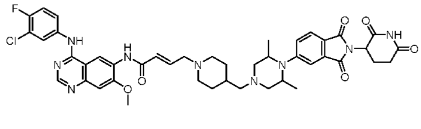 | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)-3,5-dimethylpiperazin-1-yl)methyl)piperidin-1-yl)but- | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)-3,5-dimethylpiperazin-1-yl)methyl)piperidin-1- |

| | | | |
|---|---|---|---|
| | | 2-enamide | yl)but-2-enamide |
| | 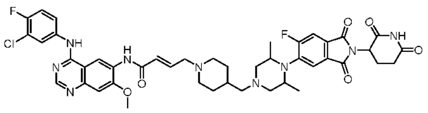 | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)but-2-enamide |
| | 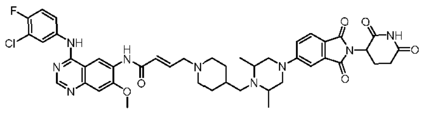 | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)-2,6-dimethylpiperazin-1-yl)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)-2,6-dimethylpiperazin-1-yl)methyl)piperidin-1-yl)but-2-enamide |
| | 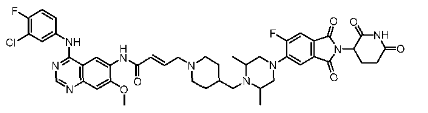 | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)but-2-enamide |
| | 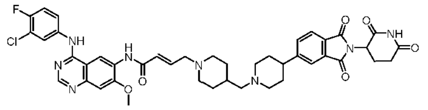 | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)piperidin-1-yl)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)piperidin-1-yl)methyl)piperidin-1-yl)but-2-enamide |
| | 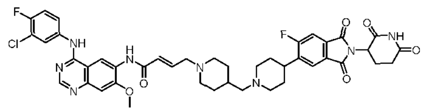 | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)piperidin-1-yl)methyl)piperidin-1-yl)but-2-enamide | (E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)piperidin-1-yl)methyl)piperidin-1-yl)but-2-enamide |
| | 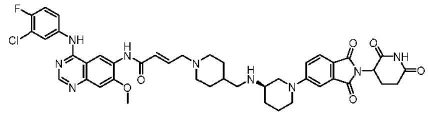 | (E)-N-(4-((3-chloro-4-fluorophenyl)amino)-7-methoxyquinazolin-6-yl)-4-(4-((((3R)-1-(2-(2,6-dioxopiperidin-3-yl)-1,3-dioxoisoindolin-5-yl)piperidin-3-yl)amino)methyl)piperidin-1-yl)but-2-enamide | (E)-N-(4-((3-chloro-4-fluorophenyl)amino)-7-methoxyquinazolin-6-yl)-4-(4-((((3R)-1-(2-(2,6-dioxopiperidin-3-yl)-1,3-dioxoisoindolin-5-yl)piperidin-3-yl)amino)methyl)piperidin-1-yl)but-2-enamide |

| | | | |
|---|---|---|---|
| | 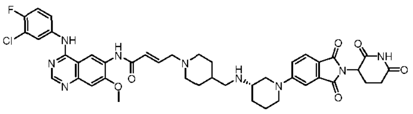 | (E)-N-(4-((3-chloro-4-fluorophenyl)amino)-7-methoxyquinazolin-6-yl)-4-(4-((((3S)-1-(2-(2,6-dioxopiperidin-3-yl)-1,3-dioxoisoindolin-5-yl)piperidin-3-yl)amino)methyl)piperidin-1-yl)but-2-enamide | (E)-N-(4-((3-chloro-4-fluorophenyl)amino)-7-methoxyquinazolin-6-yl)-4-(4-((((3S)-1-(2-(2,6-dioxopiperidin-3-yl)-1,3-dioxoisoindolin-5-yl)piperidin-3-yl)amino)methyl)piperidin-1-yl)but-2-enamide |
| | 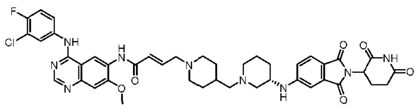 | (E)-N-(4-((3-chloro-4-fluorophenyl)amino)-7-methoxyquinazolin-6-yl)-4-(4-(((3S)-3-((2-(2,6-dioxopiperidin-3-yl)-1,3-dioxoisoindolin-5-yl)amino)piperidin-1-yl)methyl)piperidin-1-yl)but-2-enamide | (E)-N-(4-((3-chloro-4-fluorophenyl)amino)-7-methoxyquinazolin-6-yl)-4-(4-(((3S)-3-((2-(2,6-dioxopiperidin-3-yl)-1,3-dioxoisoindolin-5-yl)amino)piperidin-1-yl)methyl)piperidin-1-yl)but-2-enamide |
| | 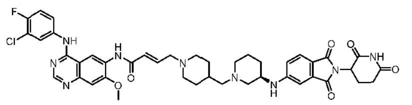 | (E)-N-(4-((3-chloro-4-fluorophenyl)amino)-7-methoxyquinazolin-6-yl)-4-(4-(((3R)-3-((2-(2,6-dioxopiperidin-3-yl)-1,3-dioxoisoindolin-5-yl)amino)piperidin-1-yl)methyl)piperidin-1-yl)but-2-enamide | (E)-N-(4-((3-chloro-4-fluorophenyl)amino)-7-methoxyquinazolin-6-yl)-4-(4-(((3R)-3-((2-(2,6-dioxopiperidin-3-yl)-1,3-dioxoisoindolin-5-yl)amino)piperidin-1-yl)methyl)piperidin-1-yl)but-2-enamide |
| | 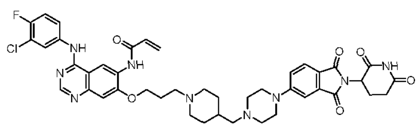 | N-(4-((3-chloro-4-fluorophenyl)amino)-7-(3-(4-((4-(2-(2,6-dioxopiperidin-3-yl)-1,3-dioxoisoindolin-5-yl)piperazin-1-yl)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-1,3-dioxoisoindolin-5-yl)piperazin-1-yl)methyl)piperidin-1-yl)propoxy)quinazolin-6-yl)acrylamide |
| | 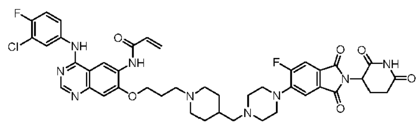 | N-(4-((3-chloro-4-fluorophenyl)amino)-7-(3-(4-((4-(2-(2,6-dioxopiperidin-3-yl)-6-fluoro-1,3-dioxoisoindolin-5-yl)piperazin-1-yl)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-6-fluoro-1,3-dioxoisoindolin-5-yl)piperazin-1-yl)methyl)piperidin-1-yl)propoxy)quinazolin-6-yl)acrylamide |
| | 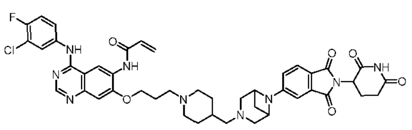 | N-(4-((3-chloro-4-fluorophenyl)amino)-7-(3-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)propoxy)quinazolin-6-yl)acrylamide | N-(4-((3-chloro-4-fluorophenyl)amino)-7-(3-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)propoxy)quinazolin-6-yl)acrylamide |
| | 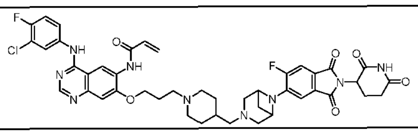 | N-(4-((3-chloro-4-fluorophenyl)amino)-7-(3-(4-((6-(2-(2,6-dioxopiperidin-3-yl)-6- | N-(4-((3-chloro-4-fluorophenyl)amino)-7-(3-(4-((6-(2-(2,6-dioxopiperidin-3-yl)-6- |

| | | | |
|---|---|---|---|
| | | fluoro-1,3-dioxoisoindolin-5-yl)-3,6-diazabicyclo[3.1.1]heptan-3-yl)methyl)piperidin-1-yl)propoxy)quinazolin-6-yl)acrylamide | fluoro-1,3-dioxoisoindolin-5-yl)-3,6-diazabicyclo[3.1.1]heptan-3-yl)methyl)piperidin-1-yl)propoxy)quinazolin-6-yl)acrylamide |
| | 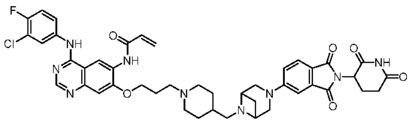 | N-(4-((3-chloro-4-fluorophenyl)amino)-7-(3-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)propoxy)quinazolin-6-yl)acrylamide | N-(4-((3-chloro-4-fluorophenyl)amino)-7-(3-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)propoxy)quinazolin-6-yl)acrylamide |
| | 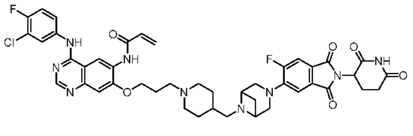 | N-(4-((3-chloro-4-fluorophenyl)amino)-7-(3-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)propoxy)quinazolin-6-yl)acrylamide | N-(4-((3-chloro-4-fluorophenyl)amino)-7-(3-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)propoxy)quinazolin-6-yl)acrylamide |
| | 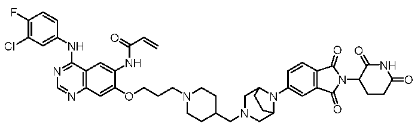 | N-(4-((3-chloro-4-fluorophenyl)amino)-7-(3-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)propoxy)quinazolin-6-yl)acrylamide | N-(4-((3-chloro-4-fluorophenyl)amino)-7-(3-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)propoxy)quinazolin-6-yl)acrylamide |
| | 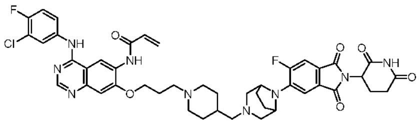 | N-(4-((3-chloro-4-fluorophenyl)amino)-7-(3-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)propoxy)quinazolin-6-yl)acrylamide | N-(4-((3-chloro-4-fluorophenyl)amino)-7-(3-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)propoxy)quinazolin-6-yl)acrylamide |
| | 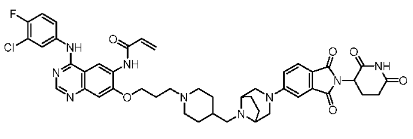 | N-(4-((3-chloro-4-fluorophenyl)amino)-7-(3-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)propoxy)quinazolin-6-yl)acrylamide | N-(4-((3-chloro-4-fluorophenyl)amino)-7-(3-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)propoxy)quinazolin-6-yl)acrylamide |
| | 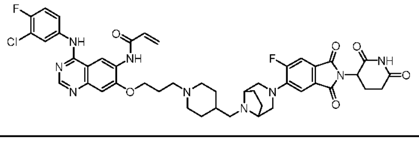 | N-(4-((3-chloro-4-fluorophenyl)amino)-7-(3-(4-((3-(2-(2,6-dioxopiperidin-3-yl)-6-fluoro-1,3-dioxoisoindolin- | N-(4-((3-chloro-4-fluorophenyl)amino)-7-(3-(4-((3-(2-(2,6-dioxopiperidin-3-yl)-6-fluoro-1,3-dioxoisoindolin- |

| | | | |
|---|---|---|---|
| | | 5-yl)-3,8-diazabicyclo[3.2.1]octan-8-yl)methyl)piperidin-1-yl)propoxy)quinazolin-6-yl)acrylamide | 5-yl)-3,8-diazabicyclo[3.2.1]octan-8-yl)methyl)piperidin-1-yl)propoxy)quinazolin-6-yl)acrylamide |
| | 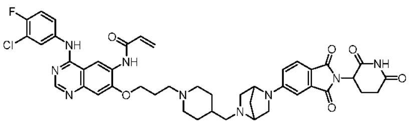 | N-(4-((3-chloro-4-fluorophenyl)amino)-7-(3-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)propoxy)quinazolin-6-yl)acrylamide | N-(4-((3-chloro-4-fluorophenyl)amino)-7-(3-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)propoxy)quinazolin-6-yl)acrylamide |
| | 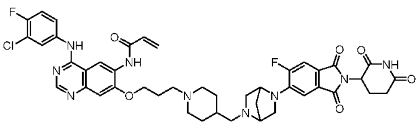 | N-(4-((3-chloro-4-fluorophenyl)amino)-7-(3-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)propoxy)quinazolin-6-yl)acrylamide | N-(4-((3-chloro-4-fluorophenyl)amino)-7-(3-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)propoxy)quinazolin-6-yl)acrylamide |
| | 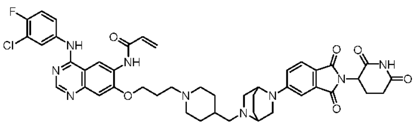 | N-(4-((3-chloro-4-fluorophenyl)amino)-7-(3-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)propoxy)quinazolin-6-yl)acrylamide | N-(4-((3-chloro-4-fluorophenyl)amino)-7-(3-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)propoxy)quinazolin-6-yl)acrylamide |
| | 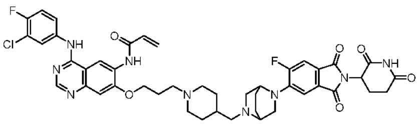 | N-(4-((3-chloro-4-fluorophenyl)amino)-7-(3-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)propoxy)quinazolin-6-yl)acrylamide | N-(4-((3-chloro-4-fluorophenyl)amino)-7-(3-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)propoxy)quinazolin-6-yl)acrylamide |
| | 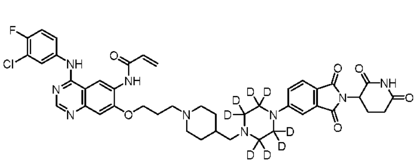 | N-(4-((3-chloro-4-fluorophenyl)amino)-7-(3-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)propoxy)quinazolin-6-yl)acrylamide |
| | 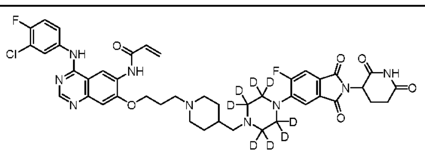 | N-(4-((3-chloro-4-fluorophenyl)amino)-7-(3-(4-((4-(2-(2,6-dioxopiperidin-3-yl)-6-fluoro-1,3-dioxoisoindolin- | N-(4-((3-chloro-4-fluorophenyl)amino)-7-(3-(4-((4-(2-(2,6-dioxopiperidin-3-yl)-6-fluoro-1,3-dioxoisoindolin- |

| | | | |
|---|---|---|---|
| | | 5-yl)piperazin-1-yl-2,2,3,3,5,5,6,6-*d*$_8$)methyl)piperidin-1-yl)propoxy)quinazolin-6-yl)acrylamide | 5-yl)piperazin-1-yl-2,2,3,3,5,5,6,6-*d*$_8$)methyl)piperidin-1-yl)propoxy)quinazolin-6-yl)acrylamide |
| | 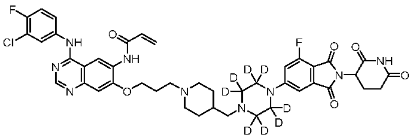 | N-(4-((3-chloro-4-fluorophenyl)amino)-7-(3-(4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide |
| | 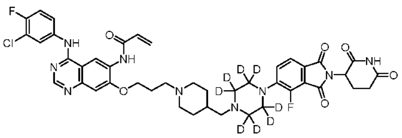 | N-(4-((3-chloro-4-fluorophenyl)amino)-7-(3-(4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide |
| | 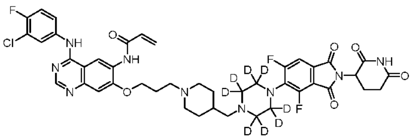 | N-(4-((3-chloro-4-fluorophenyl)amino)-7-(3-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide |
| | 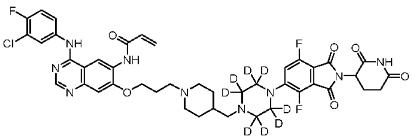 | N-(4-((3-chloro-4-fluorophenyl)amino)-7-(3-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide |
| | 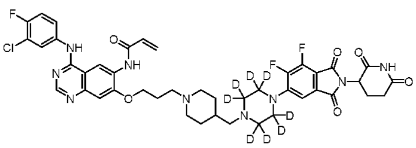 | N-(4-((3-chloro-4-fluorophenyl)amino)-7-(3-(4-((4-(2-(2,6-dioxopiperidin-3-yl)-6,7-difluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-6,7-difluoro-1,3-dioxoisoindolin-5-yl)piperazin-1-yl-2,2,3,3,5,5,6,6-*d*$_8$)methyl)piperidin-1-yl)propoxy)quinazolin-6-yl)acrylamide |

| | | | |
|---|---|---|---|
| | 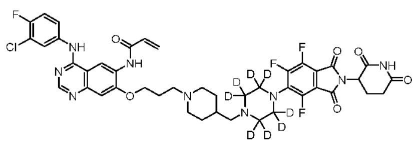 | N-(4-((3-chloro-4-fluorophenyl)amino)-7-(3-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)propoxy)quinazolin-6-yl)acrylamide |
| | 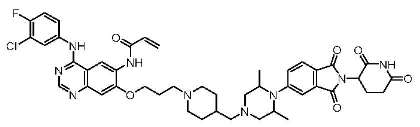 | N-(4-((3-chloro-4-fluorophenyl)amino)-7-(3-(4-((4-(2-(2,6-dioxopiperidin-3-yl)-1,3-dioxoisoindolin-5-yl)-3,5-dimethylpiperazin-1-yl)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-1,3-dioxoisoindolin-5-yl)-3,5-dimethylpiperazin-1-yl)methyl)piperidin-1-yl)propoxy)quinazolin-6-yl)acrylamide |
| | 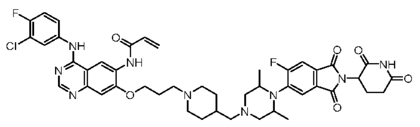 | N-(4-((3-chloro-4-fluorophenyl)amino)-7-(3-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)propoxy)quinazolin-6-yl)acrylamide |
| | 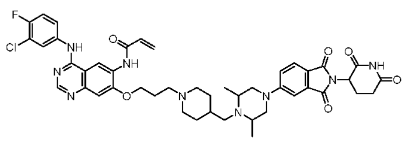 | N-(4-((3-chloro-4-fluorophenyl)amino)-7-(3-(4-((4-(2-(2,6-dioxopiperidin-3-yl)-1,3-dioxoisoindolin-5-yl)-2,6-dimethylpiperazin-1-yl)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-1,3-dioxoisoindolin-5-yl)-2,6-dimethylpiperazin-1-yl)methyl)piperidin-1-yl)propoxy)quinazolin-6-yl)acrylamide |
| | 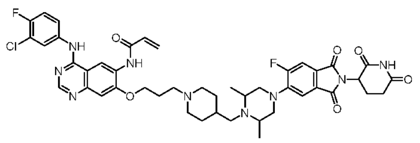 | N-(4-((3-chloro-4-fluorophenyl)amino)-7-(3-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)propoxy)quinazolin-6-yl)acrylamide |
| | 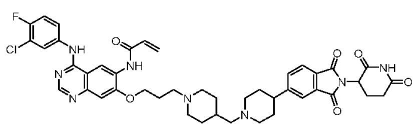 | N-(4-((3-chloro-4-fluorophenyl)amino)-7-(3-(4-((4-(2-(2,6-dioxopiperidin-3-yl)-1,3-dioxoisoindolin-5-yl)piperidin-1-yl)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-1,3-dioxoisoindolin-5-yl)piperidin-1-yl)methyl)piperidin-1-yl)propoxy)quinazolin-6-yl)acrylamide |

| | | | |
|---|---|---|---|
| | 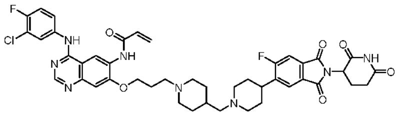 | N-(4-((3-chloro-4-fluorophenyl)amino)-7-(3-(4-((4-(2-(2,6-dioxopiperidin-3-yl)-6-fluoro-1,3-dioxoisoindolin-5-yl)piperidin-1-yl)methyl)piperidin-1-yl)propoxy)quinazolin-6-yl)acrylamide | N-(4-((3-chloro-4-fluorophenyl)amino)-7-(3-(4-((4-(2-(2,6-dioxopiperidin-3-yl)-6-fluoro-1,3-dioxoisoindolin-5-yl)piperidin-1-yl)methyl)piperidin-1-yl)propoxy)quinazolin-6-yl)acrylamide |
| | 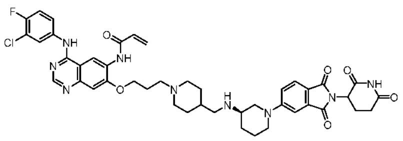 | N-(4-((3-chloro-4-fluorophenyl)amino)-7-(3-(4-((((3R)-1-(2-(2,6-dioxopiperidin-3-yl)-1,3-dioxoisoindolin-5-yl)piperidin-3-yl)amino)methyl)piperidin-1-yl)propoxy)quinazolin-6-yl)acrylamide | N-(4-((3-chloro-4-fluorophenyl)amino)-7-(3-(4-((((3R)-1-(2-(2,6-dioxopiperidin-3-yl)-1,3-dioxoisoindolin-5-yl)piperidin-3-yl)amino)methyl)piperidin-1-yl)propoxy)quinazolin-6-yl)acrylamide |
| | 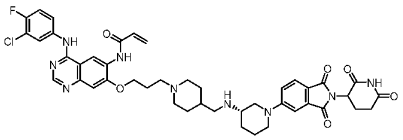 | N-(4-((3-chloro-4-fluorophenyl)amino)-7-(3-(4-((((3S)-1-(2-(2,6-dioxopiperidin-3-yl)-1,3-dioxoisoindolin-5-yl)piperidin-3-yl)amino)methyl)piperidin-1-yl)propoxy)quinazolin-6-yl)acrylamide | N-(4-((3-chloro-4-fluorophenyl)amino)-7-(3-(4-((((3S)-1-(2-(2,6-dioxopiperidin-3-yl)-1,3-dioxoisoindolin-5-yl)piperidin-3-yl)amino)methyl)piperidin-1-yl)propoxy)quinazolin-6-yl)acrylamide |
| | 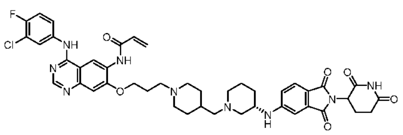 | N-(4-((3-chloro-4-fluorophenyl)amino)-7-(3-(4-(((3S)-3-((2-(2,6-dioxopiperidin-3-yl)-1,3-dioxoisoindolin-5-yl)amino)piperidin-1-yl)methyl)piperidin-1-yl)propoxy)quinazolin-6-yl)acrylamide | N-(4-((3-chloro-4-fluorophenyl)amino)-7-(3-(4-(((3S)-3-((2-(2,6-dioxopiperidin-3-yl)-1,3-dioxoisoindolin-5-yl)amino)piperidin-1-yl)methyl)piperidin-1-yl)propoxy)quinazolin-6-yl)acrylamide |
| | 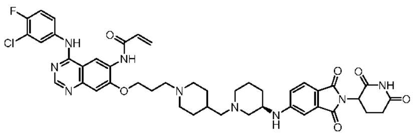 | N-(4-((3-chloro-4-fluorophenyl)amino)-7-(3-(4-(((3R)-3-((2-(2,6-dioxopiperidin-3-yl)-1,3-dioxoisoindolin-5-yl)amino)piperidin-1-yl)methyl)piperidin-1-yl)propoxy)quinazolin-6-yl)acrylamide | N-(4-((3-chloro-4-fluorophenyl)amino)-7-(3-(4-(((3R)-3-((2-(2,6-dioxopiperidin-3-yl)-1,3-dioxoisoindolin-5-yl)amino)piperidin-1-yl)methyl)piperidin-1-yl)propoxy)quinazolin-6-yl)acrylamide |
| | 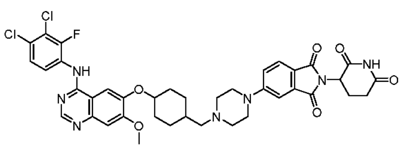 | 5-(4-((4-((4-((3,4-dichloro-2-fluorophenyl)amino)-7-methoxyquinazolin-6-yl)oxy)cyclohexyl)methyl)piperazin-1-yl)-2-(2,6-dioxopiperidin-3-yl)isoindoline-1,3-dione | 5-(4-((4-((4-((3,4-dichloro-2-fluorophenyl)amino)-7-methoxyquinazolin-6-yl)oxy)cyclohexyl)methyl)piperazin-1-yl)-2-(2,6-dioxopiperidin-3-yl)isoindoline-1,3-dione |
| | 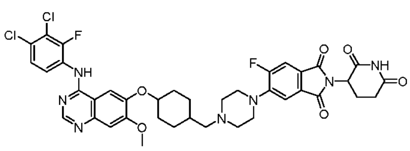 | 5-(4-((4-((4-((3,4-dichloro-2-fluorophenyl)amino)-7-methoxyquinazolin-6-yl)oxy)cyclohexyl)methyl)piperazin-1-yl)-2-(2,6-dioxopiperidin-3-yl)-6- | 5-(4-((4-((4-((3,4-dichloro-2-fluorophenyl)amino)-7-methoxyquinazolin-6-yl)oxy)cyclohexyl)methyl)piperazin-1-yl)-2-(2,6-dioxopiperidin-3-yl)-6- |

| | | | |
|---|---|---|---|
| | | fluoroisoindoline-1,3-dione | fluoroisoindoline-1,3-dione |
| | 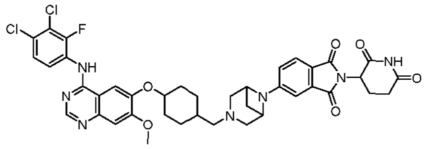 | 5-(3-((4-((4-((3,4-dichloro-2-fluorophenyl)amino)-7-methoxyquinazolin-6-yl)oxy)cyclohexyl)methyl)-3,6-diazabicyclo[3.1.1]heptan-6-yl)-2-(2,6-dioxopiperidin-3-yl)isoindoline-1,3-dione | 5-(3-((4-((4-((3,4-dichloro-2-fluorophenyl)amino)-7-methoxyquinazolin-6-yl)oxy)cyclohexyl)methyl)-3,6-diazabicyclo[3.1.1]heptan-6-yl)-2-(2,6-dioxopiperidin-3-yl)isoindoline-1,3-dione |
| | 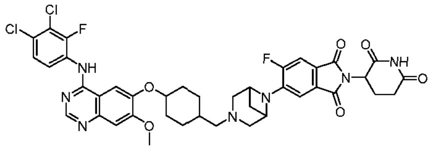 | 5-(3-((4-((4-((3,4-dichloro-2-fluorophenyl)amino)-7-methoxyquinazolin-6-yl)oxy)cyclohexyl)methyl)-3,6-diazabicyclo[3.1.1]heptan-6-yl)-2-(2,6-dioxopiperidin-3-yl)-6-fluoroisoindoline-1,3-dione | 5-(3-((4-((4-((3,4-dichloro-2-fluorophenyl)amino)-7-methoxyquinazolin-6-yl)oxy)cyclohexyl)methyl)-3,6-diazabicyclo[3.1.1]heptan-6-yl)-2-(2,6-dioxopiperidin-3-yl)-6-fluoroisoindoline-1,3-dione |
| | 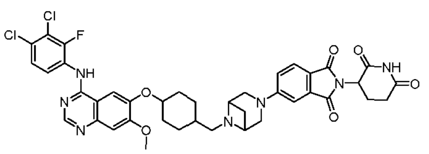 | 5-(6-((4-((4-((3,4-dichloro-2-fluorophenyl)amino)-7-methoxyquinazolin-6-yl)oxy)cyclohexyl)methyl)-3,6-diazabicyclo[3.1.1]heptan-3-yl)-2-(2,6-dioxopiperidin-3-yl)isoindoline-1,3-dione | 5-(6-((4-((4-((3,4-dichloro-2-fluorophenyl)amino)-7-methoxyquinazolin-6-yl)oxy)cyclohexyl)methyl)-3,6-diazabicyclo[3.1.1]heptan-3-yl)-2-(2,6-dioxopiperidin-3-yl)isoindoline-1,3-dione |
| | 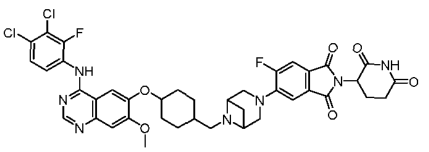 | 5-(6-((4-((4-((3,4-dichloro-2-fluorophenyl)amino)-7-methoxyquinazolin-6-yl)oxy)cyclohexyl)methyl)-3,6-diazabicyclo[3.1.1]heptan-3-yl)-2-(2,6-dioxopiperidin-3-yl)-6-fluoroisoindoline-1,3-dione | 5-(6-((4-((4-((3,4-dichloro-2-fluorophenyl)amino)-7-methoxyquinazolin-6-yl)oxy)cyclohexyl)methyl)-3,6-diazabicyclo[3.1.1]heptan-3-yl)-2-(2,6-dioxopiperidin-3-yl)-6-fluoroisoindoline-1,3-dione |
| | 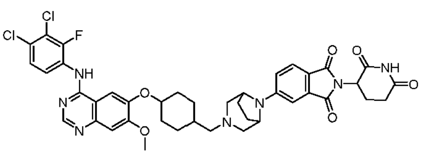 | 5-(3-((4-((4-((3,4-dichloro-2-fluorophenyl)amino)-7-methoxyquinazolin-6-yl)oxy)cyclohexyl)methyl)-3,8-diazabicyclo[3.2.1]octan-8-yl)-2-(2,6-dioxopiperidin-3-yl)isoindoline-1,3-dione | 5-(3-((4-((4-((3,4-dichloro-2-fluorophenyl)amino)-7-methoxyquinazolin-6-yl)oxy)cyclohexyl)methyl)-3,8-diazabicyclo[3.2.1]octan-8-yl)-2-(2,6-dioxopiperidin-3-yl)isoindoline-1,3-dione |
| | 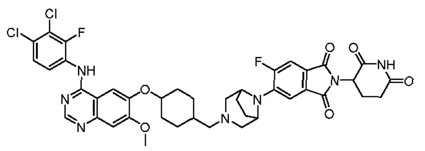 | 5-(3-((4-((4-((3,4-dichloro-2-fluorophenyl)amino)-7-methoxyquinazolin-6-yl)oxy)cyclohexyl)methyl)-3,8-diazabicyclo[3.2.1]octan-8-yl)-2-(2,6-dioxopiperidin-3-yl)-6-fluoroisoindoline-1,3-dione | 5-(3-((4-((4-((3,4-dichloro-2-fluorophenyl)amino)-7-methoxyquinazolin-6-yl)oxy)cyclohexyl)methyl)-3,8-diazabicyclo[3.2.1]octan-8-yl)-2-(2,6-dioxopiperidin-3-yl)-6-fluoroisoindoline-1,3-dione |

| | | | |
|---|---|---|---|
| | 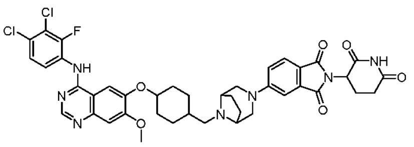 | 5-(8-((4-((4-((3,4-dichloro-2-fluorophenyl)amino)-7-methoxyquinazolin-6-yl)oxy)cyclohexyl)methyl)-3,8-diazabicyclo[3.2.1]octan-3-yl)-2-(2,6-dioxopiperidin-3-yl)isoindoline-1,3-dione | 5-(8-((4-((4-((3,4-dichloro-2-fluorophenyl)amino)-7-methoxyquinazolin-6-yl)oxy)cyclohexyl)methyl)-3,8-diazabicyclo[3.2.1]octan-3-yl)-2-(2,6-dioxopiperidin-3-yl)isoindoline-1,3-dione |
| | 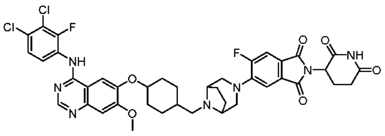 | 5-(8-((4-((4-((3,4-dichloro-2-fluorophenyl)amino)-7-methoxyquinazolin-6-yl)oxy)cyclohexyl)methyl)-3,8-diazabicyclo[3.2.1]octan-3-yl)-2-(2,6-dioxopiperidin-3-yl)-6-fluoroisoindoline-1,3-dione | 5-(8-((4-((4-((3,4-dichloro-2-fluorophenyl)amino)-7-methoxyquinazolin-6-yl)oxy)cyclohexyl)methyl)-3,8-diazabicyclo[3.2.1]octan-3-yl)-2-(2,6-dioxopiperidin-3-yl)-6-fluoroisoindoline-1,3-dione |
| | 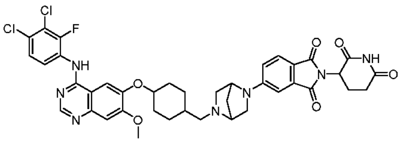 | 5-(5-((4-((4-((3,4-dichloro-2-fluorophenyl)amino)-7-methoxyquinazolin-6-yl)oxy)cyclohexyl)methyl)-2,5-diazabicyclo[2.2.1]heptan-2-yl)-2-(2,6-dioxopiperidin-3-yl)isoindoline-1,3-dione | 5-(5-((4-((4-((3,4-dichloro-2-fluorophenyl)amino)-7-methoxyquinazolin-6-yl)oxy)cyclohexyl)methyl)-2,5-diazabicyclo[2.2.1]heptan-2-yl)-2-(2,6-dioxopiperidin-3-yl)isoindoline-1,3-dione |
| | 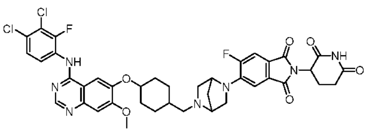 | 5-(5-((4-((4-((3,4-dichloro-2-fluorophenyl)amino)-7-methoxyquinazolin-6-yl)oxy)cyclohexyl)methyl)-2,5-diazabicyclo[2.2.1]heptan-2-yl)-2-(2,6-dioxopiperidin-3-yl)-6-fluoroisoindoline-1,3-dione | 5-(5-((4-((4-((3,4-dichloro-2-fluorophenyl)amino)-7-methoxyquinazolin-6-yl)oxy)cyclohexyl)methyl)-2,5-diazabicyclo[2.2.1]heptan-2-yl)-2-(2,6-dioxopiperidin-3-yl)-6-fluoroisoindoline-1,3-dione |
| | 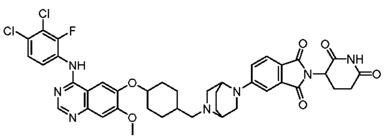 | 5-(5-((4-((4-((3,4-dichloro-2-fluorophenyl)amino)-7-methoxyquinazolin-6-yl)oxy)cyclohexyl)methyl)-2,5-diazabicyclo[2.2.2]octan-2-yl)-2-(2,6-dioxopiperidin-3-yl)isoindoline-1,3-dione | 5-(5-((4-((4-((3,4-dichloro-2-fluorophenyl)amino)-7-methoxyquinazolin-6-yl)oxy)cyclohexyl)methyl)-2,5-diazabicyclo[2.2.2]octan-2-yl)-2-(2,6-dioxopiperidin-3-yl)isoindoline-1,3-dione |
| | 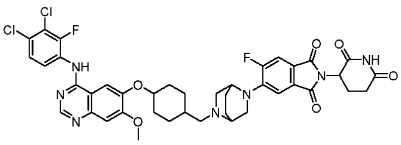 | 5-(5-((4-((4-((3,4-dichloro-2-fluorophenyl)amino)-7-methoxyquinazolin-6-yl)oxy)cyclohexyl)methyl)-2,5-diazabicyclo[2.2.2]octan-2-yl)-2-(2,6-dioxopiperidin-3-yl)-6-fluoroisoindoline-1,3-dione | 5-(5-((4-((4-((3,4-dichloro-2-fluorophenyl)amino)-7-methoxyquinazolin-6-yl)oxy)cyclohexyl)methyl)-2,5-diazabicyclo[2.2.2]octan-2-yl)-2-(2,6-dioxopiperidin-3-yl)-6-fluoroisoindoline-1,3-dione |
| | 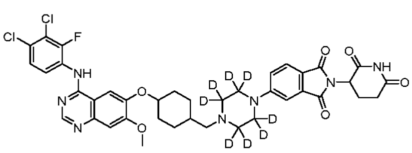 | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3- | 5-(4-((4-((4-((3,4-dichloro-2-fluorophenyl)amino)-7-methoxyquinazolin-6-yl)oxy)cyclohexyl)methyl) piperazin-1-yl-2,2,3,3,5,5,6,6-$d_8$)-2-(2,6- |

| | | | |
|---|---|---|---|
| | | yl)isoindoline-1,3-dione | dioxopiperidin-3-yl)isoindoline-1,3-dione |
| | 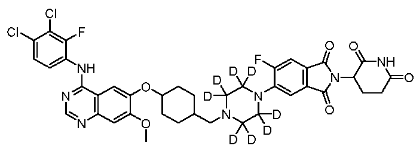 | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-6-fluoroisoindoline-1,3-dione | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-6-fluoroisoindoline-1,3-dione |
| | 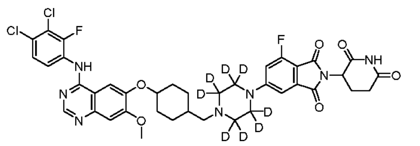 | 6-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4-fluoroisoindoline-1,3-dione | 6-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4-fluoroisoindoline-1,3-dione |
| | 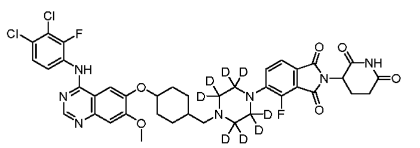 | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4-fluoroisoindoline-1,3-dione | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4-fluoroisoindoline-1,3-dione |
| | 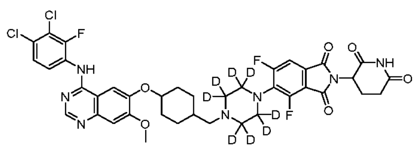 | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4,6-difluoroisoindoline-1,3-dione | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4,6-difluoroisoindoline-1,3-dione |
| | 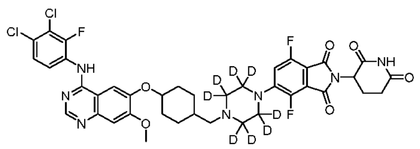 | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4,7-difluoroisoindoline-1,3-dione | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4,7-difluoroisoindoline-1,3-dione |
| | 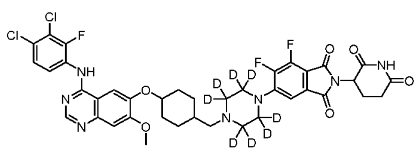 | 6-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4,5-difluoroisoindoline-1,3-dione | 6-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4,5-difluoroisoindoline-1,3-dione |
| | 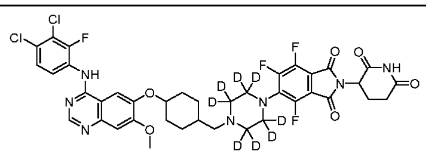 | 5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6- | 5-(4-((4-((4-((3,4-dichloro-2-fluorophenyl)amino)-7-methoxyquinazolin-6-yl)oxy)cyclohexyl)methyl)piperazin-1-yl- |

| | | | |
|---|---|---|---|
| | | $d_8$)-2-(2,6-dioxopiperidin-3-yl)-4,6,7-trifluoroisoindoline-1,3-dione | 2,2,3,3,5,5,6,6-$d_8$)-2-(2,6-dioxopiperidin-3-yl)-4,6,7-trifluoroisoindoline-1,3-dione |
| | 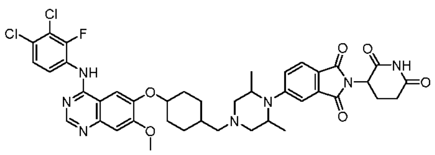 | 5-(4-((4-((4-((3,4-dichloro-2-fluorophenyl)amino)-7-methoxyquinazolin-6-yl)oxy)cyclohexyl)methyl)-2,6-dimethylpiperazin-1-yl)-2-(2,6-dioxopiperidin-3-yl)isoindoline-1,3-dione | 5-(4-((4-((4-((3,4-dichloro-2-fluorophenyl)amino)-7-methoxyquinazolin-6-yl)oxy)cyclohexyl)methyl)-2,6-dimethylpiperazin-1-yl)-2-(2,6-dioxopiperidin-3-yl)isoindoline-1,3-dione |
| | 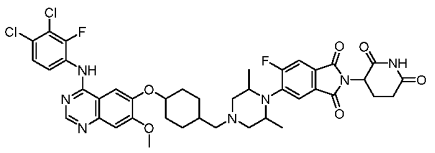 | 5-(4-((4-((4-((3,4-dichloro-2-fluorophenyl)amino)-7-methoxyquinazolin-6-yl)oxy)cyclohexyl)methyl)-2,6-dimethylpiperazin-1-yl)-2-(2,6-dioxopiperidin-3-yl)-6-fluoroisoindoline-1,3-dione | 5-(4-((4-((4-((3,4-dichloro-2-fluorophenyl)amino)-7-methoxyquinazolin-6-yl)oxy)cyclohexyl)methyl)-2,6-dimethylpiperazin-1-yl)-2-(2,6-dioxopiperidin-3-yl)-6-fluoroisoindoline-1,3-dione |
| | 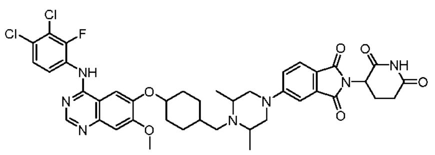 | 5-(4-((4-((4-((3,4-dichloro-2-fluorophenyl)amino)-7-methoxyquinazolin-6-yl)oxy)cyclohexyl)methyl)-3,5-dimethylpiperazin-1-yl)-2-(2,6-dioxopiperidin-3-yl)isoindoline-1,3-dione | 5-(4-((4-((4-((3,4-dichloro-2-fluorophenyl)amino)-7-methoxyquinazolin-6-yl)oxy)cyclohexyl)methyl)-3,5-dimethylpiperazin-1-yl)-2-(2,6-dioxopiperidin-3-yl)isoindoline-1,3-dione |
| | 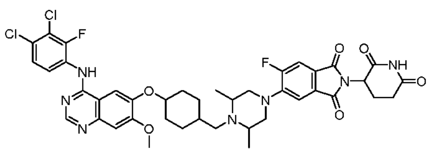 | 5-(4-((4-((4-((3,4-dichloro-2-fluorophenyl)amino)-7-methoxyquinazolin-6-yl)oxy)cyclohexyl)methyl)-3,5-dimethylpiperazin-1-yl)-2-(2,6-dioxopiperidin-3-yl)-6-fluoroisoindoline-1,3-dione | 5-(4-((4-((4-((3,4-dichloro-2-fluorophenyl)amino)-7-methoxyquinazolin-6-yl)oxy)cyclohexyl)methyl)-3,5-dimethylpiperazin-1-yl)-2-(2,6-dioxopiperidin-3-yl)-6-fluoroisoindoline-1,3-dione |
| | 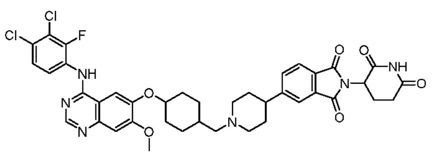 | 5-(1-((4-((4-((3,4-dichloro-2-fluorophenyl)amino)-7-methoxyquinazolin-6-yl)oxy)cyclohexyl)methyl)piperidin-4-yl)-2-(2,6-dioxopiperidin-3-yl)isoindoline-1,3-dione | 5-(1-((4-((4-((3,4-dichloro-2-fluorophenyl)amino)-7-methoxyquinazolin-6-yl)oxy)cyclohexyl)methyl) piperidin-4-yl)-2-(2,6-dioxopiperidin-3-yl)isoindoline-1,3-dione |
| | 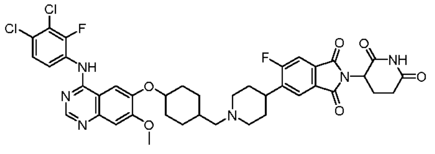 | 5-(1-((4-((4-((3,4-dichloro-2-fluorophenyl)amino)-7-methoxyquinazolin-6-yl)oxy)cyclohexyl)methyl)piperidin-4-yl)-2-(2,6-dioxopiperidin-3-yl)-6-fluoroisoindoline-1,3-dione | 5-(1-((4-((4-((3,4-dichloro-2-fluorophenyl)amino)-7-methoxyquinazolin-6-yl)oxy)cyclohexyl)methyl) piperidin-4-yl)-2-(2,6-dioxopiperidin-3-yl)-6-fluoroisoindoline-1,3-dione |
| | 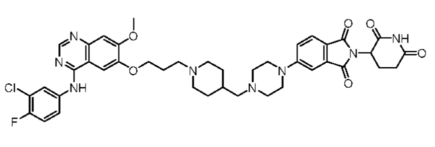 | 5-(4-((1-(3-((4-((3-chloro-4-fluorophenyl)amino)-7-methoxyquinazolin-6-yl)oxy)propyl)piperidin-4-yl)methyl)piperazin-1-yl)-2-(2,6-dioxopiperidin-3-yl)isoindoline-1,3-dione | 5-(4-((1-(3-((4-((3-chloro-4-fluorophenyl)amino)-7-methoxyquinazolin-6-yl)oxy)propyl)piperidin-4-yl)methyl)piperazin-1-yl)-2-(2,6-dioxopiperidin-3-yl)isoindoline-1,3-dione |

| | | | |
|---|---|---|---|
| | 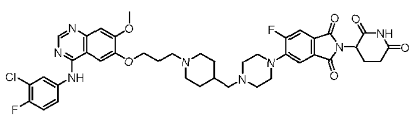 | 5-(4-((1-(3-((4-((3-chloro-4-fluorophenyl)amino)-7-methoxyquinazolin-6-yl)oxy)propyl)piperidin-4-yl)methyl)piperazin-1-yl)-2-(2,6-dioxopiperidin-3-yl)-6-fluoroisoindoline-1,3-dione | 5-(4-((1-(3-((4-((3-chloro-4-fluorophenyl)amino)-7-methoxyquinazolin-6-yl)oxy)propyl)piperidin-4-yl)methyl)piperazin-1-yl)-2-(2,6-dioxopiperidin-3-yl)-6-fluoroisoindoline-1,3-dione |
| | 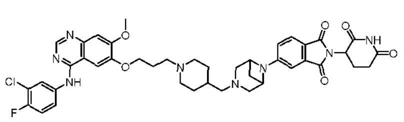 | 5-(3-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-6-yl)-2-(2,6-dioxopiperidin-3-yl)isoindoline-1,3-dione |
| | 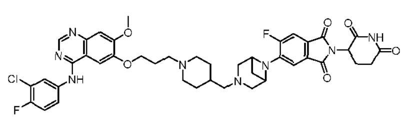 | 5-(3-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 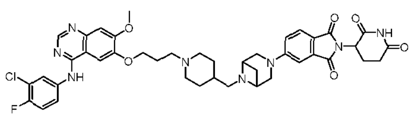 | 5-(6-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-3-yl)-2-(2,6-dioxopiperidin-3-yl)isoindoline-1,3-dione |
| | 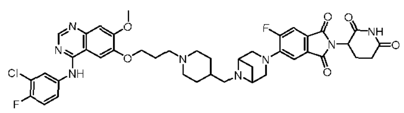 | 5-(6-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-3-yl)-2-(2,6-dioxopiperidin-3-yl)-6-fluoroisoindoline-1,3-dione | 5-(6-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-3-yl)-2-(2,6-dioxopiperidin-3-yl)-6-fluoroisoindoline-1,3-dione |
| | 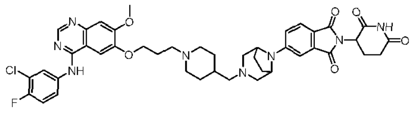 | 5-(3-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-8-yl)-2-(2,6-dioxopiperidin-3-yl)isoindoline-1,3-dione | 5-(3-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-8-yl)-2-(2,6-dioxopiperidin-3-yl)isoindoline-1,3-dione |
| | 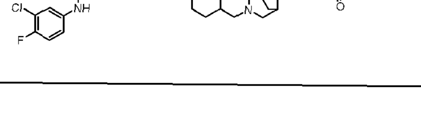 | 5-(3-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-8- | 5-(3-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-8- |

| | | | |
|---|---|---|---|
| | | yl)-2-(2,6-dioxopiperidin-3-yl)-6-fluoroisoindoline-1,3-dione | yl)-2-(2,6-dioxopiperidin-3-yl)-6-fluoroisoindoline-1,3-dione |
| | 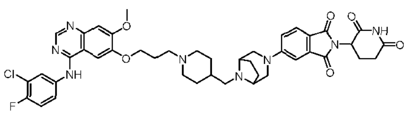 | 5-(8-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-3-yl)-2-(2,6-dioxopiperidin-3-yl)isoindoline-1,3-dione | 5-(8-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-3-yl)-2-(2,6-dioxopiperidin-3-yl)isoindoline-1,3-dione |
| | 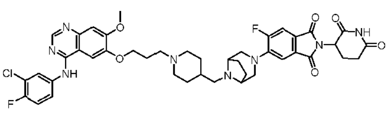 | 5-(8-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 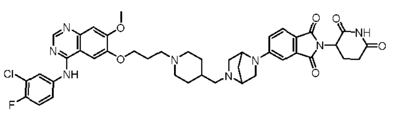 | 5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.1]heptan-2-yl)-2-(2,6-dioxopiperidin-3-yl)isoindoline-1,3-dione |
| | 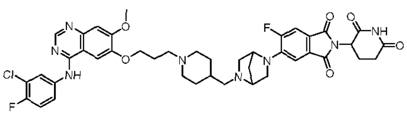 | 5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 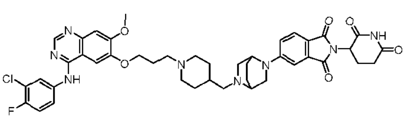 | 5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.2]octan-2-yl)-2-(2,6-dioxopiperidin-3-yl)isoindoline-1,3-dione | 5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.2]octan-2-yl)-2-(2,6-dioxopiperidin-3-yl)isoindoline-1,3-dione |
| | 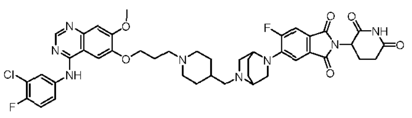 | 5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 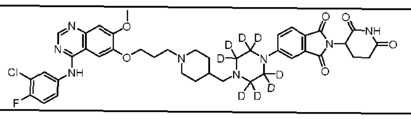 | 5-(4-((1-(3-((4-((3-chloro-4-fluorophenyl)amino)-7-methoxyquinazolin-6- | 5-(4-((1-(3-((4-((3-chloro-4-fluorophenyl)amino)-7-methoxyquinazolin-6- |

| | | | |
|---|---|---|---|
| | | yl)oxy)propyl)piperidin-4-yl)methyl)piperazin-1-yl-2,2,3,3,5,5,6,6-$d_8$)-2-(2,6-dioxopiperidin-3-yl)isoindoline-1,3-dione | yl)oxy)propyl)piperidin-4-yl)methyl)piperazin-1-yl-2,2,3,3,5,5,6,6-$d_8$)-2-(2,6-dioxopiperidin-3-yl)isoindoline-1,3-dione |
| | 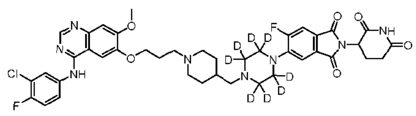 | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione |
| | 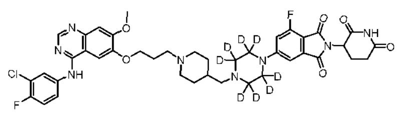 | 6-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 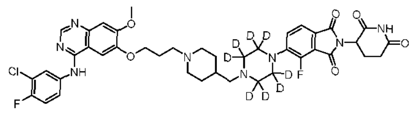 | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 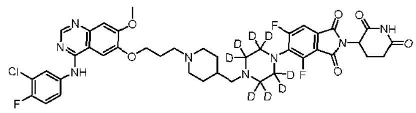 | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |
| | 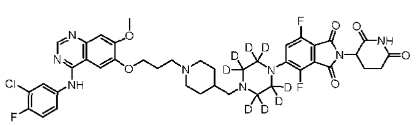 | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione |
| | 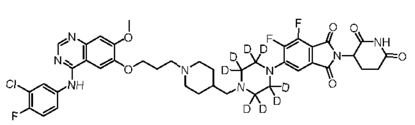 | 6-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione | 6-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione |

| | | | |
|---|---|---|---|
| | 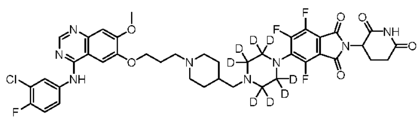 | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-*d*8)-2-(2,6-dioxopiperidin-3-yl)-4,6,7-trifluoroisoindoline-1,3-dione | 5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-*d*8)-2-(2,6-dioxopiperidin-3-yl)-4,6,7-trifluoroisoindoline-1,3-dione |
| | 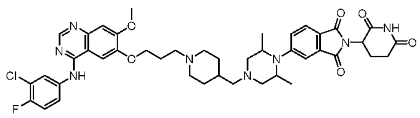 | 5-(4-((1-(3-((4-((3-chloro-4-fluorophenyl)amino)-7-methoxyquinazolin-6-yl)oxy)propyl)piperidin-4-yl)methyl)-2,6-dimethylpiperazin-1-yl)-2-(2,6-dioxopiperidin-3-yl)isoindoline-1,3-dione | 5-(4-((1-(3-((4-((3-chloro-4-fluorophenyl)amino)-7-methoxyquinazolin-6-yl)oxy)propyl)piperidin-4-yl)methyl)-2,6-dimethylpiperazin-1-yl)-2-(2,6-dioxopiperidin-3-yl)isoindoline-1,3-dione |
| | 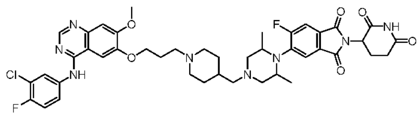 | 5-(4-((1-(3-((4-((3-chloro-4-fluorophenyl)amino)-7-methoxyquinazolin-6-yl)oxy)propyl)piperidin-4-yl)methyl)-2,6-dimethylpiperazin-1-yl)-2-(2,6-dioxopiperidin-3-yl)-6-fluoroisoindoline-1,3-dione | 5-(4-((1-(3-((4-((3-chloro-4-fluorophenyl)amino)-7-methoxyquinazolin-6-yl)oxy)propyl)piperidin-4-yl)methyl)-2,6-dimethylpiperazin-1-yl)-2-(2,6-dioxopiperidin-3-yl)-6-fluoroisoindoline-1,3-dione |
| | 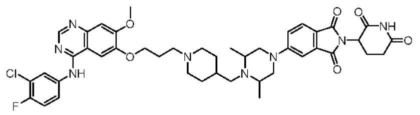 | 5-(4-((1-(3-((4-((3-chloro-4-fluorophenyl)amino)-7-methoxyquinazolin-6-yl)oxy)propyl)piperidin-4-yl)methyl)-3,5-dimethylpiperazin-1-yl)-2-(2,6-dioxopiperidin-3-yl)isoindoline-1,3-dione | 5-(4-((1-(3-((4-((3-chloro-4-fluorophenyl)amino)-7-methoxyquinazolin-6-yl)oxy)propyl)piperidin-4-yl)methyl)-3,5-dimethylpiperazin-1-yl)-2-(2,6-dioxopiperidin-3-yl)isoindoline-1,3-dione |
| | 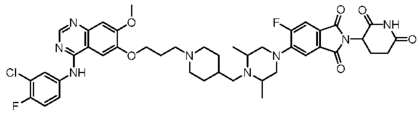 | 5-(4-((1-(3-((4-((3-chloro-4-fluorophenyl)amino)-7-methoxyquinazolin-6-yl)oxy)propyl)piperidin-4-yl)methyl)-3,5-dimethylpiperazin-1-yl)-2-(2,6-dioxopiperidin-3-yl)-6-fluoroisoindoline-1,3-dione | 5-(4-((1-(3-((4-((3-chloro-4-fluorophenyl)amino)-7-methoxyquinazolin-6-yl)oxy)propyl)piperidin-4-yl)methyl)-3,5-dimethylpiperazin-1-yl)-2-(2,6-dioxopiperidin-3-yl)-6-fluoroisoindoline-1,3-dione |
| | 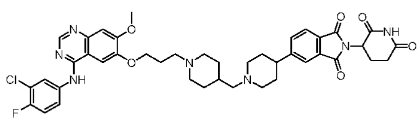 | 5-(1-((1-(3-((4-((3-chloro-4-fluorophenyl)amino)-7-methoxyquinazolin-6-yl)oxy)propyl)piperidin-4-yl)methyl)piperidin-4-yl)-2-(2,6-dioxopiperidin-3-yl)isoindoline-1,3-dione | 5-(1-((1-(3-((4-((3-chloro-4-fluorophenyl)amino)-7-methoxyquinazolin-6-yl)oxy)propyl)piperidin-4-yl)methyl)piperidin-4-yl)-2-(2,6-dioxopiperidin-3-yl)isoindoline-1,3-dione |
| | 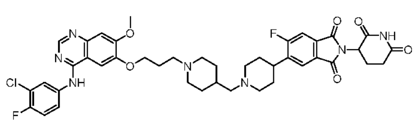 | 5-(1-((1-(3-((4-((3-chloro-4-fluorophenyl)amino)-7-methoxyquinazolin-6-yl)oxy)propyl)piperidin-4-yl)methyl)piperidin-4-yl)-2-(2,6-dioxopiperidin-3-yl)-6-fluoroisoindoline-1,3-dione | 5-(1-((1-(3-((4-((3-chloro-4-fluorophenyl)amino)-7-methoxyquinazolin-6-yl)oxy)propyl)piperidin-4-yl)methyl)piperidin-4-yl)-2-(2,6-dioxopiperidin-3-yl)-6-fluoroisoindoline-1,3-dione |

| | | | |
|---|---|---|---|
| | 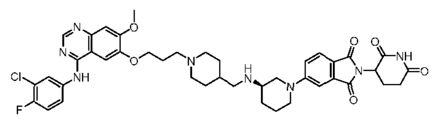 | 5-((R)-3-(((1-(3-((4-((3-chloro-4-fluorophenyl)amino)-7-methoxyquinazolin-6-yl)oxy)propyl)piperidin-4-yl)methyl)amino)piperidin-1-yl)-2-(2,6-dioxopiperidin-3-yl)isoindoline-1,3-dione | 5-((R)-3-(((1-(3-((4-((3-chloro-4-fluorophenyl)amino)-7-methoxyquinazolin-6-yl)oxy)propyl)piperidin-4-yl)methyl)amino)piperidin-1-yl)-2-(2,6-dioxopiperidin-3-yl)isoindoline-1,3-dione |
| | 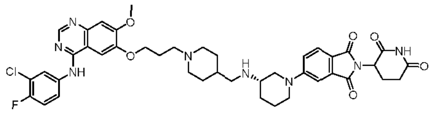 | 5-((S)-3-(((1-(3-((4-((3-chloro-4-fluorophenyl)amino)-7-methoxyquinazolin-6-yl)oxy)propyl)piperidin-4-yl)methyl)amino)piperidin-1-yl)-2-(2,6-dioxopiperidin-3-yl)isoindoline-1,3-dione | 5-((S)-3-(((1-(3-((4-((3-chloro-4-fluorophenyl)amino)-7-methoxyquinazolin-6-yl)oxy)propyl)piperidin-4-yl)methyl)amino)piperidin-1-yl)-2-(2,6-dioxopiperidin-3-yl)isoindoline-1,3-dione |
| | 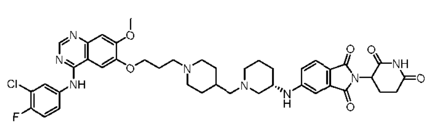 | 5-(((S)-1-((1-(3-((4-((3-chloro-4-fluorophenyl)amino)-7-methoxyquinazolin-6-yl)oxy)propyl)piperidin-4-yl)methyl)piperidin-3-yl)amino)-2-(2,6-dioxopiperidin-3-yl)isoindoline-1,3-dione | 5-(((S)-1-((1-(3-((4-((3-chloro-4-fluorophenyl)amino)-7-methoxyquinazolin-6-yl)oxy)propyl)piperidin-4-yl)methyl)piperidin-3-yl)amino)-2-(2,6-dioxopiperidin-3-yl)isoindoline-1,3-dione |
| | 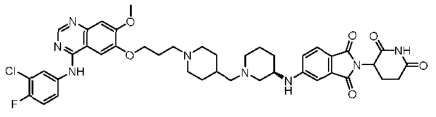 | 5-(((R)-1-((1-(3-((4-((3-chloro-4-fluorophenyl)amino)-7-methoxyquinazolin-6-yl)oxy)propyl)piperidin-4-yl)methyl)piperidin-3-yl)amino)-2-(2,6-dioxopiperidin-3-yl)isoindoline-1,3-dione | 5-(((R)-1-((1-(3-((4-((3-chloro-4-fluorophenyl)amino)-7-methoxyquinazolin-6-yl)oxy)propyl)piperidin-4-yl)methyl)piperidin-3-yl)amino)-2-(2,6-dioxopiperidin-3-yl)isoindoline-1,3-dione |
| | 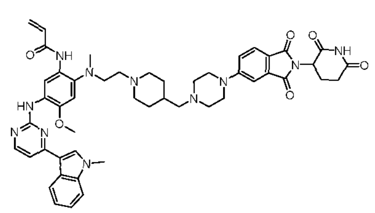 | N-(2-((2-(4-((4-(2-(2,6-dioxopiperidin-3-yl)-1,3-dioxoisoindolin-5-yl)piperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-1,3-dioxoisoindolin-5-yl)piperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 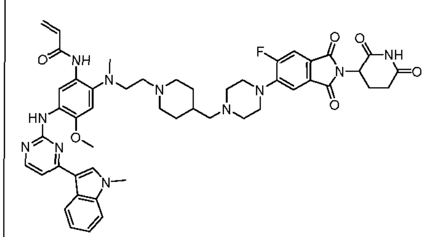 | N-(2-((2-(4-((4-(2-(2,6-dioxopiperidin-3-yl)-6-fluoro-1,3-dioxoisoindolin-5-yl)piperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-6-fluoro-1,3-dioxoisoindolin-5-yl)piperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |

| | | | |
|---|---|---|---|
| | 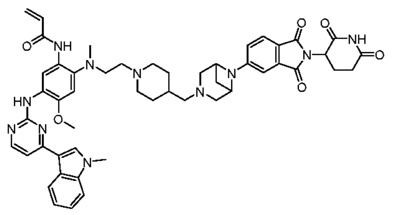 | N-(2-((2-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 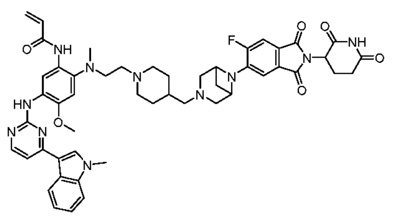 | N-(2-((2-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 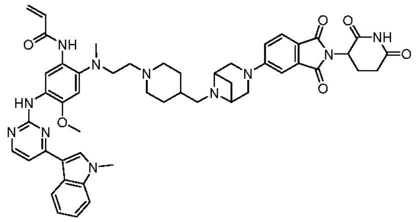 | N-(2-((2-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 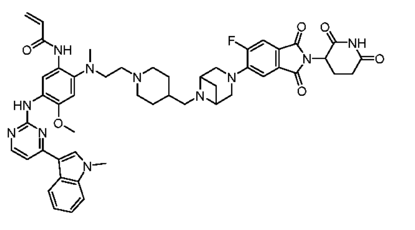 | N-(2-((2-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 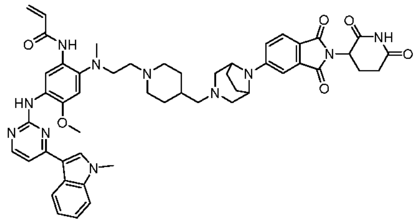 | N-(2-((2-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide |

| | | | |
|---|---|---|---|
| | 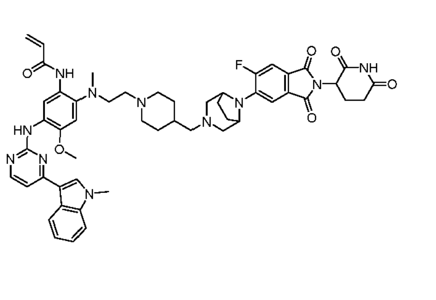 | N-(2-((2-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)ethyl)(methyl)amino)-4-methoxy-5-((4-(1-methyl-1H-indol-3-yl)pyrimidin-2-yl)amino)phenyl)acrylamid e |
| | 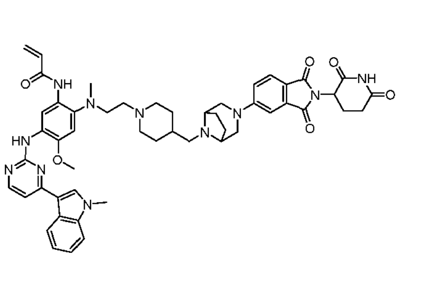 | N-(2-((2-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)ethyl)(methyl)amino)-4-methoxy-5-((4-(1-methyl-1H-indol-3-yl)pyrimidin-2-yl)amino)phenyl)acrylamid e |
| | 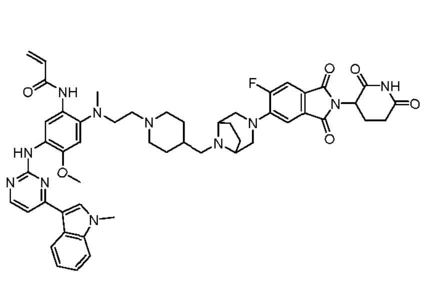 | N-(2-((2-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)ethyl)(methyl)amino)-4-methoxy-5-((4-(1-methyl-1H-indol-3-yl)pyrimidin-2-yl)amino)phenyl)acrylamid e |
| | 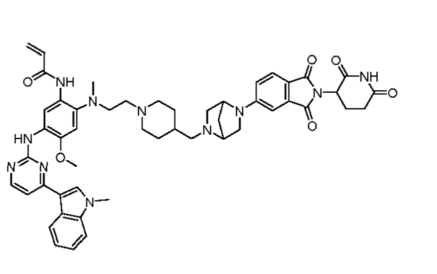 | N-(2-((2-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)ethyl)(methyl)amino)-4-methoxy-5-((4-(1-methyl-1H-indol-3-yl)pyrimidin-2-yl)amino)phenyl)acrylamid e |
| | 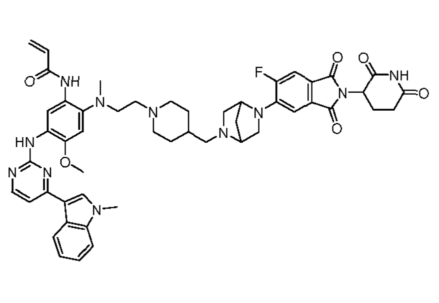 | N-(2-((2-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)ethyl)(methyl)amino)-4-methoxy-5-((4-(1-methyl-1H-indol-3-yl)pyrimidin-2-yl)amino)phenyl)acrylamid e |

| | | | |
|---|---|---|---|
| | 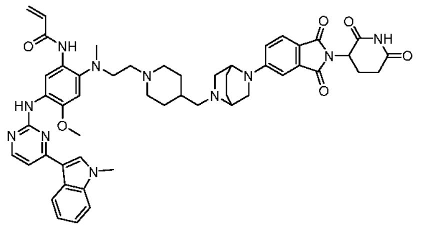 | N-(2-((2-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 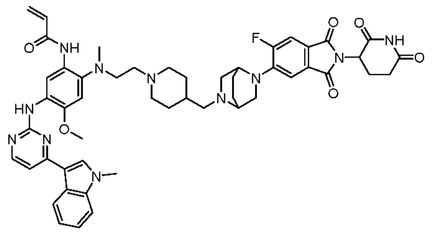 | N-(2-((2-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 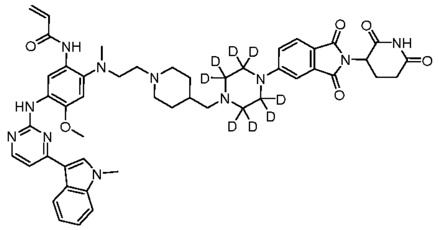 | N-(2-((2-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)ethyl)(methyl)amino)-4-methoxy-5-((4-(1-methyl-1H-indol-3-yl)pyrimidin-2-yl)amino)phenyl)acrylamide |
| | 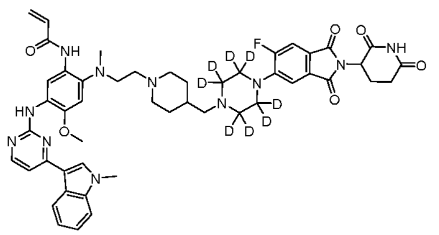 | N-(2-((2-(4-((4-(2-(2,6-dioxopiperidin-3-yl)-6-fluoro-1,3-dioxoisoindolin-5-yl)piperazin-1-yl-2,2,3,3,5,5,6,6-*d*$_8$)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-6-fluoro-1,3-dioxoisoindolin-5-yl)piperazin-1-yl-2,2,3,3,5,5,6,6-*d*$_8$)methyl)piperidin-1-yl)ethyl)(methyl)amino)-4-methoxy-5-((4-(1-methyl-1H-indol-3-yl)pyrimidin-2-yl)amino)phenyl)acrylamide |
| | 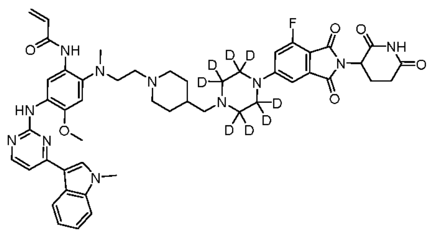 | N-(2-((2-(4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1-yl)ethyl)(methyl)amino)-4-methoxy-5-((4-(1-methyl-1H-indol-3-yl)pyrimidin-2-yl)amino)phenyl)acrylamide |

| | | | |
|---|---|---|---|
| | 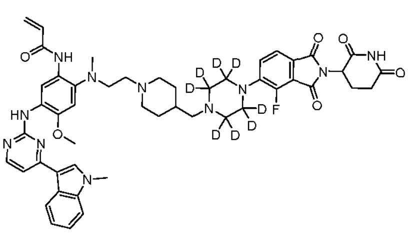 | N-(2-((2-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide |
| | 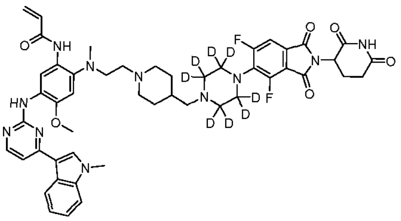 | N-(2-((2-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide |
| | 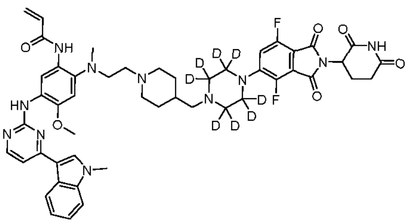 | N-(2-((2-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide |
| | 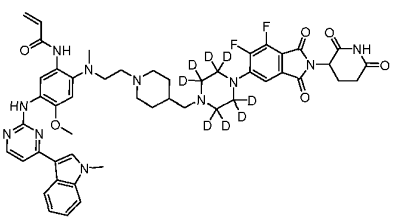 | N-(2-((2-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide |
| | 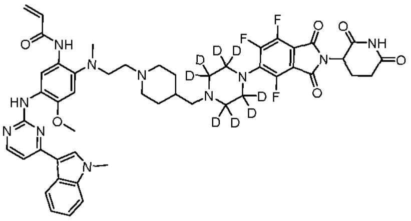 | N-(2-((2-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamid |

| | | | |
|---|---|---|---|
| | | | e |
| | 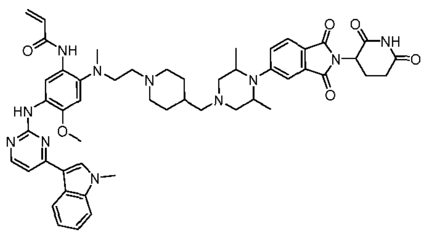 | N-(2-((2-(4-((4-(2-(2,6-dioxopiperidin-3-yl)-1,3-dioxoisoindolin-5-yl)-3,5-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-1,3-dioxoisoindolin-5-yl)-3,5-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 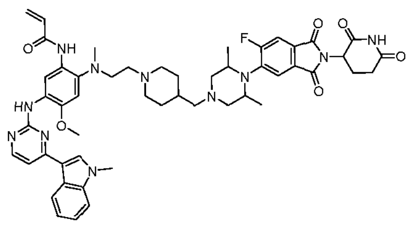 | N-(2-((2-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 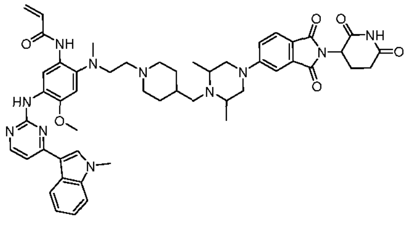 | N-(2-((2-(4-((4-(2-(2,6-dioxopiperidin-3-yl)-1,3-dioxoisoindolin-5-yl)-2,6-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-1,3-dioxoisoindolin-5-yl)-2,6-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 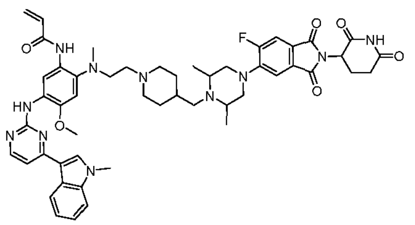 | N-(2-((2-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 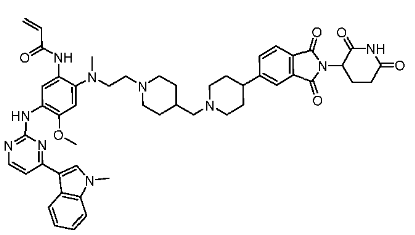 | N-(2-((2-(4-((4-(2-(2,6-dioxopiperidin-3-yl)-1,3-dioxoisoindolin-5-yl)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-1,3-dioxoisoindolin-5-yl)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |

| | | | |
|---|---|---|---|
| | 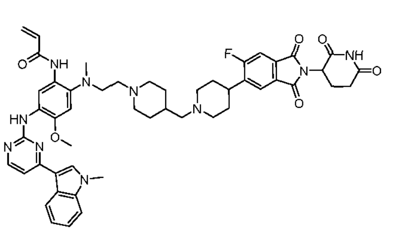 | N-(2-((2-(4-((4-(2-(2,6-dioxopiperidin-3-yl)-6-fluoro-1,3-dioxoisoindolin-5-yl)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((4-(2-(2,6-dioxopiperidin-3-yl)-6-fluoro-1,3-dioxoisoindolin-5-yl)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 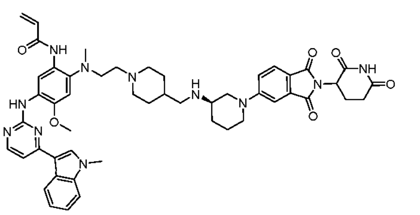 | N-(2-((2-(4-((((3R)-1-(2-(2,6-dioxopiperidin-3-yl)-1,3-dioxoisoindolin-5-yl)piperidin-3-yl)amino)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((((3R)-1-(2-(2,6-dioxopiperidin-3-yl)-1,3-dioxoisoindolin-5-yl)piperidin-3-yl)amino)methyl)piperidin-1-yl)ethyl)(methyl)amino)-4-methoxy-5-((4-(1-methyl-1H-indol-3-yl)pyrimidin-2-yl)amino)phenyl)acrylamide |
| | 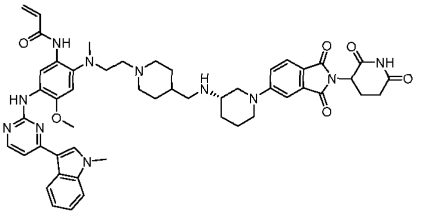 | N-(2-((2-(4-((((3S)-1-(2-(2,6-dioxopiperidin-3-yl)-1,3-dioxoisoindolin-5-yl)piperidin-3-yl)amino)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-((((3S)-1-(2-(2,6-dioxopiperidin-3-yl)-1,3-dioxoisoindolin-5-yl)piperidin-3-yl)amino)methyl)piperidin-1-yl)ethyl)(methyl)amino)-4-methoxy-5-((4-(1-methyl-1H-indol-3-yl)pyrimidin-2-yl)amino)phenyl)acrylamide |
| | 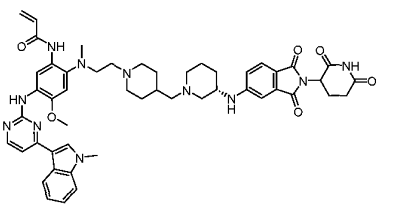 | N-(2-((2-(4-(((3S)-3-((2-(2,6-dioxopiperidin-3-yl)-1,3-dioxoisoindolin-5-yl)amino)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-(((3S)-3-((2-(2,6-dioxopiperidin-3-yl)-1,3-dioxoisoindolin-5-yl)amino)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 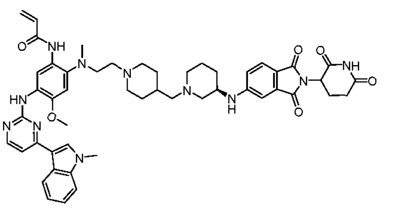 | N-(2-((2-(4-(((3R)-3-((2-(2,6-dioxopiperidin-3-yl)-1,3-dioxoisoindolin-5-yl)amino)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide | N-(2-((2-(4-(((3R)-3-((2-(2,6-dioxopiperidin-3-yl)-1,3-dioxoisoindolin-5-yl)amino)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide |
| | 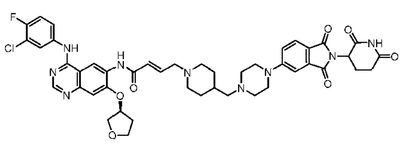 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5- | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3- |

| | | | |
|---|---|---|---|
| | | yl)piperazin-1-yl)methyl)piperidin-1-yl)but-2-enamide | dioxoisoindolin-5-yl)piperazin-1-yl)methyl)piperidin-1-yl)but-2-enamide |
| | 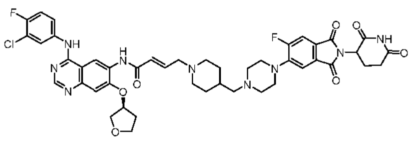 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)piperazin-1-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)piperazin-1-yl)methyl)piperidin-1-yl)but-2-enamide |
| | 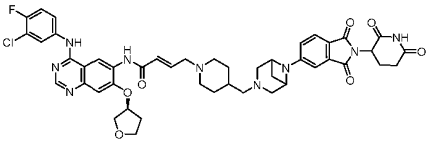 | (E)-N-(4-((3-chloro-4-fluorophenyl)amino)-7-(((S)-tetrahydrofuran-3-yl)oxy)quinazolin-6-yl)-4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)but-2-enamide |
| | 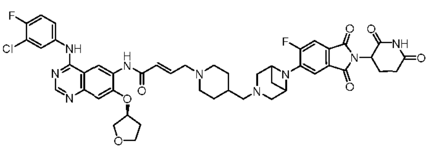 | (E)-N-(4-((3-chloro-4-fluorophenyl)amino)-7-(((S)-tetrahydrofuran-3-yl)oxy)quinazolin-6-yl)-4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)but-2-enamide |
| | 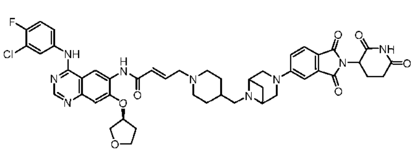 | (E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)but-2-enamide |
| | 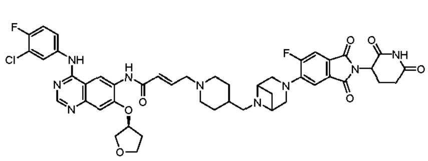 | (E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)but-2-enamide |

| | | | |
|---|---|---|---|
| | 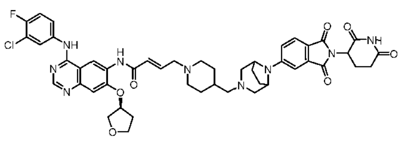 | (E)-N-(4-((3-chloro-4-fluorophenyl)amino)-7-(((S)-tetrahydrofuran-3-yl)oxy)quinazolin-6-yl)-4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)but-2-enamide |
| | 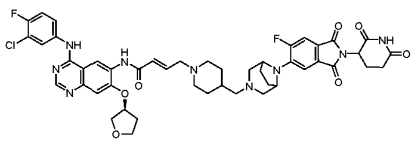 | (E)-N-(4-((3-chloro-4-fluorophenyl)amino)-7-(((S)-tetrahydrofuran-3-yl)oxy)quinazolin-6-yl)-4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)but-2-enamide |
| | 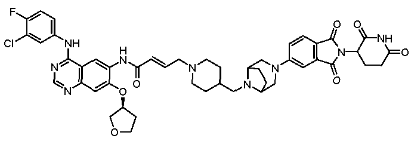 | (E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)but-2-enamide |
| | 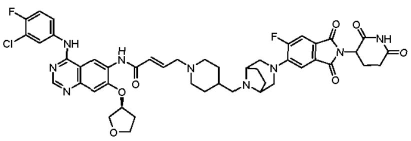 | (E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)but-2-enamide |
| | 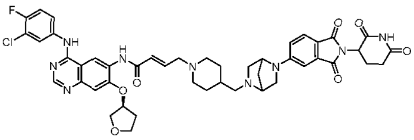 | (E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)but-2-enamide |
| | 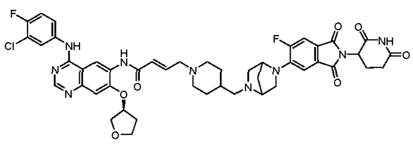 | (E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-6-fluoro-1,3- | (E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-6- |

| | | | |
|---|---|---|---|
| | | dioxoisoindolin-5-yl)-2,5-diazabicyclo[2.2.1]heptan-2-yl)methyl)piperidin-1-yl)but-2-enamide | fluoro-1,3-dioxoisoindolin-5-yl)-2,5-diazabicyclo[2.2.1]heptan-2-yl)methyl)piperidin-1-yl)but-2-enamide |
| | 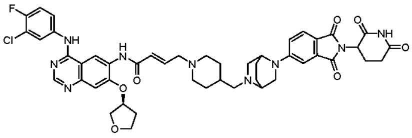 | (E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)but-2-enamide |
| | 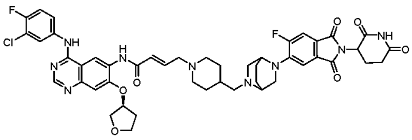 | (E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)but-2-enamide |
| | 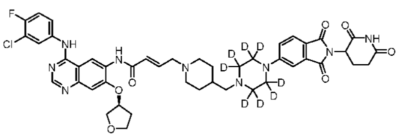 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)but-2-enamide |
| | 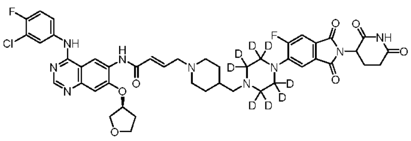 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)piperazin-1-yl-2,2,3,3,5,5,6,6-*d*$_8$)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)piperazin-1-yl-2,2,3,3,5,5,6,6-*d*$_8$)methyl)piperidin-1-yl)but-2-enamide |
| | 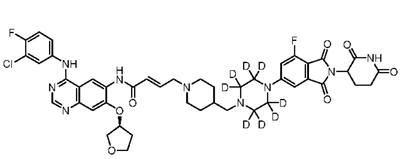 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1-yl)but- | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1- |

| | | | |
|---|---|---|---|
| | | 2-enamide | yl)but-2-enamide |
| | 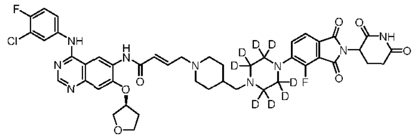 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)but-2-enamide |
| | 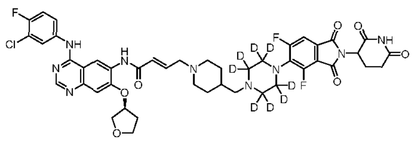 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)but-2-enamide |
| | 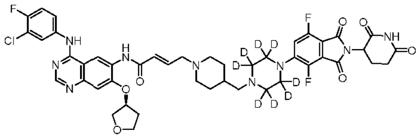 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)but-2-enamide |
| | 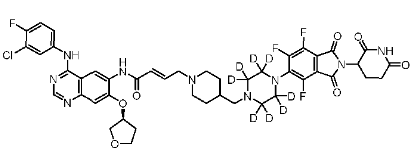 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*$_8$)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*$_8$)methyl)piperidin-1-yl)but-2-enamide |
| | 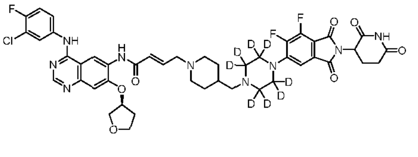 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6,7-difluoro-1,3-dioxoisoindolin-5-yl)piperazin-1-yl-2,2,3,3,5,5,6,6-*d*$_8$)methyl)piperidin-1-yl)but- | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6,7-difluoro-1,3-dioxoisoindolin-5-yl)piperazin-1-yl-2,2,3,3,5,5,6,6- |

| | | | |
|---|---|---|---|
| | | 2-enamide | $d_8$)methyl)piperidin-1-yl)but-2-enamide |
| | 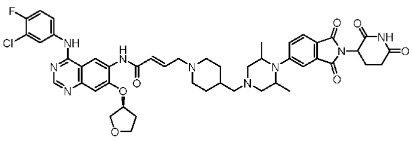 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)-3,5-dimethylpiperazin-1-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)-3,5-dimethylpiperazin-1-yl)methyl)piperidin-1-yl)but-2-enamide |
| | 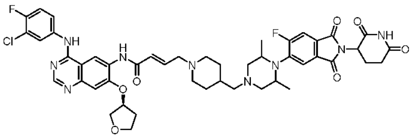 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)but-2-enamide |
| | 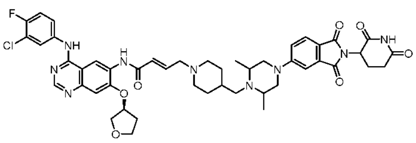 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)-2,6-dimethylpiperazin-1-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)-2,6-dimethylpiperazin-1-yl)methyl)piperidin-1-yl)but-2-enamide |
| | 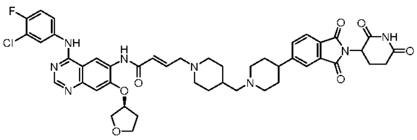 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)piperidin-1-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)piperidin-1-yl)methyl)piperidin-1-yl)but-2-enamide |
| | 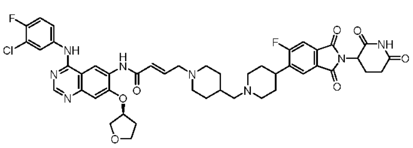 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)piperidin-1-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)piperidin-1-yl)methyl)piperidin-1-yl)but-2-enamide |
| | 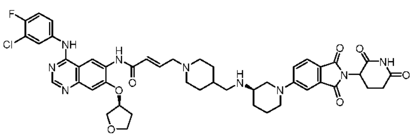 | (E)-N-(4-((3-chloro-4-fluorophenyl)amino)-7-(((S)-tetrahydrofuran-3-yl)oxy)quinazolin-6-yl)-4-(4-((((3R)-1-(2-(2,6- | (E)-N-(4-((3-chloro-4-fluorophenyl)amino)-7-(((S)-tetrahydrofuran-3-yl)oxy)quinazolin-6-yl)-4-(4-((((3R)-1-(2-(2,6- |

| | | | |
|---|---|---|---|
| | | dioxopiperidin-3-yl)-1,3-dioxoisoindolin-5-yl)piperidin-3-yl)amino)methyl)piperidin-1-yl)but-2-enamide | dioxopiperidin-3-yl)-1,3-dioxoisoindolin-5-yl)piperidin-3-yl)amino)methyl)piperidin-1-yl)but-2-enamide |
| | 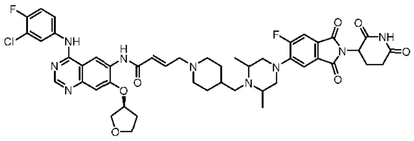 | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)but-2-enamide |
| | 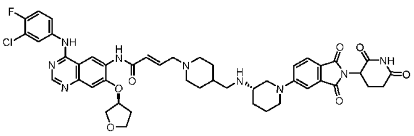 | (E)-N-(4-((3-chloro-4-fluorophenyl)amino)-7-(((S)-tetrahydrofuran-3-yl)oxy)quinazolin-6-yl)-4-(4-((((3S)-1-(2-(2,6-dioxopiperidin-3-yl)-1,3-dioxoisoindolin-5-yl)piperidin-3-yl)amino)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-((((3S)-1-(2-(2,6-dioxopiperidin-3-yl)-1,3-dioxoisoindolin-5-yl)piperidin-3-yl)amino)methyl)piperidin-1-yl)but-2-enamide |
| | 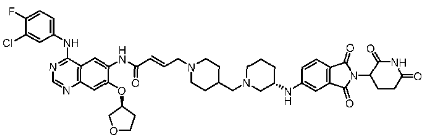 | (E)-N-(4-((3-chloro-4-fluorophenyl)amino)-7-(((S)-tetrahydrofuran-3-yl)oxy)quinazolin-6-yl)-4-(4-(((3S)-3-((2-(2,6-dioxopiperidin-3-yl)-1,3-dioxoisoindolin-5-yl)amino)piperidin-1-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-(((3S)-3-((2-(2,6-dioxopiperidin-3-yl)-1,3-dioxoisoindolin-5-yl)amino)piperidin-1-yl)methyl)piperidin-1-yl)but-2-enamide |
| | 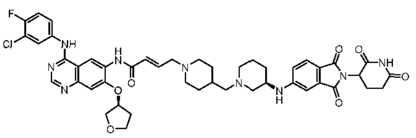 | (E)-N-(4-((3-chloro-4-fluorophenyl)amino)-7-(((S)-tetrahydrofuran-3-yl)oxy)quinazolin-6-yl)-4-(4-(((3R)-3-((2-(2,6-dioxopiperidin-3-yl)-1,3-dioxoisoindolin-5-yl)amino)piperidin-1-yl)methyl)piperidin-1-yl)but-2-enamide | (E)-N-(4-((3-chloro-4-fluorophenyl)amino)-7-(((S)-tetrahydrofuran-3-yl)oxy)quinazolin-6-yl)-4-(4-(((3R)-3-((2-(2,6-dioxopiperidin-3-yl)-1,3-dioxoisoindolin-5-yl)amino)piperidin-1-yl)methyl)piperidin-1-yl)but-2-enamide |
| | 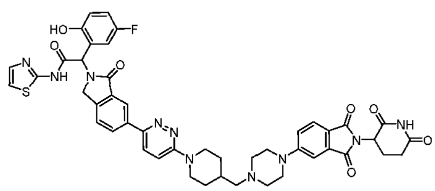 | 2-(6-(6-(4-((4-(2-(2,6-dioxopiperidin-3-yl)-1,3-dioxoisoindolin-5-yl)piperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-1,3-dioxoisoindolin-5-yl)piperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |

| | | | |
|---|---|---|---|
| | 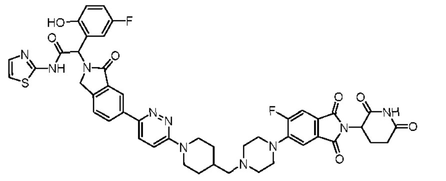 | 2-(6-(6-(4-((4-(2-(2,6-dioxopiperidin-3-yl)-6-fluoro-1,3-dioxoisoindolin-5-yl)piperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-6-fluoro-1,3-dioxoisoindolin-5-yl)piperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 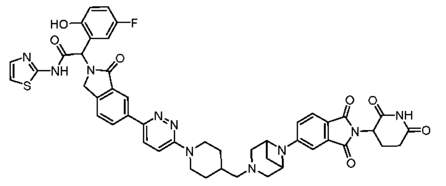 | 2-(6-(6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 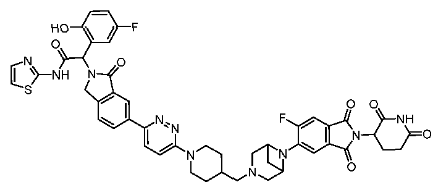 | 2-(6-(6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 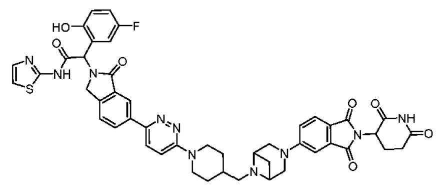 | 2-(6-(6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 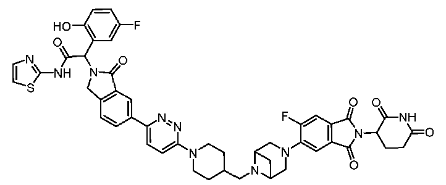 | 2-(6-(6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 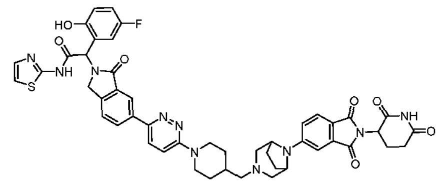 | 2-(6-(6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide |

| | | | |
|---|---|---|---|
| | 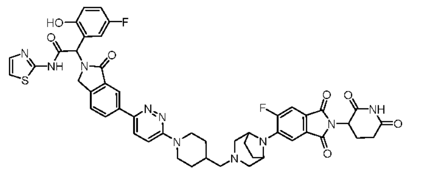 | 2-(6-(6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 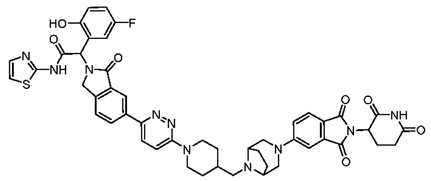 | 2-(6-(6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 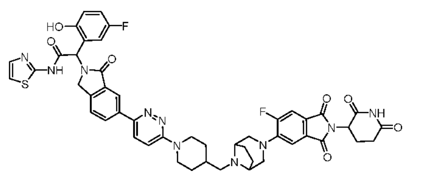 | 2-(6-(6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 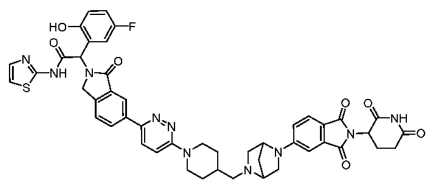 | 2-(6-(6-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 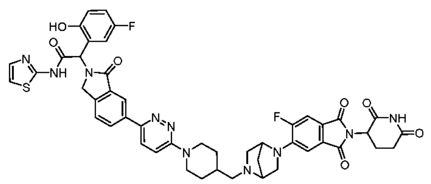 | 2-(6-(6-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 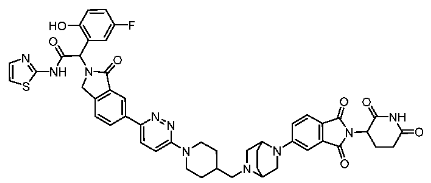 | 2-(6-(6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide |

| | | | |
|---|---|---|---|
| | 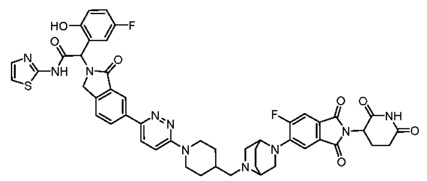 | 2-(6-(6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 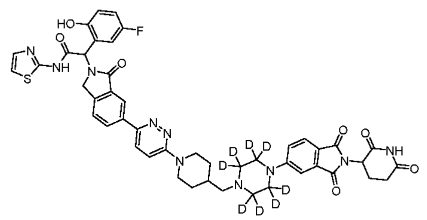 | 2-(6-(6-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide |
| | 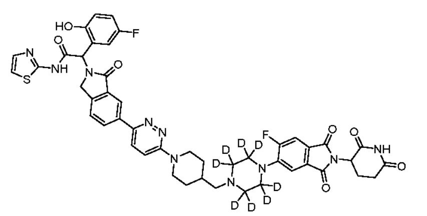 | 2-(6-(6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide |
| | 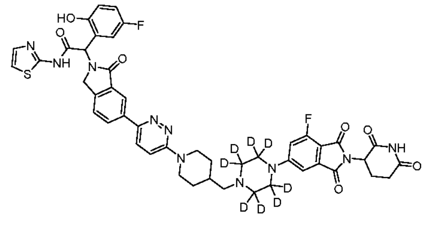 | 2-(6-(6-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide |
| | 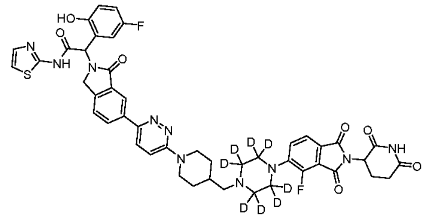 | 2-(6-(6-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide |
| | 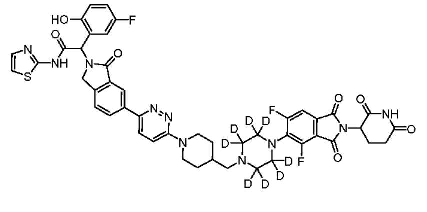 | 2-(6-(6-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5- | 2-(6-(6-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)pyridazin-3-yl)-1- |

| | | | |
|---|---|---|---|
| | | fluoro-2-hydroxyphenyl)-N-(thiazol-2-yl)acetamide | oxoisoindolin-2-yl)-2-(5-fluoro-2-hydroxyphenyl)-N-(thiazol-2-yl)acetamide |
| | 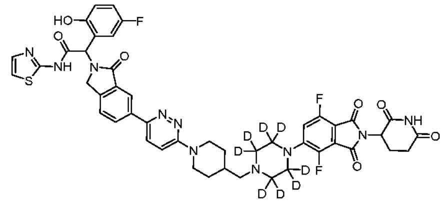 | 2-(6-(6-(4-((4-(2-(2,6-dioxopiperidin-3-yl)-4,7-difluoro-1,3-dioxoisoindolin-5-yl)piperazin-1-yl-2,2,3,3,5,5,6,6-*d*8)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-4,7-difluoro-1,3-dioxoisoindolin-5-yl)piperazin-1-yl-2,2,3,3,5,5,6,6-*d*8)methyl)piperidin-1-yl)pyridazin-3-yl)-1-oxoisoindolin-2-yl)-2-(5-fluoro-2-hydroxyphenyl)-N-(thiazol-2-yl)acetamide |
| | 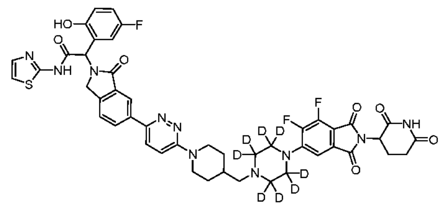 | 2-(6-(6-(4-((4-(2-(2,6-dioxopiperidin-3-yl)-6,7-difluoro-1,3-dioxoisoindolin-5-yl)piperazin-1-yl-2,2,3,3,5,5,6,6-*d*8)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-6,7-difluoro-1,3-dioxoisoindolin-5-yl)piperazin-1-yl-2,2,3,3,5,5,6,6-*d*8)methyl)piperidin-1-yl)pyridazin-3-yl)-1-oxoisoindolin-2-yl)-2-(5-fluoro-2-hydroxyphenyl)-N-(thiazol-2-yl)acetamide |
| | 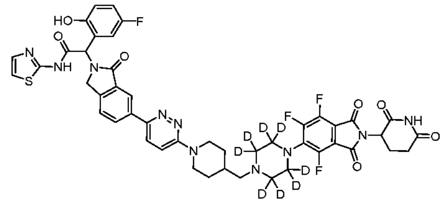 | 2-(6-(6-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*8)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*8)methyl)piperidin-1-yl)pyridazin-3-yl)-1-oxoisoindolin-2-yl)-2-(5-fluoro-2-hydroxyphenyl)-N-(thiazol-2-yl)acetamide |
| | 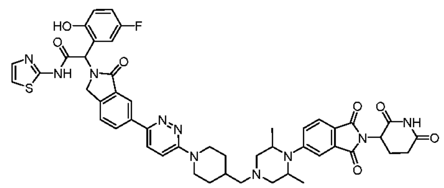 | 2-(6-(6-(4-((4-(2-(2,6-dioxopiperidin-3-yl)-1,3-dioxoisoindolin-5-yl)-3,5-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-1,3-dioxoisoindolin-5-yl)-3,5-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 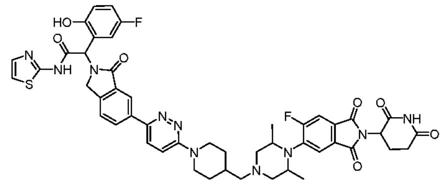 | 2-(6-(6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |

| | | | |
|---|---|---|---|
| | 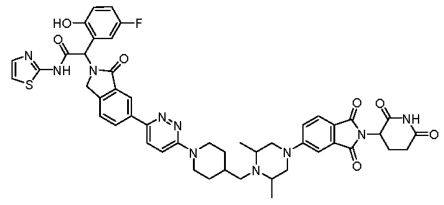 | 2-(6-(6-(4-((4-(2-(2,6-dioxopiperidin-3-yl)-1,3-dioxoisoindolin-5-yl)-2,6-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-1,3-dioxoisoindolin-5-yl)-2,6-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 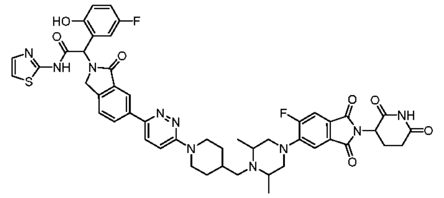 | 2-(6-(6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 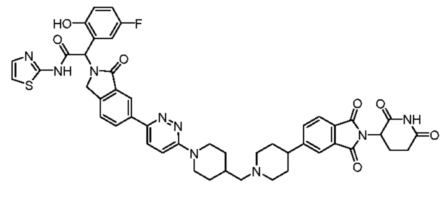 | 2-(6-(6-(4-((4-(2-(2,6-dioxopiperidin-3-yl)-1,3-dioxoisoindolin-5-yl)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-1,3-dioxoisoindolin-5-yl)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 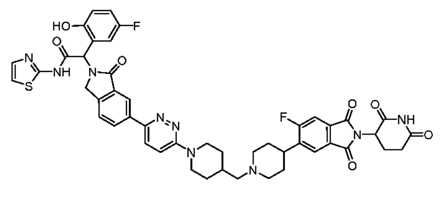 | 2-(6-(6-(4-((4-(2-(2,6-dioxopiperidin-3-yl)-6-fluoro-1,3-dioxoisoindolin-5-yl)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((4-(2-(2,6-dioxopiperidin-3-yl)-6-fluoro-1,3-dioxoisoindolin-5-yl)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 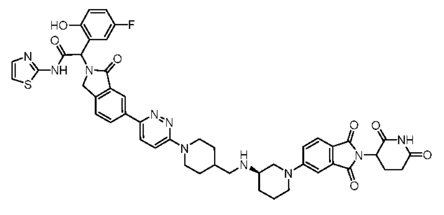 | 2-(6-(6-(4-((((3R)-1-(2-(2,6-dioxopiperidin-3-yl)-1,3-dioxoisoindolin-5-yl)piperidin-3-yl)amino)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((((3R)-1-(2-(2,6-dioxopiperidin-3-yl)-1,3-dioxoisoindolin-5-yl)piperidin-3-yl)amino)methyl)piperidin-1-yl)pyridazin-3-yl)-1-oxoisoindolin-2-yl)-2-(5-fluoro-2-hydroxyphenyl)-N-(thiazol-2-yl)acetamide |
| | 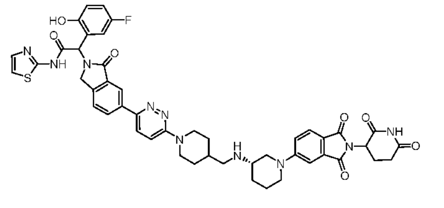 | 2-(6-(6-(4-((((3S)-1-(2-(2,6-dioxopiperidin-3-yl)-1,3-dioxoisoindolin-5-yl)piperidin-3-yl)amino)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-((((3S)-1-(2-(2,6-dioxopiperidin-3-yl)-1,3-dioxoisoindolin-5-yl)piperidin-3-yl)amino)methyl)piperidin-1-yl)pyridazin-3-yl)-1-oxoisoindolin-2-yl)-2-(5-fluoro-2-hydroxyphenyl)-N-(thiazol-2-yl)acetamide |

| | | | |
|---|---|---|---|
| | 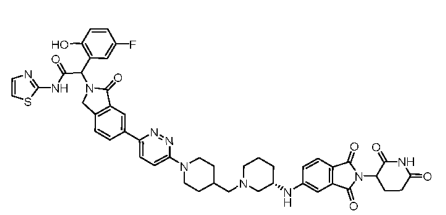 | 2-(6-(6-(4-(((3S)-3-((2-(2,6-dioxopiperidin-3-yl)-1,3-dioxoisoindolin-5-yl)amino)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-(((3S)-3-((2-(2,6-dioxopiperidin-3-yl)-1,3-dioxoisoindolin-5-yl)amino)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 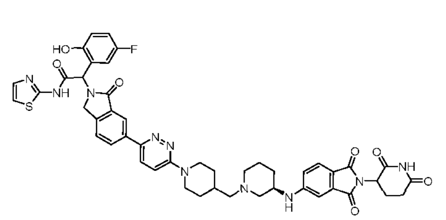 | 2-(6-(6-(4-(((3R)-3-((2-(2,6-dioxopiperidin-3-yl)-1,3-dioxoisoindolin-5-yl)amino)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide | 2-(6-(6-(4-(((3R)-3-((2-(2,6-dioxopiperidin-3-yl)-1,3-dioxoisoindolin-5-yl)amino)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide |
| | 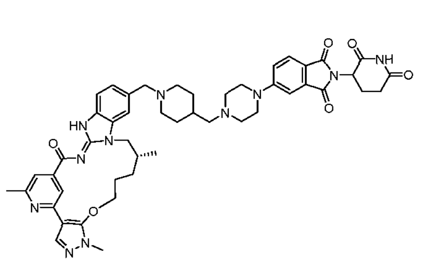 | 2-(2,6-dioxopiperidin-3-yl)-5-(4-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)piperazin-1-yl)isoindoline-1,3-dione | 2-(2,6-dioxopiperidin-3-yl)-5-(4-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)piperazin-1-yl)isoindoline-1,3-dione |
| | 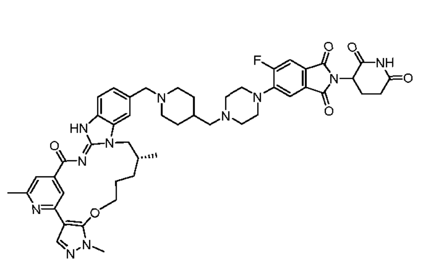 | 2-(2,6-dioxopiperidin-3-yl)-5-fluoro-6-(4-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)piperazin-1-yl)isoindoline-1,3-dione | 2-(2,6-dioxopiperidin-3-yl)-5-fluoro-6-(4-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)piperazin-1-yl)isoindoline-1,3-dione |
| | 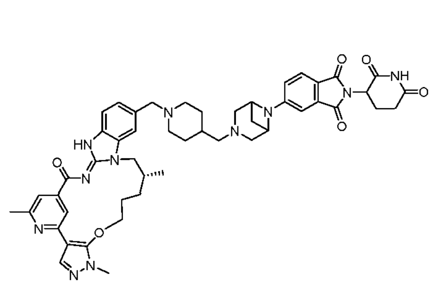 | 2-(2,6-dioxopiperidin-3-yl)-5-(3-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,6-diazabicyclo[3.1.1]heptan-6-yl)isoindoline-1,3-dione | 2-(2,6-dioxopiperidin-3-yl)-5-(3-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,6-diazabicyclo[3.1.1]heptan-6-yl)isoindoline-1,3-dione |

| | | | |
|---|---|---|---|
| | 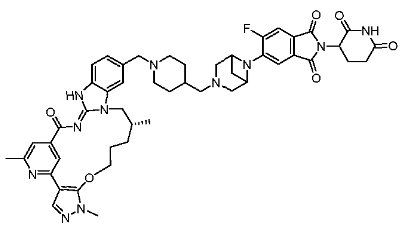 | 2-(2,6-dioxopiperidin-3-yl)-5-fluoro-6-(3-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,6-diazabicyclo[3.1.1]heptan-6-yl)isoindoline-1,3-dione | 2-(2,6-dioxopiperidin-3-yl)-5-fluoro-6-(3-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,6-diazabicyclo[3.1.1]heptan-6-yl)isoindoline-1,3-dione |
| | 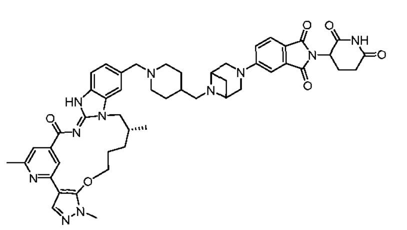 | 2-(2,6-dioxopiperidin-3-yl)-5-(6-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,6-diazabicyclo[3.1.1]heptan-3-yl)isoindoline-1,3-dione | 2-(2,6-dioxopiperidin-3-yl)-5-(6-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,6-diazabicyclo[3.1.1]heptan-3-yl)isoindoline-1,3-dione |
| | 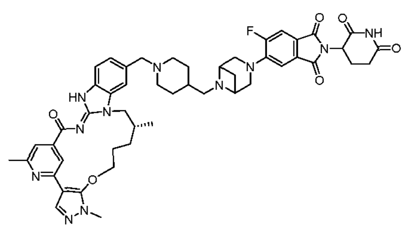 | 2-(2,6-dioxopiperidin-3-yl)-5-fluoro-6-(6-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,6-diazabicyclo[3.1.1]heptan-3-yl)isoindoline-1,3-dione | 2-(2,6-dioxopiperidin-3-yl)-5-fluoro-6-(6-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,6-diazabicyclo[3.1.1]heptan-3-yl)isoindoline-1,3-dione |
| | 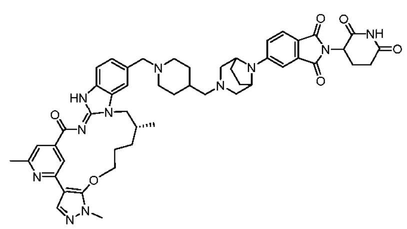 | 2-(2,6-dioxopiperidin-3-yl)-5-(3-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,8-diazabicyclo[3.2.1]octan-8-yl)isoindoline-1,3-dione | 2-(2,6-dioxopiperidin-3-yl)-5-(3-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,8-diazabicyclo[3.2.1]octan-8-yl)isoindoline-1,3-dione |
| | 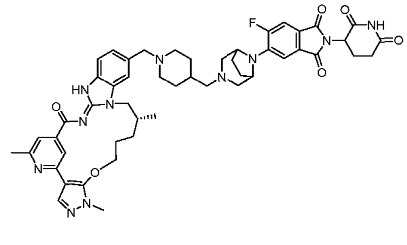 | 2-(2,6-dioxopiperidin-3-yl)-5-fluoro-6-(3-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,8- | 2-(2,6-dioxopiperidin-3-yl)-5-fluoro-6-(3-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,8- |

| | | | |
|---|---|---|---|
| | | diazabicyclo[3.2.1]octan-8-yl)isoindoline-1,3-dione | diazabicyclo[3.2.1]octan-8-yl)isoindoline-1,3-dione |
| | 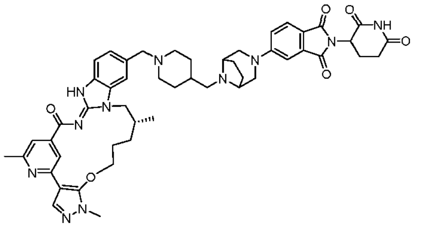 | 2-(2,6-dioxopiperidin-3-yl)-5-(8-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,8-diazabicyclo[3.2.1]octan-3-yl)isoindoline-1,3-dione | 2-(2,6-dioxopiperidin-3-yl)-5-(8-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,8-diazabicyclo[3.2.1]octan-3-yl)isoindoline-1,3-dione |
| | 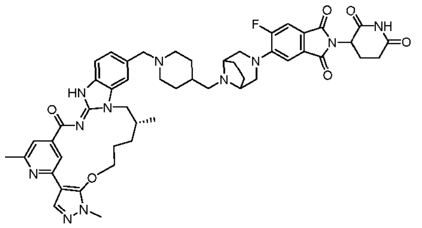 | 2-(2,6-dioxopiperidin-3-yl)-5-fluoro-6-(8-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,8-diazabicyclo[3.2.1]octan-3-yl)isoindoline-1,3-dione | 2-(2,6-dioxopiperidin-3-yl)-5-fluoro-6-(8-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-3,8-diazabicyclo[3.2.1]octan-3-yl)isoindoline-1,3-dione |
| | 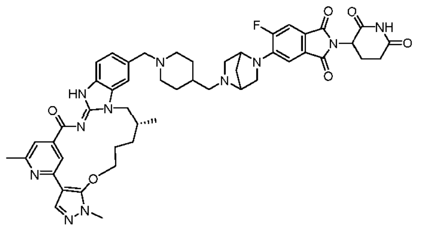 | 2-(2,6-dioxopiperidin-3-yl)-5-fluoro-6-(5-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-2,5-diazabicyclo[2.2.1]heptan-2-yl)isoindoline-1,3-dione | 2-(2,6-dioxopiperidin-3-yl)-5-fluoro-6-(5-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-2,5-diazabicyclo[2.2.1]heptan-2-yl)isoindoline-1,3-dione |
| | 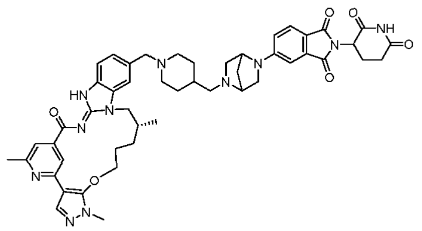 | 2-(2,6-dioxopiperidin-3-yl)-5-(5-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-2,5-diazabicyclo[2.2.1]heptan-2-yl)isoindoline-1,3-dione | 2-(2,6-dioxopiperidin-3-yl)-5-(5-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-2,5-diazabicyclo[2.2.1]heptan-2-yl)isoindoline-1,3-dione |
| | 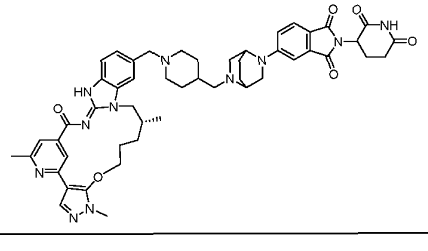 | 2-(2,6-dioxopiperidin-3-yl)-5-(5-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane- | 2-(2,6-dioxopiperidin-3-yl)-5-(5-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane |

| | | | |
|---|---|---|---|
| | | $5^6$-yl)methyl)piperidin-4-yl)methyl)-2,5-diazabicyclo[2.2.2]octan-2-yl)isoindoline-1,3-dione | -$5^6$-yl)methyl)piperidin-4-yl)methyl)-2,5-diazabicyclo[2.2.2]octan-2-yl)isoindoline-1,3-dione |
| | 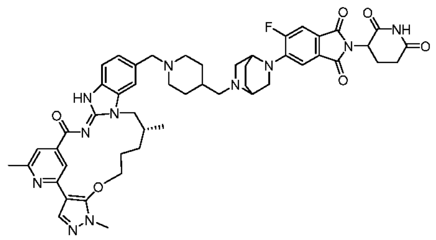 | 2-(2,6-dioxopiperidin-3-yl)-5-fluoro-6-(5-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)-2,5-diazabicyclo[2.2.2]octan-2-yl)isoindoline-1,3-dione | 2-(2,6-dioxopiperidin-3-yl)-5-fluoro-6-(5-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)-2,5-diazabicyclo[2.2.2]octan-2-yl)isoindoline-1,3-dione |
| | 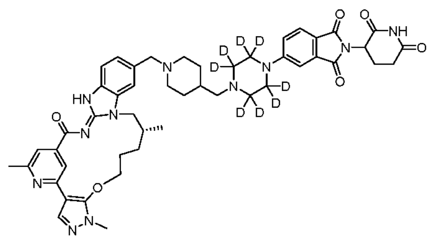 | 2-(2,6-dioxopiperidin-3-yl)-5-(4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-5-(4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl-2,2,3,3,5,5,6,6-$d_8$)isoindoline-1,3-dione |
| | 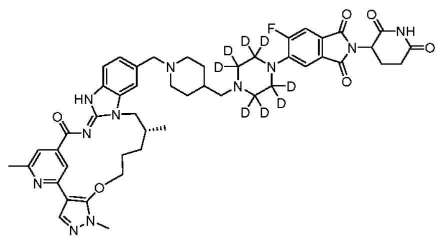 | 2-(2,6-dioxopiperidin-3-yl)-5-fluoro-6-(4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-5-fluoro-6-(4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl-2,2,3,3,5,5,6,6-$d_8$)isoindoline-1,3-dione |
| | 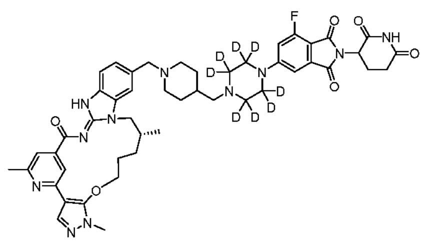 | 2-(2,6-dioxopiperidin-3-yl)-4-fluoro-6-(4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-6-(4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl-2,2,3,3,5,5,6,6-$d_8$)isoindoline-1,3-dione |

| | | | |
|---|---|---|---|
| | 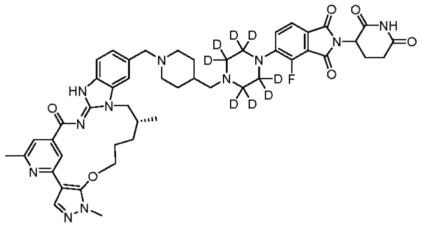 | 2-(2,6-dioxopiperidin-3-yl)-4-fluoro-5-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-5-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione |
| | 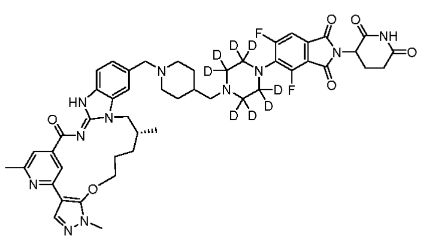 | 2-(2,6-dioxopiperidin-3-yl)-4,6-difluoro-5-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-4,6-difluoro-5-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione |
| | 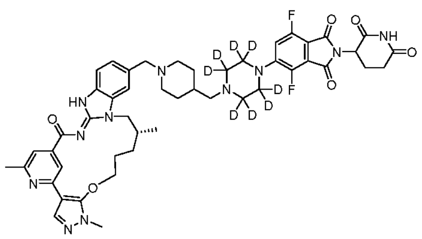 | 2-(2,6-dioxopiperidin-3-yl)-4,7-difluoro-5-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-4,7-difluoro-5-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione |
| | 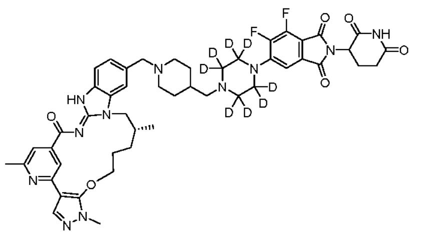 | 2-(2,6-dioxopiperidin-3-yl)-4,5-difluoro-6-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione | 2-(2,6-dioxopiperidin-3-yl)-4,5-difluoro-6-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione |
| | 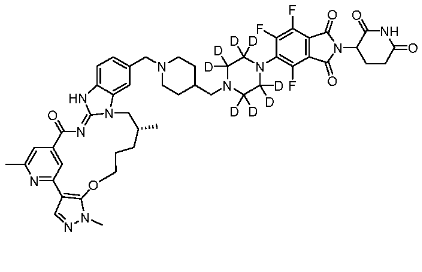 | 2-(2,6-dioxopiperidin-3-yl)-4,5,7-trifluoro-6-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl- | 2-(2,6-dioxopiperidin-3-yl)-4,5,7-trifluoro-6-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl- |

| | | | |
|---|---|---|---|
| | | 2,2,3,3,5,5,6,6-$d_8$)isoindoline-1,3-dione | 2,2,3,3,5,5,6,6-$d_8$)isoindoline-1,3-dione |
| | 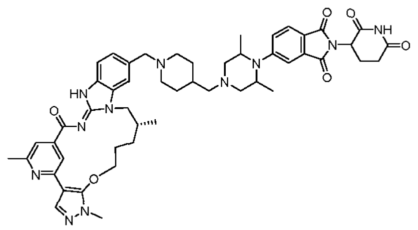 | 5-(2,6-dimethyl-4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl)-2-(2,6-dioxopiperidin-3-yl)isoindoline-1,3-dione | 5-(2,6-dimethyl-4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl)-2-(2,6-dioxopiperidin-3-yl)isoindoline-1,3-dione |
| | 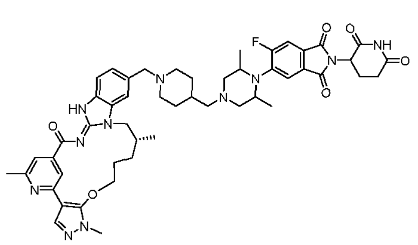 | 5-(2,6-dimethyl-4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl)-2-(2,6-dioxopiperidin-3-yl)-6-fluoroisoindoline-1,3-dione | 5-(2,6-dimethyl-4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl)-2-(2,6-dioxopiperidin-3-yl)-6-fluoroisoindoline-1,3-dione |
| | 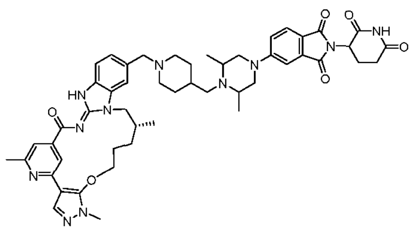 | 5-(3,5-dimethyl-4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl)-2-(2,6-dioxopiperidin-3-yl)isoindoline-1,3-dione | 5-(3,5-dimethyl-4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl)-2-(2,6-dioxopiperidin-3-yl)isoindoline-1,3-dione |
| | 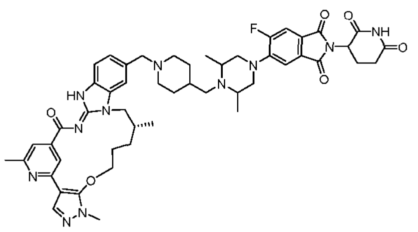 | 5-(3,5-dimethyl-4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl)-2-(2,6-dioxopiperidin-3-yl)-6-fluoroisoindoline-1,3-dione | 5-(3,5-dimethyl-4-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperazin-1-yl)-2-(2,6-dioxopiperidin-3-yl)-6-fluoroisoindoline-1,3-dione |
| | 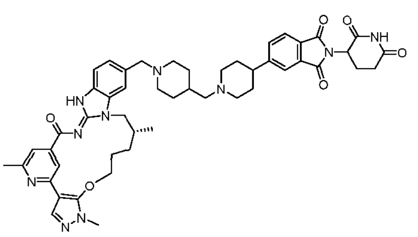 | 2-(2,6-dioxopiperidin-3-yl)-5-(1-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperidin-4- | 2-(2,6-dioxopiperidin-3-yl)-5-(1-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperidin-4- |

| | | | |
|---|---|---|---|
| | | yl)isoindoline-1,3-dione | yl)isoindoline-1,3-dione |
| | 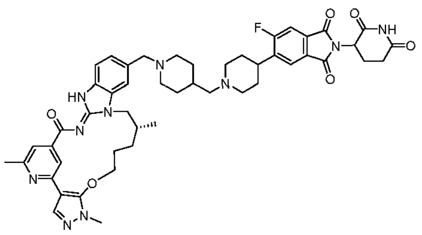 | 2-(2,6-dioxopiperidin-3-yl)-5-fluoro-6-(1-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperidin-4-yl)isoindoline-1,3-dione | 2-(2,6-dioxopiperidin-3-yl)-5-fluoro-6-(1-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperidin-4-yl)isoindoline-1,3-dione |
| | 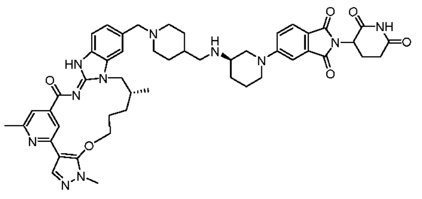 | 2-(2,6-dioxopiperidin-3-yl)-5-((R)-3-(((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)amino)piperidin-1-yl)isoindoline-1,3-dione | 2-(2,6-dioxopiperidin-3-yl)-5-((R)-3-(((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)amino)piperidin-1-yl)isoindoline-1,3-dione |
| | 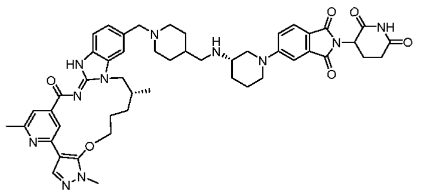 | 2-(2,6-dioxopiperidin-3-yl)-5-((S)-3-(((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)amino)piperidin-1-yl)isoindoline-1,3-dione | 2-(2,6-dioxopiperidin-3-yl)-5-((S)-3-(((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)amino)piperidin-1-yl)isoindoline-1,3-dione |
| | 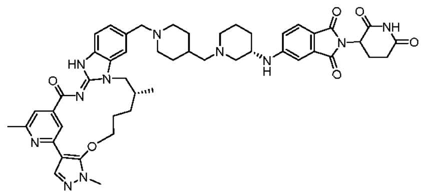 | 2-(2,6-dioxopiperidin-3-yl)-5-(((S)-1-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperidin-3-yl)amino)isoindoline-1,3-dione | 2-(2,6-dioxopiperidin-3-yl)-5-(((S)-1-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperidin-3-yl)amino)isoindoline-1,3-dione |
| | 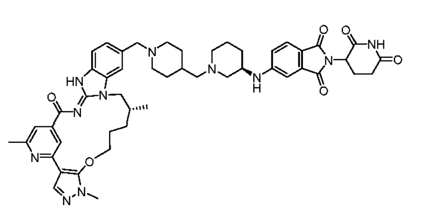 | 2-(2,6-dioxopiperidin-3-yl)-5-(((R)-1-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperidin-3-yl)amino)isoindoline-1,3-dione | 2-(2,6-dioxopiperidin-3-yl)-5-(((R)-1-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperidin-3-yl)amino)isoindoline-1,3-dione |

| | | | |
|---|---|---|---|
| | 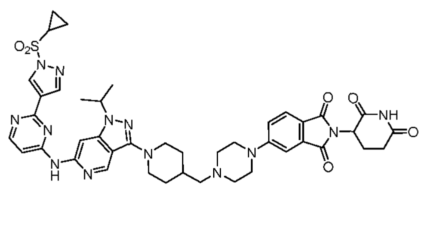 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl)-2-(2,6-dioxopiperidin-3-yl)isoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl)-2-(2,6-dioxopiperidin-3-yl)isoindoline-1,3-dione |
| | 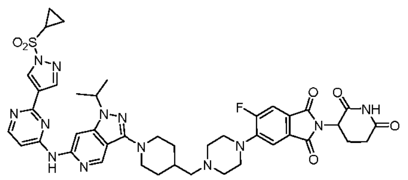 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl)-2-(2,6-dioxopiperidin-3-yl)-6-fluoroisoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl)-2-(2,6-dioxopiperidin-3-yl)-6-fluoroisoindoline-1,3-dione |
| | 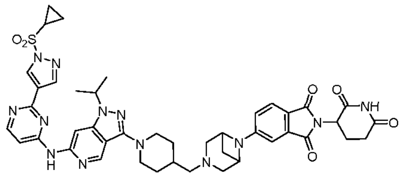 | 5-(3-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-6-yl)-2-(2,6-dioxopiperidin-3-yl)isoindoline-1,3-dione | 5-(3-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-6-yl)-2-(2,6-dioxopiperidin-3-yl)isoindoline-1,3-dione |
| | 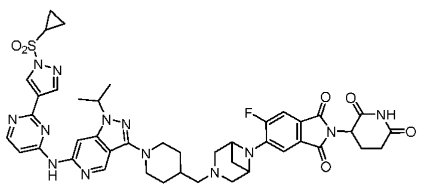 | 5-(3-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 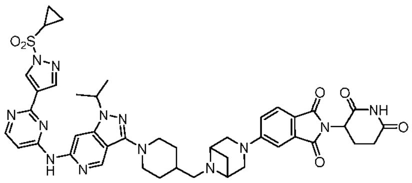 | 5-(6-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-3-yl)-2-(2,6-dioxopiperidin-3-yl)isoindoline-1,3-dione | 5-(6-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-3-yl)-2-(2,6-dioxopiperidin-3-yl)isoindoline-1,3-dione |
| | 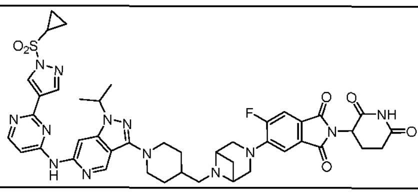 | 5-(6-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)- | 5-(6-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)- |

| | | | |
|---|---|---|---|
| | | 3,6-diazabicyclo[3.1.1]heptan-3-yl)-2-(2,6-dioxopiperidin-3-yl)-6-fluoroisoindoline-1,3-dione | 3,6-diazabicyclo[3.1.1]heptan-3-yl)-2-(2,6-dioxopiperidin-3-yl)-6-fluoroisoindoline-1,3-dione |
| | 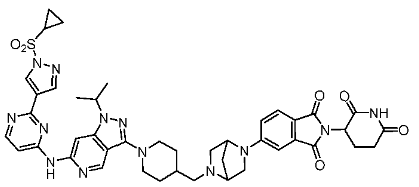 | 5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.1]heptan-2-yl)-2-(2,6-dioxopiperidin-3-yl)isoindoline-1,3-dione | 5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.1]heptan-2-yl)-2-(2,6-dioxopiperidin-3-yl)isoindoline-1,3-dione |
| | 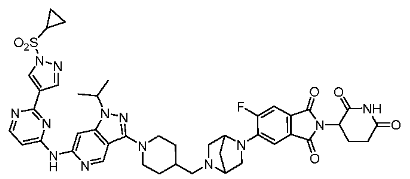 | 5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 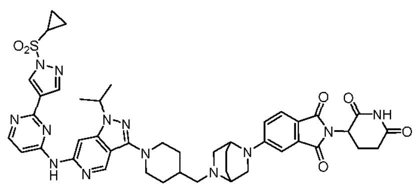 | 5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.2]octan-2-yl)-2-(2,6-dioxopiperidin-3-yl)isoindoline-1,3-dione | 5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.2]octan-2-yl)-2-(2,6-dioxopiperidin-3-yl)isoindoline-1,3-dione |
| | 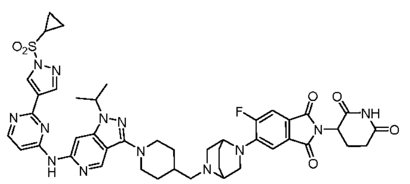 | 5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.2]octan-2-yl)-2-(2,6-dioxopiperidin-3-yl)-6-fluoroisoindoline-1,3-dione |
| | 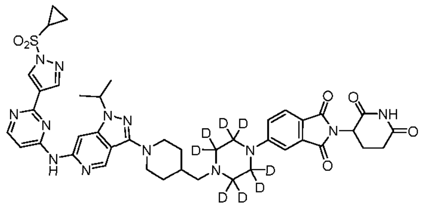 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)isoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)isoindoline-1,3-dione |

| | | | |
|---|---|---|---|
| | 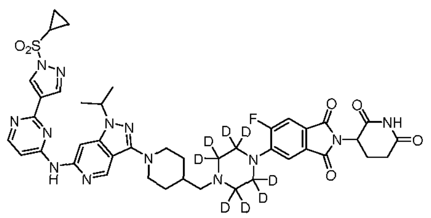 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-6-fluoroisoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-6-fluoroisoindoline-1,3-dione |
| | 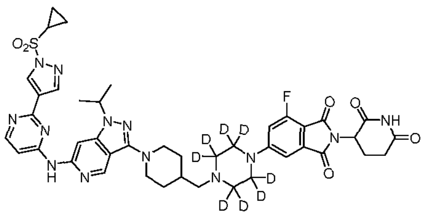 | 6-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-4-fluoroisoindoline-1,3-dione | 6-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-4-fluoroisoindoline-1,3-dione |
| | 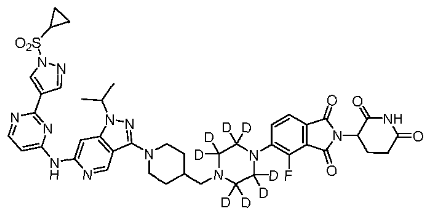 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-4-fluoroisoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-4-fluoroisoindoline-1,3-dione |
| | 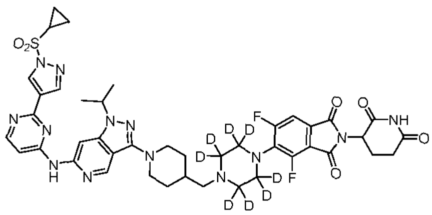 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-4,6-difluoroisoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-4,6-difluoroisoindoline-1,3-dione |
| | 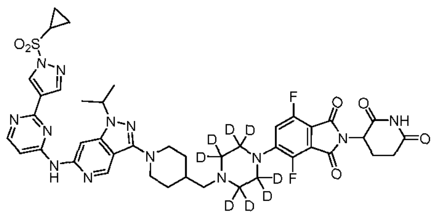 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-4,7-difluoroisoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-*d*$_8$)-2-(2,6-dioxopiperidin-3-yl)-4,7-difluoroisoindoline-1,3-dione |

| | | | |
|---|---|---|---|
| | 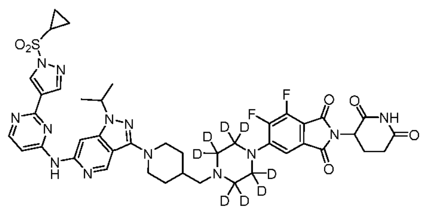 | 6-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione | 6-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione |
| | 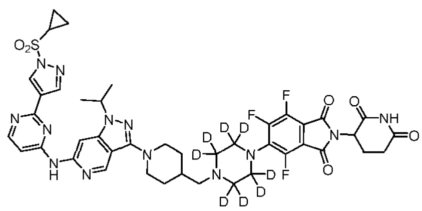 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |
| | 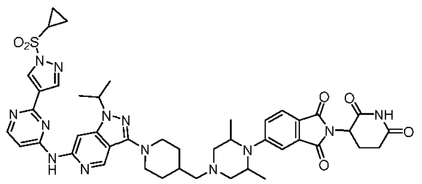 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,6-dimethylpiperazin-1-yl)-2-(2,6-dioxopiperidin-3-yl)isoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,6-dimethylpiperazin-1-yl)-2-(2,6-dioxopiperidin-3-yl)isoindoline-1,3-dione |
| | 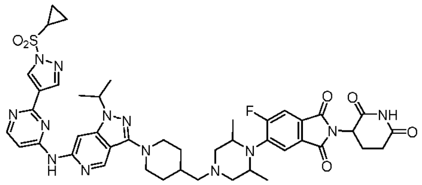 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,6-dimethylpiperazin-1-yl)-2-(2,6-dioxopiperidin-3-yl)-6-fluoroisoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,6-dimethylpiperazin-1-yl)-2-(2,6-dioxopiperidin-3-yl)-6-fluoroisoindoline-1,3-dione |
| | 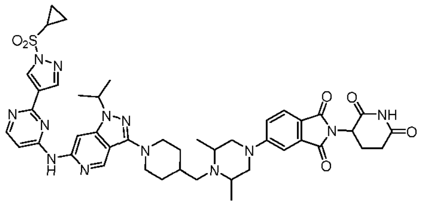 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,5-dimethylpiperazin-1-yl)-2-(2,6-dioxopiperidin-3-yl)isoindoline-1,3-dione | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,5-dimethylpiperazin-1-yl)-2-(2,6-dioxopiperidin-3-yl)isoindoline-1,3-dione |
| | 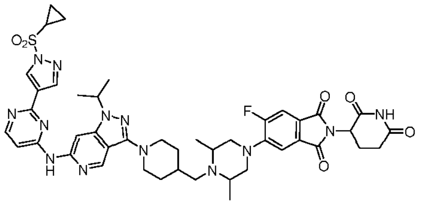 | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,5-dimethylpiperazin-1-yl)-2-(2,6-dioxopiperidin-3-yl)- | 5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,5-dimethylpiperazin-1-yl)-2-(2,6-dioxopiperidin- |

| | | | |
|---|---|---|---|
| | | 6-fluoroisoindoline-1,3-dione | 3-yl)-6-fluoroisoindoline-1,3-dione |
| | 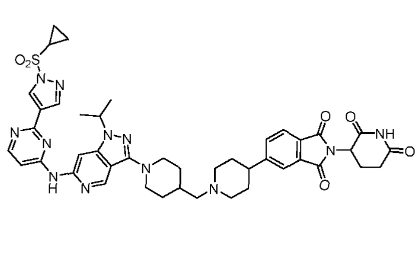 | 5-(1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-4-yl)-2-(2,6-dioxopiperidin-3-yl)isoindoline-1,3-dione | 5-(1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-4-yl)-2-(2,6-dioxopiperidin-3-yl)isoindoline-1,3-dione |
| | 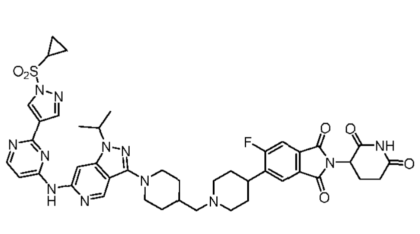 | 5-(1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-4-yl)-2-(2,6-dioxopiperidin-3-yl)-6-fluoroisoindoline-1,3-dione | 5-(1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-4-yl)-2-(2,6-dioxopiperidin-3-yl)-6-fluoroisoindoline-1,3-dione |
| | 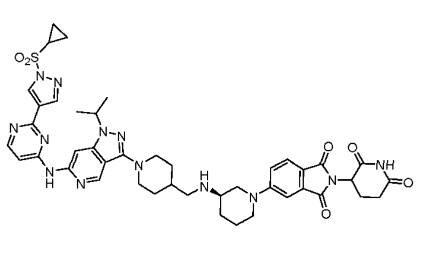 | 5-((R)-3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)amino)piperidin-1-yl)-2-(2,6-dioxopiperidin-3-yl)isoindoline-1,3-dione | 5-((R)-3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)amino)piperidin-1-yl)-2-(2,6-dioxopiperidin-3-yl)isoindoline-1,3-dione |
| | 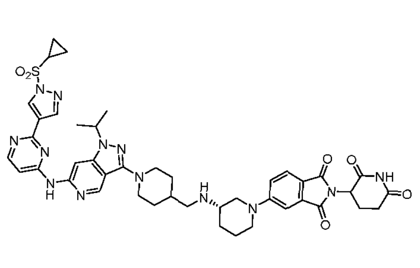 | 5-((S)-3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)amino)piperidin-1-yl)-2-(2,6-dioxopiperidin-3-yl)isoindoline-1,3-dione | 5-((S)-3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)amino)piperidin-1-yl)-2-(2,6-dioxopiperidin-3-yl)isoindoline-1,3-dione |
| | 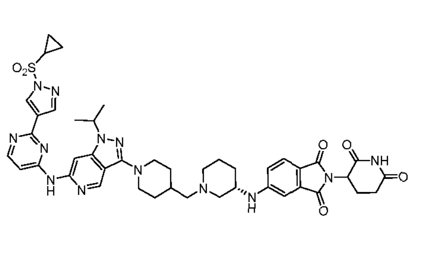 | 5-(((S)-1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-3-yl)amino)-2-(2,6-dioxopiperidin-3-yl)isoindoline-1,3-dione | 5-(((S)-1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-3-yl)amino)-2-(2,6-dioxopiperidin-3-yl)isoindoline-1,3-dione |
| | 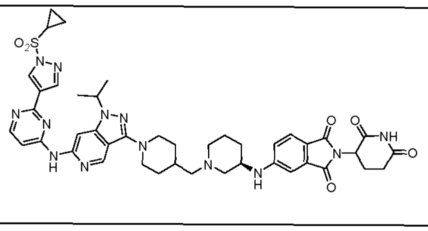 | 5-(((R)-1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-3- | 5-(((R)-1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-3- |

| | | | |
|---|---|---|---|
| | | yl)amino)-2-(2,6-dioxopiperidin-3-yl)isoindoline-1,3-dione | yl)amino)-2-(2,6-dioxopiperidin-3-yl)isoindoline-1,3-dione |
| | 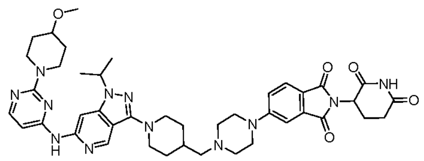 | 2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl)isoindoline-1,3-dione | 2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl)isoindoline-1,3-dione |
| | 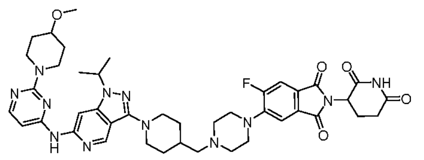 | 2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl)isoindoline-1,3-dione | 2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl)isoindoline-1,3-dione |
| | 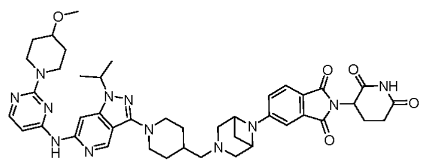 | 2-(2,6-dioxopiperidin-3-yl)-5-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-6-yl)isoindoline-1,3-dione | 2-(2,6-dioxopiperidin-3-yl)-5-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-6-yl)isoindoline-1,3-dione |
| | 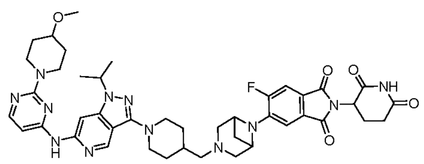 | 2-(2,6-dioxopiperidin-3-yl)-5-fluoro-6-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-6-yl)isoindoline-1,3-dione | 2-(2,6-dioxopiperidin-3-yl)-5-fluoro-6-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-6-yl)isoindoline-1,3-dione |
| | 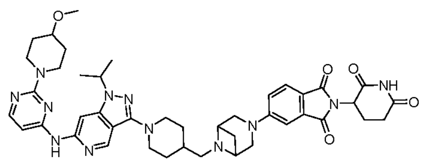 | 2-(2,6-dioxopiperidin-3-yl)-5-(6-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-3-yl)isoindoline-1,3-dione | 2-(2,6-dioxopiperidin-3-yl)-5-(6-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-3-yl)isoindoline-1,3-dione |
| | 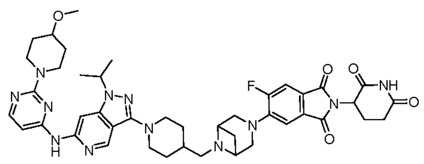 | 2-(2,6-dioxopiperidin-3-yl)-5-fluoro-6-(6-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-3-yl)isoindoline-1,3-dione | 2-(2,6-dioxopiperidin-3-yl)-5-fluoro-6-(6-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-3-yl)isoindoline-1,3-dione |

| | | | |
|---|---|---|---|
| | 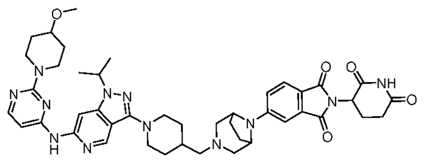 | 2-(2,6-dioxopiperidin-3-yl)-5-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-8-yl)isoindoline-1,3-dione | 2-(2,6-dioxopiperidin-3-yl)-5-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-8-yl)isoindoline-1,3-dione |
| | 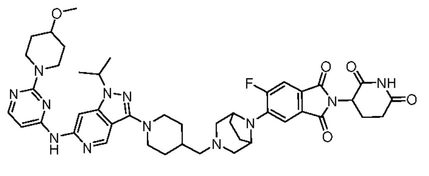 | 2-(2,6-dioxopiperidin-3-yl)-5-fluoro-6-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-8-yl)isoindoline-1,3-dione | 2-(2,6-dioxopiperidin-3-yl)-5-fluoro-6-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-8-yl)isoindoline-1,3-dione |
| | 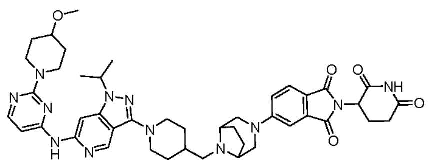 | 2-(2,6-dioxopiperidin-3-yl)-5-(8-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-3-yl)isoindoline-1,3-dione | 2-(2,6-dioxopiperidin-3-yl)-5-(8-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-3-yl)isoindoline-1,3-dione |
| | 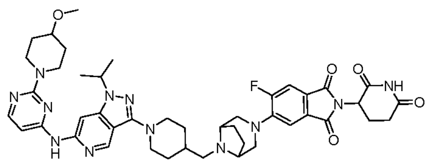 | 2-(2,6-dioxopiperidin-3-yl)-5-fluoro-6-(8-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-3-yl)isoindoline-1,3-dione | 2-(2,6-dioxopiperidin-3-yl)-5-fluoro-6-(8-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-3-yl)isoindoline-1,3-dione |
| | 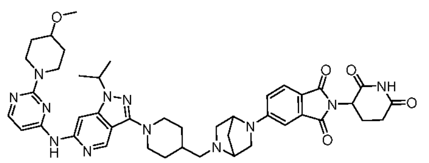 | 2-(2,6-dioxopiperidin-3-yl)-5-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.1]heptan-2-yl)isoindoline-1,3-dione | 2-(2,6-dioxopiperidin-3-yl)-5-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.1]heptan-2-yl)isoindoline-1,3-dione |
| | 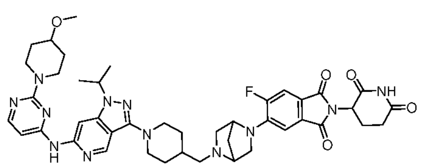 | 2-(2,6-dioxopiperidin-3-yl)-5-fluoro-6-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.1]heptan-2-yl)isoindoline-1,3-dione | 2-(2,6-dioxopiperidin-3-yl)-5-fluoro-6-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.1]heptan-2-yl)isoindoline-1,3-dione |

| | | | |
|---|---|---|---|
| | 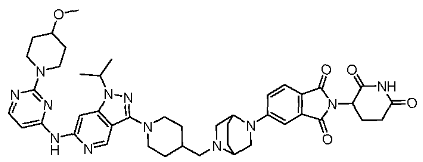 | 2-(2,6-dioxopiperidin-3-yl)-5-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.2]octan-2-yl)isoindoline-1,3-dione | 2-(2,6-dioxopiperidin-3-yl)-5-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.2]octan-2-yl)isoindoline-1,3-dione |
| | 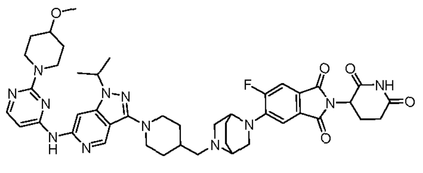 | 2-(2,6-dioxopiperidin-3-yl)-5-fluoro-6-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.2]octan-2-yl)isoindoline-1,3-dione | 2-(2,6-dioxopiperidin-3-yl)-5-fluoro-6-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.2]octan-2-yl)isoindoline-1,3-dione |
| | 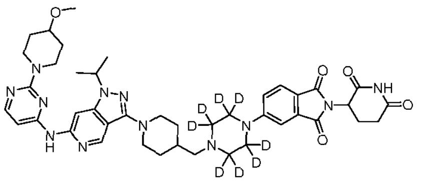 | 2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione | 2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione |
| | 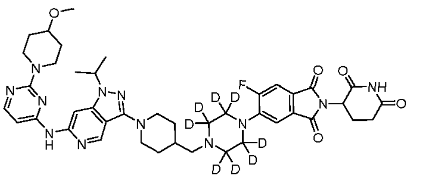 | 2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione | 2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione |
| | 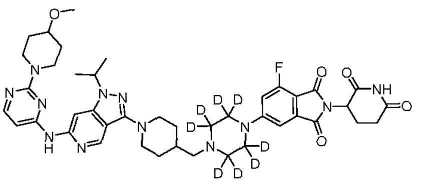 | 2-(2,6-dioxopiperidin-3-yl)-4-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione |
| | 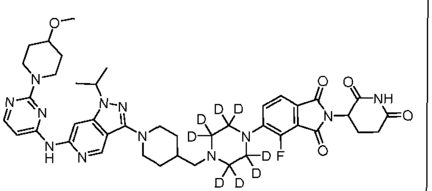 | 2-(2,6-dioxopiperidin-3-yl)-4-fluoro-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione |

| | | | |
|---|---|---|---|
| | 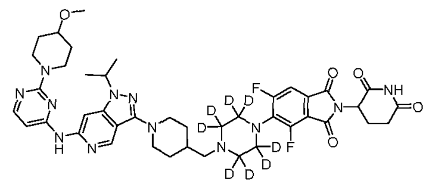 | 2-(2,6-dioxopiperidin-3-yl)-4,6-difluoro-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-*d*8)isoindoline-1,3-dione | 2-(2,6-dioxopiperidin-3-yl)-4,6-difluoro-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-*d*8)isoindoline-1,3-dione |
| | 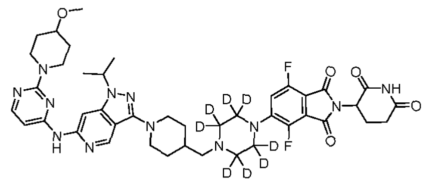 | 2-(2,6-dioxopiperidin-3-yl)-4,7-difluoro-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-*d*8)isoindoline-1,3-dione | 2-(2,6-dioxopiperidin-3-yl)-4,7-difluoro-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-*d*8)isoindoline-1,3-dione |
| | 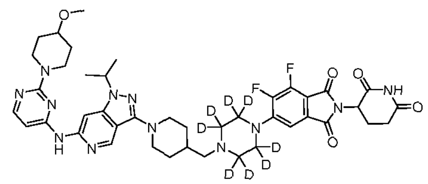 | 2-(2,6-dioxopiperidin-3-yl)-4,5-difluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-*d*8)isoindoline-1,3-dione | 2-(2,6-dioxopiperidin-3-yl)-4,5-difluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-*d*8)isoindoline-1,3-dione |
| | 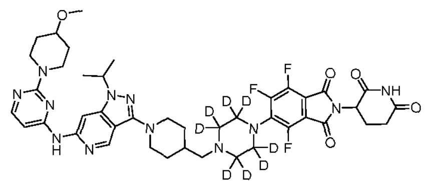 | 2-(2,6-dioxopiperidin-3-yl)-4,5,7-trifluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-*d*8)isoindoline-1,3-dione | 2-(2,6-dioxopiperidin-3-yl)-4,5,7-trifluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-*d*8)isoindoline-1,3-dione |
| | 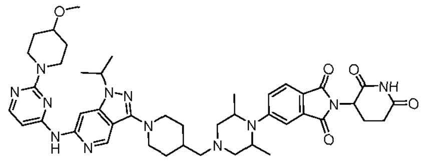 | 2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,6-dimethylpiperazin-1-yl)isoindoline-1,3-dione | 2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,6-dimethylpiperazin-1-yl)isoindoline-1,3-dione |
| | 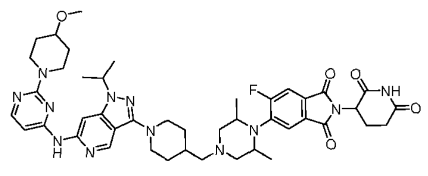 | 2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,6-dimethylpiperazin-1-yl)isoindoline-1,3-dione | 2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,6-dimethylpiperazin-1- |

| | | | |
|---|---|---|---|
| | | | yl)isoindoline-1,3-dione |
| | 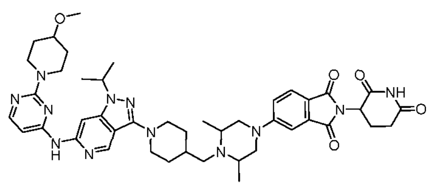 | 2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,5-dimethylpiperazin-1-yl)isoindoline-1,3-dione | 2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,5-dimethylpiperazin-1-yl)isoindoline-1,3-dione |
| | 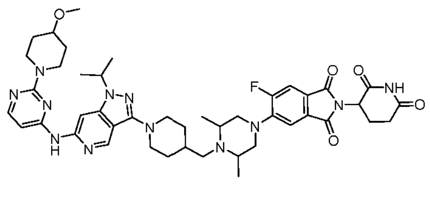 | 2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,5-dimethylpiperazin-1-yl)isoindoline-1,3-dione | 2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,5-dimethylpiperazin-1-yl)isoindoline-1,3-dione |
| | 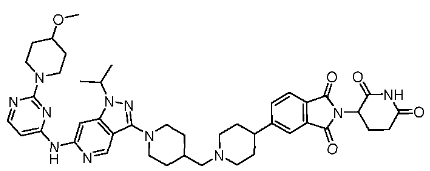 | 2-(2,6-dioxopiperidin-3-yl)-5-(1-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperidin-4-yl)isoindoline-1,3-dione | 2-(2,6-dioxopiperidin-3-yl)-5-(1-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperidin-4-yl)isoindoline-1,3-dione |
| | 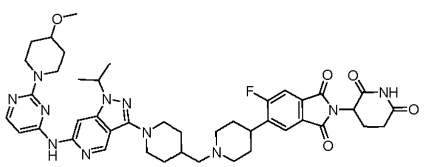 | 2-(2,6-dioxopiperidin-3-yl)-5-fluoro-6-(1-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperidin-4-yl)isoindoline-1,3-dione | 2-(2,6-dioxopiperidin-3-yl)-5-fluoro-6-(1-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperidin-4-yl)isoindoline-1,3-dione |
| | 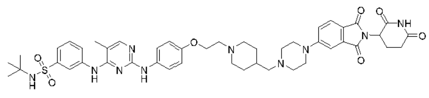 | N-(tert-butyl)-3-((2-((4-(2-(4-((4-(2-(2,6-dioxopiperidin-3-yl)-1,3-dioxoisoindolin-5-yl)piperazin-1-yl)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-1,3-dioxoisoindolin-5-yl)piperazin-1-yl)methyl)piperidin-1-yl)ethoxy)phenyl)amino)-5-methylpyrimidin-4-yl)amino)benzenesulfonamide |
| | 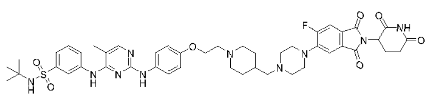 | N-(tert-butyl)-3-((2-((4-(2-(4-((4-(2-(2,6-dioxopiperidin-3-yl)-6-fluoro-1,3-dioxoisoindolin-5-yl)piperazin-1-yl)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-6-fluoro-1,3-dioxoisoindolin-5-yl)piperazin-1-yl)methyl)piperidin-1-yl)ethoxy)phenyl)amino)-5-methylpyrimidin-4-yl)amino)benzenesulfonamide |

| | | | |
|---|---|---|---|
| | 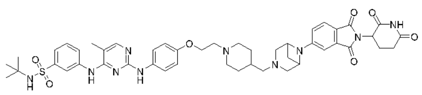 | N-(tert-butyl)-3-((2-((4-(2-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)ethoxy)phenyl)amino)-5-methylpyrimidin-4-yl)amino)benzenesulfonamide | N-(tert-butyl)-3-((2-((4-(2-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)ethoxy)phenyl)amino)-5-methylpyrimidin-4-yl)amino)benzenesulfonamide |
| | 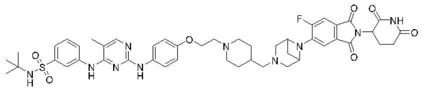 | N-(tert-butyl)-3-((2-((4-(2-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)ethoxy)phenyl)amino)-5-methylpyrimidin-4-yl)amino)benzenesulfonamide | N-(tert-butyl)-3-((2-((4-(2-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)ethoxy)phenyl)amino)-5-methylpyrimidin-4-yl)amino)benzenesulfonamide |
| | 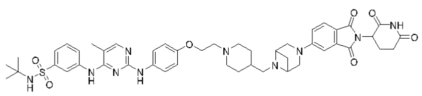 | N-(tert-butyl)-3-((2-((4-(2-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)ethoxy)phenyl)amino)-5-methylpyrimidin-4-yl)amino)benzenesulfonamide | N-(tert-butyl)-3-((2-((4-(2-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)ethoxy)phenyl)amino)-5-methylpyrimidin-4-yl)amino)benzenesulfonamide |
| | 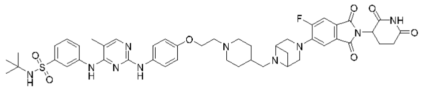 | N-(tert-butyl)-3-((2-((4-(2-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)ethoxy)phenyl)amino)-5-methylpyrimidin-4-yl)amino)benzenesulfonamide | N-(tert-butyl)-3-((2-((4-(2-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)ethoxy)phenyl)amino)-5-methylpyrimidin-4-yl)amino)benzenesulfonamide |
| | 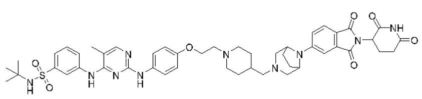 | N-(tert-butyl)-3-((2-((4-(2-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)ethoxy)phenyl)amino)-5-methylpyrimidin-4-yl)amino)benzenesulfonamide | N-(tert-butyl)-3-((2-((4-(2-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)ethoxy)phenyl)amino)-5-methylpyrimidin-4-yl)amino)benzenesulfonamide |
| | 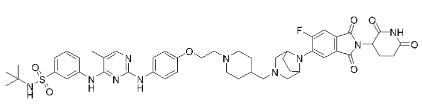 | N-(tert-butyl)-3-((2-((4-(2-(4-((8-(2-(2,6-dioxopiperidin-3-yl)-6-fluoro-1,3-dioxoisoindolin-5-yl)-3,8-diazabicyclo[3.2.1]octan-3- | N-(tert-butyl)-3-((2-((4-(2-(4-((8-(2-(2,6-dioxopiperidin-3-yl)-6-fluoro-1,3-dioxoisoindolin-5-yl)-3,8-diazabicyclo[3.2.1]octan-3- |

| | | | |
|---|---|---|---|
| | | yl)methyl)piperidin-1-yl)ethoxy)phenyl)amino)-5-methylpyrimidin-4-yl)amino)benzenesulfonamide | yl)methyl)piperidin-1-yl)ethoxy)phenyl)amino)-5-methylpyrimidin-4-yl)amino)benzenesulfonamide |
| | 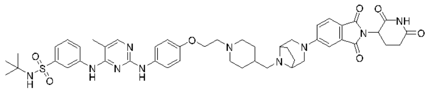 | N-(tert-butyl)-3-((2-((4-(2-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)ethoxy)phenyl)amino)-5-methylpyrimidin-4-yl)amino)benzenesulfonamide | N-(tert-butyl)-3-((2-((4-(2-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)ethoxy)phenyl)amino)-5-methylpyrimidin-4-yl)amino)benzenesulfonamide |
| | 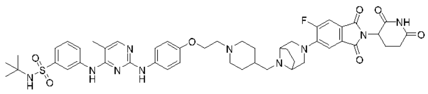 | N-(tert-butyl)-3-((2-((4-(2-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)ethoxy)phenyl)amino)-5-methylpyrimidin-4-yl)amino)benzenesulfonamide | N-(tert-butyl)-3-((2-((4-(2-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)ethoxy)phenyl)amino)-5-methylpyrimidin-4-yl)amino)benzenesulfonamide |
| | 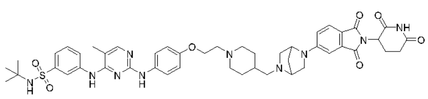 | N-(tert-butyl)-3-((2-((4-(2-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)ethoxy)phenyl)amino)-5-methylpyrimidin-4-yl)amino)benzenesulfonamide | N-(tert-butyl)-3-((2-((4-(2-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)ethoxy)phenyl)amino)-5-methylpyrimidin-4-yl)amino)benzenesulfonamide |
| | 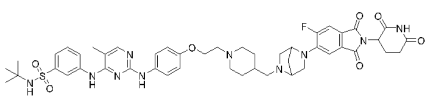 | N-(tert-butyl)-3-((2-((4-(2-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)ethoxy)phenyl)amino)-5-methylpyrimidin-4-yl)amino)benzenesulfonamide | N-(tert-butyl)-3-((2-((4-(2-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)ethoxy)phenyl)amino)-5-methylpyrimidin-4-yl)amino)benzenesulfonamide |
| | 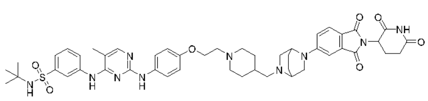 | N-(tert-butyl)-3-((2-((4-(2-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)ethoxy)phenyl)amino)-5-methylpyrimidin-4-yl)amino)benzenesulfonamide | N-(tert-butyl)-3-((2-((4-(2-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)ethoxy)phenyl)amino)-5-methylpyrimidin-4-yl)amino)benzenesulfonamide |

| | | | |
|---|---|---|---|
| | 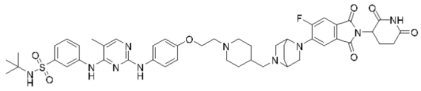 | N-(tert-butyl)-3-((2-((4-(2-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)ethoxy)phenyl)amino)-5-methylpyrimidin-4-yl)amino)benzenesulfonamide | N-(tert-butyl)-3-((2-((4-(2-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)ethoxy)phenyl)amino)-5-methylpyrimidin-4-yl)amino)benzenesulfonamide |
| | 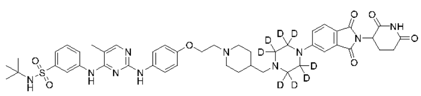 | N-(tert-butyl)-3-((2-((4-(2-(4-((4-(2-(2,6-dioxopiperidin-3-yl)-1,3-dioxoisoindolin-5-yl)piperazin-1-yl-2,2,3,3,5,5,6,6-*d*8)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-1,3-dioxoisoindolin-5-yl)piperazin-1-yl-2,2,3,3,5,5,6,6-*d*8)methyl)piperidin-1-yl)ethoxy)phenyl)amino)-5-methylpyrimidin-4-yl)amino)benzenesulfonamide |
| | 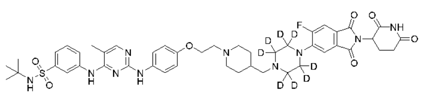 | N-(tert-butyl)-3-((2-((4-(2-(4-((4-(2-(2,6-dioxopiperidin-3-yl)-6-fluoro-1,3-dioxoisoindolin-5-yl)piperazin-1-yl-2,2,3,3,5,5,6,6-*d*8)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-6-fluoro-1,3-dioxoisoindolin-5-yl)piperazin-1-yl-2,2,3,3,5,5,6,6-*d*8)methyl)piperidin-1-yl)ethoxy)phenyl)amino)-5-methylpyrimidin-4-yl)amino)benzenesulfonamide |
| | 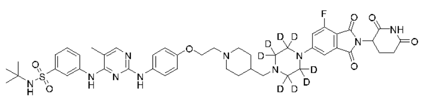 | N-(tert-butyl)-3-((2-((4-(2-(4-((4-(2-(2,6-dioxopiperidin-3-yl)-7-fluoro-1,3-dioxoisoindolin-5-yl)piperazin-1-yl-2,2,3,3,5,5,6,6-*d*8)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-7-fluoro-1,3-dioxoisoindolin-5-yl)piperazin-1-yl-2,2,3,3,5,5,6,6-*d*8)methyl)piperidin-1-yl)ethoxy)phenyl)amino)-5-methylpyrimidin-4-yl)amino)benzenesulfonamide |
| | 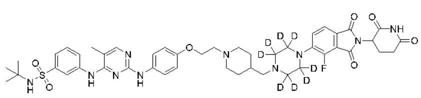 | N-(tert-butyl)-3-((2-((4-(2-(4-((4-(2-(2,6-dioxopiperidin-3-yl)-4-fluoro-1,3-dioxoisoindolin-5-yl)piperazin-1-yl-2,2,3,3,5,5,6,6-*d*8)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-4-fluoro-1,3-dioxoisoindolin-5-yl)piperazin-1-yl-2,2,3,3,5,5,6,6-*d*8)methyl)piperidin-1-yl)ethoxy)phenyl)amino)-5-methylpyrimidin-4-yl)amino)benzenesulfonamide |
| | 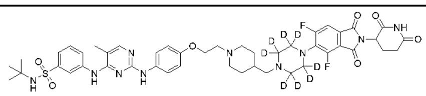 | N-(tert-butyl)-3-((2-((4-(2-(4-((4-(2-(2,6-dioxopiperidin-3-yl)-4,6- | N-(tert-butyl)-3-((2-((4-(2-(4-((4-(2-(2,6-dioxopiperidin-3-yl)-4,6- |

| | | | |
|---|---|---|---|
| | | difluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide | difluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide |
| | 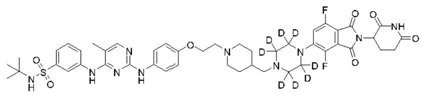 | N-(tert-butyl)-3-((2-((4-(2-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide |
| | 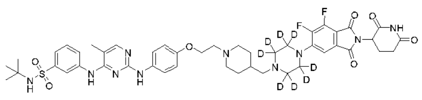 | N-(tert-butyl)-3-((2-((4-(2-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide |
| | 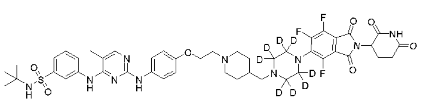 | N-(tert-butyl)-3-((2-((4-(2-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide |
| | 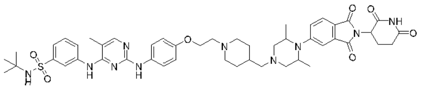 | N-(tert-butyl)-3-((2-((4-(2-(4-((4-(2-(2,6-dioxopiperidin-3-yl)-1,3-dioxoisoindolin-5-yl)-3,5-dimethylpiperazin-1-yl)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-1,3-dioxoisoindolin-5-yl)-3,5-dimethylpiperazin-1-yl)methyl)piperidin-1-yl)ethoxy)phenyl)amino)-5-methylpyrimidin-4-yl)amino)benzenesulfonamide |
| | 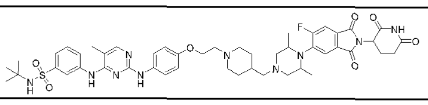 | N-(tert-butyl)-3-((2-((4-(2-(4-((4-(2-(2,6-dioxopiperidin-3-yl)-6- | N-(tert-butyl)-3-((2-((4-(2-(4-((4-(2-(2,6-dioxopiperidin-3-yl)-6- |

| | | | |
|---|---|---|---|
| | | fluoro-1,3-dioxoisoindolin-5-yl)-3,5-dimethylpiperazin-1-yl)methyl)piperidin-1-yl)ethoxy)phenyl)amino)-5-methylpyrimidin-4-yl)amino)benzenesulfonamide | fluoro-1,3-dioxoisoindolin-5-yl)-3,5-dimethylpiperazin-1-yl)methyl)piperidin-1-yl)ethoxy)phenyl)amino)-5-methylpyrimidin-4-yl)amino)benzenesulfonamide |
| | 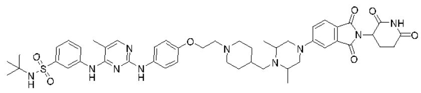 | N-(tert-butyl)-3-((2-((4-(2-(4-((4-(2-(2,6-dioxopiperidin-3-yl)-1,3-dioxoisoindolin-5-yl)-2,6-dimethylpiperazin-1-yl)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-1,3-dioxoisoindolin-5-yl)-2,6-dimethylpiperazin-1-yl)methyl)piperidin-1-yl)ethoxy)phenyl)amino)-5-methylpyrimidin-4-yl)amino)benzenesulfonamide |
| | 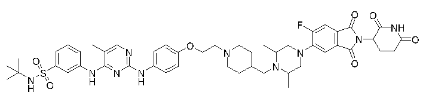 | N-(tert-butyl)-3-((2-((4-(2-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)ethoxy)phenyl)amino)-5-methylpyrimidin-4-yl)amino)benzenesulfonamide |
| | 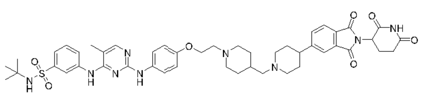 | N-(tert-butyl)-3-((2-((4-(2-(4-((4-(2-(2,6-dioxopiperidin-3-yl)-1,3-dioxoisoindolin-5-yl)piperidin-1-yl)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-1,3-dioxoisoindolin-5-yl)piperidin-1-yl)methyl)piperidin-1-yl)ethoxy)phenyl)amino)-5-methylpyrimidin-4-yl)amino)benzenesulfonamide |
| | 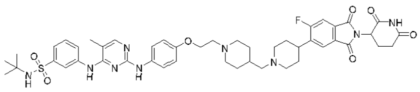 | N-(tert-butyl)-3-((2-((4-(2-(4-((4-(2-(2,6-dioxopiperidin-3-yl)-6-fluoro-1,3-dioxoisoindolin-5-yl)piperidin-1-yl)methyl)piperidin-1-yl)ethoxy)phenyl)amino)-5-methylpyrimidin-4-yl)amino)benzenesulfonamide | N-(tert-butyl)-3-((2-((4-(2-(4-((4-(2-(2,6-dioxopiperidin-3-yl)-6-fluoro-1,3-dioxoisoindolin-5-yl)piperidin-1-yl)methyl)piperidin-1-yl)ethoxy)phenyl)amino)-5-methylpyrimidin-4-yl)amino)benzenesulfonamide |
| | 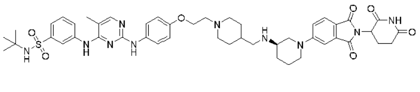 | N-(tert-butyl)-3-((2-((4-(2-(4-((((3R)-1-(2-(2,6-dioxopiperidin-3-yl)-1,3-dioxoisoindolin-5-yl)piperidin-3-yl)amino)methyl)piperidin-1-yl)ethoxy)phenyl)amino)-5-methylpyrimidin-4-yl)amino)benzenesulfonamid | N-(tert-butyl)-3-((2-((4-(2-(4-((((3R)-1-(2-(2,6-dioxopiperidin-3-yl)-1,3-dioxoisoindolin-5-yl)piperidin-3-yl)amino)methyl)piperidin-1-yl)ethoxy)phenyl)amino)-5-methylpyrimidin-4- |

| | | | |
|---|---|---|---|
| | | e | yl)amino)benzenesulfonamide |
| | 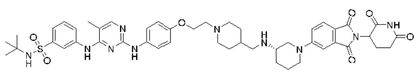 | N-(tert-butyl)-3-((2-((4-(2-(4-((((3S)-1-(2-(2,6-dioxopiperidin-3-yl)-1,3-dioxoisoindolin-5-yl)piperidin-3-yl)amino)methyl)piperidin-1-yl)ethoxy)phenyl)amino)-5-methylpyrimidin-4-yl)amino)benzenesulfonamide | N-(tert-butyl)-3-((2-((4-(2-(4-((((3S)-1-(2-(2,6-dioxopiperidin-3-yl)-1,3-dioxoisoindolin-5-yl)piperidin-3-yl)amino)methyl)piperidin-1-yl)ethoxy)phenyl)amino)-5-methylpyrimidin-4-yl)amino)benzenesulfonamide |
| | 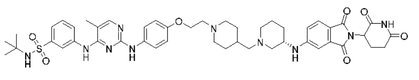 | N-(tert-butyl)-3-((2-((4-(2-(4-(((3S)-3-((2-(2,6-dioxopiperidin-3-yl)-1,3-dioxoisoindolin-5-yl)amino)piperidin-1-yl)methyl)piperidin-1-yl)ethoxy)phenyl)amino)-5-methylpyrimidin-4-yl)amino)benzenesulfonamide | N-(tert-butyl)-3-((2-((4-(2-(4-(((3S)-3-((2-(2,6-dioxopiperidin-3-yl)-1,3-dioxoisoindolin-5-yl)amino)piperidin-1-yl)methyl)piperidin-1-yl)ethoxy)phenyl)amino)-5-methylpyrimidin-4-yl)amino)benzenesulfonamide |
| | 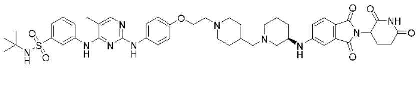 | N-(tert-butyl)-3-((2-((4-(2-(4-(((3R)-3-((2-(2,6-dioxopiperidin-3-yl)-1,3-dioxoisoindolin-5-yl)amino)piperidin-1-yl)methyl)piperidin-1-yl)ethoxy)phenyl)amino)-5-methylpyrimidin-4-yl)amino)benzenesulfonamide | N-(tert-butyl)-3-((2-((4-(2-(4-(((3R)-3-((2-(2,6-dioxopiperidin-3-yl)-1,3-dioxoisoindolin-5-yl)amino)piperidin-1-yl)methyl)piperidin-1-yl)ethoxy)phenyl)amino)-5-methylpyrimidin-4-yl)amino)benzenesulfonamide |
| | 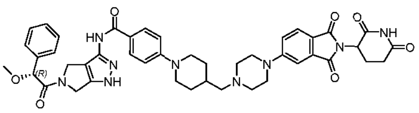 | 4-(4-((4-(2-(2,6-dioxopiperidin-3-yl)-1,3-dioxoisoindolin-5-yl)piperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-1,3-dioxoisoindolin-5-yl)piperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 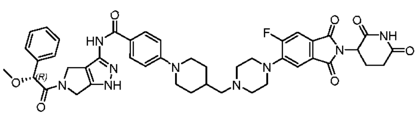 | 4-(4-((4-(2-(2,6-dioxopiperidin-3-yl)-6-fluoro-1,3-dioxoisoindolin-5-yl)piperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-6-fluoro-1,3-dioxoisoindolin-5-yl)piperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 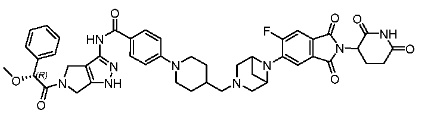 | 4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-N-(5-((R)-2-methoxy-2- | 4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-N-(5-((R)-2-methoxy- |

| | | | |
|---|---|---|---|
| | | phenylacetyl)-1,4,5,6-tetrahydropyrrolo[3,4-c]pyrazol-3-yl)benzamide | 2-phenylacetyl)-1,4,5,6-tetrahydropyrrolo[3,4-c]pyrazol-3-yl)benzamide |
| | 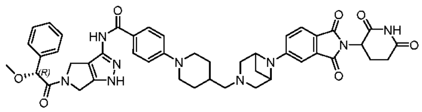 | 4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 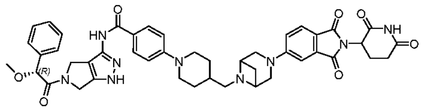 | 4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 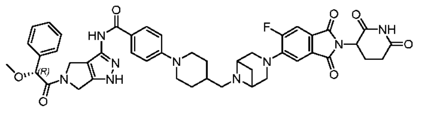 | 4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 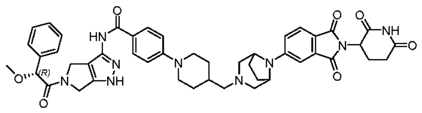 | 4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 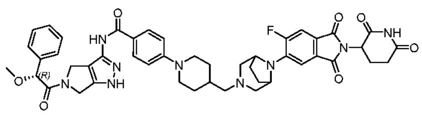 | 4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 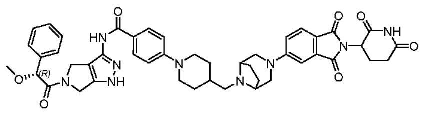 | 4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-N-(5-((R)-2-methoxy-2-phenylacetyl)-1,4,5,6-tetrahydropyrrolo[3,4- | 4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-N-(5-((R)-2-methoxy-2-phenylacetyl)-1,4,5,6-tetrahydropyrrolo[3,4- |

| | | | |
|---|---|---|---|
| | | c]pyrazol-3-yl)benzamide | c]pyrazol-3-yl)benzamide |
| | 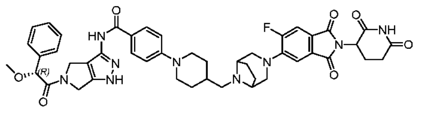 | 4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 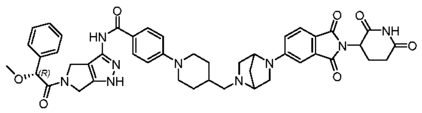 | 4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 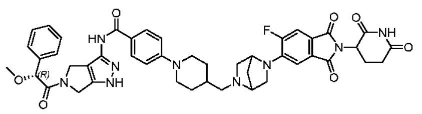 | 4-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 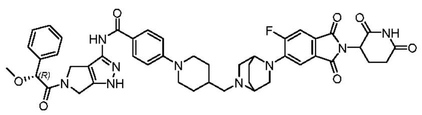 | 4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 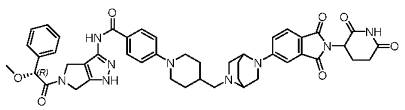 | 4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 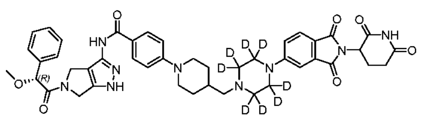 | 4-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)-N-(5-((R)-2-methoxy-2-phenylacetyl)-1,4,5,6-tetrahydropyrrolo[3,4- | 4-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)-N-(5-((R)-2-methoxy-2-phenylacetyl)-1,4,5,6-tetrahydropyrrolo[3,4- |

| | | | |
|---|---|---|---|
| | | c]pyrazol-3-yl)benzamide | c]pyrazol-3-yl)benzamide |
| | 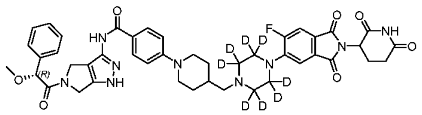 | 4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide |
| | 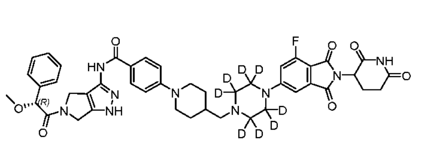 | 4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide |
| | 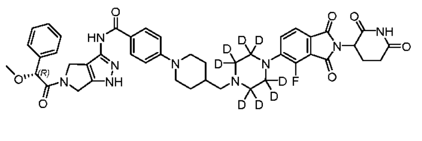 | 4-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide |
| | 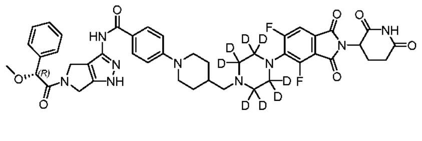 | 4-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide |
| | 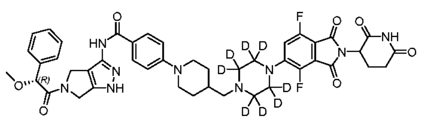 | 4-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide |
| | 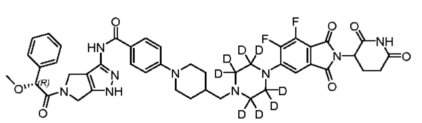 | 4-(4-((4-(2-(2,6-dioxopiperidin-3-yl)-6,7-difluoro-1,3-dioxoisoindolin-5-yl)piperazin-1-yl-2,2,3,3,5,5,6,6- | 4-(4-((4-(2-(2,6-dioxopiperidin-3-yl)-6,7-difluoro-1,3-dioxoisoindolin-5-yl)piperazin-1-yl- |

| | | | |
|---|---|---|---|
| | | $d_8$)methyl)piperidin-1-yl)-N-(5-((R)-2-methoxy-2-phenylacetyl)-1,4,5,6-tetrahydropyrrolo[3,4-c]pyrazol-3-yl)benzamide | 2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide |
| | 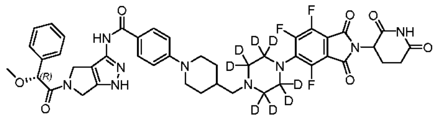 | 4-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide |
| | 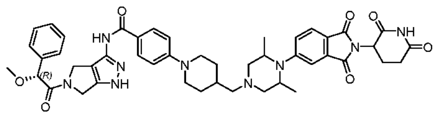 | 4-(4-((4-(2-(2,6-dioxopiperidin-3-yl)-1,3-dioxoisoindolin-5-yl)-3,5-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-1,3-dioxoisoindolin-5-yl)-3,5-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 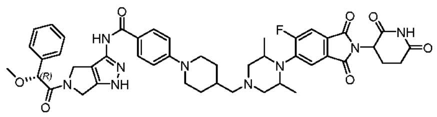 | 4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 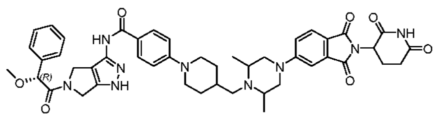 | 4-(4-((4-(2-(2,6-dioxopiperidin-3-yl)-1,3-dioxoisoindolin-5-yl)-2,6-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-1,3-dioxoisoindolin-5-yl)-2,6-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 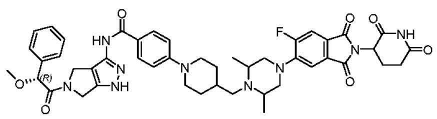 | 4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |

| | | | |
|---|---|---|---|
| | 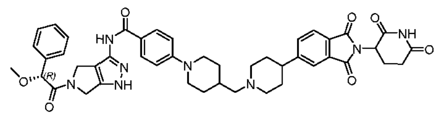 | 4-(4-((4-(2-(2,6-dioxopiperidin-3-yl)-1,3-dioxoisoindolin-5-yl)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-1,3-dioxoisoindolin-5-yl)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 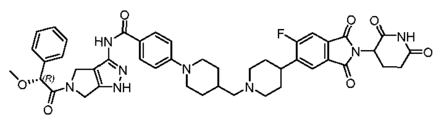 | 4-(4-((4-(2-(2,6-dioxopiperidin-3-yl)-6-fluoro-1,3-dioxoisoindolin-5-yl)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((4-(2-(2,6-dioxopiperidin-3-yl)-6-fluoro-1,3-dioxoisoindolin-5-yl)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 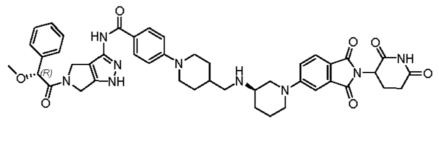 | 4-(4-((((3R)-1-(2-(2,6-dioxopiperidin-3-yl)-1,3-dioxoisoindolin-5-yl)piperidin-3-yl)amino)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((((3R)-1-(2-(2,6-dioxopiperidin-3-yl)-1,3-dioxoisoindolin-5-yl)piperidin-3-yl)amino)methyl)piperidin-1-yl)-N-(5-((R)-2-methoxy-2-phenylacetyl)-1,4,5,6-tetrahydropyrrolo[3,4-c]pyrazol-3-yl)benzamide |
| | 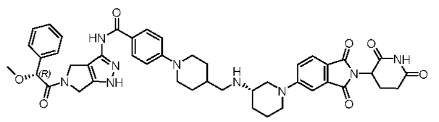 | 4-(4-((((3S)-1-(2-(2,6-dioxopiperidin-3-yl)-1,3-dioxoisoindolin-5-yl)piperidin-3-yl)amino)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-((((3S)-1-(2-(2,6-dioxopiperidin-3-yl)-1,3-dioxoisoindolin-5-yl)piperidin-3-yl)amino)methyl)piperidin-1-yl)-N-(5-((R)-2-methoxy-2-phenylacetyl)-1,4,5,6-tetrahydropyrrolo[3,4-c]pyrazol-3-yl)benzamide |
| | 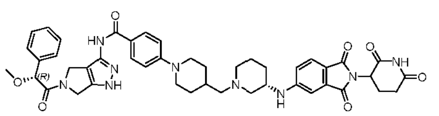 | 4-(4-(((3S)-3-((2-(2,6-dioxopiperidin-3-yl)-1,3-dioxoisoindolin-5-yl)amino)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-(((3S)-3-((2-(2,6-dioxopiperidin-3-yl)-1,3-dioxoisoindolin-5-yl)amino)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |
| | 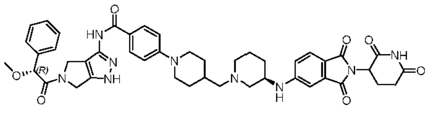 | 4-(4-(((3R)-3-((2-(2,6-dioxopiperidin-3-yl)-1,3-dioxoisoindolin-5-yl)amino)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide | 4-(4-(((3R)-3-((2-(2,6-dioxopiperidin-3-yl)-1,3-dioxoisoindolin-5-yl)amino)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide |

| | | | |
|---|---|---|---|
| | 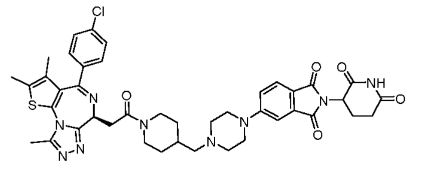 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl)-2-(2,6-dioxopiperidin-3-yl)isoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl)-2-(2,6-dioxopiperidin-3-yl)isoindoline-1,3-dione |
| | 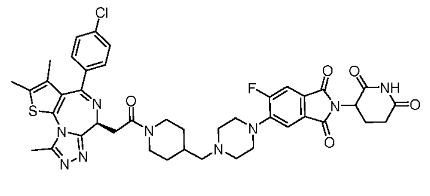 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl)-2-(2,6-dioxopiperidin-3-yl)-6-fluoroisoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl)-2-(2,6-dioxopiperidin-3-yl)-6-fluoroisoindoline-1,3-dione |
| | 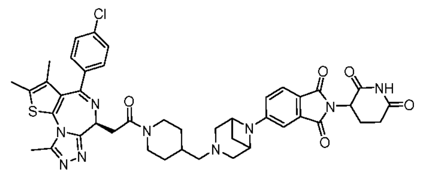 | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-6-yl)-2-(2,6-dioxopiperidin-3-yl)isoindoline-1,3-dione |
| | 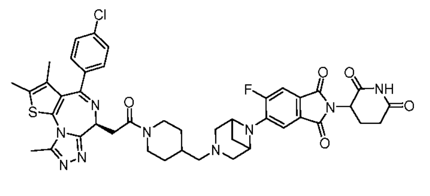 | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 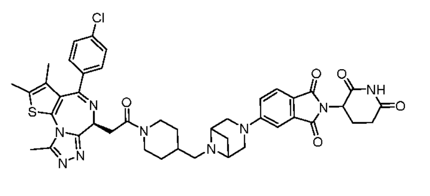 | 5-(6-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-3-yl)-2-(2,6-dioxopiperidin-3-yl)isoindoline-1,3-dione |
| | 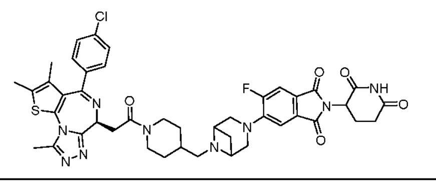 | 5-(6-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4- | 5-(6-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4- |

| | | | |
|---|---|---|---|
| | | yl)methyl)-3,6-diazabicyclo[3.1.1]heptan-3-yl)-2-(2,6-dioxopiperidin-3-yl)-6-fluoroisoindoline-1,3-dione | yl)methyl)-3,6-diazabicyclo[3.1.1]heptan-3-yl)-2-(2,6-dioxopiperidin-3-yl)-6-fluoroisoindoline-1,3-dione |
| | 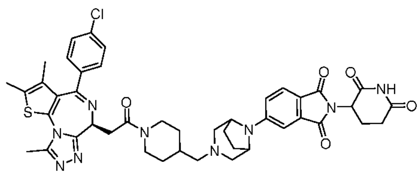 | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)isoindoline-1,3-dione | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)isoindoline-1,3-dione |
| | 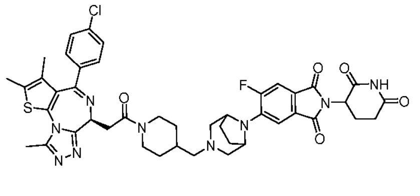 | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)-6-fluoroisoindoline-1,3-dione |
| | 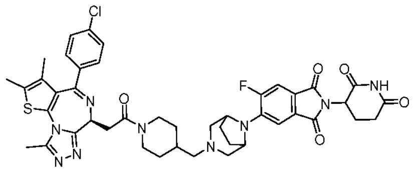 | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)-6-fluoroisoindoline-1,3-dione |
| | 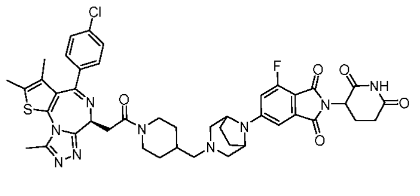 | 6-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)-4-fluoroisoindoline-1,3-dione | 6-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)-4-fluoroisoindoline-1,3-dione |
| | 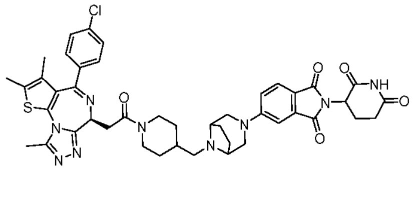 | 5-(8-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-3-yl)-2-(2,6-dioxopiperidin-3-yl)isoindoline-1,3-dione | 5-(8-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-3-yl)-2-(2,6-dioxopiperidin-3-yl)isoindoline-1,3-dione |

| | | | |
|---|---|---|---|
| | 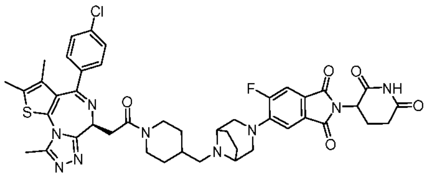 | 5-(8-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 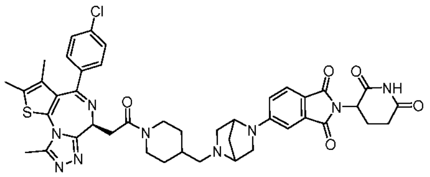 | 5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.1]heptan-2-yl)-2-(2,6-dioxopiperidin-3-yl)isoindoline-1,3-dione |
| | 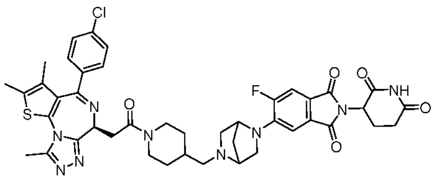 | 5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 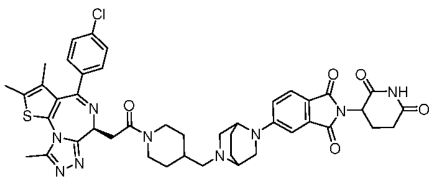 | 5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.2]octan-2-yl)-2-(2,6-dioxopiperidin-3-yl)isoindoline-1,3-dione | 5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.2]octan-2-yl)-2-(2,6-dioxopiperidin-3-yl)isoindoline-1,3-dione |
| | 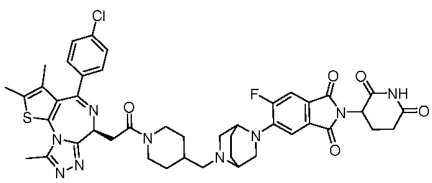 | 5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.2]octan-2-yl)-2-(2,6-dioxopiperidin-3-yl)-6-fluoroisoindoline-1,3-dione |

| | | | |
|---|---|---|---|
| | 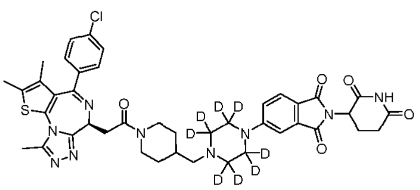 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)isoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)isoindoline-1,3-dione |
| | 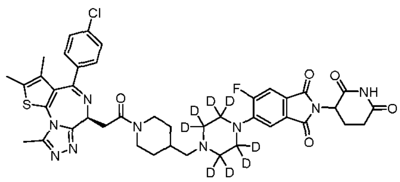 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)-6-fluoroisoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)-6-fluoroisoindoline-1,3-dione |
| | 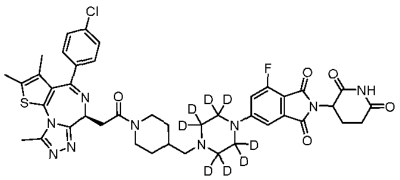 | 6-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)-4-fluoroisoindoline-1,3-dione | 6-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)-4-fluoroisoindoline-1,3-dione |
| | 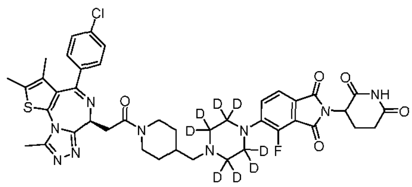 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)-4-fluoroisoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)-4-fluoroisoindoline-1,3-dione |
| | 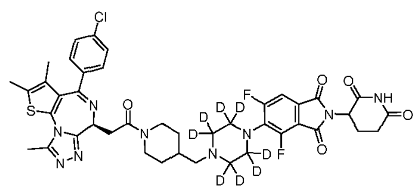 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)-4,6-difluoroisoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-*d*8)-2-(2,6-dioxopiperidin-3-yl)-4,6-difluoroisoindoline-1,3-dione |
| | 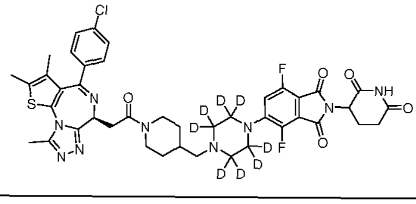 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl- | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl- |

| | | | |
|---|---|---|---|
| | | 2,2,3,3,5,5,6,6-$d_8$)-2-(2,6-dioxopiperidin-3-yl)-4,7-difluoroisoindoline-1,3-dione | 2,2,3,3,5,5,6,6-$d_8$)-2-(2,6-dioxopiperidin-3-yl)-4,7-difluoroisoindoline-1,3-dione |
| | 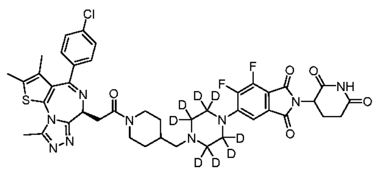 | 6-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione | 6-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione |
| | 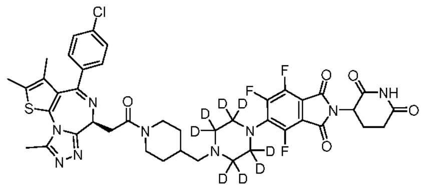 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |
| | 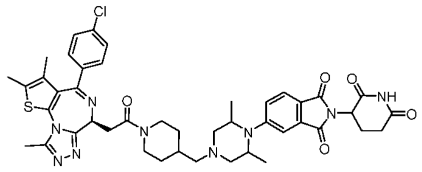 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,6-dimethylpiperazin-1-yl)-2-(2,6-dioxopiperidin-3-yl)isoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,6-dimethylpiperazin-1-yl)-2-(2,6-dioxopiperidin-3-yl)isoindoline-1,3-dione |
| | 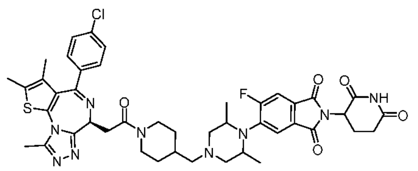 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,6-dimethylpiperazin-1-yl)-2-(2,6-dioxopiperidin-3-yl)-6-fluoroisoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,6-dimethylpiperazin-1-yl)-2-(2,6-dioxopiperidin-3-yl)-6-fluoroisoindoline-1,3-dione |
| | 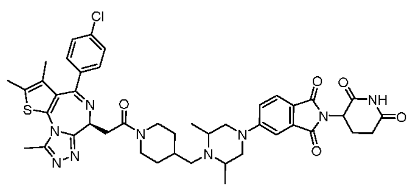 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,5-dimethylpiperazin-1-yl)-2-(2,6-dioxopiperidin-3-yl)isoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,5-dimethylpiperazin-1-yl)-2-(2,6-dioxopiperidin-3-yl)isoindoline-1,3-dione |

| | | | |
|---|---|---|---|
| | 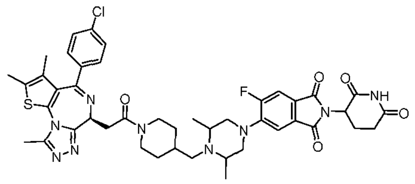 | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,5-dimethylpiperazin-1-yl)-2-(2,6-dioxopiperidin-3-yl)-6-fluoroisoindoline-1,3-dione | 5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,5-dimethylpiperazin-1-yl)-2-(2,6-dioxopiperidin-3-yl)-6-fluoroisoindoline-1,3-dione |
| | 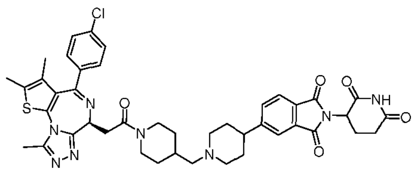 | 5-(1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-4-yl)-2-(2,6-dioxopiperidin-3-yl)isoindoline-1,3-dione | 5-(1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-4-yl)-2-(2,6-dioxopiperidin-3-yl)isoindoline-1,3-dione |
| | 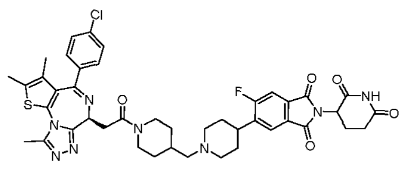 | 5-(1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-4-yl)-2-(2,6-dioxopiperidin-3-yl)-6-fluoroisoindoline-1,3-dione | 5-(1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-4-yl)-2-(2,6-dioxopiperidin-3-yl)-6-fluoroisoindoline-1,3-dione |
| | 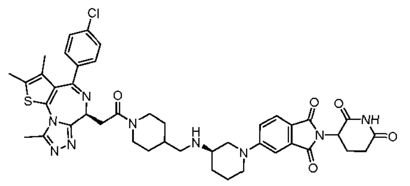 | 5-((R)-3-(((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)piperidin-1-yl)-2-(2,6-dioxopiperidin-3-yl)isoindoline-1,3-dione | 5-((R)-3-(((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)piperidin-1-yl)-2-(2,6-dioxopiperidin-3-yl)isoindoline-1,3-dione |
| | 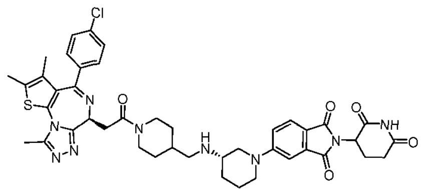 | 5-((S)-3-(((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)piperidin-1-yl)-2-(2,6-dioxopiperidin-3-yl)isoindoline-1,3-dione | 5-((S)-3-(((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)piperidin-1-yl)-2-(2,6-dioxopiperidin-3-yl)isoindoline-1,3-dione |
| | 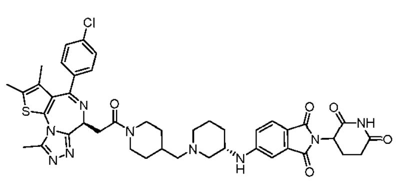 | 5-(((S)-1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-3-yl)amino)-2-(2,6- | 5-(((S)-1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-3-yl)amino)-2-(2,6- |

| | | | |
|---|---|---|---|
| | | dioxopiperidin-3-yl)isoindoline-1,3-dione | dioxopiperidin-3-yl)isoindoline-1,3-dione |
| | 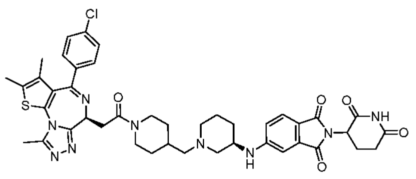 | 5-(((R)-1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-3-yl)amino)-2-(2,6-dioxopiperidin-3-yl)isoindoline-1,3-dione | 5-(((R)-1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-3-yl)amino)-2-(2,6-dioxopiperidin-3-yl)isoindoline-1,3-dione |
| | 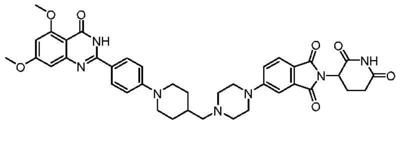 | 5-(4-((1-(4-(5,7-dimethoxy-4-oxo-3,4-dihydroquinazolin-2-yl)phenyl)piperidin-4-yl)methyl)piperazin-1-yl)-2-(2,6-dioxopiperidin-3-yl)isoindoline-1,3-dione | 5-(4-((1-(4-(5,7-dimethoxy-4-oxo-3,4-dihydroquinazolin-2-yl)phenyl)piperidin-4-yl)methyl)piperazin-1-yl)-2-(2,6-dioxopiperidin-3-yl)isoindoline-1,3-dione |
| | 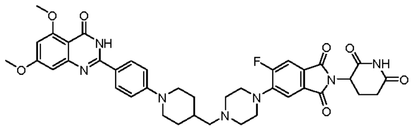 | 5-(4-((1-(4-(5,7-dimethoxy-4-oxo-3,4-dihydroquinazolin-2-yl)phenyl)piperidin-4-yl)methyl)piperazin-1-yl)-2-(2,6-dioxopiperidin-3-yl)-6-fluoroisoindoline-1,3-dione | 5-(4-((1-(4-(5,7-dimethoxy-4-oxo-3,4-dihydroquinazolin-2-yl)phenyl)piperidin-4-yl)methyl)piperazin-1-yl)-2-(2,6-dioxopiperidin-3-yl)-6-fluoroisoindoline-1,3-dione |
| | 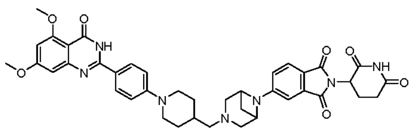 | 5-(3-((1-(4-(5,7-dimethoxy-4-oxo-3,4-dihydroquinazolin-2-yl)phenyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(4-(5,7-dimethoxy-4-oxo-3,4-dihydroquinazolin-2-yl)phenyl)piperidin-4-yl)methyl)-3,6-diazabicyclo[3.1.1]heptan-6-yl)-2-(2,6-dioxopiperidin-3-yl)isoindoline-1,3-dione |
| | 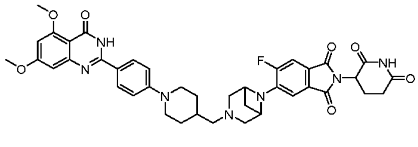 | 5-(3-((1-(4-(5,7-dimethoxy-4-oxo-3,4-dihydroquinazolin-2-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(4-(5,7-dimethoxy-4-oxo-3,4-dihydroquinazolin-2-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 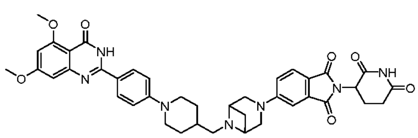 | 5-(6-((1-(4-(5,7-dimethoxy-4-oxo-3,4-dihydroquinazolin-2-yl)phenyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(4-(5,7-dimethoxy-4-oxo-3,4-dihydroquinazolin-2-yl)phenyl)piperidin-4-yl)methyl)-3,6-diazabicyclo[3.1.1]heptan-3-yl)-2-(2,6-dioxopiperidin-3-yl)isoindoline-1,3-dione |
| | 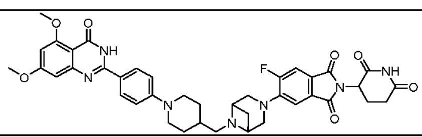 | 5-(6-((1-(4-(5,7-dimethoxy-4-oxo-3,4-dihydroquinazolin-2-yl)phenyl)piperidin-4- | 5-(6-((1-(4-(5,7-dimethoxy-4-oxo-3,4-dihydroquinazolin-2-yl)phenyl)piperidin-4- |

| | | | |
|---|---|---|---|
| | | yl)methyl)-3,6-diazabicyclo[3.1.1]heptan-3-yl)-2-(2,6-dioxopiperidin-3-yl)-6-fluoroisoindoline-1,3-dione | yl)methyl)-3,6-diazabicyclo[3.1.1]heptan-3-yl)-2-(2,6-dioxopiperidin-3-yl)-6-fluoroisoindoline-1,3-dione |
| | 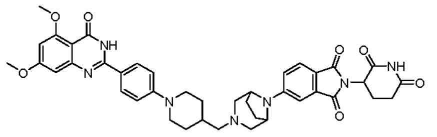 | 5-(3-((1-(4-(5,7-dimethoxy-4-oxo-3,4-dihydroquinazolin-2-yl)phenyl)piperidin-4-yl)methyl)-3,8-diazabicyclo[3.2.1]octan-8-yl)-2-(2,6-dioxopiperidin-3-yl)isoindoline-1,3-dione | 5-(3-((1-(4-(5,7-dimethoxy-4-oxo-3,4-dihydroquinazolin-2-yl)phenyl)piperidin-4-yl)methyl)-3,8-diazabicyclo[3.2.1]octan-8-yl)-2-(2,6-dioxopiperidin-3-yl)isoindoline-1,3-dione |
| | 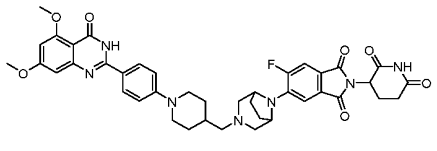 | 5-(3-((1-(4-(5,7-dimethoxy-4-oxo-3,4-dihydroquinazolin-2-yl)phenyl)piperidin-4-yl)methyl)-3,8-diazabicyclo[3.2.1]octan-8-yl)-2-(2,6-dioxopiperidin-3-yl)isoindoline-1,3-dione | 5-(3-((1-(4-(5,7-dimethoxy-4-oxo-3,4-dihydroquinazolin-2-yl)phenyl)piperidin-4-yl)methyl)-3,8-diazabicyclo[3.2.1]octan-8-yl)-2-(2,6-dioxopiperidin-3-yl)isoindoline-1,3-dione |
| | 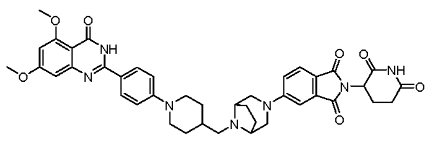 | 5-(8-((1-(4-(5,7-dimethoxy-4-oxo-3,4-dihydroquinazolin-2-yl)phenyl)piperidin-4-yl)methyl)-3,8-diazabicyclo[3.2.1]octan-3-yl)-2-(2,6-dioxopiperidin-3-yl)isoindoline-1,3-dione | 5-(8-((1-(4-(5,7-dimethoxy-4-oxo-3,4-dihydroquinazolin-2-yl)phenyl)piperidin-4-yl)methyl)-3,8-diazabicyclo[3.2.1]octan-3-yl)-2-(2,6-dioxopiperidin-3-yl)isoindoline-1,3-dione |
| | 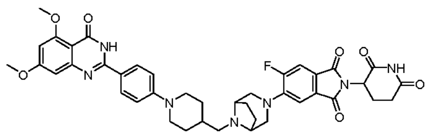 | 5-(8-((1-(4-(5,7-dimethoxy-4-oxo-3,4-dihydroquinazolin-2-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(4-(5,7-dimethoxy-4-oxo-3,4-dihydroquinazolin-2-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 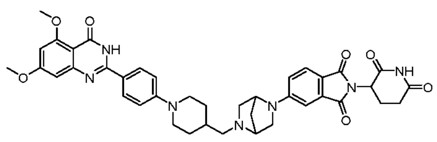 | 5-(5-((1-(4-(5,7-dimethoxy-4-oxo-3,4-dihydroquinazolin-2-yl)phenyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(4-(5,7-dimethoxy-4-oxo-3,4-dihydroquinazolin-2-yl)phenyl)piperidin-4-yl)methyl)-2,5-diazabicyclo[2.2.1]heptan-2-yl)-2-(2,6-dioxopiperidin-3-yl)isoindoline-1,3-dione |
| | 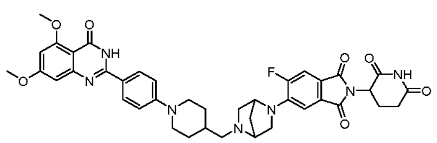 | 5-(5-((1-(4-(5,7-dimethoxy-4-oxo-3,4-dihydroquinazolin-2-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(4-(5,7-dimethoxy-4-oxo-3,4-dihydroquinazolin-2-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione |

| | | | |
|---|---|---|---|
| | 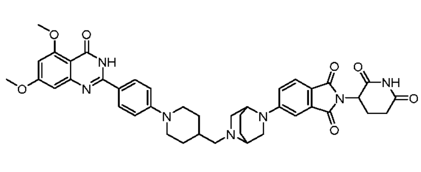 | 5-(5-((1-(4-(5,7-dimethoxy-4-oxo-3,4-dihydroquinazolin-2-yl)phenyl)piperidin-4-yl)methyl)-2,5-diazabicyclo[2.2.2]octan-2-yl)-2-(2,6-dioxopiperidin-3-yl)isoindoline-1,3-dione | 5-(5-((1-(4-(5,7-dimethoxy-4-oxo-3,4-dihydroquinazolin-2-yl)phenyl)piperidin-4-yl)methyl)-2,5-diazabicyclo[2.2.2]octan-2-yl)-2-(2,6-dioxopiperidin-3-yl)isoindoline-1,3-dione |
| | 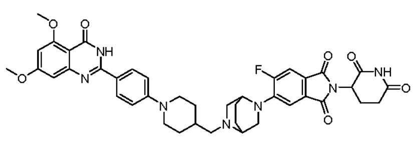 | 5-(5-((1-(4-(5,7-dimethoxy-4-oxo-3,4-dihydroquinazolin-2-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(4-(5,7-dimethoxy-4-oxo-3,4-dihydroquinazolin-2-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 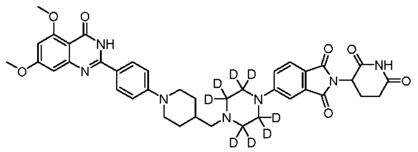 | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)isoindoline-1,3-dione | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)isoindoline-1,3-dione |
| | 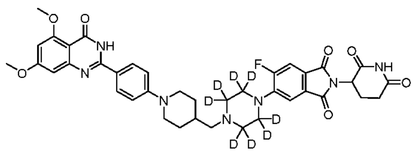 | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione |
| | 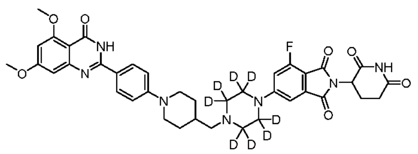 | 6-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 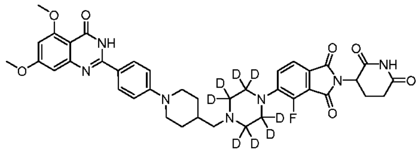 | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 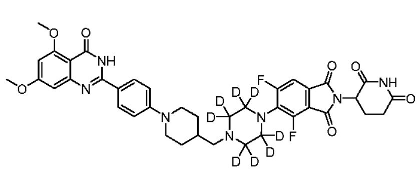 | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |

| | | | |
|---|---|---|---|
| | 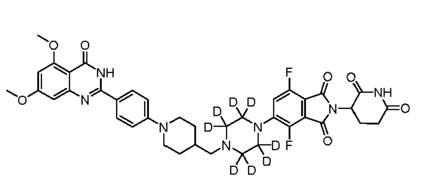 | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-*d*8)-2-(2,6-dioxopiperidin-3-yl)-4,7-difluoroisoindoline-1,3-dione | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-*d*8)-2-(2,6-dioxopiperidin-3-yl)-4,7-difluoroisoindoline-1,3-dione |
| | 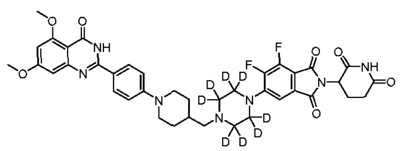 | 6-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-*d*8)-2-(2,6-dioxopiperidin-3-yl)-4,5-difluoroisoindoline-1,3-dione | 6-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-*d*8)-2-(2,6-dioxopiperidin-3-yl)-4,5-difluoroisoindoline-1,3-dione |
| | 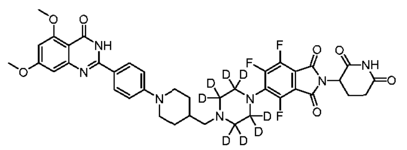 | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-*d*8)-2-(2,6-dioxopiperidin-3-yl)-4,6,7-trifluoroisoindoline-1,3-dione | 5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-*d*8)-2-(2,6-dioxopiperidin-3-yl)-4,6,7-trifluoroisoindoline-1,3-dione |
| | 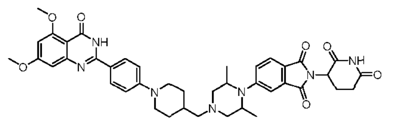 | 5-(4-((1-(4-(5,7-dimethoxy-4-oxo-3,4-dihydroquinazolin-2-yl)phenyl)piperidin-4-yl)methyl)-2,6-dimethylpiperazin-1-yl)-2-(2,6-dioxopiperidin-3-yl)isoindoline-1,3-dione | 5-(4-((1-(4-(5,7-dimethoxy-4-oxo-3,4-dihydroquinazolin-2-yl)phenyl)piperidin-4-yl)methyl)-2,6-dimethylpiperazin-1-yl)-2-(2,6-dioxopiperidin-3-yl)isoindoline-1,3-dione |
| | 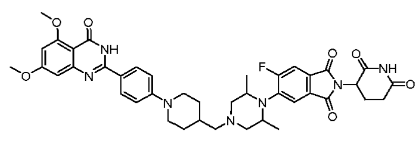 | 5-(4-((1-(4-(5,7-dimethoxy-4-oxo-3,4-dihydroquinazolin-2-yl)phenyl)piperidin-4-yl)methyl)-2,6-dimethylpiperazin-1-yl)-2-(2,6-dioxopiperidin-3-yl)-6-fluoroisoindoline-1,3-dione | 5-(4-((1-(4-(5,7-dimethoxy-4-oxo-3,4-dihydroquinazolin-2-yl)phenyl)piperidin-4-yl)methyl)-2,6-dimethylpiperazin-1-yl)-2-(2,6-dioxopiperidin-3-yl)-6-fluoroisoindoline-1,3-dione |
| | 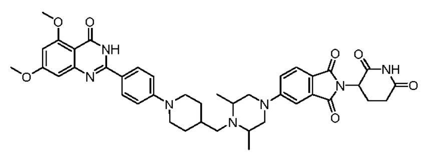 | 5-(4-((1-(4-(5,7-dimethoxy-4-oxo-3,4-dihydroquinazolin-2-yl)phenyl)piperidin-4-yl)methyl)-3,5-dimethylpiperazin-1-yl)-2-(2,6-dioxopiperidin-3-yl)isoindoline-1,3-dione | 5-(4-((1-(4-(5,7-dimethoxy-4-oxo-3,4-dihydroquinazolin-2-yl)phenyl)piperidin-4-yl)methyl)-3,5-dimethylpiperazin-1-yl)-2-(2,6-dioxopiperidin-3-yl)isoindoline-1,3-dione |
| | 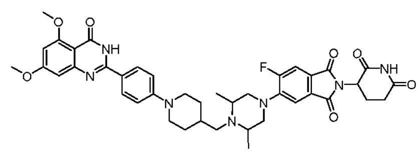 | 5-(4-((1-(4-(5,7-dimethoxy-4-oxo-3,4-dihydroquinazolin-2-yl)phenyl)piperidin-4-yl)methyl)-3,5-dimethylpiperazin-1-yl)-2- | 5-(4-((1-(4-(5,7-dimethoxy-4-oxo-3,4-dihydroquinazolin-2-yl)phenyl)piperidin-4-yl)methyl)-3,5-dimethylpiperazin-1-yl)-2- |

| | | | |
|---|---|---|---|
| | | (2,6-dioxopiperidin-3-yl)-6-fluoroisoindoline-1,3-dione | (2,6-dioxopiperidin-3-yl)-6-fluoroisoindoline-1,3-dione |
| | 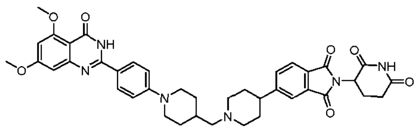 | 5-(1-((1-(4-(5,7-dimethoxy-4-oxo-3,4-dihydroquinazolin-2-yl)phenyl)piperidin-4-yl)methyl)piperidin-4-yl)-2-(2,6-dioxopiperidin-3-yl)isoindoline-1,3-dione | 5-(1-((1-(4-(5,7-dimethoxy-4-oxo-3,4-dihydroquinazolin-2-yl)phenyl)piperidin-4-yl)methyl)piperidin-4-yl)-2-(2,6-dioxopiperidin-3-yl)isoindoline-1,3-dione |
| | 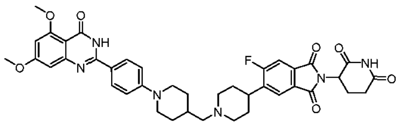 | 5-(1-((1-(4-(5,7-dimethoxy-4-oxo-3,4-dihydroquinazolin-2-yl)phenyl)piperidin-4-yl)methyl)piperidin-4-yl)-2-(2,6-dioxopiperidin-3-yl)-6-fluoroisoindoline-1,3-dione | 5-(1-((1-(4-(5,7-dimethoxy-4-oxo-3,4-dihydroquinazolin-2-yl)phenyl)piperidin-4-yl)methyl)piperidin-4-yl)-2-(2,6-dioxopiperidin-3-yl)-6-fluoroisoindoline-1,3-dione |
| | 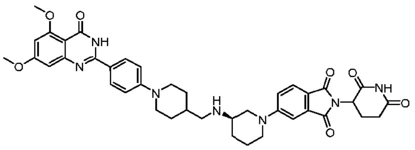 | 5-((R)-3-(((1-(4-(5,7-dimethoxy-4-oxo-3,4-dihydroquinazolin-2-yl)phenyl)piperidin-4-yl)methyl)amino)piperidin-1-yl)-2-(2,6-dioxopiperidin-3-yl)isoindoline-1,3-dione | 5-((R)-3-(((1-(4-(5,7-dimethoxy-4-oxo-3,4-dihydroquinazolin-2-yl)phenyl)piperidin-4-yl)methyl)amino)piperidin-1-yl)-2-(2,6-dioxopiperidin-3-yl)isoindoline-1,3-dione |
| | 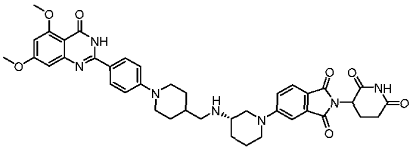 | 5-((S)-3-(((1-(4-(5,7-dimethoxy-4-oxo-3,4-dihydroquinazolin-2-yl)phenyl)piperidin-4-yl)methyl)amino)piperidin-1-yl)-2-(2,6-dioxopiperidin-3-yl)isoindoline-1,3-dione | 5-((S)-3-(((1-(4-(5,7-dimethoxy-4-oxo-3,4-dihydroquinazolin-2-yl)phenyl)piperidin-4-yl)methyl)amino)piperidin-1-yl)-2-(2,6-dioxopiperidin-3-yl)isoindoline-1,3-dione |
| | 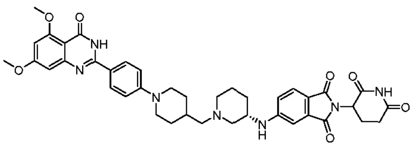 | 5-(((S)-1-((1-(4-(5,7-dimethoxy-4-oxo-3,4-dihydroquinazolin-2-yl)phenyl)piperidin-4-yl)methyl)piperidin-3-yl)amino)-2-(2,6-dioxopiperidin-3-yl)isoindoline-1,3-dione | 5-(((S)-1-((1-(4-(5,7-dimethoxy-4-oxo-3,4-dihydroquinazolin-2-yl)phenyl)piperidin-4-yl)methyl)piperidin-3-yl)amino)-2-(2,6-dioxopiperidin-3-yl)isoindoline-1,3-dione |
| | 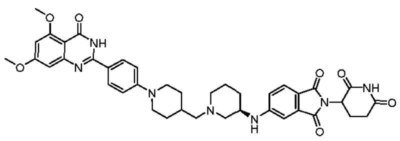 | 5-(((R)-1-((1-(4-(5,7-dimethoxy-4-oxo-3,4-dihydroquinazolin-2-yl)phenyl)piperidin-4-yl)methyl)piperidin-3-yl)amino)-2-(2,6-dioxopiperidin-3-yl)isoindoline-1,3-dione | 5-(((R)-1-((1-(4-(5,7-dimethoxy-4-oxo-3,4-dihydroquinazolin-2-yl)phenyl)piperidin-4-yl)methyl)piperidin-3-yl)amino)-2-(2,6-dioxopiperidin-3-yl)isoindoline-1,3-dione |
| | 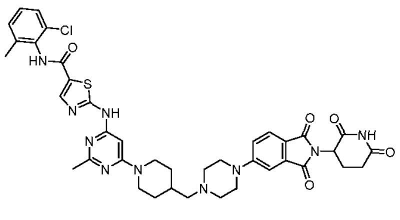 | N-(2-chloro-6-methylphenyl)-2-((6-(4-((4-(2-(2,6-dioxopiperidin-3-yl)-1,3-dioxoisoindolin-5-yl)piperazin-1-yl)methyl)piperidin-1-yl)-2-methylpyrimidin-4-yl)amino)thiazole-5- | N-(2-chloro-6-methylphenyl)-2-((6-(4-((4-(2-(2,6-dioxopiperidin-3-yl)-1,3-dioxoisoindolin-5-yl)piperazin-1-yl)methyl)piperidin-1-yl)-2-methylpyrimidin-4-yl)amino)thiazole-5- |

| | | | |
|---|---|---|---|
| | | carboxamide | carboxamide |
| | 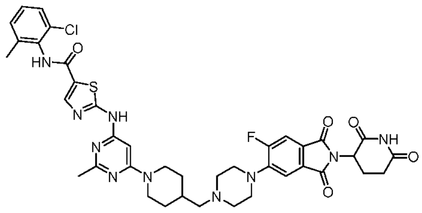 | N-(2-chloro-6-methylphenyl)-2-((6-(4-((4-(2-(2,6-dioxopiperidin-3-yl)-6-fluoro-1,3-dioxoisoindolin-5-yl)piperazin-1-yl)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-6-fluoro-1,3-dioxoisoindolin-5-yl)piperazin-1-yl)methyl)piperidin-1-yl)-2-methylpyrimidin-4-yl)amino)thiazole-5-carboxamide |
| GT-03630 | 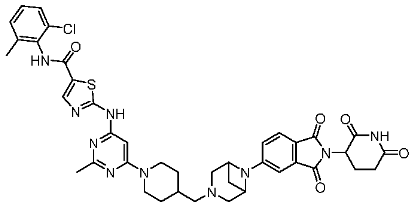 | N-(2-chloro-6-methylphenyl)-2-((6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-2-methylpyrimidin-4-yl)amino)thiazole-5-carboxamide | N-(2-chloro-6-methylphenyl)-2-((6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-2-methylpyrimidin-4-yl)amino)thiazole-5-carboxamide |
| GT-03628 | 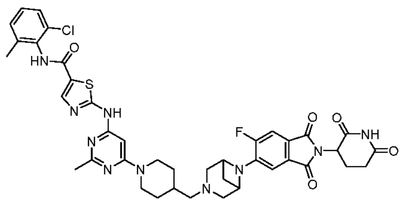 | N-(2-chloro-6-methylphenyl)-2-((6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-2-methylpyrimidin-4-yl)amino)thiazole-5-carboxamide | N-(2-chloro-6-methylphenyl)-2-((6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-2-methylpyrimidin-4-yl)amino)thiazole-5-carboxamide |
| GT-03631 | 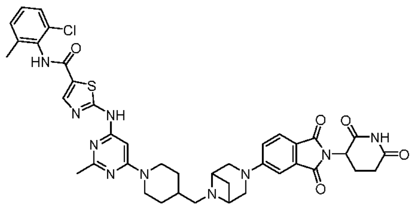 | N-(2-chloro-6-methylphenyl)-2-((6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-2-methylpyrimidin-4-yl)amino)thiazole-5-carboxamide | N-(2-chloro-6-methylphenyl)-2-((6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-2-methylpyrimidin-4-yl)amino)thiazole-5-carboxamide |
| GT-03538 | 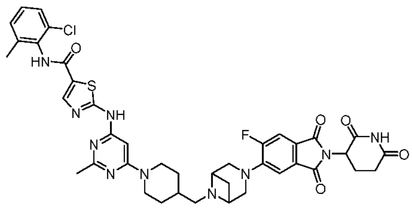 | N-(2-chloro-6-methylphenyl)-2-((6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-2-methylpyrimidin-4-yl)amino)thiazole-5-carboxamide | N-(2-chloro-6-methylphenyl)-2-((6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-2-methylpyrimidin-4-yl)amino)thiazole-5-carboxamide |
| GT-03629 | 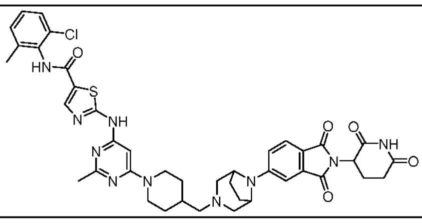 | N-(2-chloro-6-methylphenyl)-2-((6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-2- | N-(2-chloro-6-methylphenyl)-2-((6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)- |

| | | | |
|---|---|---|---|
| | | methylpyrimidin-4-yl)amino)thiazole-5-carboxamide | 2-methylpyrimidin-4-yl)amino)thiazole-5-carboxamide |
| GT-03627 | 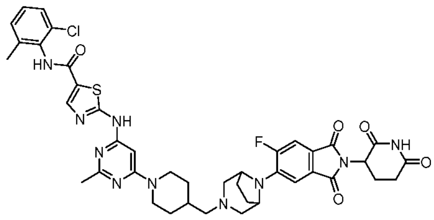 | N-(2-chloro-6-methylphenyl)-2-((6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-2-methylpyrimidin-4-yl)amino)thiazole-5-carboxamide | N-(2-chloro-6-methylphenyl)-2-((6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-2-methylpyrimidin-4-yl)amino)thiazole-5-carboxamide |
| GT-03632 | 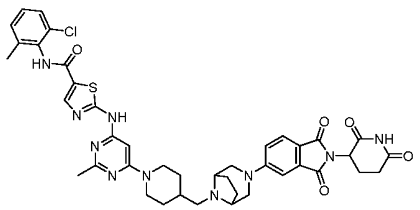 | N-(2-chloro-6-methylphenyl)-2-((6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-2-methylpyrimidin-4-yl)amino)thiazole-5-carboxamide | N-(2-chloro-6-methylphenyl)-2-((6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-2-methylpyrimidin-4-yl)amino)thiazole-5-carboxamide |
| GT-03537 | 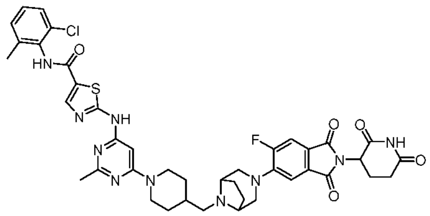 | N-(2-chloro-6-methylphenyl)-2-((6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-2-methylpyrimidin-4-yl)amino)thiazole-5-carboxamide | N-(2-chloro-6-methylphenyl)-2-((6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-2-methylpyrimidin-4-yl)amino)thiazole-5-carboxamide |
| | 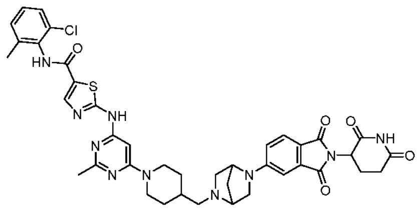 | N-(2-chloro-6-methylphenyl)-2-((6-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-2-methylpyrimidin-4-yl)amino)thiazole-5-carboxamide | N-(2-chloro-6-methylphenyl)-2-((6-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-2-methylpyrimidin-4-yl)amino)thiazole-5-carboxamide |
| | 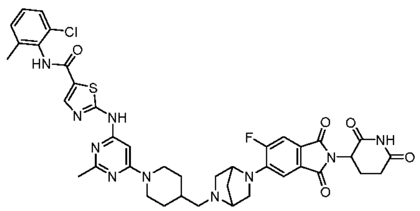 | N-(2-chloro-6-methylphenyl)-2-((6-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-2-methylpyrimidin-4-yl)amino)thiazole-5-carboxamide | N-(2-chloro-6-methylphenyl)-2-((6-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-2-methylpyrimidin-4-yl)amino)thiazole-5-carboxamide |

| | | | |
|---|---|---|---|
| | 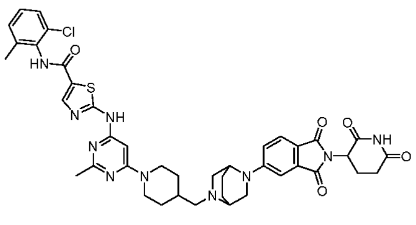 | N-(2-chloro-6-methylphenyl)-2-((6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-2-methylpyrimidin-4-yl)amino)thiazole-5-carboxamide | N-(2-chloro-6-methylphenyl)-2-((6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-2-methylpyrimidin-4-yl)amino)thiazole-5-carboxamide |
| | 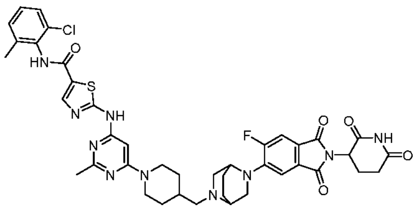 | N-(2-chloro-6-methylphenyl)-2-((6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-2-methylpyrimidin-4-yl)amino)thiazole-5-carboxamide | N-(2-chloro-6-methylphenyl)-2-((6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-2-methylpyrimidin-4-yl)amino)thiazole-5-carboxamide |
| | 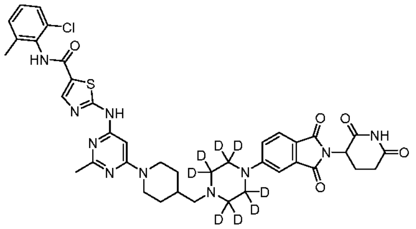 | N-(2-chloro-6-methylphenyl)-2-((6-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide |
| | 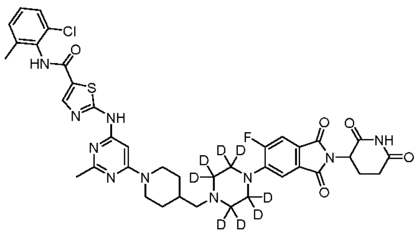 | N-(2-chloro-6-methylphenyl)-2-((6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide |
| | 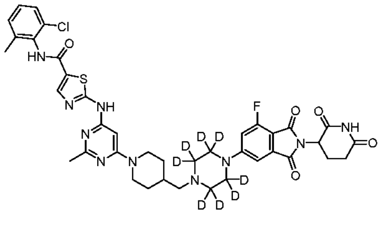 | N-(2-chloro-6-methylphenyl)-2-((6-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide |

| | | | |
|---|---|---|---|
| | 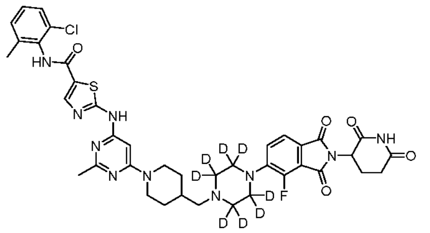 | N-(2-chloro-6-methylphenyl)-2-((6-(4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide |
| | 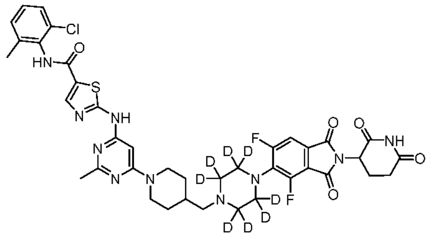 | N-(2-chloro-6-methylphenyl)-2-((6-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-4,6-difluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide |
| | 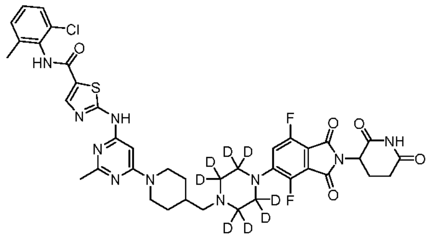 | N-(2-chloro-6-methylphenyl)-2-((6-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-4,7-difluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide |
| | 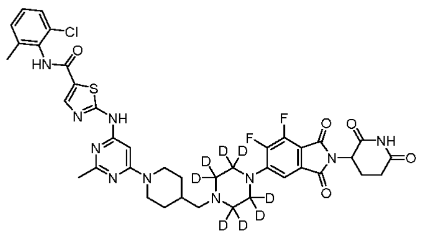 | N-(2-chloro-6-methylphenyl)-2-((6-(4-((4-(2-(2,6-dioxopiperidin-3-yl)-6,7-difluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-6,7-difluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide |
| | 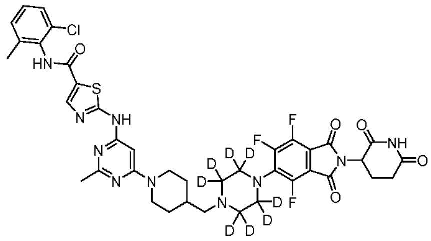 | N-(2-chloro-6-methylphenyl)-2-((6-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*$_8$)methyl)piperidin-1-yl)-2-methylpyrimidin-4-yl)amino)thiazole-5-carboxamide |

| | | | |
|---|---|---|---|
| | 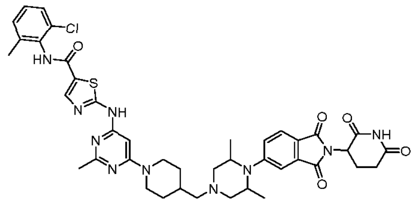 | N-(2-chloro-6-methylphenyl)-2-((6-(4-((4-(2-(2,6-dioxopiperidin-3-yl)-1,3-dioxoisoindolin-5-yl)-3,5-dimethylpiperazin-1-yl)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-1,3-dioxoisoindolin-5-yl)-3,5-dimethylpiperazin-1-yl)methyl)piperidin-1-yl)-2-methylpyrimidin-4-yl)amino)thiazole-5-carboxamide |
| | 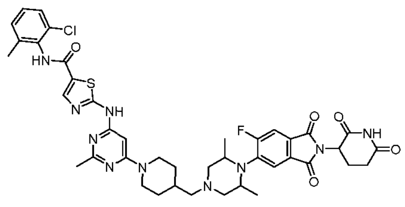 | N-(2-chloro-6-methylphenyl)-2-((6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-2-methylpyrimidin-4-yl)amino)thiazole-5-carboxamide |
| | 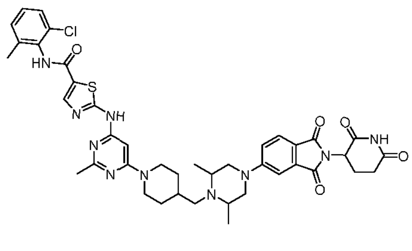 | N-(2-chloro-6-methylphenyl)-2-((6-(4-((4-(2-(2,6-dioxopiperidin-3-yl)-1,3-dioxoisoindolin-5-yl)-2,6-dimethylpiperazin-1-yl)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-1,3-dioxoisoindolin-5-yl)-2,6-dimethylpiperazin-1-yl)methyl)piperidin-1-yl)-2-methylpyrimidin-4-yl)amino)thiazole-5-carboxamide |
| | 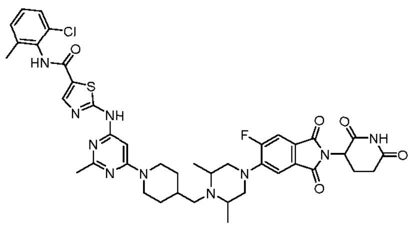 | N-(2-chloro-6-methylphenyl)-2-((6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-2-methylpyrimidin-4-yl)amino)thiazole-5-carboxamide |
| | 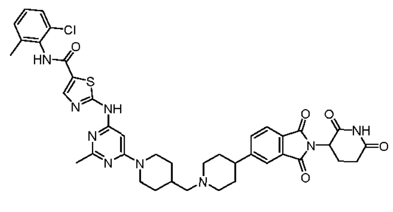 | N-(2-chloro-6-methylphenyl)-2-((6-(4-((4-(2-(2,6-dioxopiperidin-3-yl)-1,3-dioxoisoindolin-5-yl)piperidin-1-yl)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-1,3-dioxoisoindolin-5-yl)piperidin-1-yl)methyl)piperidin-1-yl)-2-methylpyrimidin-4-yl)amino)thiazole-5-carboxamide |
| | 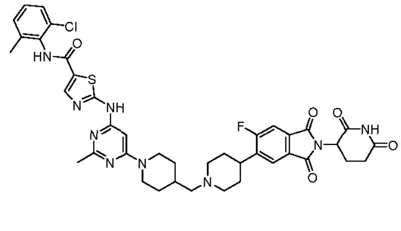 | N-(2-chloro-6-methylphenyl)-2-((6-(4-((4-(2-(2,6-dioxopiperidin-3-yl)-6-fluoro-1,3-dioxoisoindolin-5-yl)*piperidin*-1-yl)methyl)piperidin-1-yl)-2-methylpyrimidin-4-yl)amino)thiazole-5- | N-(2-chloro-6-methylphenyl)-2-((6-(4-((4-(2-(2,6-dioxopiperidin-3-yl)-6-fluoro-1,3-dioxoisoindolin-5-yl)*piperidin*-1-yl)methyl)piperidin-1-yl)-2-methylpyrimidin-4-yl)amino)thiazole-5- |

| | | | |
|---|---|---|---|
| | | carboxamide | carboxamide |
| | 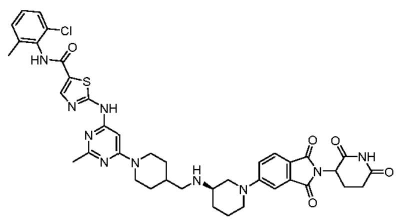 | N-(2-chloro-6-methylphenyl)-2-((6-(4-((((3R)-1-(2-(2,6-dioxopiperidin-3-yl)-1,3-dioxoisoindolin-5-yl)piperidin-3-yl)amino)methyl)piperidin-1-yl)-2-methylpyrimidin-4-yl)amino)thiazole-5-carboxamide | N-(2-chloro-6-methylphenyl)-2-((6-(4-((((3R)-1-(2-(2,6-dioxopiperidin-3-yl)-1,3-dioxoisoindolin-5-yl)piperidin-3-yl)amino)methyl)piperidin-1-yl)-2-methylpyrimidin-4-yl)amino)thiazole-5-carboxamide |
| | 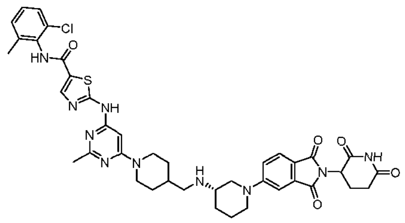 | N-(2-chloro-6-methylphenyl)-2-((6-(4-((((3S)-1-(2-(2,6-dioxopiperidin-3-yl)-1,3-dioxoisoindolin-5-yl)piperidin-3-yl)amino)methyl)piperidin-1-yl)-2-methylpyrimidin-4-yl)amino)thiazole-5-carboxamide | N-(2-chloro-6-methylphenyl)-2-((6-(4-((((3S)-1-(2-(2,6-dioxopiperidin-3-yl)-1,3-dioxoisoindolin-5-yl)piperidin-3-yl)amino)methyl)piperidin-1-yl)-2-methylpyrimidin-4-yl)amino)thiazole-5-carboxamide |
| | 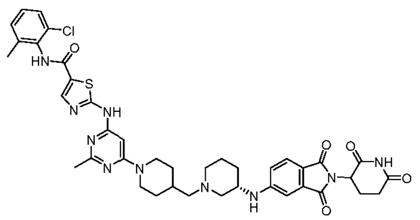 | N-(2-chloro-6-methylphenyl)-2-((6-(4-(((3S)-3-((2-(2,6-dioxopiperidin-3-yl)-1,3-dioxoisoindolin-5-yl)amino)piperidin-1-yl)methyl)piperidin-1-yl)-2-methylpyrimidin-4-yl)amino)thiazole-5-carboxamide | N-(2-chloro-6-methylphenyl)-2-((6-(4-(((3S)-3-((2-(2,6-dioxopiperidin-3-yl)-1,3-dioxoisoindolin-5-yl)amino)piperidin-1-yl)methyl)piperidin-1-yl)-2-methylpyrimidin-4-yl)amino)thiazole-5-carboxamide |
| | 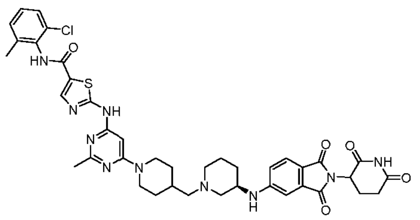 | N-(2-chloro-6-methylphenyl)-2-((6-(4-(((3R)-3-((2-(2,6-dioxopiperidin-3-yl)-1,3-dioxoisoindolin-5-yl)amino)piperidin-1-yl)methyl)piperidin-1-yl)-2-methylpyrimidin-4-yl)amino)thiazole-5-carboxamide | N-(2-chloro-6-methylphenyl)-2-((6-(4-(((3R)-3-((2-(2,6-dioxopiperidin-3-yl)-1,3-dioxoisoindolin-5-yl)amino)piperidin-1-yl)methyl)piperidin-1-yl)-2-methylpyrimidin-4-yl)amino)thiazole-5-carboxamide |
| | 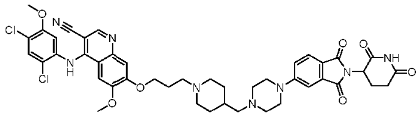 | 4-((2,4-dichloro-5-methoxyphenyl)amino)-7-(3-(4-((4-(2-(2,6-dioxopiperidin-3-yl)-1,3-dioxoisoindolin-5-yl)piperazin-1-yl)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-1,3-dioxoisoindolin-5-yl)piperazin-1-yl)methyl)piperidin-1-yl)propoxy)-6-methoxyquinoline-3-carbonitrile |
| | 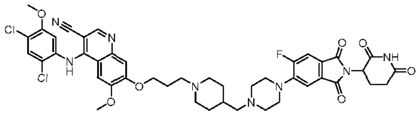 | 4-((2,4-dichloro-5-methoxyphenyl)amino)-7-(3-(4-((4-(2-(2,6-dioxopiperidin-3-yl)-6-fluoro-1,3-dioxoisoindolin-5-yl)piperazin-1-yl)methyl)piperidin-1- | 4-((2,4-dichloro-5-methoxyphenyl)amino)-7-(3-(4-((4-(2-(2,6-dioxopiperidin-3-yl)-6-fluoro-1,3-dioxoisoindolin-5-yl)piperazin-1-yl)methyl)piperidin-1- |

| | | | |
|---|---|---|---|
| | | yl)propoxy)-6-methoxyquinoline-3-carbonitrile | yl)propoxy)-6-methoxyquinoline-3-carbonitrile |
| | 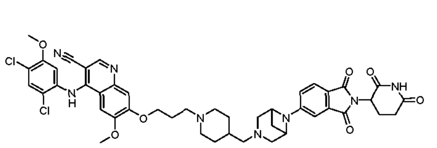 | 4-((2,4-dichloro-5-methoxyphenyl)amino)-7-(3-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)propoxy)-6-methoxyquinoline-3-carbonitrile | 4-((2,4-dichloro-5-methoxyphenyl)amino)-7-(3-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)propoxy)-6-methoxyquinoline-3-carbonitrile |
| | 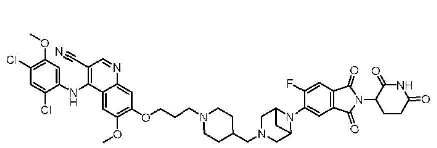 | 4-((2,4-dichloro-5-methoxyphenyl)amino)-7-(3-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)propoxy)-6-methoxyquinoline-3-carbonitrile | 4-((2,4-dichloro-5-methoxyphenyl)amino)-7-(3-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)propoxy)-6-methoxyquinoline-3-carbonitrile |
| | 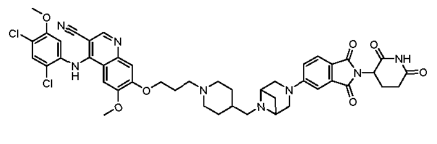 | 4-((2,4-dichloro-5-methoxyphenyl)amino)-7-(3-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)propoxy)-6-methoxyquinoline-3-carbonitrile | 4-((2,4-dichloro-5-methoxyphenyl)amino)-7-(3-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)propoxy)-6-methoxyquinoline-3-carbonitrile |
| | 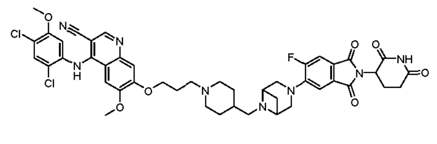 | 4-((2,4-dichloro-5-methoxyphenyl)amino)-7-(3-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)propoxy)-6-methoxyquinoline-3-carbonitrile | 4-((2,4-dichloro-5-methoxyphenyl)amino)-7-(3-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)propoxy)-6-methoxyquinoline-3-carbonitrile |
| | 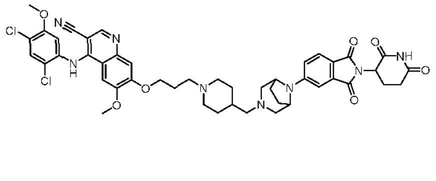 | 4-((2,4-dichloro-5-methoxyphenyl)amino)-7-(3-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)propoxy)-6-methoxyquinoline-3-carbonitrile | 4-((2,4-dichloro-5-methoxyphenyl)amino)-7-(3-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)propoxy)-6-methoxyquinoline-3-carbonitrile |

| | | | |
|---|---|---|---|
| | 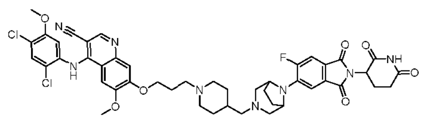 | 4-((2,4-dichloro-5-methoxyphenyl)amino)-7-(3-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)propoxy)-6-methoxyquinoline-3-carbonitrile | 4-((2,4-dichloro-5-methoxyphenyl)amino)-7-(3-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)propoxy)-6-methoxyquinoline-3-carbonitrile |
| | 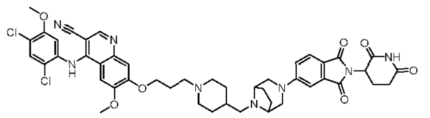 | 4-((2,4-dichloro-5-methoxyphenyl)amino)-7-(3-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)propoxy)-6-methoxyquinoline-3-carbonitrile | 4-((2,4-dichloro-5-methoxyphenyl)amino)-7-(3-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)propoxy)-6-methoxyquinoline-3-carbonitrile |
| | 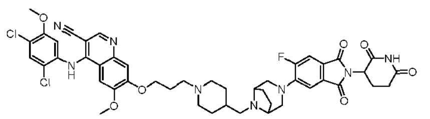 | 4-((2,4-dichloro-5-methoxyphenyl)amino)-7-(3-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)propoxy)-6-methoxyquinoline-3-carbonitrile | 4-((2,4-dichloro-5-methoxyphenyl)amino)-7-(3-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)propoxy)-6-methoxyquinoline-3-carbonitrile |
| | 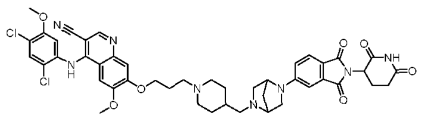 | 4-((2,4-dichloro-5-methoxyphenyl)amino)-7-(3-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)propoxy)-6-methoxyquinoline-3-carbonitrile | 4-((2,4-dichloro-5-methoxyphenyl)amino)-7-(3-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)propoxy)-6-methoxyquinoline-3-carbonitrile |
| | 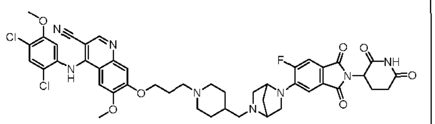 | 4-((2,4-dichloro-5-methoxyphenyl)amino)-7-(3-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)propoxy)-6-methoxyquinoline-3-carbonitrile | 4-((2,4-dichloro-5-methoxyphenyl)amino)-7-(3-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)propoxy)-6-methoxyquinoline-3-carbonitrile |
| | 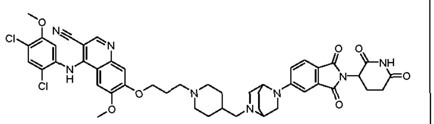 | 4-((2,4-dichloro-5-methoxyphenyl)amino)-7-(3-(4-((5-(2-(2,6-dioxopiperidin-3-yl)-1,3-dioxoisoindolin-5-yl)-2,5- | 4-((2,4-dichloro-5-methoxyphenyl)amino)-7-(3-(4-((5-(2-(2,6-dioxopiperidin-3-yl)-1,3-dioxoisoindolin-5-yl)-2,5- |

| | | | |
|---|---|---|---|
| | | diazabicyclo[2.2.2]octan-2-yl)methyl)piperidin-1-yl)propoxy)-6-methoxyquinoline-3-carbonitrile | diazabicyclo[2.2.2]octan-2-yl)methyl)piperidin-1-yl)propoxy)-6-methoxyquinoline-3-carbonitrile |
| | 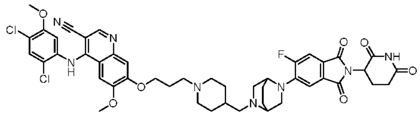 | 4-((2,4-dichloro-5-methoxyphenyl)amino)-7-(3-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)propoxy)-6-methoxyquinoline-3-carbonitrile | 4-((2,4-dichloro-5-methoxyphenyl)amino)-7-(3-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)propoxy)-6-methoxyquinoline-3-carbonitrile |
| | 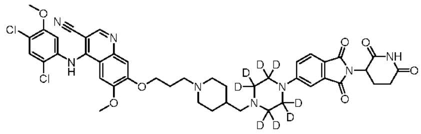 | 4-((2,4-dichloro-5-methoxyphenyl)amino)-7-(3-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile |
| | 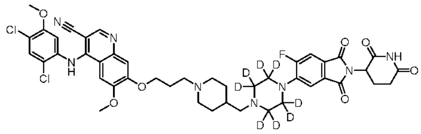 | 4-((2,4-dichloro-5-methoxyphenyl)amino)-7-(3-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile |
| | 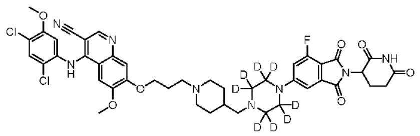 | 4-((2,4-dichloro-5-methoxyphenyl)amino)-7-(3-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile |
| | 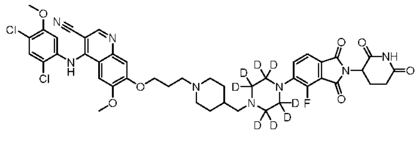 | 4-((2,4-dichloro-5-methoxyphenyl)amino)-7-(3-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)propoxy)-6- | 4-((2,4-dichloro-5-methoxyphenyl)amino)-7-(3-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)propoxy)-6- |

| | | | |
|---|---|---|---|
| | | methoxyquinoline-3-carbonitrile | methoxyquinoline-3-carbonitrile |
| | 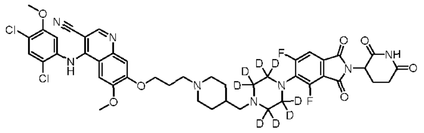 | 4-((2,4-dichloro-5-methoxyphenyl)amino)-7-(3-(4-((4-(2-(2,6-dioxopiperidin-3-yl)-4,6-difluoro-1,3-dioxoisoindolin-5-yl)piperazin-1-yl-2,2,3,3,5,5,6,6-*d*8)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-4,6-difluoro-1,3-dioxoisoindolin-5-yl)piperazin-1-yl-2,2,3,3,5,5,6,6-*d*8)methyl)piperidin-1-yl)propoxy)-6-methoxyquinoline-3-carbonitrile |
| | 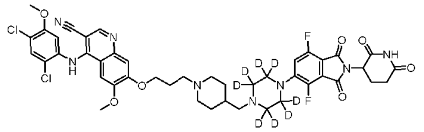 | 4-((2,4-dichloro-5-methoxyphenyl)amino)-7-(3-(4-((4-(2-(2,6-dioxopiperidin-3-yl)-4,7-difluoro-1,3-dioxoisoindolin-5-yl)piperazin-1-yl-2,2,3,3,5,5,6,6-*d*8)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-4,7-difluoro-1,3-dioxoisoindolin-5-yl)piperazin-1-yl-2,2,3,3,5,5,6,6-*d*8)methyl)piperidin-1-yl)propoxy)-6-methoxyquinoline-3-carbonitrile |
| | 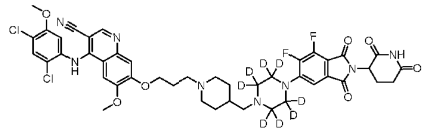 | 4-((2,4-dichloro-5-methoxyphenyl)amino)-7-(3-(4-((4-(2-(2,6-dioxopiperidin-3-yl)-6,7-difluoro-1,3-dioxoisoindolin-5-yl)piperazin-1-yl-2,2,3,3,5,5,6,6-*d*8)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-6,7-difluoro-1,3-dioxoisoindolin-5-yl)piperazin-1-yl-2,2,3,3,5,5,6,6-*d*8)methyl)piperidin-1-yl)propoxy)-6-methoxyquinoline-3-carbonitrile |
| | 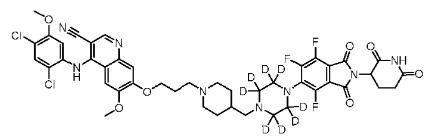 | 4-((2,4-dichloro-5-methoxyphenyl)amino)-7-(3-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*8)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-4,6,7-trifluoro-1,3-dioxoisoindolin-5-yl)piperazin-1-yl-2,2,3,3,5,5,6,6-*d*8)methyl)piperidin-1-yl)propoxy)-6-methoxyquinoline-3-carbonitrile |
| | 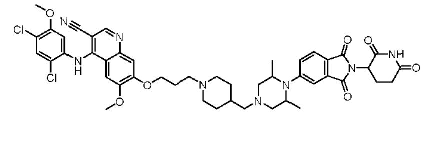 | 4-((2,4-dichloro-5-methoxyphenyl)amino)-7-(3-(4-((4-(2-(2,6-dioxopiperidin-3-yl)-1,3-dioxoisoindolin-5-yl)-3,5-dimethylpiperazin-1-yl)methyl)piperidin-1-yl)propoxy)-6- | 4-((2,4-dichloro-5-methoxyphenyl)amino)-7-(3-(4-((4-(2-(2,6-dioxopiperidin-3-yl)-1,3-dioxoisoindolin-5-yl)-3,5-dimethylpiperazin-1-yl)methyl)piperidin-1-yl)propoxy)-6- |

| | | | |
|---|---|---|---|
| | | methoxyquinoline-3-carbonitrile | methoxyquinoline-3-carbonitrile |
| | 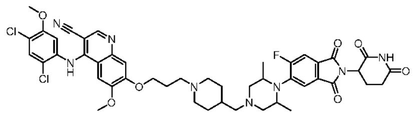 | 4-((2,4-dichloro-5-methoxyphenyl)amino)-7-(3-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)propoxy)-6-methoxyquinoline-3-carbonitrile |
| | 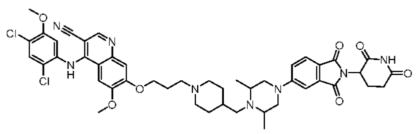 | 4-((2,4-dichloro-5-methoxyphenyl)amino)-7-(3-(4-((4-(2-(2,6-dioxopiperidin-3-yl)-1,3-dioxoisoindolin-5-yl)-2,6-dimethylpiperazin-1-yl)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-1,3-dioxoisoindolin-5-yl)-2,6-dimethylpiperazin-1-yl)methyl)piperidin-1-yl)propoxy)-6-methoxyquinoline-3-carbonitrile |
| | 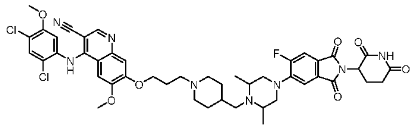 | 4-((2,4-dichloro-5-methoxyphenyl)amino)-7-(3-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)propoxy)-6-methoxyquinoline-3-carbonitrile |
| | 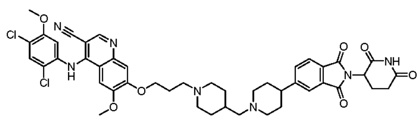 | 4-((2,4-dichloro-5-methoxyphenyl)amino)-7-(3-(4-((4-(2-(2,6-dioxopiperidin-3-yl)-1,3-dioxoisoindolin-5-yl)piperidin-1-yl)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-1,3-dioxoisoindolin-5-yl)piperidin-1-yl)methyl)piperidin-1-yl)propoxy)-6-methoxyquinoline-3-carbonitrile |
| | 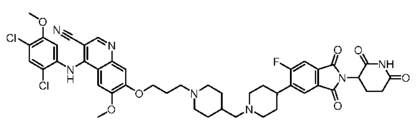 | 4-((2,4-dichloro-5-methoxyphenyl)amino)-7-(3-(4-((4-(2-(2,6-dioxopiperidin-3-yl)-6-fluoro-1,3-dioxoisoindolin-5-yl)piperidin-1-yl)methyl)piperidin-1-yl)propoxy)-6-methoxyquinoline-3-carbonitrile | 4-((2,4-dichloro-5-methoxyphenyl)amino)-7-(3-(4-((4-(2-(2,6-dioxopiperidin-3-yl)-6-fluoro-1,3-dioxoisoindolin-5-yl)piperidin-1-yl)methyl)piperidin-1-yl)propoxy)-6-methoxyquinoline-3-carbonitrile |
| | 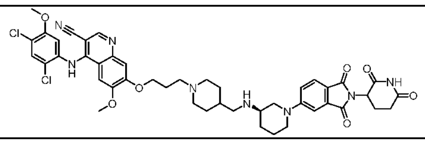 | 4-((2,4-dichloro-5-methoxyphenyl)amino)-7-(3-(4-((((3R)-1-(2-(2,6-dioxopiperidin-3-yl)-1,3- | 4-((2,4-dichloro-5-methoxyphenyl)amino)-7-(3-(4-((((3R)-1-(2-(2,6-dioxopiperidin-3-yl)-1,3- |

| | | | |
|---|---|---|---|
| | | dioxoisoindolin-5-yl)piperidin-3-yl)amino)methyl)piperidin-1-yl)propoxy)-6-methoxyquinoline-3-carbonitrile | dioxoisoindolin-5-yl)piperidin-3-yl)amino)methyl)piperidin-1-yl)propoxy)-6-methoxyquinoline-3-carbonitrile |
| | 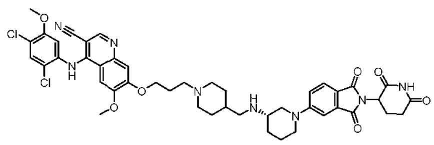 | 4-((2,4-dichloro-5-methoxyphenyl)amino)-7-(3-(4-((((3S)-1-(2-(2,6-dioxopiperidin-3-yl)-1,3-dioxoisoindolin-5-yl)piperidin-3-yl)amino)methyl)piperidin-1-yl)propoxy)-6-methoxyquinoline-3-carbonitrile | 4-((2,4-dichloro-5-methoxyphenyl)amino)-7-(3-(4-((((3S)-1-(2-(2,6-dioxopiperidin-3-yl)-1,3-dioxoisoindolin-5-yl)piperidin-3-yl)amino)methyl)piperidin-1-yl)propoxy)-6-methoxyquinoline-3-carbonitrile |
| | 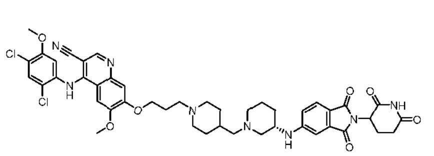 | 4-((2,4-dichloro-5-methoxyphenyl)amino)-7-(3-(4-(((3S)-3-((2-(2,6-dioxopiperidin-3-yl)-1,3-dioxoisoindolin-5-yl)amino)piperidin-1-yl)methyl)piperidin-1-yl)propoxy)-6-methoxyquinoline-3-carbonitrile | 4-((2,4-dichloro-5-methoxyphenyl)amino)-7-(3-(4-(((3S)-3-((2-(2,6-dioxopiperidin-3-yl)-1,3-dioxoisoindolin-5-yl)amino)piperidin-1-yl)methyl)piperidin-1-yl)propoxy)-6-methoxyquinoline-3-carbonitrile |
| | 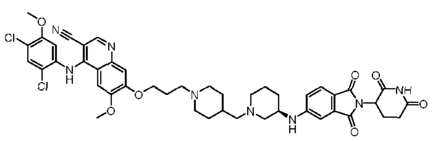 | 4-((2,4-dichloro-5-methoxyphenyl)amino)-7-(3-(4-(((3R)-3-((2-(2,6-dioxopiperidin-3-yl)-1,3-dioxoisoindolin-5-yl)amino)piperidin-1-yl)methyl)piperidin-1-yl)propoxy)-6-methoxyquinoline-3-carbonitrile | 4-((2,4-dichloro-5-methoxyphenyl)amino)-7-(3-(4-(((3R)-3-((2-(2,6-dioxopiperidin-3-yl)-1,3-dioxoisoindolin-5-yl)amino)piperidin-1-yl)methyl)piperidin-1-yl)propoxy)-6-methoxyquinoline-3-carbonitrile |
| | 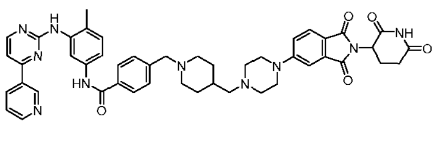 | 4-((4-((4-(2-(2,6-dioxopiperidin-3-yl)-1,3-dioxoisoindolin-5-yl)piperazin-1-yl)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-1,3-dioxoisoindolin-5-yl)piperazin-1-yl)methyl)piperidin-1-yl)methyl)-N-(4-methyl-3-((4-(pyridin-3-yl)pyrimidin-2-yl)amino)phenyl)benzamide |
| | 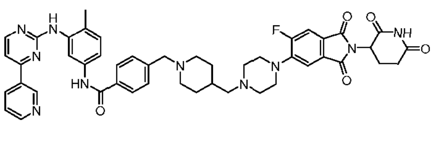 | 4-((4-((4-(2-(2,6-dioxopiperidin-3-yl)-6-fluoro-1,3-dioxoisoindolin-5-yl)piperazin-1-yl)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-6-fluoro-1,3-dioxoisoindolin-5-yl)piperazin-1-yl)methyl)piperidin-1-yl)methyl)-N-(4-methyl-3-((4-(pyridin-3-yl)pyrimidin-2-yl)amino)phenyl)benzamide |

| | | | |
|---|---|---|---|
| | 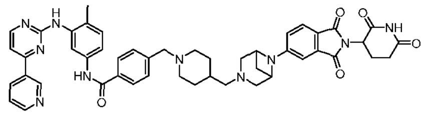 | 4-((4-((6-(2-(2,6-dioxopiperidin-3-yl)-1,3-dioxoisoindolin-5-yl)-3,6-diazabicyclo[3.1.1]heptan-3-yl)methyl)piperidin-1-yl)methyl)-N-(4-methyl-3-((4-(pyridin-3-yl)pyrimidin-2-yl)amino)phenyl)benzamide | 4-((4-((6-(2-(2,6-dioxopiperidin-3-yl)-1,3-dioxoisoindolin-5-yl)-3,6-diazabicyclo[3.1.1]heptan-3-yl)methyl)piperidin-1-yl)methyl)-N-(4-methyl-3-((4-(pyridin-3-yl)pyrimidin-2-yl)amino)phenyl)benzamide |
| | 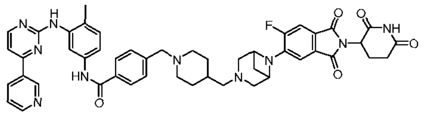 | 4-((4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)methyl)-N-(4-methyl-3-((4-(pyridin-3-yl)pyrimidin-2-yl)amino)phenyl)benzamide | 4-((4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)methyl)-N-(4-methyl-3-((4-(pyridin-3-yl)pyrimidin-2-yl)amino)phenyl)benzamide |
| | 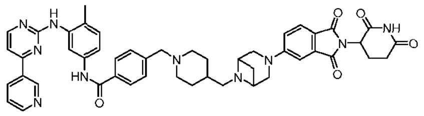 | 4-((4-((3-(2-(2,6-dioxopiperidin-3-yl)-1,3-dioxoisoindolin-5-yl)-3,6-diazabicyclo[3.1.1]heptan-6-yl)methyl)piperidin-1-yl)methyl)-N-(4-methyl-3-((4-(pyridin-3-yl)pyrimidin-2-yl)amino)phenyl)benzamide | 4-((4-((3-(2-(2,6-dioxopiperidin-3-yl)-1,3-dioxoisoindolin-5-yl)-3,6-diazabicyclo[3.1.1]heptan-6-yl)methyl)piperidin-1-yl)methyl)-N-(4-methyl-3-((4-(pyridin-3-yl)pyrimidin-2-yl)amino)phenyl)benzamide |
| | 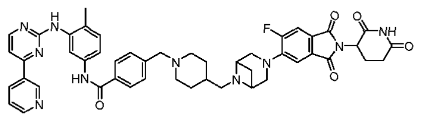 | 4-((4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)methyl)-N-(4-methyl-3-((4-(pyridin-3-yl)pyrimidin-2-yl)amino)phenyl)benzamide | 4-((4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)methyl)-N-(4-methyl-3-((4-(pyridin-3-yl)pyrimidin-2-yl)amino)phenyl)benzamide |
| | 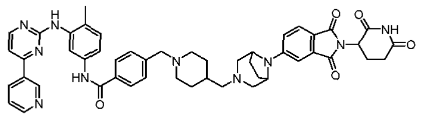 | 4-((4-((8-(2-(2,6-dioxopiperidin-3-yl)-1,3-dioxoisoindolin-5-yl)-3,8-diazabicyclo[3.2.1]octan-3-yl)methyl)piperidin-1-yl)methyl)-N-(4-methyl-3-((4-(pyridin-3-yl)pyrimidin-2-yl)amino)phenyl)benzamide | 4-((4-((8-(2-(2,6-dioxopiperidin-3-yl)-1,3-dioxoisoindolin-5-yl)-3,8-diazabicyclo[3.2.1]octan-3-yl)methyl)piperidin-1-yl)methyl)-N-(4-methyl-3-((4-(pyridin-3-yl)pyrimidin-2-yl)amino)phenyl)benzamide |
| | 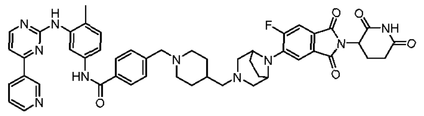 | 4-((4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1- | 4-((4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1- |

| | | | |
|---|---|---|---|
| | | yl)methyl)-N-(4-methyl-3-((4-(pyridin-3-yl)pyrimidin-2-yl)amino)phenyl)benzamide | yl)methyl)-N-(4-methyl-3-((4-(pyridin-3-yl)pyrimidin-2-yl)amino)phenyl)benzamide |
| | 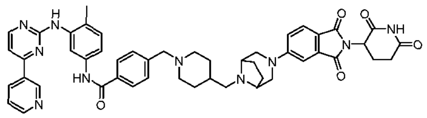 | 4-((4-((3-(2-(2,6-dioxopiperidin-3-yl)-1,3-dioxoisoindolin-5-yl)-3,8-diazabicyclo[3.2.1]octan-8-yl)methyl)piperidin-1-yl)methyl)-N-(4-methyl-3-((4-(pyridin-3-yl)pyrimidin-2-yl)amino)phenyl)benzamide | 4-((4-((3-(2-(2,6-dioxopiperidin-3-yl)-1,3-dioxoisoindolin-5-yl)-3,8-diazabicyclo[3.2.1]octan-8-yl)methyl)piperidin-1-yl)methyl)-N-(4-methyl-3-((4-(pyridin-3-yl)pyrimidin-2-yl)amino)phenyl)benzamide |
| | 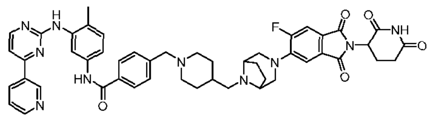 | 4-((4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)methyl)-N-(4-methyl-3-((4-(pyridin-3-yl)pyrimidin-2-yl)amino)phenyl)benzamide | 4-((4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)methyl)-N-(4-methyl-3-((4-(pyridin-3-yl)pyrimidin-2-yl)amino)phenyl)benzamide |
| | 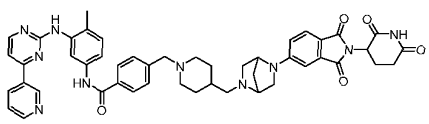 | 4-((4-((5-(2-(2,6-dioxopiperidin-3-yl)-1,3-dioxoisoindolin-5-yl)-2,5-diazabicyclo[2.2.1]heptan-2-yl)methyl)piperidin-1-yl)methyl)-N-(4-methyl-3-((4-(pyridin-3-yl)pyrimidin-2-yl)amino)phenyl)benzamide | 4-((4-((5-(2-(2,6-dioxopiperidin-3-yl)-1,3-dioxoisoindolin-5-yl)-2,5-diazabicyclo[2.2.1]heptan-2-yl)methyl)piperidin-1-yl)methyl)-N-(4-methyl-3-((4-(pyridin-3-yl)pyrimidin-2-yl)amino)phenyl)benzamide |
| | 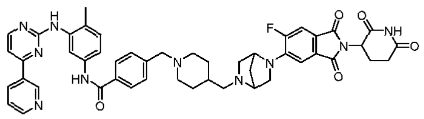 | 4-((4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)methyl)-N-(4-methyl-3-((4-(pyridin-3-yl)pyrimidin-2-yl)amino)phenyl)benzamide | 4-((4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)methyl)-N-(4-methyl-3-((4-(pyridin-3-yl)pyrimidin-2-yl)amino)phenyl)benzamide |
| | 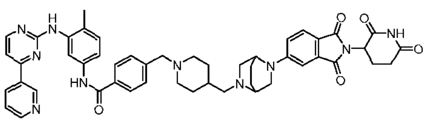 | 4-((4-((5-(2-(2,6-dioxopiperidin-3-yl)-1,3-dioxoisoindolin-5-yl)-2,5-diazabicyclo[2.2.2]octan-2-yl)methyl)piperidin-1-yl)methyl)-N-(4-methyl-3-((4-(pyridin-3-yl)pyrimidin-2-yl)amino)phenyl)benzamide | 4-((4-((5-(2-(2,6-dioxopiperidin-3-yl)-1,3-dioxoisoindolin-5-yl)-2,5-diazabicyclo[2.2.2]octan-2-yl)methyl)piperidin-1-yl)methyl)-N-(4-methyl-3-((4-(pyridin-3-yl)pyrimidin-2-yl)amino)phenyl)benzamide |

| | | | |
|---|---|---|---|
| | 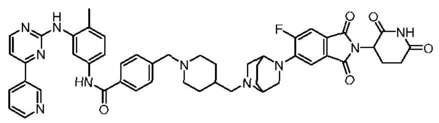 | 4-((4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)methyl)-N-(4-methyl-3-((4-(pyridin-3-yl)pyrimidin-2-yl)amino)phenyl)benzamide | 4-((4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)methyl)-N-(4-methyl-3-((4-(pyridin-3-yl)pyrimidin-2-yl)amino)phenyl)benzamide |
| | 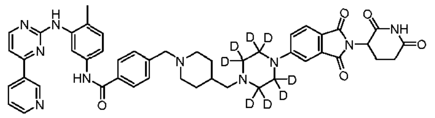 | 4-((4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)methyl)-N-(4-methyl-3-((4-(pyridin-3-yl)pyrimidin-2-yl)amino)phenyl)benzamide |
| | 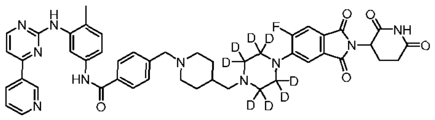 | 4-((4-((4-(2-(2,6-dioxopiperidin-3-yl)-6-fluoro-1,3-dioxoisoindolin-5-yl)piperazin-1-yl-2,2,3,3,5,5,6,6-*d*$_8$)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-6-fluoro-1,3-dioxoisoindolin-5-yl)piperazin-1-yl-2,2,3,3,5,5,6,6-*d*$_8$)methyl)piperidin-1-yl)methyl)-N-(4-methyl-3-((4-(pyridin-3-yl)pyrimidin-2-yl)amino)phenyl)benzamide |
| | 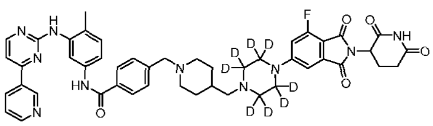 | 4-((4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-7-fluoro-1,3-dioxoisoindolin-5-yl)piperazin-1-yl-2,2,3,3,5,5,6,6-*d*$_8$)methyl)piperidin-1-yl)methyl)-N-(4-methyl-3-((4-(pyridin-3-yl)pyrimidin-2-yl)amino)phenyl)benzamide |
| | 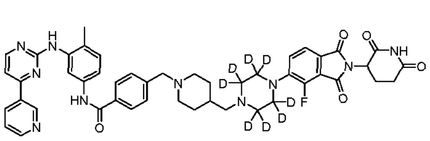 | 4-((4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-4-fluoro-1,3-dioxoisoindolin-5-yl)piperazin-1-yl-2,2,3,3,5,5,6,6-*d*$_8$)methyl)piperidin-1-yl)methyl)-N-(4-methyl-3-((4-(pyridin-3-yl)pyrimidin-2-yl)amino)phenyl)benzamide |

| | | | |
|---|---|---|---|
| | 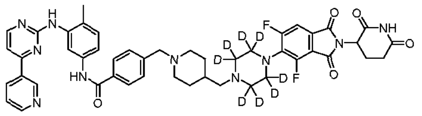 | 4-((4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide |
| | 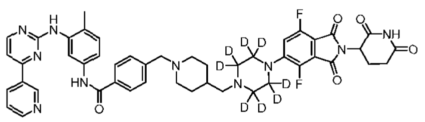 | 4-((4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide |
| | 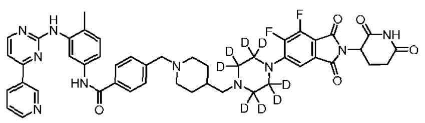 | 4-((4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide |
| | 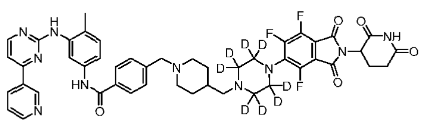 | 4-((4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide |
| | 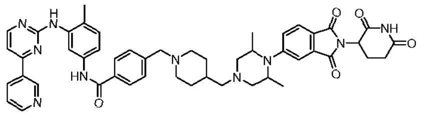 | 4-((4-((4-(2-(2,6-dioxopiperidin-3-yl)-1,3-dioxoisoindolin-5-yl)-3,5-dimethylpiperazin-1-yl)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-1,3-dioxoisoindolin-5-yl)-3,5-dimethylpiperazin-1-yl)methyl)piperidin-1-yl)methyl)-N-(4-methyl-3-((4-(pyridin-3-yl)pyrimidin-2-yl)amino)phenyl)benzamide |

| | | | |
|---|---|---|---|
| | 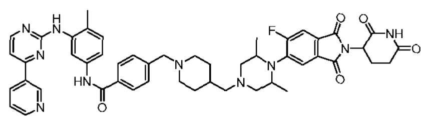 | 4-((4-((4-(2-(2,6-dioxopiperidin-3-yl)-6-fluoro-1,3-dioxoisoindolin-5-yl)-3,5-dimethylpiperazin-1-yl)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-6-fluoro-1,3-dioxoisoindolin-5-yl)-3,5-dimethylpiperazin-1-yl)methyl)piperidin-1-yl)methyl)-N-(4-methyl-3-((4-(pyridin-3-yl)pyrimidin-2-yl)amino)phenyl)benzamide |
| | 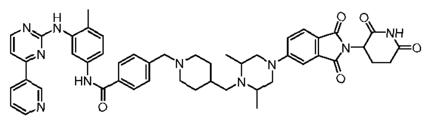 | 4-((4-((4-(2-(2,6-dioxopiperidin-3-yl)-1,3-dioxoisoindolin-5-yl)-2,6-dimethylpiperazin-1-yl)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-1,3-dioxoisoindolin-5-yl)-2,6-dimethylpiperazin-1-yl)methyl)piperidin-1-yl)methyl)-N-(4-methyl-3-((4-(pyridin-3-yl)pyrimidin-2-yl)amino)phenyl)benzamide |
| | 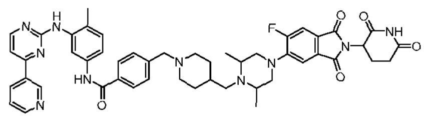 | 4-((4-((4-(2-(2,6-dioxopiperidin-3-yl)-6-fluoro-1,3-dioxoisoindolin-5-yl)-2,6-dimethylpiperazin-1-yl)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-6-fluoro-1,3-dioxoisoindolin-5-yl)-2,6-dimethylpiperazin-1-yl)methyl)piperidin-1-yl)methyl)-N-(4-methyl-3-((4-(pyridin-3-yl)pyrimidin-2-yl)amino)phenyl)benzamide |
| | 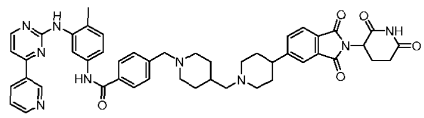 | 4-((4-((4-(2-(2,6-dioxopiperidin-3-yl)-1,3-dioxoisoindolin-5-yl)piperidin-1-yl)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-1,3-dioxoisoindolin-5-yl)piperidin-1-yl)methyl)piperidin-1-yl)methyl)-N-(4-methyl-3-((4-(pyridin-3-yl)pyrimidin-2-yl)amino)phenyl)benzamide |
| | 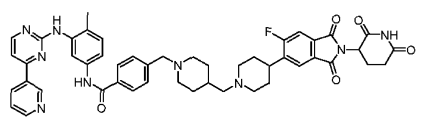 | 4-((4-((4-(2-(2,6-dioxopiperidin-3-yl)-6-fluoro-1,3-dioxoisoindolin-5-yl)piperidin-1-yl)methyl)piperidin-1-yl)methyl)-N-(4-methyl-3-((4-(pyridin-3-yl)pyrimidin-2-yl)amino)phenyl)benzamide | 4-((4-((4-(2-(2,6-dioxopiperidin-3-yl)-6-fluoro-1,3-dioxoisoindolin-5-yl)piperidin-1-yl)methyl)piperidin-1-yl)methyl)-N-(4-methyl-3-((4-(pyridin-3-yl)pyrimidin-2-yl)amino)phenyl)benzamide |
| | 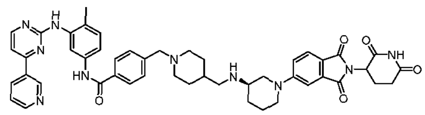 | 4-((4-((((3R)-1-(2-(2,6-dioxopiperidin-3-yl)-1,3-dioxoisoindolin-5-yl)piperidin-3-yl)amino)methyl)piperidin-1-yl)methyl)-N-(4-methyl-3- | 4-((4-((((3R)-1-(2-(2,6-dioxopiperidin-3-yl)-1,3-dioxoisoindolin-5-yl)piperidin-3-yl)amino)methyl)piperidin-1-yl)methyl)-N-(4-methyl- |

| | | | |
|---|---|---|---|
| | | ((4-(pyridin-3-yl)pyrimidin-2-yl)amino)phenyl)benzamide | 3-((4-(pyridin-3-yl)pyrimidin-2-yl)amino)phenyl)benzamide |
| | 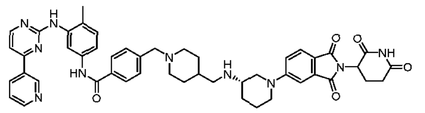 | 4-((4-((((3S)-1-(2-(2,6-dioxopiperidin-3-yl)-1,3-dioxoisoindolin-5-yl)piperidin-3-yl)amino)methyl)piperidin-1-yl)methyl)-N-(4-methyl-3-((4-(pyridin-3-yl)pyrimidin-2-yl)amino)phenyl)benzamide | 4-((4-((((3S)-1-(2-(2,6-dioxopiperidin-3-yl)-1,3-dioxoisoindolin-5-yl)piperidin-3-yl)amino)methyl)piperidin-1-yl)methyl)-N-(4-methyl-3-((4-(pyridin-3-yl)pyrimidin-2-yl)amino)phenyl)benzamide |
| | 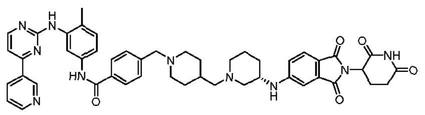 | 4-((4-(((3S)-3-((2-(2,6-dioxopiperidin-3-yl)-1,3-dioxoisoindolin-5-yl)amino)piperidin-1-yl)methyl)piperidin-1-yl)methyl)-N-(4-methyl-3-((4-(pyridin-3-yl)pyrimidin-2-yl)amino)phenyl)benzamide | 4-((4-(((3S)-3-((2-(2,6-dioxopiperidin-3-yl)-1,3-dioxoisoindolin-5-yl)amino)piperidin-1-yl)methyl)piperidin-1-yl)methyl)-N-(4-methyl-3-((4-(pyridin-3-yl)pyrimidin-2-yl)amino)phenyl)benzamide |
| | 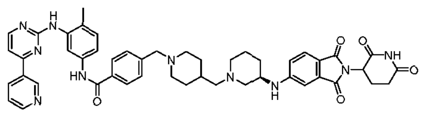 | 4-((4-(((3R)-3-((2-(2,6-dioxopiperidin-3-yl)-1,3-dioxoisoindolin-5-yl)amino)piperidin-1-yl)methyl)piperidin-1-yl)methyl)-N-(4-methyl-3-((4-(pyridin-3-yl)pyrimidin-2-yl)amino)phenyl)benzamide | 4-((4-(((3R)-3-((2-(2,6-dioxopiperidin-3-yl)-1,3-dioxoisoindolin-5-yl)amino)piperidin-1-yl)methyl)piperidin-1-yl)methyl)-N-(4-methyl-3-((4-(pyridin-3-yl)pyrimidin-2-yl)amino)phenyl)benzamide |
| | 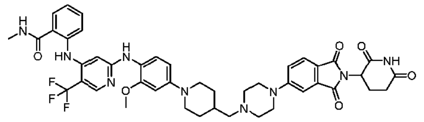 | 2-((2-((4-(4-((4-(2-(2,6-dioxopiperidin-3-yl)-1,3-dioxoisoindolin-5-yl)piperazin-1-yl)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-1,3-dioxoisoindolin-5-yl)piperazin-1-yl)methyl)piperidin-1-yl)-2-methoxyphenyl)amino)-5-(trifluoromethyl)pyridin-4-yl)amino)-N-methylbenzamide |
| | 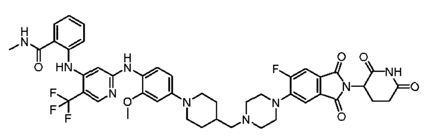 | 2-((2-((4-(4-((4-(2-(2,6-dioxopiperidin-3-yl)-6-fluoro-1,3-dioxoisoindolin-5-yl)piperazin-1-yl)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-6-fluoro-1,3-dioxoisoindolin-5-yl)piperazin-1-yl)methyl)piperidin-1-yl)-2-methoxyphenyl)amino)-5-(trifluoromethyl)pyridin-4-yl)amino)-N-methylbenzamide |
| | 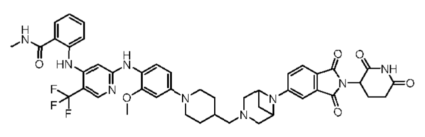 | 2-((2-((4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-2-methoxyphenyl)amino)-5- | 2-((2-((4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-2- |

| | | | |
|---|---|---|---|
| | | (trifluoromethyl)pyridin-4-yl)amino)-N-methylbenzamide | methoxyphenyl)amino)-5-(trifluoromethyl)pyridin-4-yl)amino)-N-methylbenzamide |
| | 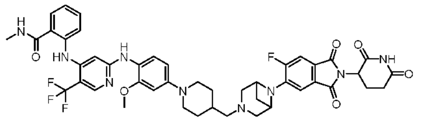 | 2-((2-((4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-2-methoxyphenyl)amino)-5-(trifluoromethyl)pyridin-4-yl)amino)-N-methylbenzamide | 2-((2-((4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-2-methoxyphenyl)amino)-5-(trifluoromethyl)pyridin-4-yl)amino)-N-methylbenzamide |
| | 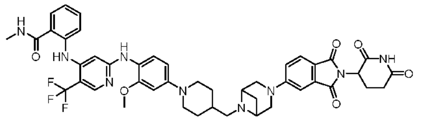 | 2-((2-((4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-2-methoxyphenyl)amino)-5-(trifluoromethyl)pyridin-4-yl)amino)-N-methylbenzamide | 2-((2-((4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-2-methoxyphenyl)amino)-5-(trifluoromethyl)pyridin-4-yl)amino)-N-methylbenzamide |
| | 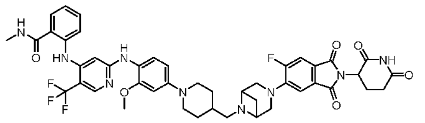 | 2-((2-((4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-2-methoxyphenyl)amino)-5-(trifluoromethyl)pyridin-4-yl)amino)-N-methylbenzamide | 2-((2-((4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-2-methoxyphenyl)amino)-5-(trifluoromethyl)pyridin-4-yl)amino)-N-methylbenzamide |
| | 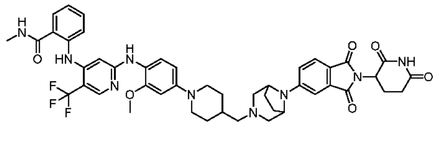 | 2-((2-((4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-2-methoxyphenyl)amino)-5-(trifluoromethyl)pyridin-4-yl)amino)-N-methylbenzamide | 2-((2-((4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-2-methoxyphenyl)amino)-5-(trifluoromethyl)pyridin-4-yl)amino)-N-methylbenzamide |
| | 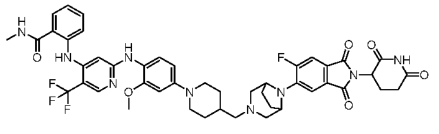 | 2-((2-((4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-2-methoxyphenyl)amino)-5-(trifluoromethyl)pyridin-4-yl)amino)-N-methylbenzamide | 2-((2-((4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-2-methoxyphenyl)amino)-5-(trifluoromethyl)pyridin-4-yl)amino)-N-methylbenzamide |

| | | | |
|---|---|---|---|
| | 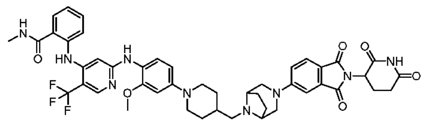 | 2-((2-((4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-2-methoxyphenyl)amino)-5-(trifluoromethyl)pyridin-4-yl)amino)-N-methylbenzamide | 2-((2-((4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-2-methoxyphenyl)amino)-5-(trifluoromethyl)pyridin-4-yl)amino)-N-methylbenzamide |
| | 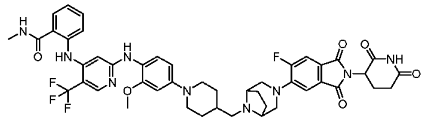 | 2-((2-((4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-2-methoxyphenyl)amino)-5-(trifluoromethyl)pyridin-4-yl)amino)-N-methylbenzamide | 2-((2-((4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-2-methoxyphenyl)amino)-5-(trifluoromethyl)pyridin-4-yl)amino)-N-methylbenzamide |
| | 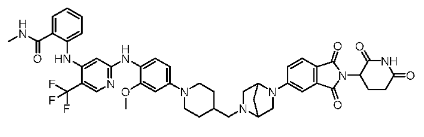 | 2-((2-((4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-2-methoxyphenyl)amino)-5-(trifluoromethyl)pyridin-4-yl)amino)-N-methylbenzamide | 2-((2-((4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-2-methoxyphenyl)amino)-5-(trifluoromethyl)pyridin-4-yl)amino)-N-methylbenzamide |
| | 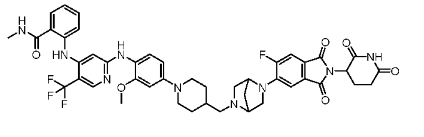 | 2-((2-((4-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-2-methoxyphenyl)amino)-5-(trifluoromethyl)pyridin-4-yl)amino)-N-methylbenzamide | 2-((2-((4-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-2-methoxyphenyl)amino)-5-(trifluoromethyl)pyridin-4-yl)amino)-N-methylbenzamide |
| | 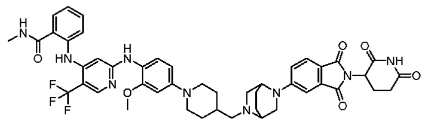 | 2-((2-((4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-2-methoxyphenyl)amino)-5-(trifluoromethyl)pyridin-4-yl)amino)-N-methylbenzamide | 2-((2-((4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-2-methoxyphenyl)amino)-5-(trifluoromethyl)pyridin-4-yl)amino)-N-methylbenzamide |
| | 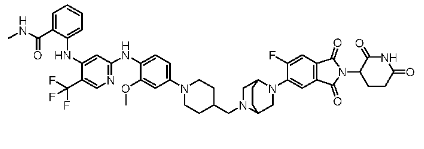 | 2-((2-((4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-2-methoxyphenyl)amino)-5-(trifluoromethyl)pyridin-4-yl)amino)-N- | 2-((2-((4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-2-methoxyphenyl)amino)-5-(trifluoromethyl)pyridin-4-yl)amino)-N- |

| | | | |
|---|---|---|---|
| | | methylbenzamide | methylbenzamide |
| | 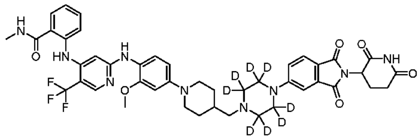 | 2-((2-((4-(4-((4-(2-(2,6-dioxopiperidin-3-yl)-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide |
| | 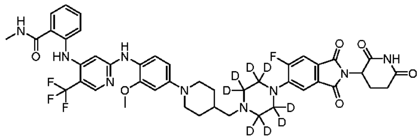 | 2-((2-((4-(4-((4-(2-(2,6-dioxopiperidin-3-yl)-6-fluoro-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-6-fluoro-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide |
| | 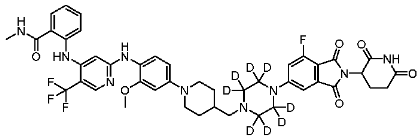 | 2-((2-((4-(4-((4-(2-(2,6-dioxopiperidin-3-yl)-7-fluoro-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-7-fluoro-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide |
| | 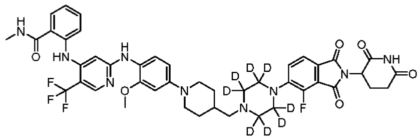 | 2-((2-((4-(4-((4-(2-(2,6-dioxopiperidin-3-yl)-4-fluoro-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-4-fluoro-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide |
| | 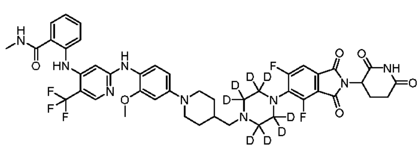 | 2-((2-((4-(4-((4-(2-(2,6-dioxopiperidin-3-yl)-4,6-difluoro-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-4,6-difluoro-1,3-dioxoisoindolin-5-yl)piperazin-1-yl-2,2,3,3,5,5,6,6-*d*8)methyl)piperidin-1-yl)-2-methoxyphenyl)amino)-5-(trifluoromethyl)pyridin-4-yl)amino)-N-methylbenzamide |
| | 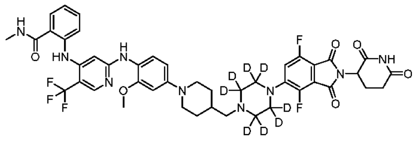 | 2-((2-((4-(4-((4-(2-(2,6-dioxopiperidin-3-yl)-4,7-difluoro-1,3-dioxoisoindolin-5-yl)piperazin-1-yl-2,2,3,3,5,5,6,6-*d*8)methyl)piperidin-1-yl)-2- | 2-((2-((4-(4-((4-(2-(2,6-dioxopiperidin-3-yl)-4,7-difluoro-1,3-dioxoisoindolin-5-yl)piperazin-1-yl-2,2,3,3,5,5,6,6- |

| | | | |
|---|---|---|---|
| | | methoxyphenyl)amino)-5-(trifluoromethyl)pyridin-4-yl)amino)-N-methylbenzamide | $d_8$)methyl)piperidin-1-yl)-2-methoxyphenyl)amino)-5-(trifluoromethyl)pyridin-4-yl)amino)-N-methylbenzamide |
| | 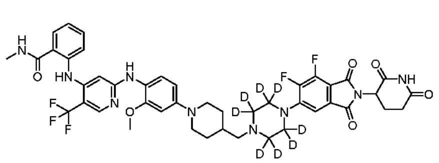 | 2-((2-((4-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide |
| | 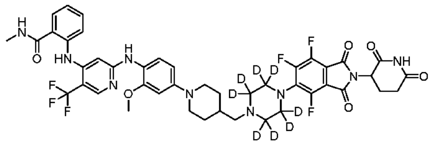 | 2-((2-((4-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide |
| | 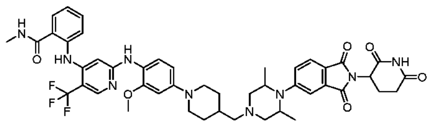 | 2-((2-((4-(4-((4-(2-(2,6-dioxopiperidin-3-yl)-1,3-dioxoisoindolin-5-yl)-3,5-dimethylpiperazin-1-yl)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-1,3-dioxoisoindolin-5-yl)-3,5-dimethylpiperazin-1-yl)methyl)piperidin-1-yl)-2-methoxyphenyl)amino)-5-(trifluoromethyl)pyridin-4-yl)amino)-N-methylbenzamide |
| | 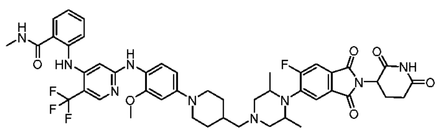 | 2-((2-((4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-2-methoxyphenyl)amino)-5-(trifluoromethyl)pyridin-4-yl)amino)-N-methylbenzamide |
| | 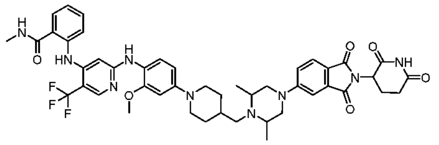 | 2-((2-((4-(4-((4-(2-(2,6-dioxopiperidin-3-yl)-1,3-dioxoisoindolin-5-yl)-2,6-dimethylpiperazin-1-yl)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-1,3-dioxoisoindolin-5-yl)-2,6-dimethylpiperazin-1-yl)methyl)piperidin-1-yl)-2-methoxyphenyl)amino)-5-(trifluoromethyl)pyridin-4-yl)amino)-N-methylbenzamide |

| | | | |
|---|---|---|---|
| | 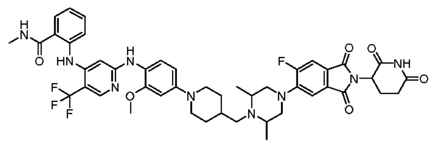 | 2-((2-((4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-2-methoxyphenyl)amino)-5-(trifluoromethyl)pyridin-4-yl)amino)-N-methylbenzamide |
| | 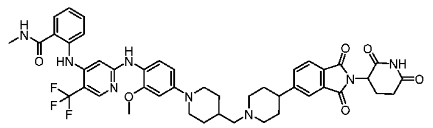 | 2-((2-((4-(4-((4-(2-(2,6-dioxopiperidin-3-yl)-1,3-dioxoisoindolin-5-yl)piperidin-1-yl)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-1,3-dioxoisoindolin-5-yl)piperidin-1-yl)methyl)piperidin-1-yl)-2-methoxyphenyl)amino)-5-(trifluoromethyl)pyridin-4-yl)amino)-N-methylbenzamide |
| | 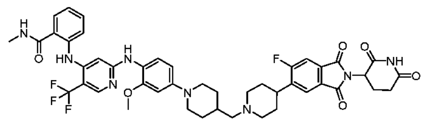 | 2-((2-((4-(4-((4-(2-(2,6-dioxopiperidin-3-yl)-6-fluoro-1,3-dioxoisoindolin-5-yl)piperidin-1-yl)methyl)piperidin-1-yl)-2-methoxyphenyl)amino)-5-(trifluoromethyl)pyridin-4-yl)amino)-N-methylbenzamide | 2-((2-((4-(4-((4-(2-(2,6-dioxopiperidin-3-yl)-6-fluoro-1,3-dioxoisoindolin-5-yl)piperidin-1-yl)methyl)piperidin-1-yl)-2-methoxyphenyl)amino)-5-(trifluoromethyl)pyridin-4-yl)amino)-N-methylbenzamide |
| | 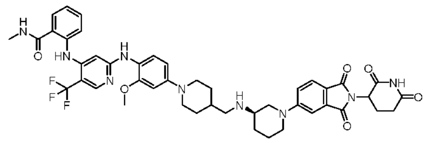 | 2-((2-((4-(4-((((3R)-1-(2-(2,6-dioxopiperidin-3-yl)-1,3-dioxoisoindolin-5-yl)piperidin-3-yl)amino)methyl)piperidin-1-yl)-2-methoxyphenyl)amino)-5-(trifluoromethyl)pyridin-4-yl)amino)-N-methylbenzamide | 2-((2-((4-(4-((((3R)-1-(2-(2,6-dioxopiperidin-3-yl)-1,3-dioxoisoindolin-5-yl)piperidin-3-yl)amino)methyl)piperidin-1-yl)-2-methoxyphenyl)amino)-5-(trifluoromethyl)pyridin-4-yl)amino)-N-methylbenzamide |
| | 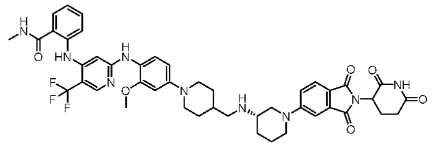 | 2-((2-((4-(4-((((3S)-1-(2-(2,6-dioxopiperidin-3-yl)-1,3-dioxoisoindolin-5-yl)piperidin-3-yl)amino)methyl)piperidin-1-yl)-2-methoxyphenyl)amino)-5-(trifluoromethyl)pyridin-4-yl)amino)-N-methylbenzamide | 2-((2-((4-(4-((((3S)-1-(2-(2,6-dioxopiperidin-3-yl)-1,3-dioxoisoindolin-5-yl)piperidin-3-yl)amino)methyl)piperidin-1-yl)-2-methoxyphenyl)amino)-5-(trifluoromethyl)pyridin-4-yl)amino)-N-methylbenzamide |
| | 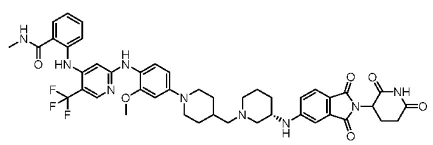 | 2-((2-((4-(4-(((3S)-3-((2-(2,6-dioxopiperidin-3-yl)-1,3-dioxoisoindolin-5-yl)amino)piperidin-1-yl)methyl)piperidin-1-yl)-2-methoxyphenyl)amino)-5-(trifluoromethyl)pyridin-4-yl)amino)-N-methylbenzamide | 2-((2-((4-(4-(((3S)-3-((2-(2,6-dioxopiperidin-3-yl)-1,3-dioxoisoindolin-5-yl)amino)piperidin-1-yl)methyl)piperidin-1-yl)-2-methoxyphenyl)amino)-5-(trifluoromethyl)pyridin-4-yl)amino)-N-methylbenzamide |

| | | | |
|---|---|---|---|
| | 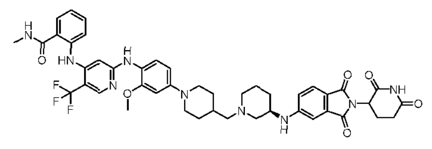 | 2-((2-((4-(4-(((3R)-3-((2-(2,6-dioxopiperidin-3-yl)-1,3-dioxoisoindolin-5-yl)amino)piperidin-1-yl)methyl)piperidin-1-yl)-2-methoxyphenyl)amino)-5-(trifluoromethyl)pyridin-4-yl)amino)-N-methylbenzamide | 2-((2-((4-(4-(((3R)-3-((2-(2,6-dioxopiperidin-3-yl)-1,3-dioxoisoindolin-5-yl)amino)piperidin-1-yl)methyl)piperidin-1-yl)-2-methoxyphenyl)amino)-5-(trifluoromethyl)pyridin-4-yl)amino)-N-methylbenzamide |
| | 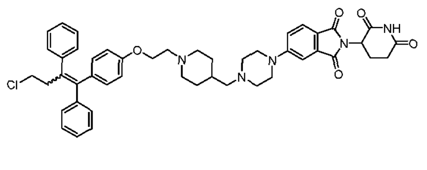 | 5-(4-((1-(2-(4-(4-chloro-1,2-diphenylbut-1-en-1-yl)phenoxy)ethyl)piperidin-4-yl)methyl)piperazin-1-yl)-2-(2,6-dioxopiperidin-3-yl)isoindoline-1,3-dione | 5-(4-((1-(2-(4-(4-chloro-1,2-diphenylbut-1-en-1-yl)phenoxy)ethyl)piperidin-4-yl)methyl)piperazin-1-yl)-2-(2,6-dioxopiperidin-3-yl)isoindoline-1,3-dione |
| | 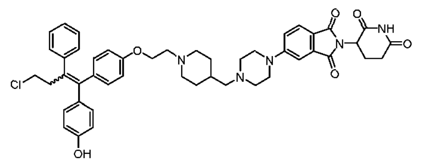 | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl)-2-(2,6-dioxopiperidin-3-yl)isoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl)-2-(2,6-dioxopiperidin-3-yl)isoindoline-1,3-dione |
| | 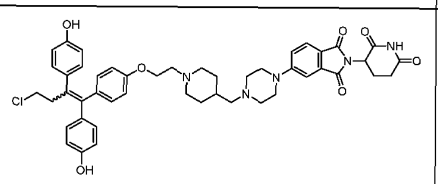 | 5-(4-((1-(2-(4-(4-chloro-1,2-bis(4-hydroxyphenyl)but-1-en-1-yl)phenoxy)ethyl)piperidin-4-yl)methyl)piperazin-1-yl)-2-(2,6-dioxopiperidin-3-yl)isoindoline-1,3-dione | 5-(4-((1-(2-(4-(4-chloro-1,2-bis(4-hydroxyphenyl)but-1-en-1-yl)phenoxy)ethyl)piperidin-4-yl)methyl)piperazin-1-yl)-2-(2,6-dioxopiperidin-3-yl)isoindoline-1,3-dione |
| | 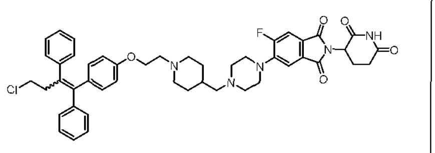 | 5-(4-((1-(2-(4-(4-chloro-1,2-diphenylbut-1-en-1-yl)phenoxy)ethyl)piperidin-4-yl)methyl)piperazin-1-yl)-2-(2,6-dioxopiperidin-3-yl)-6-fluoroisoindoline-1,3-dione | 5-(4-((1-(2-(4-(4-chloro-1,2-diphenylbut-1-en-1-yl)phenoxy)ethyl)piperidin-4-yl)methyl)piperazin-1-yl)-2-(2,6-dioxopiperidin-3-yl)-6-fluoroisoindoline-1,3-dione |
| | 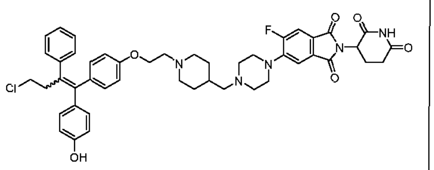 | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl)-2-(2,6-dioxopiperidin-3-yl)-6-fluoroisoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl)-2-(2,6-dioxopiperidin-3-yl)-6-fluoroisoindoline-1,3-dione |
| | 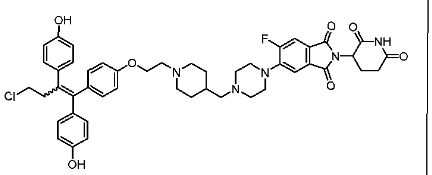 | 5-(4-((1-(2-(4-(4-chloro-1,2-bis(4-hydroxyphenyl)but-1-en-1-yl)phenoxy)ethyl)piperidin-4-yl)methyl)piperazin-1-yl)-2-(2,6-dioxopiperidin-3-yl)-6-fluoroisoindoline-1,3-dione | 5-(4-((1-(2-(4-(4-chloro-1,2-bis(4-hydroxyphenyl)but-1-en-1-yl)phenoxy)ethyl)piperidin-4-yl)methyl)piperazin-1-yl)-2-(2,6-dioxopiperidin-3-yl)-6-fluoroisoindoline-1,3-dione |
| | 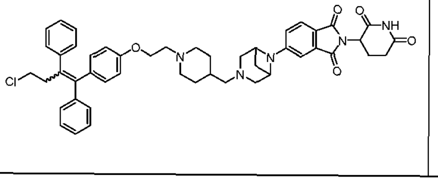 | 5-(3-((1-(2-(4-(4-chloro-1,2-diphenylbut-1-en-1-yl)phenoxy)ethyl)piperidin-4-yl)methyl)-3,6-diazabicyclo[3.1.1]heptan-6-yl)-2-(2,6-dioxopiperidin-3- | 5-(3-((1-(2-(4-(4-chloro-1,2-diphenylbut-1-en-1-yl)phenoxy)ethyl)piperidin-4-yl)methyl)-3,6-diazabicyclo[3.1.1]heptan-6-yl)-2-(2,6- |

| | | | |
|---|---|---|---|
| | | yl)isoindoline-1,3-dione | dioxopiperidin-3-yl)isoindoline-1,3-dione |
| | 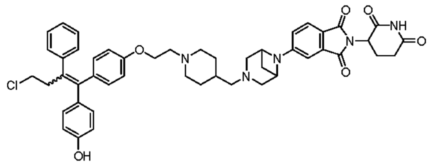 | 5-(3-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione |
| | 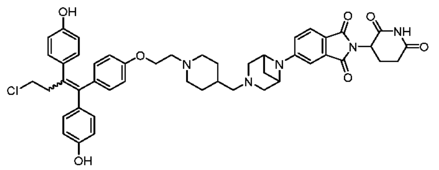 | 5-(3-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione |
| | 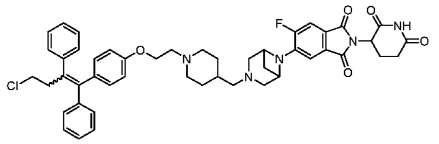 | 5-(3-((1-(2-(4-(4-chloro-1,2-diphenylbut-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(2-(4-(4-chloro-1,2-diphenylbut-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 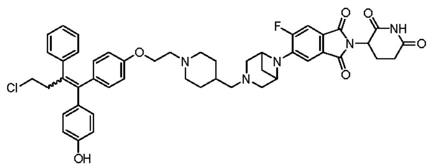 | 5-(3-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 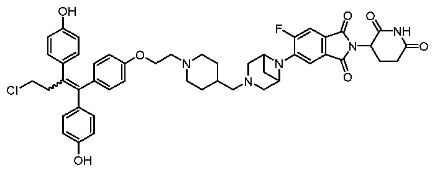 | 5-(3-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 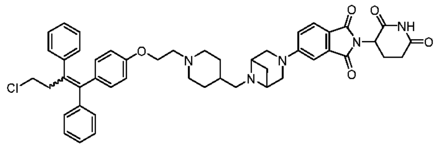 | 5-(6-((1-(2-(4-(4-chloro-1,2-diphenylbut-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(2-(4-(4-chloro-1,2-diphenylbut-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione |

| | | | |
|---|---|---|---|
| | 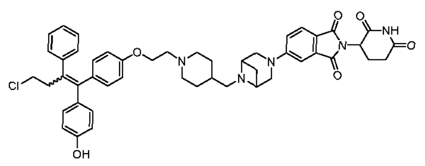 | 5-(6-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione |
| | 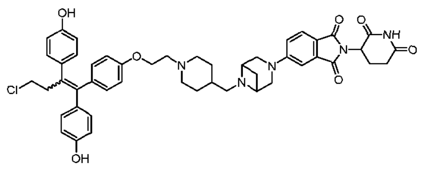 | 5-(6-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione |
| | 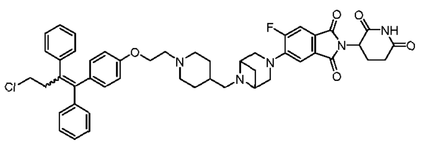 | 5-(6-((1-(2-(4-(4-chloro-1,2-diphenylbut-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione | 5-(6-((1-(2-(4-(4-chloro-1,2-diphenylbut-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione |
| | 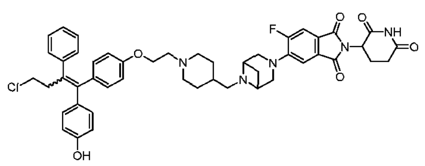 | 5-(6-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione | 5-(6-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione |
| | 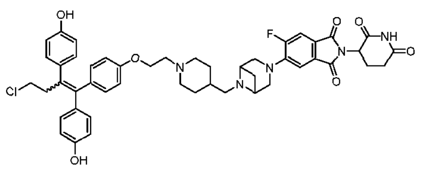 | 5-(6-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione | 5-(6-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione |
| | 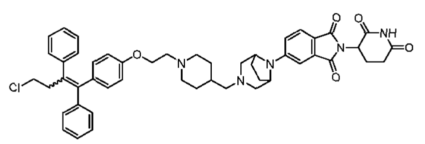 | 5-(3-((1-(2-(4-(4-chloro-1,2-diphenylbut-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione | 5-(3-((1-(2-(4-(4-chloro-1,2-diphenylbut-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione |
| | 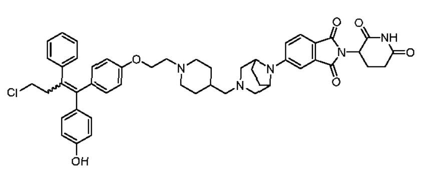 | 5-(3-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-8-yl)-2-(2,6-dioxopiperidin-3- | 5-(3-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-8-yl)-2-(2,6-dioxopiperidin- |

| | | | |
|---|---|---|---|
| | | yl)isoindoline-1,3-dione | 3-yl)isoindoline-1,3-dione |
| | 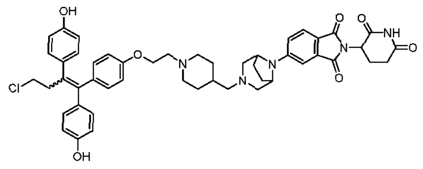 | 5-(3-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione | 5-(3-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione |
| | 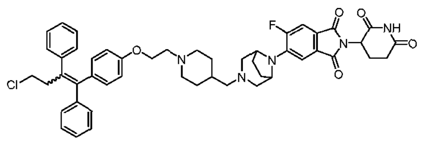 | 5-(3-((1-(2-(4-(4-chloro-1,2-diphenylbut-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(2-(4-(4-chloro-1,2-diphenylbut-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione |
| | 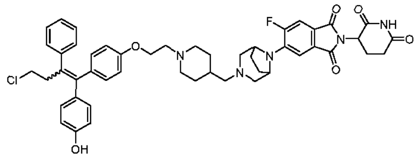 | 5-(3-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione |
| | 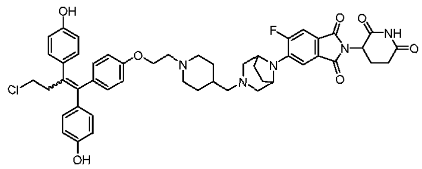 | 5-(3-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione |
| | 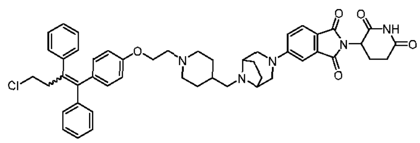 | 5-(8-((1-(2-(4-(4-chloro-1,2-diphenylbut-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione | 5-(8-((1-(2-(4-(4-chloro-1,2-diphenylbut-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione |
| | 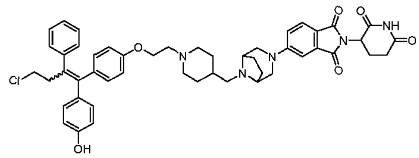 | 5-(8-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione | 5-(8-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione |
| | 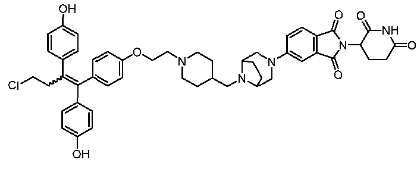 | 5-(8-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione | 5-(8-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione |

| | | | |
|---|---|---|---|
| | 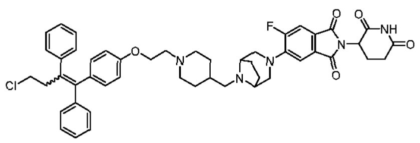 | 5-(8-((1-(2-(4-(4-chloro-1,2-diphenylbut-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(2-(4-(4-chloro-1,2-diphenylbut-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 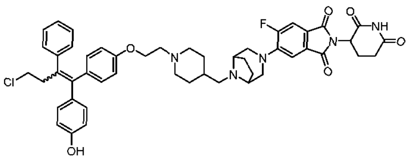 | 5-(8-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 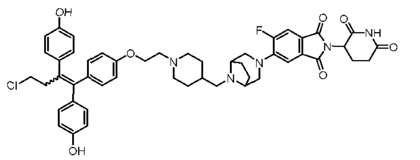 | 5-(8-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 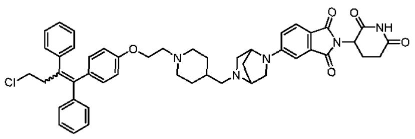 | 5-(5-((1-(2-(4-(4-chloro-1,2-diphenylbut-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(2-(4-(4-chloro-1,2-diphenylbut-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione |
| | 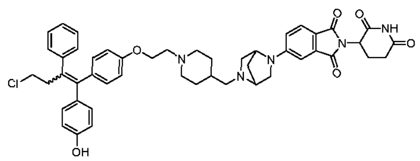 | 5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione |
| | 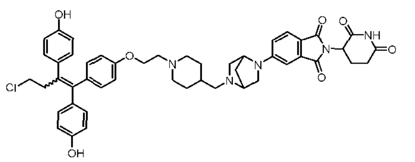 | 5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione |
| | 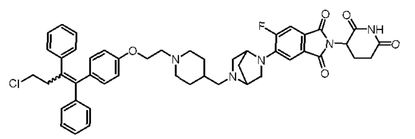 | 5-(5-((1-(2-(4-(4-chloro-1,2-diphenylbut-1-en-1-yl)phenoxy)ethyl)piperidin-4-yl)methyl)-2,5-diazabicyclo[2.2.1]heptan-2-yl)-2-(2,6-dioxopiperidin-3- | 5-(5-((1-(2-(4-(4-chloro-1,2-diphenylbut-1-en-1-yl)phenoxy)ethyl)piperidin-4-yl)methyl)-2,5-diazabicyclo[2.2.1]heptan-2-yl)-2-(2,6- |

| | | | |
|---|---|---|---|
| | | yl)-6-fluoroisoindoline-1,3-dione | dioxopiperidin-3-yl)-6-fluoroisoindoline-1,3-dione |
| | 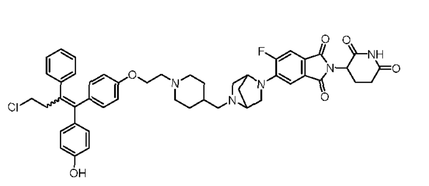 | 5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 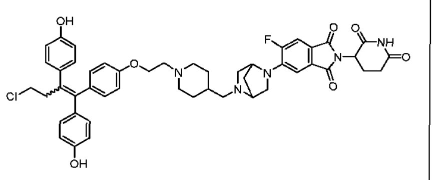 | 5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 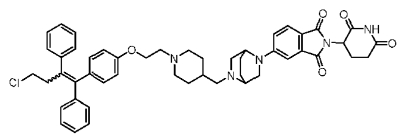 | 5-(5-((1-(2-(4-(4-chloro-1,2-diphenylbut-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione | 5-(5-((1-(2-(4-(4-chloro-1,2-diphenylbut-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione |
| | 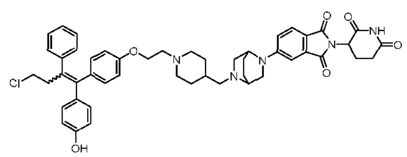 | 5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione | 5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione |
| | 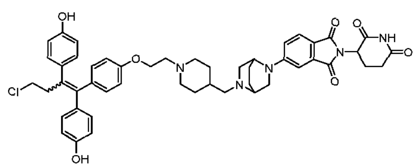 | 5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione | 5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione |
| | 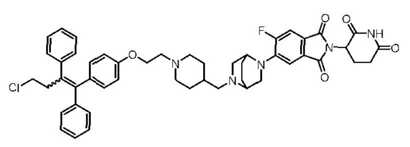 | 5-(5-((1-(2-(4-(4-chloro-1,2-diphenylbut-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(2-(4-(4-chloro-1,2-diphenylbut-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 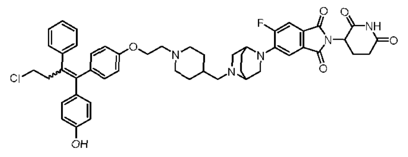 | 5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.2]octan-2-yl)-2-(2,6-dioxopiperidin-3- | 5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.2]octan-2-yl)-2-(2,6-dioxopiperidin- |

| | | | |
|---|---|---|---|
| | | yl)-6-fluoroisoindoline-1,3-dione | 3-yl)-6-fluoroisoindoline-1,3-dione |
| | 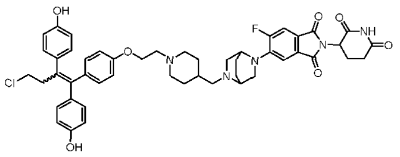 | 5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 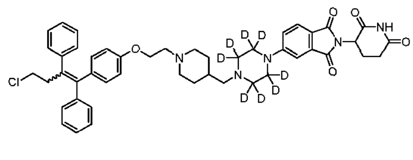 | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione |
| | 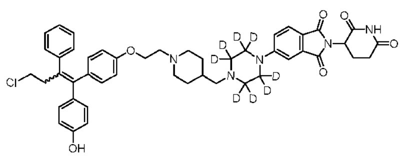 | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione |
| | 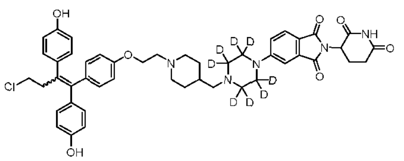 | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione |
| | 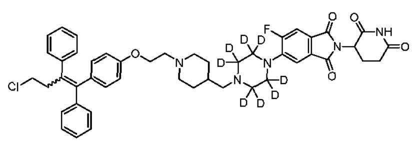 | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione |
| | 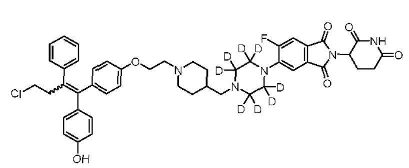 | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione |
| | 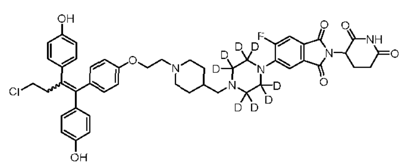 | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6- | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)- |

| | | | |
|---|---|---|---|
| | | fluoroisoindoline-1,3-dione | 6-fluoroisoindoline-1,3-dione |
| | 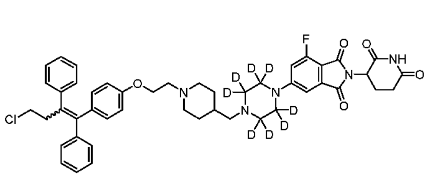 | 6-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 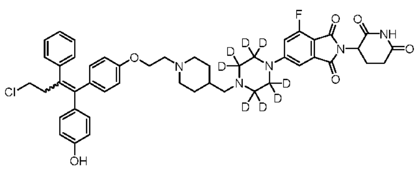 | 6-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 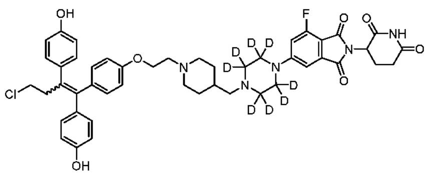 | 6-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 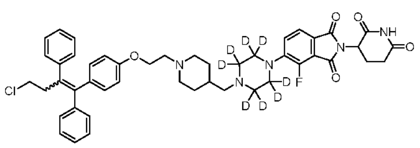 | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 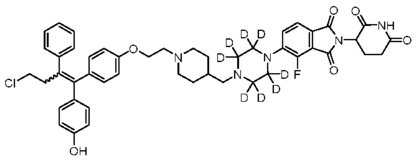 | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 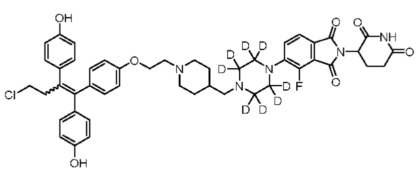 | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |

| | | | |
|---|---|---|---|
| | 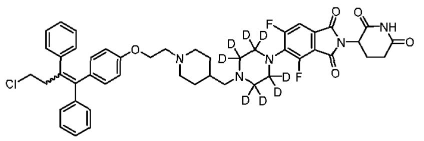 | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |
| | 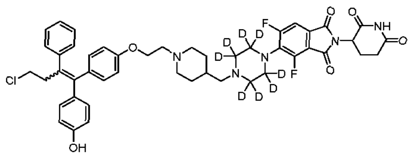 | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |
| | 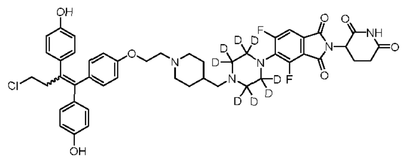 | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |
| | 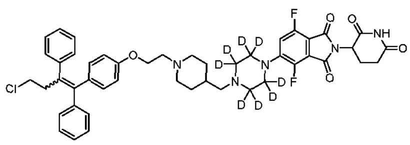 | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione |
| | 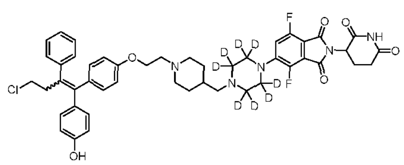 | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione |
| | 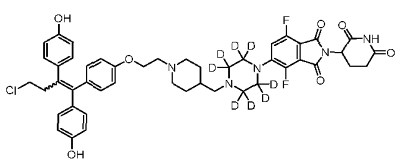 | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione |
| | 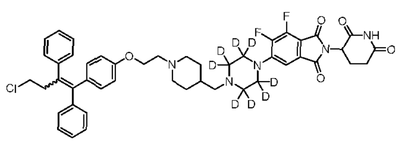 | 6-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,5- | 6-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)- |

| | | | |
|---|---|---|---|
| | | difluoroisoindoline-1,3-dione | 4,5-difluoroisoindoline-1,3-dione |
| | 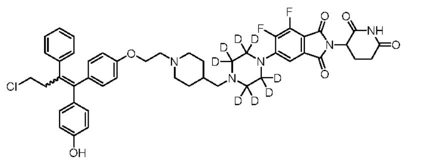 | 6-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione | 6-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione |
| | 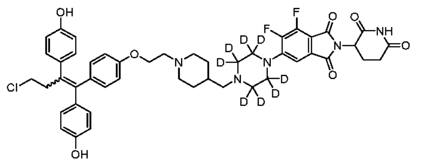 | 6-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione | 6-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione |
| | 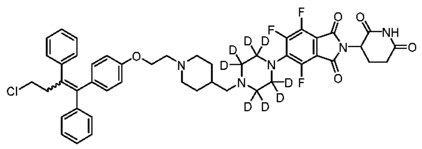 | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |
| | 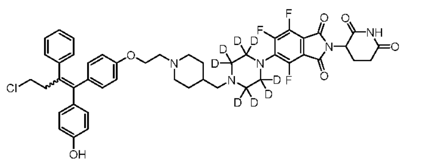 | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |
| | 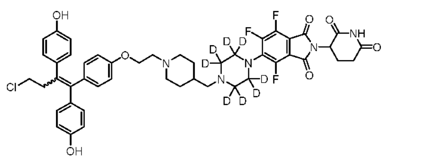 | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |
| | 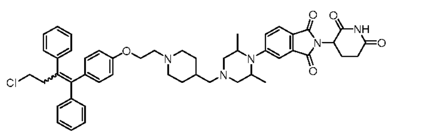 | 5-(4-((1-(2-(4-(4-chloro-1,2-diphenylbut-1-en-1-yl)phenoxy)ethyl)piperidin-4-yl)methyl)-2,6-dimethylpiperazin-1-yl)-2-(2,6-dioxopiperidin-3-yl)isoindoline-1,3-dione | 5-(4-((1-(2-(4-(4-chloro-1,2-diphenylbut-1-en-1-yl)phenoxy)ethyl)piperidin-4-yl)methyl)-2,6-dimethylpiperazin-1-yl)-2-(2,6-dioxopiperidin-3-yl)isoindoline-1,3-dione |
| | 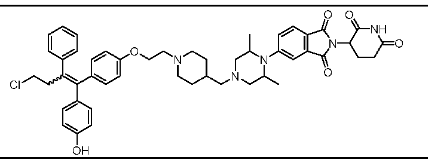 | 5-(4-((1-(2-(4-(4-chloro-1-(4-hydroxyphenyl)-2-phenylbut-1-en-1-yl)phenoxy)ethyl)piperidin-4-yl)methyl)-2,6- | 5-(4-((1-(2-(4-(4-chloro-1-(4-hydroxyphenyl)-2-phenylbut-1-en-1-yl)phenoxy)ethyl)piperidin-4-yl)methyl)-2,6- |

| | | | |
|---|---|---|---|
| | | dimethylpiperazin-1-yl)-2-(2,6-dioxopiperidin-3-yl)isoindoline-1,3-dione | dimethylpiperazin-1-yl)-2-(2,6-dioxopiperidin-3-yl)isoindoline-1,3-dione |
| | 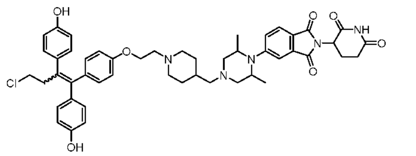 | 5-(4-((1-(2-(4-(4-chloro-1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)isoindoline-1,3-dione | 5-(4-((1-(2-(4-(4-chloro-1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)isoindoline-1,3-dione |
| | 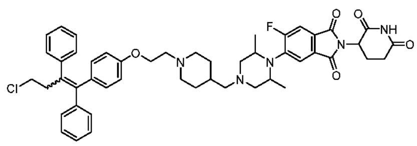 | 5-(4-((1-(2-(4-(4-chloro-1,2-diphenylbut-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione | 5-(4-((1-(2-(4-(4-chloro-1,2-diphenylbut-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione |
| | 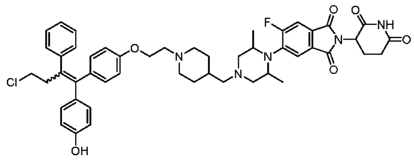 | 5-(4-((1-(2-(4-(4-chloro-1-(4-hydroxyphenyl)-2-phenylbut-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione |
| | 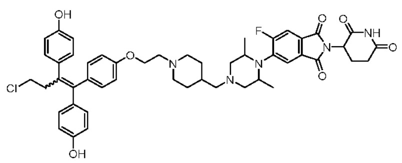 | 5-(4-((1-(2-(4-(4-chloro-1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione | 5-(4-((1-(2-(4-(4-chloro-1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione |
| | 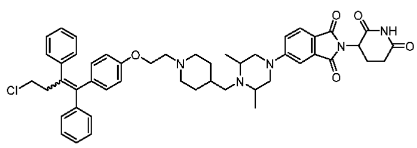 | 5-(4-((1-(2-(4-(4-chloro-1,2-diphenylbut-1-en-1-yl)phenoxy)ethyl)piperidin-4-yl)methyl)-3,5-dimethylpiperazin-1-yl)-2-(2,6-dioxopiperidin-3-yl)isoindoline-1,3-dione | 5-(4-((1-(2-(4-(4-chloro-1,2-diphenylbut-1-en-1-yl)phenoxy)ethyl)piperidin-4-yl)methyl)-3,5-dimethylpiperazin-1-yl)-2-(2,6-dioxopiperidin-3-yl)isoindoline-1,3-dione |
| | 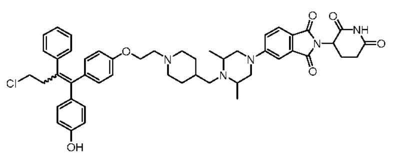 | 5-(4-((1-(2-(4-(4-chloro-1-(4-hydroxyphenyl)-2-phenylbut-1-en-1-yl)phenoxy)ethyl)piperidin-4-yl)methyl)-3,5-dimethylpiperazin-1-yl)-2-(2,6-dioxopiperidin-3-yl)isoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)-3,5-dimethylpiperazin-1-yl)-2-(2,6-dioxopiperidin-3-yl)isoindoline-1,3-dione |
| | 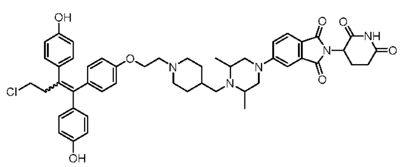 | 5-(4-((1-(2-(4-(4-chloro-1,2-bis(4-hydroxyphenyl)but-1-en-1-yl)phenoxy)ethyl)piperidin-4-yl)methyl)-3,5-dimethylpiperazin-1-yl)-2- | 5-(4-((1-(2-(4-(4-chloro-1,2-bis(4-hydroxyphenyl)but-1-en-1-yl)phenoxy)ethyl)piperidin-4-yl)methyl)-3,5-dimethylpiperazin-1-yl)-2- |

| | | | |
|---|---|---|---|
| | | (2,6-dioxopiperidin-3-yl)isoindoline-1,3-dione | (2,6-dioxopiperidin-3-yl)isoindoline-1,3-dione |
| | 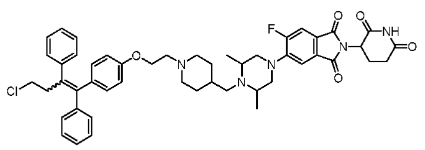 | 5-(4-((1-(2-(4-(4-chloro-1,2-diphenylbut-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione | 5-(4-((1-(2-(4-(4-chloro-1,2-diphenylbut-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione |
| | 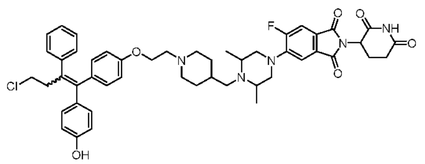 | 5-(4-((1-(2-(4-(4-chloro-1-(4-hydroxyphenyl)-2-phenylbut-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione | 5-(4-((1-(2-(4-(4-chloro-1-(4-hydroxyphenyl)-2-phenylbut-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione |
| | 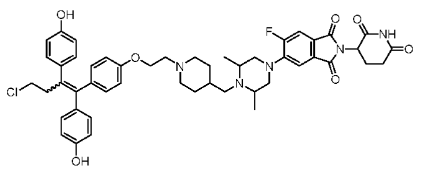 | 5-(4-((1-(2-(4-(4-chloro-1,2-bis(4-hydroxyphenyl)but-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione | 5-(4-((1-(2-(4-(4-chloro-1,2-bis(4-hydroxyphenyl)but-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione |
| | 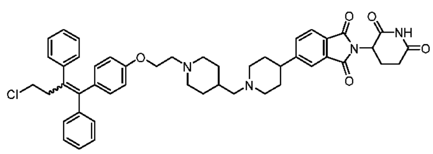 | 5-(1-((1-(2-(4-(4-chloro-1,2-diphenylbut-1-en-1-yl)phenoxy)ethyl)piperidin-4-yl)methyl)piperidin-4-yl)-2-(2,6-dioxopiperidin-3-yl)isoindoline-1,3-dione | 5-(1-((1-(2-(4-(4-chloro-1,2-diphenylbut-1-en-1-yl)phenoxy)ethyl)piperidin-4-yl)methyl)piperidin-4-yl)-2-(2,6-dioxopiperidin-3-yl)isoindoline-1,3-dione |
| | 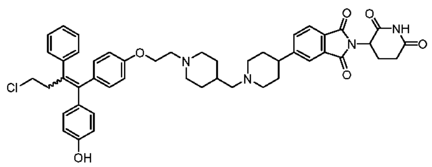 | 5-(1-((1-(2-(4-(4-chloro-1-(4-hydroxyphenyl)-2-phenylbut-1-en-1-yl)phenoxy)ethyl)piperidin-4-yl)methyl)piperidin-4-yl)-2-(2,6-dioxopiperidin-3-yl)isoindoline-1,3-dione | 5-(1-((1-(2-(4-(4-chloro-1-(4-hydroxyphenyl)-2-phenylbut-1-en-1-yl)phenoxy)ethyl)piperidin-4-yl)methyl)piperidin-4-yl)-2-(2,6-dioxopiperidin-3-yl)isoindoline-1,3-dione |
| | 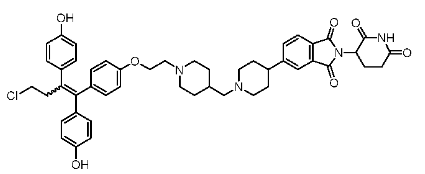 | 5-(1-((1-(2-(4-(4-chloro-1,2-bis(4-hydroxyphenyl)but-1-en-1-yl)phenoxy)ethyl)piperidin-4-yl)methyl)piperidin-4-yl)-2-(2,6-dioxopiperidin-3-yl)isoindoline-1,3-dione | 5-(1-((1-(2-(4-(4-chloro-1,2-bis(4-hydroxyphenyl)but-1-en-1-yl)phenoxy)ethyl)piperidin-4-yl)methyl)piperidin-4-yl)-2-(2,6-dioxopiperidin-3-yl)isoindoline-1,3-dione |
| | 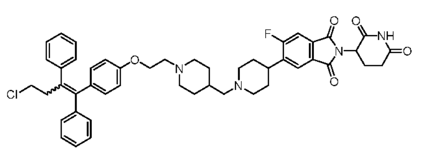 | 5-(1-((1-(2-(4-(4-chloro-1,2-diphenylbut-1-en-1-yl)phenoxy)ethyl)piperidin-4-yl)methyl)piperidin-4-yl)-2-(2,6-dioxopiperidin-3-yl)-6-fluoroisoindoline-1,3-dione | 5-(1-((1-(2-(4-(4-chloro-1,2-diphenylbut-1-en-1-yl)phenoxy)ethyl)piperidin-4-yl)methyl)piperidin-4-yl)-2-(2,6-dioxopiperidin-3-yl)-6-fluoroisoindoline-1,3-dione |

| | | | |
|---|---|---|---|
| | 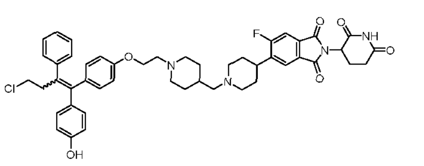 | 5-(1-((1-(2-(4-(4-chloro-1-(4-hydroxyphenyl)-2-phenylbut-1-en-1-yl)phenoxy)ethyl)piperidin-4-yl)methyl)piperidin-4-yl)-2-(2,6-dioxopiperidin-3-yl)-6-fluoroisoindoline-1,3-dione | 5-(1-((1-(2-(4-(4-chloro-1-(4-hydroxyphenyl)-2-phenylbut-1-en-1-yl)phenoxy)ethyl)piperidin-4-yl)methyl)piperidin-4-yl)-2-(2,6-dioxopiperidin-3-yl)-6-fluoroisoindoline-1,3-dione |
| | 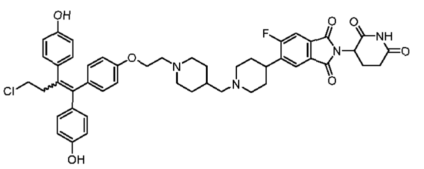 | 5-(1-((1-(2-(4-(4-chloro-1,2-bis(4-hydroxyphenyl)but-1-en-1-yl)phenoxy)ethyl)piperidin-4-yl)methyl)piperidin-4-yl)-2-(2,6-dioxopiperidin-3-yl)-6-fluoroisoindoline-1,3-dione | 5-(1-((1-(2-(4-(4-chloro-1,2-bis(4-hydroxyphenyl)but-1-en-1-yl)phenoxy)ethyl)piperidin-4-yl)methyl)piperidin-4-yl)-2-(2,6-dioxopiperidin-3-yl)-6-fluoroisoindoline-1,3-dione |
| | 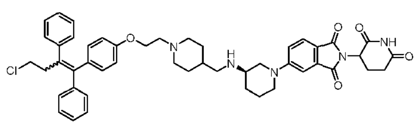 | 5-((R)-3-(((1-(2-(4-(4-chloro-1,2-diphenylbut-1-en-1-yl)phenoxy)ethyl)piperidin-4-yl)methyl)amino)piperidin-1-yl)-2-(2,6-dioxopiperidin-3-yl)isoindoline-1,3-dione | 5-((R)-3-(((1-(2-(4-(4-chloro-1,2-diphenylbut-1-en-1-yl)phenoxy)ethyl)piperidin-4-yl)methyl)amino)piperidin-1-yl)-2-(2,6-dioxopiperidin-3-yl)isoindoline-1,3-dione |
| | 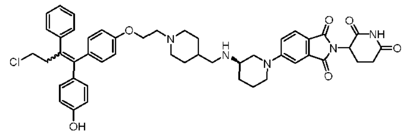 | 5-((R)-3-(((1-(2-(4-(4-chloro-1-(4-hydroxyphenyl)-2-phenylbut-1-en-1-yl)phenoxy)ethyl)piperidin-4-yl)methyl)amino)piperidin-1-yl)-2-(2,6-dioxopiperidin-3-yl)isoindoline-1,3-dione | 5-((R)-3-(((1-(2-(4-(4-chloro-1-(4-hydroxyphenyl)-2-phenylbut-1-en-1-yl)phenoxy)ethyl)piperidin-4-yl)methyl)amino)piperidin-1-yl)-2-(2,6-dioxopiperidin-3-yl)isoindoline-1,3-dione |
| | 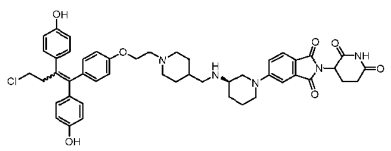 | 5-((R)-3-(((1-(2-(4-(4-chloro-1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione | 5-((R)-3-(((1-(2-(4-(4-chloro-1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione |
| | 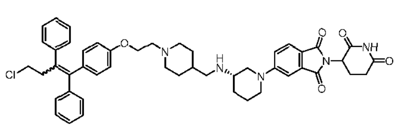 | 5-((S)-3-(((1-(2-(4-(4-chloro-1,2-diphenylbut-1-en-1-yl)phenoxy)ethyl)piperidin-4-yl)methyl)amino)piperidin-1-yl)-2-(2,6-dioxopiperidin-3-yl)isoindoline-1,3-dione | 5-((S)-3-(((1-(2-(4-(4-chloro-1,2-diphenylbut-1-en-1-yl)phenoxy)ethyl)piperidin-4-yl)methyl)amino)piperidin-1-yl)-2-(2,6-dioxopiperidin-3-yl)isoindoline-1,3-dione |
| | 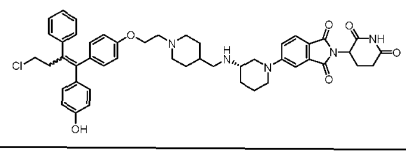 | 5-((S)-3-(((1-(2-(4-(4-chloro-1-(4-hydroxyphenyl)-2-phenylbut-1-en-1-yl)phenoxy)ethyl)piperidin-4- | 5-((S)-3-(((1-(2-(4-(4-chloro-1-(4-hydroxyphenyl)-2-phenylbut-1-en-1-yl)phenoxy)ethyl)piperidin |

| | | | |
|---|---|---|---|
| | | yl)methyl)amino)piperidin-1-yl)-2-(2,6-dioxopiperidin-3-yl)isoindoline-1,3-dione | -4-yl)methyl)amino)piperidin-1-yl)-2-(2,6-dioxopiperidin-3-yl)isoindoline-1,3-dione |
| | 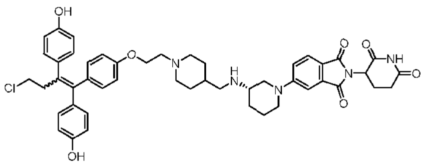 | 5-((S)-3-(((1-(2-(4-(4-chloro-1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione | 5-((S)-3-(((1-(2-(4-(4-chloro-1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione |
| | 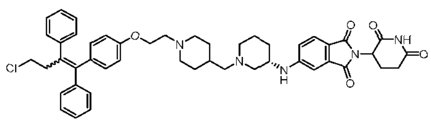 | 5-(((S)-1-((1-(2-(4-(4-chloro-1,2-diphenylbut-1-en-1-yl)phenoxy)ethyl)piperidin-4-yl)methyl)piperidin-3-yl)amino)-2-(2,6-dioxopiperidin-3-yl)isoindoline-1,3-dione | 5-(((S)-1-((1-(2-(4-(4-chloro-1,2-diphenylbut-1-en-1-yl)phenoxy)ethyl)piperidin-4-yl)methyl)piperidin-3-yl)amino)-2-(2,6-dioxopiperidin-3-yl)isoindoline-1,3-dione |
| | 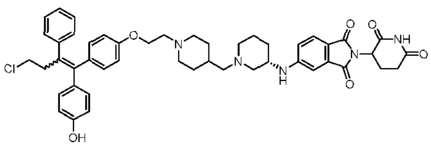 | 5-(((S)-1-((1-(2-(4-(4-chloro-1-(4-hydroxyphenyl)-2-phenylbut-1-en-1-yl)phenoxy)ethyl)piperidin-4-yl)methyl)piperidin-3-yl)amino)-2-(2,6-dioxopiperidin-3-yl)isoindoline-1,3-dione | 5-(((S)-1-((1-(2-(4-(4-chloro-1-(4-hydroxyphenyl)-2-phenylbut-1-en-1-yl)phenoxy)ethyl)piperidin-4-yl)methyl)piperidin-3-yl)amino)-2-(2,6-dioxopiperidin-3-yl)isoindoline-1,3-dione |
| | 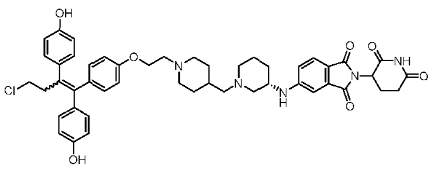 | 5-(((S)-1-((1-(2-(4-(4-chloro-1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione | 5-(((S)-1-((1-(2-(4-(4-chloro-1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione |
| | 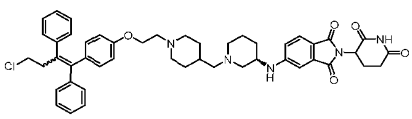 | 5-(((R)-1-((1-(2-(4-(4-chloro-1,2-diphenylbut-1-en-1-yl)phenoxy)ethyl)piperidin-4-yl)methyl)piperidin-3-yl)amino)-2-(2,6-dioxopiperidin-3-yl)isoindoline-1,3-dione | 5-(((R)-1-((1-(2-(4-(4-chloro-1,2-diphenylbut-1-en-1-yl)phenoxy)ethyl)piperidin-4-yl)methyl)piperidin-3-yl)amino)-2-(2,6-dioxopiperidin-3-yl)isoindoline-1,3-dione |
| | 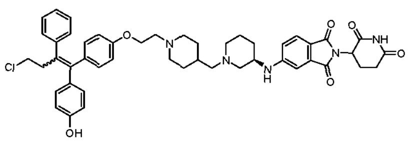 | 5-(((R)-1-((1-(2-(4-(4-chloro-1-(4-hydroxyphenyl)-2-phenylbut-1-en-1-yl)phenoxy)ethyl)piperidin-4-yl)methyl)piperidin-3-yl)amino)-2-(2,6-dioxopiperidin-3-yl)isoindoline-1,3-dione | 5-(((R)-1-((1-(2-(4-(4-chloro-1-(4-hydroxyphenyl)-2-phenylbut-1-en-1-yl)phenoxy)ethyl)piperidin-4-yl)methyl)piperidin-3-yl)amino)-2-(2,6-dioxopiperidin-3-yl)isoindoline-1,3-dione |

| | | | |
|---|---|---|---|
| | 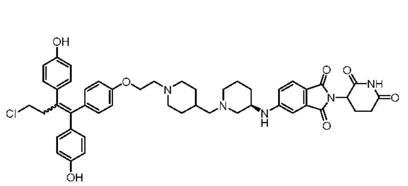 | 5-(((R)-1-((1-(2-(4-(4-chloro-1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione | 5-(((R)-1-((1-(2-(4-(4-chloro-1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione |
| | 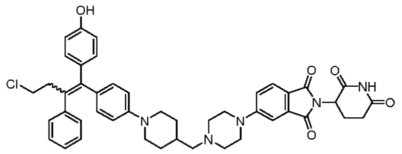 | 5-(4-((1-(4-(4-chloro-1-(4-hydroxyphenyl)-2-phenylbut-1-en-1-yl)phenyl)piperidin-4-yl)methyl)piperazin-1-yl)-2-(2,6-dioxopiperidin-3-yl)isoindoline-1,3-dione | 5-(4-((1-(4-(4-chloro-1-(4-hydroxyphenyl)-2-phenylbut-1-en-1-yl)phenyl)piperidin-4-yl)methyl)piperazin-1-yl)-2-(2,6-dioxopiperidin-3-yl)isoindoline-1,3-dione |
| | 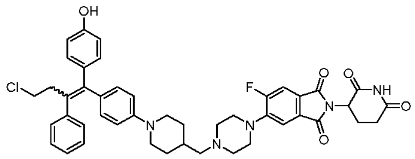 | 5-(4-((1-(4-(4-chloro-1-(4-hydroxyphenyl)-2-phenylbut-1-en-1-yl)phenyl)piperidin-4-yl)methyl)piperazin-1-yl)-2-(2,6-dioxopiperidin-3-yl)-6-fluoroisoindoline-1,3-dione | 5-(4-((1-(4-(4-chloro-1-(4-hydroxyphenyl)-2-phenylbut-1-en-1-yl)phenyl)piperidin-4-yl)methyl)piperazin-1-yl)-2-(2,6-dioxopiperidin-3-yl)-6-fluoroisoindoline-1,3-dione |
| | 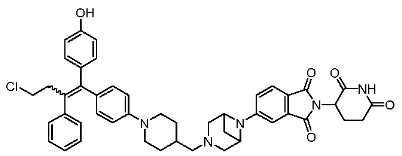 | 5-(3-((1-(4-(4-chloro-1-(4-hydroxyphenyl)-2-phenylbut-1-en-1-yl)phenyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(4-(4-chloro-1-(4-hydroxyphenyl)-2-phenylbut-1-en-1-yl)phenyl)piperidin-4-yl)methyl)-3,6-diazabicyclo[3.1.1]heptan-6-yl)-2-(2,6-dioxopiperidin-3-yl)isoindoline-1,3-dione |
| | 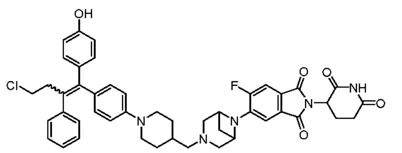 | 5-(3-((1-(4-(4-chloro-1-(4-hydroxyphenyl)-2-phenylbut-1-en-1-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(4-(4-chloro-1-(4-hydroxyphenyl)-2-phenylbut-1-en-1-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 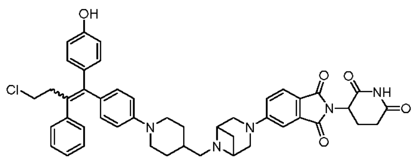 | 5-(6-((1-(4-(4-chloro-1-(4-hydroxyphenyl)-2-phenylbut-1-en-1-yl)phenyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(4-(4-chloro-1-(4-hydroxyphenyl)-2-phenylbut-1-en-1-yl)phenyl)piperidin-4-yl)methyl)-3,6-diazabicyclo[3.1.1]heptan-3-yl)-2-(2,6-dioxopiperidin-3-yl)isoindoline-1,3-dione |
| | 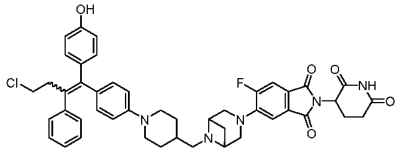 | 5-(6-((1-(4-(4-chloro-1-(4-hydroxyphenyl)-2-phenylbut-1-en-1-yl)phenyl)piperidin-4-yl)methyl)-3,6-diazabicyclo[3.1.1]heptan-3-yl)-2-(2,6-dioxopiperidin-3-yl)-6-fluoroisoindoline-1,3- | 5-(6-((1-(4-(4-chloro-1-(4-hydroxyphenyl)-2-phenylbut-1-en-1-yl)phenyl)piperidin-4-yl)methyl)-3,6-diazabicyclo[3.1.1]heptan-3-yl)-2-(2,6-dioxopiperidin-3-yl)-6- |

| | | | |
|---|---|---|---|
| | | dione | fluoroisoindoline-1,3-dione |
| | 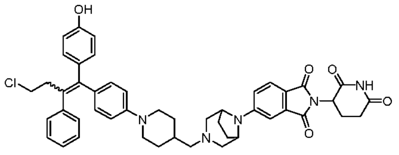 | 5-(3-((1-(4-(4-chloro-1-(4-hydroxyphenyl)-2-phenylbut-1-en-1-yl)phenyl)piperidin-4-yl)methyl)-3,8-diazabicyclo[3.2.1]octan-8-yl)-2-(2,6-dioxopiperidin-3-yl)isoindoline-1,3-dione | 5-(3-((1-(4-(4-chloro-1-(4-hydroxyphenyl)-2-phenylbut-1-en-1-yl)phenyl)piperidin-4-yl)methyl)-3,8-diazabicyclo[3.2.1]octan-8-yl)-2-(2,6-dioxopiperidin-3-yl)isoindoline-1,3-dione |
| | 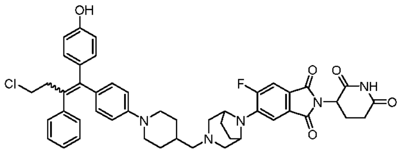 | 5-(3-((1-(4-(4-chloro-1-(4-hydroxyphenyl)-2-phenylbut-1-en-1-yl)phenyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(4-(4-chloro-1-(4-hydroxyphenyl)-2-phenylbut-1-en-1-yl)phenyl)piperidin-4-yl)methyl)-3,8-diazabicyclo[3.2.1]octan-8-yl)-2-(2,6-dioxopiperidin-3-yl)-6-fluoroisoindoline-1,3-dione |
| | 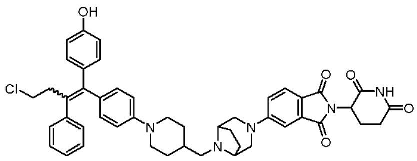 | 5-(8-((1-(4-(4-chloro-1-(4-hydroxyphenyl)-2-phenylbut-1-en-1-yl)phenyl)piperidin-4-yl)methyl)-3,8-diazabicyclo[3.2.1]octan-3-yl)-2-(2,6-dioxopiperidin-3-yl)isoindoline-1,3-dione | 5-(8-((1-(4-(4-chloro-1-(4-hydroxyphenyl)-2-phenylbut-1-en-1-yl)phenyl)piperidin-4-yl)methyl)-3,8-diazabicyclo[3.2.1]octan-3-yl)-2-(2,6-dioxopiperidin-3-yl)isoindoline-1,3-dione |
| | 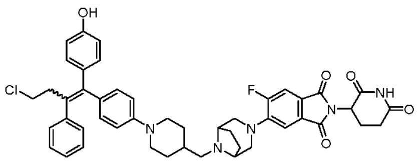 | 5-(8-((1-(4-(4-chloro-1-(4-hydroxyphenyl)-2-phenylbut-1-en-1-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(4-(4-chloro-1-(4-hydroxyphenyl)-2-phenylbut-1-en-1-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 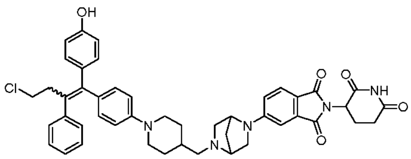 | 5-(5-((1-(4-(4-chloro-1-(4-hydroxyphenyl)-2-phenylbut-1-en-1-yl)phenyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(4-(4-chloro-1-(4-hydroxyphenyl)-2-phenylbut-1-en-1-yl)phenyl)piperidin-4-yl)methyl)-2,5-diazabicyclo[2.2.1]heptan-2-yl)-2-(2,6-dioxopiperidin-3-yl)isoindoline-1,3-dione |
| | 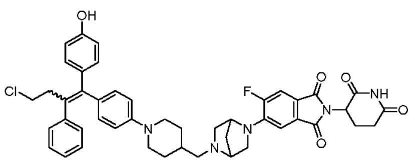 | 5-(5-((1-(4-(4-chloro-1-(4-hydroxyphenyl)-2-phenylbut-1-en-1-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(4-(4-chloro-1-(4-hydroxyphenyl)-2-phenylbut-1-en-1-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 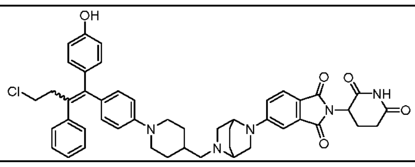 | 5-(5-((1-(4-(4-chloro-1-(4-hydroxyphenyl)-2-phenylbut-1-en-1-yl)phenyl)piperidin-4-yl)methyl)-2,5- | 5-(5-((1-(4-(4-chloro-1-(4-hydroxyphenyl)-2-phenylbut-1-en-1-yl)phenyl)piperidin-4-yl)methyl)-2,5- |

| | | | |
|---|---|---|---|
| | | diazabicyclo[2.2.2]octan-2-yl)-2-(2,6-dioxopiperidin-3-yl)isoindoline-1,3-dione | diazabicyclo[2.2.2]octan-2-yl)-2-(2,6-dioxopiperidin-3-yl)isoindoline-1,3-dione |
| | 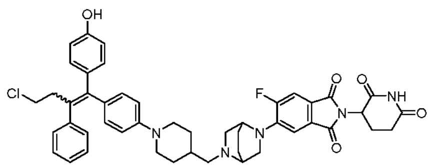 | 5-(5-((1-(4-(4-chloro-1-(4-hydroxyphenyl)-2-phenylbut-1-en-1-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(4-(4-chloro-1-(4-hydroxyphenyl)-2-phenylbut-1-en-1-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 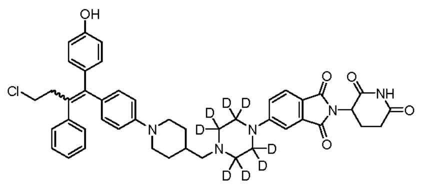 | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)isoindoline-1,3-dione | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)isoindoline-1,3-dione |
| | 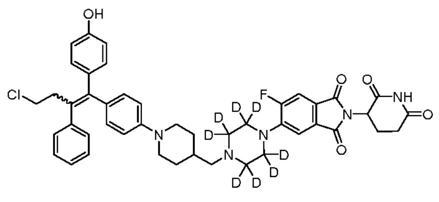 | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)-6-fluoroisoindoline-1,3-dione | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)-6-fluoroisoindoline-1,3-dione |
| | 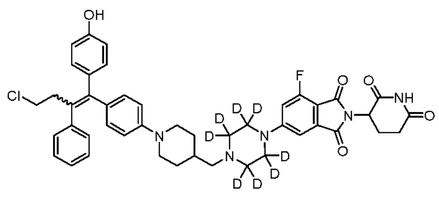 | 6-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)-4-fluoroisoindoline-1,3-dione | 6-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)-4-fluoroisoindoline-1,3-dione |
| | 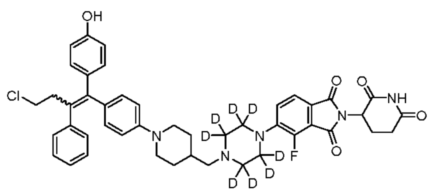 | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)-4-fluoroisoindoline-1,3-dione | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)-4-fluoroisoindoline-1,3-dione |
| | 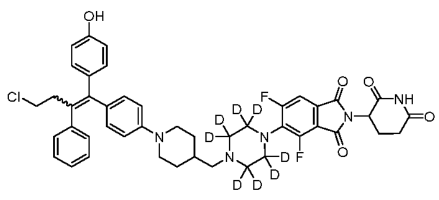 | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)-4,6-difluoroisoindoline-1,3-dione | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-*d*$_8$)-2-(2,6-dioxopiperidin-3-yl)-4,6-difluoroisoindoline-1,3-dione |

| | | | |
|---|---|---|---|
| | 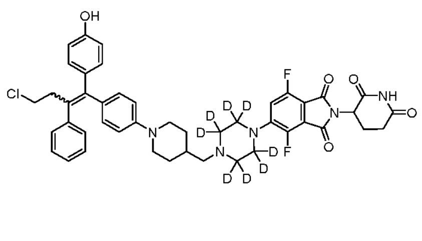 | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione |
| | 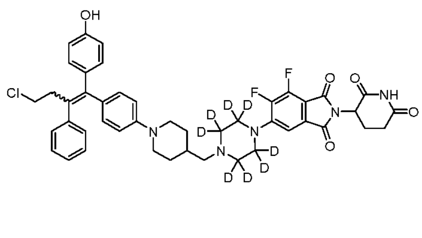 | 6-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione | 6-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione |
| | 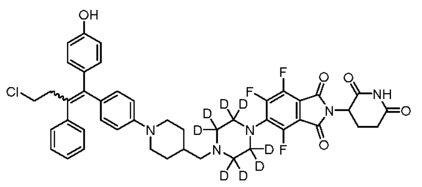 | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |
| | 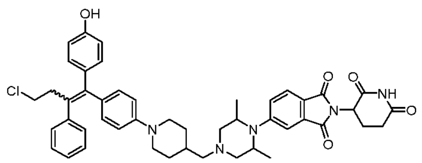 | 5-(4-((1-(4-(4-chloro-1-(4-hydroxyphenyl)-2-phenylbut-1-en-1-yl)phenyl)piperidin-4-yl)methyl)-2,6-dimethylpiperazin-1-yl)-2-(2,6-dioxopiperidin-3-yl)isoindoline-1,3-dione | 5-(4-((1-(4-(4-chloro-1-(4-hydroxyphenyl)-2-phenylbut-1-en-1-yl)phenyl)piperidin-4-yl)methyl)-2,6-dimethylpiperazin-1-yl)-2-(2,6-dioxopiperidin-3-yl)isoindoline-1,3-dione |
| | 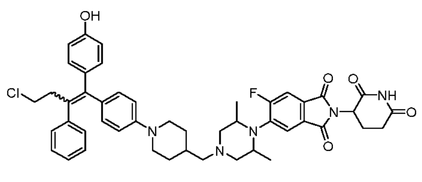 | 5-(4-((1-(4-(4-chloro-1-(4-hydroxyphenyl)-2-phenylbut-1-en-1-yl)phenyl)piperidin-4-yl)methyl)-2,6-dimethylpiperazin-1-yl)-2-(2,6-dioxopiperidin-3-yl)-6-fluoroisoindoline-1,3-dione | 5-(4-((1-(4-(4-chloro-1-(4-hydroxyphenyl)-2-phenylbut-1-en-1-yl)phenyl)piperidin-4-yl)methyl)-2,6-dimethylpiperazin-1-yl)-2-(2,6-dioxopiperidin-3-yl)-6-fluoroisoindoline-1,3-dione |
| | 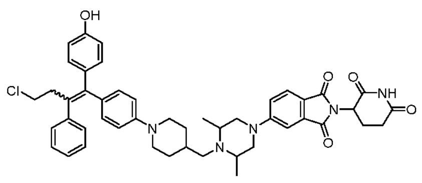 | 5-(4-((1-(4-(4-chloro-1-(4-hydroxyphenyl)-2-phenylbut-1-en-1-yl)phenyl)piperidin-4-yl)methyl)-3,5-dimethylpiperazin-1-yl)-2-(2,6-dioxopiperidin-3-yl)isoindoline-1,3-dione | 5-(4-((1-(4-(4-chloro-1-(4-hydroxyphenyl)-2-phenylbut-1-en-1-yl)phenyl)piperidin-4-yl)methyl)-3,5-dimethylpiperazin-1-yl)-2-(2,6-dioxopiperidin-3-yl)isoindoline-1,3-dione |
| | 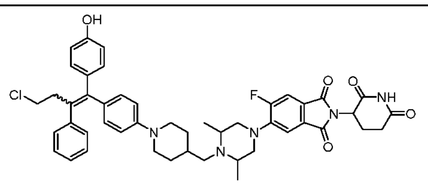 | 5-(4-((1-(4-(4-chloro-1-(4-hydroxyphenyl)-2-phenylbut-1-en-1-yl)phenyl)piperidin-4-yl)methyl)-3,5-dimethylpiperazin-1-yl)-2- | 5-(4-((1-(4-(4-chloro-1-(4-hydroxyphenyl)-2-phenylbut-1-en-1-yl)phenyl)piperidin-4-yl)methyl)-3,5-dimethylpiperazin-1-yl)-2- |

| | | | |
|---|---|---|---|
| | | (2,6-dioxopiperidin-3-yl)-6-fluoroisoindoline-1,3-dione | (2,6-dioxopiperidin-3-yl)-6-fluoroisoindoline-1,3-dione |
| | 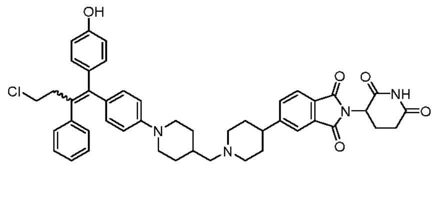 | 5-(1-((1-(4-(4-chloro-1-(4-hydroxyphenyl)-2-phenylbut-1-en-1-yl)phenyl)piperidin-4-yl)methyl)piperidin-4-yl)-2-(2,6-dioxopiperidin-3-yl)isoindoline-1,3-dione | 5-(1-((1-(4-(4-chloro-1-(4-hydroxyphenyl)-2-phenylbut-1-en-1-yl)phenyl)piperidin-4-yl)methyl)piperidin-4-yl)-2-(2,6-dioxopiperidin-3-yl)isoindoline-1,3-dione |
| | 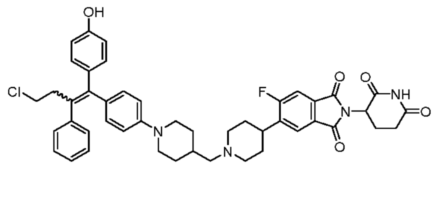 | 5-(1-((1-(4-(4-chloro-1-(4-hydroxyphenyl)-2-phenylbut-1-en-1-yl)phenyl)piperidin-4-yl)methyl)piperidin-4-yl)-2-(2,6-dioxopiperidin-3-yl)-6-fluoroisoindoline-1,3-dione | 5-(1-((1-(4-(4-chloro-1-(4-hydroxyphenyl)-2-phenylbut-1-en-1-yl)phenyl)piperidin-4-yl)methyl)piperidin-4-yl)-2-(2,6-dioxopiperidin-3-yl)-6-fluoroisoindoline-1,3-dione |
| | 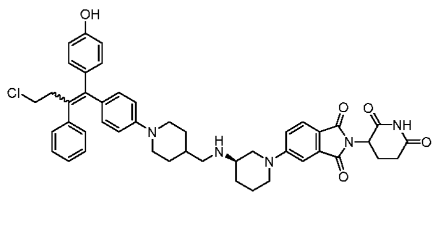 | 5-((R)-3-(((1-(4-(4-chloro-1-(4-hydroxyphenyl)-2-phenylbut-1-en-1-yl)phenyl)piperidin-4-yl)methyl)amino)piperidin-1-yl)-2-(2,6-dioxopiperidin-3-yl)isoindoline-1,3-dione | 5-((R)-3-(((1-(4-(4-chloro-1-(4-hydroxyphenyl)-2-phenylbut-1-en-1-yl)phenyl)piperidin-4-yl)methyl)amino)piperidin-1-yl)-2-(2,6-dioxopiperidin-3-yl)isoindoline-1,3-dione |
| | 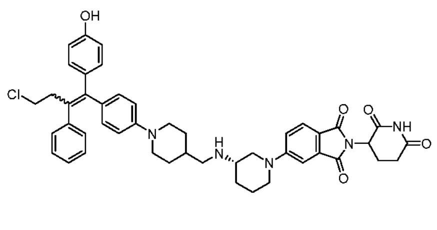 | 5-((S)-3-(((1-(4-(4-chloro-1-(4-hydroxyphenyl)-2-phenylbut-1-en-1-yl)phenyl)piperidin-4-yl)methyl)amino)piperidin-1-yl)-2-(2,6-dioxopiperidin-3-yl)isoindoline-1,3-dione | 5-((S)-3-(((1-(4-(4-chloro-1-(4-hydroxyphenyl)-2-phenylbut-1-en-1-yl)phenyl)piperidin-4-yl)methyl)amino)piperidin-1-yl)-2-(2,6-dioxopiperidin-3-yl)isoindoline-1,3-dione |
| | 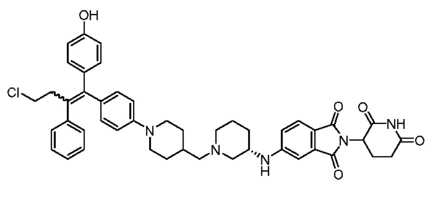 | 5-(((S)-1-((1-(4-(4-chloro-1-(4-hydroxyphenyl)-2-phenylbut-1-en-1-yl)phenyl)piperidin-4-yl)methyl)piperidin-3-yl)amino)-2-(2,6-dioxopiperidin-3-yl)isoindoline-1,3-dione | 5-(((S)-1-((1-(4-(4-chloro-1-(4-hydroxyphenyl)-2-phenylbut-1-en-1-yl)phenyl)piperidin-4-yl)methyl)piperidin-3-yl)amino)-2-(2,6-dioxopiperidin-3-yl)isoindoline-1,3-dione |
| | 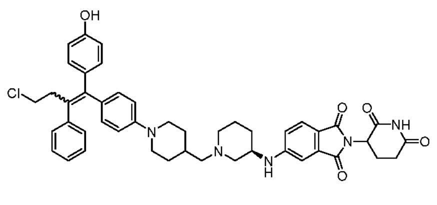 | 5-(((R)-1-((1-(4-(4-chloro-1-(4-hydroxyphenyl)-2-phenylbut-1-en-1-yl)phenyl)piperidin-4-yl)methyl)piperidin-3-yl)amino)-2-(2,6-dioxopiperidin-3-yl)isoindoline-1,3-dione | 5-(((R)-1-((1-(4-(4-chloro-1-(4-hydroxyphenyl)-2-phenylbut-1-en-1-yl)phenyl)piperidin-4-yl)methyl)piperidin-3-yl)amino)-2-(2,6-dioxopiperidin-3-yl)isoindoline-1,3-dione |
| | 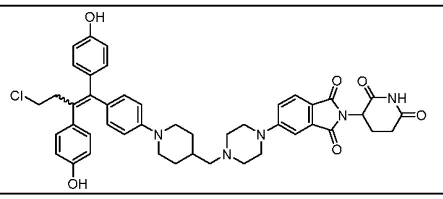 | 5-(4-((1-(4-(4-chloro-1,2-bis(4-hydroxyphenyl)but-1-en-1-yl)phenyl)piperidin-4-yl)methyl)piperazin-1-yl)-2-(2,6-dioxopiperidin-3-yl)isoindoline-1,3-dione | 5-(4-((1-(4-(4-chloro-1,2-bis(4-hydroxyphenyl)but-1-en-1-yl)phenyl)piperidin-4-yl)methyl)piperazin-1-yl)-2-(2,6-dioxopiperidin-3-yl)isoindoline-1,3-dione |

| | | | |
|---|---|---|---|
| | 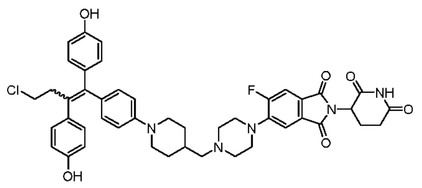 | 5-(4-((1-(4-(4-chloro-1,2-bis(4-hydroxyphenyl)but-1-en-1-yl)phenyl)piperidin-4-yl)methyl)piperazin-1-yl)-2-(2,6-dioxopiperidin-3-yl)-6-fluoroisoindoline-1,3-dione | 5-(4-((1-(4-(4-chloro-1,2-bis(4-hydroxyphenyl)but-1-en-1-yl)phenyl)piperidin-4-yl)methyl)piperazin-1-yl)-2-(2,6-dioxopiperidin-3-yl)-6-fluoroisoindoline-1,3-dione |
| | 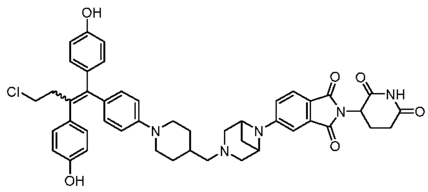 | 5-(3-((1-(4-(4-chloro-1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(4-(4-chloro-1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)isoindoline-1,3-dione |
| | 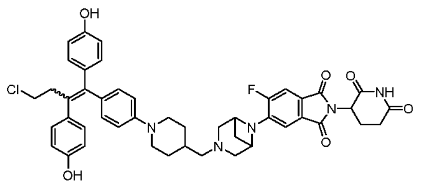 | 5-(3-((1-(4-(4-chloro-1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(4-(4-chloro-1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 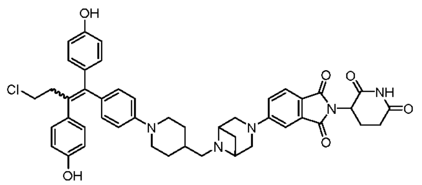 | 5-(6-((1-(4-(4-chloro-1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(4-(4-chloro-1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)isoindoline-1,3-dione |
| | 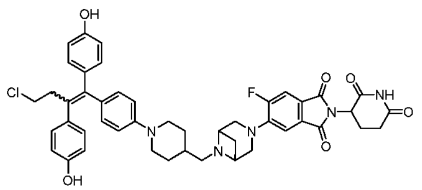 | 5-(6-((1-(4-(4-chloro-1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)-6-fluoroisoindoline-1,3-dione | 5-(6-((1-(4-(4-chloro-1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)-6-fluoroisoindoline-1,3-dione |
| | 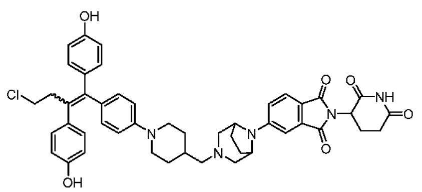 | 5-(3-((1-(4-(4-chloro-1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)isoindoline-1,3-dione | 5-(3-((1-(4-(4-chloro-1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)isoindoline-1,3-dione |
| | 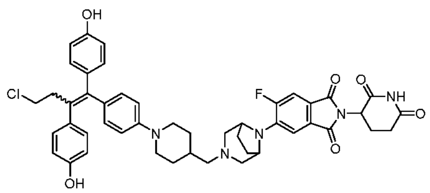 | 5-(3-((1-(4-(4-chloro-1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(4-(4-chloro-1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)-6-fluoroisoindoline-1,3-dione |

| | | | |
|---|---|---|---|
| | 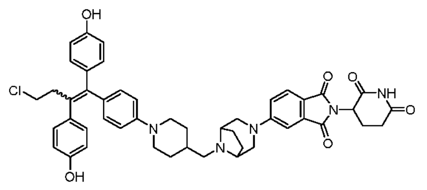 | 5-(8-((1-(4-(4-chloro-1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)isoindoline-1,3-dione | 5-(8-((1-(4-(4-chloro-1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)isoindoline-1,3-dione |
| | 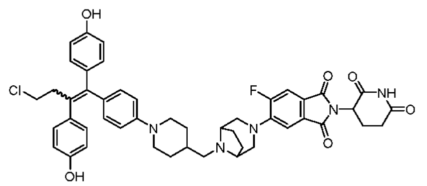 | 5-(8-((1-(4-(4-chloro-1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(4-(4-chloro-1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 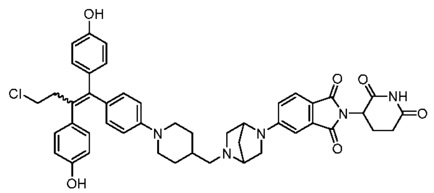 | 5-(5-((1-(4-(4-chloro-1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(4-(4-chloro-1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)isoindoline-1,3-dione |
| | 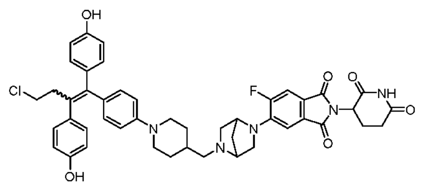 | 5-(5-((1-(4-(4-chloro-1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(4-(4-chloro-1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 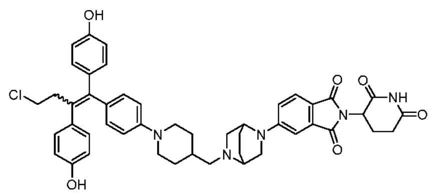 | 5-(5-((1-(4-(4-chloro-1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)isoindoline-1,3-dione | 5-(5-((1-(4-(4-chloro-1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)isoindoline-1,3-dione |
| | 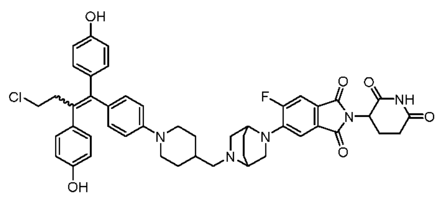 | 5-(5-((1-(4-(4-chloro-1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(4-(4-chloro-1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 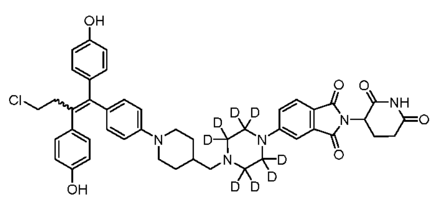 | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)isoindoline-1,3-dione | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)isoindoline-1,3-dione |

| | | | |
|---|---|---|---|
| | 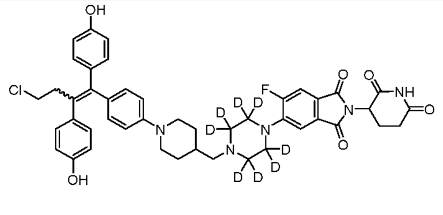 | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione |
| | 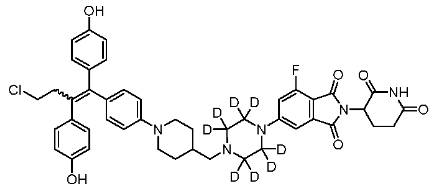 | 6-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 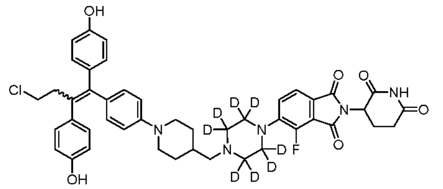 | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 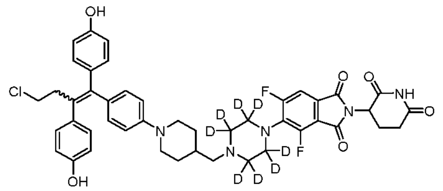 | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |
| | 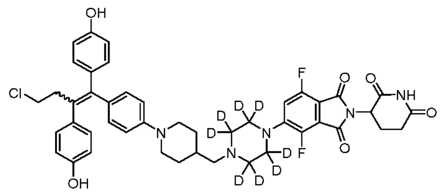 | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione |
| | 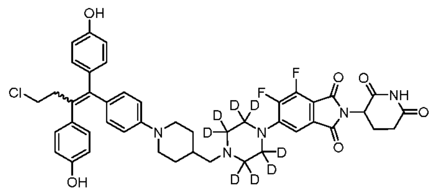 | 6-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione | 6-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione |
| | 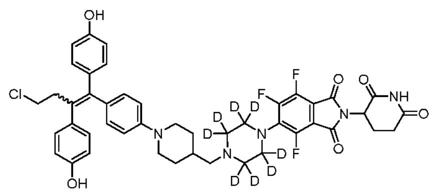 | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |

| | | | |
|---|---|---|---|
| | 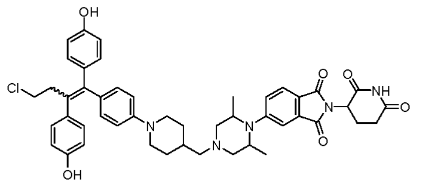 | 5-(4-((1-(4-(4-chloro-1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)isoindoline-1,3-dione | 5-(4-((1-(4-(4-chloro-1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)isoindoline-1,3-dione |
| | 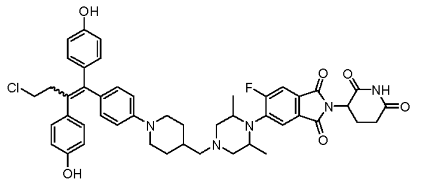 | 5-(4-((1-(4-(4-chloro-1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)-6-fluoroisoindoline-1,3-dione | 5-(4-((1-(4-(4-chloro-1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)-6-fluoroisoindoline-1,3-dione |
| | 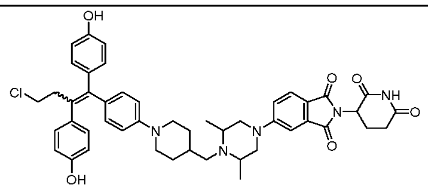 | 5-(4-((1-(4-(4-chloro-1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)isoindoline-1,3-dione | 5-(4-((1-(4-(4-chloro-1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)isoindoline-1,3-dione |
| | 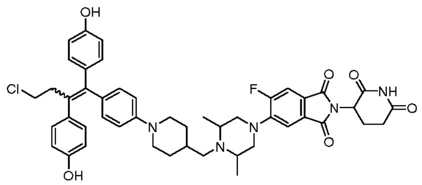 | 5-(4-((1-(4-(4-chloro-1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)-6-fluoroisoindoline-1,3-dione | 5-(4-((1-(4-(4-chloro-1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)-6-fluoroisoindoline-1,3-dione |
| | 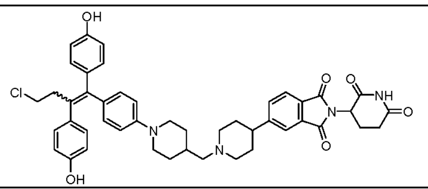 | 5-(1-((1-(4-(4-chloro-1,2-bis(4-hydroxyphenyl)but-1-en-1-yl)phenyl)piperidin-4-yl)methyl)piperidin-4-yl)-2-(2,6-dioxopiperidin-3-yl)isoindoline-1,3-dione | 5-(1-((1-(4-(4-chloro-1,2-bis(4-hydroxyphenyl)but-1-en-1-yl)phenyl)piperidin-4-yl)methyl)piperidin-4-yl)-2-(2,6-dioxopiperidin-3-yl)isoindoline-1,3-dione |
| | 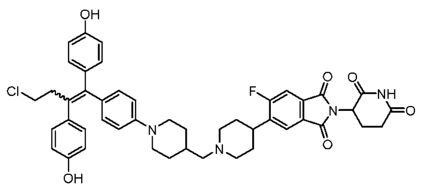 | 5-(1-((1-(4-(4-chloro-1,2-bis(4-hydroxyphenyl)but-1-en-1-yl)phenyl)piperidin-4-yl)methyl)piperidin-4-yl)-2-(2,6-dioxopiperidin-3-yl)-6-fluoroisoindoline-1,3-dione | 5-(1-((1-(4-(4-chloro-1,2-bis(4-hydroxyphenyl)but-1-en-1-yl)phenyl)piperidin-4-yl)methyl)piperidin-4-yl)-2-(2,6-dioxopiperidin-3-yl)-6-fluoroisoindoline-1,3-dione |
| | 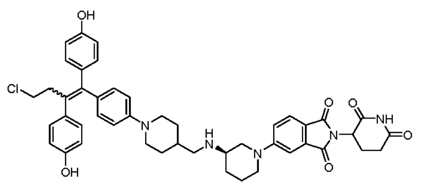 | 5-((R)-3-(((1-(4-(4-chloro-1,2-bis(4-hydroxyphenyl)but-1-en-1-yl)phenyl)piperidin-4-yl)methyl)amino)piperidin-1-yl)-2-(2,6-dioxopiperidin-3-yl)isoindoline-1,3-dione | 5-((R)-3-(((1-(4-(4-chloro-1,2-bis(4-hydroxyphenyl)but-1-en-1-yl)phenyl)piperidin-4-yl)methyl)amino)piperidin-1-yl)-2-(2,6-dioxopiperidin-3-yl)isoindoline-1,3-dione |
| | 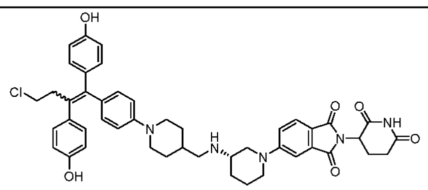 | 5-((S)-3-(((1-(4-(4-chloro-1,2-bis(4-hydroxyphenyl)but-1-en-1-yl)phenyl)piperidin-4-yl)methyl)amino)piperidin-1-yl)-2-(2,6-dioxopiperidin-3-yl)isoindoline-1,3-dione | 5-((S)-3-(((1-(4-(4-chloro-1,2-bis(4-hydroxyphenyl)but-1-en-1-yl)phenyl)piperidin-4-yl)methyl)amino)piperidin-1-yl)-2-(2,6-dioxopiperidin-3- |

| | | | |
|---|---|---|---|
| | | | yl)isoindoline-1,3-dione |
| | 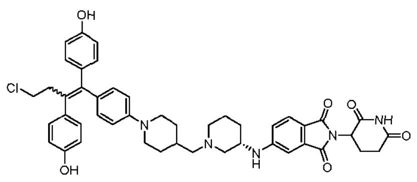 | 5-(((S)-1-((1-(4-(4-chloro-1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione | 5-(((S)-1-((1-(4-(4-chloro-1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione |
| | 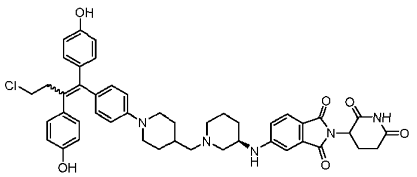 | 5-(((R)-1-((1-(4-(4-chloro-1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione | 5-(((R)-1-((1-(4-(4-chloro-1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione |
| | 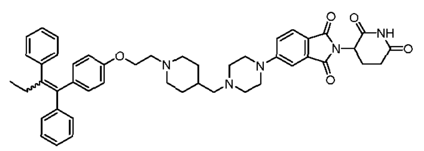 | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl)isoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl)isoindoline-1,3-dione |
| | 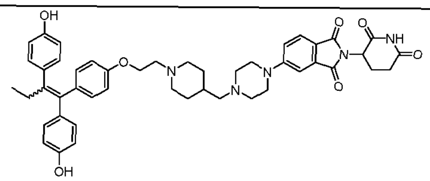 | 5-(4-((1-(2-(4-(1,2-bis(4-hydroxyphenyl)but-1-en-1-yl)phenoxy)ethyl)piperidin-4-yl)methyl)piperazin-1-yl)-2-(2,6-dioxopiperidin-3-yl)isoindoline-1,3-dione | 5-(4-((1-(2-(4-(1,2-bis(4-hydroxyphenyl)but-1-en-1-yl)phenoxy)ethyl)piperidin-4-yl)methyl)piperazin-1-yl)-2-(2,6-dioxopiperidin-3-yl)isoindoline-1,3-dione |
| | 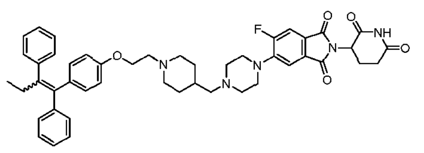 | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl)-6-fluoroisoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl)-6-fluoroisoindoline-1,3-dione |
| | 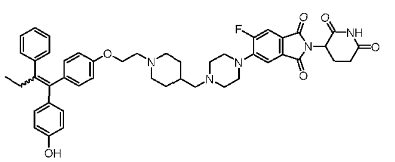 | 2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)piperazin-1-yl)isoindoline-1,3-dione | 2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)piperazin-1-yl)isoindoline-1,3-dione |
| | 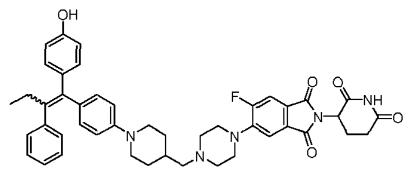 | 2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)piperazin-1-yl)isoindoline-1,3-dione | 2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)piperazin-1-yl)isoindoline-1,3-dione |
| | 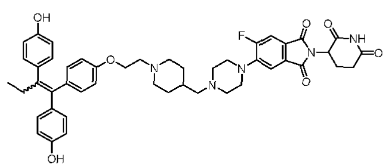 | 5-(4-((1-(2-(4-(1,2-bis(4-hydroxyphenyl)but-1-en-1-yl)phenoxy)ethyl)piperidin-4-yl)methyl)piperazin-1-yl)-2-(2,6-dioxopiperidin-3-yl)-6-fluoroisoindoline-1,3-dione | 5-(4-((1-(2-(4-(1,2-bis(4-hydroxyphenyl)but-1-en-1-yl)phenoxy)ethyl)piperidin-4-yl)methyl)piperazin-1-yl)-2-(2,6-dioxopiperidin-3-yl)-6-fluoroisoindoline-1,3-dione |

| | | | |
|---|---|---|---|
| | 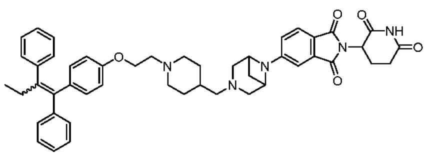 | 2-(2,6-dioxopiperidin-3-yl)-5-(3-((1-(2-(4-(1,2-diphenylbut-1-en-1-yl)phenoxy)ethyl)piperidin-4-yl)methyl)-3,6-diazabicyclo[3.1.1]heptan-6-yl)isoindoline-1,3-dione | 2-(2,6-dioxopiperidin-3-yl)-5-(3-((1-(2-(4-(1,2-diphenylbut-1-en-1-yl)phenoxy)ethyl)piperidin-4-yl)methyl)-3,6-diazabicyclo[3.1.1]heptan-6-yl)isoindoline-1,3-dione |
| | 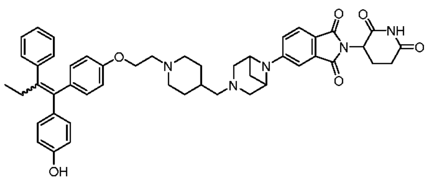 | 2-(2,6-dioxopiperidin-3-yl)-5-(3-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-6-yl)isoindoline-1,3-dione | 2-(2,6-dioxopiperidin-3-yl)-5-(3-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-6-yl)isoindoline-1,3-dione |
| | 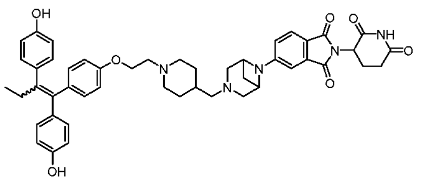 | 5-(3-((1-(2-(4-(1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(2-(4-(1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione |
| | 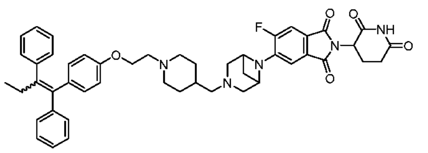 | 2-(2,6-dioxopiperidin-3-yl)-5-(3-((1-(2-(4-(1,2-diphenylbut-1-en-1-yl)phenoxy)ethyl)piperidin-4-yl)methyl)-3,6-diazabicyclo[3.1.1]heptan-6-yl)-6-fluoroisoindoline-1,3-dione | 2-(2,6-dioxopiperidin-3-yl)-5-(3-((1-(2-(4-(1,2-diphenylbut-1-en-1-yl)phenoxy)ethyl)piperidin-4-yl)methyl)-3,6-diazabicyclo[3.1.1]heptan-6-yl)-6-fluoroisoindoline-1,3-dione |
| | 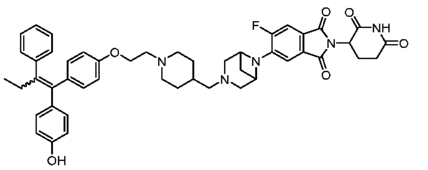 | 2-(2,6-dioxopiperidin-3-yl)-5-fluoro-6-(3-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-6-yl)isoindoline-1,3-dione | 2-(2,6-dioxopiperidin-3-yl)-5-fluoro-6-(3-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-6-yl)isoindoline-1,3-dione |
| | 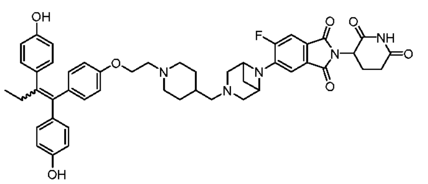 | 5-(3-((1-(2-(4-(1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(2-(4-(1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 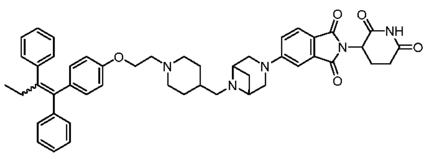 | 2-(2,6-dioxopiperidin-3-yl)-5-(6-((1-(2-(4-(1,2-diphenylbut-1-en-1-yl)phenoxy)ethyl)piperidin-4-yl)methyl)-3,6-diazabicyclo[3.1.1]heptan-3-yl)isoindoline-1,3-dione | 2-(2,6-dioxopiperidin-3-yl)-5-(6-((1-(2-(4-(1,2-diphenylbut-1-en-1-yl)phenoxy)ethyl)piperidin-4-yl)methyl)-3,6-diazabicyclo[3.1.1]heptan-3-yl)isoindoline-1,3-dione |

| | | | |
|---|---|---|---|
| | 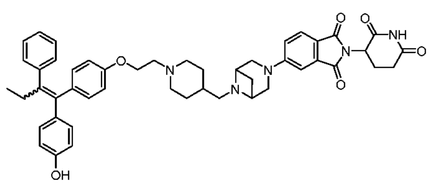 | 2-(2,6-dioxopiperidin-3-yl)-5-(6-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-3-yl)isoindoline-1,3-dione | 2-(2,6-dioxopiperidin-3-yl)-5-(6-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-3-yl)isoindoline-1,3-dione |
| | 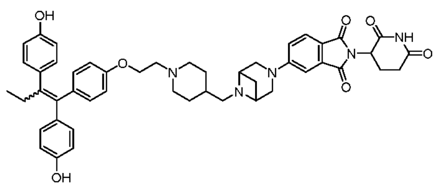 | 5-(6-((1-(2-(4-(1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(2-(4-(1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione |
| | 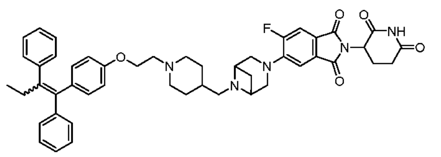 | 2-(2,6-dioxopiperidin-3-yl)-5-(6-((1-(2-(4-(1,2-diphenylbut-1-en-1-yl)phenoxy)ethyl)piperidin-4-yl)methyl)-3,6-diazabicyclo[3.1.1]heptan-3-yl)-6-fluoroisoindoline-1,3-dione | 2-(2,6-dioxopiperidin-3-yl)-5-(6-((1-(2-(4-(1,2-diphenylbut-1-en-1-yl)phenoxy)ethyl)piperidin-4-yl)methyl)-3,6-diazabicyclo[3.1.1]heptan-3-yl)-6-fluoroisoindoline-1,3-dione |
| | 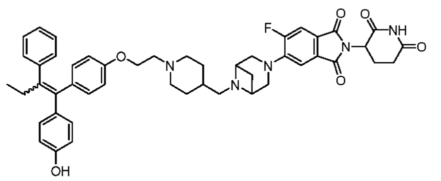 | 2-(2,6-dioxopiperidin-3-yl)-5-fluoro-6-(6-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-3-yl)isoindoline-1,3-dione | 2-(2,6-dioxopiperidin-3-yl)-5-fluoro-6-(6-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-3-yl)isoindoline-1,3-dione |
| | 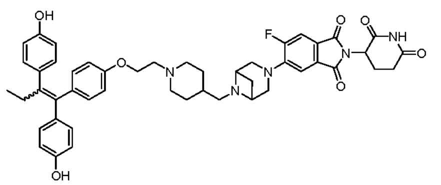 | 5-(6-((1-(2-(4-(1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione | 5-(6-((1-(2-(4-(1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione |
| | 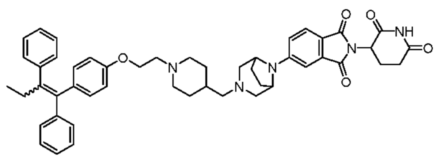 | 2-(2,6-dioxopiperidin-3-yl)-5-(3-((1-(2-(4-(1,2-diphenylbut-1-en-1-yl)phenoxy)ethyl)piperidin-4-yl)methyl)-3,8-diazabicyclo[3.2.1]octan-8-yl)isoindoline-1,3-dione | 2-(2,6-dioxopiperidin-3-yl)-5-(3-((1-(2-(4-(1,2-diphenylbut-1-en-1-yl)phenoxy)ethyl)piperidin-4-yl)methyl)-3,8-diazabicyclo[3.2.1]octan-8-yl)isoindoline-1,3-dione |
| | 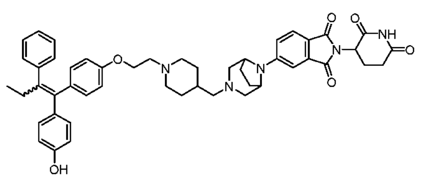 | 2-(2,6-dioxopiperidin-3-yl)-5-(3-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-8-yl)isoindoline-1,3-dione | 2-(2,6-dioxopiperidin-3-yl)-5-(3-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-8-yl)isoindoline-1,3-dione |

| | | | |
|---|---|---|---|
| | 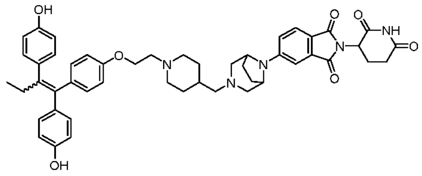 | 5-(3-((1-(2-(4-(1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione | 5-(3-((1-(2-(4-(1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione |
| | 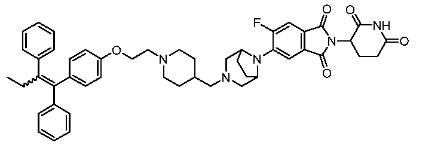 | 2-(2,6-dioxopiperidin-3-yl)-5-(3-((1-(2-(4-(1,2-diphenylbut-1-en-1-yl)phenoxy)ethyl)piperidin-4-yl)methyl)-3,8-diazabicyclo[3.2.1]octan-8-yl)-6-fluoroisoindoline-1,3-dione | 2-(2,6-dioxopiperidin-3-yl)-5-(3-((1-(2-(4-(1,2-diphenylbut-1-en-1-yl)phenoxy)ethyl)piperidin-4-yl)methyl)-3,8-diazabicyclo[3.2.1]octan-8-yl)-6-fluoroisoindoline-1,3-dione |
| | 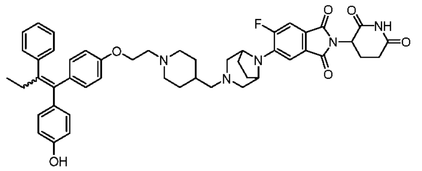 | 2-(2,6-dioxopiperidin-3-yl)-5-fluoro-6-(3-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-8-yl)isoindoline-1,3-dione | 2-(2,6-dioxopiperidin-3-yl)-5-fluoro-6-(3-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-8-yl)isoindoline-1,3-dione |
| | 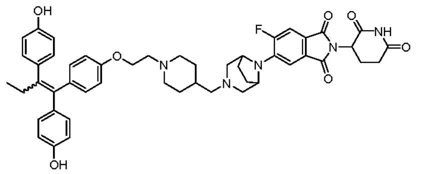 | 5-(3-((1-(2-(4-(1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(2-(4-(1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione |
| | 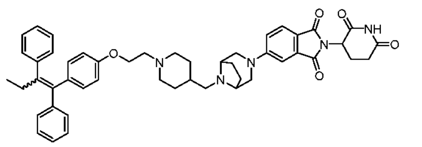 | 2-(2,6-dioxopiperidin-3-yl)-5-(8-((1-(2-(4-(1,2-diphenylbut-1-en-1-yl)phenoxy)ethyl)piperidin-4-yl)methyl)-3,8-diazabicyclo[3.2.1]octan-3-yl)isoindoline-1,3-dione | 2-(2,6-dioxopiperidin-3-yl)-5-(8-((1-(2-(4-(1,2-diphenylbut-1-en-1-yl)phenoxy)ethyl)piperidin-4-yl)methyl)-3,8-diazabicyclo[3.2.1]octan-3-yl)isoindoline-1,3-dione |
| | 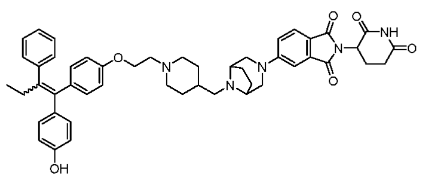 | 2-(2,6-dioxopiperidin-3-yl)-5-(8-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-3-yl)isoindoline-1,3-dione | 2-(2,6-dioxopiperidin-3-yl)-5-(8-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-3-yl)isoindoline-1,3-dione |
| | 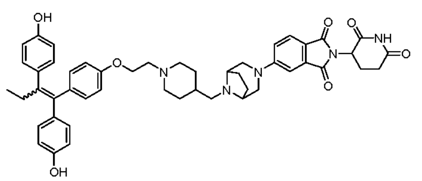 | 5-(8-((1-(2-(4-(1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione | 5-(8-((1-(2-(4-(1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione |
| | 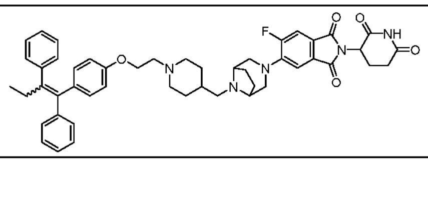 | 2-(2,6-dioxopiperidin-3-yl)-5-(8-((1-(2-(4-(1,2-diphenylbut-1-en-1-yl)phenoxy)ethyl)piperidin-4-yl)methyl)-3,8- | 2-(2,6-dioxopiperidin-3-yl)-5-(8-((1-(2-(4-(1,2-diphenylbut-1-en-1-yl)phenoxy)ethyl)piperidin-4-yl)methyl)-3,8- |

| | | | |
|---|---|---|---|
| | | diazabicyclo[3.2.1]octan-3-yl)-6-fluoroisoindoline-1,3-dione | diazabicyclo[3.2.1]octan-3-yl)-6-fluoroisoindoline-1,3-dione |
| | 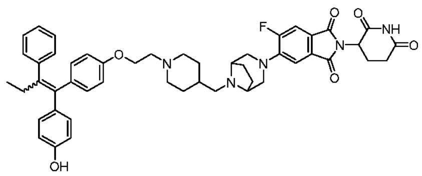 | 2-(2,6-dioxopiperidin-3-yl)-5-fluoro-6-(8-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-3-yl)isoindoline-1,3-dione | 2-(2,6-dioxopiperidin-3-yl)-5-fluoro-6-(8-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-3-yl)isoindoline-1,3-dione |
| | 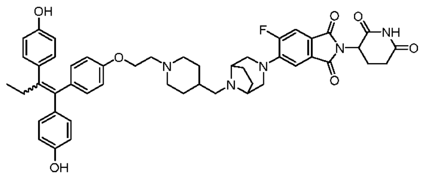 | 5-(8-((1-(2-(4-(1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(2-(4-(1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 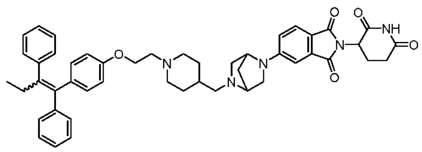 | 2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.1]heptan-2-yl)isoindoline-1,3-dione | 2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.1]heptan-2-yl)isoindoline-1,3-dione |
| | 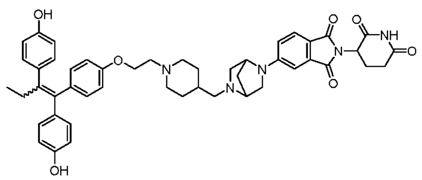 | 5-(5-((1-(2-(4-(1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(2-(4-(1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione |
| | 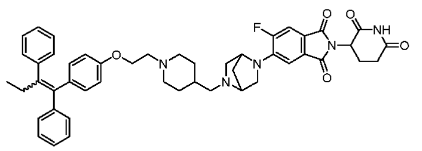 | 2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.1]heptan-2-yl)-6-fluoroisoindoline-1,3-dione | 2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.1]heptan-2-yl)-6-fluoroisoindoline-1,3-dione |
| | 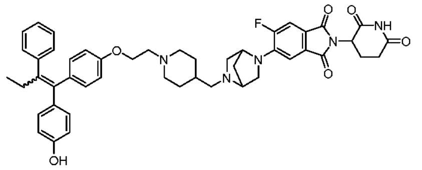 | 2-(2,6-dioxopiperidin-3-yl)-5-fluoro-6-(5-((1-(2-(4-(1-(4-hydroxyphenyl)-2-phenylbut-1-en-1-yl)phenoxy)ethyl)piperidin-4-yl)methyl)-2,5-diazabicyclo[2.2.1]heptan-2-yl)isoindoline-1,3-dione | 2-(2,6-dioxopiperidin-3-yl)-5-fluoro-6-(5-((1-(2-(4-(1-(4-hydroxyphenyl)-2-phenylbut-1-en-1-yl)phenoxy)ethyl)piperidin-4-yl)methyl)-2,5-diazabicyclo[2.2.1]heptan-2-yl)isoindoline-1,3-dione |
| | 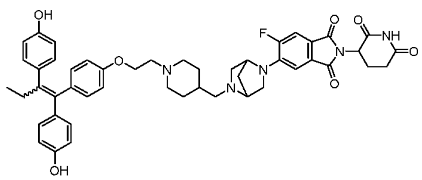 | 5-(5-((1-(2-(4-(1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(2-(4-(1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione |

| | | | |
|---|---|---|---|
| | 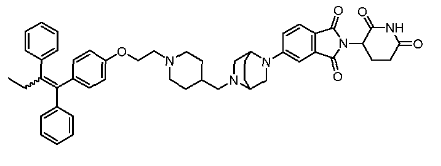 | 2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.2]octan-2-yl)isoindoline-1,3-dione | 2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.2]octan-2-yl)isoindoline-1,3-dione |
| | 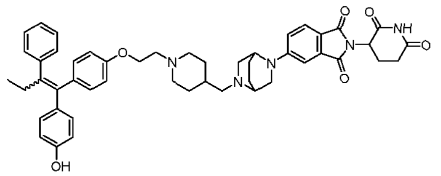 | 2-(2,6-dioxopiperidin-3-yl)-5-(5-((1-(2-(4-(1-(4-hydroxyphenyl)-2-phenylbut-1-en-1-yl)phenoxy)ethyl)piperidin-4-yl)methyl)-2,5-diazabicyclo[2.2.2]octan-2-yl)isoindoline-1,3-dione | 2-(2,6-dioxopiperidin-3-yl)-5-(5-((1-(2-(4-(1-(4-hydroxyphenyl)-2-phenylbut-1-en-1-yl)phenoxy)ethyl)piperidin-4-yl)methyl)-2,5-diazabicyclo[2.2.2]octan-2-yl)isoindoline-1,3-dione |
| | 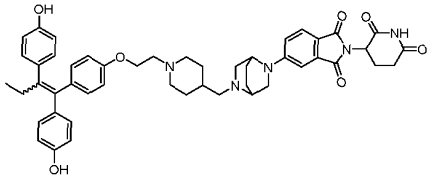 | 5-(5-((1-(2-(4-(1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione | 5-(5-((1-(2-(4-(1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione |
| | 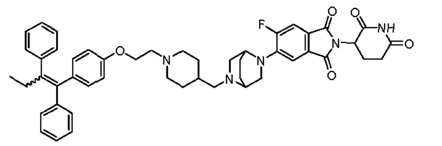 | 2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.2]octan-2-yl)-6-fluoroisoindoline-1,3-dione | 2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.2]octan-2-yl)-6-fluoroisoindoline-1,3-dione |
| | 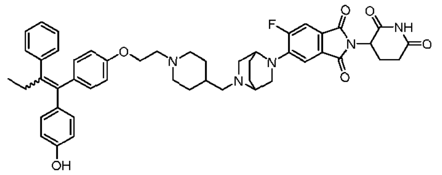 | 2-(2,6-dioxopiperidin-3-yl)-5-fluoro-6-(5-((1-(2-(4-(1-(4-hydroxyphenyl)-2-phenylbut-1-en-1-yl)phenoxy)ethyl)piperidin-4-yl)methyl)-2,5-diazabicyclo[2.2.2]octan-2-yl)isoindoline-1,3-dione | 2-(2,6-dioxopiperidin-3-yl)-5-fluoro-6-(5-((1-(2-(4-(1-(4-hydroxyphenyl)-2-phenylbut-1-en-1-yl)phenoxy)ethyl)piperidin-4-yl)methyl)-2,5-diazabicyclo[2.2.2]octan-2-yl)isoindoline-1,3-dione |
| | 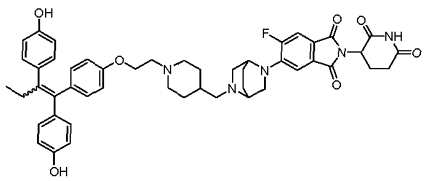 | 5-(5-((1-(2-(4-(1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(2-(4-(1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 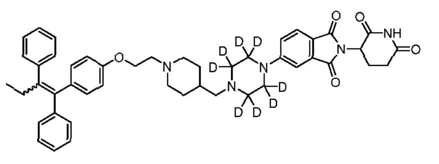 | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)isoindoline-1,3-dione |

| | | | |
|---|---|---|---|
| | 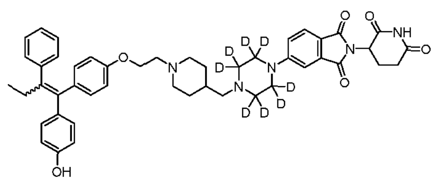 | 2-(2,6-dioxopiperidin-3-yl)-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione |
| | 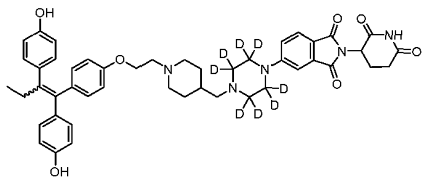 | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione |
| | 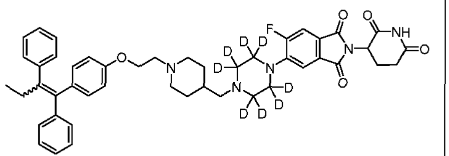 | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-6-fluoroisoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-6-fluoroisoindoline-1,3-dione |
| | 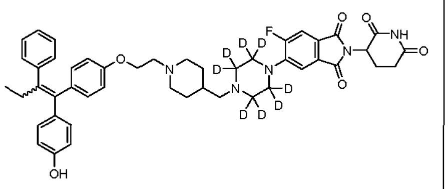 | 2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione |
| | 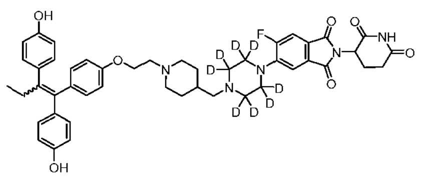 | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione |
| | 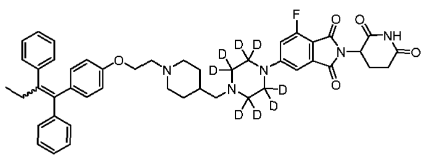 | 2-(2,6-dioxopiperidin-3-yl)-6-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4-fluoroisoindoline-1,3-dione | 2-(2,6-dioxopiperidin-3-yl)-6-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4-fluoroisoindoline-1,3-dione |
| | 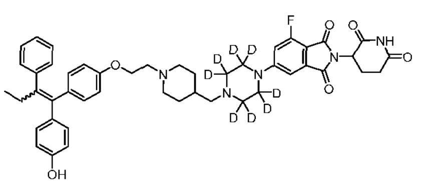 | 2-(2,6-dioxopiperidin-3-yl)-4-fluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione |

| | | | |
|---|---|---|---|
| | 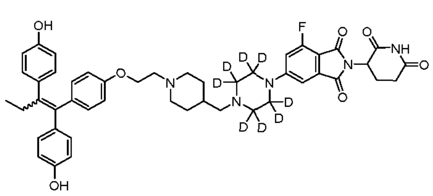 | 6-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 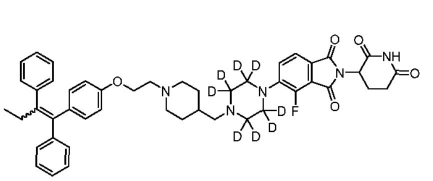 | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4-fluoroisoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4-fluoroisoindoline-1,3-dione |
| | 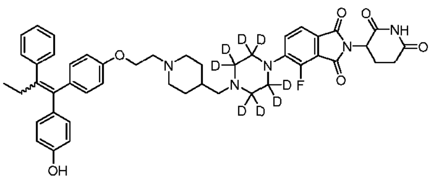 | 2-(2,6-dioxopiperidin-3-yl)-4-fluoro-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione |
| | 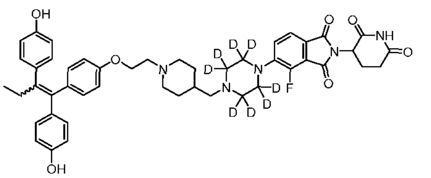 | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 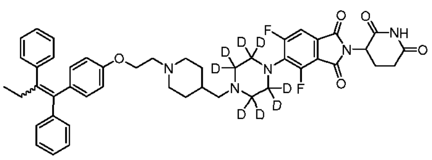 | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4,6-difluoroisoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4,6-difluoroisoindoline-1,3-dione |
| | 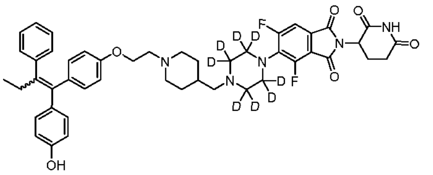 | 2-(2,6-dioxopiperidin-3-yl)-4,6-difluoro-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-4,6-difluoro-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione |
| | 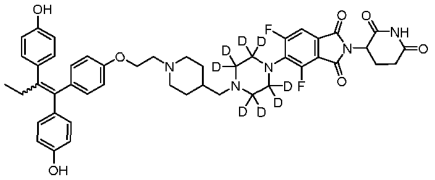 | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |

| | | | |
|---|---|---|---|
| | 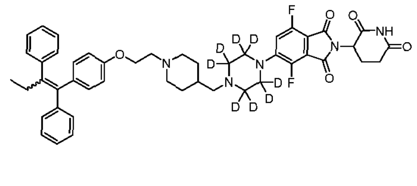 | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-*d*8)-4,7-difluoroisoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-*d*8)-4,7-difluoroisoindoline-1,3-dione |
| | 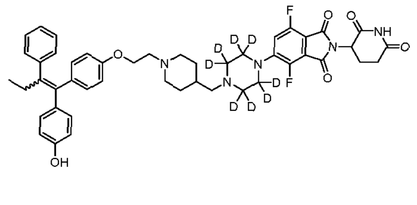 | 2-(2,6-dioxopiperidin-3-yl)-4,7-difluoro-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-*d*8)isoindoline-1,3-dione | 2-(2,6-dioxopiperidin-3-yl)-4,7-difluoro-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-*d*8)isoindoline-1,3-dione |
| | 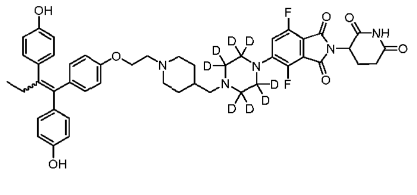 | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-*d*8)-2-(2,6-dioxopiperidin-3-yl)-4,7-difluoroisoindoline-1,3-dione | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-*d*8)-2-(2,6-dioxopiperidin-3-yl)-4,7-difluoroisoindoline-1,3-dione |
| | 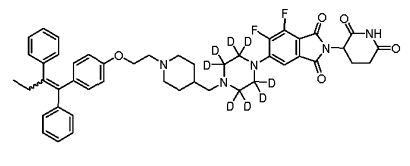 | 2-(2,6-dioxopiperidin-3-yl)-6-(4-((1-(2-(4-(1,2-diphenylbut-1-en-1-yl)phenoxy)ethyl)piperidin-4-yl)methyl)piperazin-1-yl-2,2,3,3,5,5,6,6-*d*8)-4,5-difluoroisoindoline-1,3-dione | 2-(2,6-dioxopiperidin-3-yl)-6-(4-((1-(2-(4-(1,2-diphenylbut-1-en-1-yl)phenoxy)ethyl)piperidin-4-yl)methyl)piperazin-1-yl-2,2,3,3,5,5,6,6-*d*8)-4,5-difluoroisoindoline-1,3-dione |
| | 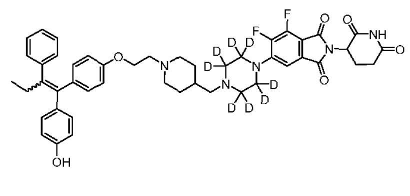 | 2-(2,6-dioxopiperidin-3-yl)-4,5-difluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-*d*8)isoindoline-1,3-dione | 2-(2,6-dioxopiperidin-3-yl)-4,5-difluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-*d*8)isoindoline-1,3-dione |
| | 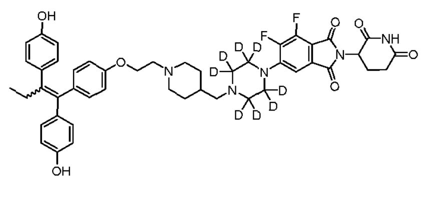 | 6-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-*d*8)-2-(2,6-dioxopiperidin-3-yl)-4,5-difluoroisoindoline-1,3-dione | 6-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-*d*8)-2-(2,6-dioxopiperidin-3-yl)-4,5-difluoroisoindoline-1,3-dione |
| | 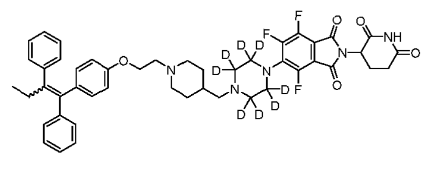 | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-*d*8)-4,6,7-trifluoroisoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-*d*8)-4,6,7-trifluoroisoindoline-1,3-dione |

| | | | |
|---|---|---|---|
| | 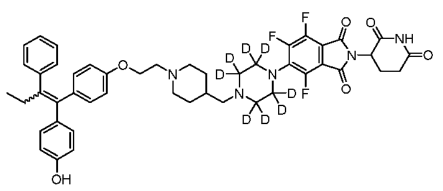 | 2-(2,6-dioxopiperidin-3-yl)-4,5,7-trifluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-4,5,7-trifluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione |
| | 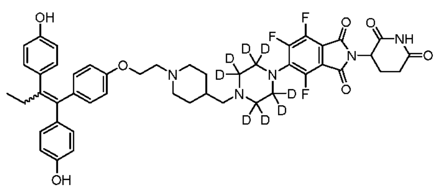 | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |
| | 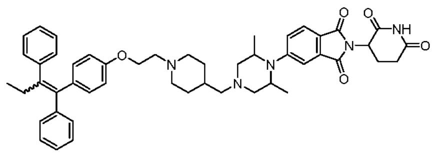 | 2-(2,6-dioxopiperidin-3-yl)-5-(4-((1-(2-(4-(1,2-diphenylbut-1-en-1-yl)phenoxy)ethyl)piperidin-4-yl)methyl)-2,6-dimethylpiperazin-1-yl)isoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)-2,6-dimethylpiperazin-1-yl)isoindoline-1,3-dione |
| | 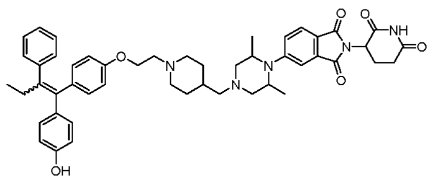 | 2-(2,6-dioxopiperidin-3-yl)-5-(4-((1-(2-(4-(1-(4-hydroxyphenyl)-2-phenylbut-1-en-1-yl)phenoxy)ethyl)piperidin-4-yl)methyl)-2,6-dimethylpiperazin-1-yl)isoindoline-1,3-dione | 2-(2,6-dioxopiperidin-3-yl)-5-(4-((1-(2-(4-(1-(4-hydroxyphenyl)-2-phenylbut-1-en-1-yl)phenoxy)ethyl)piperidin-4-yl)methyl)-2,6-dimethylpiperazin-1-yl)isoindoline-1,3-dione |
| | 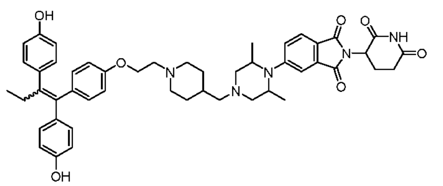 | 5-(4-((1-(2-(4-(1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)isoindoline-1,3-dione | 5-(4-((1-(2-(4-(1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)isoindoline-1,3-dione |
| | 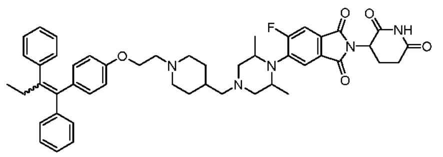 | 2-(2,6-dioxopiperidin-3-yl)-5-(4-((1-(2-(4-(1,2-diphenylbut-1-en-1-yl)phenoxy)ethyl)piperidin-4-yl)methyl)-2,6-dimethylpiperazin-1-yl)-6-fluoroisoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)-2,6-dimethylpiperazin-1-yl)-6-fluoroisoindoline-1,3-dione |
| | 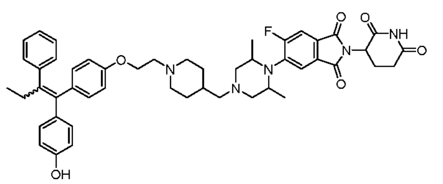 | 2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)-2,6-dimethylpiperazin-1-yl)isoindoline-1,3-dione | 2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)-2,6-dimethylpiperazin-1-yl)isoindoline-1,3-dione |

| | | | |
|---|---|---|---|
| | 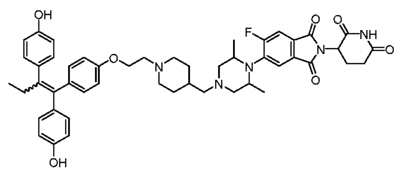 | 5-(4-((1-(2-(4-(1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione | 5-(4-((1-(2-(4-(1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione |
| | 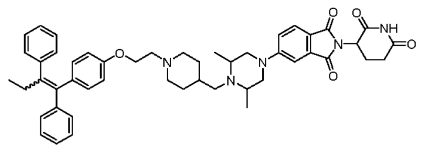 | 2-(2,6-dioxopiperidin-3-yl)-5-(4-((1-(2-(4-(1,2-diphenylbut-1-en-1-yl)phenoxy)ethyl)piperidin-4-yl)methyl)-3,5-dimethylpiperazin-1-yl)isoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)-3,5-dimethylpiperazin-1-yl)isoindoline-1,3-dione |
| | 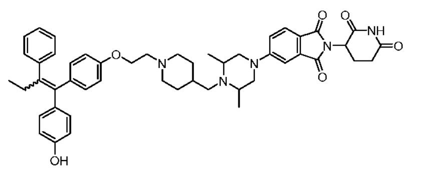 | 2-(2,6-dioxopiperidin-3-yl)-5-(4-((1-(2-(4-(1-(4-hydroxyphenyl)-2-phenylbut-1-en-1-yl)phenoxy)ethyl)piperidin-4-yl)methyl)-3,5-dimethylpiperazin-1-yl)isoindoline-1,3-dione | 2-(2,6-dioxopiperidin-3-yl)-5-(4-((1-(2-(4-(1-(4-hydroxyphenyl)-2-phenylbut-1-en-1-yl)phenoxy)ethyl)piperidin-4-yl)methyl)-3,5-dimethylpiperazin-1-yl)isoindoline-1,3-dione |
| | 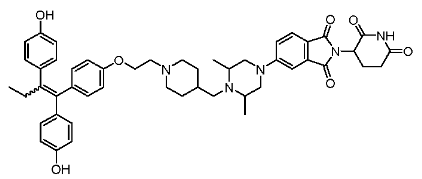 | 5-(4-((1-(2-(4-(1,2-bis(4-hydroxyphenyl)but-1-en-1-yl)phenoxy)ethyl)piperidin-4-yl)methyl)-3,5-dimethylpiperazin-1-yl)-2-(2,6-dioxopiperidin-3-yl)isoindoline-1,3-dione | 5-(4-((1-(2-(4-(1,2-bis(4-hydroxyphenyl)but-1-en-1-yl)phenoxy)ethyl)piperidin-4-yl)methyl)-3,5-dimethylpiperazin-1-yl)-2-(2,6-dioxopiperidin-3-yl)isoindoline-1,3-dione |
| | 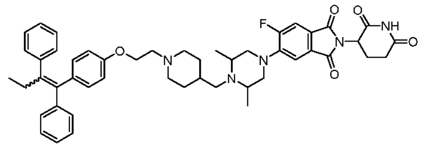 | 2-(2,6-dioxopiperidin-3-yl)-5-(4-((1-(2-(4-(1,2-diphenylbut-1-en-1-yl)phenoxy)ethyl)piperidin-4-yl)methyl)-3,5-dimethylpiperazin-1-yl)-6-fluoroisoindoline-1,3-dione | 2-(2,6-dioxopiperidin-3-yl)-5-(4-((1-(2-(4-(1,2-diphenylbut-1-en-1-yl)phenoxy)ethyl)piperidin-4-yl)methyl)-3,5-dimethylpiperazin-1-yl)-6-fluoroisoindoline-1,3-dione |
| | 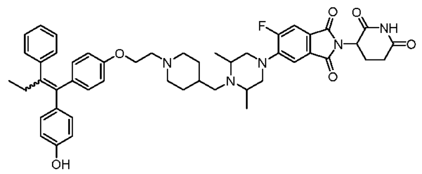 | 2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)-3,5-dimethylpiperazin-1-yl)isoindoline-1,3-dione | 2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)-3,5-dimethylpiperazin-1-yl)isoindoline-1,3-dione |
| | 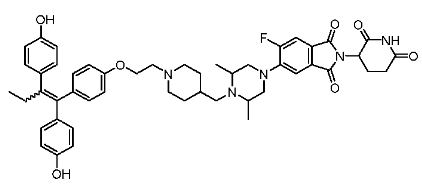 | 5-(4-((1-(2-(4-(1,2-bis(4-hydroxyphenyl)but-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione | 5-(4-((1-(2-(4-(1,2-bis(4-hydroxyphenyl)but-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione |
| | 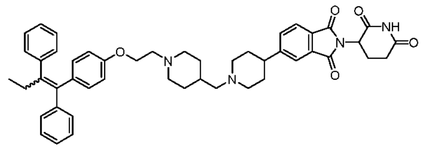 | 2-(2,6-dioxopiperidin-3-yl)-5-(1-((1-(2-(4-(1,2-diphenylbut-1-en-1-yl)phenoxy)ethyl)piperidin-4-yl)methyl)piperidin-4- | 2-(2,6-dioxopiperidin-3-yl)-5-(1-((1-(2-(4-(1,2-diphenylbut-1-en-1-yl)phenoxy)ethyl)piperidin-4-yl)methyl)piperidin-4- |

| | | | |
|---|---|---|---|
| | | yl)isoindoline-1,3-dione | yl)isoindoline-1,3-dione |
| | 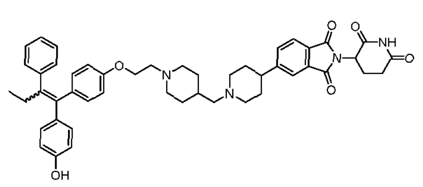 | 2-(2,6-dioxopiperidin-3-yl)-5-(1-((1-(2-(4-(1-(4-hydroxyphenyl)-2-phenylbut-1-en-1-yl)phenoxy)ethyl)piperidin-4-yl)methyl)piperidin-4-yl)isoindoline-1,3-dione | 2-(2,6-dioxopiperidin-3-yl)-5-(1-((1-(2-(4-(1-(4-hydroxyphenyl)-2-phenylbut-1-en-1-yl)phenoxy)ethyl)piperidin-4-yl)methyl)piperidin-4-yl)isoindoline-1,3-dione |
| | 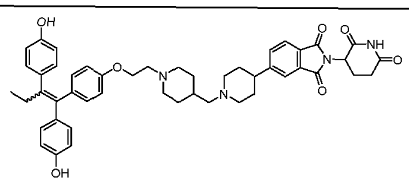 | 5-(1-((1-(2-(4-(1,2-bis(4-hydroxyphenyl)but-1-en-1-yl)phenoxy)ethyl)piperidin-4-yl)methyl)piperidin-4-yl)-2-(2,6-dioxopiperidin-3-yl)isoindoline-1,3-dione | 5-(1-((1-(2-(4-(1,2-bis(4-hydroxyphenyl)but-1-en-1-yl)phenoxy)ethyl)piperidin-4-yl)methyl)piperidin-4-yl)-2-(2,6-dioxopiperidin-3-yl)isoindoline-1,3-dione |
| | 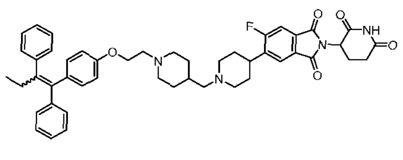 | 2-(2,6-dioxopiperidin-3-yl)-5-(1-((1-(2-(4-(1,2-diphenylbut-1-en-1-yl)phenoxy)ethyl)piperidin-4-yl)methyl)piperidin-4-yl)-6-fluoroisoindoline-1,3-dione | 2-(2,6-dioxopiperidin-3-yl)-5-(1-((1-(2-(4-(1,2-diphenylbut-1-en-1-yl)phenoxy)ethyl)piperidin-4-yl)methyl)piperidin-4-yl)-6-fluoroisoindoline-1,3-dione |
| | 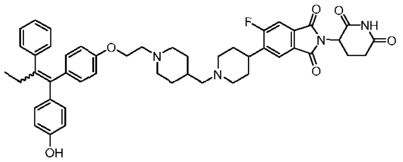 | 2-(2,6-dioxopiperidin-3-yl)-5-fluoro-6-(1-((1-(2-(4-(1-(4-hydroxyphenyl)-2-phenylbut-1-en-1-yl)phenoxy)ethyl)piperidin-4-yl)methyl)piperidin-4-yl)isoindoline-1,3-dione | 2-(2,6-dioxopiperidin-3-yl)-5-fluoro-6-(1-((1-(2-(4-(1-(4-hydroxyphenyl)-2-phenylbut-1-en-1-yl)phenoxy)ethyl)piperidin-4-yl)methyl)piperidin-4-yl)isoindoline-1,3-dione |
| | 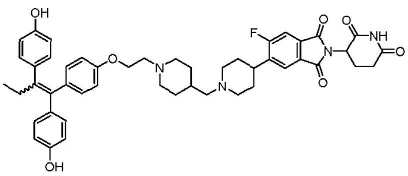 | 5-(1-((1-(2-(4-(1,2-bis(4-hydroxyphenyl)but-1-en-1-yl)phenoxy)ethyl)piperidin-4-yl)methyl)piperidin-4-yl)-2-(2,6-dioxopiperidin-3-yl)-6-fluoroisoindoline-1,3-dione | 5-(1-((1-(2-(4-(1,2-bis(4-hydroxyphenyl)but-1-en-1-yl)phenoxy)ethyl)piperidin-4-yl)methyl)piperidin-4-yl)-2-(2,6-dioxopiperidin-3-yl)-6-fluoroisoindoline-1,3-dione |
| | 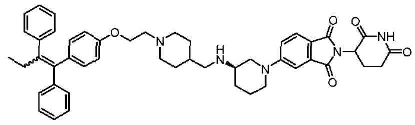 | 2-(2,6-dioxopiperidin-3-yl)-5-((R)-3-(((1-(2-(4-(1,2-diphenylbut-1-en-1-yl)phenoxy)ethyl)piperidin-4-yl)methyl)amino)piperidin-1-yl)isoindoline-1,3-dione | 2-(2,6-dioxopiperidin-3-yl)-5-((R)-3-(((1-(2-(4-(1,2-diphenylbut-1-en-1-yl)phenoxy)ethyl)piperidin-4-yl)methyl)amino)piperidin-1-yl)isoindoline-1,3-dione |
| | 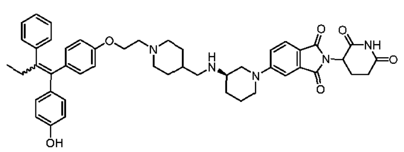 | 2-(2,6-dioxopiperidin-3-yl)-5-((R)-3-(((1-(2-(4-(1-(4-hydroxyphenyl)-2-phenylbut-1-en-1-yl)phenoxy)ethyl)piperidin-4-yl)methyl)amino)piperidin-1-yl)isoindoline-1,3-dione | 2-(2,6-dioxopiperidin-3-yl)-5-((R)-3-(((1-(2-(4-(1-(4-hydroxyphenyl)-2-phenylbut-1-en-1-yl)phenoxy)ethyl)piperidin-4-yl)methyl)amino)piperidin-1-yl)isoindoline-1,3-dione |
| | 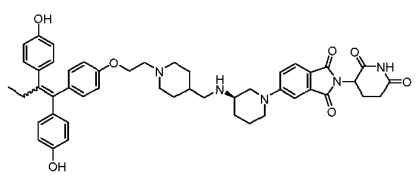 | 5-((R)-3-(((1-(2-(4-(1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione | 5-((R)-3-(((1-(2-(4-(1,2-bis(4-hydroxyphenyl)but-1-en-1-yl)phenoxy)ethyl)piperidin-4-yl)methyl)amino)piperidin-1-yl)-2-(2,6-dioxopiperidin-3- |

| | | | |
|---|---|---|---|
| | | | yl)isoindoline-1,3-dione |
| | 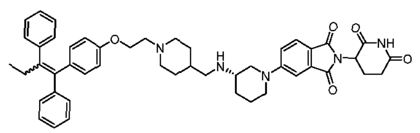 | 2-(2,6-dioxopiperidin-3-yl)-5-((S)-3-(((1-(2-(4-(1,2-diphenylbut-1-en-1-yl)phenoxy)ethyl)piperidin-4-yl)methyl)amino)piperidin-1-yl)isoindoline-1,3-dione | 2-(2,6-dioxopiperidin-3-yl)-5-((S)-3-(((1-(2-(4-(1,2-diphenylbut-1-en-1-yl)phenoxy)ethyl)piperidin -4-yl)methyl)amino)piperidin-1-yl)isoindoline-1,3-dione |
| | 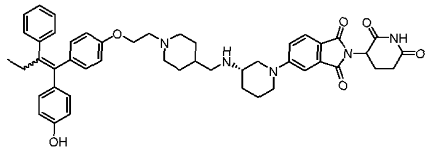 | 2-(2,6-dioxopiperidin-3-yl)-5-((S)-3-(((1-(2-(4-(1-(4-hydroxyphenyl)-2-phenylbut-1-en-1-yl)phenoxy)ethyl)piperidin-4-yl)methyl)amino)piperidin-1-yl)isoindoline-1,3-dione | 2-(2,6-dioxopiperidin-3-yl)-5-((S)-3-(((1-(2-(4-(1-(4-hydroxyphenyl)-2-phenylbut-1-en-1-yl)phenoxy)ethyl)piperidin -4-yl)methyl)amino)piperidin-1-yl)isoindoline-1,3-dione |
| | 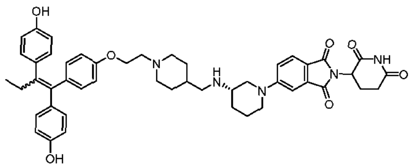 | 5-((S)-3-(((1-(2-(4-(1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione | 5-((S)-3-(((1-(2-(4-(1,2-bis(4-hydroxyphenyl)but-1-en-1-yl)phenoxy)ethyl)piperidin -4-yl)methyl)amino)piperidin-1-yl)-2-(2,6-dioxopiperidin-3-yl)isoindoline-1,3-dione |
| | 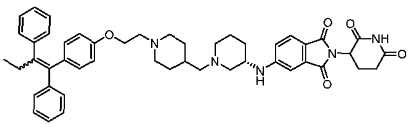 | 2-(2,6-dioxopiperidin-3-yl)-5-(((S)-1-((1-(2-(4-(1,2-diphenylbut-1-en-1-yl)phenoxy)ethyl)piperidin-4-yl)methyl)piperidin-3-yl)amino)isoindoline-1,3-dione | 2-(2,6-dioxopiperidin-3-yl)-5-(((S)-1-((1-(2-(4-(1,2-diphenylbut-1-en-1-yl)phenoxy)ethyl)piperidin -4-yl)methyl)piperidin-3-yl)amino)isoindoline-1,3-dione |
| | 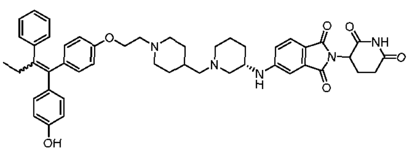 | 2-(2,6-dioxopiperidin-3-yl)-5-(((S)-1-((1-(2-(4-(1-(4-hydroxyphenyl)-2-phenylbut-1-en-1-yl)phenoxy)ethyl)piperidin-4-yl)methyl)piperidin-3-yl)amino)isoindoline-1,3-dione | 2-(2,6-dioxopiperidin-3-yl)-5-(((S)-1-((1-(2-(4-(1-(4-hydroxyphenyl)-2-phenylbut-1-en-1-yl)phenoxy)ethyl)piperidin -4-yl)methyl)piperidin-3-yl)amino)isoindoline-1,3-dione |
| | 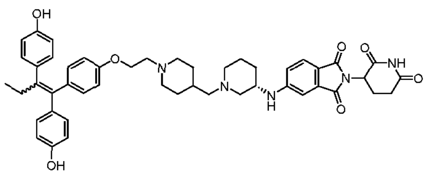 | 5-(((S)-1-((1-(2-(4-(1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione | 5-(((S)-1-((1-(2-(4-(1,2-bis(4-hydroxyphenyl)but-1-en-1-yl)phenoxy)ethyl)piperidin -4-yl)methyl)piperidin-3-yl)amino)-2-(2,6-dioxopiperidin-3-yl)isoindoline-1,3-dione |
| | 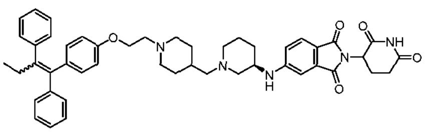 | 2-(2,6-dioxopiperidin-3-yl)-5-(((R)-1-((1-(2-(4-(1,2-diphenylbut-1-en-1-yl)phenoxy)ethyl)piperidin-4-yl)methyl)piperidin-3-yl)amino)isoindoline-1,3-dione | 2-(2,6-dioxopiperidin-3-yl)-5-(((R)-1-((1-(2-(4-(1,2-diphenylbut-1-en-1-yl)phenoxy)ethyl)piperidin -4-yl)methyl)piperidin-3-yl)amino)isoindoline-1,3-dione |

| | | | |
|---|---|---|---|
| | 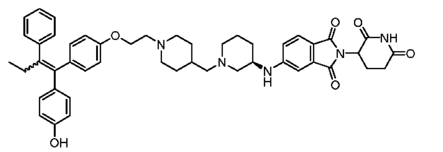 | 2-(2,6-dioxopiperidin-3-yl)-5-(((R)-1-((1-(2-(4-(1-(4-hydroxyphenyl)-2-phenylbut-1-en-1-yl)phenoxy)ethyl)piperidin-4-yl)methyl)piperidin-3-yl)amino)isoindoline-1,3-dione | 2-(2,6-dioxopiperidin-3-yl)-5-(((R)-1-((1-(2-(4-(1-(4-hydroxyphenyl)-2-phenylbut-1-en-1-yl)phenoxy)ethyl)piperidin-4-yl)methyl)piperidin-3-yl)amino)isoindoline-1,3-dione |
| | 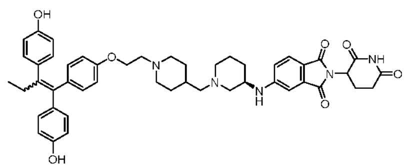 | 5-(((R)-1-((1-(2-(4-(1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione | 5-(((R)-1-((1-(2-(4-(1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione |
| | 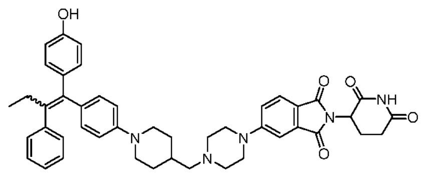 | 2-(2,6-dioxopiperidin-3-yl)-5-(4-((1-(4-(1-(4-hydroxyphenyl)-2-phenylbut-1-en-1-yl)phenyl)piperidin-4-yl)methyl)piperazin-1-yl)isoindoline-1,3-dione | 2-(2,6-dioxopiperidin-3-yl)-5-(4-((1-(4-(1-(4-hydroxyphenyl)-2-phenylbut-1-en-1-yl)phenyl)piperidin-4-yl)methyl)piperazin-1-yl)isoindoline-1,3-dione |
| | 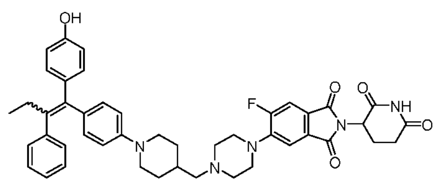 | 5-(4-((1-(4-(4-chloro-1-(4-hydroxyphenyl)-2-phenylbut-1-en-1-yl)phenyl)piperidin-4-yl)methyl)piperazin-1-yl)-2-(2,6-dioxopiperidin-3-yl)-6-fluoroisoindoline-1,3-dione | 5-(4-((1-(4-(4-chloro-1-(4-hydroxyphenyl)-2-phenylbut-1-en-1-yl)phenyl)piperidin-4-yl)methyl)piperazin-1-yl)-2-(2,6-dioxopiperidin-3-yl)-6-fluoroisoindoline-1,3-dione |
| | 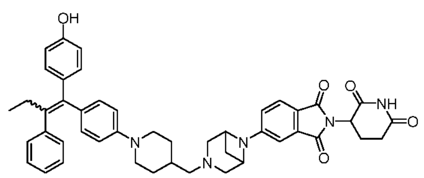 | 2-(2,6-dioxopiperidin-3-yl)-5-(3-((1-(4-(1-(4-hydroxyphenyl)-2-phenylbut-1-en-1-yl)phenyl)piperidin-4-yl)methyl)-3,6-diazabicyclo[3.1.1]heptan-6-yl)isoindoline-1,3-dione | 2-(2,6-dioxopiperidin-3-yl)-5-(3-((1-(4-(1-(4-hydroxyphenyl)-2-phenylbut-1-en-1-yl)phenyl)piperidin-4-yl)methyl)-3,6-diazabicyclo[3.1.1]heptan-6-yl)isoindoline-1,3-dione |
| | 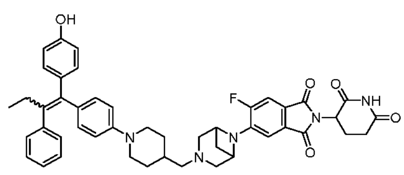 | 2-(2,6-dioxopiperidin-3-yl)-5-fluoro-6-(3-((1-(4-(1-(4-hydroxyphenyl)-2-phenylbut-1-en-1-yl)phenyl)piperidin-4-yl)methyl)-3,6-diazabicyclo[3.1.1]heptan-6-yl)isoindoline-1,3-dione | 2-(2,6-dioxopiperidin-3-yl)-5-fluoro-6-(3-((1-(4-(1-(4-hydroxyphenyl)-2-phenylbut-1-en-1-yl)phenyl)piperidin-4-yl)methyl)-3,6-diazabicyclo[3.1.1]heptan-6-yl)isoindoline-1,3-dione |
| | 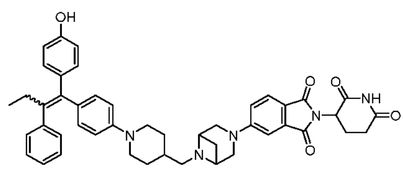 | 2-(2,6-dioxopiperidin-3-yl)-5-(6-((1-(4-(1-(4-hydroxyphenyl)-2-phenylbut-1-en-1-yl)phenyl)piperidin-4-yl)methyl)-3,6-diazabicyclo[3.1.1]heptan-3-yl)isoindoline-1,3-dione | 2-(2,6-dioxopiperidin-3-yl)-5-(6-((1-(4-(1-(4-hydroxyphenyl)-2-phenylbut-1-en-1-yl)phenyl)piperidin-4-yl)methyl)-3,6-diazabicyclo[3.1.1]heptan-3-yl)isoindoline-1,3-dione |

| | | | |
|---|---|---|---|
| | 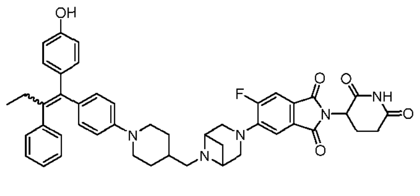 | 2-(2,6-dioxopiperidin-3-yl)-5-fluoro-6-(6-((1-(4-(1-(4-hydroxyphenyl)-2-phenylbut-1-en-1-yl)phenyl)piperidin-4-yl)methyl)-3,6-diazabicyclo[3.1.1]heptan-3-yl)isoindoline-1,3-dione | 2-(2,6-dioxopiperidin-3-yl)-5-fluoro-6-(6-((1-(4-(1-(4-hydroxyphenyl)-2-phenylbut-1-en-1-yl)phenyl)piperidin-4-yl)methyl)-3,6-diazabicyclo[3.1.1]heptan-3-yl)isoindoline-1,3-dione |
| | 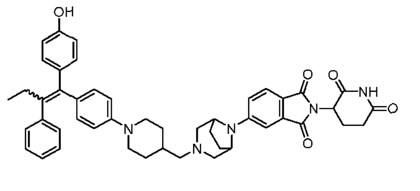 | 2-(2,6-dioxopiperidin-3-yl)-5-(3-((1-(4-(1-(4-hydroxyphenyl)-2-phenylbut-1-en-1-yl)phenyl)piperidin-4-yl)methyl)-3,8-diazabicyclo[3.2.1]octan-8-yl)isoindoline-1,3-dione | 2-(2,6-dioxopiperidin-3-yl)-5-(3-((1-(4-(1-(4-hydroxyphenyl)-2-phenylbut-1-en-1-yl)phenyl)piperidin-4-yl)methyl)-3,8-diazabicyclo[3.2.1]octan-8-yl)isoindoline-1,3-dione |
| | 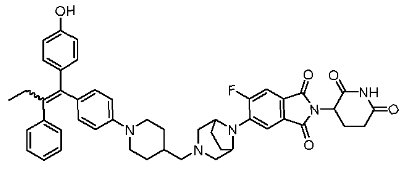 | 2-(2,6-dioxopiperidin-3-yl)-5-fluoro-6-(3-((1-(4-(1-(4-hydroxyphenyl)-2-phenylbut-1-en-1-yl)phenyl)piperidin-4-yl)methyl)-3,8-diazabicyclo[3.2.1]octan-8-yl)isoindoline-1,3-dione | 2-(2,6-dioxopiperidin-3-yl)-5-fluoro-6-(3-((1-(4-(1-(4-hydroxyphenyl)-2-phenylbut-1-en-1-yl)phenyl)piperidin-4-yl)methyl)-3,8-diazabicyclo[3.2.1]octan-8-yl)isoindoline-1,3-dione |
| | 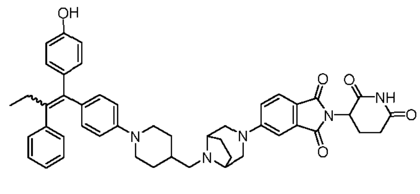 | 2-(2,6-dioxopiperidin-3-yl)-5-(8-((1-(4-(1-(4-hydroxyphenyl)-2-phenylbut-1-en-1-yl)phenyl)piperidin-4-yl)methyl)-3,8-diazabicyclo[3.2.1]octan-3-yl)isoindoline-1,3-dione | 2-(2,6-dioxopiperidin-3-yl)-5-(8-((1-(4-(1-(4-hydroxyphenyl)-2-phenylbut-1-en-1-yl)phenyl)piperidin-4-yl)methyl)-3,8-diazabicyclo[3.2.1]octan-3-yl)isoindoline-1,3-dione |
| | 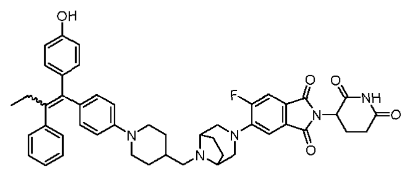 | 2-(2,6-dioxopiperidin-3-yl)-5-fluoro-6-(8-((1-(4-(1-(4-hydroxyphenyl)-2-phenylbut-1-en-1-yl)phenyl)piperidin-4-yl)methyl)-3,8-diazabicyclo[3.2.1]octan-3-yl)isoindoline-1,3-dione | 2-(2,6-dioxopiperidin-3-yl)-5-fluoro-6-(8-((1-(4-(1-(4-hydroxyphenyl)-2-phenylbut-1-en-1-yl)phenyl)piperidin-4-yl)methyl)-3,8-diazabicyclo[3.2.1]octan-3-yl)isoindoline-1,3-dione |
| | 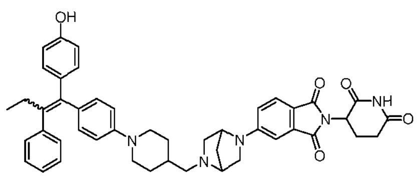 | 2-(2,6-dioxopiperidin-3-yl)-5-(5-((1-(4-(1-(4-hydroxyphenyl)-2-phenylbut-1-en-1-yl)phenyl)piperidin-4-yl)methyl)-2,5-diazabicyclo[2.2.1]heptan-2-yl)isoindoline-1,3-dione | 2-(2,6-dioxopiperidin-3-yl)-5-(5-((1-(4-(1-(4-hydroxyphenyl)-2-phenylbut-1-en-1-yl)phenyl)piperidin-4-yl)methyl)-2,5-diazabicyclo[2.2.1]heptan-2-yl)isoindoline-1,3-dione |
| | 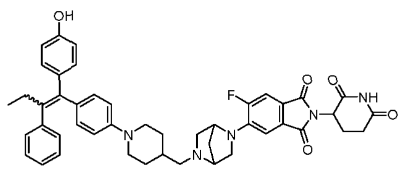 | 2-(2,6-dioxopiperidin-3-yl)-5-fluoro-6-(5-((1-(4-(1-(4-hydroxyphenyl)-2-phenylbut-1-en-1-yl)phenyl)piperidin-4-yl)methyl)-2,5-diazabicyclo[2.2.1]heptan-2-yl)isoindoline-1,3-dione | 2-(2,6-dioxopiperidin-3-yl)-5-fluoro-6-(5-((1-(4-(1-(4-hydroxyphenyl)-2-phenylbut-1-en-1-yl)phenyl)piperidin-4-yl)methyl)-2,5-diazabicyclo[2.2.1]heptan-2-yl)isoindoline-1,3-dione |

| | | | |
|---|---|---|---|
| | 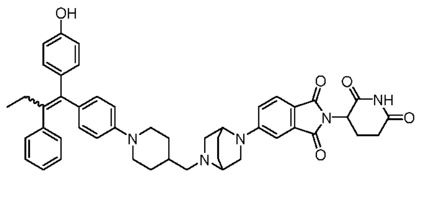 | 2-(2,6-dioxopiperidin-3-yl)-5-(5-((1-(4-(1-(4-hydroxyphenyl)-2-phenylbut-1-en-1-yl)phenyl)piperidin-4-yl)methyl)-2,5-diazabicyclo[2.2.2]octan-2-yl)isoindoline-1,3-dione | 2-(2,6-dioxopiperidin-3-yl)-5-(5-((1-(4-(1-(4-hydroxyphenyl)-2-phenylbut-1-en-1-yl)phenyl)piperidin-4-yl)methyl)-2,5-diazabicyclo[2.2.2]octan-2-yl)isoindoline-1,3-dione |
| | 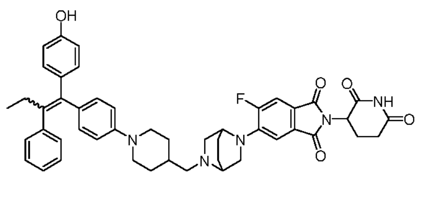 | 2-(2,6-dioxopiperidin-3-yl)-5-fluoro-6-(5-((1-(4-(1-(4-hydroxyphenyl)-2-phenylbut-1-en-1-yl)phenyl)piperidin-4-yl)methyl)-2,5-diazabicyclo[2.2.2]octan-2-yl)isoindoline-1,3-dione | 2-(2,6-dioxopiperidin-3-yl)-5-fluoro-6-(5-((1-(4-(1-(4-hydroxyphenyl)-2-phenylbut-1-en-1-yl)phenyl)piperidin-4-yl)methyl)-2,5-diazabicyclo[2.2.2]octan-2-yl)isoindoline-1,3-dione |
| | 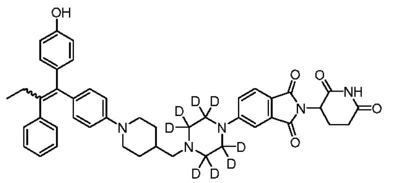 | 2-(2,6-dioxopiperidin-3-yl)-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione | 2-(2,6-dioxopiperidin-3-yl)-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione |
| | 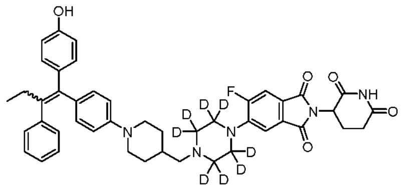 | 2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione | 2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione |
| | 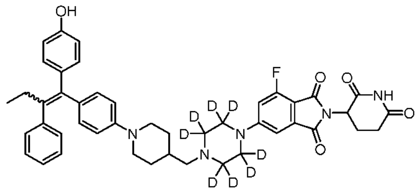 | 2-(2,6-dioxopiperidin-3-yl)-4-fluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione | 2-(2,6-dioxopiperidin-3-yl)-4-fluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione |
| | 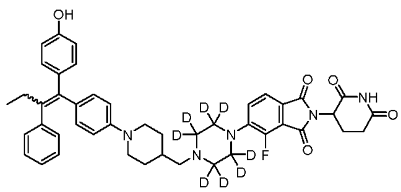 | 2-(2,6-dioxopiperidin-3-yl)-4-fluoro-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione | 2-(2,6-dioxopiperidin-3-yl)-4-fluoro-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione |
| | 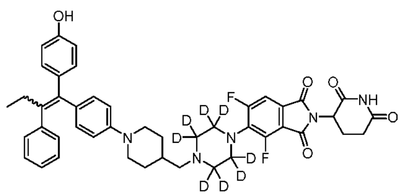 | 2-(2,6-dioxopiperidin-3-yl)-4,6-difluoro-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione | 2-(2,6-dioxopiperidin-3-yl)-4,6-difluoro-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*$_8$)isoindoline-1,3-dione |

| | | | |
|---|---|---|---|
| | 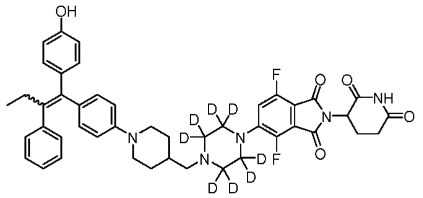 | 2-(2,6-dioxopiperidin-3-yl)-4,7-difluoro-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*8)isoindoline-1,3-dione | 2-(2,6-dioxopiperidin-3-yl)-4,7-difluoro-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*8)isoindoline-1,3-dione |
| | 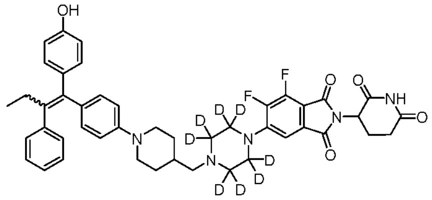 | 2-(2,6-dioxopiperidin-3-yl)-4,5-difluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*8)isoindoline-1,3-dione | 2-(2,6-dioxopiperidin-3-yl)-4,5-difluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*8)isoindoline-1,3-dione |
| | 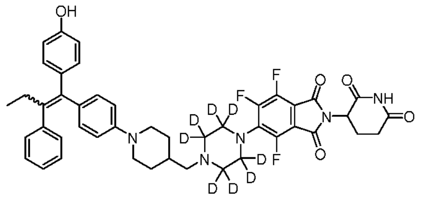 | 2-(2,6-dioxopiperidin-3-yl)-4,5,7-trifluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*8)isoindoline-1,3-dione | 2-(2,6-dioxopiperidin-3-yl)-4,5,7-trifluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-*d*8)isoindoline-1,3-dione |
| | 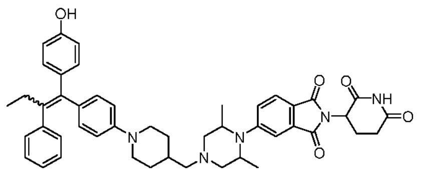 | 2-(2,6-dioxopiperidin-3-yl)-5-(4-((1-(4-(1-(4-hydroxyphenyl)-2-phenylbut-1-en-1-yl)phenyl)piperidin-4-yl)methyl)-2,6-dimethylpiperazin-1-yl)isoindoline-1,3-dione | 2-(2,6-dioxopiperidin-3-yl)-5-(4-((1-(4-(1-(4-hydroxyphenyl)-2-phenylbut-1-en-1-yl)phenyl)piperidin-4-yl)methyl)-2,6-dimethylpiperazin-1-yl)isoindoline-1,3-dione |
| | 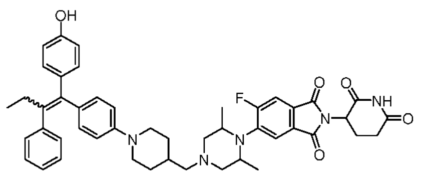 | 2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)-2,6-dimethylpiperazin-1-yl)isoindoline-1,3-dione | 2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)-2,6-dimethylpiperazin-1-yl)isoindoline-1,3-dione |
| | 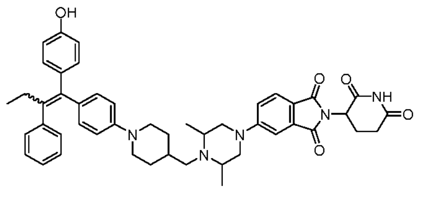 | 2-(2,6-dioxopiperidin-3-yl)-5-(4-((1-(4-(1-(4-hydroxyphenyl)-2-phenylbut-1-en-1-yl)phenyl)piperidin-4-yl)methyl)-3,5-dimethylpiperazin-1-yl)isoindoline-1,3-dione | 2-(2,6-dioxopiperidin-3-yl)-5-(4-((1-(4-(1-(4-hydroxyphenyl)-2-phenylbut-1-en-1-yl)phenyl)piperidin-4-yl)methyl)-3,5-dimethylpiperazin-1-yl)isoindoline-1,3-dione |
| | 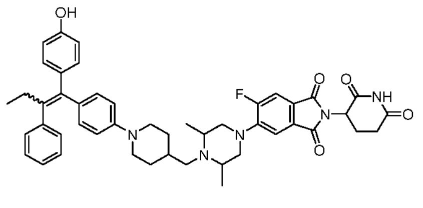 | 2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)-3,5-dimethylpiperazin-1-yl)isoindoline-1,3-dione | 2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)-3,5-dimethylpiperazin-1-yl)isoindoline-1,3-dione |

| | | | |
|---|---|---|---|
| | 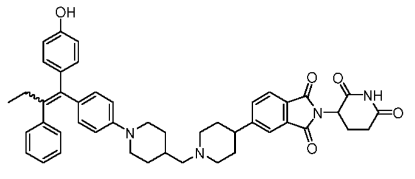 | 2-(2,6-dioxopiperidin-3-yl)-5-(1-((1-(4-(1-(4-hydroxyphenyl)-2-phenylbut-1-en-1-yl)phenyl)piperidin-4-yl)methyl)piperidin-4-yl)isoindoline-1,3-dione | 2-(2,6-dioxopiperidin-3-yl)-5-(1-((1-(4-(1-(4-hydroxyphenyl)-2-phenylbut-1-en-1-yl)phenyl)piperidin-4-yl)methyl)piperidin-4-yl)isoindoline-1,3-dione |
| | 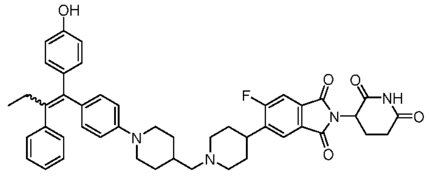 | 2-(2,6-dioxopiperidin-3-yl)-5-fluoro-6-(1-((1-(4-(1-(4-hydroxyphenyl)-2-phenylbut-1-en-1-yl)phenyl)piperidin-4-yl)methyl)piperidin-4-yl)isoindoline-1,3-dione | 2-(2,6-dioxopiperidin-3-yl)-5-fluoro-6-(1-((1-(4-(1-(4-hydroxyphenyl)-2-phenylbut-1-en-1-yl)phenyl)piperidin-4-yl)methyl)piperidin-4-yl)isoindoline-1,3-dione |
| | 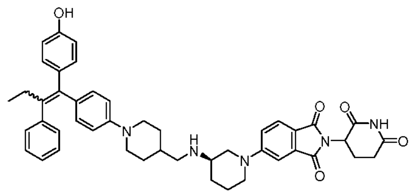 | 2-(2,6-dioxopiperidin-3-yl)-5-((R)-3-(((1-(4-(1-(4-hydroxyphenyl)-2-phenylbut-1-en-1-yl)phenyl)piperidin-4-yl)methyl)amino)piperidin-1-yl)isoindoline-1,3-dione | 2-(2,6-dioxopiperidin-3-yl)-5-((R)-3-(((1-(4-(1-(4-hydroxyphenyl)-2-phenylbut-1-en-1-yl)phenyl)piperidin-4-yl)methyl)amino)piperidin-1-yl)isoindoline-1,3-dione |
| | 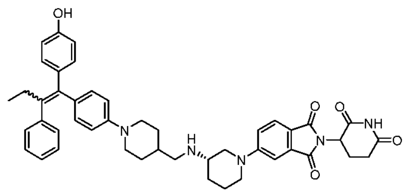 | 2-(2,6-dioxopiperidin-3-yl)-5-((S)-3-(((1-(4-(1-(4-hydroxyphenyl)-2-phenylbut-1-en-1-yl)phenyl)piperidin-4-yl)methyl)amino)piperidin-1-yl)isoindoline-1,3-dione | 2-(2,6-dioxopiperidin-3-yl)-5-((S)-3-(((1-(4-(1-(4-hydroxyphenyl)-2-phenylbut-1-en-1-yl)phenyl)piperidin-4-yl)methyl)amino)piperidin-1-yl)isoindoline-1,3-dione |
| | 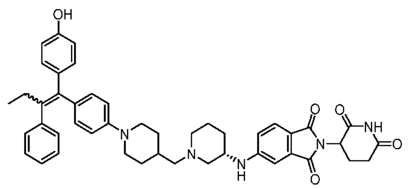 | 2-(2,6-dioxopiperidin-3-yl)-5-(((S)-1-((1-(4-(1-(4-hydroxyphenyl)-2-phenylbut-1-en-1-yl)phenyl)piperidin-4-yl)methyl)piperidin-3-yl)amino)isoindoline-1,3-dione | 2-(2,6-dioxopiperidin-3-yl)-5-(((S)-1-((1-(4-(1-(4-hydroxyphenyl)-2-phenylbut-1-en-1-yl)phenyl)piperidin-4-yl)methyl)piperidin-3-yl)amino)isoindoline-1,3-dione |
| | 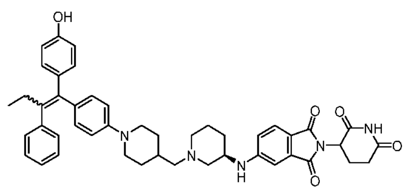 | 2-(2,6-dioxopiperidin-3-yl)-5-(((R)-1-((1-(4-(1-(4-hydroxyphenyl)-2-phenylbut-1-en-1-yl)phenyl)piperidin-4-yl)methyl)piperidin-3-yl)amino)isoindoline-1,3-dione | 2-(2,6-dioxopiperidin-3-yl)-5-(((R)-1-((1-(4-(1-(4-hydroxyphenyl)-2-phenylbut-1-en-1-yl)phenyl)piperidin-4-yl)methyl)piperidin-3-yl)amino)isoindoline-1,3-dione |
| | 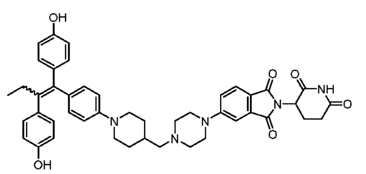 | 5-(4-((1-(4-(1,2-bis(4-hydroxyphenyl)but-1-en-1-yl)phenyl)piperidin-4-yl)methyl)piperazin-1-yl)-2-(2,6-dioxopiperidin-3-yl)isoindoline-1,3-dione | 5-(4-((1-(4-(1,2-bis(4-hydroxyphenyl)but-1-en-1-yl)phenyl)piperidin-4-yl)methyl)piperazin-1-yl)-2-(2,6-dioxopiperidin-3-yl)isoindoline-1,3-dione |
| | 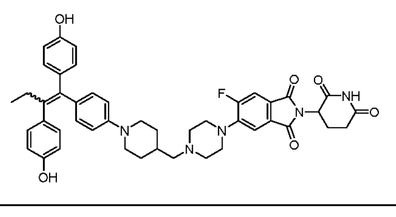 | 5-(4-((1-(4-(1,2-bis(4-hydroxyphenyl)but-1-en-1-yl)phenyl)piperidin-4-yl)methyl)piperazin-1-yl)-2-(2,6-dioxopiperidin-3-yl)-6-fluoroisoindoline-1,3-dione | 5-(4-((1-(4-(1,2-bis(4-hydroxyphenyl)but-1-en-1-yl)phenyl)piperidin-4-yl)methyl)piperazin-1-yl)-2-(2,6-dioxopiperidin-3-yl)-6-fluoroisoindoline-1,3-dione |

| | | | |
|---|---|---|---|
| | 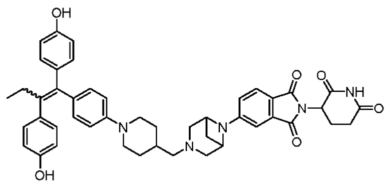 | 5-(3-((1-(4-(1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)isoindoline-1,3-dione | 5-(3-((1-(4-(1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)isoindoline-1,3-dione |
| | 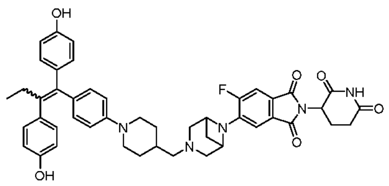 | 5-(3-((1-(4-(1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione | 5-(3-((1-(4-(1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione |
| | 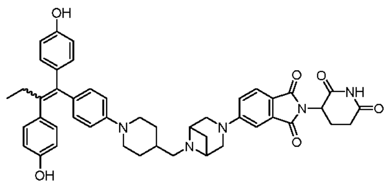 | 5-(6-((1-(4-(1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)isoindoline-1,3-dione | 5-(6-((1-(4-(1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)isoindoline-1,3-dione |
| | 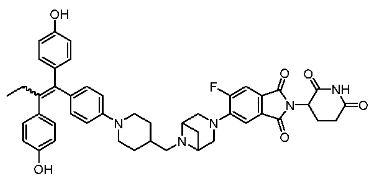 | 5-(6-((1-(4-(1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)-6-fluoroisoindoline-1,3-dione | 5-(6-((1-(4-(1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)-6-fluoroisoindoline-1,3-dione |
| | 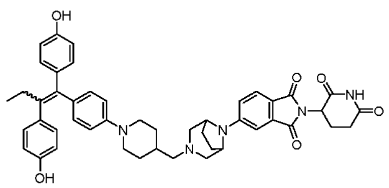 | 5-(3-((1-(4-(1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)isoindoline-1,3-dione | 5-(3-((1-(4-(1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)isoindoline-1,3-dione |
| | 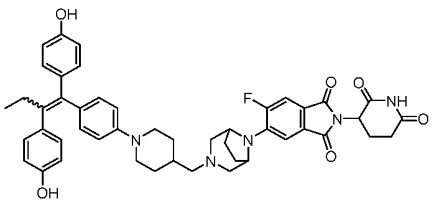 | 5-(3-((1-(4-(1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)-6-fluoroisoindoline-1,3-dione | 5-(3-((1-(4-(1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)-6-fluoroisoindoline-1,3-dione |
| | 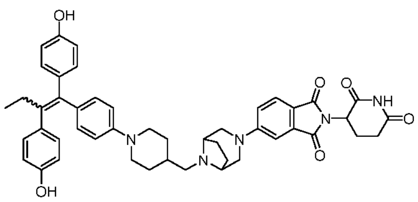 | 5-(8-((1-(4-(1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)isoindoline-1,3-dione | 5-(8-((1-(4-(1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)isoindoline-1,3-dione |

| | | | |
|---|---|---|---|
| | 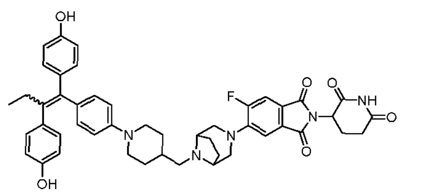 | 5-(8-((1-(4-(1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione | 5-(8-((1-(4-(1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione |
| | 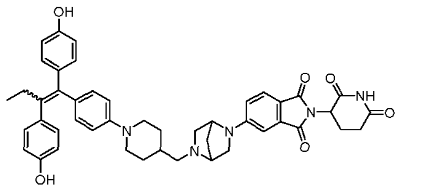 | 5-(5-((1-(4-(1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)isoindoline-1,3-dione | 5-(5-((1-(4-(1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)isoindoline-1,3-dione |
| | 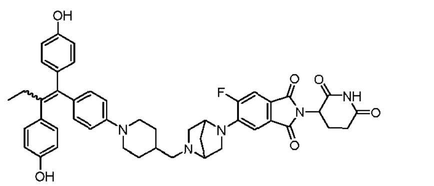 | 5-(5-((1-(4-(1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione | 5-(5-((1-(4-(1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione |
| | 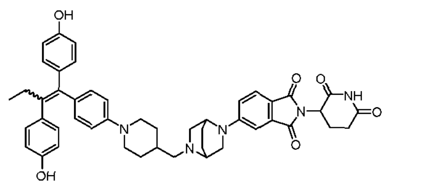 | 5-(5-((1-(4-(1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)isoindoline-1,3-dione | 5-(5-((1-(4-(1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)isoindoline-1,3-dione |
| | 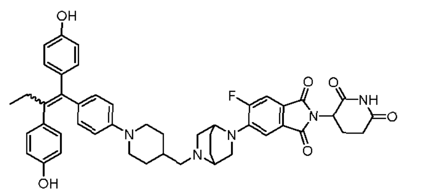 | 5-(5-((1-(4-(1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione | 5-(5-((1-(4-(1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione |
| | 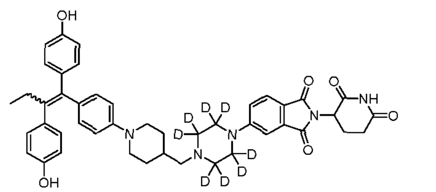 | 5-(4-((1-(4-(1,2-bis(4-hydroxyphenyl)but-1-en-1-yl)phenyl)piperidin-4-yl)methyl)piperazin-1-yl-2,2,3,3,5,5,6,6-*d*$_8$)-2-(2,6-dioxopiperidin-3-yl)isoindoline-1,3-dione | 5-(4-((1-(4-(1,2-bis(4-hydroxyphenyl)but-1-en-1-yl)phenyl)piperidin-4-yl)methyl)piperazin-1-yl-2,2,3,3,5,5,6,6-*d*$_8$)-2-(2,6-dioxopiperidin-3-yl)isoindoline-1,3-dione |
| | 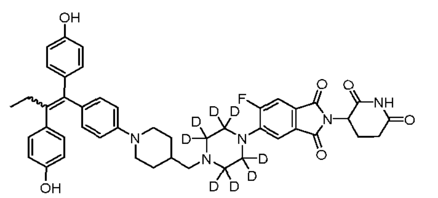 | 5-(4-((1-(4-(1,2-bis(4-hydroxyphenyl)but-1-en-1-yl)phenyl)piperidin-4-yl)methyl)piperazin-1-yl-2,2,3,3,5,5,6,6-*d*$_8$)-2-(2,6-dioxopiperidin-3-yl)-6-fluoroisoindoline-1,3-dione | 5-(4-((1-(4-(1,2-bis(4-hydroxyphenyl)but-1-en-1-yl)phenyl)piperidin-4-yl)methyl)piperazin-1-yl-2,2,3,3,5,5,6,6-*d*$_8$)-2-(2,6-dioxopiperidin-3-yl)-6-fluoroisoindoline-1,3-dione |

| | | | |
|---|---|---|---|
| | 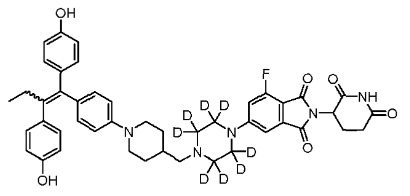 | 6-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 6-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 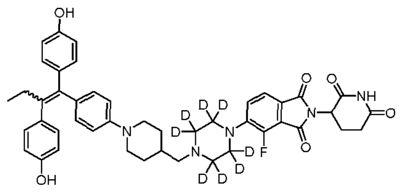 | 5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione | 5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione |
| | 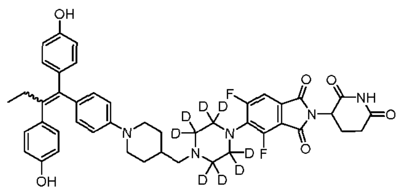 | 5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione | 5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione |
| | 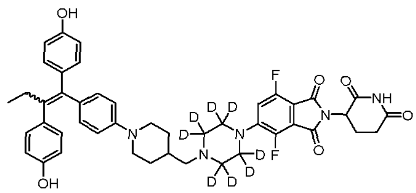 | 5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione | 5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione |
| | 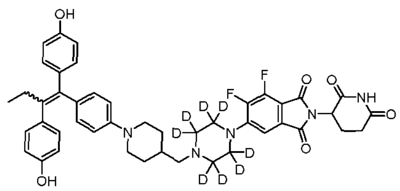 | 6-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione | 6-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione |
| | 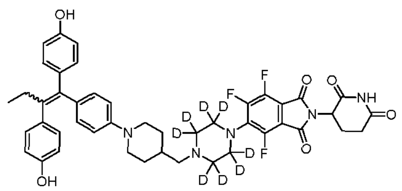 | 5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione | 5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione |
| | 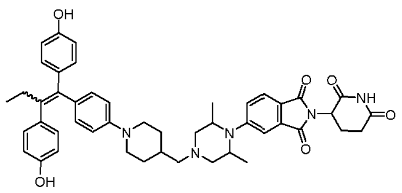 | 5-(4-((1-(4-(1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)isoindoline-1,3-dione | 5-(4-((1-(4-(1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)isoindoline-1,3-dione |

| | | | |
|---|---|---|---|
| | 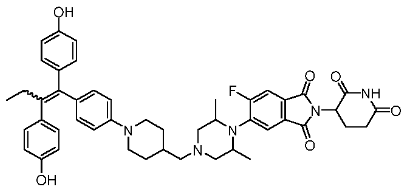 | 5-(4-((1-(4-(1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)-6-fluoroisoindoline-1,3-dione | 5-(4-((1-(4-(1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)-6-fluoroisoindoline-1,3-dione |
| | 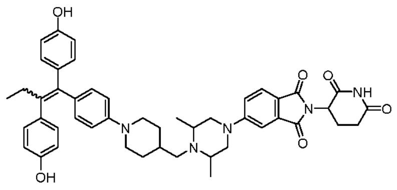 | 5-(4-((1-(4-(1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)isoindoline-1,3-dione | 5-(4-((1-(4-(1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)isoindoline-1,3-dione |
| | 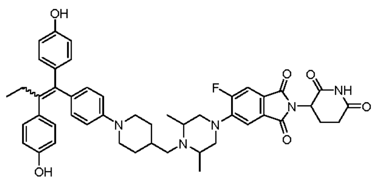 | 5-(4-((1-(4-(1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)-6-fluoroisoindoline-1,3-dione | 5-(4-((1-(4-(1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)-6-fluoroisoindoline-1,3-dione |
| | 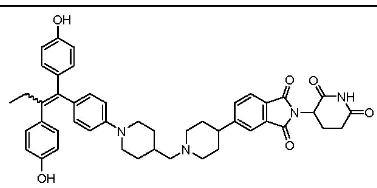 | 5-(1-((1-(4-(1,2-bis(4-hydroxyphenyl)but-1-en-1-yl)phenyl)piperidin-4-yl)methyl)piperidin-4-yl)-2-(2,6-dioxopiperidin-3-yl)isoindoline-1,3-dione | 5-(1-((1-(4-(1,2-bis(4-hydroxyphenyl)but-1-en-1-yl)phenyl)piperidin-4-yl)methyl)piperidin-4-yl)-2-(2,6-dioxopiperidin-3-yl)isoindoline-1,3-dione |
| | 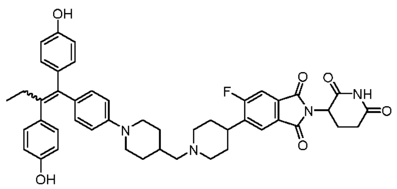 | 5-(1-((1-(4-(1,2-bis(4-hydroxyphenyl)but-1-en-1-yl)phenyl)piperidin-4-yl)methyl)piperidin-4-yl)-2-(2,6-dioxopiperidin-3-yl)-6-fluoroisoindoline-1,3-dione | 5-(1-((1-(4-(1,2-bis(4-hydroxyphenyl)but-1-en-1-yl)phenyl)piperidin-4-yl)methyl)piperidin-4-yl)-2-(2,6-dioxopiperidin-3-yl)-6-fluoroisoindoline-1,3-dione |
| | 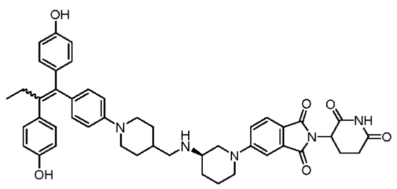 | 5-((R)-3-(((1-(4-(1,2-bis(4-hydroxyphenyl)but-1-en-1-yl)phenyl)piperidin-4-yl)methyl)amino)piperidin-1-yl)-2-(2,6-dioxopiperidin-3-yl)isoindoline-1,3-dione | 5-((R)-3-(((1-(4-(1,2-bis(4-hydroxyphenyl)but-1-en-1-yl)phenyl)piperidin-4-yl)methyl)amino)piperidin-1-yl)-2-(2,6-dioxopiperidin-3-yl)isoindoline-1,3-dione |
| | 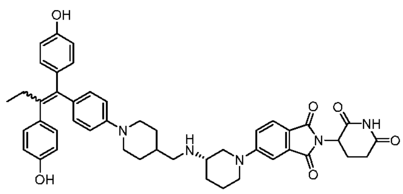 | 5-((S)-3-(((1-(4-(1,2-bis(4-hydroxyphenyl)but-1-en-1-yl)phenyl)piperidin-4-yl)methyl)amino)piperidin-1-yl)-2-(2,6-dioxopiperidin-3-yl)isoindoline-1,3-dione | 5-((S)-3-(((1-(4-(1,2-bis(4-hydroxyphenyl)but-1-en-1-yl)phenyl)piperidin-4-yl)methyl)amino)piperidin-1-yl)-2-(2,6-dioxopiperidin-3-yl)isoindoline-1,3-dione |
| | 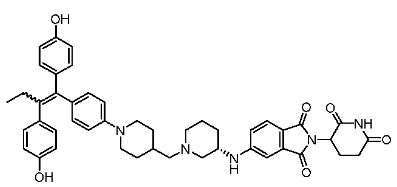 | 5-(((S)-1-((1-(4-(1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione | 5-(((S)-1-((1-(4-(1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione |

| | | | |
|---|---|---|---|
| | 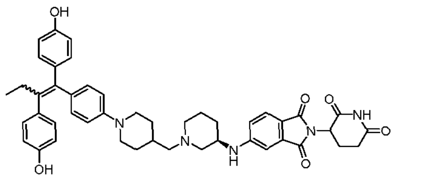 | 5-(((R)-1-((1-(4-(1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione | 5-(((R)-1-((1-(4-(1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione |
| | 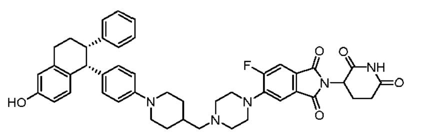 | 2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl)isoindoline-1,3-dione | 2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl)isoindoline-1,3-dione |
| | 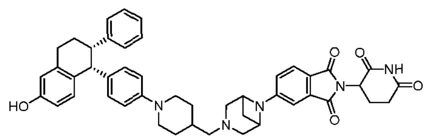 | 2-(2,6-dioxopiperidin-3-yl)-5-(3-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-6-yl)isoindoline-1,3-dione | 2-(2,6-dioxopiperidin-3-yl)-5-(3-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-6-yl)isoindoline-1,3-dione |
| | 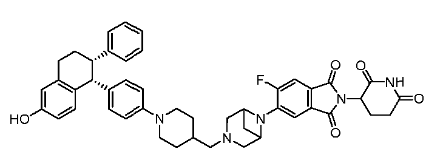 | 2-(2,6-dioxopiperidin-3-yl)-5-fluoro-6-(3-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-6-yl)isoindoline-1,3-dione | 2-(2,6-dioxopiperidin-3-yl)-5-fluoro-6-(3-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-6-yl)isoindoline-1,3-dione |
| | 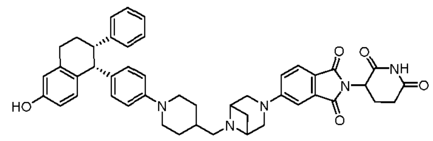 | 2-(2,6-dioxopiperidin-3-yl)-5-(6-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-3-yl)isoindoline-1,3-dione | 2-(2,6-dioxopiperidin-3-yl)-5-(6-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-3-yl)isoindoline-1,3-dione |
| | 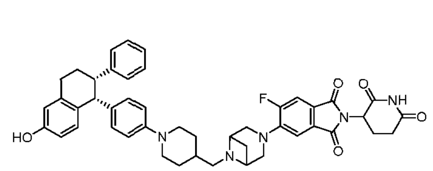 | 2-(2,6-dioxopiperidin-3-yl)-5-fluoro-6-(6-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-3-yl)isoindoline-1,3-dione | 2-(2,6-dioxopiperidin-3-yl)-5-fluoro-6-(6-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-3-yl)isoindoline-1,3-dione |
| | 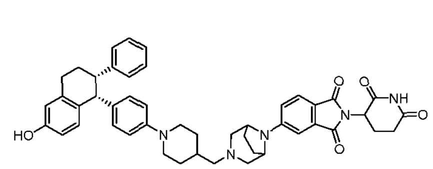 | 2-(2,6-dioxopiperidin-3-yl)-5-(3-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-8-yl)isoindoline-1,3-dione | 2-(2,6-dioxopiperidin-3-yl)-5-(3-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-8-yl)isoindoline-1,3-dione |

| | | | |
|---|---|---|---|
| | 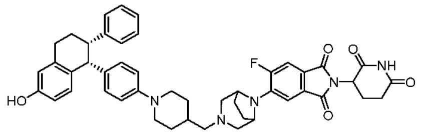 | 2-(2,6-dioxopiperidin-3-yl)-5-fluoro-6-(3-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-8-yl)isoindoline-1,3-dione | 2-(2,6-dioxopiperidin-3-yl)-5-fluoro-6-(3-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-8-yl)isoindoline-1,3-dione |
| | 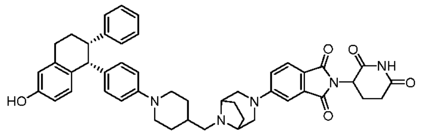 | 2-(2,6-dioxopiperidin-3-yl)-5-(8-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-3-yl)isoindoline-1,3-dione | 2-(2,6-dioxopiperidin-3-yl)-5-(8-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-3-yl)isoindoline-1,3-dione |
| | 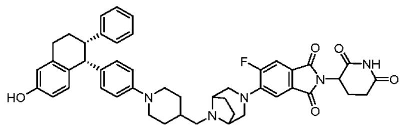 | 2-(2,6-dioxopiperidin-3-yl)-5-fluoro-6-(8-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-3-yl)isoindoline-1,3-dione | 2-(2,6-dioxopiperidin-3-yl)-5-fluoro-6-(8-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-3-yl)isoindoline-1,3-dione |
| | 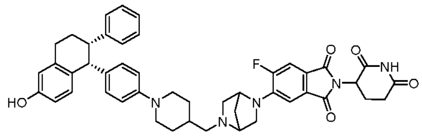 | 2-(2,6-dioxopiperidin-3-yl)-5-fluoro-6-(5-((1-(4-((1R,2S)-6-hydroxy-2-phenyl-1,2,3,4-tetrahydronaphthalen-1-yl)phenyl)piperidin-4-yl)methyl)-2,5-diazabicyclo[2.2.1]heptan-2-yl)isoindoline-1,3-dione | 2-(2,6-dioxopiperidin-3-yl)-5-fluoro-6-(5-((1-(4-((1R,2S)-6-hydroxy-2-phenyl-1,2,3,4-tetrahydronaphthalen-1-yl)phenyl)piperidin-4-yl)methyl)-2,5-diazabicyclo[2.2.1]heptan-2-yl)isoindoline-1,3-dione |
| | 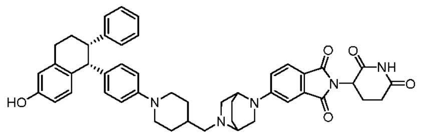 | 2-(2,6-dioxopiperidin-3-yl)-5-(5-((1-(4-((1R,2S)-6-hydroxy-2-phenyl-1,2,3,4-tetrahydronaphthalen-1-yl)phenyl)piperidin-4-yl)methyl)-2,5-diazabicyclo[2.2.2]octan-2-yl)isoindoline-1,3-dione | 2-(2,6-dioxopiperidin-3-yl)-5-(5-((1-(4-((1R,2S)-6-hydroxy-2-phenyl-1,2,3,4-tetrahydronaphthalen-1-yl)phenyl)piperidin-4-yl)methyl)-2,5-diazabicyclo[2.2.2]octan-2-yl)isoindoline-1,3-dione |
| | 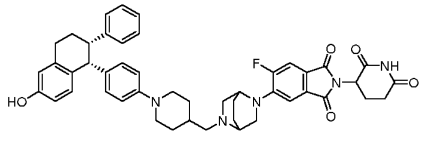 | 2-(2,6-dioxopiperidin-3-yl)-5-fluoro-6-(5-((1-(4-((1R,2S)-6-hydroxy-2-phenyl-1,2,3,4-tetrahydronaphthalen-1-yl)phenyl)piperidin-4-yl)methyl)-2,5-diazabicyclo[2.2.2]octan-2-yl)isoindoline-1,3-dione | 2-(2,6-dioxopiperidin-3-yl)-5-fluoro-6-(5-((1-(4-((1R,2S)-6-hydroxy-2-phenyl-1,2,3,4-tetrahydronaphthalen-1-yl)phenyl)piperidin-4-yl)methyl)-2,5-diazabicyclo[2.2.2]octan-2-yl)isoindoline-1,3-dione |
| | 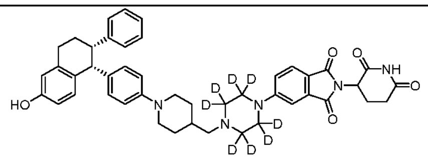 | 2-(2,6-dioxopiperidin-3-yl)-5-(4-((1-(4-((1R,2S)-6-hydroxy-2-phenyl-1,2,3,4-tetrahydronaphthalen-1-yl)phenyl)piperidin-4-yl)methyl)piperazin-1-yl- | 2-(2,6-dioxopiperidin-3-yl)-5-(4-((1-(4-((1R,2S)-6-hydroxy-2-phenyl-1,2,3,4-tetrahydronaphthalen-1-yl)phenyl)piperidin-4-yl)methyl)piperazin-1-yl- |

| | | | |
|---|---|---|---|
| | | 2,2,3,3,5,5,6,6-$d_8$)isoindoline-1,3-dione | 2,2,3,3,5,5,6,6-$d_8$)isoindoline-1,3-dione |
| | 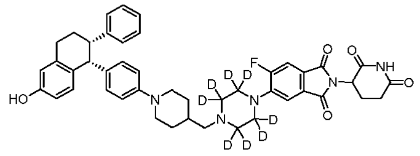 | 2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione | 2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione |
| | 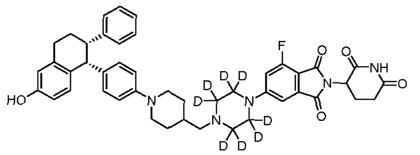 | 2-(2,6-dioxopiperidin-3-yl)-4-fluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione |
| | 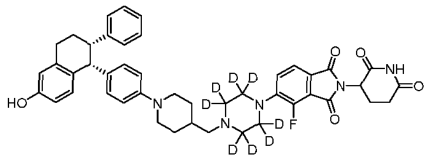 | 2-(2,6-dioxopiperidin-3-yl)-4-fluoro-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione |
| | 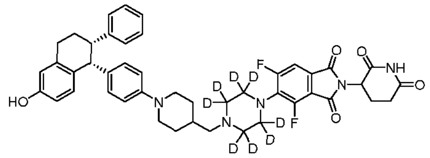 | 2-(2,6-dioxopiperidin-3-yl)-4,6-difluoro-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione | 2-(2,6-dioxopiperidin-3-yl)-4,6-difluoro-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione |
| | 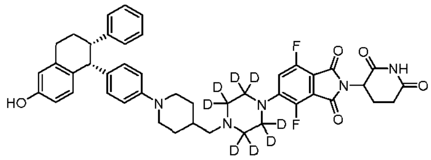 | 2-(2,6-dioxopiperidin-3-yl)-4,7-difluoro-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione | 2-(2,6-dioxopiperidin-3-yl)-4,7-difluoro-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione |
| | 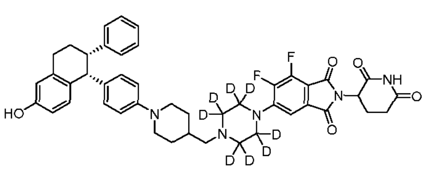 | 2-(2,6-dioxopiperidin-3-yl)-4,5-difluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione | 2-(2,6-dioxopiperidin-3-yl)-4,5-difluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione |

| | | | |
|---|---|---|---|
| | 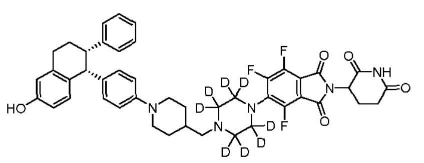 | 2-(2,6-dioxopiperidin-3-yl)-4,5,7-trifluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione | 2-(2,6-dioxopiperidin-3-yl)-4,5,7-trifluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione |
| | 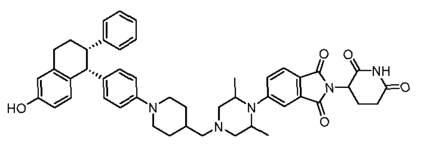 | 2-(2,6-dioxopiperidin-3-yl)-5-(4-((1-(4-((1R,2S)-6-hydroxy-2-phenyl-1,2,3,4-tetrahydronaphthalen-1-yl)phenyl)piperidin-4-yl)methyl)-2,6-dimethylpiperazin-1-yl)isoindoline-1,3-dione | 2-(2,6-dioxopiperidin-3-yl)-5-(4-((1-(4-((1R,2S)-6-hydroxy-2-phenyl-1,2,3,4-tetrahydronaphthalen-1-yl)phenyl)piperidin-4-yl)methyl)-2,6-dimethylpiperazin-1-yl)isoindoline-1,3-dione |
| | 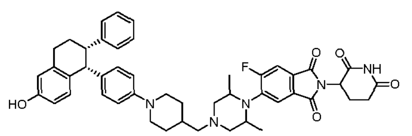 | 2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)-2,6-dimethylpiperazin-1-yl)isoindoline-1,3-dione | 2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)-2,6-dimethylpiperazin-1-yl)isoindoline-1,3-dione |
| | 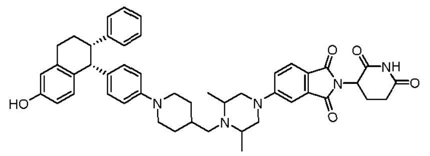 | 2-(2,6-dioxopiperidin-3-yl)-5-(4-((1-(4-((1R,2S)-6-hydroxy-2-phenyl-1,2,3,4-tetrahydronaphthalen-1-yl)phenyl)piperidin-4-yl)methyl)-3,5-dimethylpiperazin-1-yl)isoindoline-1,3-dione | 2-(2,6-dioxopiperidin-3-yl)-5-(4-((1-(4-((1R,2S)-6-hydroxy-2-phenyl-1,2,3,4-tetrahydronaphthalen-1-yl)phenyl)piperidin-4-yl)methyl)-3,5-dimethylpiperazin-1-yl)isoindoline-1,3-dione |
| | 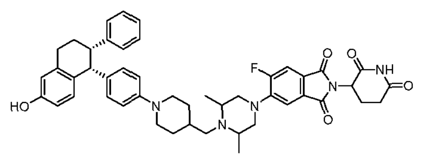 | 2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)-3,5-dimethylpiperazin-1-yl)isoindoline-1,3-dione | 2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)-3,5-dimethylpiperazin-1-yl)isoindoline-1,3-dione |
| | 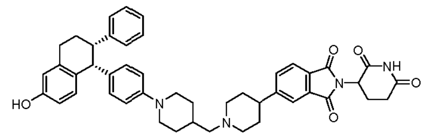 | 2-(2,6-dioxopiperidin-3-yl)-5-(1-((1-(4-((1R,2S)-6-hydroxy-2-phenyl-1,2,3,4-tetrahydronaphthalen-1-yl)phenyl)piperidin-4-yl)methyl)piperidin-4-yl)isoindoline-1,3-dione | 2-(2,6-dioxopiperidin-3-yl)-5-(1-((1-(4-((1R,2S)-6-hydroxy-2-phenyl-1,2,3,4-tetrahydronaphthalen-1-yl)phenyl)piperidin-4-yl)methyl)piperidin-4-yl)isoindoline-1,3-dione |
| | 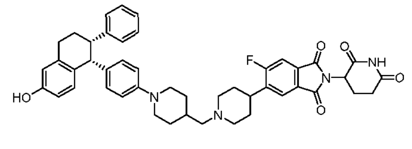 | 2-(2,6-dioxopiperidin-3-yl)-5-fluoro-6-(1-((1-(4-((1R,2S)-6-hydroxy-2-phenyl-1,2,3,4-tetrahydronaphthalen-1-yl)phenyl)piperidin-4-yl)methyl)piperidin-4-yl)isoindoline-1,3-dione | 2-(2,6-dioxopiperidin-3-yl)-5-fluoro-6-(1-((1-(4-((1R,2S)-6-hydroxy-2-phenyl-1,2,3,4-tetrahydronaphthalen-1-yl)phenyl)piperidin-4-yl)methyl)piperidin-4-yl)isoindoline-1,3-dione |

| | | | |
|---|---|---|---|
| | 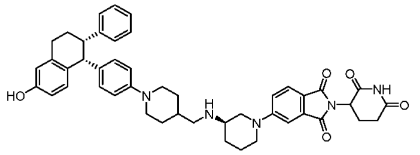 | 2-(2,6-dioxopiperidin-3-yl)-5-((R)-3-(((1-(4-((1R,2S)-6-hydroxy-2-phenyl-1,2,3,4-tetrahydronaphthalen-1-yl)phenyl)piperidin-4-yl)methyl)amino)piperidin-1-yl)isoindoline-1,3-dione | 2-(2,6-dioxopiperidin-3-yl)-5-((R)-3-(((1-(4-((1R,2S)-6-hydroxy-2-phenyl-1,2,3,4-tetrahydronaphthalen-1-yl)phenyl)piperidin-4-yl)methyl)amino)piperidin-1-yl)isoindoline-1,3-dione |
| | 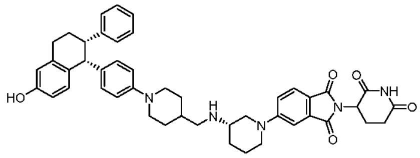 | 2-(2,6-dioxopiperidin-3-yl)-5-((S)-3-(((1-(4-((1R,2S)-6-hydroxy-2-phenyl-1,2,3,4-tetrahydronaphthalen-1-yl)phenyl)piperidin-4-yl)methyl)amino)piperidin-1-yl)isoindoline-1,3-dione | 2-(2,6-dioxopiperidin-3-yl)-5-((S)-3-(((1-(4-((1R,2S)-6-hydroxy-2-phenyl-1,2,3,4-tetrahydronaphthalen-1-yl)phenyl)piperidin-4-yl)methyl)amino)piperidin-1-yl)isoindoline-1,3-dione |
| | 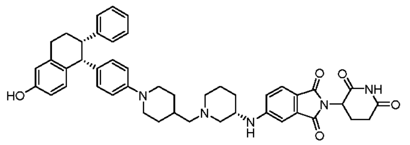 | 2-(2,6-dioxopiperidin-3-yl)-5-(((S)-1-((1-(4-((1R,2S)-6-hydroxy-2-phenyl-1,2,3,4-tetrahydronaphthalen-1-yl)phenyl)piperidin-4-yl)methyl)piperidin-3-yl)amino)isoindoline-1,3-dione | 2-(2,6-dioxopiperidin-3-yl)-5-(((S)-1-((1-(4-((1R,2S)-6-hydroxy-2-phenyl-1,2,3,4-tetrahydronaphthalen-1-yl)phenyl)piperidin-4-yl)methyl)piperidin-3-yl)amino)isoindoline-1,3-dione |
| | 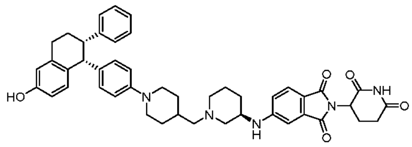 | 2-(2,6-dioxopiperidin-3-yl)-5-(((R)-1-((1-(4-((1R,2S)-6-hydroxy-2-phenyl-1,2,3,4-tetrahydronaphthalen-1-yl)phenyl)piperidin-4-yl)methyl)piperidin-3-yl)amino)isoindoline-1,3-dione | 2-(2,6-dioxopiperidin-3-yl)-5-(((R)-1-((1-(4-((1R,2S)-6-hydroxy-2-phenyl-1,2,3,4-tetrahydronaphthalen-1-yl)phenyl)piperidin-4-yl)methyl)piperidin-3-yl)amino)isoindoline-1,3-dione |
| | 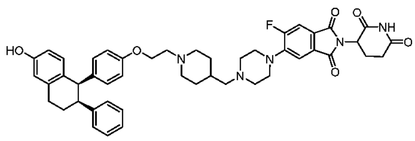 | 2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl)isoindoline-1,3-dione | 2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl)isoindoline-1,3-dione |
| | 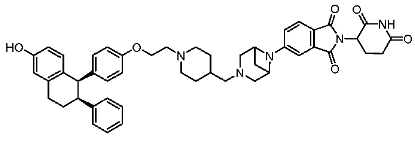 | 2-(2,6-dioxopiperidin-3-yl)-5-(3-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-6-yl)isoindoline-1,3-dione | 2-(2,6-dioxopiperidin-3-yl)-5-(3-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-6-yl)isoindoline-1,3-dione |
| | 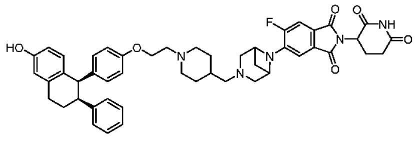 | 2-(2,6-dioxopiperidin-3-yl)-5-fluoro-6-(3-((1-(2-(4-((1R,2S)-6-hydroxy-2-phenyl-1,2,3,4-tetrahydronaphthalen-1-yl)phenoxy)ethyl)piperidin-4-yl)methyl)-3,6- | 2-(2,6-dioxopiperidin-3-yl)-5-fluoro-6-(3-((1-(2-(4-((1R,2S)-6-hydroxy-2-phenyl-1,2,3,4-tetrahydronaphthalen-1-yl)phenoxy)ethyl)piperidin-4-yl)methyl)-3,6- |

| | | | |
|---|---|---|---|
| | | diazabicyclo[3.1.1]heptan-6-yl)isoindoline-1,3-dione | diazabicyclo[3.1.1]heptan-6-yl)isoindoline-1,3-dione |
| | 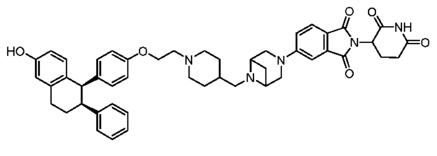 | 2-(2,6-dioxopiperidin-3-yl)-5-(6-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-3-yl)isoindoline-1,3-dione | 2-(2,6-dioxopiperidin-3-yl)-5-(6-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-3-yl)isoindoline-1,3-dione |
| | 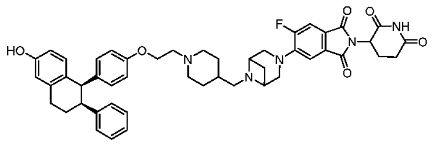 | 2-(2,6-dioxopiperidin-3-yl)-5-fluoro-6-(6-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-3-yl)isoindoline-1,3-dione | 2-(2,6-dioxopiperidin-3-yl)-5-fluoro-6-(6-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-3-yl)isoindoline-1,3-dione |
| | 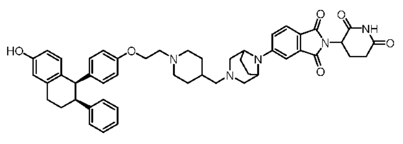 | 2-(2,6-dioxopiperidin-3-yl)-5-(3-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-8-yl)isoindoline-1,3-dione | 2-(2,6-dioxopiperidin-3-yl)-5-(3-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-8-yl)isoindoline-1,3-dione |
| | 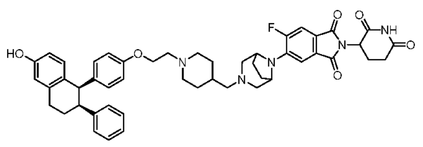 | 2-(2,6-dioxopiperidin-3-yl)-5-fluoro-6-(3-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-8-yl)isoindoline-1,3-dione | 2-(2,6-dioxopiperidin-3-yl)-5-fluoro-6-(3-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-8-yl)isoindoline-1,3-dione |
| | 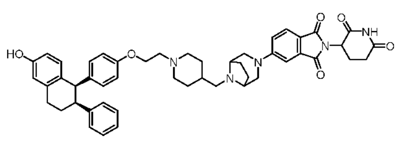 | 2-(2,6-dioxopiperidin-3-yl)-5-(8-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-3-yl)isoindoline-1,3-dione | 2-(2,6-dioxopiperidin-3-yl)-5-(8-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-3-yl)isoindoline-1,3-dione |
| | 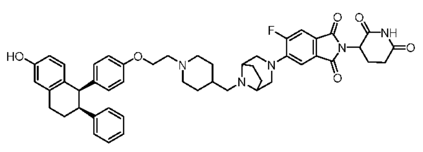 | 2-(2,6-dioxopiperidin-3-yl)-5-fluoro-6-(8-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-3-yl)isoindoline-1,3-dione | 2-(2,6-dioxopiperidin-3-yl)-5-fluoro-6-(8-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-3-yl)isoindoline-1,3-dione |

| | | | |
|---|---|---|---|
| | 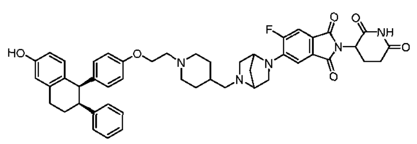 | 2-(2,6-dioxopiperidin-3-yl)-5-fluoro-6-(5-((1-(2-(4-((1R,2S)-6-hydroxy-2-phenyl-1,2,3,4-tetrahydronaphthalen-1-yl)phenoxy)ethyl)piperidin-4-yl)methyl)-2,5-diazabicyclo[2.2.1]heptan-2-yl)isoindoline-1,3-dione | 2-(2,6-dioxopiperidin-3-yl)-5-fluoro-6-(5-((1-(2-(4-((1R,2S)-6-hydroxy-2-phenyl-1,2,3,4-tetrahydronaphthalen-1-yl)phenoxy)ethyl)piperidin-4-yl)methyl)-2,5-diazabicyclo[2.2.1]heptan-2-yl)isoindoline-1,3-dione |
| | 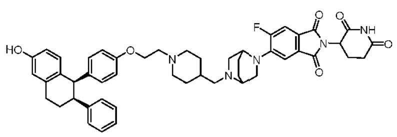 | 2-(2,6-dioxopiperidin-3-yl)-5-fluoro-6-(5-((1-(2-(4-((1R,2S)-6-hydroxy-2-phenyl-1,2,3,4-tetrahydronaphthalen-1-yl)phenoxy)ethyl)piperidin-4-yl)methyl)-2,5-diazabicyclo[2.2.2]octan-2-yl)isoindoline-1,3-dione | 2-(2,6-dioxopiperidin-3-yl)-5-fluoro-6-(5-((1-(2-(4-((1R,2S)-6-hydroxy-2-phenyl-1,2,3,4-tetrahydronaphthalen-1-yl)phenoxy)ethyl)piperidin-4-yl)methyl)-2,5-diazabicyclo[2.2.2]octan-2-yl)isoindoline-1,3-dione |
| | 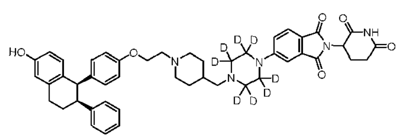 | 2-(2,6-dioxopiperidin-3-yl)-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione |
| | 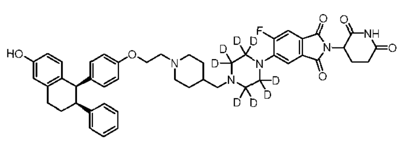 | 2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione |
| | 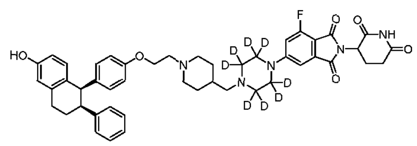 | 2-(2,6-dioxopiperidin-3-yl)-4-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione |
| | 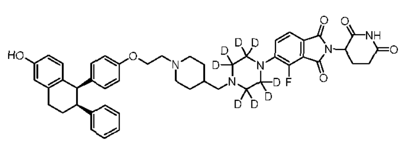 | 2-(2,6-dioxopiperidin-3-yl)-4-fluoro-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-4-fluoro-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione |
| | 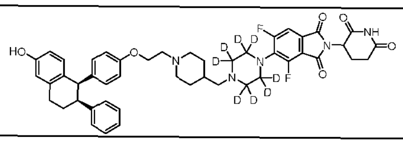 | 2-(2,6-dioxopiperidin-3-yl)-4,6-difluoro-5-(4-((1-(2-(4-((1R,2S)-6-hydroxy-2-phenyl-1,2,3,4- | 2-(2,6-dioxopiperidin-3-yl)-4,6-difluoro-5-(4-((1-(2-(4-((1R,2S)-6-hydroxy-2-phenyl-1,2,3,4- |

| | | | |
|---|---|---|---|
| | | tetrahydronaphthalen-1-yl)phenoxy)ethyl)piperidin-4-yl)methyl)piperazin-1-yl-2,2,3,3,5,5,6,6-$d_8$)isoindoline-1,3-dione | tetrahydronaphthalen-1-yl)phenoxy)ethyl)piperidin-4-yl)methyl)piperazin-1-yl-2,2,3,3,5,5,6,6-$d_8$)isoindoline-1,3-dione |
| | 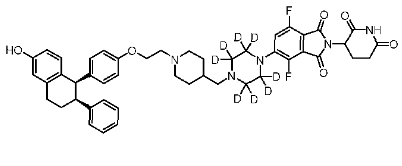 | 2-(2,6-dioxopiperidin-3-yl)-4,7-difluoro-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-4,7-difluoro-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione |
| | 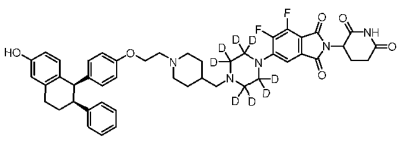 | 2-(2,6-dioxopiperidin-3-yl)-4,5-difluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-4,5-difluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione |
| | 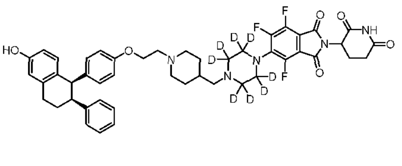 | 2-(2,6-dioxopiperidin-3-yl)-4,5,7-trifluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione | 2-(2,6-dioxopiperidin-3-yl)-4,5,7-trifluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione |
| | 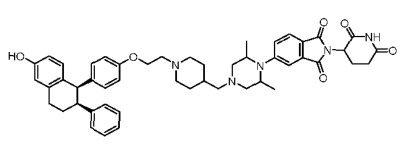 | 2-(2,6-dioxopiperidin-3-yl)-5-(4-((1-(2-(4-((1R,2S)-6-hydroxy-2-phenyl-1,2,3,4-tetrahydronaphthalen-1-yl)phenoxy)ethyl)piperidin-4-yl)methyl)-2,6-dimethylpiperazin-1-yl)isoindoline-1,3-dione | 2-(2,6-dioxopiperidin-3-yl)-5-(4-((1-(2-(4-((1R,2S)-6-hydroxy-2-phenyl-1,2,3,4-tetrahydronaphthalen-1-yl)phenoxy)ethyl)piperidin-4-yl)methyl)-2,6-dimethylpiperazin-1-yl)isoindoline-1,3-dione |
| | 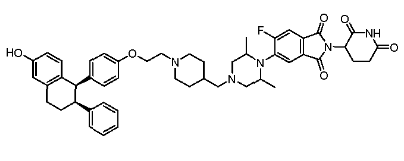 | 2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)-2,6-dimethylpiperazin-1-yl)isoindoline-1,3-dione | 2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)-2,6-dimethylpiperazin-1-yl)isoindoline-1,3-dione |
| | 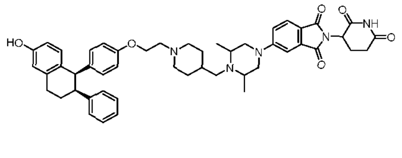 | 2-(2,6-dioxopiperidin-3-yl)-5-(4-((1-(2-(4-((1R,2S)-6-hydroxy-2-phenyl-1,2,3,4-tetrahydronaphthalen-1-yl)phenoxy)ethyl)piperidin-4-yl)methyl)-3,5-dimethylpiperazin-1-yl)isoindoline-1,3-dione | 2-(2,6-dioxopiperidin-3-yl)-5-(4-((1-(2-(4-((1R,2S)-6-hydroxy-2-phenyl-1,2,3,4-tetrahydronaphthalen-1-yl)phenoxy)ethyl)piperidin-4-yl)methyl)-3,5-dimethylpiperazin-1- |

| | | | |
|---|---|---|---|
| | | | yl)isoindoline-1,3-dione |
| | 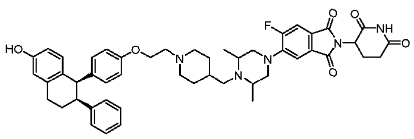 | 2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)-3,5-dimethylpiperazin-1-yl)isoindoline-1,3-dione | 2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)-3,5-dimethylpiperazin-1-yl)isoindoline-1,3-dione |
| | 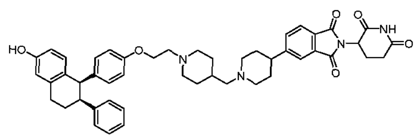 | 2-(2,6-dioxopiperidin-3-yl)-5-(1-((1-(2-(4-((1R,2S)-6-hydroxy-2-phenyl-1,2,3,4-tetrahydronaphthalen-1-yl)phenoxy)ethyl)piperidin-4-yl)methyl)piperidin-4-yl)isoindoline-1,3-dione | 2-(2,6-dioxopiperidin-3-yl)-5-(1-((1-(2-(4-((1R,2S)-6-hydroxy-2-phenyl-1,2,3,4-tetrahydronaphthalen-1-yl)phenoxy)ethyl)piperidin-4-yl)methyl)piperidin-4-yl)isoindoline-1,3-dione |
| | 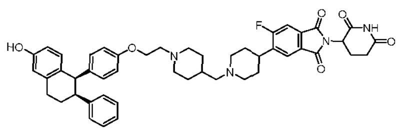 | 2-(2,6-dioxopiperidin-3-yl)-5-fluoro-6-(1-((1-(2-(4-((1R,2S)-6-hydroxy-2-phenyl-1,2,3,4-tetrahydronaphthalen-1-yl)phenoxy)ethyl)piperidin-4-yl)methyl)piperidin-4-yl)isoindoline-1,3-dione | 2-(2,6-dioxopiperidin-3-yl)-5-fluoro-6-(1-((1-(2-(4-((1R,2S)-6-hydroxy-2-phenyl-1,2,3,4-tetrahydronaphthalen-1-yl)phenoxy)ethyl)piperidin-4-yl)methyl)piperidin-4-yl)isoindoline-1,3-dione |
| | 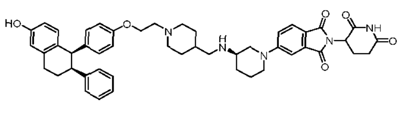 | 2-(2,6-dioxopiperidin-3-yl)-5-((R)-3-(((1-(2-(4-((1R,2S)-6-hydroxy-2-phenyl-1,2,3,4-tetrahydronaphthalen-1-yl)phenoxy)ethyl)piperidin-4-yl)methyl)amino)piperidin-1-yl)isoindoline-1,3-dione | 2-(2,6-dioxopiperidin-3-yl)-5-((R)-3-(((1-(2-(4-((1R,2S)-6-hydroxy-2-phenyl-1,2,3,4-tetrahydronaphthalen-1-yl)phenoxy)ethyl)piperidin-4-yl)methyl)amino)piperidin-1-yl)isoindoline-1,3-dione |
| | 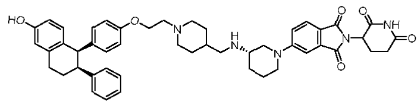 | 2-(2,6-dioxopiperidin-3-yl)-5-((S)-3-(((1-(2-(4-((1R,2S)-6-hydroxy-2-phenyl-1,2,3,4-tetrahydronaphthalen-1-yl)phenoxy)ethyl)piperidin-4-yl)methyl)amino)piperidin-1-yl)isoindoline-1,3-dione | 2-(2,6-dioxopiperidin-3-yl)-5-((S)-3-(((1-(2-(4-((1R,2S)-6-hydroxy-2-phenyl-1,2,3,4-tetrahydronaphthalen-1-yl)phenoxy)ethyl)piperidin-4-yl)methyl)amino)piperidin-1-yl)isoindoline-1,3-dione |
| | 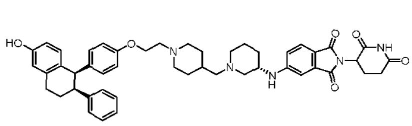 | 2-(2,6-dioxopiperidin-3-yl)-5-(((S)-1-((1-(2-(4-((1R,2S)-6-hydroxy-2-phenyl-1,2,3,4-tetrahydronaphthalen-1-yl)phenoxy)ethyl)piperidin-4-yl)methyl)piperidin-3-yl)amino)isoindoline-1,3-dione | 2-(2,6-dioxopiperidin-3-yl)-5-(((S)-1-((1-(2-(4-((1R,2S)-6-hydroxy-2-phenyl-1,2,3,4-tetrahydronaphthalen-1-yl)phenoxy)ethyl)piperidin-4-yl)methyl)piperidin-3-yl)amino)isoindoline-1,3-dione |
| | 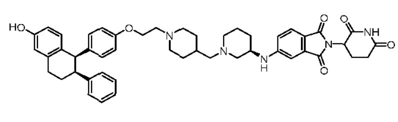 | 2-(2,6-dioxopiperidin-3-yl)-5-(((R)-1-((1-(2-(4-((1R,2S)-6-hydroxy-2-phenyl-1,2,3,4-tetrahydronaphthalen-1-yl)phenoxy)ethyl)piperidin- | 2-(2,6-dioxopiperidin-3-yl)-5-(((R)-1-((1-(2-(4-((1R,2S)-6-hydroxy-2-phenyl-1,2,3,4-tetrahydronaphthalen-1- |

| | | | |
|---|---|---|---|
| | | 4-yl)methyl)piperidin-3-yl)amino)isoindoline-1,3-dione | yl)phenoxy)ethyl)piperidin-4-yl)methyl)piperidin-3-yl)amino)isoindoline-1,3-dione |
| GT-03772 | 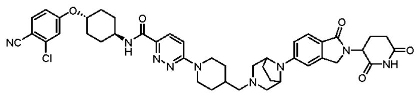 | N-((1r,4r)-4-(3-chloro-4-cyanophenoxy)cyclohexyl)-6-(4-((8-(2-(2,6-dioxopiperidin-3-yl)-1-oxoisoindolin-5-yl)-3,8-diazabicyclo[3.2.1]octan-3-yl)methyl)piperidin-1-yl)pyridazine-3-carboxamide | N-((1r,4r)-4-(3-chloro-4-cyanophenoxy)cyclohexyl)-6-(4-((8-(2-(2,6-dioxopiperidin-3-yl)-1-oxoisoindolin-5-yl)-3,8-diazabicyclo[3.2.1]octan-3-yl)methyl)piperidin-1-yl)pyridazine-3-carboxamide |
| GT-03886 | 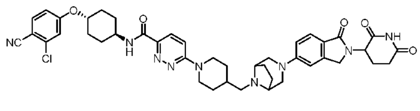 | N-((1r,4r)-4-(3-chloro-4-cyanophenoxy)cyclohexyl)-6-(4-((3-(2-(2,6-dioxopiperidin-3-yl)-1-oxoisoindolin-5-yl)-3,8-diazabicyclo[3.2.1]octan-8-yl)methyl)piperidin-1-yl)pyridazine-3-carboxamide | N-((1r,4r)-4-(3-chloro-4-cyanophenoxy)cyclohexyl)-6-(4-((3-(2-(2,6-dioxopiperidin-3-yl)-1-oxoisoindolin-5-yl)-3,8-diazabicyclo[3.2.1]octan-8-yl)methyl)piperidin-1-yl)pyridazine-3-carboxamide |
| GT-03518 | 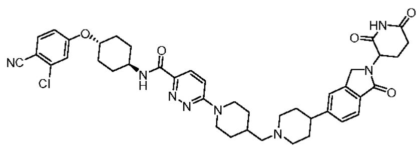 | *N*-((1r,4r)-4-(3-chloro-4-cyanophenoxy)cyclohexyl)-6-(4-((4-(2-(2,6-dioxopiperidin-3-yl)-1-oxoisoindolin-5-yl)piperidin-1-yl)methyl)piperidin-1-yl)pyridazine-3-carboxamide | *N*-((1r,4r)-4-(3-chloro-4-cyanophenoxy)cyclohexyl)-6-(4-((4-(2-(2,6-dioxopiperidin-3-yl)-1-oxoisoindolin-5-yl)piperidin-1-yl)methyl)piperidin-1-yl)pyridazine-3-carboxamide |
| GT-03519 | 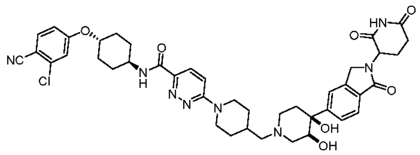 | *N*-((1r,4r)-4-(3-chloro-4-cyanophenoxy)cyclohexyl)-6-(4-(((3S,4S)-4-(2-(2,6-dioxopiperidin-3-yl)-1-oxoisoindolin-5-yl)-3,4-dihydroxypiperidin-1-yl)methyl)piperidin-1-yl)pyridazine-3-carboxamide | *N*-((1r,4r)-4-(3-chloro-4-cyanophenoxy)cyclohexyl)-6-(4-(((3S,4S)-4-(2-(2,6-dioxopiperidin-3-yl)-1-oxoisoindolin-5-yl)-3,4-dihydroxypiperidin-1-yl)methyl)piperidin-1-yl)pyridazine-3-carboxamide |
| GT-03520 | 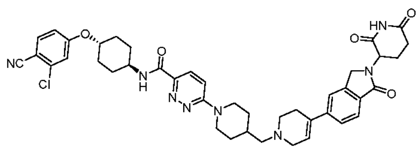 | *N*-((1r,4r)-4-(3-chloro-4-cyanophenoxy)cyclohexyl)-6-(4-((4-(2-(2,6-dioxopiperidin-3-yl)-1-oxoisoindolin-5-yl)-3,6-dihydropyridin-1(2H)-yl)methyl)piperidin-1-yl)pyridazine-3-carboxamide | *N*-((1r,4r)-4-(3-chloro-4-cyanophenoxy)cyclohexyl)-6-(4-((4-(2-(2,6-dioxopiperidin-3-yl)-1-oxoisoindolin-5-yl)-3,6-dihydropyridin-1(2H)-yl)methyl)piperidin-1-yl)pyridazine-3-carboxamide |
| GT-03525 | 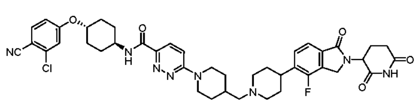 | *N*-((1r,4r)-4-(3-chloro-4-cyanophenoxy)cyclohexyl)-6-(4-((4-(2-(2,6-dioxopiperidin-3-yl)-4-fluoro-1-oxoisoindolin-5-yl)piperidin-1-yl)methyl)piperidin-1-yl)pyridazine-3-carboxamide | *N*-((1r,4r)-4-(3-chloro-4-cyanophenoxy)cyclohexyl)-6-(4-((4-(2-(2,6-dioxopiperidin-3-yl)-4-fluoro-1-oxoisoindolin-5-yl)piperidin-1-yl)methyl)piperidin-1-yl)pyridazine-3-carboxamide |

| | | | |
|---|---|---|---|
| GT-03540 | 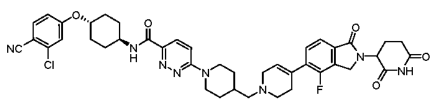 | *N*-((1r,4r)-4-(3-chloro-4-cyanophenoxy)cyclohexyl)-6-(4-((4-(2-(2,6-dioxopiperidin-3-yl)-4-fluoro-1-oxoisoindolin-5-yl)-3,6-dihydropyridin-1(2H)-yl)methyl)piperidin-1-yl)pyridazine-3-carboxamide | *N*-((1r,4r)-4-(3-chloro-4-cyanophenoxy)cyclohexyl)-6-(4-((4-(2-(2,6-dioxopiperidin-3-yl)-4-fluoro-1-oxoisoindolin-5-yl)-3,6-dihydropyridin-1(2H)-yl)methyl)piperidin-1-yl)pyridazine-3-carboxamide |
| GT-03580 | 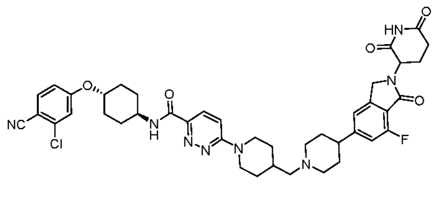 | *N*-((1r,4r)-4-(3-chloro-4-cyanophenoxy)cyclohexyl)-6-(4-((4-(2-(2,6-dioxopiperidin-3-yl)-7-fluoro-1-oxoisoindolin-5-yl)piperidin-1-yl)methyl)piperidin-1-yl)pyridazine-3-carboxamide | *N*-((1r,4r)-4-(3-chloro-4-cyanophenoxy)cyclohexyl)-6-(4-((4-(2-(2,6-dioxopiperidin-3-yl)-7-fluoro-1-oxoisoindolin-5-yl)piperidin-1-yl)methyl)piperidin-1-yl)pyridazine-3-carboxamide |
| GT-03589 | 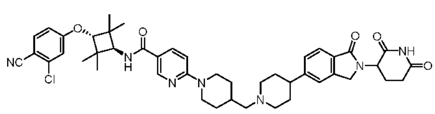 | *N*-((1r,3r)-3-(3-chloro-4-cyanophenoxy)-2,2,4,4-tetramethylcyclobutyl)-6-(4-((4-(2-(2,6-dioxopiperidin-3-yl)-1-oxoisoindolin-5-yl)piperidin-1-yl)methyl)piperidin-1-yl)nicotinamide | *N*-((1r,3r)-3-(3-chloro-4-cyanophenoxy)-2,2,4,4-tetramethylcyclobutyl)-6-(4-((4-(2-(2,6-dioxopiperidin-3-yl)-1-oxoisoindolin-5-yl)piperidin-1-yl)methyl)piperidin-1-yl)nicotinamide |
| GT-03590 | 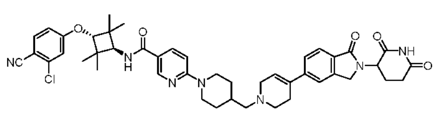 | *N*-((1r,3r)-3-(3-chloro-4-cyanophenoxy)-2,2,4,4-tetramethylcyclobutyl)-6-(4-((4-(2-(2,6-dioxopiperidin-3-yl)-1-oxoisoindolin-5-yl)-3,6-dihydropyridin-1(2H)-yl)methyl)piperidin-1-yl)nicotinamide | *N*-((1r,3r)-3-(3-chloro-4-cyanophenoxy)-2,2,4,4-tetramethylcyclobutyl)-6-(4-((4-(2-(2,6-dioxopiperidin-3-yl)-1-oxoisoindolin-5-yl)-3,6-dihydropyridin-1(2H)-yl)methyl)piperidin-1-yl)nicotinamide |
| GT-03665 | 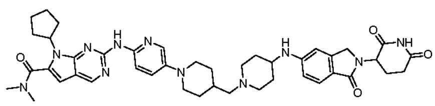 | 7-cyclopentyl-2-((5-(4-((4-((2-(2,6-dioxopiperidin-3-yl)-1-oxoisoindolin-5-yl)amino)piperidin-1-yl)methyl)piperidin-1-yl)pyridin-2-yl)amino)-N,N-dimethyl-7H-pyrrolo[2,3-d]pyrimidine-6-carboxamide | 7-cyclopentyl-2-((5-(4-((4-((2-(2,6-dioxopiperidin-3-yl)-1-oxoisoindolin-5-yl)amino)piperidin-1-yl)methyl)piperidin-1-yl)pyridin-2-yl)amino)-N,N-dimethyl-7H-pyrrolo[2,3-d]pyrimidine-6-carboxamide |
| GT-03533 | 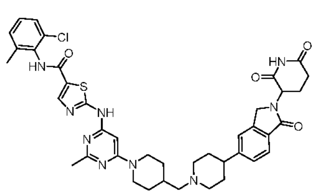 | N-(2-chloro-6-methylphenyl)-2-((6-(4-((4-(2-(2,6-dioxopiperidin-3-yl)-1-oxoisoindolin-5-yl)piperidin-1-yl)methyl)piperidin-1-yl)-2-methylpyrimidin-4-yl)amino)thiazole-5-carboxamide | N-(2-chloro-6-methylphenyl)-2-((6-(4-((4-(2-(2,6-dioxopiperidin-3-yl)-1-oxoisoindolin-5-yl)piperidin-1-yl)methyl)piperidin-1-yl)-2-methylpyrimidin-4-yl)amino)thiazole-5-carboxamide |

| | | | |
|---|---|---|---|
| GT-03534 | 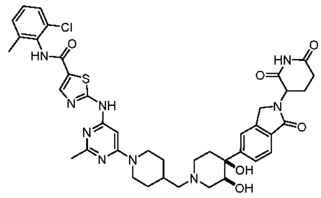 | N-(2-chloro-6-methylphenyl)-2-((6-(4-(((3S,4S)-4-(2-(2,6-dioxopiperidin-3-yl)-1-oxoisoindolin-5-yl)-3,4-dihydroxypiperidin-1-yl)methyl)piperidin-1-yl)-2-methylpyrimidin-4-yl)amino)thiazole-5-carboxamide | N-(2-chloro-6-methylphenyl)-2-((6-(4-(((3S,4S)-4-(2-(2,6-dioxopiperidin-3-yl)-1-oxoisoindolin-5-yl)-3,4-dihydroxypiperidin-1-yl)methyl)piperidin-1-yl)-2-methylpyrimidin-4-yl)amino)thiazole-5-carboxamide |

**II. Other Forms of Compounds** (including salts, enantiomers, stereoisomers, solvates, isotopically enriched analogs, prodrugs, or polymorphs of compounds)

The compounds of the present disclosure have the structures of any one of Formula (I), Formula (II), Formula (III), Formula (III-1), Formula (III-2), Formula (III-3), Formula (III-4), Formula (III-5), Formula (III-6), Formula (III-7), Formula (III-8), Formula (III-9), Formula (III-10), Formula (III-11), Formula (III-12), Formula (III-13), Formula (III-14), Formula (III-15), Formula (III-16), Formula (III-17), Formula (III-18), Formula (III-19), Formula (III-20), Formula (III-21), Formula (III-22), Formula (III-23) or Formula (III-24). Unless otherwise specified, all references to the compounds of the present disclosure also include compounds of any one of Formula (I), Formula (II), Formula (III), Formula (III-1), Formula (III-2), Formula (III-3), Formula (III-4), Formula (III-5), Formula (III-6), Formula (III-7), Formula (III-8), Formula (III-9), Formula (III-10), Formula (III-11), Formula (III-12), Formula (III-13), Formula (III-14), Formula (III-15), Formula (III-16), Formula (III-17), Formula (III-18), Formula (III-19), Formula (III-20), Formula (III-21), Formula (III-22), Formula (III-23) or Formula (III-24) and specific compounds within the scope of these general formulae.

It should be recognized that compounds of the present disclosure (including Formula (I), Formula (II), Formula (III), Formula (III-1), Formula (III-2), Formula (III-3), Formula (III-4), Formula (III-5), Formula (III-6), Formula (III-7), Formula (III-8), Formula (III-9), Formula (III-10), Formula (III-11), Formula (III-12), Formula (III-13), Formula (III-14), Formula (III-15), Formula (III-16), Formula (III-17), Formula (III-18), Formula (III-19), Formula (III-20), Formula (III-21), Formula (III-22), Formula (III-23) and Formula (III-24)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure can be pharmaceutically acceptable salts including, but not limited to, hydrohalides (including hydrochlorides and hydrobromates), sulfates, citrates, maleates, sulfonates, citrates, lactates, L-tartrates, fumarates, L-malates, hippurates, D-glucuronates, glycolates, mucates, orotates, glycinates, alaninates, argininates, cinnamates, pamoates, benzenesulfonates, methanesulfonates, acetates, propionates, valerates, triphenylacetates, L-prolinates, ferulates, 2-hydroxyethanesulfonates, nitrates, gentisates, salicylates, glutarates, stearates, camphorsulfonates, phosphates, dihydrophosphates, pyrophosphates, metaphosphates, oxalates, malonates, benzoates, mandelates, succinates, trifluoroacetates, hydroxyacetates, or p-toluenesulfonates, etc. The compounds of the present disclosure can exist as non-solvated or solvated forms in pharmaceutically acceptable solvents such as water, ethanol, and the like. In some embodiments, compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or $^2H$).

**III. Compositions/Formulations**

In some embodiments, the present disclosure provides a pharmaceutical composition comprising as active ingredient the compound of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers,

dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I), Formula (II) or Formula (III) described in the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of Formula (I), Formula (II) or Formula (III) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I), Formula (II) or Formula (III) of the present disclosure into conventional, dispersible, chewable, orally disintegrating or rapidly dissolving formulations, or sustained-release capsules or controlled-release capsules as needed.

### IV. Kits/Packaged Products

The compounds of Formula (I), Formula (II) or Formula (III) or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

### V. Methods and Uses

The compounds of Formula (I) or Formula (II) or a pharmaceutically acceptable salt, solvate, isotopically enriched analog, polymorph, prodrug, stereoisomer (including enantiomer), or mixture of stereoisomers thereof can be used as a medicament. Especially the compounds of Formula (I) or Formula (II) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of a cereblon protein-mediated disease or disorder.

The present disclosure provides a method for preventing and/or treating a cereblon protein-mediated disease or disorder in a subject, comprising administering to the subject a therapeutically effective amount of the compounds of Formula (I) or Formula (II) of the present disclosure or a pharmaceutically acceptable salt thereof, or the pharmaceutical composition of the present disclosure comprising as an active ingredient the compounds of Formula (I) or Formula (II) of the present disclosure or a pharmaceutically acceptable salt thereof. In some embodiments, the cereblon protein-mediated disease or disorder comprises: tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure,

myocardial infarction, Unverricht's syndrome, Richter syndrome (RS), acute liver failure, or diabetes. In some embodiments, the cereblon protein-mediated disease or disorder includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; congestive heart failure; myocardial infarction; multiple organ dysfunction caused by cachexia and septic shock; and acute liver failure.

In the method for preventing and/or treating a cereblon protein-mediated disease or disorder in a subject, the compound of Formula (I) or Formula (II) of the present disclosure or the pharmaceutical composition comprising as an active ingredient the compound of Formula (I) or Formula (II) of the present disclosure of the present disclosure is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

The compound of Formula (III) or a pharmaceutically acceptable salt, solvate, isotopically enriched analog, polymorph, prodrug, stereoisomer (including enantiomer), or mixture of stereoisomers thereof can be used as a medicament. Especially the compound of Formula (III) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease. In some embodiments, the disease or disorder includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; brain

glioma; astroblastoma; ovarian cancer; bronchial cancer; prostate cancer; breast cancer, including triple negative breast cancer, sporadic breast cancer and patients with Cowden syndrome; pancreatic cancer; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

The present disclosure provides a method for preventing and/or treating a disease or disorder in a subject, comprising administering to the subject a therapeutically effective amount of the compound of Formula (III) of the present disclosure or a pharmaceutically acceptable salt thereof, or the pharmaceutical composition of the present disclosure comprising as an active ingredient the compound of Formula (III) of the present disclosure or a pharmaceutically acceptable salt thereof, wherein the disease or disorder includes, but is not limited to, tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease. In some embodiments, the disease or disorder includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc.; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute

liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

The term "treatment" or "treating" refers to the administration of the compound of Formula (I), Formula (II) or Formula (III) or a pharmaceutically acceptable salt thereof according to the present disclosure, or the pharmaceutical composition containing, as an active ingredient, the compound of Formula (I), Formula (II) or Formula (III) or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

The compound of Formula (I) or Formula (II) or a pharmaceutically acceptable salt, solvate, isotopically enriched analog, polymorph, prodrug, stereoisomer (including enantiomer), or mixture of stereoisomers thereof of the present disclosure can be used to prepare the compound of Formula (III) of the present disclosure. The compound of Formula (III) of the present disclosure can be used to treat and/or prevent a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

## VI. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "U represents a bond" means that U is a bond linker. In other words, when U represents a bond, the group R in the structure of Formula (I) is directly connected to phenyl ring in the structure of Formula (I). For example, the wording "$R^{12}$ represents a bond" means that $R^{12}$ is a bond linker. In other words, when $R^{12}$ represents a bond, the ring carbon atom connected to $R^{12}$ in the structure of Formula (PBM-7) is directly connected to $R^1$ of the compound of Formula (III).

As used herein, the term "inserted" of the expression "one or more groups $R_e$ and/or one or more groups $R_f$ and/or any combination of one or more groups $R_e$ and $R_f$ are inserted into the backbone carbon chain of the linear or branched $C_{2-30}$ alkylene group", used alone or in combination, has a known definition in the art, which can mean that carbon-carbon bond between one or more pairs of adjacent carbon atoms in the referenced backbone carbon chain is interrupted by the groups $R_e$, $R_f$, or a combination of $R_e$ and Rε. Herein, examples of the above-mentioned expression "one or more groups...... are inserted into" may include, but are not limited to, that one or more (1-30, 1-20, or 1-15, 1-10, 1-9, 1-8, 1-7, 1-6, 1-5, 1-4, 1-3, or 1-2, or 1) groups $R_e$ as defined herein and/or one or more (1-30, 1-20, or 1-15, 1-10, 1-9, 1-8, 1-7, 1-6, 1-5, 1-4, 1-3, or 1-2, or 1) groups $R_f$ as defined herein and/or one or more (1-30, 1-20, or 1-15, 1-10, 1-9, 1-8, 1-7, 1-6, 1-5, 1-4, 1-3, or 1-2 , or 1) combinations of $R_e$ with $R_f$ are inserted into the backbone carbon chain, and the resulting backbone chain group conforms to the covalent bond theory. For example, the expression "one or more groups $R_e$ and/or one or more groups Rε and/or any combination of one or more groups $R_e$ and $R_f$ are inserted into the backbone carbon chain of the linear or branched $C_{2-30}$ alkylene group" can refer to that one or more (e.g., 1-30, 1-20, 1-15, 1-10, 1-8, 1-7, 1-6, 1-5, 1 -4, 1-3, 1-2, or 1) groups $R_e$ and/or $R_f$ and/or one or more combinations of $R_e$ with $R_f$ are inserted between one or more pairs of any two adjacent carbon atoms of the backbone carbon chain of the linear or branched $C_{2-30}$ alkylene group, resulting in the formation of a backbone chain group containing one or more (e.g., 1-30, 1-20, 1-15, 1-10, 1-8, 1-7, 1-6, 1-5, 1 -4, 1-3, 1-2, or 1) fragments "$-CH_2-R_e-CH_2-$" and/or one or more (e.g., 1-30, 1-20, 1-15, 1-10, 1-8, 1-7, 1-6, 1-5, 1-4, 1-3, 1-2, or 1) fragments "$-CH_2-R_f-CH_2-$" and/or one or more (e.g., 1-30, 1-20, 1-15, 1-10, 1-8, 1-7, 1-6, 1-5, 1-4, 1-3, 1-2, or 1) fragments "$-CH_2-R_e-R_f-CH_2-$", where each $R_e$ are the same or different, each $R_f$ are the same or different, and are as defined herein.

Herein, it should be understood that the expression "one or more groups $R_e$ and/or one or more groups $R_f$ or any combination of one or more groups $R_e$ and $R_f$ are optionally inserted into the backbone carbon chain of the linear or branched $C_{2-30}$ alkylene group" includes embodiments where "one or more groups $R_e$ and/or one or more groups $R_f$ or any combination of one or more groups $R_e$ and $R_f$ are inserted into the backbone carbon chain of the linear or branched $C_{2-30}$ alkylene group", as well as embodiments where "no one or more groups $R_e$ and/or one or more groups Rε or any combination of one or more groups $R_e$ and $R_f$ are inserted into the backbone carbon chain of the linear or branched $C_{2-30}$ alkylene group".

Herein, a bond interrupted by a wavy line shows the point of attachment of the depicted group to the rest of the molecule. For example, the group of Formula (ULM) depicted below

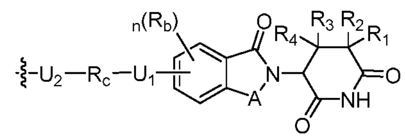

shows the point of attachment of $U_2$ in said group to LIN of the compound of Formula (III).

As used herein, the expression "a hydrogen atom of one or more $CH_2$ of the linear or branched $C_{x-y}$ alkylene is replaced by...", used alone or in combination, means that a hydrogen atom of any one or more $CH_2$ of the linear or branched $C_{x-y}$ alkylene is replaced by a substituent(s) as defined herein. Herein, the term "one or more" of "a hydrogen atom of one or more $CH_2$ of groups #-U-$CH_2$-, #-U-$(CH_2)_2$-, #-U-$(CH_2)_3$-, #-U-$(CH_2)_4$-, #-U-$(CH_2)_5$-, #-U-$(CH_2)_6$-, #-U-$(CH_2)_7$-, #-U-$(CH_2)_8$-, #-U-$(CH_2)_9$-, #-U-$(CH_2)_{10}$-, #-U-$(CH_2)_{11}$-, #-U-$(CH_2)_{12}$-, #-U-$(CH_2)_{13}$-, #-U-$(CH_2)_{14}$-, #-U-$(CH_2)_{15}$-, #-U-$(CH_2)_{16}$-, #-U-$(CH_2)_{17}$-, #-U-$(CH_2)_{18}$-, #-U-$(CH_2)_{19}$-, #-U-$(CH_2)_{20}$-, #-U-$(CH_2)_{21}$-, #-U-$(CH_2)_{22}$-, #-U-$(CH_2)_{25}$-, or #-U-$(CH_2)_{30}$-" may refer to part or all hydrogen atoms of each referenced alkylene group, including but not limited to 1-60 hydrogens. In some embodiments, the expression " a hydrogen atom of one or more $CH_2$" may refer to part or all of the hydrogen atoms of the referenced alkylene group, including but not limited to 1-30, such as 1-25, 1-20, 1-15, 1-10, 1-5, 1-4, 1-3, 1-2 or 1 hydrogen atoms. In some embodiments, the expression "a hydrogen atom of one or more $CH_2$" may include 1-3 of the plurality of hydrogen atoms of the referenced alkylene group. The number of hydrogens to be replaced is in principle not limited in any way, or is automatically limited by the size of the building unit.

As used herein, the wordings "optionally substituted" and "unsubstituted or substituted" can be used interchangeably. The term "substituted" usually means that one or more hydrogen atoms in the referenced structure are replaced by the same or different specific substituents.

As used herein, the term "oxo" or "oxo group" refers to =O.

As used herein, the term "nicotinoyl" refers to

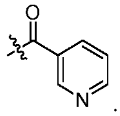

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "$C_x$-$C_y$ alkyl" or "$C_{x-y}$ alkyl" (x and y each being an integer) refers to a linear or branched alkyl group containing from x to y carbon atoms. The term "$C_{1-10}$ alkyl" used alone or in combination in the present disclosure refers to a linear or branched alkyl group containing from 1 to 10 carbon atoms. Non-limiting examples of the $C_{1-10}$ alkyl of the present disclosure may include a $C_{1-9}$ alkyl, $C_{1-8}$ alkyl, $C_{2-8}$ alkyl, $C_{1-7}$ alkyl, $C_{1-6}$ alkyl, $C_{1-5}$ alkyl, and $C_{1-4}$ alkyl. Representative examples include methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The term "$C_{1-3}$ alkyl" or "$C_1$-$C_3$ alkyl" in the present disclosure refers to an alkyl group containing from 1 to 3 carbon atoms, and representative examples thereof include methyl, ethyl, n-propyl, and isopropyl. In the present disclosure, the "alkyl" is optionally substituted with one or more substituents optionally selected from the group consisting of halogen, hydroxyl, cyano, $C_{1-3}$ alkyl, $C_{1-3}$ alkoxy, trifluoromethyl, heterocyclyl, or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group are replaced with one or more halogens. The term "halogenated $C_{x-Cy}$ alkyl" or "halogenated $C_{x-y}$ alkyl" (x and y are each an integer) refers to a linear or branched alkyl containing from x to y carbon atoms substituted with one or more halogens. The term "halogenated $C_{1-10}$ alkyl" used alone or in combination in the present invention refers to a linear or branched alkyl group containing from 1 to 10 carbon atoms substituted with one or more halogens. Examples of the halogenated $C_{1-10}$ alkyl group of the present disclosure include halogenated $C_{1-9}$ alkyl group, e.g., halogenated $C_{1-8}$ alkyl group, halogenated $C_{2-8}$ alkyl group, halogenated $C_{1-7}$ alkyl group, halogenated $C_{1-6}$ alkyl, halogenated $C_{1-5}$ alkyl, or halogenated $C_{1-4}$ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halo-$C_{1-3}$ alkyl" or "halo-$C_{1-}C_3$ alkyl" of the present disclosure refers to an alkyl group containing from 1 to 3 carbon atoms substituted with one or more halogens, and its representative examples include halomethyl, haloethyl, halo-n-propyl and haloisopropyl.

As used herein, the term "deuterated alkyl", used alone or in combination, refers to a linear or branched alkyl group substituted with one or more deuterium atoms, wherein one or more hydrogen atom(s) of the alkyl group are replaced with one or more deuterium atoms. The term "deuterated $C_{x-Cy}$ alkyl" or "deuterated $C_{x-y}$ alkyl" (x and y are each an integer) refers to a linear or branched alkyl containing from x to y carbon atoms substituted with one or more deuterium atoms. The term "deuterated $C_{1-10}$ alkyl" used alone or in combination in the present invention refers to a linear or branched alkyl group containing from 1 to 10 carbon atoms substituted with one or more deuterium atoms. Examples of the deuterated $C_{1-10}$ alkyl group of the present disclosure include deuterated $C_{1-9}$ alkyl group, e.g., deuterated $C_{1-8}$ alkyl group, deuterated $C_{2-8}$ alkyl group, deuterated $C_{1-7}$ alkyl group, deuterated $C_{1-6}$ alkyl, deuterated $C_{1-5}$ alkyl, or deuterated $C_{1-4}$ alkyl. Representative examples include perdeuterated methyl ($CD_3$), perdeuterated ethyl ($CD_3CD_2$), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, perdeuterated hexyl, perdeuterated heptyl, perdeuterated octyl, perdeuterated nonyl, and perdeuterated decyl. The term "deuterated $C_{1-3}$ alkyl" or "deuterated $C_{1-}C_3$ alkyl" of the present disclosure refers to an alkyl group containing from 1 to 3 carbon atoms substituted with one or more deuterium atoms, and its representative examples include perdeuterated methyl ($CD_3$) and perdeuterated ethyl ($CD_3CD_2$).

As used herein, the term "alkylene" (which is used interchangeably with "alkylene chain"), used alone or in combination, refers to a linear or branched divalent saturated hydrocarbon group composed of carbon and hydrogen atoms. The term "$C_x$-$C_y$ alkylene" or "$C_{x-y}$ alkylene" (x and y each being an integer) refers to a linear or branched alkylene group containing from x to y carbon atoms. Examples of the $C_1$-$C_{30}$ alkylene in the present disclosure may include $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene. Representative examples include, but are not limited to, methylene, ethylene, propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene,

pentacosylene, hexacosylene, heptacosylene, octacosylene, nonacosylene, and triacontylene. In the present disclosure, the "alkylene" is optionally substituted with one or more substituents optionally selected from $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "alkoxy", used alone or in combination, refers to a linear or branched alkoxy group having structural formula of -O-alkyl. Optionally, the alkyl portion of the alkoxy group may contain 1-10 carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "$C_1$-$C_3$ alkoxy" or "$C_{1-3}$ alkoxy" refers to a linear or branched alkoxy group containing from 1 to 3 carbon atoms. Representative examples of $C_{1-3}$ alkoxy include, but are not limited to, methoxy, ethoxy, n-propoxy, and isopropoxy.

As used herein, the term "alkylamino", used alone or in combination, refers to a linear or branched alkylamino group having structural formula of alkyl-NH-. Optionally, the alkyl portion of the alkylamino group may contain 1-10 carbon atoms. Representative examples of "alkylamino" include, but are not limited to, methyl-NH-, ethyl-NH-, n-propyl-NH-, isopropyl-NH-, n-butyl-NH-, isobutyl-NH-, tert-butyl-NH-, pentyl-NH-, and hexyl-NH-, etc. The term "$C_1$-$C_3$ alkyl-NH-" or "$C_{1-3}$ alkyl-NH-" refers to a linear or branched alkyl-NH- group containing from 1 to 3 carbon atoms. Representative examples of $C_{1-3}$ alkyl-NH- include, but are not limited to, methyl-NH-, ethyl-NH-, n-propyl-NH-, and isopropyl-NH-.

As used herein, the term "amino-substituted alkylene", used alone or in combination, refers to a linear or branched alkylene group substituted with amino having structural formula of $NH_2$-alkylene. Optionally, the alkylene portion of the amino-substituted alkylene group may contain 1-10 carbon atoms. The term "amino-substituted $C_{1-3}$ alkylene" or " $NH_2$-$C_{1-3}$ alkyl" refers to a linear or branched alkylene group containing from 1 to 3 carbon atoms which is substituted with amino. Representative examples of amino-substituted $C_{1-3}$ alkylene include, but are not limited to, $NH_2$-$CH_2$-, $NH_2$-$CH_2CH_2$-, and $NH_2$-$CH_2CH_2CH_2$-.

As used herein, the term "alkyl-NHC(O)-", used alone or in combination, refers to a linear or branched alkyl-NHC(O)- group having structural formula of alkyl-NHC(O)-. Optionally, the alkyl portion of the alkyl-NHC(O)- group may contain 1-10 carbon atoms. The term "$C_{1-3}$ alkyl-NHC(O)-" or "$C_1$-$C_3$ alkyl-NHC(O)-" refers to a linear or branched alkyl-NHC(O)- group containing from 1 to 3 carbon atoms. Representative examples of $C_{1-3}$ alkyl-NHC(O)- include, but are not limited to, $CH_3$-NHC(O)-, $CH_3CH_2$-NHC(O)-, and $CH_3CH_2CH_2$-NHC(O)-.

As used herein, the term "alkyl-C(O)NH-", used alone or in combination, refers to a linear or branched alkyl-C(O)NH- group having structural formula of alkyl-C(O)NH-. Optionally, the alkyl portion of the alkyl-C(O)NH- group may contain 1-10 carbon atoms. The term "$C_{1-3}$ alkyl-C(O)NH-" or "$C_1$-$C_3$ alkyl-C(O)NH-" refers to a linear or branched alkyl-C(O)NH- group containing from 1 to 3 carbon atoms. Representative examples of $C_{1-3}$ alkyl-C(O)NH- include, but are not limited to, $CH_3$-C(O)NH-, $CH_3CH_2$-C(O)NH-, and $CH_3CH_2CH_2$-C(O)NH-.

As used herein, the term "heteroaryl", used alone or in combination, refers to a 5- to 20-membered (e.g., 5- to 15-membered, 5- to 12-membered, 5- to 11-membered, 5- to 10-membered, 5-to 9-membered, 5- to 8-membered, 5- to 7-membered, 5- to 6-membered, 6- to 15-membered, or 6- to 9-membered) monocyclic, bicyclic, or polycyclic monovalent aromatic ring group containing one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl and imidazo[2,1-b]thiazolyl. Examples of tricyclic heteroaryl groups include, but are not limited to, acridinyl, benzindolyl, carbazolyl, dibenzofuranyl, and xanthyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "heteroarylene", used alone or in combination, refers to a 5- to 20-membered (e.g., 5- to 15-membered, 5- to 12-membered, 5- to 11-membered, 5- to 10-membered, 5-to 9-membered, 5- to 8-membered, 5- to 7-membered, 5- to 6-membered, 6- to 15-membered, or 6- to 9-membered) monocyclic, bicyclic, or polycyclic bivalent aromatic ring group containing at least one aromatic ring having one or more (e.g., from 1 to 6, or from 1 to 5,

or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Examples of tricyclic heteroarylene groups include, but are not limited to, acridinylene, benzindolylene, carbazolylene, dibenzofuranylene, and xanthylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "aryl", used alone or in combination, refers to a monovalent aromatic hydrocarbon group containing from 5 to 14 carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, or tri-substituted with a substituent(s) optionally selected from e.g., $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "arylene", used alone or in combination, refers to a divalent aromatic hydrocarbon group containing from 5 to 14 carbon atoms and optionally one or more fused rings, such as phenylene, naphthylene, or fluorenylene. As used herein, the "arylene" is optionally substituted. A substituted arylene refers to an arylene group optionally substituted one or more times (e.g., 1-4, 1-3, or 1-2 times) with a substituent(s). For example, arylene is mono-, di-, or tri-substituted with a substituent(s) optionally selected from e.g., $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated $\pi$-electron system) monocyclic or bicyclic or polycyclic cyclic hydrocarbon radical, which in some embodiments contains from 3 to 20 carbon atoms (i.e., $C_{3-20}$ cycloalkyl), or from 3 to 15 carbon atoms (i.e., $C_{3-15}$ cycloalkyl), or from 3 to 12 carbon atoms (i.e., $C_{3-12}$ cycloalkyl), or from 3 to 11 carbon atoms (i.e., $C_{3-11}$ cycloalkyl), or from 3 to 10 carbon atoms (i.e., $C_{3-10}$ cycloalkyl), or from 3 to 8 carbon atoms ( i.e., $C_{3-8}$ cycloalkyl), or from 3 to 7 carbon atoms (i.e., $C_{3-7}$ cycloalkyl), or from 3 to 6 carbon atoms (i.e., $C_{3-6}$ cycloalkyl). The term "cycloalkyl" includes monocyclic, bicyclic, or tricyclic cyclic hydrocarbon radical having from 3 to 20 carbon atoms. Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Bicyclic and tricyclic cycloalkyl groups include bridged cycloalkyl, fused cycloalkyl and spiro-cycloalkyl groups such as, but not limited to, decalinyl, octahydropentalenyl, octahydro-1H-indenyl, spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof. Examples of "$C_{3-6}$ cycloalkyl" include, but are not limited to, cyclopropyl, cyclobutyl, cyclopentyl, cyclopentenyl, and cyclohexyl.

As used herein, the term "cycloalkylene", used alone or in combination, refers to a saturated or partially unsaturated (i.e., containing one or more double bonds, but not having a fully conjugated $\pi$-electron system) monocyclic or bicyclic or polycyclic divalent cyclic hydrocarbon radical containing from 3 to 12 carbon atoms (e.g., 3-12, 3-11, 3-10, 3-8, 3-7, 3-6 carbon atoms). The term "cycloalkylene" includes monocyclic, bicyclic or tricyclic cycloalkylene having from 3 to 12 carbon atoms. Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Bicyclic and tricyclic cycloalkylene groups include bridged cycloalkylene, fused cycloalkylene and spiro-cycloalkylene groups such as, but not limited to, decalinylene, octahydropentalenylene, octahydro-1H-indenylene, 2,3-dihydro-1H-indenylene, spiro-cycloalkylene, adamantanylene, noradamantanylene, and norbomylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more substituents selected from $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "$C_{x-y}$ spiro-cycloalkylene" (x and y each being an integer), used alone or in combination, refers to a spiro-cycloalkylene group containing from x to y carbon atoms. As used herein, the term "$C_{7-11}$ spiro-cycloalkylene", used alone or in combination, refers to a spiro-cycloalkylene group containing from 7 to 11 carbon atoms (e.g., 7-10, 7-9 carbon atoms). Representative examples of "$C_{7-11}$ spiro-cycloalkylene" include, but are not limited to, spiro[3.3]heptylene, spiro[2.5]octylene, spiro[3.5]nonylene, spiro[4.4]nonylene, spiro[4.5]decylene, or spiro[5.5]undecylene. The "$C_{7-11}$ spiro-cycloalkylene" is optionally further substituted with one or more substituents selected from the group consisting of $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "heterocyclyl" or "heterocyclic group", used alone or in combination, refers to a 3- to 20-membered saturated or partially unsaturated (i.e., containing one or more double bonds, but not having a fully conjugated $\pi$-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclyl" may refer to a 3- to 15-membered (e.g., 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monocyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl), and diazacyclooctyl. Bicyclic and tricyclic heterocyclyl groups include bridged heterocyclyl, fused heterocyclyl and spiro-heterocyclyl groups such as, but not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (including 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "nitrogen-containing monocyclic heterocyclyl", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monocyclic monovalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. The nitrogen-containing monocyclic heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "nitrogen-containing bridged heterocyclyl", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) tricyclic monovalent cyclic hydrocarbon group containing one nitrogen atom and optionally one or more (e.g., from 1 to 5, or from 1 to 4, from 1 to 3, from 1 to 2, or 1) heteroatoms independently selected from sulfur,

oxygen, and nitrogen. Tricyclic heterocyclyl groups include bridged heterocyclyl, such as but not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, and 2,5-diazabicyclo[2.2.2]octan-2-yl. The nitrogen-containing bridged heterocyclyl may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "heterocyclylene", used alone or in combination, refers to a 3- to 20-membered saturated or partially unsaturated (i.e., containing one or more double bonds, but not having a fully conjugated $\pi$-electron system) monocyclic, bicyclic, or tricyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 3- to 15-membered (e.g., 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monocyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, and diazacycloheptanylene (e.g., 1,4-diazacycloheptanylene, 4,5-diazacycloheptanylene, 1,3-diazacycloheptanylene). Bicyclic and tricyclic heterocyclylene groups include bridged heterocyclylene, fused heterocyclylene and spiro-heterocyclylene groups such as, but not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "nitrogen-containing monocyclic heterocyclylene", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) monocyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclylene include, but are not limited to, piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, and diazacyclooctylene. The nitrogen-containing monocyclic heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "nitrogen-containing bridged heterocyclylene", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated $\pi$-electron system) tricyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Tricyclic heterocyclylene groups include bridged heterocyclylene, such as but not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, and 2,5-diazabicyclo[2.2.2]octanylene. The nitrogen-containing heterocyclylene may be unsubstituted or substituted as explicitly defined (e.g., mono-, di-, tri-, or poly-substituted) by a substituent(s) optionally selected from the group consisting of deuterium, hydroxyl, amino, mercapto, nitro, halogen, cyano, optionally deuterated $C_{1-6}$ alkyl, halogenated $C_{1-6}$ alkyl, optionally deuterated $C_{3-6}$

cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "alkynylene", used alone or in combination, refers to a linear or branched bivalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynylene include, but are not limited to, ethynylene, 1-propynylene, 1-butynylene, and 1,3-diynylene.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of $C_{2-6}$ alkynylene include, but are not limited to, ethynyl, 1-propynyl, 1-butynyl, and 1,3-diynyl.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include, but are not limited to, vinylene (e.g., -CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of $C_{2-6}$ alkenyl group include, but are not limited to, vinyl (e.g., $CH_2$=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

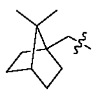, or 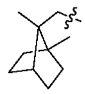.

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl.

As used herein, "adamantane" (also known as tricyclo[3.3.1.$1^{3,7}$]decane) has a definition known to those skilled in the art, and its structural formula is e.g., as follows:

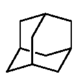.

As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows:

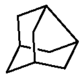 or 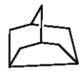.

As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.$1^{3,7}$]decan-1-amine; CAS No.: 768-94-5), with the following structural Formula:

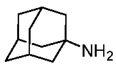 .

In the present disclosure, the term "leaving group" used alone or in combination is well known to those skilled in the art, which is a leaving molecular fragment (ion or neutral molecule) that carries a pair of electrons from a reactant in chemical reactions, as is a term used in nucleophilic substitution and elimination reactions. Common ionic leaving groups include $Cl^-$, $Br^-$, $I^-$ and sulfonate (such as p-toluenesulfonate, $TsO^-$), and neutral molecular leaving groups include water, ammonia and alcohol. In the present disclosure, those skilled in the art can select an appropriate leaving group as needed, such as but not limited to $-N_3$, halogen, methanesulfonyloxy, trifluoromethanesulfonyloxy or p-toluenesulfonyloxy, etc.

Salts or pharmaceutically acceptable salts, enantiomers, stereoisomers, solvates, polymorphs of the compounds of Formula (I), Formula (II) or Formula (III) of the present disclosure are also encompassed within the scope of the present invention.

In all embodiments of the present disclosure, the salts or pharmaceutically acceptable salts of the compounds of Formula (I), Formula (II) or Formula (III) refer to non-toxic inorganic or organic acid and/or base addition salts. Examples include: sulfates, hydrohalides (including hydrochlorides and hydrobromates), maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, para-chlorobenzenesulfonates, 1,5-naphthalenedisulfonates, 3-hydroxy-2-naphthoates, 1-hydroxy-2-naphthoates, 2-naphthalenesulfonates, hydroxyacetates, or p-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers

(rotational isomers), geometric isomers (cis/trans isomers), atropisomers, and so on.

As used herein, the term "solvate" refers to an association or complex formed by the interaction between one or more solvent molecules and compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

## Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:

| | |
|---|---|
| AcOH | acetic acid |
| Boc | t-Butyloxy carbonyl |
| Con. | concentration |
| DCM | dichloromethane |
| DIEA or DIPEA | N, N-diisopropylethylamine |
| DMF | N,N-dimethylformamide |
| DMSO | dimethyl sulfoxide |
| DIPEA | N, N-diisopropylethylamine |
| DESS-MARTIN | Dess-Martin oxidant |
| EA | ethyl acetate |
| EDCI | 1-(3-dimethylaminopropyl)-3-ethylcarbodiimide hydrochloride |
| ESI | electrospray ionization |
| equiv | equivalent |
| EtOH | ethanol |
| HATU | 2-(7-Azabenzotriazol-1 -yl)-N,N,N',N'-tetramethyl uronium hexafluorophosphate |
| HPLC | high performance liquid chromatography |
| HRMS | high resolution mass spectrometry |
| LC-MS | liquid chromatography-mass spectrometry |
| LRMS | low resolution mass spectrometry |
| LC | liquid chromatography |
| Me | methyl |
| MeCN | acetonitrile |
| MeOH | Methanol |
| MS | mass spectrometry |
| $^1$H NMR | Proton nuclear magnetic resonance |
| rt | room temperature |
| tBu | tert-butyl |
| TEA | triethylamine |
| TFA | trifluoroacetic acid |
| TLC | thin layer chromatography |
| TMS | tetramethylsilane |

In the present disclosure, the $^1$H NMR spectrum was recorded on a Bruker-500MHz nuclear magnetic resonance instrument, by using, as a solvent, $CD_3OD$ ($\delta$ = 3.31 ppm) containing 0.1% TMS (as an internal standard); or using, as a solvent, $CDCl_3$ ($\delta$ = 7.26 ppm) containing 0.1% TMS (as an internal standard); or using, as a solvent, DMSO-$d_6$ ($\delta$ =

2.50 ppm) containing 0.03% TMS (as an internal standard). LRMS spectrum was recorded on an AB Triple 4600 mass spectrometer, HPLC preparation was measured on a SHIMADZU LC-20AP type instrument, and HPLC purity was measured on a SHIMADZU LC-30AP or Waters 1525 type instrument. Unless otherwise specified, all reactions were performed in the air atmosphere. The reactions were monitored via TLC or LC-MS.

Solvents and reagents are processed as follows:

the solvents used in the reactions such as DCM, DMF, anhydrous EtOH, and anhydrous MeOH were commercially available, e.g., from Chinese Sinopharm Group;
HPLC preparation was performed by using preparative grade $CH_3CN$ and deionized water;
unless otherwise specified, various derivatives of PBM, various carbon chain linkers of different lengths (i.e., compounds used to form the groups of LIN), as well as other reagents and drugs were commercially available.

Unless otherwise specified, the materials and reagents used in the following examples are commercially available and used directly, or can be synthesized by using methods known in the art.

**General synthesis methods**

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I), Formula (II) and Formula (III) of the present disclosure according to routine techniques in the art.

The first general synthesis method of compounds of Formula (I):

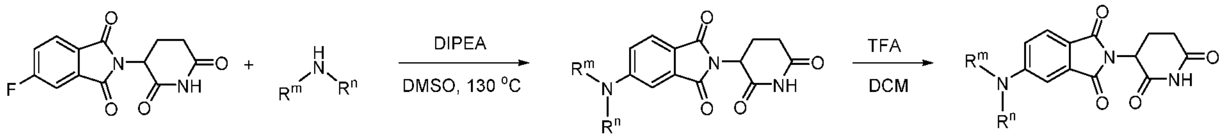


Scheme 1

In Scheme 1, $R^m$ of the substrate amine $R^m$-NH-$R^n$ represents hydrogen, and $R^n$ represents a nitrogen-containing heterocyclyl. Alternatively, $R^m$ and $R^n$, together with the nitrogen atom to which they are connected, form 4- to 10-membered monocyclic or bridged heterocyclyl containing 1-2 nitrogen atoms. One of the two nitrogen atoms in $R^m$-NH-$R^n$ is protected by Boc.

The amine alkylation reaction in Step 1 of Scheme 1 can be carried out under conventional conditions well-known to those skilled in the art. For example, the amine alkylation reaction can also be performed at room temperature to 80°C in the presence of DIEA and sodium iodide, or triethylamine and sodium iodide.

For example, Step 1: to a solution of 2-(2,6-dioxopiperidin-3-yl)-5-fluoroisoindoline-1,3-dione in DMSO (4 mL) were added the corresponding amine (1.1 equiv) and N,N-diisopropylethylamine (3 equiv). The reaction mixture was reacted at 130 °C for 3h. After monitoring the reaction and confirming its completion, the reaction mixture was extracted using ethyl acetate. The organic phases were combined, washed with water, and dried over anhydrous sodium sulfate, and filtered. The filtrate was concentrated under reduced pressure, and the resulting residue was subjected to a column chromatography (DCM: EtOAc = 2:1 to 1:1) for separation to give the Boc intermediate compound.

Step 2: to a solution of the product from step 1 in DCM (5 mL) was added $CF_3CO_2H$ (1 mL). The reaction mixture was reacted at room temperature for 3h, and concentrated under reduced pressure to give trifluoroacetate salt of the target compound.

The second general synthesis method of compounds of Formula (I):

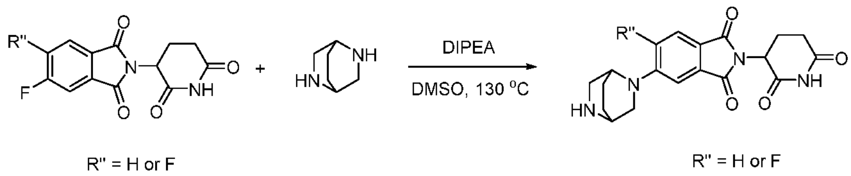


Scheme 2

To a solution of the corresponding diketone (1.0 equiv) in DMSO (2 mL) were added 2,5-diazabicyclo[2.2.2]octane (2.0 equiv) and N,N-diisopropylethylamine (3 equiv). The reaction mixture was reacted at 130 °C for 3h. After monitoring the reaction and confirming its completion, the reaction mixture was subjected to a C18 reversed-phase column chromatography (eluent (v/v): acetonitrile/(water+0.05%HCl) = 10% -100%) for separation. The collected fractions were rotary evaporated to remove acetonitrile, and the resulting residue was lyophilized to afford the hydrochloride of the target compound.

The amine alkylation reaction in Scheme 2 can be carried out under conventional conditions well-known to those skilled in the art. For example, the amine alkylation reaction can also be performed at room temperature to 80°C in the presence of DIEA and sodium iodide, or triethylamine and sodium iodide.

The first general synthesis method of compounds of Formula (II):

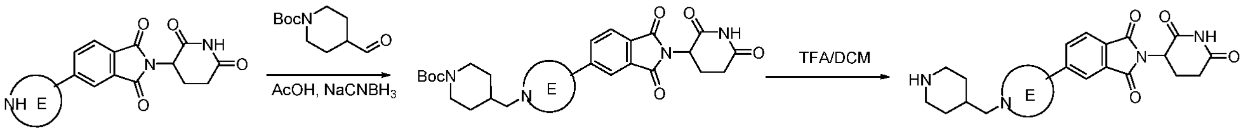


Scheme 3

In Scheme 3, ring E represents the nitrogen-containing heterocyclyl as defined herein.

The reduction amination reaction in step 1 and the deprotection in step 2 of Scheme 3 can be conventional techniques and methods well-known to those skilled in the art. For example, the reduction amination reaction can be carried out in the presence of sodium triacetoxyborohydride and 1,2-dichloroethane at room temperature to 80°C. The deprotection can be carried out under the conditions of HCl/1,4-dioxane.

For example, Step 1: to a solution of tert-butyl 4-formylpiperidine-1-carboxylate (1.0 equiv) and amine (1.0 equiv) in dichloromethane was added an appropriate amount of acetic acid. The reaction mixture was allowed to react at room temperature for 30 minutes, followed by addition of sodium cyanoborohydride (3.0 equiv) and continued reaction at room temperature. After monitoring the reaction via LCMS and confirming its completion, the reaction mixture was subjected to a C18 reversed-phase column chromatography (eluent (v/v): acetonitrile/(water+0.05%HCl) = 10% -100%) for separation. The collected fractions were rotary evaporated to remove acetonitrile, and the resulting residue was lyophilized to give the Boc intermediate compound.

Step 2: to a solution of the product from step 1 in DCM (5 mL) was added $CF_3CO_2H$ (1 mL). The reaction mixture was reacted at room temperature for 3h, and concentrated under reduced pressure to give trifluoroacetate of the target compound.

General synthesis method of compounds of Formula (III):

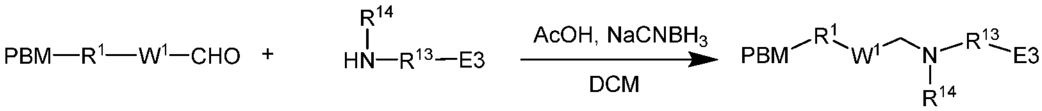


Scheme 4

In Scheme 4, PBM, $R_1$, and $W_1$ are as defined in the compounds of Formula (III) of the present disclosure. The substrate $R^{14}$-NH-$R^{13}$-E3 is a form of the compounds of Formula (I), wherein E3 represents, for example, the domides core moiety in the compounds of Formula (I); the group $R^{14}$ represents hydrogen, and $R^{13}$ represents a nitrogen-containing heterocyclyl. Alternatively, $R^{14}$ and $R^{13}$, together with the nitrogen atom to which they are connected, form 4- to 10-membered monocyclic or bridged heterocyclyl containing 1-2 nitrogen atoms.

The reduction amination reaction in Scheme 4 can be conventional techniques and methods well-known to those skilled in the art. For example, the reduction amination reaction can be carried out in the presence of sodium triacetoxyborohydride and 1,2-dichloroethane at room temperature to 80°C.

For example, to a solution of the corresponding aldehyde (1.0 equiv) and amine (1.0 equiv) in dichloromethane was added an appropriate amount of acetic acid. The reaction mixture was allowed to react at room temperature for 30 minutes, followed by addition of sodium cyanoborohydride (3.0 equiv) and continued reaction at room temperature. After monitoring the reaction via LCMS and confirming its completion, the reaction mixture was subjected to a C18 reversed-phase column chromatography for separation. The collected fractions were rotary evaporated to remove acetonitrile, and the resulting residue was lyophilized to give the target compound.

The third general synthesis method of compounds of Formula (I):

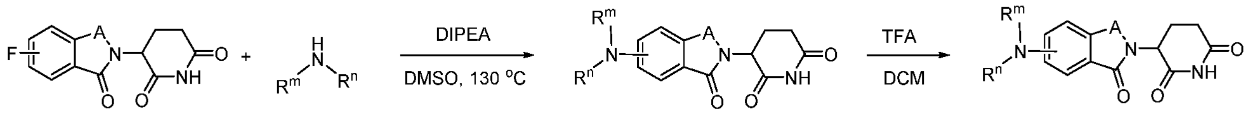


Scheme 5

In Scheme 5, A represents C(O) or $CH_2$. $R^m$ of the substrate amine $R^m$-NH-$R^n$ represents hydrogen, and $R^n$ represents a nitrogen-containing heterocyclyl. Alternatively, $R^m$ and $R^n$, together with the nitrogen atom to which they are connected, form 4- to 10-membered monocyclic or bridged heterocyclyl containing 1-2 nitrogen atoms. One of the two nitrogen atoms in $R^m$-NH-$R^n$ is protected by Boc.

The amine alkylation reaction in Step 1 of Scheme 5 can be carried out under conventional conditions well-known to those skilled in the art. For example, the amine alkylation reaction can also be performed at room temperature to 80°C in the presence of DIEA and sodium iodide, or triethylamine and sodium iodide.

The fourth general synthesis method of compounds of Formula (I):

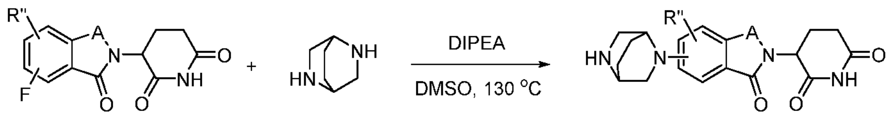


Scheme 6

In Scheme 6, A represents C(O) or $CH_2$, R" represents H or F.

The amine alkylation reaction in Scheme 6 can be carried out under conventional conditions well-known to those skilled in the art. For example, the amine alkylation reaction can also be performed at room temperature to 80°C in the presence of DIEA and sodium iodide, or triethylamine and sodium iodide.

The second general synthesis method of compounds of Formula (II):

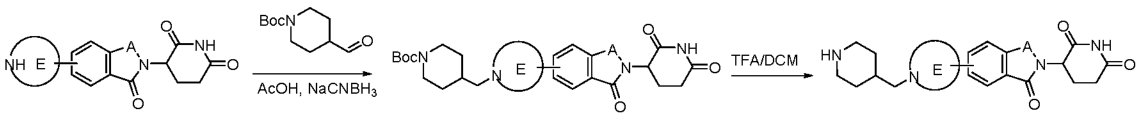


Scheme 7

In Scheme 7, A represents C(O) or $CH_2$, ring E represents nitrogen-containing heterocyclyl as defined herein.

The reduction amination reaction in step 1 and the deprotection in step 2 of Scheme 7 can be conventional techniques and methods well-known to those skilled in the art. For example, the reduction amination reaction can be carried out in the presence of sodium triacetoxyborohydride and 1,2-dichloroethane at room temperature to 80°C. The deprotection can be carried out under the conditions of HCl/1,4-dioxane.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

## Examples

### Intermediate example 1: preparation of 5-(3,5-dimethylpiperazin-1-yl)-2-(2,6-dioxopiperidin-3-yl)isoindoline-1,3-dione (GT-03099)

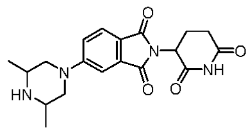

Referring to Scheme 1, 2-(2,6-dioxopiperidin-3-yl)-5-fluoroisoindoline-1,3-dione and tert-butyl 2,6-dimethylpiperazine-1-carboxylate were used to prepare the trifluoroacetate salt of the target compound (GT-03099) (yellow solid, 800.4 mg, yield 92%). $^1$H NMR (400 MHz, DMSO-$d_6$) δ 11.08 (s, 1H), 9.31 (s, 1H), 8.67 (s, 1H), 7.74 (d, *J* = 8.0 Hz, 1H), 7.51 (s, 1H), 7.37 (d, *J* = 8.0 Hz, 1H), 5.09 (dd, *J* = 12.0, 4.0 Hz, 1H), 4.25 (d, *J* = 16.0 Hz, 2H), 3.47-3.38 (m, 4H), 2.88 (q, *J* = 12.0 Hz, 2H), 2.62 - 2.50 (m, 2H), 1.31-1.01 (m, 6H). LCMS (ESI) calcd for $C_{19}H_{23}N_4O_4^+$ [M+H]$^+$, 371.42; found, 371.2.

**Intermediate example 2: preparation of 5-((R)-3-aminopiperidin-1-yl)-2-(2,6-dioxopiperidin-3-yl)isoindoline-1,3-dione (GT-03204)**

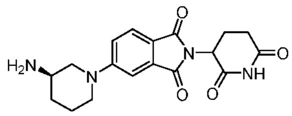

Referring to Scheme 1, 2-(2,6-dioxopiperidin-3-yl)-5-fluoroisoindoline-1,3-dione and tert-butyl (R)-piperidin-3-ylcarbamate were used to prepare the trifluoroacetate salt of the target compound (GT-03204) (yellow solid, 1.1 g, 94%). LCMS (ESI) calcd for $C_{18}H_{21}N_4O_4^+$ [M+H]$^+$, 357.39; found, 357.2.

**Intermediate example 3: preparation of 5-((S)-3-aminopiperidin-1-yl)-2-(2,6-dioxopiperidin-3-yl)isoindoline-1,3-dione (GT-03200)**

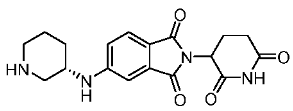

Referring to Scheme 1, 2-(2,6-dioxopiperidin-3-yl)-5-fluoroisoindoline-1,3-dione and tert-butyl (S)-3-aminopiperidin-1-carboxylate were used to prepare the trifluoroacetate salt of the target compound (GT-03202) (yellow solid, 518 mg, 55%). LCMS (ESI) calcd for $C_{18}H_{21}N_4O_4^+$ [M+H]$^+$, 357.39; found, 357.2.

**Intermediate example 4: preparation of 2-(2,6-dioxopiperidin-3-yl)-5-(((R)-piperidin-3-yl)amino)isoindoline-1,3-dione (GT-03203)**

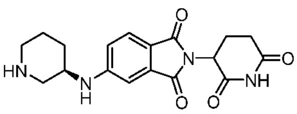

Referring to Scheme 1, 2-(2,6-dioxopiperidin-3-yl)-5-fluoroisoindoline-1,3-dione and tert-butyl (R)-3-aminopiperidin-1-carboxylate were used to prepare the trifluoroacetate salt of the target compound (GT-03203) (yellow solid, 536mg, 57%). LCMS (ESI) calcd for $C_{18}H_{21}N_4O_4^+$ [M+H]$^+$, 357.39; found, 357.2.

**Example 1: preparation of 5-(3,6-diazabicyclo[3.1.1]heptan-6-yl)-2-(2,6-dioxopiperidin-3-yl)isoindoline-1,3-dione (GT-03100)**

Referring to Scheme 1, 2-(2,6-dioxopiperidin-3-yl)-5-fluoroisoindoline-1,3-dione and tert-butyl 3,6-diazabicyclo[3.1.1]heptan-3-carboxylate were used to prepare the trifluoroacetate salt of the target compound (GT-03100) (yellow solid, 735.6 mg, yield 72%).$^1$H NMR (400 MHz, DMSO-$d_6$) δ 11.08 (s, 1H), 9.57 (s, 1H), 8.70 (s, 1H), 7.76 (d, *J* = 8.0 Hz, 1H), 7.18 (d, *J* = 4.0 Hz, 1H), 6.97 (dd, *J* = 8.0, 2.4 Hz, 1H), 5.09 (dd, *J* = 12.0, 4.0 Hz, 1H), 4.61 (d, *J* = 4.0 Hz, 2H), 3.58-3.53 (m, 2H), 3.31 (d, *J* = 12.0 Hz, 2H), 2.92-2.83 (m, 2H), 2.63-2.49 (m, 2H), 2.05-1.96 (m, 2H). LCMS (ESI) calcd for $C_{18}H_{19}N_4O_4^+$ [M+H]$^+$, 355.37; found, 355.10.

**Example 2: preparation of 5-(3,6-diazabicyclo[3.1.1]heptan-3-yl)-2-(2,6-dioxopiperidin-3-yl)isoindoline-1,3-dione (GT-03075)**

Referring to Scheme 1, 2-(2,6-dioxopiperidin-3-yl)-5-fluoroisoindoline-1,3-dione and tert-butyl 3,6-diazabicyclo[3.1.1]heptan-6-carboxylate were used to prepare the trifluoroacetate salt of the target compound (GT-03075) (yellow solid, 1.23 g, yield 80%). $^1$H NMR (400 MHz, DMSO-$d_6$) δ 11.09 (s, 1H), 9.76 (s, 1H), 8.36 (s, 1H), 7.79 (d, *J* = 12 Hz, 1H), 7.20 - 7.11 (m, 2H), 5.09 (dd, *J* = 13.0, 5.4 Hz, 1H), 4.53 (d, *J* = 4.0 Hz, 2H), 4.06-3.96 (m, 2H), 3.34 - 3.32 (m, 2H), 2.94 - 2.85 (m, 2H), 2.63 - 2.51 (m, 2H), 2.07 - 1.99 (m, 2H). LCMS (ESI) calcd for $C_{18}H_{19}N_4O_4^+$ [M+H]$^+$, 355.37; found, 355.10.

**Example 3: preparation of 5-(3,8-diazabicyclo[3.2.1]octan-8-yl)-2-(2,6-dioxopiperidin-3-yl)isoindoline-1,3-dione (GT-03210)**

Referring to Scheme 1, 2-(2,6-dioxopiperidin-3-yl)-5-fluoroisoindoline-1,3-dione and tert-butyl 3,8-diazabicyclo[3.2.1]octan-3-carboxylate were used to prepare the trifluoroacetate salt of the target compound (GT-03210) (yellow solid, 820.8 mg, yield 83%). $^1$H NMR (400 MHz, DMSO-$d_6$) δ 11.08 (s, 1H), 9.24 (s, 1H), 9.08 (s, 1H), 7.73 (d, *J* = 12.0 Hz, 1H), 7.38 (s, 1H), 7.25 (d, *J* = 12.0 Hz, 1H), 5.08 (dd, *J* = 12.0, 4.0 Hz, 1H), 4.71 (s, 2H), 3.10 (d, *J* = 4.0 Hz, 4H), 2.93-2.84 (m, 1H), 2.62 - 2.50 (m, 1H), 2.16-2.00 m, 6H). LCMS (ESI) calcd for $C_{19}H_{21}N_4O_4^+$ [M+H]$^+$, 369.40; found, 369.20.

**Example 4: preparation of 5-(3,8-diazabicyclo[3.2.1]octan-3-yl)-2-(2,6-dioxopiperidin-3-yl)isoindoline-1,3-dione (GT-03077)**

Referring to Scheme 1, 2-(2,6-dioxopiperidin-3-yl)-5-fluoroisoindoline-1,3-dione and tert-butyl 3,8-diazabicyclo[3.2.1]octan-8-carboxylate were used to prepare the trifluoroacetate salt of the target compound (GT-03077) (yellow solid, 672.1mg, yield 86%). $^1$H NMR (400 MHz, DMSO-$d_6$) δ 11.09 (s, 1H), 9.17 (s, 2H), 7.74 (d, *J* = 8.0 Hz, 1H), 7.38 (s, 1H), 7.25 (d, *J* = 8.0 Hz, 1H), 5.08 (d, *J* = 8.0 Hz, 1H), 4.19 (s, 2H), 3.97 (d, *J* = 12.0 Hz, 2H), 3.24 (d, *J* = 12.0 Hz, 2H), 2.89 (t, *J* = 12.0 Hz, 1H), 2.62-2.51 (m, 3H), 2.04-1.92 (m, 4H). LCMS (ESI) calcd for $C_{19}H_{21}N_4O_4^+$ [M+H]$^+$, 369.40; found, 369.20.

**Example 5: preparation of 5-(2,5-diazabicyclo[2.2.2]octan-2-yl)-2-(2,6-dioxopiperidin-3-yl)isoindoline-1,3-dione (GT-03076)**

Referring to Scheme 2, 2-(2,6-dioxopiperidin-3-yl)-5-fluoroisoindoline-1,3-dione and 2,5-diazabicyclo[2.2.2]octane were used to prepare the hydrochloride salt of the target compound (GT-03076) (brown solid, 173 mg, 29%). $^1$H NMR (400 MHz, DMSO-$d_6$) δ 11.06 (s, 1H), 9.45 (s, 2H), 7.70 (d, *J* = 8.0 Hz, 1H), 7.17 (s, 1H), 7.01 (d, *J* = 8.0 Hz, 1H), 5.06 (dd, *J* = 12.0, 4.0 Hz, 1H), 4.50 (s, 2H), 3.87-3.73 (m, 2H), 3.57-3.52 (m, 2H), 2.88 - 2.85 (m, 1H), 2.61-2.50 (m, 3H), 2.15 - 1.83 (m, 4H). LCMS (ESI) calcd for $C_{19}H_{21}N_4O_4^+$ [M+H]$^+$, 369.40; found, 369.30.

**Example 6: preparation of 5-(3,6-diazabicyclo[3.1.1]heptan-3-yl)-2-(2,6-dioxopiperidin-3-yl)-6-fluoroisoindoline-1,3-dione (GT-03106)**

Referring to Scheme 1, 2-(2,6-dioxopiperidin-3-yl)-5,6-difluoroisoindoline-1,3-dione and tert-butyl 3,6-diazabicyclo[3.1.1]heptane-6-carboxylate were used to prepare the trifluoroacetate salt of the target compound (GT-03106) (yellow solid, 573.4 mg, yield 87%). $^1$H NMR (400 MHz, DMSO-$d_6$) δ 11.11 (s, 1H), 9.34 (s, 1H), 8.55 (s, 1H), 7.79 (d, *J* =12.0 Hz, 1H), 7.39 (d, *J* =8.0 Hz, 1H), 5.11 (dd, *J* = 12.0, 4.0 Hz, 1H), 4.63-4.61 (m, 2H), 3.47-3.36 (m, 4H), 2.96-2.85 (m, 2H), 2.67-2.49 (m, 2H), 2.06-1.96 (m, 2H). LCMS (ESI) calcd for $C_{18}H_{18}FN_4O_4^+$ [M+H]$^+$, 373.36; found, 373.1.

**Example 7: preparation of 5-(3,6-diazabicyclo[3.1.1]heptan-6-yl)-2-(2,6-dioxopiperidin-3-yl)-6-fluoroisoindoline-1,3-dione (GT-03109)**

Referring to Scheme 1, 2-(2,6-dioxopiperidin-3-yl)-5,6-difluoroisoindoline-1,3-dione and tert-butyl 3,6-diazabicyclo[3.1.1]heptane-3-carboxylate were used to prepare the trifluoroacetate salt of the target compound (GT-03109) (yellow solid, 847.1 mg, yield 69%). LCMS (ESI) calcd for $C_{18}H_{18}FN_4O_4^+$ [M+H]$^+$, 373.36; found, 373.1.

**Example 8: preparation of 5-(3,8-diazabicyclo[3.2.1]octan-8-yl)-2-(2,6-dioxopiperidin-3-yl)-6-fluoroisoindoline-1,3-dione (GT-03211)**

Referring to Scheme 1, 2-(2,6-dioxopiperidin-3-yl)-5,6-difluoroisoindoline-1,3-dion and tert-butyl 3,8-diazabicyclo[3.2.1]octane-8-carboxylate were used to prepare the trifluoroacetate salt of the target compound (GT-03211) (1.65 mg, yield 92%). $^1$H NMR (400 MHz, DMSO-$d_6$) $\delta$ 11.12 (s, 1H), 9.09 (s, 1H), 8.85 (s, 1H), 7.78 (d, *J* =12.0 Hz, 1H), 7.57 (d, *J* =8.0 Hz, 1H), 5.11 (dd, *J* = 12.0, 8.0 Hz, 1H), 4.63 (t, *J* =4.0 Hz, 2H), 3.23-3.15 (m, 4H), 2.94-2.85 (m, 1H), 2.68-2.51 (m, 3H), 2.15-2.03 (m, 4H). LCMS (ESI) calcd for $C_{19}H_{20}FN_4O_4^+$ [M+H]$^+$, 387.39; found, 387.1.

**Example 9: preparation of 5-(3,8-diazabicyclo[3.2.1]octan-3-yl)-2-(2,6-dioxopiperidin-3-yl)-6-fluoroisoindoline-1,3-dione (GT-03107)**

Referring to Scheme 1, 2-(2,6-dioxopiperidin-3-yl)-5,6-difluoroisoindoline-1,3-dion and tert-butyl 3,8-diazabicyclo[3.2.1]octane-8-carboxylate were used to prepare the trifluoroacetate salt of the target compound (GT-03107) (yellow solid, 1.27 g, yield 86%). $^1$H NMR (400 MHz, DMSO-$d_6$) $\delta$ 11.10 (s, 1H), 9.04-8.98 (m, 2H), 7.79 (d, *J* =12.0 Hz, 1H), 7.51 (d, *J* =4.0 Hz, 1H), 5.11 (dd, *J* = 16.0, 8.0 Hz, 1H), 4.15 (s, 2H), 3.58-3.55 (m, 2H), 3.34 (d, *J* =12.0 Hz, 2H), 2.93-2.84 (m, 1H), 2.63-2.51 (m, 3H), 2.06-1.98 (m, 4H). LCMS (ESI) calcd for $C_{19}H_{20}FN_4O_4^+$ [M+H]$^+$, 387.39; found, 387.1.

**Example 10: preparation of 5-((1S,4S)-2,5-diazabicyclo[2.2.1]heptan-2-yl)-2-(2,6-dioxopiperidin-3-yl)-6-fluoroisoindoline-1,3-dione (GT-03103)**

Referring to Scheme 1, 2-(2,6-dioxopiperidin-3-yl)-5,6-difluoroisoindoline-1,3-dion and tert-butyl (1S,4S)-2,5-diazabicyclo[2.2.1]heptan-2-carboxylate were used to prepare the trifluoroacetate salt of the target compound (GT-03103) (yellow solid, 1.89 g, yield 88%). $^1$H NMR (400 MHz, DMSO-$d_6$) $\delta$ 11.09 (s, 1H), 9.06 (s, 1H), 8.57 (s, 1H), 7.72 (d, *J* =12.0 Hz, 1H), 7.32 (d, *J* =8.0 Hz, 1H), 5.08 (dd, *J* = 12.0, 4.0 Hz, 1H), 4.88 (s, 1H), 4.47 (s, 1H), 3.89 (d, *J* =12.0 Hz, 1H), 2.93-2.84 (m, 1H), 2.67-2.51 (m, 4H), 2.18 (d, *J* =8.0 Hz, 1H), 2.07-1.94 (m, 3H). LCMS (ESI) calcd for $C_{18}H_{18}FN_4O_4^+$ [M+H]$^+$, 373.36; found, 373.1.

**Example 11: preparation of 5-(3,5-dimethylpiperazin-1-yl)-2-(2,6-dioxopiperidin-3-yl)-6-fluoroisoindoline-1,3-dione (GT-03104)**

Referring to Scheme 1, 2-(2,6-dioxopiperidin-3-yl)-5,6-difluoroisoindoline-1,3-dion and tert-butyl 2,6-dimethylpiperazine-1-carboxylate were used to prepare the trifluoroacetate salt of the target compound (GT-03104) (yellow solid, 1.61 g, yield 81%). $^1$H NMR (400 MHz, DMSO-$d_6$) $\delta$ 11.11 (s, 1H), 9.16 (d, *J* =12.0 Hz, 1H), 8.51 (d, *J* =12.0 Hz, 1H), 7.80 (d, *J* =8.0 Hz, 1H), 7.63 (d, *J* =8.0 Hz, 1H), 5.12 (dd, *J* = 12.0, 4.0 Hz, 1H), 3.76 (d, *J* =12.0 Hz, 2H), 2.95-2.59 (m, 3H), 2.58-2.51 (m, 2H), 2.08-1.98 (m, 2H), 1.26 (d, *J* =6.5 Hz, 7H). LCMS (ESI) calcd for $C_{19}H_{22}FN_4O_4^+$ [M+H]$^+$, 389.41; found, 389.2.

**Example 12: preparation of 2-(2,6-dioxopiperidin-3-yl)-5-fluoro-6-(piperazin-1-yl-2,2,3,3,5,5,6,6-*$d_8$*)isoindoline-1,3-dione (GT-03108)**

Referring to Scheme 1, 2-(2,6-dioxopiperidin-3-yl)-5,6-difluoroisoindoline-1,3-dion and tert-butyl piperazine-1-carboxylate-2,2,3,3,5,5,6,6-*$d_8$* were used to prepare the trifluoroacetate salt of the target compound (GT-03108) (yellow solid, 161.0 mg, yield 48%). $^1$H NMR (400 MHz, DMSO-$d_6$) $\delta$ 11.11 (s, 1H), 8.79 (s, 2H), 7.80 (d, *J* =12.0 Hz, 1H), 7.59 (d, *J* =4.0 Hz, 1H), 5.12 (dd, *J* = 12.0, 4.0 Hz, 1H), 2.93-2.84 (m, 1H), 2.67-2.51 (m, 2H), 2.07-2.01 (m, 1H). LCMS (ESI) calcd for $C_{17}H_{10}D_8FN_4O_4^+$ [M+H]$^+$, 369.40; found, 369.2.

**Example 13: preparation of 5-(2,5-diazabicyclo[2.2.2]octan-2-yl)-2-(2,6-dioxopiperidin-3-yl)-6-fluoroisoindoline-1,3-dione (GT-03105)**

Referring to Scheme 2, 2-(2,6-dioxopiperidin-3-yl)-5,6-difluoroisoindoline-1,3-dion and 2,5-diazabicyclo[2.2.2]octane were used to prepare the hydrochloride of the target compound (GT-03105) (brown solid, 200 mg, 44%). $^1H$ NMR (400 MHz, DMSO-$d_6$) δ 11.09 (s, 1H), 9.51 (s, 2H), 7.70 (d, *J* =12.0 Hz, 1H), 7.32 (d, *J* =8.0 Hz, 1H), 5.08 (dd, *J* = 12.0, 4.0 Hz, 1H), 4.25 (s, 1H), 3.95 (d, *J* =12.0 Hz, 1H), 3.79 (s, 1H), 3.66 (d, *J* =12.0 Hz, 1H), 2.93-2.84 (m, 1H), 2.66-2.54 (m, 3H), 2.12-2.01 (m, 4H), 1.88-1.84 (m, 2H). LCMS (ESI) calcd for $C_{19}H_{20}FN_4O_4^+$ $[M+H]^+$, 387.39; found, 387.2.

**Intermediate example 5: preparation of N-((1r,4r)-4-(3-chloro-4-cyanophenoxy)cyclohexyl)-6-(4-formylpiperidin-1-yl)pyridazine-3-carboxamide**

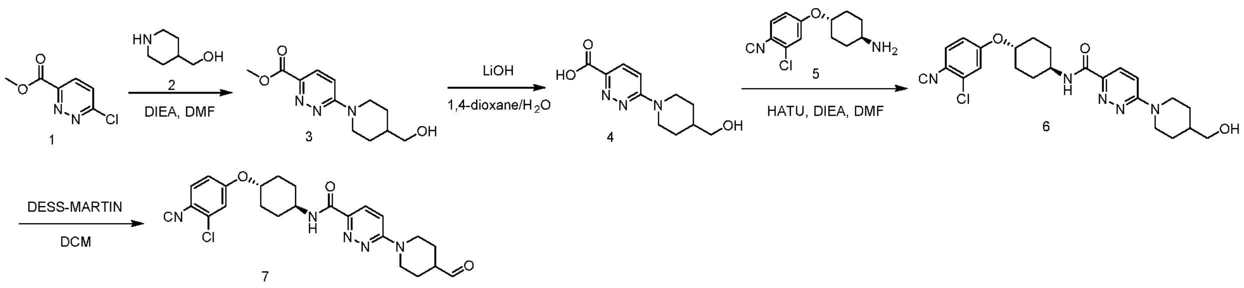


**Step 1: preparation of methyl 6-(4-(hydroxymethyl)piperidin-1-yl)pyridazine-3-carboxylate**

To a solution of methyl 6-chloropyridazine-3-carboxylate (1, 2.00 g, 11.589 mmol) and piperidin-4-ylmethanol (2, 1.406 mL, 12.169 mmol) in anhydrous DMF was added DIEA (5.746 mL, 34.768 mmol). The reaction solution was stirred at 60°C for 6h. After monitoring the reaction via LCMS and confirming its completion, water was added to the reaction solution. The resulting mixture was then extracted using ethyl acetate. The organic phases were combined, washed with saturated brine, and concentrated under reduced pressure to remove organic solvent. The resulting crude product was subjected to a silica gel column chromatography (eluent (v/v): PE to PE/EA (1/1)) for purification to give the compound methyl 6-(4-(hydroxymethyl)piperidin-1-yl)pyridazine-3-carboxylate (white solid, 2.50 g, yield 84.19%). LCMS (ESI) calcd for $C_{12}H_{18}N_3O_3^+$ $[M+H]^+$, 252.13; found, 252.20.

**Step 2: preparation of 6-(4-(hydroxymethyl)piperidin-1-yl)pyridazine-3-carboxylic acid**

To a mixed solution of methyl 6-(4-(hydroxymethyl)piperidin-1-yl)pyridazine-3-carboxylate (3, 2.5 g, 9.949 mmol) in 1,4-dioxane and water was added lithium hydroxide (1.68 g, 39.796 mmol). The reaction solution was stirred at room temperature for 5h. After monitoring the reaction via LCMS and confirming its completion, the reaction solution was concentrated under reduced pressure to remove organic solvent. The pH of the reaction solution was adjusted to 6 using concentrated HCl. The reaction solution was lyophilized using a lyophilizer to remove water, yielding 6-(4-(hydroxymethyl)piperidin-1-yl)pyridazine-3-carboxylic acid (4, pale yellow solid, 2.00 g, yield 81.36%). LCMS (ESI) calcd for $C_{11}H_{16}N_3O_3^+$ $[M+H]^+$, 238.11; found, 238.10.

**Step 3: preparation of N-((1r,4r)-4-(3-chloro-4-cyanophenoxy)cyclohexyl)-6-(4-(hydroxymethyl)piperidin-1-yl)pyridazine-3-carboxamide**

To a mixed solution of 6-(4-(hydroxymethyl)piperidin-1-yl)pyridazine-3-carboxylic acid (4, 1.00 g, 2.950 mmol), HATU (1.68 g, 4.426 mmol) and DIEA (2.438 mL, 14.752 mmol) in anhydrous DMF was added 4-(((1r,4r)-4-aminocyclohexyl)oxy)-2-chlorobenzonitrile (5, 0.68 g, 2.360 mmol). The reaction solution was stirred at room temperature for 1h. After monitoring the reaction via LCMS and confirming its completion, water was added to the reaction solution. The resulting mixture was then extracted using ethyl acetate. The organic phases were combined, washed with saturated brine, and concentrated under reduced pressure to remove organic solvent. The resulting crude product was subjected to a silica gel column chromatography (eluent (v/v): DCM to DCM/EA (50-70%)) for purification to give the compound N-((1r,4r)-4-(3-chloro-4-cyanophenoxy)cyclohexyl)-6-(4-(hydroxymethyl)piperidin-1-yl)pyridazine-3-carboxamide (6,

white solid, 1.00 g, yield 64.75%). LCMS (ESI) calcd for $C_{24}H_{29}ClN_5O_3^+$ [M+H]$^+$, 470.19; found, 470.20.

**Step 4: preparation of N-((1r,4r)-4-(3-chloro-4-cyanophenoxy)cyclohexyl)-6-(4-formylpiperidin-1-yl)pyridazine-3-carboxamide**

To a solution of N-((1r,4r)-4-(3-chloro-4-cyanophenoxy)cyclohexyl)-6-(4-(hydroxymethyl)piperidin-1-yl)pyridazine-3-carboxamide (6, 800 mg, 1.702 mmol) in DCM was added DESS-MARTIN (1.80 g, 4.256 mmol). The reaction solution was stirred at 0°C for 1h. After monitoring the reaction via LCMS and confirming its completion, saturated aqueous $NaHCO_3$ solution was added to the reaction solution. The resulting mixture was then extracted using DCM. The organic phases were combined, washed with saturated brine, and concentrated under reduced pressure to remove organic solvent. The resulting crude product was subjected to a silica gel column chromatography (eluent (v/v): DCM to DCM/EA (0-20%)) for purification to give the target compound N-((1r,4r)-4-(3-chloro-4-cyanophenoxy)cyclohexyl)-6-(4-formylpiperidin-1-yl)pyridazine-3-carboxamide (7, white solid, 690.00 mg, yield 82.29%). LCMS (ESI) calcd for $C_{24}H_{27}ClN_5O_3^+$ [M+H]$^+$, 468.17; found, 468.10.

**Example 14: preparation of N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)pyridazine-3-carboxamide (GT-03016)**

Referring to Scheme 4, N-((1r,4r)-4-(3-chloro-4-cyanophenoxy)cyclohexyl)-6-(4-formylpiperidin-1-yl)pyridazine-3-carboxamide prepared from intermediate example 15 and 2-(2,6-dioxopiperidin-3-yl)-5-fluoro-6-(piperazin-1-yl-2,2,3,3,5,5,6,6-*$d_8$*)isoindoline-1,3-dione (GT-03108) were used to prepare the target compound (GT-03016) (yellow solid, 65.1 mg, yield 37%). $^1$H NMR (400 MHz, DMSO-*d*6) δ 11.11 (s, 1H), 10.12 (s, 1H), 8.57 (d, *J* = 8.0 Hz, 1H), 7.86 - 7.80 (m, 3H), 7.59 (d, *J* = 8.0 Hz, 1H), 7.41 - 7.37 (m, 2H), 7.13 (dd, *J* = 8.0, 4.0 Hz, 1H), 5.75 (s, 1H), 5.12 (dd, *J* = 12.0, 4.0 Hz, 1H), 4.52-4.48 (m, 3H), 3.87-3.86 (m, 1H), 3.10-3.04 (m, 3H), 2.94 - 2.85 (m, 2H), 2.69 - 2.54 (m, 2H), 2.24 (s, 1H), 2.12 - 2.01 (m, 4H), 1.91-1.91 (s, 4H), 1.64 (q, *J* = 12.0 Hz, 2H), 1.52 (q, *J* = 12.0 Hz, 2H). LCMS (ESI) calcd for $C_{41}H_{36}D_8ClFN_9O_9^+$ [M+H]$^+$, 821.36; found, 821.3/823.3.

**Example 15: preparation of N-((1r,4r)-4-(3-chloro-4-cyanophenoxy)cyclohexyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazine-3-carboxamide (GT-02765)**

Referring to Scheme 4, N-((1r,4r)-4-(3-chloro-4-cyanophenoxy)cyclohexyl)-6-(4-formylpiperidin-1-yl)pyridazine-3-carboxamide and 5-(3,8-diazabicyclo[3.2.1]octan-8-yl)-2-(2,6-dioxopiperidin-3-yl)-6-fluoroisoindoline-1,3-dione (GT-03211) were used to prepare the target compound (GT-02765) (yellow solid, 45 mg, yield 49.24%). $^1$H NMR (500 MHz, DMSO) δ 11.16 (s, 1H), 10.28 (s, 1H), 8.64 (d, *J* = 8.2 Hz, 1H), 7.92 - 7.80 (m, 3H), 7.61 (d, *J* = 7.5 Hz, 1H), 7.46 - 7.38 (m, 2H), 7.17 (dd, *J* = 8.8, 2.4 Hz, 1H), 5.16 (dd, *J* = 12.9, 5.4 Hz, 1H), 4.72 (s, 2H), 4.57 (ddd, *J* = 14.2, 10.1, 3.9 Hz, 1H), 4.48 (d, *J* = 13.0 Hz, 2H), 3.93 - 3.85 (m, 1H), 3.54 (s, 2H), 3.23 (dd, *J* = 19.4, 9.6 Hz, 2H), 3.06 (t, *J* = 11.8 Hz, 2H), 3.02 - 2.86 (m, 3H), 2.70 - 2.55 (m, 2H), 2.39 (d, *J* = 7.8 Hz, 2H), 2.26 (s, 1H), 2.17 - 2.03 (m, 5H), 1.92 (d, *J* = 11.9 Hz, 4H), 1.67 (dd, *J* = 24.2, 10.5 Hz, 2H), 1.55 (dd, *J* = 22.9, 9.8 Hz, 2H), 1.30 (dd, *J* = 17.7, 7.7 Hz, 2H). LCMS (ESI) calcd for $C_{43}H_{46}ClFN_9O_6^+$ [M+H]$^+$: 838.32, found, 838.30.

**Example 16: preparation of N-((1r,4r)-4-(3-chloro-4-cyanophenoxy)cyclohexyl)-6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridazine-3-carboxamide (GT-02752)**

Referring to Scheme 4, N-((1r,4r)-4-(3-chloro-4-cyanophenoxy)cyclohexyl)-6-(4-formylpiperidin-1-yl)pyridazine-3-carboxamide and 5-(3,8-diazabicyclo[3.2.1]octan-3-yl)-2-(2,6-dioxopiperidin-3-yl)-6-fluoroisoindoline-1,3-dione (GT-03107) were used to prepare the target compound (GT-02752) (yellow solid, 20 mg, yield 21.88%). $^1$H NMR (400 MHz, DMSO) δ 11.11 (s, 1H), 8.59 (d, *J* = 8.2 Hz, 1H), 7.87 - 7.74 (m, 3H), 7.47 (d, *J* = 7.2 Hz, 1H), 7.42 - 7.34 (m, 2H), 7.13 (dd, *J* = 8.7, 2.2 Hz, 1H), 5.11 (dd, *J* = 12.7, 5.3 Hz, 1H), 4.53 (dd, *J* = 14.3, 11.0 Hz, 3H), 4.17 (s, 1H), 3.98 (d, *J* = 12.6 Hz, 1H), 3.89 - 3.82 (m, 1H), 3.56 (d, *J* = 12.4 Hz, 2H), 3.06 (t, *J* = 12.1 Hz, 3H), 2.99 - 2.92 (m, 2H), 2.90 - 2.82 (m, 1H), 2.68 - 2.53 (m, 3H), 2.40 - 2.25 (m, 2H), 2.24 - 2.20 (m, 1H), 2.13 - 1.97 (m, 7H), 1.90 (d, *J* = 12.2 Hz, 2H), 1.64 (dd, *J* = 23.0, 10.9 Hz, 2H), 1.55 - 1.45 (m, 2H), 1.34 - 1.25 (m, 2H). LCMS (ESI) calcd for $C_{43}H_{46}ClFN_9O_6^+$ [M+H]$^+$: 838.32, found, 838.30.

**Example 17: preparation of N-((1r,4r)-4-(3-chloro-4-cyanophenoxy)cyclohexyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazine-3-carboxamide (GT-03339)**

Referring to Scheme 4, N-((1r,4r)-4-(3-chloro-4-cyanophenoxy)cyclohexyl)-6-(4-formylpiperidin-1-yl)pyridazine-3-carboxamide and 5-(3,6-diazabicyclo[3.1.1]heptan-6-yl)-2-(2,6-dioxopiperidin-3-yl)isoindoline-1,3-dione (GT-03100) were used to prepare the target compound (GT-03339) (yellow solid, 30 mg, yield 56.88%). $^{1}$H NMR (400 MHz, DMSO) $\delta$ 11.25 (s, 1H), 11.09 (s, 1H), 8.57 (d, *J* = 8.2 Hz, 1H), 7.84 (dd, *J* = 9.1, 5.7 Hz, 2H), 7.77 (d, *J* = 8.2 Hz, 1H), 7.40 (dd, *J* = 18.7, 5.9 Hz, 2H), 7.18 - 7.09 (m, 2H), 6.96 (d, *J* = 8.2 Hz, 1H), 5.10 (dd, *J* = 12.8, 5.4 Hz, 1H), 4.65 (s, 2H), 4.52 (td, *J* = 9.7, 4.8 Hz, 1H), 4.41 (d, *J* = 12.9 Hz, 2H), 3.85 (dd, *J* = 7.6, 3.6 Hz, 1H), 3.77 (s, 2H), 3.39 (dt, *J* = 12.6, 6.1 Hz, 2H), 3.06 - 2.86 (m, 3H), 2.86 - 2.72 (m, 4H), 2.65 - 2.52 (m, 2H), 2.10 (d, *J* = 9.6 Hz, 3H), 2.01 (dd, *J* = 8.9, 3.6 Hz, 1H), 1.89 (d, *J* = 11.4 Hz, 4H), 1.63 (dd, *J* = 24.2, 11.2 Hz, 2H), 1.50 (dd, *J* = 22.6, 10.5 Hz, 2H), 1.19 (dt, *J* = 14.7, 7.2 Hz, 2H). LCMS (ESI) calcd for $C_{42}H_{45}ClN_9O_6^+$ [M+H]$^+$: 806.31, found, 806.30.

**Example 18: preparation of *N*-((1r,3r)-3-(3-chloro-4-cyanophenoxy)-2,2,4,4-tetramethylcyclobutyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazine-3-carboxamide (GT-03398)**

Referring to the method of Scheme 4, the target compound (GT-03398) was prepared, which is a yellow solid (44 mg, yield 49.37%). $^{1}$H NMR (400 MHz, DMSO) $\delta$ 11.12 (s, 1H), 10.27 (s, 1H), 8.23 (d, *J* = 9.2 Hz, 1H), 7.90 (d, *J* = 8.8 Hz, 1H), 7.81 (dd, *J* = 15.7, 10.9 Hz, 2H), 7.58 (d, *J* = 7.4 Hz, 1H), 7.40 (d, *J* = 9.7 Hz, 1H), 7.24 (d, *J* = 2.4 Hz, 1H), 7.03 (dd, *J* = 8.8, 2.4 Hz, 1H), 5.12 (dd, *J* = 12.8, 5.3 Hz, 1H), 4.69 (s, 2H), 4.47 (d, *J* = 9.2 Hz, 3H), 4.00 (d, *J* = 9.1 Hz, 1H), 3.47 (s, 6H), 3.26 - 3.13 (m, 2H), 2.99 (dt, *J* = 12.9, 10.3 Hz, 4H), 2.63 - 2.56 (m, 1H), 2.36 (d, *J* = 7.7 Hz, 2H), 2.27 - 2.17 (m, 1H), 2.13 - 1.98 (m, 3H), 1.89 (d, *J* = 12.2 Hz, 2H), 1.21 (s, 6H), 1.14 (s, 6H). LCMS (ESI) calcd for $C_{45}H_{50}ClFN_9O_6^+$ [M+H]$^+$: 866.35, found, 866.30.

**Example 19: preparation of *N*-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazine-3-carboxamide (GT-03399)**

Referring to the method of Scheme 4, the target compound (GT-03399) was prepared, which is a yellow solid (40 mg, yield 45.84%). $^{1}$H NMR (400 MHz, DMSO) $\delta$ 11.10 (s, 1H), 10.66 (s, 1H), 8.24 (d, *J* = 9.3 Hz, 1H), 7.91 (d, *J* = 8.7 Hz, 1H), 7.85 (dd, *J* = 9.6, 4.1 Hz, 1H), 7.76 (d, *J* = 8.4 Hz, 1H), 7.47 - 7.34 (m, 2H), 7.28 - 7.22 (m, 2H), 7.03 (dd, *J* = 8.8, 2.3 Hz, 1H), 5.09 (dd, *J* = 12.7, 5.4 Hz, 1H), 4.55 (d, *J* = 12.4 Hz, 2H), 4.46 (s, 1H), 4.20 (s, 2H), 4.08 - 3.89 (m, 3H), 3.72 (d, *J* = 12.2 Hz, 2H), 3.09 (t, *J* = 12.4 Hz, 2H), 2.97 (dd, *J* = 13.0, 7.5 Hz, 2H), 2.93 - 2.84 (m, 1H), 2.67 - 2.52 (m, 2H), 2.36 - 2.18 (m, 3H), 2.06 - 1.92 (m, 5H), 1.32 (ddd, *J* = 21.3, 12.1, 7.0 Hz, 2H), 1.22 (s, 6H), 1.14 (s, 6H). LCMS (ESI) calcd for $C_{45}H_{5l}C1N_9O_6^+$ [M+H]$^+$: 848.36, found, 848.30.

**Example 20: preparation of *N*-((1r,3r)-3-(3-chloro-4-cyanophenoxy)-2,2,4,4-tetramethylcyclobutyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazine-3-carboxamide (GT-03400)**

Referring to the method of Scheme 4, the target compound (GT-03400) was prepared, which is a yellow solid (42 mg, yield 48.94%). $^{1}$H NMR (400 MHz, DMSO) $\delta$ 11.35 (s, 1H), 11.10 (s, 1H), 8.22 (t, *J* = 8.4 Hz, 1H), 7.90 (d, *J* = 8.8 Hz, 1H), 7.84 (d, *J* = 9.6 Hz, 1H), 7.77 (d, *J* = 8.2 Hz, 1H), 7.43 (d, *J* = 9.7 Hz, 1H), 7.23 (d, *J* = 2.3 Hz, 1H), 7.16 (d, *J* = 1.3 Hz, 1H), 7.02 (dd, *J* = 8.8, 2.3 Hz, 1H), 6.96 (dd, *J* = 8.2, 1.7 Hz, 1H), 5.10 (dd, *J* = 12.8, 5.3 Hz, 1H), 4.65 (d, *J* = 4.2 Hz, 2H), 4.43 (d, *J* = 12.0 Hz, 3H), 4.00 - 3.97 (m, 1H), 3.77 (dd, *J* = 12.3, 4.1 Hz, 4H), 3.44 - 3.34 (m, 2H), 3.02 - 2.88 (m, 3H), 2.86 - 2.74 (m, 4H), 2.56 (dd, *J* = 21.7, 11.4 Hz, 2H), 2.19 - 2.08 (m, 1H), 2.00 (dd, *J* = 8.8, 3.7 Hz, 1H), 1.90 (d, *J* = 11.5 Hz, 2H), 1.21 (s, 6H), 1.13 (s, 6H). LCMS (ESI) calcd for $C_{44}H_{49}ClN_9O_6^+$ [M+H]$^+$: 834.34, found, 834.30.

**Example 21: preparation of *N*-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)pyridazine-3-carboxamide (GT-03401)**

Referring to the method of Scheme 4, the target compound (GT-03401) was prepared, which is a yellow solid (47 mg, yield 54.76%). $^{1}$H NMR (400 MHz, DMSO) $\delta$ 11.35 (s, 1H), 11.10 (s, 1H), 8.22 (t, *J* = 8.4 Hz, 1H), 7.90 (d, *J* = 8.8 Hz, 1H), 7.84 (d, *J* = 9.6 Hz, 1H), 7.77 (d, *J* = 8.2 Hz, 1H), 7.43 (d, *J* = 9.7 Hz, 1H), 7.23 (d, *J* = 2.3 Hz, 1H), 7.16 (d, *J* = 1.3 Hz, 1H), 7.02 (dd, *J* = 8.8, 2.3 Hz, 1H), 6.96 (dd, *J* = 8.2, 1.7 Hz, 1H), 5.10 (dd, *J* = 12.8, 5.3 Hz, 1H), 4.65 (d, *J*

= 4.2 Hz, 2H), 4.43 (d, *J* = 12.0 Hz, 3H), 4.00 - 3.97 (m, 1H), 3.77 (dd, *J* = 12.3, 4.1 Hz, 4H), 3.44 - 3.34 (m, 2H), 3.02 - 2.88 (m, 3H), 2.86 - 2.74 (m, 4H), 2.56 (dd, *J* = 21.7, 11.4 Hz, 2H), 2.19 - 2.08 (m, 1H), 2.00 (dd, *J* = 8.8, 3.7 Hz, 1H), 1.90 (d, *J* = 11.5 Hz, 2H), 1.21 (s, 6H), 1.13 (s, 6H). LCMS (ESI) calcd for $C_{44}H_{49}ClN_9O_6^+$ [M+H]$^+$: 834.34, found, 834.30.

**Example 22: preparation of N-((1r,3r)-3-(3-chloro-4-cyanophenoxy)-2,2,4,4-tetramethylcyclobutyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)nicotinamide (GT-03402)**

Referring to the method of Scheme 4, the target compound (GT-03402) was prepared, which is a yellow solid (51 mg, yield 58.35%). $^1$H NMR (400 MHz, DMSO) δ 11.12 (s, 1H), 10.60 (s, 1H), 8.57 (d, *J* = 1.8 Hz, 1H), 8.15 (d, *J* = 8.8 Hz, 1H), 7.91 (t, *J* = 9.2 Hz, 2H), 7.78 (d, *J* = 12.1 Hz, 1H), 7.57 (d, *J* = 7.5 Hz, 1H), 7.18 (dd, *J* = 13.9, 6.1 Hz, 2H), 7.00 (dd, *J* = 8.8, 2.3 Hz, 1H), 5.12 (dd, *J* = 12.8, 5.4 Hz, 1H), 4.68 (s, 2H), 4.40 (d, *J* = 12.8 Hz, 2H), 4.34 (s, 1H), 4.06 (d, *J* = 9.2 Hz, 1H), 3.49 (d, *J* = 10.7 Hz, 2H), 3.21 - 3.05 (m, 4H), 2.98 - 2.84 (m, 3H), 2.67 - 2.51 (m, 2H), 2.42 (d, *J* = 7.6 Hz, 2H), 2.25 (s, 1H), 2.13 - 2.00 (m, 3H), 1.91 (d, *J* = 12.3 Hz, 2H), 1.29 (dd, *J* = 17.1, 6.1 Hz, 2H), 1.22 (s, 6H), 1.12 (s, 6H). LCMS (ESI) calcd for $C_{46}H_{51}ClFN_8O_6^+$ [M+H]$^+$: 865.35, found, 865.30.

**Example 23: preparation of N-((1r,3r)-3-(3-chloro-4-cyanophenoxy)-2,2,4,4-tetramethylcyclobutyl)-6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)nicotinamide (GT-03403)**

Referring to the method of Scheme 4, the target compound (GT-03403) was prepared, which is a yellow solid (51 mg, yield 58.35%). $^1$H NMR (400 MHz, DMSO) δ 11.27 (s, 1H), 11.12 (s, 1H), 8.55 (d, *J* = 1.9 Hz, 1H), 8.14 (d, *J* = 8.5 Hz, 1H), 7.91 (t, *J* = 10.7 Hz, 2H), 7.77 (d, *J* = 10.9 Hz, 1H), 7.33 (d, *J* = 7.3 Hz, 1H), 7.18 (dd, *J* = 18.6, 5.3 Hz, 2H), 7.00 (dd, *J* = 8.8, 2.3 Hz, 1H), 5.11 (dd, *J* = 12.8, 5.4 Hz, 1H), 4.65 (s, 2H), 4.41 - 4.32 (m, 3H), 4.06 (d, *J* = 9.2 Hz, 1H), 3.85 (d, *J* = 8.6 Hz, 3H), 3.38 (dd, *J* = 11.3, 6.6 Hz, 3H), 3.04 (t, *J* = 12.3 Hz, 2H), 2.94 - 2.80 (m, 5H), 2.65 - 2.51 (m, 2H), 2.19 (s, 1H), 2.03 (d, *J* = 5.6 Hz, 1H), 1.94 (d, *J* = 10.8 Hz, 2H), 1.22 (s, 6H), 1.12 (s, 6H). LCMS (ESI) calcd for $C_{45}H_{49}ClFN_8O_6^+$ [M+H]$^+$: 851.34, found, 851.30.

**Example 24: preparation of N-((1r,3r)-3-(3-chloro-4-cyanophenoxy)-2,2,4,4-tetramethylcyclobutyl)-6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)nicotinamide (GT-03404)**

Referring to the method of Scheme 4, the target compound (GT-03404) was prepared, which is a yellow solid (13 mg, yield 14.82%). $^1$H NMR (400 MHz, DMSO) δ 11.09 (d, *J* = 15.3 Hz, 2H), 8.55 (d, *J* = 2.0 Hz, 1H), 8.10 (d, *J* = 8.8 Hz, 1H), 7.88 (dd, *J* = 21.0, 7.8 Hz, 2H), 7.77 (d, *J* = 10.9 Hz, 1H), 7.33 (d, *J* = 7.3 Hz, 1H), 7.21 (d, *J* = 2.3 Hz, 1H), 7.10 (d, *J* = 5.2 Hz, 1H), 7.00 (dd, *J* = 8.8, 2.3 Hz, 1H), 5.11 (dd, *J* = 12.8, 5.4 Hz, 1H), 4.65 (s, 2H), 4.40 - 4.32 (m, 3H), 4.05 (d, *J* = 9.1 Hz, 1H), 3.84 (d, *J* = 10.2 Hz, 2H), 3.39 (d, *J* = 9.2 Hz, 2H), 3.01 (t, *J* = 12.0 Hz, 2H), 2.96 - 2.70 (m, 6H), 2.62 - 2.56 (m, 1H), 2.21 - 2.15 (m, 1H), 2.05 - 1.99 (m, 1H), 1.96 - 1.88 (m, 2H), 1.22 (d, *J* = 5.5 Hz, 8H), 1.11 (s, 6H). LCMS (ESI) calcd for $C_{45}H_{49}ClFN_8O_6^+$ [M+H]$^+$: 851.34, found, 851.30.

**Example 25: preparation of N-((1r,3r)-3-(3-chloro-4-cyanophenoxy)-2,2,4,4-tetramethylcyclobutyl)-6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)nicotinamide (GT-03425)**

Referring to the method of Scheme 4, the target compound (GT-03425) was prepared, which is a yellow solid (41 mg, yield 47.90%). $^1$H NMR (400 MHz, DMSO) δ 11.09 (s, 1H), 10.50 (s, 1H), 8.57 (d, *J* = 1.8 Hz, 1H), 8.10 (d, *J* = 8.6 Hz, 1H), 7.88 (dd, *J* = 13.9, 8.9 Hz, 2H), 7.75 (d, *J* = 8.4 Hz, 1H), 7.40 (s, 1H), 7.27 (d, *J* = 7.7 Hz, 1H), 7.20 (d, *J* = 2.3 Hz, 1H), 7.09 (d, *J* = 6.9 Hz, 1H), 7.00 (dd, *J* = 8.8, 2.3 Hz, 1H), 5.09 (dd, *J* = 12.9, 5.3 Hz, 1H), 4.77 (s, 2H), 4.41 - 4.30 (m, 3H), 4.05 (d, *J* = 9.2 Hz, 1H), 3.43 (d, *J* = 10.6 Hz, 2H), 3.06 (d, *J* = 10.1 Hz, 4H), 2.93 - 2.82 (m, 3H), 2.66 - 2.53 (m, 2H), 2.43 (d, *J* = 7.6 Hz, 2H), 2.26 - 2.16 (m, 1H), 2.12 - 1.98 (m, 3H), 1.87 (d, *J* = 11.6 Hz, 2H), 1.22 (s, 8H), 1.11 (s, 6H). LCMS (ESI) calcd for $C_{46}H_{52}ClN_8O_6^+$ [M+H]$^+$: 847.36, found, 847.30.

**Example 26: preparation of N-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)nicotinamide (GT-03426)**

Referring to the method of Scheme 4, the target compound (GT-03426) was prepared, which is a yellow solid (30 mg, yield 35.05%). $^1$H NMR (400 MHz, DMSO) δ 11.09 (s, 1H), 10.65 (s, 1H), 8.62 (d, *J* = 1.7 Hz, 1H), 8.05 (d, *J* =

8.5 Hz, 1H), 7.91 (d, *J* = 8.7 Hz, 1H), 7.75 (t, *J* = 7.6 Hz, 2H), 7.38 (d, *J* = 17.2 Hz, 1H), 7.23 (dd, *J* = 18.2, 4.7 Hz, 2H), 7.10-6.96 (m, 2H), 5.09 (dd, *J* = 12.9, 5.3 Hz, 1H), 4.47 (d, *J* = 13.3 Hz, 2H), 4.33 (s, 1H), 4.20 (s, 2H), 4.06 (d, *J* = 9.2 Hz, 1H), 4.00 (d, *J* = 12.5 Hz, 2H), 3.72 (d, *J* = 12.1 Hz, 2H), 3.09 - 2.92 (m, 4H), 2.87 (dd, *J* = 16.8, 5.3 Hz, 1H), 2.61 (ddd, *J* = 27.2, 8.2, 3.3 Hz, 2H), 2.34 - 2.19 (m, 3H), 2.06 - 1.91 (m, 5H), 1.31 - 1.21 (m, 8H), 1.12 (s, 6H). LCMS (ESI) calcd for $C_{46}H_{52}ClN_8O_6^+$ $[M+H]^+$: 847.36, found, 847.30.

**Example 27: preparation of N-((1r,3r)-3-(3-chloro-4-cyanophenoxy)-2,2,4,4-tetramethylcyclobutyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)nicotinamide (GT-03427)**

Referring to the method of Scheme 4, the target compound (GT-03427) was prepared, which is a yellow solid (46 mg, yield 54.64%). $^1H$ NMR (400 MHz, DMSO) δ 11.28 (s, 1H), 11.10 (s, 1H), 8.56 (d, *J* = 1.7 Hz, 1H), 8.13 (d, *J* = 8.7 Hz, 1H), 7.90 (d, *J* = 8.7 Hz, 2H), 7.77 (d, *J* = 8.2 Hz, 1H), 7.22 - 7.11 (m, 3H), 7.02 - 6.93 (m, 2H), 5.10 (dd, *J* = 12.8, 5.3 Hz, 1H), 4.65 (d, *J* = 2.6 Hz, 2H), 4.41 - 4.31 (m, 3H), 4.06 (d, *J* = 9.1 Hz, 1H), 3.77 (d, *J* = 8.7 Hz, 2H), 3.38 (dd, *J* = 11.9, 5.9 Hz, 2H), 3.00 (t, *J* = 12.0 Hz, 2H), 2.92 - 2.73 (m, 5H), 2.56 (dd, *J* = 22.1, 11.2 Hz, 2H), 2.13 (dd, *J* = 16.1, 9.7 Hz, 1H), 2.04 - 1.98 (m, 1H), 1.91 (d, *J* = 11.6 Hz, 2H), 1.22 (s, 8H), 1.12 (s, 6H). LCMS (ESI) calcd for $C_{45}H_{50}ClN_8O_6^+$ $[M+H]^+$: 833.35, found, 833.40.

**Example 28: preparation of *N*-((1r,4r)-4-(3-chloro-4-cyanophenoxy)cyclohexyl)-6-(3-((1-(2-(2,6-dioxopiperidin-3-yl)-6-fluoro-1,3-dioxoisoindolin-5-yl)piperidin-4-yl)methyl)-3,8-diazabicyclo[3.2.1]octan-8-yl)pyridazine-3-carboxamide (GT-03449)**

Referring to the method of Scheme 4, the target compound (GT-03449) was prepared, which is a yellow solid (37 mg, yield 34.19%). $^1H$ NMR (400 MHz, DMSO) δ 11.10 (s, 1H), 10.42 (s, 1H), 8.70 (d, *J* = 8.2 Hz, 1H), 7.96 (d, *J* = 9.3 Hz, 1H), 7.85 (d, *J* = 8.8 Hz, 1H), 7.70 (d, *J* = 11.4 Hz, 1H), 7.40 (dd, *J* = 18.6, 5.2 Hz, 3H), 7.13 (dd, *J* = 8.8, 2.3 Hz, 1H), 5.10 (dd, *J* = 12.7, 5.3 Hz, 1H), 4.90 (s, 2H), 4.53 (ddd, *J* = 14.0, 10.0, 4.0 Hz, 1H), 3.96 - 3.81 (m, 1H), 3.54 (dd, *J* = 25.2, 11.6 Hz, 4H), 3.14 (t, *J* = 10.2 Hz, 2H), 2.90 (dd, *J* = 25.8, 13.0 Hz, 4H), 2.58 (dd, *J* = 24.1, 7.2 Hz, 2H), 2.47 - 2.36 (m, 2H), 2.13 - 1.99 (m, 6H), 1.88 (s, 4H), 1.65 (d, *J* = 12.6 Hz, 2H), 1.52 (dd, *J* = 22.5, 10.2 Hz, 2H), 1.38 (dd, *J* = 22.4, 11.6 Hz, 2H). LCMS (ESI) calcd for $C_{43}H_{46}ClFN_9O_6^+$ $[M+H]^+$: 838.32, found, 838.30.

**Example 29: preparation of *N*-((1r,4r)-4-(3-chloro-4-cyanophenoxy)cyclohexyl)-6-(8-((1-(2-(2,6-dioxopiperidin-3-yl)-6-fluoro-1,3-dioxoisoindolin-5-yl)piperidin-4-yl)methyl)-3,8-diazabicyclo[3.2.1]octan-3-yl)pyridazine-3-carboxamide (GT-03450)**

Referring to the method of Scheme 4, the target compound (GT-03450) was prepared, which is a yellow solid (35 mg, yield 32.36%). $^1H$ NMR (400 MHz, DMSO) δ 11.11 (s, 2H), 8.67 (d, *J* = 8.1 Hz, 1H), 7.99 - 7.89 (m, 1H), 7.85 (d, *J* = 8.8 Hz, 1H), 7.73 (d, *J* = 11.4 Hz, 1H), 7.48 (d, *J* = 7.4 Hz, 1H), 7.44 - 7.34 (m, 2H), 7.13 (dd, *J* = 8.8, 2.2 Hz, 1H), 5.11 (dd, *J* = 12.8, 5.3 Hz, 1H), 4.60 - 4.47 (m, 1H), 4.37 - 4.10 (m, 4H), 4.00 - 3.70 (m, 3H), 3.66 (d, *J* = 11.2 Hz, 2H), 3.08 - 2.84 (m, 5H), 2.66 - 2.52 (m, 2H), 2.37 - 2.17 (m, 3H), 2.14 - 2.01 (m, 5H), 1.90 (d, *J* = 8.4 Hz, 4H), 1.65 (dd, *J* = 24.3, 11.7 Hz, 2H), 1.56 - 1.39 (m, 4H). LCMS (ESI) calcd for $C_{43}H_{46}ClFN_9O_6^+$ $[M+H]^+$: 838.32, found, 838.30.

**Example 30: preparation of *N*-((1r,4r)-4-(3-chloro-4-cyanophenoxy)cyclohexyl)-6-(3-((1-(2-(2,6-dioxopiperidin-3-yl)-6-fluoro-1,3-dioxoisoindolin-5-yl)piperidin-4-yl)methyl)-3,6-diazabicyclo[3.1.1]heptan-6-yl)pyridazine-3-carboxamide (GT-03451)**

Referring to the method of Scheme 4, the target compound (GT-03451) was prepared, which is a yellow solid (46.00 mg, yield 43.26%). $^1H$ NMR (400 MHz, DMSO) δ 11.36 (s, 1H), 11.10 (s, 1H), 8.69 (d, *J* = 8.2 Hz, 1H), 8.00 (d, *J* = 9.2 Hz, 1H), 7.85 (d, *J* = 8.8 Hz, 1H), 7.69 (d, *J* = 11.4 Hz, 1H), 7.39 (dd, *J* = 14.0, 4.9 Hz, 2H), 7.22 (d, *J* = 9.2 Hz, 1H), 7.12 (dd, *J* = 8.8, 2.4 Hz, 1H), 5.09 (dd, *J* = 12.8, 5.4 Hz, 1H), 4.66 (d, *J* = 5.7 Hz, 2H), 4.52 (td, *J* = 10.2, 5.0 Hz, 1H), 3.90 - 3.78 (m, 3H), 3.54 (d, *J* = 12.4 Hz, 3H), 2.97 - 2.83 (m, 4H), 2.76 (t, *J* = 10.0 Hz, 3H), 2.57 (dd, *J* = 23.6, 7.5 Hz, 2H), 2.18 - 1.96 (m, 4H), 1.86 (d, *J* = 10.4 Hz, 5H), 1.63 (dd, *J* = 23.8, 10.9 Hz, 2H), 1.54 - 1.45 (m, 2H), 1.34 (dd, *J* = 22.1, 13.0 Hz, 2H). LCMS (ESI) calcd for $C_{42}H_{44}ClFN_9O_6^+$ $[M+H]^+$: 824.30, found, 824.30.

**Example 31: preparation of *N*-((1r,4r)-4-(3-chloro-4-cyanophenoxy)cyclohexyl)-6-(6-((1-(2-(2,6-dioxopiperidin-3-yl)-6-fluoro-1,3-dioxoisoindolin-5-yl)piperidin-4-yl)methyl)-3,6-diazabicyclo[3.1.1]heptan-3-yl)pyridazine-3-carboxamide (GT-03470)**

Referring to the method of Scheme 4, the target compound (GT-03470) was prepared, which is a yellow solid

(55.00 mg, yield 51.72%). $^1$H NMR (400 MHz, DMSO) δ 11.10 (d, *J* = 2.0 Hz, 1H), 8.70 - 8.64 (m, 1H), 7.98 (dd, *J* = 9.4, 3.3 Hz, 1H), 7.85 (dd, *J* = 8.8, 1.9 Hz, 1H), 7.72 (dd, *J* = 8.8, 5.8 Hz, 1H), 7.50 - 7.45 (m, 1H), 7.38 (d, *J* = 1.4 Hz, 1H), 7.24 (dd, *J* = 9.5, 6.9 Hz, 1H), 7.15 - 7.12 (m, 1H), 5.12 - 5.08 (m, 1H), 4.56 - 4.51 (m, 2H), 4.22 - 4.08 (m, 2H), 4.00 (d, *J* = 13.7 Hz, 1H), 3.90 - 3.84 (m, 1H), 3.64 (dd, *J* = 21.8, 9.6 Hz, 3H), 2.92 (dd, *J* = 17.1, 6.5 Hz, 4H), 2.59 (d, *J* = 17.9 Hz, 2H), 2.11 (d, *J* = 9.9 Hz, 3H), 2.05 - 1.98 (m, 3H), 1.89 (s, 4H), 1.66 (dd, *J* = 23.9, 12.1 Hz, 3H), 1.56 - 1.38 (m, 5H). LCMS (ESI) calcd for $C_{42}H_{44}ClFN_9O_6^+$ [M+H]$^+$: 824.30, found, 824.30.

**Example 32: preparation of N-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)nicotinamide (GT-03471)**

Referring to the method of Scheme 4, the target compound (GT-03471) was prepared, which is a yellow solid (33 mg, yield 39.20%). $^1$H NMR (400 MHz, DMSO) δ 11.09 (d, *J* = 5.3 Hz, 1H), 8.63 - 8.52 (m, 1H), 8.13 (t, *J* = 9.1 Hz, 1H), 7.90 (d, *J* = 8.7 Hz, 2H), 7.86 - 7.72 (m, 1H), 7.17 (dd, *J* = 21.7, 13.4 Hz, 3H), 7.00 (dd, *J* = 8.7, 1.9 Hz, 1H), 5.09 (dt, *J* = 11.2, 5.4 Hz, 1H), 4.65 (s, 1H), 4.47 (dd, *J* = 17.6, 8.6 Hz, 2H), 4.34 (d, *J* = 2.9 Hz, 1H), 4.15 - 4.04 (m, 2H), 4.00 (d, *J* = 13.2 Hz, 1H), 3.92 (d, *J* = 12.9 Hz, 1H), 3.76 (dd, *J* = 11.7, 3.6 Hz, 2H), 3.44 - 3.35 (m, 2H), 3.17 - 2.96 (m, 3H), 2.83 (dd, *J* = 31.1, 14.3 Hz, 3H), 2.59 (d, *J* = 18.7 Hz, 2H), 2.18 (t, *J* = 7.4 Hz, 1H), 2.10 - 1.78 (m, 4H), 1.32 - 1.22 (m, 8H), 1.12 (s, 6H). LCMS (ESI) calcd for $C_{45}H_{50}ClN_8O_6^+$ [M+H]$^+$: 833.35, found, 833.30.

**Example 33: preparation of N-((1r,3r)-3-(3-chloro-4-cyanophenoxy)-2,2,4,4-tetramethylcyclobutyl)-6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)nicotinamide (GT-03481)**

Referring to the method of Scheme 4, the target compound (GT-03481) was prepared, which is a yellow solid (39.00 mg, yield 45.56%). $^1$H NMR (400 MHz, DMSO) δ 11.08 (s, 1H), 10.78 (s, 1H), 8.59 (s, 1H), 8.11 (d, *J* = 8.3 Hz, 1H), 7.88 (dd, *J* = 20.8, 8.2 Hz, 2H), 7.72 (dd, *J* = 8.4, 5.2 Hz, 1H), 7.26 - 7.08 (m, 3H), 7.01 (dd, *J* = 8.7, 2.2 Hz, 2H), 5.07 (dd, *J* = 12.8, 5.5 Hz, 1H), 4.57 (s, 1H), 4.44 (d, *J* = 11.0 Hz, 2H), 4.33 (s, 1H), 4.06 (d, *J* = 9.2 Hz, 1H), 3.98 (d, *J* = 8.8 Hz, 1H), 3.86 (d, *J* = 9.8 Hz, 1H), 3.26 - 3.19 (m, 2H), 3.07 (t, *J* = 11.7 Hz, 2H), 2.93 - 2.84 (m, 1H), 2.63 - 2.55 (m, 2H), 2.22 (dd, *J* = 18.1, 9.9 Hz, 1H), 1.99 (dd, *J* = 21.5, 8.7 Hz, 4H), 1.84 (d, *J* = 3.9 Hz, 1H), 1.29 (d, *J* = 11.5 Hz, 2H), 1.22 (s, 6H), 1.12 (s, 6H). LCMS (ESI) calcd for $C_{46}H_{52}ClN_8O_6^+$ [M+H]$^+$: 847.36, found, 847.40.

**Example 34: preparation of N-((1r,3r)-3-(3-chloro-4-cyanophenoxy)-2,2,4,4-tetramethylcyclobutyl)-6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)nicotinamide (GT-03482)**

Referring to the method of Scheme 4, the target compound (GT-03482) was prepared, which is a yellow solid (35.00 mg, yield 39.24%). $^1$H NMR (400 MHz, DMSO) δ 11.12 (s, 1H), 10.92 (s, 1H), 8.60 (d, *J* = 2.0 Hz, 1H), 8.16 (d, *J* = 7.8 Hz, 1H), 7.91 (t, *J* = 7.0 Hz, 2H), 7.80 (d, *J* = 11.5 Hz, 1H), 7.48 (d, *J* = 7.3 Hz, 1H), 7.20 (t, *J* = 7.8 Hz, 2H), 7.01 (dd, *J* = 8.8, 2.4 Hz, 1H), 5.12 (dd, *J* = 12.8, 5.4 Hz, 1H), 4.48 (d, *J*= 13.0 Hz, 2H), 4.35 (s, 1H), 4.18 (s, 2H), 4.07 (d, *J*= 9.2 Hz, 1H), 3.95 (d, *J* = 12.5 Hz, 2H), 3.58 (d, *J* = 11.3 Hz, 2H), 3.12 (t, *J* = 11.9 Hz, 2H), 3.02 - 2.85 (m, 3H), 2.66-2.51 (m, 2H), 2.28 (ddd, *J* = 27.6, 7.6, 3.1 Hz, 3H), 2.09 - 1.98 (m, 5H), 1.38 - 1.28 (m, 2H), 1.23 (s, 6H), 1.13 (s, 6H). LCMS (ESI) calcd for $C_{46}H_{51}ClFN_8O_6^+$ [M+H]$^+$: 865.35, found, 865.40.

**Example 35: preparation of N-((1r,4r)-4-(3-chloro-4-cyanophenoxy)cyclohexyl)-6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridazine-3-carboxamide (GT-03486)**

Referring to the method of Scheme 4, the target compound (GT-03486) was prepared, which is a yellow solid (66 mg, yield 74.19%). $^1$H NMR (400 MHz, DMSO) δ 11.34 (s, 1H), 11.11 (s, 1H), 8.58 (d, *J* = 8.2 Hz, 1H), 7.86 - 7.82 (m, 2H), 7.76 (d, *J* = 10.9 Hz, 1H), 7.45 (d, *J* = 9.7 Hz, 1H), 7.37 (d, *J* = 2.3 Hz, 1H), 7.32 (d, *J* = 7.3 Hz, 1H), 7.12 (dd, *J* = 8.8, 2.4 Hz, 1H), 5.11 (dd, *J* = 12.7, 5.4 Hz, 1H), 4.65 (s, 2H), 4.53 (dd, *J* = 9.1, 5.1 Hz, 1H), 4.42 (d, *J* = 13.2 Hz, 2H), 3.37 (dd, *J* = 14.0, 10.5 Hz, 2H), 3.00 (t, *J* = 12.1 Hz, 2H), 2.94 - 2.80 (m, 5H), 2.62 - 2.52 (m, 2H), 2.26 - 2.16 (m, 1H), 2.10 (d, *J* = 10.0 Hz, 2H), 2.01 (dd, *J* = 11.3, 6.1 Hz, 1H), 1.91 (t, *J* = 13.5 Hz, 4H), 1.63 (d, *J* = 12.9 Hz, 2H), 1.54 - 1.46 (m, 2H), 1.22 (dd,J= 20.0, 10.3 Hz, 2H). LCMS (ESI) calcd for $C_{42}H_{44}ClFN_9O_6^+$ [M+H]$^+$: 824.30, found, 824.30.

**Example 36: preparation of N-((1r,4r)-4-(3-chloro-4-cyanophenoxy)cyclohexyl)-6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridazine-3-carboxamide (GT-03487)**

Referring to the method of Scheme 4, the target compound (GT-03487) was prepared, which is a yellow solid (39 mg, yield 44.22%). $^1$H NMR (400 MHz, DMSO) δ 11.39 (s, 1H), 11.11 (s, 1H), 8.58 (d, *J* = 8.1 Hz, 1H), 7.83 (t, *J* = 4.8 Hz, 2H), 7.76 (d, *J* = 10.9 Hz, 1H), 7.43 (d, *J* = 9.8 Hz, 1H), 7.37 (s, 1H), 7.32 (d, *J* = 7.3 Hz, 1H), 7.13 (d, *J* = 2.2 Hz, 1H), 5.11 (dd, *J* = 12.8, 5.4 Hz, 1H), 4.64 (s, 1H), 4.54 - 4.50 (m, 2H), 4.41 (s, 1H), 3.38 (d, *J* = 0.8 Hz, 1H), 3.01 (dd, *J* = 21.9, 9.6 Hz, 3H), 2.89 (d, *J* = 2.2 Hz, 2H), 2.59 (d, *J* = 18.6 Hz, 2H), 2.25 - 2.19 (m, 1H), 2.10 (d, *J* = 11.1 Hz, 3H), 2.02 (dd, *J* = 9.0, 3.8 Hz, 2H), 1.89 (d, *J* = 12.4 Hz, 4H), 1.67 - 1.46 (m, 8H), 1.30 - 1.20 (m, 3H). LCMS (ESI) calcd for $C_{42}H_{44}ClFN_9O_6^+$ [M+H]$^+$: 824.30, found, 824.30.

**Example 37: preparation of N-((1r,4r)-4-(3-chloro-4-cyanophenoxy)cyclohexyl)-6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridazine-3-carboxamide (GT-03488)**

Referring to the method of Scheme 4, the target compound (GT-03488) was prepared, which is a yellow solid (71 mg, yield 80.88%). $^1$H NMR (400 MHz, DMSO) δ 11.09 (s, 1H), 10.34 (s, 1H), 8.58 (d, *J* = 8.2 Hz, 1H), 7.88 - 7.80 (m, 2H), 7.75 (d, *J* = 8.4 Hz, 1H), 7.39 (dd, *J* = 9.6, 4.1 Hz, 3H), 7.26 (dd, *J* = 9.6, 7.9 Hz, 1H), 7.13 (dd, *J* = 8.8, 2.4 Hz, 1H), 5.09 (dd, *J* = 12.9, 5.4 Hz, 1H), 4.77 (s, 2H), 4.59 - 4.49 (m, 1H), 4.41 (d, *J* = 13.2 Hz, 2H), 3.84 (dt, *J* = 9.6, 6.5 Hz, 1H), 3.43 (d, *J* = 10.4 Hz, 2H), 3.04 (dd, *J* = 24.0, 12.3 Hz, 4H), 2.97 - 2.83 (m, 3H), 2.68 - 2.53 (m, 2H), 2.40 (d, *J* = 7.7 Hz, 2H), 2.21 (s, 1H), 2.14 - 1.99 (m, 5H), 1.88 (t, *J* = 10.9 Hz, 4H), 1.67 - 1.48 (m, 4H), 1.22 (dd, *J* = 22.2, 11.4 Hz, 2H). LCMS (ESI) calcd for $C_{43}H_{47}ClN_9O_6^+$ [M+H]$^+$: 820.33, found, 820.30.

**Example 38: preparation of N-((1r,4r)-4-(3-chloro-4-cyanophenoxy)cyclohexyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazine-3-carboxamide (GT-03489)**

Referring to the method of Scheme 4, the target compound (GT-03489) was prepared, which is a yellow solid (72 mg, yield 82.02%). $^1$H NMR (400 MHz, DMSO) δ 11.39 (s, 1H), 11.09 (s, 1H), 8.63 (d, *J* = 8.1 Hz, 1H), 7.95 (d, *J* = 9.6 Hz, 1H), 7.84 (d, *J* = 8.8 Hz, 1H), 7.77 - 7.63 (m, 2H), 7.35 (t, *J* = 5.9 Hz, 2H), 7.24 (d, *J* = 8.6 Hz, 1H), 7.12 (dd, *J* = 8.8, 2.1 Hz, 1H), 5.09 (dd, *J* = 12.8, 5.3 Hz, 1H), 4.51 (d, *J* = 10.6 Hz, 3H), 3.97 (d, *J* = 12.1 Hz, 2H), 3.92 - 3.78 (m, 3H), 3.17 (t, *J* = 11.6 Hz, 2H), 3.02 - 2.89 (m, 2H), 2.57 (dd, *J* = 19.0, 10.4 Hz, 2H), 2.37 (d, *J* = 9.9 Hz, 1H), 2.21 (d, *J* = 2.4 Hz, 2H), 2.15 - 2.00 (m, 5H), 1.92 (dd, *J* = 21.8, 8.9 Hz, 4H), 1.64 (dd, *J* = 23.4, 11.7 Hz, 2H), 1.57 - 1.46 (m, 2H), 1.34 (dt, *J* = 15.0, 9.1 Hz, 2H). LCMS (ESI) calcd for $C_{43}H_{47}ClN_9O_6^+$ [M+H]$^+$: 820.33, found, 820.30.

**Example 39: preparation of N-((1r,4r)-4-(3-chloro-4-cyanophenoxy)cyclohexyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)pyridazine-3-carboxamide (GT-03490)**

Referring to the method of Scheme 4, the target compound (GT-03490) was prepared, which is a yellow solid (49 mg, yield 56.79%). $^1$H NMR (400 MHz, DMSO) δ 11.09 (d, *J* = 5.5 Hz, 1H), 8.59 (t, *J* = 8.3 Hz, 1H), 7.84 (d, *J* = 8.6 Hz, 2H), 7.77 (dd, *J* = 8.3, 4.9 Hz, 1H), 7.38 (t, *J* = 7.3 Hz, 2H), 7.20 - 7.07 (m, 3H), 5.12 - 5.05 (m, 1H), 4.64 (s, 1H), 4.51 (d, *J* = 7.2 Hz, 3H), 3.98 (dd, *J* = 27.3, 14.1 Hz, 2H), 3.85 (dd, *J* = 7.1, 3.4 Hz, 1H), 3.48 - 3.31 (m, 2H), 3.16 (dd, *J* = 10.1, 6.4 Hz, 1H), 3.02 (dd, *J* = 28.0, 13.7 Hz, 2H), 2.82 (d, *J* = 17.7 Hz, 2H), 2.59 (d, *J* = 19.0 Hz, 2H), 2.08 (t, *J* = 10.4 Hz, 3H), 2.03 - 1.98 (m, 1H), 1.88 (d, *J* = 3.9 Hz, 4H), 1.75 - 1.42 (m, 6H), 1.27 (ddd, *J* = 32.5, 18.8, 10.9 Hz, 3H). LCMS (ESI) calcd for $C_{42}H_{45}ClN_9O_6^+$ [M+H]$^+$: 806.31, found, 806.40.

**Example 40: preparation of N-((1r,3r)-3-(3-chloro-4-cyanophenoxy)-2,2,4,4-tetramethylcyclobutyl)-6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)nicotinamide (GT-03494)**

Referring to the method of Scheme 4, the target compound (GT-03494) was prepared, which is a yellow solid (40 mg, yield 45.84%). $^1$H NMR (400 MHz, DMSO) δ 11.11 (s, 1H), 8.58 (s, 1H), 8.16 (d, *J* = 8.4 Hz, 1H), 7.90 (d, *J* = 8.7 Hz, 2H), 7.72 (dd, *J* = 12.4, 1.8 Hz, 1H), 7.35 (dd, *J* = 7.5, 3.4 Hz, 1H), 7.21 (d, *J* = 2.4 Hz, 2H), 7.01 (dd, *J* = 8.8, 2.4 Hz, 1H), 5.10 (dd, *J* = 12.9, 5.2 Hz, 1H), 4.45 (d, *J* = 13.7 Hz, 2H), 4.33 (d, *J* = 11.9 Hz, 2H), 4.07 (d, *J* = 9.2 Hz, 1H), 3.90 (s, 1H), 3.74 (d, *J* = 13.0 Hz, 2H), 3.60 (d, *J* = 13.7 Hz, 2H), 3.24 - 3.18 (m, 2H), 3.10 (t, *J* = 13.2 Hz, 2H), 2.88 (td, *J* = 14.3, 7.4 Hz, 1H), 2.64 - 2.52 (m, 2H), 2.32 - 2.14 (m, 2H), 2.15 - 1.95 (m, 5H), 1.92 - 1.84 (m, 1H), 1.37 - 1.25

(m, 2H), 1.23 (s, 6H), 1.12 (s, 6H). LCMS (ESI) calcd for $C_{46}H_{53}ClFN_8O_6^+$ $[M+H]^+$: 867.37, found, 867.40.

**Example 41: preparation of *N*-((1r,4r)-4-(3-chloro-4-cyanophenoxy)cyclohexyl)-6-(4-((4-(2-(2,6-dioxopiperidin-3-yl)-1-oxoisoindolin-5-yl)piperidin-1-yl)methyl)piperidin-1-yl)pyridazine-3-carboxamide (GT-03518)**

Referring to the method of Scheme 4, the target compound (GT-03518) was prepared, which is a yellow solid (49.00 mg, yield 58.84%). $^1$H NMR (400 MHz, DMSO) δ 10.99 (s, 1H), 10.64 (s, 1H), 8.61 (d, *J* = 8.2 Hz, 1H), 7.86 (dd, *J* = 9.2, 7.2 Hz, 2H), 7.71 (d, *J* = 7.9 Hz, 1H), 7.50 (d, *J* = 6.3 Hz, 2H), 7.40 (dd, *J* = 18.0, 5.2 Hz, 2H), 7.13 (dd, *J* = 8.8, 2.4 Hz, 1H), 5.11 (dd, *J* = 13.2, 5.1 Hz, 1H), 4.58 - 4.41 (m, 4H), 4.32 (d, *J* = 17.5 Hz, 1H), 3.62 (d, *J* = 11.3 Hz, 2H), 3.20 - 2.85 (m, 8H), 2.60 (d, *J* = 16.7 Hz, 1H), 2.44 - 2.28 (m, 3H), 2.13 - 1.86 (m, 9H), 1.67 - 1.48 (m, 4H), 1.28 (dd, *J* = 19.6, 9.5 Hz, 2H). LCMS (ESI) calcd for $C_{42}H_{48}ClN_8O_5^+$ $[M+H]^+$: 779.34, found, 779.30.

**Example 42: preparation of *N*-((1r,4r)-4-(3-chloro-4-cyanophenoxy)cyclohexyl)-6-(4-(((3S,4S)-4-(2-(2,6-dioxopiperidin-3-yl)-1-oxoisoindolin-5-yl)-3,4-dihydroxypiperidin-1-yl)methyl)piperidin-1-yl)pyridazine-3-carboxamide (GT-03519)**

Referring to the method of Scheme 4, the target compound (GT-03519) was prepared, which is a yellow solid (39.00 mg, yield 44.99%). $^1$H NMR (400 MHz, DMSO) δ 10.99 (s, 1H), 10.88 (s, 1H), 8.61 (d, *J* = 8.2 Hz, 1H), 7.90 - 7.83 (m, 2H), 7.74 (d, *J* = 8.0 Hz, 1H), 7.65 (dd, *J* = 13.6, 5.4 Hz, 1H), 7.47 (d, *J* = 9.7 Hz, 1H), 7.39 (d, *J* = 2.4 Hz, 1H), 7.13 (dd, *J* = 8.8, 2.4 Hz, 1H), 5.12 (dd, *J* = 13.2, 4.9 Hz, 1H), 4.63 - 4.44 (m, 5H), 4.37 (d, *J* = 11.5 Hz, 1H), 3.38 (dd, *J* = 36.3, 11.4 Hz, 2H), 3.19 - 3.03 (m, 6H), 2.94 (ddd, *J* = 17.8, 16.6, 9.6 Hz, 1H), 2.67 - 2.58 (m, 1H), 2.41 - 2.22 (m, 2H), 2.16 - 1.97 (m, 5H), 1.90 (d, *J* = 10.8 Hz, 2H), 1.79 (d, *J* = 13.2 Hz, 1H), 1.72 - 1.39 (m, 5H), 1.29 (dt, *J* = 20.3, 9.0 Hz, 3H). LCMS (ESI) calcd for $C_{42}H_{48}ClN_8O_7^+$ $[M+H]^+$: 811.33, found, 811.30.

**Example 43: preparation of *N*-((1r,4r)-4-(3-chloro-4-cyanophenoxy)cyclohexyl)-6-(4-((4-(2-(2,6-dioxopiperidin-3-yl)-1-oxoisoindolin-5-yl)-3,6-dihydropyridin-1(2H)-yl)methyl)piperidin-1-yl)pyridazine-3-carboxamide (GT-03520)**

Referring to the method of Scheme 4, the target compound (GT-03520) was prepared, which is a yellow solid (46.00 mg, yield 55.38%). $^1$H NMR (400 MHz, DMSO) δ 11.01 (s, 1H), 10.78 (s, 1H), 8.62 (d, *J* = 8.2 Hz, 1H), 7.86 (dd, *J* = 9.2, 5.9 Hz, 2H), 7.74 (d, *J* = 8.3 Hz, 2H), 7.65 (d, *J* = 8.0 Hz, 1H), 7.49 (d, *J* = 9.7 Hz, 1H), 7.38 (d, *J* = 2.4 Hz, 1H), 7.13 (dd, *J* = 8.8, 2.4 Hz, 1H), 6.33 (s, 1H), 5.13 (dd, *J* = 13.2, 5.1 Hz, 1H), 4.60 - 4.43 (m, 4H), 4.35 (d, *J* = 17.6 Hz, 1H), 4.16 - 4.06 (m, 1H), 3.69 - 3.62 (m, 1H), 3.30 (dd, *J* = 16.3, 8.3 Hz, 1H), 3.20 - 3.02 (m, 5H), 2.97 - 2.87 (m, 1H), 2.77 (dd, *J* = 17.9, 10.2 Hz, 1H), 2.60 (d, *J* = 14.6 Hz, 1H), 2.46 - 2.26 (m, 2H), 2.13 - 1.85 (m, 7H), 1.67 - 1.48 (m, 4H), 1.31 (dd, *J* = 14.5, 7.8 Hz, 2H). LCMS (ESI) calcd for $C_{42}H_{46}ClN_8O_5^+$ $[M+H]^+$: 777.32, found, 777.30.

**Example 44: preparation of *N*-((1r,4r)-4-(3-chloro-4-cyanophenoxy)cyclohexyl)-6-(4-((4-(2-(2,6-dioxopiperidin-3-yl)-4-fluoro-1-oxoisoindolin-5-yl)piperidin-1-yl)methyl)piperidin-1-yl)pyridazine-3-carboxamide (GT-03525)**

Referring to the method of Scheme 4, the target compound (GT-03525) was prepared, which is a yellow solid (27 mg, yield 31.69%). $^1$H NMR (400 MHz, DMSO) δ 11.02 (s, 1H), 8.62 (d, *J* = 8.2 Hz, 1H), 7.87 (t, *J* = 9.2 Hz, 2H), 7.64 (d, *J* = 7.8 Hz, 1H), 7.51 (dd, *J* = 8.2, 4.7 Hz, 2H), 7.39 (d, *J* = 2.4 Hz, 1H), 7.14 (dd, *J* = 8.8, 2.4 Hz, 1H), 5.13 (dd, *J* = 13.2, 5.0 Hz, 1H), 4.61 - 4.48 (m, 4H), 4.40 (d, *J* = 17.3 Hz, 1H), 3.87 (dd, *J* = 7.7, 3.5 Hz, 1H), 3.64 (d, *J* = 11.1 Hz, 2H), 3.36 - 3.27 (m, 1H), 3.13 (dd, *J* = 24.8, 11.8 Hz, 4H), 3.04 - 2.88 (m, 3H), 2.61 (d, *J* = 16.6 Hz, 1H), 2.47 - 2.40 (m, 2H), 2.35 - 2.24 (m, 1H), 2.15 - 2.00 (m, 5H), 1.91 (d, *J* = 10.0 Hz, 4H), 1.59 (dq, *J* = 23.0, 10.3 Hz, 5H), 1.32 (dd, *J* = 14.4, 7.9 Hz, 2H). LCMS (ESI) calcd for $C_{42}H_{47}ClFN_80_5^+$ $[M+H]^+$: 797.33, found, 797.30.

**Example 45: preparation of *N*-((1r,4r)-4-(3-chloro-4-cyanophenoxy)cyclohexyl)-6-(4-((4-(2-(2,6-dioxopiperidin-3-yl)-4-fluoro-1-oxoisoindolin-5-yl)-3,6-dihydropyridin-1(2H)-yl)methyl)piperidin-1-yl)pyridazine-3-carboxamide (GT-03540)**

Referring to the method of Scheme 4, the target compound (GT-03540) was prepared, which is a yellow solid (53 mg, yield 62.37%). $^1$H NMR (400 MHz, DMSO) δ 11.02 (s, 1H), 8.61 (d, *J* = 8.2 Hz, 1H), 7.86 (t, *J* = 8.8 Hz, 2H), 7.60 (dd, *J* = 17.2, 7.2 Hz, 2H), 7.50 (d, *J* = 9.7 Hz, 1H), 7.38 (d, *J* = 2.3 Hz, 1H), 7.13 (dd, *J* = 8.8, 2.3 Hz, 1H), 6.13 (s, 1H), 5.13 (dd, *J* = 13.2, 5.0 Hz, 1H), 4.62 - 4.48 (m, 4H), 4.41 (d, *J* = 17.5 Hz, 1H), 3.97 - 3.75 (m, 3H), 3.64 (d, *J* = 11.5 Hz, 1H), 3.34 - 3.23 (m, 1H), 3.11 (t, *J* = 11.5 Hz, 5H), 2.97 - 2.87 (m, 1H), 2.64 (dd, *J* = 26.9, 17.4 Hz, 2H), 2.48 - 2.37 (m, 1H), 2.29 (dt, *J* = 14.9, 7.7 Hz, 1H), 2.18 - 1.96 (m, 5H), 1.90 (d, *J* = 10.1 Hz, 2H), 1.64 (dd, *J* = 23.6, 10.9 Hz, 2H), 1.52 (t, *J* = 11.3 Hz, 2H), 1.28 (dt, *J* = 24.3, 11.7 Hz, 2H). LCMS (ESI) calcd for $C_{42}H_{45}ClFN_8O_5^+$ $[M+H]^+$: 795.31, found,

795.30.

**Example 46: preparation of *N*-((1r,4r)-4-(3-chloro-4-cyanophenoxy)cyclohexyl)-6-(4-((4-(2-(2,6-dioxopiperidin-3-yl)-7-fluoro-1-oxoisoindolin-5-yl)piperidin-1-yl)methyl)piperidin-1-yl)pyridazine-3-carboxamide (GT-03580)**

Referring to the method of Scheme 4, the target compound (GT-03580) was prepared, which is a yellow solid (50 mg, yield 58.61%). $^1$H NMR (400 MHz, DMSO) δ 11.00 (s, 1H), 8.61 (d, *J* = 8.1 Hz, 1H), 7.85 (d, *J* = 8.6 Hz, 2H), 7.47 (d, *J* = 9.4 Hz, 1H), 7.40 - 7.30 (m, 2H), 7.24 - 7.06 (m, 2H), 5.08 (dd, *J* = 13.3, 5.0 Hz, 1H), 4.56 - 4.45 (m, 4H), 4.34 (d, *J* = 17.9 Hz, 1H), 3.62 (d, *J* = 10.9 Hz, 2H), 3.18 - 2.97 (m, 7H), 2.94 - 2.84 (m, 1H), 2.60 (d, *J* = 16.0 Hz, 1H), 2.47 - 2.20 (m, 4H), 2.10 (d, *J* = 9.5 Hz, 2H), 2.06 - 1.94 (m, 5H), 1.90 (d, *J* = 10.2 Hz, 2H), 1.64 (dd, *J* = 23.9, 11.3 Hz, 2H), 1.55 - 1.43 (m, 2H), 1.40 - 1.10 (m, 3H). LCMS (ESI) calcd for $C_{42}H_{47}ClFN_8O_5^+$ [M+H]$^+$: 797.33, found, 797.30.

**Example 47: preparation of *N*-((1r,3r)-3-(3-chloro-4-cyanophenoxy)-2,2,4,4-tetramethylcyclobutyl)-6-(4-((4-(2-(2,6-dioxopiperidin-3-yl)-1-oxoisoindolin-5-yl)piperidin-1-yl)methyl)piperidin-1-yl)nicotinamide (GT-03589)**

Referring to the method of Scheme 4, the target compound (GT-03589) was prepared, which is a yellow solid (53 mg, yield 65.07%). $^1$H NMR (400 MHz, DMSO) δ 10.99 (s, 1H), 8.59 (d, *J* = 1.6 Hz, 1H), 8.16 (d, *J* = 8.4 Hz, 1H), 7.91 (d, *J* = 8.7 Hz, 2H), 7.72 (d, *J* = 7.9 Hz, 1H), 7.49 (s, 1H), 7.43 (d, *J* = 8.0 Hz, 1H), 7.21 (d, *J* = 2.3 Hz, 2H), 7.01 (dd, *J* = 8.8, 2.3 Hz, 1H), 5.11 (dd, *J* = 13.2, 5.0 Hz, 1H), 4.46 (d, *J* = 17.0 Hz, 3H), 4.32 (d, *J* = 17.2 Hz, 2H), 4.07 (d, *J* = 9.1 Hz, 1H), 3.63 (d, *J* = 11.6 Hz, 2H), 3.00 (ddt, *J* = 22.7, 18.1, 8.5 Hz, 8H), 2.60 (d, *J* = 16.8 Hz, 1H), 2.37 (ddd, *J* = 37.5, 19.9, 11.1 Hz, 4H), 2.00 (t, *J* = 14.4 Hz, 5H), 1.32 - 1.22 (m, 8H), 1.13 (s, 6H). LCMS (ESI) calcd for $C_{45}H_{53}ClN_7O_5^+$ [M+H]$^+$: 806.37, found, 806.40.

**Example 48: preparation of *N*-((1r,3r)-3-(3-chloro-4-cyanophenoxy)-2,2,4,4-tetramethylcyclobutyl)-6-(4-((4-(2-(2,6-dioxopiperidin-3-yl)-1-oxoisoindolin-5-yl)-3,6-dihydropyridin-1(2H)-yl)methyl)piperidin-1-yl)nicotinamide (GT-03590)**

Referring to the method of Scheme 4, the target compound (GT-03590) was prepared, which is a yellow solid (45 mg, yield 55.38%). $^1$H NMR (400 MHz, DMSO) δ 11.00 (s, 1H), 10.67 (s, 1H), 8.59 (d, *J* = 2.1 Hz, 1H), 8.19 (d, *J* = 11.0 Hz, 1H), 7.91 (d, *J* = 8.7 Hz, 2H), 7.78 -7.69 (m, 2H), 7.66 (d, *J* = 8.0 Hz, 1H), 7.21 (d, *J* = 2.4 Hz, 2H), 7.02 - 6.99 (m, 1H), 6.34 (s, 1H), 5.13 (dd, *J* = 13.2, 5.1 Hz, 1H), 4.65 - 4.54 (m, 1H), 4.50 - 4.43 (m, 3H), 4.38 - 4.33 (m, 2H), 4.07 (d, *J* = 9.2 Hz, 2H), 3.82 (dd, *J* = 15.9, 5.1 Hz, 2H), 3.71 - 3.63 (m, 2H), 3.34 - 3.27 (m, 1H), 3.14 (s, 2H), 3.02 (d, *J* = 8.6 Hz, 1H), 2.91 (dd, *J* = 10.6, 6.8 Hz, 1H), 2.78 (dd, *J* = 19.9, 11.1 Hz, 1H), 2.64 - 2.58 (m, 1H), 2.47 - 2.32 (m, 2H), 2.05 - 1.98 (m, 2H), 1.26 (d, *J* = 22.0 Hz, 8H), 1.13 (s, 6H). LCMS (ESI) calcd for $C_{45}H_{51}ClN_7O_5^+$ [M+H]$^+$: 804.36, found, 804.40.

**Example 49: preparation of N-((1r,4r)-4-(3-chloro-4-cyanophenoxy)cyclohexyl)-6-(4-((8-(2-(2,6-dioxopiperidin-3-yl)-1-oxoisoindolin-5-yl)-3,8-diazabicyclo[3.2.1]octan-3-yl)methyl)piperidin-1-yl)pyridazine-3-carboxamide (GT-03772)**

Referring to the method of Scheme 4, the target compound (GT-03772) was prepared, which is a yellow solid (33 mg, yield 38.30%). $^1$H NMR (400 MHz, DMSO) δ 10.94 (s, 1H), 10.48 (s, 1H), 8.57 (d, *J* = 8.2 Hz, 1H), 7.84 (dd, *J* = 9.2, 3.4 Hz, 2H), 7.58 (d, *J* = 8.4 Hz, 1H), 7.43 (d, *J* = 9.7 Hz, 1H), 7.38 (d, *J* = 2.4 Hz, 1H), 7.14 - 7.01 (m, 3H), 5.06 (dd, *J* = 13.2, 5.1 Hz, 1H), 4.64 - 4.50 (m, 3H), 4.41 (d, *J* = 12.9 Hz, 2H), 4.34 (d, *J* = 17.0 Hz, 1H), 4.23 (d, *J* = 17.0 Hz, 1H), 3.41 (d, *J* = 10.6 Hz, 2H), 3.03 (t, *J* = 11.3 Hz, 4H), 2.95 - 2.83 (m, 3H), 2.59 (d, *J* = 17.3 Hz, 1H), 2.46 - 2.33 (m, 3H), 2.22 (s, 1H), 2.08 (t, *J* = 11.9 Hz, 4H), 1.88 (dd, *J* = 7.3, 4.3 Hz, 4H), 1.70 - 1.44 (m, 5H), 1.37 - 1.11 (m, 3H). LCMS (ESI) calcd for $C_{43}H_{49}ClN_9O_5^+$ [M+H]$^+$: 806.35, found, 806.40.

**Example 50: preparation of N-((1r,4r)-4-(3-chloro-4-cyanophenoxy)cyclohexyl)-6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)pyridazine-3-carboxamide (GT-03773)**

Referring to the method of Scheme 4, the target compound (GT-03773) was prepared, which is a yellow solid (49 mg, yield 54.71%). $^1$H NMR (400 MHz, DMSO) δ 11.09 (s, 1H), 8.59 (d, *J* = 8.2 Hz, 1H), 7.85 (d, *J* = 8.8 Hz, 2H), 7.71 (dd, *J* = 12.4, 2.1 Hz, 1H), 7.46 (d, *J* = 9.5 Hz, 1H), 7.40 - 7.31 (m, 2H), 7.13 (dd, *J* = 8.8, 2.4 Hz, 1H), 5.09 (dd, *J* = 12.9, 5.3 Hz, 1H), 4.56 - 4.44 (m, 3H), 4.31 (s, 1H), 3.95 - 3.79 (m, 4H), 3.67 (d, *J* = 42.8 Hz, 1H), 3.29 - 3.13 (m, 3H), 3.06 (t, *J* = 12.1 Hz, 2H), 2.95 - 2.82 (m, 1H), 2.62 - 2.54 (m, 2H), 2.28 - 2.21 (m, 1H), 2.04 (ddd, *J* = 24.0, 18.7, 11.4

Hz, 7H), 1.93 - 1.84 (m, 3H), 1.76 - 1.42 (m, 5H), 1.34 - 1.24 (m, 2H). LCMS (ESI) calcd for $C_{43}H_{46}ClFN_9O_6^+$ [M+H]$^+$: 838.32, found, 838.40.

**Example 51: preparation of N-((1r,4r)-4-(3-chloro-4-cyanophenoxy)cyclohexyl)-6-(4-((5-(2-(2,6-dioxopiperidin-3-yl)-1,3-dioxoisoindolin-5-yl)-2,5-diazabicyclo [2.2.2] octan-2-yl)methyl)piperidin-1-yl)pyridazine-3-carboxamide (GT-03885)**

Referring to the method of Scheme 4, the target compound (GT-03885) was prepared, which is a yellow solid (52.00 mg, yield 59.32%). LCMS (ESI) calcd for $C_{43}H_{47}ClN_9O_6^+$ [M+H]$^+$: 820.33, found, 820.30.

**Example 52: preparation of N-((1r,4r)-4-(3-chloro-4-cyanophenoxy)cyclohexyl)-6-(4-((3-(2-(2,6-dioxopiperidin-3-yl)-1-oxoisoindolin-5-yl)-3,8-diazabicyclo[3.2.1]octan-8-yl)methyl)piperidin-1-yl)pyridazine-3-carboxamide (GT-03886)**

Referring to the method of Scheme 4, the target compound (GT-03886) was prepared, which is a yellow solid (47.00 mg, yield 54.55%). LCMS (ESI) calcd for $C_{43}H_{49}ClN_9O_5^+$ [M+H]$^+$: 806.35, found, 806.30.

**Example 53: preparation of N-(2-chloro-6-methylphenyl)-2-((6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-2-methylpyrimidin-4-yl)amino)thiazole-5-carboxamide (GT-03627)**

Referring to the method of Scheme 4, the target compound (GT-03627) was prepared, which is a yellow solid (64 mg, yield 71.65%). $^1$H NMR (400 MHz, DMSO) δ 11.11 (s, 1H), 10.40 (s, 1H), 9.99 (s, 1H), 8.28 (s, 1H), 7.79 (d, *J* = 12.1 Hz, 1H), 7.57 (d, *J* = 7.5 Hz, 1H), 7.40 (d, *J* = 6.1 Hz, 1H), 7.28 (dd, *J*= 10.4, 6.9 Hz, 2H), 6.21 (s, 1H), 5.11 (dd, *J* = 12.8, 5.3 Hz, 1H), 4.68 (s, 2H), 3.48 (d, *J* = 10.2 Hz, 2H), 3.18 (dd, *J* = 16.5, 9.0 Hz, 2H), 3.09 - 2.77 (m, 6H), 2.57 (dd, *J* = 21.9, 11.3 Hz, 2H), 2.47 (s, 3H), 2.39 (d, *J* = 7.6 Hz, 2H), 2.24 (s, 3H), 2.20 (s, 1H), 2.10 - 1.99 (m, 3H), 1.88 (d, *J* = 11.3 Hz, 2H), 1.22 (q, *J* = 13.0 Hz, 3H). LCMS (ESI) calcd for $C_{41}H_{43}ClFN_{10}O_5S^+$ [M+H]$^+$: 842.36, found, 842.30.

**Example 54: preparation of N-(2-chloro-6-methylphenyl)-2-((6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-2-methylpyrimidin-4-yl)amino)thiazole-5-carboxamide (GT-03628)**

Referring to the method of Scheme 4, the target compound (GT-03628) was prepared, which is a yellow solid (52 mg, yield 59.21%). $^1$H NMR (400 MHz, DMSO) δ 11.28 (s, 1H), 11.11 (s, 1H), 10.04 (s, 1H), 8.31 (s, 1H), 7.76 (d, *J* = 10.9 Hz, 1H), 7.39 (dd, *J* = 7.3, 1.1 Hz, 1H), 7.34 - 7.23 (m, 3H), 6.26 (s, 1H), 5.11 (dd, *J* = 12.7, 5.4 Hz, 1H), 4.65 (s, 2H), 4.18 (s, 3H), 3.84 (d, *J*= 8.8 Hz, 3H), 3.42 (d, *J* = 30.1 Hz, 2H), 2.97 - 2.79 (m, 6H), 2.59 (d, *J* = 19.3 Hz, 1H), 2.49 (d, *J* = 1.7 Hz, 3H), 2.24 (s, 3H), 2.20 - 2.08 (m, 1H), 2.04 - 1.98 (m, 1H), 1.93 (d, *J* = 11.6 Hz, 2H), 1.19 (dd, *J* = 20.5, 9.8 Hz, 2H). LCMS (ESI) calcd for $C_{40}H_{41}ClFN_{10}O_5S^+$ [M+H]$^+$: 828.33, found, 828.30.

**Example 55: preparation of N-(2-chloro-6-methylphenyl)-2-((6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-2-methylpyrimidin-4-yl)amino)thiazole-5-carboxamide (GT-03629)**

Referring to the method of Scheme 4, the target compound (GT-03629) was prepared, which is a yellow solid (46 mg, yield 52.63%). $^1$H NMR (400 MHz, DMSO) δ 11.09 (s, 1H), 10.55 (s, 1H), 10.07 (s, 1H), 8.32 (s, 1H), 7.75 (d, *J* = 8.4 Hz, 1H), 7.39 (dd, *J* = 4.8, 1.9 Hz, 2H), 7.31 - 7.23 (m, 3H), 6.29 (s, 1H), 5.10 (dd, *J* = 12.9, 5.4 Hz, 1H), 4.77 (s, 2H), 4.19 (d, *J* = 9.2 Hz, 3H), 3.42 (d, *J* = 10.3 Hz, 2H), 3.10 - 2.97 (m, 4H), 2.88 (dd, *J* = 22.0, 8.6 Hz, 3H), 2.63 - 2.54 (m, 2H), 2.50 (s, 3H), 2.44 (d, *J* = 7.6 Hz, 2H), 2.24 (s, 3H), 2.12 - 1.98 (m, 3H), 1.88 (d, *J* = 11.4 Hz, 2H), 1.22 (dd, *J* = 21.7, 11.5 Hz, 2H). LCMS (ESI) calcd for $C_{41}H_{44}ClN_{10}O_5S^+$ [M+H]$^+$: 824.37, found, 824.30.

**Example 56: preparation of N-(2-chloro-6-methylphenyl)-2-((6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-2-methylpyrimidin-4-yl)amino)thiazole-5-carboxamide (GT-03630)**

Referring to the method of Scheme 4, the target compound (GT-03630) was prepared, which is a yellow solid (57 mg, yield 66.34%). $^1$H NMR (400 MHz, DMSO) δ 11.26 (s, 1H), 11.09 (s, 1H), 10.05 (s, 1H), 8.31 (s, 1H), 7.77 (d, *J* = 8.2 Hz, 1H), 7.42 - 7.37 (m, 1H), 7.30 - 7.23 (m, 2H), 7.16 (d, *J* = 1.1 Hz, 1H), 6.95 (dd, *J* = 8.3, 1.6 Hz, 1H), 6.26 (s, 1H), 5.10 (dd, *J* = 12.8, 5.3 Hz, 1H), 4.65 (d, *J* = 3.3 Hz, 2H), 4.21 - 4.15 (m, 2H), 3.76 (d, *J* = 7.8 Hz, 2H), 3.39 (dt,

*J* = 11.2, 5.4 Hz, 2H), 2.92 (dd, *J* = 22.4, 8.0 Hz, 3H), 2.86 - 2.71 (m, 4H), 2.67 - 2.53 (m, 2H), 2.48 (s, 3H), 2.23 (s, 3H), 2.11 (dd, *J* = 11.3, 4.3 Hz, 1H), 2.04 - 1.98 (m, 1H), 1.91 (d, *J* = 11.0 Hz, 2H), 1.18 (dd, *J* = 21.9, 11.1 Hz, 2H). LCMS (ESI) calcd for $C_{40}H_{42}ClN_{10}O_5S^+$ [M+H]$^+$: 810.34, found, 810.30.

**Example 57: preparation of N-(2-chloro-6-methylphenyl)-2-((6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-2-methylpyrimidin-4-yl)amino)thiazole-5-carboxamide (GT-03631)**

Referring to the method of Scheme 4, the target compound (GT-03631) was prepared, which is a yellow solid (55 mg, yield 64.01%). $^1$H NMR (400 MHz, DMSO) δ 11.52 (s, 1H), 11.09 (d, *J* = 6.1 Hz, 1H), 10.05 (s, 1H), 8.31 (s, 1H), 7.78 (dd, *J* = 8.3, 4.7 Hz, 1H), 7.39 (d, *J* = 7.3 Hz, 1H), 7.28 (dd, *J* = 9.6, 7.4 Hz, 2H), 7.18 (s, 1H), 7.10 (d, *J* = 8.5 Hz, 1H), 6.28 (d, *J* = 16.3 Hz, 1H), 5.13 - 5.06 (m, 1H), 4.64 (s, 1H), 4.49 (d, *J* = 6.1 Hz, 1H), 4.35 - 4.20 (m, 3H), 3.44 - 3.35 (m, 1H), 3.21 - 3.05 (m, 1H), 3.02 - 2.77 (m, 5H), 2.59 (d, *J* = 19.0 Hz, 2H), 2.48 (s, 3H), 2.24 (s, 3H), 2.21 - 2.10 (m, 1H), 2.09 - 1.79 (m, 5H), 1.52 - 0.88 (m, 3H). LCMS (ESI) calcd for $C_{40}H_{42}ClN_{10}O_5S^+$ [M+H]$^+$: 810.34, found, 810.30.

**Example 58: preparation of N-(2-chloro-6-methylphenyl)-2-((6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-2-methylpyrimidin-4-yl)amino)thiazole-5-carboxamide (GT-03632)**

Referring to the method of Scheme 4, the target compound (GT-03632) was prepared, which is a yellow solid (66 mg, yield 75.51%). $^1$H NMR (400 MHz, DMSO) δ 11.62 (s, 1H), 11.09 (s, 1H), 9.96 (s, 1H), 8.27 (s, 1H), 7.76 (d, *J* = 8.5 Hz, 1H), 7.38 (t, *J* = 10.2 Hz, 2H), 7.27 (ddd, *J* = 11.1, 7.2, 4.3 Hz, 3H), 6.20 (s, 1H), 5.09 (dd, *J* = 12.9, 5.4 Hz, 1H), 4.35 - 4.24 (m, 2H), 4.18 (d, *J* = 12.1 Hz, 3H), 3.86 (d, *J* = 14.7 Hz, 1H), 3.76 - 3.68 (m, 2H), 3.07 - 2.82 (m, 5H), 2.62 - 2.56 (m, 1H), 2.46 (s, 3H), 2.34 - 2.14 (m, 7H), 2.04 - 1.93 (m, 5H), 1.27 (dd, *J* = 21.7, 9.7 Hz, 2H). LCMS (ESI) calcd for $C_{41}H_{44}ClN_{10}O_5S^+$ [M+H]$^+$: 824.37, found, 824.30.

**Example 59: preparation of 7-cyclopentyl-2-((5-(4-((8-(2-(2,6-dioxopiperidin-3-yl)-1,3-dioxoisoindolin-5-yl)-3,8-diazabicyclo [3.2.1] octan-3-yl)methyl)piperidin-1-yl)pyridin-2-yl)amino)-N,N-dimethyl-7H-pyrrolo[2,3-d]pyrimidine-6-carboxamide (GT-03651)**

Referring to the method of Scheme 4, the target compound (GT-03651) was prepared, which is a yellow solid (73 mg, yield 81.14%). $^1$H NMR (400 MHz, DMSO) δ 11.06 (s, 1H), 9.21 (s, 1H), 8.75 (s, 1H), 8.12 (d, *J* = 9.1 Hz, 1H), 7.99 (d, *J* = 2.6 Hz, 1H), 7.95 (s, 2H), 7.66 (d, *J* = 8.5 Hz, 1H), 7.42 (dd, *J* = 9.1, 2.6 Hz, 1H), 7.21 (s, 1H), 7.11 (d, *J* = 8.2 Hz, 1H), 6.59 (s, 1H), 5.06 (dd, *J* = 12.8, 5.4 Hz, 1H), 4.81 - 4.64 (m, 1H), 3.63 (d, *J* = 10.7 Hz, 4H), 3.35 (s, 3H), 3.05 (s, 6H), 2.70 - 2.61 (m, 3H), 2.56 (dd, *J* = 8.2, 5.4 Hz, 2H), 2.41 (ddd, *J* = 9.6, 5.7, 2.4 Hz, 2H), 2.29 (d, *J* = 6.7 Hz, 2H), 1.97 (tt, *J* = 17.6, 10.9 Hz, 10H), 1.66 - 1.55 (m, 5H), 1.35 - 1.24 (m, 2H). LCMS (ESI) calcd for $C_{44}H_{52}N_{11}O_5^+$ [M+H]$^+$: 814.41, found, 814.40.

**Example 60: preparation of 7-cyclopentyl-2-((5-(4-((3-(2-(2,6-dioxopiperidin-3-yl)-1,3-dioxoisoindolin-5-yl)-3,8-diazabicyclo [3.2.1] octan-8-yl)methyl)piperidin-1-yl)pyridin-2-yl)amino)-N,N-dimethyl-7H-pyrrolo[2,3-d]pyrimidine-6-carboxamide (GT-03652)**

Referring to the method of Scheme 4, the target compound (GT-03652) was prepared, which is a yellow solid (78.00 mg, yield 86.70 %). $^1$H NMR (400 MHz, DMSO) δ 11.06 (s, 1H), 9.21 (s, 1H), 8.75 (s, 1H), 8.12 (d, *J* = 9.1 Hz, 1H), 7.99 (d, *J* = 2.6 Hz, 1H), 7.95 (s, 2H), 7.66 (d, *J* = 8.5 Hz, 1H), 7.42 (dd, *J* = 9.1, 2.6 Hz, 1H), 7.21 (s, 1H), 7.11 (d, *J* = 8.2 Hz, 1H), 6.59 (s, 1H), 5.06 (dd, *J* = 12.8, 5.4 Hz, 1H), 4.79 - 4.66 (m, 1H), 3.63 (d, *J* = 11.4 Hz, 4H), 3.35 (s, 2H), 3.05 (s, 8H), 2.69 - 2.54 (m, 5H), 2.42 (dd, *J* = 7.8, 4.8 Hz, 2H), 2.29 (d, *J* = 6.7 Hz, 2H), 2.01 - 1.86 (m, 9H), 1.61 (dt, *J* = 12.6, 6.4 Hz, 5H), 1.34 - 1.23 (m, 2H). LCMS (ESI) calcd for $C_{44}H_{52}N_{11}O_5^+$ [M+H]$^+$: 814.41, found, 814.40.

**Example 61: preparation of 7-cyclopentyl-2-((5-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridin-2-yl)amino)-N,N-dimethyl-7H-pyrrolo[2,3-d]pyrimidine-6-carboxamide (GT-03654)**

Referring to the method of Scheme 4, the target compound (GT-03654) was prepared, which is a yellow solid (75 mg, yield 82.96%). $^1$H NMR (400 MHz, DMSO) δ 11.48 (s, 2H), 11.11 (s, 1H), 8.98 (s, 1H), 8.11 (dd, *J* = 9.5, 2.3 Hz, 1H), 7.90 (s, 1H), 7.77 (d, *J* = 10.9 Hz, 1H), 7.61 (d, *J* = 9.4 Hz, 1H), 7.33 (d, *J* = 7.3 Hz, 1H), 6.81 (s, 1H), 5.11 (dd, *J* = 12.8, 5.4 Hz, 1H), 4.81 (dd, *J* = 17.3, 8.7 Hz, 1H), 4.65 (s, 2H), 3.84 (d, *J* = 9.5 Hz, 2H), 3.64 (d, *J* = 12.1 Hz, 3H), 3.05 (s, 6H), 2.85 (dd, *J* = 14.1, 8.8 Hz, 5H), 2.71 (t, *J* = 11.5 Hz, 2H), 2.64 - 2.51 (m, 2H), 2.30 (ddd, *J* = 8.6, 6.0, 3.3

Hz, 2H), 1.98 (ddd, *J* = 24.5, 16.8, 11.7 Hz, 9H), 1.68 - 1.61 (m, 2H), 1.39 - 1.25 (m, 2H). LCMS (ESI) calcd for $C_{43}H_{48}FN_{11}O_5^+$ [M+H]$^+$: 818.38, found, 818.40.

**Example 62: preparation of 7-cyclopentyl-2-((5-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3-d]pyrimidine-6-carboxamide (GT-03655)**

Referring to the method of Scheme 4, the target compound (GT-03655) was prepared, which is a yellow solid (75 mg, yield 81.56%). $^1$H NMR (400 MHz, DMSO) δ 11.50 (s, 1H), 11.11 (s, 1H), 8.99 (s, 1H), 8.13 (d, *J* = 9.7 Hz, 1H), 7.92 (s, 1H), 7.79 (d, *J* = 12.1 Hz, 1H), 7.58 (t, *J* = 9.7 Hz, 2H), 6.82 (s, 1H), 5.12 (dd, *J* = 12.9, 5.4 Hz, 1H), 4.82 (dd, *J* = 17.3, 8.6 Hz, 1H), 4.68 (s, 2H), 3.64 (d, *J* = 12.2 Hz, 3H), 3.48 (d, *J* = 10.5 Hz, 2H), 3.22 - 3.12 (m, 2H), 3.06 (s, 6H), 2.91 (ddd, *J* = 15.6, 13.7, 11.3 Hz, 3H), 2.76 (dd, *J* = 18.3, 7.4 Hz, 2H), 2.66 - 2.52 (m, 2H), 2.48 - 2.40 (m, 2H), 2.31 (ddd, *J* = 8.3, 4.2, 1.0 Hz, 2H), 2.11 - 1.94 (m, 9H), 1.65 (dd, *J* = 11.2, 5.3 Hz, 2H), 1.44 - 1.31 (m, 2H). LCMS (ESI) calcd for $C_{44}H_{51}FN_{11}O_5^+$ [M+H]$^+$: 832.40, found, 832.40.

**Example 63: preparation of 7-cyclopentyl-2-((5-(4-((4-(2-(2,6-dioxopiperidin-3-yl)-6-fluoro-1,3-dioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide (GT-03656)**

Referring to the method of Scheme 4, the target compound (GT-03656) was prepared, which is a yellow solid (81 mg, yield 92.78%). $^1$H NMR (400 MHz, DMSO) δ 11.48 (s, 1H), 11.17 (s, 1H), 11.12 (s, 1H), 9.00 (s, 1H), 8.15 (d, *J* = 7.5 Hz, 1H), 7.94 (s, 1H), 7.82 (d, *J* = 11.2 Hz, 1H), 7.61 (dd, *J* = 17.2, 8.4 Hz, 2H), 6.82 (s, 1H), 5.13 (dd, *J* = 12.8, 5.4 Hz, 1H), 4.80 (dd, *J* = 17.5, 8.8 Hz, 1H), 3.75 - 3.63 (m, 6H), 3.23 (dd, *J* = 18.8, 8.5 Hz, 3H), 3.12 (s, 2H), 3.06 (s, 6H), 2.95 - 2.79 (m, 3H), 2.65 - 2.52 (m, 2H), 2.35 - 2.27 (m, 2H), 2.14 - 1.92 (m, 9H), 1.70 - 1.62 (m, 2H), 1.41 (dd, *J* = 20.5, 10.1 Hz, 2H). LCMS (ESI) calcd for $C_{42}H_{49}FN_{11}O_5^+$ [M+H]$^+$: 806.38, found, 806.40.

**Example 64: preparation of 7-cyclopentyl-2-((5-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridin-2-yl)amino)-N,N-dimethyl-7H-pyrrolo[2,3-d]pyrimidine-6-carboxamide (GT-03657)**

Referring to the method of Scheme 4, the target compound (GT-03657) was prepared, which is a yellow solid (79 mg, yield 84.73%). $^1$H NMR (400 MHz, DMSO) δ 11.40 (s, 1H), 11.10 (d, *J* = 5.1 Hz, 1H), 8.98 (d, *J* = 2.9 Hz, 1H), 8.11 (dd, *J* = 14.2, 6.1 Hz, 1H), 7.93 (d, *J* = 10.4 Hz, 1H), 7.81 - 7.71 (m, 1H), 7.63 (dd, *J* = 7.6, 3.1 Hz, 1H), 7.36 (t, *J* = 7.8 Hz, 1H), 6.81 (s, 1H), 5.16 - 5.05 (m, 1H), 4.84 - 4.77 (m, 1H), 4.58 (s, 1H), 4.42 (dd, *J* = 20.1, 9.4 Hz, 1H), 4.22 - 4.12 (m, 2H), 3.70 (t, *J* = 11.0 Hz, 3H), 3.44 - 3.39 (m, 3H), 3.06 (s, 6H), 2.95 - 2.85 (m, 2H), 2.81 - 2.70 (m, 2H), 2.65 - 2.53 (m, 2H), 2.35 - 2.27 (m, 2H), 2.07 - 1.90 (m, 9H), 1.66 (dd, *J* = 12.4, 7.0 Hz, 2H), 1.40 (dd, *J* = 24.6, 13.2 Hz, 2H). LCMS (ESI) calcd for $C_{43}H_{49}FN_{11}O_5^+$ [M+H]$^+$: 818.38, found, 818.40.

**Example 65: preparation of 7-cyclopentyl-2-((5-(4-((3-(2-(2,6-dioxopiperidin-3-yl)-6-fluoro-1,3-dioxoisoindolin-5-yl)-3,8-diazabicyclo [3.2.1] octan-8-yl)methyl)piperidin-1-yl)pyridin-2-yl)amino)-N,N-dimethyl-7H-pyrrolo[2,3-d]pyrimidine-6-carboxamide (GT-03658)**

Referring to the method of Scheme 4, the target compound (GT-03658) was prepared, which is a yellow solid (27 mg, yield 29.36%). $^1$H NMR (400 MHz, DMSO) δ 11.57 (s, 1H), 11.11 (s, 1H), 9.01 (s, 1H), 8.18 (d, *J* = 9.7 Hz, 1H), 7.99 (s, 1H), 7.80 (d, *J* = 11.5 Hz, 1H), 7.66 (s, 1H), 7.47 (d, *J* = 7.2 Hz, 1H), 6.83 (s, 1H), 5.12 (dd, *J* = 12.6, 5.4 Hz, 1H), 4.84 - 4.79 (m, 1H), 4.19 (s, 1H), 4.05 (d, *J* = 12.4 Hz, 2H), 3.72 (d, *J* = 12.1 Hz, 3H), 3.57 (d, *J* = 11.1 Hz, 2H), 3.06 (s, 6H), 2.99 (dd, *J* = 15.5, 10.9 Hz, 2H), 2.85 (dd, *J* = 19.1, 9.6 Hz, 3H), 2.63 - 2.53 (m, 2H), 2.29 (ddd, *J* = 22.1, 14.2, 6.3 Hz, 6H), 2.06 - 1.97 (m, 7H), 1.65 (d, *J* = 5.9 Hz, 3H), 1.47 (dd, *J* = 24.2, 13.2 Hz, 2H). LCMS (ESI) calcd for $C_{44}H_{51}FN_{11}O_5^+$ [M+H]$^+$: 832.40, found, 832.40.

**Example 66: preparation of 7-cyclopentyl-2-((5-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridin-2-yl)amino)-N,N-dimethyl-7H-pyrrolo[2,3-d]pyrimidine-6-carboxamide (GT-03659)**

Referring to the method of Scheme 4, the target compound (GT-03659) was prepared, which is a yellow solid (82 mg, yield 7 94.63%). $^1$H NMR (400 MHz, DMSO) δ 11.44 (s, 2H), 11.09 (s, 1H), 8.98 (s, 1H), 8.09 (d, *J* = 9.5 Hz, 1H), 7.89 (s, 1H), 7.78 (d, *J* = 8.2 Hz, 1H), 7.61 (d, *J* = 9.2 Hz, 1H), 7.16 (s, 1H), 6.97 (d, *J* = 8.2 Hz, 1H), 6.81 (s, 1H), 5.10 (dd, *J* = 12.8, 5.4 Hz, 1H), 4.79 (dd, *J* = 17.5, 8.8 Hz, 1H), 4.65 (d, *J* = 4.5 Hz, 2H), 3.76 (d, *J* = 8.3 Hz, 2H), 3.63

(d, *J* = 11.9 Hz, 2H), 3.06 (s, 6H), 2.98 - 2.73 (m, 6H), 2.65 (dd, *J* = 22.4, 10.5 Hz, 3H), 2.55 (d, *J* = 11.4 Hz, 1H), 2.35 - 2.27 (m, 2H), 1.98 (ddd, *J* = 19.9, 17.5, 13.3 Hz, 9H), 1.64 (dd, *J* = 10.8, 4.9 Hz, 2H), 1.33 (dd, *J* = 22.9, 11.9 Hz, 2H). LCMS (ESI) calcd for $C_{43}H_{59}N_{11}O_5^+$ [M+H]$^+$: 800.39, found, 800.50.

**Example 66: preparation of 7-cyclopentyl-2-((5-(4-((4-(2-(2,6-dioxopiperidin-3-yl)-1,3-dioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide (GT-03660)**

Referring to the method of Scheme 4, the target compound (GT-03660) was prepared, which is a yellow solid (57 mg, yield 66.78%). $^1$H NMR (400 MHz, DMSO) δ 11.53 (s, 1H), 11.22 (s, 1H), 11.09 (s, 1H), 9.00 (s, 1H), 8.16 (dd, *J* = 9.6, 2.6 Hz, 1H), 7.94 (s, 1H), 7.76 (d, *J* = 8.5 Hz, 1H), 7.62 (d, *J* = 9.5 Hz, 1H), 7.49 (s, 1H), 7.36 (dd, *J* = 8.7, 1.9 Hz, 1H), 6.82 (s, 1H), 5.10 (dd, *J* = 12.9, 5.4 Hz, 1H), 4.81 (p, *J* = 8.8 Hz, 1H), 4.19 (d, *J* = 12.9 Hz, 2H), 3.73 - 3.65 (m, 5H), 3.19 - 3.08 (m, 4H), 3.06 (s, 6H), 2.86 (ddd, *J* = 26.7, 13.2, 7.2 Hz, 3H), 2.65 - 2.52 (m, 2H), 2.35 - 2.27 (m, 2H), 2.20 - 1.86 (m, 9H), 1.68 - 1.60 (m, 2H), 1.40 (dd, *J* = 20.7, 10.2 Hz, 2H). LCMS (ESI) calcd for $C_{42}H_{50}N_{11}O_5^+$ [M+H]$^+$: 788.39, found, 788.40.

**Example 67: preparation of 7-cyclopentyl-2-((5-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)pyridin-2-yl)amino)-N,N-dimethyl-7H-pyrrolo[2,3-d]pyrimidine-6-carboxamide (GT-03661)**

Referring to the method of Scheme 4, the target compound (GT-03660) was prepared, which is a yellow solid (57 mg, yield 64.47%). $^1$H NMR (400 MHz, DMSO) δ 11.55 (s, 1H), 11.08 (s, 1H), 9.00 (d, *J* = 4.0 Hz, 1H), 8.13 (dd, *J* = 9.8, 2.8 Hz, 1H), 7.95 (d, *J* = 18.1 Hz, 1H), 7.79 (dd, *J* = 8.5, 3.6 Hz, 1H), 7.60 (d, *J* = 9.4 Hz, 1H), 7.19 (dd, *J* = 8.5, 1.6 Hz, 1H), 7.12 (dd, *J* = 8.7, 2.2 Hz, 1H), 6.82 (d, *J* = 1.8 Hz, 1H), 5.11 - 5.06 (m, 1H), 4.84 - 4.79 (m, 1H), 4.65 (s, 1H), 4.15 (d, *J* = 12.2 Hz, 1H), 4.07 - 3.99 (m, 2H), 3.93 (d, *J* = 13.3 Hz, 2H), 3.68 (d, *J* = 12.4 Hz, 3H), 2.95 - 2.70 (m, 6H), 2.59 (d, *J* = 19.1 Hz, 2H), 2.30 (dd, *J* = 10.0, 7.3 Hz, 3H), 2.09 - 1.87 (m, 12H), 1.65 (d, *J* = 4.8 Hz, 3H), 1.46 - 1.35 (m, 2H). LCMS (ESI) calcd for $C_{43}H_{50}N_{11}O_5^+$ [M+H]$^+$: 800.39, found, 800.40.

**Example 68: preparation of 7-cyclopentyl-2-((5-(4-((4-((2-(2,6-dioxopiperidin-3-yl)-1-oxoisoindolin-5-yl)amino)piperidin-1-yl)methyl)piperidin-1-yl)pyridin-2-yl)amino)-N,N-dimethyl-7H-pyrrolo[2,3-d]pyrimidine-6-carboxamide (GT-03665)**

Referring to the method of Scheme 4, the target compound (GT-03665) was prepared, which is a yellow solid (75 mg, yield 82.55%). $^1$H NMR (400 MHz, DMSO) δ 11.63 (s, 1H), 10.93 (s, 1H), 9.02 (s, 1H), 8.20 (d, *J* = 9.5 Hz, 1H), 8.06 (s, 1H), 7.67 (dd, *J* = 9.5, 3.3 Hz, 1H), 7.46 -7.34 (m, 1H), 6.83 (s, 1H), 6.75 (s, 1H), 5.02 (dd, *J* = 13.2, 5.0 Hz, 1H), 4.84 - 4.77 (m, 1H), 4.29 (d, *J* = 16.8 Hz, 2H), 4.16 (d, *J* = 16.8 Hz, 2H), 3.88 - 3.78 (m, 1H), 3.69 (d, *J* = 11.8 Hz, 2H), 3.58 (d, *J* = 10.5 Hz, 2H), 3.41 - 3.33 (m, 1H), 3.05 (s, 7H), 3.00 (s, 1H), 2.93 - 2.82 (m, 3H), 2.58 (d, *J* = 16.7 Hz, 1H), 2.36 - 2.26 (m, 3H), 1.99 (ddd, *J* = 19.0, 17.8, 9.6 Hz, 12H), 1.69 - 1.59 (m, 2H), 1.46 (d, *J* = 11.0 Hz, 2H). LCMS (ESI) calcd for $C_{43}H_{52}N_{11}O_4^+$ [M+H]$^+$: 789.34, found, 789.30.

**Example 69: preparation of N-(2-chloro-6-methylphenyl)-2-((6-(4-((4-(2-(2,6-dioxopiperidin-3-yl)-1-oxoisoindolin-5-yl)piperidin-1-yl)methyl)piperidin-1-yl)-2-methylpyrimidin-4-yl)amino)thiazole-5-carboxamide (GT-03533)**

Referring to the method of Scheme 4, the target compound (GT-03533) was prepared, which is a white solid (26 mg, yield 32%). $^1$H NMR (300 MHz, DMSO) δ 11.45 (s, 1H), 11.02 (s, 1H), 9.91 (s, 1H), 8.22 (d, *J* = 14.3 Hz, 2H), 7.68 (d, *J* = 7.8 Hz, 1H), 7.53 (s, 1H), 7.43 (d, *J* = 7.6 Hz, 2H), 7.38 - 7.22 (m, 2H), 6.10 (s, 1H), 5.14 (dd, *J* = 13.2, 5.0 Hz, 1H), 4.39 (dd, *J* = 42.6, 17.3 Hz, 4H), 3.05 - 2.86 (m, 6H), 2.63 (d, *J* = 15.9 Hz, 2H), 2.43 (s, 4H), 2.27 (s, 6H), 2.13 - 1.97 (m, 4H), 1.83 (d, *J* = 13.9 Hz, 6H). LCMS (ESI) calcd for $C_{40}H_{45}ClN_9O_4S^+$ [M+H]$^+$: 782.3, found, 782.0.

**Example 70: preparation of N-(2-chloro-6-methylphenyl)-2-((6-(4-(((3S,4S)-4-(2-(2,6-dioxopiperidin-3-yl)-1-oxoisoindolin-5-yl)-3,4-dihydroxypiperidin-1-yl)methyl)piperidin-1-yl)-2-methylpyrimidin-4-yl)amino)thiazole-5-carboxamide (GT-03534)**

Referring to the method of Scheme 4, the target compound (GT-03534) was prepared, which is a white solid (4 mg, yield 4%). $^1$H NMR (300 MHz, DMSO) δ 11.45 (s, 1H), 11.02 (s, 1H), 9.91 (s, 1H), 8.22 (d, *J* = 19.0 Hz, 2H), 7.72 (d, *J* = 17.8 Hz, 3H), 7.44 (d, *J* = 6.8 Hz, 1H), 7.30 (d, *J* = 7.5 Hz, 2H), 6.10 (s, 1H), 5.13 (s, 1H), 4.78 (s, 1H), 4.60 - 4.53 (m, 1H), 4.40 (dd, *J* = 41.8, 17.2 Hz, 6H), 3.92 (s, 1H), 2.91 (s, 2H), 2.76 (s, 2H), 2.65 (s, 2H), 2.44 (s, 4H), 2.27 (s, 5H), 1.88 (s, 6H). LCMS (ESI) calcd for $C_{40}H_{45}ClN_9O_6S^+$ [M+H]$^+$: 813.2, found, 814.2.

**Example 71: preparation of N-(2-chloro-6-methylphenyl)-2-((6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-2-methylpyrimidin-4-yl)amino)thiazole-5-carboxamide (GT-03537)**

Referring to the method of Scheme 4, the target compound (GT-03537) was prepared, which is a yellow solid (10 mg, yield 11.23%). $^1$H NMR (400 MHz, DMSO) δ 11.39 (s, 1H), 11.09 (s, 1H), 9.86 (s, 1H), 8.21 (s, 1H), 7.68 (d, *J* = 12.1 Hz, 1H), 7.43 - 7.18 (m, 4H), 6.07 (s, 1H), 5.09 (dd, *J* = 12.8, 5.3 Hz, 1H), 4.28 (s, 2H), 3.35 (d, *J* = 9.5 Hz, 2H), 3.27 (s, 3H), 3.10 (d, *J* = 10.4 Hz, 2H), 2.88 (t, *J* = 12.1 Hz, 3H), 2.59 (d, *J* = 18.0 Hz, 1H), 2.40 (s, 3H), 2.23 (d, *J* = 7.7 Hz, 5H), 2.02 (d, *J* = 5.2 Hz, 1H), 1.89 (s, 4H), 1.71 (d, *J* = 6.8 Hz, 3H), 1.09 (d, *J* = 9.1 Hz, 2H). LCMS (ESI) calcd for $C_{41}H_{43}ClFN_{10}O_5S^+$ [M+H]$^+$: 840.27, found, 841.2.

**Example 72: preparation of N-(2-chloro-6-methylphenyl)-2-((6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-2-methylpyrimidin-4-yl)amino)thiazole-5-carboxamide (GT-03538)**

Referring to the method of Scheme 4, the target compound (GT-03538) was prepared, which is a yellow solid (20 mg, yield 21.69%). $^1$H NMR (400 MHz, DMSO) δ 11.36 (s, 1H), 11.08 (s, 1H), 9.85 (s, 1H), 8.20 (s, 1H), 7.62 (d, *J* = 13.9 Hz, 1H), 7.39 (d, *J* = 7.8 Hz, 1H), 7.27 (d, *J* = 11.7 Hz, 3H), 6.04 (s, 1H), 5.32 (s, 1H), 5.08 (d, *J* = 13.0 Hz, 1H), 4.22 (s, 2H), 3.86 (d, *J* = 10.6 Hz, 2H), 3.62 (s, 4H), 2.83 (s, 2H), 2.55 (d, *J* = 11.3 Hz, 1H), 2.36 (d, *J* = 22.5 Hz, 5H), 2.25 (d, *J* = 9.4 Hz, 5H), 2.02 (d, *J* = 7.4 Hz, 2H), 1.78 (s, 2H), 1.60 (d, *J* = 8.5 Hz, 2H), 1.48 - 1.42 (m, 1H). LCMS (ESI) calcd for $C_{40}H_{41}ClFN_{10}O_5S^+$ [M+H]$^+$: 826.26, found, 827.5.

**Biological activity assay**

**Reagents and Materials**

| Reagents and materials | Suppliers or Source |
|---|---|
| Human prostate cancer cell line: DU-145 | ATCC (American Type Culture Collection) |
| Human prostate cancer cell line: LNcap | ATCC |
| Human ovarian cancer cell line: PC-3 | ATCC |
| dexamethasone-resistant multiple myeloma cell line: MM. 1R | ATCC |
| Human diffuse large B-cell lymphoma cell line: WSU-DLCL2 | Shanghai Medicilon Inc. (test) |
| Human gastric cancer cell line: MGC80-3 | Dalian Meilun Biotech Co., Ltd. |
| Human colorectal cancer cell line: SW620 | ATCC |
| Human non-small cell lung cancer cell line: A549 | ATCC |
| Human T cell acute lymphoblastic leukemia cell line: CCRF-CEM | Dalian Meilun Biotech Co., Ltd. |
| Human liver cancer cell line: HEP3B2.7-1 | Dalian Meilun Biotech Co., Ltd. |
| Human Burkitt's lymphoma cell line : Daudi | Dalian Meilun Biotech Co., Ltd. |
| Human acute lymphoblastic leukemia cell line: Kasumi-1 | ATCC |
| Human diffuse large B-cell lymphoma cell line: SU-DHL-6 | ATCC |
| Human diffuse large B-cell lymphoma cell line: TMD8 | Kyinno Biotechnology Co., Ltd (test) |
| Human prostate cancer cell line: 22RV1 | Shanghai Medicilon Inc. (test) |
| Human prostate cancer cell line: C4-2 | Shanghai Medicilon Inc. (test) |
| CEREBLON BINDING KITS kit | Cisbio Company |
| F-12K Medium | ATCC |
| EMEM Medium | Dalian Meilun Biotech Co., Ltd. |
| Meilun Cell Counting Kit-8 | Dalian Meilun Biotech Co., Ltd. |
| Fetal bovine serum (FBS) | Sunrise Science Products Company |
| Penicillin-Streptomycin | Beijing TransGen Biotech Co., Ltd. |

**Methods:**

**Determination of CRBN-binding affinity of compounds**

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:

**1.** According to the instructions of the CEREBLON BINDING kits, the compounds to be tested of the present invention and lenalidomide were serially diluted using diluent #9 (1X) solution to obtain a final concentration of 2 μM for both the tested compounds and lenalidomide solution.
**2.** 2.5 μL of the above 2 μM of the tested compounds and lenalidomide solution, as well as the same volume of diluent #9 (1X) solution were taken and added to each well of a 96-well plate, respectively. Then, 2.5 μL of (human Cereblon WT GST-tagged protein) solution was added to each well. Finally, 5 μL of the uniformly mixed thalidomide-Red reagent and GST Eu antibody working solution were added to each of the above wells. The final concentration of the tested compounds and lenalidomide in each well is 0.5 μM.
**3.** The blank control wells were sequentially added with 2.5 μL of diluent #9 (1X) solution, 2.5 μL of ligand binding buffer, and 5 μL of uniformly mixed thalidomide-Red reagent and GST Eu antibody working solution.
**4.** After sealing and incubating the solutions in the above wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method:

$$R_{compound} = OD\ 665\ nm_{compound} / OD\ 620\ nm_{compound} - OD\ 665\ nm_{control} / OD\ 620\ nm_{control}$$

$$R_{Std0} = OD\ 665\ nm_{Std0} / OD\ 620\ nm_{Std0} - OD\ 665\ nm_{control} / OD\ 620\ nm_{control}$$

$$\text{inhibition rate (\%)} = (1 - R_{compound}/R_{Std0}) * 100$$

Note: OD 665 nm $_{compound}$ is the absorbance value of each well of the compounds to be tested of the present disclosure at an emission wavelength of 665 nm.

OD 620 nm $_{compound}$ is the absorbance value of each well of the compounds to be tested of the present disclosure at an emission wavelength of 620 nm.

OD 665 nm $_{control}$ is the absorbance value of the blank control well at an emission wavelength of 665 nm.

OD 620 nm $_{control}$ is the absorbance value of the blank control well at an emission wavelength of 620 nm.

OD 665 nm $_{Std0}$ is the absorbance value of the solvent control well at an emission wavelength of 665 nm.

OD 620 nm $_{Std0}$ is the absorbance value of the solvent control well at an emission wavelength of 620 nm.

The test results show that the CRBN binding activity $IC_{50}$ value of lenalidomide (Lena-$NH_2$) is 2.38 μM. The binding ability of the compounds of the present invention to CRBN is superior to or equivalent to that of lenalidomide. The results of the binding affinity test are shown in Table 5 below.

Table 5. HTRF inhibitory activity (ICso, μM) of the compounds of the present invention

| Compound No. | HTRF $IC_{50}$ (μM) | Compound No. | HTRF $IC_{50}$ (μM) |
|---|---|---|---|
| Lena-$NH_2$ | 2.38 | GT-03100 | 9.80 |
| GT-03106 | 4.04 | GT-03075 | 5.09 |
| GT-03109 | 3.55 | GT-03210 | 2.69 |
| GT-03077 | 5.20 | GT-03211 | 9.80 |
| GT-03108 | 8.75 | GT-03076 | 0.65 |

**Determination of half inhibitory concentration ($IC_{50}$) of the compounds against tumor cells:**

**Cell cultures**

The tumor cells used herein were cultured in a cell culture medium listed in table 6 at 37°C in an incubator with 5% $CO_2$. Prior to experimentation, all cells used were correctly identified by STR profiling, and tested negative for mycoplasma through routine screenings.

Table 6. Tumor cells and cell culture medium used in the present disclosure

| Cell line | Cell culture medium used |
|---|---|
| CCRF-CEM | RPMI 1640 culture medium supplemented with 10% FBS, as well as penicillin at a final concentration of 100 U/mL and streptomycin at a final concentration of 100 μg/mL |
| Kasumi-1 | |
| WSU-DLCL2 | |
| MM.1R | |
| MGC80-3 | |
| Daudi | |
| SU-DHL-6 | |
| TMD8 | |
| SW620 | L-15 culture medium supplemented with 10% FBS, as well as penicillin at a final concentration of 100 U/mL and streptomycin at a final concentration of 100 μg/mL |
| DU-145 | EMEM culture medium supplemented with 10% FBS, as well as penicillin at a final concentration of 100 U/mL and streptomycin at a final concentration of 100 μg/mL |
| LNcap | F-12K culture medium supplemented with 10% FBS, as well as penicillin at a final concentration of 100 U/mL and streptomycin at a final concentration of 100 μg/mL |
| PC-3 | |
| A549 | |
| HEP3B2.7-1 | MEM culture medium supplemented with 10% FBS, as well as penicillin at a final concentration of 100 U/mL and streptomycin at a final concentration of 100 μg/mL |

**Assay method:**

$IC_{50}$ values of the compounds of the present disclosure (including the compounds in Table 4 and compounds of examples 1-72) were measured using the Meilun Cell Counting Kit-8 kit from Dalian Meilun Biotech Co., Ltd. Assay details are as follows: tumor cells were seeded in a 96-well plate at a density listed in Table 7. After 24h, 100 μL of the inoculated cells were treated with 0.5μL of the compounds of the present disclosure (including the compounds in Table 4 and compounds of examples 1-72) at 10 different successively decreasing concentrations (starting at the highest concentration of 10μM; 5-fold serial dilutions). After the cells were treated with the compounds of the present disclosure for a period of time (the treatment times listed in Table 7), the cell viability detection reagent was added to the culture medium for cell viability assessment according to the instructions provided with the CCK-8 kit. DMSO served as the negative control, and corresponding commercial inhibitors (including Enzalutamide (CAS No.: 915087-33-1); Ribociclib (CAS No.: 1211443-58-1); Dasatinib (CAS No.: 302962-49-8)) were used as the positive control, both of which treated the cells using the same protocol as that of the compounds of the present disclosure. The growth inhibition of the compounds of the present disclosure on cells was plotted by Prism Graphpad software, and the $IC_{50}$ values of the compounds of the present disclosure were calculated therefrom. Results were shown in Tables 8-10 below.

Table 7. Seeding protocol and treatment time for tumor cells

| Cells | Cells seeding protocol | The times for treating cells with the compounds of the present disclosure (hours) |
|---|---|---|
| Kasumi-1 | cells were seeded in 100 μL RPMI1640 medium containing serum at a density of 10,000 cells/well | 72h |
| MM.1R | | |
| SU-DHL-6 | | |
| TNM8 | | |
| CCRF-CEM | cells were seeded in 100 μL RPMI1640 medium containing serum at a density of 4000 cells/well | 96h |
| WSU-DLCL2 | | |
| MGC80-3 | | |
| Daudi | | |
| DU-145 | cells were seeded in 100 μL EMEM medium containing serum at a density of 4000 cells/well | 96h |
| SW620 | cells were seeded in 100 μL L-15 medium containing serum at a density of 4000 cells/well | 96h |
| LNcap | cells were seeded in 100 μL F-12K medium containing serum at a density of 4000 cells/well | 96h |
| PC-3 | | |
| A549 | | |
| HEP3B2.7-1 | cells were seeded in 100 μL MEM medium containing serum at a density of 4000 cells/well | 96h |

Table 8. $IC_{50}$ values (in nM) of the compounds of examples of the present disclosure, designed based on the AR inhibitors, for inhibiting the proliferation of tumor cells

| Comp. No. /names | LNcap | PC-3 | DU-145 | MM.1 R | WSU-DLCL 2 | MGC8 0-3 | SW620 | A549 | CCRF-CEM | Hep3B 2.1-7 |
|---|---|---|---|---|---|---|---|---|---|---|
| Enzaluta mide | - | >1000 0 | >1000 0 | >1000 0 | >1000 0 | - | >10000 | - | - | - |
| GT-03339 | B | B | B | - | B | A | - | - | - | - |
| GT-03490 | B | - | D | B | B | B | - | - | - | - |
| GT-03488 | A+ | - | A | A+ | - | A+ | - | A | - | A+ |
| GT-03489 | B | - | D | C | B | B | - | - | - | B |
| GT-03486 | B | - | - | B | B | A | - | B | B | - |
| GT-03487 | B | - | D | B | B | B | - | - | - | - |
| GT-02765 | A+ | A+ | A | B | A+ | A+ | A+ | A+ | | A+ |
| GT-03773 | - | - | - | - | - | - | B | - | - | - |
| GT-03016 | C | C | C | D | D | B | A | - | - | - |
| GT-03449 | A | - | C | B | - | A | - | - | B | A |
| GT-03450 | B | - | D | B | C | B | - | - | - | - |
| GT-03451 | B | - | D | B | B | C | - | - | - | - |
| GT-03427 | C | - | D | - | B | B | - | - | - | - |
| GT-03471 | C | - | - | - | D | B | - | - | - | B |
| GT-03425 | A | - | C | B | A | A+ | - | - | - | A+ |
| GT-03426 | A | - | - | B | - | A | - | - | - | A+ |
| GT-03481 | B | - | - | - | B | B | - | B | - | A+ |
| GT-03403 | C | B | B | - | E | C | - | - | - | - |
| GT-03404 | C | - | - | - | - | B | - | - | - | B |
| GT-03402 | A | A | A | - | B | - | - | B | - | - |
| GT-03482 | A | - | B | B | B | A | - | - | - | B |
| GT-03494 | A | - | D | - | A | A | - | B | - | A |
| GT-03400 | B | B | B | - | C | B | - | - | - | - |
| GT-03401 | C | B | C | - | D | B | - | - | - | - |

(continued)

| Comp. No. /names | LNcap | PC-3 | DU-145 | MM.1 R | WSU-DLCL 2 | MGC8 0-3 | SW620 | A549 | CCRF-CEM | Hep3B 2.1-7 |
|---|---|---|---|---|---|---|---|---|---|---|
| GT-03399 | B | B | B | - | C | - | - | - | - | B |
| GT-03398 | A | A+ | A | - | - | A+ | - | A | - | - |

Table 9. $IC_{50}$ values (in nM) of the compounds of examples of the present disclosure, designed based on the CDK4/6 inhibitors for inhibiting the proliferation of tumor cells

| Comp. No. /names | SW620 |
|---|---|
| Ribociclib | >10000 |
| GT-03651 | B |

Table 10. $IC_{50}$ values (in nM) of the compounds of examples of the present disclosure, designed based on the BCR-ABL inhibitors for inhibiting the proliferation of tumor cells

| Comp. No. /names | Daudi | Kasumi-1 | SU-DHL-6 | TMD8 |
|---|---|---|---|---|
| Dasatinib | B | A+ | A+ | A+ |
| GT-03630 | - | A+ | B | - |
| GT-03628 | - | B | - | - |
| GT-03631 | B | B | A+ | - |
| GT-03538 | B | B | A+ | A+ |
| GT-03629 | - | B | B | - |
| GT-03627 | - | B | B | - |
| GT-03632 | A | A+ | A+ | - |
| GT-03537 | B | A | A+ | A+ |
| Note: In Tables 8-10, the symbols A+, A, B, C, D, and E are defined as follows: A+ ≤ 50 nM; 50 nM < A ≤ 100 nM; 100 nM < B ≤ 500 nM; 500 nM < C ≤ 1000 nM; 1000 nM < D ≤ 5000 nM; 5000 nM < E < 10000 nM; symbol "-" represents undetected. | | | | |

The results indicate that the compounds of the present invention (including the compounds in Table 4 and Examples 1-72) can inhibit the proliferation of tumor cells (as shown in Tables 8-10 and Figure 1), with inhibitory effects superior to or comparable with the corresponding positive control drugs (including enzalutamide, ribociclib, and dasatinib), as well as degradation effect better than that of the corresponding positive control drugs.

**Determination of Half-Maximal Target Protein Degradation Concentration ($DC_{50}$) of the compounds of the present disclosure to target proteins**

**Western-blot assay**

(1) Cell seeding: tumor cells (human prostate cancer cells 22RV1 or human prostate cancer cells C4-2) were added to a 6-well plate at a cell density of $2.5\times10^5$/mL. The total volume of cell suspension in each well is 2mL. The compounds of the present disclosure (including the compounds in table 4 and the compounds of example 1-72) to be tested were set at 5 concentration gradients of 1nM, 2nM, 10nM, 20nM and 50nM, respectively. Meanwhile, DMSO solvent (containing 0 nM of the compounds to be tested) was set as a negative control.
(2) Protein collecting: after treating the cells with the compounds for 24 hours, the cell culture medium was removed. The cells were washed with 1ml of PBS, followed by removing the PBS and sequentially addition of 100μL of RAPI lysis buffer (containing protease inhibitors). The mixture was placed on ice. The lysed cells were scraped off using a cell scraper, and transferred into a 1.5ml EP tube. Following this, the tube was placed on ice for 30-60 min, and centrifuged at 12,000 rpm for 5 min. The supernatant was aspirated as the extracted total cell protein. Protein concentrations were measured using the Bradford method, and bromophenol blue was incorporated as a loading dye.
(3) Electrophoresis: 15μg of the proteins collected after cell lysis were loaded for electrophoresis. Electrophoresis set at a constant voltage of 80V for 15min, and once the dye entered the separating gel, the voltage was adjusted to 120V for 40min.

(4) Membrane transfer: filter paper and nitrocellulose membrane (NC membrane) were prepared in an appropriate size. Then the filter paper and NC membrane were soaked in the transfer electrophoresis buffer. They were then placed in the electrophoresis tank in the following order: "filter paper-gel-NC membrane-filter paper," and the membrane was transferred with a constant current of 400mA for 1h. Subsequent antibody incubation and development are performed according to the antibody instructions provided by Cell Signaling Technology.

Determining Half-Maximal Degradation Concentration ($DC_{50}$) value (the drug concentration required for degrading proteins by 50%, abbreviated as $DC_{50}$) reads method: comparing the grayscale values of the Western blotting bands for the drug treatment with those of the Western blotting bands after treatment with blank DMSO. The $DC_{50}$ is identified within the range of drug concentrations where the grayscale value matches half of the grayscale value of the corresponding Western blotting band after treatment with blank DMSO.

Calculation of the $DC_{50}$ values can be performed using ImageJ to measure the grayscale values of Western blotting bands after drug treatment. A curve fitting the relationship between drug concentration and grayscale values is then generated to estimate the drug concentration corresponding to half of the grayscale value. A curve is then fitted to the relationship between drug concentration and grayscale values, allowing estimation of the drug concentration that corresponds to half of the grayscale value.

The present invention assessed the effects of the compounds of the present disclosure (including compounds in Table 4 and compounds of examples 1-72) on the protein expression levels of full-length androgen receptor (AR-FL) and androgen receptor splice variant 7 (AR-V7) in human prostate cancer cell lines 22RV1 and C4-2. Human prostate cancer cell line 22RV1 highly expresses AR-FL and AR-V7 splice variant, while human prostate cancer cell line C4-2 highly expresses AR-FL. The expression of AR-V7 splice variant, which lacks the LBD domain, represents one of the significant mechanisms that contribute to resistance against the AR antagonist enzalutamide. The Western-blot detection results were shown in Figures 2-3.

Experimental results demonstrate that, compared with the DMSO control group, the compounds of the present invention (including compounds in Table 4 and compounds of examples 1-72) exhibit a significant degradation effect on both AR-FL and the AR-V7 splice variant.

The basic principles, main features and advantages of the present disclosure are shown and described above. Those skilled in the art should understand that the present disclosure is not limited by the foregoing embodiments, and they can make various changes, substitutions and alterations herein without departing from the spirit and scope of the present disclosure. These changes, substitutions and alterations fall within the scope of the present disclosure. The claimed scope of the present disclosure is defined by the appended claims and their equivalents.

## Claims

**1.** A compound of Formula (I)

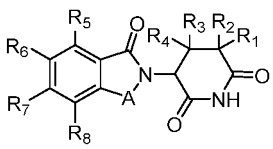

Formula (I)

or salts, enantiomers, stereoisomers, solvates, isotopically enriched analogs, prodrugs, or polymorphs thereof,

wherein A represents CO, $CH_2$ or $CD_2$;

$R_1$, $R_2$, $R_3$ and $R_4$ are the same or different and each independently represent hydrogen, deuterium, halogen, optionally deuterated $C_{1-6}$ alkyl, optionally deuterated $C_{1-6}$ alkoxy, or halogenated $C_{1-6}$ alkyl;

$R_5$, $R_6$, $R_7$ and $R_8$ are the same or different and each independently represent $R_9$, hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl, or $C_{2-6}$ alkynyl, wherein one to three of $R_5$, $R_6$, $R_7$ and $R_8$ represents $R_9$;

$R_9$ represents R-U- group, wherein

U represents a bond, or U represents $N(R_{10})$ or O, wherein $R_{10}$ represents H or optionally deuterated $C_{1-6}$ alkyl;

R represents optionally substituted nitrogen-containing heterocyclyl, said optionally substituted nitrogen-containing heterocyclyl is optionally substituted nitrogen-containing monocyclic heterocyclyl or optionally substituted nitrogen-containing bridged heterocyclyl;

wherein

when two to four of $R_5$, $R_6$, $R_7$, and $R_8$ independently represent $R_9$, the compound of Formula (I) is optionally partially or fully deuterated; or
when only one of $R_5$, $R_6$, $R_7$, and $R_8$ represents $R_9$, the compound of Formula (I) is optionally partially or fully deuterated;
wherein in case that only one of $R_5$, $R_6$, $R_7$, and $R_8$ represents $R_9$ and the compound of Formula (I) is not deuterated:

when R represents optionally substituted nitrogen-containing bridged heterocyclyl, the following compounds are excluded:

5-(2,5-diazabicyclo[2.2.1]heptan-2-yl)-2-(2,6-dioxopiperidin-3-yl)isoindoline-1,3-dione;
5-(2,5-diazabicyclo[2.2.1]heptan-2-yl)-2-(2,6-dioxopiperidin-3-yl)-6-fluoroisoindoline-1,3-dione;
5-(2,5-diazabicyclo[2.2.1]heptan-2-yl)-2-(2,6-dioxopiperidin-3-yl)-6-fluoroisoindoline-1,3-dione;
3-(5-(2,5-diazabicyclo[2.2.1]heptan-2-yl)-1-oxolsoindolin-2-yl)pipendine-2,6-dione; 3-(5-(8-azabicyclo[3.2.1]octan-3-yl)-1-oxoisoindolin-2-yl)piperidine-2,6-dione; and
3-(5-(9-ethyl-3-methyl-3,9-diazabicyclo[3.3.1]nonan-7-yl)-1-oxoisoindolin-2-yl)piperidine-2,6-dione; or

when A represents CO and one of $R_6$ and $R_7$ represents $R_9$, where R represents substituted nitrogen-containing monocyclic heterocyclyl, and the other one of $R_6$ and $R_7$, as well as $R_5$ and $R_8$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, one to three of the other one of $R_6$ and $R_7$ as well as $R_5$ and $R_8$ represents halogen, and the nitrogen-containing monocyclic heterocyclyl is substituted with one or more substituents selected from the group consisting of hydroxy, amino, mercapto, nitro, halogen, cyano, $C_{1-6}$ alkyl, $C_{1-6}$ alkoxy, halogenated $C_{1-6}$ alkyl, $NH_2$-$C_{1-6}$ alkylene, $C_{1-6}$ alkyl-NH-, $C_{1-6}$ alkyl-NHC(O)-, $C_{1-6}$ alkyl-C(O)NH- or any combination thereof;
when A represents CO and one of $R_6$ and $R_7$ represents $R_9$, where R represents unsubstituted nitrogen-containing monocyclic heterocyclyl, the other one of $R_6$ and $R_7$ represents hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, and $R_5$ and $R_8$ each independently represent halogen;
when A represents CO and one of $R_5$ and $R_8$ represents $R_9$, where R represents substituted nitrogen-containing monocyclic heterocyclyl, and the other one of $R_5$ and $R_8$, as well as $R_6$ and $R_7$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, one to three of the other one of $R_5$ and $R_8$ as well as $R_6$ and $R_7$ represents halogen, and the nitrogen-containing monocyclic heterocyclyl is substituted with one or more substituents selected from the group consisting of hydroxy, amino, mercapto, nitro, halogen, cyano, CHO, $C_{1-6}$ alkyl, halogenated $C_{1-6}$ alkyl, $C_{3-6}$ cycloalkyl, $C_{1-6}$ alkoxy, $C_{1-6}$ alkyl-NH-, $NH_2$-$C_{1-6}$ alkylene, $C_{1-6}$ alkyl-NHC(O)-, $C_{1-6}$ alkyl-C(O)NH- or any combination thereof;
when A represents CO and one of $R_5$ and $R_8$ represents $R_9$, where R represents unsubstituted nitrogen-containing monocyclic heterocyclyl, the other one of $R_5$ and $R_8$ and/or $R_7$ each independently represent halogen, and $R_6$ represents hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl;
when A represents $CH_2$ and one of $R_6$ and $R_7$ represents $R_9$, where R represents substituted nitrogen-containing monocyclic heterocyclyl, and the other one of $R_6$ and $R_7$, as well as $R_5$ and $R_8$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, one to three of the other one of $R_6$ and $R_7$ as well as $R_5$ and $R_8$ represents halogen, and the nitrogen-containing monocyclic heterocyclyl is substituted with one or more substituents selected from the group consisting of hydroxy, amino, mercapto, nitro, halogen, CHO, cyano, $C_{1-6}$ alkyl, halogenated $C_{1-6}$ alkyl, $C_{3-6}$ cycloalkyl, $C_{1-6}$ alkoxy, $C_{1-6}$ alkyl-NH-, $NH_2$-$C_{1-6}$ alkylene, $C_{1-6}$ alkyl-NHC(O)-, $C_{1-6}$ alkyl-C(O)NH- or any combination thereof;
when A represents $CH_2$ and one of $R_6$ and $R_7$ represents $R_9$, where R represents unsubstituted nitrogen-containing monocyclic heterocyclyl, $R_5$ represents halogen, and the other one of $R_6$ and $R_7$ and/or $R_8$

each independently represent halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl;

when A represents $CH_2$ and one of $R_5$ and $R_8$ represents $R_9$, where R represents substituted nitrogen-containing monocyclic heterocyclyl, and the other one of $R_5$ and $R_8$, as well as $R_6$ and $R_7$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, one to three of the other one of $R_5$ and $R_8$ as well as $R_6$ and $R_7$ represent halogen, and the nitrogen-containing monocyclic heterocyclyl is substituted with one or more substituents selected from the group consisting of hydroxy, amino, mercapto, nitro, halogen, CHO, cyano, $C_{1-6}$ alkyl, halogenated $C_{1-6}$ alkyl, $C_{3-6}$ cycloalkyl, $C_{1-6}$ alkoxy, $C_{1-6}$ alkyl-NH-, $NH_2$-$C_{1-6}$ alkylene, $C_{1-6}$ alkyl-NHC(O)-, $C_{1-6}$ alkyl-C(O)NH- or any combination thereof; or

when A represents $CH_2$ and one of $R_5$ and $R_8$ represents $R_9$, where R represents unsubstituted nitrogen-containing monocyclic heterocyclyl, and the other one of $R_5$ and $R_8$, as well as $R_6$ and $R_7$ each independently represent hydrogen, halogen, hydroxy, mercapto, nitro, amino, cyano, $C_{1-6}$ alkyl, $C_{1-6}$ alkoxy, halogenated $C_{1-6}$ alkyl, $C_{2-6}$ alkenyl, or $C_{2-6}$ alkynyl, the other one of $R_5$ and $R_8$, as well as $R_6$ and $R_7$ are not simultaneously hydrogen, and when $R_6$ is halogen, the other one of $R_5$ and $R_8$, as well as $R_7$ are not simultaneously hydrogen.

**2.** The compound of Formula (I) or salts, enantiomers, stereoisomers, solvates, isotopically enriched analogs, prodrugs, or polymorphs thereof as claimed in claim 1, wherein the compound of Formula (I) is also of Formula (I-1) or Formula (I-2):

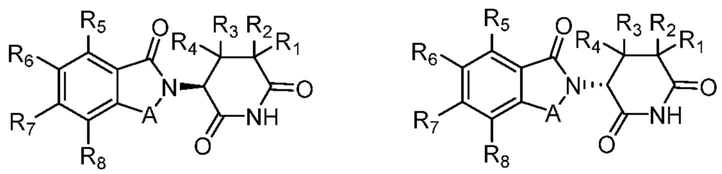

Formula (I-1) or Formula (I-2)

wherein A, $R_1$, $R_2$, $R_3$, $R_4$, $R_5$, $R_6$, $R_7$ and $R_8$ are as defined in claim 1.

**3.** The compound of Formula (I) or salts, enantiomers, stereoisomers, solvates, isotopically enriched analogs, prodrugs, or polymorphs thereof as claimed in claim 1 or 2, wherein when two to four of $R_5$, $R_6$, $R_7$ and $R_8$ independently represent $R_9$, the compound of Formula (I) is optionally partially deuterated, for example, carrying 1 to 20 deuterium atom, or the compound of Formula (I) is optionally fully deuterated.

**4.** The compound of Formula (I) or salts, enantiomers, stereoisomers, solvates, isotopically enriched analogs, prodrugs, or polymorphs thereof as claimed in claim 1 or 2, wherein when only one of $R_5$, $R_6$, $R_7$ and $R_8$ represents $R_9$, the compound of Formula (I) is partially deuterated, for example, carrying 1 to 15 deuterium atom, or the compound of Formula (I) is fully deuterated.

**5.** The compound of Formula (I) or salts, enantiomers, stereoisomers, solvates, isotopically enriched analogs, prodrugs, or polymorphs thereof as claimed in claim 1 or 2, wherein the nitrogen-containing monocyclic heterocyclyl or nitrogen-containing bridged heterocyclyl is optionally substituted with one or more substituents selected from the group consisting of deuterium, hydroxy, amino, mercapto, nitro, halogen, cyano, CHO, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

**6.** The compound of Formula (I) or salts, enantiomers, stereoisomers, solvates, isotopically enriched analogs, prodrugs, or polymorphs thereof as claimed in claim 1 or 2, wherein

the nitrogen-containing monocyclic heterocyclyl is a 4- to 10-membered monocyclic heterocyclyl group containing one nitrogen atom and optionally a heteroatom selected from nitrogen, oxygen, and sulfur, e.g., piperidinyl, piperazinyl, morpholinyl, azetidinyl, pyrrolidinyl, imidazolidinyl, pyrazolidyl, oxazolidinyl, thiazolidinyl, thiomorpholinyl, azepanyl, diazacycloheptanyl, azacyclooctyl or diazacyclooctyl; or

the nitrogen-containing bridged heterocyclyl is a $C_{5-20}$ bridged heterocyclyl group containing one nitrogen atom

and optionally a heteroatom selected from nitrogen, oxygen, and sulfur, e.g., azabicyclo[3.1.1]heptanyl, azabicyclo[2.2.1]heptanyl, azabicyclo[3.2.1]octanyl, azabicyclo[2.2.2]octanyl, diazabicyclo [3.1.1]heptanyl, diazabicyclo[2.2.1]heptanyl, diazabicyclo[3.2.1]octanyl or diazabicyclo[2.2.2]octanyl, such as the following groups:

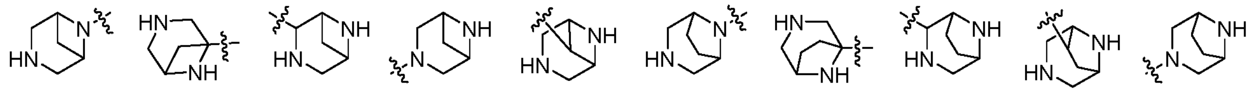

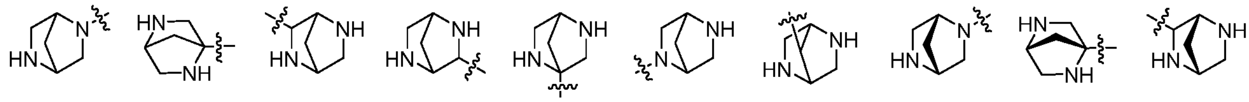

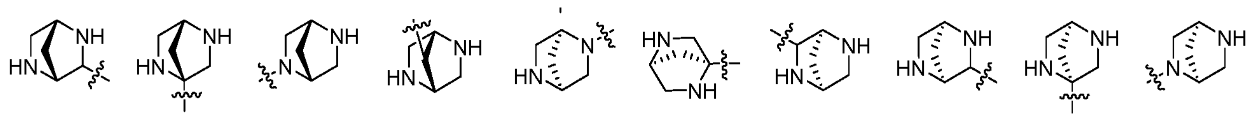

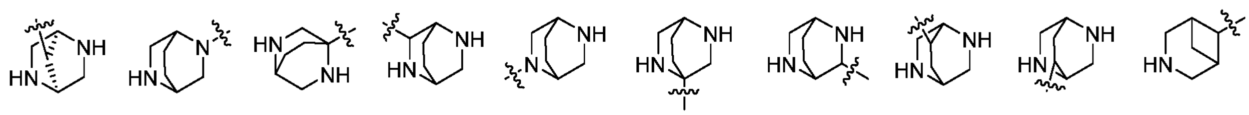

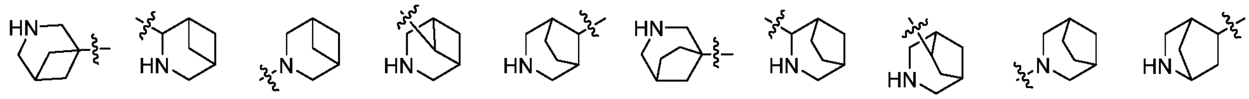

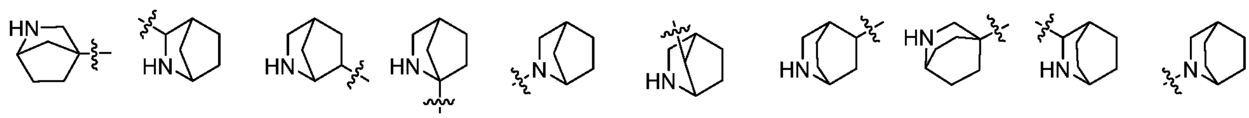

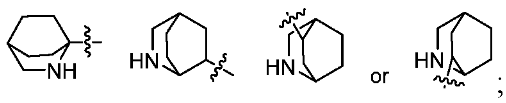

or ;

wherein the nitrogen-containing monocyclic heterocyclyl and nitrogen-containing bridged heterocyclyl are optionally substituted with one or more substituents selected from the group consisting of deuterium, hydroxy, amino, mercapto, nitro, halogen, cyano, CHO, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

**7.** The compound of Formula (I) or salts, enantiomers, stereoisomers, solvates, isotopically enriched analogs, prodrugs, or polymorphs thereof as claimed in claim 1 or 2, wherein $R_9$ represents the following groups:

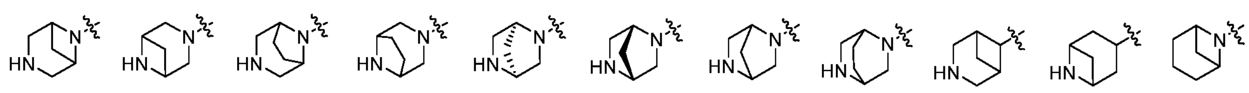

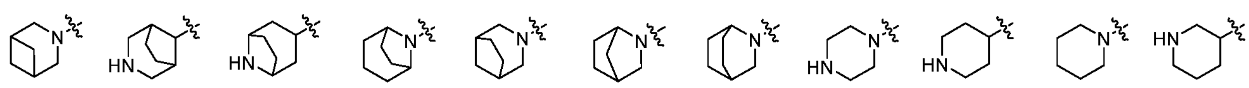

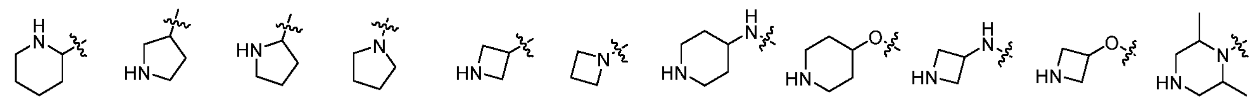

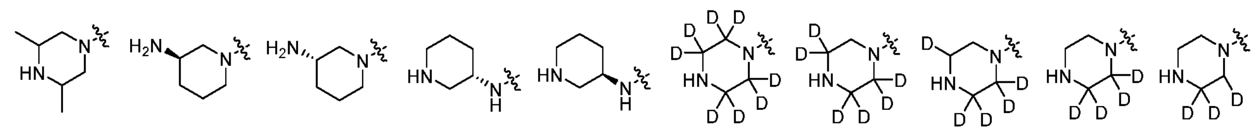

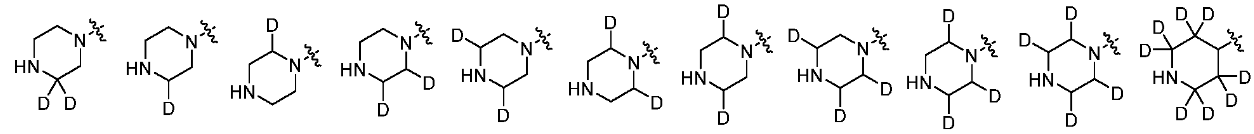

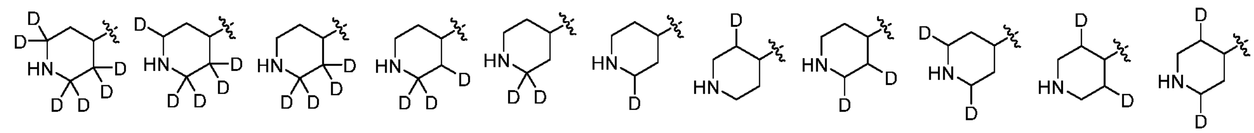

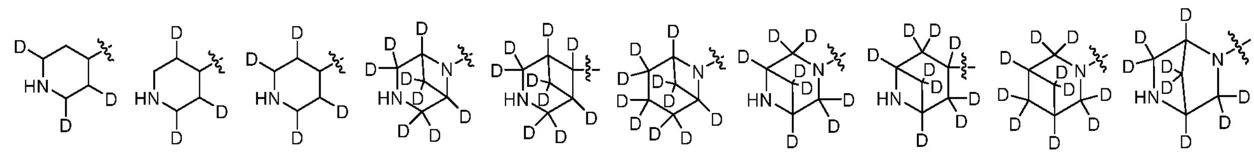

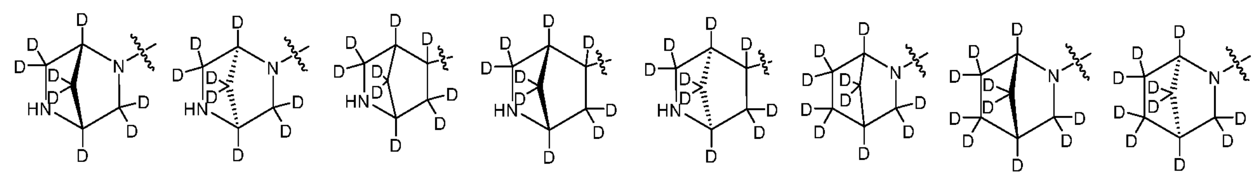

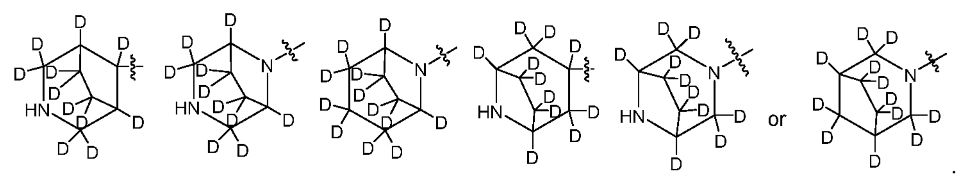

8. The compound of Formula (I) or salts, enantiomers, stereoisomers, solvates, isotopically enriched analogs, prodrugs, or polymorphs thereof as claimed in claim 1, which is selected from:

5-(3,6-diazabicyclo[3.1.1]heptan-6-yl)-2-(2,6-dioxopiperidin-3-yl)isoindoline-1,3-dione;
5-(3,6-diazabicyclo[3.1.1]heptan-3-yl)-2-(2,6-dioxopiperidin-3-yl)isoindoline-1,3-dione;
5-(3,8-diazabicyclo[3.2.1]octan-8-yl)-2-(2,6-dioxopiperidin-3-yl)isoindoline-1,3-dione;
5-(3,8-diazabicyclo[3.2.1]octan-3-yl)-2-(2,6-dioxopiperidin-3-yl)isoindoline-1,3-dione;
5-(2,5-diazabicyclo[2.2.2]octan-2-yl)-2-(2,6-dioxopiperidin-3-yl)isoindoline-1,3-dione;
2-(2,6-dioxopiperidin-3-yl)-5-(piperazin-1-yl-2,2,3,3,5,5,6,6-$d_8$)isoindoline-1,3-dione;
5-(3-azabicyclo[3.1.1]heptan-6-yl)-2-(2,6-dioxopiperidin-3-yl)isoindoline-1,3-dione;
5-(6-azabicyclo[3.1.1]heptan-3-yl)-2-(2,6-dioxopiperidin-3-yl)isoindoline-1,3-dione;
5-(3-azabicyclo[3.2.1]octan-8-yl)-2-(2,6-dioxopiperidin-3-yl)isoindoline-1,3-dione;
5-(8-azabicyclo[3.2.1]octan-3-yl)-2-(2,6-dioxopiperidin-3-yl)isoindoline-1,3-dione;
5-(2-azabicyclo[2.2.1]heptan-5-yl)-2-(2,6-dioxopiperidin-3-yl)isoindoline-1,3-dione;
5-(2-azabicyclo[2.2.2]octan-5-yl)-2-(2,6-dioxopiperidin-3-yl)isoindoline-1,3-dione;

5-(3,6-diazabicyclo[3.1.1]heptan-6-yl)-2-(2,6-dioxopiperidin-3-yl)-6-fluoroisoindoline-1,3-dione;
5-(3,6-diazabicyclo[3.1.1]heptan-3-yl)-2-(2,6-dioxopiperidin-3-yl)-6-fluoroisoindoline-1,3-dione;
5-(3,8-diazabicyclo[3.2.1]octan-8-yl)-2-(2,6-dioxopiperidin-3-yl)-6-fluoroisoindoline-1,3-dione;
5-(3,8-diazabicyclo[3.2.1]octan-3-yl)-2-(2,6-dioxopiperidin-3-yl)-6-fluoroisoindoline-1,3-dione;
5-((1S,4S)-2,5-diazabicyclo[2.2.1]heptan-2-yl)-2-(2,6-dioxopiperidin-3-yl)-6-fluoroisoindoline-1,3-dione;
5-(2,5-diazabicyclo[2.2.2]octan-2-yl)-2-(2,6-dioxopiperidin-3-yl)-6-fluoroisoindoline-1,3-dione;
5-(2,6-dimethylpiperazin-1-yl)-2-(2,6-dioxopiperidin-3-yl)-6-fluoroisoindoline-1,3-dione;
5-(3,5-dimethylpiperazin-1-yl)-2-(2,6-dioxopiperidin-3-yl)-6-fluoroisoindoline-1,3-dione;
2-(2,6-dioxopiperidin-3-yl)-5-fluoro-6-(piperazin-1-yl-2,2,3,3,5,5,6,6-$d_8$)isoindoline-1,3-dione;
2-(2,6-dioxopiperidin-3-yl)-4-fluoro-5-(piperazin-1-yl-2,2,3,3,5,5,6,6-$d_8$)isoindoline-1,3-dione;
2-(2,6-dioxopiperidin-3-yl)-4-fluoro-6-(piperazin-1-yl-2,2,3,3,5,5,6,6-$d_8$)isoindoline-1,3-dione;
2-(2,6-dioxopipendin-3-yl)-4,7-difluoro-5-(piperazin-1-yl-2,2,3,3,5,5,6,6-$J_8$)isoindoline-1,3-dione;
2-(2,6-dioxopiperidin-3-yl)-4,6-difluoro-5-(piperazin-1-yl-2,2,3,3,5,5,6,6-$d_8$)isoindoline-1,3-dione;
2-(2,6-dioxopiperidin-3-yl)-4,5-difluoro-6-(piperazin-1-yl-2,2,3,3,5,5,6,6-$d_8$)isoindoline-1,3-dione;
2-(2,6-dioxopiperidin-3-yl)-4,5,7-trifluoro-6-(piperazin-1-yl-2,2,3,3,5,5,6,6-$d_8$)isoindoline-1,3-dione;
5-(3,6-diazabicyclo[3.1.1]heptan-6-yl)-2-(2,6-dioxopiperidin-3-yl)-4-fluoroisoindoline-1,3-dione;
5-(3,6-diazabicyclo[3.1.1]heptan-3-yl)-2-(2,6-dioxopiperidin-3-yl)-4-fluoroisoindoline-1,3-dione;
5-(3,8-diazabicyclo[3.2.1]octan-8-yl)-2-(2,6-dioxopiperidin-3-yl)-4-fluoroisoindoline-1,3-dione;
5-(3,8-diazabicyclo[3.2.1]octan-3-yl)-2-(2,6-dioxopiperidin-3-yl)-4-fluoroisoindoline-1,3-dione;
5-(2,5-diazabicyclo[2.2.1]heptan-2-yl)-2-(2,6-dioxopiperidin-3-yl)-4-fluoroisoindoline-1,3-dione;
5-(2,5-diazabicyclo[2.2.2]octan-2-yl)-2-(2,6-dioxopiperidin-3-yl)-4-fluoroisoindoline-1,3-dione;
5-(2,6-dimethylpiperazin-1-yl)-2-(2,6-dioxopiperidin-3-yl)-4-fluoroisoindoline-1,3-dione;
5-(3,5-dimethylpiperazin-1-yl)-2-(2,6-dioxopiperidin-3-yl)-4-fluoroisoindoline-1,3-dione;
6-(3,6-diazabicyclo[3.1.1]heptan-6-yl)-2-(2,6-dioxopiperidin-3-yl)-4-fluoroisoindoline-1,3-dione;
6-(3,6-diazabicyclo[3.1.1]heptan-6-yl)-2-(2,6-dioxopiperidin-3-yl)-4,5-difluoroisoindoline-1,3-dione;
5-(3,6-diazabicyclo[3.1.1]heptan-6-yl)-2-(2,6-dioxopiperidin-3-yl)-4,7-difluoroisoindoline-1,3-dione;
5-(3,6-diazabicyclo[3.1.1]heptan-6-yl)-2-(2,6-dioxopiperidin-3-yl)-4,6-difluoroisoindoline-1,3-dione;
5-(3,6-diazabicyclo[3.1.1]heptan-6-yl)-2-(2,6-dioxopiperidin-3-yl)-4,6,7-trifluoroisoindoline-1,3-dione;
6-(3,6-diazabicyclo[3.1.1]heptan-3-yl)-2-(2,6-dioxopiperidin-3-yl)-4-fluoroisoindoline-1,3-dione;
6-(3,6-diazabicyclo[3.1.1]heptan-3-yl)-2-(2,6-dioxopiperidin-3-yl)-4,5-difluoroisoindoline-1,3-dione;
5-(3,6-diazabicyclo[3.1.1]heptan-3-yl)-2-(2,6-dioxopiperidin-3-yl)-4,7-difluoroisoindoline-1,3-dione;
5-(3,6-diazabicyclo[3.1.1]heptan-3-yl)-2-(2,6-dioxopiperidin-3-yl)-4,6-difluoroisoindoline-1,3-dione;
5-(3,6-diazabicyclo[3.1.1]heptan-3-yl)-2-(2,6-dioxopiperidin-3-yl)-4,6,7-trifluoroisoindoline-1,3-dione;
6-(3,8-diazabicyclo[3.2.1]octan-8-yl)-2-(2,6-dioxopiperidin-3-yl)-4-fluorolsoindoline-1,3-dione;
6-(3,8-diazabicyclo[3.2.1]octan-8-yl)-2-(2,6-dioxopiperidin-3-yl)-4,5-difluoroisoindoline-1,3-dione;
5-(3,8-diazabicyclo[3.2.1]octan-8-yl)-2-(2,6-dioxopiperidin-3-yl)-4,7-difluoroisoindoline-1,3-dione;
5-(3,8-diazabicyclo[3.2.1]octan-8-yl)-2-(2,6-dioxopiperidin-3-yl)-4,6-difluoroisoindoline-1,3-dione;
5-(3,8-diazabicyclo[3.2.1]octan-8-yl)-2-(2,6-dioxopiperidin-3-yl)-4,6,7-trifluoroisoindoline-1,3-dione;
6-(3,8-diazabicyclo[3.2.1]octan-3-yl)-2-(2,6-dioxopiperidin-3-yl)-4-fluoroisoindoline-1,3-dione;
6-(3,8-diazabicyclo[3.2.1]octan-3-yl)-2-(2,6-dioxopiperidin-3-yl)-4,5-diflluoroisoindoline-1,3-dione;
5-(3,8-diazabicyclo[3.2.1]octan-3-yl)-2-(2,6-dioxopiperidin-3-yl)-4,7-difluoroisoindoline-1,3-dione;
5-(3,8-diazabicyclo[3.2.1]octan-3-yl)-2-(2,6-dioxopiperidin-3-yl)-4,6-difluoroisoindoline-1,3-dione;
5-(3,8-diazabicyclo[3.2.1]octan-3-yl)-2-(2,6-dioxopiperidin-3-yl)-4,6,7-trifluoroisoindoline-1,3-dione;
2-(2,6-dioxopiperidin-3-yl)-4,5,7-trifluoro-6-(piperazin-1-yl)isoindoline-1,3-dione;
6-(2,5-diazabicyclo[2.2.1]heptan-2-yl)-2-(2,6-dioxopiperidin-3-yl)-4-fluoroisoindoline-1,3-dione;
6-(2,5-diazabicyclo[2.2.1]heptan-2-yl)-2-(2,6-dioxopiperidin-3-yl)-4,5-difluoroisoindoline-1,3-dione;
5-(2,5-diazabicyclo[2.2.1]heptan-2-yl)-2-(2,6-dioxopiperidin-3-yl)-4,7-difluoroisoindoline-1,3-dione;
5-(2,5-diazabicyclo[2.2.1]heptan-2-yl)-2-(2,6-dioxopiperidin-3-yl)-4,6-difluoroisoindoline-1,3-dione;
5-(2,5-diazabicyclo[2.2.1]heptan-2-yl)-2-(2,6-dioxopiperidin-3-yl)-4,6,7-trifluoroisoindoline-1,3-dione;
6-(2,5-diazabicyclo[2.2.2]octan-2-yl)-2-(2,6-dioxopiperidin-3-yl)-4-fluoroisoindoline-1,3-dione;
5-(2,5-diazabicyclo[2.2.1]heptan-2-yl)-2-(2,6-dioxopiperidin-3-yl)-4,6-difluoroisoindoline-1,3-dione;
5-(2,5-diazabicyclo[2.2.2]octan-2-yl)-2-(2,6-dioxopiperidin-3-yl)-4,7-difluoroisoindoline-1,3-dione;
5-(2,5-diazabicyclo[2.2.2]octan-2-yl)-2-(2,6-dioxopiperidin-3-yl)-4,6-difluoroisoindoline-1,3-dione;
5-(2,5-diazabicyclo[2.2.2]octan-2-yl)-2-(2,6-dioxopiperidin-3-yl)-4,6,7-trifluoroisoindoline-1,3-dione;
6-(2,6-dimethylpiperazin-1-yl)-2-(2,6-dioxopiperidin-3-yl)-4-fluoroisoindoline-1,3-dione;
6-(2,6-dimethylpiperazin-1-yl)-2-(2,6-dioxopiperidin-3-yl)-4,5-difluoroisoindoline-1,3-dione;
5-(2,6-dimethylpiperazin-1-yl)-2-(2,6-dioxopiperidin-3-yl)-4,7-difluoroisoindoline-1,3-dione;
5-(2,6-dimethylpiperazin-1-yl)-2-(2,6-dioxopiperidin-3-yl)-4,6-difluoroisoindoline-1,3-dione;

5 -(2, 6-dimethylpip erazin-1-yl)-2-(2, 6-dioxopip eridin-3 -yl)-4, 6, 7-trifluoroi soindoline-1, 3 -dione;
6-(3,6-diazabicyclo[3.1.1]heptan-6-yl)-2-(2,6-dioxopiperidin-3-yl)-4-fluoroisoindoline-1,3-dione;
6-(3,6-diazabicyclo[3.1.1]heptan-3-yl)-2-(2,6-dioxopiperidin-3-yl)-4-fluoroisoindoline-1,3-dione;
6-(3,8-diazabicyclo[3.2.1]octan-8-yl)-2-(2,6-dioxopiperidin-3-yl)-4-fluoroisoindoline-1,3-dione;
6-(3,8-diazabicyclo[3.2.1]octan-3-yl)-2-(2,6-dioxopiperidin-3-yl)-4-fluoroisoindoline-1,3-dione;
6-(2,5-diazabicyclo[2.2.1]heptan-2-yl)-2-(2,6-dioxopiperidin-3-yl)-4-fluoroisoindoline-1,3-dione;
6-(2,5-diazabicyclo[2.2.2]octan-2-yl)-2-(2,6-dioxopiperidin-3-yl)-4-fluoroisoindoline-1,3-dione;
6-(2,6-dimethylpiperazin-1-yl)-2-(2,6-dioxopiperidin-3-yl)-4-fluoroisoindoline-1,3-dione;
6-(3,5-dimethylpiperazin-1-yl)-2-(2,6-dioxopiperidin-3-yl)-4-fluoroisoindoline-1,3-dione;
6-(3,5-dimethylpiperazin-1-yl)-2-(2,6-dioxopiperidin-3-yl)-4,5-difluoroisoindoline-1,3-dione;
5-(3,5-dimethylpiperazin-1-yl)-2-(2,6-dioxopiperidin-3-yl)-4,7-difluoroisoindoline-1,3-dione;
5-(3,5-dimethylpiperazin-1-yl)-2-(2,6-dioxopiperidin-3-yl)-4,6-difluoroisoindoline-1,3-dione;
5-(3,5-dimethylpiperazin-1-yl)-2-(2,6-dioxopiperidin-3-yl)-4,6,7-trifluoroisoindoline-1,3-dione;
3-(5-(3,6-diazabicyclo[3.1.1]heptan-6-yl)-1-oxoisoindolin-2-yl)piperidine-2,6-dione;
3-(5-(3,6-diazabicyclo[3.1.1]heptan-3-yl)-1-oxoisoindolin-2-yl)piperidine-2,6-dione;
3-(5-(3,8-diazabicyclo[3.2.1]octan-8-yl)-1-oxoisoindolin-2-yl)piperidine-2,6-dione;
3-(5-(3,8-diazabicyclo[3.2.1]octan-3-yl)-1-oxoisoindolin-2-yl)piperidine-2,6-dione;
3-(5-((1R,4R)-2,5-diazabicyclo[2.2.1]heptan-2-yl)-6-fluoro-1-oxoisoindolin-2-yl)piperidine-2,6-dione;
3-(5-((1S,4S)-2,5-diazabicyclo[2.2.1]heptan-2-yl)-6-fluoro-1-oxoisoindolin-2-yl)piperidine-2,6-dione;
3-(5-(2,5-diazabicyclo[2.2.2]octan-2-yl)-1-oxoisoindolin-2-yl)piperidine-2,6-dione;
3-(1-oxo-5-(piperazin-1-yl-2,2,3,3,5,5,6,6-$d_8$)isoindolin-2-yl)piperidine-2,6-dione;
3-(5-(3-azabicyclo[3.1.1]heptan-6-yl)-1-oxoisoindolin-2-yl)piperidine-2,6-dione;
3-(5-(6-azabicyclo[3.1.1]heptan-3-yl)-1-oxoisoindolin-2-yl)piperidine-2,6-dione;
3-(5-(3-azabicyclo[3.2.1]octan-8-yl)-1-oxoisoindolin-2-yl)piperidine-2,6-dione;
3-(5-(3,6-diazabicyclo[3.1.1]heptan-6-yl)-6-fluoro-1-oxoisoindolin-2-yl)piperidine-2,6-dione;
3-(5-(3,6-diazabicyclo[3.1.1]heptan-3-yl)-6-fluoro-1-oxoisoindolin-2-yl)piperidine-2,6-dione;
3-(5-(3,8-diazabicyclo[3.2.1]octan-8-yl)-6-fluoro-1-oxoisoindolin-2-yl)piperidine-2,6-dione;
3-(5-(3,8-diazabicyclo[3.2.1]octan-3-yl)-6-fluoro-1-oxoisoindolin-2-yl)piperidine-2,6-dione;
3-(5-(2,5-diazabicyclo[2.2.2]octan-2-yl)-6-fluoro-1-oxoisoindolin-2-yl)piperidine-2,6-dione;
3-(5-(2,6-dimethylpiperazin-1-yl)-6-fluoro-1-oxoisoindolin-2-yl)piperidine-2,6-dione;
3-(5-(3,5-dimethylpiperazin-1-yl)-6-fluoro-1-oxoisoindolin-2-yl)piperidine-2,6-dione;
3-(6-fluoro-1-oxo-5-(piperazin-1-yl-2,2,3,3,5,5,6,6-$d_8$)isoindolin-2-yl)plperldine-2,6-dione;
3-(5-(3,6-diazabicyclo[3.1.1]heptan-6-yl)-4-fluoro-1-oxoisoindolin-2-yl)piperidine-2,6-dione;
3-(5-(3,6-diazabicyclo[3.1.1]heptan-3-yl)-4-fluoro-1-oxoisoindolin-2-yl)piperidine-2,6-dione;
3-(5-(3,8-diazabicyclo[3.2.1]octan-8-yl)-4-fluoro-1-oxoisoindolin-2-yl)piperidine-2,6-dione;
3-(5-(3,8-diazabicyclo[3.2.1]octan-3-yl)-4-fluoro-1-oxoisoindolin-2-yl)piperidine-2,6-dione;
3-(5-(2,5-diazabicyclo[2.2.1]heptan-2-yl)-4-fluoro-1-oxoisoindolin-2-yl)piperidine-2,6-dione;
3-(5-(2,5-diazabicyclo[2.2.2]octan-2-yl)-4-fluoro-1-oxoisoindolin-2-yl)piperidine-2,6-dione;
3-(4-fluoro-1-oxo-5-(piperazin-1-yl-2,2,3,3,5,5,6,6-$d_8$)isoindolin-2-yl)piperidine-2,6-dione;
3-(5-(2,6-dimethylpiperazin-1-yl)-4-fluoro-1-oxoisoindolin-2-yl)piperidine-2,6-dione;
3-(5-(3,5-dimethylpiperazin-1-yl)-4-fluoro-1-oxoisoindolin-2-yl)piperidine-2,6-dione;
3-(5-(3,6-diazabicyclo[3.1.1]heptan-6-yl)-7-fluoro-1-oxoisoindolin-2-yl)piperidine-2,6-dione;
3-(5-(3,6-diazabicyclo[3.1.1]heptan-3-yl)-7-fluoro-1-oxoisoindolin-2-yl)piperidine-2,6-dione;
3-(5-(3,8-diazabicyclo[3.2.1]octan-8-yl)-7-fluoro-1-oxoisoindolin-2-yl)piperidine-2,6-dione;
3-(5-(3,8-diazabicyclo[3.2.1]octan-3-yl)-7-fluoro-1-oxoisoindolin-2-yl)piperidine-2,6-dione;
3-(5-(2,5-diazabicyclo[2.2.1]heptan-2-yl)-7-fluoro-1-oxoisoindolin-2-yl)piperidine-2,6-dione;
3-(5-(2,5-diazabicyclo[2.2.2]octan-2-yl)-7-fluoro-1-oxoisoindolin-2-yl)piperidine-2,6-dione;
3-(7-fluoro-1-oxo-5-(piperazin-1-yl-2,2,3,3,5,5,6,6-$d_8$)isoindolin-2-yl)piperidine-2,6-dione;
3-(5-(2,6-dimethylpiperazin-1-yl)-7-fluoro-1-oxoisoindolin-2-yl)piperidine-2,6-dione;
3-(5-(3,5-dimethylpiperazin-1-yl)-7-fluoro-1-oxoisoindolin-2-yl)piperidine-2,6-dione;
3-(6,7-difluoro-1-oxo-5-(piperazin-1-yl)isoindolin-2-yl)piperidine-2,6-dione;
3-(4,7-difluoro-1-oxo-5-(piperazin-1-yl)isoindolin-2-yl)piperidine-2,6-dione;
3-(4,6,7-trifluoro-1-oxo-5-(piperazin-1-yl)isoindolin-2-yl)piperidine-2,6-dione;
3-(5-(3,6-diazabicyclo[3.1.1]heptan-6-yl)-7-fluoro-1-oxoisoindolin-2-yl)piperidine-2,6-dione;
3-(5-(3,6-diazabicyclo[3.1.1]heptan-6-yl)-6,7-difluoro-1-oxoisoindolin-2-yl)piperidine-2,6-dione;
3-(5-(3,6-diazabicyclo[3.1.1]heptan-6-yl)-4,7-difluoro-1-oxoisoindolin-2-yl)piperidine-2,6-dione;
3-(5-(3,6-diazabicyclo[3.1.1]heptan-6-yl)-4,6-difluoro-1-oxoisoindolin-2-yl)piperidine-2,6-dione;
3-(5-(3,6-diazabicyclo[3.1.1]heptan-6-yl)-4,6,7-trifluoro-1-oxoisoindolin-2-yl)piperidine-2,6-dione;

3-(5-(3,6-diazabicyclo[3.1.1]heptan-3-yl)-7-fluoro-1-oxoisoindolin-2-yl)piperidine-2,6-dione;
3-(5-(3,6-diazabicyclo[3.1.1]heptan-3-yl)-6,7-difluoro-1-oxoisoindolin-2-yl)piperidine-2,6-dione;
3-(5-(3,6-diazabicyclo[3.1.1]heptan-3-yl)-4,7-difluoro-1-oxoisoindolin-2-yl)piperidine-2,6-dione;
3-(5-(3,6-diazabicyclo[3.1.1]heptan-3-yl)-4,6-difluoro-1-oxoisoindolin-2-yl)piperidine-2,6-dione;
3-(5-(3,6-diazabicyclo[3.1.1]heptan-3-yl)-4,6,7-trifluoro-1-oxoisoindolin-2-yl)piperidine-2,6-dione;
3-(5-(3,8-diazabicyclo[3.2.1]octan-8-yl)-7-fluoro-1-oxoisoindolin-2-yl)piperidine-2,6-dione;
3-(5-(3,8-diazabicyclo[3.2.1]octan-8-yl)-6,7-difluoro-1-oxoisoindolin-2-yl)piperidine-2,6-dione;
3-(5-(3,8-diazabicyclo[3.2.1]octan-8-yl)-4,7-difluoro-1-oxoisoindolin-2-yl)piperidine-2,6-dione;
3-(5-(3,8-diazabicyclo[3.2.1]octan-8-yl)-4,6-difluoro-1-oxoisoindolin-2-yl)piperidine-2,6-dione;
3-(5-(3,8-diazabicyclo[3.2.1]octan-8-yl)-4,6,7-trifluoro-1-oxoisoindolin-2-yl)piperidine-2,6-dione;
3-(5-(3,8-diazabicyclo[3.2.1]octan-3-yl)-7-fluoro-1-oxoisoindolin-2-yl)piperidine-2,6-dione;
3-(5-(3,8-diazabicyclo[3.2.1]octan-3-yl)-6,7-difluoro-1-oxoisoindolin-2-yl)piperidine-2,6-dione;
3-(5-(3,8-diazabicyclo[3.2.1]octan-3-yl)-4,7-difluoro-1-oxoisoindolin-2-yl)piperidine-2,6-dione;
3-(5-(3,8-diazabicyclo[3.2.1]octan-3-yl)-4,6-difluoro-1-oxoisoindolin-2-yl)piperidine-2,6-dione;
3-(5-(3,8-diazabicyclo[3.2.1]octan-3-yl)-4,6,7-trifluoro-1-oxoisoindolin-2-yl)piperidine-2,6-dione;
3-(5-(2,5-diazabicyclo[2.2.1]heptan-2-yl)-7-fluoro-1-oxoisoindolin-2-yl)piperidine-2,6-dione;
3-(5-(2,5-diazabicyclo[2.2.1]heptan-2-yl)-6,7-difluoro-1-oxoisoindolin-2-yl)piperidine-2,6-dione;
3-(5-(2,5-diazabicyclo[2.2.1]heptan-2-yl)-4,7-difluoro-1-oxoisoindolin-2-yl)piperidine-2,6-dione;
3-(5-(2,5-diazabicyclo[2.2.1]heptan-2-yl)-4,6-difluoro-1-oxoisoindolin-2-yl)piperidine-2,6-dione;
3-(5-(2,5-diazabicyclo[2.2.1]heptan-2-yl)-4,6,7-trifluoro-1-oxoisoindolin-2-yl)piperidine-2,6-dione;
3-(5-(2,5-diazabicyclo[2.2.2]octan-2-yl)-7-fluoro-1-oxoisoindolin-2-yl)piperidine-2,6-dione;
3-(5-(2,5-diazabicyclo[2.2.2]octan-2-yl)-6,7-difluoro-1-oxoisoindolin-2-yl)piperidine-2,6-dione;
3-(5-(2,5-diazabicyclo[2.2.2]octan-2-yl)-4,7-difluoro-1-oxoisoindolin-2-yl)piperidine-2,6-dione;
3-(5-(2,5-diazabicyclo[2.2.2]octan-2-yl)-4,6-difluoro-1-oxoisoindolin-2-yl)piperidine-2,6-dione;
3-(5-(2,5-diazabicyclo[2.2.2]octan-2-yl)-4,6,7-trifluoro-1-oxoisoindolin-2-yl)piperidine-2,6-dione;
3-(7-fluoro-1-oxo-5-(piperazin-1-yl-2,2,3,3,5,5,6,6-$d_8$)isoindolin-2-yl)piperidine-2,6-dione;
3-(6,7-difluoro-1-oxo-5-(piperazin-1-yl-2,2,3,3,5,5,6,6-$d_8$)isoindolin-2-yl)piperidine-2,6-dione;
3-(4,7-difluoro-1-oxo-5-(piperazin-1-yl-2,2,3,3,5,5,6,6-$d_8$)isoindolin-2-yl)piperidine-2,6-dione;
3-(4,6-difluoro-1-oxo-5-(piperazin-1-yl-2,2,3,3,5,5,6,6-$d_8$)isoindolin-2-yl)piperidine-2,6-dione;
3-(4,6,7-trifluoro-1-oxo-5-(piperazin-1-yl-2,2,3,3,5,5,6,6-$d_8$)isoindolin-2-yl)piperidine-2,6-dione;
3-(5-(2,6-dimethylpiperazin-1-yl)-6,7-difluoro-1-oxoisoindolin-2-yl)piperidine-2,6-dione;
3-(5-(2,6-dimethylpiperazin-1-yl)-4,6-difluoro-1-oxoisoindolin-2-yl)piperidine-2,6-dione;
3-(5-(2,6-dimethylpiperazin-1-yl)-4,7-difluoro-1-oxoisoindolin-2-yl)piperidine-2,6-dione;
3-(5-(2,6-dimethylpiperazin-1-yl)-4,6,7-trifluoro-1-oxoisoindolin-2-yl)piperidine-2,6-dione;
3-(5-(3,5-dimethylpiperazin-1-yl)-6,7-difluoro-1-oxoisoindolin-2-yl)piperidine-2,6-dione;
3-(5-(3,5-dimethylpiperazin-1-yl)-4,7-difluoro-1-oxoisoindolin-2-yl)piperidine-2,6-dione;
3-(5-(3,5-dimethylpiperazin-1-yl)-4,6-difluoro-1-oxoisoindolin-2-yl)piperidine-2,6-dione;
3-(5-(3,5-dimethylpiperazin-1-yl)-4,6,7-trifluoro-1-oxoisoindolin-2-yl)piperidine-2,6-dione;
4-(3,6-diazabicyclo[3.1.1]heptan-6-yl)-2-(2,6-dioxopiperidin-3-yl)isoindoline-1,3-dione;
4-(3,6-diazabicyclo[3.1.1]heptan-3-yl)-2-(2,6-dioxopiperidin-3-yl)isoindoline-1,3-dione;
4-((1R,5S)-3,8-diazabicyclo[3.2.1]octan-8-yl)-2-(2,6-dioxopiperidin-3-yl)isoindoline-1,3-dione;
4-(2,5-diazabicyclo[2.2.1]heptan-2-yl)-2-(2,6-dioxopiperidin-3-yl)isoindoline-1,3-dione;
4-(2,5-diazabicyclo[2.2.2]octan-2-yl)-2-(2,6-dioxopiperidin-3-yl)isoindoline-1,3-dione;
4-((1R,5S)-3,8-diazabicyclo[3.2.1]octan-3-yl)-2-(2,6-dioxopiperidin-3-yl)isoindoline-1,3-dione;
2-(2,6-dioxopiperidin-3-yl)-4-(piperazin-1-yl-2,2,3,3,5,5,6,6-$d_8$)isoindoline-1,3-dione;
2-(2,6-dioxopiperidin-3-yl)-5-fluoro-4-(piperazin-1-yl)isoindoline-1,3-dione;
2-(2,6-dioxopiperidin-3-yl)-4-fluoro-7-(piperazin-1-yl)isoindoline-1,3-dione;
2-(2,6-dioxopiperidin-3-yl)-4,5-difluoro-7-(piperazin-1-yl)isoindoline-1,3-dione;
2-(2,6-dioxopiperidin-3-yl)-5,6-difluoro-4-(piperazin-1-yl)isoindoline-1,3-dione;
2-(2,6-dioxopiperidin-3-yl)-5,7-difluoro-4-(piperazin-1-yl)isoindoline-1,3-dione;
2-(2,6-dioxopiperidin-3-yl)-4,5,6-trifluoro-7-(piperazin-1-yl)isoindoline-1,3-dione;
4-(3,6-diazabicyclo[3.1.1]heptan-6-yl)-2-(2,6-dioxopiperidin-3-yl)-5-fluoroisoindoline-1,3-dione;
4-(3,6-diazabicyclo[3.1.1]heptan-6-yl)-2-(2,6-dioxopiperidin-3-yl)-6-fluoroisoindoline-1,3-dione;
4-(3,6-diazabicyclo[3.1.1]heptan-6-yl)-2-(2,6-dioxopiperidin-3-yl)-7-fluoroisoindoline-1,3-dione;
7-(3,6-diazabicyclo[3.1.1]heptan-6-yl)-2-(2,6-dioxopiperidin-3-yl)-4,5-difluoroisoindoline-1,3-dione;
4-(3,6-diazabicyclo[3.1.1]heptan-6-yl)-2-(2,6-dioxopiperidin-3-yl)-5,6-difluoroisoindoline-1,3-dione;
4-(3,6-diazabicyclo[3.1.1]heptan-6-yl)-2-(2,6-dioxopiperidin-3-yl)-5,7-difluoroisoindoline-1,3-dione;
4-(3,6-diazabicyclo[3.1.1]heptan-6-yl)-2-(2,6-dioxopiperidin-3-yl)-5,6,7-trifluoroisoindoline-1,3-dione;

4-(3,6-diazabicyclo[3.1.1]heptan-3-yl)-2-(2,6-dioxopiperidin-3-yl)-5-fluoroisoindoline-1,3-dione;
4-(3,6-diazabicyclo[3.1.1]heptan-3-yl)-2-(2,6-dioxopiperidin-3-yl)-6-fluoroisoindoline-1,3-dione;
4-(3,6-diazabicyclo[3.1.1]heptan-3-yl)-2-(2,6-dioxopiperidin-3-yl)-7-fluoroisoindoline-1,3-dione;
7-(3,6-diazabicyclo[3.1.1]heptan-3-yl)-2-(2,6-dioxopiperidin-3-yl)-4,5-difluoroisoindoline-1,3-dione;
4-(3,6-diazabicyclo[3.1.1]heptan-3-yl)-2-(2,6-dioxopiperidin-3-yl)-5,6-difluoroisoindoline-1,3-dione;
4-(3,6-diazabicyclo[3.1.1]heptan-3-yl)-2-(2,6-dioxopiperidin-3-yl)-5,7-difluoroisoindoline-1,3-dione;
4-(3,6-diazabicyclo[3.1.1]heptan-3-yl)-2-(2,6-dioxopiperidin-3-yl)-5,6,7-trifluoroisoindoline-1,3-dione;
4-((1R,5S)-3,8-diazabicyclo[3.2.1]octan-8-yl)-2-(2,6-dioxopiperidin-3-yl)-5-fluoroisoindoline-1,3-dione;
4-((1R,5S)-3,8-diazabicyclo[3.2.1]octan-8-yl)-2-(2,6-dioxopiperidin-3-yl)-6-fluoroisoindoline-1,3-dione;
4-((1R,5S)-3,8-diazabicyclo[3.2.1]octan-8-yl)-2-(2,6-dioxopiperidin-3-yl)-7-fluoroisoindoline-1,3-dione;
7-((1R,5S)-3,8-diazabicyclo[3.2.1]octan-8-yl)-2-(2,6-dioxopiperidin-3-yl)-4,5-difluoroisoindoline-1,3-dione;
4-((1R,5S)-3,8-diazabicyclo[3.2.1]octan-8-yl)-2-(2,6-dioxopiperidin-3-yl)-5,6-difluoroisoindoline-1,3-dione;
4-((1R,5S)-3,8-diazabicyclo[3.2.1]octan-8-yl)-2-(2,6-dioxopiperidin-3-yl)-5,7-difluoroisoindoline-1,3-dione;
4-((1R,5S)-3,8-diazabicyclo[3.2.1]octan-8-yl)-2-(2,6-dioxopiperidin-3-yl)-5,6,7-trifluoroisoindoline-1,3 -dione;
4-((1R,5S)-3,8-diazabicyclo[3.2.1]octan-3-yl)-2-(2,6-dioxopiperidin-3-yl)-5-fluoroisoindoline-1,3-dione;
4-((1R,5S)-3,8-diazabicyclo[3.2.1]octan-3-yl)-2-(2,6-dioxopiperidin-3-yl)-6-fluoroisoindoline-1,3-dione;
4-((1R,5S)-3,8-diazabicyclo[3.2.1]octan-3-yl)-2-(2,6-dioxopiperidin-3-yl)-7-fluoroisoindoline-1,3-dione;
7-((1R,5S)-3,8-diazabicyclo[3.2.1]octan-3-yl)-2-(2,6-dioxopiperidin-3-yl)-4,5-difluoroisoindoline-1,3-dione;
4-((1R,5S)-3,8-diazabicyclo[3.2.1]octan-3-yl)-2-(2,6-dioxopiperidin-3-yl)-5,6-difluoroisoindoline-1,3-dione;
4-((1R,5S)-3,8-diazabicyclo[3.2.1]octan-3-yl)-2-(2,6-dioxopiperidin-3-yl)-5,7-difluoroisoindoline-1,3-dione;
4-((1R,5S)-3,8-diazabicyclo[3.2.1]octan-3-yl)-2-(2,6-dioxopiperidin-3-yl)-5,6,7-trifluoroisoindoline-1,3 -dione;
4-(2,5-diazabicyclo[2.2.1]heptan-2-yl)-2-(2,6-dioxopiperidin-3-yl)-5-fluoroisoindoline-1,3-dione;
4-(2,5-diazabicyclo[2.2.1]heptan-2-yl)-2-(2,6-dioxopiperidin-3-yl)-6-fluoroisoindoline-1,3-dione;
4-(2,5-diazabicyclo[2.2.1]heptan-2-yl)-2-(2,6-dioxopiperidin-3-yl)-7-fluoroisoindoline-1,3-dione;
7-(2,5-diazabicyclo[2.2.1]heptan-2-yl)-2-(2,6-dioxopiperidin-3-yl)-4,5-difluoroisoindoline-1,3-dione;
4-(2,5-diazabicyclo[2.2.1]heptan-2-yl)-2-(2,6-dioxopiperidin-3-yl)-5,6-difluoroisoindoline-1,3-dione;
4-(2,5-diazabicyclo[2.2.1]heptan-2-yl)-2-(2,6-dioxopiperidin-3-yl)-5,7-difluoroisoindoline-1,3-dione;
4-(2,5-diazabicyclo[2.2.1]heptan-2-yl)-2-(2,6-dioxopiperidin-3-yl)-5,6,7-trifluoroisoindoline-1,3-dione;
4-(2,5-diazabicyclo[2.2.2]octan-2-yl)-2-(2,6-dioxopiperidin-3-yl)-5-fluoroisoindoline-1,3-dione;
4-(2,5-diazabicyclo[2.2.2]octan-2-yl)-2-(2,6-dioxopiperidin-3-yl)-6-fluoroisoindoline-1,3-dione;
4-(2,5-diazabicyclo[2.2.2]octan-2-yl)-2-(2,6-dioxopiperidin-3-yl)-7-fluoroisoindoline-1,3-dione;
7-(2,5-diazabicyclo[2.2.2]octan-2-yl)-2-(2,6-dioxopiperidin-3-yl)-4,5-difluoroisoindoline-1,3-dione;
4-(2,5-diazabicyclo[2.2.2]octan-2-yl)-2-(2,6-dioxopiperidin-3-yl)-5,6-difluoroisoindoline-1,3-dione;
4-(2,5-diazabicyclo[2.2.2]octan-2-yl)-2-(2,6-dioxopiperidin-3-yl)-5,7-difluoroisoindoline-1,3-dione;
4-(2,5-diazabicyclo[2.2.2]octan-2-yl)-2-(2,6-dioxopiperidin-3-yl)-5,6,7-trifluoroisoindoline-1,3-dione;
4-(2,6-dimethylpiperazin-1-yl)-2-(2,6-dioxopiperidin-3-yl)-5-fluoroisoindoline-1,3-dione;
4-(2,6-dimethylpiperazin-1-yl)-2-(2,6-dioxopiperidin-3-yl)-6-fluoroisoindoline-1,3-dione;
4-(2,6-dimethylpiperazin-1-yl)-2-(2,6-dioxopiperidin-3-yl)-7-fluoroisoindoline-1,3-dione;
7-(2,6-dimethylpiperazin-1-yl)-2-(2,6-dioxopiperidin-3-yl)-4,5-difluoroisoindoline-1,3-dione;
4-(2,6-dimethylpiperazin-1-yl)-2-(2,6-dioxopiperidin-3-yl)-5,6-difluoroisoindoline-1,3-dione;
4-(2,6-dimethylpiperazin-1-yl)-2-(2,6-dioxopiperidin-3-yl)-5,7-difluoroisoindoline-1,3-dione;
4-(2,6-dimethylpiperazin-1-yl)-2-(2,6-dioxopiperidin-3-yl)-5,6,7-trifluoroisoindoline-1,3-dione;
2-(2,6-dioxopiperidin-3-yl)-5-fluoro-4-(piperazin-1-yl-2,2,3,3,5,5,6,6-$d_8$)isoindoline-1,3-dione;
2-(2,6-dioxopiperidin-3-yl)-6-fluoro-4-(piperazin-1-yl-2,2,3,3,5,5,6,6-$d_8$)isoindoline-1,3-dione;
2-(2,6-dioxopiperidin-3-yl)-4-fluoro-7-(piperazin-1-yl-2,2,3,3,5,5,6,6-$d_8$)isoindoline-1,3-dione;
2-(2,6-dioxopiperidin-3-yl)-4,5-difluoro-7-(piperazin-1-yl-2,2,3,3,5,5,6,6-$d_8$)isoindoline-1,3-dione;
2-(2,6-dioxopiperidin-3-yl)-5,7-difluoro-4-(piperazin-1-yl-2,2,3,3,5,5,6,6-$d_8$)isoindoline-1,3-dione;
2-(2,6-dioxopiperidin-3-yl)-5,6-difluoro-4-(piperazin-1-yl-2,2,3,3,5,5,6,6-$d_8$)isoindoline-1,3-dione;
2-(2,6-dioxopiperidin-3-yl)-4,5,6-trifluoro-7-(piperazin-1-yl-2,2,3,3,5,5,6,6-$d_8$)isoindoline-1,3-dione;
2-(2,6-dioxopiperidin-3-yl)-5-fluoro-4-(piperidin-4-yl)isoindoline-1,3-dione;
2-(2,6-dioxopiperidin-3-yl)-4-fluoro-7-(piperidin-4-yl)isoindoline-1,3-dione;
2-(2,6-dioxopiperidin-3-yl)-4,5-difluoro-7-(piperidin-4-yl)isoindoline-1,3-dione;
2-(2,6-dioxopiperidin-3-yl)-5,6-difluoro-4-(piperidin-4-yl)isoindoline-1,3-dione;
2-(2,6-dioxopiperidin-3-yl)-5,7-difluoro-4-(piperidin-4-yl)isoindoline-1,3-dione;
2-(2,6-dioxopiperidin-3-yl)-4,5,6-trifluoro-7-(piperidin-4-yl)isoindoline-1,3-dione;
4-(3-azabicyclo[3.1.1]heptan-6-yl)-2-(2,6-dioxopiperidin-3-yl)-5-fluoroisoindoline-1,3-dione;
4-(3-azabicyclo[3.1.1]heptan-6-yl)-2-(2,6-dioxopiperidin-3-yl)-6-fluoroisoindoline-1,3-dione;
4-(3-azabicyclo[3.1.1]heptan-6-yl)-2-(2,6-dioxopiperidin-3-yl)-7-fluoroisoindoline-1,3-dione;

7-(3-azabicyclo[3.1.1]heptan-6-yl)-2-(2,6-dioxopiperidin-3-yl)-4,5-difluoroisoindoline-1,3-dione;
4-(3-azabicyclo[3.1.1]heptan-6-yl)-2-(2,6-dioxopiperidin-3-yl)-5,6-difluoroisoindoline-1,3-dione;
4-(3-azabicyclo[3.1.1]heptan-6-yl)-2-(2,6-dioxopiperidin-3-yl)-5,7-difluoroisoindoline-1,3-dione;
4-(3-azabicyclo[3.1.1]heptan-6-yl)-2-(2,6-dioxopiperidin-3-yl)-5,6,7-trifluoroisoindoline-1,3-dione;
4-(6-azabicyclo[3.1.1]heptan-3-yl)-2-(2,6-dioxopiperidin-3-yl)-5-fluoroisoindoline-1,3-dione;
4-(6-azabicyclo[3.1.1]heptan-3-yl)-2-(2,6-dioxopiperidin-3-yl)-6-fluoroisoindoline-1,3-dione;
4-(6-azabicyclo[3.1.1]heptan-3-yl)-2-(2,6-dioxopiperidin-3-yl)-7-fluoroisoindoline-1,3-dione;
7-(6-azabicyclo[3.1.1]heptan-3-yl)-2-(2,6-dioxopiperidin-3-yl)-4,5-difluoroisoindoline-1,3-dione;
4-(6-azabicyclo[3.1.1]heptan-3-yl)-2-(2,6-dioxopiperidin-3-yl)-5,6-difluoroisoindoline-1,3-dione;
4-(6-azabicyclo[3.1.1]heptan-3-yl)-2-(2,6-dioxopiperidin-3-yl)-5,7-difluoroisoindoline-1,3-dione;
4-(6-azabicyclo[3.1.1]heptan-3-yl)-2-(2,6-dioxopiperidin-3-yl)-5,6,7-trifluoroisoindoline-1,3-dione;
4-((1R,5S,8r)-3-azabicyclo[3.2.1]octan-8-yl)-2-(2,6-dioxopiperidin-3-yl)-5-fluoroisoindoline-1,3-dione;
4-((1R,5S,8r)-3-azabicyclo[3.2.1]octan-8-yl)-2-(2,6-dioxopiperidin-3-yl)-6-fluoroisoindoline-1,3-dione;
4-((1R,5S,8r)-3-azabicyclo[3.2.1]octan-8-yl)-2-(2,6-dioxopiperidin-3-yl)-7-fluoroisoindoline-1,3-dione;
7-((1R,5S,8r)-3-azabicyclo[3.2.1]octan-8-yl)-2-(2,6-dioxopiperidin-3-yl)-4,5-difluoroisoindoline-1,3-dione;
4-((1R,5S,8r)-3-azabicyclo[3.2.1]octan-8-yl)-2-(2,6-dioxopiperidin-3-yl)-5,6-difluoroisoindoline-1,3-dione;
4-((1R,5S,8r)-3-azabicyclo[3.2.1]octan-8-yl)-2-(2,6-dioxopiperidin-3-yl)-5,7-difluoroisoindoline-1,3-dione;
4-((1R,5S,8r)-3-azabicyclo[3.2.1]octan-8-yl)-2-(2,6-dioxopiperidin-3-yl)-5,6,7-trifluoroisoindoline-1,3 -dione;
4-(3,5-dimethylpiperidin-4-yl)-2-(2,6-dioxopiperidin-3-yl)-5-fluoroisoindoline-1,3-dione;
4-(3,5-dimethylpiperidin-4-yl)-2-(2,6-dioxopiperidin-3-yl)-6-fluoroisoindoline-1,3-dione;
4-(3,5-dimethylpiperidin-4-yl)-2-(2,6-dioxopiperidin-3-yl)-7-fluoroisoindoline-1,3-dione;
7-(3,5-dimethylpiperidin-4-yl)-2-(2,6-dioxopiperidin-3-yl)-4,5-difluoroisoindoline-1,3-dione;
4-(3,5-dimethylpiperidin-4-yl)-2-(2,6-dioxopiperidin-3-yl)-5,6-difluoroisoindoline-1,3-dione;
4-(3,5-dimethylpiperidin-4-yl)-2-(2,6-dioxopiperidin-3-yl)-5,7-difluoroisoindoline-1,3-dione;
4-(3,5-dimethylpiperidin-4-yl)-2-(2,6-dioxopiperidin-3-yl)-5,6,7-trifluoroisoindoline-1,3-dione;
3-(4-(3,6-diazabicyclo[3.1.1]heptan-6-yl)-1-oxoisoindolin-2-yl)piperidine-2,6-dione;
3-(4-(3,6-diazabicyclo[3.1.1]heptan-3-yl)-1-oxoisoindolin-2-yl)piperidine-2,6-dione;
3-(4-((1R,5S)-3,8-diazabicyclo[3.2.1]octan-8-yl)-1-oxoisoindolin-2-yl)piperidine-2,6-dione;
3-(4-(2,5-diazabicyclo[2.2.1]heptan-2-yl)-1-oxoisoindolin-2-yl)piperidine-2,6-dione;
3-(4-(2,5-diazabicyclo[2.2.2]octan-2-yl)-1-oxoisoindolin-2-yl)piperidine-2,6-dione;
3-(4-((1R,5S)-3,8-diazabicyclo[3.2.1]octan-3-yl)-1-oxoisoindolin-2-yl)piperidine-2,6-dione;
3-(1-oxo-4-(piperazin-1-yl-2,2,3,3,5,5,6,6-$d_8$)isoindolin-2-yl)piperidine-2,6-dione;
3-(5-fluoro-1-oxo-4-(piperazin-1-yl)isoindolin-2-yl)piperidine-2,6-dione;
3-(7-fluoro-1-oxo-4-(piperazin-1-yl)isoindolin-2-yl)piperidine-2,6-dione;
3-(6,7-difluoro-1-oxo-4-(piperazin-1-yl)isoindolin-2-yl)piperidine-2,6-dione;
3-(5,6-difluoro-1-oxo-4-(piperazin-1-yl)isoindolin-2-yl)piperidine-2,6-dione;
3-(5,7-difluoro-1-oxo-4-(piperazin-1-yl)isoindolin-2-yl)piperidine-2,6-dione;
3-(5,6,7-trifluoro-1-oxo-4-(piperazin-1-yl)isoindolin-2-yl)piperidine-2,6-dione;
3-(4-(3,6-diazabicyclo[3.1.1]heptan-6-yl)-5-fluoro-1-oxoisoindolin-2-yl)piperidine-2,6-dione;
3-(4-(3,6-diazabicyclo[3.1.1]heptan-6-yl)-6-fluoro-1-oxoisoindolin-2-yl)piperidine-2,6-dione;
3-(4-(3,6-diazabicyclo[3.1.1]heptan-6-yl)-7-fluoro-1-oxoisoindolin-2-yl)piperidine-2,6-dione;
3-(4-(3,6-diazabicyclo[3.1.1]heptan-6-yl)-6,7-difluoro-1-oxoisoindolin-2-yl)piperidine-2,6-dione;
3-(4-(3,6-diazabicyclo[3.1.1]heptan-6-yl)-5,6-difluoro-1-oxoisoindolin-2-yl)piperidine-2,6-dione;
3-(4-(3,6-diazabicyclo[3.1.1]heptan-6-yl)-5,7-difluoro-1-oxoisoindolin-2-yl)piperidine-2,6-dione;
3-(4-(3,6-diazabicyclo[3.1.1]heptan-6-yl)-5,6,7-trifluoro-1-oxoisoindolin-2-yl)piperidine-2,6-dione;
3-(4-(3,6-diazabicyclo[3.1.1]heptan-3-yl)-5-fluoro-1-oxoisoindolin-2-yl)piperidine-2,6-dione;
3-(4-(3,6-diazabicyclo[3.1.1]heptan-3-yl)-6-fluoro-1-oxoisoindolin-2-yl)piperidine-2,6-dione;
3-(4-(3,6-diazabicyclo[3.1.1]heptan-3-yl)-7-fluoro-1-oxoisoindolin-2-yl)piperidine-2,6-dione;
3-(4-(3,6-diazabicyclo[3.1.1]heptan-3-yl)-6,7-difluoro-1-oxoisoindolin-2-yl)piperidine-2,6-dione;
3-(4-(3,6-diazabicyclo[3.1.1]heptan-3-yl)-5,6-difluoro-1-oxoisoindolin-2-yl)piperidine-2,6-dione;
3-(4-(3,6-diazabicyclo[3.1.1]heptan-3-yl)-5,7-difluoro-1-oxoisoindolin-2-yl)piperidine-2,6-dione;
3-(4-(3,6-diazabicyclo[3.1.1]heptan-3-yl)-5,6,7-trifluoro-1-oxoisoindolin-2-yl)piperidine-2,6-dione;
3-(4-((1R,5S)-3,8-diazabicyclo[3.2.1]octan-8-yl)-5-fluoro-1-oxoisoindolin-2-yl)piperidine-2,6-dione;
3-(4-((1R,5S)-3,8-diazabicyclo[3.2.1]octan-8-yl)-6-fluoro-1-oxoisoindolin-2-yl)piperidine-2,6-dione;
3-(4-((1R,5S)-3,8-diazabicyclo[3.2.1]octan-8-yl)-7-fluoro-1-oxoisoindolin-2-yl)piperidine-2,6-dione;
3-(4-((1R,5S)-3,8-diazabicyclo[3.2.1]octan-8-yl)-6,7-difluoro-1-oxoisoindolin-2-yl)piperidine-2,6-dione;
3-(4-((1R,5S)-3,8-diazabicyclo[3.2.1]octan-8-yl)-5,6-difluoro-1-oxoisoindolin-2-yl)piperidine-2,6-dione;
3-(4-((1R,5S)-3,8-diazabicyclo[3.2.1]octan-8-yl)-5,7-difluoro-1-oxoisoindolin-2-yl)piperidine-2,6-dione;

3-(4-((1R,5S)-3,8-diazabicyclo[3.2.1]octan-8-yl)-5,6,7-trifluoro-1-oxoisoindolin-2-yl)piperidine-2,6-dione;
3-(4-((1R,5S)-3,8-diazabicyclo[3.2.1]octan-3-yl)-5-fluoro-1-oxoisoindolin-2-yl)piperidine-2,6-dione;
3-(4-((1R,5S)-3,8-diazabicyclo[3.2.1]octan-3-yl)-6-fluoro-1-oxoisoindolin-2-yl)piperidine-2,6-dione;
3-(4-((1R,5S)-3,8-diazabicyclo[3.2.1]octan-3-yl)-7-fluoro-1-oxoisoindolin-2-yl)piperidine-2,6-dione;
3-(4-((1R,5S)-3,8-diazabicyclo[3.2.1]octan-3-yl)-6,7-difluoro-1-oxoisoindolin-2-yl)piperidine-2,6-dione;
3-(4-((1R,5S)-3,8-diazabicyclo[3.2.1]octan-3-yl)-5,6-difluoro-1-oxoisoindolin-2-yl)piperidine-2,6-dione;
3-(4-((1R,5S)-3,8-diazabicyclo[3.2.1]octan-3-yl)-5,7-difluoro-1-oxoisoindolin-2-yl)piperidine-2,6-dione;
3-(4-((1R,5S)-3,8-diazabicyclo[3.2.1]octan-3-yl)-5,6,7-trifluoro-1-oxoisoindolin-2-yl)piperidine-2,6-dione;
3-(4-(2,5-diazabicyclo[2.2.1]heptan-2-yl)-5-fluoro-1-oxoisoindolin-2-yl)piperidine-2,6-dione;
3-(4-(2,5-diazabicyclo[2.2.1]heptan-2-yl)-6-fluoro-1-oxoisoindolin-2-yl)piperidine-2,6-dione;
3-(4-(2,5-diazabicyclo[2.2.1]heptan-2-yl)-7-fluoro-1-oxoisoindolin-2-yl)piperidine-2,6-dione;
3-(4-(2,5-diazabicyclo[2.2.1]heptan-2-yl)-6,7-difluoro-1-oxoisoindolin-2-yl)piperidine-2,6-dione;
3-(4-(2,5-diazabicyclo[2.2.1]heptan-2-yl)-5,6-difluoro-1-oxoisoindolin-2-yl)piperidine-2,6-dione;
3-(4-(2,5-diazabicyclo[2.2.1]heptan-2-yl)-5,7-difluoro-1-oxoisoindolin-2-yl)piperidine-2,6-dione;
3-(4-(2,5-diazabicyclo[2.2.1]heptan-2-yl)-5,6,7-trifluoro-1-oxoisoindolin-2-yl)piperidine-2,6-dione;
3-(4-(2,5-diazabicyclo[2.2.2]octan-2-yl)-5-fluoro-1-oxoisoindolin-2-yl)piperidine-2,6-dione;
3-(4-(2,5-diazabicyclo[2.2.2]octan-2-yl)-6-fluoro-1-oxoisoindolin-2-yl)piperidine-2,6-dione;
3-(4-(2,5-diazabicyclo[2.2.2]octan-2-yl)-7-fluoro-1-oxoisoindolin-2-yl)piperidine-2,6-dione;
3-(4-(2,5-diazabicyclo[2.2.2]octan-2-yl)-6,7-difluoro-1-oxoisoindolin-2-yl)piperidine-2,6-dione;
3-(4-(2,5-diazabicyclo[2.2.2]octan-2-yl)-5,6-difluoro-1-oxoisoindolin-2-yl)piperidine-2,6-dione;
3-(4-(2,5-diazabicyclo[2.2.2]octan-2-yl)-5,7-difluoro-1-oxoisoindolin-2-yl)piperidine-2,6-dione;
3-(4-(2,5-diazabicyclo[2.2.2]octan-2-yl)-5,6,7-trifluoro-1-oxoisoindolin-2-yl)piperidine-2,6-dione;
3-(4-(2,6-dimethylpiperazin-1-yl)-5-fluoro-1-oxoisoindolin-2-yl)piperidine-2,6-dione;
3-(4-(2,6-dimethylpiperazin-1-yl)-6-fluoro-1-oxoisoindolin-2-yl)piperidine-2,6-dione;
3-(4-(2,6-dimethylpiperazin-1-yl)-7-fluoro-1-oxoisoindolin-2-yl)piperidine-2,6-dione;
3-(4-(2,6-dimethylpiperazin-1-yl)-6,7-difluoro-1-oxoisoindolin-2-yl)piperidine-2,6-dione;
3-(4-(2,6-dimethylpiperazin-1-yl)-5,6-difluoro-1-oxoisoindolin-2-yl)piperidine-2,6-dione;
3-(4-(2,6-dimethylpiperazin-1-yl)-5,7-difluoro-1-oxoisoindolin-2-yl)piperidine-2,6-dione;
3-(4-(2,6-dimethylpiperazin-1-yl)-5,6,7-trifluoro-1-oxoisoindolin-2-yl)piperidine-2,6-dione;
3-(5-fluoro-1-oxo-4-(piperazin-1-yl-2,2,3,3,5,5,6,6-$d_8$)isoindolin-2-yl)piperidine-2,6-dione;
3-(6-fluoro-1-oxo-4-(piperazin-1-yl-2,2,3,3,5,5,6,6-$d_8$)isoindolin-2-yl)piperidine-2,6-dione;
3-(7-fluoro-1-oxo-4-(piperazin-1-yl-2,2,3,3,5,5,6,6-$d_8$)isoindolin-2-yl)piperidine-2,6-dione;
3-(6,7-difluoro-1-oxo-4-(piperazin-1-yl-2,2,3,3,5,5,6,6-$d_8$)isoindolin-2-yl)piperidine-2,6-dione;
3-(5,7-difluoro-1-oxo-4-(piperazin-1-yl-2,2,3,3,5,5,6,6-$d_8$)isoindolin-2-yl)piperidine-2,6-dione;
3-(5,6-difluoro-1-oxo-4-(piperazm-1-yl-2,2,3,3,5,5,6,6-$d_8$)isoindolin-2-yl)piperidine-2,6-dione;
3-(5,6,7-trifluoro-1-oxo-4-(piperazin-1-yl-2,2,3,3,5,5,6,6-$d_8$)isoindolin-2-yl)piperidine-2,6-dione;
3-(5-fluoro-1-oxo-4-(piperidin-4-yl)isoindolin-2-yl)piperidine-2,6-dione;
3-(7-fluoro-1-oxo-4-(piperidin-4-yl)isoindolin-2-yl)piperidine-2,6-dione;
3-(6,7-difluoro-1-oxo-4-(piperidin-4-yl)isoindolin-2-yl)piperidine-2,6-dione;
3-(5,6-difluoro-1-oxo-4-(piperidin-4-yl)isoindolin-2-yl)piperidine-2,6-dione;
3-(5,7-difluoro-1-oxo-4-(piperidin-4-yl)isoindolin-2-yl)piperidine-2,6-dione;
3-(5,6,7-trifluoro-1-oxo-4-(piperidin-4-yl)isoindolin-2-yl)piperidine-2,6-dione;
3-(4-(3-azabicyclo[3.1.1]heptan-6-yl)-5-fluoro-1-oxoisoindolin-2-yl)piperidine-2,6-dione;
3-(4-(3-azabicyclo[3.1.1]heptan-6-yl)-6-fluoro-1-oxoisoindolin-2-yl)piperidine-2,6-dione;
3-(4-(3-azabicyclo[3.1.1]heptan-6-yl)-7-fluoro-1-oxoisoindolin-2-yl)piperidine-2,6-dione;
3-(4-(3-azabicyclo[3.1.1]heptan-6-yl)-6,7-difluoro-1-oxoisoindolin-2-yl)piperidine-2,6-dione;
3-(4-(3-azabicyclo[3.1.1]heptan-6-yl)-5,6-difluoro-1-oxoisoindolin-2-yl)piperidine-2,6-dione;
3-(4-(3-azabicyclo[3.1.1]heptan-6-yl)-5,7-difluoro-1-oxoisoindolin-2-yl)piperidine-2,6-dione;
3-(4-(3-azabicyclo[3.1.1]heptan-6-yl)-5,6,7-trifluoro-1-oxoisoindolin-2-yl)piperidine-2,6-dione;
3-(4-(6-azabicyclo[3.1.1]heptan-3-yl)-5-fluoro-1-oxoisoindolin-2-yl)piperidine-2,6-dione;
3-(4-(6-azabicyclo[3.1.1]heptan-3-yl)-6-fluoro-1-oxoisoindolin-2-yl)piperidine-2,6-dione;
3-(4-(6-azabicyclo[3.1.1]heptan-3-yl)-7-fluoro-1-oxoisoindolin-2-yl)piperidine-2,6-dione;
3-(4-(6-azabicyclo[3.1.1]heptan-3-yl)-6,7-difluoro-1-oxoisoindolin-2-yl)piperidine-2,6-dione;
3-(4-(6-azabicyclo[3.1.1]heptan-3-yl)-5,6-difluoro-1-oxoisoindolin-2-yl)piperidine-2,6-dione;
3-(4-(6-azabicyclo[3.1.1]heptan-3-yl)-5,7-difluoro-1-oxoisoindolin-2-yl)piperidine-2,6-dione;
3-(4-(6-azabicyclo[3.1.1]heptan-3-yl)-5,6,7-trifluoro-1-oxoisoindolin-2-yl)piperidine-2,6-dione;
3-(4-((1R,5S,8r)-3-azabicyclo[3.2.1]octan-8-yl)-5-fluoro-1-oxoisoindolin-2-yl)piperidine-2,6-dione;
3-(4-((1R,5S,8r)-3-azabicyclo[3.2.1]octan-8-yl)-6-fluoro-1-oxoisoindolin-2-yl)piperidine-2,6-dione;

3-(4-((1R,5S,8r)-3-azabicyclo[3.2.1]octan-8-yl)-7-fluoro-1-oxoisoindolin-2-yl)piperidine-2,6-dione;
3-(4-((1R,5S,8r)-3-azabicyclo[3.2.1]octan-8-yl)-6,7-difluoro-1-oxoisoindolin-2-yl)piperidine-2,6-dione;
3-(4-((1R,5S,8r)-3-azabicyclo[3.2.1]octan-8-yl)-5,6-difluoro-1-oxoisoindolin-2-yl)piperidine-2,6-dione;
3-(4-((1R,5S,8r)-3-azabicyclo[3.2.1]octan-8-yl)-5,7-difluoro-1-oxoisoindolin-2-yl)piperidine-2,6-dione;
3-(4-((1R,5S,8r)-3-azabicyclo[3.2.1]octan-8-yl)-5,6,7-trifluoro-1-oxoisoindolin-2-yl)piperidine-2,6-dione;
3-(4-(3,5-dimethylpiperidin-4-yl)-5-fluoro-1-oxoisoindolin-2-yl)piperidine-2,6-dione;
3-(4-(3,5-dimethylpiperidin-4-yl)-6-fluoro-1-oxoisoindolin-2-yl)piperidine-2,6-dione;
3-(4-(3,5-dimethylpiperidin-4-yl)-7-fluoro-1-oxoisoindolin-2-yl)piperidine-2,6-dione;
3-(4-(3,5-dimethylpiperidin-4-yl)-6,7-difluoro-1-oxoisoindolin-2-yl)piperidine-2,6-dione;
3-(4-(3,5-dimethylpiperidin-4-yl)-5,6-difluoro-1-oxoisoindolin-2-yl)piperidine-2,6-dione;
3-(4-(3,5-dimethylpiperidin-4-yl)-5,7-difluoro-1-oxoisoindolin-2-yl)piperidine-2,6-dione; and
3-(4-(3,5-dimethylpiperidin-4-yl)-5,6,7-trifluoro-1-oxoisoindolin-2-yl)piperidine-2,6-dione.

**9.** A compound of Formula (II)

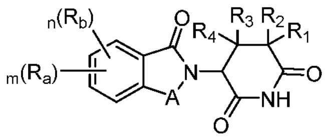

Formula (II)

or salts, enantiomers, stereoisomers, solvates, isotopically enriched analogs, prodrugs, or polymorphs thereof,

wherein A represents CO, $CH_2$ or $CD_2$;
$R_1$, $R_2$, $R_3$ and $R_4$ are the same or different and each independently represent hydrogen, deuterium, halogen, optionally deuterated $C_{1-6}$ alkyl, optionally deuterated $C_{1-6}$ alkoxy, or halogenated $C_{1-6}$ alkyl;
$(R_b)_n$ indicates that benzene ring is substituted with n $R_b$ substituents, and each $R_b$ are the same or different and each independently represent hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl or $C_{2-6}$ alkynyl;
$(R_a)_m$ indicates that the benzene ring is substituted with m $R_a$ substituents, and each $R_a$ are the same or different and each independently represents the structure of the following formula:

$$W\text{-}LIN\text{-}U_2\text{-}R_c\text{-}U_1\text{-} ,$$

wherein

$U_1$ and $U_2$ are the same or different and each independently represent a bond, $N(R_d)$ or O, wherein $R_d$ represents H or optionally deuterated $C_{1-6}$ alkyl;
$R_c$ represents optionally substituted nitrogen-containing monocyclic heterocyclylene or optionally substituted nitrogen-containing bridged heterocyclylene;
W represents hydrogen, optionally substituted nitrogen-containing monocyclic heterocyclyl, optionally substituted nitrogen-containing bridged heterocyclyl, optionally substituted cycloalkyl, optionally substituted heteroaryl, optionally substituted aryl, $C_{2-6}$ alkenyl, $C_{2-6}$ alkynyl, a leaving group, CHO, COOH, $NH_2$, hydroxy or mercapto; and
wherein in case that the compound of Formula (II) is partially or fully deuterated:

when W represents optionally substituted nitrogen-containing monocyclic heterocyclyl, optionally substituted nitrogen-containing bridged heterocyclyl, optionally substituted cycloalkyl, optionally substituted heteroaryl or optionally substituted aryl, LIN represents a bond, optionally substituted methylene, or optionally substituted linear or branched $C_{2-30}$ alkylene, wherein one or more groups $R_e$ and/or one or more groups Rε and/or any combination of one or more groups $R_e$ and $R_f$ are optionally inserted into the backbone carbon chain of the linear or branched $C_{2-30}$ alkylene group, wherein carbon-carbon bond between one or more pairs of adjacent carbon atoms in the backbone carbon chain is interrupted by the group $R_e$, $R_f$, or a combination of $R_e$ and $R_f$; wherein each $R_e$ are selected from the group consisting of O, $N(R_g)$, C(O), C(O)O, OC(O), S(O), $S(O)_2$, $S(O)_2NH$,

$NHS(O)_2$, $C(O)N(R_g)$, $N(R_g)C(O)$, or $N(R_g)C(O)N(R_g)$, where each $R_g$ independently represent H or $C_{1-6}$ alkyl, and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof; or

when W represents hydrogen, $C_{2-6}$ alkenyl, $C_{2-6}$ alkynyl, a leaving group, CHO, COOH, $NH_2$, hydroxy or mercapto, LIN represents optionally substituted methylene, or optionally substituted linear or branched $C_{2-30}$ alkylene, wherein one or more groups $R_e$ and/or one or more groups $R_f$ and/or any combination of one or more groups $R_e$ and $R_f$ are optionally inserted into the backbone carbon chain of the linear or branched $C_{2-30}$ alkylene group, wherein carbon-carbon bond between one or more pairs of adjacent carbon atoms in the backbone carbon chain is interrupted by the group $R_e$, $R_f$, or a combination of $R_e$ and $R_f$;

wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl, and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof; or

in case that the compound of Formula (II) is not deuterated, LIN represents optionally substituted methylene, and W represents optionally substituted nitrogen-containing monocyclic heterocyclyl or optionally substituted nitrogen-containing bridged heterocyclyl; and

m represents an integer of 1, 2, 3 or 4, n represents an integer of 0, 1, 2 or 3, and the sum of m and n is an integer of 1, 2, 3 or 4;

with the proviso that when the compound of Formula (II) is not deuterated, the following compounds are excluded:

3-(4,6-difluoro-1-oxo-5-(4-(piperidin-4-ylmethyl)piperazin-1-yl)isoindolin-2-yl)piperidine-2,6-dione;
2-(2,6-dioxopiperidin-3-yl)-5-(4-(piperidin-4-ylmethyl)piperazin-1-yl)isoindoline-1,3-dione;
3-(4-chloro-1-oxo-5-(4-(piperidin-4-ylmethyl)piperidin-1-yl)isoindolin-2-yl)piperidine-2,6-dione;
5-(4-(azetidin-3-ylmethyl)piperazin-1-yl)-2-(2,6-dioxopiperidin-3-yl)isoindoline-1,3-dione; and
2-(2,6-dioxopiperidin-3-yl)-5-(3-(piperazin-1-ylmethyl)azetidin-1-yl)isoindoline-1,3-dione.

**10.** The compound of Formula (II) or salts, enantiomers, stereoisomers, solvates, isotopically enriched analogs, prodrugs, or polymorphs thereof as claimed in claim 9, wherein the compound of Formula (II) is also of Formula (II-1) or Formula (II-2):

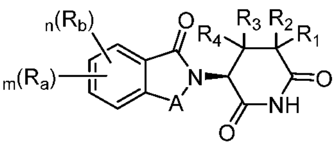

Formula (II)-1

or

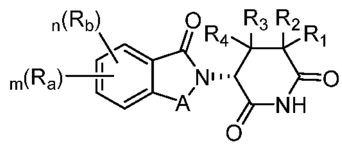

Formula (II)-2

wherein A, $R_1$, $R_2$, $R_3$, $R_4$, $R_a$, $R_b$, m, and n are as defined in claim 9.

**11.** The compound of Formula (II) or salts, enantiomers, stereoisomers, solvates, isotopically enriched analogs, prodrugs, or polymorphs thereof as claimed in claim 9 or 10, wherein

when the compound of Formula (II) is partially deuterated (for example, carrying 1 to 20 deuterium atoms) or fully deuterated, and W represents optionally substituted nitrogen-containing monocyclic heterocyclyl, optionally substituted nitrogen-containing bridged heterocyclyl, optionally substituted cycloalkyl, optionally substituted heteroaryl or optionally substituted aryl, LIN represents a bond, optionally substituted methylene, or optionally substituted linear or branched $C_{2-30}$ alkylene, wherein one or more groups $R_e$ and/or one or more groups Rε and/or any combination of one or more groups $R_e$ and $R_f$ are optionally inserted into the backbone carbon chain of the linear or branched $C_{2-30}$ alkylene group, wherein carbon-carbon bond between one or more pairs of adjacent carbon atoms in the backbone carbon chain is interrupted by the group $R_e$, $R_f$, or a combination of $R_e$ and Rε; each $R_e$ are selected from the group consisting of O, $N(R_g)$, C(O), C(O)O, S(O), $S(O)_2$, $S(O)_2NH$, $NHS(O)_2$, OC(O), $C(O)N(R_g)$, $N(R_g)C(O)$, or $N(R_g)C(O)N(R_g)$, where each $R_g$ independently represent H or $C_{1-6}$ alkyl, and in case that two or more groups $R_e$ are inserted into the backbone carbon chain of the linear or branched $C_{2-30}$ alkylene group, the two or more groups $R_e$ are not directly connected to each other; and each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl, optionally deuterated $C_{3-6}$ cycloalkyl, hydroxyl, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof; or

when the compound of Formula (II) is partially deuterated (for example, carrying 1 to 20 deuterium atom) or fully deuterated, and W represents hydrogen, $C_{2-6}$ alkenyl, $C_{2-6}$ alkynyl, a leaving group, CHO, COOH, $NH_2$, hydroxy or mercapto, LIN represents optionally substituted methylene or optionally substituted linear or branched $C_{2-30}$ alkylene, wherein one or more groups $R_e$ and/or one or more groups $R_f$ and/or any combination of one or more groups $R_e$ and $R_f$ are optionally inserted into the backbone carbon chain of the linear or branched $C_{2-30}$ alkylene group, wherein carbon-carbon bond between one or more pairs of adjacent carbon atoms in the backbone carbon chain is interrupted by the group $R_e$, $R_f$, or a combination of $R_e$ and $R_f$; each $R_e$ are selected from the group consisting of O, $N(R_g)$, C(O), C(O)O, S(O), $S(O)_2$, $S(O)_2NH$, $NHS(O)_2$, OC(O), $C(O)N(R_g)$, $N(R_g)C(O)$, or $N(R_g)C(O)N(R_g)$, where each $R_g$ independently represent H or $C_{1-6}$ alkyl, and in case that two or more groups $R_e$ are inserted into the backbone carbon chain of the linear or branched $C_{2-30}$ alkylene group, the two or more groups $R_e$ are not directly connected to each other; and each Rε are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl, optionally deuterated $C_{3-6}$ cycloalkyl, hydroxyl, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof; or

when the compound of Formula (II) is not deuterated, LIN represents optionally substituted methylene, and W represents optionally substituted nitrogen-containing monocyclic heterocyclyl or optionally substituted nitrogen-containing bridged heterocyclyl;

wherein the methylene and the linear or branched $C_{2-30}$ alkylene are optionally substituted with one or more substituents selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

**12.** The compound of Formula (II) or salts, enantiomers, stereoisomers, solvates, isotopically enriched analogs, prodrugs, or polymorphs thereof as claimed in claim 9 or 10, wherein $R_c$ represents the following groups:

monocyclic $C_{3-10}$ heterocyclylene group containing one nitrogen atom and optionally a heteroatom selected from nitrogen, oxygen, and sulfur, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene or diazacyclooctylene; or

$C_{5-20}$ bridged heterocyclylene group containing one nitrogen atom and optionally a heteroatom selected from

nitrogen, oxygen, and sulfur, e.g., azabicyclo[3.1. 1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene or diazabicyclo[2.2.2]octanylene, such as the following groups:

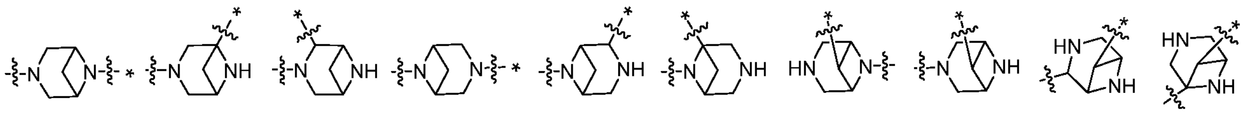

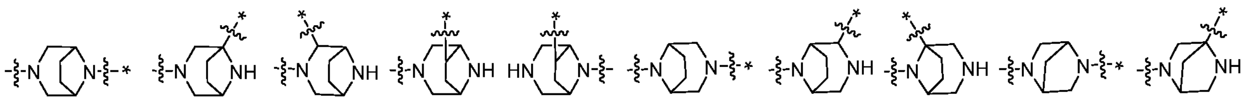

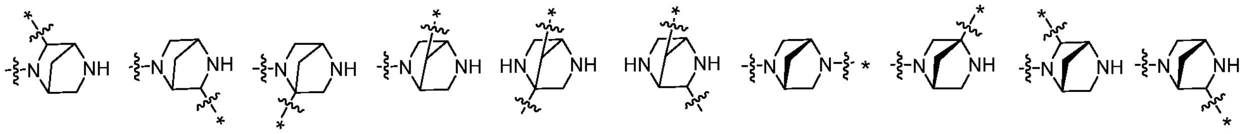

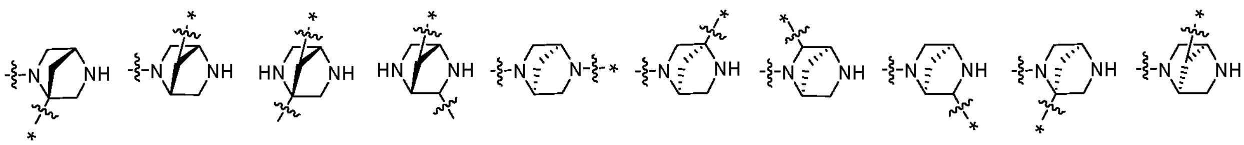

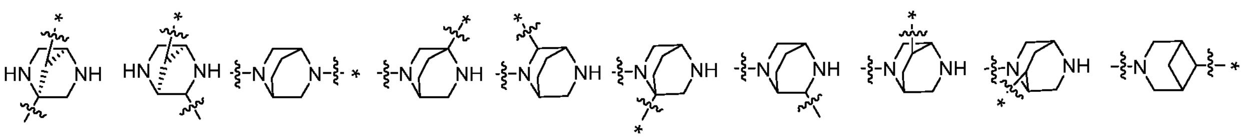

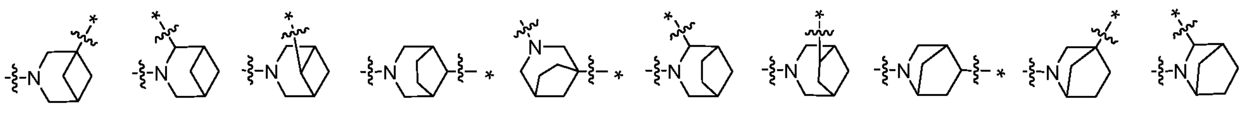

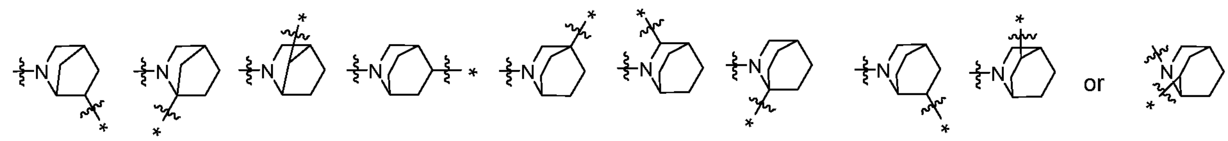
or

wherein symbol * indicates the point of attachment to $U_1$,
wherein the monocyclic $C_{3-10}$ heterocyclylene and the $C_{5-20}$ bridged heterocyclylene group are each independently optionally substituted with one or more substituents selected from the group consisting of deuterium, hydroxy, amino, mercapto, nitro, halogen, cyano, CHO, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

**13.** The compound of Formula (II) or salts, enantiomers, stereoisomers, solvates, isotopically enriched analogs, prodrugs, or polymorphs thereof as claimed in claim 9 or 10, wherein $R_c$ represents the following groups:

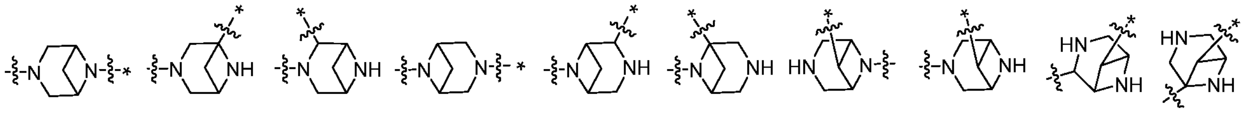

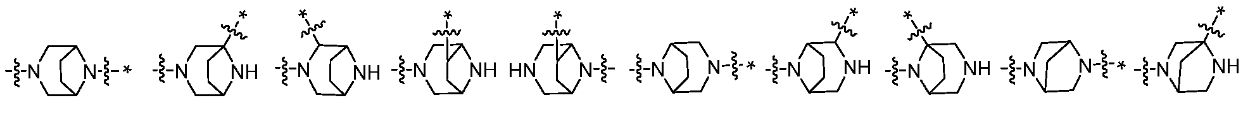

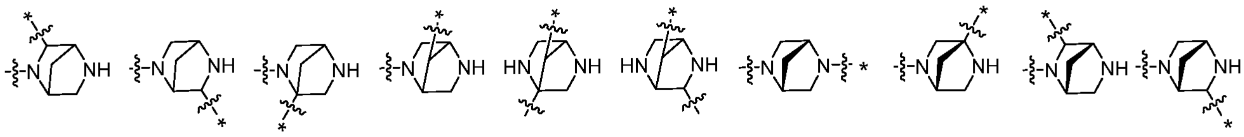

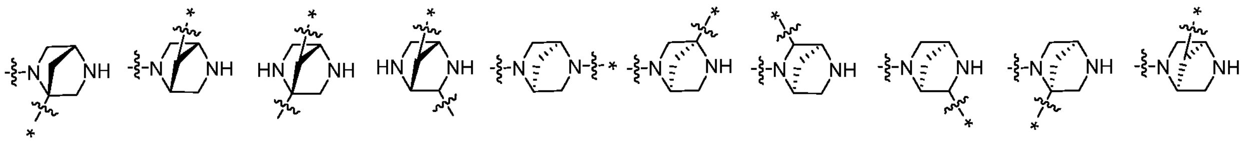

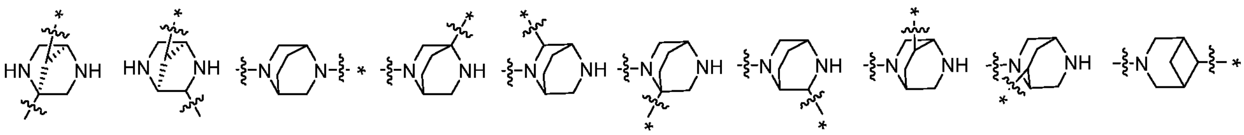

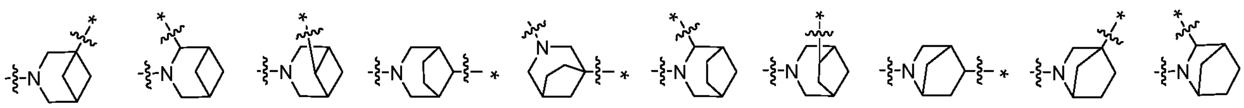

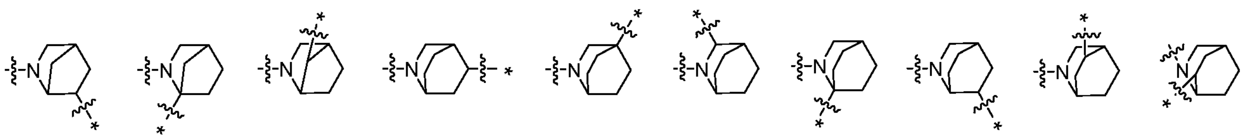

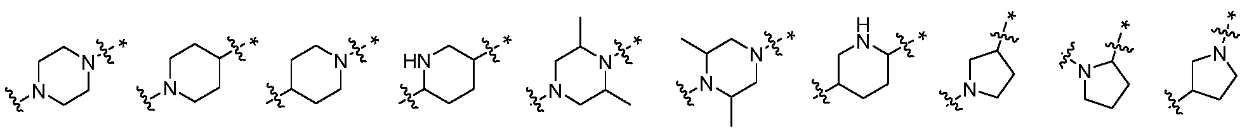

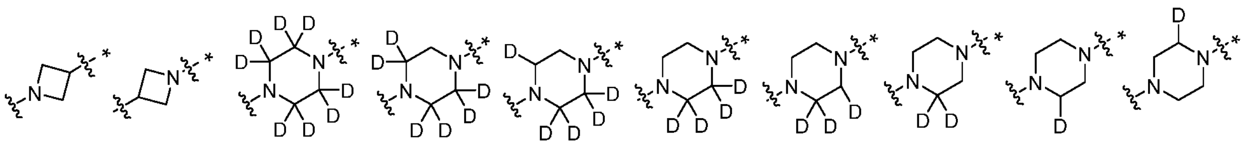

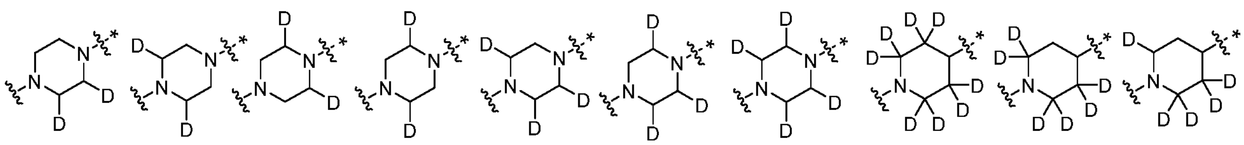

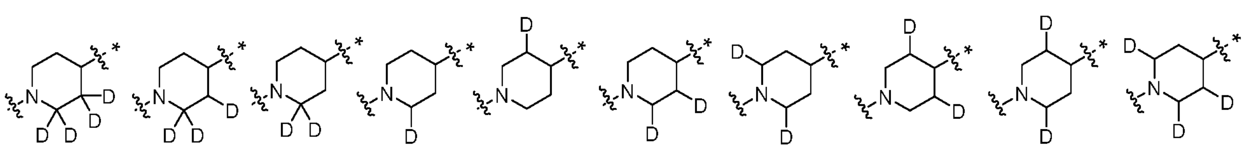

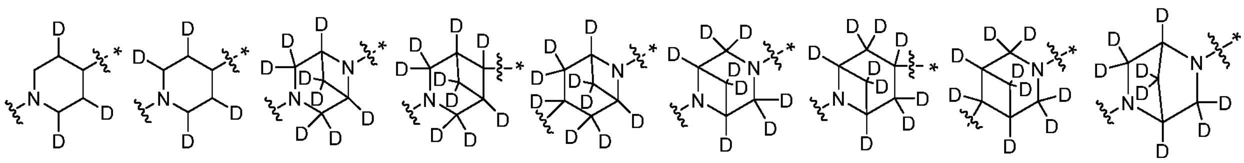

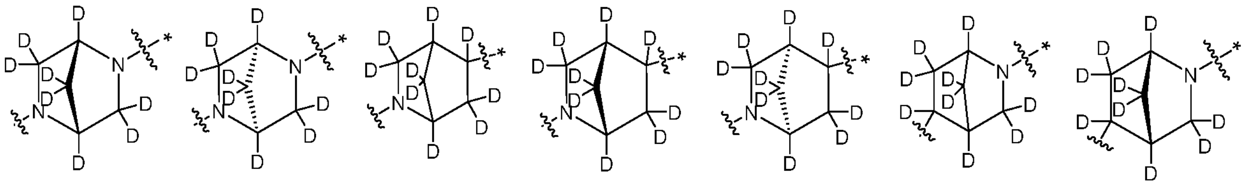

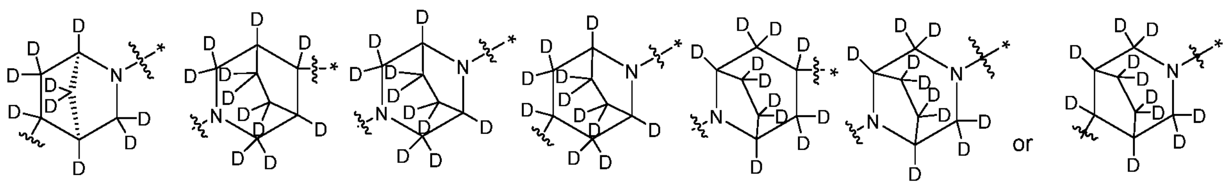

;

wherein symbol * indicates the point of attachment to $U_1$.

**14.** The compound of Formula (II) or salts, enantiomers, stereoisomers, solvates, isotopically enriched analogs, prodrugs, or polymorphs thereof as claimed in claim 9 or 10, wherein W represents the following groups:

monocyclic $C_{3-10}$ heterocyclyl group containing one nitrogen atom and optionally a heteroatom selected from nitrogen, oxygen, and sulfur, e.g., piperidinyl, piperazinyl, morpholinyl, azetidinyl, pyrrolidinyl, imidazolidyl, pyrazolidyl, oxazolidinyl, thiazolidinyl, thiomorpholinyl, azepanyl, diazacycloheptanyl, azacyclooctyl or diazacyclooctyl; or

$C_{5-20}$ bridged heterocyclyl group containing one nitrogen atom and optionally a heteroatom selected from nitrogen, oxygen, and sulfur, e.g., azabicyclo[3.1. 1]heptanyl, azabicyclo[2.2.1]heptanyl, azabicyclo[3.2. 1]octanyl, azabicyclo[2.2.2]octanyl, diazabicyclo[3.1.1]heptanyl, diazabicyclo[2.2.1]heptanyl, diazabicyclo[3.2.1]octanyl or diazabicyclo[2.2.2]octanyl, such as the following groups:

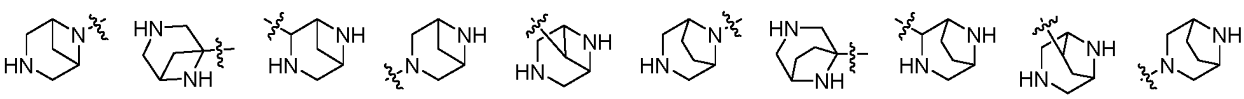

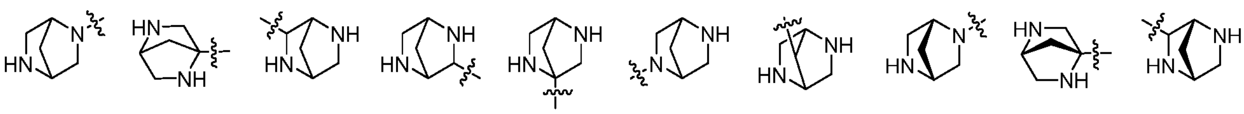

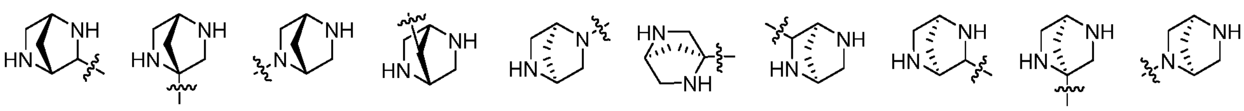

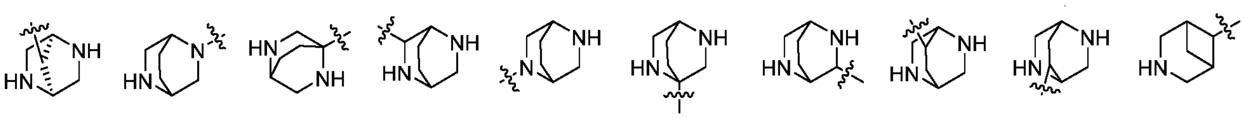

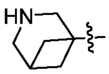 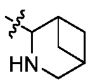 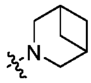 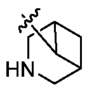 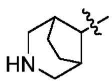 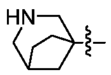 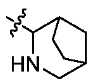 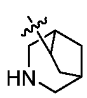 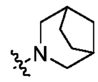 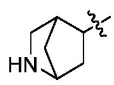

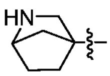 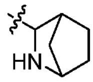 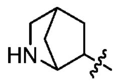 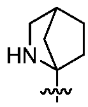 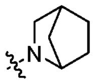 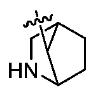 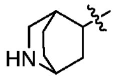 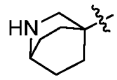 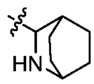 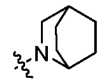

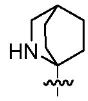 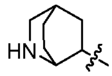 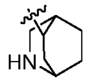 or 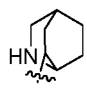 ;

or

saturated or partially unsaturated monocyclic or bicyclic or polycyclic $C_{3-20}$ cycloalkyl, e.g., cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, $C_{7-11}$ spiro-cycloalkyl, adamantanyl, noradamantanyl, bornyl, bicyclo[2.2.1]heptyl or bicyclo[2.2.1]heptenyl; or

heteroaryl, e.g., 5- to 20-membered monocyclic or bicyclic $C_{5-20}$ aromatic ring group containing one or more heteroatoms independently selected from the group consisting of oxygen, nitrogen, and sulfur, e.g., furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl; or

$C_{5-14}$ aryl optionally containing one or more fused rings, e.g., phenyl, naphthyl or fluorenyl; and

wherein the $C_{3-10}$ heterocyclyl, $C_{5-20}$ bridged heterocyclyl, $C_{3-20}$ cycloalkyl, heteroaryl and $C_{5-14}$ aryl are each independently optionally substituted with one or more substituents selected from the group consisting of deuterium, hydroxy, amino, mercapto, nitro, halogen, cyano, CHO, COOH, $N_3$, methanesulfonyloxy, trifluoromethanesulfonyloxy, p-toluenesulfonyloxy, $C_{1-6}$ alkyl, deuterated $C_{1-6}$ alkyl, halogenated $C_{1-6}$ alkyl, optionally substituted $C_{3-8}$ cycloalkyl (e.g., substituted with one or more substituents selected from the group consisting of deuterium, halogen, amino, hydroxy, mercapto, cyano, CHO, COOH, $N_3$, methanesulfonyloxy, trifluoromethanesulfonyloxy and p-toluenesulfonyloxy), optionally substituted 4- to 9-membered heterocyclyl (e.g., substituted with one or more substituents selected from the group consisting of deuterium, halogen, amino, hydroxy, mercapto, cyano, CHO, COOH, $N_3$, methanesulfonyloxy, trifluoromethanesulfonyloxy and p-toluenesulfonyloxy), $C_{1-6}$ alkoxy, deuterated $C_{1-6}$ alkyl-O-, $C_{1-6}$ alkyl-NH-, deuterated $C_{1-6}$ alkyl-NH-, $NH_2$-$C_{1-6}$ alkylene, $C_{1-6}$ alkyl-NHC(O)-, deuterated $C_{1-6}$ alkyl-NHC(O)-, $C_{1-6}$ alkyl-C(O)NH-, deuterated $C_{1-6}$ alkyl-C(O)NH- or any combination thereof.

**15.** The compound of Formula (II) or salts, enantiomers, stereoisomers, solvates, isotopically enriched analogs, prodrugs, or polymorphs thereof as claimed in claim 9 or 10, wherein LIN represents the structure of the following formula:

#-$C_{1-30}$ alkylene-;
#-$(C(R^{a1})(R^{a2}))_{n1}$-$(R_e(C(R^{a3})(R^{a4}))_{n2})_{m1}$-;
#-$(C(R^{a1})(R^{a2}))_{n1}$-$(R_e(C(R^{a3})(R^{a4}))_{n2})_{m1}$-$(R_e(C(R^{a5})(R^{a6}))_{n3})_{m2}$-;
#-$(C(R^{a1})(R^{a2}))_{n1}$-$(R_e(C(R^{a3})(R^{a4}))_{n2})_{m1}$-$(R_e(C(R^{a5})(R^{a6}))_{n3})_{m2}$-$(R_e(C(R^{a7})(R^{a8}))_{n4})_{m3}$-;
#-$(C(R^{a1})(R^{a2}))_{n1}$-$(R_f(C(R^{a3})(R^{a4}))_{n2})_{m1}$-;
#-$(C(R^{a1})(R^{a2}))_{n1}$-$(R_f(C(R^{a3})(R^{a4}))_{n2})_{m1}$-$(R_f(C(R^{a5})(R^{a6}))_{n3})_{m2}$-;
#-$(C(R^{a1})(R^{a2}))_{n1}$-$(R_f(C(R^{a3})(R^{a4}))_{n2)m1}$-$(R_f(CR^{a5})(R^{a6}))_{n3})_{m2}$-$(R_f(C(R^{a7})(R^{a8}))_{n4})_{m3}$-;
#-$(C(R^{a1})(R^{a2}))_{n1}$-$(R_e$-$R_f$-$(C(R^{a3})(R^{a4}))_{n2})_{m1}$-;
#-$(C(R^{a1})(R^{a2}))_{n1}$-$(R_e(C(R^{a3})(R^{a4}))_{n2})_{m1}$-$(R_f(C(R^{a5})(R^{a6}))_{n3})_{m2}$-;
#-$(C(R^{a1})(R^{a2}))_{n1}$-$(R_{f-}R_e$-$(C(R^{a3})(R^{a4}))_{n2})_{m1}$-; or
#-$(C(R^{a1})(R^{a2}))_{n1}$-$(R_f(C(R^{a3})(R^{a4}))_{n2})_{m1}$-$(R_e(C(R^{a5})(R^{a6}))_{n3})_{m2}$-;

wherein each $R_e$ are selected from the group consisting of O, $N(R_g)$, C(O), C(O)O, OC(O), S(O), $S(O)_2$, $S(O)_2NH$,

$NHS(O)_2$, $C(O)N(R_g)$, $N(R_g)C(O)$, or $N(R_g)C(O)N(R_g)$, where each $R_g$ independently represent H or $C_{1-6}$ alkyl; wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano, or any combination thereof;

a hydrogen atom of one or more $CH_2$ groups of the $C_{1-30}$ alkylene group is optionally replaced with a substituent selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, or any combination thereof;

$R^{a1}$, $R^{a2}$, $R^{a3}$, $R^{a4}$, $R^{a5}$, $R^{a6}$, $R^{a7}$, and $R^{a8}$ each independently represent H, deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, or any combination thereof;

symbol # indicates the point of attachment to $U_2$; and

n1, n2, n3, n4, m1, m2, and m3 each independently represent an integer of 1, 2, 3, 4, 5, 6, 7, 8, 9, 10, 11, 12, 13, 14, or 15.

**16.** The compound of Formula (II) or salts, enantiomers, stereoisomers, solvates, isotopically enriched analogs, prodrugs, or polymorphs thereof as claimed in claim 9 or 10, wherein LIN represents the structure of the following formula:

$\#\text{-}(C(R^{a1})(R^{a2}))_{n1}\text{-}(O(C(R^{a3})(R^{a4}))_{n2})_{m1}\text{-}$;
$\#\text{-}(C(R^{a1})(R^{a2}))_{n1}\text{-}(O(C(R^{a3})(R^{a4}))_{n2})_{m1}\text{-}(O(C(R^{a5})(R^{a6}))_{n3})_{m2}\text{-}$;
$\#\text{-}(C(R^{a1})(R^{a2}))_{n1}\text{-}(O(C(R^{a3})(R^{a4}))_{n2})_{m1}\text{-}(O(C(R^{a5})(R^{a6}))_{n3})_{m2}\text{-}(O(C(R^{a7})(R^{a8}))_{n4})_{m3}\text{-}$;
$\#\text{-}(C(R^{a1})(Ra^{2}))_{n1}\text{-}(N(R_g)(C(R^{a3})(R^{a4}))_{n2})_{m1}\text{-}$;
$\#\text{-}(C(R^{a1})(R^{a2}))_{n1}\text{-}(N(R_g)(C(R^{a3})(R^{a4}))_{n2})_{m1}\text{-}(N(R_g)(C(R^{a5})(R^{a6}))_{n3})_{m2}\text{-}$;
$\#\text{-}(C(R^{a1})(R^{a2}))_{n1}\text{-}(N(R_g)(C(R^{a3})(R^{a4}))_{n2})_{m1}\text{-}(N(R_g)(C(R^{a5})(R^{a6}))_{n3})_{m2}\text{-}(N(R_g)(C(R^{a7})(R^{a8}))_{n4})_{m3}\text{-}$;
$\#\text{-}(C(R^{a1})(R^{a2}))_{n1}\text{-}(C(O)N(R_g)\text{-}(C(R^{a3})(R^{a4}))_{n2})_{m1}\text{-}$;
$\#\text{-}(C(R^{a1})(R^{a2}))_{n1}\text{-}(C(O)N(R_g)\text{-}(C(R^{a3})(R^{a4}))_{n2})_{m1}\text{-}(C(O)N(R_g)\text{-}(C(R^{a5})(R^{a6}))_{n3)m2}\text{-}$;

$\#\text{-}(C(R^{a1})(R^{a2}))_{n1}\text{-}(C(O)N(R_g)\text{-}(C(R^{a3})(R^{a4}))_{n2})_{m1}\text{-}(C(O)N(R_g)\text{-}(C(R^{a5})(R^{a6}))_{n3})_{m2}\text{-}(C(O)N(R_g)\text{-}(C(R^{a7})(R^{a8}))_{n4})_{m3}\text{-}$;
$\#\text{-}(C(R^{a1})(R^{a2}))_{n1}\text{-}(C(O)N(R_g)\text{-}(C(R^{a3})(R^{a4}))_{n2})_{m1}\text{-}(O\text{-}(C(R^{a5})(R^{a6}))_{n3})_{m2}\text{-}$;
$\#\text{-}(C(R^{a1})(R^{a2}))_{n1}\text{-}C(O)N(R_g)\text{-}(C(R^{a3})(R^{a4}))_{n2}\text{-}(O(C(R^{a5})(R^{a6}))_{n3})_{m1}\text{-}$;
$\#\text{-}(C(R^{a1})(R^{a2}))_{n1}\text{-}(N(R_g)C(O)\text{-}(C(R^{a3})(R^{a4}))_{n2})_{m1}\text{-}$;
$\#\text{-}(C(R^{a1})(R^{a2}))_{n1}\text{-}(N(R_g)C(O)\text{-}(C(R^{a3})(R^{a4}))_{n2})_{m1}\text{-}(O\text{-}(C(R^{a5})(R^{a6}))_{n3})_{m2}\text{-}$;
$\#\text{-}(C(R^{a1})(R^{a2}))_{n1}\text{-}(N(R_g)C(O)\text{-}(C(R^{a3})(R^{a4}))_{n2})_{m1}\text{-}(O\text{-}(C(R^{a5})(R^{a6}))_{n3})_{m2}\text{-}(O\text{-}(C(R_g)(R^{a8}))_{n4})_{m3}\text{-}$;
$\#\text{-}(C(R^{a1})(R^{a2}))_{n1}\text{-}N(R_g)C(O)\text{-}(C(R^{a3})(R^{a4}))_{n2}\text{-}(O(C(R^{a5})(R^{a6}))_{n3})_{m1}\text{-}$;
$\#\text{-}(C(R^{a1})(R^{a2}))_{n1}\text{-}N(R_g)C(O)N(R_g)\text{-}(C(R^{a3})(R^{a4}))_{n2}\text{-}$;
$\#\text{-}(C(R^{a1})(R^{a2}))_{n1}\text{-}C(O)\text{-}(C(R^{a3})(R^{a4}))_{n2}\text{-}$;
$\#\text{-}(C(R^{a1})(R^{a2}))_{n1}\text{-}(\text{arylene-}(C(R^{a3})(R^{a4}))_{n2})_{m1}\text{-}$;
$\#\text{-}(C(R^{a1})(R^{a2}))_{n1}\text{-}(\text{arylene-}(C(R^{a3})(R^{a4}))_{n2})_{m1}\text{-arylene-}(C(R^{a5})(R^{a6}))_{n3})\text{-}$;
$\#\text{-}(C(R^{a1})(R^{a2}))_{n1}\text{-}(\text{heterocyclylene-}(C(R^{a3})(R^{a4}))_{n2})_{m1}\text{-}$;
$\#\text{-}(C(R^{a1})(R^{a2}))_{n1}\text{-}(\text{heterocyclylene-}(C(R^{a3})(R^{a4}))_{n2})_{m1}\text{-}(\text{heterocyclylene-}(C(R^{a5})(R^{a6}))_{n3})_{m2}\text{-}$;
$\#\text{-}(C(R^{a1})(R^{a2}))_{n1}\text{-}(\text{heteroarylene-}(C(R^{a3})(R^{a4}))_{n2})_{m1}\text{-}$;
$\#\text{-}(C(R^{a1})(R^{a2}))_{n1}\text{-}(\text{heteroarylene-}(C(R^{a3})(R^{a4}))_{n2})_{m1}\text{-}(\text{heteroarylene-}(C(R^{a5})(R^{a6}))_{n3})_{m2}\text{-}$;
$\#\text{-}(C(R^{a1})(R^{a2}))_{n1}\text{-}(\text{cycloalkylene-}(C(R^{a3})(R^{a4}))_{n2})_{m1}\text{-}$; or
$\#\text{-}(C(R^{a1})(R^{a2}))_{n1}\text{-}(\text{cycloalkylene-}(C(R^{a3})(R^{a4}))_{n2})_{m1}\text{-}(\text{cycloalkylene-}(C(R^{a5})(R^{a6}))_{n3})_{m2}\text{-}$;

wherein each $R_g$ independently represent H or $C_{1-6}$ alkyl;

the cycloalkylene, arylene, heterocyclylene, and heteroarylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano, or any combination thereof;

R$^{a1}$, R$^{a2}$, R$^{a3}$, R$^{a4}$, R$^{a5}$, R$^{a6}$, R$^{a7}$ and R$^{a8}$ each independently represent H, deuterium, hydroxy, amino, mercapto, nitro, halogen, cyano, optionally deuterated C$_{1-6}$ alkyl, halogenated C$_{1-6}$ alkyl, optionally deuterated C$_{3-6}$ cycloalkyl, optionally deuterated C$_{1-6}$ alkoxy, optionally deuterated C$_{1-6}$ alkyl-NH-, NH$_2$-C$_{1-6}$ alkylene, optionally deuterated C$_{1-6}$ alkyl-NHC(O)-, optionally deuterated C$_{1-6}$ alkyl-C(O)NH-, or any combination thereof;

symbol # indicates the point of attachment to U$_2$; and

n1, n2, n3, n4, m1, m2, and m3 each independently represent an integer of 1, 2, 3, 4, 5, 6, 7, 8, 9, 10, 11, 12, 13, 14, or 15.

**17.** The compound of Formula (II) or salts, enantiomers, stereoisomers, solvates, isotopically enriched analogs, prodrugs, or polymorphs thereof as claimed in claim 9 or 10, wherein LIN represents the following groups:

#-CH$_2$-, #-(CH$_2$)$_2$-, #-(CH$_2$)$_3$-, #-(CH$_2$)$_4$-, #-(CH$_2$)$_5$-, #-(CH$_2$)$_6$-, #-(CH$_2$)$_7$-, #-(CH$_2$)$_8$-, #-(CH$_2$)$_9$-, #-(CH$_2$)$_{10}$-, #-(CH$_2$)$_{11}$-, #-(CH$_2$)$_{12}$-, #-(CH$_2$)$_{13}$-, #-(CH$_2$)$_{14}$-, #-(CH$_2$)$_{15}$-, #-(CH$_2$)$_{16}$-, #-(CH$_2$)$_{17}$-, #-(CH$_2$)$_{18}$-, #-(CH$_2$)$_{19}$-, #-(CH$_2$)$_{20}$-, #-(CH$_2$)$_{21}$-, #-(CH$_2$)$_{22}$-, #-(CH$_2$)$_{25}$-, or #-(CH$_2$)$_{30}$-;#-CH$_2$-O-CH$_2$-, #-CH$_2$-O-(CH$_2$)$_2$-, #-(CH$_2$)$_1$-O-(CH$_2$)$_3$-, #-(CH$_2$)$_1$-O-(CH$_2$)$_4$-, #-(CH$_2$)$_1$-O-(CH$_2$)$_5$-, #-(CH$_2$)$_1$-O-(CH$_2$)$_6$-, #-(CH$_2$)$_1$-O-(CH$_2$)$_7$-, #-(CH$_2$)$_1$-O-(CH$_2$)$_8$-, #-(CH$_2$)$_1$-O-(CH$_2$)$_9$-, #-(CH$_2$)$_1$-O-(CH$_2$)$_{10}$-, #-(CH$_2$)$_2$-O-(CH$_2$)$_1$-, #-(CH$_2$)$_2$-O-(CH$_2$)$_2$-, #-(CH$_2$)$_2$-O-(CH$_2$)$_3$-, #-(CH$_2$)$_2$-O-(CH$_2$)$_4$-, #-(CH$_2$)$_2$-O-(CH$_2$)$_5$-, #-(CH$_2$)$_2$-O-(CH$_2$)$_6$-, #-(CH$_2$)$_2$-O-(CH$_2$)$_7$-, #-(CH$_2$)$_2$-O-(CH$_2$)$_8$-, #-(CH$_2$)$_2$-O-(CH$_2$)$_9$-, #-(CH$_2$)$_2$-O-(CH$_2$)$_{10}$-, #-(CH$_2$)$_2$-O-(CH$_2$)$_{11}$-, #-(CH$_2$)$_2$-O-(CH$_2$)$_{12}$-, #-(CH$_2$)$_3$-O-(CH$_2$)$_1$-, #-(CH$_2$)$_3$-O-(CH$_2$)$_2$-, #-(CH$_2$)$_3$-O-(CH$_2$)$_3$-, #-(CH$_2$)$_3$-O-(CH$_2$)$_4$-, #-(CH$_2$)$_3$-O-(CH$_2$)$_5$-, #-(CH$_2$)$_3$-O-(CH$_2$)$_6$-, #-(CH$_2$)$_3$-O-(CH$_2$)$_7$-, #-(CH$_2$)$_4$-O-(CH$_2$)$_1$-, #-(CH$_2$)$_4$-O-(CH$_2$)$_2$-, #-(CH$_2$)$_4$-O-(CH$_2$)$_3$-, #-(CH$_2$)$_4$-O-(CH$_2$)$_4$-, #-(CH$_2$)$_4$-O-(CH$_2$)$_5$-, #-(CH$_2$)$_4$-O-(CH$_2$)$_6$-, #-(CH$_2$)$_5$-O-(CH$_2$)$_1$-, #-(CH$_2$)$_5$-O-(CH$_2$)$_2$-, #-(CH$_2$)$_5$-O-(CH$_2$)$_3$-, #-(CH$_2$)$_5$-O-(CH$_2$)$_4$-, #-(CH$_2$)$_5$-O-(CH$_2$)$_5$-, #-(CH$_2$)$_6$-O-(CH$_2$)$_1$-, #-(CH$_2$)$_6$-O-(CH$_2$)$_2$-, #-(CH$_2$)$_6$-O-(CH$_2$)$_3$-, #-(CH$_2$)$_6$-O-(CH$_2$)$_4$-, #-(CH$_2$)$_7$-O-(CH$_2$)$_1$-, #-(CH$_2$)$_7$-O-(CH$_2$)$_2$-, #-(CH$_2$)$_7$-O-(CH$_2$)$_3$-, #-(CH$_2$)$_8$-O-(CH$_2$)$_1$-, #-(CH$_2$)$_8$-O-(CH$_2$)$_2$-, #-CH(CH$_3$)-O-(CH$_2$)$_1$-, #-CH(CH$_3$)-O-(CH$_2$)$_2$-, #-CH(CH$_3$)-O-(CH$_2$)$_3$-, #-CH(CH$_3$)-O-(CH$_2$)$_4$-, #-CH(CH$_3$)-O-(CH$_2$)$_5$-, #-CH(CH$_3$)-O-(CH$_2$)$_6$-, #-CH(CH$_3$)-O-(CH$_2$)$_7$-, #-CH(CH$_3$)-O-(CH$_2$)$_8$-, #-CH(CH$_3$)-O-(CH$_2$)$_9$-, #-CH(CH$_3$)-O-(CH$_2$)$_{10}$-, #-CH$_2$-(O(CH$_2$)$_2$)$_2$-, #-CH$_2$-(O(CH$_2$)$_2$)$_3$-, #-CH$_2$-(O(CH$_2$)$_2$)$_4$-, #-CH$_2$-(O(CH$_2$)$_2$)$_5$-, #-CH$_2$-(O(CH$_2$)$_2$)$_6$-, #-CH$_2$-(O(CH$_2$)$_2$)$_7$-, #-CH$_2$-(O(CH$_2$)$_2$)$_8$-, #-CH$_2$-(O(CH$_2$)$_2$)$_9$-, #-CH$_2$-(O(CH$_2$)$_2$)$_{10}$-, #-CH$_2$-(O(CH$_2$)$_2$)$_1$-OCH$_2$-, #-CH$_2$-(O(CH$_2$)$_2$)$_2$-OCH$_2$-, #-CH$_2$-(O(CH$_2$)$_2$)$_3$-OCH$_2$-, #-CH$_2$-(O(CH$_2$)$_2$)$_4$-OCH$_2$-, #-CH$_2$-(O(CH$_2$)$_2$)$_5$-OCH$_2$-, #-CH$_2$-(O(CH$_2$)$_2$)$_6$-OCH$_2$-, #-CH$_2$-(O(CH$_2$)$_2$)$_7$-OCH$_2$-, #-CH$_2$-(O(CH$_2$)$_2$)$_8$-OCH$_2$-, #-CH$_2$-(O(CH$_2$)$_2$)$_9$-OCH$_2$-, #-CH$_2$-(O(CH$_2$)$_2$)$_{10}$-OCH$_2$-, #-(CH$_2$)$_2$-(O(CH$_2$)$_2$)$_2$-, #-(CH$_2$)$_2$-(O(CH$_2$)$_2$)$_3$-, #-(CH$_2$)$_2$-(O(CH$_2$)$_2$)$_4$-, #-(CH$_2$)$_2$-(O(CH$_2$)$_2$)$_5$-, #-(CH$_2$)$_2$-(O(CH$_2$)$_2$)$_6$-, #-(CH$_2$)$_2$-(O(CH$_2$)$_2$)$_7$-, #-(CH$_2$)$_2$-(O(CH$_2$)$_2$)$_8$-, #-(CH$_2$)$_2$-(O(CH$_2$)$_2$)$_9$-, #-(CH$_2$)$_2$-(O(CH$_2$)$_2$)$_{10}$-, #-(CH$_2$)$_3$-(O(CH$_2$)$_2$)$_2$-, #-(CH$_2$)$_3$-(O(CH$_2$)$_2$)$_3$-, #-(CH$_2$)$_3$-(O(CH$_2$)$_2$)$_4$-, #-(CH$_2$)$_3$-(O(CH$_2$)$_2$)$_5$-, #-(CH$_2$)$_3$-(O(CH$_2$)$_2$)$_6$-, #-(CH$_2$)$_3$-(O(CH$_2$)$_2$)$_7$-, #-(CH$_2$)$_3$-(O(CH$_2$)$_2$)$_8$-, #-(CH$_2$)$_3$-(O(CH$_2$)$_2$)$_9$-, #-(CH$_2$)$_3$-(O(CH$_2$)$_2$)$_{10}$-, #-(CH$_2$)$_4$-(O(CH$_2$)$_2$)$_2$-, #-(CH$_2$)$_4$-(O(CH$_2$)$_2$)$_3$-, #-(CH$_2$)$_4$-(O(CH$_2$)$_2$)$_4$-, #-(CH$_2$)$_4$-(O(CH$_2$)$_2$)$_5$-, #-(CH$_2$)$_4$-(O(CH$_2$)$_2$)$_6$-, #-(CH$_2$)$_4$-(O(CH$_2$)$_2$)$_7$-, #-(CH$_2$)$_4$-(O(CH$_2$)$_2$)$_8$-, #-(CH$_2$)$_4$-(O(CH$_2$)$_2$)$_9$-, #-(CH$_2$)$_4$-(O(CH$_2$)$_2$)$_{10}$-, #-CH$_2$-(O(CH$_2$)$_3$)$_2$-, #-CH$_2$-(O(CH$_2$)$_3$)$_3$-, #-CH$_2$-(O(CH$_2$)$_3$)$_4$-, #-CH$_2$-(O(CH$_2$)$_3$)$_5$-, #-CH$_2$-(O(CH$_2$)$_3$)$_6$-, #-CH$_2$-(O(CH$_2$)$_3$)$_7$-, #-CH$_2$-(O(CH$_2$)$_3$)$_8$-, 9-CH$_2$-(O(CH$_2$)$_3$)$_9$-, 9-CH$_2$-(O(CH$_2$)$_3$)$_{10}$-, #-(CH$_2$)$_2$-(O(CH$_2$)$_3$)$_2$-, #-(CH$_2$)$_2$-(O(CH$_2$)$_3$)$_3$-, #-(CH$_2$)$_2$-(O(CH$_2$)$_3$)$_4$-, #-(CH$_2$)$_2$-(O(CH$_2$)$_3$)$_5$-, #-(CH$_2$)$_2$-(O(CH$_2$)$_3$)$_6$-, #-(CH$_2$)$_2$-(O(CH$_2$)$_3$)$_7$-, #-(CH$_2$)$_2$-(O(CH$_2$)$_3$)$_8$-, #-(CH$_2$)$_2$-(O(CH$_2$)$_3$)$_9$-, #-(CH$_2$)$_2$-(O(CH$_2$)$_3$)$_{10}$-, #-(CH$_2$)$_3$-(O(CH$_2$)$_3$)$_2$-, #-(CH$_2$)$_3$-(O(CH$_2$)$_3$)$_3$-, #-(CH$_2$)$_3$-(O(CH$_2$)$_3$)$_4$-, #-(CH$_2$)$_3$-(O(CH$_2$)$_3$)$_5$-, #-(CH$_2$)$_3$-(O(CH$_2$)$_3$)$_6$-, #-(CH$_2$)$_3$-(O(CH$_2$)$_3$)$_7$-, #-(CH$_2$)$_3$-(O(CH$_2$)$_3$)$_8$-, #-(CH$_2$)$_3$-(O(CH$_2$)$_3$)$_9$-, #-(CH$_2$)$_3$-(O(CH$_2$)$_3$)$_{10}$-, #-CH$_2$-O-(CH$_2$)$_2$-O-(CH$_2$)$_3$-, #-CH$_2$-(O(CH$_2$)$_2$)$_2$-(O(CH$_2$)$_3$)$_2$-, #-CH$_2$-(O(CH$_2$)$_2$)$_3$-(O(CH$_2$)$_3$)$_3$-, #-CH$_2$-(O(CH$_2$)$_2$)$_4$-(O(CH$_2$)$_3$)$_4$-, #-CH$_2$-(O(CH$_2$)$_2$)$_5$-(O(CH$_2$)$_3$)$_5$-, #-CH$_2$-(O(CH$_2$)$_2$)$_6$-(O(CH$_2$)$_3$)$_6$-, #-(CH$_2$)$_2$-O-(CH$_2$)$_2$-O-(CH$_2$)$_3$-, #-(CH$_2$)$_2$-(O(CH$_2$)$_2$)$_2$-(O(CH$_2$)$_3$)$_2$-, #-(CH$_2$)$_2$-(O(CH$_2$)$_2$)$_3$-(O(CH$_2$)$_3$)$_3$-, #-(CH$_2$)$_2$-(O(CH$_2$)$_2$)$_4$-(O(CH$_2$)$_3$)$_4$-, #-(CH$_2$)$_2$-(O(CH$_2$)$_2$)$_5$-(O(CH$_2$)$_3$)$_5$-, #-(CH$_2$)$_2$-(O(CH$_2$)$_2$)$_6$-(O(CH$_2$)$_3$)$_6$-, #-(CH$_2$)$_3$-O-(CH$_2$)$_2$-O-(CH$_2$)$_3$-, #-(CH$_2$)$_3$-(O(CH$_2$)$_2$)$_2$-(O(CH$_2$)$_3$)$_2$-, #-(CH$_2$)$_3$-(O(CH$_2$)$_2$)$_3$-(O(CH$_2$)$_3$)$_3$-, #-(CH$_2$)$_3$-(O(CH$_2$)$_2$)$_4$-(O(CH$_2$)$_3$)$_4$-, #-(CH$_2$)$_3$-(O(CH$_2$)$_2$)$_5$-(O(CH$_2$)$_3$)$_5$-, #-(CH$_2$)$_3$-(O(CH$_2$)$_2$)$_6$-(O(CH$_2$)$_3$)$_6$-, #-CH$_2$-O-(CH$_2$)$_3$-O-(CH$_2$)$_2$-, #-CH$_2$-(O(CH$_2$)$_3$)$_2$-(O(CH$_2$)$_2$)$_2$-, #-CH$_2$-(O(CH$_2$)$_3$)$_3$-(O(CH$_2$)$_2$)$_3$-, #-CH$_2$-(O(CH$_2$)$_3$)$_4$-(O(CH$_2$)$_2$)$_4$-, #-CH$_2$-(O(CH$_2$)$_3$)$_5$-(O(CH$_2$)$_2$)$_5$-, #-CH$_2$-(O(CH$_2$)$_3$)$_6$-(O(CH$_2$)$_2$)$_6$-, #-(CH$_2$)$_2$-O-(CH$_2$)$_3$-O-(CH$_2$)$_2$-, #-(CH$_2$)$_2$-(O(CH$_2$)$_3$)$_2$-(O(CH$_2$)$_2$)$_2$-, #-(CH$_2$)$_2$-(O(CH$_2$)$_3$)$_3$-(O(CH$_2$)$_2$)$_3$-, #-(CH$_2$)$_2$-(O(CH$_2$)$_3$)$_4$-(O(CH$_2$)$_2$)$_4$-, #-(CH$_2$)$_2$-(O(CH$_2$)$_3$)$_5$-(O(CH$_2$)$_2$)$_5$-, #-(CH$_2$)$_2$-(O(CH$_2$)$_3$)$_6$-(O(CH$_2$)$_2$)$_6$-, #-(CH$_2$)$_3$-O-(CH$_2$)$_3$-O-(CH$_2$)$_2$-, #-(CH$_2$)$_3$-(O(CH$_2$)$_3$)$_2$-(O(CH$_2$)$_2$)$_2$-, #-(CH$_2$)$_3$-(O(CH$_2$)$_3$)$_3$-(O(CH$_2$)$_2$)$_3$-, #-(CH$_2$)$_3$-(O(CH$_2$)$_3$)$_4$-(O(CH$_2$)$_2$)$_4$-, #-(CH$_2$)$_3$-(O(CH$_2$)$_3$)$_5$-(O(CH$_2$)$_2$)$_5$-, #-(CH$_2$)$_3$-(O(CH$_2$)$_3$)$_6$-(O(CH$_2$)$_2$)$_6$-, #-CH$_2$-O-(CH$_2$)$_2$-O-CH$_2$-, #-(CH$_2$)$_2$-O-(CH$_2$)$_2$-O-CH$_2$-, #-(CH$_2$)$_2$-(O(CH$_2$)$_2$)$_2$-O-(CH$_2$)$_3$-, #-(CH$_2$)$_2$-(O(CH$_2$)$_2$)$_3$-O-(CH$_2$)$_3$-, #-(CH$_2$)$_2$-(O(CH$_2$)$_2$)$_4$-O-(CH$_2$)$_3$-, #-(CH$_2$)$_5$-(O(CH$_2$)$_2$)$_2$-O-(CH$_2$)$_5$-, #-(CH$_2$)$_5$-(O(CH$_2$)$_2$)$_2$-O-(CH$_2$)$_6$-,

#-$(CH_2)_1$-N($R_g$)-$(CH_2)_1$-, #-$(CH_2)_1$-N($R_g$)-$(CH_2)_2$-, #-$(CH_2)_1$-N($R_g$)-$(CH_2)_3$-, #-$(CH_2)_1$-N($R_g$)-$(CH_2)_4$-, #-$(CH_2)_1$-N($R_g$)-$(CH_2)_5$-, #-$(CH_2)_1$-N($R_g$)-$(CH_2)_6$-, #-$(CH_2)_1$-N($R_g$)-$(CH_2)_7$-, #-$(CH_2)_1$-N($R_g$)-$(CH_2)_8$-, #-$(CH_2)_1$-N($R_g$)-$(CH_2)_9$-, #-$(CH_2)_1$-N($R_g$)-$(CH_2)_{10}$-, #-$(CH_2)_2$-N($R_g$)-$(CH_2)_1$-, #-$(CH_2)_2$-N($R_g$)-$(CH_2)_2$-, #-$(CH_2)_2$-N($R_g$)-$(CH_2)_3$-, #-$(CH_2)_2$-N($R_g$)-$(CH_2)_4$-, #-$(CH_2)_2$-N($R_g$)-$(CH_2)_5$-, #-$(CH_2)_2$-N($R_g$)-$(CH_2)_6$-, #-$(CH_2)_2$-N($R_g$)-$(CH_2)_7$-, #-$(CH_2)_2$-N($R_g$)-$(CH_2)_8$-, #-$(CH_2)_2$-N($R_g$)-$(CH_2)_9$-, #-$(CH_2)_2$-N($R_g$)-$(CH_2)_{10}$-, #-$(CH_2)_2$-N($R_g$)-$(CH_2)_{11}$-, #-$(CH_2)_2$-N($R_g$)-$(CH_2)_{12}$-, #-$(CH_2)_3$-N($R_g$)-$(CH_2)_1$-, #-$(CH_2)_3$-N($R_g$)-$(CH_2)_2$-, #-$(CH_2)_3$-N($R_g$)-$(CH_2)_3$-, #-$(CH_2)_4$-N($R_g$)-$(CH_2)_1$-, #-$(CH_2)_4$-N($R_g$)-$(CH_2)_2$-, #-$(CH_2)_4$-N($R_g$)-$(CH_2)_3$-, #-$(CH_2)_4$-N($R_g$)-$(CH_2)_4$-, #-$(CH_2)_5$-N($R_g$)-$(CH_2)_1$-, #-$(CH_2)_5$-N($R_g$)-$(CH_2)_2$-, #-$(CH_2)_5$-N($R_g$)-$(CH_2)_3$-, #-$(CH_2)_5$-N($R_g$)-$(CH_2)_4$-, #-$(CH_2)_5$-N($R_g$)-$(CH_2)_5$-, #-$(CH_2)_6$-N($R_g$)-$(CH_2)_1$-, #-$(CH_2)_6$-N($R_g$)-$(CH_2)_2$-, #-$(CH_2)_6$-N($R_g$)-$(CH_2)_3$-, #-$(CH_2)_7$-N($R_g$)-$(CH_2)_1$-, #-$(CH_2)_7$-N($R_g$)-$(CH_2)_2$-, #-$(CH_2)_7$-N($R_g$)-$(CH_2)_3$-, #-$(CH_2)_8$-N($R_g$)-$(CH_2)_1$-, #-$(CH_2)_8$-N($R_g$)-$(CH_2)_2$-, #-$(CH_2)_8$-N($R_g$)-$(CH_2)_3$-, #-CH($CH_3$)-N($R_g$)-$(CH_2)_1$-, #-CH($CH_3$)-N($R_g$)-$(CH_2)_2$-, #-CH($CH_3$)-N($R_g$)-$(CH_2)_3$-, #-CH($CH_3$)-N($R_g$)-$(CH_2)_4$-, #-CH($CH_3$)-N($R_g$)-$(CH_2)_5$-, #-CH($CH_3$)-N($R_g$)-$(CH_2)_6$-, #-CH($CH_3$)-N($R_g$)-$(CH_2)_7$-, #-CH($CH_3$)-N($R_g$)-$(CH_2)_8$-, #-CH($CH_3$)-N($R_g$)-$(CH_2)_9$-, #-CH($CH_3$)-N($R_g$)-$(CH_2)_{10}$-, #-$CH_2$C(O)NH$CH_2$-, #-$(CH_2)_2$C(O)NH$(CH_2)_2$-, #-$(CH_2)_2$C(O)NH$(CH_2)_3$-, #-$(CH_2)_2$C(O)NH$(CH_2)_4$-, #-$(CH_2)_2$C(O)NH$(CH_2)_5$-, #-$(CH_2)_3$C(O)NH$(CH_2)_3$-, #-$(CH_2)_3$C(O)NH$(CH_2)_4$-, #-$(CH_2)_4$C(O)NH$(CH_2)_4$-, #-$(CH_2)_5$C(O)NH$(CH_2)_5$-, #-$(CH_2)_6$C(O)NH$(CH_2)_7$-, #-$(CH_2)_6$C(O)NH$(CH_2)_6$-, #-$(CH_2)_7$C(O)NH$(CH_2)_7$-, #-$(CH_2)_8$C(O)NH$(CH_2)_8$, U-$(CH_2)_9$C(O)NH$(CH_2)_9$-, #-$(CH_2)_{10}$C(O)NH$(CH_2)_{10}$-, #-$(CH_2)_2$C(O)NH$(CH_2)_2$-O-$(CH_2)_2$-, #-$CH_2$NHC(O)$CH_2$-, #-$(CH_2)_2$NHC(O)$(CH_2)_2$-, #-$(CH_2)_2$NHC(O)$(CH_2)_3$-, #-$(CH_2)_2$NHC(O)$(CH_2)_4$-, #-$(CH_2)_2$NHC(O)$(CH_2)_5$-, #-$(CH_2)_3$NHC(O)$(CH_2)_3$-, #-$(CH_2)_3$NHC(O)$(CH_2)_4$-, #-$(CH_2)_4$NHC(O)$(CH_2)_4$-, #-$(CH_2)_5$NHC(O)$(CH_2)_5$-, #-$(CH_2)_6$NHC(O)$(CH_2)_7$-, #-$(CH_2)_6$NHC(O)$(CH_2)_6$-, #-$(CH_2)_7$NHC(O)$(CH_2)_7$-, #-$(CH_2)_8$NHC(O)$(CH_2)_8$, #-$(CH_2)_9$NHC(O)$(CH_2)_9$-, #-$(CH_2)_{10}$NHC(O)$(CH_2)_{10}$-, #-$(CH_2)_4$NHC(O)$(CH_2)_8$-, #-$(CH_2)_2$NHC(O)$(CH_2)_2$-O-$(CH_2)_2$-, #-$(CH_2)_4$NHC(O)$CH_2$-, #-$CH_2$-piperidinylene-$CH_2$-, #-$CH_2$-piperidinylene-$(CH_2)_2$-, #-$CH_2$-piperidinylene-$(CH_2)_3$-, #-$CH_2$-piperidinylene-$(CH_2)_4$-, #-$CH_2$-piperidinylene-$(CH_2)_5$-, #-$CH_2$-piperidinylene-$(CH_2)_6$-, #-$CH_2$-piperidinylene-$(CH_2)_7$-, #-$CH_2$-piperidinylene-$(CH_2)_8$-, #-$(CH_2)_2$-piperidinylene-$(CH_2)_1$-, #-$(CH_2)_2$-piperidinylene-$(CH_2)_2$-, #-$(CH_2)_2$-piperidinylene-$(CH_2)_3$-, #-$(CH_2)_2$-piperidinylene-$(CH_2)_4$-, #-$(CH_2)_2$-piperidinylene-$(CH_2)_5$-, #-$(CH_2)_2$-piperidinylene-$(CH_2)_6$-, #-$(CH_2)_2$-piperidinylene-$(CH_2)_7$-, #-$(CH_2)_2$-piperidinylene-$(CH_2)_8$-, #-$(CH_2)_3$-piperidinylene-$CH_2$-, #-$(CH_2)_3$-piperidinylene-$(CH_2)_2$-, #-$(CH_2)_3$-piperidinylene-$(CH_2)_3$-, #-$(CH_2)_3$-piperidinylene-$(CH_2)_4$-, #-$(CH_2)_3$-piperidinylene-$(CH_2)_5$-, #-$(CH_2)_3$-piperidinylene-$(CH_2)_6$-, #-$(CH_2)_3$-piperidinylene-$(CH_2)_7$-, #-$(CH_2)_3$-piperidinylene-$(CH_2)_8$-, #-$(CH_2)_4$-piperidinylene-$CH_2$-, #-$(CH_2)_4$-piperidinylene-$(CH_2)_2$-, #-$(CH_2)_4$-piperidinylene-$(CH_2)_3$-, #-$(CH_2)_4$-piperidinylene-$(CH_2)_4$-, #-$(CH_2)_4$-piperidinylene-$(CH_2)_5$-, #-$(CH_2)_4$-piperidinylene-$(CH_2)_6$-, #-$(CH_2)_4$-piperidinylene-$(CH_2)_7$-, #-$(CH_2)_4$-piperidinylene-$(CH_2)_8$-, #-$(CH_2)_5$-piperidinylene-$(CH_2)_1$-, #-$(CH_2)_5$-piperidinylene-$(CH_2)_2$-, #-$(CH_2)_5$-piperidinylene-$(CH_2)_3$-, #-$(CH_2)_5$-piperidinylene-$(CH_2)_4$-, #-$(CH_2)_5$-piperidinylene-$(CH_2)_5$-, #-$(CH_2)_5$-piperidinylene-$(CH_2)_6$-, #-$(CH_2)_5$-piperidinylene-$(CH_2)_7$-, #-$(CH_2)_5$-piperidinylene-$(CH_2)_8$-, #-$(CH_2)_6$-piperidinylene-$(CH_2)_1$-, #-$(CH_2)_6$-piperidinylene-$(CH_2)_2$-, #-$(CH_2)_6$-piperidinylene-$(CH_2)_3$-, #-$(CH_2)_6$-piperidinylene-$(CH_2)_4$-, #-$(CH_2)_6$-piperidinylene-$(CH_2)_5$-, #-$(CH_2)_6$-piperidinylene-$(CH_2)_6$-, #-$(CH_2)_6$-piperidinylene-$(CH_2)_7$-, #-$(CH_2)_6$-piperidinylene-$(CH_2)_8$-, #-$(CH_2)_7$-piperidinylene-$(CH_2)_1$-, #-$(CH_2)_7$-piperidinylene-$(CH_2)_2$-, #-$(CH_2)_7$-piperidinylene-$(CH_2)_3$-, #-$(CH_2)_7$-piperidinylene-$(CH_2)_4$-, #-$(CH_2)_7$-piperidinylene-$(CH_2)_8$-, #-$(CH_2)_8$-piperidinylene-$CH_2$-, #-$(CH_2)_8$-piperidinylene-$(CH_2)_2$-, #-$(CH_2)_8$-piperidinylene-$(CH_2)_3$-, #-$(CH_2)_8$-piperidinylene-$(CH_2)_4$-, #-$(CH_2)_8$-piperidinylene-$(CH_2)_5$-, #-$(CH_2)_8$-piperidinylene-$(CH_2)_6$-, #-$(CH_2)_8$-piperidinylene-$(CH_2)_7$-, #-$(CH_2)_8$-piperidinylene-$(CH_2)_8$-, #-$CH_2$-N($R_g$)-$CH_2$-piperidinylene-$CH_2$-, #-$CH_2$-N($R_g$)-$CH_2$-piperidinylene-$(CH_2)_2$-, #-$CH_2$-N($R_g$)-$CH_2$-piperidinylene-$(CH_2)_3$-, #-$CH_2$-N($R_g$)-$CH_2$-piperidinylene-$(CH_2)_4$-, #-$CH_2$-N($R_g$)-$CH_2$-piperidinylene-$(CH_2)_5$-, #-$CH_2$-N($R_g$)-$CH_2$-piperidinylene-$(CH_2)_6$-, #-$CH_2$-N($R_g$)-$CH_2$-piperidinylene-$(CH_2)_7$-, #-$CH_2$-N($R_g$)-$CH_2$-piperidinylene-$(CH_2)_8$-, #-$CH_2$-N($R_g$)-$(CH_2)_2$-piperidinylene-$CH_2$-, #-$CH_2$-N($R_g$)-$(CH_2)_2$-piperidinylene-$(CH_2)_2$-, #-$CH_2$-N($R_g$)-$(CH_2)_2$-piperidinylene-$(CH_2)_3$-, #-$CH_2$-N($R_g$)-$(CH_2)_2$-piperidinylene-$(CH_2)_4$-, #-$CH_2$-N($R_g$)-$(CH_2)_2$-piperidinylene-$(CH_2)_5$-, #-$CH_2$-N($R_g$)-$(CH_2)_2$-piperidinylene-$(CH_2)_6$-, #-$CH_2$-N($R_g$)-$(CH_2)_2$-piperidinylene-$(CH_2)_7$-, #-$CH_2$-N($R_g$)-$(CH_2)_2$-piperidinylene-$(CH_2)_8$-, #-$(CH_2)_2$-N($R_g$)-$CH_2$-piperidinylene-$CH_2$-, #-$(CH_2)_2$-N($R_g$)-$CH_2$-piperidinylene-$(CH_2)_2$-, #-$(CH_2)_2$-N($R_g$)-$CH_2$-piperidinylene-$(CH_2)_3$-, #-$(CH_2)_2$-N($R_g$)-$CH_2$-piperidinylene-$(CH_2)_4$-, #-$(CH_2)_2$-N($R_g$)-$CH_2$-piperidinylene-$(CH_2)_5$-, #-$(CH_2)_2$-N($R_g$)-$CH_2$-piperidinylene-$(CH_2)_6$-, #-$(CH_2)_2$-N($R_g$)-$CH_2$-piperidinylene-$(CH_2)_7$-, #-$(CH_2)_2$-N($R_g$)-$CH_2$-piperidinylene-$(CH_2)_8$-, #-$(CH_2)_3$-N($R_g$)-$CH_2$-piperidinylene-$CH_2$-, #-$(CH_2)_3$-N($R_g$)-$CH_2$-piperidinylene-$(CH_2)_2$-, #-$(CH_2)_3$-N($R_g$)-$CH_2$-piperidinylene-$(CH_2)_3$-, #-$(CH_2)_3$-N($R_g$)-$CH_2$-piperidinylene-$(CH_2)_8$-, #-$(CH_2)_4$-N($R_g$)-$CH_2$-piperidinylene-$CH_2$-, #-$(CH_2)_4$-N($R_g$)-$CH_2$-piperidinylene-$(CH_2)_2$-, #-$(CH_2)_4$-N($R_g$)-$CH_2$-piperidinylene-$(CH_2)_3$-, #-$(CH_2)_4$-N($R_g$)-$CH_2$-piperidinylene-$(CH_2)_8$-, #-$(CH_2)_5$-N($R_g$)-$CH_2$-piperidinylene-$(CH_2)_3$-, #-$(CH_2)_5$-N($R_g$)-$CH_2$-piperidinylene-$(CH_2)_8$-, #-$(CH_2)_6$-N($R_g$)-$CH_2$-piperidinylene-$(CH_2)_3$-, #-$(CH_2)_6$-N($R_g$)-$CH_2$-piperidinylene-$(CH_2)_8$-,

#-$(CH_2)_7$-N($R_g$)-$CH_2$-piperidinylene-$(CH_2)_3$-, #-$(CH_2)_7$-N($R_g$)-$CH_2$-piperidinylene-$(CH_2)_8$-, #-$(CH_2)_8$-N($R_g$)-$CH_2$-piperidinylene-$CH_2$-, #-$(CH_2)_8$-N($R_g$)-$CH_2$-piperidinylene-$(CH_2)_2$-, #-$(CH_2)_8$-N($R_g$)-$CH_2$-piperidinylene-$(CH_2)_3$-, #-$(CH_2)_8$-N($R_g$)-$CH_2$-piperidinylene-$(CH_2)_4$-, #-$(CH_2)_8$-N($R_g$)-$CH_2$-piperidinylene-$(CH_2)_5$-, #-$(CH_2)_8$-N($R_g$)-$CH_2$-piperidinylene-$(CH_2)_6$-, #-$(CH_2)_8$-N($R_g$)-$CH_2$-piperidinylene-$(CH_2)_7$-, #-$(CH_2)_8$-N($R_g$)-$CH_2$-piperidinylene-$(CH_2)_8$-, #-$CH_2$-piperazinylene-$CH_2$-, #-$CH_2$-piperazinylene-$(CH_2)_2$-, #-$CH_2$-piperazinylene-$(CH_2)_3$-, #-$CH_2$-piperazinylene-$(CH_2)_4$-, #-$CH_2$-piperazinylene-$(CH_2)_5$-, #-$CH_2$-piperazinylene-$(CH_2)_6$-, #-$CH_2$-piperazinylene-$(CH_2)_7$-, #-$CH_2$-piperazinylene-$(CH_2)_8$-, #-$(CH_2)_2$-piperazinylene-$(CH_2)_1$-, #-$(CH_2)_2$-piperazinylene-$(CH_2)_2$-, #-$(CH_2)_2$-piperazinylene-$(CH_2)_3$-, #-$(CH_2)_2$-piperazinylene-$(CH_2)_4$-, #-$(CH_2)_2$-piperazinylene-$(CH_2)_5$-, #-$(CH_2)_2$-piperazinylene-$(CH_2)_6$-, #-$(CH_2)_2$-piperazinylene-$(CH_2)_7$-, #-$(CH_2)_2$-piperazinylene-$(CH_2)_8$-, #-$(CH_2)_3$-piperazinylene-$CH_2$-, #-$(CH_2)_3$-piperazinylene-$(CH_2)_2$-, #-$(CH_2)_3$-piperazinylene-$(CH_2)_3$-, #-$(CH_2)_3$-piperazinylene-$(CH_2)_4$-, #-$(CH_2)_3$-piperazinylene-$(CH_2)_5$-, #-$(CH_2)_3$-piperazinylene-$(CH_2)_6$-, #-$(CH_2)_3$-piperazinylene-$(CH_2)_7$-, #-$(CH_2)_3$-piperazinylene-$(CH_2)_8$-, #-$(CH_2)_4$-piperazinylene-$CH_2$-, #-$(CH_2)_4$-piperazinylene-$(CH_2)_2$-, #-$(CH_2)_4$-piperazinylene-$(CH_2)_3$-, #-$(CH_2)_4$-piperazinylene-$(CH_2)_4$-, #-$(CH_2)_4$-piperazinylene-$(CH_2)_5$-, #-$(CH_2)_4$-piperazinylene-$(CH_2)_6$-, #-$(CH_2)_4$-piperazinylene-$(CH_2)_7$-, #-$(CH_2)_4$-piperazinylene-$(CH_2)_8$-, #-$(CH_2)_5$-piperazinylene-$(CH_2)_1$-, #-$(CH_2)_5$-piperazinylene-$(CH_2)_2$-, #-$(CH_2)_5$-piperazinylene-$(CH_2)_3$-, #-$(CH_2)_5$-piperazinylene-$(CH_2)_4$-, #-$(CH_2)_5$-piperazinylene-$(CH_2)_5$-, #-$(CH_2)_5$-piperazinylene-$(CH_2)_6$-, #-$(CH_2)_5$-piperazinylene-$(CH_2)_7$-, #-$(CH_2)_5$-piperazinylene-$(CH_2)_8$-, #-$(CH_2)_6$-piperazinylene-$(CH_2)_1$-, #-$(CH_2)_6$-piperazinylene-$(CH_2)_2$-, #-$(CH_2)_6$-piperazinylene-$(CH_2)_3$-, #-$(CH_2)_6$-piperazinylene-$(CH_2)_4$-, #-$(CH_2)_6$-piperazinylene-$(CH_2)_5$-, #-$(CH_2)_6$-piperazinylene-$(CH_2)_6$-, #-$(CH_2)_6$-piperazinylene-$(CH_2)_7$-, #-$(CH_2)_6$-piperazinylene-$(CH_2)_8$-, #-$(CH_2)_7$-piperazinylene-$(CH_2)_1$-, #-$(CH_2)_7$-piperazinylene-$(CH_2)_2$-, #-$(CH_2)_7$-piperazinylene-$(CH_2)_3$-, #-$(CH_2)_7$-piperazinylene-$(CH_2)_4$-, #-$(CH_2)_7$-piperazinylene-$(CH_2)_8$-, #-$(CH_2)_8$-piperazinylene-$CH_2$-, #-$(CH_2)_8$-piperazinylene-$(CH_2)_2$-, #-$(CH_2)_8$-piperazinylene-$(CH_2)_3$-, #-$(CH_2)_8$-piperazinylene-$(CH_2)_4$-, #-$(CH_2)_8$-piperazinylene-$(CH_2)_5$-, #-$(CH_2)_8$-piperazinylene-$(CH_2)_6$-, #-$(CH_2)_8$-piperazinylene-$(CH_2)_7$-, or #-$(CH_2)_8$-piperazinylene-$(CH_2)_8$-;

wherein the piperidinylene and the piperazinylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano, or any combination thereof;

each $R_g$ independently represent H or $C_{1-6}$ alkyl;

symbol # indicates the point of attachment to $U_2$; and

n1, n2, n3, n4, m1, m2, and m3 each independently represent an integer of 1, 2, 3, 4, 5, 6, 7, 8, 9, 10, 11, 12, 13, 14, or 15.

**18.** The compound of Formula (II) or salts, enantiomers, stereoisomers, solvates, isotopically enriched analogs, prodrugs, or polymorphs thereof as claimed in claim 9 or 10, wherein $R_a$ represents the following groups:

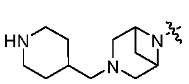 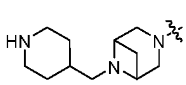 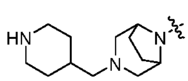 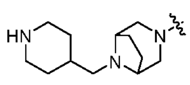 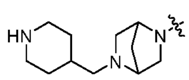 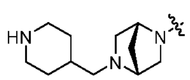

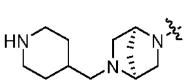 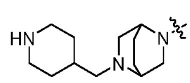 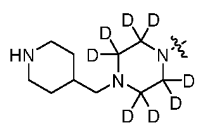 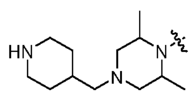 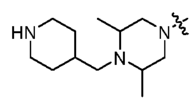 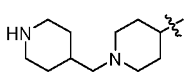

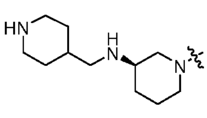 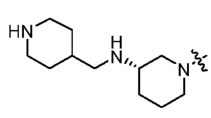 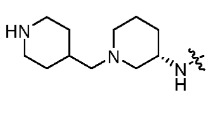 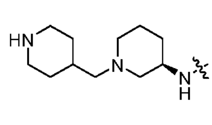 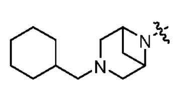 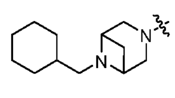

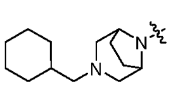 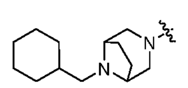 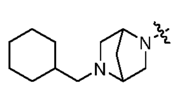 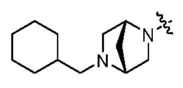 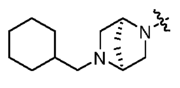 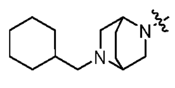

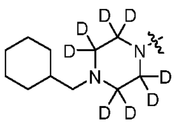 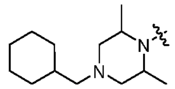 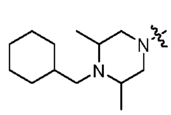 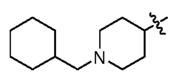 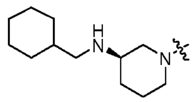 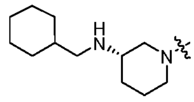

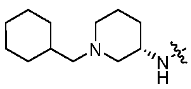 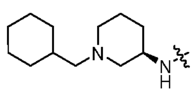 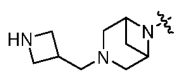 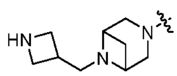 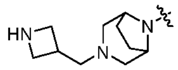 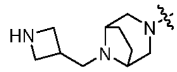

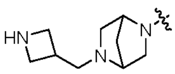 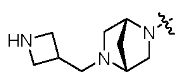 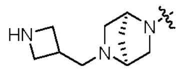 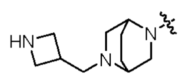 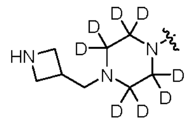 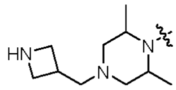

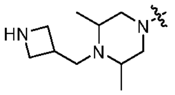 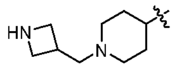 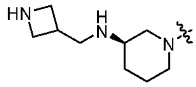 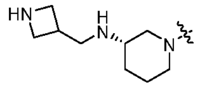 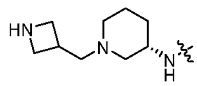 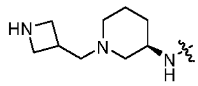

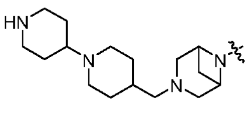 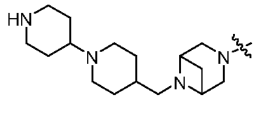 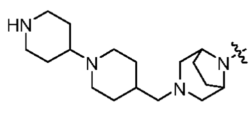 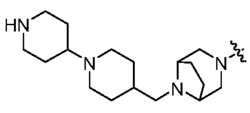 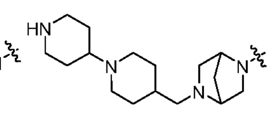

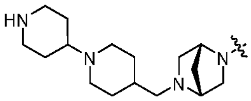 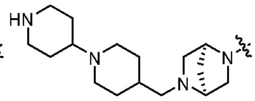 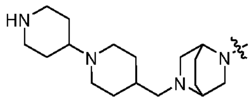 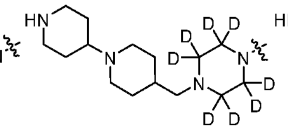 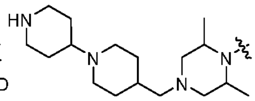

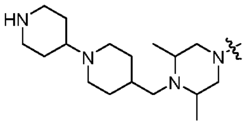 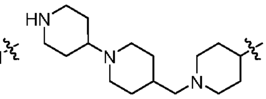 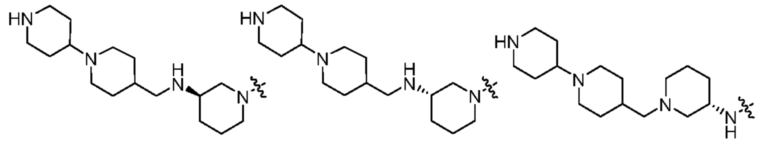

or

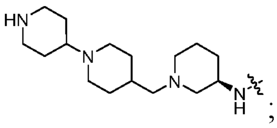 ;

wherein the groups are optionally partially or fully deuterated.

**19.** The compound of Formula (II) or salts, enantiomers, stereoisomers, solvates, isotopically enriched analogs, prodrugs, or polymorphs thereof as claimed in claim 9, which is selected from:

2-(2,6-dioxopiperidin-3-yl)-5-(3-(piperidin-4-ylmethyl)-3,6-diazabicyclo[3.1.1]heptan-6-yl)isoindoline-1,3-dione;
2-(2,6-dioxopiperidin-3-yl)-5-fluoro-6-(3-(piperidin-4-ylmethyl)-3,6-diazabicyclo[3.1.1]heptan-6-yl)isoindoline-1,3-dione;
2-(2,6-dioxopiperidin-3-yl)-5-(6-(piperidin-4-ylmethyl)-3,6-diazabicyclo[3.1.1]heptan-3-yl)isoindoline-1,3-dione;

2-(2,6-dioxopiperidin-3-yl)-5-fluoro-6-(6-(piperidin-4-ylmethyl)-3,6-diazabicyclo[3.1.1]heptan-3 -yl)isoindoline-1,3-dione;
2-(2,6-dioxopiperidin-3-yl)-5-(3-(piperidin-4-ylmethyl)-3,8-diazabicyclo[3.2.1]octan-8-yl)isoindoline-1,3-dione;
2-(2,6-dioxopiperidin-3-yl)-5-fluoro-6-(3-(piperidin-4-ylmethyl)-3,8-diazabicyclo[3.2.1]octan-8-yl)isoindoline-1,3-dione;
2-(2,6-dioxopiperidin-3-yl)-5-(8-(piperidin-4-ylmethyl)-3,8-diazabicyclo[3.2.1]octan-3-yl)isoindoline-1,3-dione;
2-(2,6-dioxopiperidin-3-yl)-5-fluoro-6-(8-(piperidin-4-ylmethyl)-3,8-diazabicyclo[3.2.1]octan-3-yl)isoindoline-1,3-dione;
2-(2,6-dioxopiperidin-3-yl)-5-(5-(piperidin-4-ylmethyl)-2,5-diazabicyclo[2.2.1]heptan-2-yl)isoindoline-1,3-dione;
2-(2,6-dioxopiperidin-3-yl)-5-fluoro-6-(5-(piperidin-4-ylmethyl)-2,5-diazabicyclo[2.2.1]heptan-2-yl)isoindoline-1,3-dione;
2-(2,6-dioxopiperidin-3-yl)-5-((1R,4R)-5-(piperidin-4-ylmethyl)-2,5-diazabicyclo[2.2.1]heptan-2-yl)isoindoline-1,3-dione;
2-(2,6-dioxopiperidin-3-yl)-5-fluoro-6-((1R,4R)-5-(piperidin-4-ylmethyl)-2,5-diazabicyclo[2.2.1]heptan-2-yl)isoindoline-1,3-dione;
2-(2,6-dioxopiperidin-3-yl)-5-((1S,4S)-5-(piperidin-4-ylmethyl)-2,5-diazabicyclo[2.2.1]heptan-2-yl)isoindoline-1,3-dione;
2-(2,6-dioxopiperidin-3-yl)-5-fluoro-6-((1S,4S)-5-(piperidin-4-ylmethyl)-2,5-diazabicyclo[2.2.1]heptan-2-yl)isoindoline-1,3-dione;
2-(2,6-dioxopiperidin-3-yl)-5-(5-(piperidin-4-ylmethyl)-2,5-diazabicyclo[2.2.2]octan-2-yl)isoindoline-1,3-dione;
2-(2,6-dioxopiperidin-3-yl)-5-fluoro-6-(5-(piperidin-4-ylmethyl)-2,5-diazabicyclo[2.2.2]octan-2-yl)isoindoline-1,3-dione;
2-(2,6-dioxopiperidin-3-yl)-5-(4-(piperidin-4-ylmethyl)piperazin-1-yl-2,2,3,3,5,5,6,6-d8)isoindoline-1,3-dione;
2-(2,6-dioxopiperidin-3-yl)-5-fluoro-6-(4-(piperidin-4-ylmethyl)piperazin-1-yl-2,2,3,3,5,5,6,6-d8)isoindoline-1,3-dione;
2-(2,6-dioxopiperidin-3-yl)-4-fluoro-6-(4-(piperidin-4-ylmethyl)piperazin-1-yl-2,2,3,3,5,5,6,6-d8)isoindoline-1,3-dione;
2-(2,6-dioxopiperidin-3-yl)-4-fluoro-5-(4-(piperidin-4-ylmethyl)piperazin-1-yl-2,2,3,3,5,5,6,6-d8)isoindoline-1,3-dione;
2-(2,6-dioxopiperidin-3-yl)-4,6-difluoro-5-(4-(piperidin-4-ylmethyl)piperazin-1-yl-2,2,3,3,5,5,6,6-d8)isoindoline-1,3-dione;
2-(2,6-dioxopiperidin-3-yl)-4,7-difluoro-5-(4-(piperidin-4-ylmethyl)piperazin-1-yl-2,2,3,3,5,5,6,6-d8)isoindoline-1,3-dione;
2-(2,6-dioxopiperidin-3-yl)-4,5-difluoro-6-(4-(piperidin-4-ylmethyl)piperazin-1-yl-2,2,3,3,5,5,6,6-d8)isoindoline-1,3-dione;
2-(2,6-dioxopiperidin-3-yl)-4,5,7-trifluoro-6-(4-(piperidin-4-ylmethyl)piperazin-1-yl-2,2,3,3,5,5,6,6-d8)isoindoline-1,3-dione;
5-(2,6-dimethyl-4-(piperidin-4-ylmethyl)piperazin-1-yl)-2-(2,6-dioxopiperidin-3-yl)isoindoline-1,3-dione;
5-(2,6-dimethyl-4-(piperidin-4-ylmethyl)piperazin-1-yl)-2-(2,6-dioxopiperidin-3-yl)-6-fluoroisoindoline-1,3-dione;
5-(3,5-dimethyl-4-(piperidin-4-ylmethyl)piperazin-1-yl)-2-(2,6-dioxopiperidin-3-yl)-6-fluoroisoindoline-1,3-dione;
5-(3,5-dimethyl-4-(piperidin-4-ylmethyl)piperazin-1-yl)-2-(2,6-dioxopiperidin-3-yl)isoindoline-1,3-dione;
2-(2,6-dioxopiperidin-3-yl)-5-(1-(piperidin-4-ylmethyl)piperidin-4-yl)isoindoline-1,3-dione;
2-(2,6-dioxopiperidin-3-yl)-5-fluoro-6-(1-(piperidin-4-ylmethyl)piperidin-4-yl)isoindoline-1,3-dione;
2-(2,6-dioxopiperidin-3-yl)-5-((R)-3-((piperidin-4-ylmethyl)amino)piperidin-1-yl)isoindoline-1,3-dione;
2-(2,6-dioxopiperidin-3-yl)-5-((S)-3-((piperidin-4-ylmethyl)amino)piperidin-1-yl)isoindoline-1,3-dione;
2-(2,6-dioxopiperidin-3-yl)-5-(((S)-1-(piperidin-4-ylmethyl)piperidin-3-yl)amino)isoindoline-1,3-dione;
2-(2,6-dioxopiperidin-3-yl)-5-(((R)-1-(piperidin-4-ylmethyl)piperidin-3-yl)amino)isoindoline-1,3-dione;
5-(3-(azetidin-3-ylmethyl)-3,6-diazabicyclo[3.1.1]heptan-6-yl)-2-(2,6-dioxopiperidin-3-yl)isoindoline-1,3-dione;
5-(3-(azetidin-3-ylmethyl)-3,6-diazabicyclo[3.1.1]heptan-6-yl)-2-(2,6-dioxopiperidin-3-yl)-6-fluoroisoindoline-1,3-dione;
5-(6-(azetidin-3-ylmethyl)-3,6-diazabicyclo[3.1.1]heptan-3-yl)-2-(2,6-dioxopiperidin-3-yl)isoindoline-1,3-dione;
5-(6-(azetidin-3-ylmethyl)-3,6-diazabicyclo[3.1.1]heptan-3-yl)-2-(2,6-dioxopiperidin-3-yl)-6-fluoroisoindoline-1,3-dione;
5-(3-(azetidin-3-ylmethyl)-3,8-diazabicyclo[3.2.1]octan-8-yl)-2-(2,6-dioxopiperidin-3-yl)isoindoline-1,3-dione;
5-(3-(azetidin-3-ylmethyl)-3,8-diazabicyclo[3.2.1]octan-8-yl)-2-(2,6-dioxopiperidin-3-yl)-6-fluoroisoindoline-1,3-dione;
5-(8-(azetidin-3-ylmethyl)-3,8-diazabicyclo[3.2.1]octan-3-yl)-2-(2,6-dioxopiperidin-3-yl)isoindoline-1,3-dione;
2-(2,6-dioxopiperidin-3-yl)-5-fluoro-6-(8-(piperidin-4-ylmethyl)-3,8-diazabicyclo[3.2.1]octan-3-yl)isoindoline-1,3-dione;
5-(5-(azetidin-3-ylmethyl)-2,5-diazabicyclo[2.2.1]heptan-2-yl)-2-(2,6-dioxopiperidin-3-yl)isoindoline-1,3-dione;
5-(5-(azetidin-3-ylmethyl)-2,5-diazabicyclo[2.2.1]heptan-2-yl)-2-(2,6-dioxopiperidin-3-yl)-6-fluoroisoindoline-1,3-dione;
5-((1R,4R)-5-(azetidin-3-ylmethyl)-2,5-diazabicyclo[2.2.1]heptan-2-yl)-2-(2,6-dioxopiperidin-3-yl)isoindoline-1,3-dione;
5-((1R,4R)-5-(azetidin-3-ylmethyl)-2,5-diazabicyclo[2.2.1]heptan-2-yl)-2-(2,6-dioxopiperidin-3-yl)-6-fluoroisoindoline-1,3-dione;
5-((1S,4S)-5-(azetidin-3-ylmethyl)-2,5-diazabicyclo[2.2.1]heptan-2-yl)-2-(2,6-dioxopiperidin-3-yl)isoindoline-1,3-dione;
5-((1S,4S)-5-(azetidin-3-ylmethyl)-2,5-diazabicyclo[2.2.1]heptan-2-yl)-2-(2,6-dioxopiperidin-3-yl)-6-fluoroisoindoline-1,3-dione;
5-(5-(azetidin-3-ylmethyl)-2,5-diazabicyclo[2.2.2]octan-2-yl)-2-(2,6-dioxopiperidin-3-yl)isoindoline-1,3-dione;
5-(5-(azetidin-3-ylmethyl)-2,5-diazabicyclo[2.2.2]octan-2-yl)-2-(2,6-dioxopiperidin-3-yl)-6-fluoroisoindoline-1,3-dione;
5-(4-(azetidin-3-ylmethyl)piperazin-1-yl-2,2,3,3,5,5,6,6-d8)-2-(2,6-dioxopiperidin-3-yl)isoindoline-1,3-dione;
5-(4-(azetidin-3-ylmethyl)piperazin-1-yl-2,2,3,3,5,5,6,6-d8)-2-(2,6-dioxopiperidin-3-yl)-6-fluoroisoindoline-1,3-dione;
6-(4-(azetidin-3-ylmethyl)piperazin-1-yl-2,2,3,3,5,5,6,6-d8)-2-(2,6-dioxopiperidin-3-yl)-4-fluoroisoindoline-1,3-dione;
5-(4-(azetidin-3-ylmethyl)piperazin-1-yl-2,2,3,3,5,5,6,6-d8)-2-(2,6-dioxopiperidin-3-yl)-4-fluoroisoindoline-1,3-dione;
5-(4-(azetidin-3-ylmethyl)piperazin-1-yl-2,2,3,3,5,5,6,6-d8)-2-(2,6-dioxopiperidin-3-yl)-4,6-difluoroisoindoline-1,3-dione;
5-(4-(azetidin-3-ylmethyl)piperazin-1-yl-2,2,3,3,5,5,6,6-d8)-2-(2,6-dioxopiperidin-3-yl)-4,7-difluoroisoindoline-1,3-dione;
6-(4-(azetidin-3-ylmethyl)piperazin-1-yl-2,2,3,3,5,5,6,6-d8)-2-(2,6-dioxopiperidin-3-yl)-4,5-difluoroisoindoline-1,3-dione;
5-(4-(azetidin-3-ylmethyl)piperazin-1-yl-2,2,3,3,5,5,6,6-d8)-2-(2,6-dioxopiperidin-3-yl)-4,6,7-trifluoroisoindoline-1,3-dione;
5-(4-(azetidin-3-ylmethyl)-2,6-dimethylpiperazin-1-yl)-2-(2,6-dioxopiperidin-3-yl)isoindoline-1,3-dione;
5-(4-(azetidin-3-ylmethyl)-2,6-dimethylpiperazin-1-yl)-2-(2,6-dioxopiperidin-3-yl)-6-fluoroisoindoline-1,3-dione;
5-(4-(azetidin-3-ylmethyl)-3,5-dimethylpiperazin-1-yl)-2-(2,6-dioxopiperidin-3-yl)-6-fluoroisoindoline-1,3-dione;
5-(4-(azetidin-3-ylmethyl)-3,5-dimethylpiperazin-1-yl)-2-(2,6-dioxopiperidin-3-yl)isoindoline-1,3-dione;
5-(1-(azetidin-3-ylmethyl)piperidin-4-yl)-2-(2,6-dioxopiperidin-3-yl)isoindoline-1,3-dione;
5-(1-(azetidin-3-ylmethyl)piperidin-4-yl)-2-(2,6-dioxopiperidin-3-yl)-6-fluoroisoindoline-1,3-dione;
5-((R)-3-((azetidin-3-ylmethyl)amino)piperidin-1-yl)-2-(2,6-dioxopiperidin-3-yl)isoindoline-1,3-dione;
5-((S)-3-((azetidin-3-ylmethyl)amino)piperidin-1-yl)-2-(2,6-dioxopiperidin-3-yl)isoindoline-1,3-dione;
5-(((S)-1-(azetidin-3-ylmethyl)piperidin-3-yl)amino)-2-(2,6-dioxopiperidin-3-yl)isoindoline-1,3-dione; and
5-(((R)-1-(azetidin-3-ylmethyl)piperidin-3-yl)amino)-2-(2,6-dioxopiperidin-3-yl)isoindoline-1,3-dione.

**20.** A compound of Formula (III)

PBM-$R^1$-$W^1$-LIN-ULM Formula (III)

or salts, enantiomers, stereoisomers, solvates, isotopically enriched analogs, prodrugs, or polymorphs thereof, wherein PBM-$R^1$-$W^1$- represents a targeting moiety capable of binding protein, LTLM represents a E3 ubiquitin ligase ligand moiety, LIN is a linker moiety, and PBM-$R^1$-$W^1$- is covalently bonded to LTLM through LIN; LTLM represents the structure of Formula (LTLM):

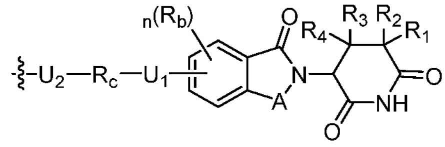

Formula (ULM)

wherein A represents CO, $CH_2$ or $CD_2$;
$R_1$, $R_2$, $R_3$ and $R_4$ are the same or different and each independently represent hydrogen, deuterium, halogen, optionally deuterated $C_{1-6}$ alkyl, optionally deuterated $C_{1-6}$ alkoxy, or halogenated $C_{1-6}$ alkyl;
$(R_b)_n$ indicates that benzene ring is optionally substituted with n $R_b$ groups, and each $R_b$ are the same or different and each independently represent hydrogen, deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkyl, $C_{2-6}$ alkenyl or $C_{2-6}$ alkynyl;
n represents an integer of 0, 1, 2 or 3;
$U_1$ and $U_2$ are the same or different and each independently represent a bond, $N(R_d)$ or O, wherein $R_d$ represents H or optionally deuterated $C_{1-6}$ alkyl;
$R_c$ represents optionally substituted nitrogen-containing monocyclic heterocyclylene or optionally substituted nitrogen-containing bridged heterocyclylene;

LIN represents a bond, optionally substituted methylene, or optionally substituted linear or branched $C_{2-30}$ alkylene, wherein one or more groups $R_e$ and/or one or more groups $R_f$ and/or any combination of one or more groups $R_e$ and $R_f$ are optionally inserted into the backbone carbon chain of the linear or branched $C_{2-30}$ alkylene group, wherein carbon-carbon bond between one or more pairs of adjacent carbon atoms in the backbone carbon chain is interrupted by the group $R_e$, $R_f$, or a combination of $R_e$ and $R_f$; wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl, and in case that two or more groups $R_e$ are inserted into the backbone carbon chain of the linear or branched $C_{2-30}$ alkylene group, the two or more groups $R_e$ are not directly connected to each other; and wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof;
$W^1$ is t interconnected rings $W^2$ and represented by the following formula:

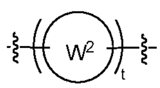

each ring $W^2$ are the same or different and each independently represent optionally substituted nitrogen-containing monocyclic heterocyclylene, optionally substituted nitrogen-containing bridged heterocyclylene, or optionally substituted cycloalkylene, and
t represents an integer of 1 or 2; and

$R^1$ represents:

a bond, -O-, optionally substituted $C_{1-6}$ alkylene (e.g., methylene), $-N(R^{1a})-R^{2a}-***$ (e.g.,

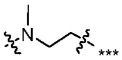

,

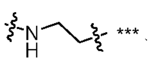

), $-O-R^{2a}-***$ (e.g.,

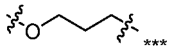, 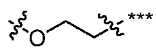

), $-C(O)NH-R^{2a}-$*** or$-NHC(O)-R^{2a}-$*** (e.g.,

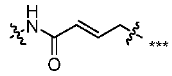

), wherein symbol *** indicates the point of attachment to $W^1$, $R^{1a}$ is H or $C_{1-6}$ alkyl, and $R^{2a}$ is optionally substituted $C_{1-6}$ alkylene or optionally substituted $C_{1-6}$ alkenylene; or

$C_{6-10}$ aryl or heteroaryl containing one or two 5- to 7-membered rings and 1 to 4 heteroatoms selected from nitrogen, oxygen, and sulfur, wherein the $C_{6-10}$ aryl and heteroaryl are each optionally substituted with one or more $R^{3a}$, and each $R^{3a}$ are the same or different and are each independently halogen, hydroxy, cyano, amino, nitro, $C_{1-6}$ alkyl, halogenated $C_{1-6}$ alkyl, $C_{1-6}$ alkoxy, or halogenated $C_{1-6}$ alkoxy;

wherein one or more hydrogens of the compound of Formula (III) are optionally replaced with deuterium; wherein when the compound of Formula (III) is not deuterated, the following compounds are excluded:

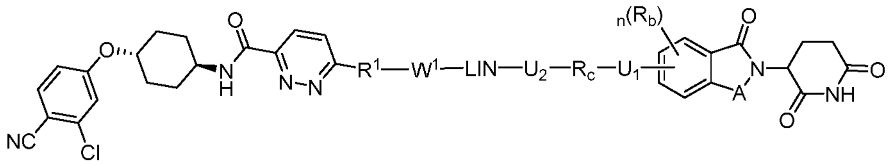

Formula (IV-1)

in Formula (IV-1), $R^1$ represents a bond, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, n represents an integer of 1, 2 or 3, and $U_1$ and $U_2$ are the same or different and each independently represent a bond, NH or O; or

in Formula (IV-1), $R^1$ represents a bond, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, n represents an integer of 1, 2 or 3, and $U_1$ and $U_2$ are the same or different and each independently represent a bond, NH or O;

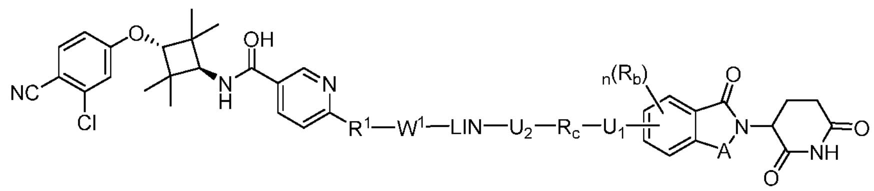

Formula (IV-2)

in Formula (IV-2), $R^1$ represents a bond, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, n represents an integer of 1, 2 or 3, and $U_1$ and $U_2$ are the same or different and each independently represent a bond, NH or O; or

in Formula (IV-2), $R^1$ represents a bond, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents fluoro, and n represents an integer of 1, 2 or 3, and $U_1$ and $U_2$ are the same or different and each independently represent a bond, NH or O;

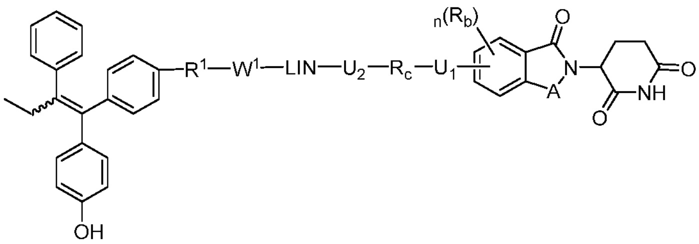

Formula (IV-3)

in Formula (IV-3), $R^1$ represents a bond or $-O-CH_2-CH_2-$, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O; or
in Formula (IV-3), $R^1$ represents a bond or $-O-CH_2-CH_2-$, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O;
and

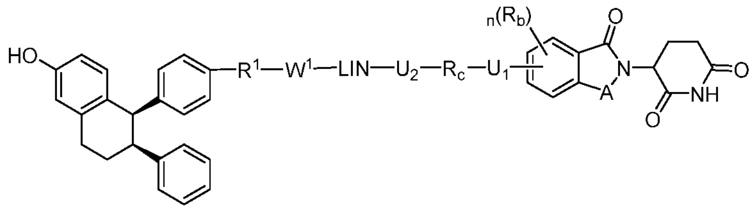

Formula (IV-4)

in Formula (IV-4), $R^1$ represents a bond or represents $-O-CH_2-CH_2-$, $W^1$ represents piperidinylene, LIN represents methylene, $R_c$ represents piperazinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O; or
in Formula (IV-4), $R^1$ represents a bond or represents $-O-CH_2-CH_2-$, $W^1$ represents piperazinylene, LIN represents methylene, $R_c$ represents piperidinylene, 2,5-diazabicyclo[2.2.1]heptanylene, 8-azabicyclo[3.2.1]octanylene, or 9-ethyl-3-methyl-3,9-diazabicyclo[3.3.1]nonanylene, $R_b$ represents hydrogen, and $U_1$ and $U_2$ are the same or different and each independently represent a bond, NH or O.

**21.** The compound of Formula (III) or salts, enantiomers, stereoisomers, solvates, isotopically enriched analogs, prodrugs, or polymorphs thereof as claimed in claim 20, wherein the PBM is a small molecule ligand targeting EGFR, CDK4/6, ALK, RET, FAK, BCR-ABL, BTK, AR, ER or BET.

**22.** The compound of Formula (III) or salts, enantiomers, stereoisomers, solvates, isotopically enriched analogs, prodrugs, or polymorphs thereof as claimed in claim 21, wherein the PBM comprises the structures of the following formula:

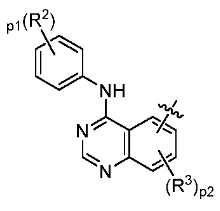

Formula (PBM-1)

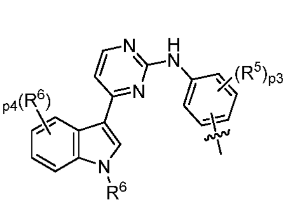

Formula (PBM-2)

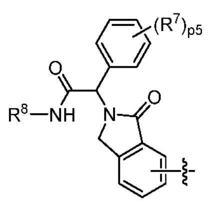

Formula (PBM-3)

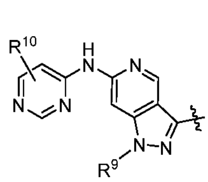

Formula (PBM-4)

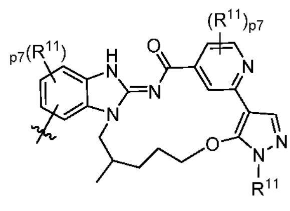

Formula (PBM-5)

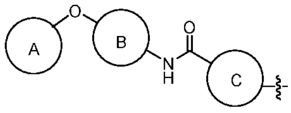
Formula (PBM-6)

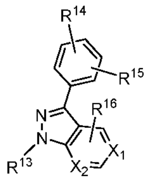
Formula (PBM-7)

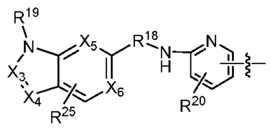
Formula (PBM-8)

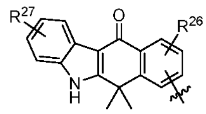
Formula (PBM-9)

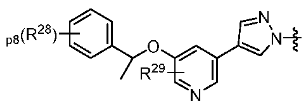
Formula (PBM-10)

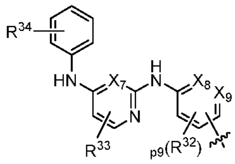
Formula (PBM-11)

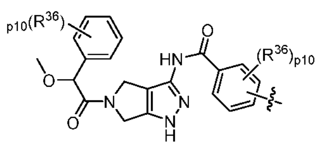
Formula (PBM-12)

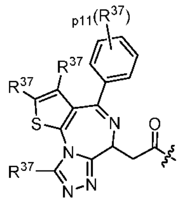
Formula (PBM-13)

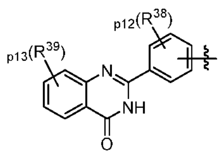
Formula (PBM-14)

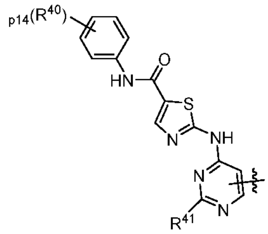
Formula (PBM-15)

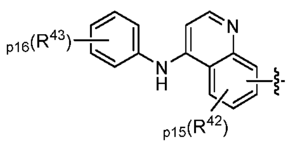
Formula (PBM-16)

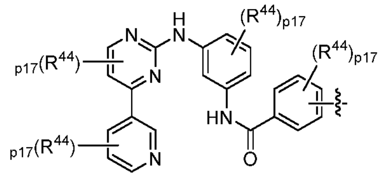
Formula (PBM-17)

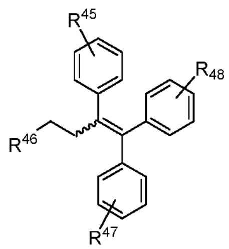
Formula (PBM-18)

or

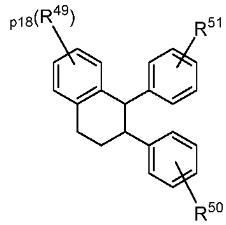
Formula (PBM-19)

wherein

$(R^2)_{p1}$ indicates that the benzene ring in Formula (PBM-1) is substituted with p1 $R^2$, and each $R^2$ are the same or different and each independently represent halogen or halogenated $C_{1-6}$ alkyl;

$(R^3)_{p2}$ indicates that the quinazoline ring in Formula (PBM-1) is substituted with p2 $R^3$, and each $R^3$ are the same or different and each independently represent $C_{1-10}$ alkyl, deuterated $C_{1-10}$ alkyl, $C_{1-10}$ alkoxy, heterocyclyloxy, or -NHC(O)$R^4$, wherein $R^4$ is $C_{1-10}$ alkyl or $C_{2-10}$ alkenyl, e.g., $R^3$ represents methyl, methoxy,

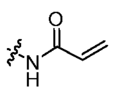 or 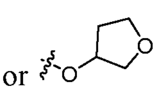,

such as

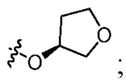;

p1 represents an integer of 1, 2, 3, 4 or 5;

p2 represents an integer of 1, 2 or 3;

$(R^5)_{p3}$ indicates that the benzene ring in Formula (PBM-2) is substituted with p3 $R^5$, and each $R^5$ are the same or different and each independently represent $C_{1-10}$ alkyl, deuterated $C_{1-10}$ alkyl, $C_{1-10}$ alkoxy, or -NHC(O)$R^4$, wherein $R^4$ is $C_{1-10}$ alkyl, deuterated $C_{1-10}$ alkyl or $C_{2-10}$ alkenyl, e.g., $R^5$ represents methyl, methoxy or

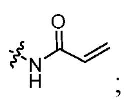;

p3 represents an integer of 1, 2, 3 or 4;

$(R^6)_{p4}$ indicates that the 1H-indole ring in Formula (PBM-2) is substituted with p4 $R^6$, and each $R^6$ are the same or different and each independently represent $C_{1-10}$ alkyl or deuterated $C_{1-10}$ alkyl;
p4 represents an integer of 0, 1, 2 or 3;
$(R^7)_{p5}$ indicates that the benzene ring in Formula (PBM-3) is substituted with p5 $R^7$, and each $R^7$ are the same or different and each independently represent halogen, hydroxy, $C_{1-6}$ alkyl, halogenated $C_{1-6}$ alkyl, deuterated $C_{1-6}$ alkyl, $NO_2$, $NH_2$, or cyano;
p5 represents an integer of 2, 3, 4 or 5;
$R^8$ represents $C_{6-10}$ aryl or heteroaryl containing one or two 5- to 7-membered rings and 1 to 4 heteroatoms selected from nitrogen, oxygen, and sulfur, wherein the $C_{6-10}$ aryl and heteroaryl are each optionally substituted with one or more $R^{4a}$, and each $R^{4a}$ are the same or different and are each independently halogen, hydroxy, cyano, amino, nitro, $C_{1-6}$ alkyl, halogenated $C_{1-6}$ alkyl, deuterated $C_{1-6}$ alkyl, $C_{1-6}$ alkoxy, or halogenated $C_{1-6}$ alkoxy;
$R^9$ represents $C_{1-10}$ alkyl (e.g.,

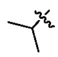

) or deuterated $C_{1-10}$ alkyl;
$R^{10}$ represents

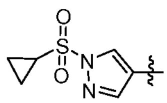 or 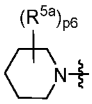,

wherein $(R^{5a})_{p6}$ indicates that the piperidine ring is substituted with p6 $R^{5a}$, and each $R^{5a}$ are the same or different and each independently represent deuterium, halogen, hydroxy, $C_{1-6}$ alkyl, halogenated $C_{1-6}$ alkyl, deuterated $C_{1-6}$ alkyl, $C_{1-6}$ alkoxy, deuterated $C_{1-6}$ alkoxy, $NO_2$, $NH_2$, or cyano, p6 represents an integer of 1, 2, 3, 4 or 5;
$(R^{11})_{p7}$ indicates that the benzene ring and the pyridine ring in Formula (PBM-5) are each optionally substituted with p7 $R^{11}$, and each $R^{11}$ are the same or different and each independently represent halogen, $C_{1-6}$ alkyl, halogenated $C_{1-6}$ alkyl, or deuterated $C_{1-6}$ alkyl, p7 represents an integer of 0, 1, 2 or 3;
ring A in Formula (PBM-6) represents $C_{6-10}$ aryl or 5- to 20-membered monocyclic or bicyclic heteroaryl containing from 1 to 4 heteroatoms selected from the group consisting of oxygen, nitrogen, and sulfur, and the ring A is optionally substituted with one or more $R^{6a}$, and each $R^{6a}$ are the same or different and each independently represent deuterium, halogen, hydroxy, cyano, amino, nitro, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{2-6}$ alkenyl, or $C_{2-6}$ alkynyl;
ring B in Formula (PBM-6) represents $C_{3-15}$ cycloalkyl or 3- to 20-membered heterocyclyl containing from 1 to 4 heteroatoms selected from the group consisting of oxygen, nitrogen, and sulfur, and the ring B is optionally substituted with one or more $R^{7a}$, and each $R^{7a}$ are the same or different and each independently represent deuterium, halogen, $C_{1-6}$ alkyl, halogenated $C_{1-6}$ alkyl, deuterated $C_{1-6}$ alkyl, $C_{1-6}$ alkoxy, or halogenated $C_{1-6}$ alkoxy;
ring C in Formula (PBM-6) represents 5- to 20-membered monocyclic or bicyclic heteroaryl containing from 1 to 4 heteroatoms selected from the group consisting of oxygen, nitrogen, and sulfur, and the ring C is optionally substituted with one or more $R^{8a}$, and each $R^{8a}$ are the same or different and each independently represent deuterium, halogen, hydroxy, cyano, amino, $C_{1-6}$ alkyl, halogenated $C_{1-6}$ alkyl, deuterated $C_{1-6}$ alkyl, $C_{1-6}$ alkoxy, or halogenated $C_{1-6}$ alkoxy;
$X_1$ in Formula (PBM-7) represents N or $CR^{12}$, wherein $R^{12}$ is hydrogen or represents a bond, and $X_2$ represents CH or N;
$R^{13}$ is hydrogen or represents a bond, wherein when $R^{13}$ represents a bond, $X_1$ represents N or CH, and the nitrogen atom in the ring connected to $R^{13}$ is directly connected to $R^1$; or when $R^{13}$ is hydrogen, $X_1$ represents $CR^{12}$, wherein $R^{12}$ represents a bond, and the carbon atom in the ring connected to $R^{12}$ is directly connected to $R^1$;
$R^{14}$ represents -O-aryl, -O-heteroaryl, $-C_{1-3}$-alkylene-NHC(O)-heteroaryl, or $-C_{1-3}$-alkylene-C(O)NH-heteroaryl, wherein the aryl and heteroaryl are each independently optionally substituted with one or more $R^{9a}$, and each $R^{9a}$ are the same or different and each independently represent deuterium, halogen, hydroxy, cyano, amino, $C_{1-6}$ alkyl, halogenated $C_{1-6}$ alkyl, deuterated $C_{1-6}$ alkyl, $C_{1-6}$ alkoxy, or halogenated $C_{1-6}$ alkoxy;
$R^{15}$ represents deuterium, hydrogen, halogen, $C_{1-6}$ alkyl, halogenated $C_{1-6}$ alkyl, deuterated $C_{1-6}$ alkyl or $-NHC(O)R^{17}$, wherein $R^{17}$ is $C_{1-10}$ alkyl or $C_{2-10}$ alkenyl, e.g., $R^{15}$ represents hydrogen, methyl,

or 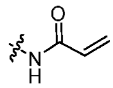 ;

$R^{16}$ represents hydrogen, cyano, or amino;
$X_3$ in Formula (PBM-8) represents $CR^{21}$ or a fragment

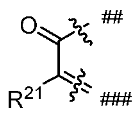 ,

wherein symbol ## indicates the point of attachment to N atom adjacent to $X_3$, symbol ### indicates the point of attachment to $X_4$, and $R^{21}$ represents deuterium, hydrogen, halogen, $C_{1-6}$ alkyl, halogenated $C_{1-6}$ alkyl, deuterated $C_{1-6}$ alkyl, -C(O)-$C_{1-6}$ alkyl, -C(O)-deuterated $C_{1-6}$ alkyl, or -C(O)$NR^{22}R^{23}$, wherein $R^{22}$ and $R^{23}$ are the same or different and each independently represent hydrogen, $C_{1-6}$ alkyl or deuterated $C_{1-6}$ alkyl, and $R^{22}$ and $R^{23}$ are not simultaneously hydrogen;
$X_4$ in Formula (PBM-8) represents N or $CR^{24}$, wherein $R^{24}$ represents hydrogen, deuterium, halogen, $C_{1-6}$ alkyl, deuterated $C_{1-6}$ alkyl or halogenated $C_{1-6}$ alkyl;
$X_5$ and $X_6$ each independently represent CH or N;
$R^{18}$ represents a bond or optionally substituted heteroarylene (e.g., halogenated heteroarylene);
$R^{19}$ represents hydrogen, $C_{1-6}$ alkyl, deuterated $C_{1-6}$ alkyl, halogenated $C_{1-6}$ alkyl, or optionally substituted $C_{3-7}$ cycloalkyl;
$R^{20}$ and $R^{25}$ are the same or different and each independently represent hydrogen, halogen, $C_{1-6}$ alkyl, deuterated $C_{1-6}$ alkyl or halogenated $C_{1-6}$ alkyl;
$R^{26}$ represents hydrogen, halogen, $C_{1-6}$ alkyl, deuterated $C_{1-6}$ alkyl or halogenated $C_{1-6}$ alkyl;
$R^{27}$ represents hydrogen, cyano, halogen, $C_{1-6}$ alkyl, deuterated $C_{1-6}$ alkyl or halogenated $C_{1-6}$ alkyl;
$(R^{28})_{p8}$ indicates that the benzene ring in Formula (PBM-10) is substituted with p8 $R^{28}$ substituents, and each $R^{28}$ are the same or different and each independently represent halogen, $C_{1-6}$ alkyl, deuterated $C_{1-6}$ alkyl or halogenated $C_{1-6}$ alkyl;
p8 represents an integer of 1, 2, 3 or 4;
$R^{29}$ represents $NR^{30}R^{31}$, wherein $R^{30}$ and $R^{31}$ are the same or different and each independently represent hydrogen, $C_{1-6}$ alkyl, or deuterated $C_{1-6}$ alkyl;
$X_7$, $X_8$ and $X_9$ in Formula (PBM-11) are the same or different and each independently represent N or CH;
$(R^{32})_{p9}$ indicates that the 6-membered ring containing $X_8$ and $X_9$ in Formula (PBM-11) is optionally substituted with p9 $R^{32}$ groups, and each $R^{32}$ are the same or different and each independently represent halogen, $C_{1-6}$ alkoxy, $C_{1-6}$ alkyl, deuterated $C_{1-6}$ alkyl or halogenated $C_{1-6}$ alkyl;
p9 represents an integer of 0, 1, 2, 3 or 4;
$R^{33}$ represents halogen, $C_{1-6}$ alkoxy, $C_{1-6}$ alkyl, deuterated $C_{1-6}$ alkyl or halogenated $C_{1-6}$ alkyl;
$R^{34}$ represents -C(O)$NHR^{35}$, -NHC(O)-$R^{35}$, -$S(O)_2NHR^{35}$, -$S(O)_2R^{35}$ or -$P(O)(R^{35})_2$, wherein each $R^{35}$ are the same or different and each independently represent $C_{1-6}$ alkyl or deuterated $C_{1-6}$ alkyl;
$(R^{36})_{p10}$ indicates that the benzene ring in Formula (PBM-12) is optionally substituted with p10 $R^{36}$ groups, and each $R^{36}$ are the same or different and each independently represent halogen, $C_{1-6}$ alkoxy, $C_{1-6}$ alkyl, deuterated $C_{1-6}$ alkyl or halogenated $C_{1-6}$ alkyl;
p10 represents an integer of 0, 1, 2, 3 or 4;
$(R^{37})_{p11}$ indicates that the benzene ring in Formula (PBM-13) is substituted with p11 $R^{37}$ groups, and each $R^{37}$ are the same or different and each independently represent halogen, $C_{1-6}$ alkoxy, $C_{1-6}$ alkyl, deuterated $C_{1-6}$ alkyl or halogenated $C_{1-6}$ alkyl;
p11 represents an integer of 0, 1, 2, 3 or 4;
$(R^{38})_{p12}$ indicates that the benzene ring in Formula (PBM-14) is substituted with p12 $R^{38}$ groups, and each $R^{38}$ are the same or different and each independently represent halogen, $C_{1-6}$ alkoxy, $C_{1-6}$ alkyl, deuterated $C_{1-6}$ alkyl or halogenated $C_{1-6}$ alkyl;
p12 represents an integer of 0, 1, 2, 3 or 4;
$(R^{39})_{p13}$ indicates that the 4-oxo-3,4-dihydroquinazoline ring in Formula (PBM-14) is substituted with p13 $R^{39}$ groups, and each $R^{39}$ are the same or different and each independently represent halogen, $C_{1-6}$ alkoxy, $C_{1-6}$ alkyl, deuterated $C_{1-6}$ alkyl or halogenated $C_{1-6}$ alkyl;
p13 represents an integer of 1, 2, 3 or 4;

$(R^{40})_{p14}$ indicates that the benzene ring in Formula (PBM-15) is substituted with p14 $R^{40}$ groups, and each $R^{40}$ are the same or different and each independently represent halogen, $C_{1-6}$ alkoxy, $C_{1-6}$ alkyl, deuterated $C_{1-6}$ alkyl or halogenated $C_{1-6}$ alkyl;

p14 represents an integer of 1, 2, 3 or 4;

$R^{41}$ represents halogen, $C_{1-6}$ alkyl, deuterated $C_{1-6}$ alkyl or halogenated $C_{1-6}$ alkyl;

$(R^{42})_{p15}$ indicates that the quinoline ring in Formula (PBM-16) is substituted with p15 $R^{42}$ groups, and each $R^{42}$ are the same or different and each independently represent halogen, cyano, $C_{1-6}$ alkoxy, $C_{1-6}$ alkyl, deuterated $C_{1-6}$ alkyl or halogenated $C_{1-6}$ alkyl;

p15 represents an integer of 2, 3 or 4;

$(R^{43})_{p16}$ indicates that the benzene ring in Formula (PBM-16) is substituted with p16 $R^{43}$ groups, and each $R^{43}$ are the same or different and each independently represent halogen, $C_{1-6}$ alkoxy, $C_{1-6}$ alkyl, deuterated $C_{1-6}$ alkyl or halogenated $C_{1-6}$ alkyl;

p16 represents an integer of 3 or 4;

$(R^{44})_{p17}$ indicates that the benzene, pyrimidine and pyridine rings in Formula (PBM-17) are each optionally substituted with p17 $R^{44}$ groups, and each $R^{44}$ are the same or different and each independently represent halogen, $C_{1-6}$ alkoxy, $C_{1-6}$ alkyl, deuterated $C_{1-6}$ alkyl or halogenated $C_{1-6}$ alkyl;

p17 represents an integer of 0, 1, 2, 3 or 4;

one of $R^{45}$, $R^{46}$, $R^{47}$ and $R^{48}$ represents a bond, and the remaining are the same or different and each independently represent hydrogen, halogen, or hydroxy, wherein when one of $R^{45}$, $R^{46}$, $R^{47}$ and $R^{48}$ represents a bond, the benzene ring or methylene in Formula (PBM-18) connected to the bond is directly connected to $R^1$;

symbol "〰" connected to double bond in Formula (PBM-18) represents a bond in stereo-configuration (cis or trans configuration);

$(R^{49})_{p18}$ indicates that 1,2,3,4-tetrahydronaphthalene ring in Formula (PBM-19) is substituted with p18 $R^{49}$ groups, and each $R^{49}$ are the same or different and each independently represent hydrogen, hydroxy, halogen, $C_{1-6}$ alkyl, deuterated $C_{1-6}$ alkyl or halogenated $C_{1-6}$ alkyl;

p18 represents an integer of 0, 1, 2, 3 or 4; and

one of $R^{50}$ and $R^{51}$ represents a bond, and another represents hydrogen, halogen, $C_{1-6}$ alkyl, deuterated $C_{1-6}$ alkyl or halogenated $C_{1-6}$ alkyl, wherein when one of $R^{50}$ and $R^{51}$ represents a bond, the benzene ring in Formula (PBM-19) connected to the bond is directly connected to $R^1$.

**23.** The compound of Formula (III) or salts, enantiomers, stereoisomers, solvates, isotopically enriched analogs, prodrugs, or polymorphs thereof as claimed in claim 21, wherein the PBM represents the structures of the following formula:

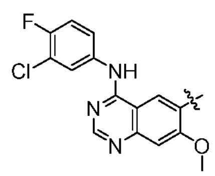
Formula (PBM-1-1)

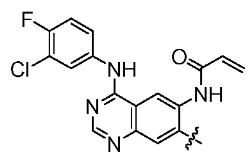
Formula (PBM-1-2)

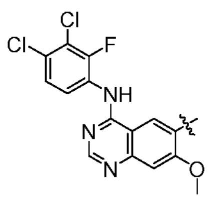
Formula (PBM-1-3)

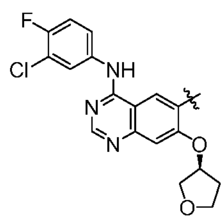
Formula (PBM-1-4)

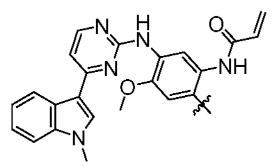
Formula (PBM-2-1)

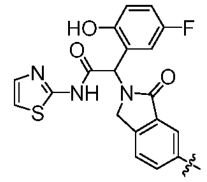
Formula (PBM-3-1)

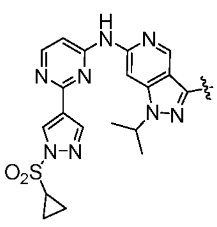
Formula (PBM-4-1)

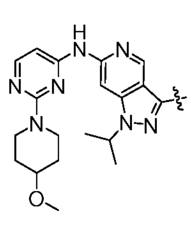
Formula (PBM-4-2)

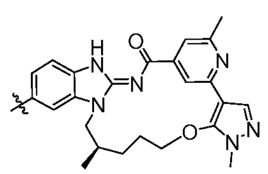
Formula (PBM-5-1)

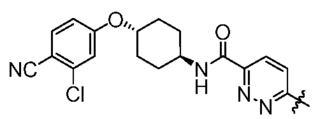
Formula (PBM-6-1)

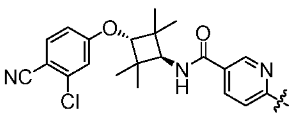
Formula (PBM-6-2)

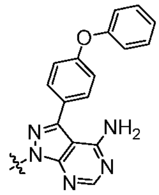
Formula (PBM-7-1)

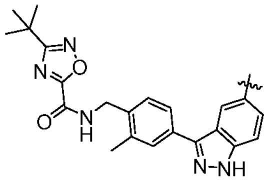
Formula (PBM-7-2)

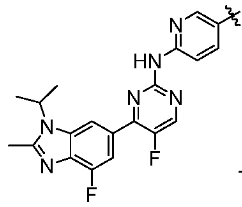
Formula (PBM-8-1)

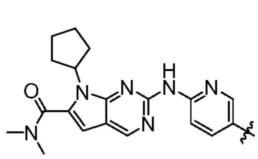
Formula (PBM-8-2)

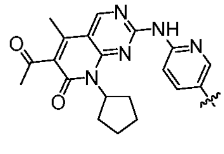
Formula (PBM-8-3)

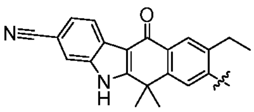
Formula (PBM-9-1)

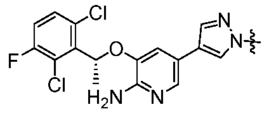
Formula (PBM-10-1)

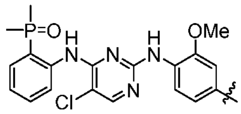
Formula (PBM-11-1)

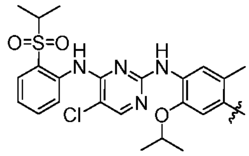
Formula (PBM-11-2)

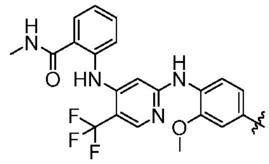
Formula (PBM-11-3)

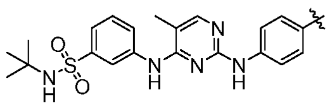
Formula (PBM-11-4)

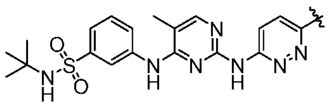
Formula (PBM-11-5)

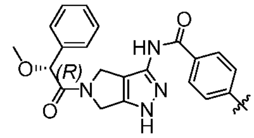
Formula (PBM-12-1)

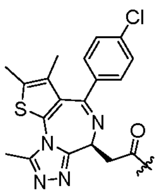
Formula (PBM-13-1)

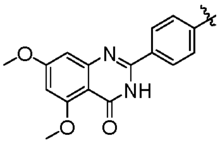
Formula (PBM-14-1)

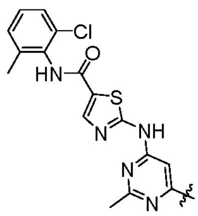
Formula (PBM-15-1)

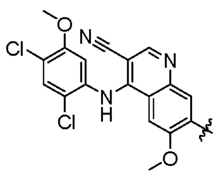
Formula (PBM-16-1)

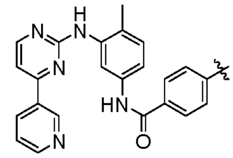
Formula (PBM-17-1)

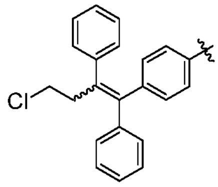
Formula (PBM-18-1)

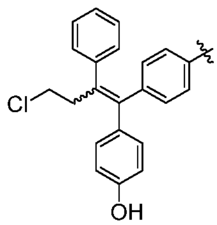
Formula (PBM-18-2)

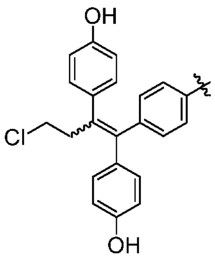
Formula (PBM-18-3)

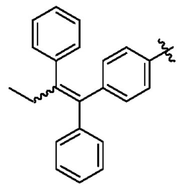
Formula (PBM-18-4)

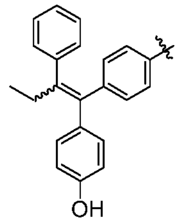
Formula (PBM-18-5)

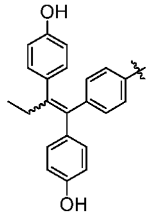
Formula (PBM-18-6)

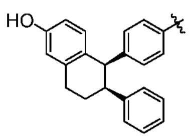
Formula (PBM-19-1) or

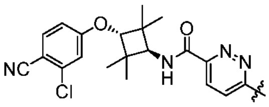
Formula (PBM-6-3).

**24.** The compound of Formula (III) or salts, enantiomers, stereoisomers, solvates, isotopically enriched analogs, prodrugs, or polymorphs thereof as claimed in claim 20, wherein the $R^1$ represents the structures of the following formula:

a bond, -O-, optionally substituted $C_{1-6}$ alkylene (e.g., methylene, ethylene, propylene, isopropylene, butylene, tert-butylene, pentylene and hexylene), $-N(R^{1a})-R^{2a}-$*** (e.g., $-N(CH_3)-CH_2CH_2-$***, $-NH-CH_2CH_2-$***), $-O-R^{2a}-$*** (e.g., $-O-(CH_2)_3-$***, $-O-CH_2-$*** and $-O-(CH_2)_2-$***), $-C(O)NH-R^{2a}-$*** (e.g., $-C(O)NH-(CH_2)_2-$***, $-C(O)NH-(CH_2)_3-$***, $-C(O)NH-CH=CH-CH_2-$***, and $-C(O)NH-CH_2-CH=CH-CH_2-$***, ), or $-NHC(O)-R^{2a}$ -*** (e.g., $-NHC(O)-CH=CH-CH_2-$***, - $NHC(O)-CH_2-CH=CH-CH_2-$***, $-NHC(O)-(CH_2)_2-$***, and $-NHC(O)-(CH_2)_3-$***), wherein symbol *** indicates the point of attachment to $W^1$, $R^{1a}$ is hydrogen or $C_{1-6}$ alkyl, and $R^{2a}$ is optionally substituted $C_{1-6}$ alkylene or optionally substituted $C_{2-6}$ alkenylene; or

phenylene or naphthylene, wherein the phenylene or naphthylene is independently optionally substituted with one or more $R^{3a}$, and each $R^{3a}$ are the same or different and each independently represent halogen, hydroxy, cyano, amino, nitro, $C_{1-6}$ alkyl, halogenated $C_{1-6}$ alkyl, $C_{1-6}$ alkoxy, or halogenated $C_{1-6}$ alkoxy; or

furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene, and imidazo[2,1-b]thiazolylene, with each group being independently optionally substituted with one or more $R^{3a}$, and each $R^{3a}$ are the same or different and are each independently halogen, hydroxy, cyano, amino, nitro, $C_{1-6}$ alkyl, halogenated $C_{1-6}$ alkyl, $C_{1-6}$ alkoxy, or halogenated $C_{1-6}$ alkoxy.

**25.** The compound of Formula (III) or salts, enantiomers, stereoisomers, solvates, isotopically enriched analogs, prodrugs, or polymorphs thereof as claimed in claim 20, wherein $W^1$ is t interconnected rings $W^2$ and represented by the following formula:

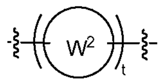

wherein each ring $W^2$ are the same or different and each independently represent the following groups:

monocyclic $C_{3-10}$ heterocyclylene group containing one nitrogen atom and optionally a heteroatom selected from nitrogen, oxygen, and sulfur, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene or diazacyclooctylene; or

$C_{5-20}$ bridged heterocyclylene group containing one nitrogen atom and optionally a heteroatom selected from nitrogen, oxygen, and sulfur, e.g., azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene or diazabicyclo[2.2.2]octanylene, such as the following groups:

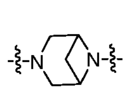 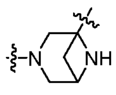 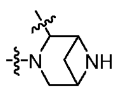 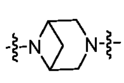 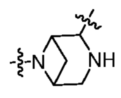 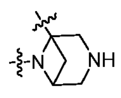 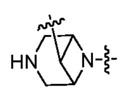 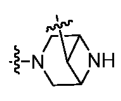 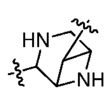 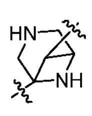

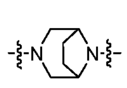 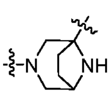 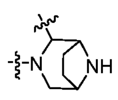 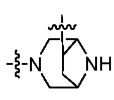 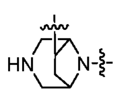 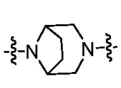 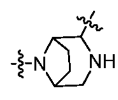 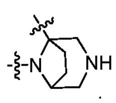 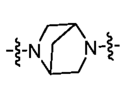 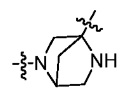

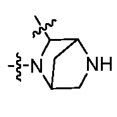 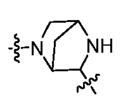 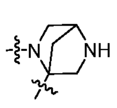 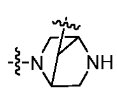 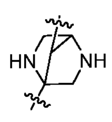 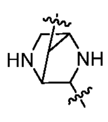 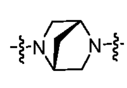 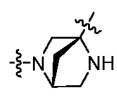 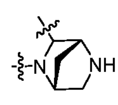 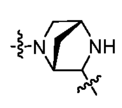

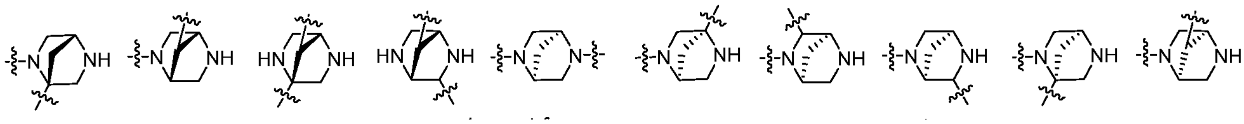

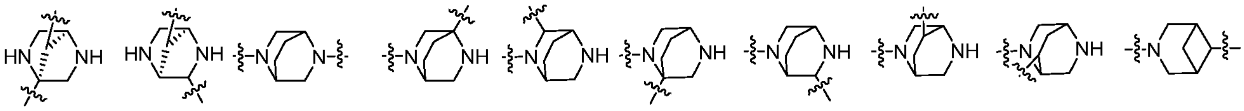

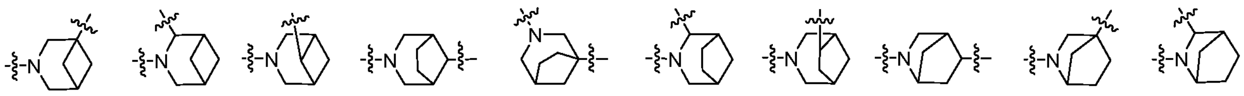

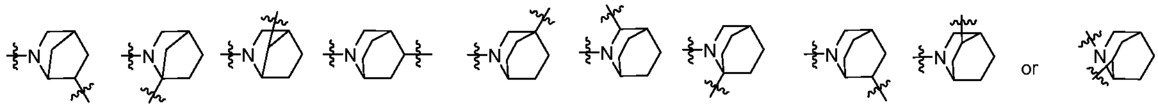
or
; or

$C_{3-15}$ cycloalkylene, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, $C_{5-15}$ spiro-cycloalkylene, adamantanylene, noradamantanylene, bornylene, bicyclo[2.2.1]heptylene or bicyclo[2.2.1]heptenylene;

wherein the monocyclic $C_{3-10}$ heterocyclylene, $C_{5-20}$ bridged heterocyclylene, and $C_{3-15}$ cycloalkylene are each independently optionally substituted with one or more substituents selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof; and

t represents an integer of 1 or 2.

**26.** The compound of Formula (III) or salts, enantiomers, stereoisomers, solvates, isotopically enriched analogs, prodrugs, or polymorphs thereof as claimed in claim 25, wherein the $W^1$ represents the following groups:

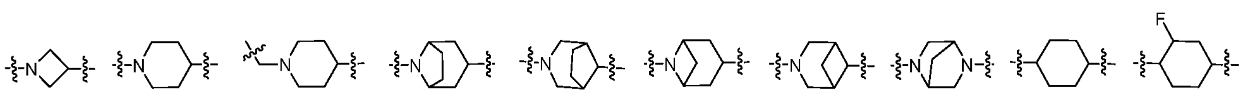

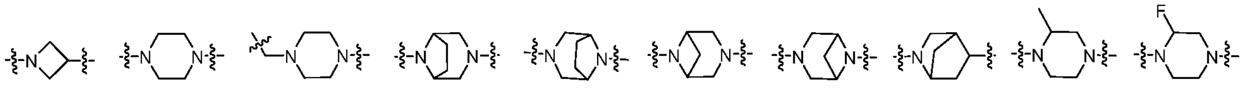

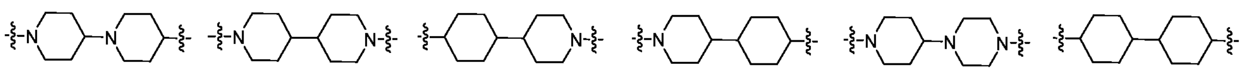

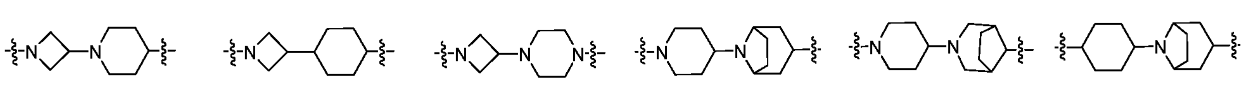

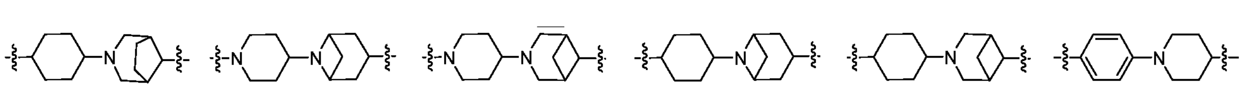

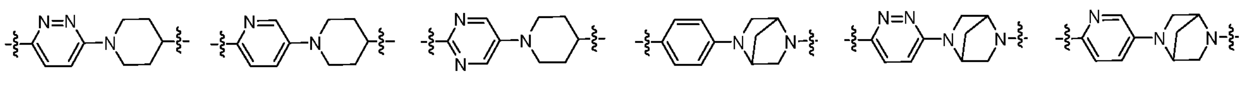

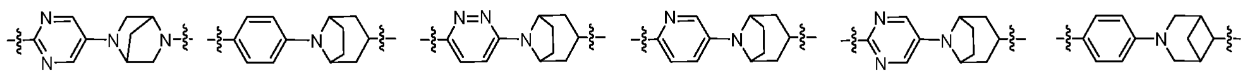

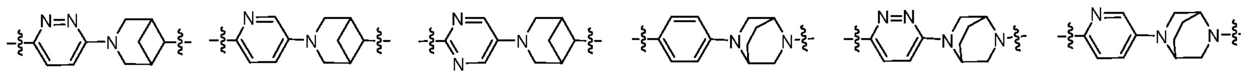

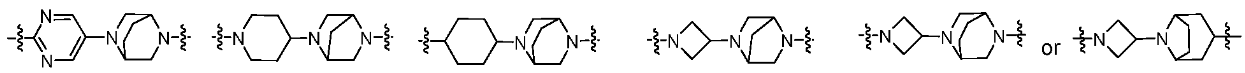

wherein the groups are optionally substituted with one or more substituents selected from the group consisting of deuterium, halogen, cyano, amino, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, deuterated $C_{1-6}$ alkoxy, or any combination thereof.

**27.** The compound of Formula (III) or salts, enantiomers, stereoisomers, solvates, isotopically enriched analogs, prodrugs, or polymorphs thereof as claimed in claim 20, wherein the $R^1$-$W^1$ represents the following groups:

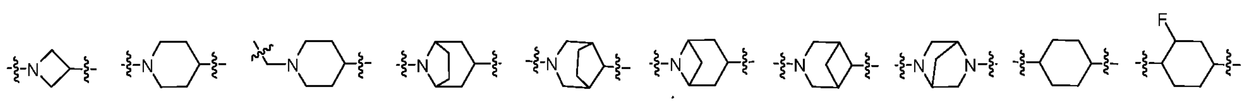

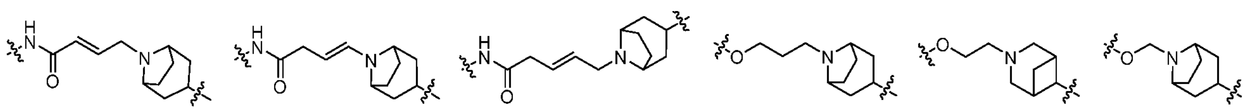

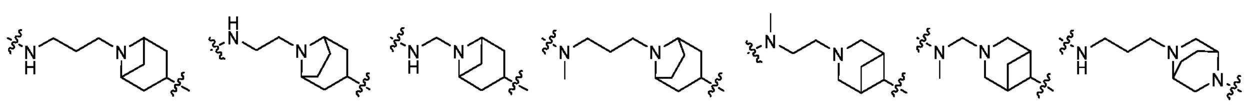

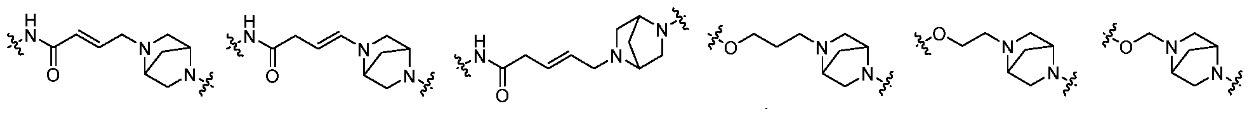

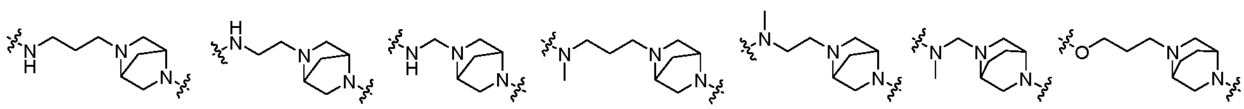

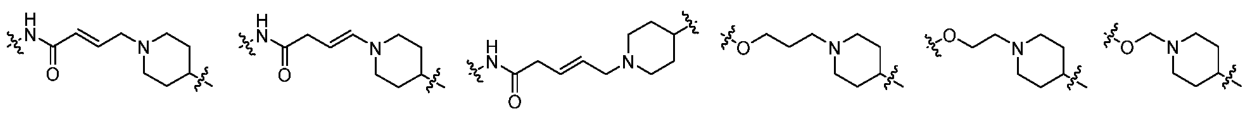

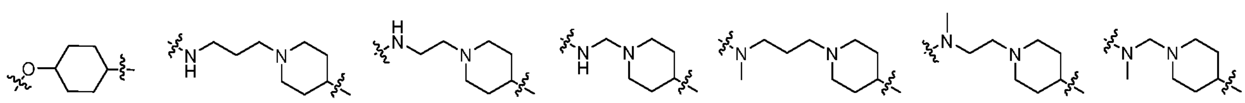

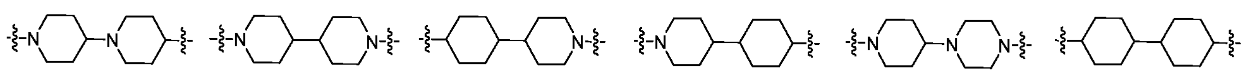

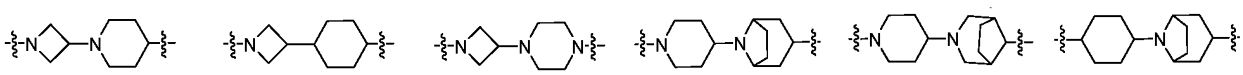

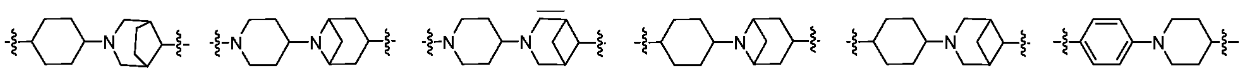

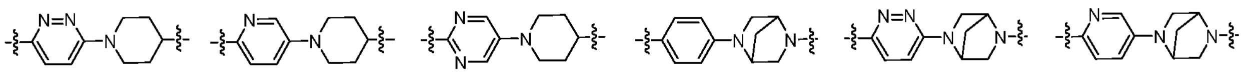

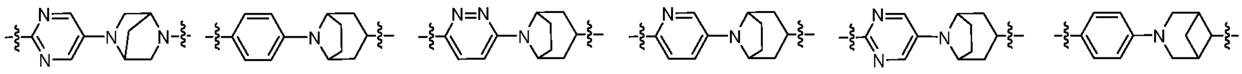

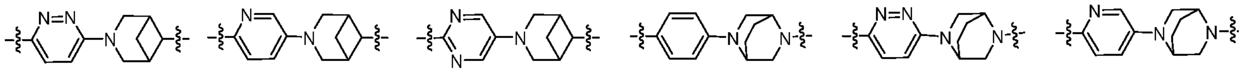

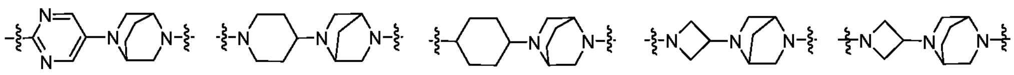 or 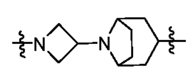,

wherein the groups are optionally substituted with one or more substituents selected from the group consisting of deuterium, halogen, cyano, amino, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, deuterated $C_{1-6}$ alkoxy, or any combination thereof.

**28.** The compound of Formula (III) or salts, enantiomers, stereoisomers, solvates, isotopically enriched analogs, prodrugs, or polymorphs thereof as claimed in claim 20, wherein the LTLM represents the structures of Formula (ULM-1), (LTLM-2), (LTLM-3), (LTLM-4), (ULM-5), (LTLM-6), (ULM-7), (ULM-8), (ULM-9), or (ULM-10):

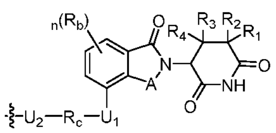
Formula (ULM-1)

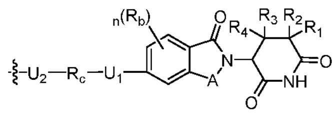
Formula (ULM-2)

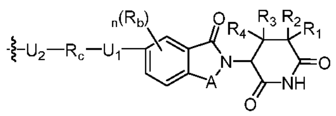
Formula (ULM-3)

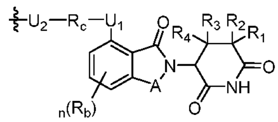
Formula (ULM-4)

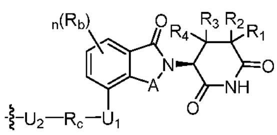
Formula (ULM-5)

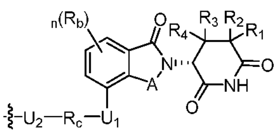
Formula (ULM-6)

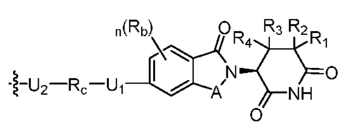
Formula (ULM-7)

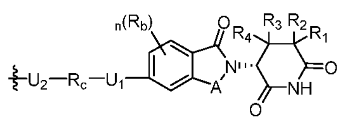
Formula (ULM-8)

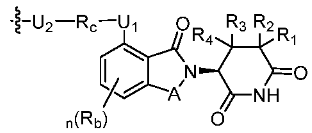
Formula (ULM-9)

or

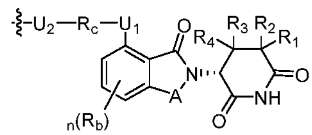
Formula (ULM-10)

;

wherein A represents CO, $CH_2$ or $CD_2$;

$R_1$, $R_2$, $R_3$ and $R_4$ are the same or different and each independently represent hydrogen, deuterium, halogen, optionally deuterated $C_{1-6}$ alkyl, optionally deuterated $C_{1-6}$ alkoxy, or halogenated $C_{1-6}$ alkyl;

$(R_b)_n$ indicates that benzene ring is optionally substituted with n $R_b$ groups, and each $R_b$ are the same or different and each independently represent hydrogen, deuterium, halogen, hydroxy, mercapto, nitro, amino, cyano,

optionally deuterated $C_{1-6}$ alkyl, optionally deuterated $C_{1-6}$ alkoxy, halogenated $C_{1-6}$ alkyl, $C_{2-6}$ alkenyl or $C_{2-6}$ alkynyl;

n represents an integer of 0, 1, 2 or 3;

$U_1$ and $U_2$ are the same or different and each independently represent a bond, $N(R_d)$ or O, wherein $R_d$ represents H or optionally deuterated $C_{1-6}$ alkyl;

$R_c$ represents monocyclic $C_{3-10}$ heterocyclylene group containing one nitrogen atom and optionally a heteroatom selected from nitrogen, oxygen, and sulfur, e.g., piperidinylene, piperazinylene, morpholinylene, azetidinylene, pyrrolidinylene, imidazolidylene, pyrazolidylene, oxazolidinylene, thiazolidinylene, thiomorpholinylene, azepanylene, diazacycloheptanylene, azacyclooctylene or diazacyclooctylene; or

$R_c$ represents $C_{5-20}$ bridged heterocyclylene group containing one nitrogen atom and optionally a heteroatom selected from nitrogen, oxygen, and sulfur, e.g., azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene or diazabicyclo[2.2.2]octanylene, such as the following groups:

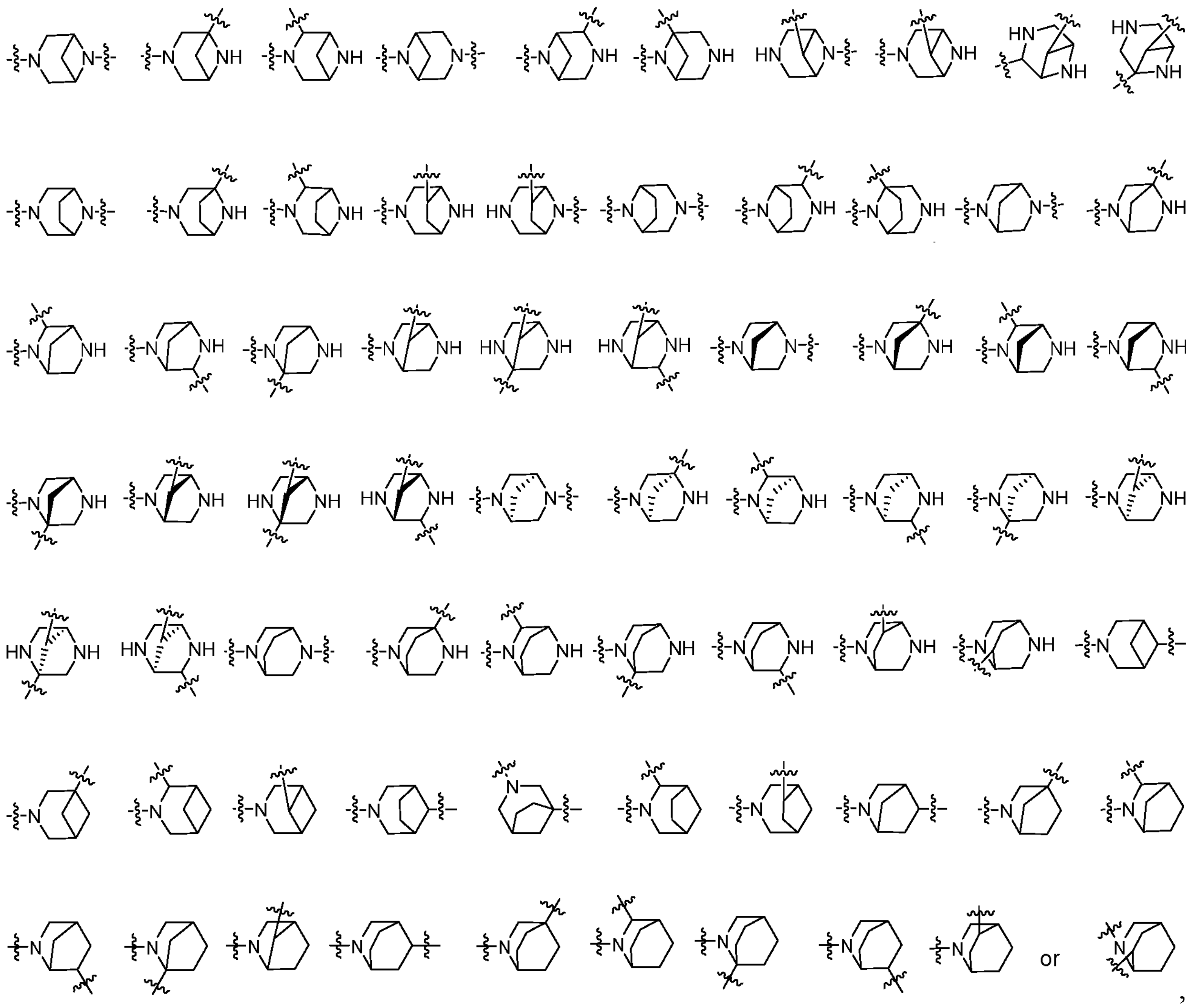

or ,

wherein the monocyclic $C_{3-10}$ heterocyclylene and $C_{5-20}$ bridged heterocyclylene are each independently optionally substituted with one or more substituents selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

**29.** The compound of Formula (III) or salts, enantiomers, stereoisomers, solvates, isotopically enriched analogs, prodrugs, or polymorphs thereof as claimed in claim 28, wherein the $R_c$ represents the following groups:

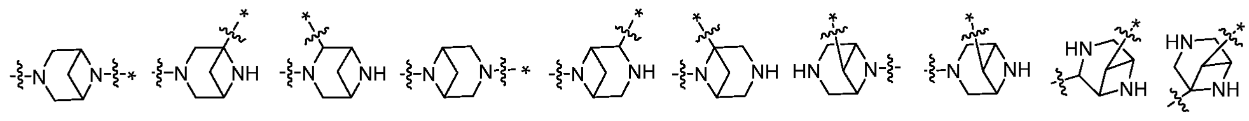

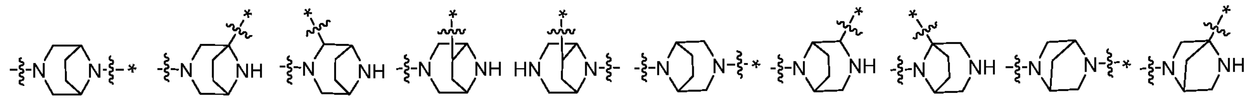

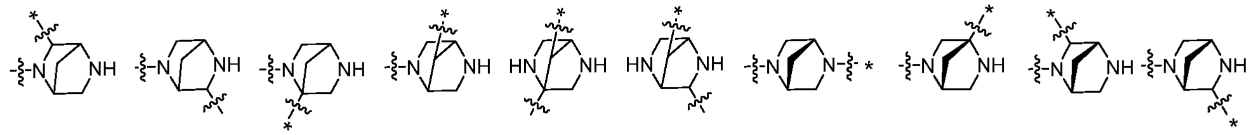

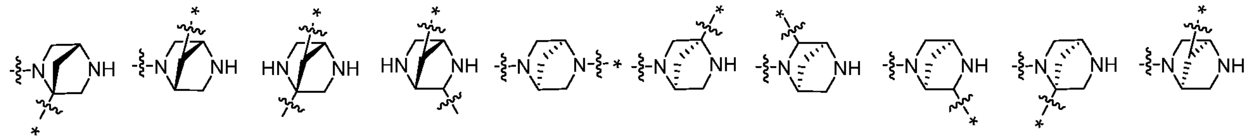

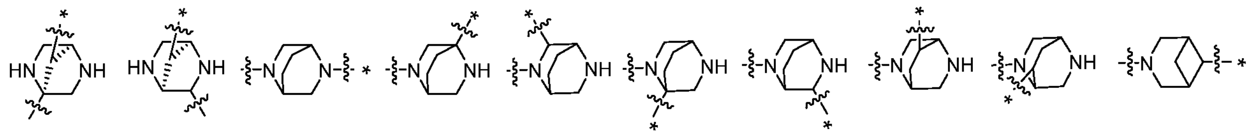

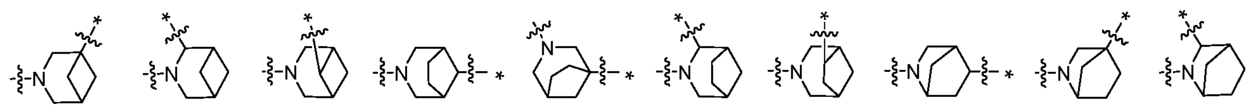

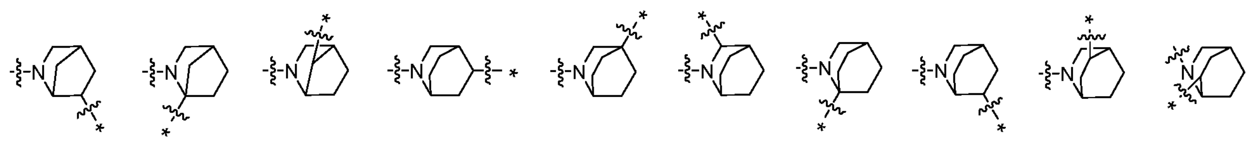

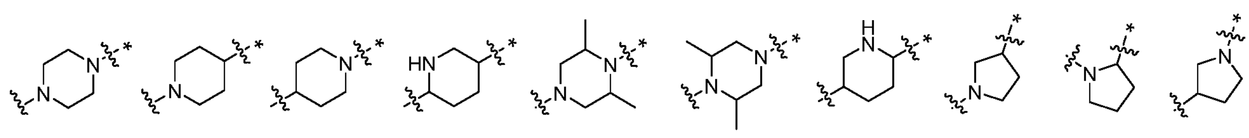

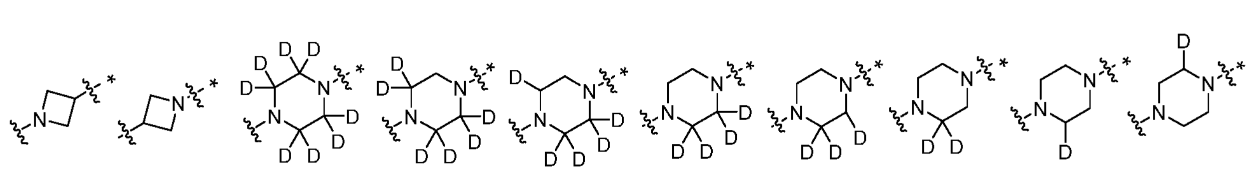

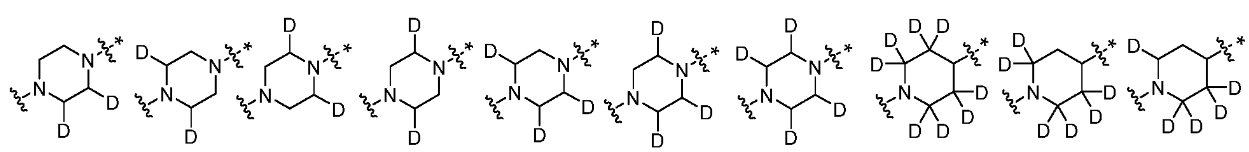

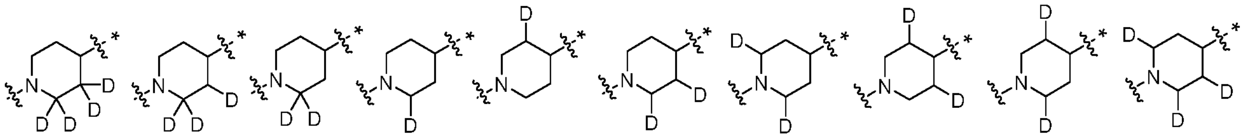

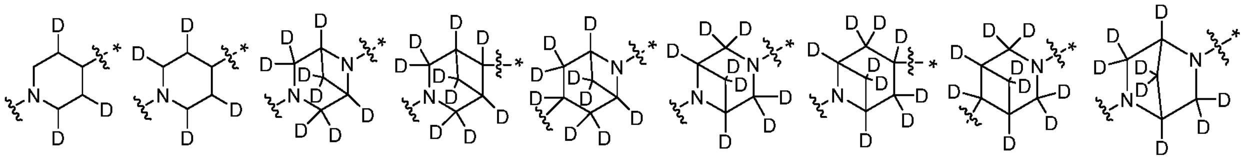

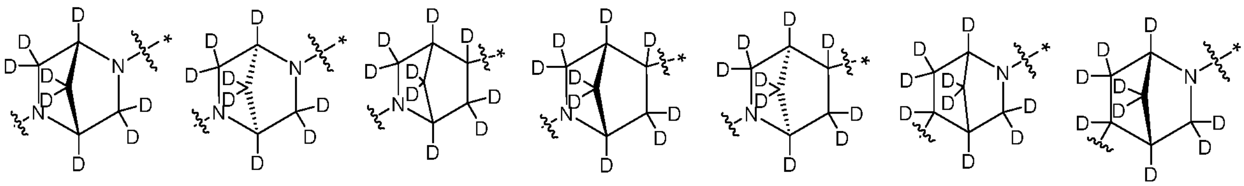

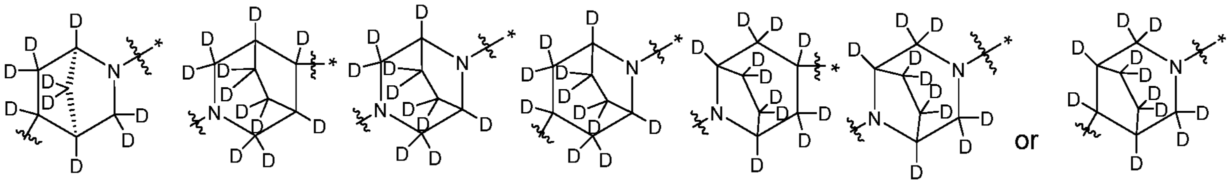
or ;

wherein symbol * indicates the point of attachment to $U_1$.

**30.** The compound of Formula (III) or salts, enantiomers, stereoisomers, solvates, isotopically enriched analogs, prodrugs, or polymorphs thereof as claimed in claim 28, wherein the LTLM represents the structures of Formula (ULM-11), (LTLM-12), (LTLM-13), (LTLM-14), (LTLM-15), (ULM-16), (LTLM-17), (ULM-18) , (ULM-19), (LTLM-20), (ULM-21) or (LTLM-22):

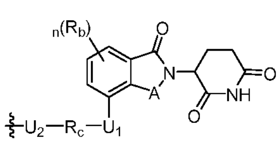
Formula (ULM-11)

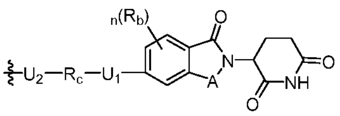
Formula (ULM-12)

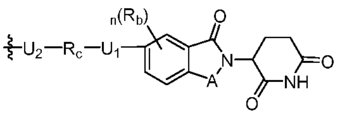
Formula (ULM-13)

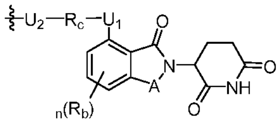
Formula (ULM-14)

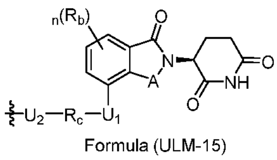
Formula (ULM-15)

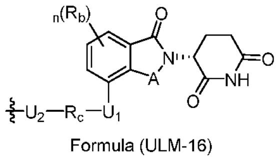
Formula (ULM-16)

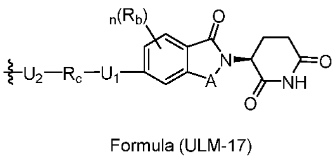
Formula (ULM-17)

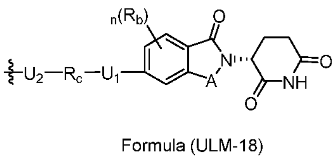
Formula (ULM-18)

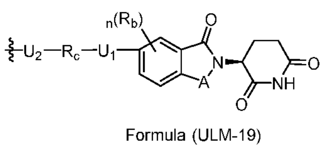
Formula (ULM-19)

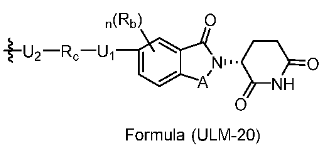
Formula (ULM-20)

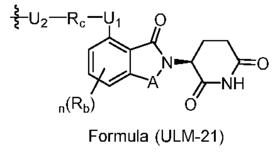
Formula (ULM-21)

or

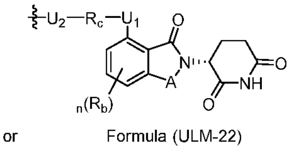
Formula (ULM-22) ;

wherein A, $(R_b)_n$, $R_b$, $R_c$, $U_1$, and $U_2$ are as defined in claim 27.

**31.** The compound of Formula (III) or salts, enantiomers, stereoisomers, solvates, isotopically enriched analogs, prodrugs, or polymorphs thereof as claimed in claim 20, wherein the LTLM represents the structure of the following formula:

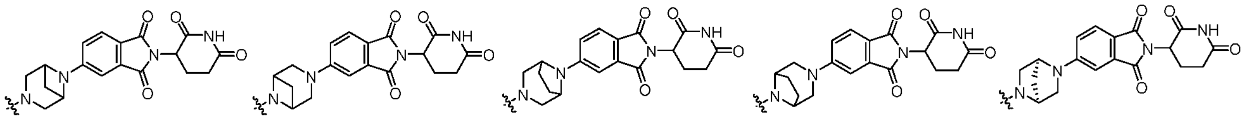

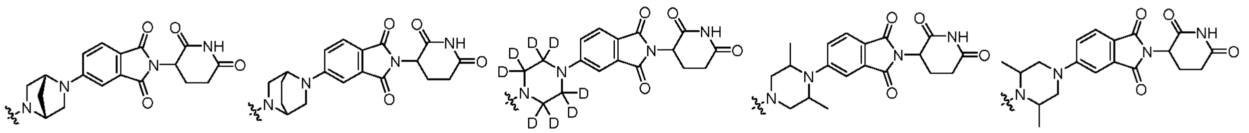

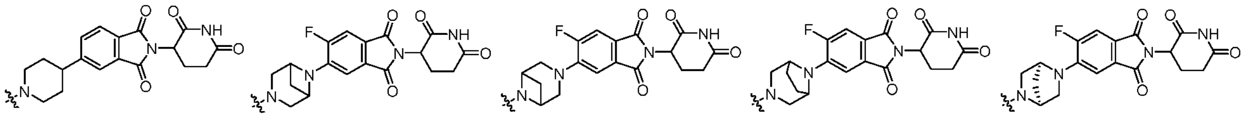

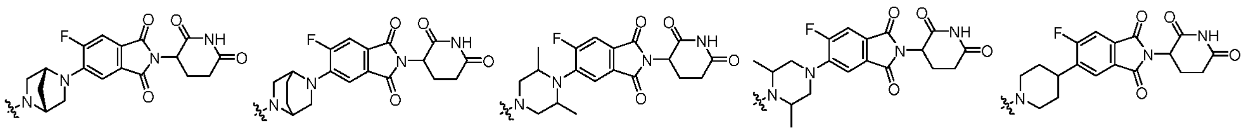

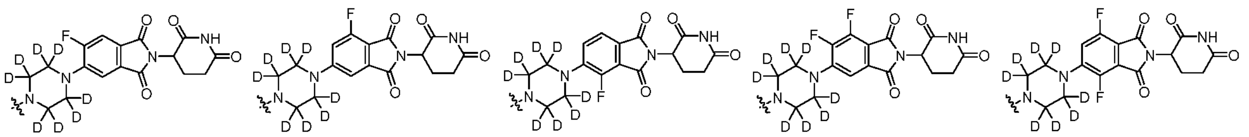

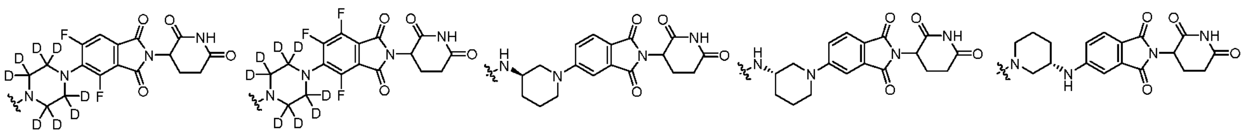

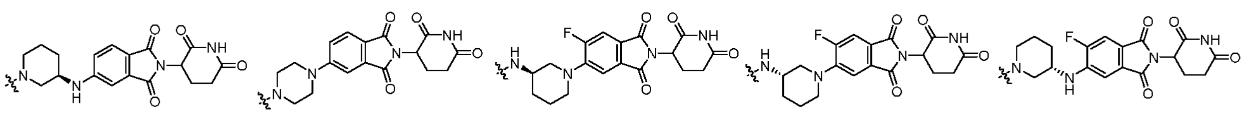

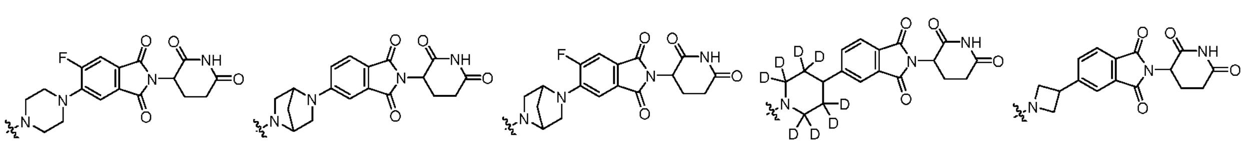

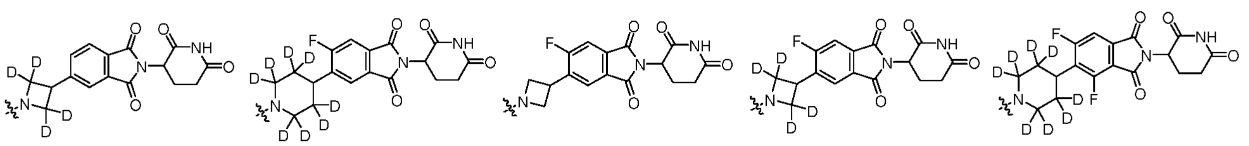

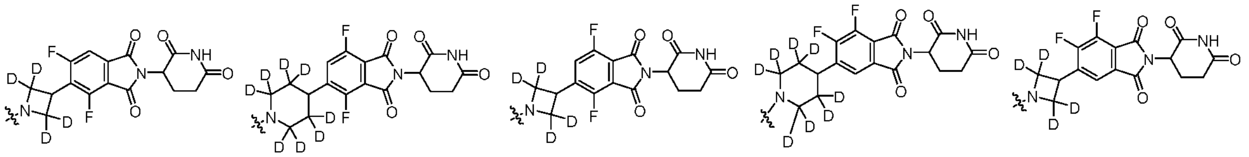

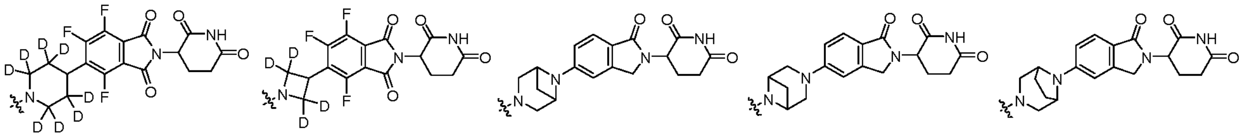

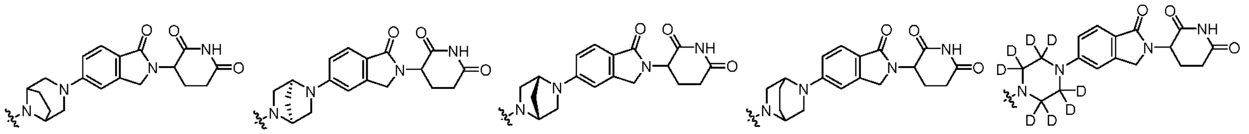

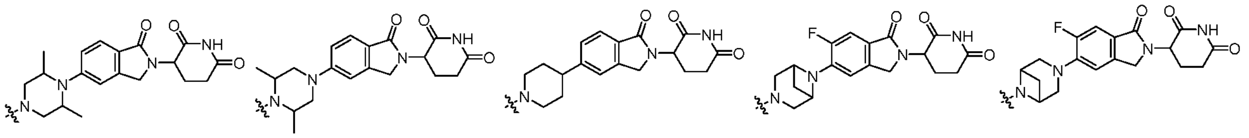

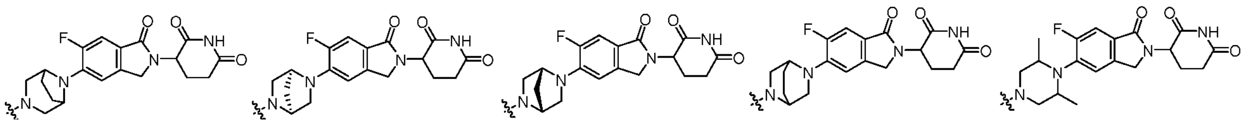

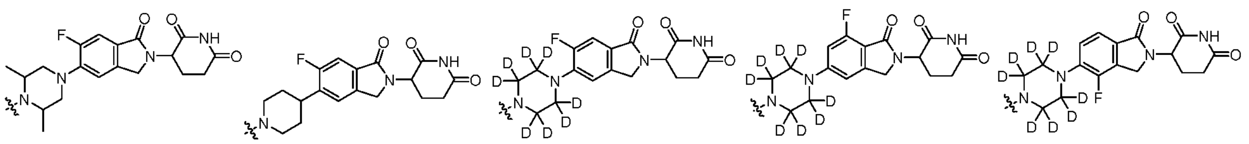

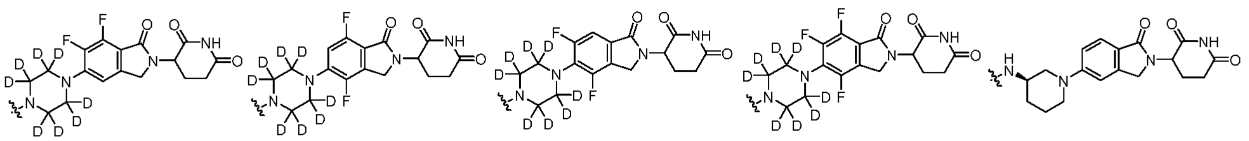

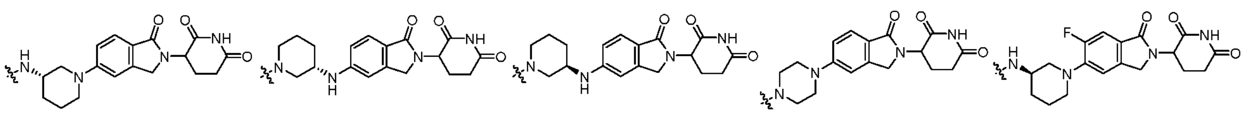

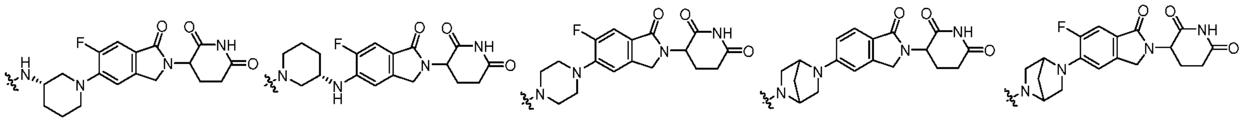

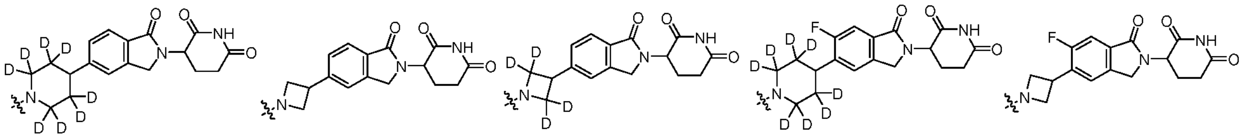

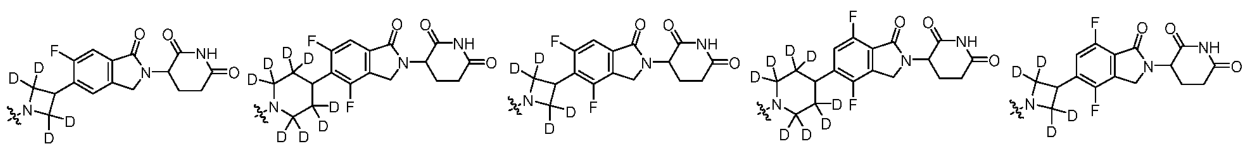

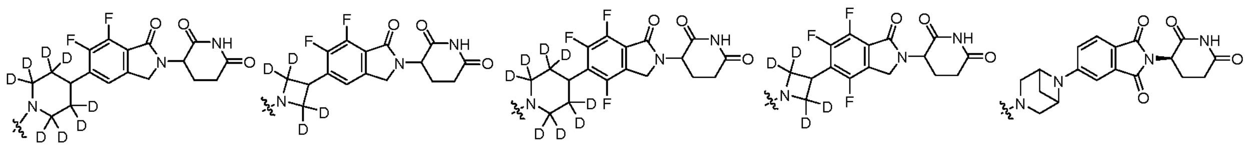

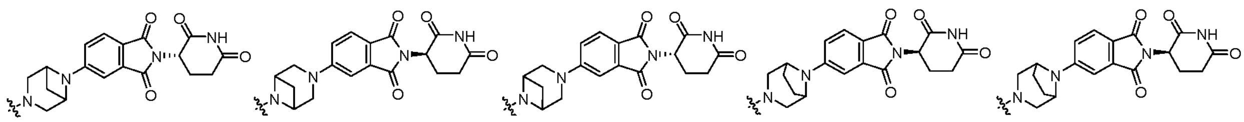

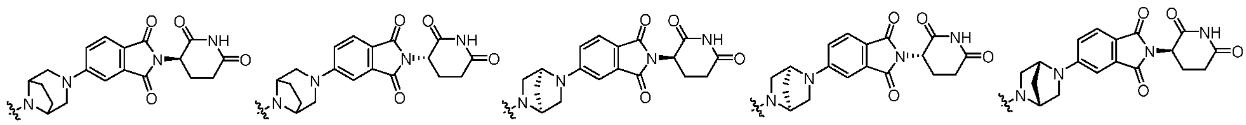

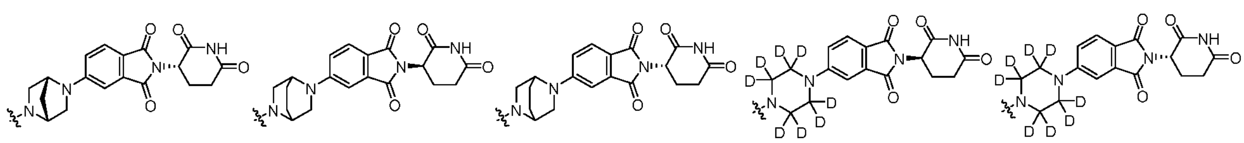

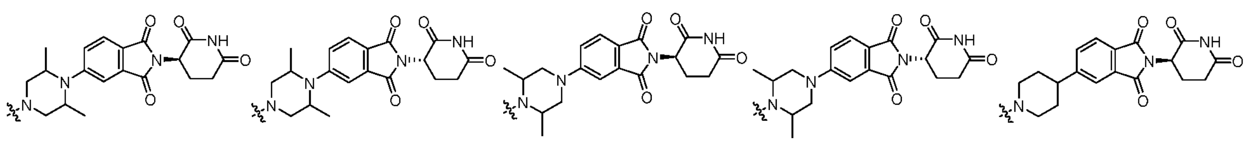

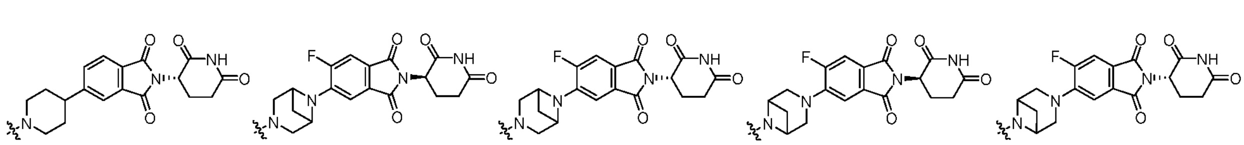

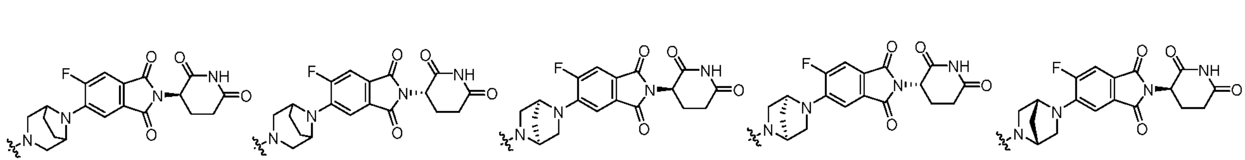

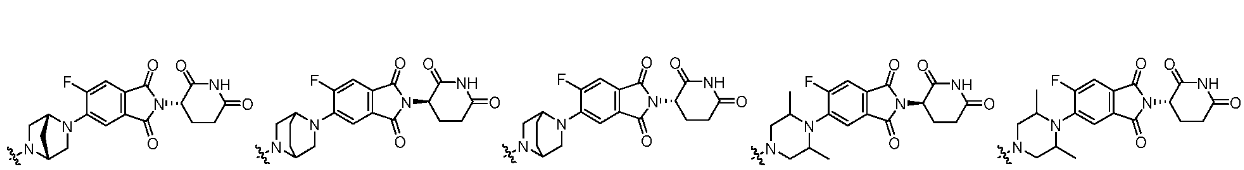

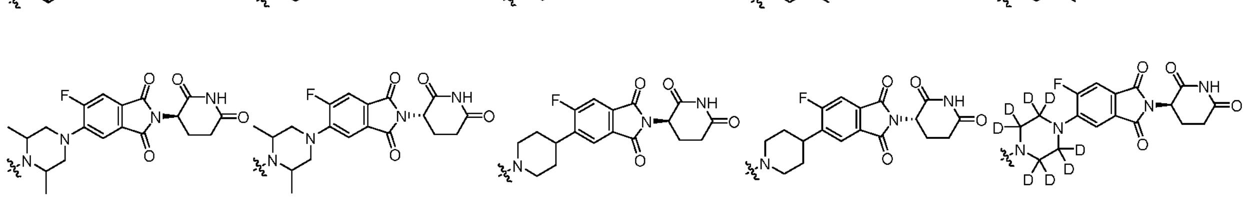

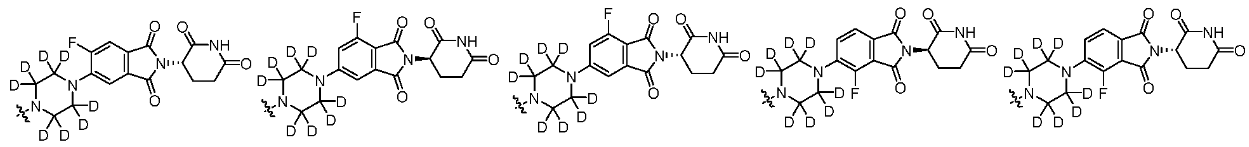

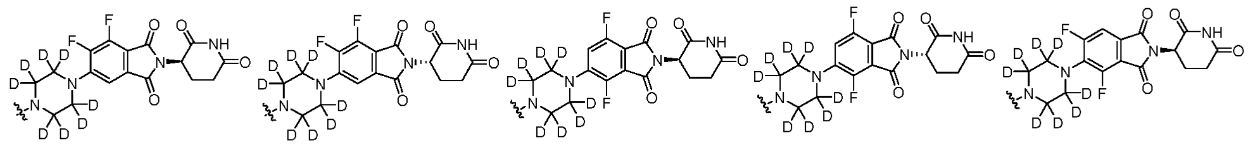

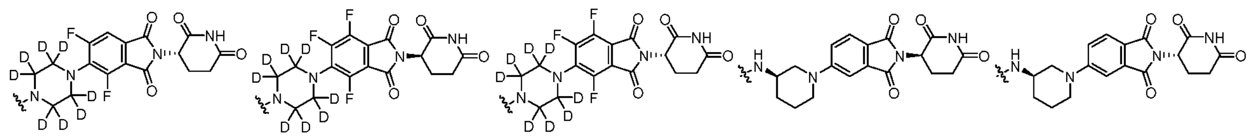

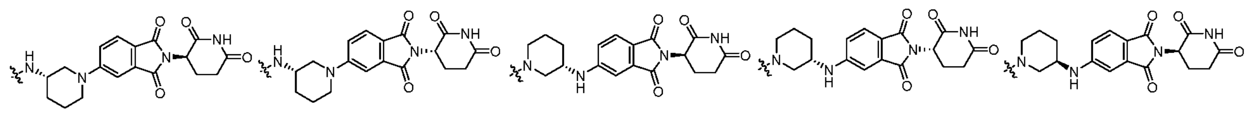

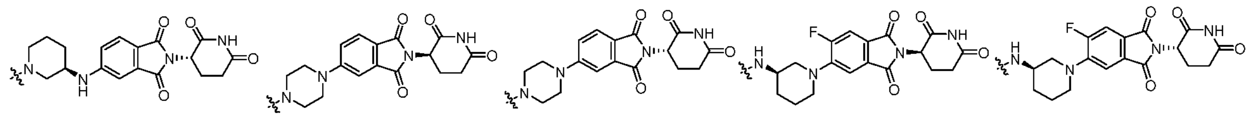

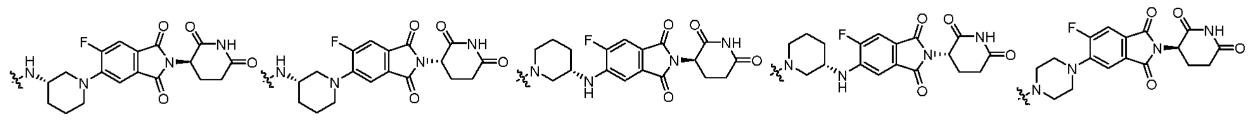

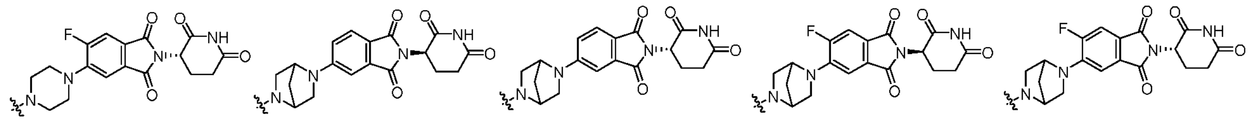

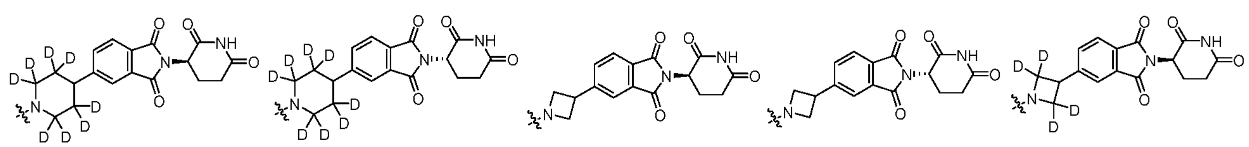

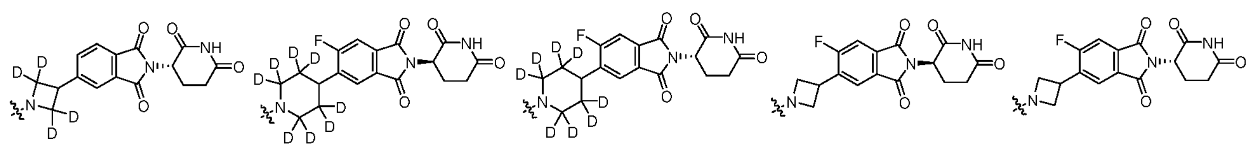

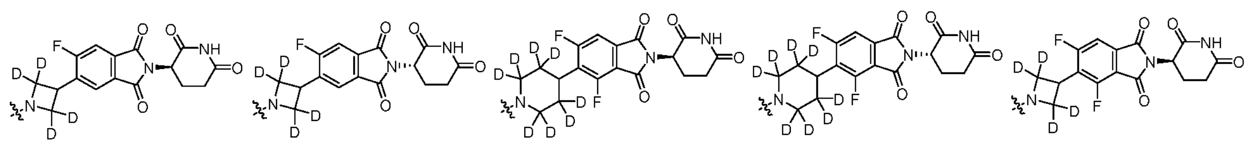

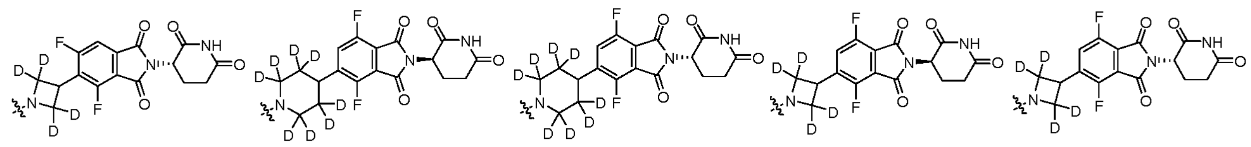

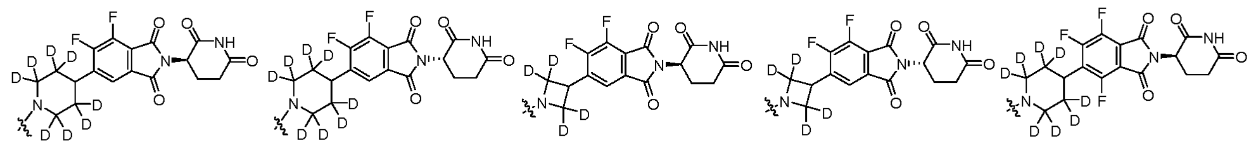

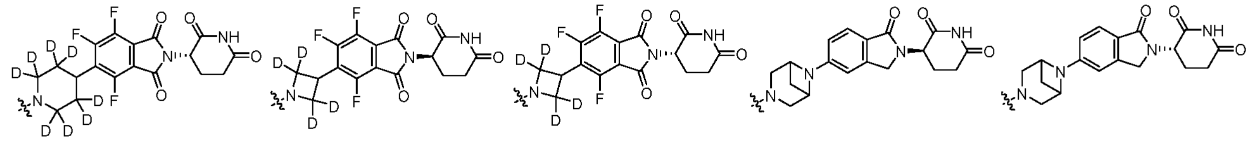

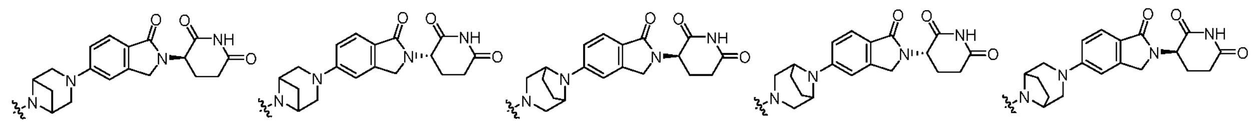

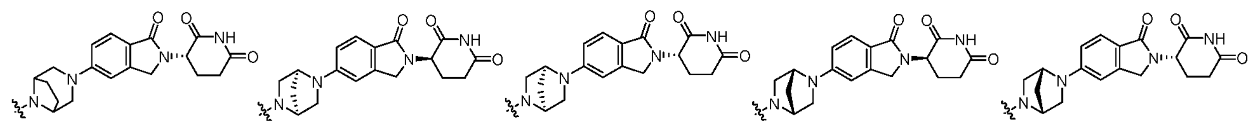

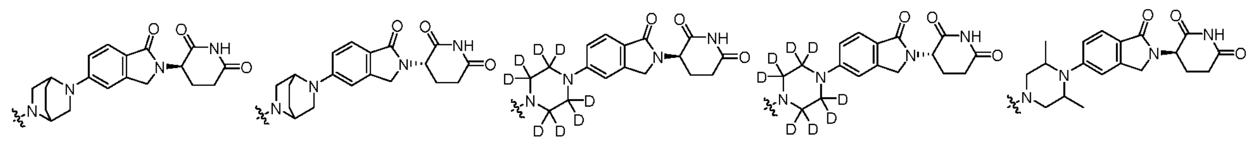

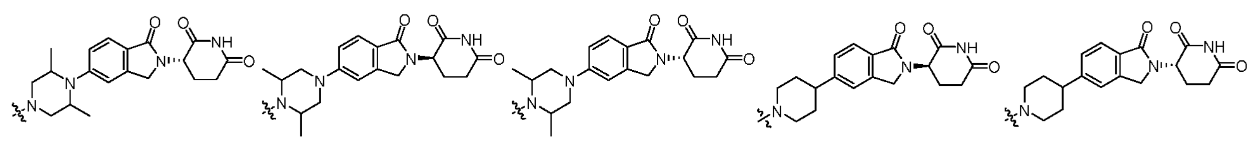

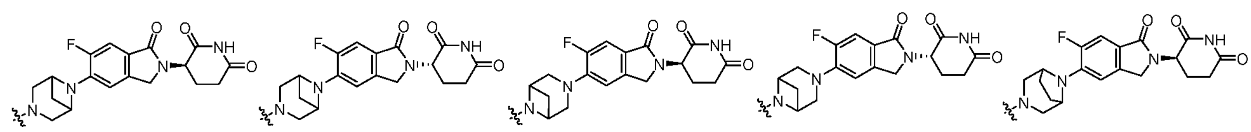

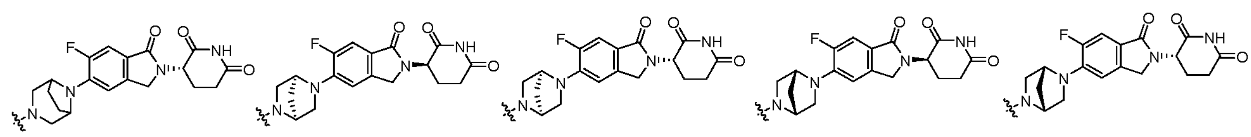

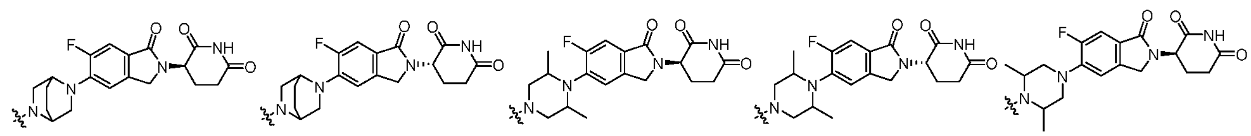

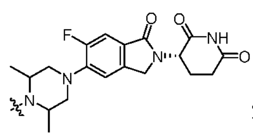
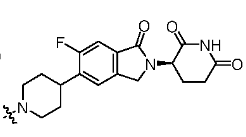
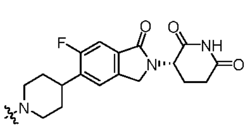
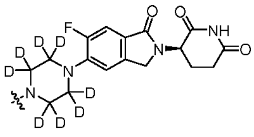
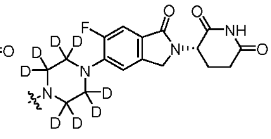
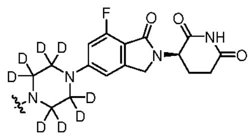
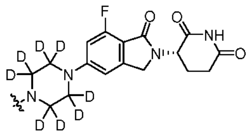
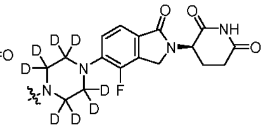
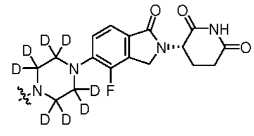
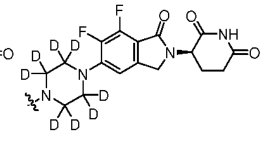
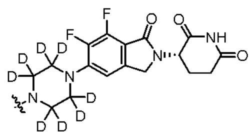
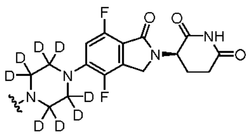
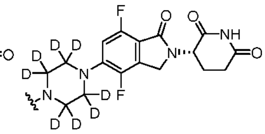
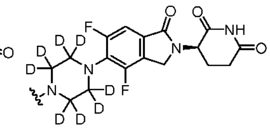
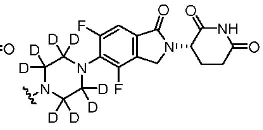
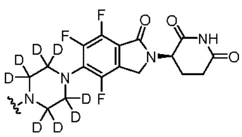
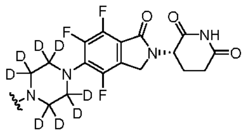
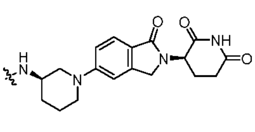
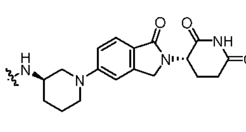
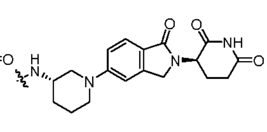
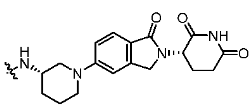
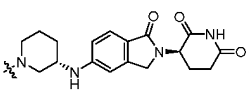
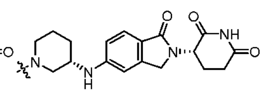
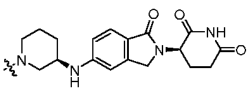
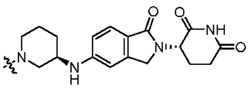
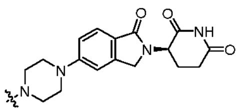
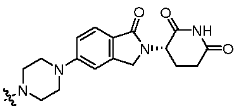
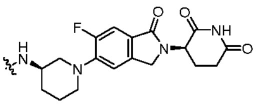
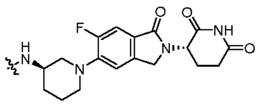
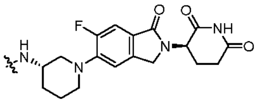
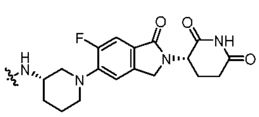
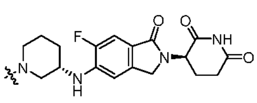
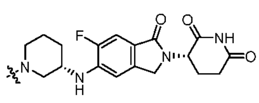
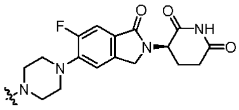
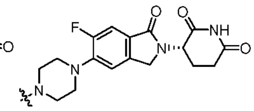
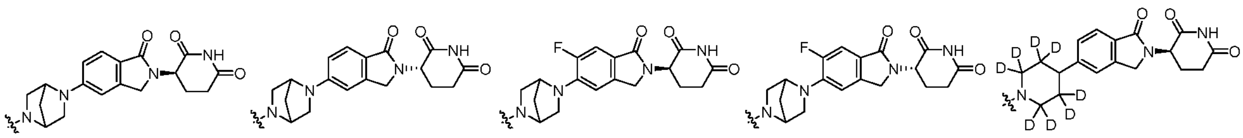
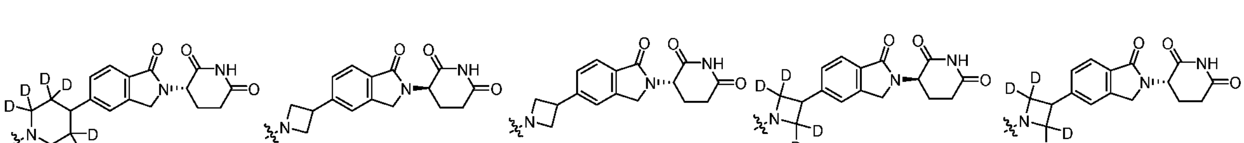
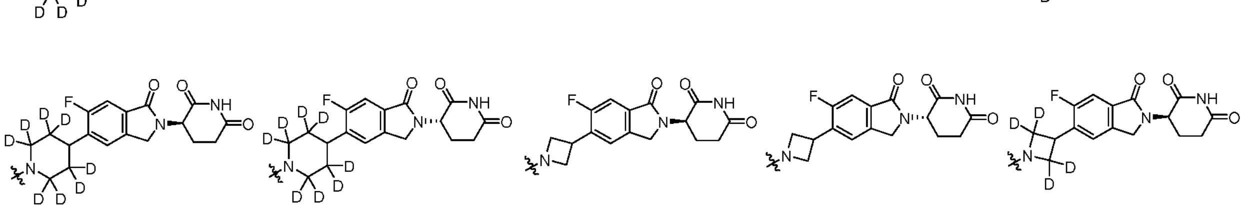

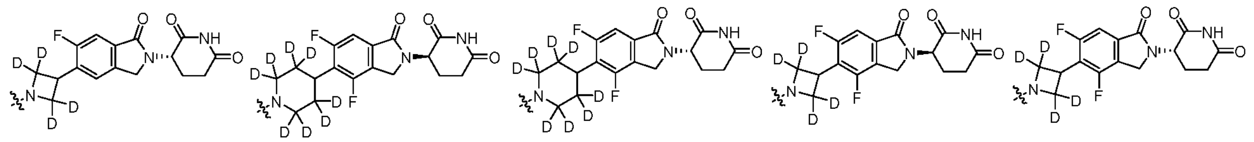

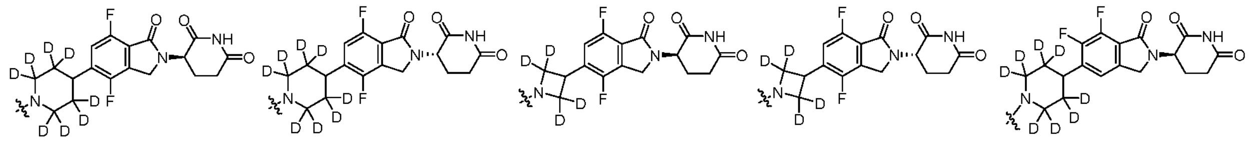

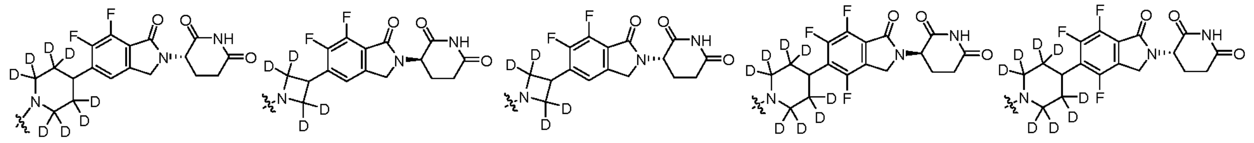

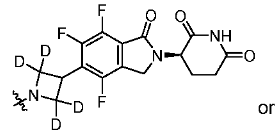 or 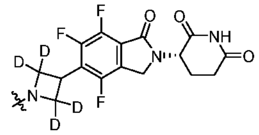.

**32.** The compound of Formula (III) or salts, enantiomers, stereoisomers, solvates, isotopically enriched analogs, prodrugs, or polymorphs thereof as claimed in any one of claims 20-31, wherein the LIN represents the structures of the following formula:

#-$C_{1-3}$o alkylene-;
#-$(C(R^{a1})(R^{a2}))_{n1}$-$(R_e(C(R^{a3})(R^{a4}))_{n2})_{m1}$-;
#-$(C(R^{a1})(R^{a2}))_{n1}$-$(R_e(C(R^{a3})(R^{a4}))_{n2})_{m1}$-$(R_e(C(R^{a5})(R^{a6}))_{n3})_{m2}$-;
#-$(C(R^{a1})(R^{a2}))_{n1}$-$(R_e(C(R^{a3})(R^{a4}))_{n2})_{m}$-$(R_e(C(R^{a5})(R^{a6}))_{n3})_{m2}$-$(R_e(C(R^{a7})(R^{a8}))_{n4})_{m3}$-;
#-$(C(R^{a1})(R^{a2}))_{n1}$-$(R_f(C(R^{a3})(R^{a4}))_{n2})_{m1}$-;
#-$(C(R^{a1})(R^{a2}))_{n1}$-$(R_f(C(R^{a3})(R^{a4}))_{n2})_{m1}$-$(R_f(C(R^{a5})(R^{a6}))_{n3})_{m2}$-;
#-$(C(R^{a1})(R^{a2}))_{n1}$-$(R_f(C(R^{a3})(R^{a4}))_{n2})_{m1}$-$(R_f(C(R^{a5})(R^{a6}))_{n3})_{m2}$-$(R_f(C(R^{a7})(R^{a8}))_{n4})_{m3}$-;
#-$(C(R^{a1})(R^{a2}))_{n1}$-$(R_e$-$R_f$-$(C(R^{a3})(R^{a4}))_{n2})_{m1}$-;
#-$(C(R^{a1})(R^{a2}))_{n1}$-$(R_e(C(R^{a3})(R^{a4}))_{n2})_{m1}$-$(R_f(C(R^{a5})(R^{a6}))_{n3})_{m2}$-;
#-$(C(R^{a1})(R^{a2}))_{n1}$-$(R_f$-$R_e$-$(C(R^{a3})(R^{a4}))_{n2})_{m1}$-; or
#-$(C(R^{a1})(Ra2))_{n1}$-$(R_f(C(R^{a3})(R^{a4}))_{n2})_{m1}$-$(R_e(C(R^{a5})(R^{a6}))_{n3})_{m2}$-;

wherein each $R_e$ are selected from the group consisting of O, $N(R_g)$, C(O), C(O)O, OC(O), S(O), $S(O)_2$, $S(O)_2NH$, $NHS(O)_2$, $C(O)N(R_g)$, $N(R_g)C(O)$, or $N(R_g)C(O)N(R_g)$, where each $R_g$ independently represent H or $C_{1-6}$ alkyl;

wherein each $R_f$ are selected from the group consisting of cycloalkylene, arylene, heterocyclylene, heteroarylene, alkynylene, alkenylene, or any combination thereof, wherein the cycloalkylene, arylene, heterocyclylene, and heteroarylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano, or any combination thereof;

a hydrogen atom of one or more $CH_2$ groups of the $C_{1-30}$ alkylene group is optionally replaced with a substituent selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, or any combination thereof;

$R^{a1}$, $R^{a2}$, $R^{a3}$, $R^{a4}$, $R^{a5}$, $R^{a6}$, $R^{a7}$, and $R^{a8}$ each independently represent H, deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, or any combination thereof;

symbol # indicates the point of attachment to $U_2$; and

n1, n2, n3, n4, m1, m2, and m3 each independently represent an integer of 1, 2, 3, 4, 5, 6, 7, 8, 9, 10, 11, 12, 13, 14, or 15.

**33.** The compound of Formula (III) or salts, enantiomers, stereoisomers, solvates, isotopically enriched analogs, prodrugs, or polymorphs thereof as claimed in any one of claims 20-31, wherein LIN represents the structure of the following formula:

$\#\text{-}(C(R^{a1})(R^{a2}))_{n1}\text{-}(O(C(R^{a3})(R^{a4}))_{n2})_{m1}\text{-};$

$\#\text{-}(C({}_R{}^{a1})(R^{a2}))_{n1}\text{-}(O(C(R^{a3})(R^{a4}))_{n2})_{m1}\text{-}(O(C({}_R{}^{a5})({}_R{}^{a6}))_{n3})_{m2}\text{-};$

$\#\text{-}(C(R^{a1})(R^{a2}))_{n1}\text{-}(O(C(R^{a3})(R^{a4}))_{n2})_{m1}\text{-}(O(C(R^{a5})(R^{a6}))_{n3})_{m2}\text{-}(O(C(R^{a7})(R^{a8}))_{n4})_{m3}\text{-};$

$\#\text{-}(C(R^{a1})(R^{a2}))_{n1}\text{-}(N(R_g)(C(R^{a3})(R^{a4}))_{n2})_{m1}\text{-};$

$\#\text{-}(C(R^{a1})(R^{a2}))_{n1}\text{-}(N(R_g)(C(R^{a3})(R^{a4}))_{n2})_{m1}\text{-}(N(R_g)(C(R^{a5})(R^{a6}))_{n3})_{m2}\text{-};$

$\#\text{-}(C(R^{a1})(R^{a2}))_{n1}\text{-}(N(R_g)(C(R^{a3})(R^{a4}))_{n2})_{m1}\text{-}(N(R_g)(C(R^{a5})(R^{a6}))_{n3})_{m2}\text{-}(N(R_g)(C(R^{a7})(R^{a8}))_{n4})_{m3}\text{-};$

$\#\text{-}(C(R^{a1})(R^{a2}))_{n1}\text{-}(C(O)N(R_g)\text{-}(C(R^{a3})(R^{a4}))_{n2})_{m1}\text{-};$

$\#\text{-}(C(R^{a1})(R^{a2}))_{n1}\text{-}(C(O)N(R_g)\text{-}(C(R^{a3})(R^{a4}))_{n2})_{m1}\text{-}(C(O)N(R_g)\text{-}(C(R^{a5})(R^{a6}))_{n3})_{m2}\text{-};$

$\#\text{-}(C(R^{a1})(R^{a2}))_{n1}\text{-}(C(O)N(R_g)\text{-}(C(R^{a3})(R^{a4}))_{n2})_{m1}\text{-}(C(O)N(R_g)\text{-}(C(R^{a5})(R^{a6}))_{n3})_{m2}\text{-}(C(O)N(R_g)\text{-}(C(R^{a7})(R^{a8}))_{n4})_{m3}\text{-};$

$\#\text{-}(C(R^{a1})(R^{a2}))_{n1}\text{-}(C(O)N(R_g)\text{-}(C(R^{a3})(R^{a4}))_{n2})_{m1}\text{-}(O\text{-}(C(R^{a5})(R^{a6}))_{n3})_{m2}\text{-};$

$\#\text{-}(C(R^{a1})(R^{a2}))_{n1}\text{-}C(O)N(R_g)\text{-}(C(R^{a3})(R^{a4}))_{n2}\text{-}(O(C(R^{a5})(R^{a6}))_{n3})_{m1}\text{-};$

$\#\text{-}(C(R^{a1})(R^{a2}))_{n1}\text{-}(N(R_g)C(O)\text{-}(C(R^{a3})(R^{a4}))_{n2})_{m1}\text{-};$

$\#\text{-}(C(R^{a1})(R^{a2}))_{n1}\text{-}(N(R_g)C(O)\text{-}(C(R^{a3})(R^{a4}))_{n2})_{m1}\text{-}(O\text{-}(C(R^{a5})(R^{a6}))_{n3})_{m2}\text{-};$

$\#\text{-}(C(R^{a1})(R^{a2}))_{n1}\text{-}(N(R_g)C(O)\text{-}(C(R^{a3})(R^{a4}))_{n2})_{m1}\text{-}(O\text{-}(C(R^{a5})(R^{a6}))_{n3})_{m2}\text{-}(O\text{-}(C(R_g)(R^{a8}))_{n4})_{m3}\text{-};$

$\#\text{-}(C(R^{a1})(R^{a2}))_{n1}\text{-}N(R_g)C(O)\text{-}(C(R^{a3})(R^{a4}))_{n2}\text{-}(O(C(R^{a5})(R^{a6}))_{n3})_{m1}\text{-};$

$\#\text{-}(C(R^{a1})(R^{a2}))_{n1}\text{-}N(R_g)C(O)N(R_g)\text{-}(C(R^{a3})(R^{a4}))_{n2}\text{-};$

$\#\text{-}(C(R^{a1})(R^{a2}))_{n1}\text{-}C(O)\text{-}(C(R^{a3})(R^{a4}))_{n2}\text{-};$

$\#\text{-}(C(R^{a1})(Ra^{2}))_{n1}\text{-}(\text{arylene-}(C(R^{a3})(R^{a4}))_{n2})_{m1}\text{-};$

$\#\text{-}(C(R^{a1})(R^{a2})_{n1}\text{-}(\text{arylene-}(C(R^{a3})(R^{a4}))_{n2})_{m1}\text{-arylene-}(C(R^{a5})(R^{a6}))_{n3}\text{-};$

$\#\text{-}(C(R^{a1})(R^{a2}))_{n1}\text{-}(\text{heterocyclylene-}(C(R^{a3})(R^{a4}))_{n2})_{m1}\text{-};$

$\#\text{-}(C(R^{a1})(R^{a2}))_{n1}\text{-}(\text{heterocyclylene-}(C(R^{a3})(R^{a4}))_{n2})_{m1}\text{-}(\text{heterocyclylene-}(C(R^{a5})(R^{a6}))_{n3})_{m2}\text{-};$

$\#\text{-}(C(R^{a1})(R^{a2}))_{n1}\text{-}(\text{heteroarylene-}(C(R^{a3})(R^{a4}))_{n2})_{m1}\text{-};$

$\#\text{-}(C(R^{a1})(R^{a2}))_{n1}\text{-}(\text{heteroarylene-}(C(R^{a3})(R^{a4}))_{n2})_{m1}\text{-}(\text{heteroarylene-}(C(R^{a5})(R^{a6}))_{n3})_{m2}\text{-};$

$\#\text{-}(C(R^{a1})(R^{a2}))_{n1}\text{-}(\text{cycloalkylene-}(C(R^{a3})(R^{a4}))_{n2})_{m1}\text{-};$ or

$\#\text{-}(C(R^{a1})(R^{a2}))_{n1}\text{-}(\text{cycloalkylene-}(C(R^{a3})(R^{a4}))_{n2})_{m1}\text{-}(\text{cycloalkylene-}(C(R^{a5})(R^{a6}))_{n3})_{m2}\text{-};$

wherein each $R_g$ independently represent H or $C_{1-6}$ alkyl;
the cycloalkylene, arylene, heterocyclylene, and heteroarylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano, or any combination thereof;
$R^{a1}$, $R^{a2}$, $R^{a3}$, $R^{a4}$, $R^{a5}$, $R^{a6}$, $R^{a7}$ and $R^{a8}$ each independently represent H, deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, or any combination thereof;
symbol # indicates the point of attachment to $U_2$; and
n1, n2, n3, n4, m1, m2, and m3 each independently represent an integer of 1, 2, 3, 4, 5, 6, 7, 8, 9, 10, 11, 12, 13, 14, or 15.

**34.** The compound of Formula (III) or salts, enantiomers, stereoisomers, solvates, isotopically enriched analogs, prodrugs, or polymorphs thereof as claimed in any one of claims 20-31, wherein LIN represents the following groups:

$\#\text{-}CH_2\text{-}$, $\#\text{-}(CH_2)_2\text{-}$, $\#\text{-}(CH_2)_3\text{-}$, $\#\text{-}(CH_2)_4\text{-}$, $\#\text{-}(CH_2)_5\text{-}$, $\#\text{-}(CH_2)_6\text{-}$, $\#\text{-}(CH_2)_7\text{-}$, $\#\text{-}(CH_2)_8\text{-}$, $\#\text{-}(CH_2)_9\text{-}$, $\#\text{-}(CH_2)_{10}\text{-}$, $\#\text{-}(CH_2)_{11}\text{-}$, $\#\text{-}(CH_2)_{12}\text{-}$, $\#\text{-}(CH_2)_{13}\text{-}$, $\#\text{-}(CH_2)_{14}\text{-}$, $\#\text{-}(CH_2)_{15}\text{-}$, $\#\text{-}(CH_2)_{16}\text{-}$, $\#\text{-}(CH_2)_{17}\text{-}$, $\#\text{-}(CH_2)_{18}\text{-}$, $\#\text{-}(CH_2)_{19}\text{-}$, $\#\text{-}(CH_2)_{20}\text{-}$, $\#\text{-}(CH_2)_{21}\text{-}$, $\#\text{-}(CH_2)_{22}\text{-}$, $\#\text{-}(CH_2)_{25}\text{-}$, or $\#\text{-}(CH_2)_{30}\text{-}$; $\#\text{-}CH_2\text{-}O\text{-}CH_2\text{-}$, $\#\text{-}CH_2\text{-}O\text{-}(CH_2)_2\text{-}$, $\#\text{-}(CH_2)_1\text{-}O\text{-}(CH_2)_3\text{-}$, $\#\text{-}(CH_2)_1\text{-}O\text{-}(CH_2)_4\text{-}$, $\#\text{-}(CH_2)_1\text{-}O\text{-}(CH_2)_5\text{-}$, $\#\text{-}(CH_2)_1\text{-}O\text{-}(CH_2)_6\text{-}$, $\#\text{-}(CH_2)_1\text{-}O\text{-}(CH_2)_7\text{-}$, $\#\text{-}(CH_2)_1\text{-}O\text{-}(CH_2)_8\text{-}$, $\#\text{-}(CH_2)_1\text{-}O\text{-}(CH_2)_9\text{-}$, $\#\text{-}(CH_2)_1\text{-}O\text{-}(CH_2)_{10}\text{-}$, $\#\text{-}(CH_2)_2\text{-}O\text{-}(CH_2)_1\text{-}$, $\#\text{-}(CH_2)_2\text{-}O\text{-}(CH_2)_2\text{-}$,

#-$(CH_2)_2$-O-$(CH_2)_3$-, #-$(CH_2)_2$-O-$(CH_2)_4$-, #-$(CH_2)_2$-O-$(CH_2)_5$-, #-$(CH_2)_2$-O-$(CH_2)_6$-, #-$(CH_2)_2$-O-$(CH_2)_7$-, #-$(CH_2)_2$-O-$(CH_2)_8$-, #-$(CH_2)_2$-O-$(CH_2)_9$-, #-$(CH_2)_2$-O-$(CH_2)_{10}$-, #-$(CH_2)_2$-O-$(CH_2)_{11}$-, #-$(CH_2)_2$-O-$(CH_2)_{12}$-, #-$(CH_2)_3$-O-$(CH_2)_1$-, #-$(CH_2)_3$-O-$(CH_2)_2$-, #-$(CH_2)_3$-O-$(CH_2)_3$-, #-$(CH_2)_3$-O-$(CH_2)_4$-, #-$(CH_2)_3$-O-$(CH_2)_5$-, #-$(CH_2)_3$-O-$(CH_2)_6$-, #-$(CH_2)_3$-O-$(CH_2)_7$-, #-$(CH_2)_4$-O-$(CH_2)_1$-, #-$(CH_2)_4$-O-$(CH_2)_2$-, #-$(CH_2)_4$-O-$(CH_2)_3$-, #-$(CH_2)_4$-O-$(CH_2)_4$-, #-$(CH_2)_4$-O-$(CH_2)_5$-, #-$(CH_2)_4$-O-$(CH_2)_6$-, #-$(CH_2)_5$-O-$(CH_2)_1$-, #-$(CH_2)_5$-O-$(CH_2)_2$-, #-$(CH_2)_5$-O-$(CH_2)_3$-, #-$(CH_2)_5$-O-$(CH_2)_4$-, #-$(CH_2)_5$-O-$(CH_2)_5$-, #-$(CH_2)_6$-O-$(CH_2)_1$-, #-$(CH_2)_6$-O-$(CH_2)_2$-, #-$(CH_2)_6$-O-$(CH_2)_3$-, #-$(CH_2)_6$-O-$(CH_2)_4$-, #-$(CH_2)_7$-O-$(CH_2)_1$-, #-$(CH_2)_7$-O-$(CH_2)_2$-, #-$(CH_2)_7$-O-$(CH_2)_3$-, #-$(CH_2)_8$-O-$(CH_2)_1$-, #-$(CH_2)_8$-O-$(CH_2)_2$-, #-$CH(CH_3)$-O-$(CH_2)_1$-, #-$CH(CH_3)$-O-$(CH_2)_2$-, #-$CH(CH_3)$-O-$(CH_2)_3$-, #-$CH(CH_3)$-O-$(CH_2)_4$-, #-$CH(CH_3)$-O-$(CH_2)_5$-, #-$CH(CH_3)$-O-$(CH_2)_6$-, #-$CH(CH_3)$-O-$(CH_2)_7$-, #-$CH(CH_3)$-O-$(CH_2)_8$-, #-$CH(CH_3)$-O-$(CH_2)_9$-, #-$CH(CH_3)$-O-$(CH_2)_{10}$-, #-$CH_2$-$(O(CH_2)_2)_2$-, #-$CH_2$-$(O(CH_2)_2)_3$-, #-$CH_2$-$(O(CH_2)_2)_4$-, #-$CH_2$-$(O(CH_2)_2)_5$-, #-$CH_2$-$(O(CH_2)_2)_6$-, #-$CH_2$-$(O(CH_2)_2)_7$-, #-$CH_2$-$(O(CH_2)_2)_8$-, #-$CH_2$-$(O(CH_2)_2)_9$-, #-$CH_2$-$(O(CH_2)_2)_{10}$-, #-$CH_2$-$(O(CH_2)_2)_1$-$OCH_2$-, #-$CH_2$-$(O(CH_2)_2)_2$-$OCH_2$-, #-$CH_2$-$(O(CH_2)_2)_3$-$OCH_2$-, #-$CH_2$-$(O(CH_2)_2)_4$-$OCH_2$-, #-$CH_2$-$(O(CH_2)_2)_5$-$OCH_2$-, #-$CH_2$-$(O(CH_2)_2)_6$-$OCH_2$-, #-$CH_2$-$(O(CH_2)_2)_7$-$OCH_2$-, #-$CH_2$-$(O(CH_2)_2)_8$-$OCH_2$-, #-$CH_2$-$(O(CH_2)_2)_9$-$OCH_2$-, #-$CH_2$-$(O(CH_2)_2)_{10}$-$OCH_2$-, #-$(CH_2)_2$-$(O(CH_2)_2)_2$-, #-$(CH_2)_2$-$(O(CH_2)_2)_3$-, #-$(CH_2)_2$-$(O(CH_2)_2)_4$-, #-$(CH_2)_2$-$(O(CH_2)_2)_5$-, #-$(CH_2)_2$-$(O(CH_2)_2)_6$-, #-$(CH_2)_2$-$(O(CH_2)_2)_7$-, #-$(CH_2)_2$-$(O(CH_2)_2)_8$-, #-$(CH_2)_2$-$(O(CH_2)_2)_9$-, #-$(CH_2)_2$-$(O(CH_2)_2)_{10}$-, #-$(CH_2)_3$-$(O(CH_2)_2)_2$-, #-$(CH_2)_3$-$(O(CH_2)_2)_3$-, #-$(CH_2)_3$-$(O(CH_2)_2)_4$-, #-$(CH_2)_3$-$(O(CH_2)_2)_5$-, #-$(CH_2)_3$-$(O(CH_2)_2)_6$-, #-$(CH_2)_3$-$(O(CH_2)_2)_7$-, #-$(CH_2)_3$-$(O(CH_2)_2)_8$-, #-$(CH_2)_3$-$(O(CH_2)_2)_9$-, #-$(CH_2)_3$-$(O(CH_2)_2)_{10}$-, #-$(CH_2)_4$-$(O(CH_2)_2)_2$-, #-$(CH_2)_4$-$(O(CH_2)_2)_3$-, #-$(CH_2)_4$-$(O(CH_2)_2)_4$-, #-$(CH_2)_4$-$(O(CH_2)_2)_5$-, #-$(CH_2)_4$-$(O(CH_2)_2)_6$-, #-$(CH_2)_4$-$(O(CH_2)_2)_7$-, #-$(CH_2)_4$-$(O(CH_2)_2)_8$-, #-$(CH_2)_4$-$(O(CH_2)_2)_9$-, #-$(CH_2)_4$-$(O(CH_2)_2)_{10}$-, #-$CH_2$-$(O(CH_2)_3)_2$-, #-$CH_2$-$(O(CH_2)_3)_3$-, #-$CH_2$-$(O(CH_2)_3)_4$-, #-$CH_2$-$(O(CH_2)_3)_5$-, #-$CH_2$-$(O(CH_2)_3)_6$-, #-$CH_2$-$(O(CH_2)_3)_7$-, #-$CH_2$-$(O(CH_2)_3)_8$-, #-$CH_2$-$(O(CH_2)_3)_9$-, #-$CH_2$-$(O(CH_2)_3)_{10}$-, #-$(CH_2)_2$-$(O(CH_2)_3)_2$-, #-$(CH_2)_2$-$(O(CH_2)_3)_3$-, #-$(CH_2)_2$-$(O(CH_2)_3)_4$-, #-$(CH_2)_2$-$(O(CH_2)_3)_5$-, #-$(CH_2)_2$-$(O(CH_2)_3)_6$-, #-$(CH_2)_2$-$(O(CH_2)_3)_7$-, #-$(CH_2)_2$-$(O(CH_2)_3)_8$-, #-$(CH_2)_2$-$(O(CH_2)_3)_9$-, #-$(CH_2)_2$-$(O(CH_2)_3)_{10}$-, #-$(CH_2)_3$-$(O(CH_2)_3)_2$-, #-$(CH_2)_3$-$(O(CH_2)_3)_3$-, #-$(CH_2)_3$-$(O(CH_2)_3)_4$-, #-$(CH_2)_3$-$(O(CH_2)_3)_5$-, #-$(CH_2)_3$-$(O(CH_2)_3)_6$-, #-$(CH_2)_3$-$(O(CH_2)_3)_7$-, #-$(CH_2)_3$-$(O(CH_2)_3)_8$-, #-$(CH_2)_3$-$(O(CH_2)_3)_9$-, #-$(CH_2)_3$-$(O(CH_2)_3)_{10}$-, #-$CH_2$-O-$(CH_2)_2$-O-$(CH_2)_3$-, #-$CH_2$-$(O(CH_2)_2)_2$-$(O(CH_2)_3)_2$-, #-$CH_2$-$(O(CH_2)_2)_3$-$(O(CH_2)_3)_3$-, #-$CH_2$-$(O(CH_2)_2)_4$-$(O(CH_2)_3)_4$-, #-$CH_2$-$(O(CH_2)_2)_5$-$(O(CH_2)_3)_5$-, #-$CH_2$-$(O(CH_2)_2)_6$-$(O(CH_2)_3)_6$-, #-$(CH_2)_2$-O-$(CH_2)_2$-O-$(CH_2)_3$-, #-$(CH_2)_2$-$(O(CH_2)_2)_2$-$(O(CH_2)_3)_2$-, #-$(CH_2)_2$-$(O(CH_2)_2)_3$-$(O(CH_2)_3)_3$-, #-$(CH_2)_2$-$(O(CH_2)_2)_4$-$(O(CH_2)_3)_4$-, #-$(CH_2)_2$-$(O(CH_2)_2)_5$-$(O(CH_2)_3)_5$-, #-$(CH_2)_2$-$(O(CH_2)_2)_6$-$(O(CH_2)_3)_6$-, #-$(CH_2)_3$-O-$(CH_2)_2$-O-$(CH_2)_3$-, #-$(CH_2)_3$-$(O(CH_2)_2)_2$-$(O(CH_2)_3)_2$-, #-$(CH_2)_3$-$(O(CH_2)_2)_3$-$(O(CH_2)_3)_3$-, #-$(CH_2)_3$-$(O(CH_2)_2)_4$-$(O(CH_2)_3)_4$-, #-$(CH_2)_3$-$(O(CH_2)_2)_5$-$(O(CH_2)_3)_5$-, #-$(CH_2)_3$-$(O(CH_2)_2)_6$-$(O(CH_2)_3)_6$-, #-$CH_2$-O-$(CH_2)_3$-O-$(CH_2)_2$-, #-$CH_2$-$(O(CH_2)_3)_2$-$(O(CH_2)_2)_2$-, #-$CH_2$-$(O(CH_2)_3)_3$-$(O(CH_2)_2)_3$-, #-$CH_2$-$(O(CH_2)_3)_4$-$(O(CH_2)_2)_4$-, #-$CH_2$-$(O(CH_2)_3)_5$-$(O(CH_2)_2)_5$-, #-$CH_2$-$(O(CH_2)_3)_6$-$(O(CH_2)_2)_6$-, #-$(CH_2)_2$-O-$(CH_2)_3$-O-$(CH_2)_2$-, #-$(CH_2)_2$-$(O(CH_2)_3)_2$-$(O(CH_2)_2)_2$-, #-$(CH_2)_2$-$(O(CH_2)_3)_3$-$(O(CH_2)_2)_3$-, #-$(CH_2)_2$-$(O(CH_2)_3)_4$-$(O(CH_2)_2)_4$-, #-$(CH_2)_2$-$(O(CH_2)_3)_5$-$(O(CH_2)_2)_5$-, #-$(CH_2)_2$-$(O(CH_2)_3)_6$-$(O(CH_2)_2)_6$-, #-$(CH_2)_3$-O-$(CH_2)_3$-O-$(CH_2)_2$-, #-$(CH_2)_3$-$(O(CH_2)_3)_2$-$(O(CH_2)_2)_2$-, #-$(CH_2)_3$-$(O(CH_2)_3)_3$-$(O(CH_2)_2)_3$-, #-$(CH_2)_3$-$(O(CH_2)_3)_4$-$(O(CH_2)_2)_4$-, #-$(CH_2)_3$-$(O(CH_2)_3)_5$-$(O(CH_2)_2)_5$-, #-$(CH_2)_3$-$(O(CH_2)_3)_6$-$(O(CH_2)_2)_6$-, #-$CH_2$-O-$(CH_2)_2$-O-$CH_2$-, #-$(CH_2)_2$-O-$(CH_2)_2$-O-$CH_2$-, #-$(CH_2)_2$-$(O(CH_2)_2)_2$-O-$(CH_2)_3$-, #-$(CH_2)_2$-$(O(CH_2)_2)_3$-O-$(CH_2)_3$-, #-$(CH_2)_2$-$(O(CH_2)_2)_4$-O-$(CH_2)_3$-, #-$(CH_2)_5$-$(O(CH_2)_2)_2$-O-$(CH_2)_5$-, #-$(CH_2)_5$-$(O(CH_2)_2)_2$-O-$(CH_2)_6$-, #-$(CH_2)_1$-$N(R_g)$-$(CH_2)_1$-, #-$(CH_2)_1$-$N(R_g)$-$(CH_2)_2$-, #-$(CH_2)_1$-$N(R_g)$-$(CH_2)_3$-, #-$(CH_2)_1$-$N(R_g)$-$(CH_2)_4$-, #-$(CH_2)_1$-$N(R_g)$-$(CH_2)_5$-, #-$(CH_2)_1$-$N(R_g)$-$(CH_2)_6$-, #-$(CH_2)_1$-$N(R_g)$-$(CH_2)_7$-, #-$(CH_2)_1$-$N(R_g)$-$(CH_2)_8$-, #-$(CH_2)_1$-$N(R_g)$-$(CH_2)_9$-, #-$(CH_2)_1$-$N(R_g)$-$(CH_2)_{10}$-, #-$(CH_2)_2$-$N(R_g)$-$(CH_2)_1$-, #-$(CH_2)_2$-$N(R_g)$-$(CH_2)_2$-, #-$(CH_2)_2$-$N(R_g)$-$(CH_2)_3$-, #-$(CH_2)_2$-$N(R_g)$-$(CH_2)_4$-, #-$(CH_2)_2$-$N(R_g)$-$(CH_2)_5$-, #-$(CH_2)_2$-$N(R_g)$-$(CH_2)_6$-, #-$(CH_2)_2$-$N(R_g)$-$(CH_2)_7$-, #-$(CH_2)_2$-$N(R_g)$-$(CH_2)_8$-, #-$(CH_2)_2$-$N(R_g)$-$(CH_2)_9$-, #-$(CH_2)_2$-$N(R_g)$-$(CH_2)_{10}$-, #-$(CH_2)_2$-$N(R_g)$-$(CH_2)_{11}$-, #-$(CH_2)_2$-$N(R_g)$-$(CH_2)_{12}$-, #-$(CH_2)_3$-$N(R_g)$-$(CH_2)_1$-, #-$(CH_2)_3$-$N(R_g)$-$(CH_2)_2$-, #-$(CH_2)_3$-$N(R_g)$-$(CH_2)_3$-, #-$(CH_2)_4$-$N(R_g)$-$(CH_2)_1$-, #-$(CH_2)_4$-$N(R_g)$-$(CH_2)_2$-, #-$(CH_2)_4$-$N(R_g)$-$(CH_2)_3$-, #-$(CH_2)_4$-$N(R_g)$-$(CH_2)_4$-, #-$(CH_2)_5$-$N(R_g)$-$(CH_2)_1$-, #-$(CH_2)_5$-$N(R_g)$-$(CH_2)_2$-, #-$(CH_2)_5$-$N(R_g)$-$(CH_2)_3$-, #-$(CH_2)_5$-$N(R_g)$-$(CH_2)_4$-, #-$(CH_2)_5$-$N(R_g)$-$(CH_2)_5$-, #-$(CH_2)_6$-$N(R_g)$-$(CH_2)_1$-, #-$(CH_2)_6$-$N(R_g)$-$(CH_2)_2$-, #-$(CH_2)_6$-$N(R_g)$-$(CH_2)_3$-, #-$(CH_2)_7$-$N(R_g)$-$(CH_2)_1$-, #-$(CH_2)_7$-$N(R_g)$-$(CH_2)_2$-, #-$(CH_2)_7$-$N(R_g)$-$(CH_2)_3$-, #-$(CH_2)_8$-$N(R_g)$-$(CH_2)_1$-, #-$(CH_2)_8$-$N(R_g)$-$(CH_2)_2$-, #-$(CH_2)_8$-$N(R_g)$-$(CH_2)_3$-, #-$CH(CH_3)$-$N(R_g)$-$(CH_2)_1$-, #-$CH(CH_3)$-$N(R_g)$-$(CH_2)_2$-, #-$CH(CH_3)$-$N(R_g)$-$(CH_2)_3$-, #-$CH(CH_3)$-$N(R_g)$-$(CH_2)_4$-, #-$CH(CH_3)$-$N(R_g)$-$(CH_2)_5$-, #-$CH(CH_3)$-$N(R_g)$-$(CH_2)_6$-, #-$CH(CH_3)$-$N(R_g)$-$(CH_2)_7$-, #-$CH(CH_3)$-$N(R_g)$-$(CH_Z)$s-, #-$CH(CH_3)$-$N(R_g)$-$(CH_2)_9$-, #-$CH(CH_3)$-$N(R_g)$-$(CH_2)_{10}$-, #-$CH_2C(O)NHCH_2$-, #-$(CH_2)_2C(O)NH(CH_2)_2$-, #-$(CH_2)_2C(O)NH(CH_2)_3$-, #-$(CH_2)_2C(O)NH(CH_2)_4$-, #-$(CH_2)_2C(O)NH(CH_2)_5$-, #-$(CH_2)_3C(O)NH(CH_2)_3$-, #-$(CH_2)_3C(O)NH(CH_2)_4$-, #-$(CH_2)_4C(O)NH(CH_2)_4$-, #-$(CH_2)_5C(O)NH(CH_2)_5$-, #-$(CH_2)_6C(O)NH(CH_2)_7$-, #-$(CH_2)_6C(O)NH(CH_2)_6$-, #-$(CH_2)_7C(O)NH(CH_2)_7$-, #-$(CH_2)_8C(O)NH(CH_2)_8$, U-$(CH_2)_9C(O)NH(CH_2)_9$-, #-$(CH_2)_{10}C(O)NH(CH_2)_{10}$-,

#-$(CH_2)_2$C(O)NH$(CH_2)_2$-O-$(CH_2)_2$-, #-$CH_2$NHC(O)$CH_2$-, #-$(CH_2)_2$NHC(O)$(CH_2)_2$-, #-$(CH_2)_2$NHC(O)$(CH_2)_3$-, #-$(CH_2)_2$NHC(O)$(CH_2)_4$-, #-$(CH_2)_2$NHC(O)$(CH_2)_5$-, #-$(CH_2)_3$NHC(O)$(CH_2)_3$-, #-$(CH_2)_3$NHC(O)$(CH_2)_4$-, #-$(CH_2)_4$NHC(O)$(CH_2)_4$-, #-$(CH_2)_5$NHC(O)$(CH_2)_5$-, #-$(CH_2)_6$NHC(O)$(CH_2)_7$-, #-$(CH_2)_6$NHC(O)$(CH_2)_6$-, #-$(CH_2)_7$NHC(O)$(CH_2)_7$-, #-$(CH_2)_8$NHC(O)$(CH_2)_8$, #-$(CH_2)_9$NHC(O)$(CH_2)_9$-, #-$(CH_2)_{10}$NHC(O)$(CH_2)_{10}$-, #-$(CH_2)_4$NHC(O)$(CH_2)_8$-, #-$(CH_2)_2$NHC(O)$(CH_2)_2$-O-$(CH_2)_2$-, #-$(CH_2)_4$NHC(O)$CH_2$-, #-$CH_2$-piperidinylene-$CH_2$-, #-$CH_2$-piperidinylene-$(CH_2)_2$-, #-$CH_2$-piperidinylene-$(CH_2)_3$-, #-$CH_2$-piperidinylene-$(CH_2)_4$-, #-$CH_2$-piperidinylene-$(CH_2)_5$-, #-$CH_2$-piperidinylene-$(CH_2)_6$-, #-$CH_2$-piperidinylene-$(CH_2)_7$-, #-$CH_2$-piperidinylene-$(CH_2)_8$-, #-$(CH_2)_2$-piperidinylene-$(CH_2)_1$-, #-$(CH_2)_2$-piperidinylene-$(CH_2)_2$-, #-$(CH_2)_2$-piperidinylene-$(CH_2)_3$-, #-$(CH_2)_2$-piperidinylene-$(CH_2)_4$-, #-$(CH_2)_2$-piperidinylene-$(CH_2)_5$-, #-$(CH_2)_2$-piperidinylene-$(CH_2)_6$-, #-$(CH_2)_2$-piperidinylene-$(CH_2)_7$-, #-$(CH_2)_2$-piperidinylene-$(CH_2)_8$-, #-$(CH_2)_3$-piperidinylene-$CH_2$-, #-$(CH_2)_3$-piperidinylene-$(CH_2)_2$-, #-$(CH_2)_3$-piperidinylene-$(CH_2)_3$-, #-$(CH_2)_3$-piperidinylene-$(CH_2)_4$-, #-$(CH_2)_3$-piperidinylene-$(CH_2)_5$-, #-$(CH_2)_3$-piperidinylene-$(CH_2)_6$-, #-$(CH_2)_3$-piperidinylene-$(CH_2)_7$-, #-$(CH_2)_3$-piperidinylene-$(CH_2)_8$-, #-$(CH_2)_4$-piperidinylene-$CH_2$-, #-$(CH_2)_4$-piperidinylene-$(CH_2)_2$-, #-$(CH_2)_4$-piperidinylene-$(CH_2)_3$-, #-$(CH_2)_4$-piperidinylene-$(CH_2)_4$-, #-$(CH_2)_4$-piperidinylene-$(CH_2)_5$-, #-$(CH_2)_4$-piperidinylene-$(CH_2)_6$-, #-$(CH_2)_4$-piperidinylene-$(CH_2)_7$-, #-$(CH_2)_4$-piperidinylene-$(CH_2)_8$-, #-$(CH_2)_5$-piperidinylene-$(CH_2)_1$-, #-$(CH_2)_5$-piperidinylene-$(CH_2)_2$-, #-$(CH_2)_5$-piperidinylene-$(CH_2)_3$-, #-$(CH_2)_5$-piperidinylene-$(CH_2)_4$-, #-$(CH_2)_5$-piperidinylene-$(CH_2)_5$-, #-$(CH_2)_5$-piperidinylene-$(CH_2)_6$-, #-$(CH_2)_5$-piperidinylene-$(CH_2)_7$-, #-$(CH_2)_5$-piperidinylene-$(CH_2)_8$-, #-$(CH_2)_6$-piperidinylene-$(CH_2)_1$-, #-$(CH_2)_6$-piperidinylene-$(CH_2)_2$-, #-$(CH_2)_6$-piperidinylene-$(CH_2)_3$-, #-$(CH_2)_6$-piperidinylene-$(CH_2)_4$-, #-$(CH_2)_6$-piperidinylene-$(CH_2)_5$-, #-$(CH_2)_6$-piperidinylene-$(CH_2)_6$-, #-$(CH_2)_6$-piperidinylene-$(CH_2)_7$-, #-$(CH_2)_6$-piperidinylene-$(CH_2)_8$-, #-$(CH_2)_7$-piperidinylene-$(CH_2)_1$-, #-$(CH_2)_7$-piperidinylene-$(CH_2)_2$-, #-$(CH_2)_7$-piperidinylene-$(CH_2)_3$-, #-$(CH_2)_7$-piperidinylene-$(CH_2)_4$-, #-$(CH_2)_7$-piperidinylene-$(CH_2)_8$-, #-$(CH_2)_8$-piperidinylene-$CH_2$-, #-$(CH_2)_8$-piperidinylene-$(CH_2)_2$-, #-$(CH_2)_8$-piperidinylene-$(CH_2)_3$-, #-$(CH_2)_8$-piperidinylene-$(CH_2)_4$-, #-$(CH_2)_8$-pipendinylene-$(CH_2)_5$-, #-$(CH_2)_8$-piperidinylene-$(CH_2)_6$-, #-$(CH_2)_8$-piperidinylene-$(CH_2)_7$-, #-$(CH_2)_8$-piperidinylene-$(CH_2)_8$-, #-$CH_2$-N($R_g$)-$CH_2$-piperidinylene-$CH_2$-, #-$CH_2$-N($R_g$)-$CH_2$-piperidinylene-$(CH_2)_2$-, #-$CH_2$-N($R_g$)-$CH_2$-piperidinylene-$(CH_2)_3$-, #-$CH_2$-N($R_g$)-$CH_2$-piperidinylene-$(CH_2)_4$-, #-$CH_2$-N($R_g$)-$CH_2$-piperidinylene-$(CH_2)_5$-, #-$CH_2$-N($R_g$)-$CH_2$-piperidinylene-$(CH_2)_6$-, #-$CH_2$-N($R_g$)-$CH_2$-piperidinylene-$(CH_2)_7$-, #-$CH_2$-N($R_g$)-$CH_2$-piperidinylene-$(CH_2)_8$-, #-$CH_2$-N($R_g$)-$(CH_2)_2$-piperidinylene-$CH_2$-, #-$CH_2$-N($R_g$)-$(CH_2)_2$-piperidinylene-$(CH_2)_2$-, #-$CH_2$-N($R_g$)-$(CH_2)_2$-piperidinylene-$(CH_2)_3$-, #-$CH_2$-N($R_g$)-$(CH_2)_2$-piperidinylene-$(CH_2)_4$-, #-$CH_2$-N($R_g$)-$(CH_2)_2$-piperidinylene-$(CH_2)_5$-, #-$CH_2$-N($R_g$)-$(CH_2)_2$-piperidinylene-$(CH_2)_6$-, #-$CH_2$-N($R_g$)-$(CH_2)_2$-piperidinylene-$(CH_2)_7$-, #-$CH_2$-N($R_g$)-$(CH_2)_2$-piperidinylene-$(CH_2)_8$-, #-$(CH_2)_2$-N($R_g$)-$CH_2$-piperidinylene-$CH_2$-, #-$(CH_2)_2$-N($R_g$)-$CH_2$-piperidinylene-$(CH_2)_2$-, #-$(CH_2)_2$-N($R_g$)-$CH_2$-piperidinylene-$(CH_2)_3$-, #-$(CH_2)_2$-N($R_g$)-$CH_2$-piperidinylene-$(CH_2)_4$-, #-$(CH_2)_2$-N($R_g$)-$CH_2$-piperidinylene-$(CH_2)_5$-, #-$(CH_2)_2$-N($R_g$)-$CH_2$-piperidinylene-$(CH_2)_6$-, #-$(CH_2)_2$-N($R_g$)-$CH_2$-piperidinylene-$(CH_2)_7$-, #-$(CH_2)_2$-N($R_g$)-$CH_2$-piperidinylene-$(CH_2)_8$-, #-$(CH_2)_3$-N($R_g$)-$CH_2$-piperidinylene-$CH_2$-, #-$(CH_2)_3$-N($R_g$)-$CH_2$-piperidinylene-$(CH_2)_2$-, #-$(CH_2)_3$-N($R_g$)-$CH_2$-piperidinylene-$(CH_2)_3$-, #-$(CH_2)_3$-N($R_g$)-$CH_2$-piperidinylene-$(CH_2)_8$-, #-$(CH_2)_4$-N($R_g$)-$CH_2$-piperidinylene-$CH_2$-, #-$(CH_2)_4$-N($R_g$)-$CH_2$-piperidinylene-$(CH_2)_2$-, #-$(CH_2)_4$-N($R_g$)-$CH_2$-piperidinylene-$(CH_2)_3$-, #-$(CH_2)_4$-N($R_g$)-$CH_2$-piperidinylene-$(CH_2)_8$-, #-$(CH_2)_5$-N($R_g$)-$CH_2$-piperidinylene-$(CH_2)_3$-, #-$(CH_2)_5$-N($R_g$)-$CH_2$-piperidinylene-$(CH_2)_8$-, #-$(CH_2)_6$-N($R_g$)-$CH_2$-piperidinylene-$(CH_2)_3$-, #-$(CH_2)_6$-N($R_g$)-$CH_2$-piperidinylene-$(CH_2)_8$-, #-$(CH_2)_7$-N($R_g$)-$CH_2$-piperidinylene-$(CH_2)_3$-, #-$(CH_2)_7$-N($R_g$)-$CH_2$-piperidinylene-$(CH_2)_8$-, #-$(CH_2)_8$-N($R_g$)-$CH_2$-piperidinylene-$CH_2$-, #-$(CH_2)_8$-N($R_g$)-$CH_2$-piperidinylene-$(CH_2)_2$-, #-$(CH_2)_8$-N($R_g$)-$CH_2$-piperidinylene-$(CH_2)_3$-, #-$(CH_2)_8$-N($R_g$)-$CH_2$-piperidinylene-$(CH_2)_4$-, #-$(CH_2)_8$-N($R_g$)-$CH_2$-piperidinylene-$(CH_2)_5$-, #-$(CH_2)_8$-N($R_g$)-$CH_2$-piperidinylene-$(CH_2)_6$-, #-$(CH_2)_8$-N($R_g$)-$CH_2$-piperidinylene-$(CH_2)_7$-, #-$(CH_2)_8$-N($R_g$)-$CH_2$-piperidinylene-$(CH_2)_8$-, #-$CH_2$-piperazinylene-$CH_2$-, #-$CH_2$-piperazinylene-$(CH_2)_2$-, #-$CH_2$-piperazinylene-$(CH_2)_3$-, #-$CH_2$-piperazinylene-$(CH_2)_4$-, #-$CH_2$-piperazinylene-$(CH_2)_5$-, #-$CH_2$-piperazinylene-$(CH_2)_6$-, #-$CH_2$-piperazinylene-$(CH_2)_7$-, #-$CH_2$-piperazinylene-$(CH_2)_8$-, #-$(CH_2)_2$-piperazinylene-$(CH_2)_1$-, #-$(CH_2)_2$-piperazinylene-$(CH_2)_2$-, #-$(CH_2)_2$-piperazinylene-$(CH_2)_3$-, #-$(CH_2)_2$-piperazinylene-$(CH_2)_4$-, #-$(CH_2)_2$-piperazinylene-$(CH_2)_5$-, #-$(CH_2)_2$-piperazinylene-$(CH_2)_6$-, #-$(CH_2)_2$-piperazinylene-$(CH_2)_7$-, #-$(CH_2)_2$-piperazinylene-$(CH_2)_8$-, #-$(CH_2)_3$-piperazinylene-$CH_2$-, #-$(CH_2)_3$-piperazinylene-$(CH_2)_2$-, #-$(CH_2)_3$-piperazinylene-$(CH_2)_3$-, #-$(CH_2)_3$-piperazinylene-$(CH_2)_4$-, #-$(CH_2)_3$-piperazinylene-$(CH_2)_5$-, #-$(CH_2)_3$-piperazinylene-$(CH_2)_6$-, #-$(CH_2)_3$-piperazinylene-$(CH_2)_7$-, #-$(CH_2)_3$-piperazinylene-$(CH_2)_8$-, #-$(CH_2)_4$-piperazinylene-$CH_2$-, #-$(CH_2)_4$-piperazinylene-$(CH_2)_2$-, #-$(CH_2)_4$-piperazinylene-$(CH_2)_3$-, #-$(CH_2)_4$-piperazinylene-$(CH_2)_4$-, #-$(CH_2)_4$-piperazinylene-$(CH_2)_5$-, #-$(CH_2)_4$-piperazinylene-$(CH_2)_6$-, #-$(CH_2)_4$-piperazinylene-$(CH_2)_7$-, #-$(CH_2)_4$-piperazinylene-$(CH_2)_8$-, #-$(CH_2)_5$-piperazinylene-$(CH_2)_1$-, #-$(CH_2)_5$-piperazinylene-$(CH_2)_2$-, #-$(CH_2)_5$-piperazinylene-$(CH_2)_3$-, #-$(CH_2)_5$-piperazinylene-$(CH_2)_4$-, #-$(CH_2)_5$-piperazinylene-$(CH_2)_5$-, #-$(CH_2)_5$-piperazinylene-$(CH_2)_6$-,

#-$(CH_2)_5$-piperazinylene-$(CH_2)_7$-, #-$(CH_2)_5$-piperazinylene-$(CH_2)_8$-, #-$(CH_2)_6$-piperazinylene-$(CH_2)_1$-, #-$(CH_2)_6$-piperazinylene-$(CH_2)_2$-, #-$(CH_2)_6$-piperazinylene-$(CH_2)_3$-, #-$(CH_2)_6$-piperazinylene-$(CH_2)_4$-, #-$(CH_2)_6$-piperazinylene-$(CH_2)_5$-, #-$(CH_2)_6$-piperazinylene-$(CH_2)_6$-, #-$(CH_2)_6$-piperazinylene-$(CH_2)_7$-, #-$(CH_2)_6$-piperazinylene-$(CH_2)_8$-, #-$(CH_2)_7$-piperazinylene-$(CH_2)_1$-, #-$(CH_2)_7$-piperazinylene-$(CH_2)_2$-, #-$(CH_2)_7$-piperazinylene-$(CH_2)_3$-, #-$(CH_2)_7$-piperazinylene-$(CH_2)_4$-, #-$(CH_2)_7$-piperazinylene-$(CH_2)_8$-, #-$(CH_2)_8$-piperazinylene-$CH_2$-, #-$(CH_2)_8$-piperazinylene-$(CH_2)_2$-, #-$(CH_2)_8$-piperazinylene-$(CH_2)_3$-, #-$(CH_2)_8$-piperazinylene-$(CH_2)_4$-, #-$(CH_2)_8$-piperazinylene-$(CH_2)_5$-, #-$(CH_2)_8$-piperazinylene-$(CH_2)_6$-, #-$(CH_2)_8$-piperazinylene-$(CH_2)_7$-, or #-$(CH_2)_8$-piperazinylene-$(CH_2)_8$-;

wherein the piperidinylene and the piperazinylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano, or any combination thereof;

each $R_g$ independently represent H or $C_{1-6}$ alkyl;

symbol # indicates the point of attachment to $U_2$; and

n1, n2, n3, n4, m1, m2, and m3 each independently represent an integer of 1, 2, 3, 4, 5, 6, 7, 8, 9, 10, 11, 12, 13, 14, or 15.

**35.** The compound of Formula (III) or salts, enantiomers, stereoisomers, solvates, isotopically enriched analogs, prodrugs, or polymorphs thereof as claimed in any one of claims 20-31, wherein - $W^1$-LIN-$U_2$-$R_c$-$U_1$- represents the following groups:

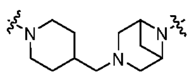 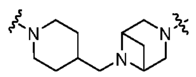 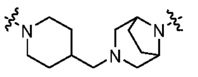 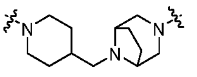 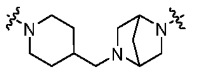 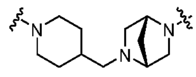

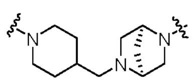 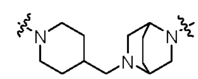 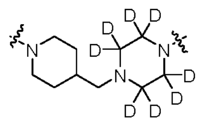 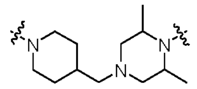 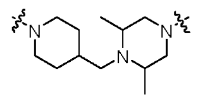 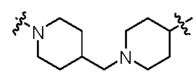

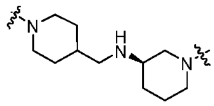 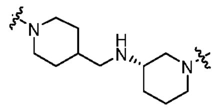 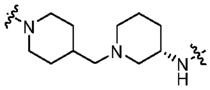 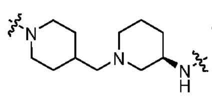 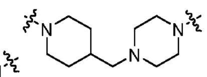 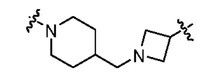

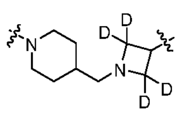 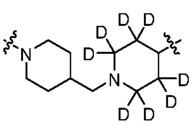 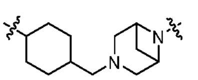 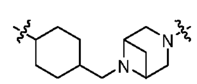 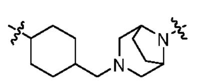 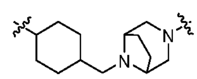

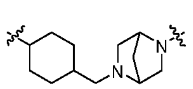 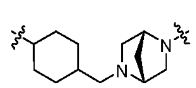 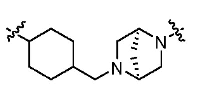 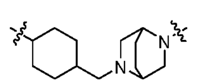 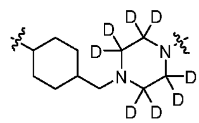 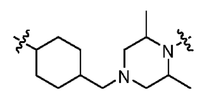

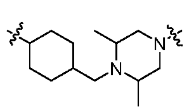 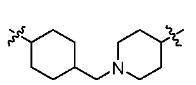 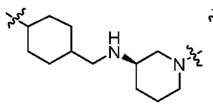 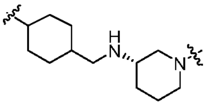 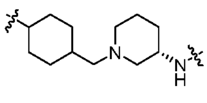 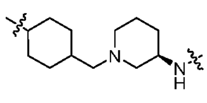

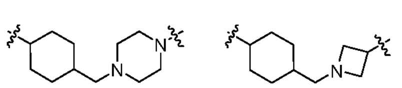 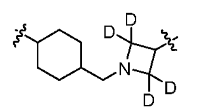 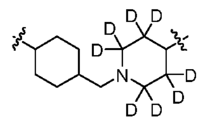 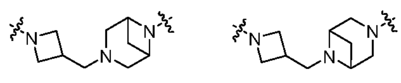

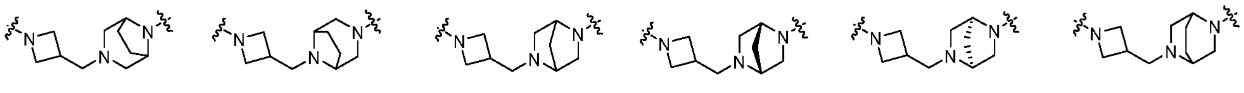

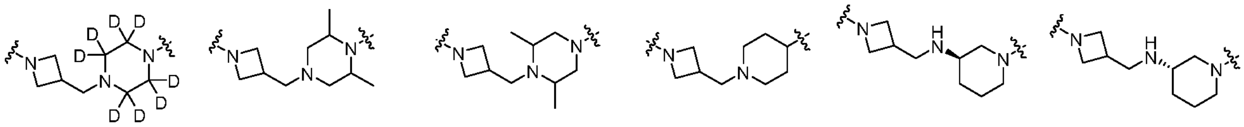

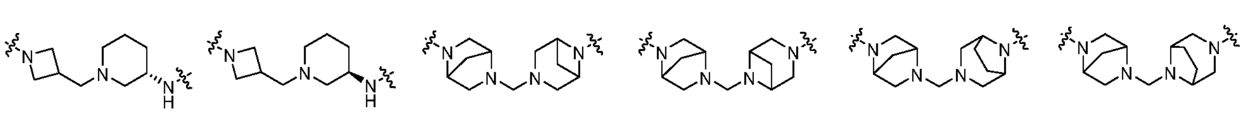

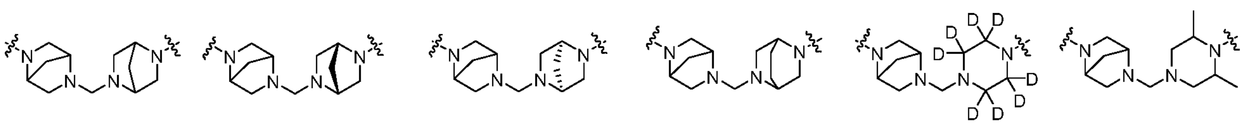

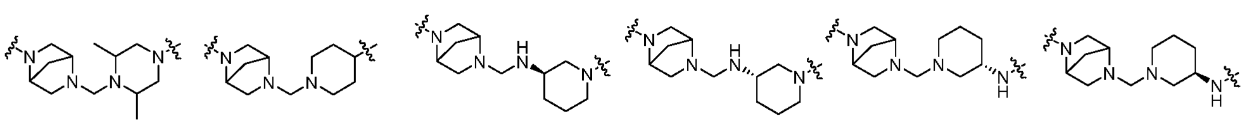

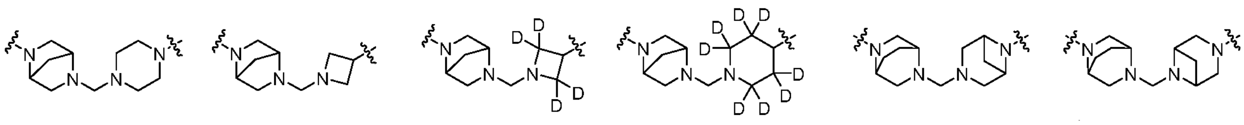

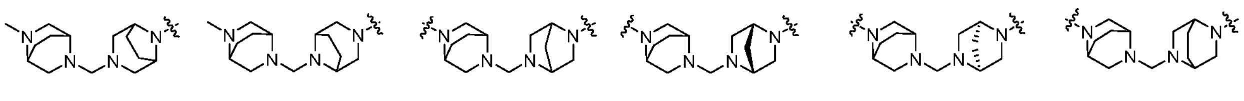

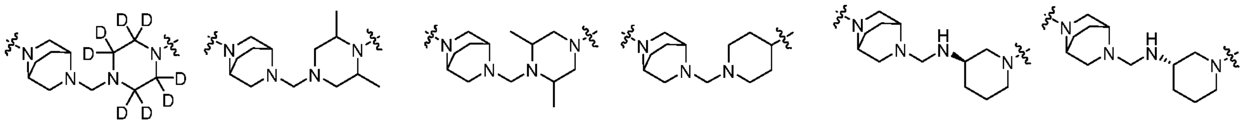

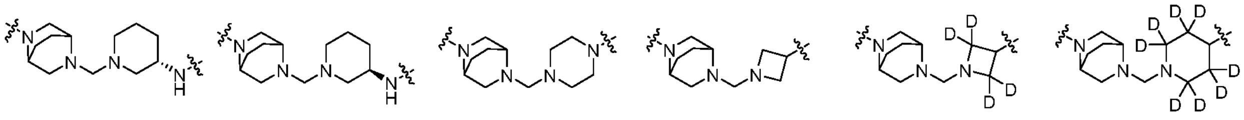

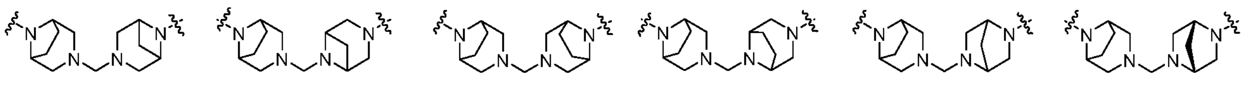

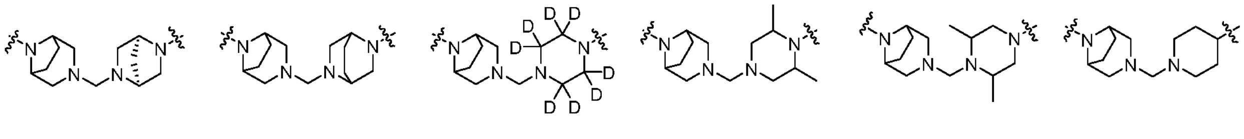

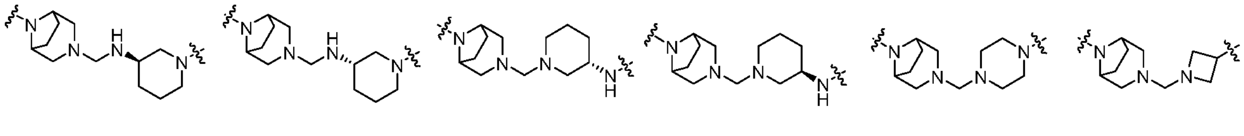

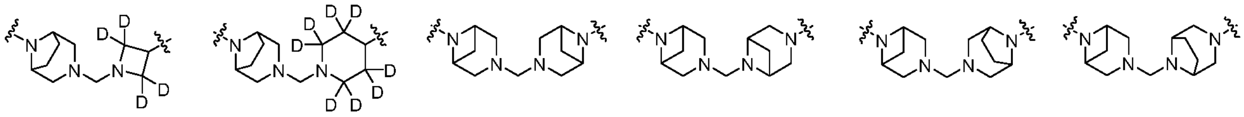

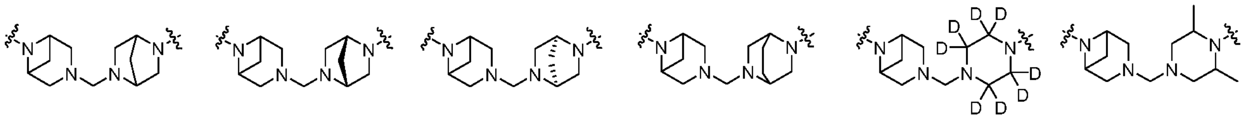

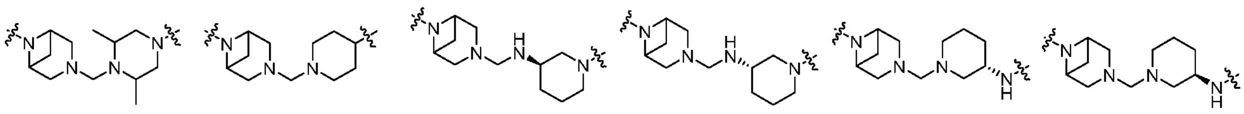

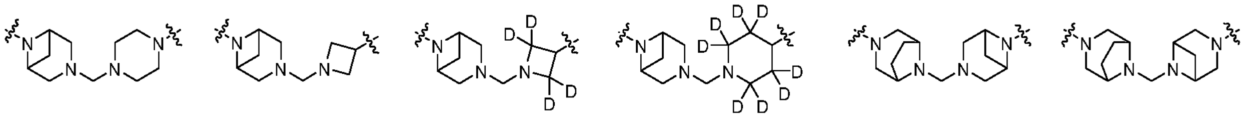

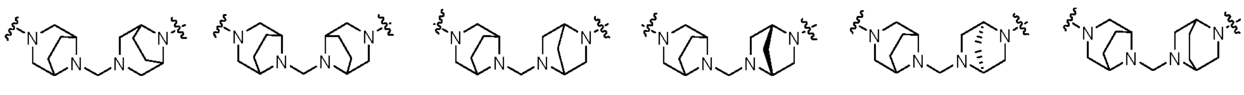

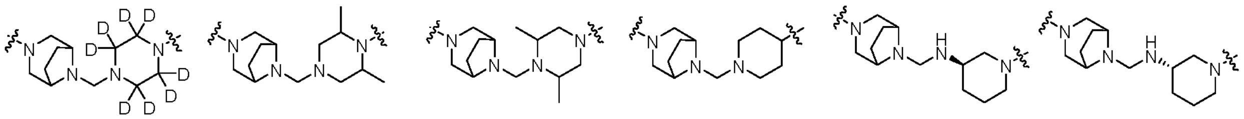

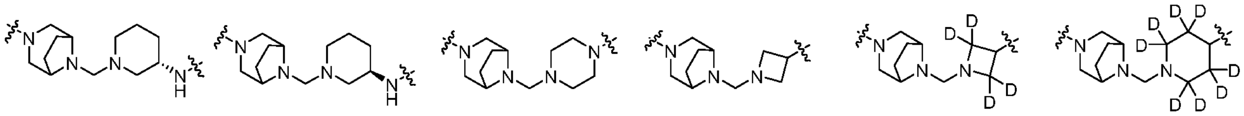

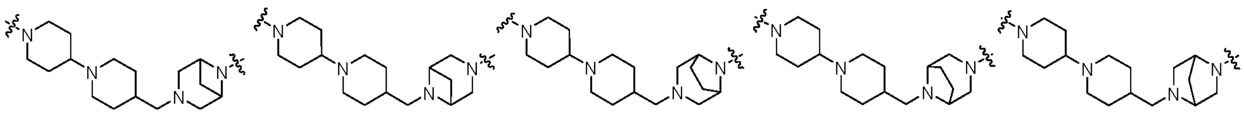

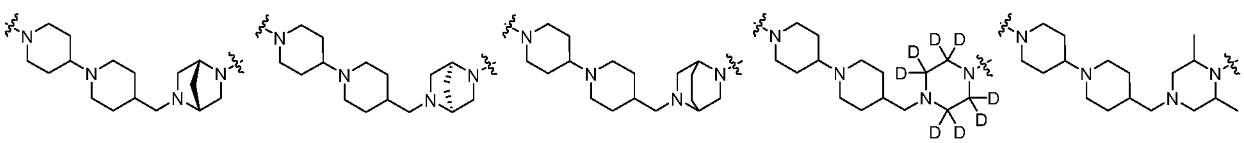

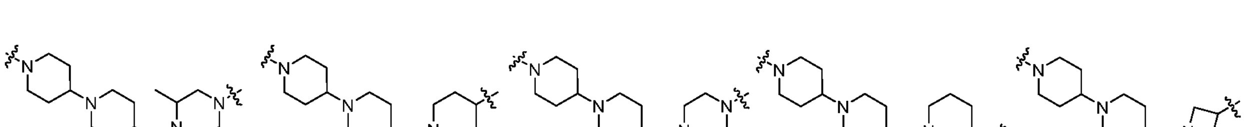

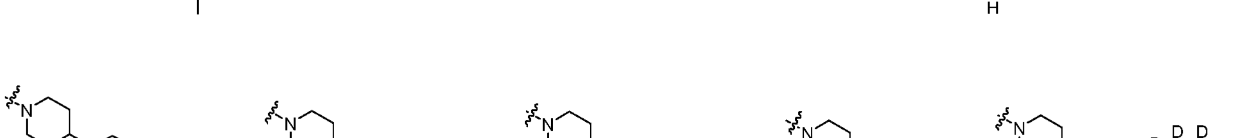

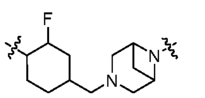 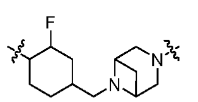 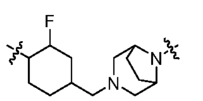 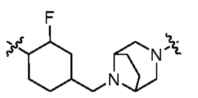 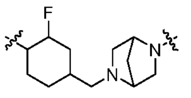 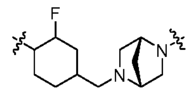

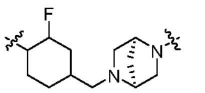 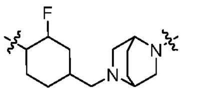 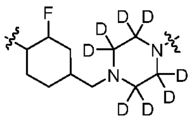 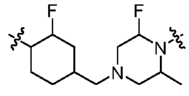 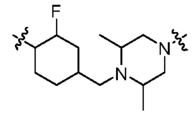 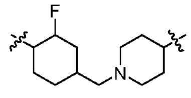

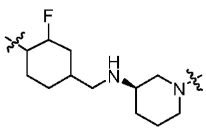 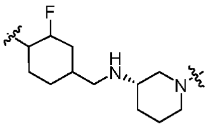 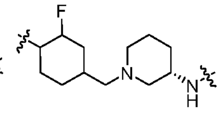 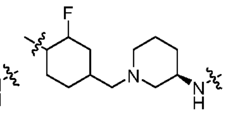 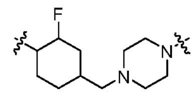 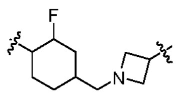

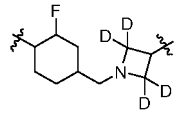 or 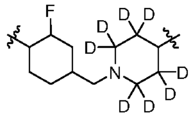 ;

wherein the groups are optionally substituted with one or more substituents selected from the group consisting of deuterium, halogen, cyano, amino, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, deuterated $C_{1-6}$ alkoxy, or any combination thereof.

**36.** The compound of Formula (III) or salts, enantiomers, stereoisomers, solvates, isotopically enriched analogs, prodrugs, or polymorphs thereof as claimed in claim 20, which is also of Formula (III-1), Formula (III-2), Formula (III-3), Formula (III-4), Formula (III-5), Formula (III-6), Formula (III-7), Formula (III-8), Formula (III-9), Formula (III-10), Formula (III-11), Formula (III-12), Formula (III-13), Formula (III-14), Formula (III-15), Formula (III-16), Formula (III-17), Formula (III-18), Formula (III-19), Formula (III-20), Formula (III-21), Formula (III-22), Formula (III-23) or Formula (III-24):

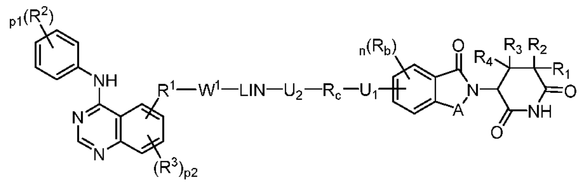
Formula (III-1)

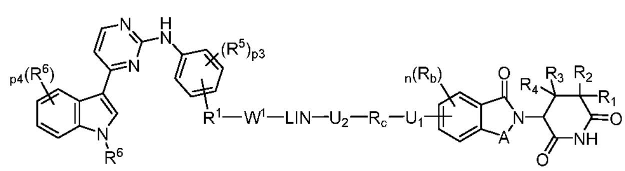
Formula (III-2)

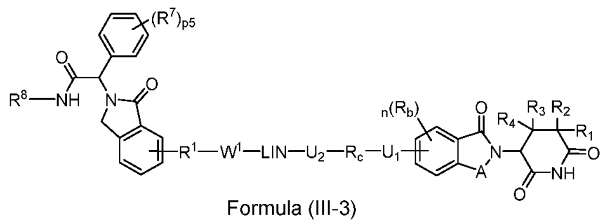
Formula (III-3)

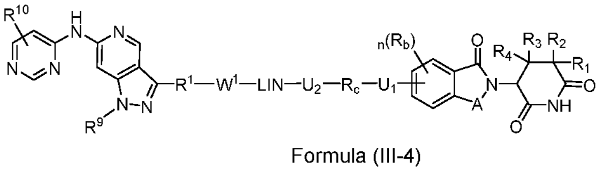
Formula (III-4)

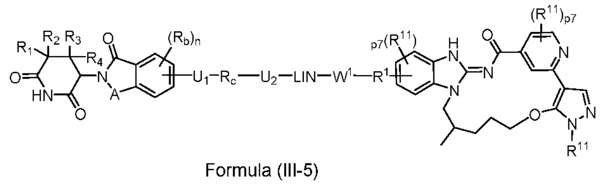
Formula (III-5)

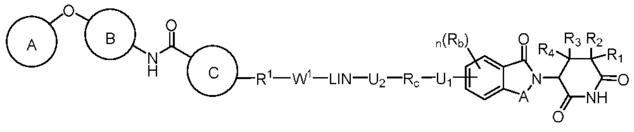
Formula (III-6)

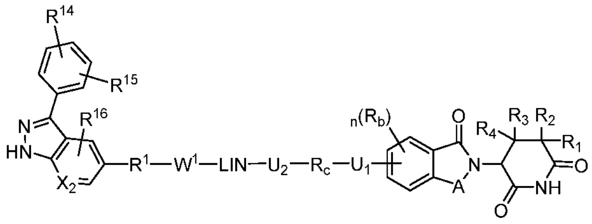

Formula (III-7)

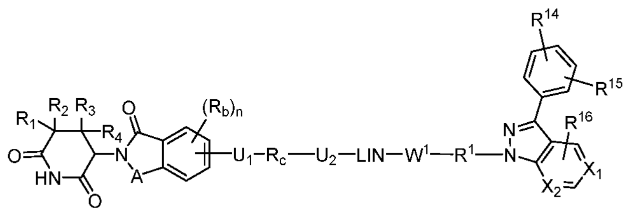

Formula (III-8)

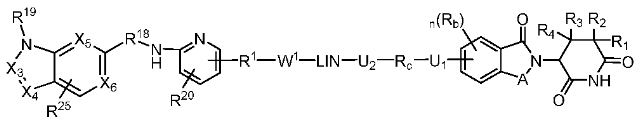

Formula (III-9)

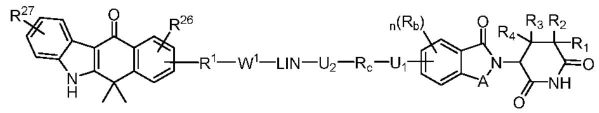

Formula (III-10)

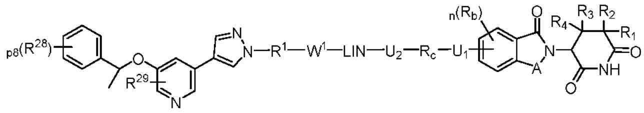

Formula (III-11)

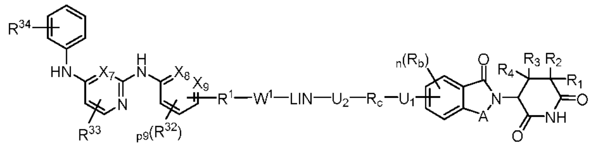

Formula (III-12)

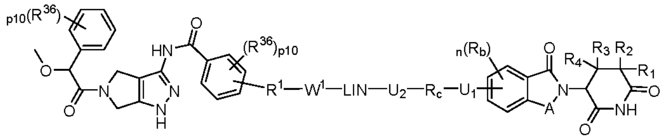

Formula (III-13)

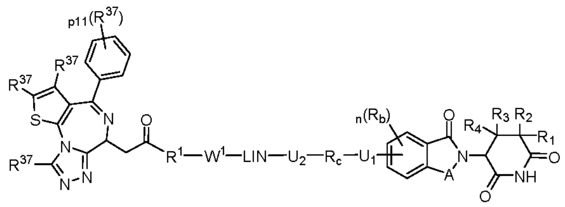

Formula (III-14)

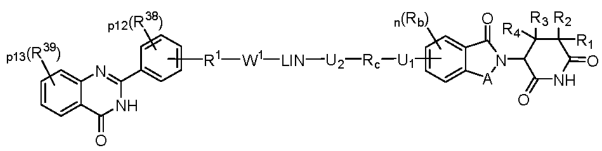

Formula (III-15)

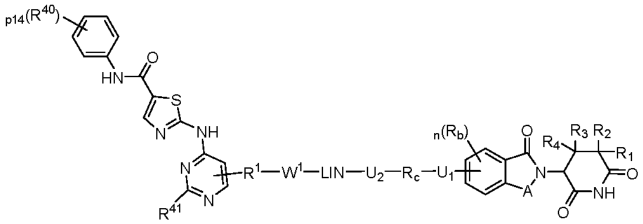

Formula (III-16)

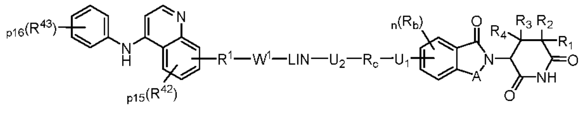

Formula (III-17)

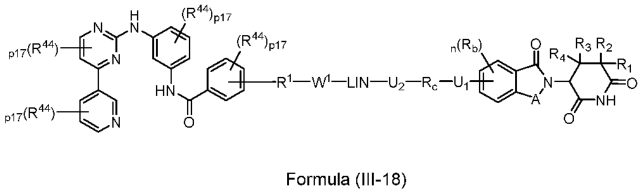

Formula (III-18)

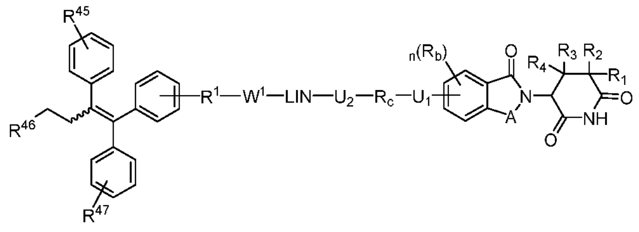

Formula (III-19)

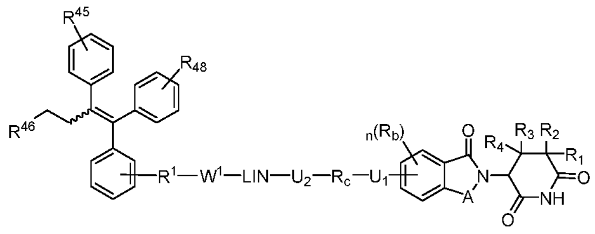

Formula (III-20)

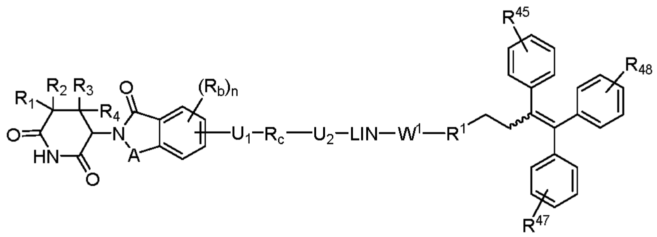

Formula (III-21)

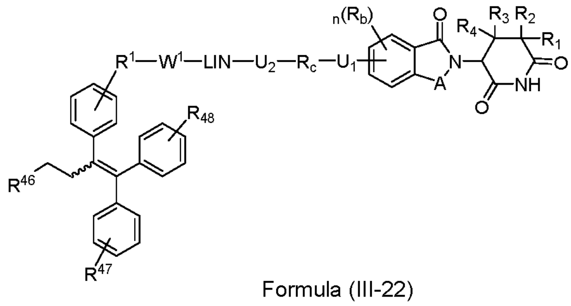

Formula (III-22)

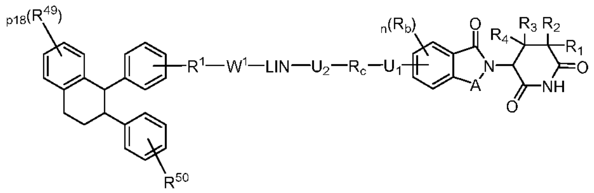

Formula (III-23)

and

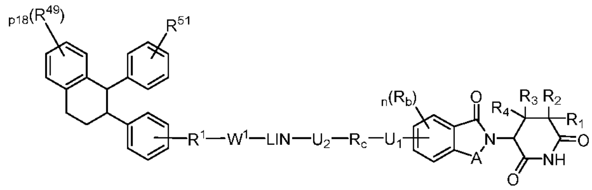

Formula (III-24)

wherein A, $R_1$, $R_2$, $R_3$, $R_4$, $(R_b)_n$, $U_1$, $U_2$, $R_c$, LIN, $W^1$, $R^1$, $(R^2)_{p1}$, $(R^3)_{p2}$, $(R^5)_{p3}$, $(R^6)_{p4}$, $(R^7)_{p5}$, $R^8$, $R^9$, $R^{10}$, $(R^{11})_{p7}$, ring A, ring B, ring C, $X_1$, $X_2$, $R^{14}$, $R^{15}$, $R^{16}$, $X_3$, $X_4$, $X_5$, $X_6$, $R^{18}$, $R^{19}$, $R^{20}$, $R^{25}$, $R^{26}$ , $R^{27}$, $(R^{28})_{p8}$, $R^{29}$, $X_7$, $X_8$, $X_9$, $(R^{32})_{p9}$, $R^{33}$, $R^{34}$, $(R^{36})_{p10}$, $(R^{37})_{p11}$, $(R^{38})_{p12}$, $(R^{39})_{p13}$, $(R^{40})_{p41}$, $R^{41}$, $(R^{42})_{p15}$, $(R^{43})_{p16}$, $(R^{44})_{p17}$, $R^{45}$, $R^{46}$, $R^{47}$ $R^{48}$ $(R^{49})_{p18}$, $R^{50}$, and $R^{51}$ are as defined in claim 22.

**37.** The compound of Formula (III) or salts, enantiomers, stereoisomers, solvates, isotopically enriched analogs, prodrugs, or polymorphs thereof as claimed in claim 20, which is selected from:

N-((1r,4r)-4-(3-chloro-4-cyanophenoxy)cyclohexyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazine-3-carboxamide;
N-((1r,4r)-4-(3-chloro-4-cyanophenoxy)cyclohexyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)pyridazine-3-carboxamide;
N-((1r,4r)-4-(3-chloro-4-cyanophenoxy)cyclohexyl)-6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridazine-3-carboxamide;
N-((1r,4r)-4-(3-chloro-4-cyanophenoxy)cyclohexyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazine-3-carboxamide;
N-((1r,4r)-4-(3-chloro-4-cyanophenoxy)cyclohexyl)-6-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridazine-3-carboxamide;
N-((1r,4r)-4-(3-chloro-4-cyanophenoxy)cyclohexyl)-6-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridazine-3-carboxamide;
N-((1r,4r)-4-(3-chloro-4-cyanophenoxy)cyclohexyl)-6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)pyridazine-3-carboxamide;
N-((1r,4r)-4-(3-chloro-4-cyanophenoxy)cyclohexyl)-6-(4-((4-(2-(2,6-dioxopiperidin-3-yl)-1,3-dioxoisoindolin-5-yl)piperazin-1-yl-2,2,3,3,5,5,6,6-*d*$_8$)methyl)piperidin-1-yl)pyridazine-3-carboxamide;
N-((1r,4r)-4-(3-chloro-4-cyanophenoxy)cyclohexyl)-6-(4-((4-(2-(2,6-dioxopiperidin-3-yl)-1,3-dioxoisoindolin-5-yl)-3,5-dimethylpiperazin-1-yl)methyl)piperidin-1-yl)pyridazine-3-carboxamide;
N-((1r,4r)-4-(3-chloro-4-cyanophenoxy)cyclohexyl)-6-(4-((4-(2-(2,6-dioxopiperidin-3-yl)-1,3-dioxoisoindolin-5-yl)-2,6-dimethylpiperazin-1-yl)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-1,3-dioxoisoindolin-5-yl)piperidin-1-yl)methyl)piperidin-1-yl)pyridazine-3-carboxamide;

N-((1r,4r)-4-(3-chloro-4-cyanophenoxy)cyclohexyl)-6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridazine-3-carboxamide;

N-((1r,4r)-4-(3-chloro-4-cyanophenoxy)cyclohexyl)-6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridazine-3-carboxamide;

N-((1r,4r)-4-(3-chloro-4-cyanophenoxy)cyclohexyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazine-3-carboxamide;

N-((1r,4r)-4-(3-chloro-4-cyanophenoxy)cyclohexyl)-6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridazine-3-carboxamide;

N-((1r,4r)-4-(3-chloro-4-cyanophenoxy)cyclohexyl)-6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)piperidin-1-yl)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-4-fluoro-1,3-dioxoisoindolin-5-yl)piperazin-1-yl-2,2,3,3,5,5,6,6-ds)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-7-fluoro-1,3-dioxoisoindolin-5-yl)piperazin-1-yl-2,2,3,3,5,5,6,6-ds)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)pipendin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)pyridazine-3-carboxamide;

N-((1r,4r)-4-(3-chloro-4-cyanophenoxy)cyclohexyl)-6-(4-((4-(2-(2,6-dioxopiperidin-3-yl)-4,6,7-trifluoro-1,3-dioxoisoindolin-5-yl)piperazin-1-yl-2,2,3,3,5,5,6,6-ds)methyl)piperidin-1-yl)pyridazine-3-carboxamide;

N-((1r,4r)-4-(3-chloro-4-cyanophenoxy)cyclohexyl)-6-(4-((((3R)-1-(2-(2,6-dioxopiperidin-3-yl)-1,3-dioxoisoindolin-5-yl)piperidin-3-yl)amino)methyl)piperidin-1-yl)pyridazine-3-carboxamide;

N-((1r,4r)-4-(3-chloro-4-cyanophenoxy)cyclohexyl)-6-(4-((((3S)-1-(2-(2,6-dioxopipendin-3-yl)-1,3-dioxoisoindolin-5-yl)piperidin-3-yl)amino)methyl)piperidin-1-yl)pyridazine-3-carboxamide;

N-((1r,4r)-4-(3-chloro-4-cyanophenoxy)cyclohexyl)-6-(4-(((3S)-3-((2-(2,6-dioxopiperidin-3-yl)-1,3-dioxoisoindolin-5-yl)amino)piperidin-1-yl)methyl)piperidin-1-yl)pyridazine-3-carboxamide;

N-((1r,4r)-4-(3-chloro-4-cyanophenoxy)cyclohexyl)-6-(4-(((3R)-3-((2-(2,6-dioxopiperidin-3-yl)-1,3-dioxoisoindolin-5-yl)amino)piperidin-1-yl)methyl)piperidin-1-yl)pyridazine-3-carboxamide;

*N*-((1r,4r)-4-(3-chloro-4-cyanophenoxy)cyclohexyl)-6-(3-((1-(2-(2,6-dioxopiperidin-3-yl)-6-fluoro-1,3-dioxoisoindolin-5-yl)piperidin-4-yl)methyl)-3,8-diazabicyclo[3.2.1]octan-8-yl)pyridazine-3-carboxamide;

*N*-((1r,4r)-4-(3-chloro-4-cyanophenoxy)cyclohexyl)-6-(8-((1-(2-(2,6-dioxopiperidin-3-yl)-6-fluoro-1,3-dioxoisoindolin-5-yl)piperidin-4-yl)methyl)-3,8-diazabicyclo[3.2.1]octan-3-yl)pyridazine-3-carboxamide;

*N*-((1r,4r)-4-(3-chloro-4-cyanophenoxy)cyclohexyl)-6-(3-((1-(2-(2,6-dioxopiperidin-3-yl)-6-fluoro-1,3-dioxoisoindolin-5-yl)piperidin-4-yl)methyl)-3,6-diazabicyclo[3.1.1]heptan-6-yl)pyridazine-3-carboxamide;

*N*-((1r,4r)-4-(3-chloro-4-cyanophenoxy)cyclohexyl)-6-(6-((1-(2-(2,6-dioxopiperidin-3-yl)-6-fluoro-1,3-dioxoisoindolin-5-yl)piperidin-4-yl)methyl)-3,6-diazabicyclo[3.1.1]heptan-3-yl)pyridazine-3-carboxamide;

N-((1r,3r)-3-(3-chloro-4-cyanophenoxy)-2,2,4,4-tetramethylcyclobutyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)nicotinamide;

N-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)nicotinamide;

N-((1r,3r)-3-(3-chloro-4-cyanophenoxy)-2,2,4,4-tetramethylcyclobutyl)-6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)nicotinamide;

N-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)nicotinamide;

N-((1r,3S)-3-(3-chloro-4-cyanophenoxy)-2,2,4,4-tetramethylcyclobutyl)-6-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)nicotinamide;
N-((1r,3R)-3-(3-chloro-4-cyanophenoxy)-2,2,4,4-tetramethylcyclobutyl)-6-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)nicotinamide;
N-((1r,3r)-3-(3-chloro-4-cyanophenoxy)-2,2,4,4-tetramethylcyclobutyl)-6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)nicotinamide;
N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)piperazin-1-yl-2,2,3,3,5,5,6,6-ds)methyl)piperidin-1-yl)nicotinamide;
N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)-3,5-dimethylpiperazin-1-yl)methyl)piperidin-1-yl)nicotinamide;
N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)-2,6-dimethylpiperazin-1-yl)methyl)piperidin-1-yl)nicotinamide;
N-((1r,3r)-3-(3-chloro-4-cyanophenoxy)-2,2,4,4-tetramethylcyclobutyl)-6-(4-((4-(2-(2,6-dioxopiperidin-3-yl)-1,3-dioxoisoindolin-5-yl)piperidin-1-yl)methyl)piperidin-1-yl)nicotinamide;
N-((1r,3r)-3-(3-chloro-4-cyanophenoxy)-2,2,4,4-tetramethylcyclobutyl)-6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)nicotinamide;
N-((1r,3r)-3-(3-chloro-4-cyanophenoxy)-2,2,4,4-tetramethylcyclobutyl)-6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)nicotinamide;
N-((1r,3r)-3-(3-chloro-4-cyanophenoxy)-2,2,4,4-tetramethylcyclobutyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)nicotinamide;
N-((1r,3r)-3-(3-chloro-4-cyanophenoxy)-2,2,4,4-tetramethylcyclobutyl)-6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)nicotinamide;
N-((1r,3r)-3-(3-chloro-4-cyanophenoxy)-2,2,4,4-tetramethylcyclobutyl)-6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)piperidin-1-yl)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)nicotinamide;
N-((lr,3r)-3-(3-chloro-4-cyanophenoxy)-2,2,4,4-tetramethylcyclobutyl)-6-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)nicotinamide;
N-((1r,3R)-3-(3-chloro-4-cyanophenoxy)-2,2,4,4-tetramethylcyclobutyl)-6-(4-((((3R)-1-(2-(2,6-dioxopiperidin-3-yl)-1,3-dioxoisoindolin-5-yl)piperidin-3-yl)amino)methyl)piperidin-1-yl)nicotinamide;
N-((1r,3 S)-3-(3-chloro-4-cyanophenoxy)-2,2,4,4-tetramethylcyclobutyl)-6-(4-((((3S)-1-(2-(2,6-dioxopiperidin-3-yl)-1,3-dioxoisoindolin-5-yl)piperidin-3-yl)amino)methyl)piperidin-1-yl)nicotinamide;
N-((1r,3r)-3-(3-chloro-4-cyanophenoxy)-2,2,4,4-tetramethylcyclobutyl)-6-(4-(((3S)-3-((2-(2,6-dioxopiperidin-3-yl)-1,3-dioxoisoindolin-5-yl)amino)piperidin-1-yl)methyl)piperidin-1-yl)nicotinamide;
N-((lr,3r)-3-(3-chloro-4-cyanophenoxy)-2,2,4,4-tetramethylcyclobutyl)-6-(4-(((3R)-3-((2-(2,6-dioxopiperidin-3-yl)-1,3-dioxoisoindolin-5-yl)amino)piperidin-1-yl)methyl)piperidin-1-yl)nicotinamide;
*N*-((1r,3r)-3-(3-chloro-4-cyanophenoxy)-2,2,4,4-tetramethylcyclobutyl)-6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazine-3-carboxamide;
*N*-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)pyridazine-3-carboxamide;
*N*-((1r,3r)-3-(3-chloro-4-cyanophenoxy)-2,2,4,4-tetramethylcyclobutyl)-6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazine-3-carboxamide;
*N*-((1r,3r)-3-(3-chloro-4-cyanophenoxy)-2,2,4,4-tetramethylcyclobutyl)-6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazine-3 -carboxamide;
5-(4-((4-(4-amino-3-(4-phenoxyphenyl)-1H-pyrazolo[3,4-d]pyrimidin-1-yl)cyclohexyl)methyl)piperazin-1-yl)-2-(2,6-dioxopiperidin-3-yl)isoindoline-1,3-dione;
5-(4-((4-(4-amino-3-(4-phenoxyphenyl)-1H-pyrazolo[3,4-d]pyrimidin-1-yl)-3-fluorocyclohexyl)methyl)piperazin-1-yl)-2-(2,6-dioxopiperidin-3-yl)isoindoline-1,3-dione;
5-(3-((4-(4-amino-3-(4-phenoxyphenyl)-1H-pyrazolo[3,4-d]pyrimidin-l-yl)cyclohexyl)methyl)-3,6-diazabicyclo[3.1.1]heptan-6-yl)-2-(2,6-dioxopiperidin-3-yl)isoindoline-1,3-dione;
5-(3-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-6-yl)-2-(2,6-dioxopiperidin-3-yl)isoindoline-1,3-dione;
5-(6-((4-(4-amino-3-(4-phenoxyphenyl)-1H-pyrazolo[3,4-d]pyrimidin-1-yl)cyclohexyl)methyl)-3,6-diazabicyclo[3.1.1]heptan-3-yl)-2-(2,6-dioxopiperidin-3-yl)isoindoline-1,3-dione;
5-(6-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-3-yl)-2-(2,6-dioxopiperidin-3-yl)isoindoline-1,3-dione;
5-(3-((4-(4-amino-3-(4-phenoxyphenyl)-1H-pyrazolo[3,4-d]pyrimidin-1-yl)cyclohexyl)methyl)-3,8-diazabicyclo[3.2.1]octan-8-yl)-2-(2,6-dioxopiperidin-3-yl)isoindoline-1,3-dione;
5-(3-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-8-yl)-2-(2,6-dioxopiperidin-3-yl)isoindoline-1,3-dione;
5-(8-((4-(4-amino-3-(4-phenoxyphenyl)-1H-pyrazolo[3,4-d]pyrimidin-1-yl)cyclohexyl)methyl)-3,8-diazabicyclo[3.2.1]octan-3-yl)-2-(2,6-dioxopiperidin-3-yl)isoindoline-1,3-dione;
5-(8-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-3-yl)-2-(2,6-dioxopiperidin-3-yl)isoindoline-1,3-dione;
5-((1S,4S)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)isoindoline-1,3-dione;
5-((1S,4S)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)isoindoline-1,3-dione;
5-((1R,4R)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)isoindoline-1,3-dione;
5-((1R,4R)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)isoindoline-1,3-dione;
5-(5-((4-(4-amino-3-(4-phenoxyphenyl)-1H-pyrazolo[3,4-d]pyrimidin-1-yl)cyclohexyl)methyl)-2,5-diazabicyclo[2.2.2]octan-2-yl)-2-(2,6-dioxopiperidin-3-yl)isoindoline-1,3-dione;
5-(5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.2]octan-2-yl)-2-(2,6-dioxopiperidin-3-yl)isoindoline-1,3-dione;
5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)isoindoline-1,3-dione;
5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)isoindoline-1,3-dione;
5-(4-((4-(4-amino-3-(4-phenoxyphenyl)-1H-pyrazolo[3,4-d]pyrimidin-1-yl)cyclohexyl)methyl)-2,6-dimethylpiperazin-1-yl)-2-(2,6-dioxopiperidin-3-yl)isoindoline-1,3-dione;
5-(4-((4-(4-amino-3-(4-phenoxyphenyl)-1H-pyrazolo[3,4-d]pyrimidin-1-yl)-3-fluorocyclohexyl)methyl)-2,6-dimethylpiperazin-1-yl)-2-(2,6-dioxopiperidin-3-yl)isoindoline-1,3-dione;
5-(4-((4-(4-amino-3-(4-phenoxyphenyl)-1H-pyrazolo[3,4-d]pyrimidin-1-yl)cyclohexyl)methyl)-3,5-dimethylpiperazin-1-yl)-2-(2,6-dioxopiperidin-3-yl)isoindoline-1,3-dione;
5-(4-((4-(4-amino-3-(4-phenoxyphenyl)-1H-pyrazolo[3,4-d]pyrimidin-1-yl)-3-fluorocyclohexyl)methyl)-3,5-dimethylpiperazin-1-yl)-2-(2,6-dioxopiperidin-3-yl)isoindoline-1,3-dione;
5-(1-((4-(4-amino-3-(4-phenoxyphenyl)-1H-pyrazolo[3,4-d]pyrimidin-1-yl)cyclohexyl)methyl)piperidin-4-yl)-2-(2,6-dioxopiperidin-3-yl)isoindoline-1,3-dione;
5-(1-((4-(4-amino-3-(4-phenoxyphenyl)-1H-pyrazolo[3,4-d]pyrimidin-l-yl)-3-fluorocyclohexyl)methyl)piperidin-4-yl)-2-(2,6-dioxopiperidin-3-yl)isoindoline-1,3-dione;
5-(4-((4-(4-amino-3-(4-phenoxyphenyl)-1H-pyrazolo[3,4-d]pyrimidin-1-yl)cyclohexyl)methyl)piperazin-1-yl)-2-(2,6-dioxopiperidin-3-yl)-6-fluoroisoindoline-1,3-dione;
5-(4-((4-(4-amino-3-(4-phenoxyphenyl)-1H-pyrazolo[3,4-d]pyrimidin-1-yl)-3-fluorocyclohexyl)methyl)piperazin-1-yl)-2-(2,6-dioxopiperidin-3-yl)-6-fluorolsoindoline-1,3-dione;
5-(3-((4-(4-amino-3-(4-phenoxyphenyl)-1H-pyrazolo[3,4-d]pyrimidin-1-yl)cyclohexyl)methyl)-3,6-diazabicyclo[3.1.1]heptan-6-yl)-2-(2,6-dioxopiperidin-3-yl)-6-fluoroisoindoline-1,3-dione;
5-(3-((4-(4-amino-3 -(4-phenoxyphenyl)-1H-pyrazolo [3,4-d]pyrimidin-1 -yl)-3 - fluorocyclohexyl)methyl)-3,6-diazabicyclo[3.1.1]heptan-6-yl)-2-(2,6-dioxopiperidin-3-yl)-6-fluoroisoindoline-1,3 -dione;

5-(6-((4-(4-amino-3-(4-phenoxyphenyl)-1H-pyrazolo[3,4-d]pyrimidin-1-yl)cyclohexyl)methyl)-3,6-diazabicyclo[3.1.1]heptan-3-yl)-2-(2,6-dioxopiperidin-3-yl)-6-fluoroisoindoline-1,3-dione;
5-(6-((4-(4-amino-3-(4-phenoxyphenyl)-1H-pyrazolo[3,4-d]pyrimidin-1-yl)-3-fluorocyclohexyl)methyl)-3,6-diazabicyclo[3.1.1]heptan-3-yl)-2-(2,6-dioxopiperidin-3-yl)-6-fluoroisoindoline-1,3 -dione;
5-(3-((4-(4-amino-3-(4-phenoxyphenyl)-1H-pyrazolo[3,4-d]pyrimidin-1-yl)cyclohexyl)methyl)-3,8-diazabicyclo[3.2.1]octan-8-yl)-2-(2,6-dioxopiperidin-3-yl)-6-fluoroisoindoline-1,3-dione;
5-(3-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-8-yl)-2-(2,6-dioxopiperidin-3-yl)-6-fluoroisoindoline-1,3 -dione;
5-(8-((4-(4-amino-3-(4-phenoxyphenyl)-1H-pyrazolo[3,4-d]pyrimidin-1-yl)cyclohexyl)methyl)-3,8-diazabicyclo[3.2.1]octan-3-yl)-2-(2,6-dioxopiperidin-3-yl)-6-fluoroisoindoline-1,3-dione;
5-(8-((4-(4-amino-3-(4-phenoxyphenyl)-1H-pyrazolo[3,4-d]pyrimidin-1-yl)-3-fluorocyclohexyl)methyl)-3,8-diazabicyclo[3.2.1]octan-3-yl)-2-(2,6-dioxopiperidin-3-yl)-6-fluoroisoindoline-1,3 -dione;
5-((1S,4S)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)-6-fluoroisoindoline-1,3 -dione;
5-((1S,4S)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)-6-fluoroisoindoline-1,3 -dione;
5-((1R,4R)-5-((4-(4-amino-3-(4-phenoxyphenyl)-1H-pyrazolo[3,4-d]pyrimidin-1-yl)cyclohexyl)methyl)-2,5-diazabicyclo[2.2.1]heptan-2-yl)-2-(2,6-dioxopiperidin-3-yl)-6-fluoroisoindoline-1,3 -dione;
5-((1R,4R)-5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.1]heptan-2-yl)-2-(2,6-dioxopiperidin-3-yl)-6-fluoroisoindoline-1,3 -dione;
5-(5-((4-(4-amino-3-(4-phenoxyphenyl)-1H-pyrazolo[3,4-d]pyrimidin-1-yl)cyclohexyl)methyl)-2,5-diazabicyclo[2.2.2]octan-2-yl)-2-(2,6-dioxopiperidin-3-yl)-6-fluoroisoindoline-1,3-dione;
5-(5-((4-(4-amino-3-(4-phenoxyphenyl)-1H-pyrazolo[3,4-d]pyrimidin-1-yl)-3-fluorocyclohexyl)methyl)-2,5-diazabicyclo[2.2.2]octan-2-yl)-2-(2,6-dioxopiperidin-3-yl)-6-fluoroisoindoline-1,3 -dione;
5-(4-((4-(4-amino-3-(4-phenoxyphenyl)-1H-pyrazolo[3,4-d]pyrimidin-1-yl)cyclohexyl)methyl)-2,6-dimethylpiperazin-1-yl)-2-(2,6-dioxopiperidin-3-yl)-6-fluoroisoindoline-1,3-dione;
5-(4-((4-(4-amino-3-(4-phenoxyphenyl)-1H-pyrazolo[3,4-d]pyrimidin-1-yl)-3-fluorocyclohexyl)methyl)-2,6-dimethylpiperazin-1-yl)-2-(2,6-dioxopiperidin-3-yl)-6-fluoroisoindoline-1,3 -dione;
5-(4-((4-(4-amino-3-(4-phenoxyphenyl)-1H-pyrazolo[3,4-d]pyrimidin-1-yl)cyclohexyl)methyl)-3,5-dimethylpiperazin-1-yl)-2-(2,6-dioxopiperidin-3-yl)-6-fluoroisoindoline-1,3-dione;
5-(4-((4-(4-amino-3-(4-phenoxyphenyl)-1H-pyrazolo[3,4-d]pyrimidin-1-yl)-3-fluorocyclohexyl)methyl)-3,5-dimethylpiperazin-1-yl)-2-(2,6-dioxopiperidin-3-yl)-6-fluoroisoindoline-1,3 -dione;
5-(1-((4-(4-amino-3-(4-phenoxyphenyl)-1H-pyrazolo[3,4-d]pyrimidin-1-yl)cyclohexyl)methyl)piperidin-4-yl)-2-(2,6-dioxopiperidin-3-yl)-6-fluoroisoindoline-1,3-dione;
5-(1-((4-(4-amino-3-(4-phenoxyphenyl)-1H-pyrazolo[3,4-d]pyrimidin-1-yl)-3-fluorocyclohexyl)methyl)piperidin-4-yl)-2-(2,6-dioxopiperidin-3-yl)-6-fluoroisoindoline-1,3-dione;
5-(4-((4-(4-amino-3-(4-phenoxyphenyl)-1H-pyrazolo[3,4-d]pyrimidin-1-yl)cyclohexyl)methyl)piperazin-l-yl-2,2,3,3,5,5,6,6-$d_8$)-2-(2,6-dioxopiperidin-3-yl)-6-fluoroisoindoline-1,3 -dione;
5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-6-fluoroisoindoline-1,3 -dione;
6-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4-fluoroisoindoline-1,3 -dione;
6-(4-((4-(4-amino-3 -(4-phenoxyphenyl)-1H-pyrazolo[3,4-d]pyrimidin-1-yl)-3 - fluorocyclohexyl)methyl)piperazin-1-yl-2,2,3,3,5,5,6,6-$d_8$)-2-(2,6-dioxopiperidin-3-yl)-4-fluoroisoindoline-1,3 -dione;
5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4-fluoroisoindoline-1,3 -dione;
5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4-fluoroisoindoline-1,3 -dione;
5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,7-difluoroisoindoline-1,3-dione;
5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,7-difluoroisoindoline-1,3-dione;
5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,6-difluoroisoindoline-1,3-dione;
5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,6-difluoroisoindoline-1,3-dione;
6- (4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,5-difluoroisoindoline-1,3-dione;

6-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,5-difluoroisoindoline-1,3-dione;

5-(4-((4-(4-amino-3-(4-phenoxyphenyl)-1H-pyrazolo[3,4-d]pyrimidin-1-yl)cyclohexyl)methyl)piperazin-1-yl-2,2,3,3,5,5,6,6-$d_8$)-2-(2,6-dioxopiperidin-3-yl)-4,6,7-trifluoroisoindoline-1,3-dione;

5-(4-((4-(4-amino-3-(4-phenoxyphenyl)-1H-pyrazolo[3,4-d]pyrimidin-1-yl)-3-fluorocyclohexyl)methyl)piperazin-1-yl-2,2,3,3,5,5,6,6-$d_8$)-2-(2,6-dioxopiperidin-3-yl)-4,6,7-trifluoroisoindoline-1,3-dione;

5-((R)-3-(((4-(4-amino-3-(4-phenoxyphenyl)-1H-pyrazolo[3,4-d]pyrimidin-1-yl)cyclohexyl)methyl)amino)piperidin-1-yl)-2-(2,6-dioxopiperidin-3-yl)isoindoline-1,3-dione;

5-((3R)-3-(((4-(4-amino-3-(4-phenoxyphenyl)-1H-pyrazolo[3,4-d]pyrimidin-1-yl)-3-fluorocyclohexyl)methyl)amino)piperidin-1-yl)-2-(2,6-dioxopiperidin-3-yl)isoindoline-1,3-dione;

5-((S)-3-(((4-(4-amino-3-(4-phenoxyphenyl)-1H-pyrazolo[3,4-d]pyrimidin-1-yl)cyclohexyl)methyl)amino)piperidin-1-yl)-2-(2,6-dioxopiperidin-3-yl)isoindoline-1,3-dione;

5-((3S)-3-(((4-(4-amino-3-(4-phenoxyphenyl)-1H-pyrazolo[3,4-d]pyrimidin-1-yl)-3-fluorocyclohexyl)methyl)amino)piperidin-1-yl)-2-(2,6-dioxopiperidin-3-yl)isoindoline-1,3-dione;

5-(((S)-1-((4-(4-amino-3-(4-phenoxyphenyl)-1H-pyrazolo[3,4-d]pyrimidin-1-yl)cyclohexyl)methyl)piperidin-3-yl)amino)-2-(2,6-dioxopiperidin-3-yl)isoindoline-1,3-dione;

5-(((3S)-1-((4-(4-amino-3-(4-phenoxyphenyl)-1H-pyrazolo[3,4-d]pyrimidin-1-yl)-3-fluorocyclohexyl)methyl)piperidin-3-yl)amino)-2-(2,6-dioxopiperidin-3-yl)isoindoline-1,3-dione;

5-(((R)-1-((4-(4-amino-3-(4-phenoxyphenyl)-1H-pyrazolo[3,4-d]pyrimidin-1-yl)cyclohexyl)methyl)piperidin-3-yl)amino)-2-(2,6-dioxopiperidin-3-yl)isoindoline-1,3-dione;

5-(((3R)-1-((4-(4-amino-3-(4-phenoxyphenyl)-1H-pyrazolo[3,4-d]pyrimidin-1-yl)-3-fluorocyclohexyl)methyl)piperidin-3-yl)amino)-2-(2,6-dioxopiperidin-3-yl)isoindoline-1,3-dione;

3-(tert-butyl)-N-(4-(5-(4-(4-((4-(2-(2,6-dioxopiperidin-3-yl)-1,3-dioxoisoindolin-5-yl)piperazin-1-yl)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((4-(2-(2,6-dioxopiperidin-3-yl)-1,3-dioxoisoindolin-5-yl)piperazin-1-yl-2,2,3,3,5,5,6,6-ds)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)phenyl)-1H-indazol-3-yl)-2-methylbenzyl)-1,2,4-oxadiazole-5-carboxamide;

3-(tert-butyl)-N-(4-(5-(4-(4-((4-(2-(2,6-dioxopiperidin-3-yl)-4,6-difluoro-1,3-dioxoisoindolin-5-yl)piperazin-1-yl-2,2,3,3,5,5,6,6-ds)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((4-(2-(2,6-dioxopiperidin-3-yl)-4,6,7-trifluoro-1,3-dioxoisoindolin-5-yl)piperazin-1-yl-2,2,3,3,5,5,6,6-ds)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((4-(2-(2,6-dioxopiperidin-3-yl)-1,3-dioxoisoindolin-5-yl)-3,5-dimethylpiperazin-1-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((4-(2-(2,6-dioxopiperidin-3-yl)-1,3-dioxoisoindolin-5-yl)-2,6-dimethylpiperazin-1-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((4-(2-(2,6-dioxopiperidin-3-yl)-1,3-dioxoisoindolin-5-yl)piperidin-1-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((4-(2-(2,6-dioxopiperidin-3-yl)-6-fluoro-1,3-dioxoisoindolin-5-yl)piperazin-1-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((1S,4S)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((1R,4R)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((4-(2-(2,6-dioxopiperidin-3-yl)-6-fluoro-1,3-dioxoisoindolin-5-yl)piperidin-1-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((((3R)-1-(2-(2,6-dioxopiperidin-3-yl)-1,3-dioxoisoindolin-5-yl)piperidin-3-yl)amino)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-((((3 S)-1-(2-(2,6-dioxopiperidin-3-yl)-1,3-dioxoisoindolin-5-yl)piperidin-3-yl)amino)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((3 S)-3-((2-(2,6-dioxopiperidin-3-yl)-1,3-dioxoisoindolin-5-yl)amino)piperidin-1-yl)methyl)piperidin-1-yl)phenyl)-1H-indazol-3-yl)-2-methylbenzyl)-1,2,4-oxadiazole-5-carboxamide;
3-(tert-butyl)-N-(4-(5-(4-(4-(((3R)-3-((2-(2,6-dioxopiperidin-3-yl)-1,3-dioxoisoindolin-5-yl)amino)piperidin-1-yl)methyl)piperidin-1-yl)phenyl)-1H-indazol-3-yl)-2-methylbenzyl)-1,2,4-oxadiazole-5-carboxamide;
2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl)isoindoline-1,3-dione;
2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl)isoindoline-1,3-dione;
2-(2,6-dioxopiperidin-3-yl)-5-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-6-yl)isoindoline-1,3-dione;
2-(2,6-dioxopiperidin-3-yl)-5-fluoro-6-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-6-yl)isoindoline-1,3-dione;

2-(2,6-dioxopiperidin-3-yl)-5-(6-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-3-yl)isoindoline-1,3-dione;

2-(2,6-dioxopiperidin-3-yl)-5-fluoro-6-(6-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,6-diazabicyclo[3.1.1]heptan-3-yl)isoindoline-1,3-dione;

2-(2,6-dioxopiperidin-3-yl)-5-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-8-yl)isoindoline-1,3-dione;

2-(2,6-dioxopiperidin-3-yl)-5-fluoro-6-(3-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-8-yl)isoindoline-1,3-dione;

2-(2,6-dioxopiperidin-3-yl)-5-fluoro-6-(8-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-3-yl)isoindoline-1,3-dione;

2-(2,6-dioxopiperidin-3-yl)-5-(8-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,8-diazabicyclo[3.2.1]octan-3-yl)isoindoline-1,3-dione;

2-(2,6-dioxopiperidin-3-yl)-5-((1S,4S)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione;

2-(2,6-dioxopiperidin-3-yl)-5-fluoro-6-((1S,4S)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione;

2-(2,6-dioxopiperidin-3-yl)-5-((1R,4R)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione;

2-(2,6-dioxopiperidin-3-yl)-5-fluoro-6-((1R,4R)-5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.1]heptan-2-yl)isoindoline-1,3-dione;

2-(2,6-dioxopiperidin-3-yl)-5-(5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.2]octan-2-yl)isoindoline-1,3-dione;

2-(2,6-dioxopiperidin-3-yl)-5-fluoro-6-(5-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,5-diazabicyclo[2.2.2]octan-2-yl)isoindoline-1,3-dione;

2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-4-fluoro-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-4-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-ds)isoindoline-1,3-dione;

2-(2,6-dioxopiperidin-3-yl)-4,5-difluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-ds)isoindoline-1,3-dione;

2-(2,6-dioxopiperidin-3-yl)-4,7-difluoro-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-ds)isoindoline-1,3-dione;

2-(2,6-dioxopiperidin-3-yl)-4,6-difluoro-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-ds)isoindoline-1,3-dione;

2-(2,6-dioxopiperidin-3-yl)-4,5,7-trifluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperazin-1-yl-2,2,3,3,5,5,6,6-ds)isoindoline-1,3-dione;

2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,6-dimethylpiperazin-1-yl)isoindoline-1,3 -dione;

2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-2,6-dimethylpiperazin-1-yl)isoindoline-1,3 -dione;

2-(2,6-dioxopiperidin-3-yl)-5-fluoro-6-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,5-dimethylpiperazin-1-yl)isoindoline-1,3 -dione;

2-(2,6-dioxopiperidin-3-yl)-5-(4-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)-3,5-dimethylpiperazin-1-yl)isoindoline-1,3 -dione;

2-(2,6-dioxopiperidin-3-yl)-5-(1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperi din-4-yl)isoindoline-1,3-dione;

2-(2,6-dioxopiperidin-3-yl)-5-fluoro-6-(1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperidin-4-yl)isoindoline-1,3-dione;

2-(2,6-dioxopiperidin-3-yl)-5-((R)-3-(((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)amino)piperidin-1-yl)isoindoline-1,3-dione;

2-(2,6-dioxopiperidin-3-yl)-5-((S)-3-(((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)amino)piperidin-1-yl)isoindoline-1,3-dione;

2-(2,6-dioxopiperidin-3-yl)-5-(((R)-1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperidin-3-yl)amino)isoindoline-1,3 -dione;

2-(2,6-dioxopiperidin-3-yl)-5-(((S)-1-((1-((6-((5-fluoro-4-(4-fluoro-1-isopropyl-2-methyl-1H-benzo[d]imidazol-6-yl)pyrimidin-2-yl)amino)pyridin-3-yl)methyl)piperidin-4-yl)methyl)piperidin-3-yl)amino)isoindoline-1,3 -dione;

7-cyclopentyl-2-((5-(4-((4-(2-(2,6-dioxopiperidin-3-yl)-1,3-dioxoisoindolin-5-yl)piperazin-1-yl)methyl)piperidin-1-yl)pyridin-2-yl)amino)-N,N-dimethyl-7H-pyrrolo[2,3-d]pyrimidine-6-carboxamide;

7-cyclopentyl-2-((5-(4-((4-(2-(2,6-dioxopiperidin-3-yl)-6-fluoro-1,3-dioxoisoindolin-5-yl)piperazin-1-yl)methyl)piperidin-1-yl)pyridin-2-yl)amino)-N,N-dimethyl-7H-pyrrolo[2,3 - d]pyrimidine-6-carboxamide;

7-cyclopentyl-2-((5-(4-((6-(2-(2,6-dioxopiperidin-3-yl)-6-fluoro-1,3-dioxoisoindolin-5-yl)-3,6-diazabicyclo [3.1.1]heptan-3-yl)methyl)piperidin-1-yl)pyridin-2-yl)amino)-N,N-dimethyl-7H-pyrrolo[2,3 -d]pyrimidine-6-carboxamide;

7-cyclopentyl-2-((5-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridin-2-yl)amino)-N,N-dimethyl-7H-pyrrolo [2,3-d]pyrimidine-6-carboxamide;

7-cyclopentyl-2-((5-(4-((3-(2-(2,6-dioxopiperidin-3-yl)-1,3-dioxoisoindolin-5-yl)-3,6-diazabicyclo[3 .1.1]heptan-6-yl)methyl)piperidin-1-yl)pyridin-2-yl)amino)-N,N-dimethyl-7H-pyrrolo[2,3 -d]pyrimidine-6-carboxamide;

7-cyclopentyl-2-((5-(4-((3-(2-(2,6-dioxopiperidin-3-yl)-6-fluoro-1,3-dioxoisoindolin-5-yl)-3,6-diazabicyclo[3 .1.1]heptan-6-yl)methyl)piperidin-1-yl)pyridin-2-yl)amino)-N,N-dimethyl-7H-pyrrolo[2,3 -d]pyrimidine-6-carboxamide;

7-cyclopentyl-2-((5-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3 -d]pyrimidine-6-carboxamide;

7-cyclopentyl-2-((5-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridin-2-yl)amino)-N,N-dimethyl-7H-pyrrolo[2,3 -d]pyrimidine-6-carboxamide;

7-cyclopentyl-2-((5-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridin-2-yl)amino)-N,N-dimethyl-7H-pyrrolo[2,3 -d]pyrimidine-6-carboxamide;

7-cyclopentyl-2-((5-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridin-2-yl)amino)-N,N-dimethyl-7H-pyrrolo[2,3 -d]pyrimidine-6-carboxamide;

7-cyclopentyl-2-((5-(4-(((1R,4R)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3 -d]pyrimidine-6-carboxamide;

7-cyclopentyl-2-((5-(4-(((1S,4S)-5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3 -d]pyrimidine-6-carboxamide;

7-cyclopentyl-2-((5-(4-(((1S,4S)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-(((1R,4R)-5-(2-(2,6-dioxopiperidin-3-yl)-1,3-dioxoisoindolin-5-yl)-2,5-diazabicyclo[2.2.1]heptan-2-yl)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)pyridin-2-yl)amino)-N,N-dimethyl-7H-pyrrolo[2,3 -d]pyrimidine-6-carboxamide;
7-cyclopentyl-2-((5-(4-((4-(2-(2,6-dioxopiperidin-3-yl)-1,3-dioxoisoindolin-5-yl)-3,5-dimethylpiperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-1,3-dioxoisoindolin-5-yl)-2,6-dimethylpiperazin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-1,3-dioxoisoindolin-5-yl)piperidin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((4-(2-(2,6-dioxopiperidin-3-yl)-6-fluoro-1,3-dioxoisoindolin-5-yl)piperidin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((((3R)-1-(2-(2,6-dioxopiperidin-3-yl)-1,3-dioxoisoindolin-5-yl)piperidin-3-yl)amino)methyl)piperidin-1-yl)pyridin-2-yl)amino)-N,N-dimethyl-7H-pyrrolo[2,3-d]pyrimidine-6-carboxamide;
7-cyclopentyl-2-((5-(4-((((3S)-1-(2-(2,6-dioxopiperidin-3-yl)-1,3-dioxoisoindolin-5-yl)piperidin-3-yl)amino)methyl)piperidin-1-yl)pyridin-2-yl)amino)-N,N-dimethyl-7H-pyrrolo[2,3-d]pyrimidine-6-carboxamide;
7-cyclopentyl-2-((5-(4-(((3 S)-3-((2-(2,6-dioxopiperidin-3-yl)-1,3-dioxoisoindolin-5-yl)amino)piperidin-1-yl)methyl)piperidin-1-yl)pyridin-2-yl)amino)-N,N-dimethyl-7H-pyrrolo[2,3-d]pyrimidine-6-carboxamide;
7-cyclopentyl-2-((5-(4-(((3R)-3-((2-(2,6-dioxopiperidin-3-yl)-1,3-dioxoisoindolin-5-yl)amino)piperidin-1-yl)methyl)piperidin-1-yl)pyridin-2-yl)amino)-N,N-dimethyl-7H-pyrrolo[2,3-d]pyrimidine-6-carboxamide;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl)-2-(2,6-dioxopiperidin-3-yl)isoindoline-1,3-dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl)-2-(2,6-dioxopiperidin-3-yl)-6-fluoroisoindoline-1,3 -dione;
5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-6-yl)-2-(2,6-dioxopiperidin-3-yl)isoindoline-1,3-dione;
5-(6-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-3-yl)-2-(2,6-dioxopiperidin-3-yl)isoindoline-1,3-dione;
5-(6-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,6-diazabicyclo[3.1.1]heptan-3-yl)-2-(2,6-dioxopiperidin-3-yl)-6-fluoroisoindoline-1,3-dione;
5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-8-yl)-2-(2,6-dioxopiperidin-3-yl)-6-fluoroisoindoline-1,3-dione;
5-(3-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-8-yl)-2-(2,6-dioxopiperidin-3-yl)isoindoline-1,3-dione;
5-(8-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-3-yl)-2-(2,6-dioxopiperidin-3-yl)isoindoline-1,3-dione;
5-(8-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,8-diazabicyclo[3.2.1]octan-3-yl)-2-(2,6-dioxopiperidin-3-yl)-6-fluoroisoindoline-1,3-dione;
5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.1]heptan-2-yl)-2-(2,6-dioxopiperidin-3-yl)isoindoline-1,3-dione;
5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.2]octan-2-yl)-2-(2,6-dioxopiperidin-3-yl)isoindoline-1,3-dione;
5-(5-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
6-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
6-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,6-dimethylpiperazin-1-yl)-2-(2,6-dioxopiperidin-3-yl)isoindoline-1,3 -dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-2,6-dimethylpiperazin-1-yl)-2-(2,6-dioxopiperidin-3-yl)-6-fluoroisoindoline-1,3 -dione;
5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,5-dimethylpiperazin-1-yl)-2-(2,6-dioxopiperidin-3-yl)-6-fluoroisoindoline-1,3 -dione;

5-(4-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)-3,5-dimethylpiperazin-1-yl)-2-(2,6-dioxopiperidin-3-yl)isoindoline-1,3-dione;
5-(1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-4-yl)-2-(2,6-dioxopiperidin-3-yl)isoindoline-1,3-dione;
5-(1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-4-yl)-2-(2,6-dioxopiperidin-3-yl)-6-fluoroisoindoline-1,3 -dione;
5-((R)-3-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)amino)piperidin-1-yl)-2-(2,6-dioxopiperidin-3-yl)isoindoline-1,3-dione;
5-((S)-3-(((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)amino)piperidin-1-yl)-2-(2,6-dioxopiperidin-3-yl)isoindoline-1,3-dione;
5-(((S)-1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-3-yl)amino)-2-(2,6-dioxopiperidin-3-yl)isoindoline-1,3-dione;
5-(((R)-1-((1-(6-((6-acetyl-8-cyclopentyl-5-methyl-7-oxo-7,8-dihydropyrido[2,3-d]pyrimidin-2-yl)amino)pyridin-3-yl)piperidin-4-yl)methyl)piperidin-3-yl)amino)-2-(2,6-dioxopiperidin-3-yl)isoindoline-1,3-dione;
5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl)-2-(2,6-dioxopiperidin-3-yl)isoindoline-1,3-dione;
5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl)-2-(2,6-dioxopiperidin-3-yl)-6-fluoroisoindoline-1,3-dione;
5-(3-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-6-yl)-2-(2,6-dioxopiperidin-3-yl)-6-fluoroisoindoline-1,3 -dione;
5-(3-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-6-yl)-2-(2,6-dioxopiperidin-3-yl)isoindoline-1,3-dione;
5-(6-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(4-((5-chloro-4-((2-(dimethylphosphoryl)phenyl)amino)pyrimidin-2-yl)amino)-3-methoxyphenyl)piperidin-4-yl)methyl)-3,6-diazabicyclo[3.1.1]heptan-3-yl)-2-(2,6-dioxopiperidin-3-yl)-6-fluoroisoindoline-1,3 -dione;
5-(3-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-8-yl)-2-(2,6-dioxopiperidin-3-yl)-6-fluoroisoindoline-1,3-dione;
5-(3-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-8-yl)-2-(2,6-dioxopiperidin-3-yl)isoindoline-1,3-dione;
5-(8-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-3-yl)-2-(2,6-dioxopiperidin-3-yl)isoindoline-1,3-dione;
5-(8-((1-(4-((5-chloro-4-((2-(dimethylphosphoryl)phenyl)amino)pyrimidin-2-yl)amino)-3-methoxyphenyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.2]octan-2-yl)-2-(2,6-dioxopiperidin-3-yl)isoindoline-1,3-dione;
5-(5-((1-(4-((5-chloro-4-((2-(dimethylphosphoryl)phenyl)amino)pyrimidin-2-yl)amino)-3-methoxyphenyl)piperidin-4-yl)methyl)-2,5-diazabicyclo[2.2.2]octan-2-yl)-2-(2,6-dioxopiperidin-3-yl)-6-fluoroisoindoline-1,3 -dione;
5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)isoindoline-1,3 -dione;
5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-6-fluoroisoindoline-1,3 -dione;
6-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4-fluoroisoindoline-1,3 -dione;
5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4-fluoroisoindoline-1,3 -dione;
5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
6-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,5 -difluoroisoindoline-1,3-dione;
5-(4-((1-(4-((5-chloro-4-((2-(dimethylphosphoryl)phenyl)amino)pyrimidin-2-yl)amino)-3-methoxyphenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(4-((5-chloro-4-((2-(dimethylphosphoryl)phenyl)amino)pyrimidin-2-yl)amino)-3-methoxyphenyl)piperidin-4-yl)methyl)-2,6-dimethylpiperazin-1-yl)-2-(2,6-dioxopiperidin-3-yl)isoindoline-1,3-dione;
5-(4-((1-(4-((5-chloro-4-((2-(dimethylphosphoryl)phenyl)amino)pyrimidin-2-yl)amino)-3-methoxyphenyl)piperidin-4-yl)methyl)-2,6-dimethylpiperazin-1-yl)-2-(2,6-dioxopiperidin-3-yl)-6-fluoroisoindoline-1,3-dione;
5-(4-((1-(4-((5-chloro-4-((2-(dimethylphosphoryl)phenyl)amino)pyrimidin-2-yl)amino)-3-methoxyphenyl)piperidin-4-yl)methyl)-3,5-dimethylpiperazin-1-yl)-2-(2,6-dioxopiperidin-3-yl)isoindoline-1,3-dione;
5-(4-((1-(4-((5-chloro-4-((2-(dimethylphosphoryl)phenyl)amino)pyrimidin-2-yl)amino)-3-methoxyphenyl)piperidin-4-yl)methyl)-3,5-dimethylpiperazin-1-yl)-2-(2,6-dioxopiperidin-3-yl)-6-fluoroisoindoline-1,3-dione;
5-(1-((1-(4-((5-chloro-4-((2-(dimethylphosphoryl)phenyl)amino)pyrimidin-2-yl)amino)-3-methoxyphenyl)piperidin-4-yl)methyl)piperidin-4-yl)-2-(2,6-dioxopiperidin-3-yl)-6-fluoroisoindoline-1,3-dione;
5-(1-((1-(4-((5-chloro-4-((2-(dimethylphosphoryl)phenyl)amino)pyrimidin-2-yl)amino)-3-methoxyphenyl)piperidin-4-yl)methyl)piperidin-4-yl)-2-(2,6-dioxopiperidin-3-yl)isoindoline-1,3-dione;
5-((R)-3-(((1-(4-((5-chloro-4-((2-(dimethylphosphoryl)phenyl)amino)pyrimidin-2-yl)amino)-3-methoxyphenyl)piperidin-4-yl)methyl)amino)piperidin-1-yl)-2-(2,6-dioxopiperidin-3-yl)isoindoline-1,3-dione;
5-((S)-3-(((1-(4-((5-chloro-4-((2-(dimethylphosphoryl)phenyl)amino)pyrimidin-2-yl)amino)-3-methoxyphenyl)piperidin-4-yl)methyl)amino)piperidin-1-yl)-2-(2,6-dioxopiperidin-3-yl)isoindoline-1,3-dione;
5-(((S)-1-((1-(4-((5-chloro-4-((2-(dimethylphosphoryl)phenyl)amino)pyrimidin-2-yl)amino)-3-methoxyphenyl)piperidin-4-yl)methyl)piperidin-3-yl)amino)-2-(2,6-dioxopiperidin-3-yl)isoindoline-1,3-dione;
5-(((R)-1-((1-(4-((5-chloro-4-((2-(dimethylphosphoryl)phenyl)amino)pyrimidin-2-yl)amino)-3-methoxyphenyl)piperidin-4-yl)methyl)piperidin-3-yl)amino)-2-(2,6-dioxopiperidin-3-yl)isoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl)-2-(2,6-dioxopiperidin-3-yl)isoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl)-2-(2,6-dioxopiperidin-3-yl)-6-fluoroisoindoline-1,3 -dione;
5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-6-yl)-2-(2,6-dioxopiperidin-3-yl)isoindoline-1,3-dione;
5-(6-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,6-diazabicyclo[3.1.1]heptan-3-yl)-2-(2,6-dioxopiperidin-3-yl)-6-fluoroisoindoline-1,3-dione;
5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-8-yl)-2-(2,6-dioxopiperidin-3-yl)-6-fluoroisoindoline-1,3-dione;
5-(3-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-8-yl)-2-(2,6-dioxopiperidin-3-yl)isoindoline-1,3-dione;
5-(8-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-3-yl)-2-(2,6-dioxopiperidin-3-yl)isoindoline-1,3-dione;
5-(8-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.2]octan-2-yl)-2-(2,6-dioxopiperidin-3-yl)isoindoline-1,3-dione;
5-(5-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
6-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
6-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,6-dimethylpiperazin-1-yl)-2-(2,6-dioxopiperidin-3-yl)isoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-2,6-dimethylpiperazin-1-yl)-2-(2,6-dioxopiperidin-3-yl)-6-fluoroisoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,5-dimethylpiperazin-1-yl)-2-(2,6-dioxopiperidin-3-yl)isoindoline-1,3-dione;
5-(4-((1-(4-((5-chloro-4-((2-(isopropylsulfonyl)phenyl)amino)pyrimidin-2-yl)amino)-5-isopropoxy-2-methylphenyl)piperidin-4-yl)methyl)-3,5-dimethylpiperazin-1-yl)-2-(2,6-dioxopiperidin-3-yl)-6-fluoroisoindoline-1,3-dione;
5-(1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-4-yl)-2-(2,6-dioxopiperi din-3-yl)isoindoline-1,3-dione;
5-(1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-4-yl)-2-(2,6-dioxopiperidin-3-yl)-6-fluoroisoindoline-1,3 -dione;
5-((R)-3-(((1-(4-((5-chloro-4-((2-(isopropylsulfonyl)phenyl)amino)pyrimidin-2-yl)amino)-5-isopropoxy-2-methylphenyl)piperidin-4-yl)methyl)amino)piperidin-1-yl)-2-(2,6-dioxopiperidin-3-yl)isoindoline-1,3-dione;
5-((S)-3-(((1-(4-((5-chloro-4-((2-(isopropylsulfonyl)phenyl)amino)pyrimidin-2-yl)amino)-5-isopropoxy-2-methylphenyl)piperidin-4-yl)methyl)amino)piperidin-1-yl)-2-(2,6-dioxopiperidin-3-yl)isoindoline-1,3-dione;
5-(((S)-1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-3-yl)amino)-2-(2,6-dioxopiperidin-3-yl)isoindoline-1,3-dione;
5-(((R)-1-((1-(4-((5-chloro-4-((2-(isopropylsulfonyl)phenyl)amino)pyrimidin-2-yl)amino)-5-isopropoxy-2-methylphenyl)piperidin-4-yl)methyl)piperidin-3-yl)amino)-2-(2,6-dioxopiperidin-3-yl)isoindoline-1,3-dione;
8-(4-((4-(2-(2,6-dioxopiperidin-3-yl)-1,3-dioxoisoindolin-5-yl)piperazin-1-yl)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-6-fluoro-1,3-dioxoisoindolin-5-yl)piperazin-1-yl)methyl)piperidin-1-yl)-9-ethyl-6,6-dimethyl-11-oxo-6,11-dihydro-5H-benzo[b]carbazole-3-carbonitrile;
8-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-9-ethyl-6,6-dimethyl-11-oxo-6,11-dihydro-5H-benzo[b]carbazole-3-carbonitrile;
8-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-9-ethyl-6,6-dimethyl-11-oxo-6,11-dihydro-5H-benzo[b]carbazole-3 -carbonitrile;
8-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-9-ethyl-6,6-dimethyl-11-oxo-6,11-dihydro-5H-benzo[b]carbazole-3-carbonitrile;
8-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1 -yl)-9-ethyl-6,6-dimethyl-11 -oxo-6,11 -dihydro-5H-benzo[b]carbazole-3-carbonitrile;
8-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-9-ethyl-6,6-dimethyl-11-oxo-6,11-dihydro-5H-benzo[b]carbazole-3-carbonitrile;
8-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-9-ethyl-6,6-dimethyl-11-oxo-6,11-dihydro-5H-benzo[b]carbazole-3-carbonitrile;
8-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-9-ethyl-6,6-dimethyl-11-oxo-6,11-dihydro-5H-benzo[b]carbazole-3-carbonitrile;

8-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-9-ethyl-6,6-dimethyl-11-oxo-6,11-dihydro-5H-benzo[b]carbazole-3-carbonitrile;
8-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-9-ethyl-6,6-dimethyl-11-oxo-6,11-dihydro-5H-benzo[b]carbazole-3-carbonitrile;
8-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-9-ethyl-6,6-dimethyl-11-oxo-6,11-dihydro-5H-benzo[b]carbazole-3-carbonitrile;
8-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-9-ethyl-6,6-dimethyl-11-oxo-6,11-dihydro-5H-benzo[b]carbazole-3-carbonitrile;
8-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-9-ethyl-6,6-dimethyl-11-oxo-6,11-dihydro-5H-benzo [b] carbazole-3 -carbonitrile;
8-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-1,3-dioxoisoindolin-5-yl)-3,5-dimethylpiperazin-1-yl)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-1,3-dioxoisoindolin-5-yl)-2,6-dimethylpiperazin-1-yl)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-6-fluoro-1,3-dioxoisoindolin-5-yl)piperidin-1-yl)methyl)piperidin-1-yl)-9-ethyl-6,6-dimethyl-11-oxo-6,11-dihydro-5H-benzo[b]carbazole-3-carbonitrile;
8-(4-((4-(2-(2,6-dioxopiperidin-3-yl)-1,3-dioxoisoindolin-5-yl)piperidin-1-yl)methyl)piperidin-1-yl)-9-ethyl-6,6-dimethyl-11-oxo-6,11-dihydro-5H-benzo[b]carbazole-3-carbonitrile;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl)-2-(2,6-dioxopiperidin-3-yl)isoindoline-1,3-dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl)-2-(2,6-dioxopiperidin-3-yl)-6-fluoroisoindoline-1,3-dione;
5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-6-yl)-2-(2,6-dioxopiperidin-3-yl)isoindoline-1,3-dione;
5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-6-yl)-2-(2,6-dioxopiperidin-3-yl)-6-fluoroisoindoline-1,3 -dione;
5-(6-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-3-yl)-2-(2,6-dioxopiperidin-3-yl)isoindoline-1,3-dione;
5-(6-((4-(4-(6-amino-5-((R)-1-(2,6-dichloro-3-fluorophenyl)ethoxy)pyridin-3-yl)-1H-pyrazol-1-yl)cyclohexyl)methyl)-3,6-diazabicyclo[3.1.1]heptan-3-yl)-2-(2,6-dioxopiperidin-3-yl)-6-fluoroisoindoline-1,3 -dione;
5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-8-yl)-2-(2,6-dioxopiperidin-3-yl)isoindoline-1,3-dione;
5-(3-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-8-yl)-2-(2,6-dioxopiperidin-3-yl)-6-fluoroisoindoline-1,3 -dione;
5-(8-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-3-yl)-2-(2,6-dioxopiperidin-3-yl)isoindoline-1,3-dione;
5-(8-((4-(4-(6-amino-5-((R)-1-(2,6-dichloro-3-fluorophenyl)ethoxy)pyridin-3-yl)-1H-pyrazol-1-yl)cyclohexyl)methyl)-3,8-diazabicyclo[3.2.1]octan-3-yl)-2-(2,6-dioxopiperidin-3-yl)-6-fluoroisoindoline-1,3 -dione;

5-(5-((4-(4-(6-amino-5-((R)-1-(2,6-dichloro-3-fluorophenyl)ethoxy)pyridin-3-yl)-1H-pyrazol-1-yl)cyclohexyl)methyl)-2,5-diazabicyclo[2.2.1]heptan-2-yl)-2-(2,6-dioxopiperidin-3-yl)isoindoline-1,3-dione;
5-(5-((4-(4-(6-amino-5-((R)-1-(2,6-dichloro-3-fluorophenyl)ethoxy)pyridin-3-yl)-1H-pyrazol-1-yl)cyclohexyl)methyl)-2,5-diazabicyclo[2.2.1]heptan-2-yl)-2-(2,6-dioxopiperidin-3-yl)-6-fluoroisoindoline-1,3 -dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl)-2-(2,6-dioxopiperidin-3-yl)isoindoline-1,3-dione;
5-(5-((4-(4-(6-amino-5-((R)-1-(2,6-dichloro-3-fluorophenyl)ethoxy)pyridin-3-yl)-1H-pyrazol-1-yl)cyclohexyl)methyl)-2,5-diazabicyclo[2.2.2]octan-2-yl)-2-(2,6-dioxopiperidin-3-yl)-6-fluoroisoindoline-1,3 -dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)isoindoline-1,3-dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-6-fluoroisoindoline-1,3 -dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4-fluoroisoindoline-1,3 -dione;
6-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4-fluoroisoindoline-1,3 -dione;
6-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,5-difluoroisoindoline-1,3-dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,7-difluoroisoindoline-1,3-dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,6-difluoroisoindoline-1,3-dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)piperazin-1-yl-2,2,3,3,5,5,6,6-$d_8$)-2-(2,6-dioxopiperidin-3-yl)-4,6,7-trifluoroisoindoline-1,3 -dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)-2,6-dimethylpiperazin-1-yl)-2-(2,6-dioxopiperidin-3-yl)isoindoline-1,3-dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)-2,6-dimethylpiperazin-1-yl)-2-(2,6-dioxopiperidin-3-yl)-6-fluoroisoindoline-1,3-dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)-3,5-dimethylpiperazin-1-yl)-2-(2,6-dioxopiperidin-3-yl)isoindoline-1,3-dione;
5-(4-((4-(4-(6-amino-5-((R)-1-(2,6-dichloro-3-fluorophenyl)ethoxy)pyridin-3-yl)-1H-pyrazol-1-yl)cyclohexyl)methyl)-3,5-dimethylpiperazin-1-yl)-2-(2,6-dioxopiperidin-3-yl)-6-fluoroisoindoline-1,3-dione;
5-(1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-4-yl)-2-(2,6-dioxopiperidin-3-yl)isoindoline-1,3-dione;
5-(1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-4-yl)-2-(2,6-dioxopiperidin-3-yl)-6-fluoroisoindoline-1,3-dione;
5-((R)-3-(((4-(4-(6-amino-5-((R)-1-(2,6-dichloro-3-fluorophenyl)ethoxy)pyridin-3-yl)-1H-pyrazol-1-yl)cyclohexyl)methyl)amino)piperidin-1-yl)-2-(2,6-dioxopiperidin-3-yl)isoindoline-1,3-dione;
5-((S)-3-(((4-(4-(6-amino-5-((R)-1-(2,6-dichloro-3-fluorophenyl)ethoxy)pyridin-3-yl)-1H-pyrazol-1-yl)cyclohexyl)methyl)amino)piperidin-1-yl)-2-(2,6-dioxopiperidin-3-yl)isoindoline-1,3-dione;
5-(((S)-1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-3-yl)amino)-2-(2,6-dioxopiperidin-3-yl)isoindoline-1,3-dione;
5-(((R)-1-((4-(4-(6-amino-5-((R)-1-(2,6-dichloro-3-fluorophenyl)ethoxy)pyridin-3-yl)-1H-pyrazol-1-yl)cyclohexyl)methyl)piperidin-3-yl)amino)-2-(2,6-dioxopiperidin-3-yl)isoindoline-1,3-dione;
(E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)piperazin-1-yl)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)piperazin-1-yl)methyl)piperidin-1-yl)but-2-enamide;
(E)-N-(4-((3-chloro-4-fluorophenyl)amino)-7-methoxyquinazolin-6-yl)-4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)but-2-enamide;
(E)-N-(4-((3-chloro-4-fluorophenyl)amino)-7-methoxyquinazolin-6-yl)-4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)but-2-enamide;
(E)-N-(4-((3-chloro-4-fluorophenyl)amino)-7-methoxyquinazolin-6-yl)-4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)but-2-enamide;
(E)-N-(4-((3-chloro-4-fluorophenyl)amino)-7-methoxyquinazolin-6-yl)-4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)but-2-enamide;
(E)-N-(4-((3-chloro-4-fluorophenyl)amino)-7-methoxyquinazolin-6-yl)-4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)but-2-enamide;

(E)-N-(4-((3-chloro-4-fluorophenyl)amino)-7-methoxyquinazolin-6-yl)-4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)but-2-enamide;
(E)-N-(4-((3-chloro-4-fluorophenyl)amino)-7-methoxyquinazolin-6-yl)-4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)but-2-enamide;
(E)-N-(4-((3-chloro-4-fluorophenyl)amino)-7-methoxyquinazolin-6-yl)-4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)but-2-enamide;
(E)-N-(4-((3-chloro-4-fluorophenyl)amino)-7-methoxyquinazolin-6-yl)-4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)but-2-enamide;
(E)-N-(4-((3-chloro-4-fluorophenyl)amino)-7-methoxyquinazolin-6-yl)-4-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)but-2-enamide;
(E)-N-(4-((3-chloro-4-fluorophenyl)amino)-7-methoxyquinazolin-6-yl)-4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)but-2-enamide;
(E)-N-(4-((3-chloro-4-fluorophenyl)amino)-7-methoxyquinazolin-6-yl)-4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)-3,5-dimethylpiperazin-1-yl)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3 -yl)-1, 3 -dioxoi soindolin-5 -yl)-2, 6-dimethylpip erazin-1-yl)methyl)pip eri din-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-1,3-dioxoisoindolin-5-yl)piperidin-1-yl)methyl)piperidin-1-yl)but-2-enamide;
(E)-N-(4-((3-chloro-4-fluorophenyl)amino)-7-methoxyquinazolin-6-yl)-4-(4-((4-(2-(2,6-dioxopiperidin-3-yl)-6-fluoro-1,3-dioxoisoindolin-5-yl)piperidin-1-yl)methyl)piperidin-1-yl)but-2-enamide;
(E)-N-(4-((3-chloro-4-fluorophenyl)amino)-7-methoxyquinazolin-6-yl)-4-(4-((((3R)-1-(2-(2,6-dioxopiperidin-3-yl)-1,3-dioxoisoindolin-5-yl)piperidin-3-yl)amino)methyl)piperidin-1-yl)but-2-enamide;
(E)-N-(4-((3-chloro-4-fluorophenyl)amino)-7-methoxyquinazolin-6-yl)-4-(4-((((3S)-1-(2-(2,6-dioxopiperidin-3-yl)-1,3-dioxoisoindolin-5-yl)piperidin-3-yl)amino)methyl)piperidin-1-yl)but-2-enamide;
(E)-N-(4-((3-chloro-4-fluorophenyl)amino)-7-methoxyquinazolin-6-yl)-4-(4-(((3S)-3-((2-(2,6-dioxopiperidin-3-yl)-1,3-dioxoisoindolin-5-yl)amino)piperidin-1-yl)methyl)piperidin-1-yl)but-2-enamide;
(E)-N-(4-((3-chloro-4-fluorophenyl)amino)-7-methoxyquinazolin-6-yl)-4-(4-(((3R)-3-((2-(2,6-dioxopiperidin-3-yl)-1,3-dioxoisoindolin-5-yl)amino)piperidin-1-yl)methyl)piperidin-1-yl)but-2-enamide;
N-(4-((3-chloro-4-fluorophenyl)amino)-7-(3-(4-((4-(2-(2,6-dioxopiperidin-3-yl)-1,3-dioxoisoindolin-5-yl)piperazin-1-yl)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-6-fluoro-1,3-dioxoisoindolin-5-yl)piperazin-1-yl)methyl)piperidin-1-yl)propoxy)quinazolin-6-yl)acrylamide;
N-(4-((3-chloro-4-fluorophenyl)amino)-7-(3-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)propoxy)quinazolin-6-yl)acrylamide;
N-(4-((3-chloro-4-fluorophenyl)amino)-7-(3-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)propoxy)quinazolin-6-yl)acrylamide;

N-(4-((3-chloro-4-fluorophenyl)amino)-7-(3-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)propoxy)quinazolin-6-yl)acrylamide;
N-(4-((3-chloro-4-fluorophenyl)amino)-7-(3-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)propoxy)quinazolin-6-yl)acrylamide;
N-(4-((3-chloro-4-fluorophenyl)amino)-7-(3-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)propoxy)quinazolin-6-yl)acrylamide;
N-(4-((3-chloro-4-fluorophenyl)amino)-7-(3-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)propoxy)quinazolin-6-yl)acrylamide;
N-(4-((3-chloro-4-fluorophenyl)amino)-7-(3-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)propoxy)quinazolin-6-yl)acrylamide;
N-(4-((3-chloro-4-fluorophenyl)amino)-7-(3-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)propoxy)quinazolin-6-yl)acrylamide;
N-(4-((3-chloro-4-fluorophenyl)amino)-7-(3-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)propoxy)quinazolin-6-yl)acrylamide;
N-(4-((3-chloro-4-fluorophenyl)amino)-7-(3-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)propoxy)quinazolin-6-yl)acrylamide;
N-(4-((3-chloro-4-fluorophenyl)amino)-7-(3-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)propoxy)quinazolin-6-yl)acrylamide;
N-(4-((3-chloro-4-fluorophenyl)amino)-7-(3-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-1,3-dioxoisoindolin-5-yl)piperazin-1-yl-2,2,3,3,5,5,6,6-ds)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-6-fluoro-1,3-dioxoisoindolin-5-yl)piperazin-1-yl-2,2,3,3,5,5,6,6-ds)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-7-fluoro-1,3-dioxoisoindolin-5-yl)piperazin-1-yl-2,2,3,3,5,5,6,6-ds)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-4-fluoro-1,3-dioxoisoindolin-5-yl)piperazin-1-yl-2,2,3,3,5,5,6,6-ds)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-4,6-difluoro-1,3-dioxoisoindolin-5-yl)piperazin-1-yl-2,2,3,3,5,5,6,6-ds)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-4,7-difluoro-1,3-dioxoisoindolin-5-yl)piperazin-1-yl-2,2,3,3,5,5,6,6-ds)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-6,7-difluoro-1,3-dioxoisoindolin-5-yl)piperazin-1-yl-2,2,3,3,5,5,6,6-ds)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-1,3-dioxoisoindolin-5-yl)-3,5-dimethylpiperazin-1-yl)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-1,3-dioxoisoindolin-5-yl)-2,6-dimethylpiperazin-1-yl)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-1,3-dioxoisoindolin-5-yl)piperidin-1-yl)methyl)piperidin-1-yl)propoxy)quinazolin-6-yl)acrylamide;
N-(4-((3-chloro-4-fluorophenyl)amino)-7-(3-(4-((4-(2-(2,6-dioxopiperidin-3-yl)-6-fluoro-1,3-dioxoisoindolin-5-yl)piperidin-1-yl)methyl)piperidin-1-yl)propoxy)quinazolin-6-yl)acrylamide;
N-(4-((3-chloro-4-fluorophenyl)amino)-7-(3-(4-((((3R)-1-(2-(2,6-dioxopipendin-3-yl)-1,3-dioxoisoindolin-5-yl)piperidin-3-yl)amino)methyl)piperidin-1-yl)propoxy)quinazolin-6-yl)acrylamide;
N-(4-((3-chloro-4-fluorophenyl)amino)-7-(3-(4-((((3 S)-1-(2-(2,6-dioxopiperidin-3-yl)-1,3-dioxoisoindolin-5-yl)piperidin-3-yl)amino)methyl)piperidin-1-yl)propoxy)quinazolin-6-yl)acrylamide;
N-(4-((3-chloro-4-fluorophenyl)amino)-7-(3-(4-(((3 S)-3-((2-(2,6-dioxopiperidin-3-yl)-1,3-dioxoisoindolin-5-yl)amino)piperidin-1-yl)methyl)piperidin-1-yl)propoxy)quinazolin-6-yl)acrylamide;
N-(4-((3-chloro-4-fluorophenyl)amino)-7-(3-(4-(((3R)-3-((2-(2,6-dioxopiperidin-3-yl)-1,3-dioxoisoindolin-5-yl)amino)piperidin-1-yl)methyl)piperidin-1-yl)propoxy)quinazolin-6-yl)acrylamide;
5-(4-((4-((4-((3,4-dichloro-2-fluorophenyl)amino)-7-methoxyquinazolin-6-yl)oxy)cyclohexyl)methyl)piperazin-1-yl)-2-(2,6-dioxopiperidin-3-yl)isoindoline-1,3-dione;

5-(4-((4-((4-((3,4-dichloro-2-fluorophenyl)amino)-7-methoxyquinazolin-6-yl)oxy)cyclohexyl)methyl)piperazin-1-yl)-2-(2,6-dioxopiperidin-3-yl)-6-fluoroisoindoline-1,3-dione;
5-(3-((4-((4-((3,4-dichloro-2-fluorophenyl)amino)-7-methoxyquinazolin-6-yl)oxy)cyclohexyl)methyl)-3,6-diazabicyclo[3.1.1]heptan-6-yl)-2-(2,6-dioxopiperidin-3-yl)isoindoline-1,3-dione;
5-(3-((4-((4-((3,4-dichloro-2-fluorophenyl)amino)-7-methoxyquinazolin-6-yl)oxy)cyclohexyl)methyl)-3,6-diazabicyclo[3.1.1]heptan-6-yl)-2-(2,6-dioxopiperidin-3-yl)-6-fluoroisoindoline-1,3 -dione;
5-(6-((4-((4-((3,4-dichloro-2-fluorophenyl)amino)-7-methoxyquinazolin-6-yl)oxy)cyclohexyl)methyl)-3,6-diazabicyclo[3.1.1]heptan-3-yl)-2-(2,6-dioxopiperidin-3-yl)isoindoline-1,3-dione;
5-(6-((4-((4-((3,4-dichloro-2-fluorophenyl)amino)-7-methoxyquinazolin-6-yl)oxy)cyclohexyl)methyl)-3,6-diazabicyclo[3.1.1]heptan-3-yl)-2-(2,6-dioxopiperidin-3-yl)-6-fluoroisoindoline-1,3 -dione;
5-(3-((4-((4-((3,4-dichloro-2-fluorophenyl)amino)-7-methoxyquinazolin-6-yl)oxy)cyclohexyl)methyl)-3,8-diazabicyclo[3.2.1]octan-8-yl)-2-(2,6-dioxopiperidin-3-yl)isoindoline-1,3-dione;
5-(3-((4-((4-((3,4-dichloro-2-fluorophenyl)amino)-7-methoxyquinazolin-6-yl)oxy)cyclohexyl)methyl)-3,8-diazabicyclo[3.2.1]octan-8-yl)-2-(2,6-dioxopiperidin-3-yl)-6-fluoroisoindoline-1,3 -dione;
5-(8-((4-((4-((3,4-dichloro-2-fluorophenyl)amino)-7-methoxyquinazolin-6-yl)oxy)cyclohexyl)methyl)-3,8-diazabicyclo[3.2.1]octan-3-yl)-2-(2,6-dioxopiperidin-3-yl)isoindoline-1,3-dione;
5-(8-((4-((4-((3,4-dichloro-2-fluorophenyl)amino)-7-methoxyquinazolin-6-yl)oxy)cyclohexyl)methyl)-3,8-diazabicyclo[3.2.1]octan-3-yl)-2-(2,6-dioxopiperidin-3-yl)-6-fluoroisoindoline-1,3 -dione;
5-(5-((4-((4-((3,4-dichloro-2-fluorophenyl)amino)-7-methoxyquinazolin-6-yl)oxy)cyclohexyl)methyl)-2,5-diazabicyclo[2.2.1]heptan-2-yl)-2-(2,6-dioxopiperidin-3-yl)isoindoline-1,3-dione;
5-(5-((4-((4-((3,4-dichloro-2-fluorophenyl)amino)-7-methoxyquinazolin-6-yl)oxy)cyclohexyl)methyl)-2,5-diazabicyclo[2.2.1]heptan-2-yl)-2-(2,6-dioxopiperidin-3-yl)-6-fluoroisoindoline-1,3 -dione;
5-(5-((4-((4-((3,4-dichloro-2-fluorophenyl)amino)-7-methoxyquinazolin-6-yl)oxy)cyclohexyl)methyl)-2,5-diazabicyclo[2.2.2]octan-2-yl)-2-(2,6-dioxopiperidin-3-yl)isoindoline-1,3-dione;
5-(5-((4-((4-((3,4-dichloro-2-fluorophenyl)amino)-7-methoxyquinazolin-6-yl)oxy)cyclohexyl)methyl)-2,5-diazabicyclo[2.2.2]octan-2-yl)-2-(2,6-dioxopiperidin-3-yl)-6-fluoroisoindoline-1,3 -dione;
5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)isoindoline-1,3-dione;
5- (4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-6-fluoroisoindoline-1,3 -dione;
6-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4-fluoroisoindoline-1,3 -dione;
5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4-fluoroisoindoline-1,3 -dione;
5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4,6-difluoroisoindoline-1,3-dione;
5-(4-((4-((4-((3,4-dichloro-2-fluorophenyl)amino)-7-methoxyquinazolin-6-yl)oxy)cyclohexyl)methyl)piperazin-1-yl-2,2,3,3,5,5,6,6-$d_8$)-2-(2,6-dioxopiperidin-3-yl)-4,7-difluoroisoindoline-1,3-dione;
6-(4-((4-((4-((3,4-dichloro-2-fluorophenyl)amino)-7-methoxyquinazolin-6-yl)oxy)cyclohexyl)methyl)plperazin-1-yl-2,2,3,3,5,5,6,6-$d_8$)-2-(2,6-dioxopiperldin-3-yl)-4,5-difluoroisoindoline-1,3-dione;
5-(4-((4-((4-((3,4-dichloro-2-fluorophenyl)amino)-7-methoxyquinazolin-6-yl)oxy)cyclohexyl)methyl)plperazin-1-yl-2,2,3,3,5,5,6,6-$d_8$)-2-(2,6-dioxopiperldin-3-yl)-4,6,7-trifluoroisoindoline-1,3 -dione;
5-(4-((4-((4-((3,4-dichloro-2-fluorophenyl)amino)-7-methoxyquinazolin-6-yl)oxy)cyclohexyl)methyl)-2,6-dimethylpiperazin-1-yl)-2-(2,6-dioxopiperidin-3-yl)isoindoline-1,3-dione;
5-(4-((4-((4-((3,4-dichloro-2-fluorophenyl)amino)-7-methoxyquinazolin-6-yl)oxy)cyclohexyl)methyl)-2,6-dimethylpiperazin-1-yl)-2-(2,6-dioxopiperidin-3-yl)-6-fluoroisoindoline-1,3-dione;
5-(4-((4-((4-((3,4-dichloro-2-fluorophenyl)amino)-7-methoxyquinazolin-6-yl)oxy)cyclohexyl)methyl)-3,5-dimethylpiperazin-1-yl)-2-(2,6-dioxopiperidin-3-yl)isoindoline-1,3-dione;
5-(4-((4-((4-((3,4-dichloro-2-fluorophenyl)amino)-7-methoxyquinazolin-6-yl)oxy)cyclohexyl)methyl)-3,5-dimethylpiperazin-1-yl)-2-(2,6-dioxopiperldin-3-yl)-6-fluoroisoindoline-1,3 -dione;
5-(l-((4-((4-((3,4-dichloro-2-fluorophenyl)amino)-7-methoxyquinazolin-6-yl)oxy)cyclohexyl)methyl)piperidin-4-yl)-2-(2,6-dioxopiperidin-3-yl)isoindoline-1,3-dione;
5-(1-((4-((4-((3,4-dichloro-2-fluorophenyl)amino)-7-methoxyquinazolin-6-yl)oxy)cyclohexyl)methyl)piperidin-4-yl)-2-(2,6-dioxopiperidin-3-yl)-6-fluoroisoindoline-1,3-dione;
5-(4-((1-(3-((4-((3-chloro-4-fluorophenyl)amino)-7-methoxyquinazolin-6-yl)oxy)propyl)piperidin-4-yl)methyl)piperazin-1-yl)-2-(2,6-dioxopiperidin-3-yl)isoindoline-1,3-dione;
5-(4-((1-(3-((4-((3-chloro-4-fluorophenyl)amino)-7-methoxyquinazolin-6-yl)oxy)propyl)piperidin-4-yl)methyl)piperazin-1-yl)-2-(2,6-dioxopiperidin-3-yl)-6-fluoroisoindoline-1,3-dione;

5-(3-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-6-yl)-2-(2,6-dioxopiperidin-3-yl)isoindoline-1,3-dione;
5-(3-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-6-yl)-2-(2,6-dioxopiperidin-3-yl)-6-fluoroisoindoline-1,3 -dione;
5-(6-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(3-((4-((3-chloro-4-fluorophenyl)amino)-7-methoxyquinazolin-6-yl)oxy)propyl)piperidin-4-yl)methyl)-3,6-diazabicyclo[3.1.1]heptan-3-yl)-2-(2,6-dioxopiperidin-3-yl)-6-fluoroisoindoline-1,3 -dione;
5-(3-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-8-yl)-2-(2,6-dioxopiperidin-3-yl)isoindoline-1,3-dione;
5-(3-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-8-yl)-2-(2,6-dioxopiperidin-3-yl)-6-fluoroisoindoline-1,3 -dione;
5-(8-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-3-yl)-2-(2,6-dioxopiperidin-3-yl)isoindoline-1,3-dione;
5-(8-((1-(3-((4-((3-chloro-4-fluorophenyl)amino)-7-methoxyquinazolin-6-yl)oxy)propyl)piperidin-4-yl)methyl)-3,8-diazabicyclo[3.2.1]octan-3-yl)-2-(2,6-dioxopiperidin-3-yl)-6-fluoroisoindoline-1,3 -dione;
5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.2]octan-2-yl)-2-(2,6-dioxopiperidin-3-yl)isoindoline-1,3-dione;
5-(5-((1-(3-((4-((3-chloro-4-fluorophenyl)amino)-7-methoxyquinazolin-6-yl)oxy)propyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
6-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((l-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((l-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
6-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
5-(4-((1-(3-((4-((3-chloro-4-fluorophenyl)amino)-7-methoxyquinazolin-6-yl)oxy)propyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((l-(3-((4-((3-chloro-4-fluorophenyl)amino)-7-methoxyquinazolin-6-yl)oxy)propyl)piperidin-4-yl)methyl)-2,6-dimethylpiperazin-1-yl)-2-(2,6-dioxopiperidin-3-yl)isoindoline-1,3 -dione;
5-(4-((l-(3-((4-((3-chloro-4-fluorophenyl)amino)-7-methoxyquinazolin-6-yl)oxy)propyl)piperidin-4-yl)methyl)-2,6-dimethylpiperazin-1-yl)-2-(2,6-dioxopiperidin-3-yl)-6-fluoroisoindoline-1,3 -dione;
5-(4-((l-(3-((4-((3-chloro-4-fluorophenyl)amino)-7-methoxyquinazolin-6-yl)oxy)propyl)piperidin-4-yl)methyl)-3,5-dimethylpiperazin-1-yl)-2-(2,6-dioxopiperidin-3-yl)isoindoline-1,3-dione;
5-(4-((l-(3-((4-((3-chloro-4-fluorophenyl)amino)-7-methoxyquinazolin-6-yl)oxy)propyl)piperidin-4-yl)methyl)-3,5-dimethylpiperazin-1-yl)-2-(2,6-dioxopiperidin-3-yl)-6-fluoroisoindoline-1,3-dione;
5-(l-((l-(3-((4-((3-chloro-4-fluorophenyl)amino)-7-methoxyquinazolin-6-yl)oxy)propyl)piperidin-4-yl)methyl)piperidin-4-yl)-2-(2,6-dioxopiperidin-3-yl)isoindoline-1,3-dione;
5-(1-((1-(3-((4-((3-chloro-4-fluorophenyl)amino)-7-methoxyquinazolin-6-yl)oxy)propyl)piperidin-4-yl)methyl)piperidin-4-yl)-2-(2,6-dioxopiperidin-3-yl)-6-fluoroisoindoline-1,3-dione;
5-((R)-3-(((1-(3-((4-((3-chloro-4-fluorophenyl)amino)-7-methoxyquinazolin-6-yl)oxy)propyl)piperidin-4-yl)methyl)amino)piperidin-1-yl)-2-(2,6-dioxopiperidin-3-yl)isoindoline-1,3-dione;
5-((S)-3-(((1-(3-((4-((3-chloro-4-fluorophenyl)amino)-7-methoxyquinazolin-6-yl)oxy)propyl)piperidin-4-yl)methyl)amino)piperidin-1-yl)-2-(2,6-dioxopiperidin-3-yl)isoindoline-1,3-dione;
5-(((S)-1-((1-(3-((4-((3-chloro-4-fluorophenyl)amino)-7-methoxyquinazolin-6-yl)oxy)propyl)piperidin-4-yl)methyl)piperidin-3-yl)amino)-2-(2,6-dioxopiperidin-3-yl)isoindoline-1,3-dione;

5-(((R)-1-((1-(3-((4-((3-chloro-4-fluorophenyl)amino)-7-methoxyquinazolin-6-yl)oxy)propyl)piperidin-4-yl)methyl)piperidin-3-yl)amino)-2-(2,6-dioxopiperidin-3-yl)isoindoline-1,3-dione;
N-(2-((2-(4-((4-(2-(2,6-dioxopiperidin-3-yl)-1,3-dioxoisoindolin-5-yl)piperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((4-(2-(2,6-dioxopiperidin-3-yl)-6-fluoro-1,3-dioxoisoindolin-5-yl)piperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((3-(2-(2,6-dioxopiperidin-3-yl)-1,3-dioxoisoindolin-5-yl)-3,6-diazabicyclo[3 .1.1]heptan-6-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)ethyl)(methyl)amino)-4-methoxy-5-((4-(1-methyl-1H-indol-3-yl)pyrimidin-2-yl)amino)phenyl)acrylamide;
N-(2-((2-(4-((4-(2-(2,6-dioxopiperidin-3-yl)-4,7-difluoro-1,3-dioxoisoindolin-5-yl)piperazin-1-yl-2,2,3,3,5,5,6,6-

*$d_8$*)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((4-(2-(2,6-dioxopiperidin-3-yl)-6,7-difluoro-1,3-dioxoisoindolin-5-yl)piperazin-1-yl-2,2,3,3,5,5,6,6-*$d_8$*)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((4-(2-(2,6-dioxopiperidin-3-yl)-4,6,7-trifluoro-1,3-dioxoisoindolin-5-yl)piperazin-1-yl-2,2,3,3,5,5,6,6-*$d_8$*)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((4-(2-(2,6-dioxopiperidin-3-yl)-1,3-dioxoisoindolin-5-yl)-3,5-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((4-(2-(2,6-dioxopiperidin-3-yl)-1,3-dioxoisoindolin-5-yl)-2,6-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((4-(2-(2,6-dioxopiperidin-3-yl)-1,3-dioxoisoindolin-5-yl)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((4-(2-(2,6-dioxopiperidin-3-yl)-6-fluoro-1,3-dioxoisoindolin-5-yl)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((((3R)-1-(2-(2,6-dioxopiperidin-3-yl)-1,3-dioxoisoindolin-5-yl)piperidin-3-yl)amino)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-((((3 S)-1-(2-(2,6-dioxopiperidin-3-yl)-1,3-dioxoisoindolin-5-yl)piperidin-3-yl)amino)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-(((3S)-3-((2-(2,6-dioxopiperidin-3-yl)-1,3-dioxoisoindolin-5-yl)amino)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;

N-(2-((2-(4-(((3R)-3-((2-(2,6-dioxopiperidin-3-yl)-1,3-dioxoisoindolin-5-yl)amino)piperidin-1-yl)methyl)piperidin-1-yl)ethyl)(methyl)amino)-4-methoxy-5-((4-(1-methyl-1H-indol-3-yl)pyrimidin-2-yl)amino)phenyl)acrylamide;

(E)-N-(4-((3-chloro-4-fluorophenyl)amino)-7-(((S)-tetrahydrofuran-3-yl)oxy)quinazolin-6-yl)-4-(4-((4-(2-(2, 6-dioxopip eridin-3 -yl)-1, 3 -dioxoisoindolin-5 -yl)pip erazin-1-yl)methyl)pip eridin-1-yl)but-2-enamide;

(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)piperazin-1-yl)methyl)piperidin-1-yl)but-2-enamide;

(E)-N-(4-((3-chloro-4-fluorophenyl)amino)-7-(((S)-tetrahydrofuran-3-yl)oxy)quinazolin-6-yl)-4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)but-2-enamide;

(E)-N-(4-((3-chloro-4-fluorophenyl)amino)-7-(((S)-tetrahydrofuran-3-yl)oxy)quinazolin-6-yl)-4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)but-2-enamide;

(E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)but-2-enamide;

(E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)but-2-enamide;

(E)-N-(4-((3-chloro-4-fluorophenyl)amino)-7-(((S)-tetrahydrofuran-3-yl)oxy)quinazolin-6-yl)-4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)but-2-enamide;

(E)-N-(4-((3-chloro-4-fluorophenyl)amino)-7-(((S)-tetrahydrofuran-3-yl)oxy)quinazolin-6-yl)-4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)but-2-enamide;

(E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-1,3-dioxoisoindolin-5-yl)-3,8-diazabicyclo[3.2.1]octan-8-yl)methyl)piperidin-1 -yl)but-2-enamide;

(E)-N-(4-((3-chloro-4-fluorophenyl)amino)-7-(((S)-tetrahydrofuran-3-yl)oxy)quinazolin-6-yl)-4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)but-2-enamide;

(E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4,7-difluoro-1,3-dioxoisoindolin-5-yl)piperazin-1-yl-2,2,3,3,5,5,6,6-ds)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)-3,5-dimethylpiperazin-1-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-1,3-dioxoisoindolin-5-yl)-2,6-dimethylpiperazin-1-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2, 6-dioxopip eridin-3 -yl)-1, 3 -dioxoisoindolin-5 -yl)pip eri din-1-yl)methyl)pip eri din-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)piperidin-1-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((((3R)-1-(2-(2,6-dioxopiperidin-3-yl)-1,3-dioxoisoindolin-5-yl)piperidin-3-yl)amino)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-((((3 S)-1-(2-(2,6-dioxopiperidin-3-yl)-1,3-dioxoisoindolin-5-yl)piperidin-3-yl)amino)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-(((3 S)-3-((2-(2,6-dioxopiperidin-3-yl)-1,3-dioxoisoindolin-5-yl)amino)piperidin-1-yl)methyl)piperidin-1-yl)but-2-enamide;
(E)-N-(4-((3-chloro-4-fluorophenyl)amino)-7-(((S)-tetrahydrofuran-3-yl)oxy)quinazolin-6-yl)-4-(4-(((3R)-3-((2-(2,6-dioxopiperidin-3-yl)-1,3-dioxoisoindolin-5-yl)amino)piperidin-1-yl)methyl)piperidin-1-yl)but-2-enamide;
2-(6-(6-(4-((4-(2-(2,6-dioxopiperidin-3-yl)-1,3-dioxoisoindolin-5-yl)piperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((4-(2-(2,6-dioxopiperidin-3-yl)-6-fluoro-1,3-dioxoisoindolin-5-yl)piperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)pyridazin-3-yl)-1-oxoisoindolin-2-yl)-2-(5 - fluoro-2-hydroxyphenyl)-N-(thiazol-2-yl)acetamide;
2-(6-(6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)pyridazin-3-yl)-1-oxoisoindolin-2-yl)-2-(5 - fluoro-2-hydroxyphenyl)-N-(thiazol-2-yl)acetamide;
2-(6-(6-(4-((3-(2-(2,6-dioxopiperidin-3-yl)-1,3-dioxoisoindolin-5-yl)-3,6-diazabicyclo [3.1.1]heptan-6-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)pyridazin-3 -yl)-1-oxoisoindolin-2-yl)-2-(5 - fluoro-2-hydroxyphenyl)-N-(thiazol-2-yl)acetamide;
2-(6-(6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide;
2-(6-(6-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-((4-(2-(2,6-dioxopiperidin-3-yl)-1,3-dioxoisoindolin-5-yl)-3,5-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-((4-(2-(2,6-dioxopiperidin-3-yl)-1,3-dioxoisoindolin-5-yl)-2,6-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-((4-(2-(2,6-dioxopiperidin-3-yl)-1,3-dioxoisoindolin-5-yl)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-((4-(2-(2,6-dioxopiperidin-3-yl)-6-fluoro-1,3-dioxoisoindolin-5-yl)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-((((3R)-1-(2-(2,6-dioxopiperidin-3-yl)-1,3-dioxoisoindolin-5-yl)piperidin-3-yl)amino)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-((((3 S)-1-(2-(2,6-dioxopiperidin-3-yl)-1,3-dioxoisoindolin-5-yl)piperidin-3-yl)amino)methyl)piperidin-1 -yl)pyridazin-3 -yl)-1 -oxoisoindolin-2-yl)-2-(5 -fluoro-2-hydroxyphenyl)-N-(thiazol-2-yl)acetamide;

2-(6-(6-(4-(((3S)-3-((2-(2,6-dioxopiperidin-3-yl)-1,3-dioxoisoindolin-5-yl)amino)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;

2-(6-(6-(4-(((3R)-3-((2-(2,6-dioxopiperidin-3-yl)-1,3-dioxoisoindolin-5-yl)amino)piperidin-1-yl)methyl)piperidin-1-yl)pyridazin-3-yl)-1-oxoisoindolin-2-yl)-2-(5-fluoro-2-hydroxyphenyl)-N-(thiazol-2-yl)acetamide;

2-(2,6-dioxopiperidin-3-yl)-5-(4-((1-(((R,E)-$1^{1}$,$2^{6}$,7-trimethyl-3-oxo-$5^{2}$,$5^{3}$-dihydro-11H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)piperazin-1-yl)isoindoline-1,3-dione;

2-(2,6-dioxopiperidin-3-yl)-5-fluoro-6-(4-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)piperazin-1-yl)isoindoline-1,3-dione;

2-(2,6-dioxopiperidin-3-yl)-5-(3-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,6-diazabicyclo[3.1.1]heptan-6-yl)isoindoline-1,3-dione;

2-(2,6-dioxopiperidin-3-yl)-5-fluoro-6-(3-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,6-diazabicyclo[3.1.1]heptan-6-yl)isoindoline-1,3-dione;

2-(2,6-dioxopiperidin-3-yl)-5-(6-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,6-diazabicyclo[3.1.1]heptan-3-yl)isoindoline-1,3-dione;

2-(2,6-dioxopiperidin-3-yl)-5-fluoro-6-(6-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,6-diazabicyclo[3.1.1]heptan-3-yl)isoindoline-1,3-dione;

2-(2,6-dioxopiperidin-3-yl)-5-(3-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,8-diazabicyclo[3.2.1]octan-8-yl)isoindoline-1,3-dione;

2-(2,6-dioxopiperidin-3-yl)-5-fluoro-6-(3-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,8-diazabicyclo[3.2.1]octan-8-yl)isoindoline-1,3-dione;

2-(2,6-dioxopiperidin-3-yl)-5-(8-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,8-diazabicyclo[3.2.1]octan-3-yl)isoindoline-1,3-dione;

2-(2,6-dioxopiperidin-3-yl)-5-fluoro-6-(8-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-3,8-diazabicyclo[3.2.1]octan-3-yl)isoindoline-1,3-dione;

2-(2,6-dioxopiperidin-3-yl)-5-fluoro-6-(5-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-$5^{6}$-yl)methyl)piperidin-4-yl)methyl)-2,5-diazabicyclo[2.2.1]heptan-2-yl)isoindoline-1,3-dione;

2-(2,6-dioxopiperidin-3-yl)-5-(5-((1-(((R,E)-$1^{1}$,$2^{6}$,7-trimethyl-3-oxo-$5^{2}$,$5^{3}$-dihydro-$1^{1}$H,$5^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-2,5-diazabicyclo[2.2.1]heptan-2-yl)isoindoline-1,3-dione;

2-(2,6-dioxopiperidin-3-yl)-5-(5-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-2,5-diazabicyclo[2.2.2]octan-2-yl)isoindoline-1,3-dione;

2-(2,6-dioxopiperidin-3-yl)-5-fluoro-6-(5-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)-2,5-diazabicyclo[2.2.2]octan-2-yl)isoindoline-1,3-dione;

2-(2,6-dioxopiperidin-3-yl)-5-(4-((1-(((R,E)-1$^{1}$,2$^{6}$,7-trimethyl-3-oxo-5$^{2}$,5$^{3}$-dihydro-11H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-5-fluoro-6-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-4-fluoro-6-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-4-fluoro-5-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-4,6-difluoro-5-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-4,7-difluoro-5-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-4,5-difluoro-6-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione;

2-(2,6-dioxopiperidin-3-yl)-4,5,7-trifluoro-6-(4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl-2,2,3,3,5,5,6,6-$d_8$)isoindoline-1,3-dione;

5-(2,6-dimethyl-4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl)-2-(2,6-dioxopiperidin-3-yl)isoindoline-1,3-dione;

5-(2,6-dimethyl-4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl)-2-(2,6-dioxopiperidin-3-yl)-6-fluoroisoindoline-1,3-dione;

5-(3,5-dimethyl-4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl)-2-(2,6-dioxopiperidin-3-yl)isoindoline-1,3-dione;

5-(3,5-dimethyl-4-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperazin-1-yl)-2-(2,6-dioxopiperidin-3-yl)-6-fluoroisoindoline-1,3-dione;

2-(2,6-dioxopiperidin-3-yl)-5-(1-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperidin-4-yl)isoindoline-1,3-dione;

2-(2,6-dioxopiperidin-3-yl)-5-fluoro-6-(1-((1-(((R,E)-11,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperidin-4-yl)isoindoline-1,3 -dione;

2-(2,6-dioxopiperidin-3-yl)-5-((R)-3-(((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)amino)piperidin-1-yl)isoindoline-1,3-dione;

2-(2,6-dioxopiperidin-3-yl)-5-((S)-3-(((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)amino)piperidin-1-yl)isoindoline-1,3-dione;

2-(2,6-dioxopiperidin-3-yl)-5-(((S)-1-((1-(((R,E)-1$^{1}$,2$^{6}$,7-trimethyl-3-oxo-5$^{2}$,5$^{3}$-dihydro-1$^{1}$H,5$^{1}$H-11-oxa-4-aza-5(2,1)-benzo[d]imidazola-2(2,4)-pyridina-1(4,5)-pyrazolacycloundecaphane-5$^{6}$-yl)methyl)piperidin-4-yl)methyl)piperidin-3-yl)amino)isoindoline-1,3-dione;
2-(2,6-dioxopiperidin-3-yl)-5-(((R)-1-((1-(((R,E)-$1^1$,$2^6$,7-trimethyl-3-oxo-$5^2$,$5^3$-dihydro-$1^1$H,$5^1$H-11-oxa-4-aza-5(2,1)-benzo[d]imidazola-2(2,4)-pyridina-1(4,5)-pyrazolacycloundecaphane-$5^6$-yl)methyl)piperidin-4-yl)methyl)piperidin-3-yl)amino)isoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl)-2-(2,6-dioxopiperidin-3-yl)isoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl)-2-(2,6-dioxopiperidin-3-yl)-6-fluoroisoindoline-1,3 -dione;
5-(3-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-6-yl)-2-(2,6-dioxopiperidin-3-yl)isoindoline-1,3-dione;
5-(3-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(6-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-3-yl)-2-(2,6-dioxopiperidin-3-yl)isoindoline-1,3-dione;
5-(6-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,6-diazabicyclo[3.1.1]heptan-3-yl)-2-(2,6-dioxopiperidin-3-yl)-6-fluoroisoindoline-1,3-dione;
5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.1]heptan-2-yl)-2-(2,6-dioxopiperidin-3-yl)isoindoline-1,3-dione;
5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.2]octan-2-yl)-2-(2,6-dioxopiperidin-3-yl)isoindoline-1,3-dione;
5-(5-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
6-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
6-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,6-dimethylpiperazin-1-yl)-2-(2,6-dioxopiperidin-3-yl)isoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-2,6-dimethylpiperazin-1-yl)-2-(2,6-dioxopiperidin-3-yl)-6-fluoroisoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,5-dimethylpiperazin-1-yl)-2-(2,6-dioxopiperidin-3-yl)isoindoline-1,3-dione;
5-(4-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)-3,5-dimethylpiperazin-1-yl)-2-(2,6-dioxopiperidin-3-yl)-6-fluoroisoindoline-1,3-dione;
5-(1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-4-yl)-2-(2,6-dioxopiperidin-3-yl)isoindoline-1,3-dione;
5-(1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-4-yl)-2-(2,6-dioxopiperidin-3-yl)-6-fluoroisoindoline-1,3 -dione;
5-((R)-3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)amino)piperidin-1-yl)-2-(2,6-dioxopiperidin-3-yl)isoindoline-1,3-dione;
5-((S)-3-(((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)amino)piperidin-1-yl)-2-(2,6-dioxopiperidin-3-yl)isoindoline-1,3-dione;
5-(((S)-1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-3-yl)amino)-2-(2,6-dioxopiperidin-3-yl)isoindoline-1,3-dione;
5-(((R)-1-((1-(6-((2-(1-(cyclopropylsulfonyl)-1H-pyrazol-4-yl)pyrimidin-4-yl)amino)-1-isopropyl-1H-pyrazolo[4,3-c]pyridin-3-yl)piperidin-4-yl)methyl)piperidin-3-yl)amino)-2-(2,6-dioxopiperidin-3-yl)isoindoline-1,3-dione;
2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl)isoindoline-1,3-dione;
2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl)isoindoline-1,3 -dione;
2-(2,6-dioxopiperidin-3-yl)-5-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-6-yl)isoindoline-1,3-dione;
2-(2,6-dioxopiperidin-3-yl)-5-fluoro-6-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-6-yl)isoindoline-1,3-dione;
2-(2,6-dioxopiperidin-3-yl)-5-(6-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-3-yl)isoindoline-1,3-dione;
2-(2,6-dioxopiperidin-3-yl)-5-fluoro-6-(6-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,6-diazabicyclo[3.1.1]heptan-3-yl)isoindoline-1,3-dione;
2-(2,6-dioxopiperidin-3-yl)-5-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-8-yl)isoindoline-1,3 -dione;
2-(2,6-dioxopiperidin-3-yl)-5-fluoro-6-(3-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-8-yl)isoindoline-1,3-dione;
2-(2,6-dioxopiperidin-3-yl)-5-(8-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-3-yl)isoindoline-1,3-dione;
2-(2,6-dioxopiperidin-3-yl)-5-fluoro-6-(8-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,8-diazabicyclo[3.2.1]octan-3-yl)isoindoline-1,3-dione;
2-(2,6-dioxopiperidin-3-yl)-5-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.1]heptan-2-yl)isoindoline-1,3-dione;
2-(2,6-dioxopiperidin-3-yl)-5-fluoro-6-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.1]heptan-2-yl)isoindoline-1,3-dione;
2-(2,6-dioxopiperidin-3-yl)-5-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.2]octan-2-yl)isoindoline-1,3-dione;
2-(2,6-dioxopiperidin-3-yl)-5-fluoro-6-(5-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,5-diazabicyclo[2.2.2]octan-2-yl)isoindoline-1,3-dione;
2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione;
2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione;

2-(2,6-dioxopiperidin-3-yl)-4-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione;
2-(2,6-dioxopiperidin-3-yl)-4-fluoro-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione;
2-(2,6-dioxopiperidin-3-yl)-4,6-difluoro-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione;
2-(2,6-dioxopiperidin-3-yl)-4,7-difluoro-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione;
2-(2,6-dioxopiperidin-3-yl)-4,5-difluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione;
2-(2,6-dioxopiperidin-3-yl)-4,5,7-trifluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperazin-1-yl-2,2,3,3,5,5,6,6-$d_8$)isoindoline-1,3-dione;
2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,6-dimethylpiperazin-1-yl)isoindoline-1,3 -dione;
2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-2,6-dimethylpiperazin-1-yl)isoindoline-1,3-dione;
2-(2,6-dioxopiperidin-3-yl)-5-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,5-dimethylpiperazin-1-yl)isoindoline-1,3 -dione;
2-(2,6-dioxopiperidin-3-yl)-5-fluoro-6-(4-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)-3,5-dimethylpiperazin-1-yl)isoindoline-1,3-dione;
2-(2,6-dioxopiperidin-3-yl)-5-(1-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperidin-4-yl)isoindoline-1,3-dione;
2-(2,6-dioxopiperidin-3-yl)-5-fluoro-6-(1-((1-(1-isopropyl-6-((2-(4-methoxypiperidin-1-yl)pyrimidin-4-yl)amino)-1H-pyrazolo[4,3-c]pyridin-3-yl)piperidin-4-yl)methyl)piperidin-4-yl)isoindoline-1,3-dione;
N-(tert-butyl)-3-((2-((4-(2-(4-((4-(2-(2,6-dioxopiperidin-3-yl)-1,3-dioxoisoindolin-5-yl)piperazin-1-yl)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-6-fluoro-1,3-dioxoisoindolin-5-yl)piperazin-1-yl)methyl)piperidin-1-yl)ethoxy)phenyl)amino)-5-methylpyrimidin-4-yl)amino)benzenesulfonamide;
N-(tert-butyl)-3-((2-((4-(2-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)ethoxy)phenyl)amino)-5-methylpyrimidin-4-yl)amino)benzenesulfonamide;
N-(tert-butyl)-3-((2-((4-(2-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)ethoxy)phenyl)amino)-5-methylpyrimidin-4-yl)amino)benzenesulfonamide;
N-(tert-butyl)-3-((2-((4-(2-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)ethoxy)phenyl)amino)-5-methylpyrimidin-4-yl)amino)benzenesulfonamide;
N-(tert-butyl)-3-((2-((4-(2-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)ethoxy)phenyl)amino)-5-methylpyrimidin-4-yl)amino)benzenesulfonamide;
N-(tert-butyl)-3-((2-((4-(2-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)ethoxy)phenyl)amino)-5-methylpyrimidin-4-yl)amino)benzenesulfonamide;
N-(tert-butyl)-3-((2-((4-(2-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)ethoxy)phenyl)amino)-5-methylpyrimidin-4-yl)amino)benzenesulfonamide;
N-(tert-butyl)-3-((2-((4-(2-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)ethoxy)phenyl)amino)-5-methylpyrimidin-4-yl)amino)benzenesulfonamide;
N-(tert-butyl)-3-((2-((4-(2-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)ethoxy)phenyl)amino)-5-methylpyrimidin-4-yl)amino)benzenesulfonamide;
N-(tert-butyl)-3-((2-((4-(2-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)ethoxy)phenyl)amino)-5-methylpyrimidin-4-yl)amino)benzenesulfonamide;
N-(tert-butyl)-3-((2-((4-(2-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)ethoxy)phenyl)amino)-5-methylpyrimidin-4-yl)amino)benzenesulfonamide;
N-(tert-butyl)-3-((2-((4-(2-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)ethoxy)phenyl)amino)-5-methylpyrimidin-4-yl)amino)benzenesulfonamide;
N-(tert-butyl)-3-((2-((4-(2-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-1,3-dioxoisoindolin-5-yl)-3,5-dimethylpiperazin-1-yl)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-1,3-dioxoisoindolin-5-yl)-2,6-dimethylpiperazin-1-yl)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-1,3-dioxoisoindolin-5-yl)piperidin-1-yl)methyl)piperidin-1-yl)ethoxy)phenyl)amino)-5-methylpyrimidin-4-yl)amino)benzenesulfonamide;
N-(tert-butyl)-3-((2-((4-(2-(4-((4-(2-(2,6-dioxopiperidin-3-yl)-6-fluoro-1,3-dioxoisoindolin-5-yl)piperidin-1-yl)methyl)piperidin-1-yl)ethoxy)phenyl)amino)-5-methylpyrimidin-4-yl)amino)benzenesulfonamide;
N-(tert-butyl)-3-((2-((4-(2-(4-((((3R)-1-(2-(2,6-dioxopiperidin-3-yl)-1,3-dioxoisoindolin-5-yl)piperidin-3-yl)amino)methyl)piperidin-1-yl)ethoxy)phenyl)amino)-5-methylpyrimidin-4-yl)amino)benzenesulfonamide;
N-(tert-butyl)-3-((2-((4-(2-(4-((((3 S)-1-(2-(2,6-dioxopiperidin-3-yl)-1,3-dioxoisoindolin-5-yl)piperidin-3-yl)amino)methyl)piperidin-1-yl)ethoxy)phenyl)amino)-5-methylpyrimidin-4-yl)amino)benzenesulfonamide;
N-(tert-butyl)-3-((2-((4-(2-(4-(((3S)-3-((2-(2,6-dioxopiperidin-3-yl)-1,3-dioxoisoindolin-5-yl)amino)piperidin-1-yl)methyl)piperidin-1-yl)ethoxy)phenyl)amino)-5-methylpyrimidin-4-yl)amino)benzenesulfonamide;
N-(tert-butyl)-3-((2-((4-(2-(4-(((3R)-3-((2-(2,6-dioxopiperidin-3-yl)-1,3-dioxoisoindolin-5-yl)amino)piperidin-1-yl)methyl)piperidin-1-yl)ethoxy)phenyl)amino)-5-methylpyrimidin-4-yl)amino)benzenesulfonamide;
4-(4-((4-(2-(2,6-dioxopiperidin-3-yl)-1,3-dioxoisoindolin-5-yl)piperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-6-fluoro-1,3-dioxoisoindolin-5-yl)piperazin-1-yl)methyl)piperidin-1-yl)-N-(5-((R)-2-methoxy-2-phenylacetyl)-1,4,5,6-tetrahydropyrrolo[3,4-c]pyrazol-3 -yl)benzamide;
4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-N-(5-((R)-2-methoxy-2-phenylacetyl)-1,4,5,6-tetrahydropyrrolo[3,4-c]pyrazol-3 -yl)benzamide;
4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-N-(5-((R)-2-methoxy-2-phenylacetyl)-1,4,5,6-tetrahydropyrrolo[3,4-c]pyrazol-3 -yl)benzamide;
4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-N-(5-((R)-2-methoxy-2-phenylacetyl)-1,4,5,6-tetrahydropyrrolo[3,4-c]pyrazol-3 -yl)benzamide;
4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-N-(5-((R)-2-methoxy-2-phenylacetyl)-1,4,5,6-tetrahydropyrrolo[3,4-c]pyrazol-3 -yl)benzamide;
4-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-N-(5-((R)-2-methoxy-2-phenylacetyl)-1,4,5,6-tetrahydropyrrolo[3,4-c]pyrazol-3 -yl)benzamide;
4-(4-((4-(2-(2,6-dioxopiperidin-3-yl)-1,3-dioxoisoindolin-5-yl)piperazin-1-yl-2,2,3,3,5,5,6,6-$d_8$)niethyl)piperidin-1-yl)-N-(5-((R)-2-methoxy-2-phenylacetyl)-1,4,5,6-tetrahydropyrrolo[3,4-c]pyrazol-3 -yl)benzamide;
4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-1,3-dioxoisoindolin-5-yl)-3,5-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-1,3-dioxoisoindolin-5-yl)-2,6-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide;

4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-1,3-dioxoisoindolin-5-yl)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((4-(2-(2,6-dioxopiperidin-3-yl)-6-fluoro-1,3-dioxoisoindolin-5-yl)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((((3R)-1-(2-(2,6-dioxopiperidin-3-yl)-1,3-dioxoisoindolin-5-yl)piperidin-3-yl)amino)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-((((3S)-1-(2-(2,6-dioxopiperidin-3-yl)-1,3-dioxoisoindolin-5-yl)piperidin-3-yl)amino)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-(((3S)-3-((2-(2,6-dioxopiperidin-3-yl)-1,3-dioxoisoindolin-5-yl)amino)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide;
4-(4-(((3R)-3-((2-(2,6-dioxopiperidin-3-yl)-1,3-dioxoisoindolin-5-yl)amino)piperidin-1-yl)methyl)piperidin-1-yl)-N-(5-((R)-2-methoxy-2-phenylacetyl)-1,4,5,6-tetrahydropyrrolo[3,4-c]pyrazol-3-yl)benzamide;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl)-2-(2,6-dioxopiperidin-3-yl)isoindoline-1,3-dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl)-2-(2,6-dioxopiperidin-3-yl)-6-fluoroisoindoline-1,3-dione;
5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-6-yl)-2-(2,6-dioxopiperidin-3-yl)isoindoline-1,3-dione;
5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(6-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,6-diazabicyclo[3.1.1]heptan-3-yl)-2-(2,6-dioxopiperidin-3-yl)-6-fluoroisoindoline-1,3-dione;
5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)isoindoline-1,3-dione;
5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)-6-fluoroisoindoline-1,3-dione;
5-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)-6-fluoroisoindoline-1,3-dione;
6-(3-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-8-yl)-2-(2,6-dioxopiperidin-3-yl)-4-fluoroisoindoline-1,3-dione;
5-(8-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-3-yl)-2-(2,6-dioxopiperidin-3-yl)isoindoline-1,3-dione;
5-(8-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.2]octan-2-yl)-2-(2,6-dioxopiperidin-3-yl)isoindoline-1,3-dione;
5-(5-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-ds)-2-(2,6-dioxopiperidin-3-yl)isoindoline-1,3-dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-ds)-2-(2,6-dioxopiperidin-3-yl)-6-fluoroisoindoline-1,3-dione;
6-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-ds)-2-(2,6-dioxopiperidin-3-yl)-4-fluoroisoindoline-1,3-dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-ds)-2-(2,6-dioxopiperidin-3-yl)-4-fluoroisoindoline-1,3-dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-ds)-2-(2,6-dioxopiperidin-3-yl)-4,6-difluoroisoindoline-1,3-dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-ds)-2-(2,6-dioxopiperidin-3-yl)-4,7-difluoroisoindoline-1,3-dione;
6-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-ds)-2-(2,6-dioxopiperidin-3-yl)-4,5-difluoroisoindoline-1,3-dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl-2,2,3,3,5,5,6,6-ds)-2-(2,6-dioxopiperidin-3-yl)-4,6,7-trifluoroisoindoline-1,3-dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,6-dimethylpiperazin-1-yl)-2-(2,6-dioxopiperidin-3 -yl)isoindoline-1,3 -dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-2,6-dimethylpiperazin-1-yl)-2-(2,6-dioxopiperidin-3 -yl)-6-fluoroisoindoline-1,3 -dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,5-dimethylpiperazin-1-yl)-2-(2,6-dioxopiperidin-3 -yl)isoindoline-1,3 -dione;
5-(4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)-3,5-dimethylpiperazin-1-yl)-2-(2,6-dioxopiperidin-3-yl)-6-fluoroisoindoline-1,3-dione;
5-(1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-4-yl)-2-(2,6-dioxopiperidin-3-yl)isoindoline-1,3-dione;
5-(1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-4-yl)-2-(2,6-dioxopiperidin-3-yl)-6-fluoroisoindoline-1,3-dione;
5-((R)-3-(((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)piperidin-1-yl)-2-(2,6-dioxopiperidin-3-yl)isoindoline-1,3-dione;
5-((S)-3-(((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)piperidin-1-yl)-2-(2,6-dioxopiperidin-3-yl)isoindoline-1,3-dione;
5-(((S)-1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-3-yl)amino)-2-(2,6-dioxopiperidin-3-yl)isoindoline-1,3-dione;
5-(((R)-1-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperidin-3-yl)amino)-2-(2,6-dioxopiperidin-3-yl)isoindoline-1,3-dione;
5-(4-((1-(4-(5,7-dimethoxy-4-oxo-3,4-dihydroquinazolin-2-yl)phenyl)piperidin-4-yl)methyl)piperazin-1-yl)-2-(2,6-dioxopiperidin-3-yl)isoindoline-1,3-dione;
5-(4-((1-(4-(5,7-dimethoxy-4-oxo-3,4-dihydroquinazolin-2-yl)phenyl)piperidin-4-yl)methyl)piperazin-1-yl)-2-(2,6-dioxopiperidin-3-yl)-6-fluoroisoindoline-1,3-dione;
5-(3-((1-(4-(5,7-dimethoxy-4-oxo-3,4-dihydroquinazolin-2-yl)phenyl)piperidin-4-yl)methyl)-3,6-diazabicyclo[3.1.1]heptan-6-yl)-2-(2,6-dioxopiperidin-3-yl)isoindoline-1,3-dione;
5-(3-((1-(4-(5,7-dimethoxy-4-oxo-3,4-dihydroquinazolin-2-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(6-((1-(4-(5,7-dimethoxy-4-oxo-3,4-dihydroquinazolin-2-yl)phenyl)piperidin-4-yl)methyl)-3,6-diazabicyclo[3.1.1]heptan-3-yl)-2-(2,6-dioxopiperidin-3-yl)isoindoline-1,3-dione;

5-(6-((1-(4-(5,7-dimethoxy-4-oxo-3,4-dihydroquinazolin-2-yl)phenyl)piperidin-4-yl)methyl)-3,6-diazabicyclo[3.1.1]heptan-3-yl)-2-(2,6-dioxopiperidin-3-yl)-6-fluoroisoindoline-1,3-dione;
5-(3-((1-(4-(5,7-dimethoxy-4-oxo-3,4-dihydroquinazolin-2-yl)phenyl)piperidin-4-yl)methyl)-3,8-diazabicyclo[3.2.1]octan-8-yl)-2-(2,6-dioxopiperidin-3-yl)isoindoline-1,3-dione;
5-(3-((1-(4-(5,7-dimethoxy-4-oxo-3,4-dihydroquinazolin-2-yl)phenyl)piperidin-4-yl)methyl)-3,8-diazabicyclo[3.2.1]octan-8-yl)-2-(2,6-dioxopiperidin-3-yl)isoindoline-1,3-dione;
5-(8-((1-(4-(5,7-dimethoxy-4-oxo-3,4-dihydroquinazolin-2-yl)phenyl)piperidin-4-yl)methyl)-3,8-diazabicyclo[3.2.1]octan-3-yl)-2-(2,6-dioxopiperidin-3-yl)isoindoline-1,3-dione;
5-(8-((1-(4-(5,7-dimethoxy-4-oxo-3,4-dihydroquinazolin-2-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(5-((1-(4-(5,7-dimethoxy-4-oxo-3,4-dihydroquinazolin-2-yl)phenyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(4-(5,7-dimethoxy-4-oxo-3,4-dihydroquinazolin-2-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(4-(5,7-dimethoxy-4-oxo-3,4-dihydroquinazolin-2-yl)phenyl)piperidin-4-yl)methyl)-2,5-diazabicyclo[2.2.2]octan-2-yl)-2-(2,6-dioxopiperidin-3-yl)isoindoline-1,3-dione;
5-(5-((1-(4-(5,7-dimethoxy-4-oxo-3,4-dihydroquinazolin-2-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
6-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
6-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
5-(4-((1-(4-(5,7-dimethoxy-4-oxo-3,4-dihydroquinazolin-2-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(4-(5,7-dimethoxy-4-oxo-3,4-dihydroquinazolin-2-yl)phenyl)piperidin-4-yl)methyl)-2,6-dimethylpiperazin-1-yl)-2-(2,6-dioxopiperidin-3-yl)isoindoline-1,3-dione;
5-(4-((1-(4-(5,7-dimethoxy-4-oxo-3,4-dihydroquinazolin-2-yl)phenyl)piperidin-4-yl)methyl)-2,6-dimethylpiperazin-1-yl)-2-(2,6-dioxopiperidin-3 -yl)-6- fluoroisoindoline-1,3 -dione;
5-(4-((1-(4-(5,7-dimethoxy-4-oxo-3,4-dihydroquinazolin-2-yl)phenyl)piperidin-4-yl)methyl)-3,5-dimethylpiperazin-1-yl)-2-(2,6-dioxopiperidin-3-yl)isoindoline-1,3-dione;
5-(4-((1-(4-(5,7-dimethoxy-4-oxo-3,4-dihydroquinazolin-2-yl)phenyl)piperidin-4-yl)methyl)-3,5-dimethylpiperazin-1-yl)-2-(2,6-dioxopiperidin-3-yl)-6-fluoroisoindoline-1,3-dione;
5-(1-((1-(4-(5,7-dimethoxy-4-oxo-3,4-dihydroquinazolin-2-yl)phenyl)piperidin-4-yl)methyl)piperidin-4-yl)-2-(2,6-dioxopiperidin-3-yl)isoindoline-1,3-dione;
5-(1-((1-(4-(5,7-dimethoxy-4-oxo-3,4-dihydroquinazolin-2-yl)phenyl)piperidin-4-yl)methyl)piperidin-4-yl)-2-(2,6-dioxopiperidin-3-yl)-6-fluoroisoindoline-1,3-dione;
5-((R)-3-(((1-(4-(5,7-dimethoxy-4-oxo-3,4-dihydroquinazolin-2-yl)phenyl)piperidin-4-yl)methyl)amino)piperidin-1-yl)-2-(2,6-dioxopiperidin-3-yl)isoindoline-1,3-dione;
5-((S)-3-(((1-(4-(5,7-dimethoxy-4-oxo-3,4-dihydroquinazolin-2-yl)phenyl)piperidin-4-yl)methyl)amino)piperidin-1-yl)-2-(2,6-dioxopiperidin-3-yl)isoindoline-1,3-dione;
5-(((S)-1-((1-(4-(5,7-dimethoxy-4-oxo-3,4-dihydroquinazolin-2-yl)phenyl)piperidin-4-yl)methyl)piperidin-3-yl)amino)-2-(2,6-dioxopiperidin-3-yl)isoindoline-1,3-dione;
5-(((R)-1-((1-(4-(5,7-dimethoxy-4-oxo-3,4-dihydroquinazolin-2-yl)phenyl)piperidin-4-yl)methyl)piperidin-3-yl)amino)-2-(2,6-dioxopiperidin-3-yl)isoindoline-1,3-dione;
N-(2-chloro-6-methylphenyl)-2-((6-(4-((4-(2-(2,6-dioxopiperidin-3-yl)-1,3-dioxoisoindolin-5-yl)piperazin-1-yl)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-6-fluoro-1,3-dioxoisoindolin-5-yl)piperazin-1-yl)methyl)piperidin-1-yl)-2-methylpyrimidin-4-yl)amino)thiazole-5-carboxamide;

N-(2-chloro-6-methylphenyl)-2-((6-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-2-methylpyrimidin-4-yl)amino)thiazole-5-carboxamide;
N-(2-chloro-6-methylphenyl)-2-((6-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-2-methylpyrimidin-4-yl)amino)thiazole-5-carboxamide;
N-(2-chloro-6-methylphenyl)-2-((6-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-2-methylpyrimidin-4-yl)amino)thiazole-5-carboxamide;
N-(2-chloro-6-methylphenyl)-2-((6-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-2-methylpyrimidin-4-yl)amino)thiazole-5-carboxamide;
N-(2-chloro-6-methylphenyl)-2-((6-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-2-methylpyrimidin-4-yl)amino)thiazole-5-carboxamide;
N-(2-chloro-6-methylphenyl)-2-((6-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-2-methylpyrimidin-4-yl)amino)thiazole-5-carboxamide;
N-(2-chloro-6-methylphenyl)-2-((6-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-2-methylpyrimidin-4-yl)amino)thiazole-5-carboxamide;
N-(2-chloro-6-methylphenyl)-2-((6-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-2-methylpyrimidin-4-yl)amino)thiazole-5-carboxamide;
N-(2-chloro-6-methylphenyl)-2-((6-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-2-methylpyrimidin-4-yl)amino)thiazole-5-carboxamide;
N-(2-chloro-6-methylphenyl)-2-((6-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-2-methylpyrimidin-4-yl)amino)thiazole-5-carboxamide;
N-(2-chloro-6-methylphenyl)-2-((6-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-2-methylpyrimidin-4-yl)amino)thiazole-5-carboxamide;
N-(2-chloro-6-methylphenyl)-2-((6-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-1,3-dioxoisoindolin-5-yl)-3,5-dimethylpiperazin-1-yl)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-1,3-dioxoisoindolin-5-yl)-2,6-dimethylpiperazin-1-yl)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-2-methylpyrimidin-4-yl)amino)thiazole-5-carboxamide;
N-(2-chloro-6-methylphenyl)-2-((6-(4-((4-(2-(2, 6-dioxopiperidin-3-yl)-1, 3-dioxoisoindolin-5-yl)piperidin-1-yl)methyl)piperidin-1-yl)-2-methylpyrimidin-4-yl)amino)thiazole-5-carboxamide;
N-(2-chloro-6-methylphenyl)-2-((6-(4-((4-(2-(2,6-dioxopiperidin-3-yl)-6-fluoro-1,3-dioxoisoindolin-5-yl)piperidin-1-yl)methyl)piperidin-1-yl)-2-methylpyrimidin-4-yl)amino)thiazole-5-carboxamide;
N-(2-chloro-6-methylphenyl)-2-((6-(4-((((3R)-1-(2-(2,6-dioxopiperidin-3-yl)-1,3-dioxoisoindolin-5-yl)piperidin-3-yl)amino)methyl)piperidin-1-yl)-2-methylpyrimidin-4-yl)amino)thiazole-5-carboxamide;
N-(2-chloro-6-methylphenyl)-2-((6-(4-((((3S)-1-(2-(2,6-dioxopiperidin-3-yl)-1,3-dioxoisoindolin-5-yl)piperidin-3-yl)amino)methyl)piperidin-1-yl)-2-methylpyrimidin-4-yl)amino)thiazole-5-carboxamide;
N-(2-chloro-6-methylphenyl)-2-((6-(4-(((3S)-3-((2-(2,6-dioxopiperidin-3-yl)-1,3-dioxoisoindolin-5-yl)amino)piperidin-1-yl)methyl)piperidin-1-yl)-2-methylpyrimidin-4-yl)amino)thiazole-5-carboxamide;
N-(2-chloro-6-methylphenyl)-2-((6-(4-(((3R)-3-((2-(2,6-dioxopiperidin-3-yl)-1,3-dioxoisoindolin-5-yl)amino)piperidin-1-yl)methyl)piperidin-1-yl)-2-methylpyrimidin-4-yl)amino)thiazole-5-carboxamide;
4-((2,4-dichloro-5-methoxyphenyl)amino)-7-(3-(4-((4-(2-(2,6-dioxopiperidin-3-yl)-1,3-dioxoisoindolin-5-yl)piperazin-1-yl)methyl)piperidin-1-yl)propoxy)-6-methoxyquinoline-3-carbonitrile;
4-((2,4-dichloro-5-methoxyphenyl)amino)-7-(3-(4-((4-(2-(2,6-dioxopiperidin-3-yl)-6-fluoro-1,3-dioxoisoindolin-5-yl)piperazin-1-yl)methyl)piperidin-1-yl)propoxy)-6-methoxyquinoline-3-carbonitrile;
4-((2,4-dichloro-5-methoxyphenyl)amino)-7-(3-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)propoxy)-6-methoxyquinoline- 3 -carbonitrile;
4-((2,4-dichloro-5-methoxyphenyl)amino)-7-(3-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)propoxy)-6-methoxyquinoline- 3 -carbonitrile;
4-((2,4-dichloro-5-methoxyphenyl)amino)-7-(3-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)propoxy)-6-methoxyquinoline-3-carbonitrile;
4-((2,4-dichloro-5-methoxyphenyl)amino)-7-(3-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)propoxy)-6-methoxyquinoline-3-carbonitrile;
4-((2,4-dichloro-5-methoxyphenyl)amino)-7-(3-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)propoxy)-6-methoxyquinoline-3-carbonitrile;
4-((2,4-dichloro-5-methoxyphenyl)amino)-7-(3-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)propoxy)-6-methoxyquinoline-3-carbonitrile;
4-((2,4-dichloro-5-methoxyphenyl)amino)-7-(3-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)propoxy)-6-methoxyquinoline-3-carbonitrile;
4-((2,4-dichloro-5-methoxyphenyl)amino)-7-(3-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)propoxy)-6-methoxyquinoline-3-carbonitrile;
4-((2,4-dichloro-5-methoxyphenyl)amino)-7-(3-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)propoxy)-6-methoxyquinoline-3-carbonitrile;
4-((2,4-dichloro-5-methoxyphenyl)amino)-7-(3-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)propoxy)-6-methoxyquinoline-3-carbonitrile;
4-((2,4-dichloro-5-methoxyphenyl)amino)-7-(3-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)propoxy)-6-methoxyquinoline-3-carbonitrile;
4-((2,4-dichloro-5-methoxyphenyl)amino)-7-(3-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)propoxy)-6-methoxyquinoline-3-carbonitrile;
4-((2,4-dichloro-5-methoxyphenyl)amino)-7-(3-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile;
4-((2,4-dichloro-5-methoxyphenyl)amino)-7-(3-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile;
4-((2,4-dichloro-5-methoxyphenyl)amino)-7-(3-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile;
4-((2,4-dichloro-5-methoxyphenyl)amino)-7-(3-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile;
4-((2,4-dichloro-5-methoxyphenyl)amino)-7-(3-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)propoxy)-6-methoxy quinoline-3-carbonitrile;
4-((2,4-dichloro-5-methoxyphenyl)amino)-7-(3-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)propoxy)-6-methoxy quinoline-3-carbonitrile;
4-((2,4-dichloro-5-methoxyphenyl)amino)-7-(3-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)propoxy)-6-methoxyquinoline-3-carbonitrile;
4-((2,4-dichloro-5-methoxyphenyl)amino)-7-(3-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)propoxy)-6-methoxyquinoline-3 -carbonitrile;
4-((2,4-dichloro-5-methoxyphenyl)amino)-7-(3-(4-((4-(2-(2,6-dioxopiperidin-3-yl)-1,3-dioxoisoindolin-5-yl)-3,5-dimethylpiperazin-1-yl)methyl)piperidin-1-yl)propoxy)-6-methoxyquinoline- 3 -carbonitrile;
4-((2,4-dichloro-5-methoxyphenyl)amino)-7-(3-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)propoxy)-6-methoxyquinoline- 3 -carbonitrile;
4-((2,4-dichloro-5-methoxyphenyl)amino)-7-(3-(4-((4-(2-(2,6-dioxopiperidin-3-yl)-1,3-dioxoisoindolin-5-yl)-2,6-dimethylpiperazin-1-yl)methyl)piperidin-1-yl)propoxy)-6-methoxyquinoline- 3 -carbonitrile;
4-((2,4-dichloro-5-methoxyphenyl)amino)-7-(3-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)propoxy)-6-methoxyquinoline- 3 -carbonitrile;
4-((2,4-dichloro-5-methoxyphenyl)amino)-7-(3-(4-((4-(2-(2,6-dioxopiperidin-3-yl)-1,3-dioxoisoindolin-5-yl)piperidin-1-yl)methyl)piperidin-1-yl)propoxy)-6-methoxyquinoline-3-carbonitrile;
4-((2,4-dichloro-5-methoxyphenyl)amino)-7-(3-(4-((4-(2-(2,6-dioxopiperidin-3-yl)-6-fluoro-1,3-dioxoisoindolin-5-yl)piperidin-1-yl)methyl)piperidin-1-yl)propoxy)-6-methoxyquinoline-3-carbonitrile;
4-((2,4-dichloro-5-methoxyphenyl)amino)-7-(3-(4-((((3R)-1-(2-(2,6-dioxopiperidin-3-yl)-1,3-dioxoisoindolin-5-yl)piperidin-3-yl)amino)methyl)piperidin-1-yl)propoxy)-6-methoxyquinoline-3-carbonitrile;
4-((2,4-dichloro-5-methoxyphenyl)amino)-7-(3-(4-((((3S)-1-(2-(2,6-dioxopiperidin-3-yl)-1,3-dioxoisoindolin-5-yl)piperidin-3-yl)amino)methyl)piperidin-1-yl)propoxy)-6-methoxyquinoline-3-carbonitrile;
4-((2,4-dichloro-5-methoxyphenyl)amino)-7-(3-(4-(((3S)-3-((2-(2,6-dioxopiperidin-3-yl)-1,3-dioxoisoindolin-5-yl)amino)piperidin-1-yl)methyl)piperidin-1-yl)propoxy)-6-methoxyquinoline-3-carbonitrile;
4-((2,4-dichloro-5-methoxyphenyl)amino)-7-(3-(4-(((3R)-3-((2-(2,6-dioxopiperidin-3-yl)-1,3-dioxoisoindolin-5-yl)amino)piperidin-1-yl)methyl)piperidin-1-yl)propoxy)-6-methoxyquinoline-3-carbonitrile;
4-((4-((4-(2-(2,6-dioxopiperidin-3-yl)-1,3-dioxoisoindolin-5-yl)piperazin-1-yl)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-6-fluoro-1,3-dioxoisoindolin-5-yl)piperazin-1-yl)methyl)pip eri din-1-yl) methyl)-N-(4-methyl-3 -((4-(pyridin-3 -yl)pyrimidin-2-yl)amino)phenyl)benzamide;
4-((4-((6-(2-(2,6-dioxopiperidin-3-yl)-1,3-dioxoisoindolin-5-yl)-3,6-diazabicyclo[3.1.1]heptan-3-yl)methyl)piperidin-1-yl)methyl)-N-(4-methyl-3-((4-(pyridin-3-yl)pyrimidin-2-yl)amino)phenyl)benzamide;
4-((4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)methyl)-N-(4-methyl-3-((4-(pyridin-3-yl)pyrimidin-2-yl)amino)phenyl)benzamide;
4-((4-((3-(2-(2,6-dioxopiperidin-3-yl)-1,3-dioxoisoindolin-5-yl)-3,6-diazabicyclo[3.1.1]heptan-6-yl)methyl)piperidin-1-yl)methyl)-N-(4-methyl-3-((4-(pyridin-3-yl)pyrimidin-2-yl)amino)phenyl)benzamide;
4-((4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)methyl)-N-(4-methyl-3-((4-(pyridin-3-yl)pyrimidin-2-yl)amino)phenyl)benzamide;
4-((4-((8-(2-(2,6-dioxopiperidin-3-yl)-1,3-dioxoisoindolin-5-yl)-3,8-diazabicyclo[3.2.1]octan-3-yl)methyl)piperidin-1-yl)methyl)-N-(4-methyl-3-((4-(pyridin-3-yl)pyrimidin-2-yl)amino)phenyl)benzamide;
4-((4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)methyl)-N-(4-methyl-3-((4-(pyridin-3-yl)pyrimidin-2-yl)amino)phenyl)benzamide;
4-((4-((3-(2-(2,6-dioxopiperidin-3-yl)-1,3-dioxoisoindolin-5-yl)-3,8-diazabicyclo[3.2.1]octan-8-yl)methyl)piperidin-1-yl)methyl)-N-(4-methyl-3-((4-(pyridin-3-yl)pyrimidin-2-yl)amino)phenyl)benzamide;
4-((4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)methyl)-N-(4-methyl-3-((4-(pyridin-3-yl)pyrimidin-2-yl)amino)phenyl)benzamide;
4-((4-((5-(2-(2,6-dioxopiperidin-3-yl)-1,3-dioxoisoindolin-5-yl)-2,5-diazabicyclo[2.2.1]heptan-2-yl)methyl)piperidin-1-yl)methyl)-N-(4-methyl-3-((4-(pyridin-3-yl)pyrimidin-2-yl)amino)phenyl)benzamide;
4-((4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)methyl)-N-(4-methyl-3-((4-(pyridin-3-yl)pyrimidin-2-yl)amino)phenyl)benzamide;
4-((4-((5-(2-(2,6-dioxopiperidin-3-yl)-1,3-dioxoisoindolin-5-yl)-2,5-diazabicyclo[2.2.2]octan-2-yl)methyl)piperidin-1-yl)methyl)-N-(4-methyl-3-((4-(pyridin-3-yl)pyrimidin-2-yl)amino)phenyl)benzamide;
4-((4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-1,3-dioxoisoindolin-5-yl)-3,5-dimethylpiperazin-1-yl)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-6-fluoro-1,3-dioxoisoindolin-5-yl)-3,5-dimethylpiperazin-1-yl)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-1,3-dioxoisoindolin-5-yl)-2,6-dimethylpiperazin-1-yl)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-6-fluoro-1,3-dioxoisoindolin-5-yl)-2,6-dimethylpiperazin-1-yl)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-1,3-dioxoisoindolin-5-yl)piperidin-1-yl)methyl)piperidin-1-yl)methyl)-N-(4-methyl-3-((4-(pyridin-3-yl)pyrimidin-2-yl)amino)phenyl)benzamide;
4-((4-((4-(2-(2,6-dioxopiperidin-3-yl)-6-fluoro-1,3-dioxoisoindolin-5-yl)piperidin-1-yl)methyl)piperidin-1-yl)methyl)-N-(4-methyl-3-((4-(pyridin-3-yl)pyrimidin-2-yl)amino)phenyl)benzamide;
4-((4-((((3R)-1-(2-(2,6-dioxopiperidin-3-yl)-1,3-dioxoisoindolin-5-yl)piperidin-3-yl)amino)methyl)piperidin-1-yl)methyl)-N-(4-methyl-3-((4-(pyridin-3-yl)pyrimidin-2-yl)amino)phenyl)benzamide;
4-((4-((((3 S)-1-(2-(2,6-dioxopiperidin-3-yl)-1,3-dioxoisoindolin-5-yl)piperidin-3-yl)amino)methyl)piperidin-1-yl)methyl)-N-(4-methyl-3-((4-(pyridin-3-yl)pyrimidin-2-yl)amino)phenyl)benzamide;
4-((4-(((3S)-3-((2-(2,6-dioxopiperidin-3-yl)-1,3-dioxoisoindolin-5-yl)amino)piperidin-1-yl)methyl)piperidin-1-yl)methyl)-N-(4-methyl-3-((4-(pyridin-3-yl)pyrimidin-2-yl)amino)phenyl)benzamide;
4-((4-(((3R)-3-((2-(2,6-dioxopiperidin-3-yl)-1,3-dioxoisoindolin-5-yl)amino)piperidin-1-yl)methyl)piperidin-1-yl)methyl)-N-(4-methyl-3-((4-(pyridin-3-yl)pyrimidin-2-yl)amino)phenyl)benzamide;
2-((2-((4-(4-((4-(2-(2,6-dioxopiperidin-3-yl)-1,3-dioxoisoindolin-5-yl)piperazin-1-yl)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-6-fluoro-1,3-dioxoisoindolin-5-yl)piperazin-1-yl)methyl)piperidin-1-yl)-2-methoxyphenyl)amino)-5-(trifluoromethyl)pyridin-4-yl)amino)-N-methylbenzamide;
2-((2-((4-(4-((6-(2-(2,6-dioxopiperidin-3-yl)-1,3-dioxoisoindolin-5-yl)-3,6-diazabicyclo[3.1.1]heptan-3-yl)methyl)piperidin-1-yl)-2-methoxyphenyl)amino)-5-(trifluoromethyl)pyridin-4-yl)amino)-N-methylbenzamide;
2-((2-((4-(4-((6-(2-(2,6-dioxopiperidin-3-yl)-6-fluoro-1,3-dioxoisoindolin-5-yl)-3,6-diazabicyclo[3.1.1]heptan-3-yl)methyl)piperidin-1-yl)-2-methoxyphenyl)amino)-5-(trifluoromethyl)pyridin-4-yl)amino)-N-methylbenzamide;
2-((2-((4-(4-((3-(2-(2,6-dioxopiperidin-3-yl)-1,3-dioxoisoindolin-5-yl)-3,6-diazabicyclo[3.1.1]heptan-6-yl)methyl)piperidin-1-yl)-2-methoxyphenyl)amino)-5-(trifluoromethyl)pyridin-4-yl)amino)-N-methylbenzamide;
2-((2-((4-(4-((3-(2-(2,6-dioxopiperidin-3-yl)-6-fluoro-1,3-dioxoisoindolin-5-yl)-3,6-diazabicyclo[3.1.1]heptan-6-yl)methyl)piperidin-1-yl)-2-methoxyphenyl)amino)-5-(trifluoromethyl)pyridin-4-yl)amino)-N-methylbenzamide;
2-((2-((4-(4-((8-(2-(2,6-dioxopiperidin-3-yl)-1,3-dioxoisoindolin-5-yl)-3,8-diazabicyclo[3.2.1]octan-3-yl)methyl)piperidin-1-yl)-2-methoxyphenyl)amino)-5-(trifluoromethyl)pyridin-4-yl)amino)-N-methylbenzamide;
2-((2-((4-(4-((8-(2-(2,6-dioxopiperidin-3-yl)-6-fluoro-1,3-dioxoisoindolin-5-yl)-3,8-diazabicyclo[3.2.1]octan-3-yl)methyl)piperidin-1-yl)-2-methoxyphenyl)amino)-5-(trifluoromethyl)pyridin-4-yl)amino)-N-methylbenzamide;
2-((2-((4-(4-((3-(2-(2,6-dioxopiperidin-3-yl)-1,3-dioxoisoindolin-5-yl)-3,8-diazabicyclo[3.2.1]octan-8-yl)methyl)piperidin-1-yl)-2-methoxyphenyl)amino)-5-(trifluoromethyl)pyridin-4-yl)amino)-N-methylbenzamide;
2-((2-((4-(4-((3-(2-(2,6-dioxopiperidin-3-yl)-6-fluoro-1,3-dioxoisoindolin-5-yl)-3,8-diazabicyclo[3.2.1]octan-8-yl)methyl)piperidin-1-yl)-2-methoxyphenyl)amino)-5-(trifluoromethyl)pyridin-4-yl)amino)-N-methylbenzamide;
2-((2-((4-(4-((5-(2-(2,6-dioxopiperidin-3-yl)-1,3-dioxoisoindolin-5-yl)-2,5-diazabicyclo[2.2.1]heptan-2-yl)methyl)piperidin-1-yl)-2-methoxyphenyl)amino)-5-(trifluoromethyl)pyridin-4-yl)amino)-N-methylbenzamide;
2-((2-((4-(4-((5-(2-(2,6-dioxopiperidin-3-yl)-6-fluoro-1,3-dioxoisoindolin-5-yl)-2,5-diazabicyclo[2.2.1]heptan-2-yl)methyl)piperidin-1-yl)-2-methoxyphenyl)amino)-5-(trifluoromethyl)pyridin-4-yl)amino)-N-methylbenzamide;
2-((2-((4-(4-((5-(2-(2,6-dioxopiperidin-3-yl)-1,3-dioxoisoindolin-5-yl)-2,5-diazabicyclo[2.2.2]octan-2-yl)methyl)piperidin-1-yl)-2-methoxyphenyl)amino)-5-(trifluoromethyl)pyridin-4-yl)amino)-N-methylbenzamide;
2-((2-((4-(4-((5-(2-(2,6-dioxopiperidin-3-yl)-6-fluoro-1,3-dioxoisoindolin-5-yl)-2,5-diazabicyclo[2.2.2]octan-2-yl)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-6-fluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-7-fluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-4-fluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-4,6-difluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-4,7-difluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-6,7-difluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-4,6,7-trifluoro-1,3-dioxoisoindolin-5-yl)piperazin-1-yl-2,2,3,3,5,5,6,6-$d_8$)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-1,3-dioxoisoindolin-5-yl)-3,5-dimethylpiperazin-1-yl)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-6-fluoro-1,3-dioxoisoindolin-5-yl)-3,5-dimethylpiperazin-1-yl)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-1,3-dioxoisoindolin-5-yl)-2,6-dimethylpiperazin-1-yl)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-6-fluoro-1,3-dioxoisoindolin-5-yl)-2,6-dimethylpiperazin-1-yl)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-1,3-dioxoisoindolin-5-yl)piperidin-1-yl)methyl)piperidin-1-yl)-2-methoxyphenyl)amino)-5-(trifluoromethyl)pyridin-4-yl)amino)-N-methylbenzamide;
2-((2-((4-(4-((4-(2-(2,6-dioxopiperidin-3-yl)-6-fluoro-1,3-dioxoisoindolin-5-yl)piperidin-1-yl)methyl)piperidin-1-yl)-2-methoxyphenyl)amino)-5-(trifluoromethyl)pyridin-4-yl)amino)-N-methylbenzamide;
2-((2-((4-(4-((((3R)-1-(2-(2,6-dioxopiperidin-3-yl)-1,3-dioxoisoindolin-5-yl)piperidin-3-yl)amino)methyl)piperidin-1-yl)-2-methoxyphenyl)amino)-5-(trifluoromethyl)pyridin-4-yl)amino)-N-methylbenzamide;
2-((2-((4-(4-((((3S)-1-(2-(2,6-dioxopiperidin-3-yl)-1,3-dioxoisoindolin-5-yl)piperidin-3-yl)amino)methyl)piperidin-1-yl)-2-methoxyphenyl)amino)-5-(trifluoromethyl)pyridin-4-yl)amino)-N-methylbenzamide;
2-((2-((4-(4-(((3S)-3-((2-(2,6-dioxopiperidin-3-yl)-1,3-dioxoisoindolin-5-yl)amino)piperidin-1-yl)methyl)piperidin-1-yl)-2-methoxyphenyl)amino)-5-(trifluoromethyl)pyridin-4-yl)amino)-N-methylbenzamide;
2-((2-((4-(4-(((3R)-3-((2-(2,6-dioxopiperidin-3-yl)-1,3-dioxoisoindolin-5-yl)amino)piperidin-1-yl)methyl)piperidin-1-yl)-2-methoxyphenyl)amino)-5-(trifluoromethyl)pyridin-4-yl)amino)-N-methylbenzamide;
5-(4-((1-(2-(4-(4-chloro-1,2-diphenylbut-1-en-1-yl)phenoxy)ethyl)piperidin-4-yl)methyl)piperazin-1-yl)-2-(2,6-dioxopiperidin-3-yl)isoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)piperazin-1-yl)-2-(2,6-dioxopiperidin-3-yl)isoindoline-1,3-dione;
5-(4-((1-(2-(4-(4-chloro-1,2-bis(4-hydroxyphenyl)but-1-en-1-yl)phenoxy)ethyl)piperidin-4-yl)methyl)piperazin-1-yl)-2-(2,6-dioxopiperidin-3-yl)isoindoline-1,3-dione;
5-(4-((1-(2-(4-(4-chloro-1,2-diphenylbut-1-en-1-yl)phenoxy)ethyl)piperidin-4-yl)methyl)piperazin-1-yl)-2-(2,6-dioxopiperidin-3-yl)-6-fluoroisoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)piperazin-1-yl)-2-(2,6-dioxopiperidin-3-yl)-6-fluoroisoindoline-1,3-dione;
5-(4-((1-(2-(4-(4-chloro-1,2-bis(4-hydroxyphenyl)but-1-en-1-yl)phenoxy)ethyl)piperidin-4-yl)methyl)piperazin-1-yl)-2-(2,6-dioxopiperidin-3-yl)-6-fluoroisoindoline-1,3-dione;
5-(3-((1-(2-(4-(4-chloro-1,2-diphenylbut-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione;
5-(3-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione;
5-(3-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione;
5-(3-((1-(2-(4-(4-chloro-1,2-diphenylbut-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(3-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(3-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(6-((1-(2-(4-(4-chloro-1,2-diphenylbut-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(2-(4-(4-chloro-1,2-diphenylbut-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione;
5-(6-((1-(2-(4-(4-chloro-1-(4-hydroxyphenyl)-2-phenylbut-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione;
5-(6-((1-(2-(4-(4-chloro-1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione;

5-(3-((1-(2-(4-(4-chloro-1,2-diphenylbut-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione;
5-(3-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione;
5-(3-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione;
5-(3-((1-(2-(4-(4-chloro-1,2-diphenylbut-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione;
5-(3-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione;
5-(3-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione;
5-(8-((1-(2-(4-(4-chloro-1,2-diphenylbut-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione;
5-(8-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione;
5-(8-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione;
5-(8-((1-(2-(4-(4-chloro-1,2-diphenylbut-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(8-((1-(2-(4-(4-chloro-1-(4-hydroxyphenyl)-2-phenylbut-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(8-((1-(2-(4-(4-chloro-1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(5-((1-(2-(4-(4-chloro-1,2-diphenylbut-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(2-(4-(4-chloro-1,2-diphenylbut-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(2-(4-(4-chloro-1,2-diphenylbut-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione;
5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione;
5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione;
5-(5-((1-(2-(4-(4-chloro-1,2-diphenylbut-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(5-((1-(2-(4-(4-chloro-1-(4-hydroxyphenyl)-2-phenylbut-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(5-((1-(2-(4-(4-chloro-1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;

5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
6-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
6-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
6-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
6-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
6-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
6-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
5-(4-((1-(2-(4-(4-chloro-1,2-diphenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(2-(4-(4-chloro-1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(2-(4-(4-chloro-1,2-diphenylbut-1-en-1-yl)phenoxy)ethyl)piperidin-4-yl)methyl)-2,6-dimethylpiperazin-1-yl)-2-(2,6-dioxopiperidin-3-yl)isoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)-2,6-dimethylpiperazin-1-yl)-2-(2,6-dioxopiperidin-3-yl)isoindoline-1,3-dione;
5-(4-((1-(2-(4-(4-chloro-1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)isoindoline-1,3-dione;
5-(4-((1-(2-(4-(4-chloro-1,2-diphenylbut-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione;
5-(4-((1-(2-(4-(4-chloro-1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione;
5-(4-((1-(2-(4-(4-chloro-1,2-diphenylbut-1-en-1-yl)phenoxy)ethyl)piperidin-4-yl)methyl)-3,5-dimethylpiperazin-1-yl)-2-(2,6-dioxopiperidin-3-yl)isoindoline-1,3-dione;
5-(4-((1-(2-(4-(4-chloro-1-(4-hydroxyphenyl)-2-phenylbut-1-en-1-yl)phenoxy)ethyl)piperidin-4-yl)methyl)-3,5-dimethylpiperazin-1-yl)-2-(2,6-dioxopiperidin-3-yl)isoindoline-1,3-dione;
5-(4-((1-(2-(4-(4-chloro-1,2-bis(4-hydroxyphenyl)but-1-en-1-yl)phenoxy)ethyl)piperidin-4-yl)methyl)-3,5-dimethylpiperazin-1-yl)-2-(2,6-dioxopiperidin-3-yl)isoindoline-1,3-dione;
5-(4-((1-(2-(4-(4-chloro-1,2-diphenylbut-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione;

5-(4-((1-(2-(4-(4-chloro-1-(4-hydroxyphenyl)-2-phenylbut-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione;
5-(4-((1-(2-(4-(4-chloro-1,2-bis(4-hydroxyphenyl)but-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione;
5-(1-((1-(2-(4-(4-chloro-1,2-diphenylbut-1-en-1-yl)phenoxy)ethyl)piperidin-4-yl)methyl)piperidin-4-yl)-2-(2,6-dioxopiperidin-3-yl)isoindoline-1,3-dione;
5-(1-((1-(2-(4-(4-chloro-1-(4-hydroxyphenyl)-2-phenylbut-1-en-1-yl)phenoxy)ethyl)piperidin-4-yl)methyl)piperidin-4-yl)-2-(2,6-dioxopiperidin-3-yl)isoindoline-1,3-dione;
5-(1-((1-(2-(4-(4-chloro-1,2-bis(4-hydroxyphenyl)but-1-en-1-yl)phenoxy)ethyl)piperidin-4-yl)methyl)piperidin-4-yl)-2-(2,6-dioxopiperidin-3-yl)isoindoline-1,3-dione;
5-(1-((1-(2-(4-(4-chloro-1,2-diphenylbut-1-en-1-yl)phenoxy)ethyl)piperidin-4-yl)methyl)piperidin-4-yl)-2-(2,6-dioxopiperidin-3-yl)-6-fluoroisoindoline-1,3-dione;
5-(1-((1-(2-(4-(4-chloro-1-(4-hydroxyphenyl)-2-phenylbut-1-en-1-yl)phenoxy)ethyl)piperidin-4-yl)methyl)piperidin-4-yl)-2-(2,6-dioxopiperidin-3-yl)-6-fluoroisoindoline-1,3-dione;
5-(1-((1-(2-(4-(4-chloro-1,2-bis(4-hydroxyphenyl)but-1-en-1-yl)phenoxy)ethyl)piperidin-4-yl)methyl)piperidin-4-yl)-2-(2,6-dioxopiperidin-3-yl)-6-fluoroisoindoline-1,3-dione;
5-((R)-3-(((1-(2-(4-(4-chloro-1,2-diphenylbut-1-en-1-yl)phenoxy)ethyl)piperidin-4-yl)methyl)amino)piperidin-1-yl)-2-(2,6-dioxopiperidin-3-yl)isoindoline-1,3-dione;
5-((R)-3-(((1-(2-(4-(4-chloro-1-(4-hydroxyphenyl)-2-phenylbut-1-en-1-yl)phenoxy)ethyl)piperidin-4-yl)methyl)amino)piperidin-1-yl)-2-(2,6-dioxopiperidin-3-yl)isoindoline-1,3-dione;
5-((R)-3-(((1-(2-(4-(4-chloro-1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione;
5-((S)-3-(((1-(2-(4-(4-chloro-1,2-diphenylbut-1-en-1-yl)phenoxy)ethyl)piperidin-4-yl)methyl)amino)piperidin-1-yl)-2-(2,6-dioxopiperidin-3-yl)isoindoline-1,3-dione;
5-((S)-3-(((1-(2-(4-(4-chloro-1-(4-hydroxyphenyl)-2-phenylbut-1-en-1-yl)phenoxy)ethyl)piperidin-4-yl)methyl)amino)piperidin-1-yl)-2-(2,6-dioxopiperidin-3-yl)isoindoline-1,3-dione;
5-((S)-3-(((1-(2-(4-(4-chloro-1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione;
5-(((S)-1-((1-(2-(4-(4-chloro-1,2-diphenylbut-1-en-1-yl)phenoxy)ethyl)piperidin-4-yl)methyl)piperidin-3-yl)amino)-2-(2,6-dioxopiperidin-3-yl)isoindoline-1,3-dione;
5-(((S)-1-((1-(2-(4-(4-chloro-1-(4-hydroxyphenyl)-2-phenylbut-1-en-1-yl)phenoxy)ethyl)piperidin-4-yl)methyl)piperidin-3-yl)amino)-2-(2,6-dioxopiperidin-3-yl)isoindoline-1,3 -dione;
5-(((S)-1-((1-(2-(4-(4-chloro-1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione;
5-(((R)-1-((1-(2-(4-(4-chloro-1,2-diphenylbut-1-en-1-yl)phenoxy)ethyl)piperidin-4-yl)methyl)piperidin-3-yl)amino)-2-(2,6-dioxopiperidin-3-yl)isoindoline-1,3-dione;
5-(((R)-1-((1-(2-(4-(4-chloro-1-(4-hydroxyphenyl)-2-phenylbut-1-en-1-yl)phenoxy)ethyl)piperidin-4-yl)methyl)piperidin-3-yl)amino)-2-(2,6-dioxopiperidin-3-yl)isoindoline-1,3 -dione;
5-(((R)-1-((1-(2-(4-(4-chloro-1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione;
5-(4-((1-(4-(4-chloro-1-(4-hydroxyphenyl)-2-phenylbut-1-en-1-yl)phenyl)piperidin-4-yl)methyl)piperazin-1-yl)-2-(2,6-dioxopiperidin-3-yl)isoindoline-1,3-dione;
5-(4-((1-(4-(4-chloro-1-(4-hydroxyphenyl)-2-phenylbut-1-en-1-yl)phenyl)piperidin-4-yl)methyl)piperazin-1-yl)-2-(2,6-dioxopiperidin-3-yl)-6-fluoroisoindoline-1,3-dione;
5-(3-((1-(4-(4-chloro-1-(4-hydroxyphenyl)-2-phenylbut-1-en-1-yl)phenyl)piperidin-4-yl)methyl)-3,6-diazabicyclo[3.1.1]heptan-6-yl)-2-(2,6-dioxopiperidin-3-yl)isoindoline-1,3-dione;
5-(3-((1-(4-(4-chloro-1-(4-hydroxyphenyl)-2-phenylbut-1-en-1-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(6-((1-(4-(4-chloro-1-(4-hydroxyphenyl)-2-phenylbut-1-en-1-yl)phenyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(4-(4-chloro-1-(4-hydroxyphenyl)-2-phenylbut-1-en-1-yl)phenyl)piperidin-4-yl)methyl)-3,6-diazabicyclo[3.1.1]heptan-3-yl)-2-(2,6-dioxopiperidin-3-yl)-6-fluoroisoindoline-1,3-dione;
5-(3-((1-(4-(4-chloro-1-(4-hydroxyphenyl)-2-phenylbut-1-en-1-yl)phenyl)piperidin-4-yl)methyl)-3,8-diazabicyclo[3.2.1]octan-8-yl)-2-(2,6-dioxopiperidin-3-yl)isoindoline-1,3-dione;
5-(3-((1-(4-(4-chloro-1-(4-hydroxyphenyl)-2-phenylbut-1-en-1-yl)phenyl)piperidin-4-yl)methyl)-3,8-diazabicyclo[3.2.1]octan-8-yl)-2-(2,6-dioxopiperidin-3-yl)-6-fluoroisoindoline-1,3-dione;
5-(8-((1-(4-(4-chloro-1-(4-hydroxyphenyl)-2-phenylbut-1-en-1-yl)phenyl)piperidin-4-yl)methyl)-3,8-diazabicyclo[3.2.1]octan-3-yl)-2-(2,6-dioxopiperidin-3-yl)isoindoline-1,3-dione;

5-(8-((1-(4-(4-chloro-1-(4-hydroxyphenyl)-2-phenylbut-1-en-1-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(5-((1-(4-(4-chloro-1-(4-hydroxyphenyl)-2-phenylbut-1-en-1-yl)phenyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(4-(4-chloro-1-(4-hydroxyphenyl)-2-phenylbut-1-en-1-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(4-(4-chloro-1-(4-hydroxyphenyl)-2-phenylbut-1-en-1-yl)phenyl)piperidin-4-yl)methyl)-2,5-diazabicyclo[2.2.2]octan-2-yl)-2-(2,6-dioxopiperidin-3-yl)isoindoline-1,3-dione;
5-(5-((1-(4-(4-chloro-1-(4-hydroxyphenyl)-2-phenylbut-1-en-1-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
6-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
6-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
5-(4-((1-(4-(4-chloro-1-(4-hydroxyphenyl)-2-phenylbut-1-en-1-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(4-(4-chloro-1-(4-hydroxyphenyl)-2-phenylbut-1-en-1-yl)phenyl)piperidin-4-yl)methyl)-2,6-dimethylpiperazin-1-yl)-2-(2,6-dioxopiperidin-3-yl)isoindoline-1,3-dione;
5-(4-((1-(4-(4-chloro-1-(4-hydroxyphenyl)-2-phenylbut-1-en-1-yl)phenyl)piperidin-4-yl)methyl)-2,6-dimethylpiperazin-1-yl)-2-(2,6-dioxopiperidin-3-yl)-6-fluoroisoindoline-1,3-dione;
5-(4-((1-(4-(4-chloro-1-(4-hydroxyphenyl)-2-phenylbut-1-en-1-yl)phenyl)piperidin-4-yl)methyl)-3,5-dimethylpiperazin-1-yl)-2-(2,6-dioxopiperidin-3-yl)isoindoline-1,3-dione;
5-(4-((1-(4-(4-chloro-1-(4-hydroxyphenyl)-2-phenylbut-1-en-1-yl)phenyl)piperidin-4-yl)methyl)-3,5-dimethylpiperazin-1-yl)-2-(2,6-dioxopiperidin-3-yl)-6-fluoroisoindoline-1,3-dione;
5-(1-((1-(4-(4-chloro-1-(4-hydroxyphenyl)-2-phenylbut-1-en-1-yl)phenyl)piperidin-4-yl)methyl)piperidin-4-yl)-2-(2,6-dioxopiperidin-3-yl)isoindoline-1,3-dione;
5-(1-((1-(4-(4-chloro-1-(4-hydroxyphenyl)-2-phenylbut-1-en-1-yl)phenyl)piperidin-4-yl)methyl)piperidin-4-yl)-2-(2,6-dioxopiperidin-3-yl)-6-fluoroisoindoline-1,3-dione;
5 -((R)-3 -(((1 -(4-(4-chloro-1 -(4-hydroxyphenyl)-2-phenylbut-1 -en-1 -yl)phenyl)piperidin-4-yl)methyl)amino)piperidin-1-yl)-2-(2,6-dioxopiperidin-3-yl)isoindoline-1,3-dione;
5-((S)-3-(((1-(4-(4-chloro-1-(4-hydroxyphenyl)-2-phenylbut-1-en-1-yl)phenyl)piperidin-4-yl)methyl)amino)piperidin-1-yl)-2-(2,6-dioxopiperidin-3-yl)isoindoline-1,3-dione;
5-(((S)-1-((1-(4-(4-chloro-1-(4-hydroxyphenyl)-2-phenylbut-1-en-1-yl)phenyl)piperidin-4-yl)methyl)piperidin-3-yl)amino)-2-(2,6-dioxopiperidin-3-yl)isoindoline-1,3-dione;
5-(((R)-1-((1-(4-(4-chloro-1-(4-hydroxyphenyl)-2-phenylbut-1-en-1-yl)phenyl)piperidin-4-yl)methyl)piperidin-3-yl)amino)-2-(2,6-dioxopiperidin-3-yl)isoindoline-1,3-dione;
5-(4-((1-(4-(4-chloro-1,2-bis(4-hydroxyphenyl)but-1-en-1-yl)phenyl)piperidin-4-yl)methyl)piperazin-1-yl)-2-(2,6-dioxopiperidin-3-yl)isoindoline-1,3-dione;
5-(4-((1-(4-(4-chloro-1,2-bis(4-hydroxyphenyl)but-1-en-1-yl)phenyl)piperidin-4-yl)methyl)piperazin-1-yl)-2-(2,6-dioxopiperidin-3-yl)-6-fluoroisoindoline-1,3-dione;
5-(3-((1-(4-(4-chloro-1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)isoindoline-1,3-dione;
5-(3-((1-(4-(4-chloro-1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(6-((1-(4-(4-chloro-1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(4-(4-chloro-1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)-6-fluoroisoindoline-1,3-dione;

5-(3-((1-(4-(4-chloro-1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)isoindoline-1,3-dione;
5-(3-((1-(4-(4-chloro-1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)-6-fluoroisoindoline-1,3-dione;
5-(8-((1-(4-(4-chloro-1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)isoindoline-1,3-dione;
5-(8-((1-(4-(4-chloro-1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(5-((1-(4-(4-chloro-1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(4-(4-chloro-1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(4-(4-chloro-1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)isoindoline-1,3-dione;
5-(5-((1-(4-(4-chloro-1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
6-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
6-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
5-(4-((1-(4-(4-chloro-1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(4-(4-chloro-1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)isoindoline-1,3-dione;
5-(4-((1-(4-(4-chloro-1,2-bis(4-hydroxyphenyl)but-1-en-1-yl)phenyl)piperidin-4-yl)methyl)-2,6-dimethylpip erazin-1-yl)-2-(2, 6-dioxopip eridin-3 -yl)-6-fluoroi soindoline-1, 3 -dione;
5-(4-((1-(4-(4-chloro-1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)isoindoline-1,3-dione;
5-(4-((1-(4-(4-chloro-1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)-6-fluoroisoindoline-1,3-dione;
5-(1-((1-(4-(4-chloro-1,2-bis(4-hydroxyphenyl)but-1-en-1-yl)phenyl)piperidin-4-yl)methyl)piperidin-4-yl)-2-(2,6-dioxopiperidin-3-yl)isoindoline-1,3-dione;
5-(1-((1-(4-(4-chloro-1,2-bis(4-hydroxyphenyl)but-1-en-1-yl)phenyl)piperidin-4-yl)methyl)piperidin-4-yl)-2-(2,6-dioxopiperidin-3-yl)-6-fluoroisoindoline-1,3-dione;
5 -((R)-3 -(((1 -(4-(4-chloro-1,2-bis(4-hydroxyphenyl)but-1 -en-1 -yl)phenyl)piperidin-4-yl)methyl)amino)piperidin-1-yl)-2-(2,6-dioxopiperidin-3-yl)isoindoline-1,3-dione;
5 -((S)-3 -(((1 -(4-(4-chloro-1,2-bis(4-hydroxyphenyl)but-1 -en-1 -yl)phenyl)piperidin-4-yl)methyl)amino)piperidin-1-yl)-2-(2,6-dioxopiperidin-3-yl)isoindoline-1,3-dione;
5-(((S)-1-((1-(4-(4-chloro-1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione;
5-(((R)-1-((1-(4-(4-chloro-1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl)isoindoline-1,3-dione;
5-(4-((1-(2-(4-(1,2-bis(4-hydroxyphenyl)but-1-en-1-yl)phenoxy)ethyl)piperidin-4-yl)methyl)piperazin-1-yl)-2-(2,6-dioxopiperidin-3-yl)isoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl)-6-fluoroisoindoline-1,3-dione;

2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)piperazin-1-yl)isoindoline-1,3-dione;
2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)piperazin-1-yl)isoindoline-1,3-dione;
5-(4-((1-(2-(4-(1,2-bis(4-hydroxyphenyl)but-1-en-1-yl)phenoxy)ethyl)piperidin-4-yl)methyl)piperazin-1-yl)-2-(2,6-dioxopiperidin-3-yl)-6-fluoroisoindoline-1,3-dione;
2-(2,6-dioxopiperidin-3-yl)-5-(3-((1-(2-(4-(1,2-diphenylbut-1-en-1-yl)phenoxy)ethyl)piperidin-4-yl)methyl)-3,6-diazabicyclo[3.1.1]heptan-6-yl)isoindoline-1,3-dione;
2-(2,6-dioxopiperidin-3-yl)-5-(3-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-6-yl)isoindoline-1,3-dione;
5-(3-((1-(2-(4-(1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)isoindoline-1,3-dione;
2-(2,6-dioxopiperidin-3-yl)-5-(3-((1-(2-(4-(1,2-diphenylbut-1-en-1-yl)phenoxy)ethyl)piperidin-4-yl)methyl)-3,6-diazabicyclo[3.1.1]heptan-6-yl)-6-fluoroisoindoline-1,3-dione;
2-(2,6-dioxopiperidin-3-yl)-5-fluoro-6-(3-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-6-yl)isoindoline-1,3-dione;
5-(3-((1-(2-(4-(1,2-bis(4-hydroxyphenyl)but-1-en-1-yl)phenoxy)ethyl)piperidin-4-yl)methyl)-3,6-diazabicyclo[3.1.1]heptan-6-yl)-2-(2,6-dioxopiperidin-3-yl)-6-fluoroisoindoline-1,3-dione;
2-(2,6-dioxopiperidin-3-yl)-5-(6-((1-(2-(4-(1,2-diphenylbut-1-en-1-yl)phenoxy)ethyl)piperidin-4-yl)methyl)-3,6-diazabicyclo[3.1.1]heptan-3-yl)isoindoline-1,3-dione;
2-(2,6-dioxopiperidin-3-yl)-5-(6-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-3-yl)isoindoline-1,3-dione;
5-(6-((1-(2-(4-(1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)isoindoline-1,3-dione;
2-(2,6-dioxopiperidin-3-yl)-5-(6-((1-(2-(4-(1,2-diphenylbut-1-en-1-yl)phenoxy)ethyl)piperidin-4-yl)methyl)-3,6-diazabicyclo[3.1.1]heptan-3-yl)-6-fluoroisoindoline-1,3-dione;
2-(2,6-dioxopiperidin-3-yl)-5-fluoro-6-(6-((1-(2-(4-(1-(4-hydroxyphenyl)-2-phenylbut-1-en-1-yl)phenoxy)ethyl)piperidin-4-yl)methyl)-3,6-diazabicyclo[3.1.1]heptan-3-yl)isoindoline-1,3-dione;
5-(6-((1-(2-(4-(1,2-bis(4-hydroxyphenyl)but-1-en-1-yl)phenoxy)ethyl)piperidin-4-yl)methyl)-3,6-diazabicyclo[3.1.1]heptan-3-yl)-2-(2,6-dioxopiperidin-3-yl)-6-fluoroisoindoline-1,3-dione;
2-(2,6-dioxopiperidin-3-yl)-5-(3-((1-(2-(4-(1,2-diphenylbut-1-en-1-yl)phenoxy)ethyl)piperidin-4-yl)methyl)-3,8-diazabicyclo[3.2.1]octan-8-yl)isoindoline-1,3-dione;
2-(2,6-dioxopiperidin-3-yl)-5-(3-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-8-yl)isoindoline-1,3-dione;
5-(3-((1-(2-(4-(1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)isoindoline-1,3-dione;
2-(2,6-dioxopiperidin-3-yl)-5-(3-((1-(2-(4-(1,2-diphenylbut-1-en-1-yl)phenoxy)ethyl)piperidin-4-yl)methyl)-3,8-diazabicyclo[3.2.1]octan-8-yl)-6-fluoroisoindoline-1,3-dione;
2-(2,6-dioxopiperidin-3-yl)-5-fluoro-6-(3-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-8-yl)isoindoline-1,3-dione;
5-(3-((1-(2-(4-(1,2-bis(4-hydroxyphenyl)but-1-en-1-yl)phenoxy)ethyl)piperidin-4-yl)methyl)-3,8-diazabicyclo[3.2.1]octan-8-yl)-2-(2,6-dioxopiperidin-3-yl)-6-fluoroisoindoline-1,3-dione;
2-(2,6-dioxopiperidin-3-yl)-5-(8-((1-(2-(4-(1,2-diphenylbut-1-en-1-yl)phenoxy)ethyl)piperidin-4-yl)methyl)-3,8-diazabicyclo[3.2.1]octan-3-yl)isoindoline-1,3-dione;
2-(2,6-dioxopiperidin-3-yl)-5-(8-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-3-yl)isoindoline-1,3-dione;
5-(8-((1-(2-(4-(1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)isoindoline-1,3-dione;
2-(2,6-dioxopiperidin-3-yl)-5-(8-((1-(2-(4-(1,2-diphenylbut-1-en-1-yl)phenoxy)ethyl)piperidin-4-yl)methyl)-3,8-diazabicyclo[3.2.1]octan-3-yl)-6-fluoroisoindoline-1,3-dione;
2-(2,6-dioxopiperidin-3-yl)-5-fluoro-6-(8-((1-(2-(4-(1-(4-hydroxyphenyl)-2-phenylbut-1-en-1-yl)phenoxy)ethyl)piperidin-4-yl)methyl)-3,8-diazabicyclo[3.2.1]octan-3-yl)isoindoline-1,3-dione;
5-(8-((1-(2-(4-(1,2-bis(4-hydroxyphenyl)but-1-en-1-yl)phenoxy)ethyl)piperidin-4-yl)methyl)-3,8-diazabicyclo[3.2.1]octan-3-yl)-2-(2,6-dioxopiperidin-3-yl)-6-fluoroisoindoline-1,3-dione;
2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.1]heptan-2-yl)isoindoline-1,3-dione;
5-(5-((1-(2-(4-(1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)isoindoline-1,3-dione;

2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.1]heptan-2-yl)-6-fluoroisoindoline-1,3-dione;
2-(2,6-dioxopiperidin-3-yl)-5-fluoro-6-(5-((1-(2-(4-(1-(4-hydroxyphenyl)-2-phenylbut-1-en-1-yl)phenoxy)ethyl)piperidin-4-yl)methyl)-2,5-diazabicyclo[2.2.1]heptan-2-yl)isoindoline-1,3-dione;
5-(5-((1-(2-(4-(1,2-bis(4-hydroxyphenyl)but-1-en-1-yl)phenoxy)ethyl)piperidin-4-yl)methyl)-2,5-diazabicyclo[2.2.1]heptan-2-yl)-2-(2,6-dioxopiperidin-3-yl)-6-fluoroisoindoline-1,3-dione;
2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.2]octan-2-yl)isoindoline-1,3-dione;
2-(2,6-dioxopiperidin-3-yl)-5-(5-((1-(2-(4-(1-(4-hydroxyphenyl)-2-phenylbut-1-en-1-yl)phenoxy)ethyl)piperidin-4-yl)methyl)-2,5-diazabicyclo[2.2.2]octan-2-yl)isoindoline-1,3-dione;
5-(5-((1-(2-(4-(1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)isoindoline-1,3-dione;
2-(2,6-dioxopiperidin-3-yl)-5-(5-((1-(2-(4-(1,2-diphenylbut-1-en-1-yl)phenoxy)ethyl)piperidin-4-yl)methyl)-2,5-diazabicyclo[2.2.2]octan-2-yl)-6-fluoroisoindoline-1,3-dione;
2-(2,6-dioxopiperidin-3-yl)-5-fluoro-6-(5-((1-(2-(4-(1-(4-hydroxyphenyl)-2-phenylbut-1-en-1-yl)phenoxy)ethyl)piperidin-4-yl)methyl)-2,5-diazabicyclo[2.2.2]octan-2-yl)isoindoline-1,3-dione;
5-(5-((1-(2-(4-(1,2-bis(4-hydroxyphenyl)but-1-en-1-yl)phenoxy)ethyl)piperidin-4-yl)methyl)-2,5-diazabicyclo[2.2.2]octan-2-yl)-2-(2,6-dioxopiperidin-3-yl)-6-fluoroisoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)isoindoline-1,3-dione;
2-(2,6-dioxopiperidin-3-yl)-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione;
5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)isoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-6-fluoroisoindoline-1,3-dione;
2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione;
5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
2-(2,6-dioxopiperidin-3-yl)-6-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4-fluoroisoindoline-1,3-dione;
2-(2,6-dioxopiperidin-3-yl)-4-fluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione;
6-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4-fluorolsoindoline-1,3-dione;
2-(2,6-dioxopiperidin-3-yl)-4-fluoro-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione;
5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4,6-difluoroisoindoline-1,3-dione;
2-(2,6-dioxopiperidin-3-yl)-4,6-difluoro-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione;
5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4,7-difluoroisoindoline-1,3-dione;
2-(2,6-dioxopiperidin-3-yl)-4,7-difluoro-5-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione;
5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
2-(2,6-dioxopiperidin-3-yl)-6-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4,5-difluoroisoindoline-1,3-dione;
2-(2,6-dioxopiperidin-3-yl)-4,5-difluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione;

6-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)piperazin-1-yl-2,2,3,3,5,5,6,6-$d_8$)-4,6,7-trifluoroisoindoline-1,3-dione;
2-(2,6-dioxopiperidin-3-yl)-4,5,7-trifluoro-6-(4-((1-(2-(4-(1-(4-hydroxyphenyl)-2-phenylbut-1-en-1-yl)phenoxy)ethyl)piperidin-4-yl)methyl)piperazin-1-yl-2,2,3,3,5,5,6,6-$d_8$)isoindoline-1,3-dione;
5-(4-((1-(2-(4-(1,2-bis(4-hydroxyphenyl)but-1-en-1-yl)phenoxy)ethyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)-2,6-dimethylpiperazin-1-yl)isoindoline-1,3-dione;
2-(2,6-dioxopiperidin-3-yl)-5-(4-((1-(2-(4-(1-(4-hydroxyphenyl)-2-phenylbut-1-en-1-yl)phenoxy)ethyl)piperidin-4-yl)methyl)-2,6-dimethylpiperazin-1-yl)isoindoline-1,3-dione;
5-(4-((1-(2-(4-(1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)isoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)-2,6-dimethylpiperazin-1-yl)-6-fluoroisoindoline-1 ,3-dione;
2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)-2,6-dimethylpiperazin-1-yl)isoindoline-1,3-dione;
5-(4-((1-(2-(4-(1,2-bis(4-hydroxyphenyl)but-1-en-1-yl)phenoxy)ethyl)piperidin-4-yl)methyl)-2,6-dimethylpiperazin-1-yl)-2-(2,6-dioxopiperidin-3-yl)-6-fluoroisoindoline-1,3-dione;
2-(2,6-dioxopiperidin-3-yl)-5-(4-((1-(2-(4-(1,2-diphenylbut-1-en-1-yl)phenoxy)ethyl)piperidin-4-yl)methyl)-3,5-dimethylpiperazin-1-yl)isoindoline-1,3-dione;
2-(2,6-dioxopiperidin-3-yl)-5-(4-((1-(2-(4-(1-(4-hydroxyphenyl)-2-phenylbut-1-en-1-yl)phenoxy)ethyl)piperidin-4-yl)methyl)-3,5-dimethylpiperazin-1-yl)isoindoline-1,3-dione;
5-(4-((1-(2-(4-(1,2-bis(4-hydroxyphenyl)but-1-en-1-yl)phenoxy)ethyl)piperidin-4-yl)methyl)-3,5-dimethylpip erazin-1-yl)-2-(2, 6-dioxopip eridin-3 -yl)i soindoline-1, 3 -dione;
2-(2,6-dioxopiperidin-3-yl)-5-(4-((1-(2-(4-(1,2-diphenylbut-1-en-1-yl)phenoxy)ethyl)piperidin-4-yl)methyl)-3,5-dimethylpiperazin-1-yl)-6-fluoroisoindoline-1,3-dione;
2-(2,6-dioxopiperidin-3-yl)-5-fluoro-6-(4-((1-(2-(4-(1-(4-hydroxyphenyl)-2-phenylbut-1-en-1-yl)phenoxy)ethyl)piperidin-4-yl)methyl)-3,5-dimethylpiperazin-1-yl)isoindoline-1,3-dione;
5-(4-((1-(2-(4-(1,2-bis(4-hydroxyphenyl)but-1-en-1-yl)phenoxy)ethyl)piperidin-4-yl)methyl)-3,5-dimethylpiperazin-1-yl)-2-(2,6-dioxopiperidin-3-yl)-6-fluoroisoindoline-1,3-dione;
2-(2,6-dioxopiperidin-3-yl)-5-(1-((1-(2-(4-(1,2-diphenylbut-1-en-1-yl)phenoxy)ethyl)piperidin-4-yl)methyl)piperidin-4-yl)isoindoline-1,3-dione;
2-(2,6-dioxopiperidin-3-yl)-5-(1-((1-(2-(4-(1-(4-hydroxyphenyl)-2-phenylbut-1-en-1-yl)phenoxy)ethyl)piperidin-4-yl)methyl)piperidin-4-yl)isoindoline-1,3-dione;
5-(1-((1-(2-(4-(1,2-bis(4-hydroxyphenyl)but-1-en-1-yl)phenoxy)ethyl)piperidin-4-yl)methyl)piperidin-4-yl)-2-(2,6-dioxopiperidin-3-yl)isoindoline-1,3-dione;
2-(2,6-dioxopiperidin-3-yl)-5-(1-((1-(2-(4-(1,2-diphenylbut-1-en-1-yl)phenoxy)ethyl)piperidin-4-yl)methyl)piperidin-4-yl)-6-fluoroisoindoline-1,3-dione;
2-(2,6-dioxopiperidin-3-yl)-5-fluoro-6-(1-((1-(2-(4-(1-(4-hydroxyphenyl)-2-phenylbut-1-en-1-yl)phenoxy)ethyl)piperidin-4-yl)methyl)piperidin-4-yl)isoindoline-1,3-dione;
5-(1-((1-(2-(4-(1,2-bis(4-hydroxyphenyl)but-1-en-1-yl)phenoxy)ethyl)piperidin-4-yl)methyl)piperidin-4-yl)-2-(2,6-dioxopiperidin-3-yl)-6-fluoroisoindoline-1,3-dione;
2-(2,6-dioxopiperidin-3-yl)-5-((R)-3-(((1-(2-(4-(1,2-diphenylbut-1-en-1-yl)phenoxy)ethyl)piperidin-4-yl)methyl)amino)piperidin-1-yl)isoindoline-1,3-dione;
2-(2,6-dioxopiperidin-3-yl)-5-((R)-3-(((1-(2-(4-(1-(4-hydroxyphenyl)-2-phenylbut-1-en-1-yl)phenoxy)ethyl)piperidin-4-yl)methyl)amino)piperidin-1-yl)isoindoline-1,3-dione;
5-((R)-3-(((1-(2-(4-(1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione;
2-(2,6-dioxopiperidin-3-yl)-5-((S)-3-(((1-(2-(4-(1,2-diphenylbut-1-en-1-yl)phenoxy)ethyl)piperidin-4-yl)methyl)amino)piperidin-1-yl)isoindoline-1,3-dione;
2-(2,6-dioxopiperidin-3-yl)-5-((S)-3-(((1-(2-(4-(1-(4-hydroxyphenyl)-2-phenylbut-1-en-1-yl)phenoxy)ethyl)piperidin-4-yl)methyl)amino)piperidin-1-yl)isoindoline-1,3-dione;
5-((S)-3-(((1-(2-(4-(1,2-bis(4-hydroxyphenyl)but-1-en-1-yl)phenoxy)ethyl)piperidin-4-yl)methyl)amino)piperidin-1-yl)-2-(2,6-dioxopiperidin-3-yl)isoindoline-1,3-dione;
2-(2,6-dioxopiperidin-3-yl)-5-(((S)-1-((1-(2-(4-(1,2-diphenylbut-1-en-1-yl)phenoxy)ethyl)piperidin-4-yl)methyl)piperidin-3-yl)amino)isoindoline-1,3-dione;

2-(2,6-dioxopiperidin-3-yl)-5-(((S)-1-((1-(2-(4-(1-(4-hydroxyphenyl)-2-phenylbut-1-en-1-yl)phenoxy)ethyl)piperidin-4-yl)methyl)piperidin-3-yl)amino)isoindoline-1,3-dione;
5-(((S)-1-((1-(2-(4-(1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione;
2-(2,6-dioxopiperidin-3-yl)-5-(((R)-1-((1-(2-(4-(1,2-diphenylbut-1-en-1-yl)phenoxy)ethyl)piperidin-4-yl)methyl)piperidin-3-yl)amino)isoindoline-1,3-dione;
2-(2,6-dioxopiperidin-3-yl)-5-(((R)-1-((1-(2-(4-(1-(4-hydroxyphenyl)-2-phenylbut-1-en-1-yl)phenoxy)ethyl)piperidin-4-yl)methyl)piperidin-3-yl)amino)isoindoline-1,3-dione;
5-(((R)-1-((1-(2-(4-(1,2-bis(4-hydroxyphenyl)but-1-en-1-yl)phenoxy)ethyl)piperidin-4-yl)methyl)piperidin-3-yl)amino)-2-(2,6-dioxopiperidin-3-yl)isoindoline-1,3-dione;
2-(2,6-dioxopiperidin-3-yl)-5-(4-((1-(4-(1-(4-hydroxyphenyl)-2-phenylbut-1-en-1-yl)phenyl)piperidin-4-yl)methyl)piperazin-1-yl)isoindoline-1,3-dione;
5-(4-((1-(4-(4-chloro-1-(4-hydroxyphenyl)-2-phenylbut-1-en-1-yl)phenyl)piperidin-4-yl)methyl)piperazin-1-yl)-2-(2,6-dioxopiperidin-3-yl)-6-fluoroisoindoline-1,3-dione;
2-(2,6-dioxopiperidin-3-yl)-5-(3-((1-(4-(1-(4-hydroxyphenyl)-2-phenylbut-1-en-1-yl)phenyl)piperidin-4-yl)methyl)-3,6-diazabicyclo[3.1.1]heptan-6-yl)isoindoline-1,3-dione;
2-(2,6-dioxopiperidin-3-yl)-5-fluoro-6-(3-((1-(4-(1-(4-hydroxyphenyl)-2-phenylbut-1-en-1-yl)phenyl)piperidin-4-yl)methyl)-3,6-diazabicyclo[3.1.1]heptan-6-yl)isoindoline-1,3-dione;
2-(2,6-dioxopiperidin-3-yl)-5-(6-((1-(4-(1-(4-hydroxyphenyl)-2-phenylbut-1-en-1-yl)phenyl)piperidin-4-yl)methyl)-3,6-diazabicyclo[3.1.1]heptan-3-yl)isoindoline-1,3-dione;
2-(2,6-dioxopiperidin-3-yl)-5-fluoro-6-(6-((1-(4-(1-(4-hydroxyphenyl)-2-phenylbut-1-en-1-yl)phenyl)piperidin-4-yl)methyl)-3,6-diazabicyclo[3.1.1]heptan-3-yl)isoindoline-1,3-dione;
2-(2,6-dioxopiperidin-3-yl)-5-(3-((1-(4-(1-(4-hydroxyphenyl)-2-phenylbut-1-en-1-yl)phenyl)piperidin-4-yl)methyl)-3,8-diazabicyclo[3.2.1]octan-8-yl)isoindoline-1,3-dione;
2-(2,6-dioxopiperidin-3-yl)-5-fluoro-6-(3-((1-(4-(1-(4-hydroxyphenyl)-2-phenylbut-1-en-1-yl)phenyl)piperidin-4-yl)methyl)-3,8-diazabicyclo[3.2.1]octan-8-yl)isoindoline-1,3-dione;
2-(2,6-dioxopiperidin-3-yl)-5-(8-((1-(4-(1-(4-hydroxyphenyl)-2-phenylbut-1-en-1-yl)phenyl)piperidin-4-yl)methyl)-3,8-diazabicyclo[3.2.1]octan-3-yl)isoindoline-1,3-dione;
2-(2,6-dioxopiperidin-3-yl)-5-fluoro-6-(8-((1-(4-(1-(4-hydroxyphenyl)-2-phenylbut-1-en-1-yl)phenyl)piperidin-4-yl)methyl)-3,8-diazabicyclo[3.2.1]octan-3-yl)isoindoline-1,3-dione;
2-(2,6-dioxopiperidin-3-yl)-5-(5-((1-(4-(1-(4-hydroxyphenyl)-2-phenylbut-1-en-1-yl)phenyl)piperidin-4-yl)methyl)-2,5-diazabicyclo[2.2.1]heptan-2-yl)isoindoline-1,3-dione;
2-(2,6-dioxopiperidin-3-yl)-5-fluoro-6-(5-((1-(4-(1-(4-hydroxyphenyl)-2-phenylbut-1-en-1-yl)phenyl)piperidin-4-yl)methyl)-2,5-diazabicyclo[2.2.1]heptan-2-yl)isoindoline-1,3-dione;
2-(2,6-dioxopiperidin-3-yl)-5-(5-((1-(4-(1-(4-hydroxyphenyl)-2-phenylbut-1-en-1-yl)phenyl)piperidin-4-yl)methyl)-2,5-diazabicyclo[2.2.2]octan-2-yl)isoindoline-1,3-dione;
2-(2,6-dioxopiperidin-3-yl)-5-fluoro-6-(5-((1-(4-(1-(4-hydroxyphenyl)-2-phenylbut-1-en-1-yl)phenyl)piperidin-4-yl)methyl)-2,5-diazabicyclo[2.2.2]octan-2-yl)isoindoline-1,3-dione;
2-(2,6-dioxopiperidin-3-yl)-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4-fluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4-fluoro-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4,6-difluoro-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4,7-difluoro-5-(4-((1-(4-(1-(4-hydroxyphenyl)-2-phenylbut-1-en-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4,5-difluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4,5,7-trifluoro-6-(4-((1-(4-(1-(4-hydroxyphenyl)-2-phenylbut-1-en-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-5-(4-((1-(4-(1-(4-hydroxyphenyl)-2-phenylbut-1-en-1-yl)phenyl)piperidin-4-yl)methyl)-2,6-dimethylpiperazin-1-yl)iso indoline-1,3-dione;
2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)-2,6-dimethylpiperazin-1-yl)isoindoline-1,3-dione;

2-(2,6-dioxopiperidin-3-yl)-5-(4-((1-(4-(1-(4-hydroxyphenyl)-2-phenylbut-1-en-1-yl)phenyl)piperidin-4-yl)methyl)-3,5-dimethylpiperazin-1-yl)isoindoline-1,3-dione;
2-(2,6-dioxopiperidin-3-yl)-5-fluoro-6-(4-((1-(4-(1-(4-hydroxyphenyl)-2-phenylbut-1-en-1-yl)phenyl)piperidin-4-yl)methyl)-3,5-dimethylpiperazin-1-yl)isoindoline-1,3-dione;
2-(2,6-dioxopiperidin-3-yl)-5-(1-((1-(4-(1-(4-hydroxyphenyl)-2-phenylbut-1-en-1-yl)phenyl)piperidin-4-yl)methyl)piperidin-4-yl)isoindoline-1,3-dione;
2-(2,6-dioxopiperidin-3-yl)-5-fluoro-6-(1-((1-(4-(1-(4-hydroxyphenyl)-2-phenylbut-1-en-1-yl)phenyl)piperidin-4-yl)methyl)piperidin-4-yl)isoindoline-1,3-dione;
2-(2,6-dioxopiperidin-3-yl)-5-((R)-3-(((1-(4-(1-(4-hydroxyphenyl)-2-phenylbut-1-en-1-yl)phenyl)piperidin-4-yl)methyl)amino)piperidin-1-yl)isoindoline-1,3-dione;
2-(2,6-dioxopiperidin-3-yl)-5-((S)-3-(((1-(4-(1-(4-hydroxyphenyl)-2-phenylbut-1-en-1-yl)phenyl)piperidin-4-yl)methyl)amino)piperidin-1-yl)isoindoline-1,3-dione;
2-(2,6-dioxopiperidin-3-yl)-5-(((S)-1-((1-(4-(1-(4-hydroxyphenyl)-2-phenylbut-1-en-1-yl)phenyl)piperidin-4-yl)methyl)piperidin-3-yl)amino)isoindoline-1,3-dione;
2-(2,6-dioxopiperidin-3-yl)-5-(((R)-1-((1-(4-(1-(4-hydroxyphenyl)-2-phenylbut-1-en-1-yl)phenyl)piperidin-4-yl)methyl)piperidin-3-yl)amino)isoindoline-1,3-dione;
5-(4-((1-(4-(1,2-bis(4-hydroxyphenyl)but-1-en-1-yl)phenyl)piperidin-4-yl)methyl)piperazin-1-yl)-2-(2,6-dioxopiperidin-3-yl)isoindoline-1,3-dione;
5-(4-((1-(4-(1,2-bis(4-hydroxyphenyl)but-1-en-1-yl)phenyl)piperidin-4-yl)methyl)piperazin-1-yl)-2-(2,6-dioxopiperidin-3-yl)-6-fluoroisoindoline-1,3-dione;
5-(3-((1-(4-(1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)isoindoline-1,3-dione;
5-(3-((1-(4-(1,2-bis(4-hydroxyphenyl)but-1-en-1-yl)phenyl)piperidin-4-yl)methyl)-3,6-diazabicyclo[3.1.1]heptan-6-yl)-2-(2,6-dioxopiperidin-3-yl)-6-fluoroisoindoline-1,3-dione;
5-(6-((1-(4-(1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)isoindoline-1,3-dione;
5-(6-((1-(4-(1,2-bis(4-hydroxyphenyl)but-1-en-1-yl)phenyl)piperidin-4-yl)methyl)-3,6-diazabicyclo[3.1.1]heptan-3-yl)-2-(2,6-dioxopiperidin-3-yl)-6-fluoroisoindoline-1,3-dione;
5-(3-((1-(4-(1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)isoindoline-1,3-dione;
5-(3-((1-(4-(1,2-bis(4-hydroxyphenyl)but-1-en-1-yl)phenyl)piperidin-4-yl)methyl)-3,8-diazabicyclo[3.2.1]octan-8-yl)-2-(2,6-dioxopiperidin-3-yl)-6-fluoroisoindoline-1,3-dione;
5-(8-((1-(4-(1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)isoindoline-1,3-dione;
5-(8-((1-(4-(1,2-bis(4-hydroxyphenyl)but-1-en-1-yl)phenyl)piperidin-4-yl)methyl)-3,8-diazabicyclo[3.2.1]octan-3-yl)-2-(2,6-dioxopiperidin-3-yl)-6-fluoroisoindoline-1,3-dione;
5-(5-((1-(4-(1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)isoindoline-1,3-dione;
5-(5-((1-(4-(1,2-bis(4-hydroxyphenyl)but-1-en-1-yl)phenyl)piperidin-4-yl)methyl)-2,5-diazabicyclo[2.2.1]heptan-2-yl)-2-(2,6-dioxopiperidin-3-yl)-6-fluoroisoindoline-1,3-dione;
5-(5-((1-(4-(1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)isoindoline-1,3-dione;
5-(5-((1-(4-(1,2-bis(4-hydroxyphenyl)but-1-en-1-yl)phenyl)piperidin-4-yl)methyl)-2,5-diazabicyclo[2.2.2]octan-2-yl)-2-(2,6-dioxopiperidin-3-yl)-6-fluoroisoindoline-1,3-dione;
5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)isoindoline-1,3-dione;
5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-6-fluoroisoindoline-1,3-dione;
6-(4-((1-(4-(1,2-bis(4-hydroxyphenyl)but-1 -en-1 -yl)phenyl)piperidin-4-yl)methyl)piperazin-1 -yl-2,2,3,3,5,5,6,6-$d_8$)-2-(2,6-dioxopiperidin-3-yl)-4-fluoroisoindoline-1,3-dione;
5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4-fluoroisoindoline-1,3-dione;
5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6-difluoroisoindoline-1,3-dione;
5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,7-difluoroisoindoline-1,3-dione;
6-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,5-difluoroisoindoline-1,3-dione;

5-(4-((1-(4-(1,2-bis(4-hydroxyphenyl)but-1-en-1-yl)phenyl)piperidin-4-yl)methyl)piperazin-1-yl-2,2,3,3,5,5,6,6-$d_8$)-2-(2,6-dioxopiperidin-3-yl)-4,6,7-trifluoroisoindoline-1,3-dione;
5-(4-((1-(4-(1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)isoindoline-1,3-dione;
5-(4-((1-(4-(1,2-bis(4-hydroxyphenyl)but-1-en-1-yl)phenyl)piperidin-4-yl)methyl)-2,6-dimethylpiperazin-1-yl)-2-(2,6-dioxopiperidin-3-yl)-6-fluoroisoindoline-1,3-dione;
5-(4-((1-(4-(1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)isoindoline-1,3-dione;
5-(4-((1-(4-(1,2-bis(4-hydroxyphenyl)but-1-en-1-yl)phenyl)piperidin-4-yl)methyl)-3,5-dimethylpiperazin-1-yl)-2-(2,6-dioxopiperidin-3-yl)-6-fluoroisoindoline-1,3-dione;
5-(1-((1-(4-(1,2-bis(4-hydroxyphenyl)but-1-en-1-yl)phenyl)piperidin-4-yl)methyl)piperidin-4-yl)-2-(2,6-dioxopiperidin-3-yl)isoindoline-1,3-dione;
5-(1-((1-(4-(1,2-bis(4-hydroxyphenyl)but-1-en-1-yl)phenyl)piperidin-4-yl)methyl)piperidin-4-yl)-2-(2,6-dioxopiperidin-3-yl)-6-fluoroisoindoline-1,3-dione;
5-((R)-3-(((1-(4-(1,2-bis(4-hydroxyphenyl)but-1-en-1-yl)phenyl)piperidin-4-yl)methyl)amino)piperidin-1-yl)-2-(2,6-dioxopiperidin-3-yl)isoindoline-1,3-dione;
5-((S)-3-(((1-(4-(1,2-bis(4-hydroxyphenyl)but-1-en-1-yl)phenyl)piperidin-4-yl)methyl)amino)piperidin-1-yl)-2-(2,6-dioxopiperidin-3-yl)isoindoline-1,3-dione;
5-(((S)-1-((1-(4-(1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione;
5-(((R)-1-((1-(4-(1,2-bis(4-hydroxyphenyl)but-1-en-1-yl)phenyl)piperidin-4-yl)methyl)piperidin-3-yl)amino)-2-(2,6-dioxopiperidin-3-yl)isoindoline-1,3-dione;
2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl)isoindoline-1,3-dione;
2-(2,6-dioxopiperidin-3-yl)-5-(3-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-6-yl)isoindoline-1,3 -dione;
2-(2,6-dioxopiperidin-3-yl)-5-fluoro-6-(3-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-6-yl)isoindoline-1,3 -dione;
2-(2,6-dioxopiperidin-3-yl)-5-(6-((1-(4-((1R,2S)-6-hydroxy-2-phenyl-1,2,3,4-tetrahydronaphthalen-1 -yl)phenyl)piperidin-4-yl)methyl)-3,6-diazabicyclo[3.1.1]heptan-3-yl)isoindoline-1,3 -dione;
2-(2,6-dioxopiperidin-3-yl)-5-fluoro-6-(6-((1-(4-((1R,2S)-6-hydroxy-2-phenyl-1,2,3,4-tetrahydronaphthalen-1-yl)phenyl)piperidin-4-yl)methyl)-3,6-diazabicyclo[3.1.1]heptan-3-yl)isoindoline-1,3-dione;
2-(2,6-dioxopiperidin-3-yl)-5-(3-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-8-yl)isoindoline-1,3-dione;
2-(2,6-dioxopiperidin-3-yl)-5-fluoro-6-(3-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-8-yl)isoindoline-1,3-dione;
2-(2,6-dioxopiperidin-3-yl)-5-(8-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-3-yl)isoindoline-1,3-dione;
2-(2,6-dioxopiperidin-3-yl)-5-fluoro-6-(8-((1-(4-((1R,2S)-6-hydroxy-2-phenyl-1,2,3,4-tetrahydronaphthalen-1-yl)phenyl)piperidin-4-yl)methyl)-3,8-diazabicyclo[3.2.1]octan-3-yl)isoindoline-1,3-dione;
2-(2,6-dioxopiperidin-3-yl)-5-fluoro-6-(5-((1-(4-((1R,2S)-6-hydroxy-2-phenyl-1,2,3,4-tetrahydronaphthalen-1-yl)phenyl)piperidin-4-yl)methyl)-2,5-diazabicyclo[2.2.1]heptan-2-yl)isoindoline-1,3-dione;
2-(2,6-dioxopiperidin-3-yl)-5-(5-((1-(4-((1R,2S)-6-hydroxy-2-phenyl-1,2,3,4-tetrahydronaphthalen-1-yl)phenyl)piperidin-4-yl)methyl)-2,5-diazabicyclo[2.2.2]octan-2-yl)isoindoline-1,3-dione;
2-(2,6-dioxopiperidin-3-yl)-5-fluoro-6-(5-((1-(4-((1R,2S)-6-hydroxy-2-phenyl-1,2,3,4-tetrahydronaphthalen-1-yl)phenyl)piperidin-4-yl)methyl)-2,5-diazabicyclo[2.2.2]octan-2-yl)isoindoline-1,3-dione;
2-(2, 6-dioxopiperidin-3-yl)-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4-fluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4-fluoro-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4,6-difluoro-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4,7-difluoro-5-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione;

2-(2,6-dioxopiperidin-3-yl)-4,5-difluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-4,5,7-trifluoro-6-(4-((1-(4-((1R,2S)-6-hydroxy-2-phenyl-1,2,3,4-tetrahydronaphthalen-1-yl)phenyl)piperidin-4-yl)methyl)piperazin-1-yl-2,2,3,3,5,5,6,6-$d_8$)isoindoline-1,3-dione;
2-(2,6-dioxopiperidin-3-yl)-5-(4-((1-(4-((1R,2S)-6-hydroxy-2-phenyl-1,2,3,4-tetrahydronaphthalen-1-yl)phenyl)piperidin-4-yl)methyl)-2,6-dimethylpiperazin-1 -yl)isoindoline-1,3-dione;
2-(2,6-dioxopiperidin-3-yl)-5-fluoro-6-(4-((1-(4-((1R,2S)-6-hydroxy-2-phenyl-1,2,3,4-tetrahydronaphthalen-1-yl)phenyl)piperidin-4-yl)methyl)-2,6-dimethylpiperazin-1 -yl)isoindoline-1,3-dione;
2-(2,6-dioxopiperidin-3-yl)-5-(4-((1-(4-((1R,2S)-6-hydroxy-2-phenyl-1,2,3,4-tetrahydronaphthalen-1-yl)phenyl)piperidin-4-yl)methyl)-3,5-dimethylpiperazin-1-yl)isoindoline-1,3-dione;
2-(2,6-dioxopiperidin-3-yl)-5-fluoro-6-(4-((1-(4-((1R,2S)-6-hydroxy-2-phenyl-1,2,3,4-tetrahydronaphthalen-1 -yl)phenyl)piperidin-4-yl)methyl)-3,5-dimethylpiperazin-1 -yl)isoindoline-1,3-dione;
2-(2,6-dioxopiperidin-3-yl)-5-(1-((1-(4-((1R,2S)-6-hydroxy-2-phenyl-1,2,3,4-tetrahydronaphthalen-1-yl)phenyl)piperidin-4-yl)methyl)piperidin-4-yl)isoindoline-1,3-dione;
2-(2,6-dioxopiperidin-3-yl)-5-fluoro-6-(1-((1-(4-((1R,2S)-6-hydroxy-2-phenyl-1,2,3,4-tetrahydronaphthalen-1-yl)phenyl)piperidin-4-yl)methyl)piperidin-4-yl)isoindoline-1,3-dione;
2-(2,6-dioxopiperidin-3-yl)-5-((R)-3-(((1-(4-((1R,2S)-6-hydroxy-2-phenyl-1,2,3,4-tetrahydronaphthalen-1-yl)phenyl)piperidin-4-yl)methyl)amino)piperidin-1-yl)isoindoline-1,3-dione;
2-(2,6-dioxopiperidin-3-yl)-5-((S)-3-(((1-(4-((1R,2S)-6-hydroxy-2-phenyl-1,2,3,4-tetrahydronaphthalen-1-yl)phenyl)piperidin-4-yl)methyl)amino)piperidin-1-yl)isoindoline-1,3-dione;
2-(2,6-dioxopiperidin-3-yl)-5-(((S)-1-((1-(4-((1R,2S)-6-hydroxy-2-phenyl-1,2,3,4-tetrahydronaphthalen-1-yl)phenyl)piperidin-4-yl)methyl)piperidin-3 -yl)amino)isoindoline-1,3 -dione;
2-(2,6-dioxopiperidin-3-yl)-5-(((R)-1-((1-(4-((1R,2S)-6-hydroxy-2-phenyl-1,2,3,4-tetrahydronaphthalen-1-yl)phenyl)piperidin-4-yl)methyl)piperidin-3 -yl)amino)isoindoline-1,3 -dione;
2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl)isoindoline-1,3 -dione;
2-(2,6-dioxopiperidin-3-yl)-5-(3-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-6-yl)isoindoline-1,3-dione;
2-(2,6-dioxopiperidin-3-yl)-5-fluoro-6-(3-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-6-yl)isoindoline-1,3-dione;
2-(2,6-dioxopiperidin-3-yl)-5-(6-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-3-yl)isoindoline-1,3-dione;
2-(2,6-dioxopiperidin-3-yl)-5-fluoro-6-(6-((1-(2-(4-((1R,2S)-6-hydroxy-2-phenyl-1,2,3,4-tetrahydronaphthalen-1-yl)phenoxy)ethyl)piperidin-4-yl)methyl)-3,6-diazabicyclo[3.1.1]heptan-3-yl)isoindoline-1,3-dione;
2-(2,6-dioxopiperidin-3-yl)-5-(3-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-8-yl)isoindoline-1,3-dione;
2-(2,6-dioxopiperidin-3-yl)-5-fluoro-6-(3-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-8-yl)isoindoline-1,3-dione;
2-(2,6-dioxopiperidin-3-yl)-5-(8-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-3-yl)isoindoline-1,3-dione;
2-(2,6-dioxopiperidin-3-yl)-5-fluoro-6-(8-((1-(2-(4-((1R,2S)-6-hydroxy-2-phenyl-1,2,3,4-tetrahydronaphthalen-1-yl)phenoxy)ethyl)piperidin-4-yl)methyl)-3,8-diazabicyclo[3.2.1]octan-3-yl)isoindoline-1,3-dione;
2-(2,6-dioxopiperidin-3-yl)-5-fluoro-6-(5-((1-(2-(4-((1R,2S)-6-hydroxy-2-phenyl-1,2,3,4-tetrahydronaphthalen-1-yl)phenoxy)ethyl)piperidin-4-yl)methyl)-2,5-diazabicyclo[2.2.1]heptan-2-yl)isoindoline-1,3-dione;
2-(2,6-dioxopiperidin-3-yl)-5-fluoro-6-(5-((1-(2-(4-((1R,2S)-6-hydroxy-2-phenyl-1,2,3,4-tetrahydronaphthalen-1-yl)phenoxy)ethyl)piperidin-4-yl)methyl)-2,5-diazabicyclo[2.2.2]octan-2-yl)isoindoline-1,3-dione;
2-(2,6-dioxopiperidin-3-yl)-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione;
2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione;
2-(2,6-dioxopiperidin-3-yl)-4-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione;
2-(2,6-dioxopiperidin-3-yl)-4-fluoro-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione;
2-(2,6-dioxopiperidin-3-yl)-4,6-difluoro-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione;
2-(2,6-dioxopiperidin-3-yl)-4,7-difluoro-5-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione;

2-(2,6-dioxopiperidin-3-yl)-4,5-difluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione;
2-(2,6-dioxopiperidin-3-yl)-4,5,7-trifluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)piperazin-1-yl-2,2,3,3,5,5,6,6-$d_8$)isoindoline-1,3-dione;
2-(2,6-dioxopiperidin-3-yl)-5-(4-((1-(2-(4-((1R,2S)-6-hydroxy-2-phenyl-1,2,3,4-tetrahydronaphthalen-1-yl)phenoxy)ethyl)piperidin-4-yl)methyl)-2,6-dimethylpiperazin-1-yl)isoindoline-1,3 -dione;
2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)-2,6-dimethylpiperazin-1-yl)isoindoline-1,3-dione;
2-(2,6-dioxopiperidin-3-yl)-5-(4-((1-(2-(4-((1R,2S)-6-hydroxy-2-phenyl-1,2,3,4-tetrahydronaphthalen-1-yl)phenoxy)ethyl)piperidin-4-yl)methyl)-3,5-dimethylpiperazin-1-yl)isoindoline-1,3-dione;
2-(2,6-dioxopiperidin-3-yl)-5-fluoro-6-(4-((1-(2-(4-((1R,2S)-6-hydroxy-2-phenyl-1,2,3,4-tetrahydronaphthalen-1-yl)phenoxy)ethyl)piperidin-4-yl)methyl)-3,5-dimethylpiperazin-1-yl)isoindoline-1,3-dione;
2-(2,6-dioxopiperidin-3-yl)-5-(1-((1-(2-(4-((1R,2S)-6-hydroxy-2-phenyl-1,2,3,4-tetrahydronaphthalen-1-yl)phenoxy)ethyl)piperidin-4-yl)methyl)piperidin-4-yl)isoindoline-1,3-dione;
2-(2,6-dioxopiperidin-3-yl)-5-fluoro-6-(1-((1-(2-(4-((1R,2S)-6-hydroxy-2-phenyl-1,2,3,4-tetrahydronaphthalen-1-yl)phenoxy)ethyl)piperidin-4-yl)methyl)piperidin-4-yl)isoindoline-1,3-dione;
2-(2,6-dioxopiperidin-3-yl)-5-((R)-3-(((1-(2-(4-((1R,2S)-6-hydroxy-2-phenyl-1,2,3,4-tetrahydronaphthalen-1-yl)phenoxy)ethyl)piperidin-4-yl)methyl)amino)piperidin-1-yl)isoindoline-1,3-dione;
2-(2,6-dioxopiperidin-3-yl)-5-((S)-3-(((1-(2-(4-((1R,2S)-6-hydroxy-2-phenyl-1,2,3,4-tetrahydronaphthalen-1-yl)phenoxy)ethyl)piperidin-4-yl)methyl)amino)piperidin-1-yl)isoindoline-1,3-dione;
2-(2,6-dioxopiperidin-3-yl)-5-(((S)-1-((1-(2-(4-((1R,2S)-6-hydroxy-2-phenyl-1,2,3,4-tetrahydronaphthalen-1-yl)phenoxy)ethyl)piperidin-4-yl)methyl)piperidin-3-yl)amino)isoindoline-1,3-dione;
2-(2,6-dioxopiperidin-3-yl)-5-(((R)-1-((1-(2-(4-((1R,2S)-6-hydroxy-2-phenyl-1,2,3,4-tetrahydronaphthalen-1-yl)phenoxy)ethyl)piperidin-4-yl)methyl)piperidin-3-yl)amino)isoindoline-1,3-dione;
N-((1r,4r)-4-(3-chloro-4-cyanophenoxy)cyclohexyl)-6-(4-((8-(2-(2,6-dioxopiperidin-3-yl)-1-oxoisoindolin-5-yl)-3,8-diazabicyclo[3.2.1]octan-3-yl)methyl)piperidin-1-yl)pyridazine-3-carboxamide;
N-((1r,4r)-4-(3-chloro-4-cyanophenoxy)cyclohexyl)-6-(4-((3-(2-(2,6-dioxopiperidin-3-yl)-1-oxoisoindolin-5-yl)-3,8-diazabicyclo[3.2.1]octan-8-yl)methyl)piperidin-1-yl)pyridazine-3-carboxamide;
*N*-((1r,4r)-4-(3-chloro-4-cyanophenoxy)cyclohexyl)-6-(4-((4-(2-(2,6-dioxopiperidin-3-yl)-1-oxoisoindolin-5-yl)piperidin-1-yl)methyl)piperidin-1-yl)pyridazine-3-carboxamide;
*N*-((1r,4r)-4-(3-chloro-4-cyanophenoxy)cyclohexyl)-6-(4-(((3S,4S)-4-(2-(2,6-dioxopiperidin-3-yl)-1-oxoisoindolin-5-yl)-3,4-dihydroxypiperidin-1-yl)methyl)piperidin-1-yl)pyridazine-3-carboxamide;
*N*-((1r,4r)-4-(3-chloro-4-cyanophenoxy)cyclohexyl)-6-(4-((4-(2-(2,6-dioxopiperidin-3-yl)-1-oxoisoindolin-5-yl)-3,6-dihydropyridin-1(2H)-yl)methyl)piperidin-1-yl)pyridazine-3-carboxamide;
*N*-((1r,4r)-4-(3-chloro-4-cyanophenoxy)cyclohexyl)-6-(4-((4-(2-(2,6-dioxopiperidin-3-yl)-4-fluoro-1-oxoisoindolin-5-yl)piperidin-1-yl)methyl)piperidin-1-yl)pyridazine-3-carboxamide;
*N*-((1r,4r)-4-(3-chloro-4-cyanophenoxy)cyclohexyl)-6-(4-((4-(2-(2,6-dioxopiperidin-3-yl)-4-fluoro-1-oxoisoindolin-5-yl)-3,6-dihydropyridin-1(2H)-yl)methyl)piperidin-1-yl)pyridazine-3-carboxamide;
*N*-((1r,4r)-4-(3-chloro-4-cyanophenoxy)cyclohexyl)-6-(4-((4-(2-(2,6-dioxopiperidin-3-yl)-7-fluoro-1-oxoisoindolin-5-yl)piperidin-1-yl)methyl)piperidin-1-yl)pyridazine-3-carboxamide;
*N*-((1r,3r)-3-(3-chloro-4-cyanophenoxy)-2,2,4,4-tetramethylcyclobutyl)-6-(4-((4-(2-(2,6-dioxopiperidin-3-yl)-1-oxoisoindolin-5-yl)piperidin-1-yl)methyl)piperidin-1-yl)nicotinamide;
*N*-((1r,3r)-3-(3-chloro-4-cyanophenoxy)-2,2,4,4-tetramethylcyclobutyl)-6-(4-((4-(2-(2,6-dioxopiperidin-3-yl)-1-oxoisoindolin-5-yl)-3,6-dihydropyridin-1(2H)-yl)methyl)piperidin-1-yl)nicotinamide;
7-cyclopentyl-2-((5-(4-((4-((2-(2,6-dioxopiperidin-3-yl)-1-oxoisoindolin-5-yl)amino)piperidin-1-yl)methyl)piperidin-1-yl)pyridin-2-yl)amino)-N,N-dimethyl-7H-pyrrolo[2,3-d]pyrimidine-6-carboxamide;
N-(2-chloro-6-methylphenyl)-2-((6-(4-((4-(2-(2,6-dioxopiperidin-3-yl)-1-oxoisoindolin-5-yl)piperidin-1-yl)methyl)piperidin-1-yl)-2-methylpyrimidin-4-yl)amino)thiazole-5-carboxamide; and
N-(2-chloro-6-methylphenyl)-2-((6-(4-(((3S,4S)-4-(2-(2,6-dioxopiperidin-3-yl)-1-oxoisoindolin-5-yl)-3,4-dihydroxypiperidin-1-yl)methyl)piperidin-1-yl)-2-methylpyrimidin-4-yl)amino)thiazole-5-carboxamide.

**38.** The compound of Formula (III) or salts, enantiomers, stereoisomers, solvates, isotopically enriched analogs, prodrugs, or polymorphs thereof as claimed in any one of claims 20-37, which is hydrohalides (including hydrochlorides and hydrobromates), sulfates, maleates, sulfonates, citrates, lactates, lactobionates, L-tartrates, fumarates, L-malates, L-lactates, α-Ketoglutarates, hippurates, D-glucuronates, D-gluconates, α-D-glucoheptonates, glycolates, mucates, L-ascorbates, orotates, picrates, glycinates, alaninates, argininates, cinnamates, laurates, pamoates, sebacates, benzenesulfonates, methanesulfonates, ethanesulfonates, edisylates, formates, acetates, 2,2-dichloroacetates, trimethylacetates, propionates, valerates, palmitates, triphenylacetates, 2-ethylsuccinates, iodates, niacinates, L-pyroglutamates, L-prolinates, ferulates, 2-hydroxyethanesulfonates, nitrates, gentisates, cholates, salicylates, terephthalates, glutarates, adipates, stearates, oleates, undecenoates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, trifluoroacetates, pyruvates, para-chlorobenzenesulfonates, 1,5-naphthalenedisulfonates, 3-hydroxy-2-naphthoates, 1-hydroxy-2-naphthoates, 2-naphthalenesulfonates, hydroxyacetates, or p-toluenesulfonates of the compound of Formula (III).

**39.** A pharmaceutical composition comprising as active ingredient the compound of Formula (I) or pharmaceutically acceptable salts, enantiomers, stereoisomers, solvates, isotopically enriched analogs, prodrugs, or polymorphs thereof as claimed in any one of claims 1-8, or the compound of Formula (II) or pharmaceutically acceptable salts, enantiomers, stereoisomers, solvates, isotopically enriched analogs, prodrugs, or polymorphs thereof as claimed in any one of claims 9-19, and at least one pharmaceutically acceptable carrier.

**40.** The pharmaceutical composition as claimed in claim 39, further comprising at least one additional therapeutic agent.

**41.** A pharmaceutical composition comprising as active ingredient the compound of Formula (III) or pharmaceutically acceptable salts, enantiomers, stereoisomers, solvates, isotopically enriched analogs, prodrugs, or polymorphs thereof as claimed in any one of claims 20-37, and at least one pharmaceutically acceptable carrier.

**42.** The pharmaceutical composition as claimed in claim 41, further comprising at least one additional therapeutic agent, e.g., an anticancer agent.

**43.** A medicine kit or reagent kit comprising:

the compound of Formula (I) or a pharmaceutically acceptable salt thereof as claimed in any one of claims 1-8, or the pharmaceutical composition as claimed in claims 39 or 40; or
the compound of Formula (II) or a pharmaceutically acceptable salt thereof as claimed in any one of claims 9-19, or the pharmaceutical composition as claimed in claims 39 or 40; or
the compound of Formula (II) or a pharmaceutically acceptable salt thereof as claimed in any one of claims 20-37, or the pharmaceutical composition as claimed in claims 41 or 42.

**44.** Use of the compound of Formula (I) or salts, enantiomers, stereoisomers, solvates, isotopically enriched analogs, prodrugs, or polymorphs thereof as claimed in any one of claims 1-8, or the compound of Formula (II) or salts, enantiomers, stereoisomers, solvates, isotopically enriched analogs, prodrugs, or polymorphs thereof as claimed in any one of claims 9-19 for the preparation of the compound of Formula (III) as claimed in claim 20.

**45.** The use as claimed in claim 44, wherein the compound of Formula (III) can be used for the treatment and/or prevention of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

**46.** Use of the compound of Formula (I) or salts, enantiomers, stereoisomers, solvates, isotopically enriched analogs, prodrugs, or polymorphs thereof as claimed in any one of claims 1-8, or the compound of Formula (II) or salts, enantiomers, stereoisomers, solvates, isotopically enriched analogs, prodrugs, or polymorphs thereof as claimed in any one of claims 9-19 for the manufacture of a medicament for the prevention or treatment of cereblon protein-mediated disease or disorder.

**47.** The use as claimed in claim 46, wherein the cereblon protein-mediated disease or disorder comprises: tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, and diabetes.

**48.** The use as claimed in claim 46, wherein the cereblon protein-mediated disease or disorder is selected from the

group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; congestive heart failure; myocardial infarction; multiple organ dysfunction caused by cachexia and septic shock; and acute liver failure.

**49.** Use of the compound of Formula (III) or salts, enantiomers, stereoisomers, solvates, isotopically enriched analogs, prodrugs, or polymorphs thereof as claimed in any one of claims 20-37 for the manufacture of a medicament for the prevention and/or treatment of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

**50.** The use as claimed in claim 49, wherein the disease or disorder is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

**51.** The compound of Formula (I) or salts, enantiomers, stereoisomers, solvates, isotopically enriched analogs, prodrugs, or polymorphs thereof as claimed in any one of claims 1-8, or the compound of Formula (II) or salts, enantiomers, stereoisomers, solvates, isotopically enriched analogs, prodrugs, or polymorphs thereof as claimed in any one of claims 9-19, for use in the prevention and/or treatment of a cereblon protein-mediated disease or disorder.

**52.** The compound of Formula (I) or (II) or salts, enantiomers, stereoisomers, solvates, isotopically enriched analogs, prodrugs, or polymorphs thereof as claimed in claim 51, wherein the cereblon protein-mediated disease or disorder comprises: tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, and diabetes.

**53.** The compound of Formula (I) or (II) or salts, enantiomers, stereoisomers, solvates, isotopically enriched analogs, prodrugs, or polymorphs thereof as claimed in claim 52, wherein the cereblon protein-mediated disease or disorder is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; congestive heart failure; myocardial infarction; multiple organ dysfunction caused by cachexia and septic shock; and acute liver failure.

**54.** The compound of Formula (III) or salts, enantiomers, stereoisomers, solvates, isotopically enriched analogs, prodrugs, or polymorphs thereof as claimed in any one of claims 20-37, for use in the prevention and/or treatment of a disease or disorder selected from the group consisting of tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

**55.** The compound of Formula (III) or salts, enantiomers, stereoisomers, solvates, isotopically enriched analogs, prodrugs, or polymorphs thereof as claimed in claim 54, wherein the disease or disorder mediated by or associated with the target protein is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

**56.** A method for treating or preventing a cereblon protein-mediated disease or disorder in a subject, comprising administering to the subject a therapeutically effective amount of the compound of Formula (I) or salts, enantiomers, stereoisomers, solvates, isotopically enriched analogs, prodrugs, or polymorphs thereof as claimed in any one of claims 1-8, or the compound of Formula (II) or salts, enantiomers, stereoisomers, solvates, isotopically enriched analogs, prodrugs, or polymorphs thereof as claimed in any one of claims 9-19, or the pharmaceutical composition as claimed in claim 39 or 40.

**57.** The method as claimed in claim 56, wherein the cereblon protein-mediated disease or disorder comprises: tumor,

infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, and diabetes.

**58.** The method as claimed in claim 57, wherein the cereblon protein-mediated disease or disorder is selected from the group consisting of:
myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; congestive heart failure; myocardial infarction; multiple organ dysfunction caused by cachexia and septic shock; and acute liver failure.

**59.** The method as claimed in any one of claims 56-58, wherein the compound of Formula (I) or a pharmaceutically acceptable salt thereof, or the compound of Formula (II) or a pharmaceutically acceptable salt thereof, or the pharmaceutical composition is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

**60.** A method for treating or preventing a disease or disorder in a subject, comprising administering to the subject a therapeutically effective amount of the compound of Formula (III) or salts, enantiomers, stereoisomers, solvates, isotopically enriched analogs, prodrugs, or polymorphs thereof as claimed in any one of claims 20-37, or the pharmaceutical composition as claimed in claim 41 or 42, wherein the disease or disorder comprises tumor, infectious disease, autoinflammatory disease, inflammatory disease, autoimmune disease, neurological disease, respiratory disease, anemia, hemorrhagic shock, transplant rejection, multiple organ dysfunction syndrome (MODS), sarcoidosis, adult respiratory distress syndrome, congestive heart failure, myocardial infarction, Unverricht's syndrome, Richter syndrome (RS), acute liver failure, diabetes, Kennedy disease, seborrheic alopecia, hirsutism, skin disease, cardiovascular disease, dysfunctional uterine bleeding, anemia, pediatric aplastic anemia, endometriosis, transplant rejection, polycystic ovary syndrome, and thyroid disease.

**61.** The method as claimed in claim 60, wherein the disease or disorder is selected from the group consisting of:
myeloma, including multiple myeloma, plasma cell myeloma, smoldering myeloma, smoldering multiple myeloma;

myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; brain glioma; astroblastoma; ovarian cancer; bronchial cancer; prostate cancer; breast cancer, including triple negative breast cancer, sporadic breast cancer and patients with Cowden syndrome; pancreatic cancer; neuroblastoma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; autoinflammatory diseases, including gout, etc.; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; multiple organ dysfunction caused by cachexia and septic shock; acute liver failure; dysfunctional uterine bleeding; anemia; pediatric aplastic anemia; endometriosis; polycystic ovary syndrome; thyroid disease; cardiovascular diseases (e.g., coronary heart disease, congestive heart failure, myocardial infarction, atherosclerosis); skin diseases (e.g., acne); Kennedy disease; seborrheic alopecia; and hirsutism.

**62.** The method as claimed in claim 60 or 61, wherein the compound of Formula (III) or a pharmaceutically acceptable salt thereof, or the pharmaceutical composition is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

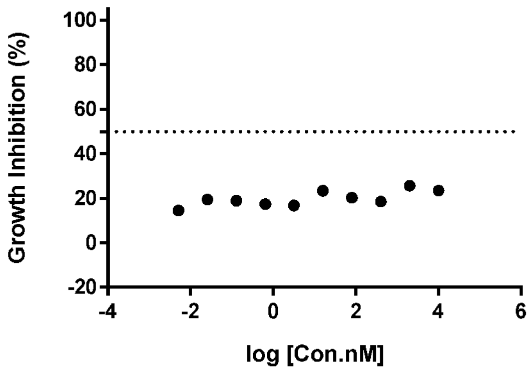


GT-02765

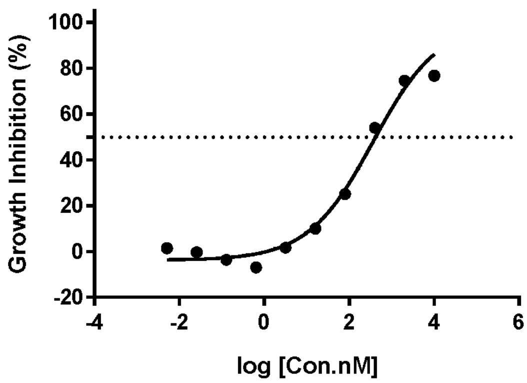


GT-03016

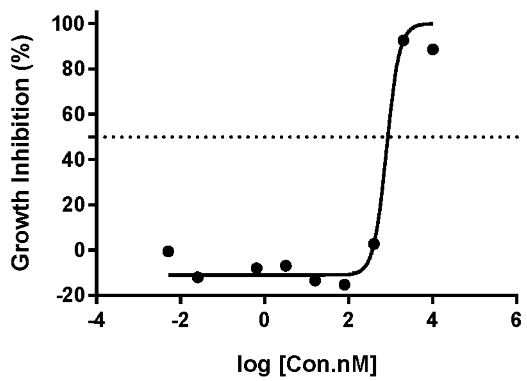


Figure 1

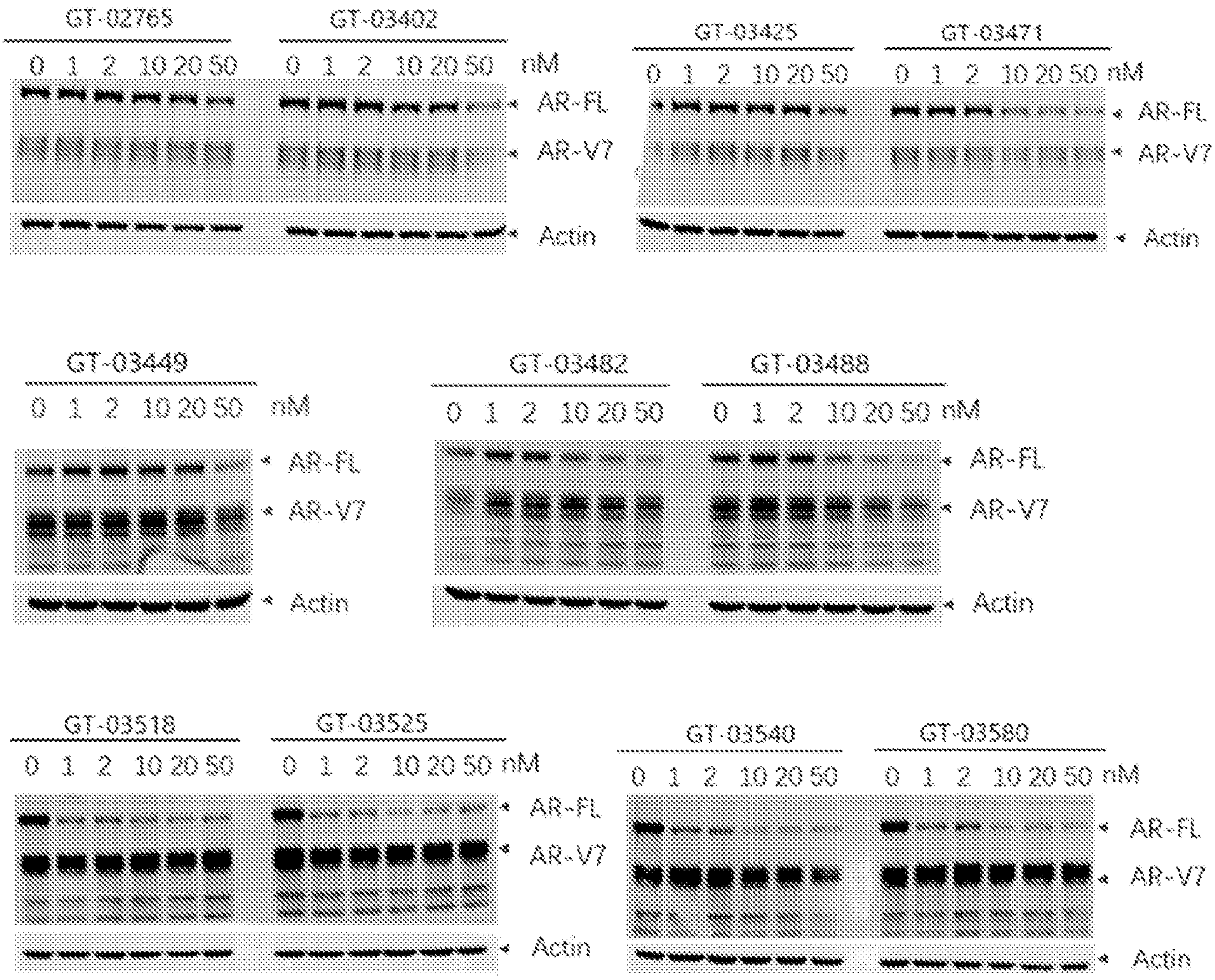
GT-02765
GT-03402
GT-03425
GT-03471
0 1 2 10 20 50 nM
AR-FL
AR-V7
Actin
GT-03449
GT-03482
GT-03488
GT-03518
GT-03525
GT-03540
GT-03580

Figure 2

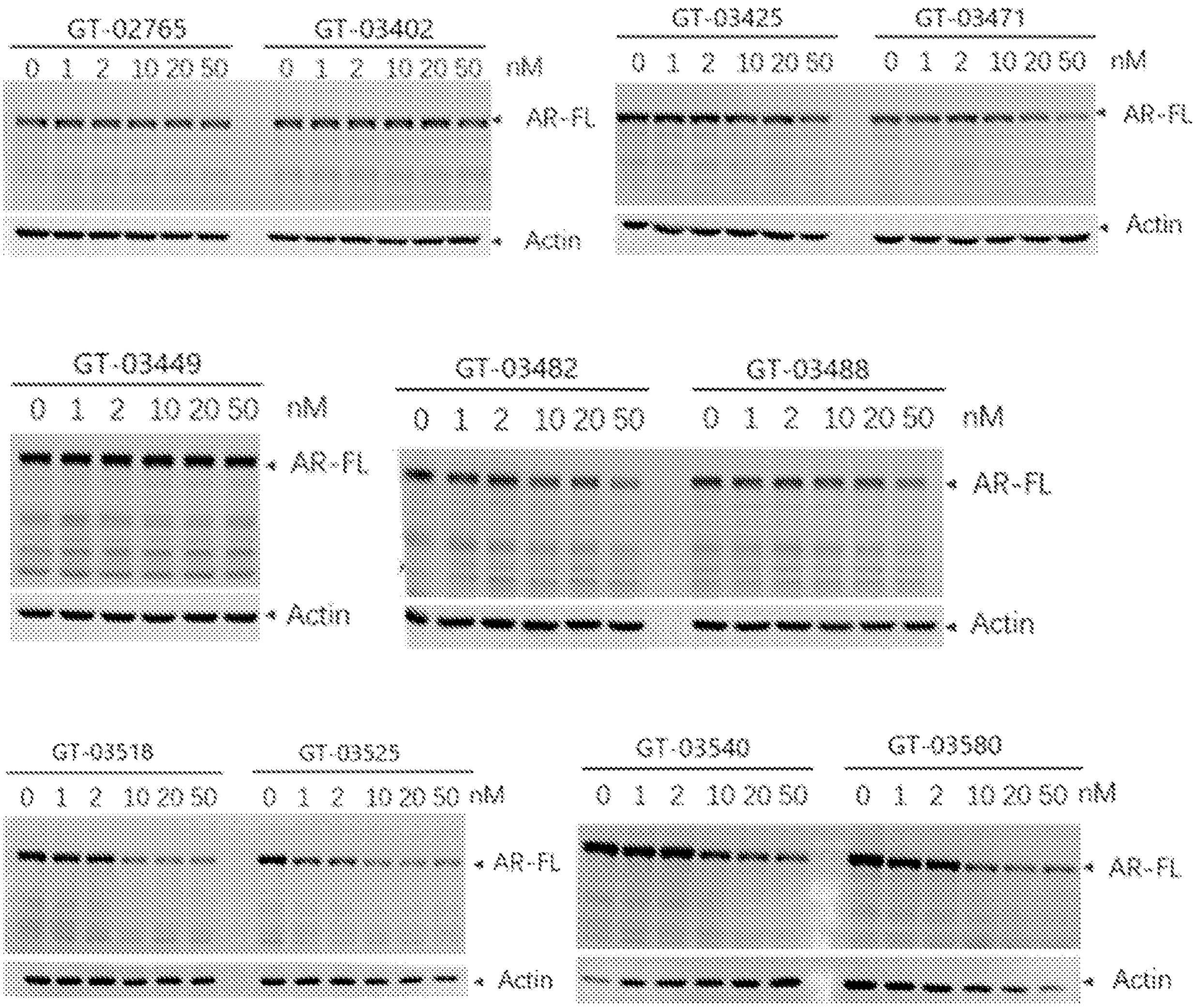
GT-02765
GT-03402
0 1 2 10 20 50
0 1 2 10 20 50 nM
AR-FL
Actin
GT-03425
GT-03471
0 1 2 10 20 50
0 1 2 10 20 50 nM
AR-FL
Actin
GT-03449
0 1 2 10 20 50 nM
AR-FL
Actin
GT-03482
GT-03488
0 1 2 10 20 50
0 1 2 10 20 50 nM
AR-FL
Actin
GT-03518
GT-03525
0 1 2 10 20 50
0 1 2 10 20 50 nM
AR-FL
Actin
GT-03540
GT-03580
0 1 2 10 20 50
0 1 2 10 20 50 nM
AR-FL
Actin

Figure 3

## INTERNATIONAL SEARCH REPORT

International application No.
**PCT/CN2022/126516**

**A. CLASSIFICATION OF SUBJECT MATTER**

C07D 403/14(2006.01)i; A61K 31/4439(2006.01)i; A61K 31/4035(2006.01)i; A61P 31/00(2006.01)i; A61P 5/00(2006.01)i; A61P 9/00(2006.01)i; A61P 11/00(2006.01)i; A61P 35/00(2006.01)i; A61P 37/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, WPI, EPODOC, CNKI, STN(REGISTRY, MARPAT, CAPLUS): 小脑蛋白, 泛素, 异吲哚, CRBN, cereblon

**C. DOCUMENTS CONSIDERED TO BE RELEVANT**

| Category* | Citation of document, with indication, where appropriate, of the relevant passages | Relevant to claim No. |
|---|---|---|
| X | WO 2021194879 A1 (ARVINAS OPERATIONS INC.) 30 September 2021 (2021-09-30) abstract, description, pp. 135-275, and claims 1-28 | 1-8, 20-32, 34-62 |
| X | WO 2021194878 A1 (ARVINAS OPERATIONS INC.) 30 September 2021 (2021-09-30) abstract, description, pp. 201-281, paragraph [00260], and claims 1-47 | 1-15, 17-32, 34-62 |
| X | WO 2021170109 A1 (CULLGEN SHANGHAI INC.) 02 September 2021 (2021-09-02) abstract, embodiments 232 and 277, and claims 1-67 | 20-32, 34-38, 41-43, 49-50, 54-55, 60-62 |
| X | WO 2021011913 A1 (ARVINAS OPERATIONS INC.) 21 January 2021 (2021-01-21) abstract, description, pp. 277-829, and claims 1-47 | 1-15, 17-32, 34-62 |
| X | DEGORCE, S. L. et al. "Discovery of Proteolysis-Targeting Chimera Molecules that Selectively Degrade the IRAK3 Pseudokinase" *Journal of Medicinal Chemistry,* Vol. 63, No. 18, 17 August 2020 (2020-08-17), pp. 10460-10473 | 20-32, 34-38, 41-43, 49-50, 54-55, 60-62 |
| X | HAN, Xin et al. "Strategies toward Discovery of Potent and Orally Bioavailable Proteolysis Targeting Chimera Degraders of Androgen Receptor for the Treatment of Prostate Cancer" *Journal of Medicinal Chemistry,* Vol. 64, No. 17, 03 September 2021 (2021-09-03), pp. 12831-12854 | 20-32, 34-38, 41-43, 49-50, 54-55, 60-62 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 January 2023** | **11 January 2023** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/126516**

**C. DOCUMENTS CONSIDERED TO BE RELEVANT**

| Category* | Citation of document, with indication, where appropriate, of the relevant passages | Relevant to claim No. |
|---|---|---|
| X | WO 2020162725 A1 (KOREA RESEARCH INSTITUTE OF CHEMICAL TECHNOLOGY et al.) 13 August 2020 (2020-08-13) abstract, description, pp. 65-231, and claims 1-14 | 20-32, 34-38, 41-43, 49-50, 54-55, 60-62 |
| X | CN 110741004 A (ARVINAS OPERATIONS, INC. et al.) 31 January 2020 (2020-01-31) abstract, description, paragraphs [1632]-[1633], figure 2, and claims 1-42 | 1-15, 17-32, 34-62 |
| X | CN 113164775 A (ARVINAS OPERATIONS, INC. et al.) 23 July 2021 (2021-07-23) abstract, description, paragraphs [1566]-[1567], pp. 282-661, and claims 1-42 | 1-15, 17-32, 34-62 |
| X | CN 112888681 A (CULLGEN (SHANGHAI), INC.) 01 June 2021 (2021-06-01) claims 1-144, description, embodiments 1-297, and table 1 | 20-32, 34-38, 41-43, 49-50, 54-55, 60-62 |
| X | CN 112262134 A (ARVINAS OPERATIONS, INC.) 22 January 2021 (2021-01-22) abstract, claims 1-38, and description, pp. 180-240 | 1-8, 20-32, 34-62 |
| X | CN 112218859 A (ARVINAS OPERATIONS, INC. et al.) 12 January 2021 (2021-01-12) abstract, claims 1-37, and description, paragraph [3738] | 20-32, 34-38, 41-43, 49-50, 54-55, 60-62 |
| X | WO 2019099926 A1 (ARVINAS INC.) 23 May 2019 (2019-05-23) abstract, claims 1-45, and description, paragraph [00193] | 20-32, 34-38, 41-43, 49-50, 54-55, 60-62 |
| X | CN 110612294 A (ARVINAS OPERATIONS, INC.) 24 December 2019 (2019-12-24) abstract, claims 1-34, and description, pp. 166-267 | 1-8, 20-32, 34-62 |
| X | CN 110612297 A (ARVINAS OPERATIONS, INC.) 24 December 2019 (2019-12-24) abstract, claims 1-31, and description, pp. 259-343 | 1-15, 17-32, 34-62 |
| X | WO 2018119357 A1 (ARVINAS INC.) 28 June 2018 (2018-06-28) abstract, claims 1-33, and description, pp. 299-370 | 20-32, 34-38, 41-43, 49-50, 54-55, 60-62 |
| X | CN 110291087 A (ARVINAS OPERATIONS, INC.) 27 September 2019 (2019-09-27) abstract, claims 1-31, and description, pp. 316-418, and figures 5-6 | 1-15, 17-32, 34-62 |
| X | CN 110506039 A (ARVINAS INC.) 26 November 2019 (2019-11-26) abstract, claims 1-34, and description, pp. 98-201 | 1-8, 20-32, 34-62 |
| X | REGISTRY. "RN: 2703770-66-3, 2703771-42-8 et al." *STN,* 30 September 2021 (2021-09-30), | 1-3, 5-8, 51-53 |
| X | REGISTRY. "RN: 2714477-27-5, 2682112-69-0 et al." *STN,* 19 October 2021 (2021-10-19), | 9-15, 17-19, 51-53 |
| X | REGISTRY. "RN: 2714476-17-0, 2714476-16-9 et al." *STN,* 19 October 2021 (2021-10-19), | 20-32, 34-38, 54-55 |
| E | WO 2022221673 A1 (ARVINAS OPERATIONS INC.) 20 October 2022 (2022-10-20) description, embodiments 1-65, and table 1 | 9-15, 17-32, 34-62 |
| PX | WO 2022198112 A1 (ARVINAS OPERATIONS INC.) 22 September 2022 (2022-09-22) description paragraphs [00255]-[00648] | 1-15, 17-32, 34-62 |
| PX | WO 2022133184 A1 (NEWAVE PHARMACEUTICAL INC.) 23 June 2022 (2022-06-23) description, pp. 128-179, and claims 1-20 | 20-32, 34-38, 41-43, 49-50, 54-55, 60-62 |
| PX | WO 2022109396 A1 (FOGHORN THERAPEUTICS INC.) 27 May 2022 (2022-05-27) abstract, description, embodiments 55-56, and claims 1-116 | 1-15, 17-32, 34-62 |
| PX | WO 2022098544 A1 (ARVINAS OPERATIONS INC.) 12 May 2022 (2022-05-12) abstract, claims 1-21, and description, paragraph [00179], and table 1 | 1-8, 20-32, 34-62 |
| PX | WO 2022087125 A1 (ARVINAS OPERATIONS INC.) 28 April 2022 (2022-04-28) abstract, claims 1-24, and description, paragraphs [00195]-[00267] | 1-8, 20-32, 34-62 |
| PX | WO 2022047145 A1 (ARVINAS OPERATIONS INC.) 03 March 2022 (2022-03-03) abstract, claims 1-34, and description, table 1 | 20-32, 34-38, 41-43, 49-50, 54-55, 60-62 |

Form PCT/ISA/210 (second sheet) (January 2015)

**INTERNATIONAL SEARCH REPORT**

International application No.
**PCT/CN2022/126516**

**C. DOCUMENTS CONSIDERED TO BE RELEVANT**

| Category* | Citation of document, with indication, where appropriate, of the relevant passages | Relevant to claim No. |
|---|---|---|
| PX | REGISTRY. "RN: 2776896-94-5, 2776897-23-3 et al." *STN,* 23 June 2022 (2022-06-23), | 1-3, 5-8, 51-53 |
| PX | REGISTRY. "RN: 2839672-28-3, 2839672-18-1 et al." *STN,* 14 October 2022 (2022-10-14), | 9-15, 17-19, 51-53 |
| PX | REGISTRY. "RN: 2839757-07-0, 2839666-96-3 et al." *STN,* 17 October 2022 (2022-10-17), | 20-32, 34-38, 54-55 |

Form PCT/ISA/210 (second sheet) (January 2015)

**INTERNATIONAL SEARCH REPORT**

International application No.
**PCT/CN2022/126516**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **56-62**
because they relate to subject matter not required to be searched by this Authority, namely:

[1] Claims 56-62 relate to a method for treatment of a disease, and in the search report, a search was conducted on the basis of a corresponding pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/126516**

| Patent document cited in search report | Publication date (day/month/year) | Patent family member(s) | Publication date (day/month/year) |
|---|---|---|---|
| WO 2021194879 A1 | 30 September 2021 | IL 296648 A | 01 November 2022 |
| | | CO 2022014718 A2 | 31 October 2022 |
| | | US 2021315896 A1 | 14 October 2021 |
| | | AU 2021244180 A1 | 03 November 2022 |
| WO 2021194878 A1 | 30 September 2021 | US 2022064168 A1 | 03 March 2022 |
| WO 2021170109 A1 | 02 September 2021 | BR 112022016901 A2 | 06 December 2022 |
| | | CA 3173262 A1 | 02 September 2021 |
| | | IL 295799 A | 01 October 2022 |
| | | AU 2021225981 A1 | 29 September 2022 |
| WO 2021011913 A1 | 21 January 2021 | US 2022315575 A1 | 06 October 2022 |
| | | CN 114867727 A | 05 August 2022 |
| | | EP 3999182 A1 | 25 May 2022 |
| | | JP 2022540935 A | 20 September 2022 |
| WO 2020162725 A1 | 13 August 2020 | US 2022105188 A1 | 07 April 2022 |
| | | EP 3922632 A1 | 15 December 2021 |
| | | KR 20200097222 A | 18 August 2020 |
| CN 110741004 A | 31 January 2020 | RU 2019121565 A3 | 14 January 2021 |
| | | BR 112019012878 A2 | 26 November 2019 |
| | | KR 20190102019 A | 02 September 2019 |
| | | AU 2021200099 A1 | 18 March 2021 |
| | | JP 2020504741 A | 13 February 2020 |
| | | WO 2018119448 A1 | 28 June 2018 |
| | | CO 2019007894 A2 | 09 October 2019 |
| | | MX 2019007649 A | 10 September 2019 |
| | | IL 267410 A | 29 August 2019 |
| | | US 2018179183 A1 | 28 June 2018 |
| | | AU 2017382436 A1 | 04 July 2019 |
| | | IL 294423 A | 01 August 2022 |
| | | CA 3047784 A1 | 28 June 2018 |
| | | EP 3558994 A1 | 30 October 2019 |
| CN 113164775 A | 23 July 2021 | EP 3846907 A1 | 14 July 2021 |
| | | KR 20210073519 A | 18 June 2021 |
| | | AU 2019335516 A1 | 11 March 2021 |
| | | WO 2020051564 A1 | 12 March 2020 |
| | | JP 2022500368 A | 04 January 2022 |
| | | CA 3109981 A1 | 12 March 2020 |
| | | AU 2022228150 A1 | 29 September 2022 |
| | | IL 281188 A | 29 April 2021 |
| CN 112888681 A | 01 June 2021 | KR 20210069634 A | 11 June 2021 |
| | | CA 3110267 A1 | 27 February 2020 |
| | | US 2021363146 A1 | 25 November 2021 |
| | | BR 112021003098 A2 | 11 May 2021 |
| | | AU 2019323446 A1 | 15 April 2021 |
| | | EP 3841098 A1 | 30 June 2021 |
| | | WO 2020038415 A1 | 27 February 2020 |
| | | IL 280984 A | 29 April 2021 |
| | | JP 2021535104 A | 16 December 2021 |
| CN 112262134 A | 22 January 2021 | JP 2021521192 A | 26 August 2021 |
| | | RU 2020136948 A | 16 May 2022 |

Form PCT/ISA/210 (patent family annex) (January 2015)

**INTERNATIONAL SEARCH REPORT**
**Information on patent family members**

International application No.
**PCT/CN2022/126516**

| Patent document cited in search report | | | Publication date (day/month/year) | Patent family member(s) | | | Publication date (day/month/year) |
|---|---|---|---|---|---|---|---|
| | | | | WO | 2019199816 | A1 | 17 October 2019 |
| | | | | KR | 20210003804 | A | 12 January 2021 |
| | | | | EP | 3774772 | A1 | 17 February 2021 |
| | | | | AU | 2019251223 | A1 | 26 November 2020 |
| | | | | IL | 277934 | A | 30 November 2020 |
| | | | | CA | 3095912 | A1 | 17 October 2019 |
| CN | 112218859 | A | 12 January 2021 | AU | 2019249231 | A1 | 15 October 2020 |
| | | | | AU | 2022206739 | A1 | 11 August 2022 |
| | | | | US | 2019315732 | A1 | 17 October 2019 |
| | | | | JP | 2021521112 | A | 26 August 2021 |
| | | | | WO | 2019195609 | A2 | 10 October 2019 |
| | | | | IL | 277729 | A | 30 November 2020 |
| | | | | BR | 112020020307 | A2 | 12 January 2021 |
| | | | | KR | 20210006356 | A | 18 January 2021 |
| | | | | CA | 3095494 | A1 | 10 October 2019 |
| | | | | RU | 2020136178 | A3 | 06 May 2022 |
| | | | | EP | 3774777 | A2 | 17 February 2021 |
| | | | | CO | 2020013864 | A2 | 10 December 2020 |
| WO | 2019099926 | A1 | 23 May 2019 | US | 2019151295 | A1 | 23 May 2019 |
| | | | | EP | 3710443 | A1 | 23 September 2020 |
| CN | 110612294 | A | 24 December 2019 | KR | 20190116315 | A | 14 October 2019 |
| | | | | CO | 2019009424 | A2 | 28 February 2020 |
| | | | | JP | 2020506922 | A | 05 March 2020 |
| | | | | BR | 112019015484 | A2 | 28 April 2020 |
| | | | | AU | 2022221386 | A1 | 15 September 2022 |
| | | | | EP | 3577109 | A1 | 11 December 2019 |
| | | | | CA | 3050309 | A1 | 09 August 2018 |
| | | | | IL | 268069 | A | 26 September 2019 |
| | | | | RU | 2019123462 | A | 27 January 2021 |
| | | | | MX | 2019009046 | A | 30 October 2019 |
| | | | | AU | 2018215212 | A1 | 11 July 2019 |
| | | | | WO | 2018144649 | A1 | 09 August 2018 |
| | | | | US | 2018215731 | A1 | 02 August 2018 |
| CN | 110612297 | A | 24 December 2019 | RU | 2019123537 | A | 25 January 2021 |
| | | | | EP | 3573977 | A1 | 04 December 2019 |
| | | | | CO | 2019009145 | A2 | 17 January 2020 |
| | | | | AU | 2018211975 | A1 | 11 July 2019 |
| | | | | WO | 2018140809 | A1 | 02 August 2018 |
| | | | | BR | 112019015312 | A2 | 10 March 2020 |
| | | | | US | 2022267305 | A1 | 25 August 2022 |
| | | | | AU | 2022221407 | A1 | 15 September 2022 |
| | | | | MX | 2019008934 | A | 05 November 2019 |
| | | | | KR | 20190113842 | A | 08 October 2019 |
| | | | | JP | 2020506914 | A | 05 March 2020 |
| | | | | IL | 268011 | A | 26 September 2019 |
| | | | | CA | 3049912 | A1 | 02 August 2018 |
| | | | | US | 2020199107 | A1 | 25 June 2020 |
| | | | | US | 2018237418 | A1 | 23 August 2018 |
| WO | 2018119357 | A1 | 28 June 2018 | EP | 3558974 | A1 | 30 October 2019 |

Form PCT/ISA/210 (patent family annex) (January 2015)

**INTERNATIONAL SEARCH REPORT**
**Information on patent family members**

International application No.
**PCT/CN2022/126516**

| Patent document cited in search report | Publication date (day/month/year) | Patent family member(s) | Publication date (day/month/year) |
|---|---|---|---|
| | | US 2022378726 A1 | 01 December 2022 |
| | | US 2018177750 A1 | 28 June 2018 |
| CN 110291087 A | 27 September 2019 | EP 3689868 A1 | 05 August 2020 |
| | | IL 266842 A | 31 July 2019 |
| | | WO 2018102725 A1 | 07 June 2018 |
| | | EP 3548483 A1 | 09 October 2019 |
| | | CO 2019007091 A2 | 09 October 2019 |
| | | AU 2020201793 A1 | 26 March 2020 |
| | | KR 20190082989 A | 10 July 2019 |
| | | RU 2019120120 A | 28 December 2020 |
| | | BR 112019011200 A2 | 08 October 2019 |
| | | US 2021139458 A1 | 13 May 2021 |
| | | CA 3042968 A1 | 07 June 2018 |
| | | JP 2020504089 A | 06 February 2020 |
| | | IL 297717 A | 01 December 2022 |
| | | AU 2017366693 A1 | 18 April 2019 |
| | | AU 2021204549 A1 | 05 August 2021 |
| | | IL 272527 A | 31 March 2020 |
| | | US 2022274955 A1 | 01 September 2022 |
| | | JP 2020100659 A | 02 July 2020 |
| | | IL 284424 A | 29 July 2021 |
| | | AU 2021257895 A1 | 18 November 2021 |
| | | US 10899742 B1 | 26 January 2021 |
| | | US 2018155322 A1 | 07 June 2018 |
| | | CN 114656452 A | 24 June 2022 |
| | | KR 20200020988 A | 26 February 2020 |
| | | MX 2019006402 A | 11 September 2019 |
| | | RU 2020106142 A | 13 April 2020 |
| | | JP 2021169479 A | 28 October 2021 |
| | | CN 111454248 A | 28 July 2020 |
| | | JP 2022023103 A | 07 February 2022 |
| CN 110506039 A | 26 November 2019 | JP 2019530715 A | 24 October 2019 |
| | | AU 2021204556 A1 | 05 August 2021 |
| | | JP 2021169481 A | 28 October 2021 |
| | | US 2022259154 A1 | 18 August 2022 |
| | | KR 20200020978 A | 26 February 2020 |
| | | US 2021171470 A1 | 10 June 2021 |
| | | MX 2019004278 A | 05 August 2019 |
| | | US 2018099940 A1 | 12 April 2018 |
| | | JP 2020100649 A | 02 July 2020 |
| | | KR 20190055260 A | 22 May 2019 |
| | | EP 3660004 A1 | 03 June 2020 |
| | | RU 2019113229 A3 | 02 November 2020 |
| | | JP 2022046497 A | 23 March 2022 |
| | | IL 265806 A | 30 June 2019 |
| | | AU 2020201792 A1 | 26 March 2020 |
| | | US 2021009528 A1 | 14 January 2021 |
| | | AU 2021277645 A1 | 23 December 2021 |
| | | US 2020095205 A1 | 26 March 2020 |
| | | CO 2019003642 A2 | 19 June 2019 |

Form PCT/ISA/210 (patent family annex) (January 2015)

**INTERNATIONAL SEARCH REPORT**
**Information on patent family members**

International application No.
**PCT/CN2022/126516**

| Patent document cited in search report | | | Publication date (day/month/year) | Patent family member(s) | | | Publication date (day/month/year) |
|---|---|---|---|---|---|---|---|
| | | | | CA | 3038979 | A1 | 19 April 2018 |
| | | | | AU | 2017341723 | A1 | 04 April 2019 |
| | | | | WO | 2018071606 | A1 | 19 April 2018 |
| | | | | US | 2021040044 | A1 | 11 February 2021 |
| | | | | IL | 283761 | A | 29 July 2021 |
| | | | | IL | 272523 | A | 31 March 2020 |
| | | | | EP | 3526202 | A1 | 21 August 2019 |
| | | | | CN | 111892577 | A | 06 November 2020 |
| WO | 2022221673 | A1 | 20 October 2022 | None | | | |
| WO | 2022198112 | A1 | 22 September 2022 | None | | | |
| WO | 2022133184 | A1 | 23 June 2022 | None | | | |
| WO | 2022109396 | A1 | 27 May 2022 | None | | | |
| WO | 2022098544 | A1 | 12 May 2022 | US | 2022144809 | A1 | 12 May 2022 |
| WO | 2022087125 | A1 | 28 April 2022 | None | | | |
| WO | 2022047145 | A1 | 03 March 2022 | TW | 202214598 | A | 16 April 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Clinical Pharmacology. People's Public Health Press, 2008
- *CHEMICAL ABSTRACTS,* 768-94-5